# EUROPEAN PATENT APPLICATION

(11) **EP 4 378 936 A1**
(43) Date of publication of application: **05.06.2024**
(21) Application number: 22848701.3
(22) Date of filing: 29.07.2022
(51) Int. Cl.: C07D 401/04, C07D 401/14, A61K 31/496, A61K 31/4439, A61P 35/00

(54) **BIFUNCTIONAL CHIMERIC HETEROCYCLIC COMPOUND AND USE THEREOF AS ANDROGEN RECEPTOR DEGRADER**

(30) Priority: 30.07.2021 CN 202110875927
(71) Applicant: Hinova Pharmaceuticals Inc., Chengdu, Sichuan 610041 (CN)
(72) Inventor: DU, Wu, Chengdu, Sichuan 610041 (CN); LI, Xinhai, Chengdu, Sichuan 610041 (CN); WEN, Kun, Chengdu, Sichuan 610041 (CN); QIN, Dekun, Chengdu, Sichuan 610041 (CN); ZHANG, Shaohua, Chengdu, Sichuan 610041 (CN); CHEN, Song, Chengdu, Sichuan 610041 (CN); DUAN, Jingyi, Chengdu, Sichuan 610041 (CN); LV, Haibin, Chengdu, Sichuan 610041 (CN); LI, Haibo, Chengdu, Sichuan 610041 (CN); LI, Yu, Chengdu, Sichuan 610041 (CN); HE, Jinyun, Chengdu, Sichuan 610041 (CN); CHEN, Muyang, Chengdu, Sichuan 610041 (CN); LIU, Shijuan, Chengdu, Sichuan 610041 (CN); FU, Yiwei, Chengdu, Sichuan 610041 (CN); GUAN, Yikai, Chengdu, Sichuan 610041 (CN); TU, Zhilin, Chengdu, Sichuan 610041 (CN)
(74) Representative: Altmann Stößel Dick Patentanwälte PartG mbB
(86) International application number: PCT/CN2022/109155
(87) International publication number: WO 2023/006097

(57) **Abstract**

A compound as shown in formula I, or an optical isomer thereof, a solvate thereof, a pharmaceutically acceptable salt thereof, a prodrug thereof, a tautomer thereof, a mesomer thereof, a racemate thereof, an enantiomer thereof, a diastereomer thereof, a mixture form thereof, a metabolite thereof, a metabolic precursor thereof, or an isotope substitute form thereof. TB is a target recognition/binding portion, L is a linking portion, and U is a ubiquitin protease recognition/binding portion, the three portions being connected by means of chemical bonds. The compound can significantly down-regulate mutated androgen receptor (AR) proteins, and has good inhibitory activity on prostate cancer cell lines. The compound of formula I can be used for preparation of a protein degradation targeted chimera for targeted regulation of androgen receptors, and a drug for treating diseases correlated with regulation by androgen receptors, and has particularly good application prospects in drugs for treating prostate cancers and breast cancers.

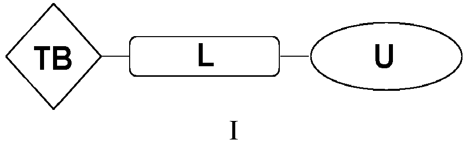

## Description

### Field of the invention

The present invention belongs to the field of medicinal synthesis, and specifically relates to a bifunctional chimeric heterocyclic compound and uses thereof.

### Background of the invention

As the growing and aging of global population, the incidence of prostate cancer continues to increase, and currently, the main treatment is androgen-deprivation therapy. Androgen receptor (AR) belongs to the nuclear receptor family and is a type of ligand-dependent transcription factor. The abnormal regulation of AR signaling pathway plays an important role in the occurrence and development of prostate cancer. It has been shown that castration-resistant prostate cancer (CRPC) still depends on the action of AR. AR contains 918 amino acids, and has a similar structure and function to other nuclear receptors. AR consists of three important domains, namely the DNA binding domain (DBD), the ligand binding domain (LBD), and the N-terminal domain (NTD), in which DBD and LBD are linked by a hinge region. LBD in the C-terminal of AR is the site where AR binds to the ligand, that determines the specificity for binding of a ligand to AR, and the binding of the ligand to LBD may activate AR. Until now, two transcriptional activation domains have been identified in AR, namely the activation function 1 (AF1) in the NTD domain and the highly conserved hydrophobic pocket activation function 2 (AF2) in the LBD domain. Before 2010, docetaxel-based chemotherapy was the only treatment that could prolong the survival of patients with metastatic CRPC. Since 2011, FDA has successively approved three inhibitors on AR signaling pathways, including abiraterone acetate and enzalutamide, which were respectively allowed in 2011 and 2012 for the treatment of metastatic CRPC, as well as apalutamide, which was just approved in 2018 for non-metastatic CRPC.

Despite some success in clinical treatment with the second-generation AR signaling pathway inhibitors abiraterone and enzalutamide, drug resistance has emerged in clinical practice. The F876L mutation in the ligand-binding region is a missense mutation that develops the resistance to enzalutamide, causing it to transition from an antagonist to an agonist. In addition, the splicing mutants of AR, especially AR-v7 mutations with the absence of ligand-binding regions, are important reasons for mediating the resistance to the second-generation mdicaments. Therefore, there is an urgent need for novel inhibitors on AR signaling pathways in clinical practice to treat CRPC.

Traditional small molecule inhibitors inhibit the function of target proteins by binding to them. However, long-term use of small molecule drugs inevitably leads to drug resistance. In order to achieve the desired effect, it is necessary for small molecule compounds to maintain a certain concentration in cells, and higher concentrations of small molecules can cause adverse reactions due to off-target effects. Therefore, finding small molecule compounds that can overcome these defects is of great significance in the development of new drugs.

In recent years, proteolytic targeting chimeras (PROTACs) have attracted widespread attention as small molecules that can induce degradation of target proteins. PROTACs, as bifunctional molecules, include a small molecule compound that can bind to the protein of interest (POI), a linker introduced at its suitable position, and a small molecule compound which can bind to E3 ubiquitin ligases. PROTACs, as a small molecule probe, can simultaneously bind to the target protein and E3 ubiquitin ligases, thereby promoting the ubiquitination of the target protein, by which the protein can be recognized and degraded by the proteasome

Over the past decade, it has been shown that many tumor-related target proteins (including AR, ER, BRD4, ERRa, RIPK2, etc.) can be regulated and degraded by PROTACs. Moreover, the latest research has confirmed the catalytic properties of PROTACs, indicating that chimeric molecules, at the concentration lower than the required concentration for a single inhibitor, can achieve the same therapeutic effect. Therefore, a novel tumor-treatment strategy using PROTACs to induce protein degradation can regulate target protein levels by the intracellular ubiquitin-proteasome system (UPS), thereby overcoming the shortcomings of traditional small molecule inhibitors.

However, nowadays, treating drug-resistant cancer remains a key and difficult point in cancer treatment. If the resistance to the available medicaments is developed, there will be no drugs used for treating patients, leading to an increased mortality rate. AR mutations (including point mutations and splicing mutations) are important causes of drug resistance, and there are still few reports on the inhibitors of the main splicing mutation, i.e. AR-v7 mutation, as a therapeutic target. Therefore, the development of novel inhibitors for treating drug-resistant cancer by degrading AR-v7 has great application prospects

### Summary of the invention

The present invention provides a compound represented by formula I, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof: 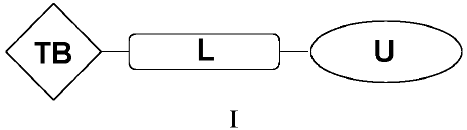 wherein TB is a target recognition/binding moiety, L is a linker, and U is a ubiquitin protease recognition/binding moiety; the three moieties are linked by a chemical bond.

Wherein, the above TB moiety has a structure represented by formula I-A: 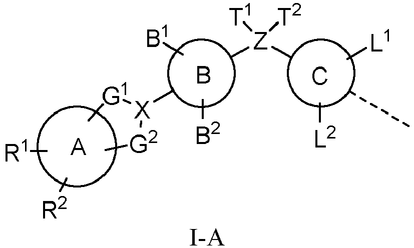
wherein, A is absent, aromatic ring, heteroaromatic ring, non-aromatic heterocycle, non-aromatic carbon ring, bridged ring, spiral ring, fused heterocycle, and fused heteroaromatic ring; the aromatic ring comprises a benzene ring; the heteroaromatic ring comprises 5-6 membered heteroaromatic rings; the non-aromatic heterocycles comprise 3-7 membered non-aromatic heterocycles; the non-aromatic carbon ring comprises 3-7 membered non-aromatic carbon rings; the fused heterocycle comprises a (5-6-membered heterocycle)-fused 5-6-membered heterocycle; the fused heteroaromatic ring comprises a (5-6-membered heteroaromatic ring)-fused 5-6-membered heterocycle, and a (5-6-membered heteroaromatic ring)-fused 5-6-membered heteroaromatic ring;
wherein, R¹ and R² are each independently selected from the group consisting of absence, hydrogen, halogen, cyano, amino, hydroxyl, C1-C6 alkoxy, C1-C6 alkylamino, C1-C6 alkylthiol, C1-C6 alkylsulfonyl, C1-C6 alkylsulfinyl, C1-C6 alkylcarbonyl, C1-C6 alkylaminocarbonyl, - NR³R⁴, -CR^{a}R³R⁴, -NR³-CO-R⁴, -CO-NR³R⁴, -NR³-SO₂-R⁴, -SO₂-NR³R⁴, -CO-R³, -SO-R³, - SO₂-R³, -CR³=CH₂, -OR³, -SR³, substituted or unsubstituted C1-C6 alkyl, substituted or unsubstituted 3-8-membered cycloalkyl, substituted or unsubstituted heterocyclyl, substituted or unsubstituted fused heterocyclyl, substituted or unsubstituted spiro-heterocyclyl, substituted or unsubstituted aromatic heterocyclyl, and substituted or unsubstituted phenyl; R¹ and R² can be linked to form a ring;
wherein, the substituted substituents are R^{a}, R³, and R⁴, which are each independently selected from the group consisting of hydrogen, halogen, hydroxyl, amino, cyano, C1-C6 alkenyl, C1-C6 alkoxy, C1-C6 alkylamino, C1-C6 alkylsulfonyl, substituted or unsubstituted C1-C6 alkyl, substituted or unsubstituted 3-8-membered cycloalkyl, substituted or unsubstituted 3-8-membered heterocyclyl, substituted or unsubstituted aromatic heterocyclyl, substituted or unsubstituted phenylcarbonyl, and sulfonyl; any two of R^{a}, R³, and R⁴ can be linked to form a 3-8-membered ring; the substituted substituents are selected from the group consisting of halogen, amino, hydroxyl, and hydroxyl-substituted C1-6 alkyl;
wherein, G¹ and G² are each independently linked to A by a chemical bond; wherein G¹ is selected from the group consisting of absence, -(CH₂)_{y}-, -(CH₂)ₘ-CR^{g1}R^{g2}-, -(CH₂)ₘ-CO-, O, S, SO, SO₂, and NR^{g1}; G² is selected from the group consisting of absence, -(CH₂)ₙ-CR^{g3}R^{g4}-, - (CH₂)ₙ-O-, O, S, SO, SO₂, and NR^{g2}; wherein y, m, and n are integers from 0 to 3; R^{g1}, R^{g2}, R^{g3}, and R^{g4} are each independently selected from the group consisting of H, C1-C6 alkyl, halogen, and hydroxyl; R^{g1} and R^{g2}, R^{g3} and R^{g4} can be linked to form a ring; when G1 and G2 are both absent, X is directly linked to A by a chemical bond;
wherein, X is selected from the group consisting of -N-, -NR⁵-, -O-, -S-, -CO-, -SO-, -SO₂-, -CONR⁵-, -NR⁵CO-, -CH-, -CHR⁵-, and -CR⁵R^{5'}-; said R⁵ and R^{5'} are each independently selected from the group consisting of H and C1-C6 alkyl;
wherein, rings B and C are each independently selected from the group consisting of substituted or unsubstituted benzene ring, thiophene, pyridine, pyrimidine, 5-6-membered aromatic heterocycle, 3-8-membered cycloalkane, and 3-8-membered heterocycle; the substituted substituents are halogen and cyano;
wherein, B¹ and B² are each independently selected from the group consisting of H, halogen, and C1-C6 alkyl; or B¹, B² and X are linked to form a ring;
wherein, Z is selected from the group consisting of -C-, -CO-, -CH-, -CH₂-, -O-, -N-, -S-, - SO-, and -SO₂-;
wherein, T¹ and T² are each independently selected from the group consisting of absence, H, hydroxyl, amino, substituted or unsubstituted C1-C6 alkyl, C1-C6 oxaalkyl, C1-C6 azaalkyl, C3-C6 cycloalkyl, acyl, C1-C6 alkoxy, and C1-C6 alkylamino; or T¹ and T² can be linked to each other to form a ring; the substituted substituent is hydroxyl or amino;
wherein, L¹ and L² are each independently selected from the group consisting of H, halogen, cyano, amino, hydroxyl, and C1-C6 alkyl;
wherein, said L moiety has a structure as represented by formula I-L: 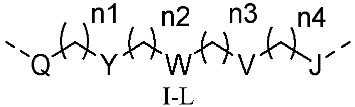
wherein, Q, J, Y, W, and V are each independently selected from the group consisting of absence, -O-, -S-, -SO-, -SO₂-, -NR^{q1}-, -NR^{j1}-, -C≡C-, -C=C-, -NR^{q1}CO-, -NR^{j1}CO-, -CO-, - CONH-, -NR^{q11}SO₂-, -CR^{q1}R^{q2}-, -CR^{y1}R^{y2}-, -CR^{w1}R^{w2}- , -CR^{v1}R^{v2}-, -CR^{j1}R^{j2}-, - [(OCH₂CH₂O)₂]ₙ₅-, -[(OCH₂CH₂O)₃]ₙ₆-, (R^{c},R^{d})-substituted or unsubstituted 3-7-membered cycloalkyl, (R^{c},R^{d})-substituted or unsubstituted 3-7-membered heterocyclyl, (R^{c},R^{d})-substituted or unsubstituted phenyl, (R^{c},R^{d})-substituted or unsubstituted aromatic heterocyclyl, (R^{c},R^{d})-substituted or unsubstituted fused aromatic heterocyclyl; said R^{c} and R^{d} are each independently selected from H, halogen, and C1-3 alkyl; or R^{c} and R^{d} can be linked to each other to form a ring;
wherein, R^{q1}, R^{q2}, R^{y1}, R^{y2}, R^{w1}, R^{w2}, R^{v1}, R^{v2}, R^{j1}, and R^{j2} are each independently selected from the group consisting of H; halo-substituted or unsubstituted C1-C6 alkyl, C3-C6 cycloalkyl, C1-C6 oxaalkyl, C1-C6 azaalkyl, C3-C6 oxacycloalkyl, and C3-C6 azacycloalkyl;
wherein, R^{q1} and R^{q2}, R^{y1} and R^{y2}, R^{w1} and R^{w2}, R^{v1} and R^{v2}, and R^{j1} and R^{j2} can be linked to each other to form a ring;
wherein, n1, n2, n3, n4, n5, and n6 are each independently selected from an integer of 0 to 6;
wherein, Q and J can freely linked to TB moiety or U moiety;
wherein, said U moiety has a structure as represented by formula I-U: 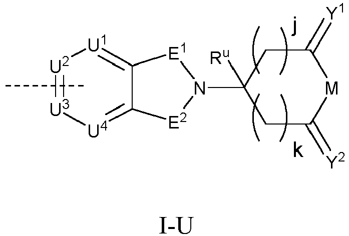
wherein, M is selected from the group consisting of -O-, -S-, -CR^{m}-, and -NR^{m}-; wherein, R^{m} is selected from the group consisting of H, C₁₋₆ alkyl, C₃₋₆ cycloalkyl, and C₃₋₆ heteocyclyl, and 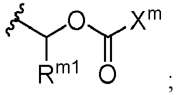
said R^{m1} is selected from the group consisting of H, C₁₋₆ alkyl, and C₃₋₆ cycloalkyl;
X^{m} is selected from the group consisting of -CR^{m2}R^{m3}-, -OR^{m2}-, and -NR^{m2}R^{m3}-, wherein, R^{m2} and R^{m3} are each independently selected from the group consisting of H, C₁₋₆ alkyl, C₃₋₆ cycloalkyl, and C₃₋₆ heteocyclyl, and C₁₋₆ oxaalkyl; R^{m2} and R^{m3} can be linked to form a ring;
E¹ and E² are each independently selected from the group consisting of -CO-, -CS-, -NR^{e1}-, -O-, -S-, -SO₂-, -CH₂-, -CD₂-, -CR^{e2}R^{e3}-, 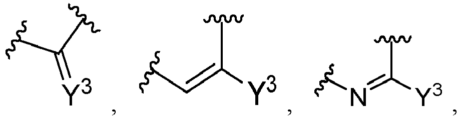 and 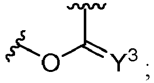 R^{e1}, R^{e2}, and R^{e3} are each independently selected from the group consisting of C₁₋₆ alkyl, H, halogen, hydroxyl, and amino;
Y¹, Y², and Y³ are each independently selected from the group consisting of H, O, S, and C₁₋₃ alkyl;
J and k are each independently selected from an integer of 0 to 3, and J and k are not both 0;
U¹, U², U³, and U⁴ are each independently selected from the group consisting of O, S, N, - CR^{g1}-, -CR^{g2}-, -CR^{g3}-, and -CR^{g4}-, wherein R^{g1}, R^{g2}, R^{g3}, and R^{g4} are each independently selected from the group consisting of H, halogen, hydroxyl, amino, thiol, sulfonyl, sulfinyl, nitro, cyano, CF₃, heterocyclyl, C₁₋₆ alkyl, C₃₋₆ cycloalkyl, C₁₋₆ alkoxy, C₁₋₆ alkylamino, C₂₋₆ alkenyl, and C₂₋₆ alkynyl.

Further, G¹ and G² in TB moiety of above compounds are linked to different atoms in A by a chemical bond, as represented by formula II-A; or, G¹ and G² in TB moiety are linked to a same atom in A by a chemical bond, as represented by formula II-B:

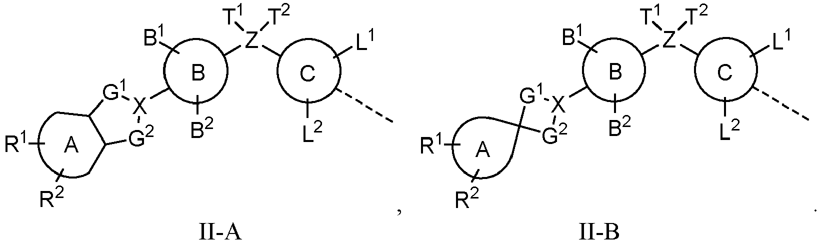

More further, in the above compounds, A is an aromatic heterocycle, G¹ and G² are not absent, and the TB moiety in the compounds has a structure as represented by formula III-A: 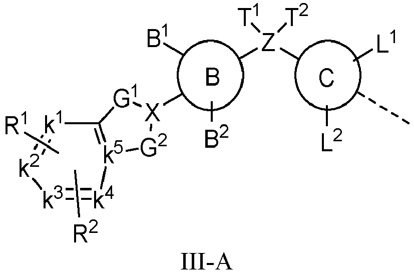 wherein, k¹, k², k³, k⁴, and k⁵ are each independently selected from CH or N, but they are not CH at the same time.

More further, in the above compounds, G¹ is -CH₂-, G² is -CH₂- or -CH₂CH₂-, and the TB moiety in the compound has a structure as represented by formula IV-A or IV-B: 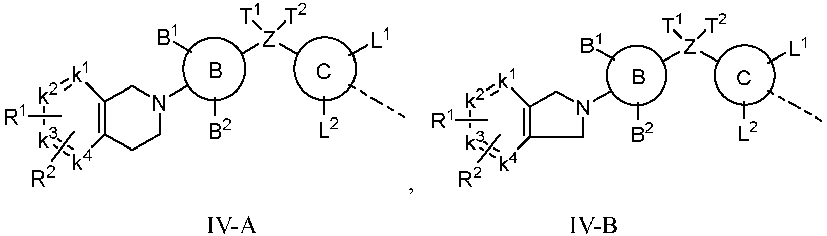 wherein, k¹, k², k³, and k⁴ are each independently selected from CH or N, but they are not CH at the same time.

Further, in the above compounds, A is an aromatic heterocycle, G² is absent, and the TB moiety in the compound has a structure as represented by formula V-A: 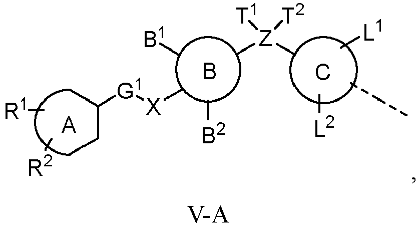
wherein, G¹ is not absent;
alternatively, the TB moiety in the compound has a structure as represented by formula V-B:

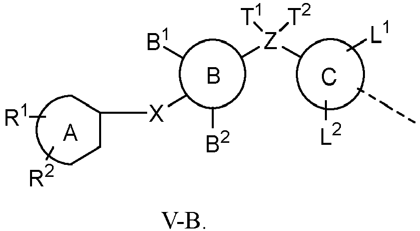

More further, in the above compounds, A is a 6-membered aromatic heterocycle, G² is absent, and the TB moiety in the compound has a structure as represented by formula VI-A: 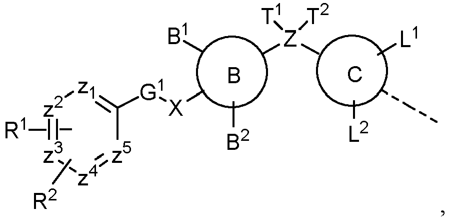 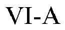
wherein, G¹ is not absent; z¹, z², z³, z⁴, and z⁵ are each independently selected from CH and N, but they are not CH at the same time;
alternatively, the TB moiety in the compound has a structure as represented by formula VI-B: 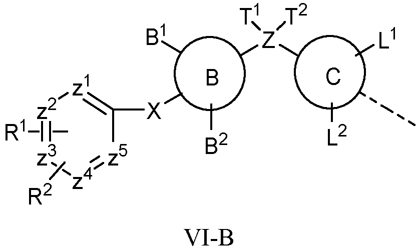
wherein, z¹, z², z³, z⁴, and z⁵ are each independently selected from CH or N, but they are not CH at the same time;

Further, in the above compounds, A is a 5-membered aromatic heterocycle, and G² is absent;
the TB moiety in the compound has a structure as represented by formula VII-A: 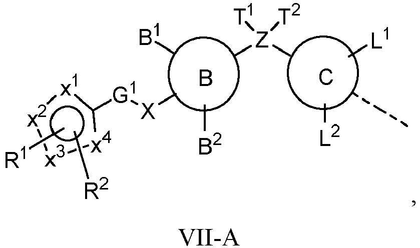
wherein, G¹ is not absent; x¹, x², x³, and x⁴ are each independently selected from CH, N, O or S, but they are not CH at the same time;
alternatively, the TB moiety in the compound has a structure as represented by formula VII-B: 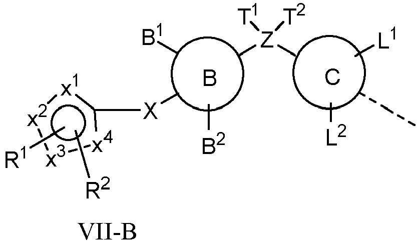
wherein, x¹, x², x³, and x⁴ are each independently selected from CH, N, O or S, and they are not CH at the same time.

Further, in the above compounds, A is a (5-membered aromatic heterocycle)-fused 6-membered aromatic heterocycle, G² is absent, and the TB moiety in the compound has a structure as represented by formula VIII-A: 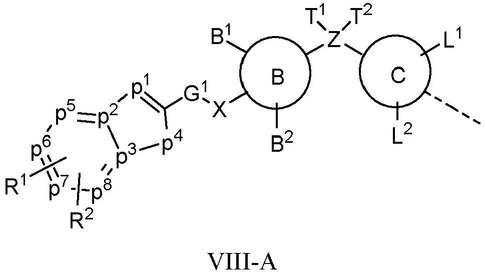 wherein, p¹, p², p³, p⁴, p⁵, p⁶, p⁷, and p⁸ are each independently selected from CH, N, O or S, and at least one of p¹, p², p³, and p⁴ is not CH, and at least one of p², p³, p⁵, p⁶, p⁷, and p⁸ is not CH.

Further, in the above compounds, A is absent, G¹ and G² are absent, R¹ is H, R² is absent, X is -O-, and the TB moiety in the compound has a structure as represented by formula IX-A:

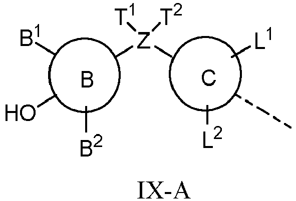

Further, 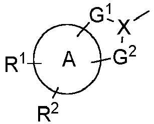 in the TB moiety of the compound has a structure as represented in the following, wherein, X, X¹, and X² are each independently selected from the group consisting of -N-, -NR⁵-, -O-, -S-, -CO-, -SO-, -SO₂-, -CONR⁵-, -NR⁵CO-, -CH-, -CHR⁵-, and - CR⁵R^{5'}-; said R⁵ and R^{5'} are each independently selected from the group consisting of H and C1-C6 alkyl:

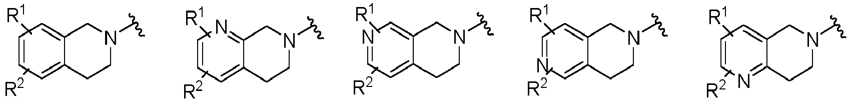

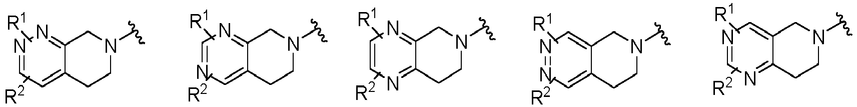

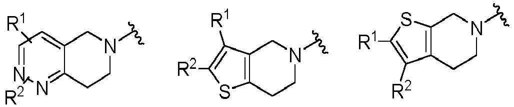

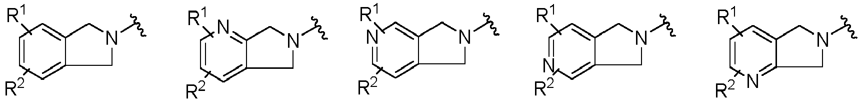

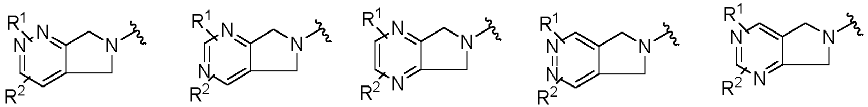

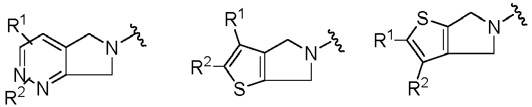

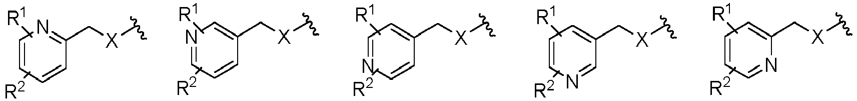

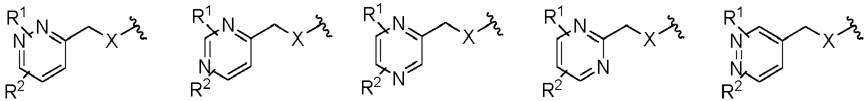

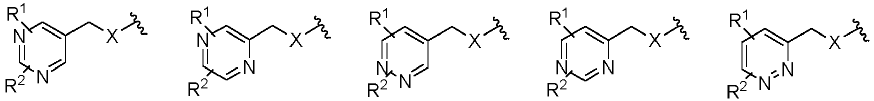

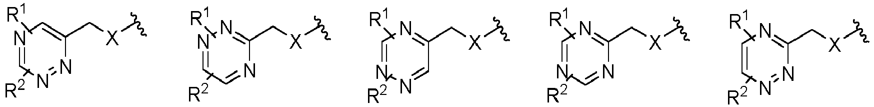

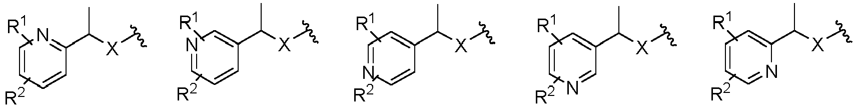

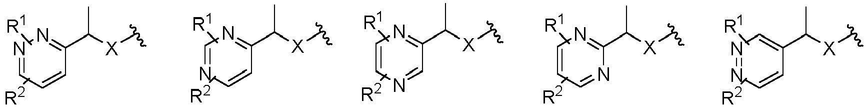

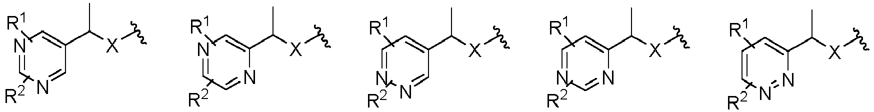

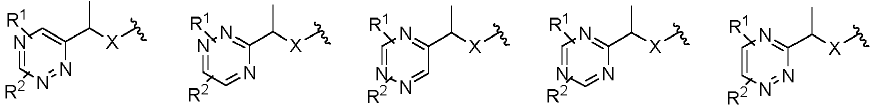

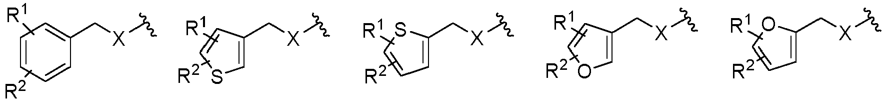

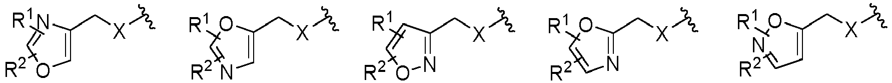

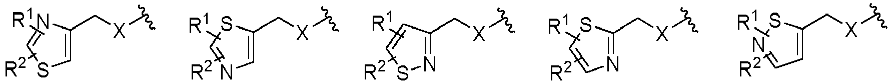

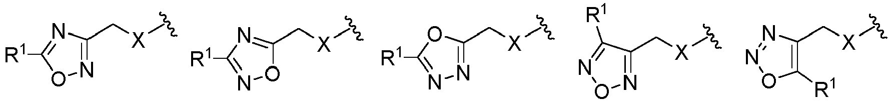

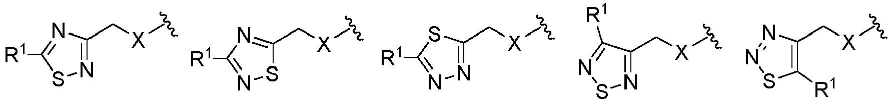

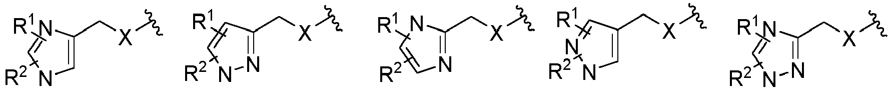

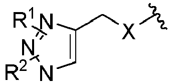

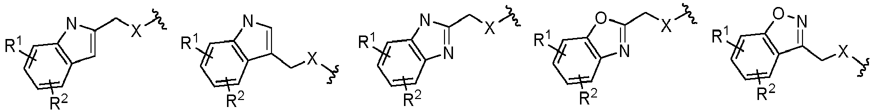

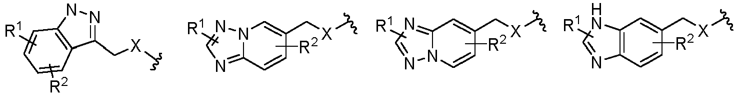

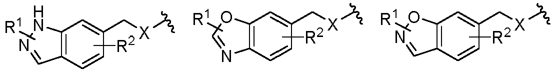

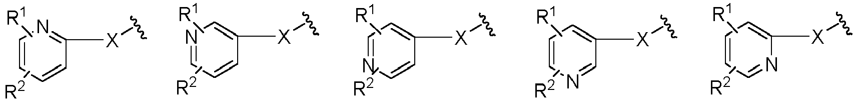

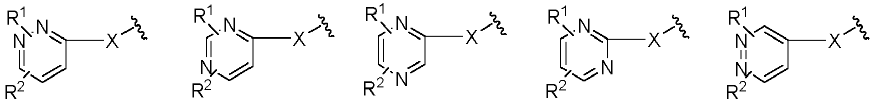

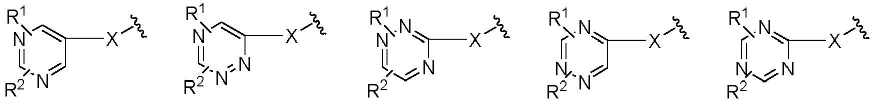

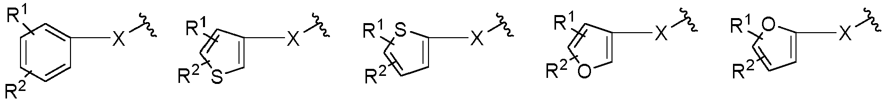

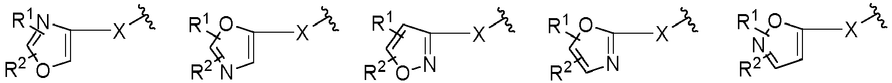

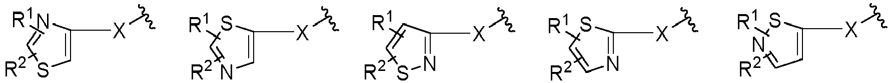

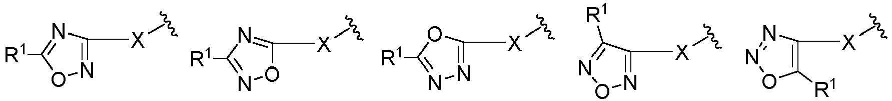

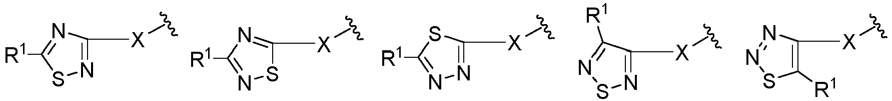

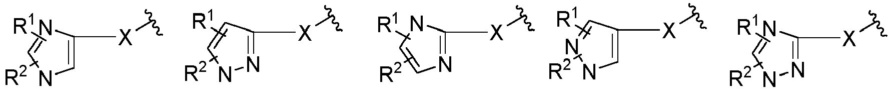

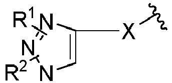

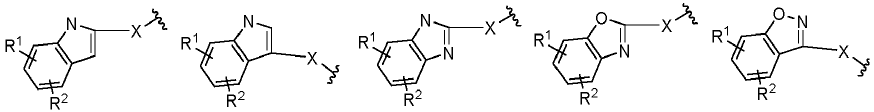

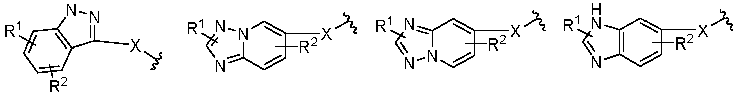

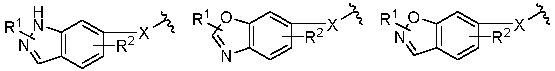

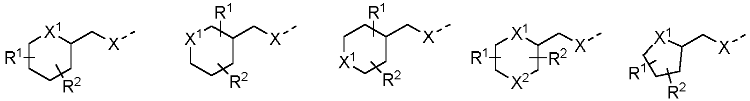

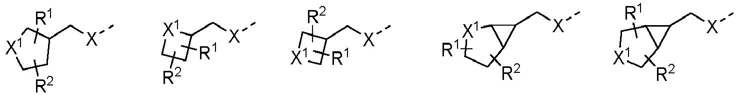

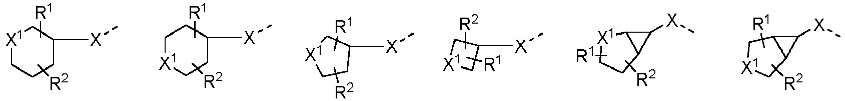

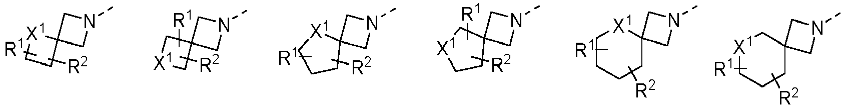

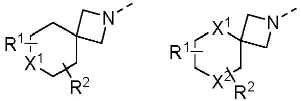

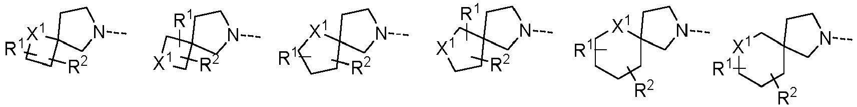

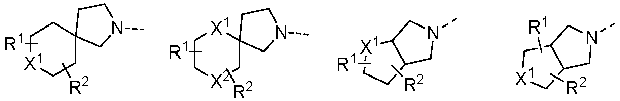

Further, 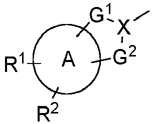 in the TB moiety of the compound has a structure as represented in the following: 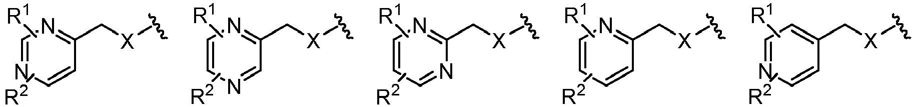 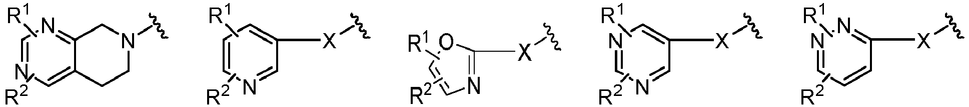 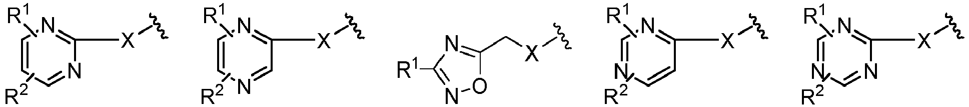 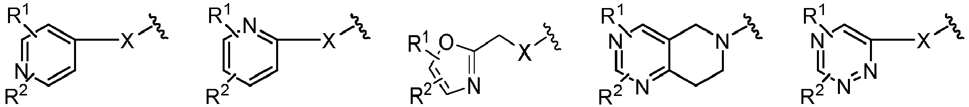 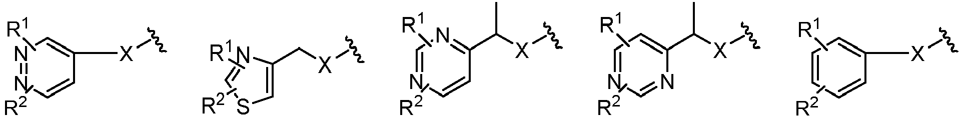 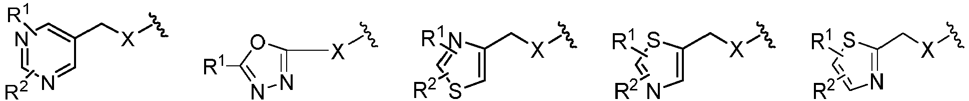 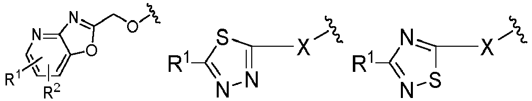 or hydroxyl.

Further, in the above compounds, R¹ and R² in the TB moiety of the compound are each independently selected from the group consisting of H, CF₃, or any one of the following structures: 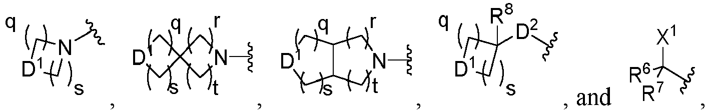
wherein, q, r, s, and t are each independently selected from an integer of 0 to 5;
wherein, D¹ and D² are each independently selected from the group consisting of -O-, -S-, - SO-, -SO₂-, -NR⁶-, -NCOR⁶-, -NSO₂R⁷-, -CR⁶X¹-, and -CR⁶R⁷-; wherein R⁶ and R⁷ are each independently selected from the group consisting of H, halogen, hydroxyl, amino, C1-C6 alkyl, C1-C6 alkoxy, and C1-C6 alkylamino; or R⁶ and R⁷ are linked to form a ring;
wherein, X¹ is selected from the group consisting of -OR⁸-, -NR⁸R⁹-, -NCOR⁸-, -NSO₂R⁹-, and -CR⁸R⁹-; wherein R⁸ and R⁹ are each independently selected from the group consisting of H, substituted or unsubstituted C1-C6 alkyl, and C3-C6 cycloalkyl; or R⁸ and R⁹ can be linked to each other to form a ring; the substituted substituent is hydroxyl or amino.

Further, in the above compounds, R¹ and R² in the TB moiety of the compound are each independently selected from the group consisting of H, CF₃, or any one of the following structures: H, methyl, ethyl, isopropyl, cyclopropyl, cyclobutyl, cyclopentyl, cyclohexyl, methoxy, ethoxy, propoxy, isopropoxy, cyclopropoxy, cyclobutoxy, cyclopentoxy, amino, methylamino, ethylamino, propylamino, isopropylamino, cyclopropylamino, cyclobutylamino, hydroxyethylamino, 1-hydroxymethylcyclopropylamino, N,N-dimethylamino, N,N-diethylamino, formyl, acetyl, propanoyl, isopropanoyl, cyclopropylcarbonyl, 1-hydroxymethyl, 1-hydroxyethyl, 1-hydroxypropyl, 1-hydroxyisopropyl, 1-hydroxycyclopropyl, aminocarbonyl, N-methylaminocarbonyl, N-ethylaminocarbonyl, N,N-dimethylcarbonyl, acridinylcarbonyl, pyrrolidinylcarbonyl, methoxycarbonyl, ethoxycarbonyl, acridinyl, tetrahydropyrrole, piperidine, morpholine, piperazine, N-methylpiperazine, N-ethylpiperazine, N-cyclopropylpiperazine, 2-methylpiperazine, 3-methylpiperazine, 2,2-dimethylpiperazine, 3,3-dimethylpiperazine, 2,3-dimethylpiperazine,

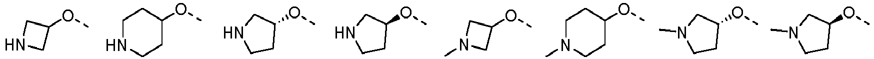

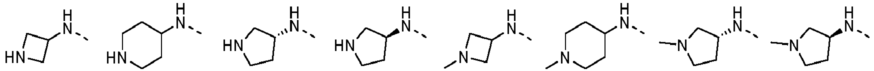

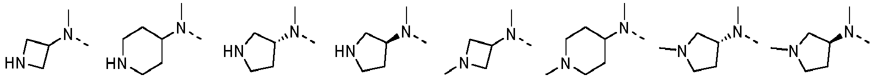

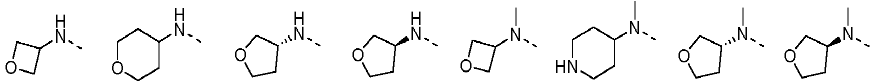

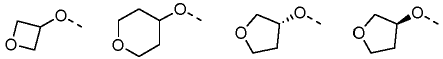

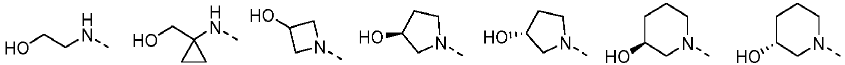

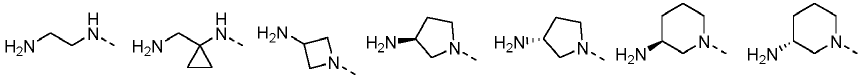

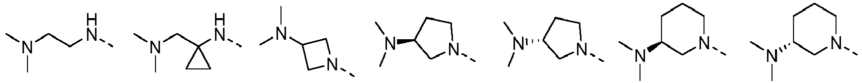

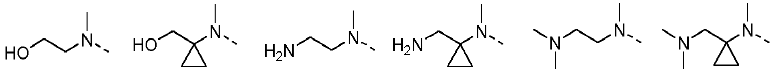

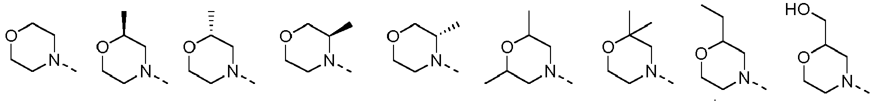

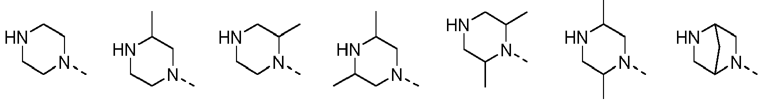

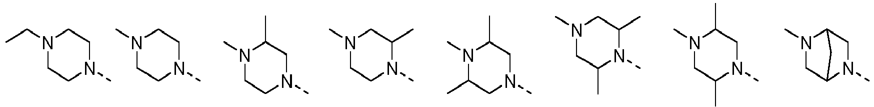

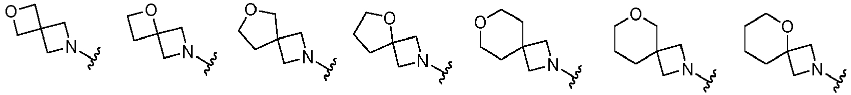

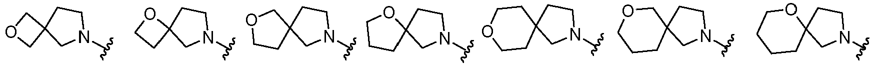

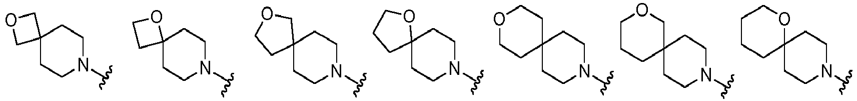

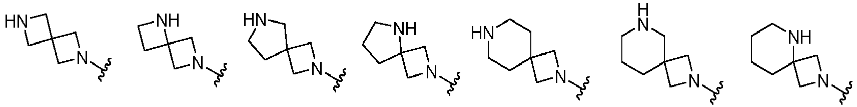

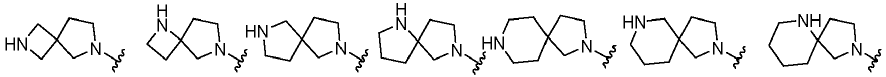

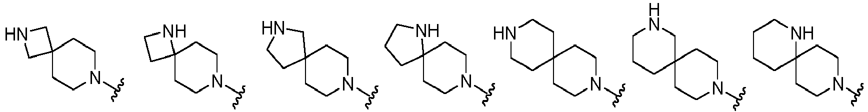

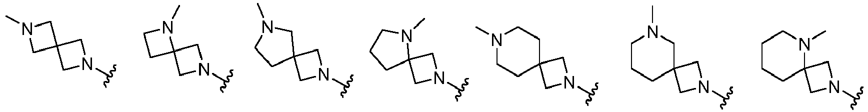

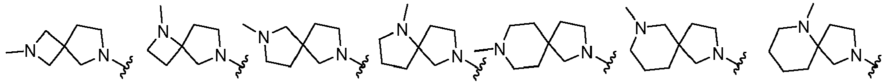

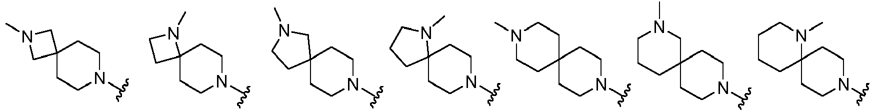

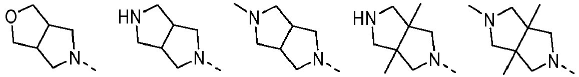

Further, in the above compounds, R¹ and R² in the TB moiety are each independently selected from the group consisting of H, F, Cl, Br, I, CH₃, amido, substituted or unsubstituted 1,2-diazolyl, 1,3-diazolyl, N-methyl-1,2-diazolyl, *N¹*-1,2,3-triazolyl, *N²*-1,2,3-triazolyl, 1,3,4-triazolyl, 1,2,4-triazolyl, 1,2,5-triazolyl, 1,2-oxazolyl, 1,3-oxazolyl, 1,2,3- oxadiazolyl, 1,2,4-oxadiazolyl, 1,3,5-oxadiazolyl, 1,2-thioxazolyl, 1,3-thioxazolyl, 1,2,3-thiadiazolyl, 1,2,4-thiadiazolyl, 1,3,5-thiadiazolyl, *N²*-4-fluoro-1,2,3-triazolyl, *N²*-4-methyl-1,2,3-triazolyl, 2-methyl-1,3,4-oxadiazolyl, 5-methyl-1,2,4-oxadiazolyl, 3-methyl-1,2,4-oxadiazolyl, pyridyl, pyridazinyl, pyrimidinyl, and pyrazinyl; the substituted substituent is selected from the group consisting of halogen, hydroxyl, amino, C1-C6 alkoxy, C1-C6 alkylamino, cyano, amido, C1-C6 alkyl, and C3-C6 cycloalkyl.

Further, in the above compounds, R¹ and R² in the TB moiety are each independently selected from the group consisting of H, amino, methylamino, dimethylamino, methylthio, methanesulfonyl, 1,2,3-triazolyl, 1,2,4-triazolyl, pyridyl, F, I, 2-methyl-1,3,4-oxadiazolyl, cyano, 5-methyl-1,2,4-oxadiazolyl, 1-hydroxyethyl, 1-hydroxypropyl, acetyl, propionyl, cyclopropylcarbonyl, cyclobutylcarbonyl, isopropionyl, 1-hydroxyisopropyl, 1-hydroxyisobutyl, methoxycarbonyl, ethoxycarbonyl, 1-hydroxymethyl, methoxy, ethoxy, cyclopropyloxy, aminocarbonyl, N-methylaminocarbonyl, 1-methyl-1,2-diazolyl, 1,2-diazolyl, 1,3-diazolyl, 3-methyl-1,2,4-oxadiazolyl, 1,2,4-oxadiazolyl, 1,3,4-oxadiazolyl, 3,5-dimethyl-1,2-oxazolyl, pyrrolidinyl, 1,3,4-thiadiazolyl, 3-methyl-1,2,4-thiadiazolyl, piperidinyl, carboxyl, 1,3-thiazolyl, -NHCN, 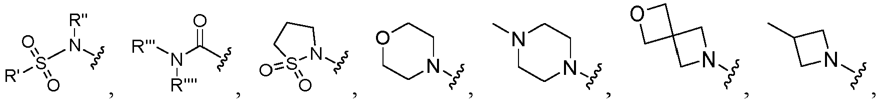 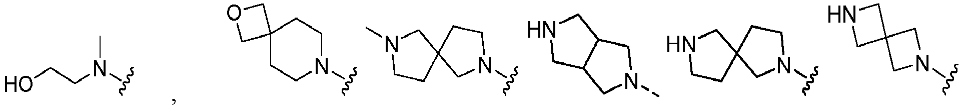 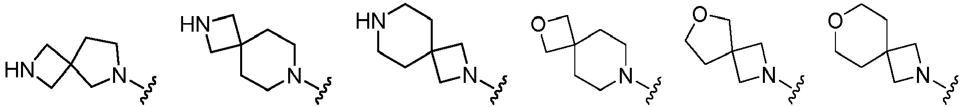 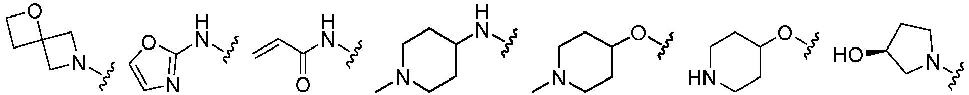 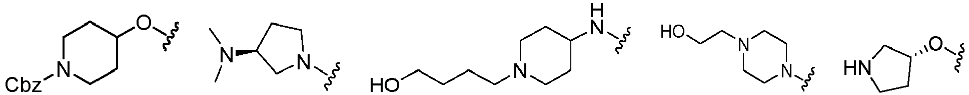 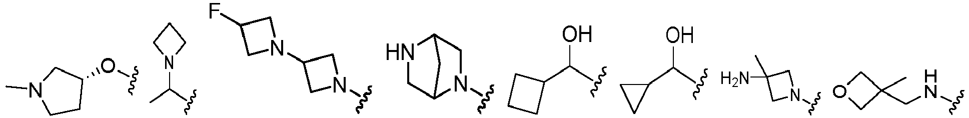 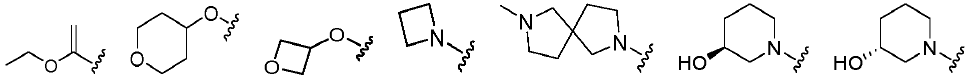 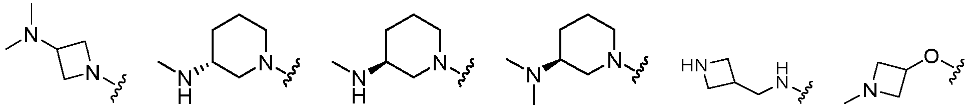 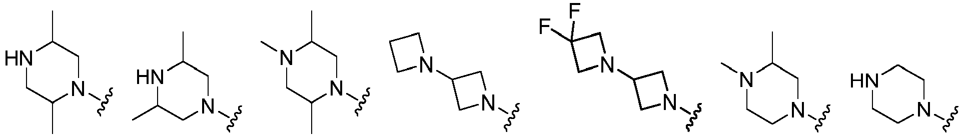 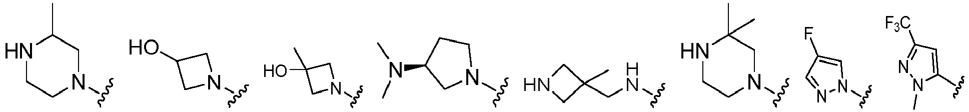 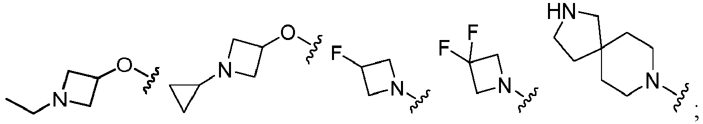
wherein, R' is selected from the group consisting of methyl, ethyl, and cyclopropyl; and R" is selected from the group consisting of H and methyl; R‴ and Rʺʺ are each independently selected from the group consisting of H, methyl, and ethyl; or, R‴ and Rʺʺ are linked to form methyl-substituted or unsubstituted 4-6-membered heterocycle;
preferably, R¹ and R² are not

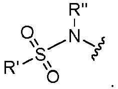

Further, in the above compounds, 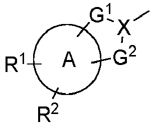 in the TB moiety has the structure selected from the group consisting of:

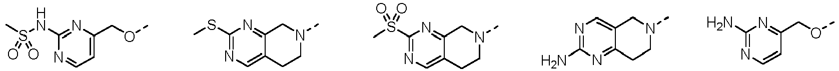

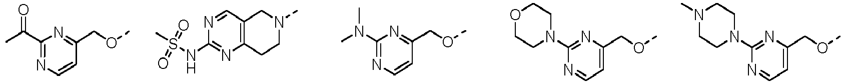

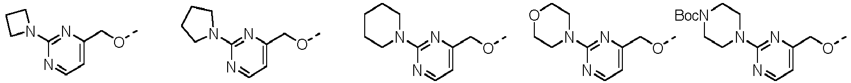

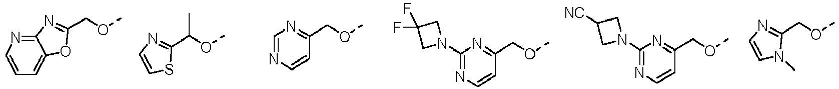

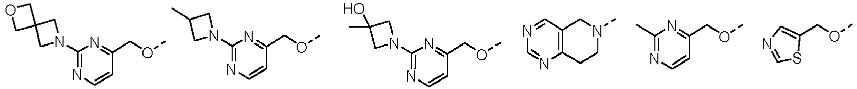

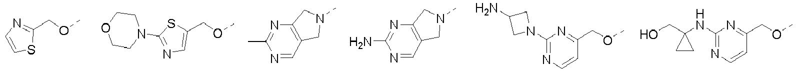

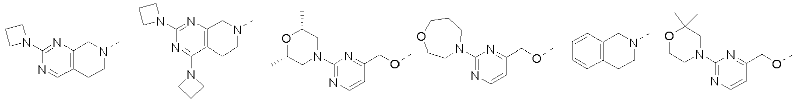

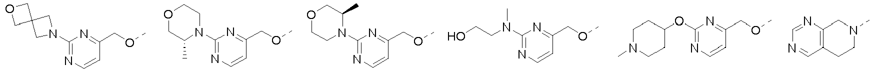

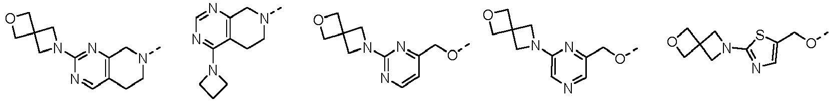

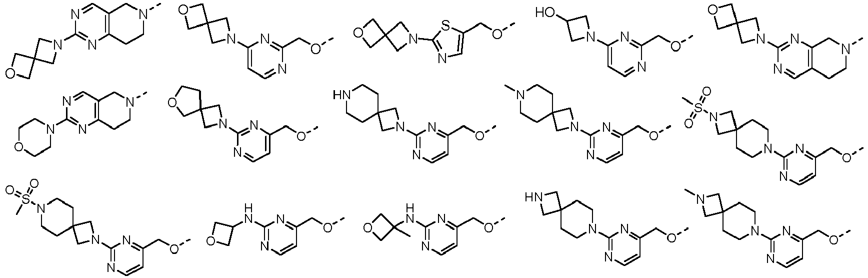

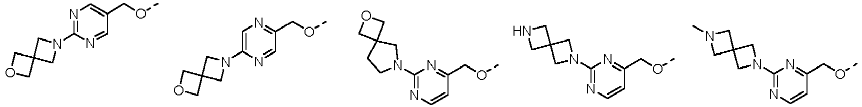

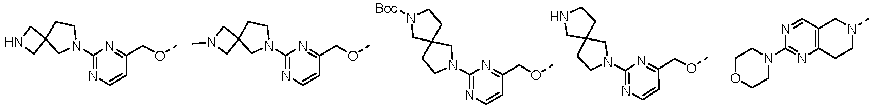

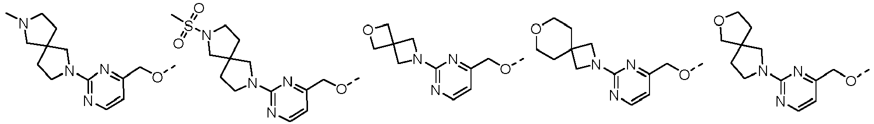

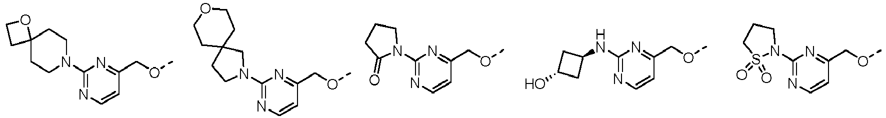

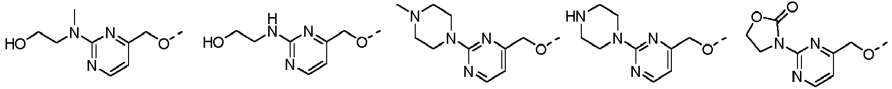

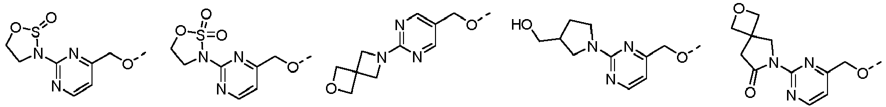

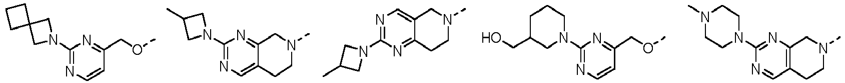

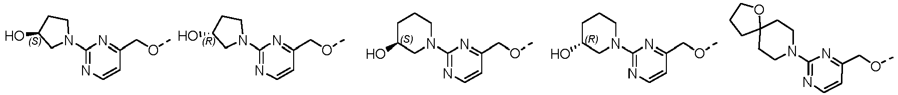

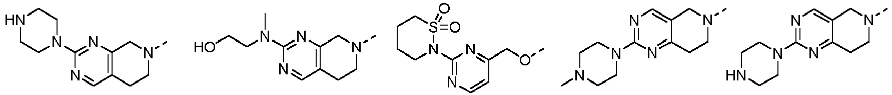

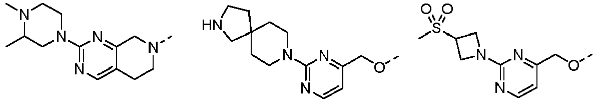

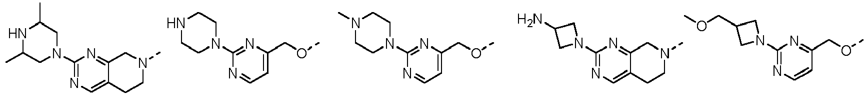

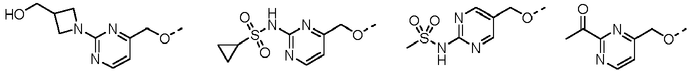

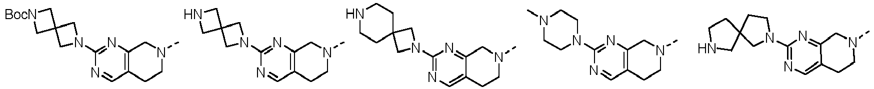

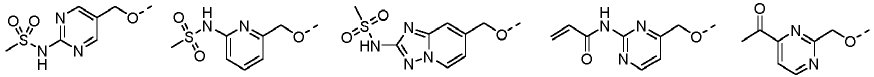

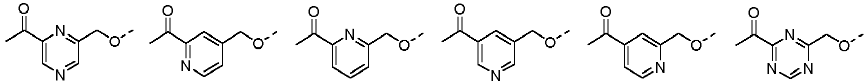

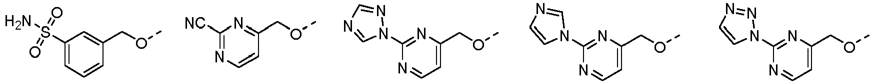

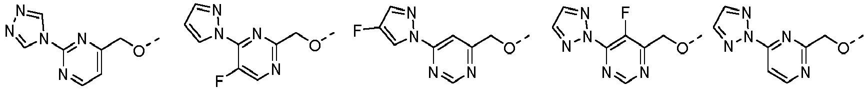

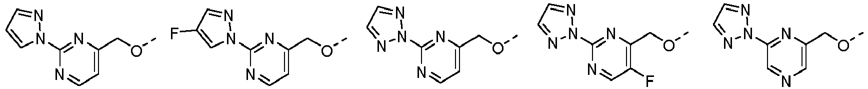

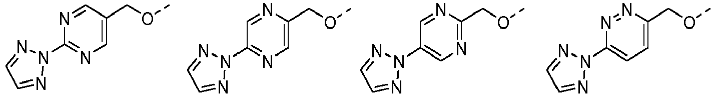

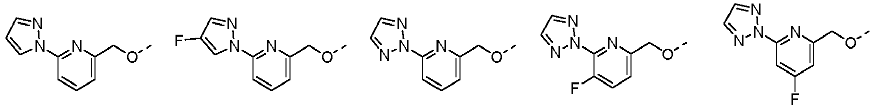

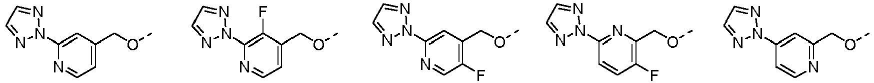

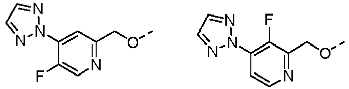

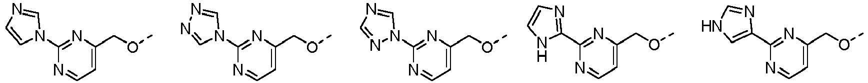

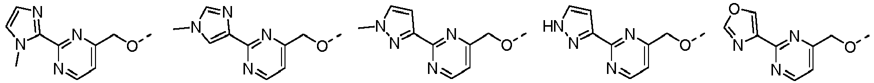

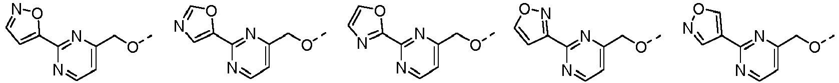

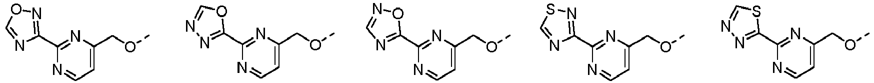

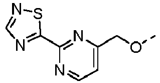

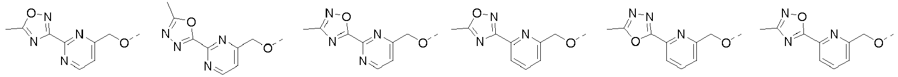

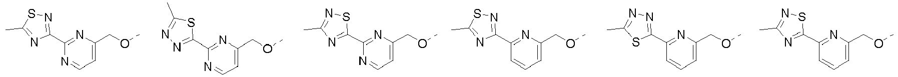

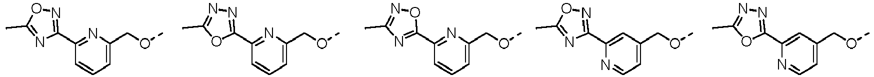

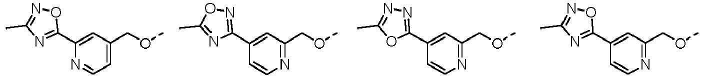

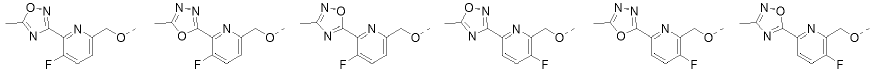

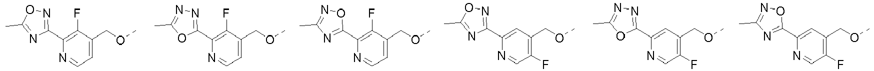

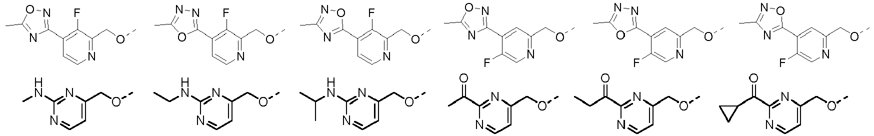

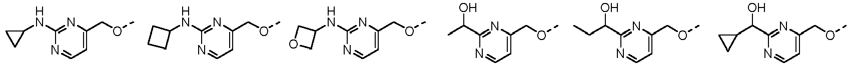

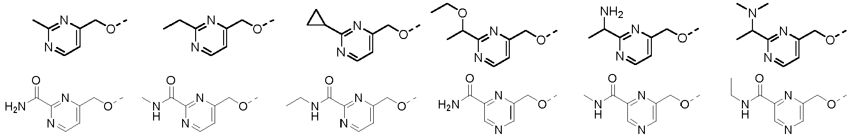

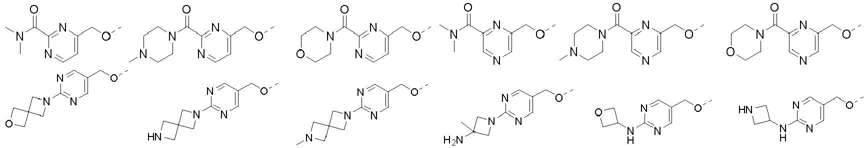

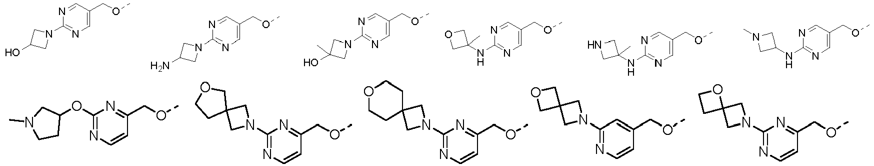

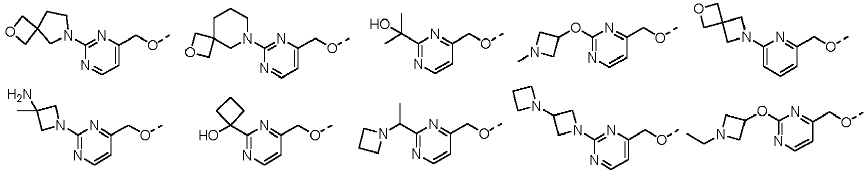

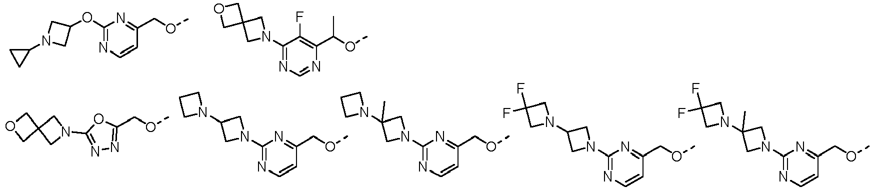

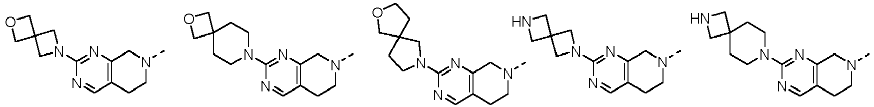

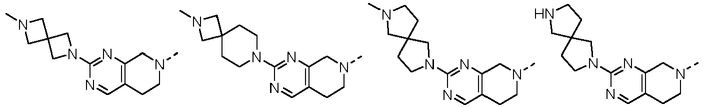

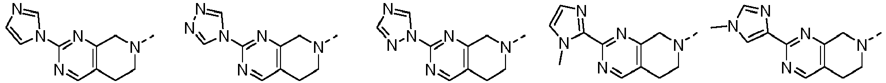

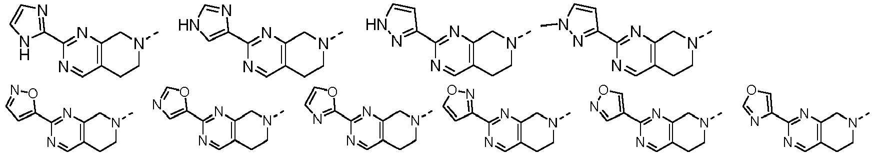

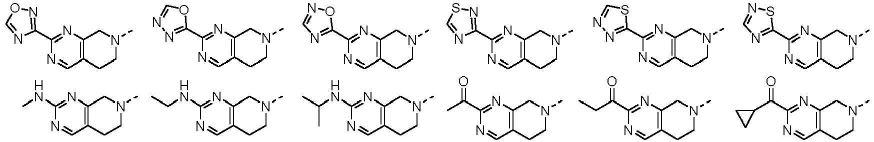

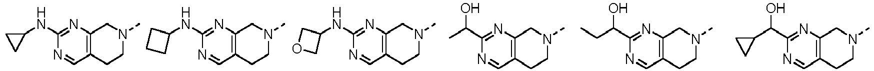

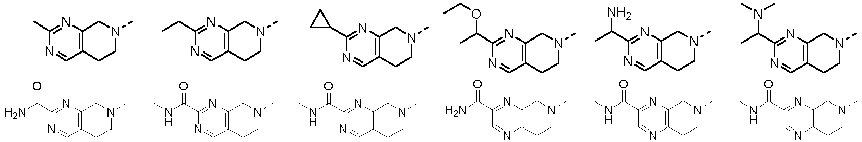

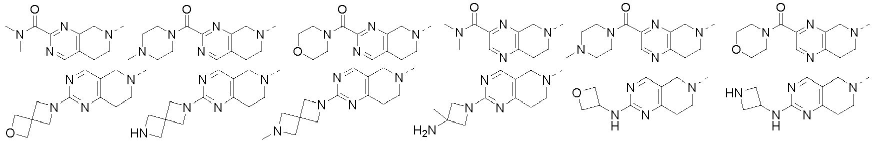

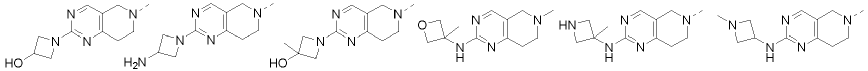

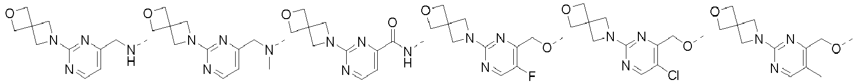

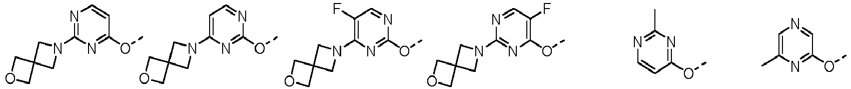

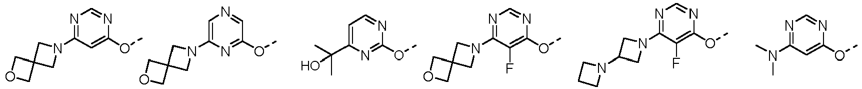

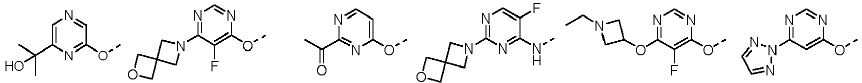

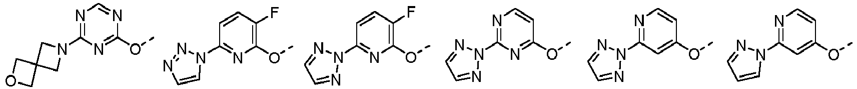

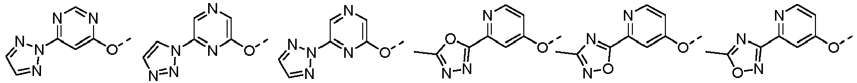

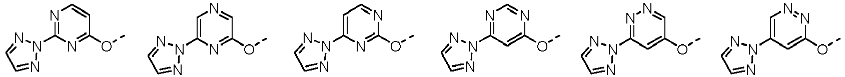

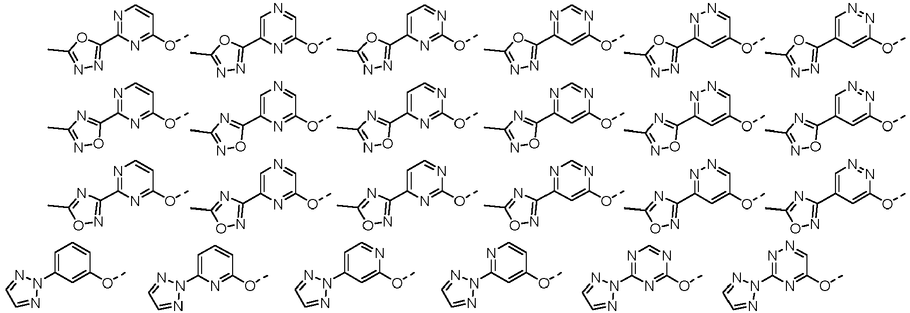

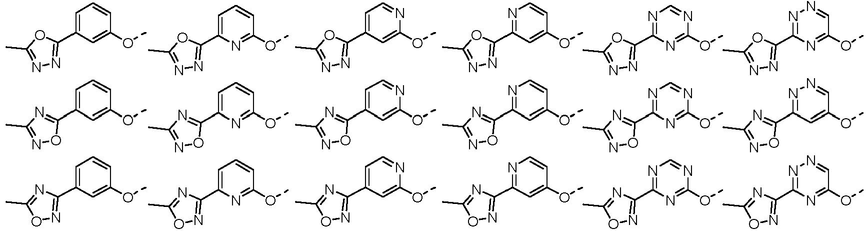

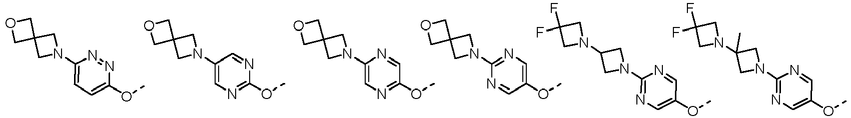

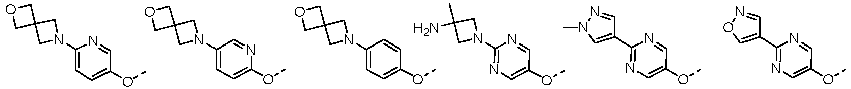

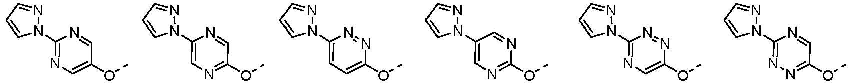

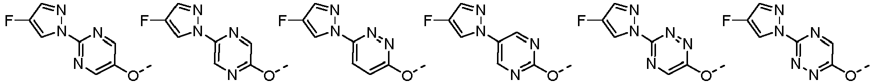

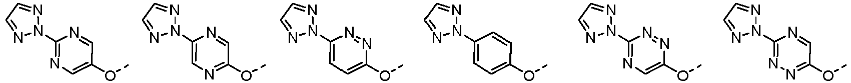

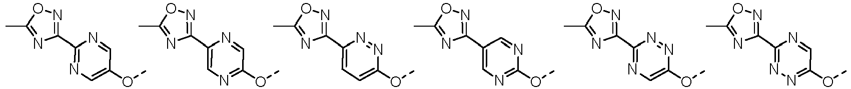

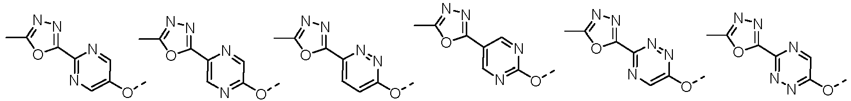

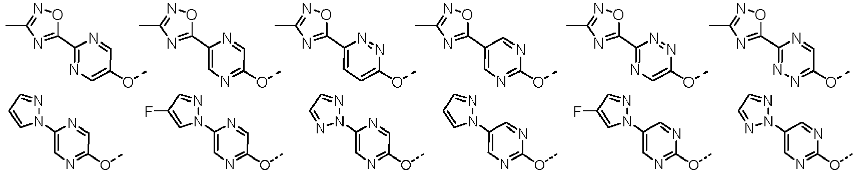

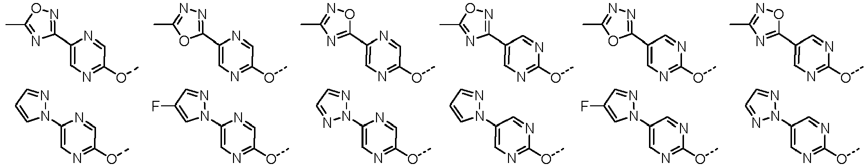

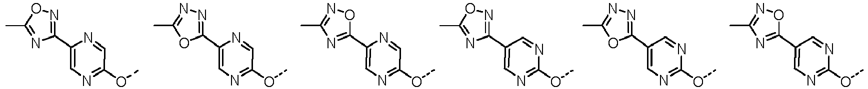

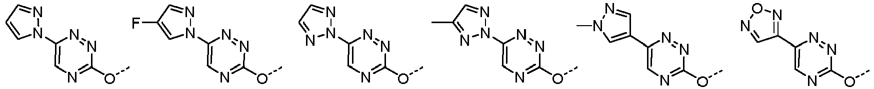

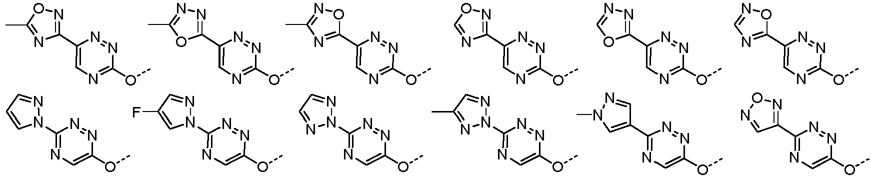

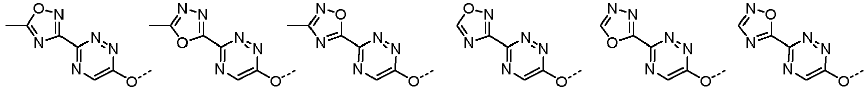

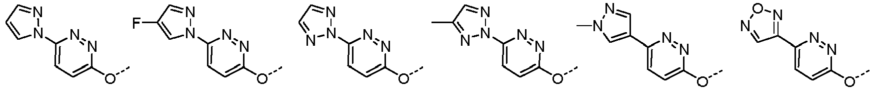

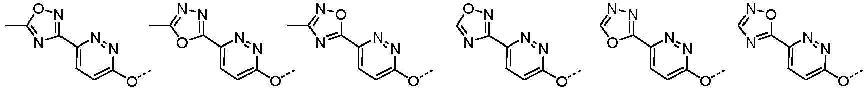

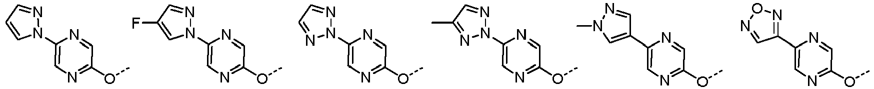

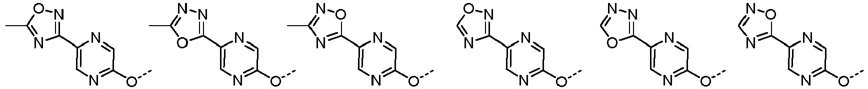

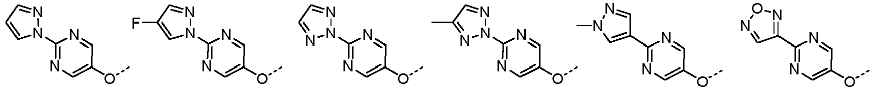

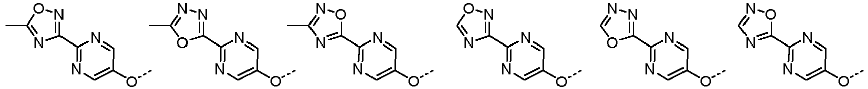

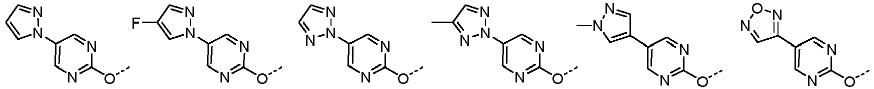

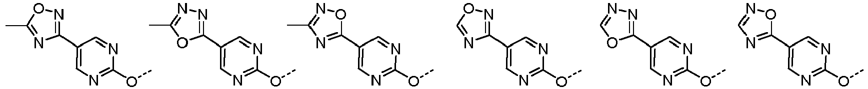

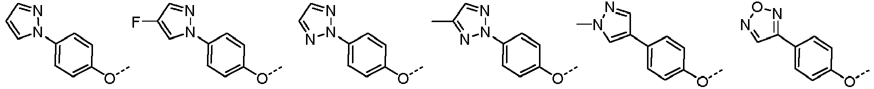

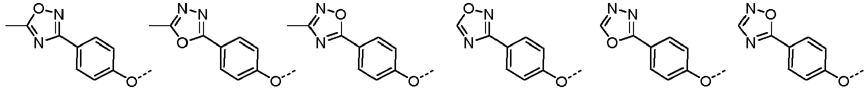

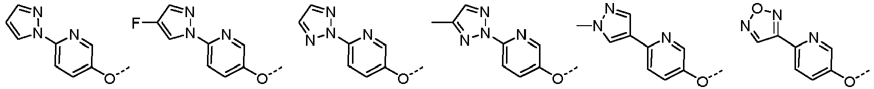

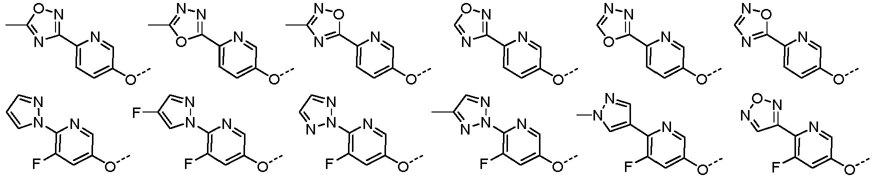

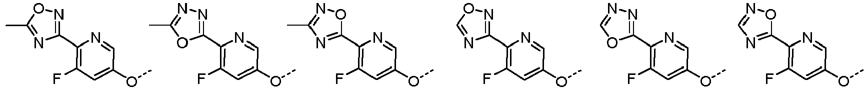

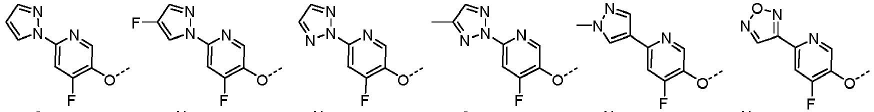

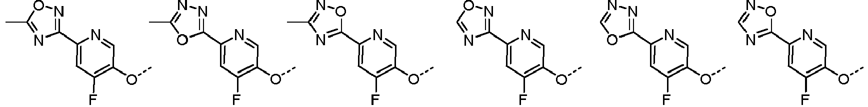

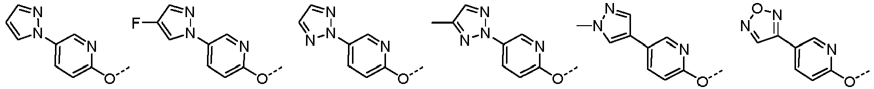

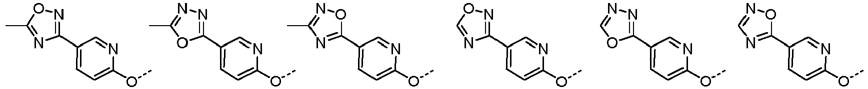

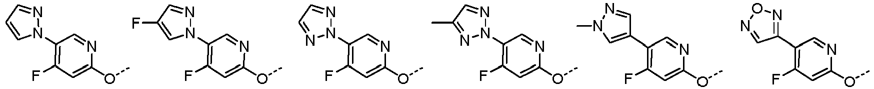

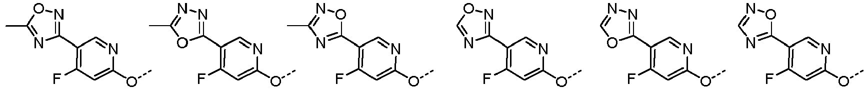

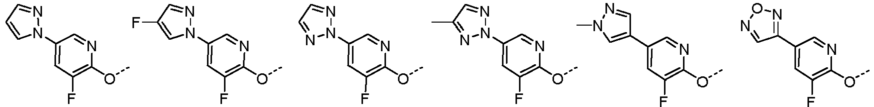

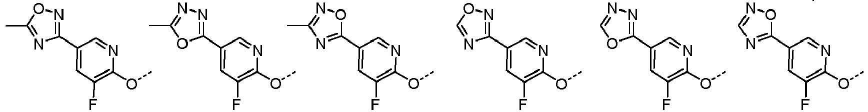

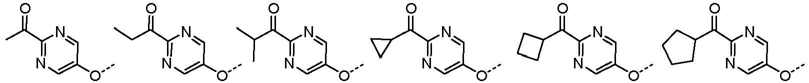

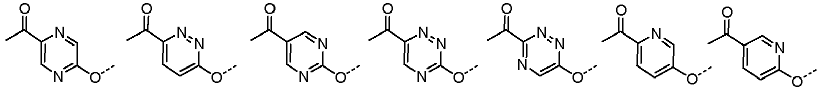

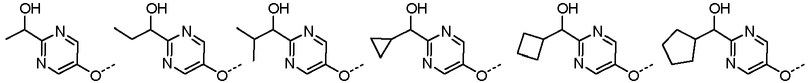

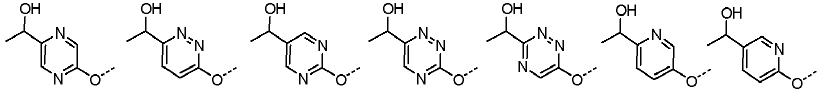

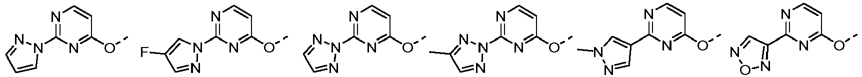

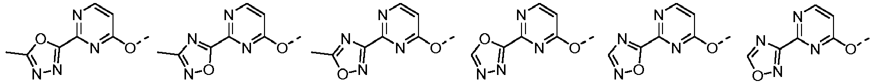

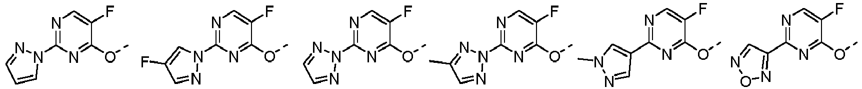

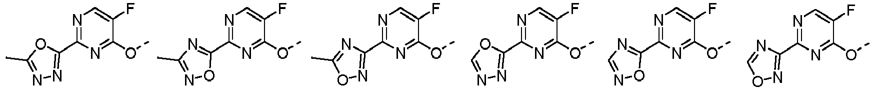

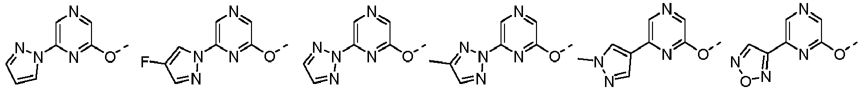

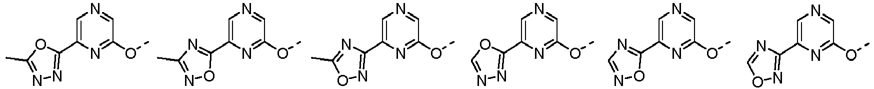

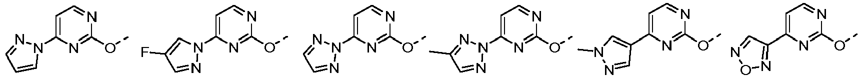

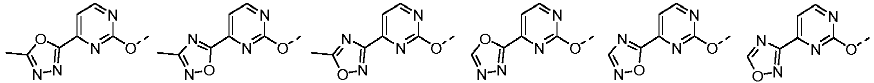

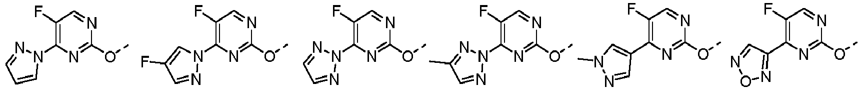

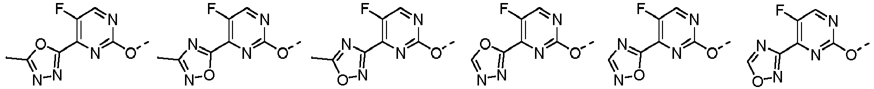

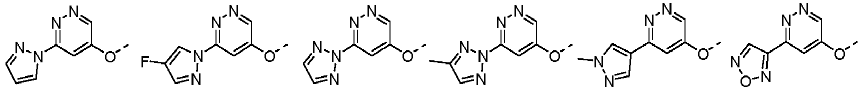

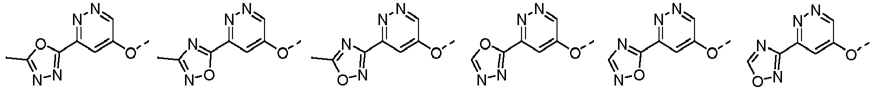

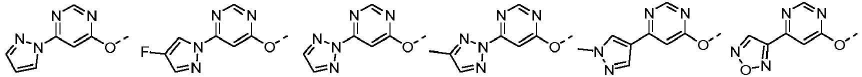

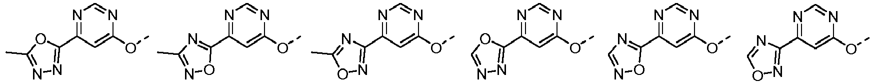

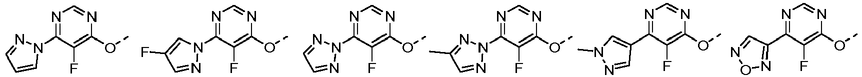

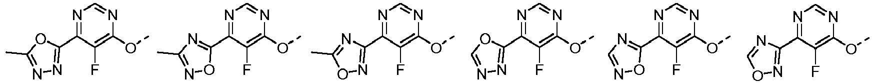

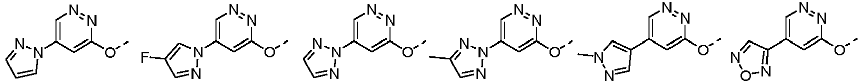

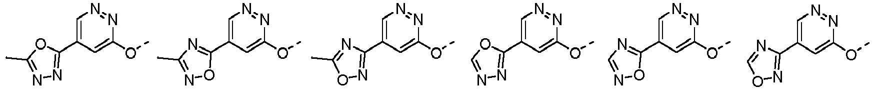

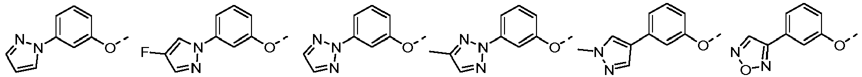

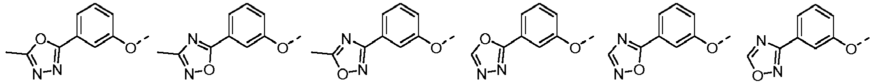

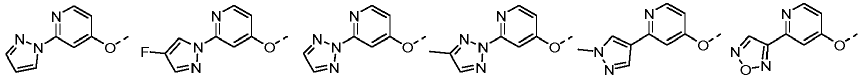

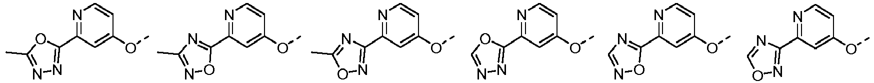

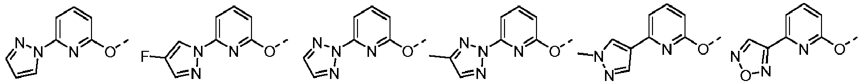

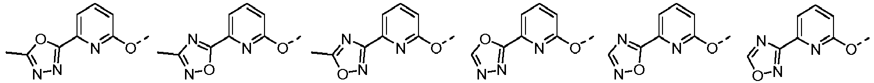

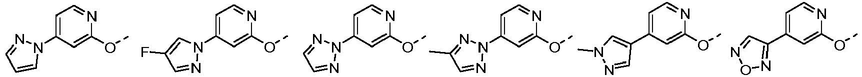

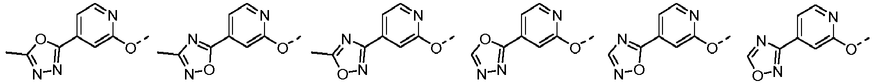

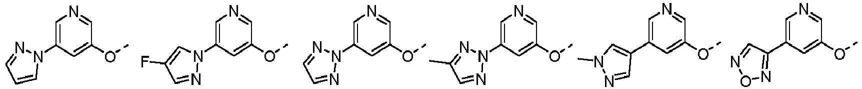

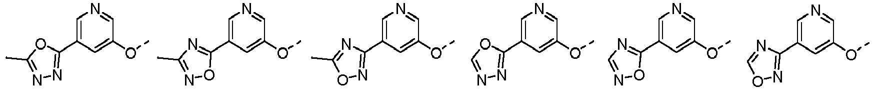

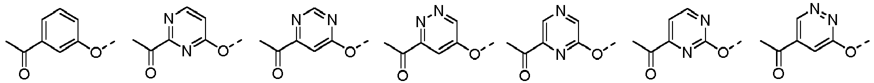

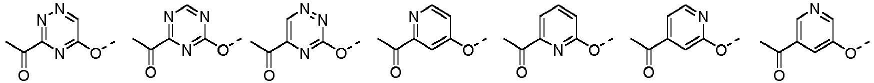

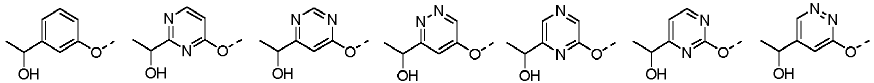

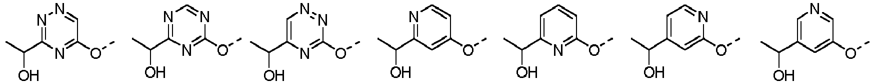

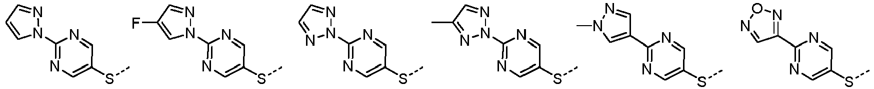

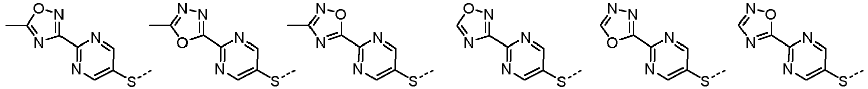

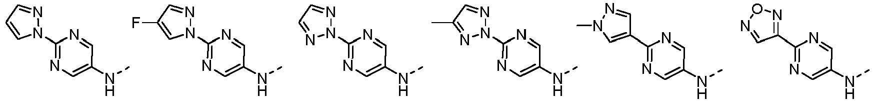

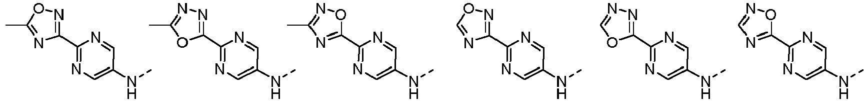

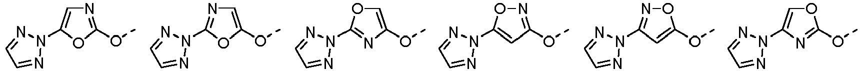

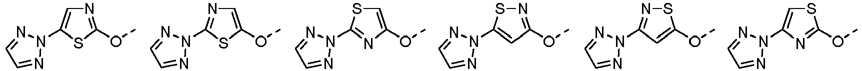

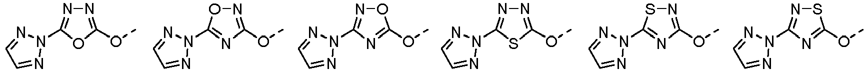

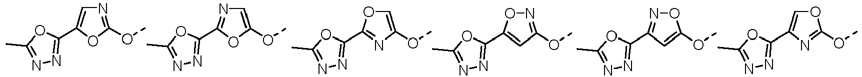

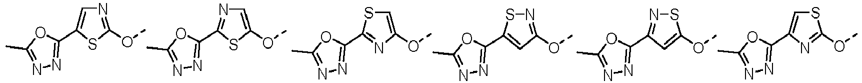

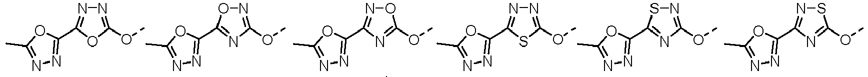

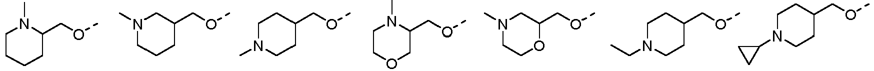

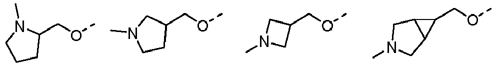

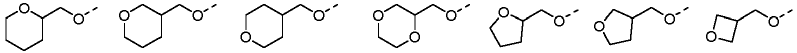

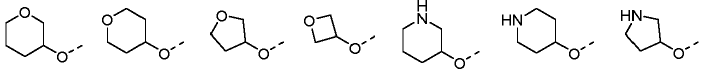

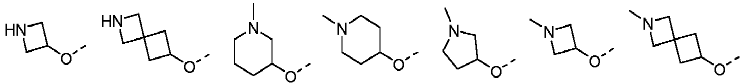

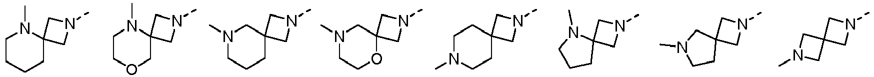

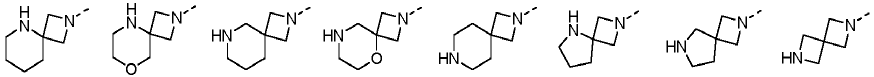

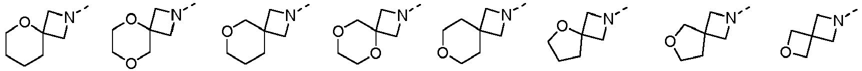

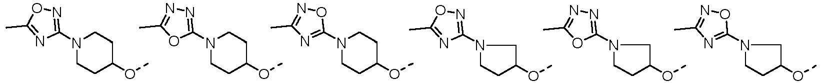

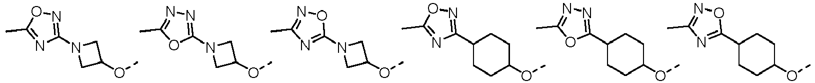

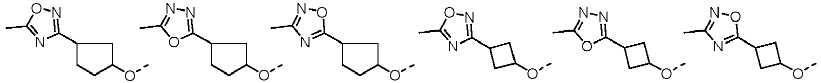

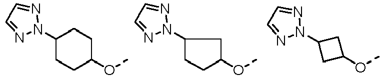

Further, in the above compounds, the structure of 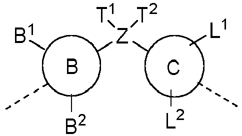 in the TB moiety is selected from the group consisting of:

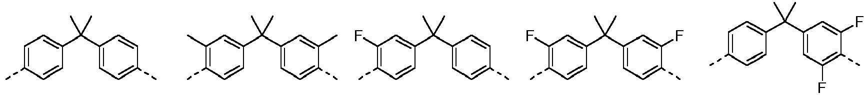

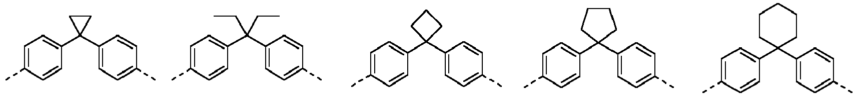

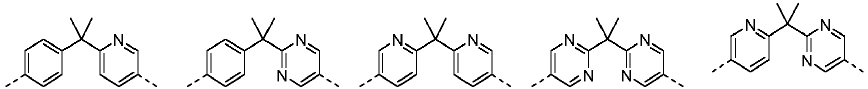

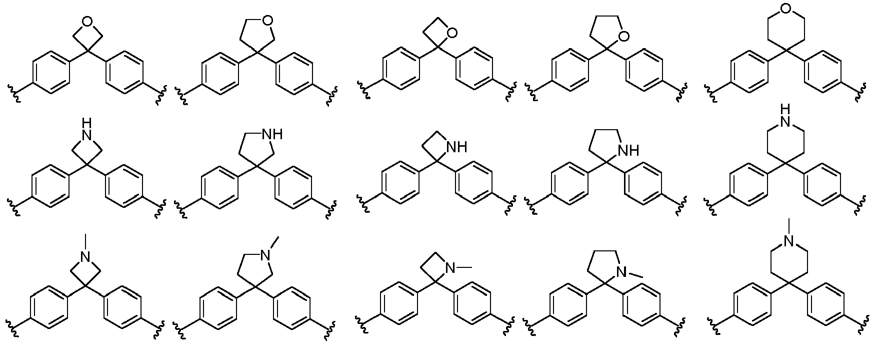

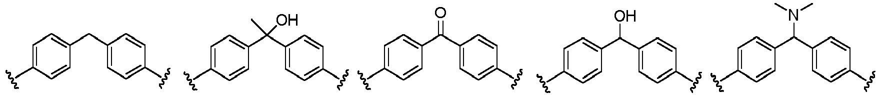

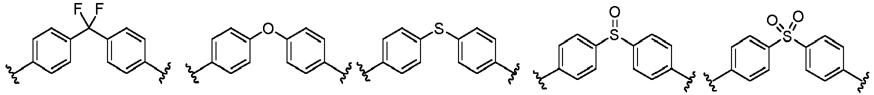

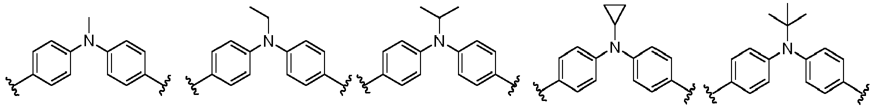

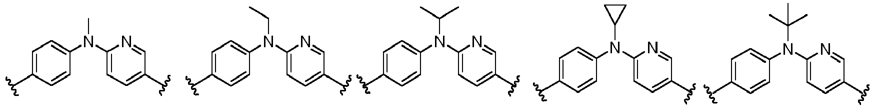

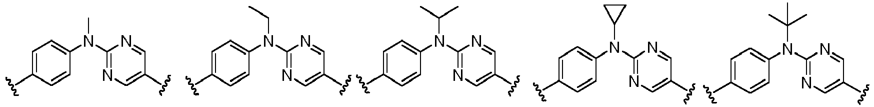

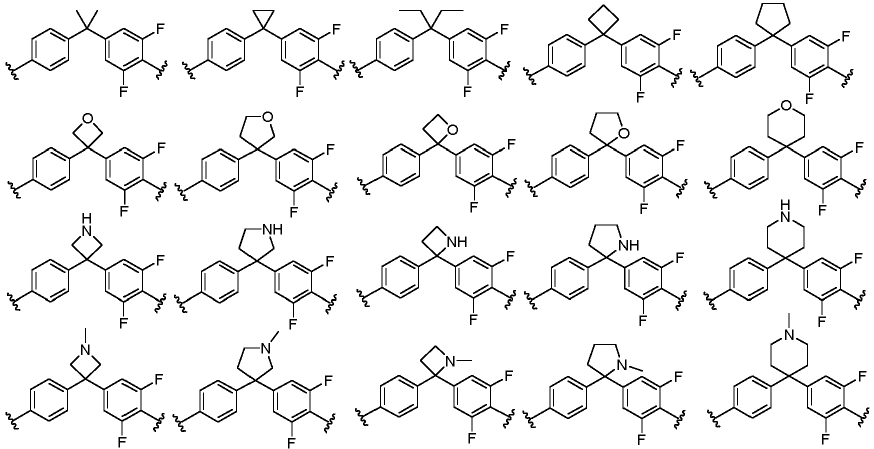

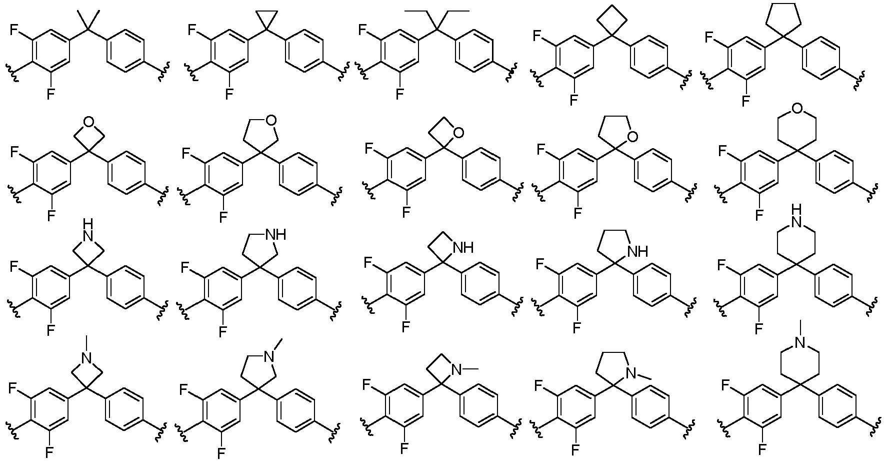

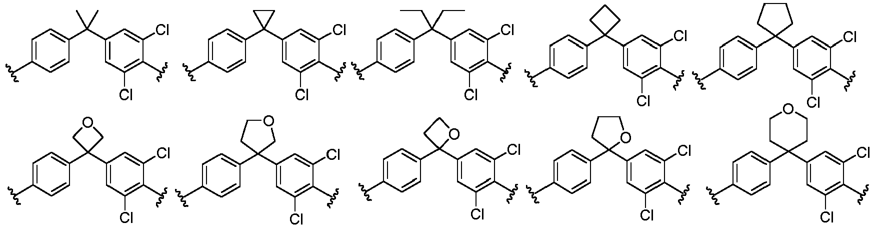

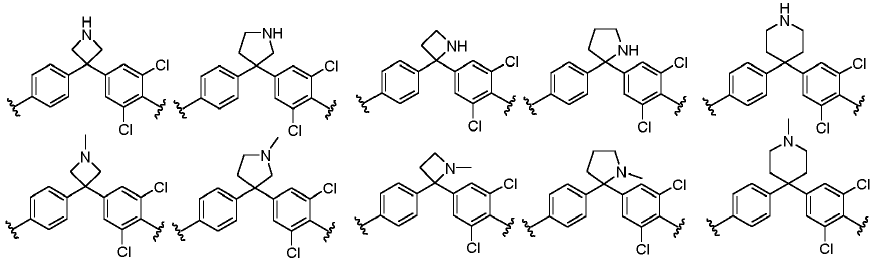

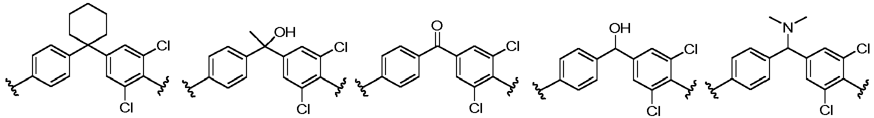

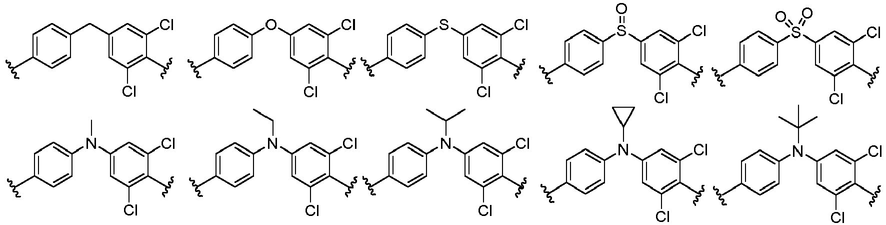

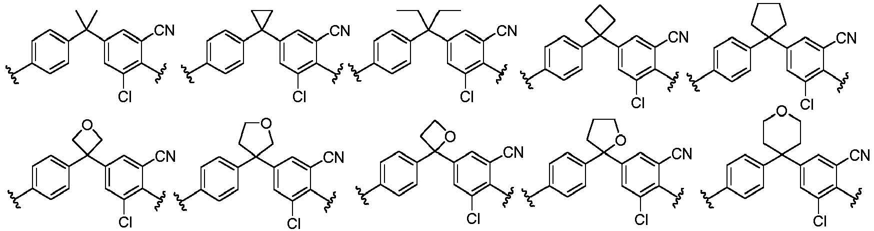

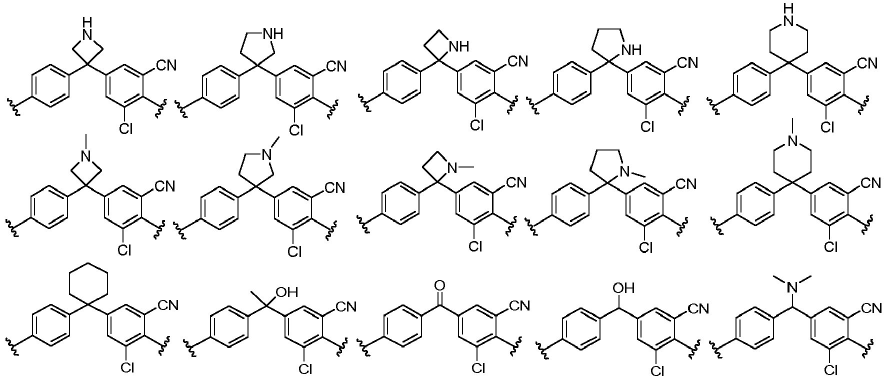

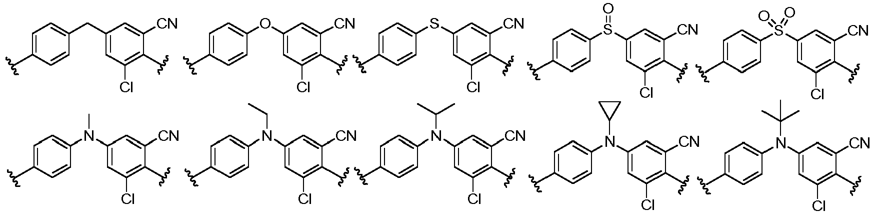

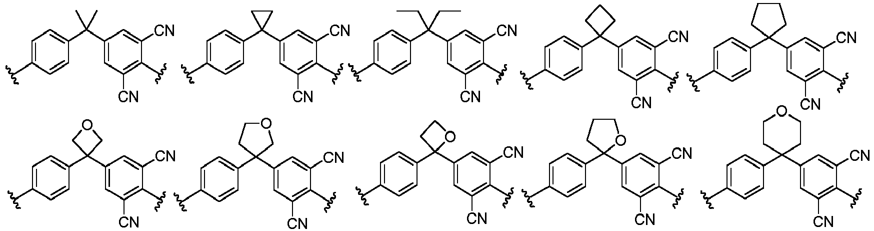

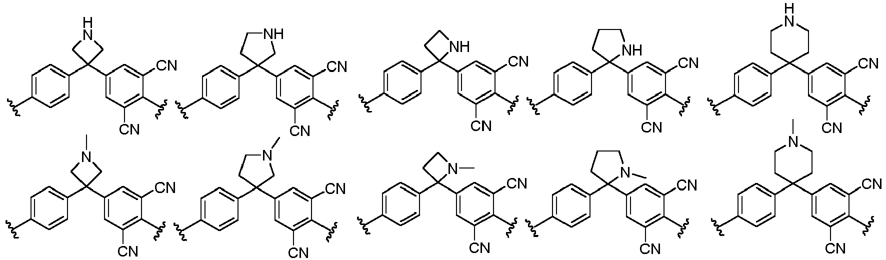

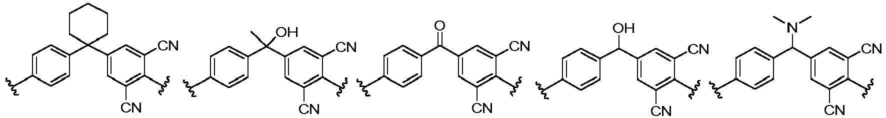

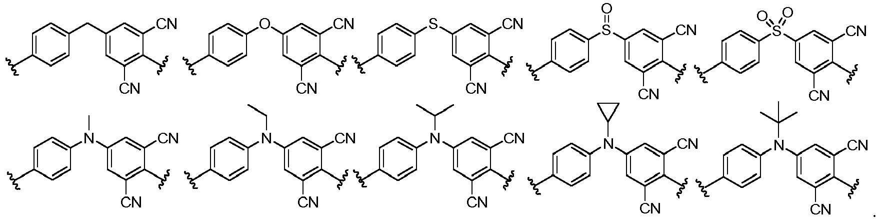

Further, in the above compounds, the structure of 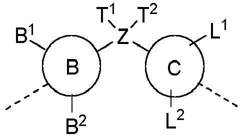 in the TB moiety is as represented by formula I-C: 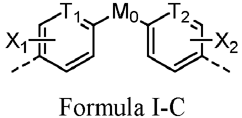 wherein, M₀ is -CRₐR_{b}- or -SO₂-; T₁ and T₂ are each independently selected from the group consisting of -CH- and -N-; X₁ and X₂ are each independently selected from the group consisting of H and halogen.

More further, in the above compounds, the structure of 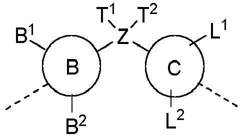 in the TB moiety is selected from the group consisting of: 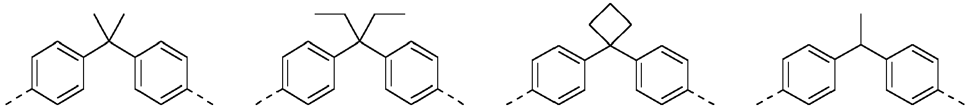 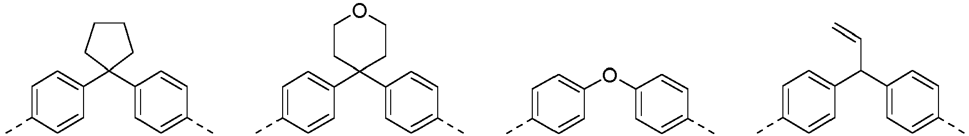 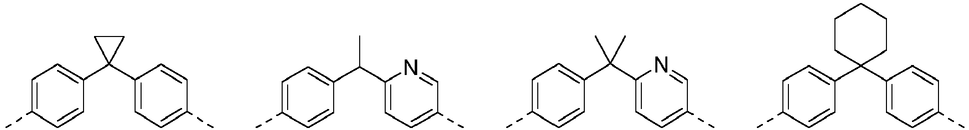 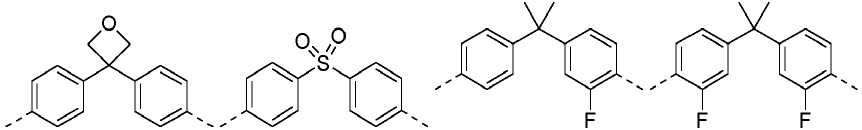 or

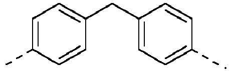

Further, in the above compounds, the structure of TB moiety is selected from the group consisting of:

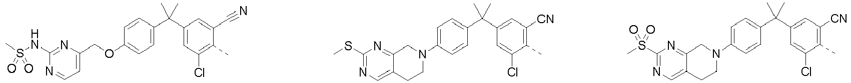

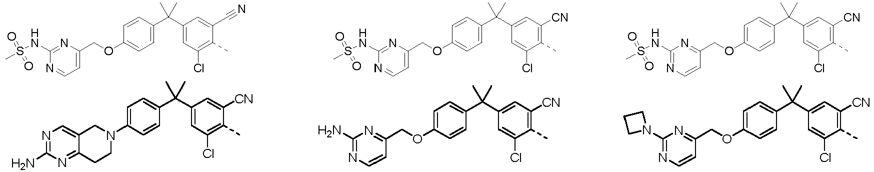

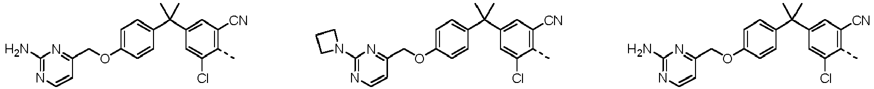

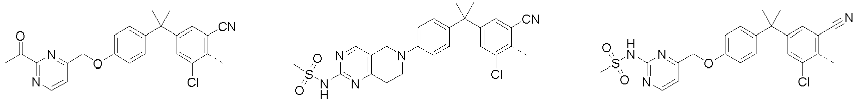

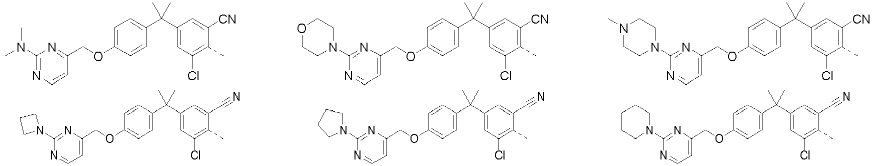

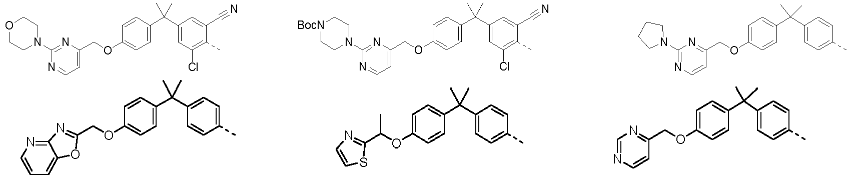

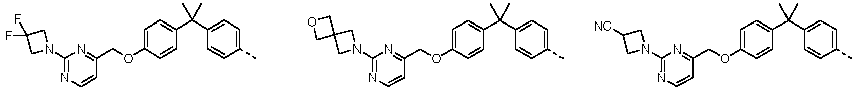

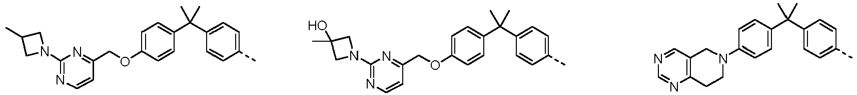

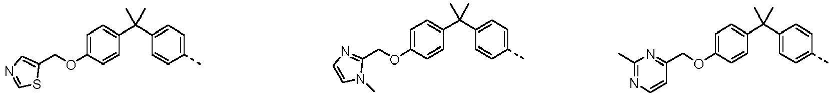

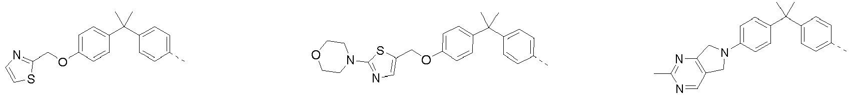

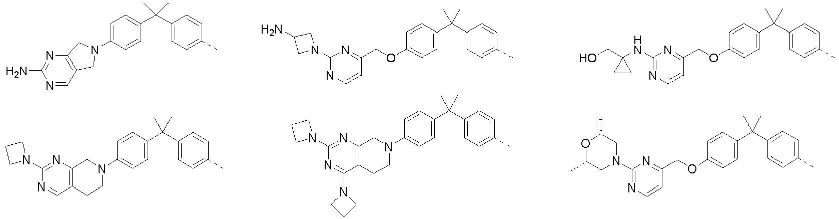

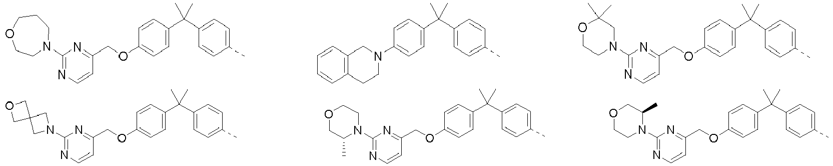

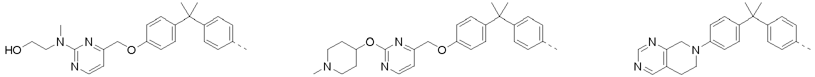

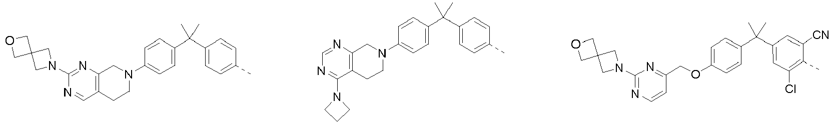

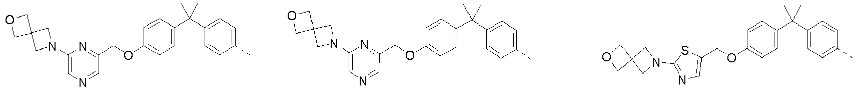

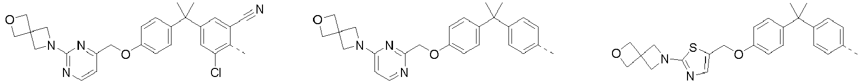

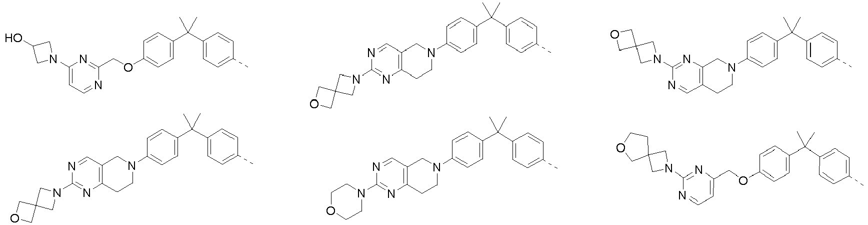

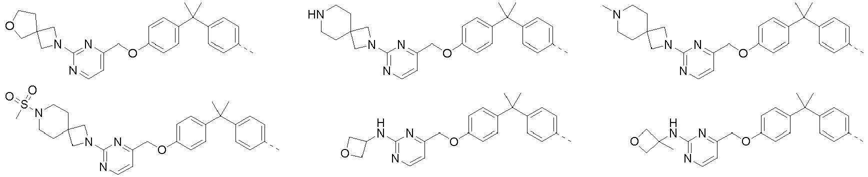

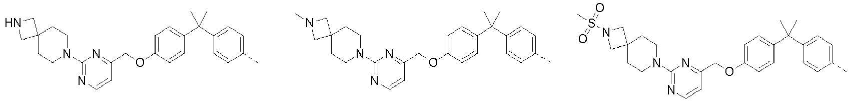

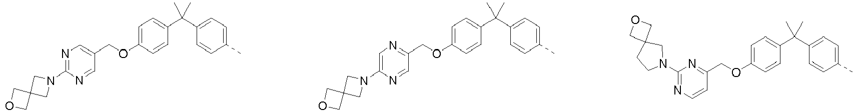

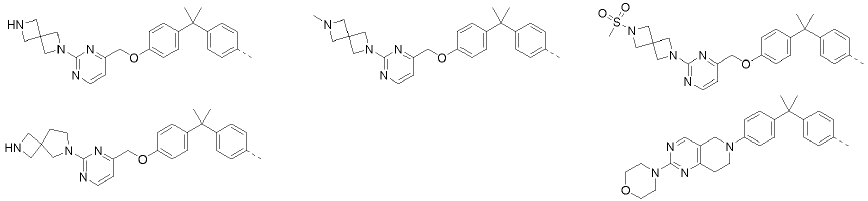

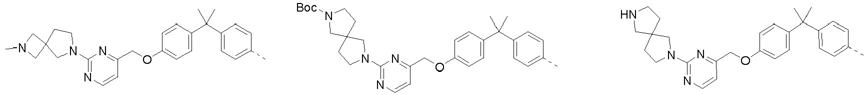

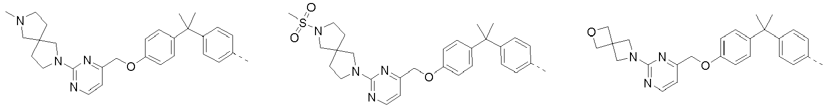

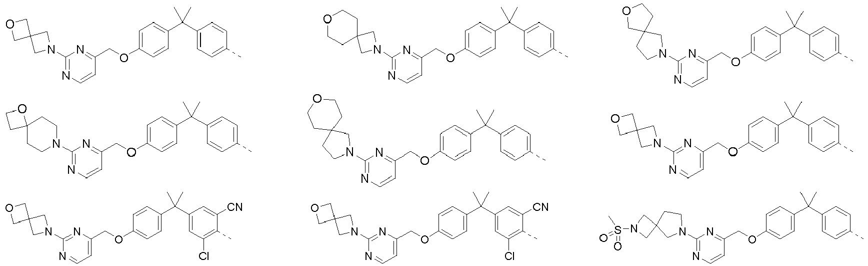

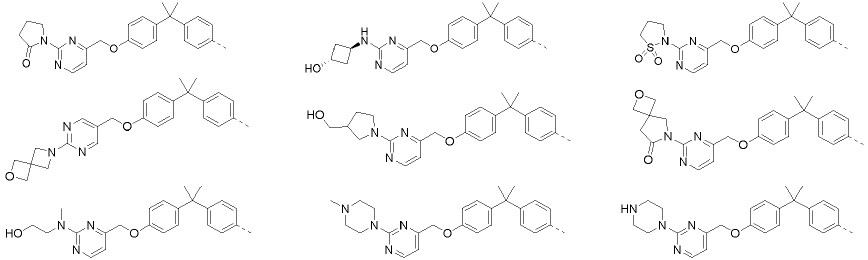

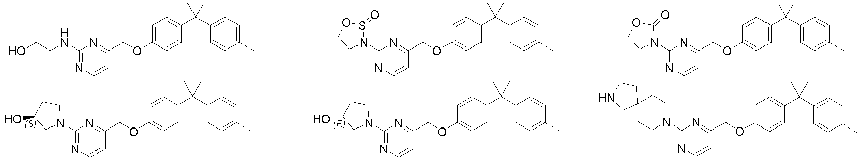

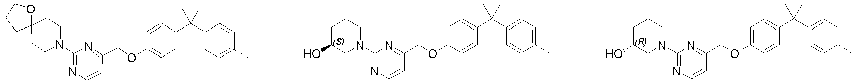

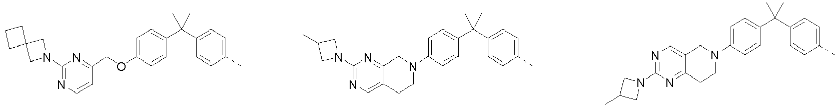

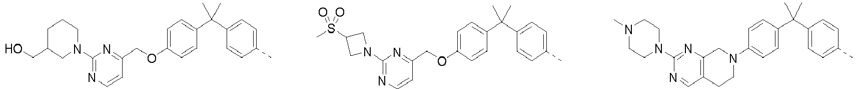

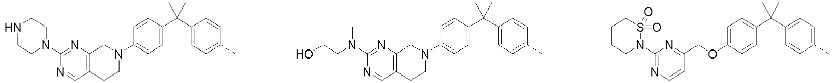

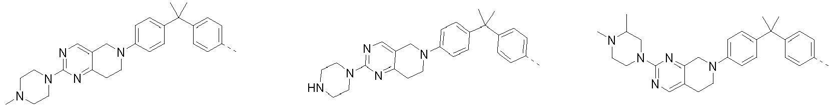

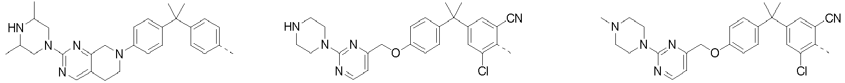

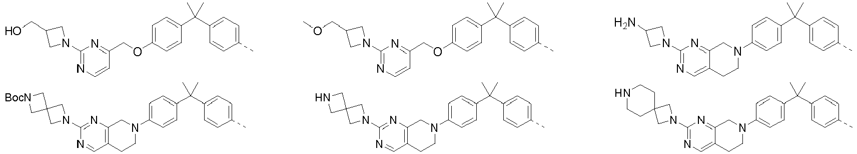

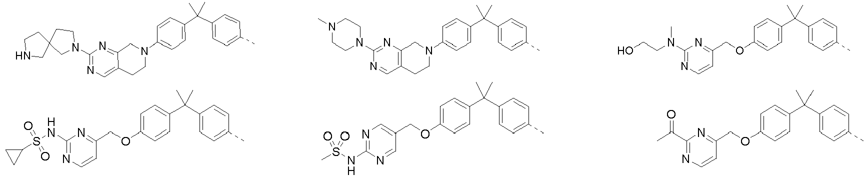

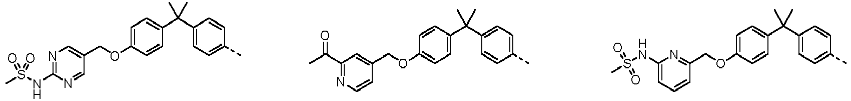

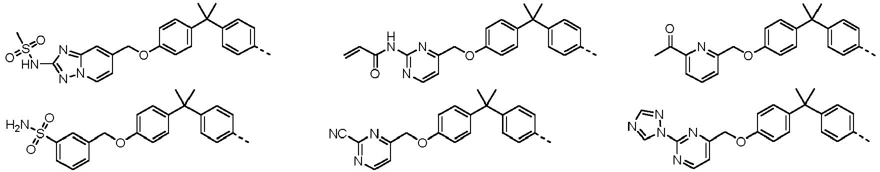

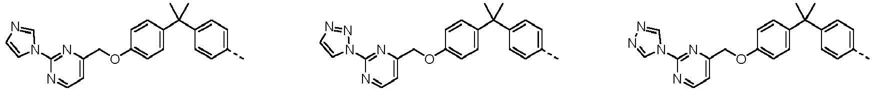

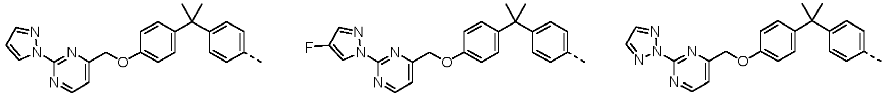

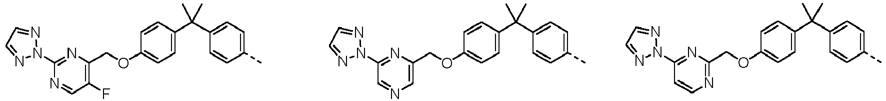

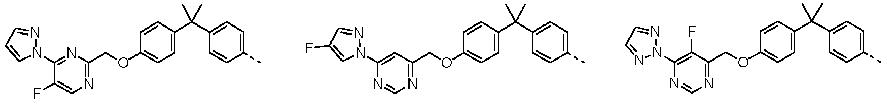

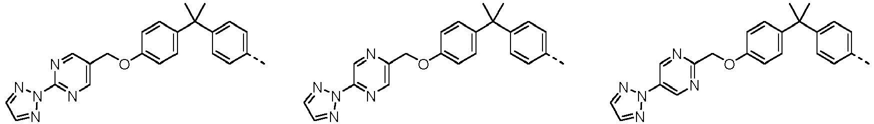

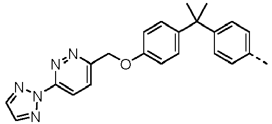

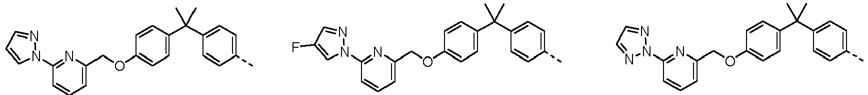

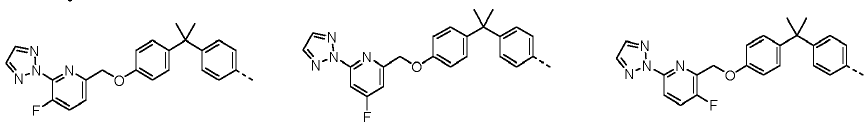

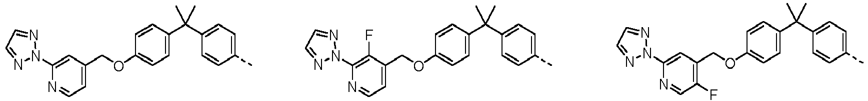

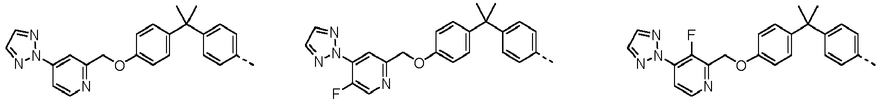

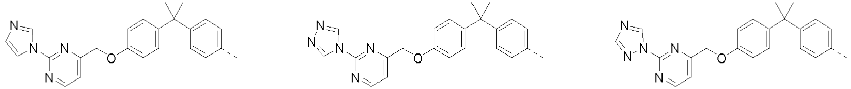

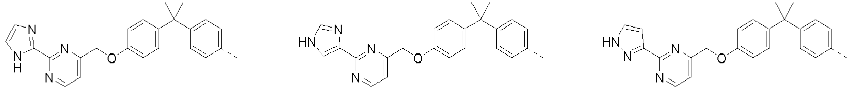

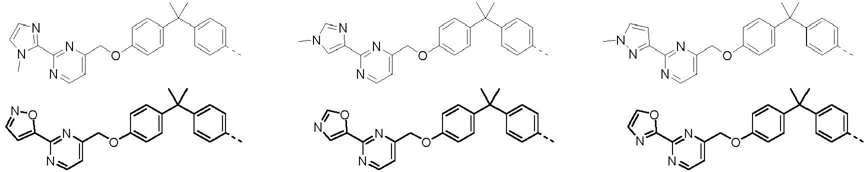

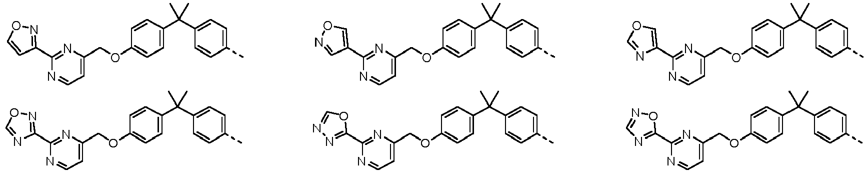

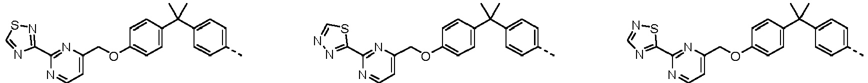

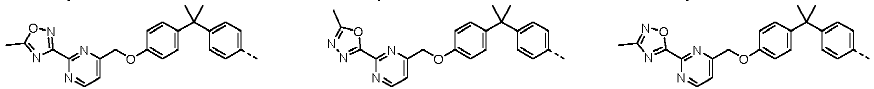

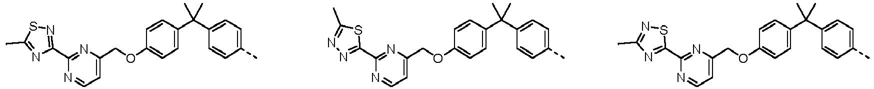

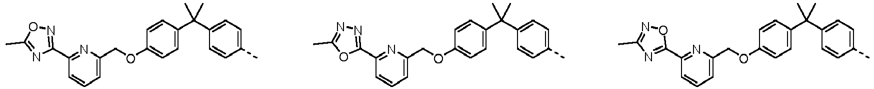

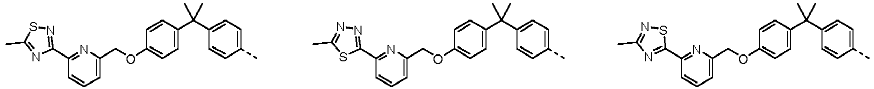

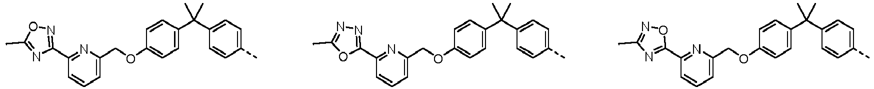

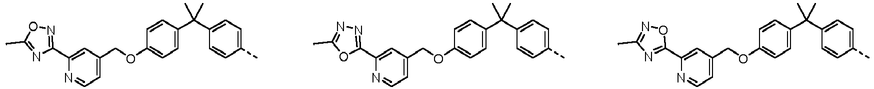

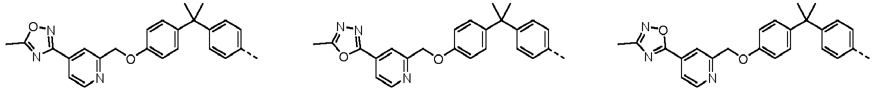

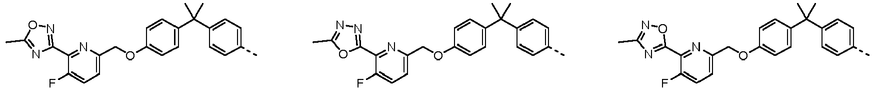

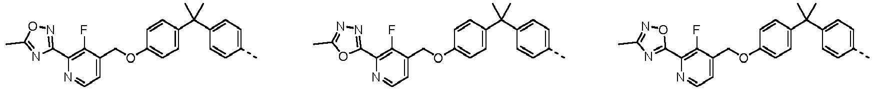

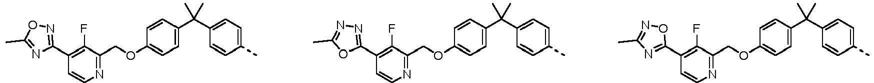

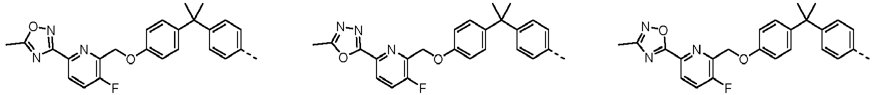

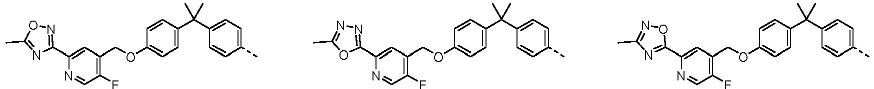

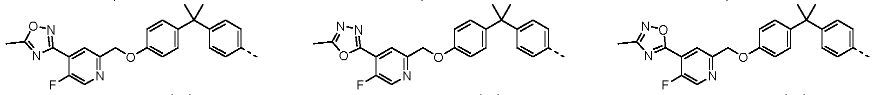

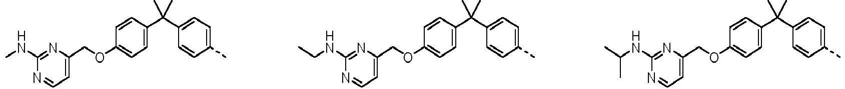

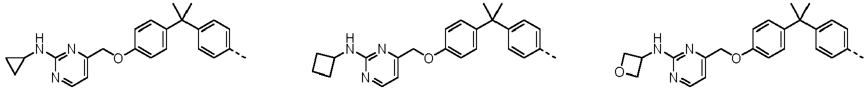

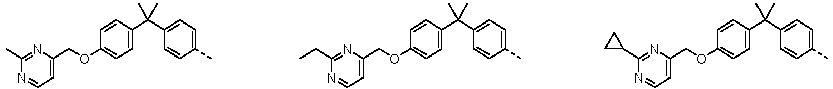

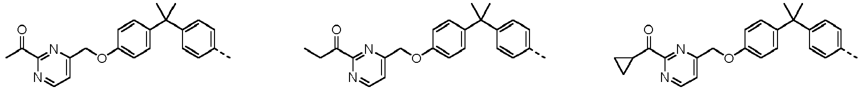

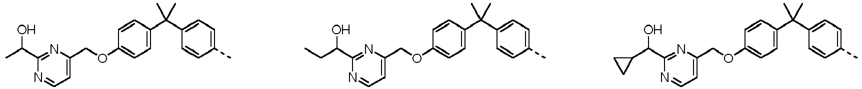

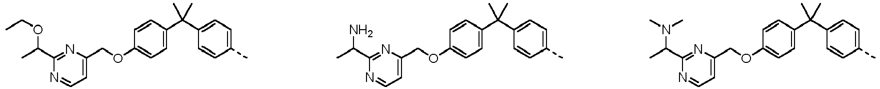

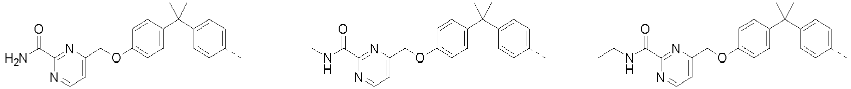

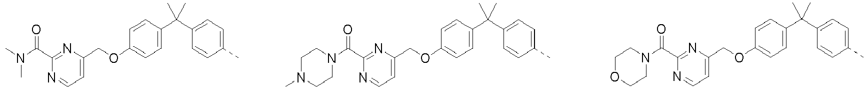

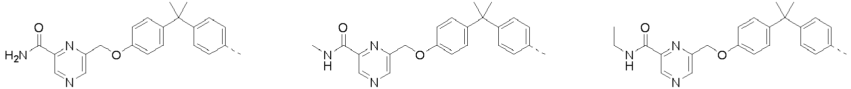

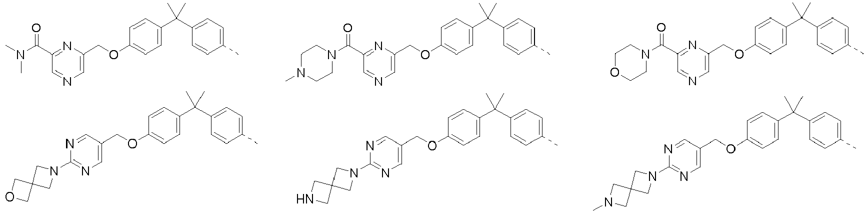

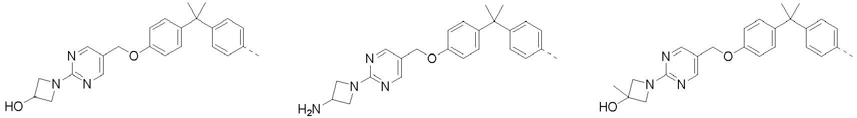

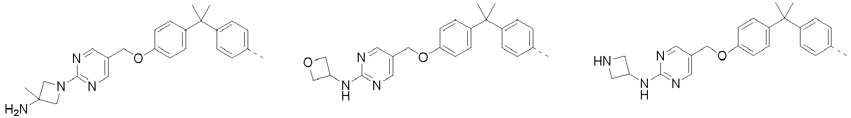

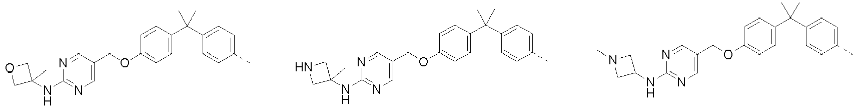

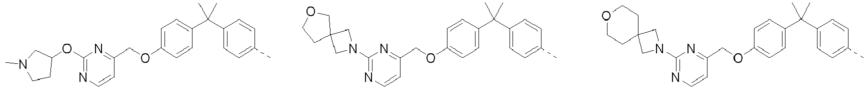

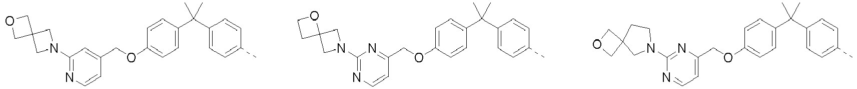

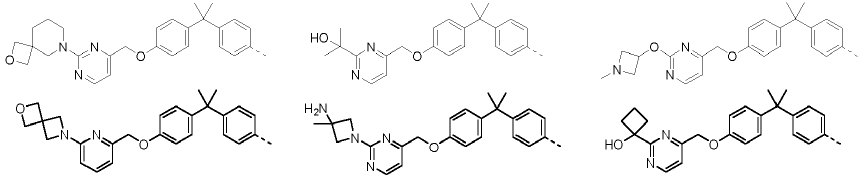

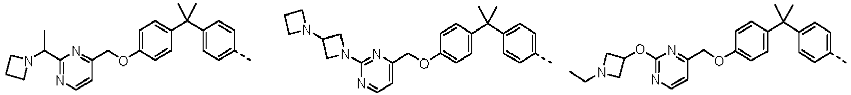

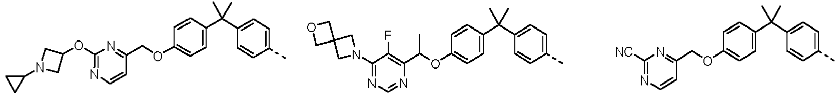

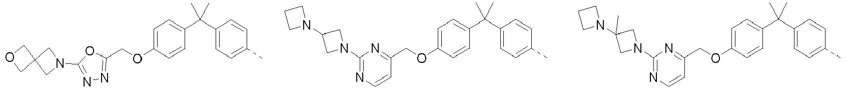

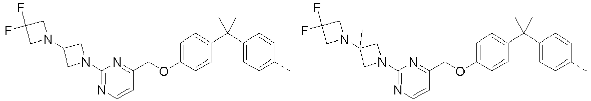

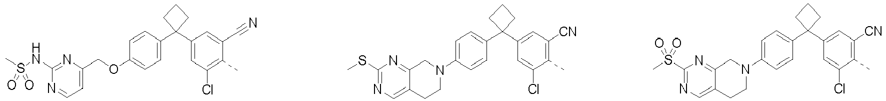

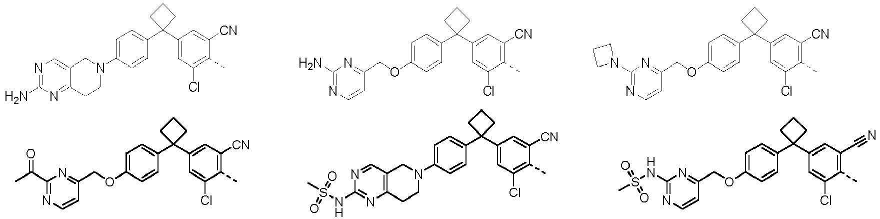

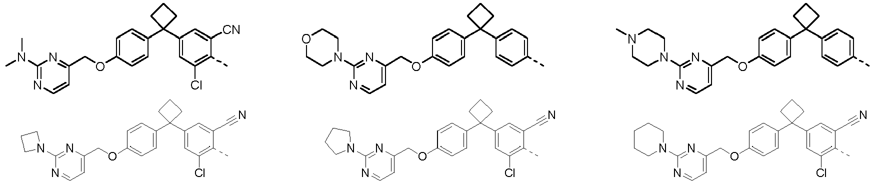

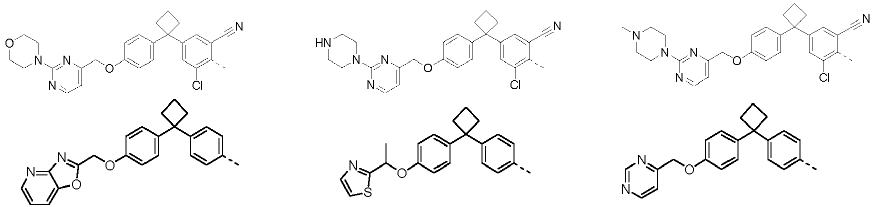

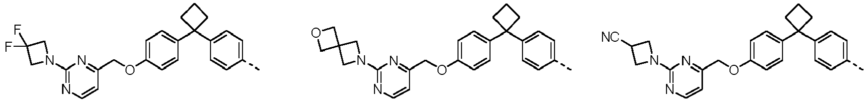

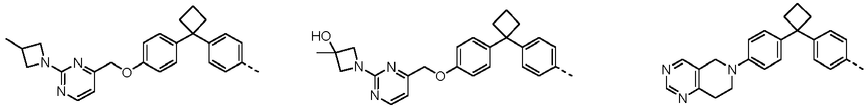

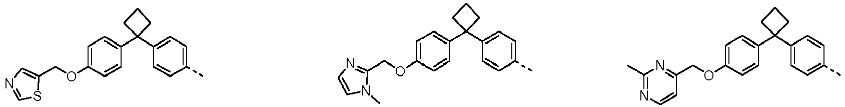

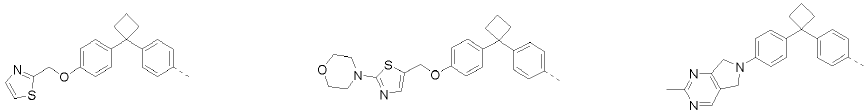

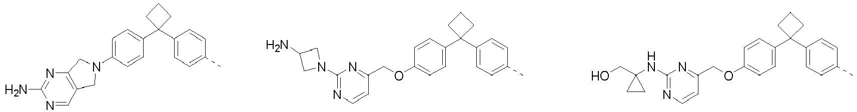

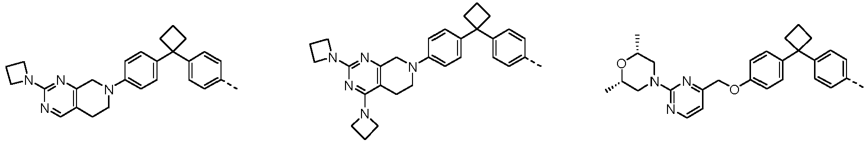

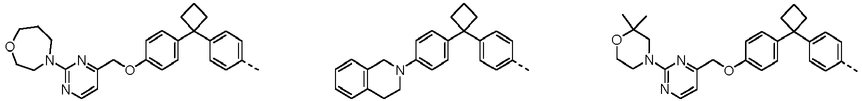

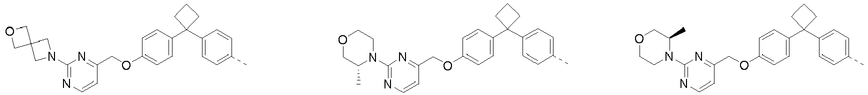

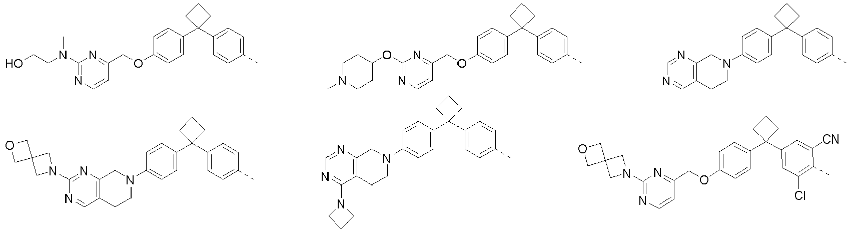

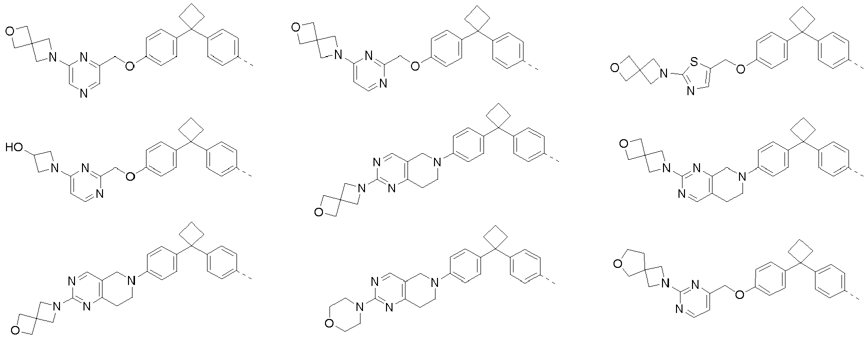

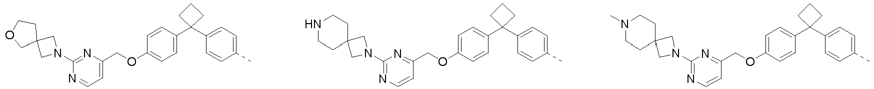

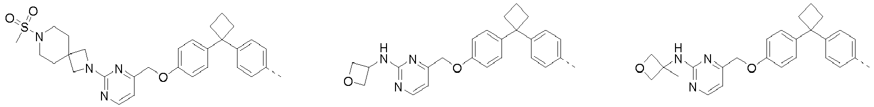

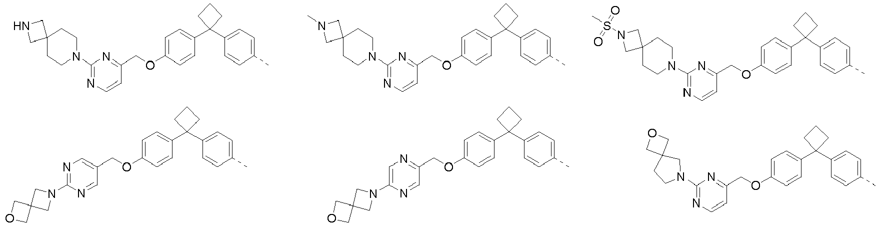

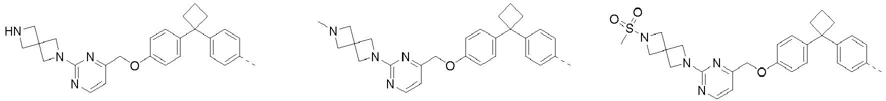

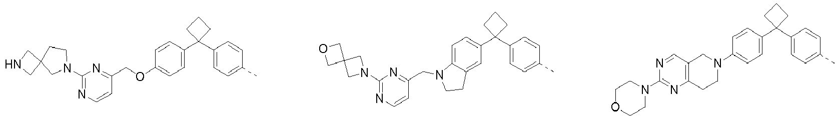

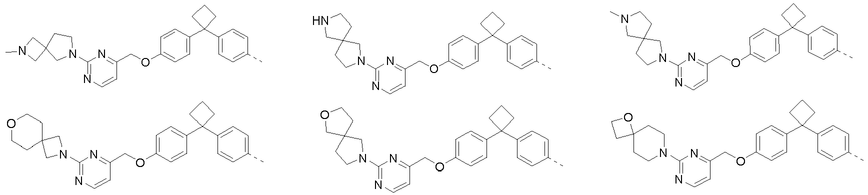

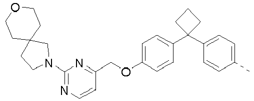

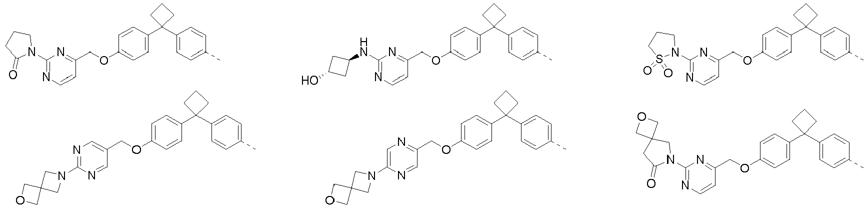

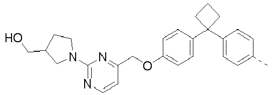

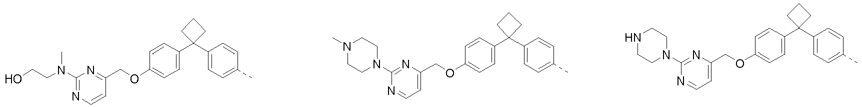

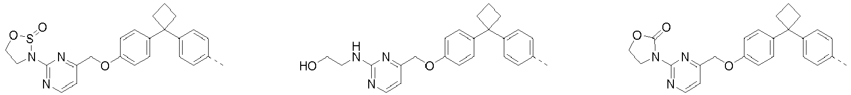

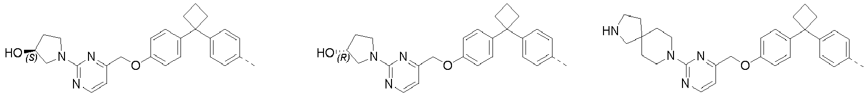

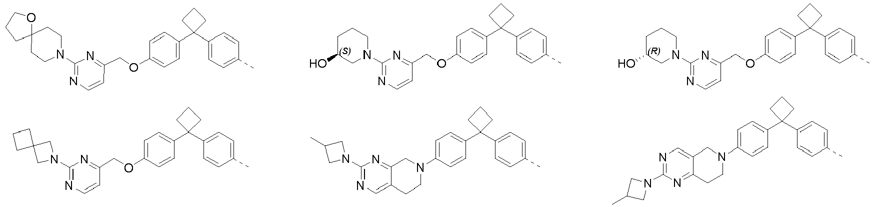

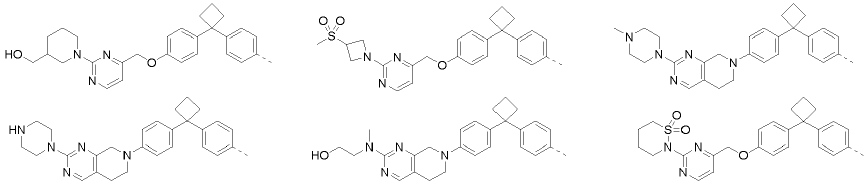

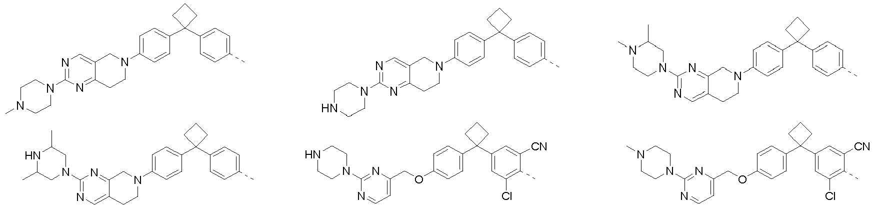

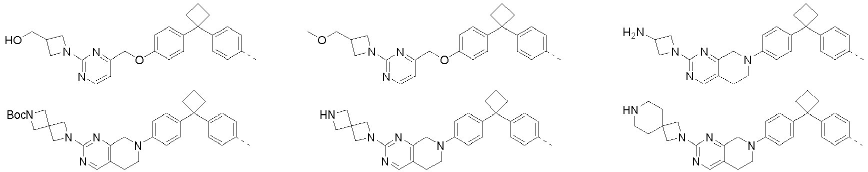

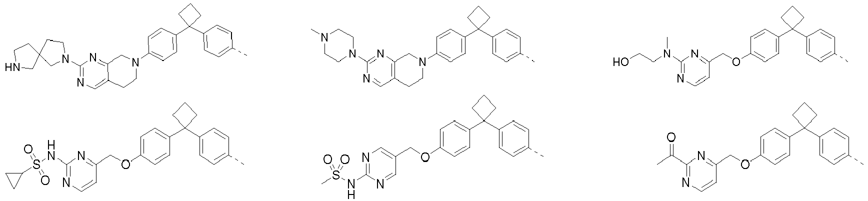

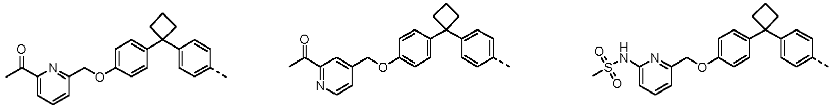

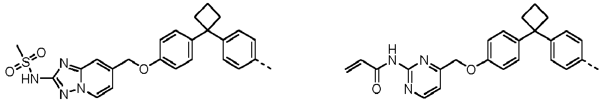

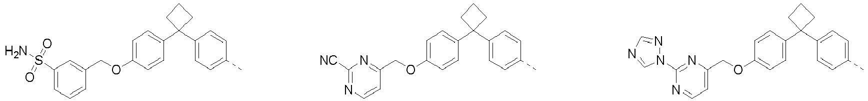

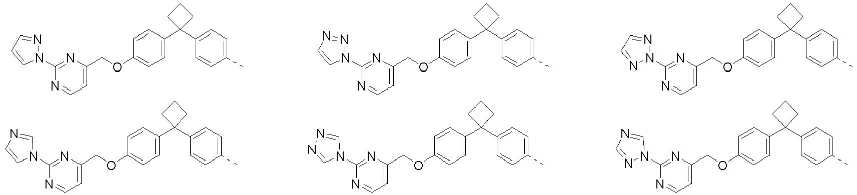

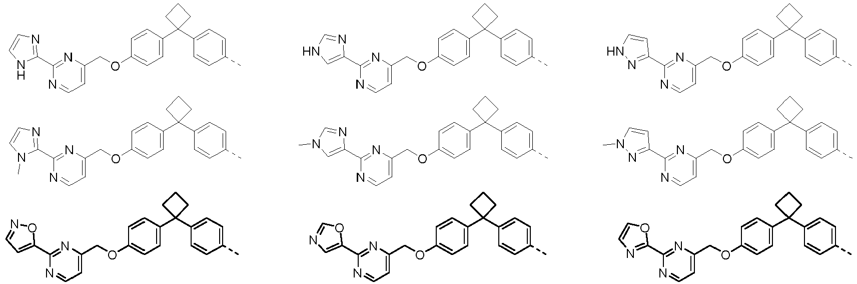

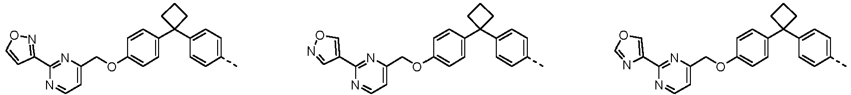

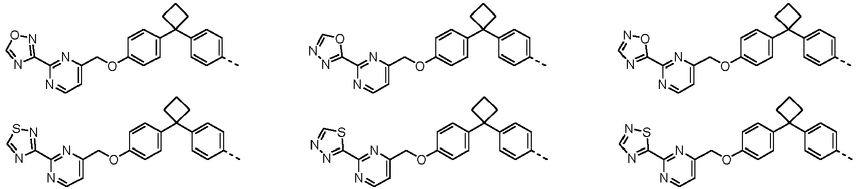

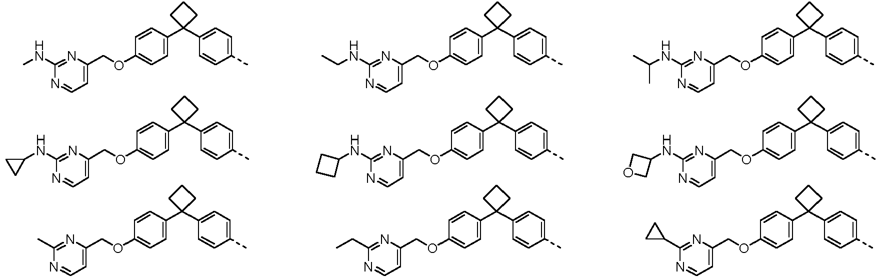

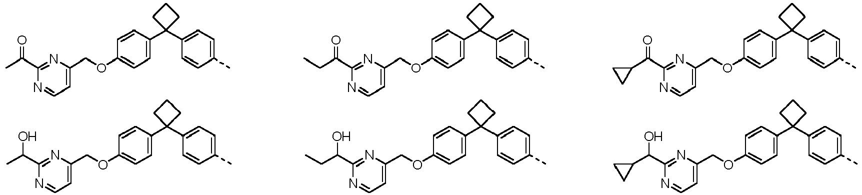

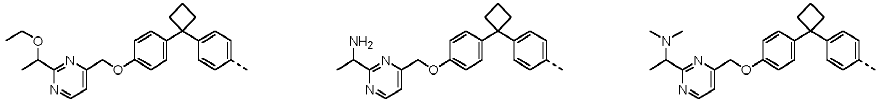

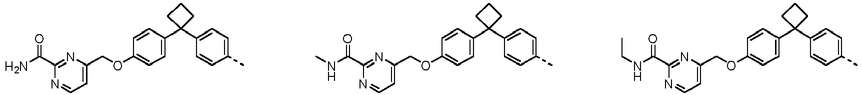

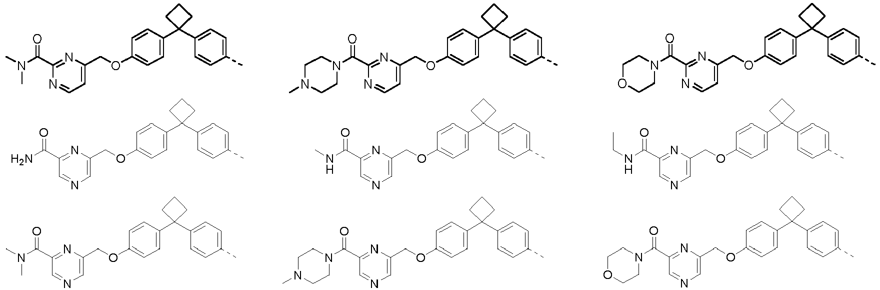

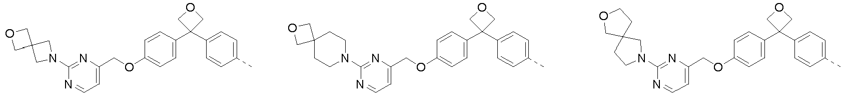

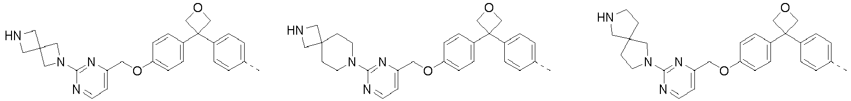

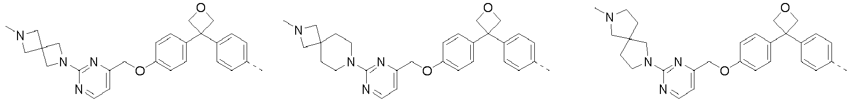

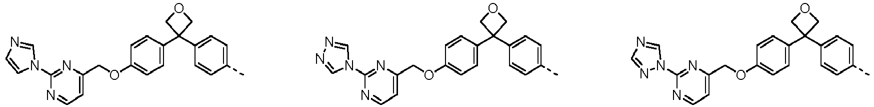

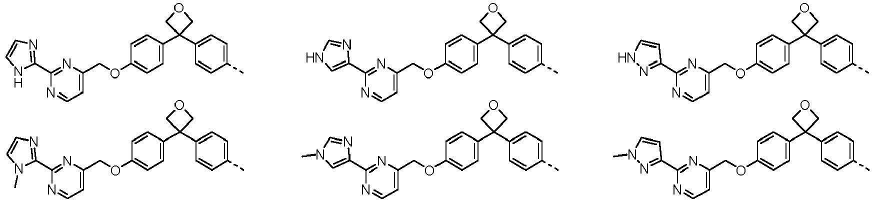

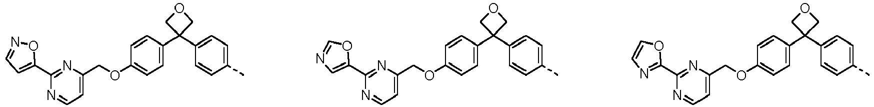

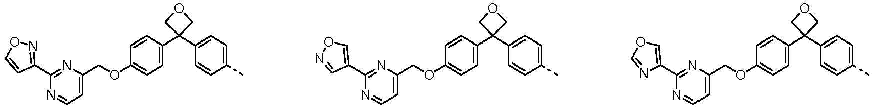

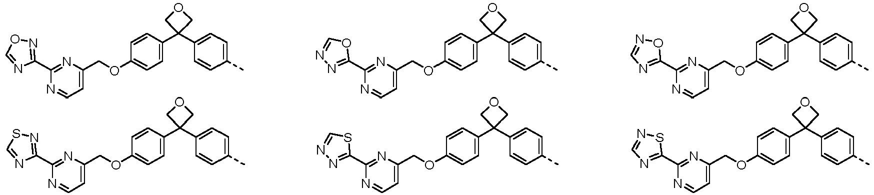

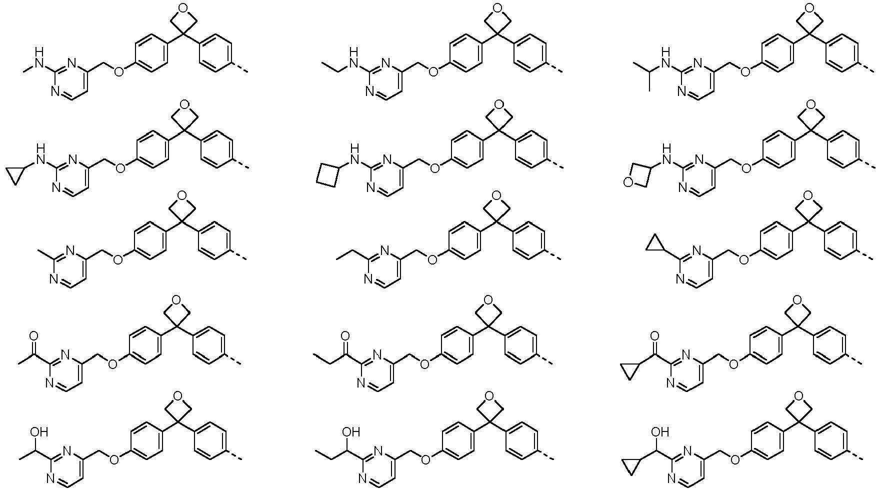

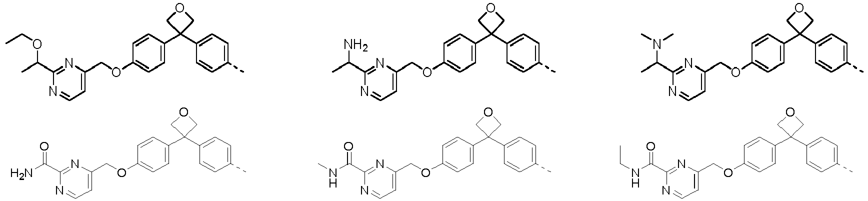

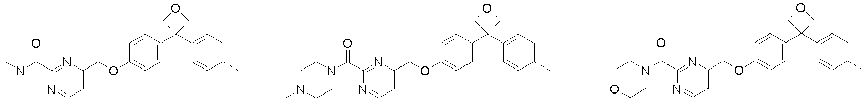

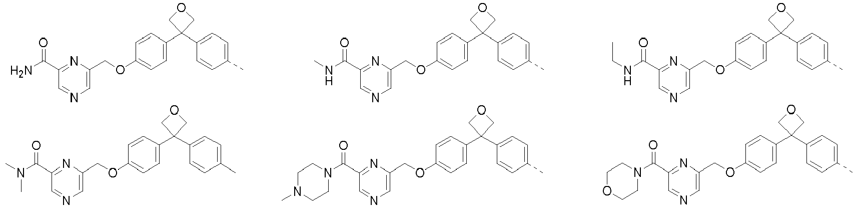

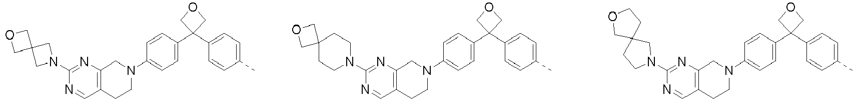

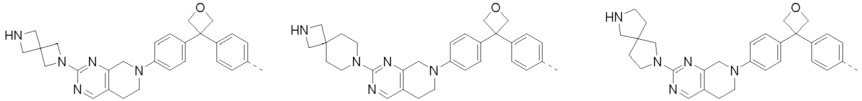

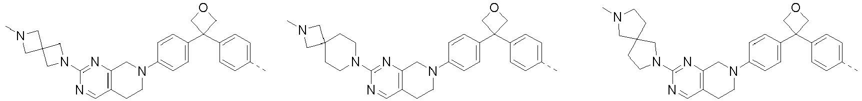

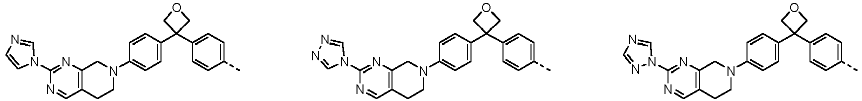

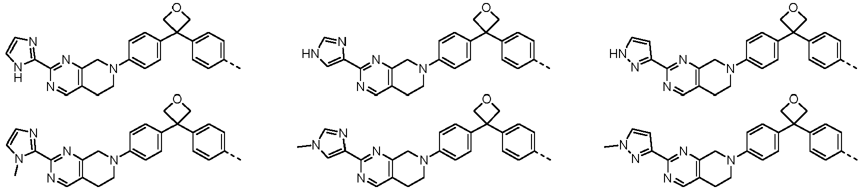

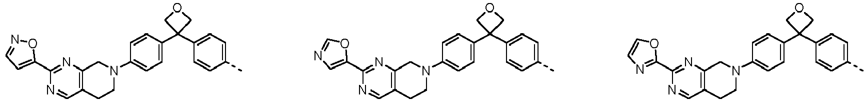

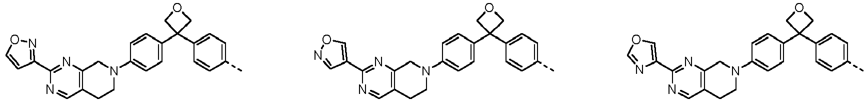

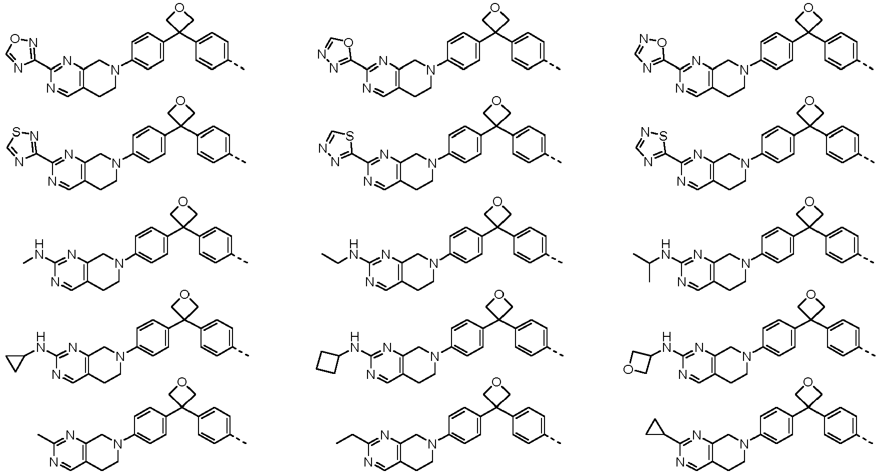

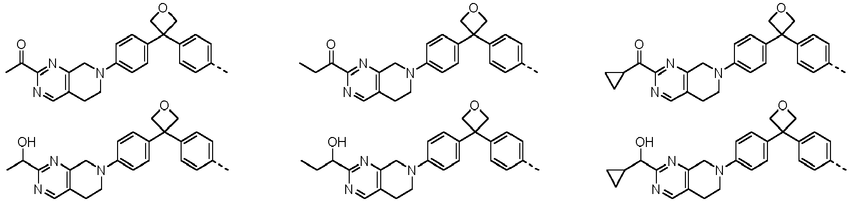

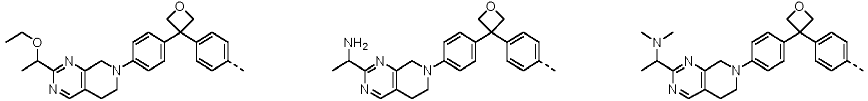

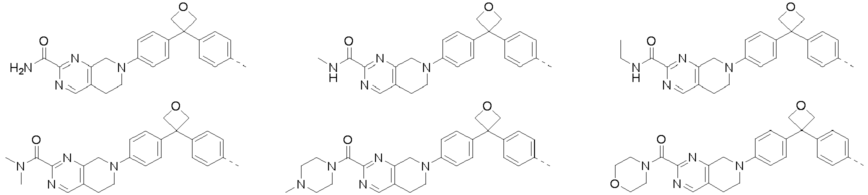

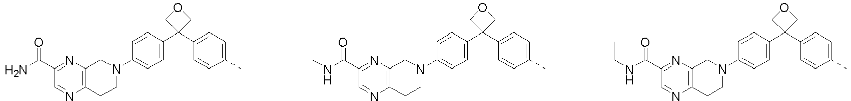

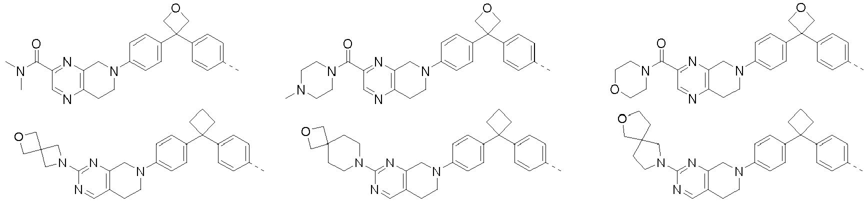

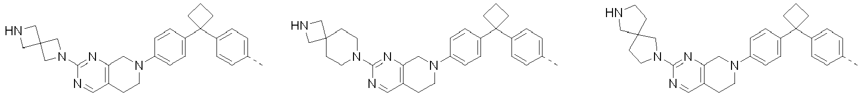

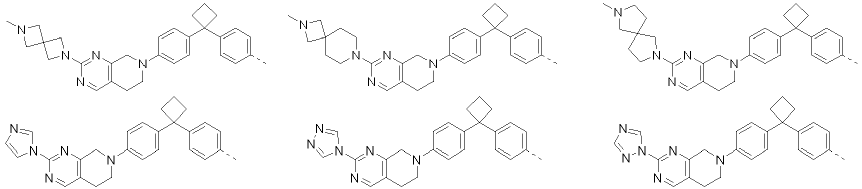

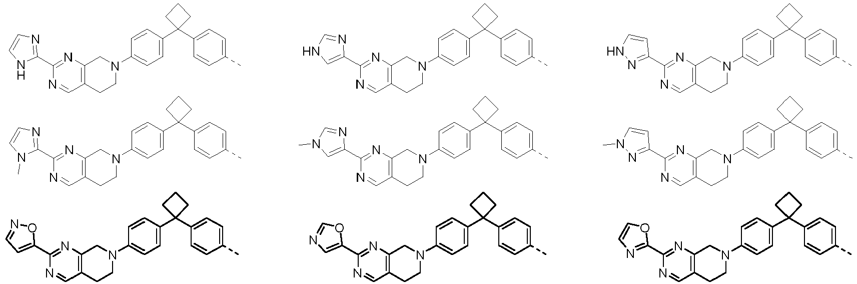

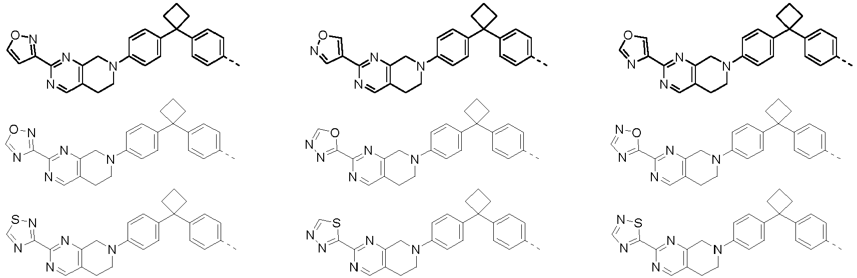

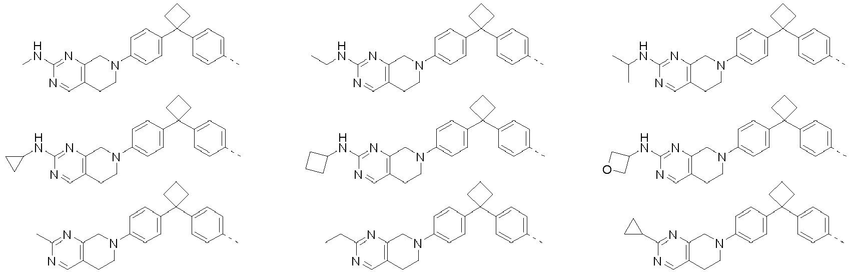

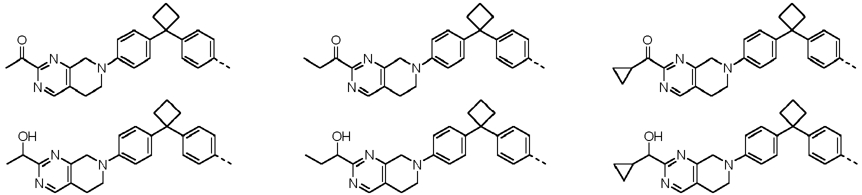

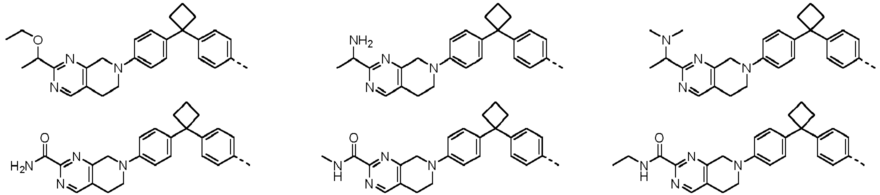

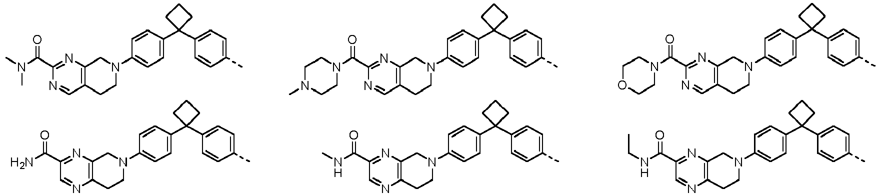

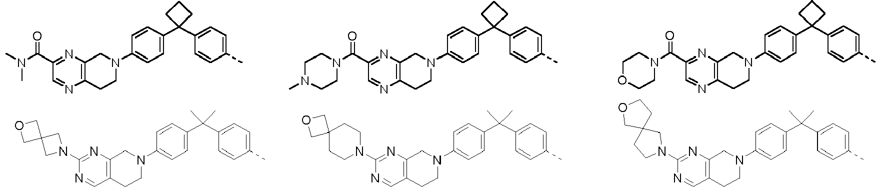

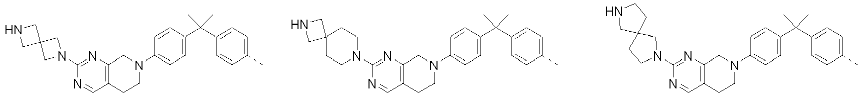

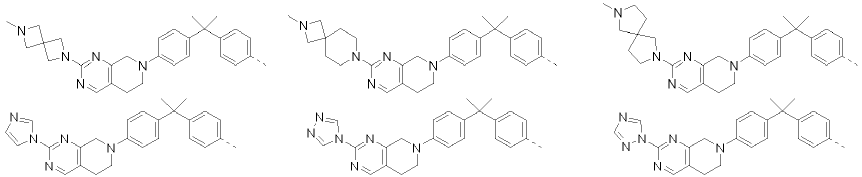

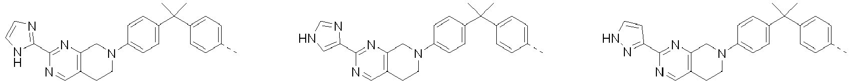

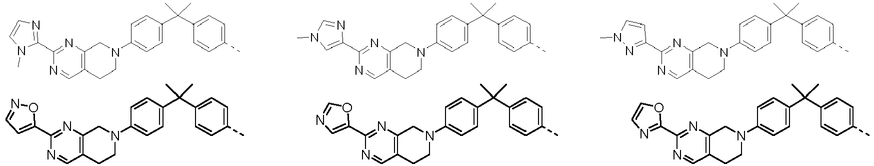

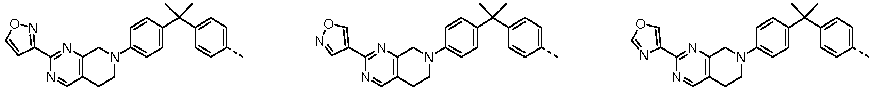

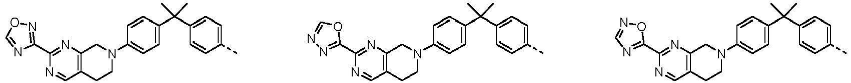

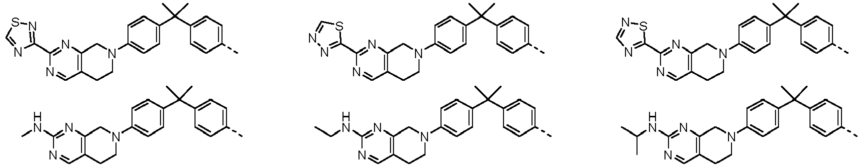

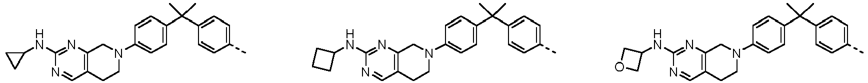

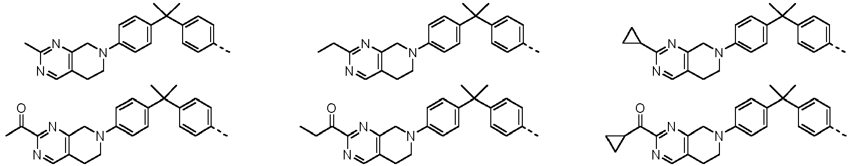

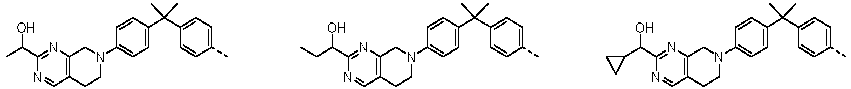

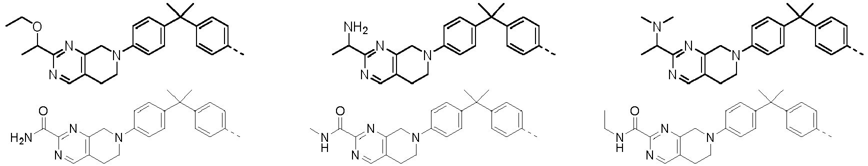

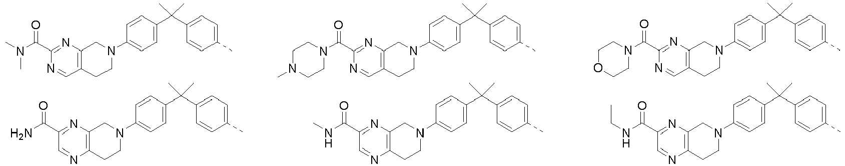

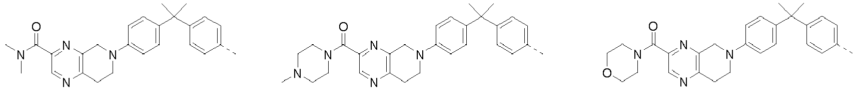

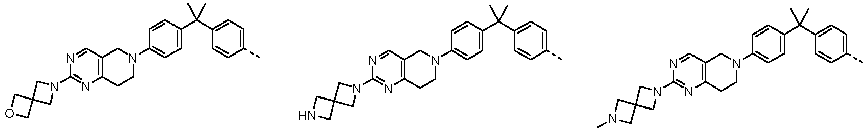

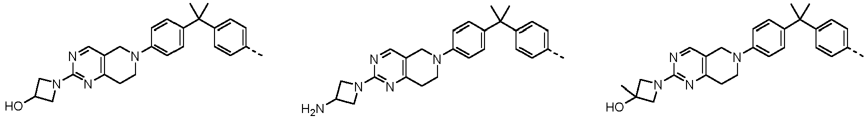

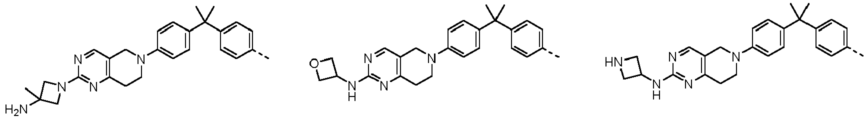

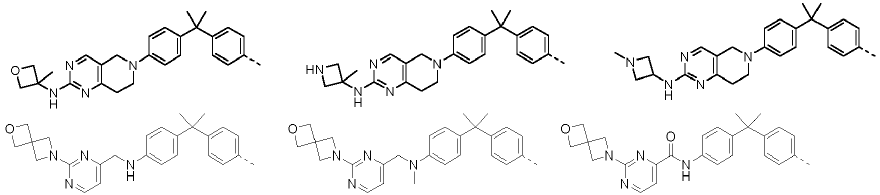

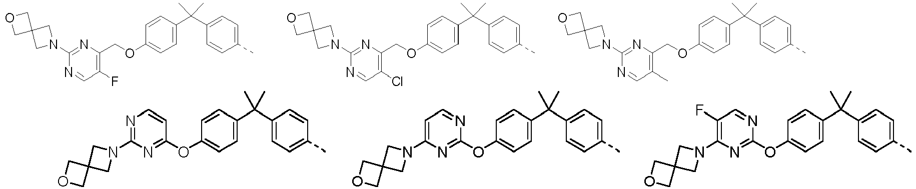

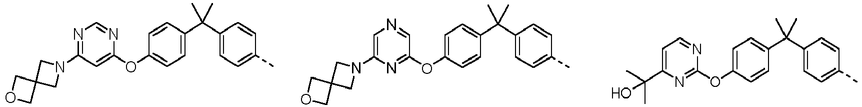

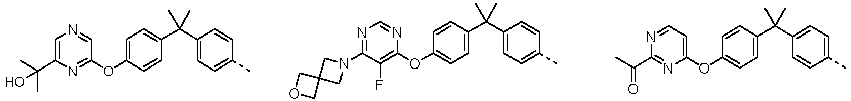

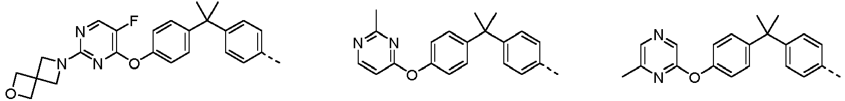

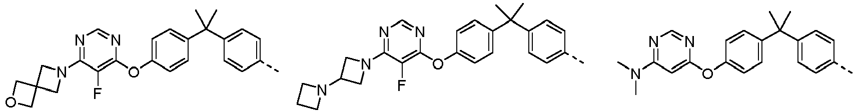

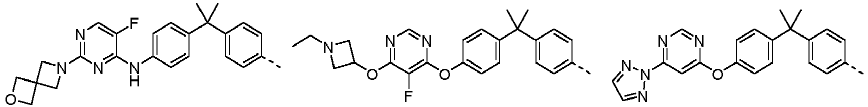

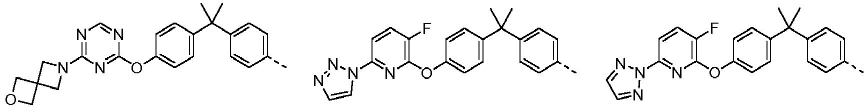

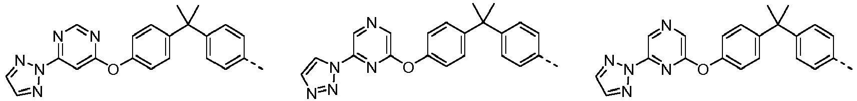

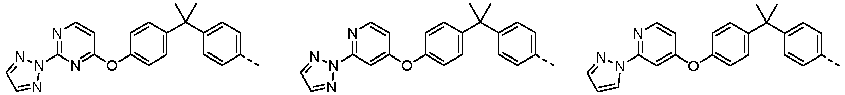

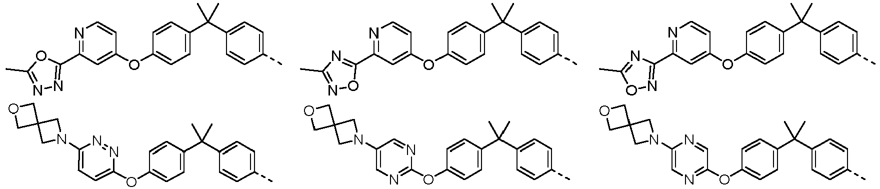

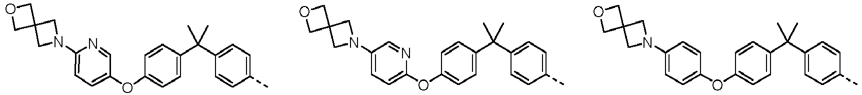

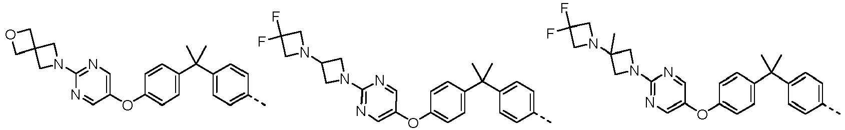

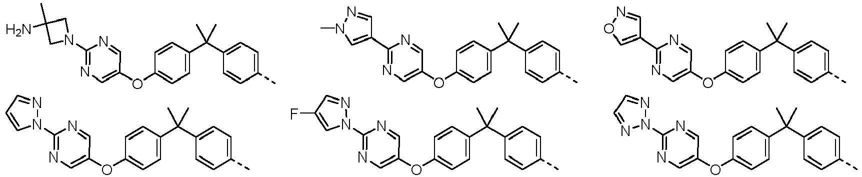

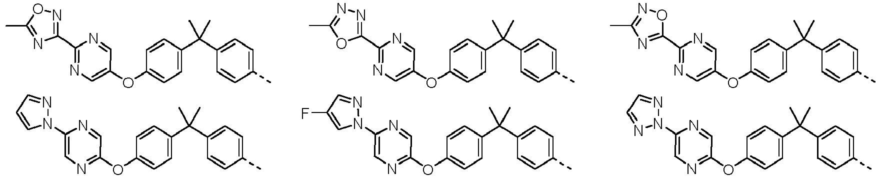

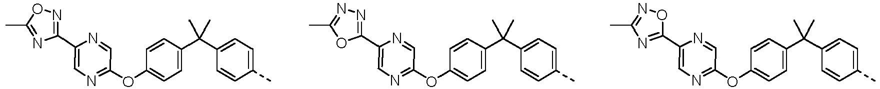

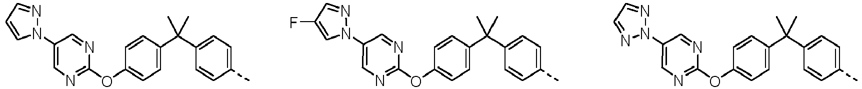

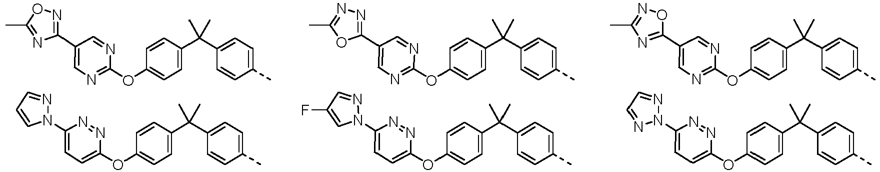

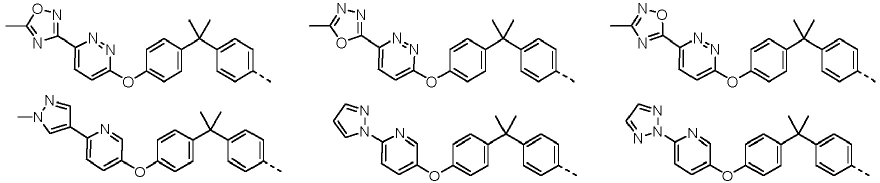

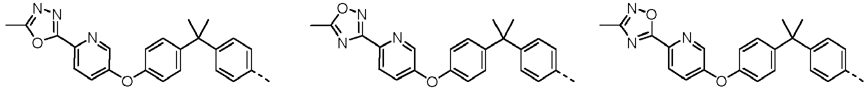

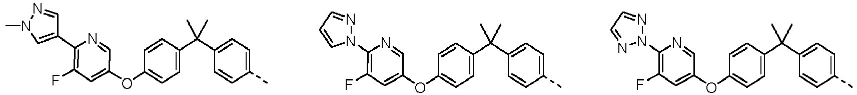

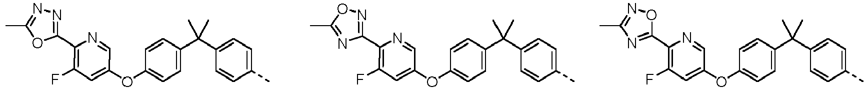

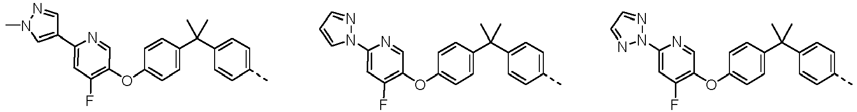

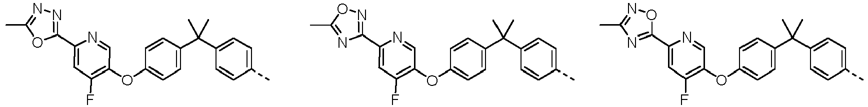

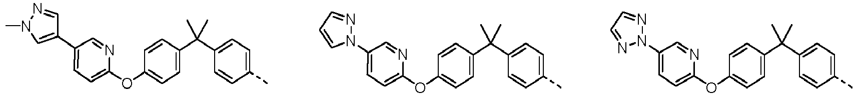

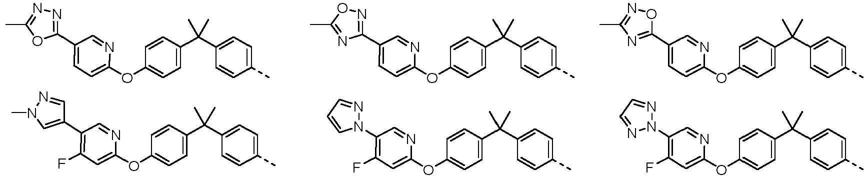

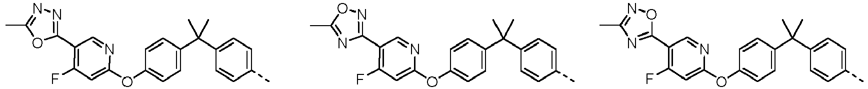

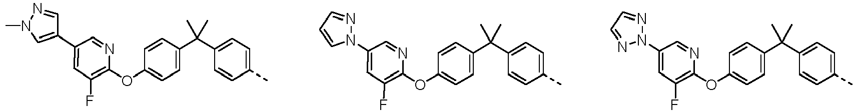

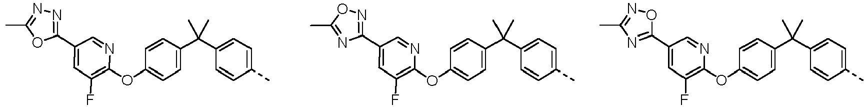

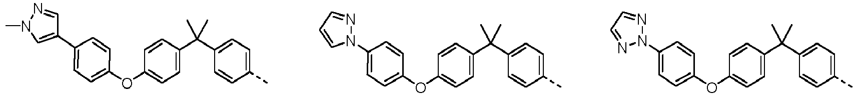

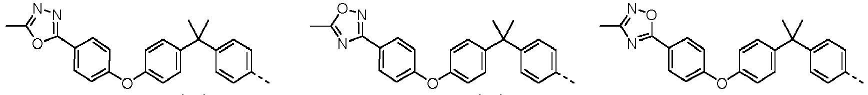

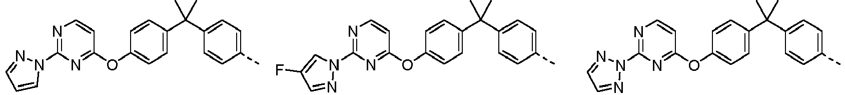

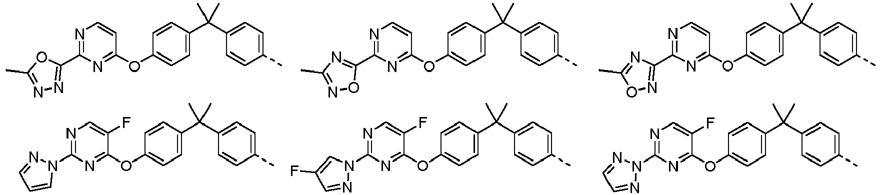

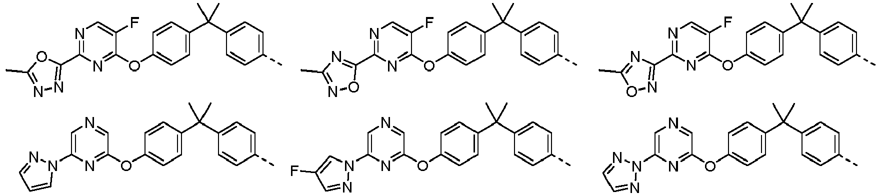

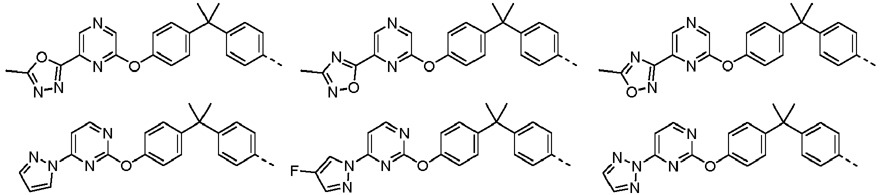

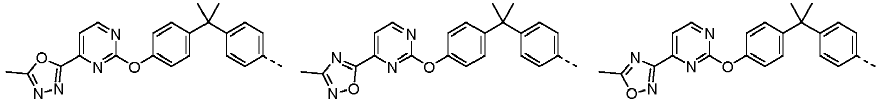

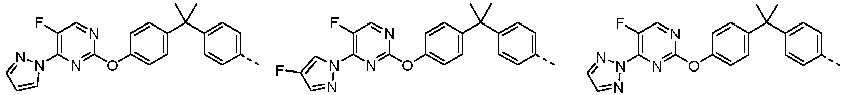

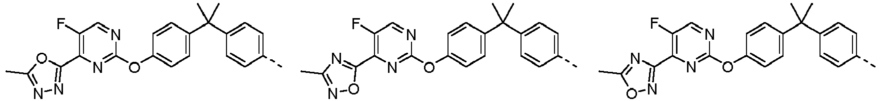

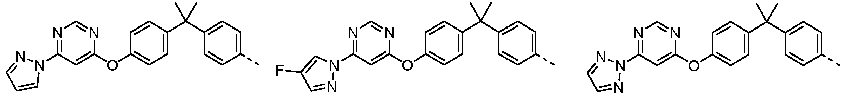

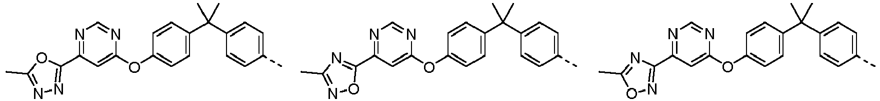

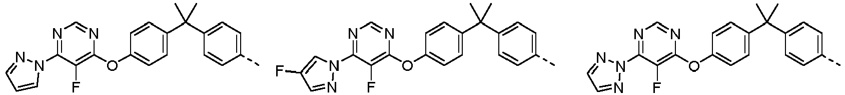

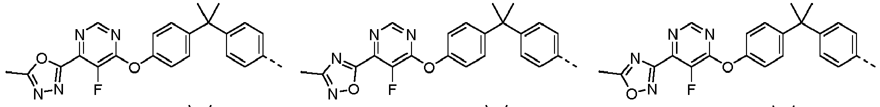

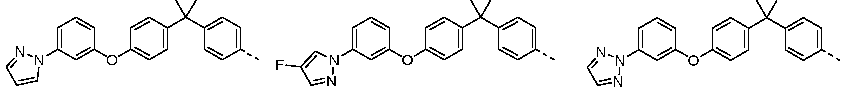

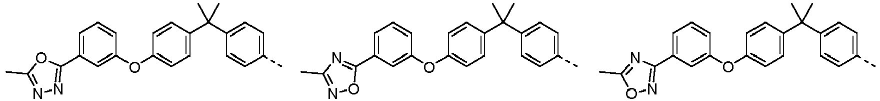

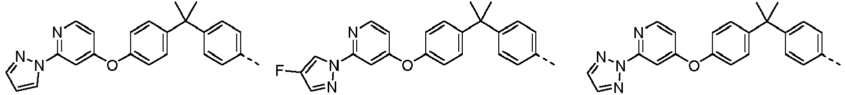

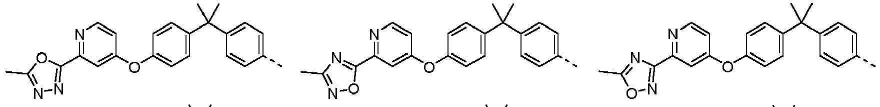

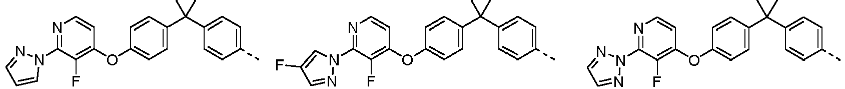

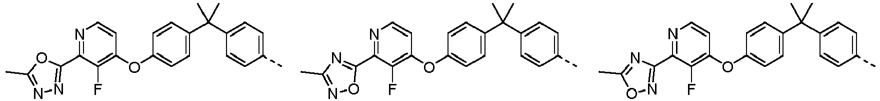

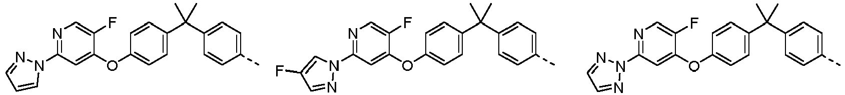

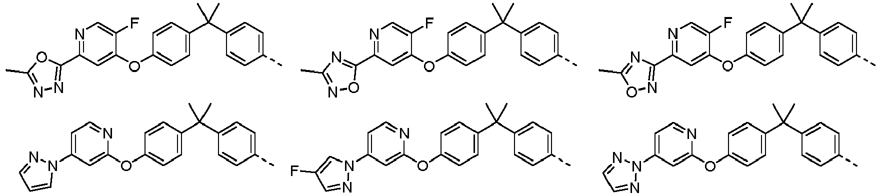

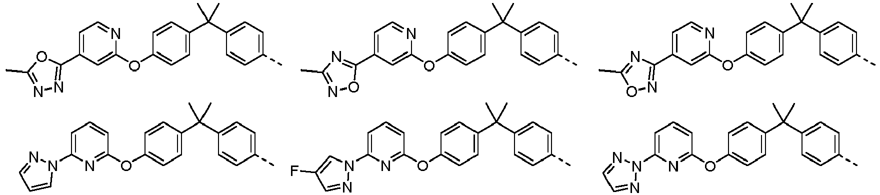

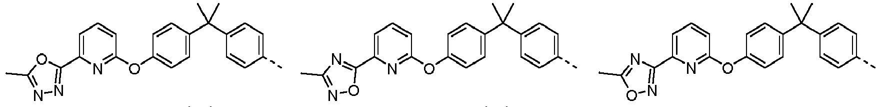

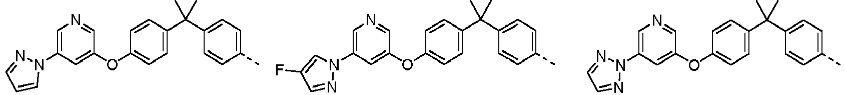

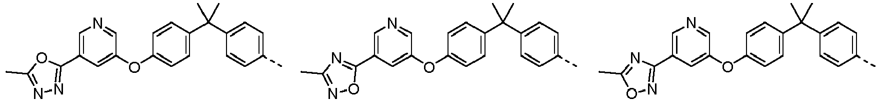

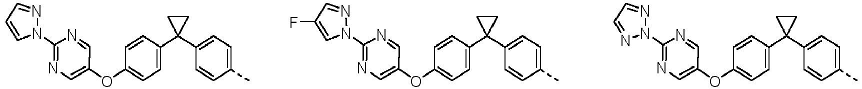

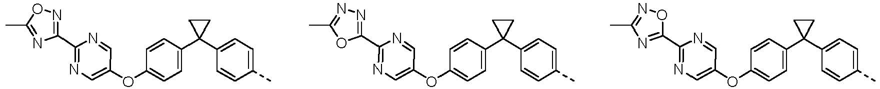

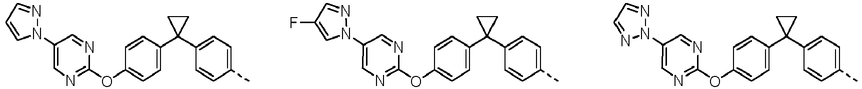

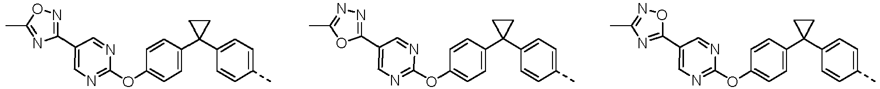

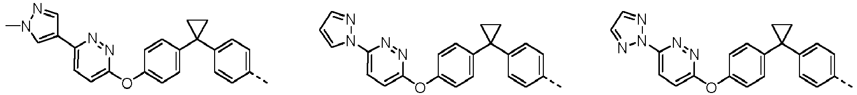

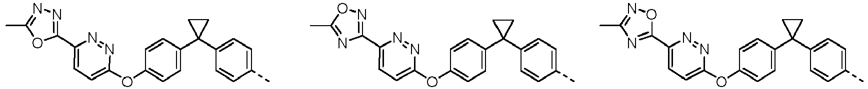

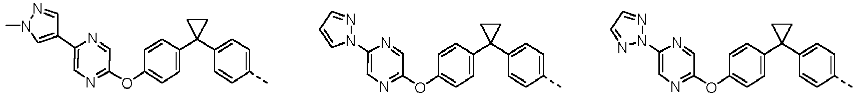

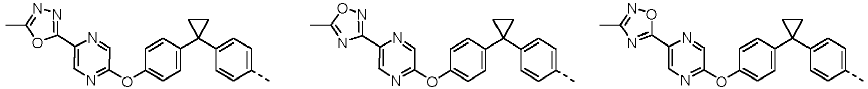

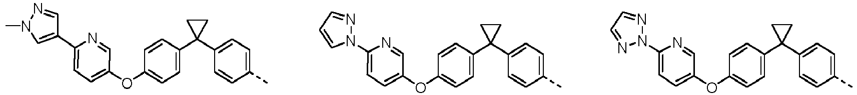

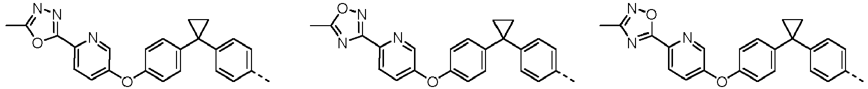

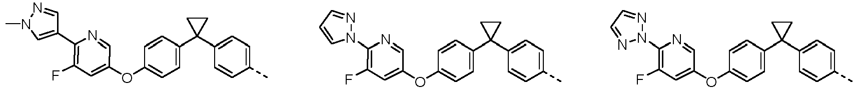

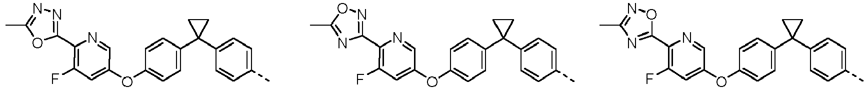

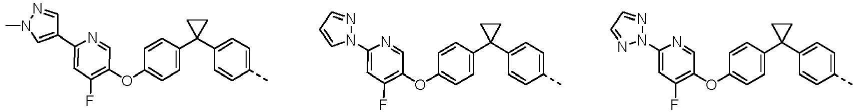

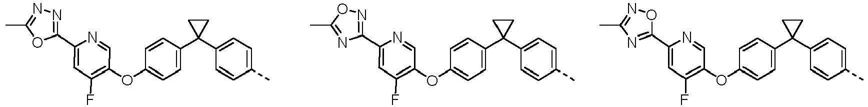

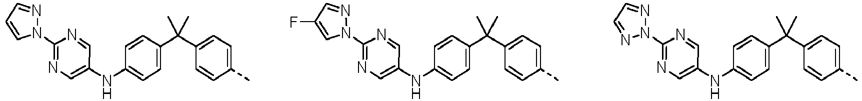

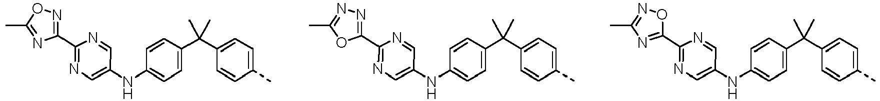

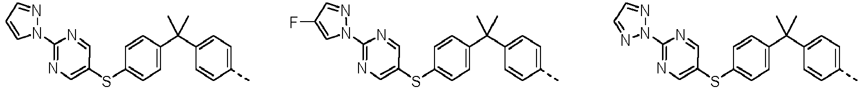

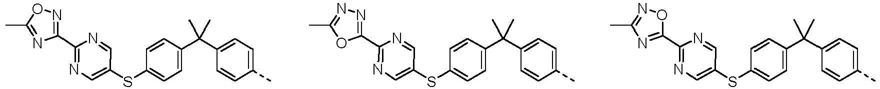

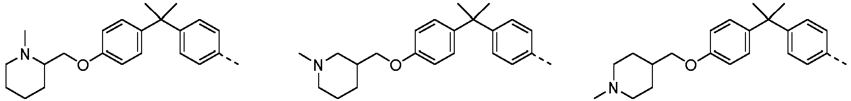

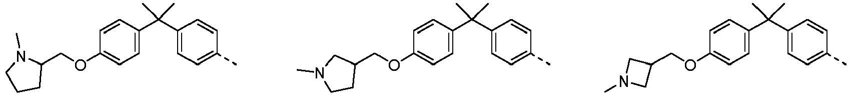

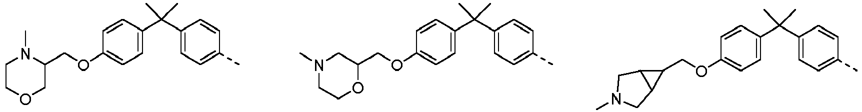

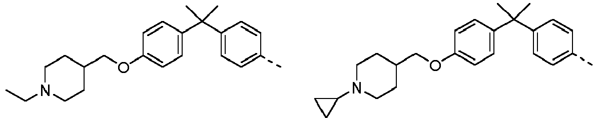

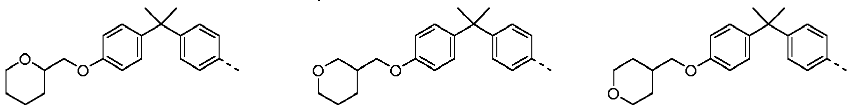

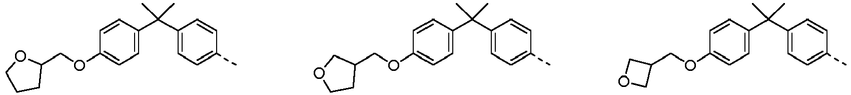

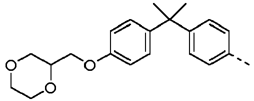

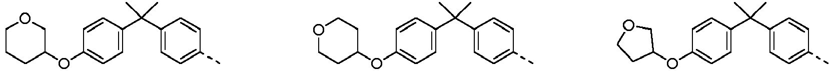

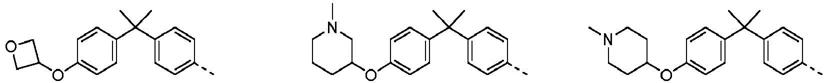

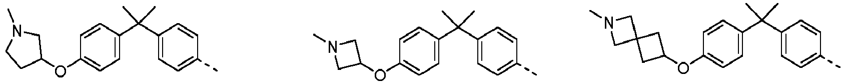

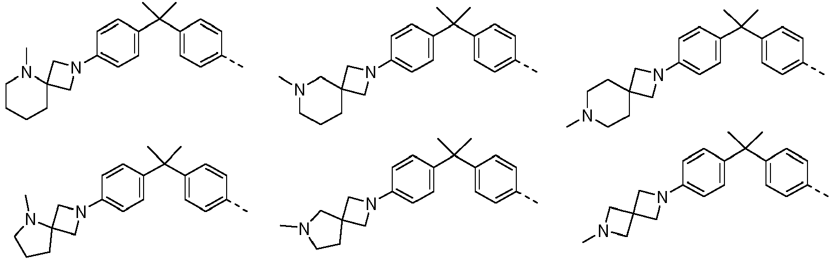

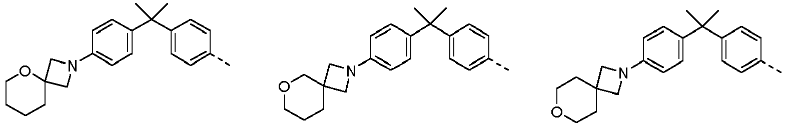

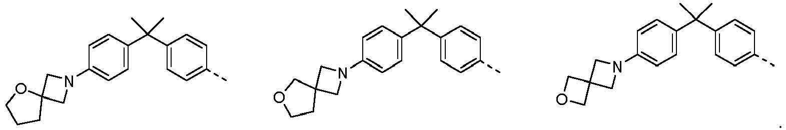

Further, in the above compounds, the structure of L moiety is selected from the group consisting of:

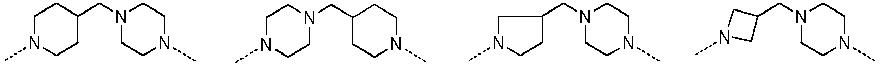

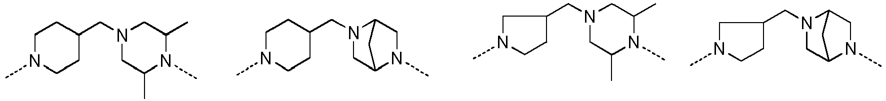

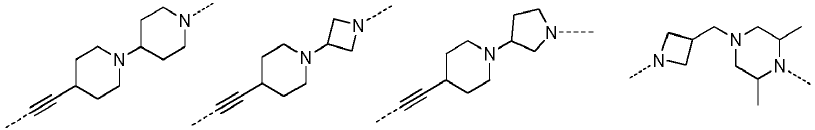

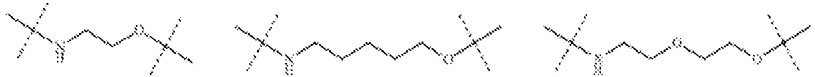

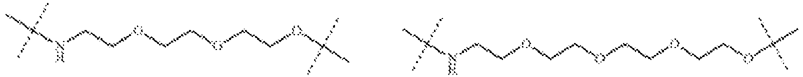

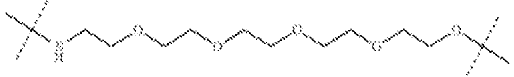

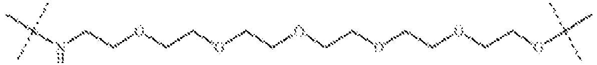

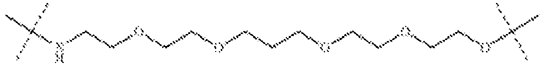

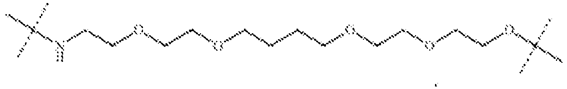

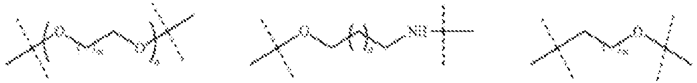

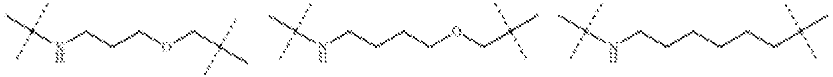

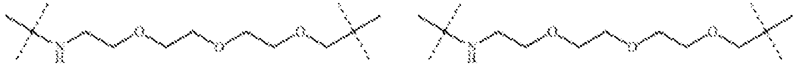

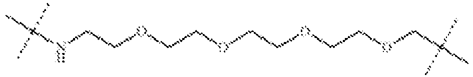

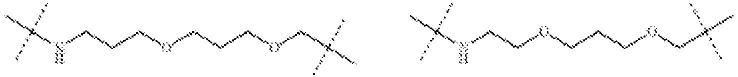

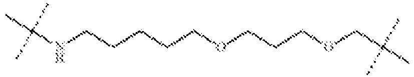

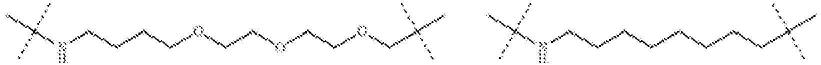

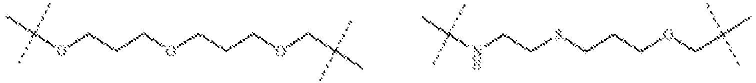

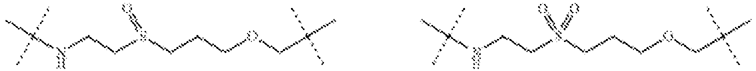

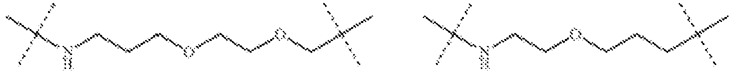

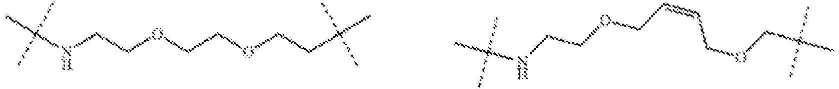

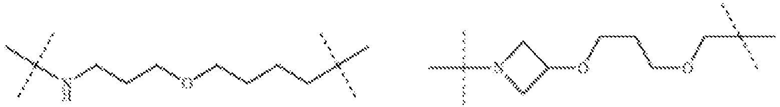

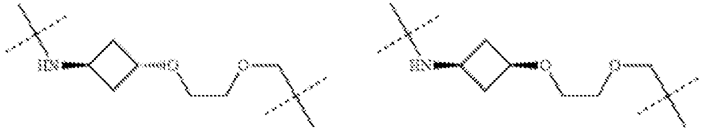

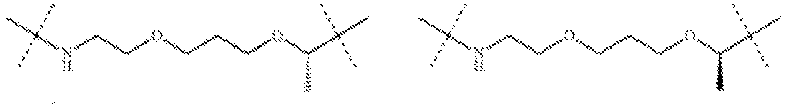

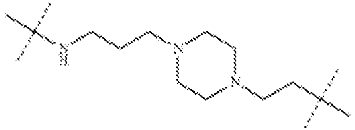

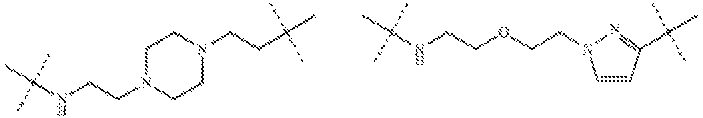

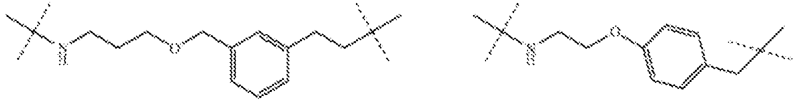

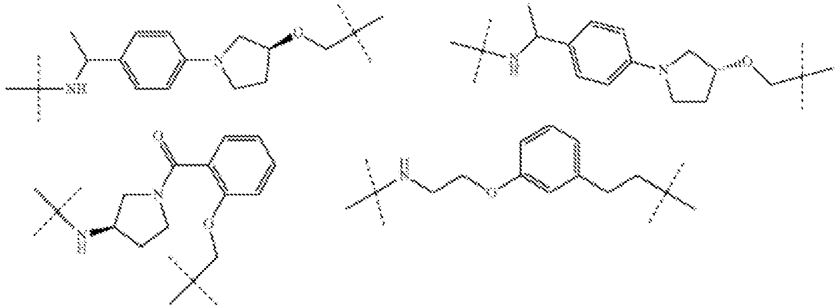

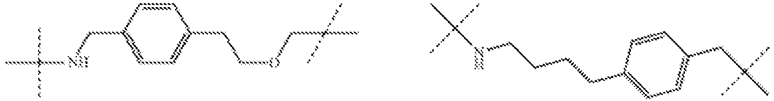

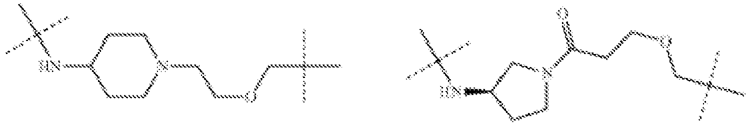

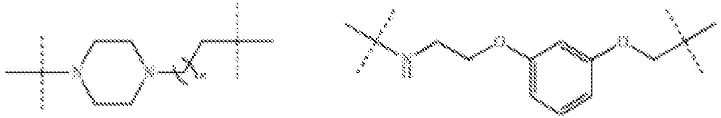

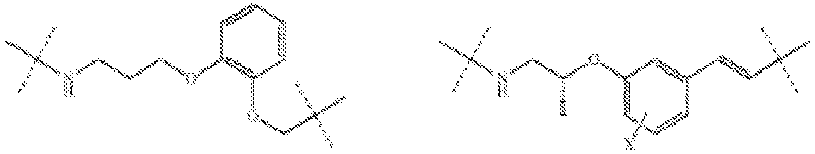

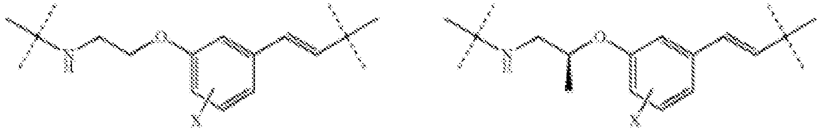

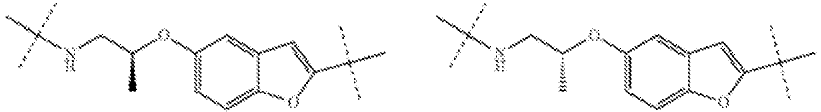

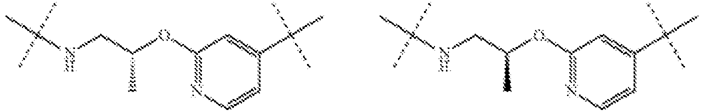

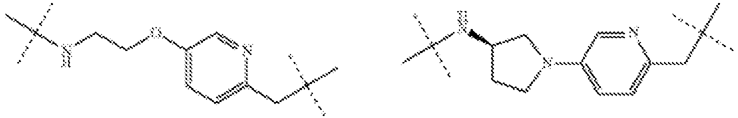

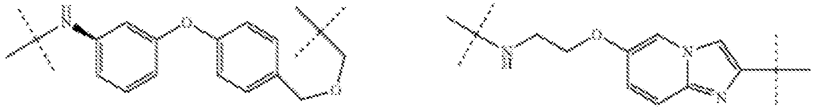

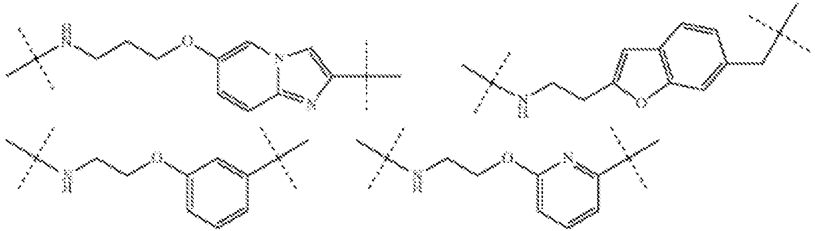

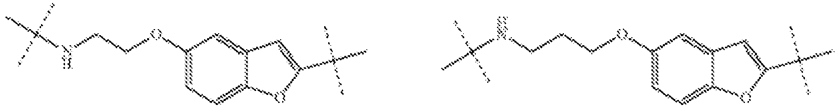

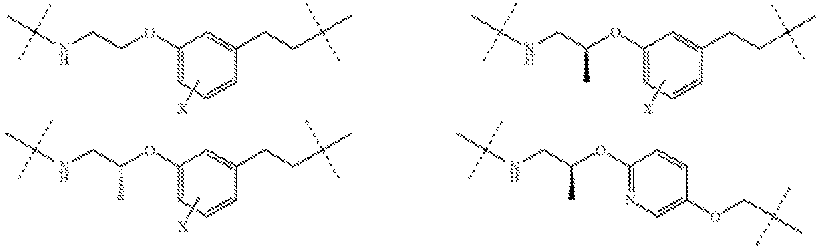

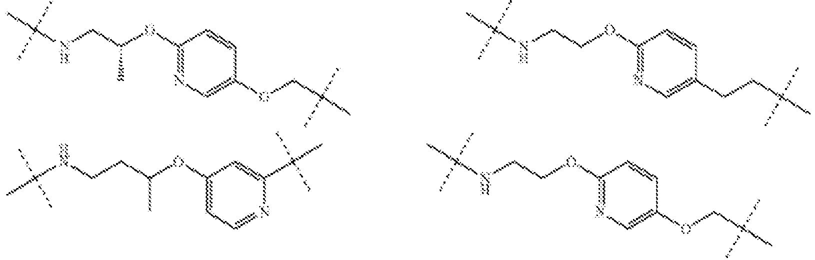

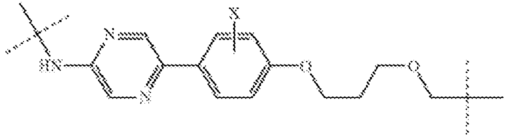

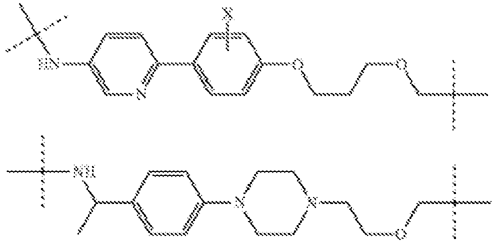

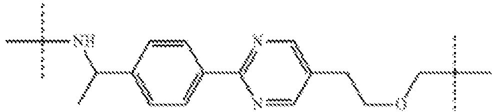

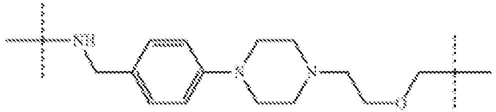

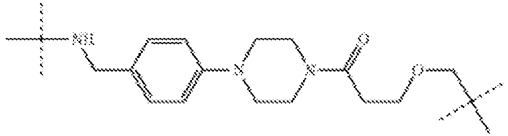

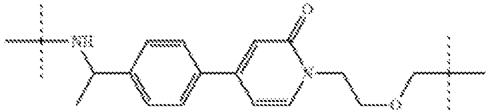

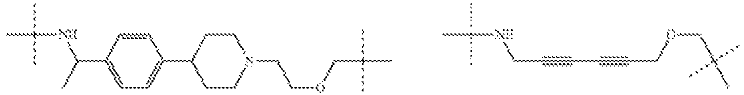

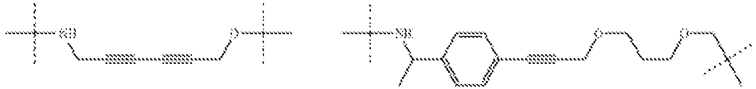

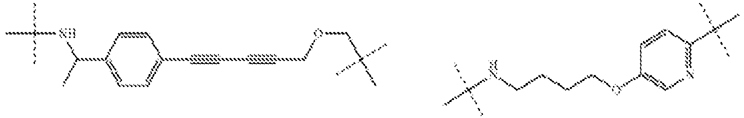

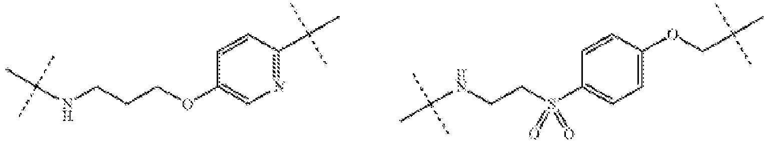

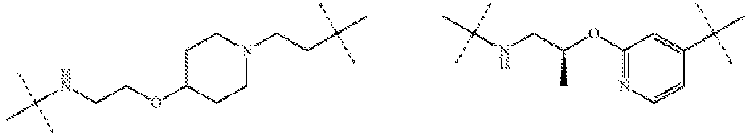

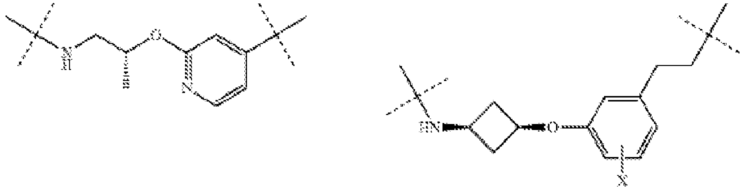

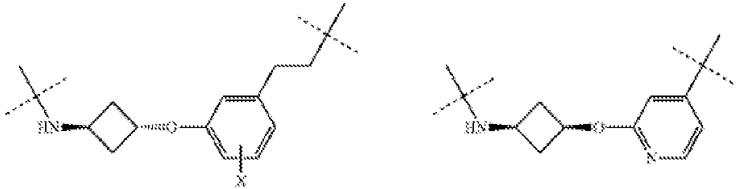

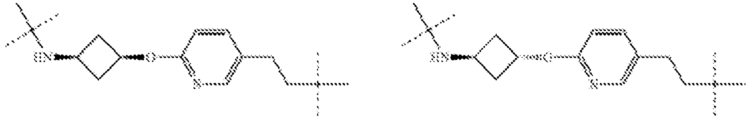

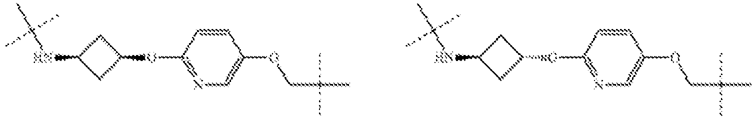

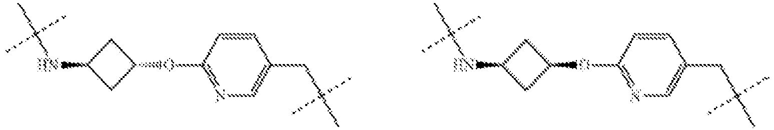

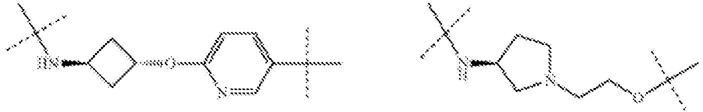

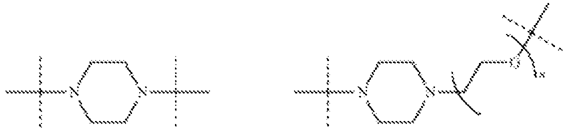

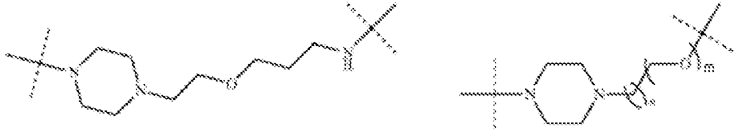

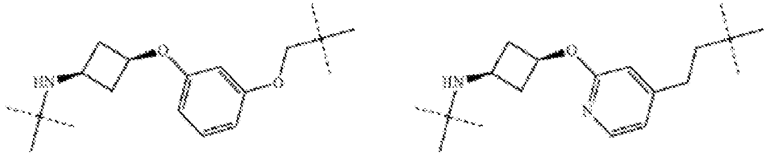

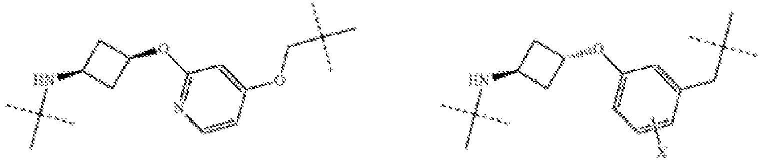

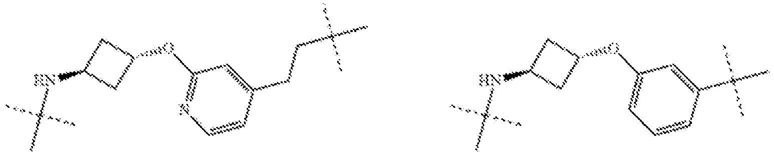

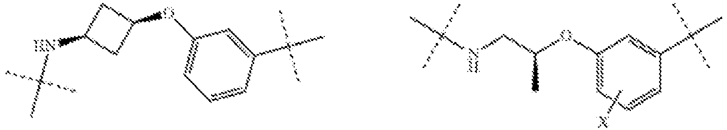

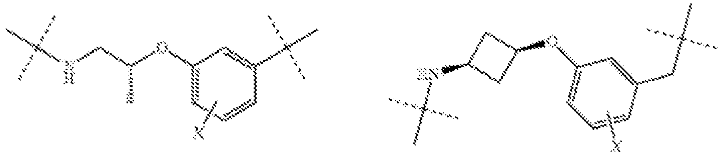

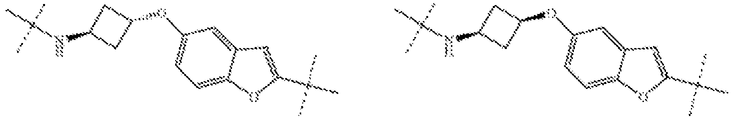

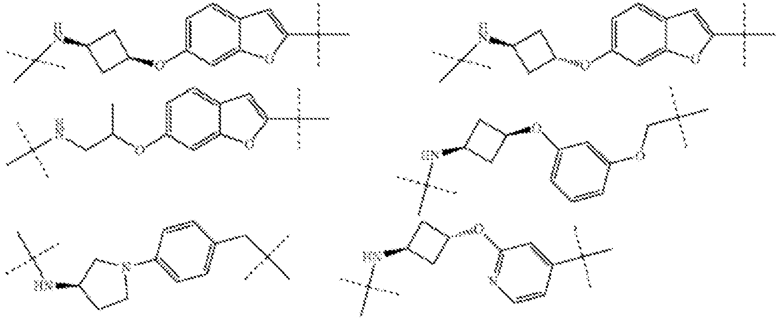

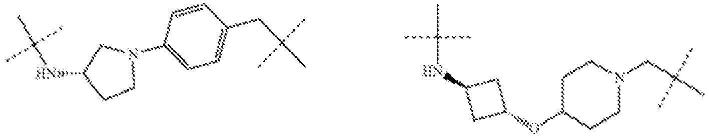

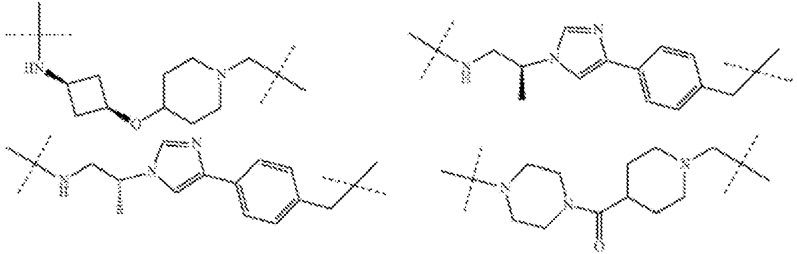

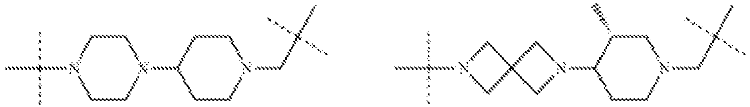

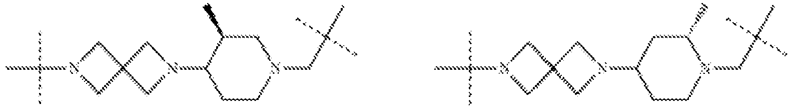

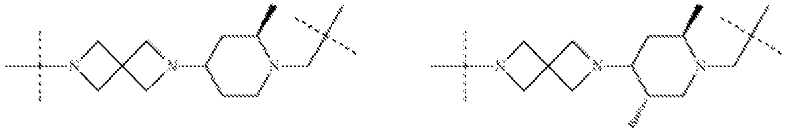

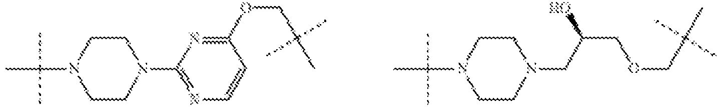

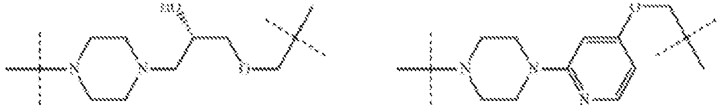

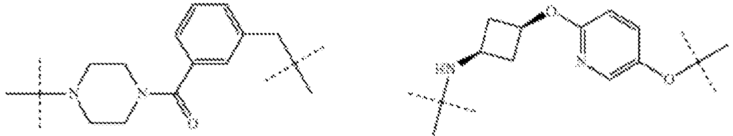

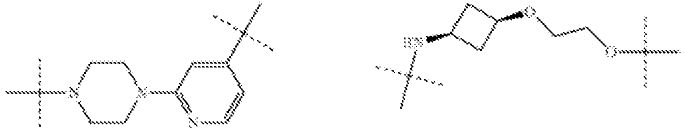

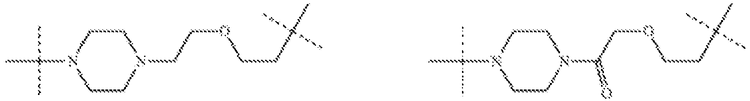

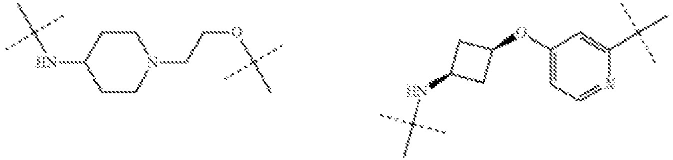

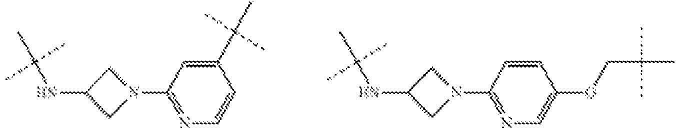

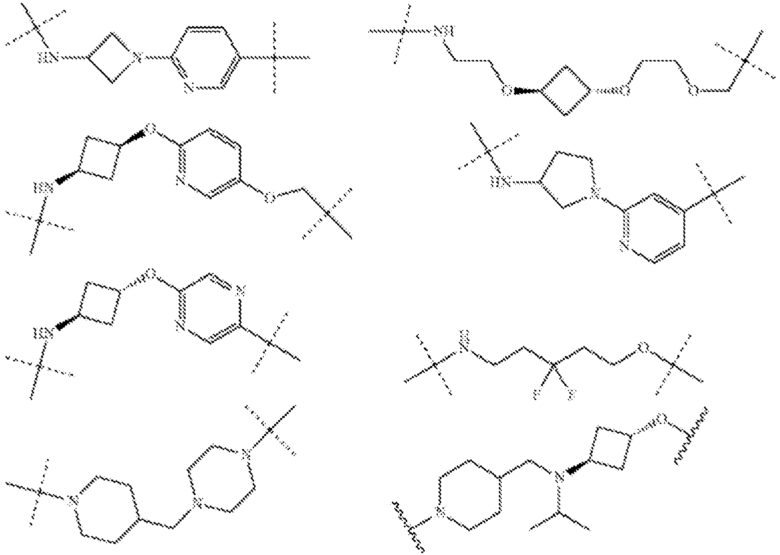

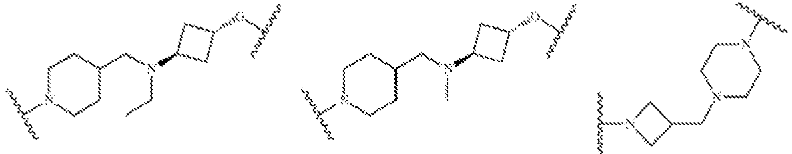

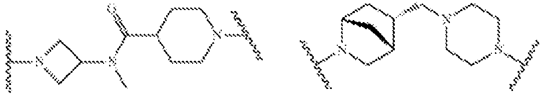

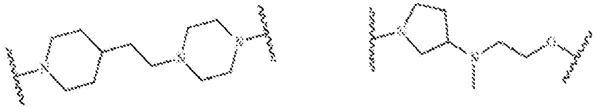

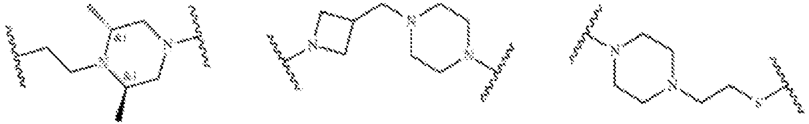

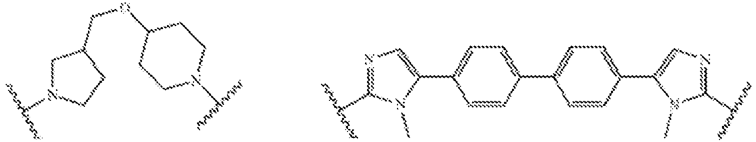

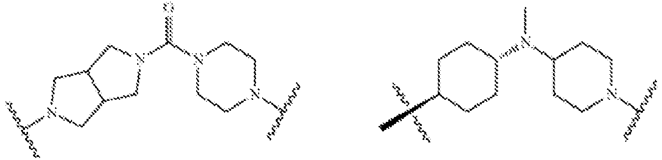

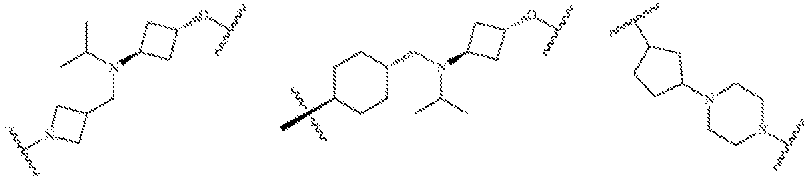

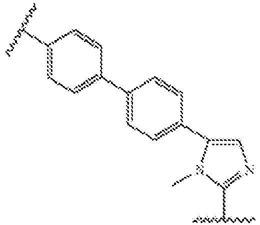

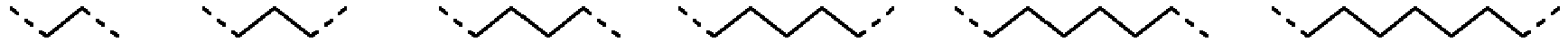

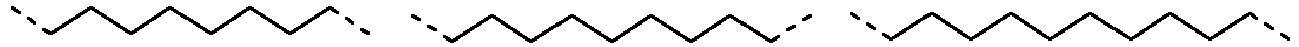

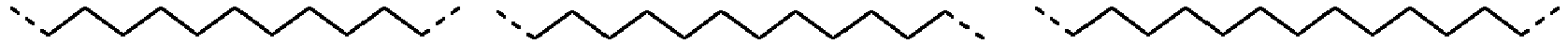

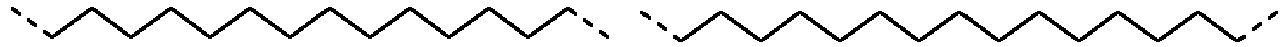

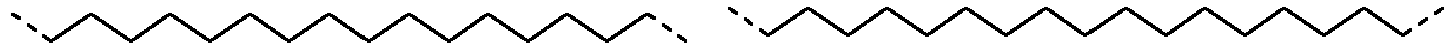

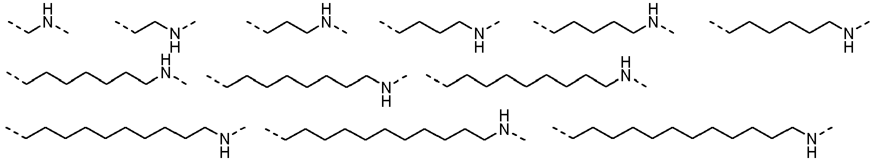

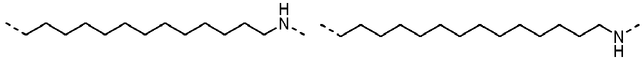

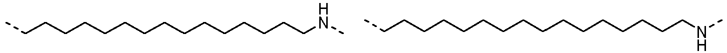

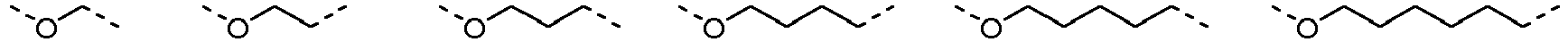

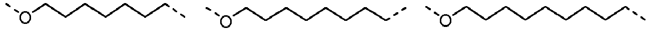

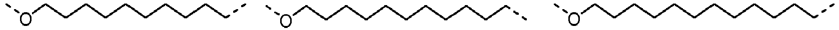

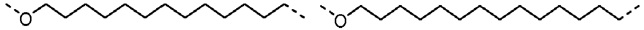

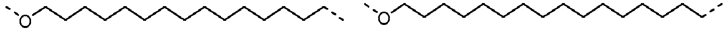

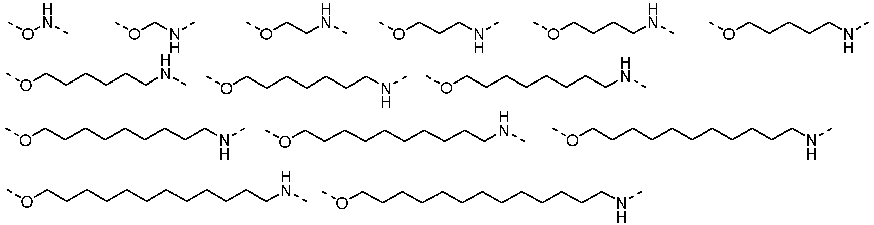

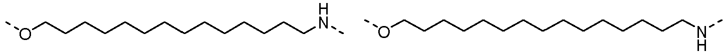

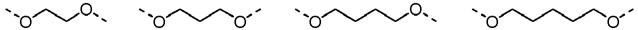

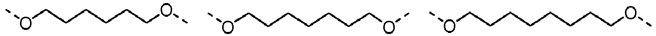

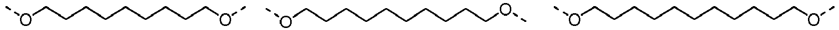

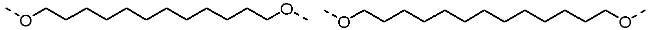

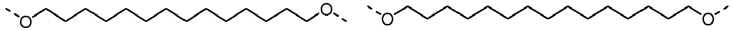

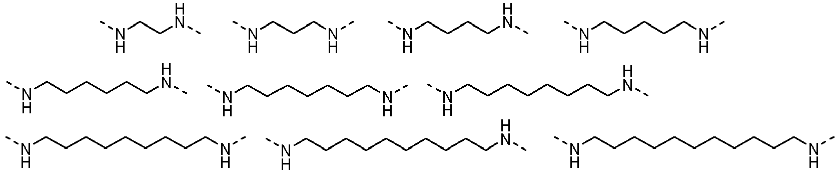

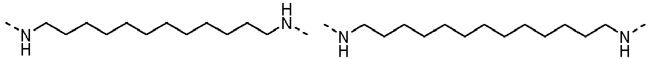

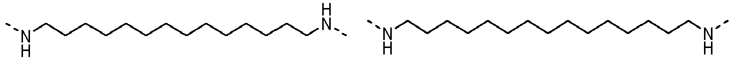

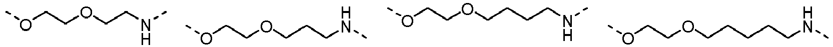

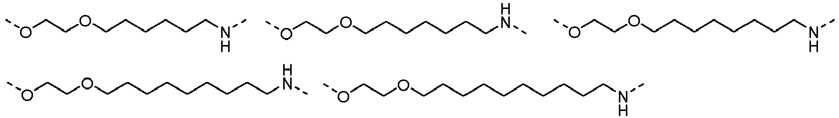

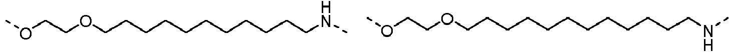

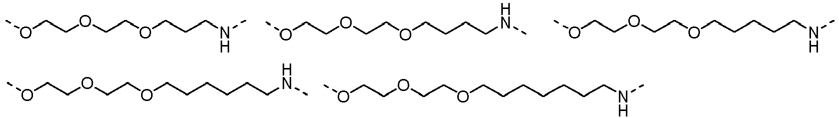

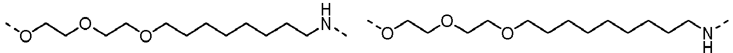

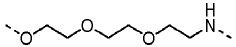

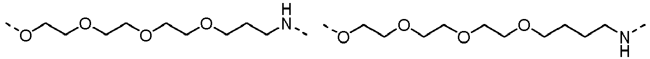

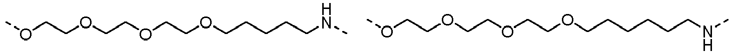

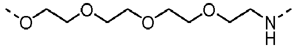

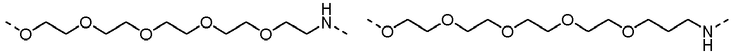

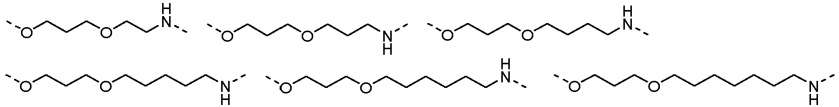

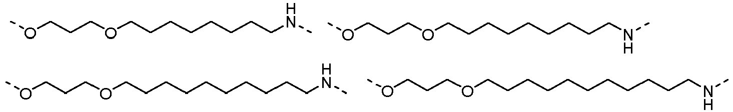

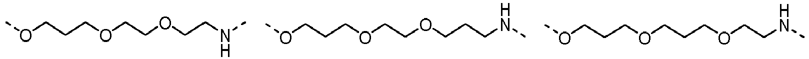

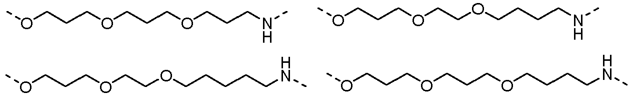

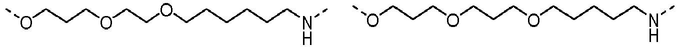

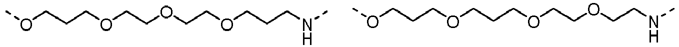

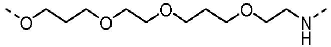

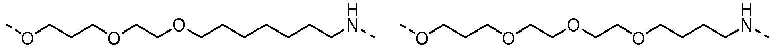

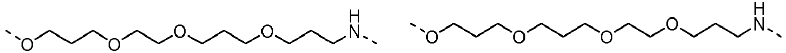

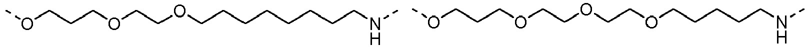

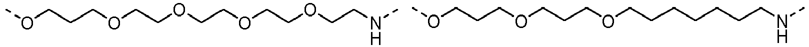

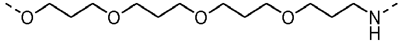

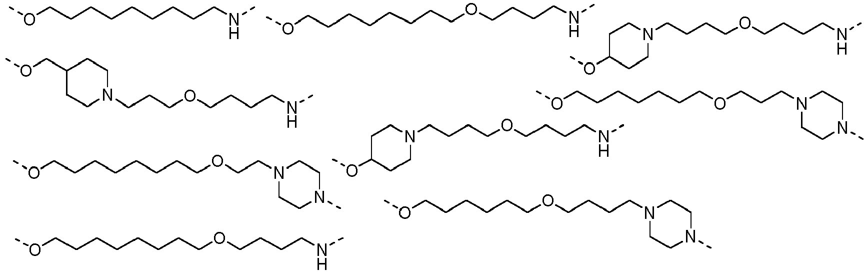

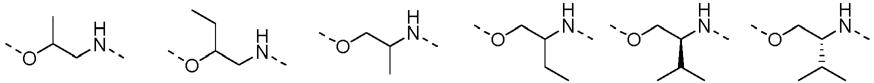

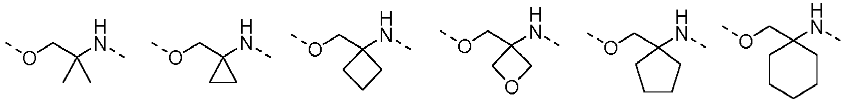

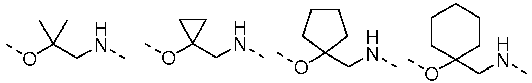

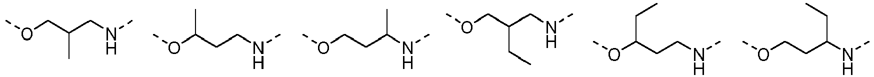

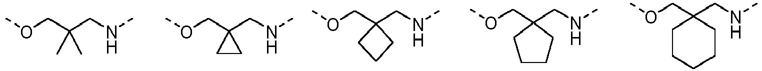

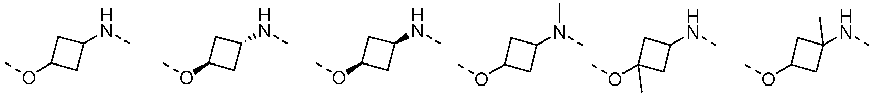

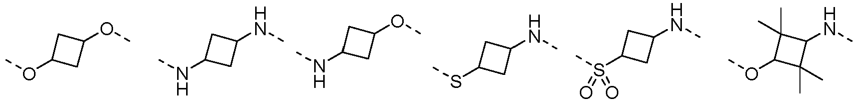

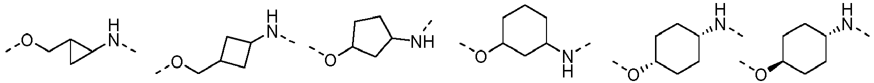

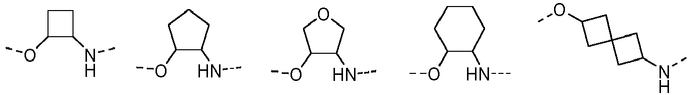

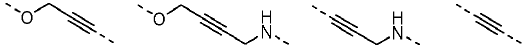

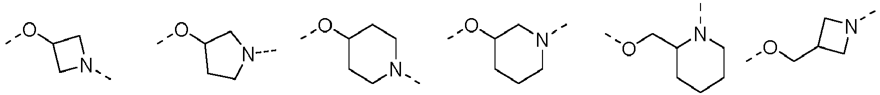

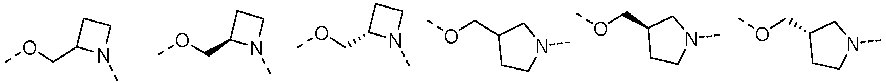

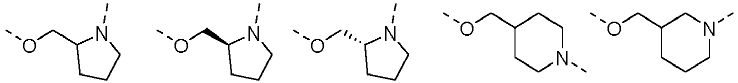

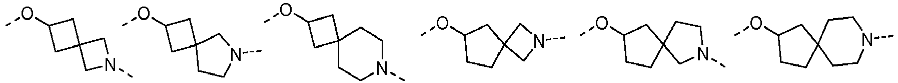

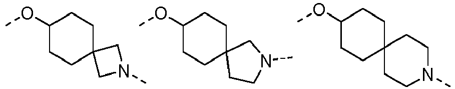

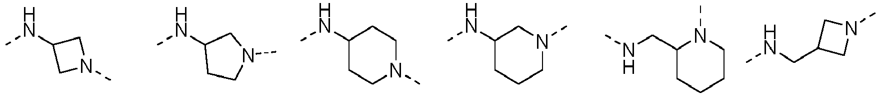

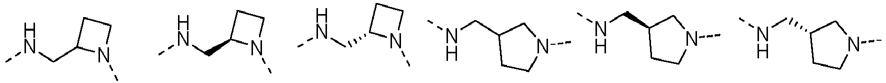

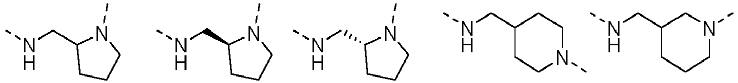

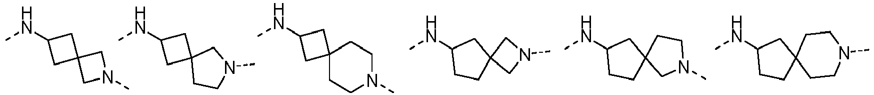

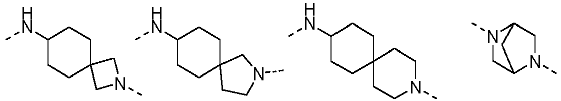

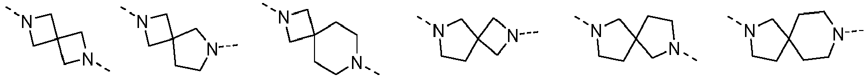

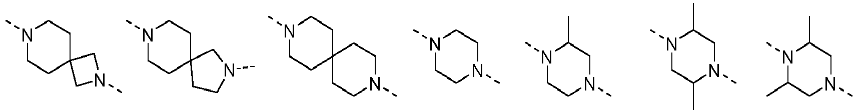

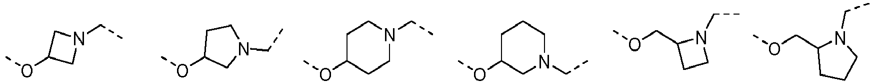

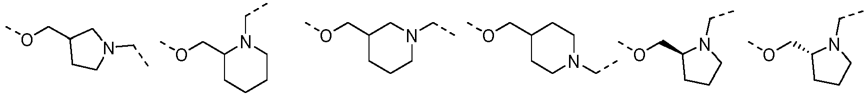

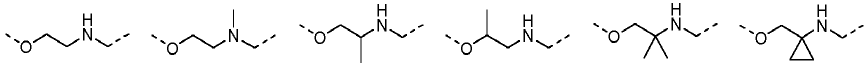

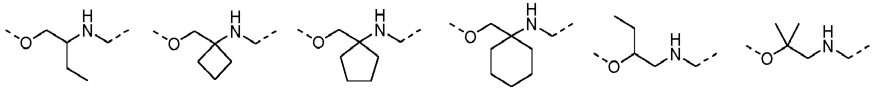

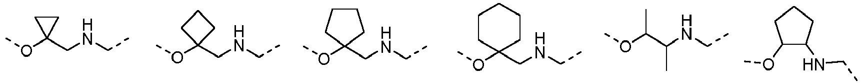

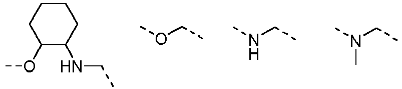

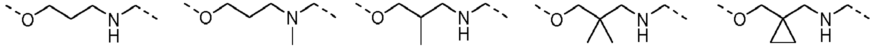

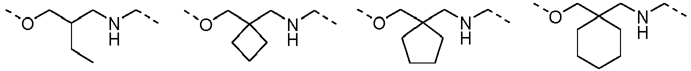

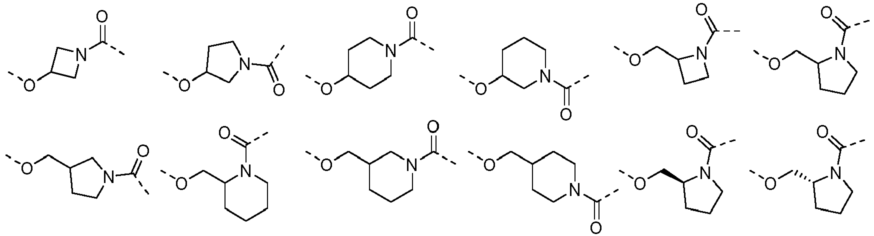

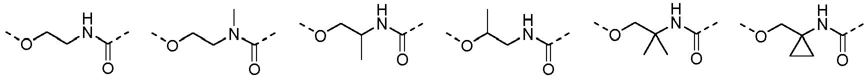

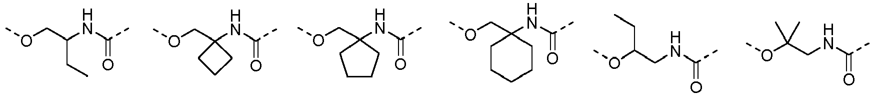

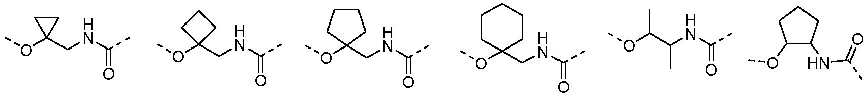

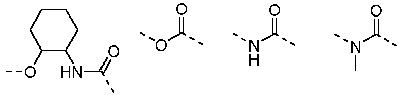

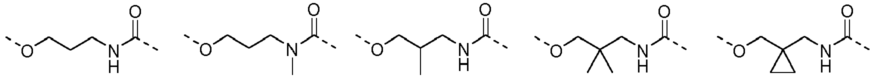

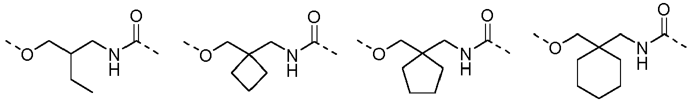

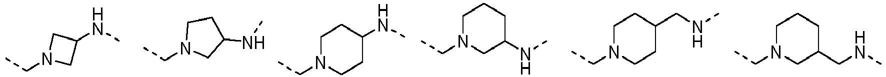

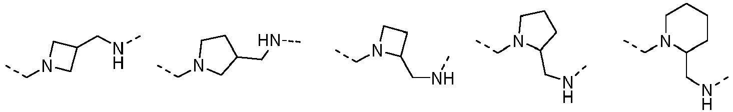

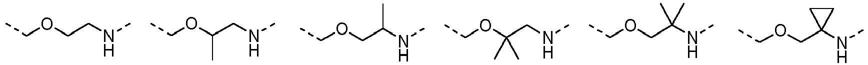

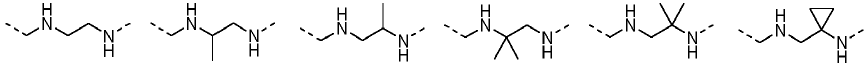

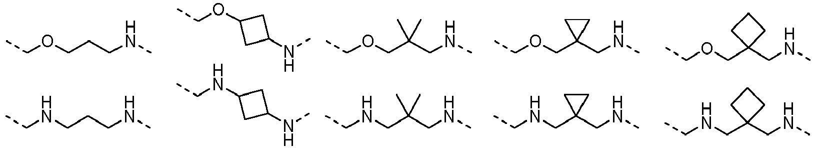

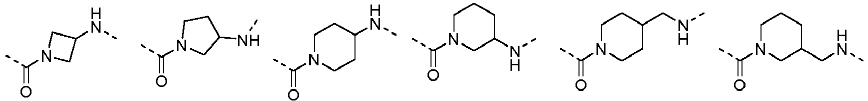

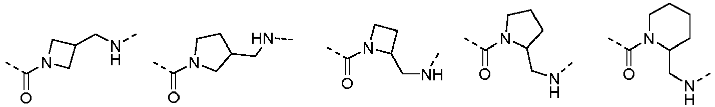

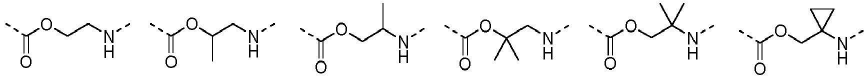

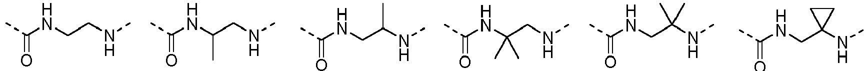

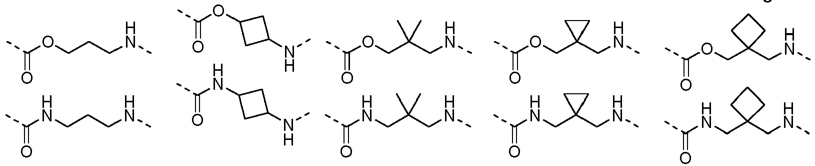

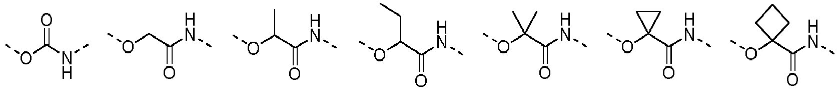

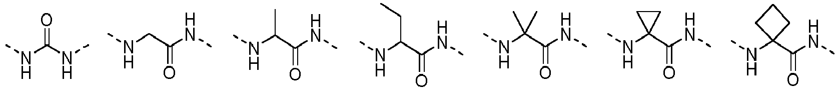

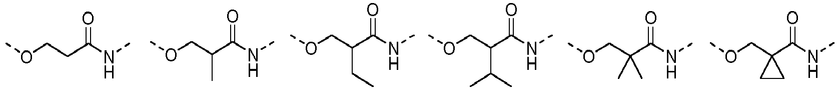

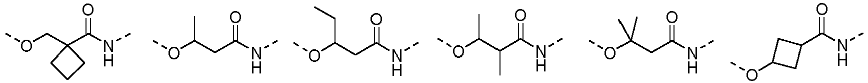

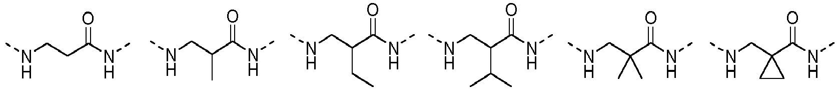

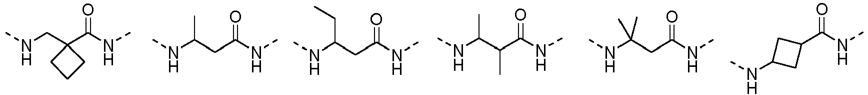

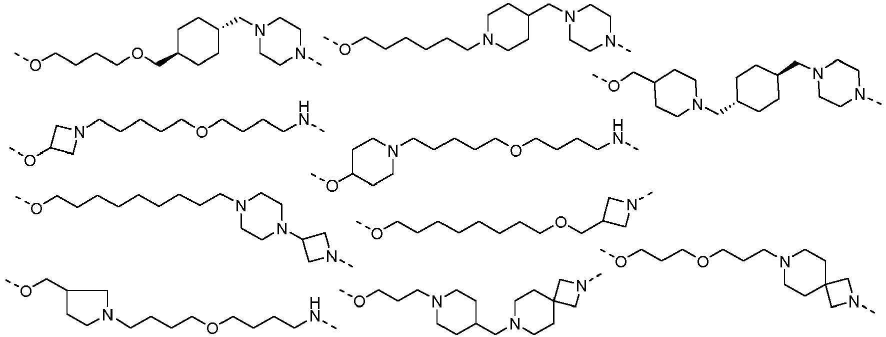

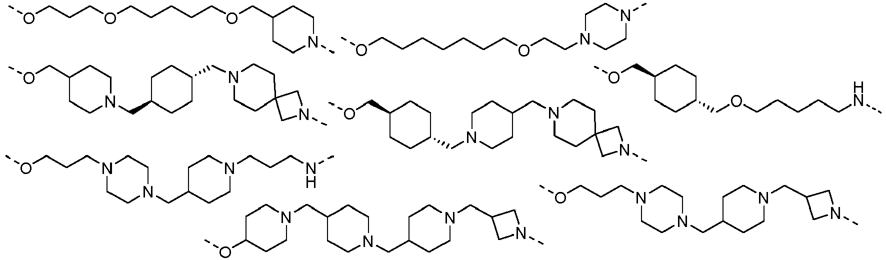

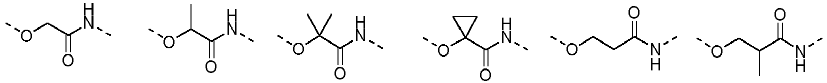

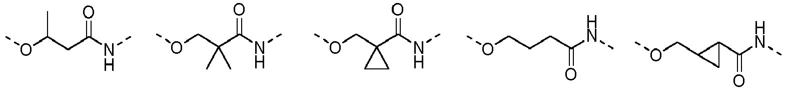

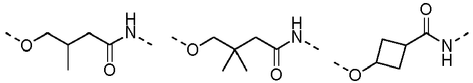

Further, in the above compounds, the structure of L moiety is selected from the group consisting of:

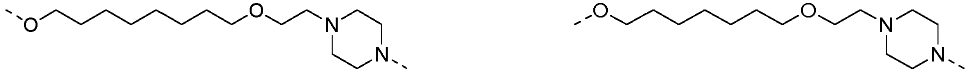

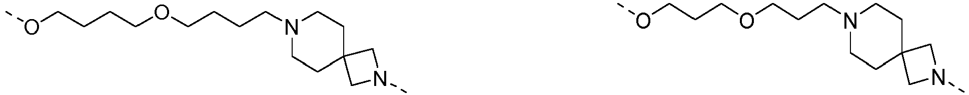

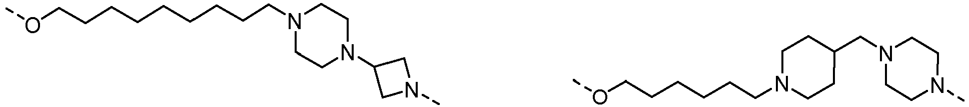

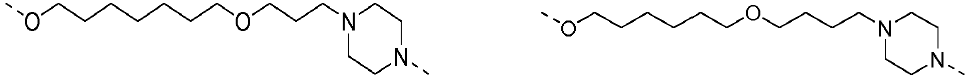

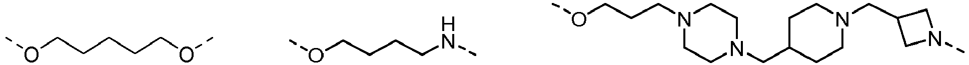

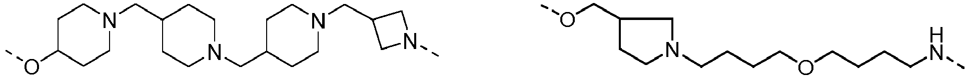

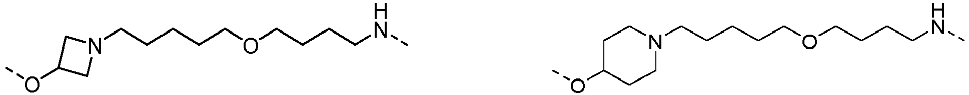

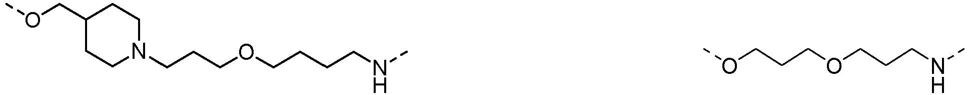

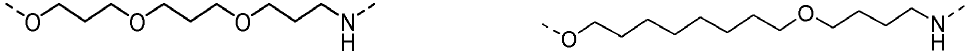

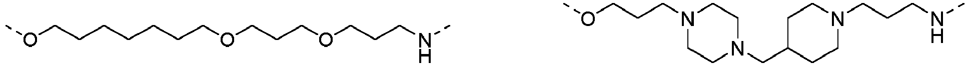

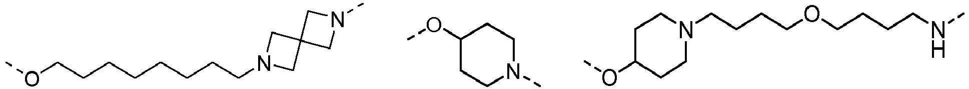

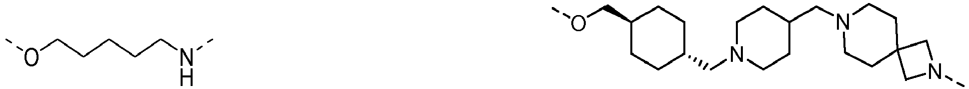

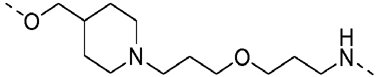

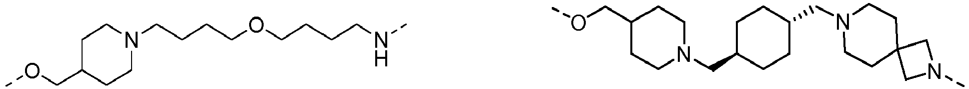

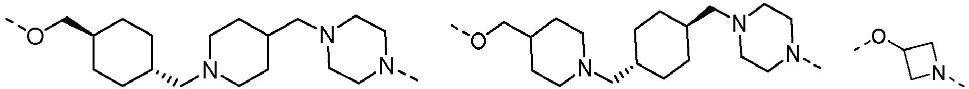

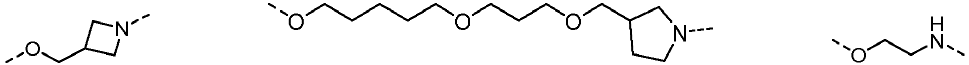

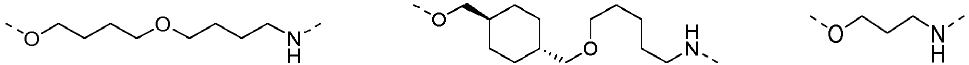

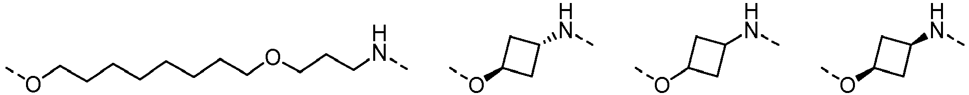

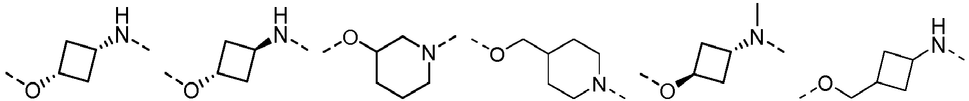

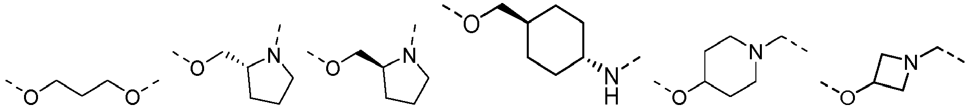

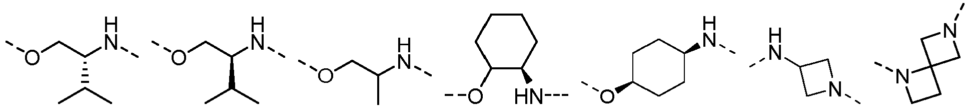

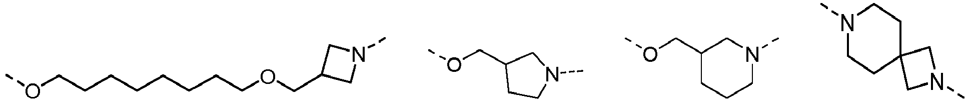

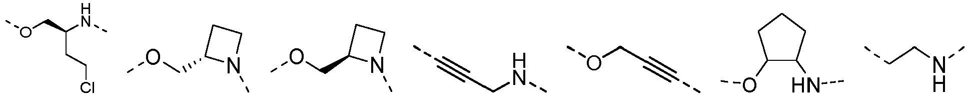

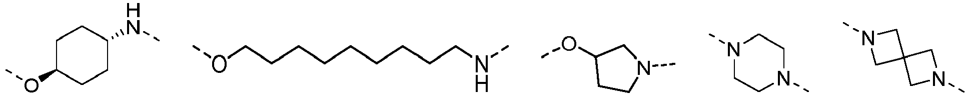

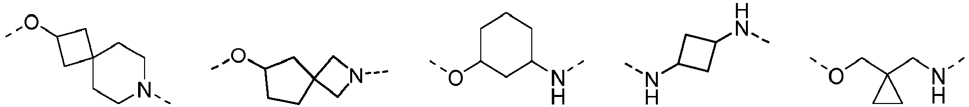

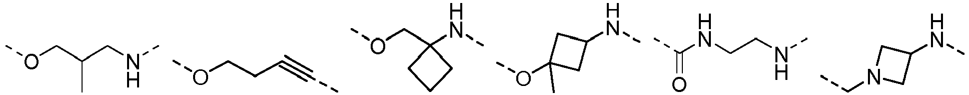

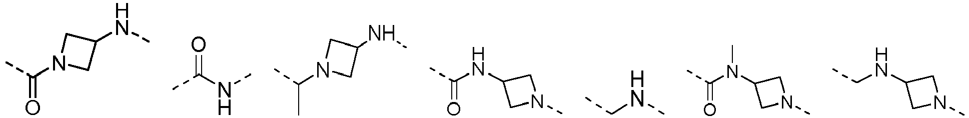

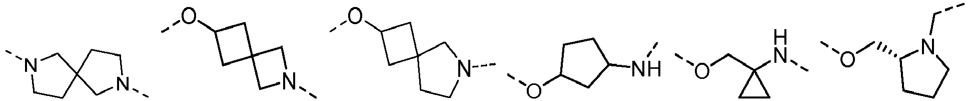

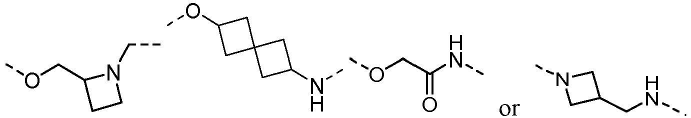

Further, in the above compounds, the structure of L moiety is as represented by formula X-A: 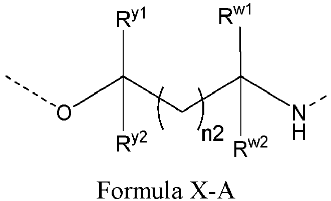
wherein, n2 is 0 or 1;
R^{y1} and R^{y2} are each independently selected from the group consisting of H and C1-C3 alkyl; R^{w1} and R^{w2} are each independently selected from the group consisting of H and halo-substituted or unsubstituted C1-C3 alkyl;
or, R^{y1} and R^{y2} are linked to form 3-4 membered saturated carbon ring; or R^{w1} and R^{w2} are linked to form 3-4 membered saturated carbon ring; or R^{y2} and R^{w2} are linked to form 3-4 membered saturated carbon ring.

More further, R^{y1} and R^{y2} are each independently selected from the group consisting of H and methyl; R^{w1} and R^{w2} are each independently selected from the group consisting of H, methyl, and Cl-substituted or unsubstituted propyl;
or, R^{y1} and R^{y2} are linked to form 3-membered saturated carbon ring; or R^{w1} and R^{w2} are linked to form 3-4 membered saturated carbon ring; or R^{y2} and R^{w2} are linked to form 4 membered saturated carbon ring.

Preferably, the structure of L moiety is selected from the group consisting of: 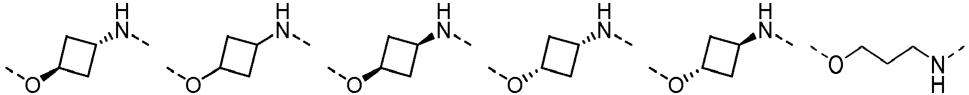 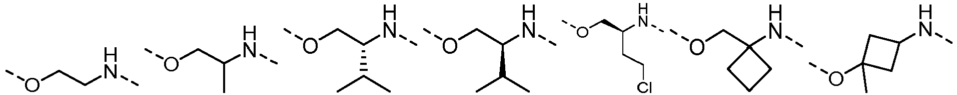 or

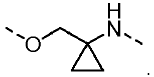

Further, the structure of U moiety is selected from the group consisting of:

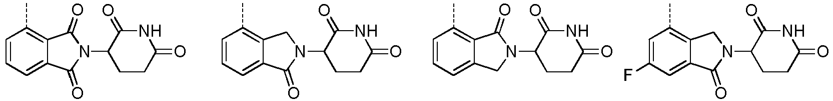

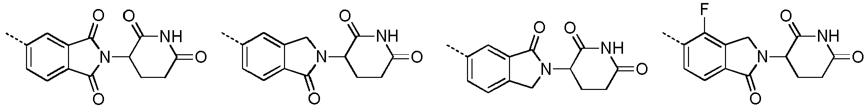

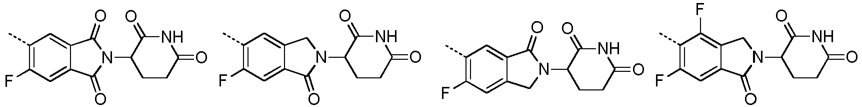

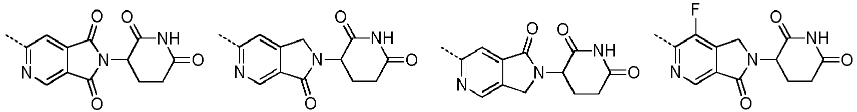

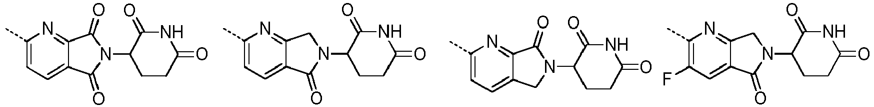

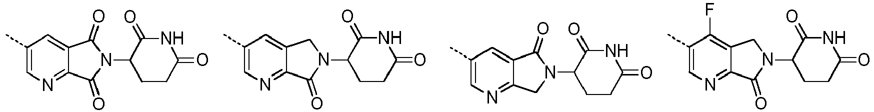

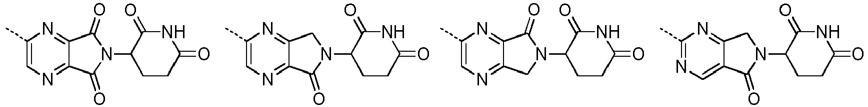

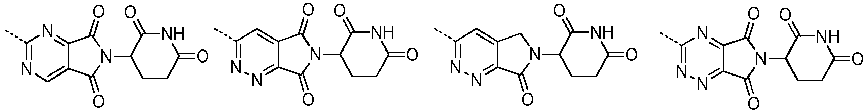

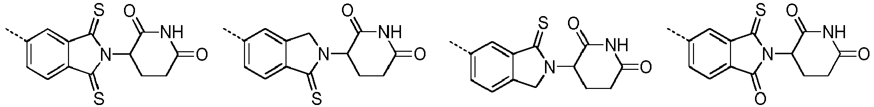

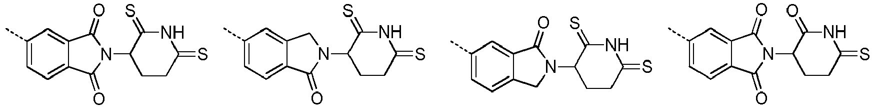

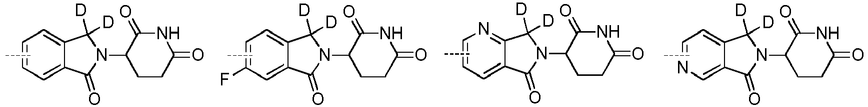

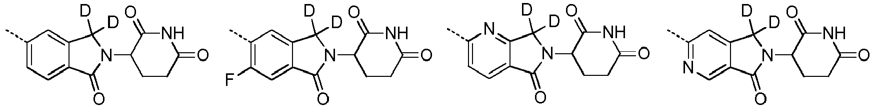

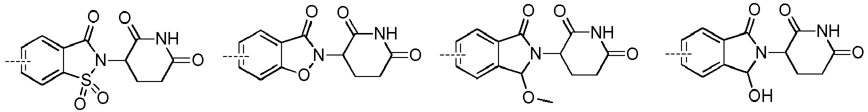

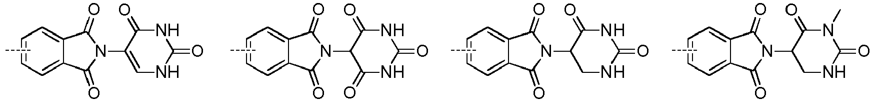

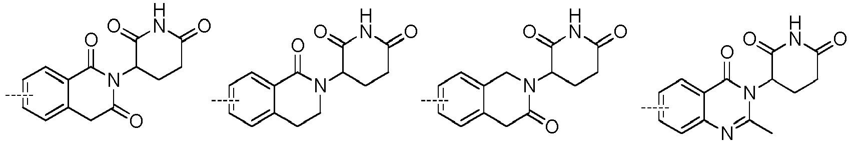

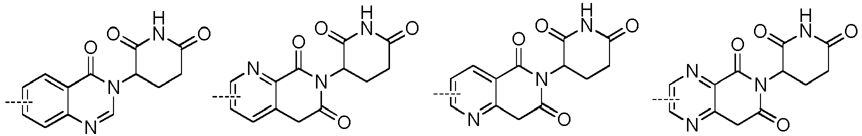

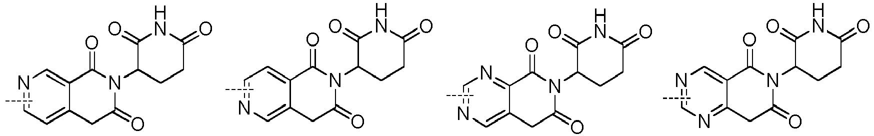

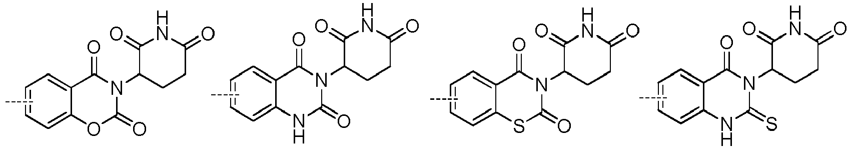

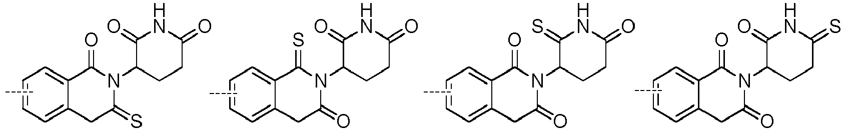

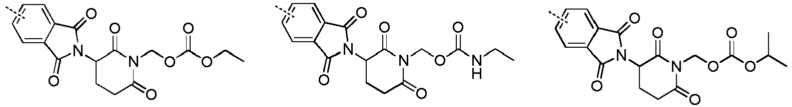

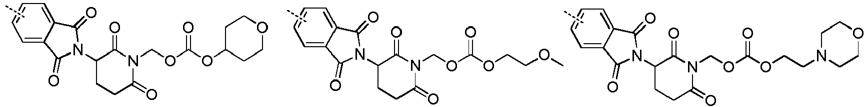

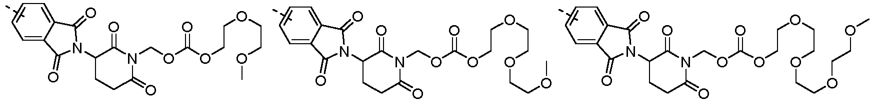

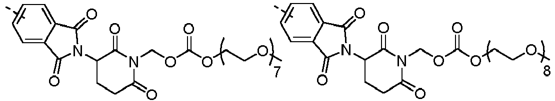

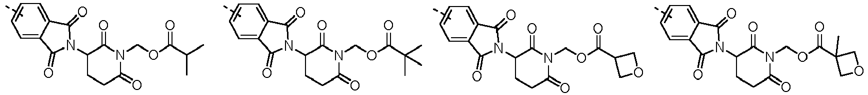

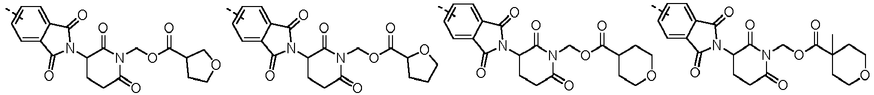

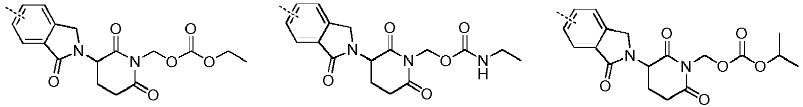

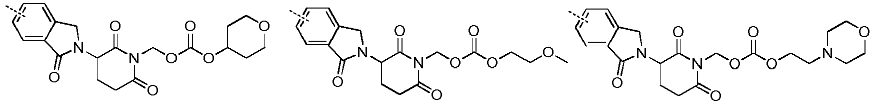

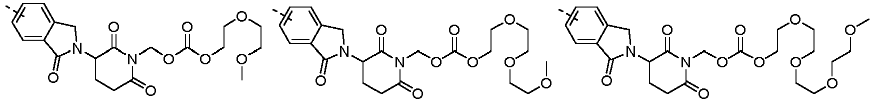

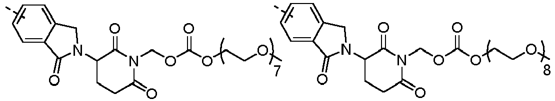

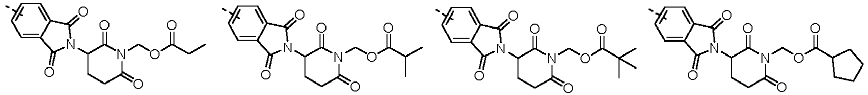

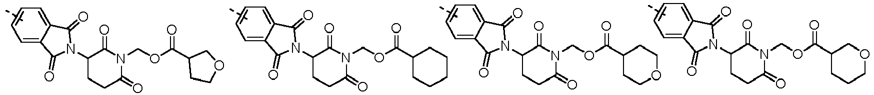

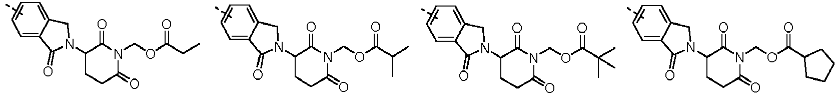

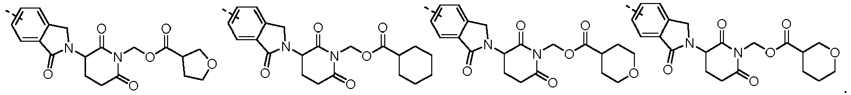

Further, the structure of 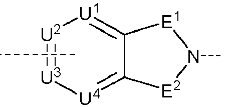 in above formula I-U is selected from the group consisting of:

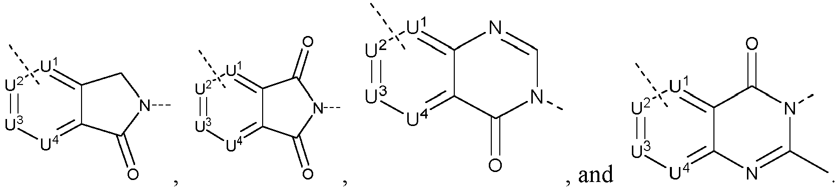

Preferably, U¹, U², U³, and U⁴ are CH; or, one of U¹, U², U³, and U⁴ is N or CX₀, and the others are CH, wherein X₀ is halogen.

Further, the structure of 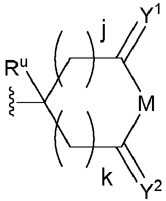 moiety in above formula I-U is as follows:

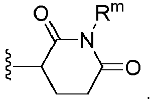

Preferably, R^{m} is selected from the group consisting of H and methyl.

Further, in the above compounds, the structure of U moiety is selected from the group consisting of:

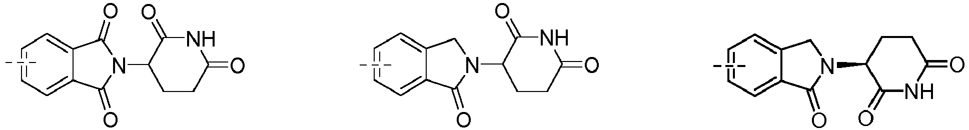

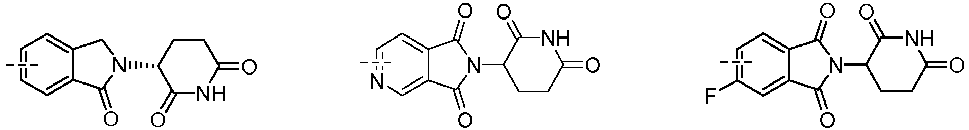

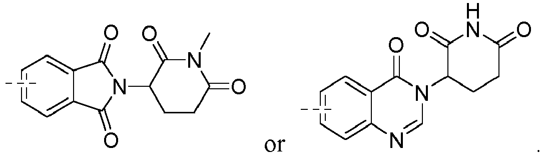

Further, in the above compounds, the compound is selected from the group consisting of:

| | | | |
|---|---|---|---|
| 1 | 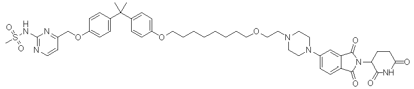 | 2 | 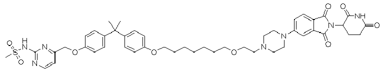 |
| 3 | 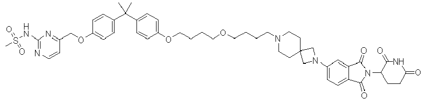 | 4 | 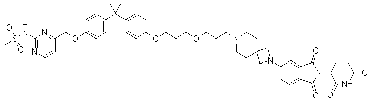 |
| 5 | 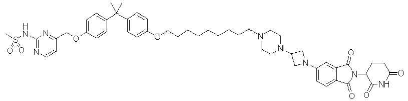 | 6 | 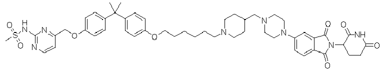 |
| 7 | 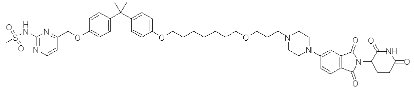 | 8 | 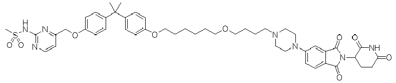 |
| 9 | 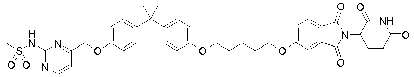 | 10 | 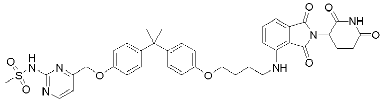 |
| 11 | 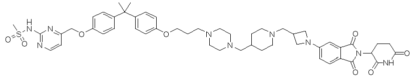 | 12 | 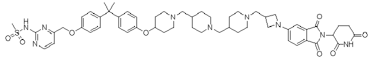 |
| 13 | 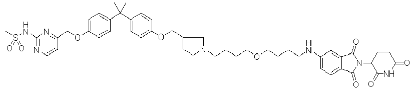 | 14 | 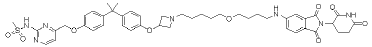 |
| 15 | 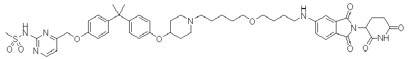 | 16 | 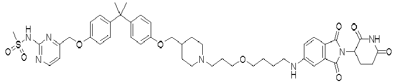 |
| 17 | 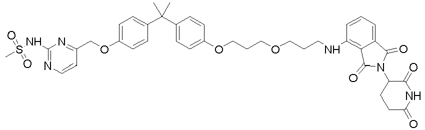 | 18 | 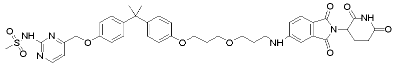 |
| 19 | 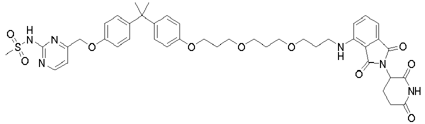 | 20 | 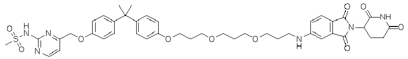 |
| 21 | 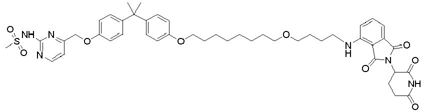 | 22 | 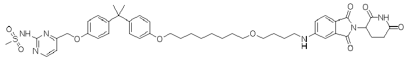 |
| 23 | 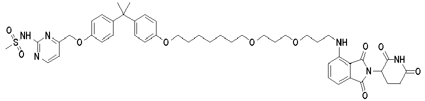 | 24 | 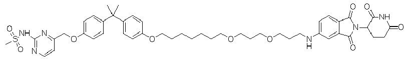 |
| 25 | 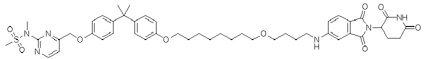 | 26 | 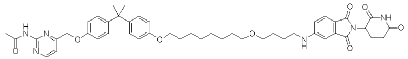 |
| 27 | 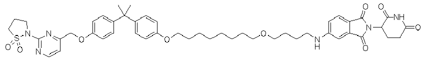 | 28 | 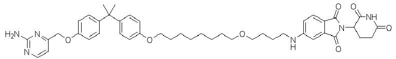 |
| 29 | 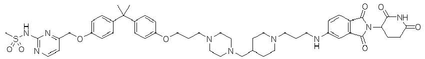 | 30 | 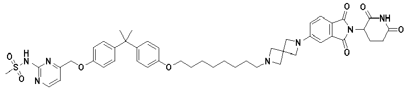 |
| 31 | 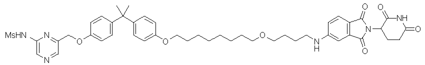 | 32 | 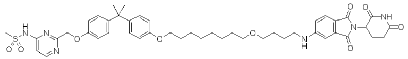 |
| 33 | 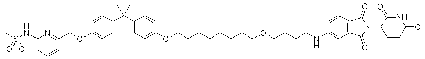 | 34 | 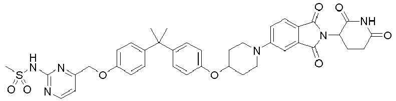 |
| 35 | 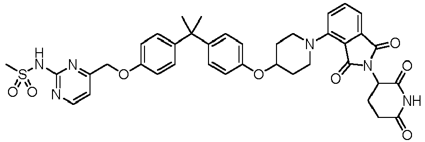 | 36 | 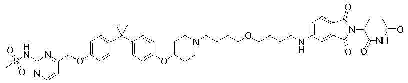 |
| 37 | 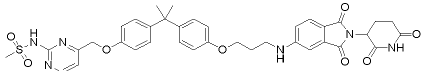 | 38 | 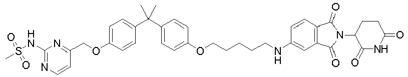 |
| 39 | 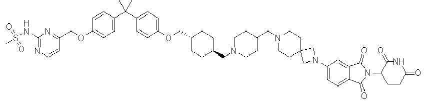 | 40 | 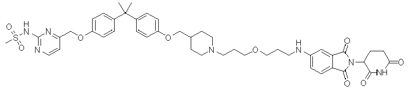 |
| 41 | 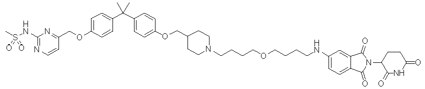 | 42 | 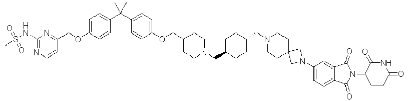 |
| 43 | 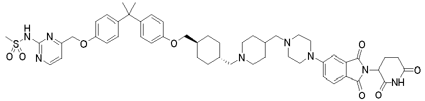 | 44 | 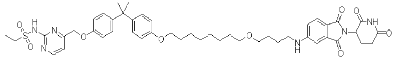 |
| 45 | 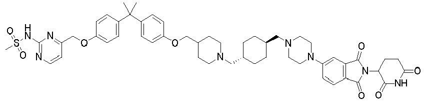 | 46 | 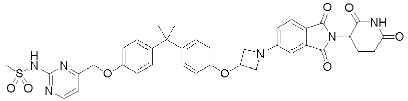 |
| 47 | 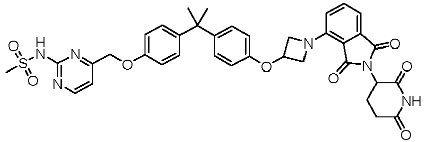 | 48 | 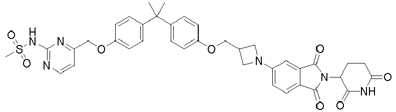 |
| 49 | 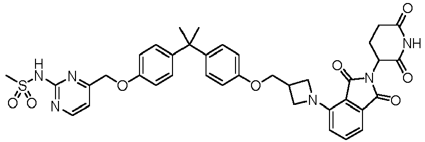 | 50 | 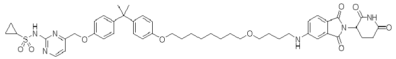 |
| 51 | 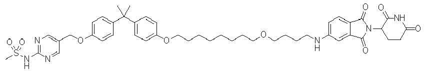 | 52 | 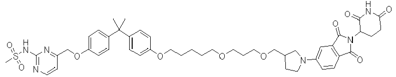 |
| 53 | 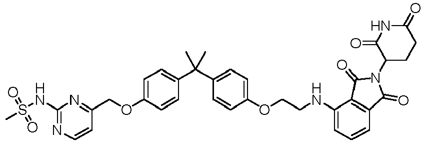 | 54 | 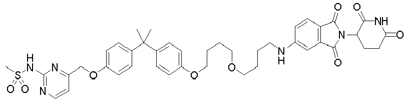 |
| 55 | 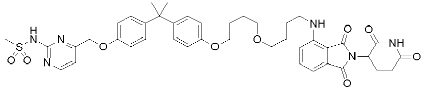 | 56 | 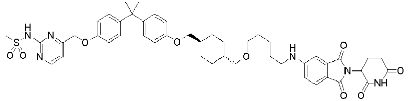 |
| 57 | 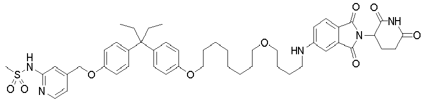 | 58 | 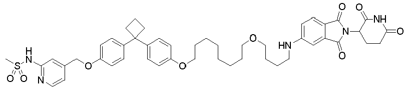 |
| 59 | 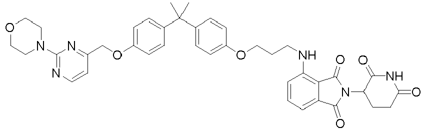 | 60 | 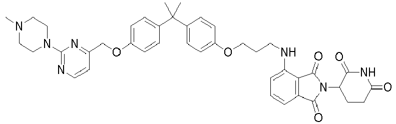 |
| 61 | 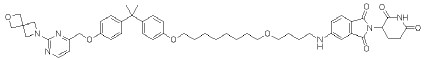 | 62 | 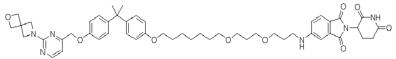 |
| 63 | 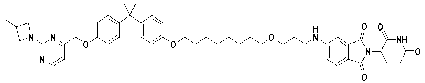 | 64 | 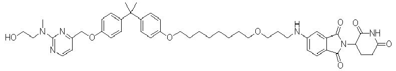 |
| 65 | 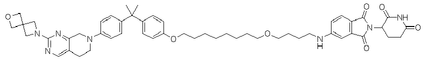 | 66 | 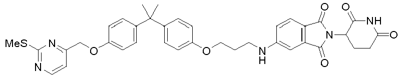 |
| 67 | 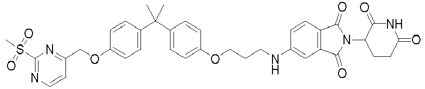 | 68 | 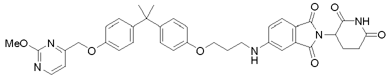 |
| 69 | 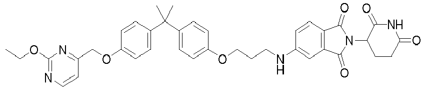 | 70 | 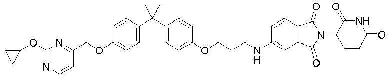 |
| 71 | 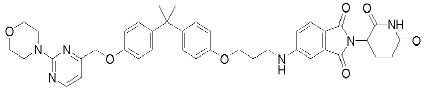 | 72 | 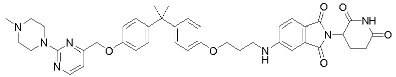 |
| 73 | 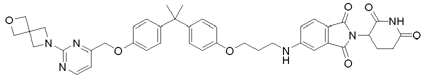 | 74 | 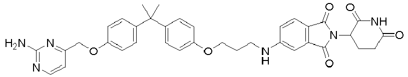 |
| 75 | 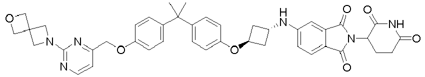 | 76 | 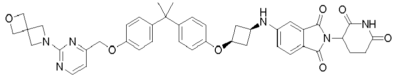 |
| 77 | 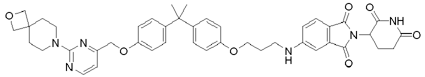 | 78 | 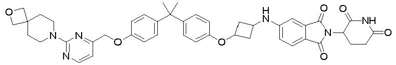 |
| 79 | 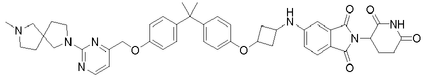 | 80 | 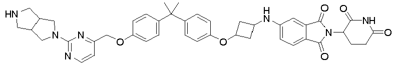 |
| 81 | 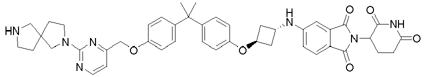 | 82 | 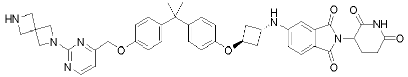 |
| 83 | 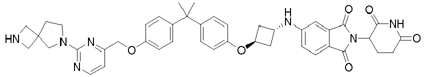 | 84 | 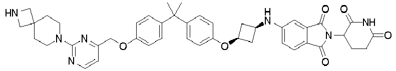 |
| 85 | 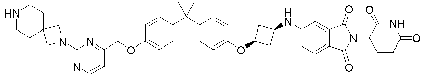 | 86 | 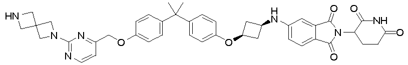 |
| 87 | 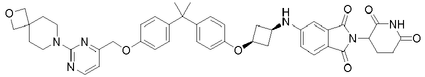 | 88 | 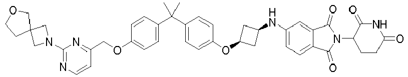 |
| 89 | 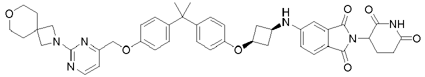 | 90 | 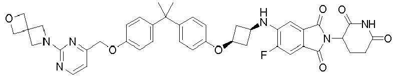 |
| 91 | 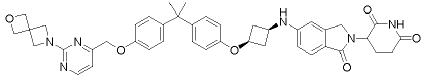 | 92 | 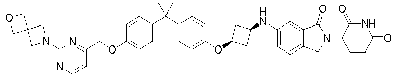 |
| 93 | 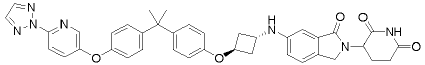 | 94 | 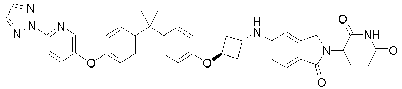 |
| 95 | 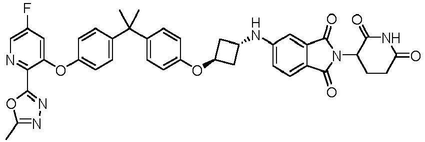 | 96 | 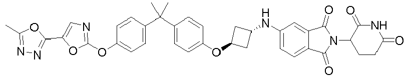 |
| 97 | 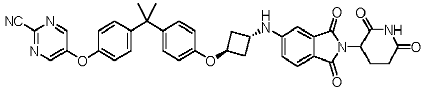 | 98 | 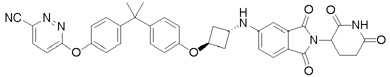 |
| 99 | 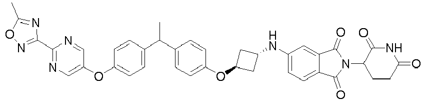 | 100 | 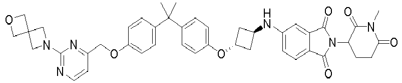 |
| 101 | 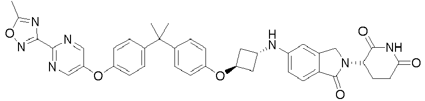 | 102 | 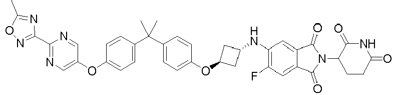 |
| 103 | 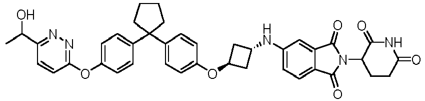 | 104 | 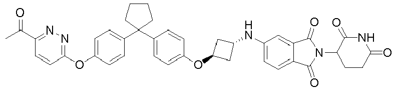 |
| 105 | 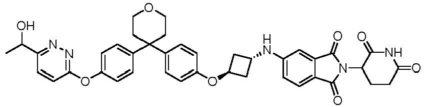 | 106 | 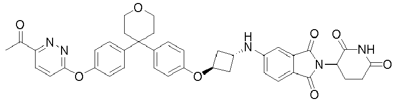 |
| 107 | 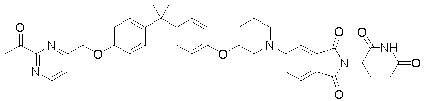 | 108 | 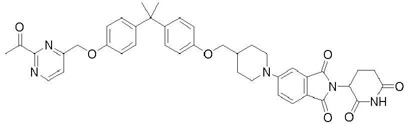 |
| 109 | 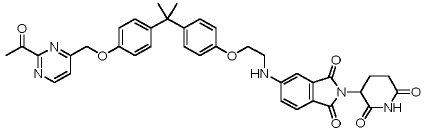 | 110 | 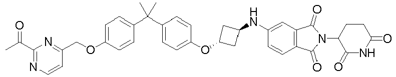 |
| 111 | 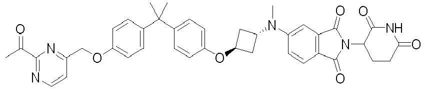 | 112 | 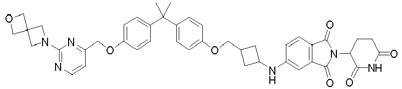 |
| 113 | 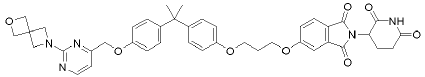 | 114 | 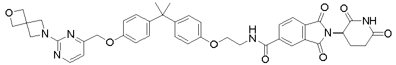 |
| 115 | 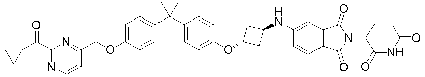 | 116 | 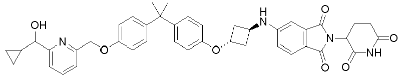 |
| 117 | 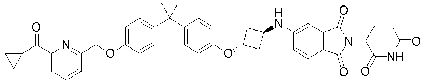 | 118 | 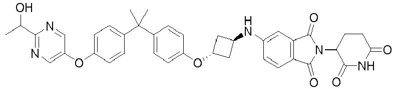 |
| 119 | 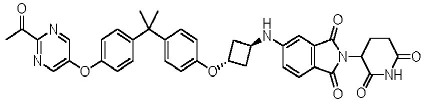 | 120 | 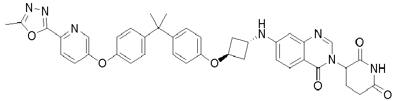 |
| | | 122 | 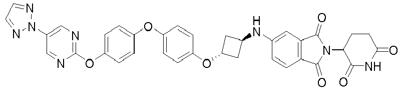 |
| 123 | 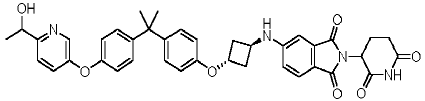 | 124 | 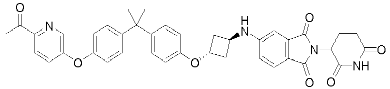 |
| 125 | 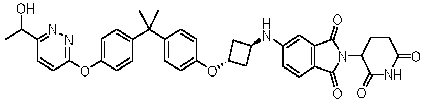 | 126 | 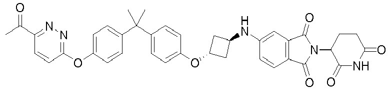 |
| 127 | 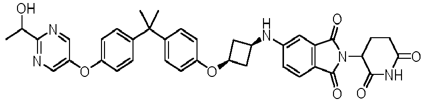 | 128 | 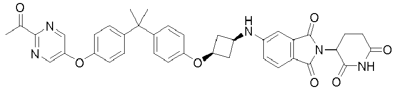 |
| 129 | 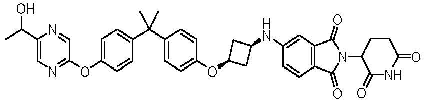 | 130 | 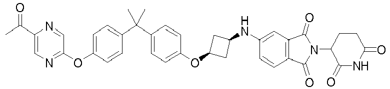 |
| 131 | 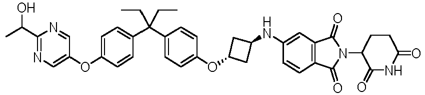 | 132 | 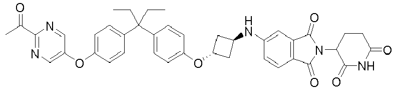 |
| 133 | 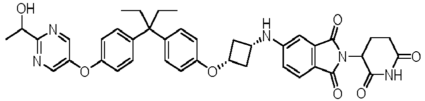 | 134 | 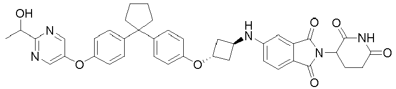 |
| 135 | 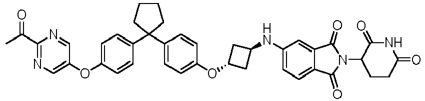 | 136 | 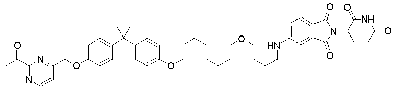 |
| 137 | 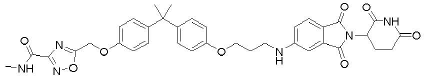 | 138 | 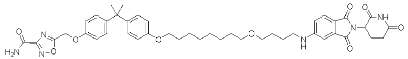 |
| 139 | 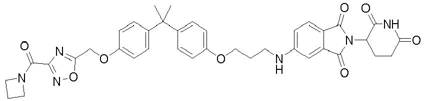 | 140 | 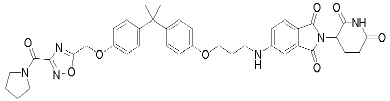 |
| 141 | 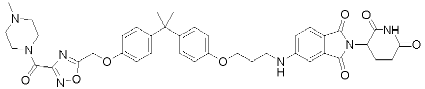 | 142 | 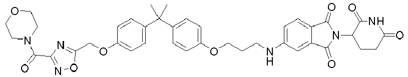 |
| 143 | 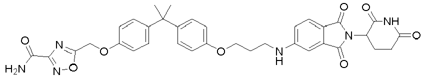 | 144 | 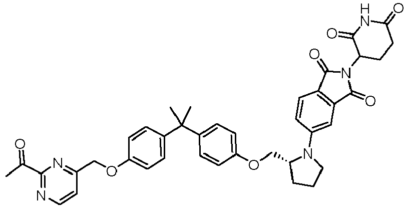 |
| 145 | 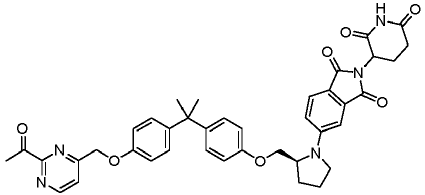 | 146 | 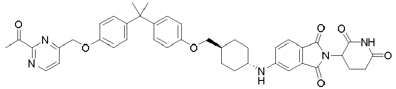 |
| 147 | 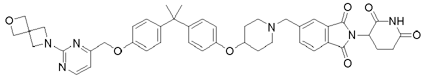 | 148 | 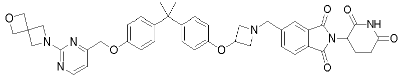 |
| 149 | 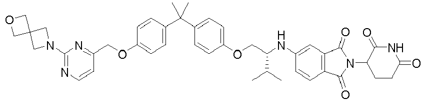 | 150 | 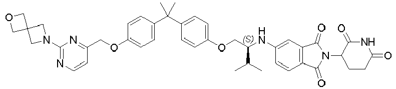 |
| 151 | 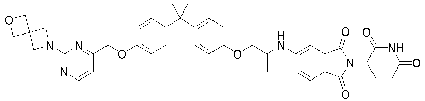 | 152 | 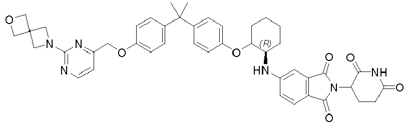 |
| 153 | 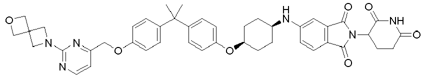 | 154 | 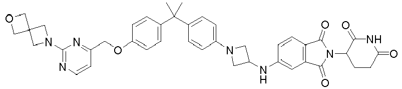 |
| 155 | 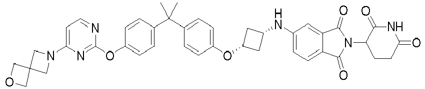 | 156 | 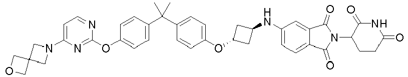 |
| 157 | 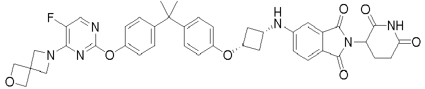 | 158 | 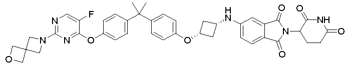 |
| 159 | 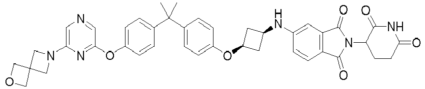 | 160 | 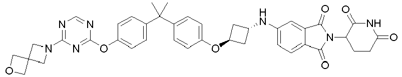 |
| 161 | 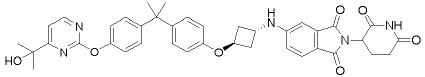 | 162 | 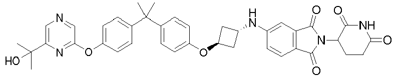 |
| 163 | 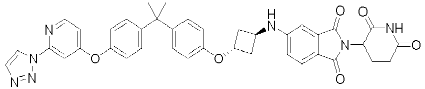 | 164 | 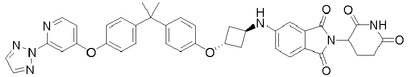 |
| 165 | 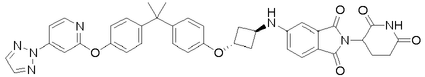 | 166 | 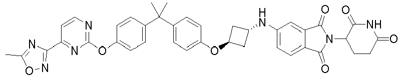 |
| 167 | 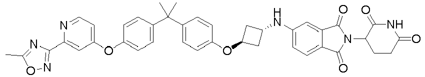 | 168 | 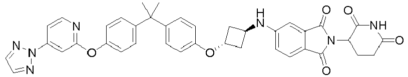 |
| 169 | 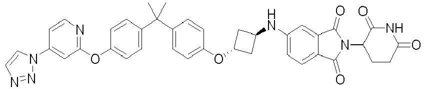 | 170 | 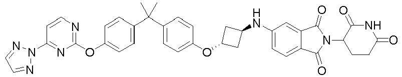 |
| 171 | 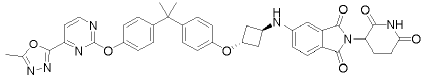 | 172 | 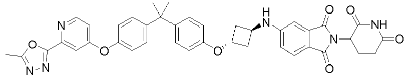 |
| 173 | 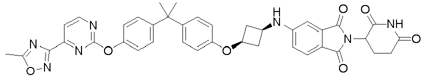 | 174 | 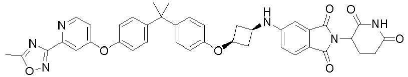 |
| 175 | 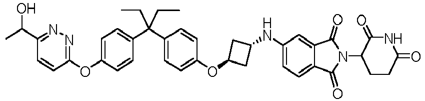 | 176 | 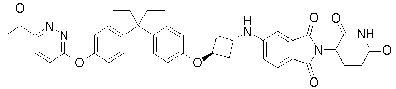 |
| 177 | 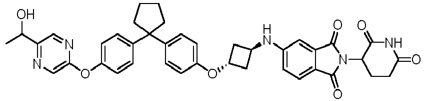 | 178 | 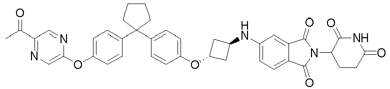 |
| 179 | 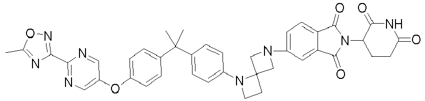 | 180 | 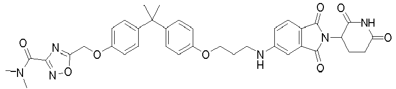 |
| 181 | 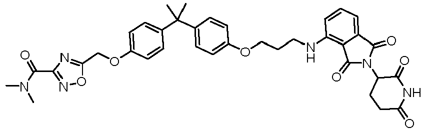 | 182 | 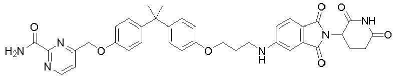 |
| 183 | 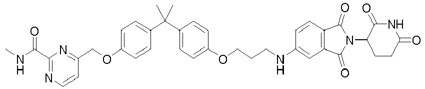 | 184 | 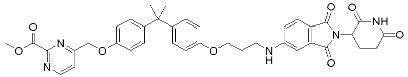 |
| 185 | 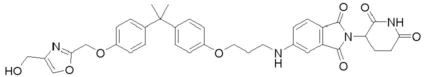 | 186 | 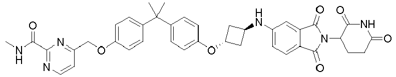 |
| 187 | 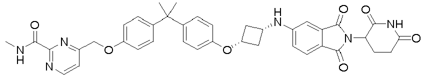 | 188 | 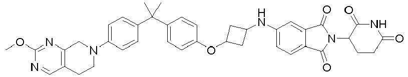 |
| 189 | 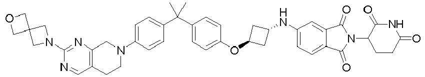 | 190 | 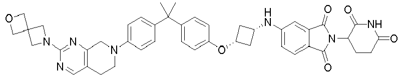 |
| 191 | 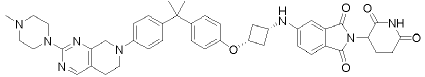 | 192 | 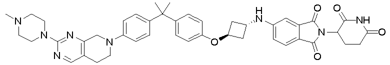 |
| 193 | 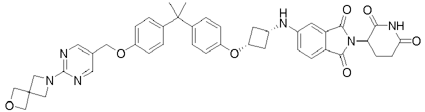 | 194 | 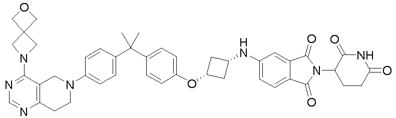 |
| 195 | 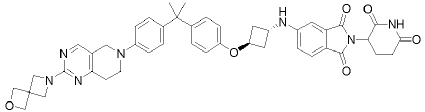 | 196 | 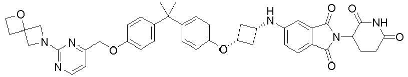 |
| 197 | 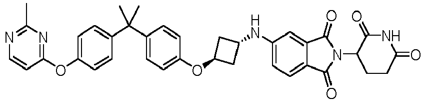 | 198 | 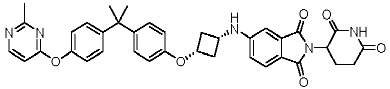 |
| 199 | 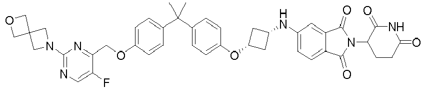 | 200 | 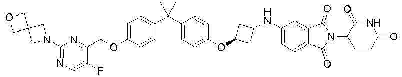 |
| 201 | 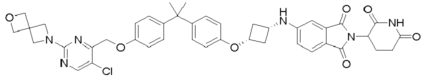 | 202 | 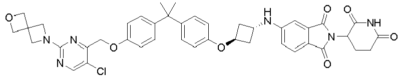 |
| 203 | 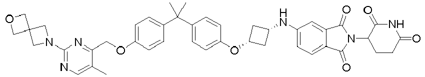 | 204 | 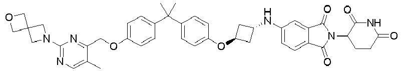 |
| 205 | 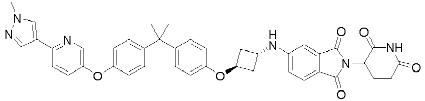 | 206 | 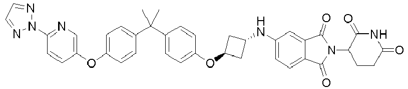 |
| 207 | 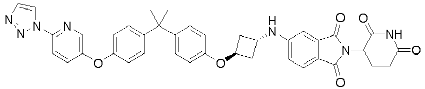 | 208 | 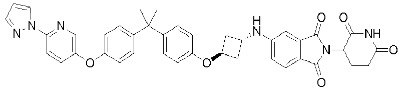 |
| 209 | 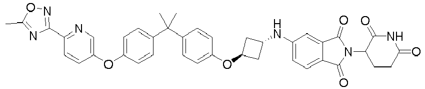 | 210 | 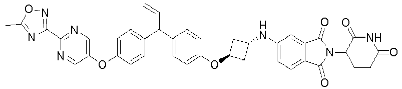 |
| 211 | 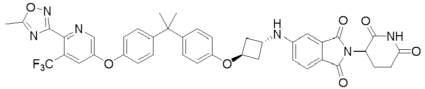 | 212 | 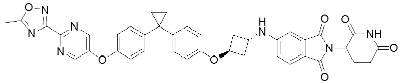 |
| 213 | 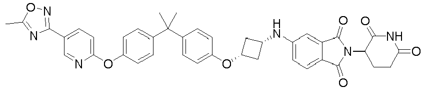 | 214 | 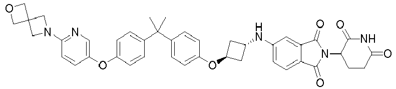 |
| 215 | 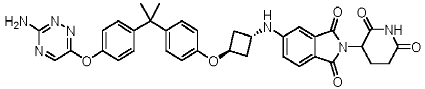 | 216 | 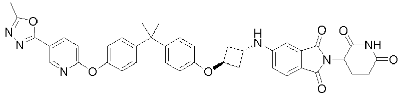 |
| 217 | 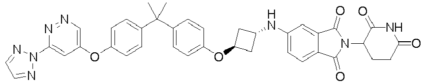 | 218 | 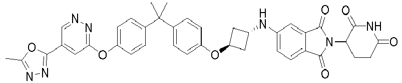 |
| 219 | 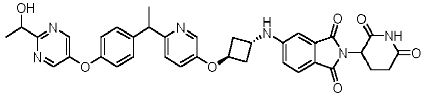 | 220 | 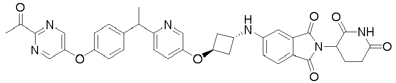 |
| 221 | 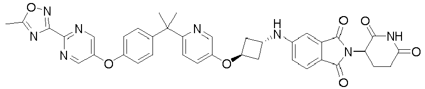 | 222 | 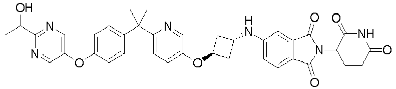 |
| 223 | 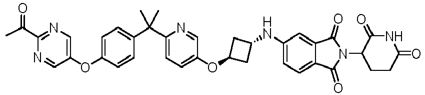 | 224 | 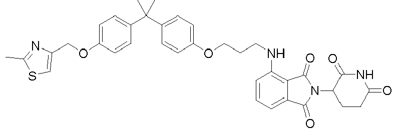 |
| 225 | 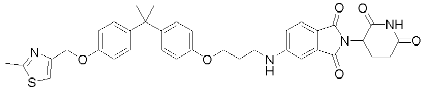 | 226 | 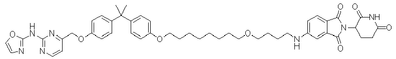 |
| 227 | 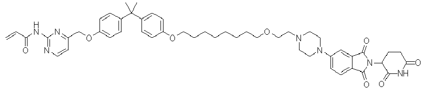 | 228 | 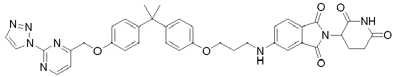 |
| 229 | 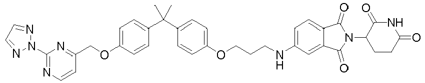 | 230 | 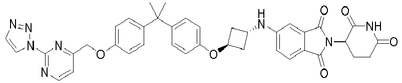 |
| 231 | 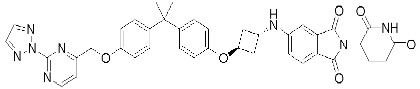 | 232 | 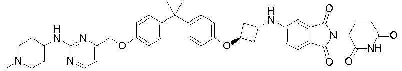 |
| 233 | 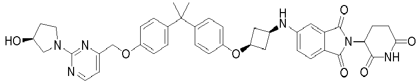 | 234 | 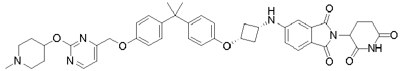 |
| 235 | 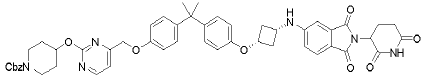 | 236 | 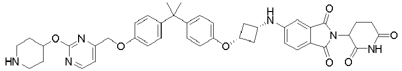 |
| 237 | 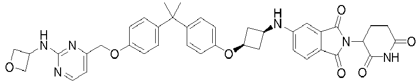 | 238 | 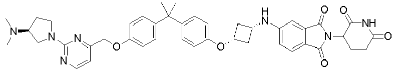 |
| 239 | 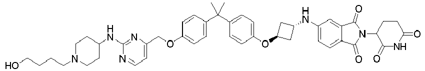 | 240 | 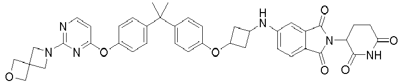 |
| 241 | 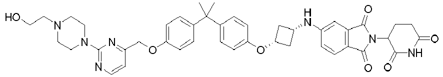 | 242 | 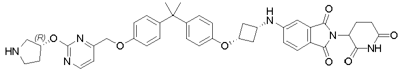 |
| 243 | 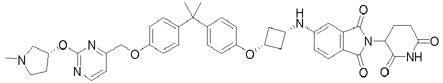 | 244 | 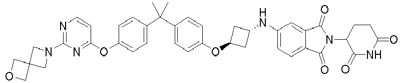 |
| 245 | 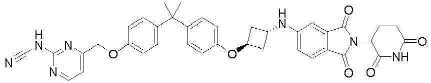 | 246 | 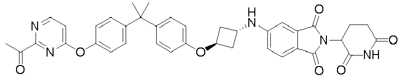 |
| 247 | 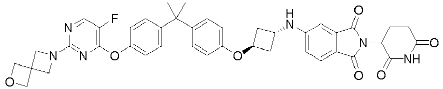 | 248 | 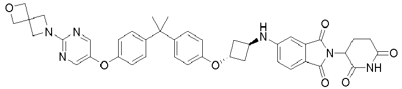 |
| 249 | 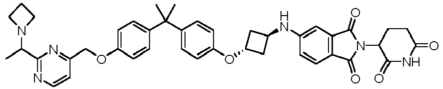 | 250 | 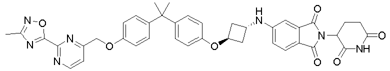 |
| 251 | 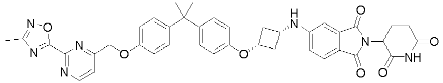 | 252 | 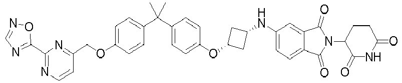 |
| 253 | 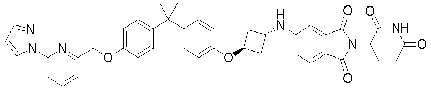 | 254 | 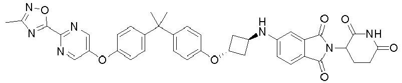 |
| 255 | 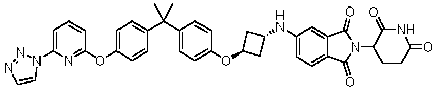 | 256 | 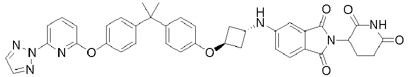 |
| 257 | 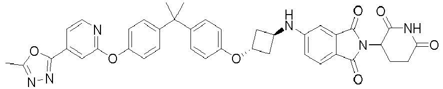 | 258 | 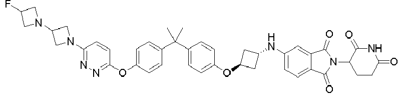 |
| 259 | 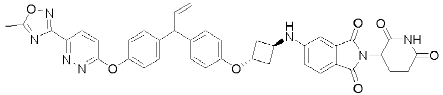 | 260 | 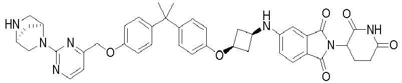 |
| 261 | 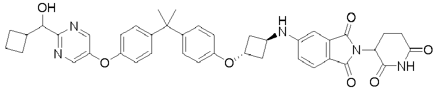 | 262 | 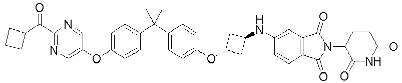 |
| 263 | 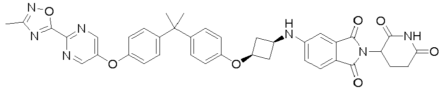 | 264 | 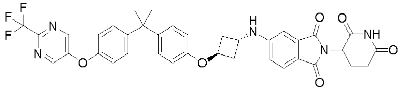 |
| 265 | 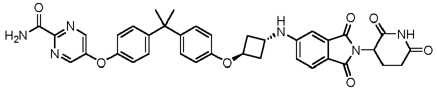 | 266 | 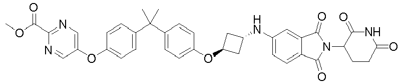 |
| 267 | 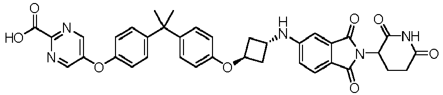 | 268 | 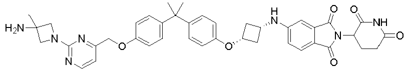 |
| 269 | 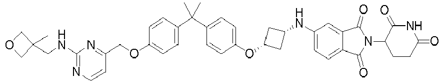 | 270 | 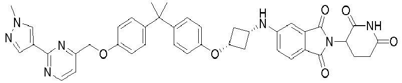 |
| 271 | 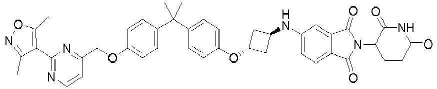 | 272 | 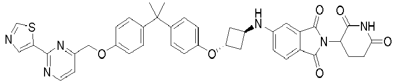 |
| 273 | 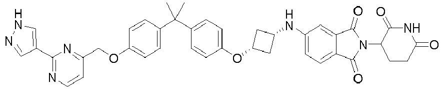 | 274 | 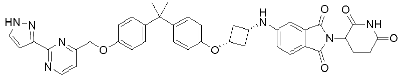 |
| 275 | 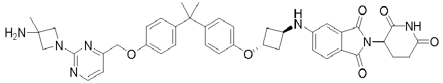 | 276 | 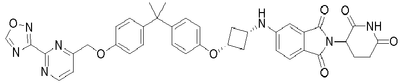 |
| 277 | 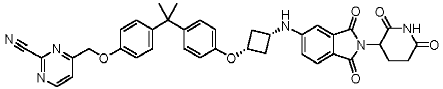 | 278 | 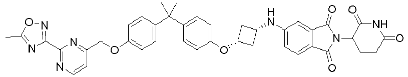 |
| 279 | 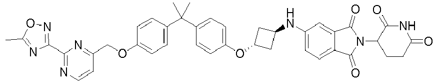 | 280 | 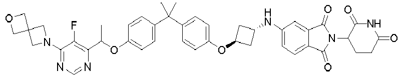 |
| 281 | 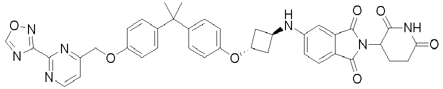 | 282 | 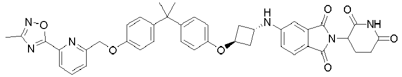 |
| 283 | 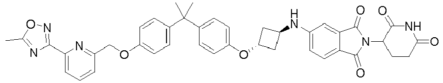 | 284 | 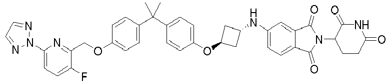 |
| 285 | 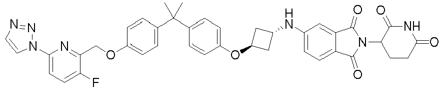 | 286 | 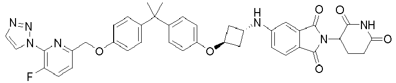 |
| 287 | 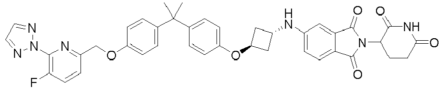 | 288 | 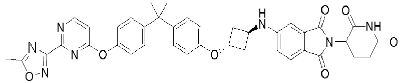 |
| 289 | 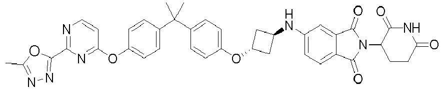 | 290 | 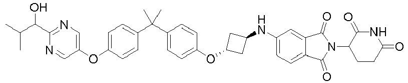 |
| 291 | 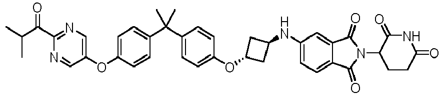 | 292 | 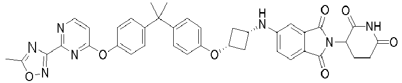 |
| 293 | 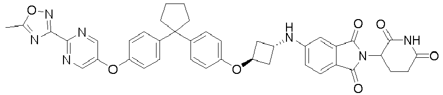 | 294 | 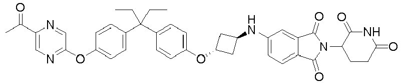 |
| 295 | 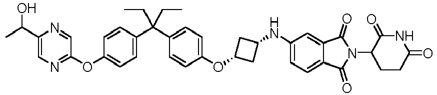 | 296 | 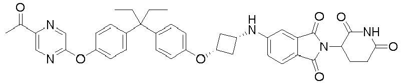 |
| 297 | 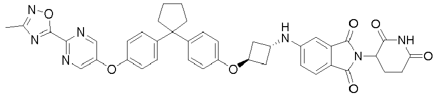 | 298 | 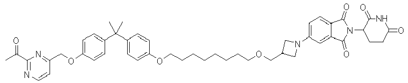 |
| 299 | 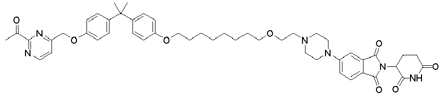 | 300 | 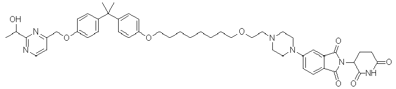 |
| 301 | 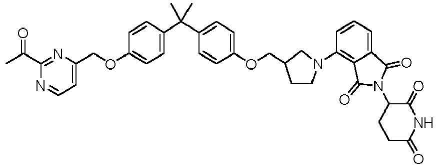 | 302 | 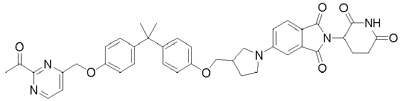 |
| 303 | 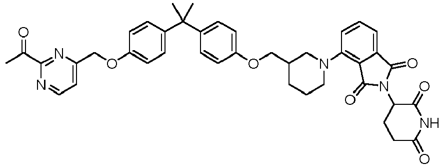 | 304 | 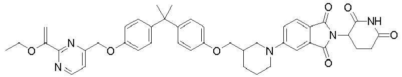 |
| 305 | 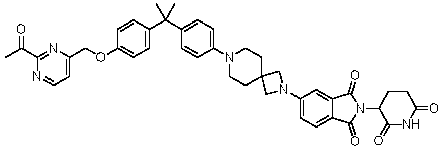 | 306 | 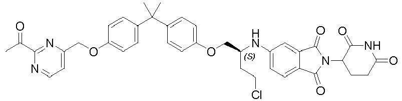 |
| 307 | 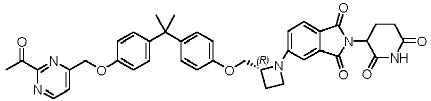 | 308 | 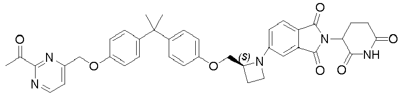 |
| 309 | 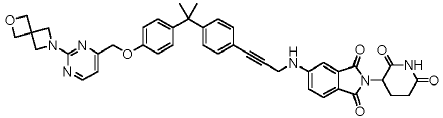 | 310 | 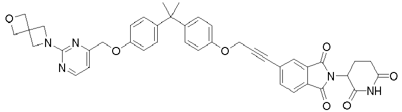 |
| 311 | 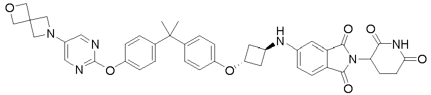 | 312 | 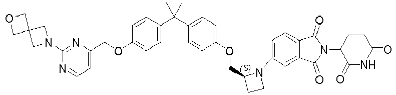 |
| 313 | 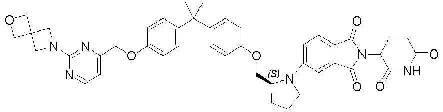 | 314 | 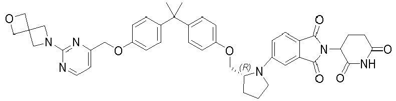 |
| 315 | 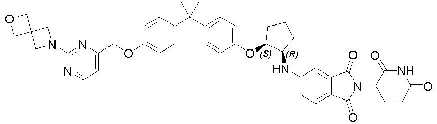 | 316 | 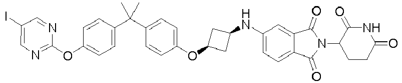 |
| 317 | 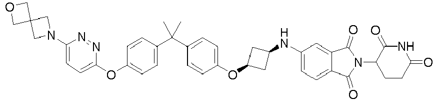 | 318 | 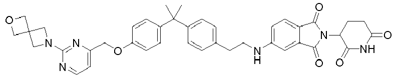 |
| 319 | 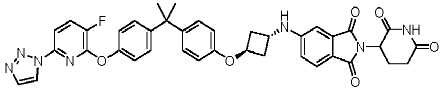 | 320 | 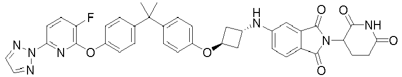 |
| 321 | 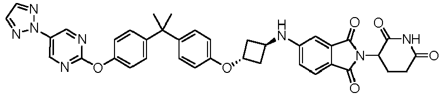 | 322 | 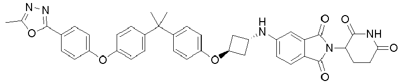 |
| 323 | 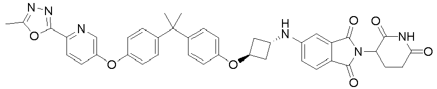 | 324 | 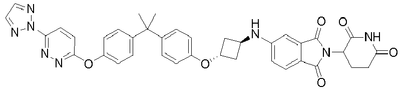 |
| 325 | 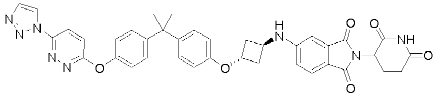 | 326 | 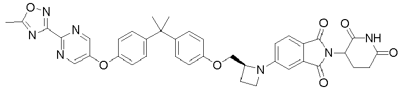 |
| 327 | 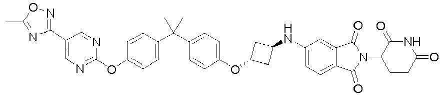 | 328 | 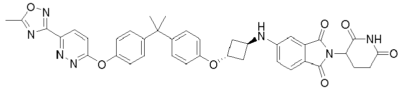 |
| 329 | 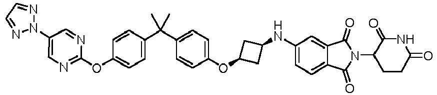 | 330 | 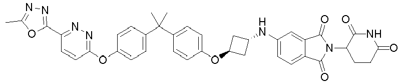 |
| 331 | 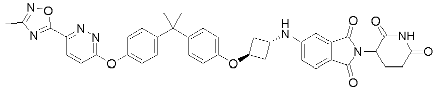 | 332 | 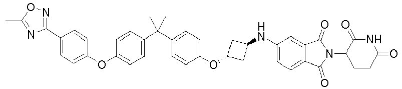 |
| 333 | 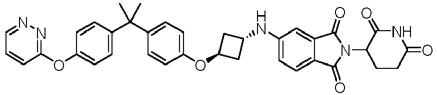 | 334 | 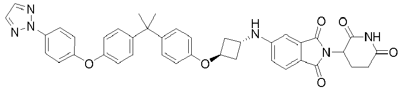 |
| 335 | 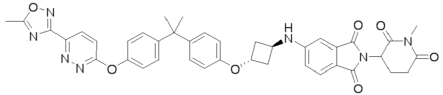 | 336 | 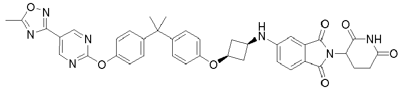 |
| 337 | 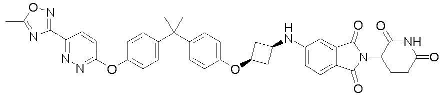 | 338 | 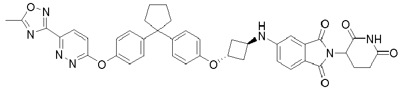 |
| 339 | 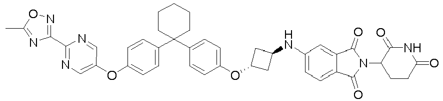 | 340 | 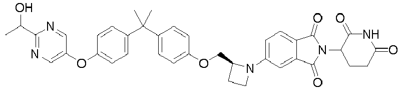 |
| 341 | 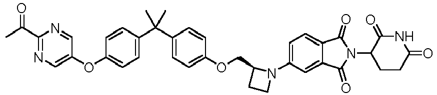 | 342 | 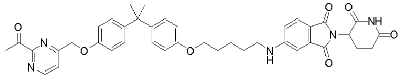 |
| 343 | 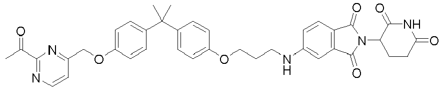 | 344 | 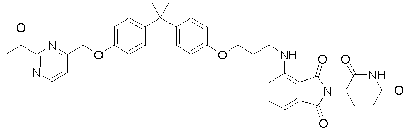 |
| 345 | 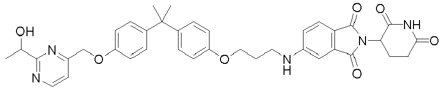 | 346 | 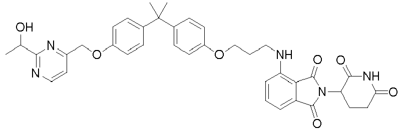 |
| 347 | 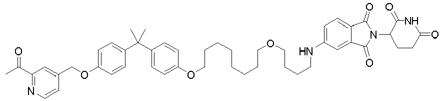 | 348 | 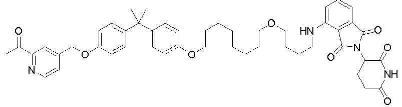 |
| 349 | 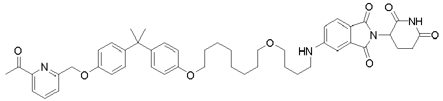 | 350 | 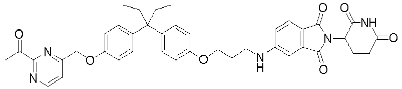 |
| 351 | 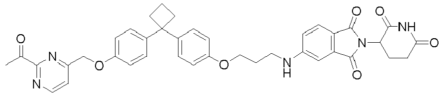 | 352 | 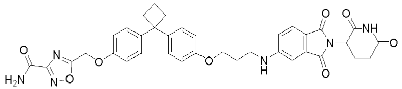 |
| 353 | 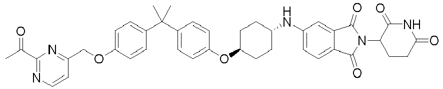 | 354 | 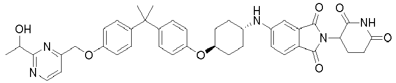 |
| 355 | 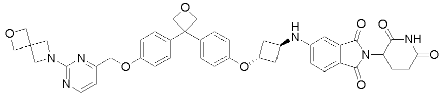 | 356 | 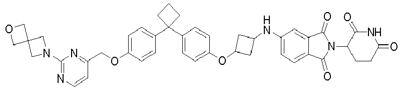 |
| 357 | 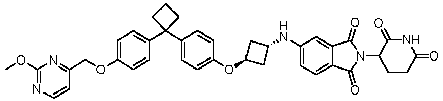 | 358 | 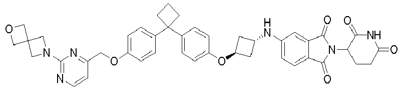 |
| 359 | 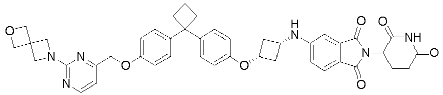 | 360 | 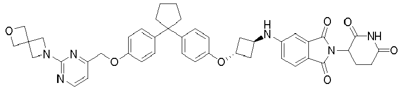 |
| 361 | 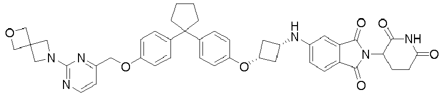 | 362 | 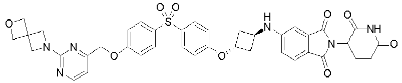 |
| 363 | 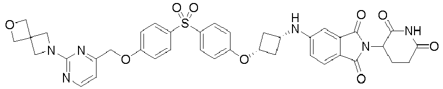 | 364 | 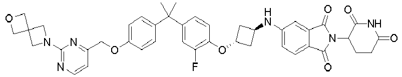 |
| 365 | 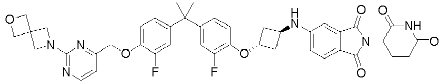 | 366 | 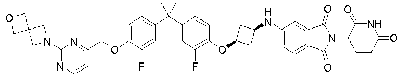 |
| 367 | 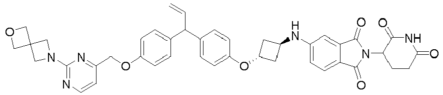 | 368 | 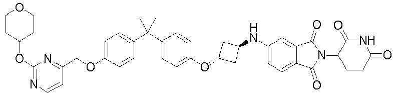 |
| 369 | 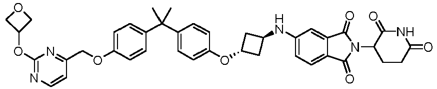 | 370 | 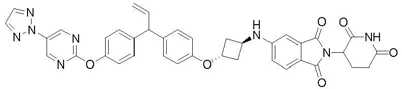 |
| 371 | 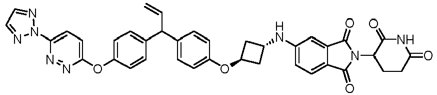 | 372 | 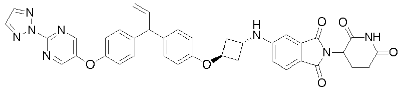 |
| 373 | 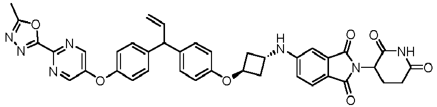 | 374 | 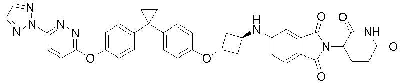 |
| 375 | 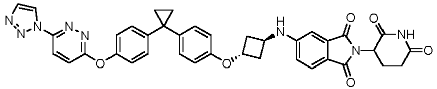 | 376 | 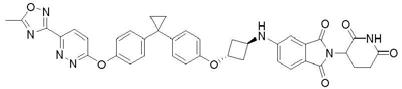 |
| 377 | 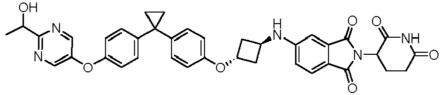 | 378 | 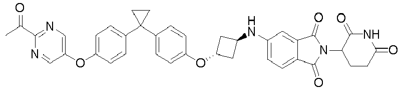 |
| 379 | 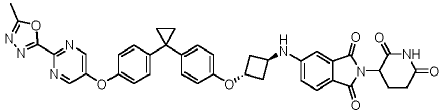 | 380 | 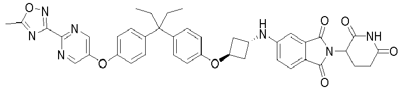 |
| 381 | 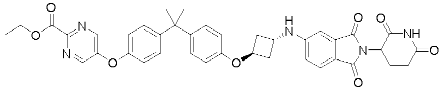 | 382 | 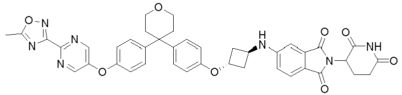 |
| 383 | 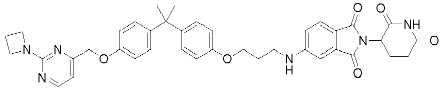 | 384 | 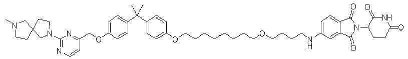 |
| 385 | 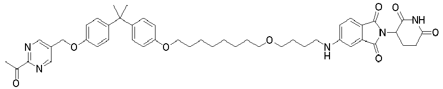 | 386 | 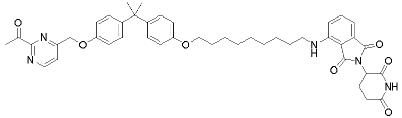 |
| 387 | 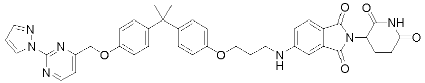 | 388 | 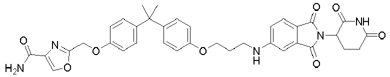 |
| 389 | 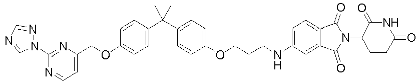 | 390 | 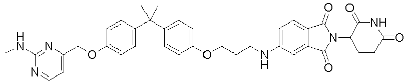 |
| 391 | 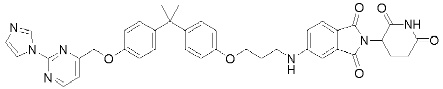 | 392 | 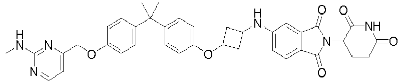 |
| 393 | 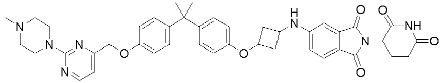 | 394 | 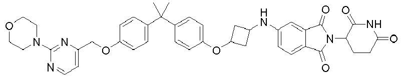 |
| 395 | 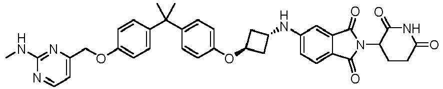 | 396 | 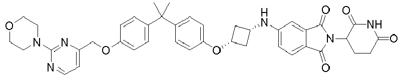 |
| 397 | 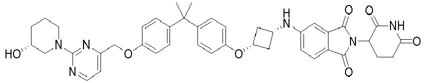 | 398 | 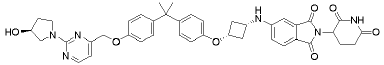 |
| 399 | 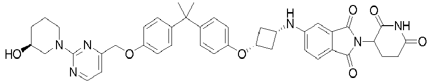 | 400 | 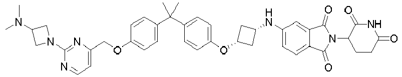 |
| 401 | 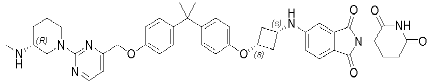 | 402 | 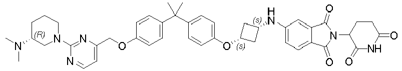 |
| 403 | 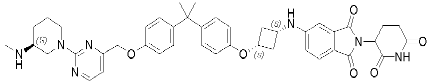 | 404 | 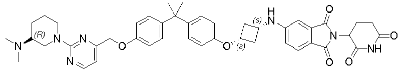 |
| 405 | 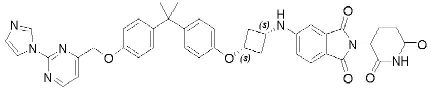 | 406 | 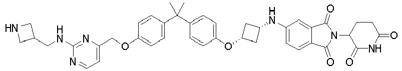 |
| 407 | 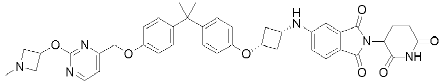 | 408 | 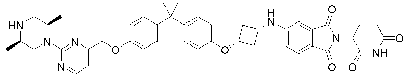 |
| 409 | 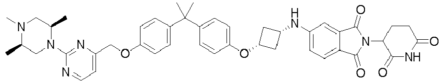 | 410 | 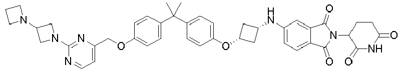 |
| 411 | 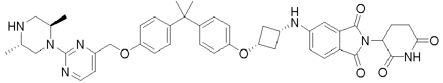 | 412 | 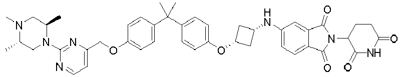 |
| 413 | 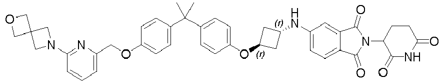 | 414 | 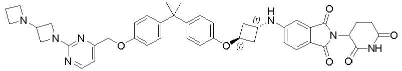 |
| 415 | 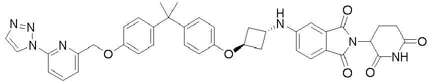 | 416 | 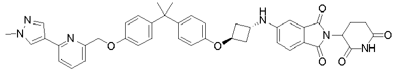 |
| 417 | 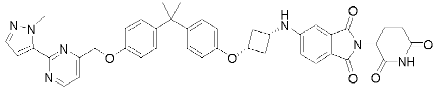 | 418 | 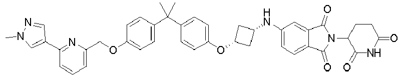 |
| 419 | 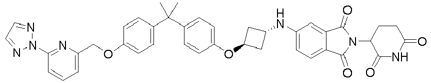 | 420 | 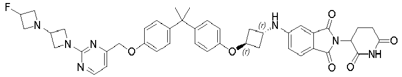 |
| 421 | 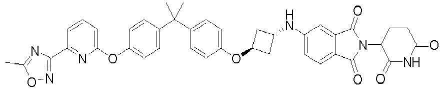 | 422 | 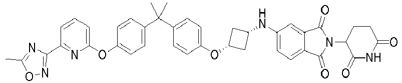 |
| 423 | 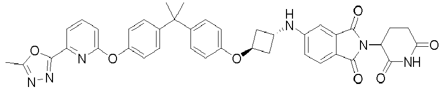 | 424 | 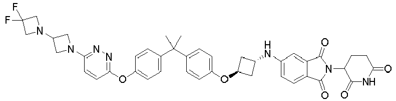 |
| 425 | 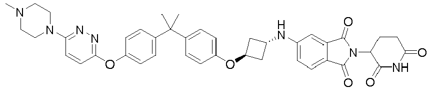 | 426 | 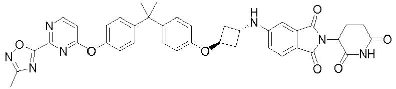 |
| 427 | 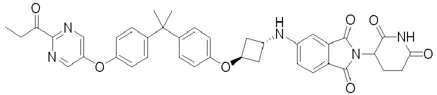 | 428 | 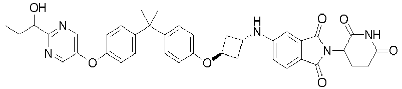 |
| 429 | 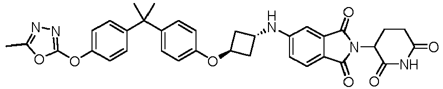 | 430 | 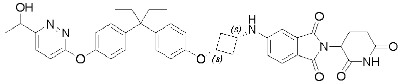 |
| 431 | 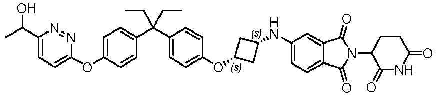 | 432 | 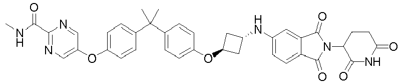 |
| 433 | 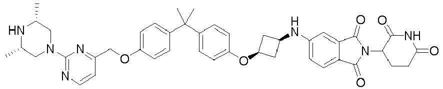 | 434 | 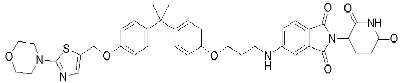 |
| 435 | 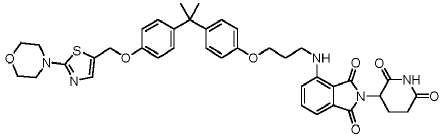 | 436 | 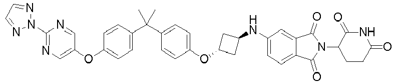 |
| 437 | 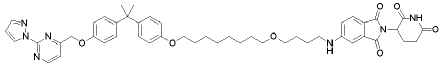 | 438 | 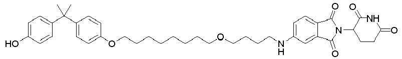 |
| 439 | 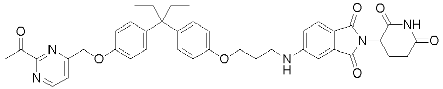 | 440 | 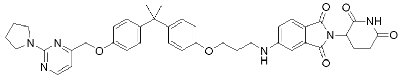 |
| 441 | 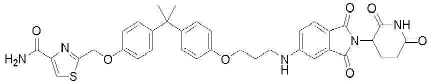 | 442 | 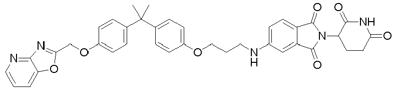 |
| 443 | 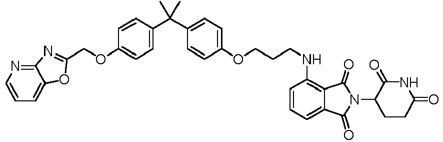 | 444 | 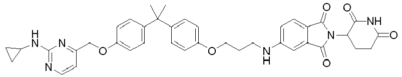 |
| 445 | 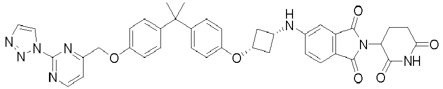 | 446 | 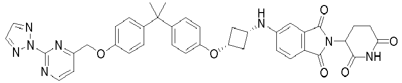 |
| 447 | 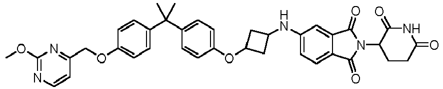 | 448 | 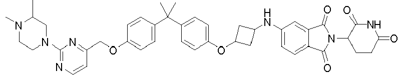 |
| 449 | 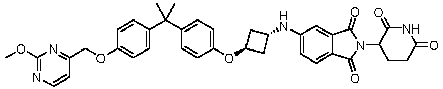 | 450 | 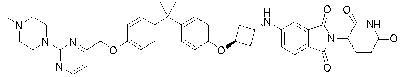 |
| 451 | 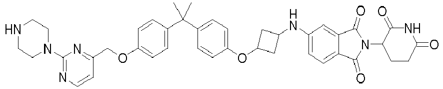 | 452 | 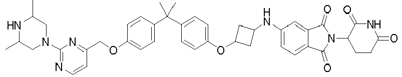 |
| 453 | 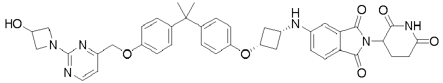 | 454 | 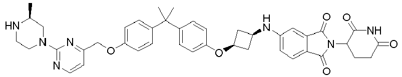 |
| 455 | 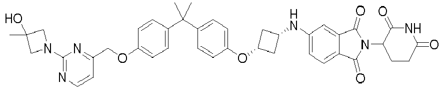 | 456 | 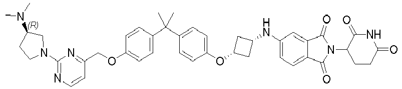 |
| 457 | 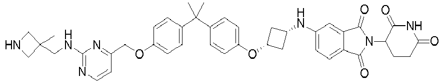 | 458 | 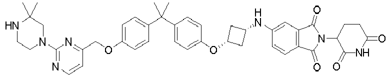 |
| 459 | 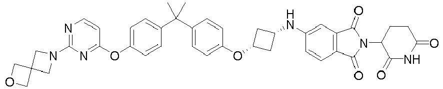 | 460 | 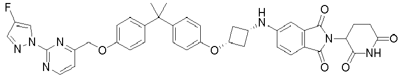 |
| 461 | 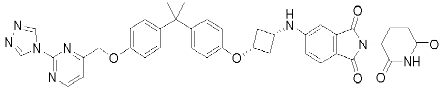 | 462 | 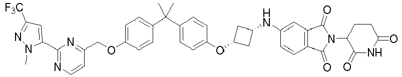 |
| 463 | 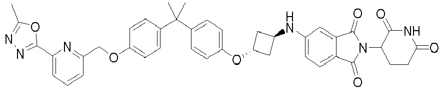 | 464 | 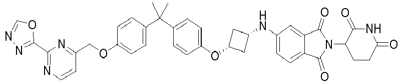 |
| 465 | 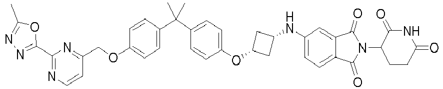 | 466 | 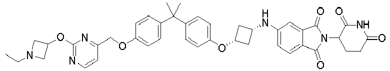 |
| 467 | 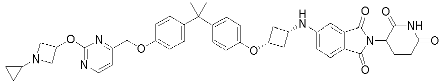 | 468 | 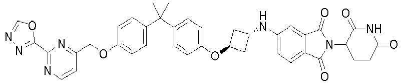 |
| 469 | 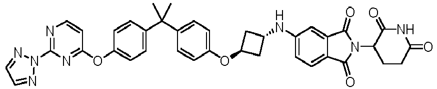 | 470 | 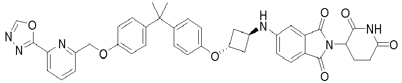 |
| 471 | 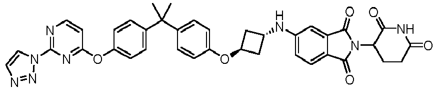 | 472 | 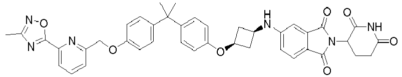 |
| 473 | 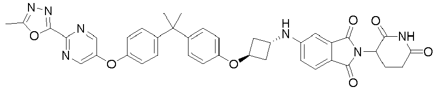 | 474 | 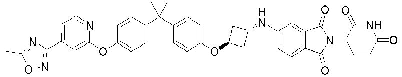 |
| 475 | 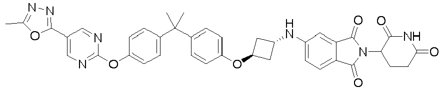 | 476 | 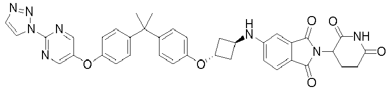 |
| 477 | 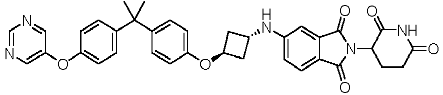 | 478 | 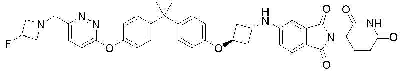 |
| 479 | 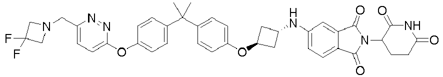 | 480 | 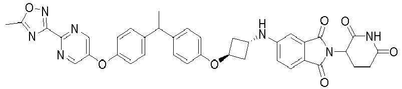 |
| 481 | 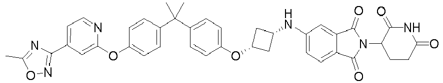 | 482 | 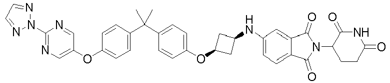 |
| 483 | 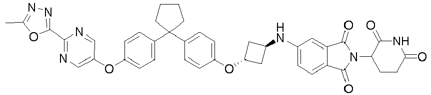 | 484 | 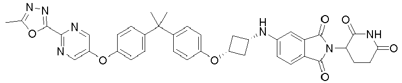 |
| 485 | 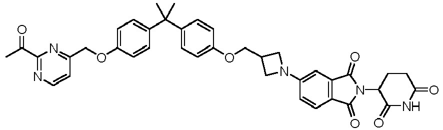 | 486 | 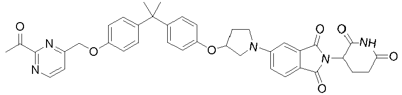 |
| 487 | 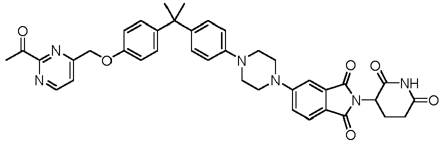 | 488 | 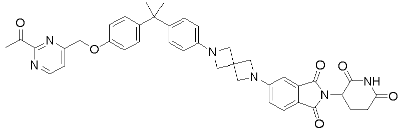 |
| 489 | 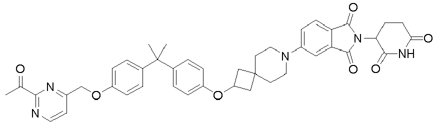 | 490 | 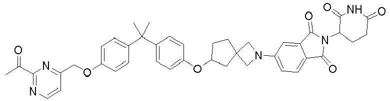 |
| 491 | 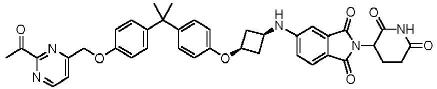 | 492 | 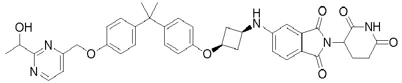 |
| 493 | 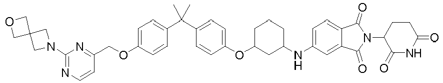 | 494 | 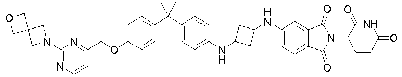 |
| 495 | 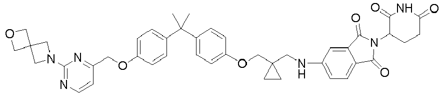 | 496 | 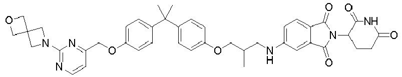 |
| 497 | 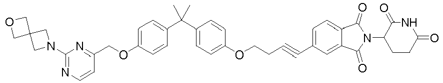 | 498 | 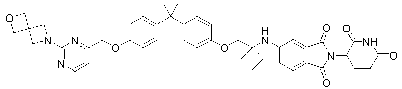 |
| 499 | 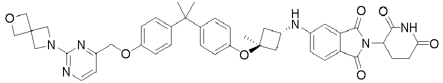 | 500 | 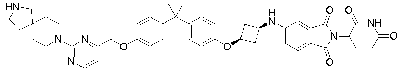 |
| 501 | 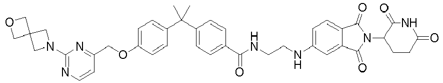 | 502 | 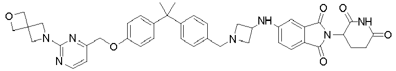 |
| 503 | 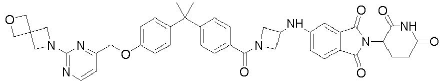 | 504 | 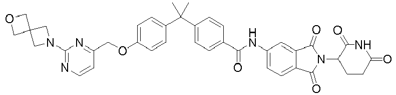 |
| 505 | 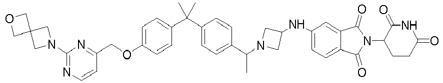 | 506 | 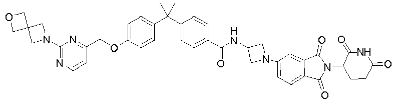 |
| 507 | 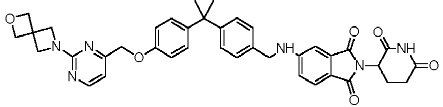 | 508 | 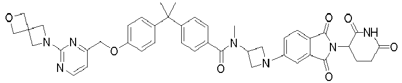 |
| 509 | 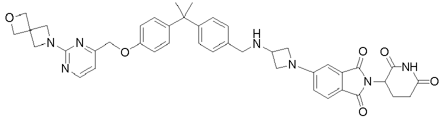 | | |
| 511 | 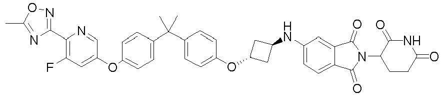 | 512 | 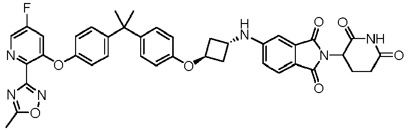 |
| 513 | 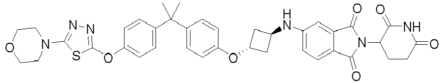 | 514 | 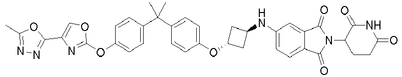 |
| 515 | 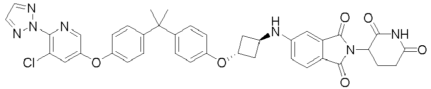 | 516 | 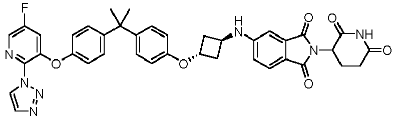 |
| 517 | 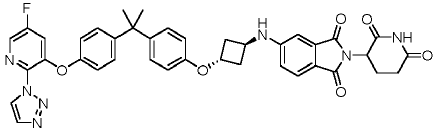 | 518 | 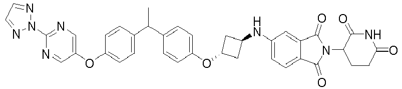 |
| 519 | 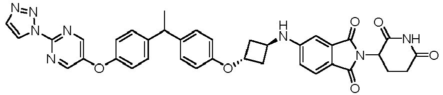 | 520 | 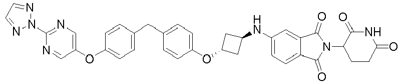 |
| 521 | 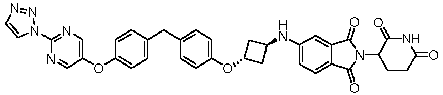 | 522 | 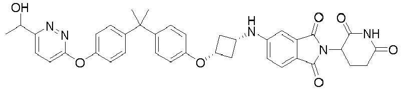 |
| 523 | 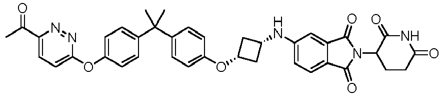 | 524 | 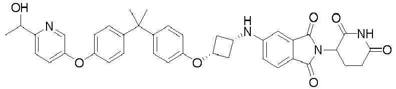 |
| 525 | 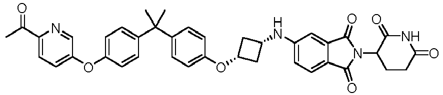 | 526 | 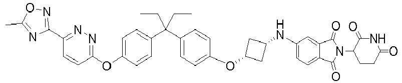 |
| 527 | 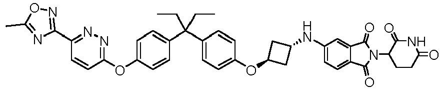 | 528 | 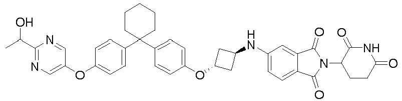 |
| 529 | 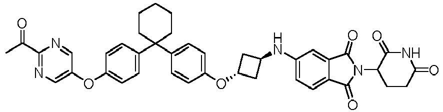 | 530 | 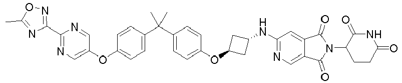 |
| 531 | 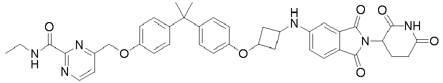 | 532 | 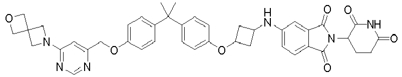 |
| 533 | 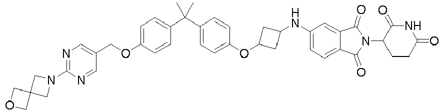 | 534 | 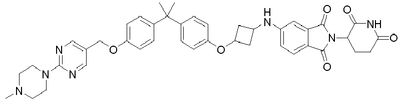 |
| 535 | 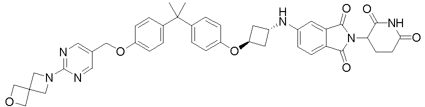 | 536 | 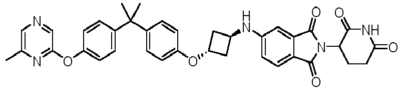 |
| 537 | 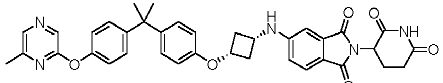 | 538 | 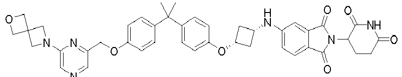 |
| 539 | 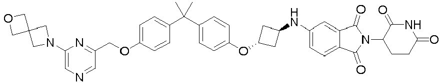 | 540 | 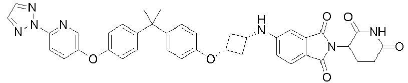 |
| 541 | 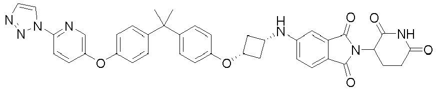 | 542 | 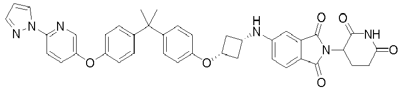 |
| 543 | 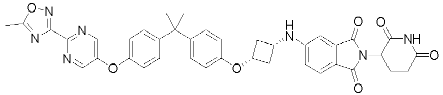 | 544 | 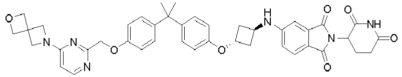 |
| 545 | 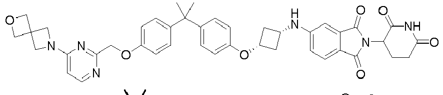 | 546 | 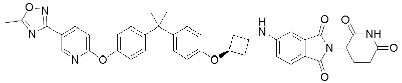 |
| 547 | 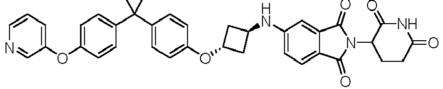 | 548 | 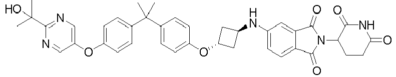 |
| 549 | 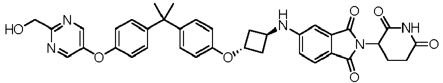 | 550 | 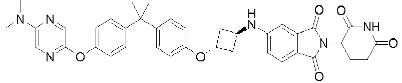 |
| 551 | 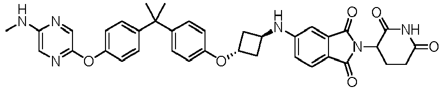 | 552 | 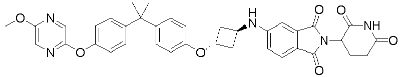 |
| 553 | 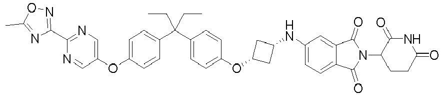 | 554 | 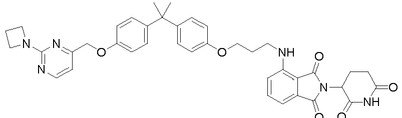 |
| 555 | 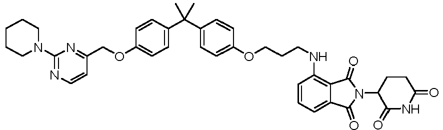 | | |
| 557 | 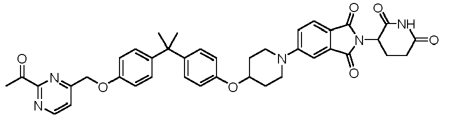 | 558 | 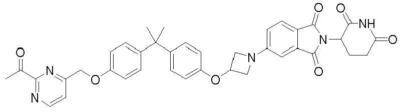 |
| 559 | 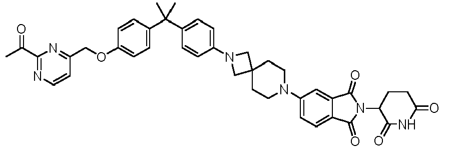 | 560 | 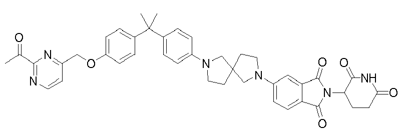 |
| 561 | 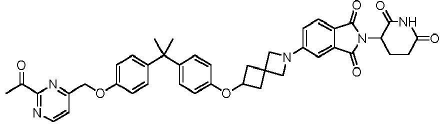 | 562 | 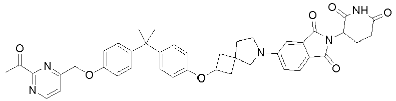 |
| 563 | 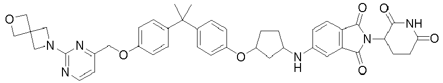 | 564 | 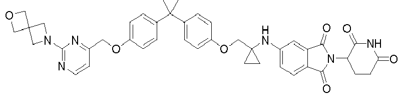 |
| 565 | 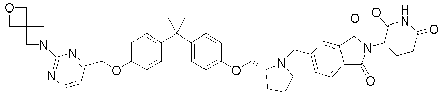 | 566 | 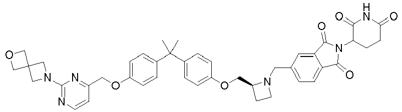 |
| 567 | 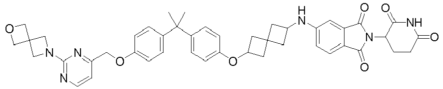 | 568 | 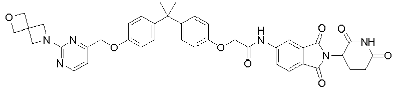 |
| 569 | 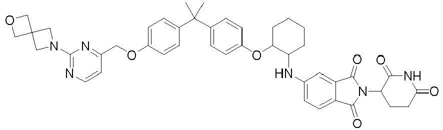 | 570 | 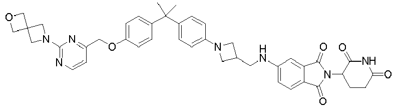 |
| 571 | 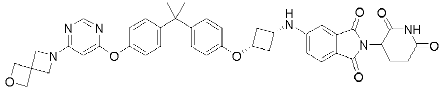 | 572 | 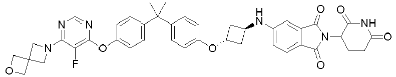 |
| 573 | 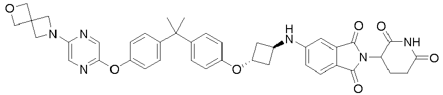 | 574 | 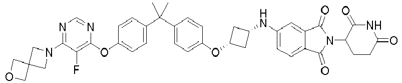 |
| 575 | 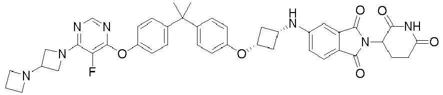 | 576 | 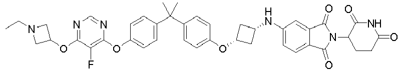 |
| 577 | 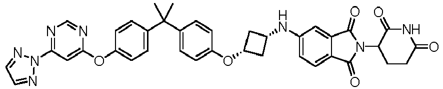 | 578 | 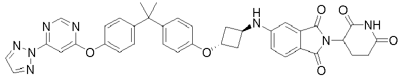 |
| 579 | 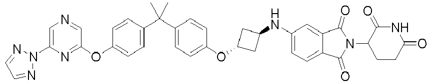 | 580 | 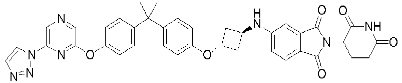 |
| 581 | 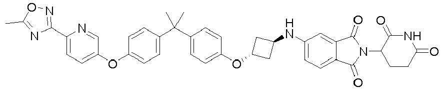 | 582 | 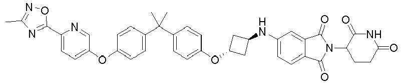 |
| 583 | 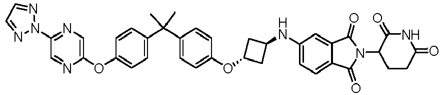 | 584 | 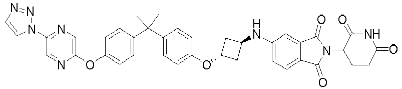 |
| 585 | 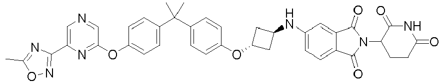 | 586 | 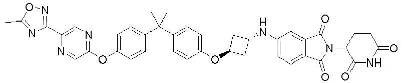 |
| 587 | 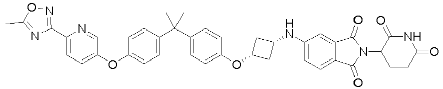 | 588 | 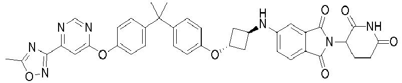 |
| 589 | 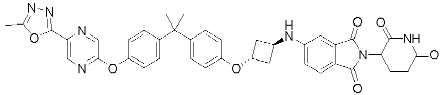 | 590 | 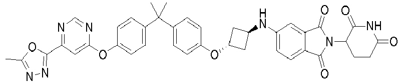 |
| 591 | 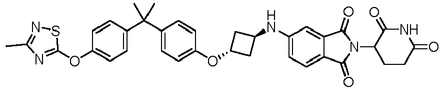 | 592 | 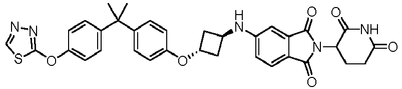 |
| 593 | 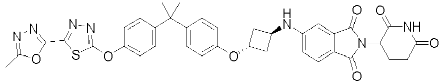 | 594 | 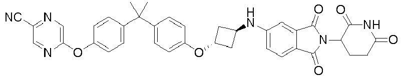 |
| 595 | 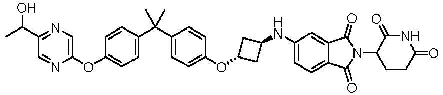 | 596 | 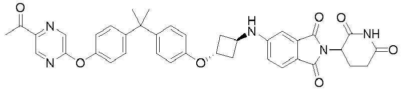 |
| 597 | 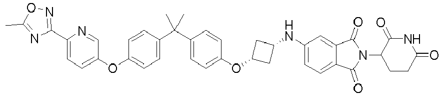 | 598 | 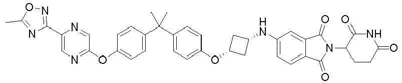 |
| 599 | 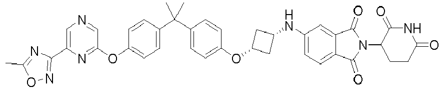 | 600 | 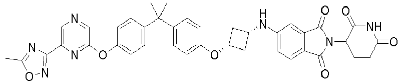 |
| 601 | 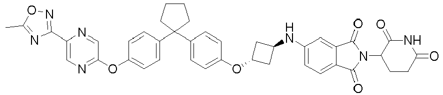 | | |

The present invention also provides a medicament, which is a preparation formed by the above compounds, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, as the acetive ingredient, in combination with pharmaceutically acceptable excipients.

The present invention also provides the use of the above compound, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof in the manufacturer of proteolysis targeting chimeras (PROTAC) for androgen receptors (AR).

Further, the proteolysis targeting chimeras can target the recognition/binding of androgen receptors.

Further, the proteolysis targeting chimeras can degrade androgen receptors.

Further, the androgen receptors include wild-type and mutant androgen receptors.

More further, the mutant androgen receptor comprises those obtained by a splice site mutation and a point mutation in androgen receptors; the preferred is an androgen receptor AR-v7 obtained by a splice site mutation.

Further, the proteolysis targeting chimeras are medicaments for treating diseases regulated by androgen receptors.

More further, the disease is cancer, alopecia, acne or corona virus disease 2019 (COVID-19).

More further, the cancer is that with positive expression of androgen receptors.

More further, the cancer is drug-resistant.

Preferably, the cancer is prostate cancer, breast cancer, ovarian cancer, bladder cancer, pancreatic cancer, hepatocellular carcinoma, endometrial cancer or salivary gland cancer.

As demonstrated by the experimental results, the compound of the present invention can target the degradation of AR, downregulate the expression level of AR, and particularly have significant degradation activity and inhibitory effect on the AR splicing variant AR-v7. The compound of the present invention can also effectively inhibit the proliferation of drug-resistant prostate cancer cells, and exhibit good metabolic stability and oral pharmacokinetic properties, therby having good application prospects in the manufacturer of protein degradation agents targeting AR as well as medicaments for the treatment of diseases regulated by AR.

For the definition of the terms used in the present invention: unless indicated otherwise, the initial definition provided for the group or the term herein is applicable to those in the whole specification; for terms not specifically defined herein, according to the disclosure content and the context, the term will have their common meaning as understood by one of ordinary skill in the art to which this invention pertains.

In the present invention, "substitution" means that one, two or more hydrogens in a molecule are substituted by other different atoms or molecules, including one, two or more substitutions on the same or different atoms in the molecule.

In the present invention, the minimum and the maximum for the content of carbon atoms in hydrocarbon groups are represented by prefixes, for example, C1-C6 alkyl or C1-6 alkyl denotes C1, C2, C3, C4, C5, and C6 alkyls, i.e. a straight or branched alkyl containing 1-6 carbon atoms, such as methyl, ethyl, propyl, isopropyl, butyl, isobutyl, tert-butyl, sec-butyl, pentanyl, hexanyl, etc. Similarly, C1-C6 alkoxy denotes C1, C2, C3, C4, C5, and C6 alkoxys.

"Solvate thereof" means a solvate formed by the compound of the present invention and a solvent, wherein the solvent includes (but is not limited to) water, ethanol, methanol, isopropanol, propanediol, tetrahydrofuran, and dichloromethane.

In the present invention, "pharmaceutically acceptable" means certain carriers, vehicles, diluents, excipients, and/or formed salts are usually chemically or physically compatible with other ingredients constituting certain pharmaceutical dosage forms, as well as physiologically compatible with the recipient.

In the present invention, "salt" means acid and/or basic salt which is formed by reaction of compound or its stereoisomer with inorganic and/or organic acid and/or base, and also includes zwitterionic salts (inner salts), and further includes quaternary ammonium salts, such as alkylammonium salt. These salts can be directly obtained during the final isolation and purification of a compound. The salts can also be obtained by mixing the compound or its stereoisomers with a certain amount of acid or base appropriately (for example, in equivalent). These salts may form a precipitate in the solution, and be collected by filtration, or recovered after evaporation of the solvent, or obtained by freeze-drying after reaction in an aqueous medium. The salt in the present invention may be compounds' hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate.

In the present invention, "aromatic ring" denotes all-carbon monocycle or fused polycycle with conjugated π electron system, such as phene and naphthalene. Said aromatic ring can be fused to other cyclic structures (including saturated and unsaturated rings), but can not contain heteroatoms such as nitrogen, oxygen, or sulfur. At the same time, the point linking to the parent must be on the carbon in the ring having the conjugated π electron system. "Monocyclic aromatic ring" refers to an all-carbon single ring with a conjugated π-electron system. Similarly, "aryl" refers to all-carbon monocyclic or fused polycyclic groups with conjugated π-electron systems, such as phenyl and naphthyl.

"Aromatic heterocycle" refers to a monocycle or fused polycycle containing a conjugated π-electron system as well as one or more heteroatoms. The system contains at least one ring heteroatom selected from N, O, or S, with the remaining ring atoms being all C, and has a fully conjugated π-electron system, e.g. furan, pyrrole, quinoline, thiophene, pyridine, pyrazole, N-alkylpyrrole, pyrimidine, pyrazine, imidazole, tetrazole, thienopyridyl, etc. The aromatic heterocycle can condense with an aromatic ring, heterocycle, or alkane ring. "Monocyclic aromatic heterocycle" refers to a single ring containing a conjugated π electron system as well as one or more heteroatoms. Similarly, "heteroaryl" refers to a monocyclic or fused polycyclic group containing one or more heteroatoms as well as a conjugated π-electron system.

"Halogen" is fluorine, chlorine, bromine, or iodine.

"Alkyl" is a hydrocarbon group formed by losing one hydrogen in an alkane molecule, such as methyl -CH₃, ethyl -CH₃CH₂, etc.

"Alkynyl" denotes aliphatic hydrocarbon groups with at least one C≡C triple bond. Said alkynyl can be straight or branched chain. When alkynyls have a limit on carbon numbers before them, for example, "C₂₋₆ alkynyls" denote a straight or branched alkynyl having 2-6 carbons.

"Alkenyl" denotes aliphatic hydrocarbon groups with at least one C=C double bond. Said alkenyl can be straight or branched chain. When alkenyls have a limit on carbon numbers before them, for example, "C₂₋₆ alkenyls" denote a straight or branched alkenyl with 2-6 carbons.

"Cycloalkane" denotes a saturated or unsaturated cyclic hydrocarbon; "cycloalkyls" denote saturated or unsaturated cyclic hydrocarbon substituents; the cyclic hydrocarbon can have one or more rings. For example, "3-8-membered cycloalkyl" denotes a cycloalkyl having 3-8 carbons.

"Heterocycle" denotes a saturated or unsaturated cyclic hydrocarbon; "heterocyclic group" denotes a saturated or unsaturated cyclic hydrocarbon substituent; the cyclic hydrocarbon may be monocyclic or polycyclic, and carry at least one ring heteroatom (including but not limited to O, S or N). For example, "3-8-membered heterocyclic group" denotes a heterocyclic group having 3-8 carbons.

"Fused heterocycle" means a saturated or unsaturated polycyclic hydrocarbon; "fused heterocyclic group" means a saturated or unsaturated polycyclic hydrocarbon group carrying at least one ring heteroatom (including but not limited to O, S or N), in which two rings share two adjacent carbon atoms or heteroatoms.

"Fused aromatic heterocycle" refers to a polycyclic structure in which at least one ring of the fused heterocycle mentioned above is aromatic; "fused aromatic heterocyclic group" refers to a group in which at least one ring of the fused heterocyclic group mentioned above is aromatic.

"Spiral ring" refers to two poly-membered rings that share one carbon atom, while "bridged ring" refers to a poly-membered ring that shares two or more carbon atoms.

"Heterospirocyclyl" means a polycyclic heterocyclic group in which two rings share one carbon atom or heteroatom.

"Alkylthio" refers to a group formed by connecting linear or branched alkyl or cycloalkyl with -S-;

"Alkylsulfonyl" refers to a group formed by connecting linear or branched alkyl or cycloalkyl with -SO₂-;

"Alkylsulfinyl" refers to a group formed by connecting linear or branched alkyl or cycloalkyl with -SO-;

"Alkylcarbonyl" refers to a group formed by connecting linear or branched alkyl or cycloalkyl with -CO-;

"Alkylaminocarbonyl" refers to a group formed by connecting linear or branched alkyl or cycloalkyl with -NH-CO- or

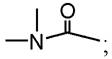

"Alkylamino" refers to a group formed by connecting linear or branched alkyl or cycloalkyl with -NH- or

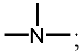

In the present invention, an isotope-substituted form of a compound refers to the compound obtained by substituting any one or more atoms in the compound with isotope(s).

For isotopes, the different nuclides of a chemical element having the same number of protons but different numbers of neutrons are isotopes each other. For example, hydrogen has three isotopes, H hydrogen, D deuterium (also known as heavy hydrogen), and T tritium (also known as superheavy hydrogen). Unless otherwise stated, hydrogen in the present invention is H; carbon has a few isotopes, 12C, 13C, and 14C. Unless otherwise specified, in the present invention, C refers to 12C.

"Cbz" denotes a protecting group benzyloxycarbonyl. "Me" means methyl.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Examples

The starting materials and equipment used in the present invention are all known products and can be obtained by purchasing those commercially available.

### Example 1: N (4-((4-(2-(4-((8-(2-(4-(2-(2,6-dioxopiperidin-3-yl)-1,3- dioxoisoindolin-5-yl)piperazin-1-yl)ethoxy)octyl)oxy)phenyl)propan-2-yl)phenoxy)methyl)pyrimidin-2-yl)methanesulfonamide

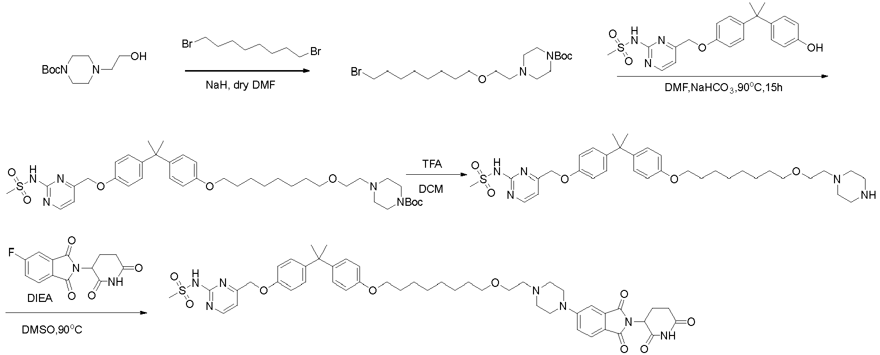

### Step 1: Synthesis of tert-butyl 4-(2-((8-bromooctyl)oxy)ethyl)piperazin-1-carboxylate

Under N₂ protection, tert-butyl 4-(2-hydroxylethyl)piperazin-1-carboxylate (2.3 g, 10 mmol) was dissolved in dry DMF (10 mL) in an ice water bath, to which was added NaH (800 mg, 20 mmol) in portions, and then the mixture was allowed to react for half an hour in an ice water bath. Then, 1,8-dibromooctane (2.7 g, 10 mmol) was added, and the resultant solution was reacted for 3h at room temperature. The reaction solution was poured to water and extracted. The organic phase was combined, washed with saturated brine, dried with anhydrous Na₂SO₄, and purified by column chromatography, to provide 1.68 g of product (yield 40%). LC/MS (ESI⁺) calcd for C₁₉H₃₇BrN₂O₃ ([M+H]⁺) *m*/*z* 421, found 421

### Step 2: Synthesis of tert-butyl 4-(2-(8-(4-(2-(2-(methanesulfonamido)pyrimidin- 4-yl)methoxy)phenyl)propan-2-yl)phenoxy)octyl)oxy)ethyl)piperazin-1-carboxylate

tert-butyl 4-(2-((8-bromooctyl)oxy)ethyl)piperazin-1-carboxylate (420 mg, 1 mmol) was dissolved in DMF (8 mL), to which was added N-(4-((4-(2-(4-hydroxylphenyl)propan-2-yl)phenoxy)methyl)pyrimidin-2-yl)methanesulfonamide (413 mg, 1mmol), followed by addition of NaHCO₃ (252 mg, 3mmol), and then the reaction solution was heated to 90 °C and reacted for 15 h. The reaction solution was poured to water and extracted. The organic phase was combined, washed with saturated brine, dried with anhydrous Na₂SO₄, and concentrated to provide 600 mg of product (yield 80%). LC/MS (ESI⁺) calcd for C₄₀H₅₉N₅O₇S ([M+H]⁺) *m*/*z* 754, found 754.

### Step 3: Synthesis of N-(4-(4-(2-(4-((8-(2-(piperazin-1-yl)ethoxy)octyl)oxy)phenyl) propan-2-yl)phenoxy)methyl)pyrimidin-2-yl)methanesulfonimide

tert-butyl 4-(2-(8-(4-(2-(2-(methanesulfonamido)pyrimidin-4-yl)methoxy)phenyl) propan-2-yl)phenoxy)octyl)oxy)ethyl)piperazin-1-carboxylate (754 mg, 1 mmol) was dissolved in 40 mL of dichloromethane, to which was added 10 mL of trifluoroacetic acid in an ice bath, and then the mixture was allowed to react for 0.5 h at room temperature. After completion of the reaction detected by TLC, the reaction system was poured into saturated NaHCO₃ solution in an ice bath. The system became alkaline, and then extracted with dichloromethane/methanol (10:1) (3*10 mL). The organic phase was washed with saturated brine, dried with anhydrous Na₂SO₄, and concentrated to provide 653 mg of product (yield 99%). LC/MS (ESI⁺) calcd for C₃₅H₅₁N₅O₅S ([M+H]⁺) *m*/*z* 654, found 654.

### Step 4: Synthesis of N-(4-((4-(2-(4-((8-(2-(4-(2-(2,6-dioxopiperidin-3-yl)-1,3- dioxoisoindolin-5-yl)piperazin-1-yl)ethoxy)octyl)oxy)phenyl)propan-2-yl)phenoxy)methyl)pyrimidin-2-yl)methanesulfonamide

N (4-(4-(2-(4-((8-(2-(piperazin-1-yl)ethoxy)octyl)oxy)phenyl)propan-2-yl)phenoxy)methyl)pyrimidin-2-yl)methanesulfonimide (653 mg, 1 mmol), 2-(2,6-dioxopiperidin-3-yl)-5-fluoroisoindolin-1,3-dione (276 mg, 1 mmol) and DIPEA (387 mg, 3 mmol) were successively added into 15 mL of DMSO, and then the solution was heated to 90 °C and reacted for 36 h. The reaction solution was naturally cooled to room temperature, and then poured to water. The resultant solution was extracted with ethyl acetate. The organic phase was dried with anhydrous Na₂SO₄, concentrated, and then separated and purified by column chromatography, to provide 455 mg of product (yield 50%). ¹H NMR (400 MHz, Chloroform-d) *δ* 8.55 (d, *J* = 5.0 Hz, 1H), 8.15 (s, 1H), 7.68 (d, *J* = 8.5 Hz, 1H), 7.27 (d, *J* = 2.3 Hz, 1H), 7.20 - 7.11 (m, 3H), 7.10 - 6.99 (m, 3H), 6.88 - 6.79 (m, 2H), 6.77 - 6.67 (m, 2H), 5.05 (s, 2H), 4.94 (dd, *J* = 12.2, 5.2 Hz, 1H), 4.10 (p, *J =* 5.7, 5.2 Hz, 2H), 3.63 (t, *J =* 5.4 Hz, 2H), 3.48 (s, 6H), 3.41 (t, *J* = 6.6 Hz, 2H), 2.93 - 2.63 (m, 8H), 2.13 (ddd, *J* = 10.3, 5.0, 2.6 Hz, 1H), 1.72 (s, 2H), 1.65 - 1.40 (m, 9H). LC/MS (ESI⁺) calcd for C₄₈H₅₉N₇O₉S ([M+H]⁺) *m*/*z* 910, found 910.

### Example 2: N-(4-((4-(2-(4-((7-(2-(4-(2-(2,6-dioxopiperidin-3-yl)-1,3- dioxoisoindolin-5-yl)piperazin-1-yl)ethoxy)heptyl)oxy)phenyl)propan-2-yl)phenoxy)methyl)pyrimidin-2-yl)methanesulfonamide

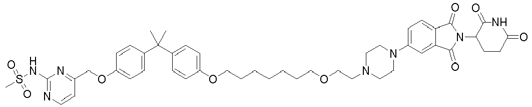

The title compound was synthesized using a method similar to that of Example 1. LC/MS (ESI⁺) calcd for C₄₇H₅₇N₇O₉S ([M+H]⁺) *m*/*z* 896, found 896.

### Example 3: Synthesis of N-(4-(4-(2-(4-(4-(4-(4-(2-(2,6-dioxopiperidin-3-yl)-1,3-dioxoisoindolin-5-yl)-2,7-diazaspiro[3.5]nonan-7-yl)butoxy)butoxy)phenyl)propan-2-yl)phenoxy)methyl)pyrimidin-2-yl)methanesulfonamide

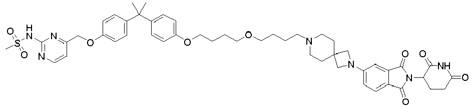

The target compound was synthesized using a preparation method similar to that of Example 1. ¹H NMR (400 MHz, Chloroform-d) *δ* 8.55 (d, *J* = 5.1 Hz, 1H), 7.63 (d, *J* = 8.3 Hz, 1H), 7.23 - 7.10 (m, 3H), 7.10 - 6.99 (m, 2H), 6.87 - 6.79 (m, 2H), 6.76 (d, *J* = 2.1 Hz, 1H), 6.74 - 6.68 (m, 2H), 6.49 (dd, *J* = 8.3, 2.2 Hz, 1H), 5.06 (s, 2H), 4.95 - 4.90 (m, 1H), 4.12 (tt, J = 7.2, 3.2 Hz, 2H), 3.72 (s, 4H), 3.48 (s, 3H), 3.40 (dt, *J* = 9.5, 5.9 Hz, 4H), 2.92 - 2.70 (m, 4H), 2.63 (s, 6H), 2.44 (d, *J* = 8.4 Hz, 2H), 2.17 - 2.09 (m, 1H), 1.91 (t, *J* = 5.5 Hz, 4H), 1.79 (q, *J* = 8.3, 7.7 Hz, 2H), 1.62 (s, 10H). LC/MS (ESI+) calcd for C₄₉H₅₉N₇O₉S ([M+H]⁺) *m*/*z* 922, found 922.

### Example 4: Synthesis of N-(4-((4-(2-(4-(3-(3-(2-(2-(2,6-dioxopiperidin-3-yl)-1,3-dioxoisoindolin-5-yl)-)2,7-diazaspiro[3.5]nonan-7-yl)propoxy)propoxy) phenyl)propan-2-yl)phenoxy)methyl)pyrimidin-2-yl)methanesulfonamide

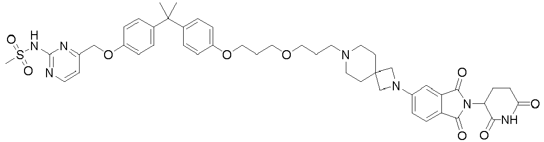

The target compound was synthesized using a method similar to that of Example 1. ¹H NMR (400 MHz, Chloroform-d) *δ* 8.56 (d, *J* = 5.0 Hz, 1H), 7.64 (d, *J* = 8.3 Hz, 1H), 7.21 - 7.09 (m, 3H), 7.08 - 7.00 (m, 2H), 6.86 - 6.79 (m, 2H), 6.77 (d, *J=* 2.1 Hz, 1H), 6.75 - 6.68 (m, 2H), 6.49 (dd, *J* = 8.3, 2.2 Hz, 1H), 5.08 (s, 2H), 4.93 (dd, *J* = 12.2, 5.3 Hz, 1H), 4.23 - 4.11 (m, 2H), 3.73 (s, 3H), 3.52 - 3.34 (m, 6H), 2.92 - 2.69 (m, 4H), 2.63 (s, 6H), 2.18 - 2.08 (m, 2H), 2.01 - 1.89 (m, 6H), 1.82 (s, 2H), 1.62 (s, 6H). LC/MS (ESI+) calcd for C₄₇H₅₅N₇O₉S ([M+H]⁺) *m*/*z* 894, found 894.

### Example 5: N (4-((4-(2-(4-((9-(4-(1-(2-(2,6-dioxopiperidin-3-yl)-1,3- dioxoisoindolin-5-yl)azetidin-3-yl)piperazin-1-yl)nonyl)oxy)phenyl)propan-2-yl)phenoxy)methyl)pyrimidin-2-yl)methanesulfonamide

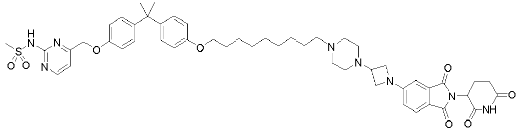

The target compound was synthesized using a method similar to that of Example 1. ¹H NMR (400 MHz, DMSO-d₆) *δ* 11.08 (s, 1H), 9.18 (s, 1H), 8.68 (d, *J* = 5.1 Hz, 1H), 7.64 (d, J = 8.2 Hz, 1H), 7.24 (d, *J* = 5.1 Hz, 1H), 7.10 (d, *J=* 8.3 Hz, 2H), 6.93 (dd, *J* = 29.4, 8.4 Hz, 4H), 6.78 (s, 1H), 6.63 (d, J = 8.3 Hz, 3H), 5.14 (s, 2H), 5.05 (dd, *J=* 13.0, 5.5 Hz, 1H), 4.17 - 3.75 (m, 6H), 3.50 (s, 3H), 3.29 (t, *J* = 6.1 Hz, 5H), 2.94 - 2.80 (m, 1H), 2.22 (t, *J* = 7.3 Hz, 8H), 2.00 (d, *J* = 12.9 Hz, 1H), 1.59 (d, *J* = 36.0 Hz, 8H), 1.41 - 1.12 (m, 12H). LC/MS (ESI+) calcd for C₅₀H₆₂N₈O₈S ([M+H]⁺) *m*/*z* 935, found 935.

### Example 6: Synthesis of N-(4-((4-(2-(4-((6-(4-((4-(2-(2,6-dioxopiperidin-3-yl)-1,3-dioxoisoindolin-5-yl))piperazin-1-yl)methyl)piperidin-1-yl)hexyl)oxy)phenyl) propan-2-yl)phenoxy)methyl)pyrimidin-2-yl)methanesulfonamide

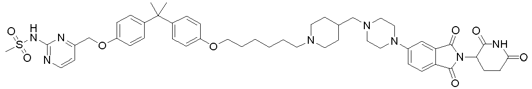

The target compound was synthesized using a method similar to that of Example 1. ¹H NMR (400 MHz, Chloroform-d) *δ* 8.55 (d, *J* = 5.0 Hz, 1H), 7.69 (d, *J* = 8.5 Hz, 1H), 7.28 (d, *J* = 2.2 Hz, 1H), 7.15 (d, *J* = 5.1 Hz, 1H), 7.12 - 7.07 (m, 2H), 7.05 (dd, *J* = 8.8, 2.3 Hz, 3H), 6.85 - 6.79 (m, 2H), 6.78 - 6.68 (m, 2H), 5.07 (s, 2H), 4.99 - 4.89 (m, 1H), 4.63 (s, 2H), 4.07 (t, *J* = 7.5 Hz, 2H), 3.43 (d, *J* = 20.5 Hz, 6H), 3.25 (d, *J* = 11.7 Hz, 2H), 2.94 - 2.68 (m, 4H), 2.55 (t, *J* = 5.2 Hz, 6H), 2.35 - 2.20 (m, 6H), 2.17 - 2.09 (m, 2H), 1.87 (d, *J* = 11.9 Hz, 3H), 1.62 (s, 6H), 1.37 - 1.17 (m, 8H). LC/MS (ESI+) calcd for C₅₀H₆₂N₈O₈S ([M+H]⁺) *m*/*z* 935, found 935.

### Example 7: N-(4-((4-(2-(4-((7-(3-(4-(2-(2,6-dioxopiperidin-3-yl)-1,3- dioxoisoindolin-5-yl)piperazin-1-yl)propoxy)heptyl)oxy)phenyl)propan-2-yl)phenoxy)methyl)pyrimidin-2-yl)methanesulfonamide

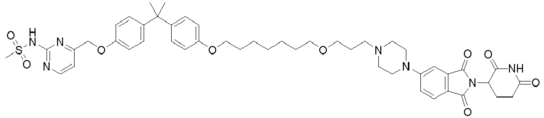

The target compound was synthesized using a method similar to that of Example 1. ¹H NMR (400 MHz, Chloroform-d) *δ* 8.55 (d, *J* = 5.0 Hz, 1H), 7.68 (dd, *J* = 8.5, 4.2 Hz, 1H), 7.28 (d, *J* = 2.4 Hz, 1H), 7.23 - 7.10 (m, 3H), 7.05 (dd, *J* = 7.7, 5.7 Hz, 3H), 6.91 - 6.78 (m, 2H), 6.78 - 6.64 (m, 2H), 5.05 (s, 2H), 4.94 (dd, *J* = 12.2, 5.3 Hz, 1H), 4.10 (q, *J* = 7.7 Hz, 2H), 3.57 - 3.32 (m, 11H), 2.94 - 2.47 (m, 12H), 2.13 (ddd, *J* = 11.9, 9.3, 5.6 Hz, 1H), 1.89 - 1.69 (m, 4H), 1.65-1.50 (m, 9H), 1.40-1.30 (m, 2H). LC/MS (ESI+) calcd for C₄₈H₅₉N₇O₉S ([M+H]⁺) *m*/*z* 910, found 910.

### Example 8: N-(4-((4-(2-(4-((6-(4-(4-(2-(2,6-dioxopiperidin-3-yl)-1,3- dioxoisoindolin-5-yl)piperazin-1-yl)butoxy)hexyl)oxy)phenyl)propan-2-yl)phenoxy)methyl)pyrimidin-2-yl)methanesulfonamide

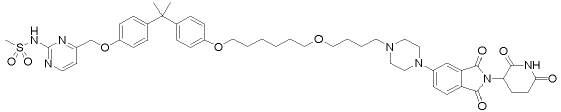

The target compound was synthesized using a method similar to that of Example 1. ¹H NMR (400 MHz, Chloroform-d) *δ* 8.55 (d, *J=* 5.1 Hz, 1H), 7.68 (dd, J = 8.5, 4.7 Hz, 1H), 7.29 (d, *J* = 3.6 Hz, 1H), 7.22 - 7.10 (m, 3H), 7.05 (dd, *J* = 7.7, 5.7 Hz, 3H), 6.93 - 6.78 (m, 2H), 6.76 - 6.62 (m, 2H), 5.06 (s, 2H), 4.94 (dd, J = 12.0, 5.5 Hz, 2H), 4.10 (t, *J* = 7.7 Hz, 2H), 3.56 - 3.32 (m, 13H), 2.94 - 2.58 (m, 10H), 2.18 - 2.02 (m, 2H), 1.76 (tq, *J* = 14.2, 6.6 Hz, 2H), 1.60 - 1.52 (m, 6H), 1.47 - 1.30 (m, 5H). LC/MS (ESI+) calcd for C₄₈H₅₉N₇O₉S ([M+H]⁺) *m*/*z* 910, found 910.

### Example 9: N-(4-((4-(2-(4-((5-((2-(2,6-dioxopiperidin-3-yl)-1,3- dioxoisoindolin-5-yl)oxy)pentyl)oxy)phenyl)propan-2-yl)phenoxy)methyl)pyrimidin-2-yl)methanesulfonamide

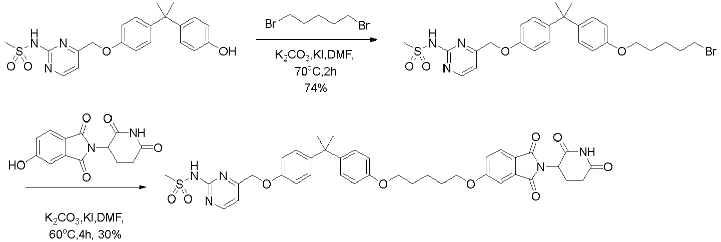

### Step 1: Synthesis of N-(4-((4-(2-(4-((5-bromopentyl)oxy)phenyl)propan-2-yl) phenoxy)methyl)pyrimidin-2-yl)methanesulfonamide

Compound *N-*(4-((4-(2-(4-hydroxylphenyl)propan-2-yl)phenoxy)methyl) pyrimidin-2-yl)methanesulfonamide (140 mg, 0.34mmol) and 1,5-dibromopentane (85 mg, 0.37 mmol) were added into 4 mL of DMF, to which were added K₂CO₃ (118 mg, 0.85 mmol) and KI (10 mg, 0.06 mmol), and then the mixture was stirred at 70 °C for 2 h. The reaction solution was cooled to room temperature, followed by addition of a small amount of water. The resultant solution was extracted with EA for three times. The organic phase was combined, washed with saturated NaCl solution for three times, dried with anhydrous Na₂SO₄, and rotatory evaporated. The residue was purified by column chromatography, to obtain the target compound (142 mg, yield 74%). LC/MS (ESI+) calcd for C₂₆H₃₂BrN₃O₄S⁺ (M + H⁺) *m*/*z* 564.1; found, 564.1.

### Step 2: Synthesis of N-(4-((4-(2-(4-((5-((2-(2,6-dioxopiperidin-3-yl)- 1,3-dioxoisoindolin-5-yl)oxy)pentyl)oxy)phenyl)propan-2-yl)phenoxy)methyl)pyrimidin-2-yl)methanesulfonamide

Compound *N*-(4-((4-(2-(4-((5-bromopentyl)oxy)phenyl)propan-2-yl)phenoxy) methyl)pyrimidin-2-yl)methanesulfonamide (70 mg, 0.13 mmol) and 2-(2,6-dioxopiperidin-3-yl)-5-hydroxyisoindolin-1,3-dione (35 mg, 0.13 mmol) were added into 3 mL of DMF, to which were added K₂CO₃ (45 mg, 0.33 mmol) and KI (10 mg, 0.06 mmol), and then the mixture was stirred at 60 °C for 4 h. The reaction solution was cooled to room temperature, followed by addition of a small amount of water. The resultant solution was extracted with EA for three times. The organic phase was combined, washed with saturated NaCl solution for three times, dried with anhydrous Na₂SO₄, and rotatory evaporated. The residue was purified by column chromatography, to obtain the target compound as yellow solids (29 mg, yield 30%). ¹H NMR (400 MHz, DMSO-*d*₆) *δ* 11.09 (s, 1H), 9.16 (s, 1H), 8.67 (t, *J* = 4.0 Hz, 1H), 7.81 (d, *J* = 8.3 Hz, 1H), 7.41 (d, *J* = 2.3 Hz, 1H), 7.32 (dd, *J* = 8.3, 2.3 Hz, 1H), 7.24 (d, *J* = 5.1 Hz, 1H), 7.13 - 7.07 (m, 2H), 7.01 - 6.94 (m, 2H), 6.93 - 6.86 (m, 2H), 6.67 - 6.60 (m, 2H), 5.18 - 5.08 (m, 3H), 4.16 (t, *J* = 6.4 Hz, 2H), 3.98 (dt, *J* = 18.0, 7.6 Hz, 2H), 3.52 (d, *J* = 5.6 Hz, 3H), 2.64 - 2.56 (m, 1H), 2.09 - 1.98 (m, 1H), 1.73 (ddt, *J* = 23.1, 15.8, 7.9 Hz, 4H), 1.54 (s, 6H), 1.44 (d, *J* = 7.5 Hz, 2H), 1.34 - 1.10 (m, 2H). LC/MS (ESI+) calcd for C₃₉H₄₁N₅O₉S (M + H⁺) *m*/*z* 756.2; found, 756.2.

### Example 10: N-(4-((4-(2-(4-(4-((2-(2,6-dioxopiperidin-3-yl)-1,3-dioxoisoindolin- 4-yl)amino)butoxy)phenyl)propan-2-yl)phenoxy)methyl)pyrimidin-2-yl)methanesulfonamide

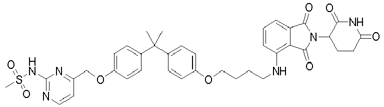

The title compound was synthesized using a method similar to that of Example 1. ¹H NMR (400 MHz, DMSO-*d*₆) *δ* 11.10 (s, 1H), 9.16 (s, 1H), 8.67 (d, *J* = 5.1 Hz, 1H), 7.55 (dd, *J* = 8.6, 7.1 Hz, 1H), 7.24 (d, *J* = 5.1 Hz, 1H), 7.13 - 7.05 (m, 3H), 7.03 - 6.94 (m, 3H), 6.92 - 6.85 (m, 2H), 6.67 - 6.61 (m, 2H), 6.57 (t, *J* = 6.0 Hz, 1H), 5.13 (s, 2H), 5.04 (dd, *J* = 12.9, 5.4 Hz, 1H), 4.01 (t, *J* = 7.4 Hz, 2H), 3.52 (s, 3H), 2.86 (ddd, *J* = 17.8, 13.8, 5.3 Hz, 1H), 2.56 (d, *J* = 17.6 Hz, 1H), 2.04 - 1.95 (m, 1H), 1.81 - 1.70 (m, 2H), 1.54 (s, 9H), 1.23 (s, 2H). LC/MS (ESI+) calcd for C₃₈H₄₀N₆O₈S (M + H⁺) *m*/*z* 741.2; found, 741.2.

### Example 11: N-(4-((4-(2-(4-(3-(4-((1-((1-(2-(2,6-dioxopiperidin-3-yl)-1,3- dioxoisoindolin-5-yl)azetidin-3-yl)methyl)piperidin-4-yl)methyl)piperazin-1-yl)propoxy)phenyl)propan-2-yl)phenoxy)methyl)pyrimidin-2-yl)methanesulfonamide

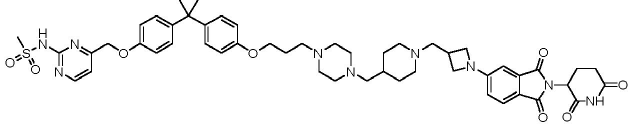

The target compound was synthesized using a method similar to that of Example 1. ¹H NMR (400 MHz, DMSO-*d*₆) *δ* 11.08 (s, 1H), 9.18 (s, 1H), 8.67 (d, *J* = 5.1 Hz, 1H), 7.60 (dd, *J* = 17.1, 8.3 Hz, 1H), 7.24 (d, *J* = 5.1 Hz, 1H), 7.11 (d, *J* = 8.6 Hz, 2H), 6.97 (d, *J* = 8.5 Hz, 2H), 6.90 (d, *J* = 8.7 Hz, 2H), 6.76 (s, 1H), 6.64 (d, *J* = 8.6 Hz, 3H), 5.12 (s, 2H), 5.04 (dd, *J* = 12.9, 5.4 Hz, 1H), 4.16 - 3.92 (m, 5H), 3.65 (dd, *J* = 8.4, 5.6 Hz, 3H), 2.95 - 2.76 (m, 5H), 2.58 (dd, *J* = 10.8, 5.5 Hz, 3H), 2.29 (dd, *J* = 17.2, 10.0 Hz, 10H), 2.15 - 1.86 (m, 8H), 1.80 (p, *J* = 7.3 Hz, 3H), 1.62 (d, *J* = 12.4 Hz, 2H), 1.55 (s, 6H). LC/MS (ESI+) calcd for C₅₁H₆₃N₉O₈S (M + H⁺) m/z 962.4; found, 962.4.

### Example 12: N-(4-(2-(4-((1-((1-((1-((2-(2,6-dioxopiperidin-3-yl)-1,3- dioxoisoindolin-5-yl)azetidin-3-yl)methyl)piperidin-4-yl)methyl)piperidin-4-yl)oxy)phenyl)propan-2-yl)phenoxy)methyl)pyrimidin-2-yl)methanesulfonamide

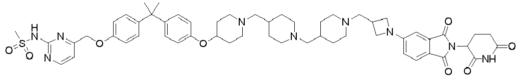

The target compound was synthesized using a method similar to that of Example 1. ¹H NMR (400 MHz, DMSO-*d*₆) *δ* 8.29 (d, *J* = 4.9 Hz, 1H), 7.09 (dd, *J* = 15.7, 8.6 Hz, 5H), 6.87 (d, *J* = 9.0 Hz, 3H), 6.80 (d, *J* = 8.5 Hz, 2H), 6.64 (td, *J* = 12.9, 11.8, 6.2 Hz, 3H), 4.90 (s, 2H), 4.32 - 4.03 (m, 6H), 3.92 (t, *J* = 7.5 Hz, 3H), 2.96 (d, *J* = 3.1 Hz, 3H), 2.78 (dq, *J* = 17.8, 8.5, 7.9 Hz, 10H), 2.63 (d, *J* = 10.1 Hz, 4H), 2.16 - 2.03 (m, 9H), 1.99 (dt, *J* = 13.9, 7.0 Hz, 4H), 1.88 (s, 10H), 1.78 (d, *J* = 10.9 Hz, 4H). LC/MS (ESI+) calcd for C₅₅H₆₉N₉O₈S (M + H⁺) *m*/*z* 1016.5; found,1016.5.

### Example 13: N-(4-((4-(2-(4-((1-(4-(4-(4-)((2-(2,6-dioxopiperidin-3-yl)-1,3- dioxoisoindolin-5-yl)amino)butoxy)butyl)pyrrolidin-3-yl)methoxy)phenyl)propan-2-yl)phenoxy)methyl)pyrimidin-2-yl)methanesulfonamide

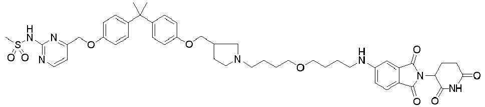

The target compound was synthesized using a method similar to that of Example 1. ¹H NMR (400 MHz, DMSO-*d*₆) *δ* 11.08 (s, 1H), 9.18 (s, 1H), 8.69 (d, *J* = 5.1 Hz, 1H), 7.55 (d, *J* = 8.3 Hz, 1H), 7.26 (d, *J* = 5.1 Hz, 1H), 7.15 - 7.08 (m, 3H), 6.98 (d, *J* = 8.6 Hz, 2H), 6.94 (d, *J* = 2.1 Hz, 1H), 6.92 - 6.88 (m, 2H), 6.84 (dd, *J* = 8.4, 2.1 Hz, 1H), 6.66 - 6.61 (m, 2H), 5.15 (s, 2H), 5.03 (dd, *J* = 12.9, 5.4 Hz, 1H), 4.05 - 3.94 (m, 2H), 3.53 (s, 3H), 3.16 (q, *J* = 6.1 Hz, 3H), 2.76 - 2.53 (m, 5H), 1.99 (tt, *J* = 9.9, 6.0 Hz, 2H), 1.70 - 1.37 (m, 19H), 1.23 (d, *J* = 3.5 Hz, 4H). LC/MS (ESI+) calcd for C₄₇H₅₇N₇O₉S (M + H⁺) *m*/*z* 896.4; found 896.4

### Example 14: N-(4-(4-(2-(4-((1-(5-(4-)((2-(2,6-dioxopiperidin-3-yl)-1,3- dioxoisoindolin-5-yl)amino)butoxy)pentyl)azetidin-3-yl)oxy)phenyl)propan-2-yl)phenoxy)methyl)pyrimidin-2-yl)methanesulfonamide

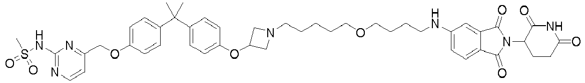

The target compound was synthesized using a method similar to that of Example 1. ¹H NMR (400 MHz, DMSO-*d*₆) *δ* 11.06 (s, 1H), 8.60 (d, *J* = 5.1 Hz, 1H), 7.55 (d, *J* = 8.4 Hz, 1H), 7.19 - 7.04 (m, 7H), 6.96 - 6.88 (m, 3H), 6.84 (dd, *J* = 8.4, 2.1 Hz, 1H), 6.70 (d, *J* = 8.7 Hz, 2H), 5.09 (s, 2H), 5.02 (dd, *J* = 12.9, 5.4 Hz, 1H), 4.70 (p, *J* = 5.7 Hz, 1H), 3.79 - 3.69 (m, 2H), 3.50 (s, 3H), 3.17 (d, *J* = 5.8 Hz, 4H), 3.00 - 2.79 (m, 5H), 2.44 (q, *J* = 5.8, 5.3 Hz, 3H), 1.98 (ddd, *J* = 11.5, 6.3, 3.7 Hz, 2H), 1.68 - 1.39 (m, 16H). LC/MS (ESI+) calcd for C₄₆H₅₅N₇O₉S (M + H⁺) *m*/*z* 882.4; found, 882.4

### Example 15: N-(4-(4-(2-(4-((1-(5-(4-)((2-(2,6-dioxopiperidin-3-yl)-1,3- dioxoisoindolin-5-yl)amino)butoxy)pentyl)piperidin-4-yl)oxy)phenyl)propan-2-yl)phenoxy)methyl)pyrimidin-2-yl)methanesulfonamide

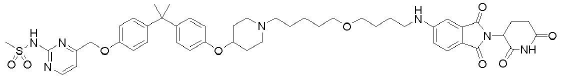

The target compound was synthesized using a method similar to that of Example 1. ¹H NMR (400 MHz, DMSO-*d*₆) *δ* 8.54 (d, *J* = 5.1 Hz, 1H), 7.54 (d, *J* = 8.3 Hz, 1H), 7.10 (dt, *J* = 17.1, 7.4 Hz, 7H), 6.97 - 6.87 (m, 4H), 6.82 (t, *J* = 10.0 Hz, 3H), 5.04 (d, *J* = 6.7 Hz, 2H), 3.26 (s, 5H), 3.15 (d, *J* = 4.7 Hz, 4H), 2.89 (q, *J* = 7.2 Hz, 6H), 2.76 (dd, *J* = 22.8, 13.0 Hz, 4H), 2.54 (s, 6H), 2.35 (dt, *J* = 19.4, 8.7 Hz, 6H), 1.95 (dq, *J* = 23.8, 7.1 Hz, 5H), 1.46 (p, *J* = 7.0 Hz, 6H). LC/MS (ESI+) calcd for C₄₈H₅₉N₇O₉S (M + H⁺) *m*/*z* 910.4; found 910.4.

### Example 16: N-(4-(4-(2-(4-((1-(3-(4-)((2-(2,6-dioxopiperidin-3-yl)-1,3-dioxoisoindolin-5-yl)amino)butoxy)propyl)piperidin-4-yl)methoxy)phenyl) propan-2-yl)phenoxy)methyl)pyrimidin-2-yl)methanesulfonamide

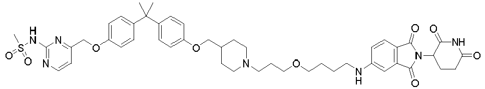

The target compound was synthesized using a method similar to that of Example 1. ¹H NMR (400 MHz, DMSO-*d*₆) *δ* 8.67 (d, *J* = 5.1 Hz, 1H), 7.59 - 7.44 (m, 1H), 7.24 (d, *J* = 5.1 Hz, 1H), 7.10 (d, *J* = 8.4 Hz, 3H), 6.96 (t, *J* = 9.0 Hz, 3H), 6.89 (d, *J* = 8.2 Hz, 2H), 6.64 (d, *J* = 8.3 Hz, 2H), 5.14 (s, 2H), 3.89 (d, *J* = 7.1 Hz, 3H), 3.16 (d, *J* = 5.6 Hz, 3H), 2.98 - 2.83 (m, 4H), 2.61 - 2.34 (m, 10H), 2.09 - 1.88 (m, 4H), 1.65 (t, *J* = 7.2 Hz, 2H), 1.56 (d, *J* = 16.9 Hz, 13H). LC/MS (ESI+) calcd for C₄₇H₅₇N₇O₉S (M + H⁺) m/z 896.4; found 896.4.

### Example 17: N-(4-((4-(2-(4-(3-(3-((2-(2,6-dioxopiperidin-3-yl)-1,3- dioxoisoindolin-4-yl)amino)propoxy)phenyl)propan-2-yl)phenoxy)methyl) pyrimidin-2-yl)methanesulfonamide

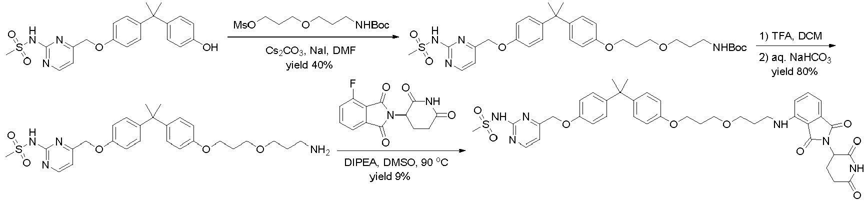

### Step 1: Synthesis of tert-butyl (3-(3-(4-(2-(4-(2-(methanesulfonamido)pyrimidin-4-yl) methoxy)phenyl)propan-2-yl)phenoxy)propoxy)carbamate

N (4-((4-(2-(4-hydroxylphenyl)propan-2-yl)phenoxy)methyl)pyrimidin-2-yl)methanesulfonamide (206 mg, 0.5 mmol), 3-(3-((tert-butoxycarbonyl)amino) propoxy)propyl methanesulfonate (155 mg, 0.5 mmol), cesium carbonate (325 mg, 1.0 mmol) and NaI (7.5 mg, 0.05 mmol) were successively added into 4 mL of DMF, and then the mixture was heated to 60 °C and reacted overnight. After completion of the reaction detected by TLC, the reaction solution was naturally cooled to room temperature, and extracted with 4 mL of ethyl acetate. The organic phase was washed with saturated brine (3*4 mL), dried with anhydrous Na₂SO₄, and concentrated. The residue was separated and purified by column chromatography, to provide 125 mg of target compound, with a yield of 40%.

The target compound was synthesized using a method similar to that of Example 1 in the following steps. ¹H NMR (400 MHz, CDCl₃) *δ* 8.55 (d, *J* = 5.1 Hz, 1H), 8.05 (s, 1H), 7.47 (dd, *J* = 8.5, 7.1 Hz, 1H), 7.19 (d, *J* = 5.0 Hz, 1H), 7.17 - 7.10 (m, 2H), 7.06 (d, *J* = 8.1 Hz, 2H), 6.92 (d, *J* = 8.6 Hz, 1H), 6.83 (d, *J* = 8.9 Hz, 2H), 6.79 - 6.65 (m, 2H), 6.48 (s, 1H), 5.05 (s, 2H), 4.90 (dd, *J* = 12.1, 5.3 Hz, 1H), 4.28 - 4.17 (m, 2H), 3.56 - 3.46 (m, 5H), 3.40 (s, 2H), 3.11 (d, *J* = 37.0 Hz, 2H), 2.91 - 2.68 (m, 3H), 2.22 (t, *J* = 7.7 Hz, 1H), 2.07 (dd, *J* = 13.2, 6.1 Hz, 2H), 1.96 - 1.85 (m, 2H). LC/MS (ESI+) calcd for C₄₀H₄₄N₆O₉S ([M+H]⁺) *m*/*z:* 785.3; found 785.3.

### Example 18: N-(4-((4-(2-(4-(3-(3-((2-(2,6-dioxopiperidin-3-yl)-1,3- dioxoisoindolin-5-yl)amino)propoxy)phenyl)propan-2-yl)phenoxy)methyl) pyrimidin-2-yl)methanesulfonamide

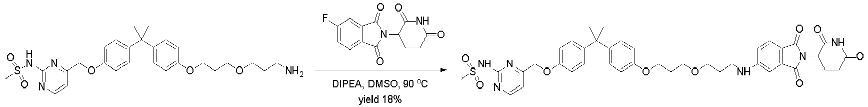

*N*-(4-((4-(2-(4-(3-(3-aminopropoxy)propoxy)phenyl)propan-2-yl)phenoxy)methyl)pyrimidin-2-yl)methanesulfonamide (41 mg, 0.075 mmol), 2-(2,6-dioxopiperidin- 3-yl)-5-fluoroisoindolin-1,3-dione (18 mg, 0.075 mmol) and DIPEA (30 mg, 0.23 mmol) were successively added into 5 mL of DMSO. The mixture was heated to 90 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography, to provide 11 mg of target compound, with a yield of 18%. ¹H NMR (400 MHz, CDCl₃) *δ* 8.56 (d, *J* = 5.1 Hz, 1H), 8.06 (s, 1H), 7.58 (d, *J* = 8.3 Hz, 1H), 7.20 (d, *J* = 5.1 Hz, 1H), 7.16 - 7.10 (m, 2H), 7.10 - 7.02 (m, 2H), 6.98 (d, *J* = 2.2 Hz, 1H), 6.85 - 6.79 (m, 2H), 6.79 - 6.74 (m, 1H), 6.74 - 6.68 (m, 2H), 5.06 (s, 2H), 4.91 (dd, *J* = 12.1, 5.3 Hz, 1H), 4.27 - 4.17 (m, 2H), 3.55 (dt, *J* = 8.3, 5.8 Hz, 4H), 3.51 (s, 3H), 3.34 (t, *J* = 6.1 Hz, 2H), 2.89 - 2.66 (m, 3H), 2.14 - 2.03 (m, 3H), 1.90 (q, *J* = 5.8 Hz, 3H), 1.62 (s, 6H). LC/MS (ESI+) calcd for C₄₀H₄₄N₆O₉S ([M+H]⁺) *m*/*z:* 785.3; found 785.3.

### Example 19: N-(4-((4-(2-(4-(3-(3-(3-((2-(2,6-dioxopiperidin-3-yl)-1,3-dioxoisoindolin-4-yl)amino)propoxy)propoxy)propoxy)phenyl)propan-2-yl)phenoxy)methyl)pyrimidin-2-yl)methanesulfonamide

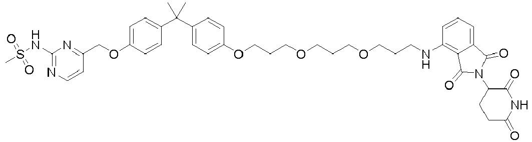

The target compound was synthesized by a method similar to that of Example 17. ¹H NMR (400 MHz, CDCl₃) *δ* 8.55 (d, *J* = 5.0 Hz, 1H), 8.22 (s, 1H), 7.47 (dd, *J* = 8.5, 7.1 Hz, 1H), 7.19 (d, *J* = 5.1 Hz, 1H), 7.17 -7.11 (m, 2H), 7.08 - 7.04 (m, 2H), 6.90 (d, *J* = 8.6 Hz, 1H), 6.87 - 6.80 (m, 2H), 6.76 - 6.67 (m, 2H), 6.44 (s, 1H), 5.05 (s, 2H), 4.90 (dd, *J* = 12.0, 5.3 Hz, 1H), 4.27 - 4.13 (m, 2H), 3.57 - 3.44 (m, 11H), 3.37 (q, *J* = 6.3 Hz, 2H), 2.93 - 2.64 (m, 3H), 2.11 (dt, *J* = 10.4, 3.5 Hz, 1H), 2.01 (p, *J* = 6.3 Hz, 2H), 1.87 (dp, *J* = 18.4, 6.1 Hz, 5H), 1.61 (s, 6H). LC/MS (ESI+) calcd for C₄₃H₅₀N₆O₁₀S ([M+H]⁺) *m*/*z:* 843.3; found 843.3.

### Example 20: N-(4-((4-(2-(4-(3-(3-(3-((2-(2,6-dioxopiperidin-3-yl)-1,3- dioxoisoindolin-5-yl)amino)propoxy)propoxy)propoxy)phenyl)propan-2-yl) phenoxy)methyl)pyrimidin-2-yl)methanesulfonamide

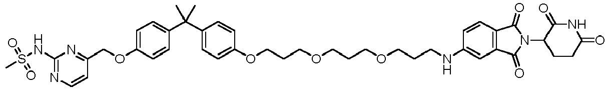

The target compound was synthesized using a method similar to that of Example 1. ¹H NMR (400 MHz, CDCl₃) *δ* 8.55 (d, *J* = 5.0 Hz, 1H), 8.13 (s, 1H), 7.57 (d, *J* = 8.3 Hz, 1H), 7.19 (d, *J* = 5.0 Hz, 1H), 7.16 - 7.11 (m, 2H), 7.09 - 7.02 (m, 2H), 6.93 (d, *J* = 2.1 Hz, 1H), 6.86 - 6.80 (m, 2H), 6.78 - 6.63 (m, 3H), 5.05 (s, 2H), 4.91 (dd, *J* = 12.1, 5.2 Hz, 1H), 4.20 (dd, *J* = 8.2, 6.3 Hz, 2H), 3.62 - 3.41 (m, 11H), 3.31 (t, *J* = 6.2 Hz, 2H), 2.94 - 2.69 (m, 3H), 2.15 - 2.07 (m, 1H), 2.07 - 1.98 (m, 2H), 1.87 (dp, *J* = 18.8, 6.1 Hz, 5H), 1.62 (s, 6H). LC/MS (ESI+) calcd for C₄₃H₅₀N₆O₁₀S ( [M+H]⁺ ) *m*/*z:* 843.3; found 843.3.

### Example 21: N-(4-((4-(2-(4-((8-(4-((2-(2,6-dioxopiperidin-3-yl)-1,3- dioxoisoindolin-4-yl)amino)butoxy)octyl)oxy)phenyl)propan-2-yl)phenoxy) methyl)pyrimidin-2-yl)methanesulfonamide

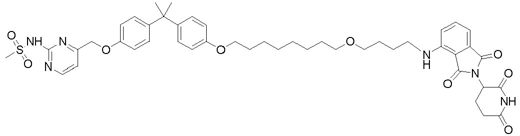

The target compound was synthesized using a method similar to that of Example 1. ¹H NMR (400 MHz, CDCl₃) *δ* 8.55 (d, *J* = 5.0 Hz, 1H), 8.01 (s, 1H), 7.48 (dd, *J* = 8.5, 7.1 Hz, 1H), 7.18 (d, *J* = 5.0 Hz, 1H), 7.14 (d, *J* = 8.7 Hz, 2H), 7.07 (dd, *J* = 7.8, 3.5 Hz, 3H), 6.89 (d, *J* = 8.5 Hz, 1H), 6.83 (d, *J* = 8.8 Hz, 2H), 6.72 (d, *J* = 8.6 Hz, 2H), 6.26 (s, 1H), 5.05 (s, 2H), 4.93 - 4.87 (m, 1H), 4.14 - 4.05 (m, 2H), 3.53 - 3.36 (m, 7H), 3.30 (s, 2H), 2.89 - 2.73 (m, 3H), 2.11 (d, *J* = 5.3 Hz, 1H), 1.75 - 1.69 (m, 4H), 1.62 (s, 6H), 1.42 (d, *J* = 5.0 Hz, 2H), 1.32 (d, *J* = 6.4 Hz, 10H). LC/MS (ESI+) calcd for C₄₆H₅₆N₆O₉S ([M+H]⁺) *m*/*z:* 869.4; found 869.4.

### Example 22: N-(4-((4-(2-(4-((8-(4-((2-(2,6-dioxopiperidin-3-yl)-1,3- dioxoisoindolin-5-yl)amino)butoxy)octyl)oxy)phenyl)propan-2-yl)phenoxy) methyl)pyrimidin-2-yl)methanesulfonamide

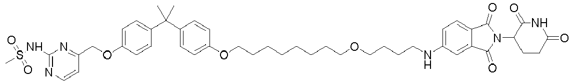

The target compound was synthesized using a method similar to that of Example 1. ¹H NMR (400 MHz, CDCl₃) *δ* 8.55 (d, *J* = 5.1 Hz, 1H), 8.02 (s, 1H), 7.59 (d, *J* = 8.3 Hz, 1H), 7.18 (d, *J* = 5.1 Hz, 1H), 7.14 (d, *J* = 8.8 Hz, 2H), 7.09 - 7.03 (m, 2H), 6.94 (d, *J* = 2.1 Hz, 1H), 6.83 (d, *J* = 8.8 Hz, 2H), 6.79 - 6.65 (m, 3H), 5.05 (s, 2H), 4.92 (dd, *J* = 12.0, 5.1 Hz, 1H), 4.13 - 4.02 (m, 2H), 3.63 (d, *J* = 6.7 Hz, 1H), 3.51 - 3.39 (m, 6H), 3.23 (t, *J* = 6.5 Hz, 2H), 2.89 - 2.67 (m, 3H), 2.15 - 2.08 (m, 1H), 1.73 (dd, *J* = 13.5, 6.4 Hz, 4H), 1.62 (s, 6H), 1.43 - 1.40 (m, 2H), 1.33 - 1.27 (m, 10H). LC/MS (ESI+) calcd for C₄₆H₅₆N₆O₉S ([M+H]⁺) *m*/*z:* 869.4; found 869.4.

### Example 23: N-(4-((4-(2-(4-((7-(3-(3-((2-(2,6-dioxopiperidin-3-yl)-1,3- dioxoisoindolin-4-yl)amino)propoxy)propoxy)heptoxy)phenyl)propan-2-yl) phenoxy)methyl)pyrimidin-2-yl)methanesulfonamide

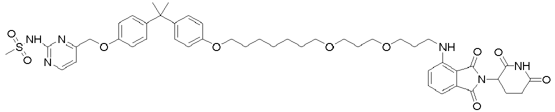

The target compound was synthesized by a method similar to that of Example 17. ¹H NMR (400 MHz, CDCl₃) *δ* 8.55 (d, *J* = 5.0 Hz, 1H), 8.08 (s, 1H), 7.48 (dd, *J* = 8.6, 7.1 Hz, 1H), 7.18 (d, *J* = 5.1 Hz, 1H), 7.16 - 7.11 (m, 2H), 7.11 - 7.02 (m, 3H), 6.91 (d, *J* = 8.5 Hz, 1H), 6.88 - 6.80 (m, 2H), 6.76 - 6.67 (m, 2H), 6.43 (s, 1H), 5.05 (s, 2H), 4.90 (dd, *J* = 12.1, 5.3 Hz, 1H), 4.15 - 4.05 (m, 2H), 3.75 (s, 1H), 3.56 - 3.48 (m, 5H), 3.48 (s, 3H), 3.38 (t, *J* = 6.7 Hz, 4H), 2.92 - 2.71 (m, 3H), 2.16 - 2.08 (m, 1H), 1.96 - 1.86 (m, 4H), 1.62 (s, 6H), 1.54 (s, 2H), 1.42 (d, *J* = 5.1 Hz, 2H), 1.31 - 1.26 (m, 6H). LC/MS (ESI+) calcd for C₄₇H₅₈N₆O₁₀S ( [M+H]⁺ ) *m*/*z:* 899.4; found 899.4.

### Example 24: N-(4-((4-(2-(4-((7-(3-(3-((2-(2,6-dioxopiperidin-3-yl)-1,3- dioxoisoindolin-5-yl)amino)propoxy)propoxy)heptoxy)phenyl)propan-2-yl) phenoxy)methyl)pyrimidin-2-yl)methanesulfonamide

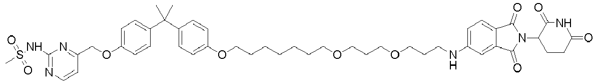

The target compound was synthesized using a method similar to that of Example 1. ¹H NMR (400 MHz, CDCl₃) *δ* 8.55 (d, *J* = 5.0 Hz, 1H), 8.09 (s, 1H), 7.59 (d, *J* = 8.3 Hz, 1H), 7.18 (d, *J* = 5.0 Hz, 1H), 7.16 - 7.10 (m, 2H), 7.10 - 7.02 (m, 2H), 6.94 (d, *J* = 1.9 Hz, 1H), 6.87 - 6.80 (m, 2H), 6.71 (dd, *J* = 8.1, 6.0 Hz, 3H), 5.05 (s, 2H), 4.92 (dd, *J* = 12.0, 5.1 Hz, 1H), 4.14 - 4.03 (m, 2H), 3.57 (t, *J* = 5.4 Hz, 2H), 3.51 (dt, *J* = 8.6, 6.3 Hz, 4H), 3.40 (t, *J* = 6.6 Hz, 2H), 3.32 (t, *J* = 6.2 Hz, 2H), 2.91 - 2.70 (m, 3H), 2.11 (dd, *J* = 8.5, 6.0 Hz, 1H), 1.88 (dq, *J* = 18.9, 6.1 Hz, 4H), 1.62 (s, 6H), 1.56 (d, *J* = 6.6 Hz, 2H), 1.42 (d, *J* = 5.4 Hz, 2H), 1.32 - 1.26 (m, 6H). LC/MS (ESI+) calcd for C₄₇H₅₈N₆O₁₀S ([M+H]⁺) *m*/*z:* 899.4; found 899.4.

### Example 25: N-(4-((4-(2-(4-((8-(4-((2-(2,6-dioxopiperidin-3-yl)-1,3- dioxoisoindolin-5-yl)amino)butoxy)octyl)oxy)phenyl)propan-2-yl) phenoxy)methyl)pyrimidin-2-yl)-N-methyl methanesulfonamide

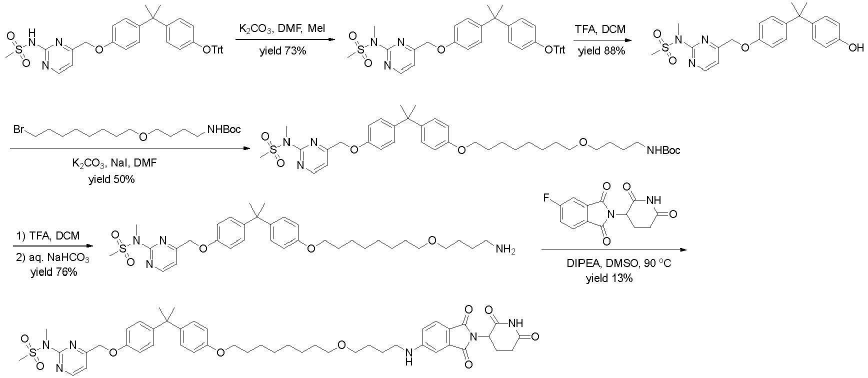

### Step 1: Synthesis of N-methyl-N-(4-((4-(2-(4-(triphenylmethoxy)phenyl) propan-2-yl)phenoxy)methyl)pyrimidin-2-yl)methanesulfonamide

*N*-(4-((4-(2-(4-(triphenylmethoxy)phenyl)propan-2-yl)phenoxy)methyl)pyrimidin-2-yl)methanesulfonamide (328 mg, 1 mmol), CH₃I (142 mg, 1 mmol) and K₂CO₃ (276 mg, 2 mmol) were successively added to 3 mL of DMF, and then the mixture was allowed to react overnight at room temperature. After completion of the reaction detected by TLC, the resultant solution was extracted with 5 mL of ethyl acetate. The organic phase was washed with saturated brine (3*5 mL), dried with anhydrous Na₂SO₄, and concentrated. The residue was separated and purified by column chromatography, to provide 248 mg of target compound, with a yield of 73%.

### Step 2: Synthesis of N-(4-((4-(2-(4-hydroxylphenyl)propan-2-yl)phenoxy)methyl) pyrimidin-2-yl)-N-methyl methanesulfonamide

Compound *N*-methyl-*N*-(4-((4-(2-(4-(triphenylmethoxy)phenyl)propan-2-yl) phenoxy)methyl)pyrimidin-2-yl)methanesulfonamide (248 mg, 0.73 mmol) was dissolved in 4 mL of dichloromethane, to which was added 2 mL of trifluoroacetic acid in an ice bath, and then the mixture was stirred at room temperature for 0.5 h. After completion of the reaction detected by TLC, the reaction system was concentrated. The residue was separated and purified by column chromatography, to provide 136 mg of target compound, with a yield of 88%.

The target compound was synthesized using a method similar to that of Example 1 in the following steps. ¹H NMR (400 MHz, CDCl₃) *δ* 8.57 (d, *J* = 5.0 Hz, 1H), 7.99 (s, 1H), 7.60 (d, *J* = 8.2 Hz, 1H), 7.22 (d, *J* = 5.0 Hz, 1H), 7.18 - 7.06 (m, 4H), 6.95 (s, 1H), 6.87 - 6.75 (m, 4H), 6.75 - 6.68 (m, 1H), 5.07 (s, 2H), 4.92 (dd, *J* = 12.1, 5.2 Hz, 1H), 3.91 (t, *J* = 6.5 Hz, 2H), 3.54 (s, 3H), 3.49 (s, 3H), 3.44 (dt, *J* = 13.2, 6.3 Hz, 4H), 3.24 (t, *J* = 6.4 Hz, 2H), 2.88 - 2.67 (m, 3H), 2.12 (dd, *J* = 8.4, 5.9 Hz, 1H), 1.74 (dq, *J* = 13.8, 6.7 Hz, 8H), 1.63 (s, 6H), 1.42 (d, *J* = 5.0 Hz, 2H), 1.34 (s, 6H). LC/MS (ESI+) calcd for C₄₇H₅₈N₆O₉S ( [M+H]⁺ ) *m*/*z:* 883.4; found 883.4.

### Example 26: N-(4-((4-(2-(4-((8-(4-((2-(2,6-dioxopiperidin-3-yl)-1,3- dioxoisoindolin-5-yl)amino)butoxy)octyl)oxy)phenyl)propan-2-yl)phenoxy)methyl)pyrimidin-2-yl)acetamide

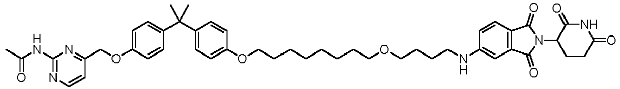

The target compound was synthesized using a method similar to that of Example 1. ¹H NMR (400 MHz, CDCl₃) *δ* 8.54 (d, *J* = 28.7 Hz, 2H), 7.59 (d, *J* = 7.7 Hz, 1H), 7.19 - 7.07 (m, 4H), 6.94 (s, 1H), 6.83 (d, *J* = 8.3 Hz, 2H), 6.78 (d, *J* = 8.6 Hz, 2H), 6.71 (d, *J* = 7.9 Hz, 1H), 5.08 (s, 2H), 4.93 (d, *J* = 11.2 Hz, 1H), 3.91 (t, *J* = 6.4 Hz, 2H), 3.45 (dt, *J* = 13.1, 6.0 Hz, 4H), 3.24 (s, 2H), 2.81 (dt, *J* = 44.5, 16.5 Hz, 3H), 2.47 (s, 3H), 2.11 (d, *J* = 10.9 Hz, 1H), 1.74 (dq, *J* = 13.5, 6.5 Hz, 8H), 1.63 (s, 6H), 1.34 (d, *J* = 6.1 Hz, 6H), 1.30 - 1.28 (m, 2H). LC/MS (ESI+) calcd for C₄₇H₅₆N₆O₈ ([M+H]⁺) *m*/*z:* 833.4; found 833.4.

### Example 27: 5-((4-((8-(4-(2-(4-((2-(1,1-dioxoisothiazolidin-2-yl)pyrimidin-4-yl) methoxy)phenyl)propan-2-yl)phenoxy)octyl)oxy)butyl)amino)-2-(2,6-dioxopiperidin-3-yl)isoindolin-1,3-dione

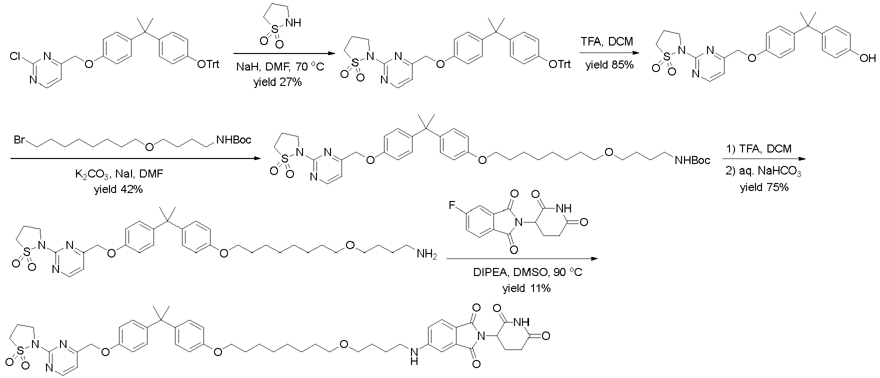

### Step 1: 2-(4-((4-(2-(4-(triphenylmethoxy)phenyl)propan-2-yl)phenoxy)methyl) pyrimidin-2-yl)isothiazolidine 1,1-dioxide

60% NaH (80 mg, 2 mmol) was dissolved in 10 mL of DMF, to which was added 1,1-dioxoisothiazolidine (242 mg, 2 mmol) in an ice bath, and then the mixture was stirred at room temperature for 10 min, followed by addition of 2-chloro-4-((4-(2-(4-(triphenylmethoxy)phenyl)propan-2-yl)phenoxy)methyl)pyrimidine (1.2 g, 2 mmol). The reaction solution was slowly heated to 70 °C and reacted overnight. After completion of the reaction detected by TLC, the reaction was quenched with saturated NH₄Cl solution in an ice bath, and the resultant solution was extracted thrice with 10 mL of ethyl acetate. The organic phase was washed with saturated brine (3*4 mL), dried with anhydrous Na₂SO₄, and concentrated. The residue was separated and purified by column chromatography, to provide 370 mg of target compound, with a yield of 27%.

The target compound was synthesized using a method similar to that of Example 1 in the following steps. ¹H NMR (400 MHz, CDCl₃) *δ* 8.56 (d, *J* = 5.1 Hz, 1H), 8.05 (s, 1H), 7.59 (d, *J* = 8.3 Hz, 1H), 7.20 (d, *J* = 5.1 Hz, 1H), 7.17 - 7.06 (m, 4H), 6.94 (d, *J* = 2.0 Hz, 1H), 6.87 - 6.75 (m, 4H), 6.72 (dd, *J* = 8.4, 2.0 Hz, 1H), 5.06 (s, 2H), 4.92 (dd, *J* = 12.1, 5.2 Hz, 1H), 4.05 (t, *J* = 6.5 Hz, 2H), 3.91 (t, *J* = 6.5 Hz, 2H), 3.52 - 3.36 (m, 6H), 3.24 (t, *J* = 6.6 Hz, 2H), 2.91 - 2.68 (m, 3H), 2.50 (p, *J* = 7.0 Hz, 2H), 2.14 - 2.08 (m, 1H), 1.73 (dt, *J* = 14.3, 7.0 Hz, 6H), 1.62 (s, 6H), 1.58 (d, *J* = 6.8 Hz, 2H), 1.42 (dd, *J* = 6.6, 4.1 Hz, 2H), 1.34 (s, 6H). LC/MS (ESI+) calcd for C₄₈H₅₈N₆O₉( [M+H]⁺ ) *m*/*z:* 895.4; found 895.4.

### Example 28: 5-((4-((8-(4-(2-(4-((2-aminopyrimidin-4-yl)methoxy)phenyl)propan- 2-yl)phenoxy)octyl)oxy)butyl)amino)-2-(2,6-dioxopiperidin-3-yl)isoindolin-1,3-dione

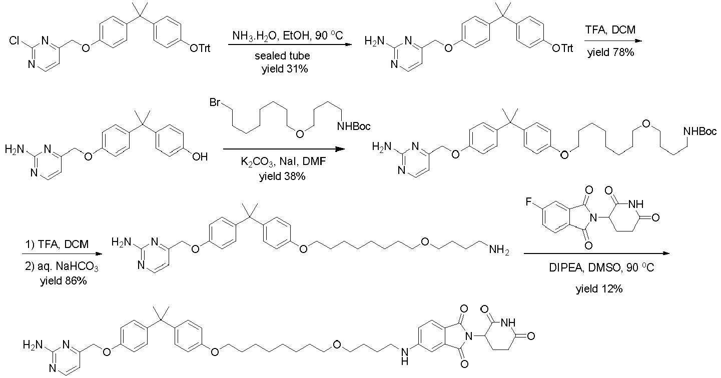

### Step 1: Synthesis of 4-((4-(2-(4-(triphenylmethoxy)phenyl)propan-2-yl)phenoxy) methyl)pyrimidin-2-amine

2-Chloro-4-((4-(2-(4-(triphenylmethoxy)phenyl)propan-2-yl)phenoxy)methyl)pyrimidine (1.8 g, 3 mmol) was dissolved in 5 mL of absolute ethanol, to which was added 2.5 mL of ammonia, and then the mixture was slowly heated to 90 °C and reacted overnight in a closed environment. After completion of the reaction detected by TLC, the resultant solution was extracted thrice with 10 mL of ethyl acetate. The organic phase was washed with saturated brine (3*4 mL), dried with anhydrous Na₂SO₄, and concentrated. The residue was separated and purified by column chromatography, to provide 530 mg of target compound, with a yield of 31%.

The target compound was synthesized using a method similar to that of Example 1 in the following. ¹H NMR (400 MHz, CDCl₃) *δ* 8.65 - 8.03 (m, 2H), 7.59 (d, *J* = 7.3 Hz, 1H), 7.21 - 7.00 (m, 6H), 6.78 (td, *J* = 19.9, 18.3, 8.4 Hz, 7H), 4.94 (d, *J* = 21.6 Hz, 3H), 3.91 (t, *J* = 6.0 Hz, 2H), 3.45 (dt, *J* = 13.1, 6.1 Hz, 4H), 3.24 (s, 2H), 2.83 (dd, *J* = 36.7, 17.3 Hz, 3H), 1.81 - 1.69 (m, 6H), 1.62 (s, 6H), 1.49 - 1.40 (m, 4H), 1.33 (d, *J* = 8.6 Hz, 6H). LC/MS (ESI+) calcd for C₄₅H₅₄N₆O₇ ( [M+H]⁺ ) *m*/*z:* 791.4; found 791.4.

### Example 29: N-(4-((4-(2-(4-(3-(4-((1-(3-((2-(2,6-dioxopiperidin-3-yl)-1,3- dioxoisoindolin-5-yl)amino)propyl)piperidin-4-yl)methyl)piperazin-1-yl)propoxy)phenyl)propan-2-yl)phenoxy)methyl)pyrimidin-2-yl)methanesulfonamide

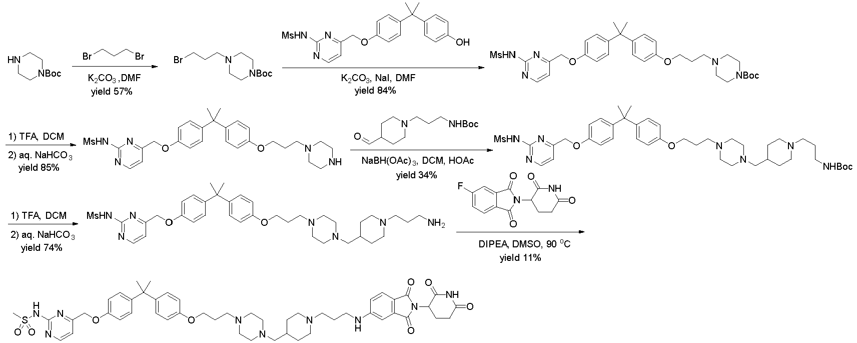

### Step 1: Synthesis of tert-butyl 4-(3-bromopropyl)piperazin-1-carboxylate

tert-butyl piperazin-1-carboxylate (1.8 g, 10 mmol), 1,3-dibromopropane (1.0 g, 5 mmol) and K₂CO₃ (2.76 g, 20 mmol) were successively added into 20 mL of DMF, and then the mixture was allowed to react overnight at room temperature. After completion of the reaction detected by TLC, the resultant solution was extracted with 20 mL of ethyl acetate. The organic phase was washed with saturated brine (3*20 mL), dried with anhydrous Na₂SO₄, and concentrated. The residue was separated and purified by column chromatography, to provide 870 mg of target compound, with a yield of 57%.

### Step 2: Synthesis of tert-butyl 4-(3-(4-(2-(4-(4-((2-(methanesulfonamido)pyrimidin- 4-yl)methoxy)phenyl)propan-2-yl)phenoxy)propyl)piperazin-1-carboxylate

*N*-(4-((4-(2-(4-hydroxylphenyl)propan-2-yl)phenoxy)methyl)pyrimidin-2-yl)methanesulfonamide (413 mg, 1 mmol), tert-butyl 4-(3-bromopropyl)piperazin-1- carboxylate (306 mg, 1 mmol), K₂CO₃ (276 mg, 2 mmol) and NaI (7.5 mg, 0.05 mmol) were successively added into 4 mL of DMF, and then the mixture was heated to 60 °C and reacted overnight. After completion of the reaction detected by TLC, the reaction solution was naturally cooled to room temperature, and then extracted with 4 mL of ethyl acetate. The organic phase was washed with saturated brine (3*4 mL), dried with anhydrous Na₂SO₄, and concentrated. The residue was separated and purified by column chromatography, to provide 539 mg of target compound, with a yield of 84%.

### Step 3: Synthesis of N-(4-((4-(2-(4-(3-(piperazin-1-yl)propoxy)phenyl)propan-2-yl) phenoxy)methyl)pyrimidin-2-yl)methanesulfonamide

Compound tert-butyl 4-(3-(4-(2-(4-(4-((2-(methanesulfonamido)pyrimidin-4-yl) methoxy)phenyl)propan-2-yl)phenoxy)propyl)piperazin-1-carboxylate (539 mg, 0.84 mmol) was dissolved in 10 mL of dichloromethane, to which was added 5 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline and extracted with dichloromethane/methanol (10:1) (3*10 mL). The organic phase was washed with saturated brine, dried with anhydrous Na₂SO₄, and concentrated. The residue was separated and purified by column chromatography, to provide 385 mg of target compound, with a yield of 85%.

### Step 4: Synthesis of tert-butyl (3-(4-(3-(4-(4-(2-(methanesulfonamido)pyrimidin-4-yl) methoxy)phenyl)propan-2-yl)phenoxy)propyl)piperazin-1-yl)methyl)piperidin-1-yl)propyl)carbamate

Compound *N*-(4-((4-(2-(4-(3-(piperazin-1-yl)propoxy)phenyl)propan-2-yl) phenoxy)methyl)pyrimidin-2-yl)methanesulfonamide (385 mg, 0.7 mmol) was dissolved in 10 mL of dichloromethane, to which were successively added tert-butyl (3-(4-formylpiperidin-1-yl)propyl)carbamate (190 mg, 0.7 mmol) and glacial acetic acid (4 mg, 0.07 mmol), and then the mixture was stirred for 10 min at room temperature, followed by addition of sodium triacetoxyborohydride (190 mg, 1.4 mmol), and then the mixture was stirred overnight at room temperature. After completion of the reaction detected by TLC, the reaction system was poured into saturated NH₄Cl solution in an ice bath, and extracted with dichloromethane (3 * 10 mL). The organic phase was washed with saturated brine, dried with anhydrous Na2SO4, and concentrated. The residue was separated and purified by column chromatography, to provide 190 mg of target compound, with a yield of 34%.

### Step 5: Synthesis of N-(4-((4-(2-(4-(3-(3-aminopropyl)piperidin-4-yl) methyl)piperazin-1-yl)propoxy)phenyl)propan-2-yl)phenoxy)methyl)pyrimidin-2-yl)methanesulfonamide

Compound tert-butyl (3-(4-(3-(4-(4-(2-(methanesulfonamido)pyrimidin-4-yl) methoxy)phenyl)propan-2-yl)phenoxy)propyl)piperazin-1-yl)methyl)piperidin-1-yl)propyl)carbamate (190 mg, 0.24 mmol) was dissolved in 10 mL of dichloromethane, to which was added 5 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 124 mg of target compound, with a yield of 74%.

### Step 6: Synthesis of N-(4-((4-(2-(4-(3-(4-((1-(3-((2-(2,6-dioxopiperidin-3-yl)-1,3-dioxoisoindolin-5-yl)amino)propyl)piperidin-4-yl)methyl)piperazin-1-yl)propoxy)phenyl)propan-2-yl)phenoxy)methyl)pyrimidin-2-yl)methanesulfonamide

N (4-((4-(2-(4-(3-(3-aminopropyl)piperidin-4-yl)methyl)piperazin-1-yl)propoxy)phenyl)propan-2-yl)phenoxy)methyl)pyrimidin-2-yl)methanesulfonamide (124 mg, 0.18 mmol), 2-(2,6-dioxopiperidin-3-yl)-5-fluoroisoindolin-1,3-dione (50 mg, 0.18 mmol) and DIPEA(70 mg, 0.54 mmol) were successively added into 5 mL of DMSO, and then the mixture was heated to 60 °C and reacted overnight. The reaction solution was naturally cooled to room temperature, and then extracted with 5 mL of ethyl acetate. The organic phase was washed with saturated brine (3*5 mL), dried with anhydrous Na₂SO₄, and concentrated. The residue was separated and purified by column chromatography to provide 18 mg of target compound, with a yield of 11%. ¹H NMR (400 MHz, CDCl₃) *δ* 8.56 (d, *J* = 5.1 Hz, 1H), 7.58 (d, *J* = 8.2 Hz, 1H), 7.20 (d, *J* = 5.0 Hz, 1H), 7.10 (dd, *J* = 25.5, 8.3 Hz, 4H), 6.92 (s, 1H), 6.80 (dd, *J* = 21.6, 8.4 Hz, 4H), 6.55 (s, 1H), 5.01 (s, 2H), 4.96 - 4.88 (m, 1H), 4.17 (d, *J* = 8.6 Hz, 2H), 3.48 (s, 3H), 3.34 (s, 2H), 3.20 (s, 2H), 2.92 - 2.67 (m, 5H), 2.46 (s, 8H), 2.15 (d, *J* = 34.5 Hz, 6H), 1.99 (s, 4H), 1.84 (d, *J* = 13.3 Hz, 2H), 1.62 (s, 6H), 1.42 (d, *J* = 5.5 Hz, 4H). LC/MS (ESI+) calcd for C₅₀H₆₃N₉O₈S( [M+H]⁺ ) *m*/*z*: 950.5; found 950.5.

### Example 30: N-(4-((4-(2-(4-((8-(6-(2-(2,6-dioxopiperidin-3-yl)-1,3- dioxoisoindolin-5-yl)-2,6-diazaspiro[3.3]heptan-2-yl)octyl)oxy)phenyl)propan-2-yl)phenoxy)methyl)pyrimidin-2-yl)methanesulfonamide

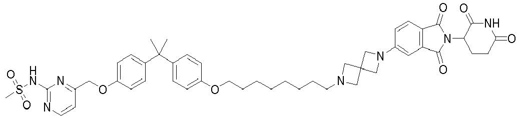

The target compound was synthesized using a method similar to that of Example 29. ¹H NMR (400 MHz, CDCl₃) *δ* 7.66 (s, 1H), 8.54 (d, *J* = 5.0 Hz, 1H), 7.62 (d, *J* = 8.3 Hz, 1H), 7.16 (d, *J* = 5.1 Hz, 1H), 7.15 - 7.01 (m, 4H), 6.95 (s, 1H), 6.87 - 6.76 (m, 2H), 6.76 - 6.66 (m, 2H), 6.46 (dd, *J* = 8.3, 2.1 Hz, 1H), 5.05 (s, 2H), 4.92 (dd, *J* = 12.0, 5.3 Hz, 1H), 4.18 - 3.95 (m, 6H), 3.48 (s, 7H), 2.90 - 2.67 (m, 3H), 2.62 - 2.42 (m, 2H), 2.16 - 2.05 (m, 1H), 1.70 (d, *J* = 7.5 Hz, 2H), 1.61 (s, 6H), 1.43 - 1.30 (m, 4H). LC/MS (ESI+) calcd for C₄₇H₅₅N₇O₈S ( [M+H]⁺) *m*/*z*: 878.4; found 878.4.

### Example 31: N-(6-((4-(2-(4-((8-(4-((2-(2,6-dioxopiperidin-3-yl)-1,3- dioxoisoindolin-5-yl)amino)butoxy)octyl)oxy)phenyl)propan-2-yl)phenoxy)methyl)pyrazin-2-yl)methanesulfonamide

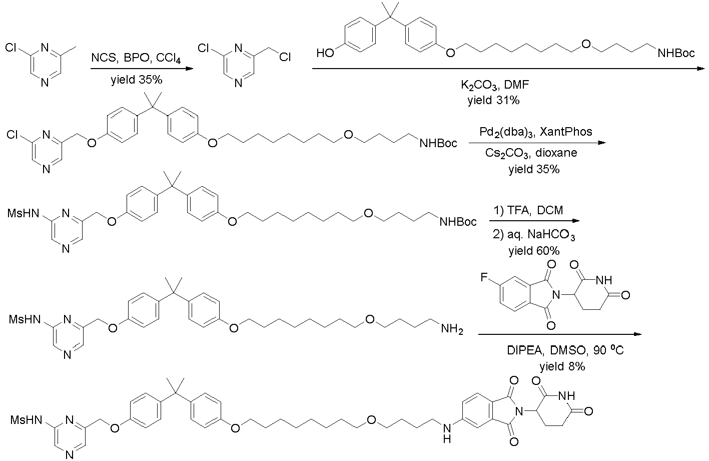

### Step 1: Synthesis of 2-chloro-6-(chloromethyl)pyrazine

2-chloro-6-methylpyrazine (6 g, 50 mmol) was dissolved in 100 ml of CCl4, to which were successively added NCS (6.65 g, 51 mmol) and BPO (1.21 g, 5 mmol), and then the system was purged with nitrogen and refluxed for 24 h under nitrogen atmosphere. The reaction solution was cooled to room temperature, filtered, and concentrated under reduced pressure. The residue was separated and purified by column chromatography, to provide 2.8 g of target compound, with a yield of 35%.

### Step 2: Synthesis of tert-butyl (4-((8-(4-(2-(4-((6-chloropyrazin-2-yl)methoxy)phenyl) propan-2-yl)phenoxy)octyl)oxy)butanecarbamate

tert-butyl (3-((8-(4-(2-(4-hydroxylphenyl)propan-2-yl)phenoxy)octyl)oxy) propyl)carbamate (1.05 g, 2 mmol), 2-chloro-4-(chloromethyl)pyrimidine (320 mg, 2 mmol) and K₂CO₃ (552 mg, 4 mmol) were successively added to 10 mL of DMF, and then the mixture was allowed to react overnight at room temperature. After completion of the reaction detected by TLC, the resultant solution was extracted with 10 mL of ethyl acetate. The organic phase was washed with saturated brine (3*10 mL), dried with anhydrous Na₂SO₄, and concentrated. The residue was separated and purified by column chromatography, to provide 400 mg of target compound, with a yield of 31%.

### Step 3: Synthesis of tert-butyl (4-((8-(4-(2-(4-((6-(methanesulfonamido)pyrazin-2-yl) methoxy)phenyl)propan-2-yl)phenoxy)octyl)oxy)carbamate

Compound tert-butyl (4-((8-(4-(2-(4-((6-chloropyrazin-2-yl)methoxy)phenyl) propan-2-yl)phenoxy)octyl)oxy)butanecarbamate (400 mg, 0.61 mmol), methanesulfonamide (115 mg, 1.22 mmol), Pd₂(dba)₃(55 mg, 0.061 mmol), XantPhos (69 mg, 0.12 mmol), and cesium carbonate (590 mg, 1.8 mmol) were added into 5 mL of dioxane, and then the reaction system was purged with helium and reacted overnight at 110 °C under helium atmosphere. After completion of the reaction detected by TLC, the reaction system was filtered over diatomaceous earth and concentrated. The residue was separated and purified by column chromatography, to provide 150 mg of target compound, with a yield of 35%.

### Step 4: Synthesis of N-(6-((4-(2-(4-((8-(4-aminobutoxy)octyl)oxy)phenyl) propan-2-yl)phenoxy)methyl)pyrazin-2-yl)methanesulfonamide

Compound tert-butyl (4-((8-(4-(2-(4-((6-(methanesulfonamido)pyrazin-2-yl) methoxy)phenyl)propan-2-yl)phenoxy)octyl)oxy)carbamate (150 mg, 0.21 mmol) was dissolved in 10 mL of dichloromethane, to which was added 5 mL trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline and extracted with dichloromethane/methanol (10:1) (3*10 mL). The organic phase was washed with saturated brine, dried with anhydrous Na₂SO₄, and concentrated. The residue was separated and purified by column chromatography, to provide 77 mg of target compound, with a yield of 60%.

### Step 5: Synthesis of N-(6-((4-(2-(4-((8-(4-((2-(2,6-dioxopiperidin-3-yl)-1,3- dioxoisoindolin-5-yl)amino)butoxy)octyl)oxy)phenyl)propan-2-yl)phenoxy)methyl) pyrazin-2-yl)methanesulfonamide

*N*-(6-((4-(2-(4-((8-(4-aminobutoxy)octyl)oxy)phenyl)propan-2-yl)phenoxy)methyl)pyrazin-2-yl)methanesulfonamide (77 mg, 0.12 mmol), 2-(2,6-dioxopiperidin- 3-yl)-5-fluoroisoindolin-1,3-dione (33 mg, 0.12 mmol) and DIPEA (60 mg, 0.45 mmol) were successively added into 5 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 8 mg of target compound, with a yield of 8%. LC/MS (ESI+) calcd for C₄₆H₅₆N₆O₉S( [M+H]⁺ ) *m*/*z:* 869.4; found 869.4.

### Example 32: N-(2-((4-(2-(4-((8-(4-((2-(2,6-dioxopiperidin-3-yl)-1,3- dioxoisoindolin-5-yl)amino)butoxy)octyl)oxy)phenyl)propan-2-yl)phenoxy) methyl)pyrimidin-4-yl)methanesulfonamide

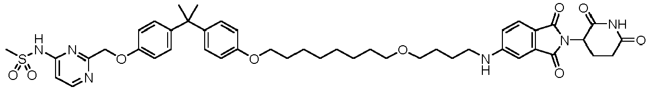

The target compound was synthesized by a method similar to that of Example 31. LC/MS (ESI+) calcd for C₄₆H₅₆N₆O₉S ( [M+H]⁺ ) *m*/*z:* 869.4; found 869.4_{∘}

### Example 33: N-(6-((4-(2-(4-((8-(4-((2-(2,6-dioxopiperidin-3-yl)-1,3- dioxoisoindolin-5-yl)amino)butoxy)octyl)oxy)phenyl)propan-2-yl)phenoxy) methyl)pyridin-2-yl)methanesulfonamide

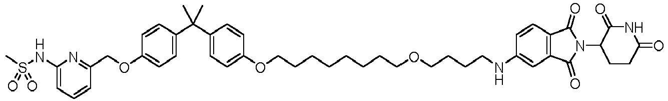

The target compound was synthesized by a method similar to that of Example 31. LC/MS (ESI+) calcd for C₄₇H₅₇N₅O₉S ( [M+H]⁺ ) *m*/*z:* 868.4; found 868.4.

### Example 34: N-(4-((4-(2-(4-((1-(2-(2,6-dioxopiperidin-3-yl)-1,3-dioxoisoindolin- 5-yl)piperidin-4-yl)oxy)phenyl)propan-2-yl)phenoxy)methyl)pyrimidin-2-yl)methanesulfonamide

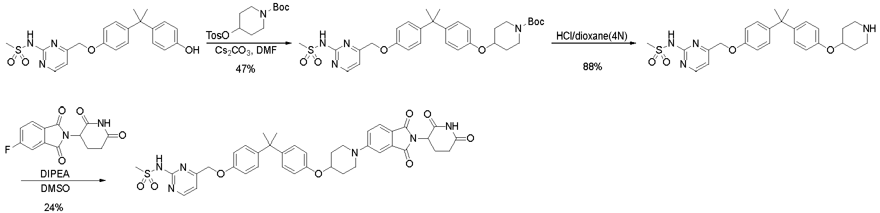

### Step 1: Synthesis of tert-butyl 4-(4-(2-(4-((2-(methanesulfonamido)pyrimidin-4-yl) methoxy)phenyl)propan-2-yl)phenoxy)piperidin-1-carboxylate

Compound *N*-(4-((4-(2-(4-hydroxylphenyl)propan-2-yl)phenoxy)methyl) pyrimidin-2-yl)methanesulfonamide (200 mg, 0.48 mmol) and tert-butyl 4-(p-methylphenoxy)piperidin-1-carboxylate (189 mg, 0.53 mmol) were added into 10 mL of DMF, to which were added Cs₂CO₃ (235 mg, 0.72 mmol) and KI (9 mg, 0.05 mmol), and then the mixture was stirred at 70 °C for 2 h. The reaction solution was cooled to room temperature, to which was added a small amount of water, and then extracted with ethyl acetate for three times. The organic phase was combined, and washed with saturated NaCl solution for three times, dried with anhydrous Na₂SO₄, and rotatory evaporated. The residue was purified by column chromatography, to obtain compound tert-butyl 4-(4-(2-(4-((2-(methanesulfonamido) pyrimidin-4-yl)methoxy)phenyl)propan-2-yl)phenoxy)piperidin-1-carboxylate (134 mg, 0.22 mmol), with a yield of 47%. LC/MS (ESI+) calcd for C₃₁H₄₀N₄O₆S ( [M+H]⁺ ) *m*/*z* 596.74; found 597.2.

### Step 2: Synthesis of N-(4-((4-(2-(4-(piperidin-4-oxy)phenyl)propan-2-yl)phenoxy) methyl)pyrimidin-2-yl)methanesulfonamide

Compound tert-butyl 4-(4-(2-(4-((2-(methanesulfonamido)pyrimidin-4-yl) methoxy)phenyl)propan-2-yl)phenoxy)piperidin-1-carboxylate (134 mg, 0.22 mmol) was added into 2 mL of 1,4-dioxane, and then the solution was stirred in an ice bath, to which was then added 4M solution of HCl in dioxane (2 mL), followed by stirring at room temperature for 15 min. The reaction solution was adjusted to be alkaline by adding saturated NaHCO₃, and then extracted with DCM for three times. The organic phase was washed with saturated NaCl solution, dried with anhydrous Na₂SO₄, and rotatory evaporated, to provide *N*-(4-((4-(2-(4-(piperidin-4-oxy)phenyl)propan- 2-yl)phenoxy)methyl)pyrimidin-2-yl)methanesulfonamide (96 mg, 0.19 mmol), with a yield of 88%. LC/MS (ESI+) calcd for C₂₆H₃₂N₄O₄S ([M+H]⁺) *m*/*z* 496.63; found 497.1.

### Step 3: Synthesis of N-(4-((4-(2-(4-((1-(2-(2,6-dioxopiperidin-3-yl)-1,3- dioxoisoindolin-5-yl)piperidin-4-yl)oxy)phenyl)propan-2-yl)phenoxy)methyl) pyrimidin-2-yl)methanesulfonamide

*N*-(4-((4-(2-(4-(piperidin-4-oxy)phenyl)propan-2-yl)phenoxy)methyl)pyrimidin-2-yl)methanesulfonamide (48 mg, 0.09 mmol) was dissolved in 5 mL of DMSO, to which wre successively added DIPEA (36 mg, 0.28mmol) and 2-(2,6-dioxopiperidin-3-yl)-5-fluoroisoindolin-1,3-dione (28 mg, 0.10 mmol), and then under N₂ protection, the mixture was heated to 60 °C and reacted overnight. The reaction was cooled to room temperature, and quenched by adding water. The resultant solution was extracted with ethyl acetate for three times. The organic phase was washed with saturated NaCl solution, dried with anhydrous Na₂SO₄, concentrated and rotatory evaporated. The residue was separated and purified by prep-TLC, to provide compound *N*-(4-((4-(2-(4-((1-(2-(2,6-dioxopiperidin-3-yl)-1,3- dioxoisoindolin-5-yl)piperidin-4-yl)oxy)phenyl)propan-2-yl)phenoxy)methyl)pyrimidin-2-yl)methanesulfonamide (16 mg, 0.02 mmol), with a yield of 24%. ¹H NMR (400 MHz, DMSO-*d*₆) *δ* 11.09 (s, 1H), 8.44 (d, *J* = 4.9 Hz, 1H), 8.26 (s, 1H), 7.67 (d, *J* = 8.5 Hz, 1H), 7.41 (t, *J* = 7.6 Hz, 1H), 7.16 - 7.07 (m, 5H), 6.98 (d, *J* = 8.2 Hz, 1H), 6.92 - 6.88 (m, 3H), 5.07 (dd, *J* = 12.9, 5.3 Hz, 1H), 5.00 (s, 2H), 4.60 (s, 1H), 3.79 (s, 2H), 3.14 (s, 3H), 2.88 (s, 2H), 2.04 - 1.96 (m, 3H), 1.66 (d, *J* = 7.8 Hz, 3H), 1.58 (d, *J* = 2.6 Hz, 6H), 1.42 - 1.31 (m, 2H). LC/MS (ESI+) calcd for C₃₉H₄₀N₆O₈S ([M+H]⁺) *m*/*z* 752.84; found 753.2.

### Example 35: N-(4-((4-(2-(4-((1-(2-(2,6-dioxopiperidin-3-yl)-1,3-dioxoisoindolin- 4-yl)piperidin-4-yl)oxy)phenyl)propan-2-yl)phenoxy)methyl)pyrimidin-2-yl)methanesulfonamide

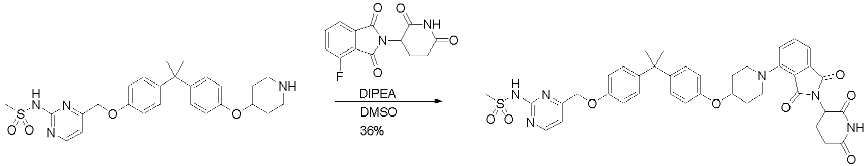

The target compound was synthesized by a method similar to that of Example 34. ¹H NMR (400 MHz, DMSO-*d*₆) *δ* 11.10 (s, 1H), 8.49 (d, *J* = 5.1 Hz, 1H), 8.26 (s, 1H), 7.72 - 7.66 (m, 1H), 7.36 (d, *J* = 4.5 Hz, 1H), 7.12 (t, *J* = 9.4 Hz, 5H), 7.00 (d, *J* = 5.4 Hz, 1H), 6.90 (t, *J* = 7.7 Hz, 3H), 5.10 (dd, *J* = 13.0, 5.3 Hz, 1H), 5.03 (s, 2H), 4.56 (s, 1H), 3.20 (s, 3H), 2.60 (s, 2H), 2.06 (s, 3H), 1.81 (s, 3H), 1.58 (s, 6H), 1.45 - 1.29 (m, 2H). LC/MS (ESI+) calcd for C₃₉H₄₀N₆O₈S ( [M+H]⁺ ) *m*/*z* 752.84; found 753.2.

### Example 36: N-(4-((4-(2-(4-((1-(4-(4-(4-)((2-(2,6-dioxopiperidin-3-yl)-1,3- dioxoisoindolin-5-yl)amino)butoxy)butyl)piperidin-4-yl)oxy)phenyl)propan-2-yl)phenoxy)methyl)pyrimidin-2-yl)methanesulfonamide

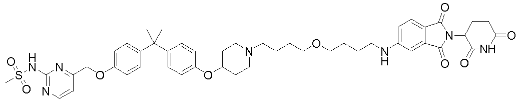

The target compound was synthesized by a method similar to that of Example 34. LC/MS (ESI+) calcd for C₄₇H₅₇N₇O₉S₁ ( [M+H]⁺ ) *m*/*z* 896.07; found 896.8.

### Example 37: N-(4-((4-(2-(4-(3-((2-(2,6-dioxopiperidin-3-yl)-1,3-dioxoisoindolin- 5-yl)amino)propoxy)phenyl)propan-2-yl)phenoxy)methyl)pyrimidin-2-yl)methanesulfonamide

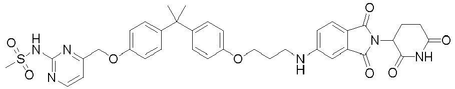

The target compound was synthesized by a method similar to that of Example 34. LC/MS (ESI+) calcd for C₃₇H₃₉N₆O₈S⁺ ([M+H]⁺) *m*/*z:* 727.2; found 727.2. ¹H NMR (400 MHz, CDCl₃) *δ* 9.09 (s, 1H), 8.60 (d, *J* = 5.1 Hz, 1H), 7.50 (d, *J* = 8.4 Hz, 1H), 7.32 (d, *J* = 5.0 Hz, 1H), 7.14 (dd, *J* = 15.0, 8.6 Hz, 4H), 6.96 (d, *J* = 2.0 Hz, 1H), 6.83 (dd, *J* = 17.2, 8.6 Hz, 4H), 6.75 (d, *J* = 8.1 Hz, 1H), 5.16 - 5.03 (m, 2H), 4.92 (dd, *J* = 12.0, 5.3 Hz, 1H), 4.14 - 4.02 (m, 2H), 3.60 - 3.47 (m, 2H), 3.46 (s, 2H), 2.91 (d, *J* = 14.6 Hz, 1H), 2.81 - 2.69 (m, 2H), 2.63 (s, 3H), 2.12 (s, 3H), 1.64 (d, *J* = 2.4 Hz, 6H).

### Example 38: N-(4-((4-(2-(4-((5-((2-(2,6-dioxopiperidin-3-yl)-1,3-dioxoisoindolin- 5-yl)amino)pentyl)oxy)phenyl)propan-2-yl)phenoxy)methyl)pyrimidin-2-yl)methanesulfonamide

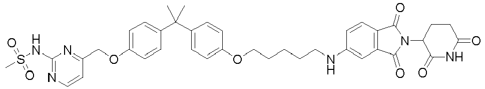

The target compound was synthesized by a method similar to that of Example 34. LC/MS (ESI+) calcd for C₃₉H₄₃N₆O₈S⁺ ([M+H]⁺) *m*/*z:* 755.3; found 755.2. ¹H NMR (400 MHz, CDCl₃) *δ* 8.62 (s, 1H), 8.60 (d, *J* = 5.2 Hz, 1H), 7.60 (d, *J* = 8.2 Hz, 1H), 7.28 (s, 1H), 7.14 (dd, *J* = 11.7, 8.5 Hz, 4H), 6.97 (s, 1H), 6.84 (d, *J* = 8.7 Hz, 2H), 6.79 (d, *J* = 8.7 Hz, 2H), 6.74 (d, *J* = 8.6 Hz, 1H), 5.09 (s, 2H), 4.93 (dd, *J* = 12.0, 5.2 Hz, 1H), 3.96 (t, *J* = 6.1 Hz, 2H), 3.45 (s, 3H), 3.27 (t, *J* = 6.8 Hz, 2H), 2.94 - 2.85 (m, 1H), 2.82 - 2.70 (m, 2H), 2.22 (t, *J* = 7.7 Hz, 1H), 2.12 (d, *J* = 7.0 Hz, 1H), 2.01 (d, *J* = 7.0 Hz, 1H), 1.83 (t, *J* = 6.9 Hz, 2H), 1.73 (s, 2H), 1.63 (s, 6H).

### Example 39: N-(4-((4-(2-(4-((1r,4r)-4-((4-((2-(2,6-dioxopiperidin-3-yl)-1,3- dioxoisoindolin-5-yl)-2,7-diazaspiro [3.5] nonan-7-yl)methyl)piperidin-1-yl)methyl)cyclohexyl)methoxy)phenyl)propan-2-yl)phenoxy)pyrimidin-2-yl)methanesulfonamide

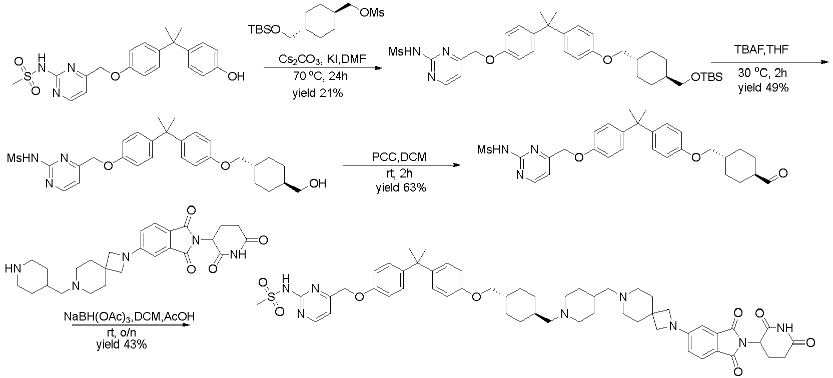

### Step 1: Synthesis of N-(4-((4-(2-(4-((1r,4r)-4-((tert-butyldimethylsilyloxy)methyl) cyclohexyl)methoxy)phenyl)propan-2-yl)phenoxy)methyl)pyrimidin-2-yl)methanesulfonamide

In a 25 mL round-bottom flask, compound N-(4-((4-(2-(4-hydroxylphenyl) propan-2-yl)phenoxy)methyl)pyrimidin-2-yl)methanesulfonamide (300.0 mg, 0.7mmol), Cs₂CO₃ (354.0 mg, 1.1 mmol), and KI (12.0 mg, 0.07 mmol) were added into 4 mL of DMF, and then the mixture was heated to 70 °C and stirred overnight. The reaction solution was cooled to room temperature, and then extracted by adding ethyl acetate and water. The organic layer was washed with saturated brine, dried, and rotatory evaporated. The residue was purified by TLC, to provide compound N-(4-((4-(2-(4-((1r,4r)-4-((tert-butyldimethylsilyloxy)methyl)cyclohexyl)methoxy)phenyl)propan-2-yl)phenoxy)methyl)pyrimidin-2-yl)methanesulfonamide (100.0 mg, 0.15 mmol), with a yield of 21%.

### Step 2: N-(4-((4-(2-(4-((1r,4r)-4-(hydroxylmethyl)cyclohexyl)methoxy)phenyl) propan-2-yl)phenoxy)methyl)pyrimidin-2-yl)methanesulfonamide

In a 25 mL round-bottom flask, compound N-(4-((4-(2-(4-((1r , 4r)-4-((tertbutyldimethylsilyloxy)methyl)cyclohexyl)methoxy)phenyl)propan-2-yl)phenoxy)methyl)pyrimidin-2-yl)methanesulfonamide (100.0 mg, 0.15 mmol) was added into 2 mL of THF, to which was added 0.3 mL of TBAF (1M, 0.3 mmol), and then the mixture was stirred overnight. The reaction was extracted by adding ethyl acetate and water. The organic layer was washed with saturated brine, dried, and rotatory evaporated. The residue was purified by prep.-TLC, to provide compound N-(4-((4-(2-(4-((1r,4r)-4-(hydroxylmethyl)cyclohexyl)methoxy)phenyl)propan-2-yl)phenoxy)methyl)pyrimidin-2-yl)methanesulfonamide (40.0 mg, 0.07 mmol), with a yield of 49%.

### Step 3: Synthesis of N-(4-((4-(2-(4-((1r,4r)-4-formylcyclohexyl)methoxy)phenyl) propan-2-yl)phenoxy)methyl)pyrimidin-2-yl)methanesulfonamide

In a 25 mL round-bottom flask, compound N-(4-((4-(2-(4-((1r,4r)-4-(hydroxylmethyl)cyclohexyl)methoxy)phenyl)propan-2-yl)phenoxy)methyl)pyrimidin-2-yl)methanesulfonamide (40.0 mg, 0.07 mmol) was added into 2 mL of dichloromethane, to which was added PCC (32.0 mg, 0.14 mmol). The reaction was stirred at room temperature for 2 h, and then extracted by adding dichloromethane and water. The organic layer was washed with saturated brine, dried, and rotatory evaporated. The residue was purified by prep.-TLC, to provide compound N-(4-((4-(2-(4-((1r,4r)-4-formylcyclohexyl)methoxy)phenyl)propan-2-yl)phenoxy)methyl)pyrimidin-2-yl)methanesulfonamide (25.0 mg, 0.04 mmol), with a yield of 63%.

### Step 4: Synthesis of N-(4-((4-(2-(4-((1r,4r)-4-((4-((2-(2,6-dioxopiperidin-3-yl)- 1,3-dioxoisoindolin-5-yl)-2,7-diazaspiro[3.5]nonan-7-yl)methyl)piperidin-1-yl)methyl)cyclohexyl)methoxy)phenyl)propan-2-yl)phenoxy)pyrimidin-2-yl)methanesulfonamide

In a 25 mL round-bottom flask, compound N-(4-((4-(2-(4-((1r,4r)-4-formylcyclohexyl)methoxy)phenyl)propan-2-yl)phenoxy)methyl)pyrimidin-2-yl)methanesulfonamide (25.0 mg, 0.04 mmol), 2-(2,6-dioxopiperidin-3-yl)-5-(7-(piperidin- 4-ylmethyl)-2,7-diazaspiro[3.5]nonan-2-yl)isoindol-1,3-dione (22.0 mg, 0.04 mmol), and sodium triacetoxyborohydride (29.0 mg, 0.12 mmol) were added into 2 mL of dichloromethane, to which was added acetic acid (3.0 mg, 0.08 mmol). The reaction was stirred overnight at room temperature, and then extracted by adding dichloromethane and saturated Na2CO3 aqueous solution.. The organic layer was washed with saturated brine, dried, and rotatory evaporated. The residue was purified by prep.-TLC, to provide compound N-(4-((4-(2-(4-((1r,4r)-4-((4-((2-(2,6-dioxopiperidin-3-yl)- 1,3-dioxoisoindolin-5-yl)-2,7-diazaspiro[3.5]nonan-7-yl)methyl)piperidin-1-yl)methyl)cyclohexyl)methoxy)phenyl)propan-2-yl)phenoxy)pyrimidin-2-yl)methanesulfonamide(20 mg, 0.02 mmol), with a yield of 43%. LC/MS (ESI+) calcd for C₅₅H₆₉N₈O₈S⁺ ([M1/2+H]⁺) *m*/*z:* 501.3; found 501.4. ¹H NMR (400 MHz, CDCl₃) *δ* 8.57 (d, *J* = 5.1 Hz, 1H), 7.61 (dd, *J* = 17.1, 8.0 Hz, 1H), 7.10 (dd, *J* = 15.2, 8.0 Hz, 5H), 6.90 (d, *J* = 8.5 Hz, 2H), 6.84 - 6.71 (m, 3H), 6.63 (t, *J* = 8.1 Hz, 1H), 5.07 (s, 3H), 3.77 - 3.67 (m, 6H), 3.29 (s, 3H), 2.89 (dd, *J* = 22.1, 9.3 Hz, 5H), 2.64 - 2.52 (m, 2H), 2.37 - 2.25 (m, 4H), 2.13 (dd, *J* = 20.8, 6.7 Hz, 5H), 1.97 (dd, *J* = 22.6, 10.7 Hz, 4H), 1.84 - 1.73 (m, 7H), 1.57 (s, 6H), 1.18 - 0.99 (m, 6H), 0.86 (d, *J* = 11.1 Hz, 2H).

### Example 40: N-(4-((4-(2-(4-((1-(3-(3-)((2-(2,6-dioxopiperidin-3-yl)-1,3- dioxoisoindolin-5-yl)amino)propoxy)propyl)piperidin-4-yl)methoxy)phenyl) propan-2-yl)phenoxy)methyl)pyrimidin-2-yl)methanesulfonamide

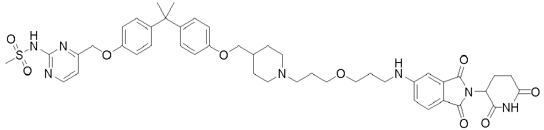

The target compound was synthesized by a method similar to that of Example 39. LC/MS (ESI+) calcd for C₄₆H₅₆N₇O₉S⁺ ( [M+H]⁺ ) *m*/*z:* 882.4; found 882.3.

### Example 41: N-(4-((4-(2-(4-((1-(4-(4-)((2-(2,6-dioxopiperidin-3-yl)-1,3- dioxoisoindolin-5-yl)amino)butoxy)butyl)piperidin-4-yl)methoxy)phenyl)propan-2-yl)phenoxy)methyl)pyrimidin-2-yl)methanesulfonamide

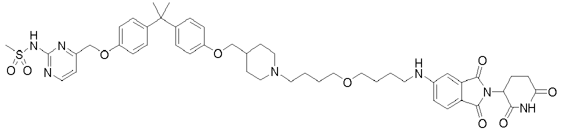

The target compound was synthesized by a method similar to that of Example 39. LC/MS (ESI+) calcd for C₄₈H₆₀N₇O₉S⁺ ([M+H]⁺) *m*/*z:* 910.4; found 910.3.

### Example 42: N-(4-(2-(4-(4-((1-((1r,4r)-4-((2-(2-(2,6-dioxopiperidin-3-yl)-1,3-dioxoisoindolin-5-yl)-2,7-diazaspiro[3.5]nonan-7-yl)methyl)cyclohexyl)methyl) piperidin-4-yl)methoxy)phenyl)propan-2-yl)phenoxy)methyl)pyrimidin-2-yl)methanesulfonamide

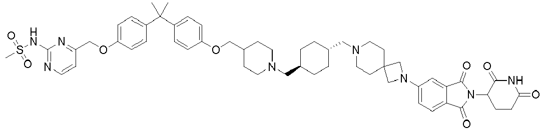

The target compound was synthesized by a method similar to that of Example 39. LC/MS (ESI+) calcd for C₅₅H₆₉N₈O₈S⁺ ([M1/2+H]⁺) *m*/*z:* 501.2; found 501.4. ¹H NMR (400 MHz, DMSO-*d*₆) *δ* 8.58 (d, *J* = 5.1 Hz, 1H), 7.61 (dd, *J* = 17.2, 8.3 Hz, 1H), 7.11 (dt, *J* = 14.4, 7.8 Hz, 6H), 6.90 (d, *J* = 8.5 Hz, 2H), 6.83 - 6.75 (m, 3H), 6.64 (d, *J* = 8.3 Hz, 1H), 5.11 - 5.00 (m, 3H), 3.74 (q, *J* = 6.9, 6.3 Hz, 6H), 2.89 (dd, *J* = 20.7, 8.9 Hz, 5H), 2.61 - 2.52 (m, 2H), 2.37 - 2.20 (m, 5H), 2.09 (dd, *J* = 20.2, 6.7 Hz, 4H), 2.03 - 1.86 (m, 5H), 1.74 (t, *J* = 11.1 Hz, 10H), 1.57 (s, 6H), 1.41 (d, *J* = 18.3 Hz, 4H).

### Example 43: N-(4-((4-(2-(4-((1r,4r)-4-((4-((4-(2-(2,6-dioxopiperidin-3-yl)-1,3-dioxoisoindolin-5-yl)piperazin-1-yl)methyl)piperidin-1-yl)methyl)cyclohexyl) methoxy)phenyl)propan-2-yl)phenoxy)methyl)pyrimidin-2-yl) methanesulfonamide

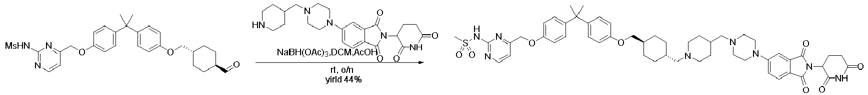

In a 25 mL round-bottom flask, compound N-(4-((4-(2-(4-((1r,4r)-4-formylcyclohexyl)methoxy)phenyl)propan-2-yl)phenoxy)methyl)pyrimidin-2-yl)methanesulfonamide (14.0 mg, 26.0 umol) and 2-(2,6-dioxopiperidin-3-yl)-5-(4-(piperidin-4-ylmethyl)piperazin-1-yl)isoindolin-1,3-dione (11.0 mg, 26.0 umol) were added into 2 mL of dichloromethane, to which was added catalytic amount of acetic acid, and then the mixture was stirred overnight. The reaction solution was rotatory evaporated in vacuum. The residue was purified by prep.-TLC (silica gel), to provide N-(4-((4-(2-(4-((1r,4r)-4-((4-((4-(2-(2,6-dioxopiperidin-3-yl)-1,3-dioxoisoquinolin-5-yl)piperazin-1-yl)methyl)piperidin-1-yl)methyl)cyclohexyl)methoxy)phenyl)propan-2-yl)phenoxy)methyl)pyrimidin-2-yl)methanesulfonamide (11 mg, 11.4 umol), as yellow solid, with a yield of 44%. LC/MS (ESI+) calcd for C₅₂H₆₅N₈O₈S⁺ ([M+H]⁺) *m*/*z:* 961.5; found 961.5. ¹H NMR (400 MHz, DMSO-*d*₆) *δ* 11.05 (s, 1H), 8.54 (d, *J* = 5.1 Hz, 1H), 7.55 (d, *J* = 8.3 Hz, 1H), 7.14 - 7.05 (m, 6H), 6.95 - 6.88 (m, 3H), 6.84 (d, *J* = 9.2 Hz, 1H), 6.79 (d, *J* = 8.3 Hz, 2H), 5.03 (d, *J* = 16.1 Hz, 3H), 3.74 (d, *J* = 5.8 Hz, 2H), 3.26 (s, 3H), 3.23 - 3.11 (m, 8H), 2.95 - 2.82 (m, 5H), 2.57 (s, 2H), 2.31 (d, *J* = 8.4 Hz, 3H), 1.95 (dt, *J* = 23.1, 9.0 Hz, 5H), 1.79 - 1.62 (m, 6H), 1.56 (s, 6H), 1.50 - 1.45 (m, 4H).

### Example 44: N-(4-(4-(2-(4-((8-(4-)((2-(2,6-dioxopiperidin-3-yl)-1,3- dioxoisoindolin-5-yl)amino)butoxy)octyl)oxy)phenyl)propan-2-yl)phenoxy)methyl)pyrimidin-2-yl)ethanesulfonamide

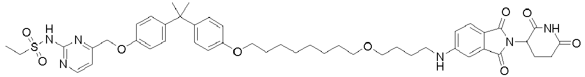

The target compound was synthesized by a method similar to that of Example 39. LC/MS (ESI+) calcd for C₄₇H₅₉N₆O₉S⁺ ([M1/2+H]⁺ ) *m*/*z:* 442.2; found 442.1. ¹H NMR (400 MHz, CDCl₃) *δ* 8.53 (d, *J* = 4.8 Hz, 1H), 8.09 (s, 1H), 7.60 (d, *J* = 7.8 Hz, 1H), 7.15 (dd, *J* = 10.6, 6.7 Hz, 3H), 7.07 (d, *J* = 8.1 Hz, 2H), 6.99 (s, 1H), 6.83 (d, *J* = 8.3 Hz, 2H), 6.73 (d, *J* = 8.4 Hz, 3H), 5.04 (s, 2H), 4.98 - 4.88 (m, 1H), 4.09 - 4.01 (m, 2H), 3.77 (q, *J* = 7.2 Hz, 2H), 3.45 (dt, *J* = 13.0, 6.2 Hz, 4H), 3.24 (s, 2H), 2.94 - 2.74 (m, 3H), 2.11 (d, *J* = 8.8 Hz, 1H), 1.72 (d, *J* = 24.2 Hz, 6H), 1.62 (s, 6H), 1.57 (s, 2H), 1.34 (d, *J* = 7.3 Hz, 11H).

### Example 45: N-(4-((4-(2-(4-((1-((1r,4r)-4-((4-(2-(2,6-dioxopiperidin-3-yl)-1,3-dioxoisoindolin-5-yl)piperazin-1-yl)methyl)cyclohexyl)methyl)piperidin-4-yl)methoxy)phenyl)propan-2-yl)phenoxy)methyl)pyrimidin-2-yl)methanesulfonamide

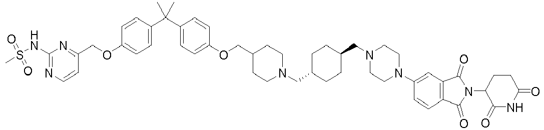

The target compound was synthesized by a method similar to that of Example 39. LC/MS (ESI+) calcd for C₅₂H₆₄N₈O₈S⁺ ([M1/2+H]⁺) *m*/*z:* 481.3; found 481.3. ¹H NMR (400 MHz, CDCl₃) *δ* 8.57 (d, *J* = 5.1 Hz, 1H), 7.69 (d, *J* = 8.5 Hz, 1H), 7.21 (d, *J* = 5.1 Hz, 1H), 7.15 (d, *J* = 8.7 Hz, 2H), 7.07 - 7.03 (m, 2H), 6.84 (d, *J* = 8.6 Hz, 2H), 6.74 (d, *J* = 8.6 Hz, 2H), 5.10 (s, 2H), 4.93 (dd, *J* = 12.2, 5.3 Hz, 1H), 4.04 (d, *J* = 6.9 Hz, 2H), 3.59 (s, 13H), 3.29 (s, 4H), 3.00 (q, *J* = 7.3 Hz, 1H), 2.91 - 2.81 (m, 2H), 2.80 - 2.72 (m, 2H), 2.65 (d, *J* = 6.5 Hz, 2H), 1.91 - 1.81 (m, 8H), 1.62 (s, 6H),1.12 - 0.81 (m, 8H).

### Example 46: N-(4-((4-(2-(4-((1-(2-(2,6-dioxopiperidin-3-yl)-1,3-dioxoisoindolin- 5-yl)azetidin-3-yl)oxy)phenyl)propan-2-yl)phenoxy)methyl)pyrimidin-2-yl)methanesulfonamide

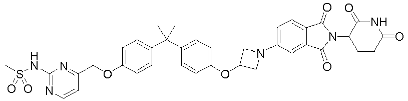

The target compound was synthesized by a method similar to that of Example 34. LC/MS (ESI+) calcd for C₃₇H₃₆N₆O₈S ( [M+H]⁺ ) *m*/*z:* 724.2; found 723.3. ¹H NMR (400 MHz, Chloroform-*d*) *δ* 8.99 (s, 1H), 8.21 (d, *J* = 7.5 Hz, 1H), 7.90 (dd, *J* = 7.4, 1.5 Hz, 1H), 7.51 (t, *J* = 7.5 Hz, 1H), 7.22 (dd, *J* = 7.5, 1.6 Hz, 1H), 7.16 (ddd, *J* = 7.7, 6.3, 1.4 Hz, 5H), 6.93 - 6.81 (m, 4H), 5.50 (t, *J* = 7.0 Hz, 1H), 5.22 - 5.06 (m, 2H), 4.73 (p, *J* = 7.0 Hz, 1H), 4.09 (dd, *J* = 12.4, 7.1 Hz, 2H), 3.97 (dd, *J* = 12.5, 7.1 Hz, 2H), 3.08 (s, 3H), 2.67 - 2.52 (m, 2H), 2.25 - 2.04 (m, 2H), 1.62 (s, 6H).

### Example 47: Synthesis of N-(4-((4-(2-(4-((1-(2-(2,6-dioxopiperidin-3-yl)-1,3-dioxoisoindolin-4-yl)azetidin-3-yl)oxy)phenyl)propan-2-yl)phenoxy)methyl) pyrimidin-2-yl)methanesulfonamide

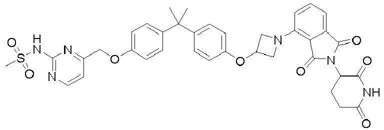

The target compound was synthesized by a method similar to that of Example 34. LC/MS (ESI+) calcd for C₃₇H₃₆N₆O₈S ( [M+H]⁺ ) *m*/*z:* 724.2; found 723.3. ¹H NMR (400 MHz, CDCl₃) *δ* 9.12 (s, 1H), 8.21 (d, *J* = 7.5 Hz, 1H), 7.70 (d, *J* = 7.5 Hz, 1H), 7.39 (d, *J* = 1.4 Hz, 1H), 7.21 - 7.11 (m, 6H), 6.93 - 6.83 (m, 4H), 5.45 (t, *J* = 7.0 Hz, 1H), 5.22 - 5.08 (m, 2H), 4.73 (p, *J* = 7.0 Hz, 1H), 4.09 - 3.87 (m, 4H), 3.08 (s, 3H), 2.68 - 2.55 (m, 2H), 2.24 - 2.02 (m, 2H), 1.62 (s, 6H).

### Example 48 : N-(4-((4-(2-(4-((1-(2-(2,6-dioxopiperidin-3-yl)-1,3-dioxoisoindolin- 5-yl)azetidin-3-yl)methoxy)phenyl)propan-2-yl)phenoxy)methyl)pyrimidin-2-yl)methanesulfonamide

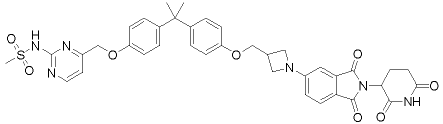

The target compound was synthesized by a method similar to that of Example 34. LC/MS (ESI+) Calcd for C₃₈H₃₆N₆O₈S (M+H⁺) *m*/*z* 738.3; found 738.3.

### Example 49 : N-(4-((4-(2-(4-((1-(2-(2,6-dioxopiperidin-3-yl)-1,3-dioxoisoindolin- 4-yl)azetidin-3-yl)methoxy)phenyl)propan-2-yl)phenoxy)methyl)pyrimidin-2-yl)methanesulfonamide

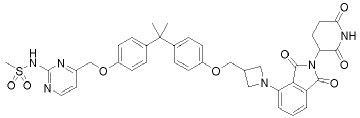

The target compound was synthesized by a method similar to that of Example 34. LC/MS (ESI+) Calcd for C₃₈R₃₆N₆O₈S (M+H⁺) *m*/*z* 738.3; found 738.3.

### Example 50: N-(4-((4-(2-(4-((8-(4-((2-(2,6-dioxopiperidin-3-yl)-1,3- dioxoisoindolin-5-yl)amino))butoxy)octyl)oxy)phenyl)propan-2-yl)phenoxy) methyl)pyrimidin-2-yl)cyclopropanesulfonamide

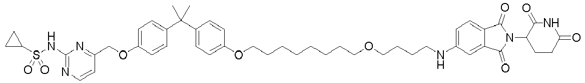

The target compound was synthesized by a method similar to that of Example 34. LC/MS (ESI+) calcd for C₄₈H₅₈N₆O₉S⁺ ([M+H]⁺) *m*/*z:* 894.4; found 894.3. ¹H NMR (400 MHz, CDCl₃) *δ* 8.53 (d, *J* = 4.8 Hz, 1H), 8.09 (s, 1H), 7.60 (d, *J* = 7.8 Hz, 1H), 7.15 (dd, *J* = 10.6, 6.7 Hz, 3H), 7.05 (d, *J* = 8.1 Hz, 2H), 6.99 (s, 1H), 6.81 (d, *J* = 8.3 Hz, 2H), 6.73 (d, *J* = 8.4 Hz, 3H), 5.04 (s, 2H), 4.98 - 4.88 (m, 1H), 4.09 - 4.01 (m, 2H), 3.77 (q, *J* = 7.2 Hz, 2H), 3.45 (dt, *J* = 13.0, 6.2 Hz, 4H), 3.24 (s, 2H), 2.94 - 2.74 (m, 3H), 2.11 (d, *J* = 8.8 Hz, 1H), 1.72 (d, *J* = 24.2 Hz, 6H), 1.62 (s, 6H), 1.57 (s, 2H), 1.43 (d, *J* = 7.3Hz, 11H).

### Example 51: N-(5-((4-(2-(4-((8-(4-((2-(2,6-dioxopiperidin-3-yl)-1,3- dioxoisoindolin-5-yl)amino))butoxy)octyl)oxy)phenyl)propan-2-yl)phenoxy) methyl)pyrimidin-2-yl)methanesulfonamide

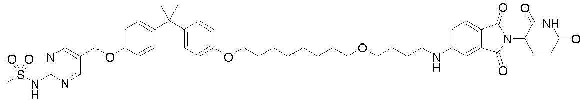

The target compound was synthesized by a method similar to that of Example 34. LC/MS (ESI+) calcd for C₄₆H₅₆N₆O₉S⁺ ([M+H]⁺) *m*/*z:* 868.4; found 868.3. ¹H NMR (400 MHz, CDCl₃) *δ* 8.66 (d, *J* = 4.8 Hz, 1H), 8.08 (s, 1H), 7.60 (d, *J* = 7.8 Hz, 1H), 7.15 (dd, *J* = 10.6, 6.7 Hz, 3H), 7.07 (d, *J* = 8.1 Hz, 2H), 6.99 (s, 1H), 6.83 (d, *J* = 8.3 Hz, 2H), 6.73 (d, *J* = 8.4 Hz, 3H), 5.04 (s, 2H), 4.98 - 4.88 (m, 1H), 4.09 - 4.01 (m, 2H), 3.45 (dt, *J* = 13.0, 6.2 Hz, 4H), 3.24 (s, 2H), 2.94 - 2.74 (m, 3H), 2.11 (d, *J* = 8.8 Hz, 1H), 1.72 (d, *J* = 24.2 Hz, 6H), 1.62 (s, 6H), 1.57 (s, 2H), 1.34 (d, *J* = 7.3 Hz, 11H).

### Example 52: N-(4-((4-(2-(4-((5-(3-((1-(2-(2,6-dioxopiperidin-3-yl)-1,3- dioxoisoindolin-5-yl)pyrrolidin-3-yl)methoxy)propoxy)pentyl)oxy) phenyl)propan-2-yl)phenoxy)methyl)pyrimidin-2-yl)methanesulfonamide

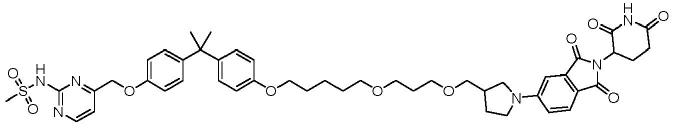

The target compound was synthesized using a method similar to that of Example 29. ¹H NMR (400 MHz, CDCl₃) *δ* 8.55 (d, *J* = 5.0 Hz, 1H), 8.10 (s, 1H), 7.64 (d, *J* = 8.4 Hz, 1H), 7.19 (d, *J* = 5.0 Hz, 1H), 7.17 - 7.10 (m, 2H), 7.09 - 7.02 (m, 2H), 6.93 (d, *J* = 2.2 Hz, 1H), 6.89 - 6.79 (m, 2H), 6.78 - 6.68 (m, 2H), 6.66 (dd, *J* = 8.5, 2.3 Hz, 1H), 5.05 (s, 2H), 4.93 (dd, *J* = 12.2, 5.3 Hz, 1H), 4.14 - 4.04 (m, 2H), 3.57 - 3.44 (m, 10H), 3.44 - 3.36 (m, 4H), 3.21 (dd, *J* = 10.2, 6.7 Hz, 1H), 2.92 - 2.60 (m, 4H), 2.21 - 2.08 (m, 2H), 1.92 - 1.69 (m, 6H), 1.40 (dq, *J* = 10.0, 7.1 Hz, 2H). LC/MS (ESI+) calcd for C₄₇H₅₆N₆O₁₀S ( [M+H]⁺ ) *m*/*z:* 897.4; found 897.4.

### Example 53 : N-(4-((4-(2-(4-(2-((2-(2,6-dioxopiperidin-3-yl)-1,3-dioxoisoindolin- 4-yl)amino)ethoxy)phenyl)isopropan-2-yl)phenoxy)methyl)pyrimidin-2-yl)methanesulfonamide

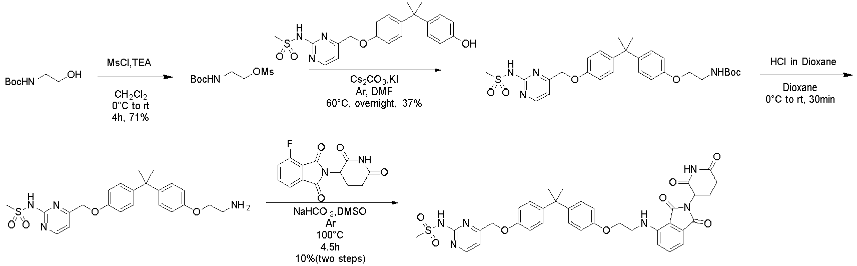

### Step 1: Synthesis of 2-((tert-butoxycarbonyl)amino)ethyl methanesulfonate

*N*-Boc-ethanolamine (2000 mg, 12.41 mmol) was weighed and placed in a single-necked round-bottom flask, to which was added dichloromethane (30 mL), and then the mixture was stirred to dissolve and become clear. Subsequently, to the reaction solution, was added triethylamine (3767 mg, 37.23 mmol). After addition, the system was transferred into an ice water bath and cooled under stirring. After 15 min, to the system, was added 10 mL solution of methanesulfonyl choride (1706 mg, 14.89 mmol) in dichloromethane. Then, the system was stirred and reacted at room temperature. After 4 h, the reaction was completed by TLC detection and stopped, followed by extraction by adding dichloromethane and water. The organic phase was washed with saturated NH₄Cl solution, and further washed successively with water and saturated brine, and dried with anhydrous Na₂SO₄. The resultant solution was rotatory evaporated to remove the solvent and provide the crude product, which was separated and purified by column chromatography, to obtain the target product as off-white solid (2100 mg, 71%).

### Step 2: Synthesis of tert-butyl (2-(4-(2-(4-((2-(methanesulfonamido)pyrimidin-4-yl) methoxy)phenyl)isopropan-2-yl)phenoxy)ethyl)carbamate

*N*-(4-((4-(2-(4-hydroxylphenyl)isopropan-2-yl)phenoxy)methyl)pyrimidin-2-yl)methanesulfonamide (230 mg, 0.56 mmol), 2-((tert-butoxycarbonyl)amino)ethyl methanesulfonate (90 mg, 0.84 mmol), KI (93 mg, 0.56 mmol) and cesium carbonate (274 mg, 0.84 mmol) were added into 6 mL of DMF, and then the system was vacuumized and purged with argon, that was repeated three times. After that, the system was heated to 60 °C and reacted under stirring. The next day, TLC detection indicated the starting materials disappeared, and then heating was removed. The reaction solution was cooled to room temperature, and extracted by adding ethyl acetate and water. The organic phase was successively washed with water and saturated brine, and dried with anhydrous Na₂SO₄. The resultant solution was rotatory evaporated to remove the solvent and provide the crude product, which was separated and purified by Prep-TLC, to obtain the target product as off-white solid (115 mg, 37%). LC/MS (ESI⁺) calcd for C₂₈H₃₆N₄O₆S ([M-55]⁺) *m*/*z* 556.2; found 501.1.

### Step 3: Synthesis of N-(4-((4-(2-(4-(2-aminoethoxy)phenyl)isopropan-2-yl)phenoxy) methyl)pyrimidin-2-yl)methanesulfonamide

Compound tert-butyl (2-(4-(2-(4-((2-(methanesulfonamido)pyrimidin-4-yl) methoxy)phenyl)isopropan-2-yl)phenoxy)ethyl)carbamate (115 mg, 0.21 mmol) was dissolved in 1 mL of 1,4-dioxane, and then transferred into an ice water bath for cooling under stirring. After 10 min, the solution of HCl in dioxane (2 mL) was added. After addition, the system was moved from the ice water bath, and then reacted at room temperature. After 30 min, TLC detection indicated completion of the reaction. The solvent was removed by rotatory evaporation. The residual dioxane was further removed by repeatedly co-evaporating with dichloromethane, to provide the hydrochloride of the target product as off-white solid, which was directly used in the next step without further purification.

### Step 4: Synthesis of compound N (4-((4-(2-(4-(2-((2-(2,6-dioxopiperidin-3-yl)-1,3-dioxoisoindolin-4-yl)amino)ethoxy)phenyl)isopropan-2-yl)phenoxy)methyl) pyrimidin-2-yl)methanesulfonamide

The previous step's *N*-(4-((4-(2-(4-(2-aminoethoxy)phenyl)isopropan-2-yl) phenoxy)methyl)pyrimidin-2-yl)methanesulfonamide hydrochloride (0.21 mmol), 2-(2,6-dioxopiperidin-3-yl)-5-fluoroisoindolin-1,3-dione (88 mg, 0.32 mmol) and NaHCO₃ (62 mg, 0.74 mmol) were added into 5 mL of dry DMSO, and then the system was vacuumized and purged with argon, that was repeated three times. After that, the system was transferred into an oil bath at 100 °C and reacted under stirring. After 4.5 h, TLC indicated almost disappearance of the starting materials. The reaction solution was cooled to room temperature, and extracted by adding ethyl acetate and water. The organic phase was successively washed with saturated NH₄Cl solution, water, and saturated brine, and then dried with anhydrous Na₂SO₄. The solvent was removed by rotatory evaporation. The residue was separated and purified by Prep-TLC, to provide the target compound as light yellow solid (16 mg, 10%). ¹H NMR (400 MHz, Chloroform-*d*) *δ* 8.63 (d, *J* = 5.0 Hz, 1H), 7.96 (s, 1H), 7.50 (dd, *J* = 8.4, 7.1 Hz, 1H), 7.27 (d, *J* = 5.0 Hz, 1H),7.20 - 7.03 (m, 6H), 6.86 (d, *J* = 8.8 Hz, 2H), 6.71 (d, *J* = 8.7 Hz, 2H), 5.13 (s, 2H), 4.87 (dd, *J* = 12.1, 5.3 Hz, 1H), 4.33 (t, *J* = 6.6 Hz, 2H), 3.68 (t, *J* = 6.5 Hz, 2H), 3.53 (s, 3H), 3.11 (br, 1H), 2.89 - 2.62 (m, 4H), 2.09 (ddd, *J* = 8.3, 6.5, 3.8 Hz, 1H), 1.63 (s, 6H).LC/MS (ESI⁺) calcd for C₃₆H₃₆N₆O₈S ([M+H]⁺) *m*/*z* 712.2; found, 713.2.

### Example 54: N-(4-((4-(2-(4-(4-(4-((2-(2,6-dioxopiperidin-3-yl)-1,3- dioxoisoindolin-5-yl)amino)butoxy)butoxy)phenyl)isopropan-2-yl)phenoxy) methyl)pyrimidin-2-yl)methanesulfonamide

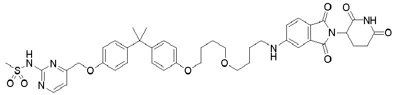

The title compound was synthesized by a method similar to that of Example 53. ¹H NMR (400 MHz, Chloroform-*d*) *δ* 8.54 (d, *J* = 5.0 Hz, 1H), 8.06 (s, 1H), 7.59 (d, *J* = 8.3 Hz, 1H), 7.19 (d, *J* = 5.1 Hz, 1H), 7.17 - 7.11 (m, 2H), 7.09 - 7.03 (m, 2H), 6.96 (d, *J* = 1.9 Hz, 1H), 6.87 - 6.80 (m, 2H), 6.73 (td, *J* = 7.7, 6.7, 2.1 Hz, 3H), 5.05 (s, 2H), 4.92 (dd, *J* = 12.2, 5.3 Hz, 1H), 4.19 - 4.07 (m, 2H), 3.53 - 3.36 (m, 7H), 3.22 (t, *J* = 6.8 Hz, 2H), 2.92 - 2.66 (m, 3H), 2.15 - 2.08 (m, 1H), 1.90 - 1.80 (m, 3H), 1.75 (dt, *J* = 13.5, 6.6 Hz, 3H), 1.67 (dt, *J* = 14.9, 6.7 Hz, 4H), 1.62 (s, 6H). LC/MS (ESI⁺) calcd for C₄₂H₄₈N₆O₉S ([M+H]⁺) *m*/*z* 812.3; found, 813.3.

### Example 55: N-(4-((4-(2-(4-(4-(4-((2-(2,6-dioxopiperidin-3-yl)-1,3- dioxoisoindolin-4-yl)amino)butoxy)butoxy)phenyl)isopropan-2-yl)phenoxy) methyl)pyrimidin-2-yl)methanesulfonamide

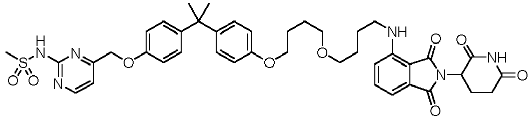

The title compound was synthesized by a method similar to that of Example 53. ¹H NMR (400 MHz, Chloroform-*d*) *δ* 8.54 (d, *J* = 5.0 Hz, 1H), 8.11 (s, 1H), 7.47 (dd, *J* = 8.5, 7.2 Hz, 1H), 7.19 (d, *J* = 5.0 Hz, 1H), 7.15 (d, *J* = 8.8 Hz, 2H), 7.10 - 7.02 (m, 3H), 6.87 (d, *J* = 8.6 Hz, 1H), 6.83 (d, *J* = 8.8 Hz, 2H), 6.72 (d, *J* = 8.7 Hz, 2H), 5.05 (s, 2H), 4.91 (dd, *J* = 12.0, 5.3 Hz, 1H), 4.20 - 4.07 (m, 2H), 3.55 - 3.39 (m, 7H), 3.28 (t, *J* = 6.6 Hz, 2H), 2.92 - 2.66 (m, 3H), 2.11 (dt, *J* = 9.8, 3.3 Hz, 1H), 2.01 (dd, *J* = 11.9, 6.2 Hz, 1H), 1.82 (dt, *J* = 14.9, 7.8 Hz, 3H), 1.76 - 1.63 (m, 6H), 1.62 (s, 6H). LC/MS (ESI⁺) calcd for C₄₂H₄₈N₆O₉S ([M+H]⁺) *m*/*z* 812.3; found, 813.3.

### Example 56: N-(4-((4-(2-(4-(((1r,4r)-4-(((5-((2-(2,6-dioxopiperidin-3-yl)-1,3-dioxoisoindolin-5-yl)amino)pentyl)oxy)methyl)cyclohexyl)methoxy)phenyl) isopropan-2-yl)phenoxy)methyl)pyrimidin-2-yl)methanesulfonamide

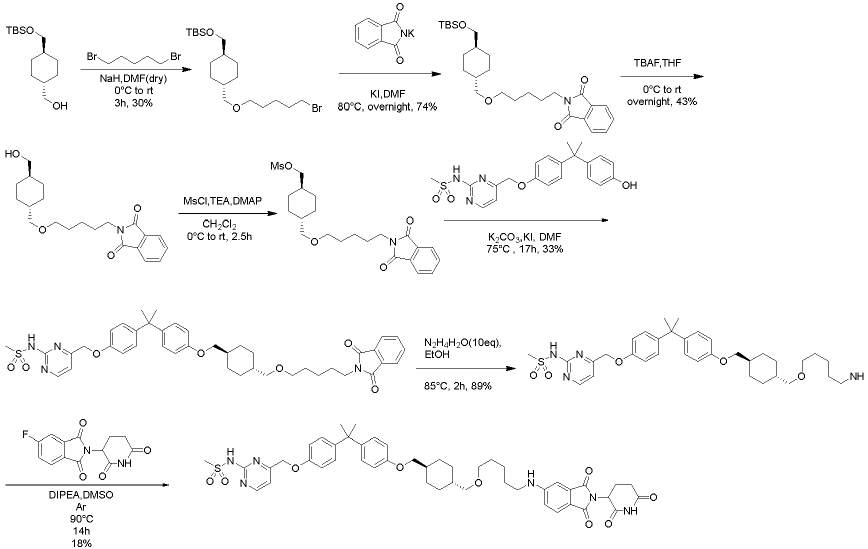

### Step 1: Synthesis of (((1r,4r)-4-(((5-bromopentyl)oxy)methyl)cyclohexyl) methoxy)(tert-butyl)dimethylsilane

((1*r*,4*r*)-4-(((tert-butyldimethylsilyl)oxy)methyl)cyclohexyl)methanol (1082 mg, 4.19 mmol) was weighed and added into a single-necked round-bottome flask, to which was added DMF (10 mL), and then the mixture was stirred to dissolve and become clear. Subsequently, the system was transferred into an ice water bath and cooled under stirring. After 15 min, to the system, was added NaH (201 mg, 5.03 mmol), and then the system was allowed to react for 10 min in an ice water bath. To the system, was added 2 mL solution of 1,5-dibromopentane (1060 mg, 4.61 mmol) in DMF. After addition, the system was stirred and reacted at room temperature. After 3 h, the starting materials disappeared by TLC detection. In an ice water bath, to the system, was added saturated NH₄Cl solution to quench the reaction. The resultant solution was extracted by adding ethyl acetate and water. The organic phase was successively washed with water and saturated brine, dried with anhydrous Na₂SO₄, and then rotatory evaporated to remove the solvent and provide the crude product, which was separated and purified by column chromatography, to obtain the target product as colorless and transparent oily liquid (512 mg, 30%).

### Step 2: Synthesis of 2-(5-(((1r,4r)-4-(((tert-butyldimethylsilyl)oxy)methyl)cyclohexyl) methoxy)pentyl)isoindolin-1,3-dione

(((1*r*,4*r*)-4-(((5-bromopentyl)oxy)methyl)cyclohexyl)methoxy)(tert-butyl)dimethylsilane (500 mg, 1.23 mmol) was weighed and placed in a single-necked round-bottome flask, to which was added DMF (12 mL), and then the mixture was stirred to dissolve and become clear. KI (204 mg, 1.23 mmol) and potassium phthalimide (228 mg, 1.23 mmol) were added to the system, and then the system was vacuumized and purged with argon, that was repeated three times. The system was allowed to react overnight in an oil bath at 80 °C. The next day, the reaction was completed by TLC detection, and then heating was removed. The reaction solution was cooled to room temperature, and then extracted by adding ethyl acetate and water. The organic phase was successively washed with water and saturated brine, dried with anhydrous Na₂SO₄, and then rotatory evaporated to remove the solvent and provide the crude product, which was separated and purified by column chromatography, to obtain the target product as off-white solid (428 mg, 74%). LC/MS (ESI⁺) calcd for C₂₇H₄₃NO₄Si ([M+H]⁺) *m*/*z* 473.3; found, 474.3.

### Step 3: Synthesis of 2-(5-(((1r,4r)-4-(hydroxylmethyl)cyclohexyl)methoxy)pentyl) isoindolin-1,3-dione

2-(5-(((1*r*,4*r*)-4-(((tert-butyldimethylsilyl)oxy)methyl)cyclohexyl)methoxy) pentyl)isoindolin-1,3-dione (428 mg, 0.90 mmol) was added into 10 mL of THF, and then the mixture was stirred to dissolve and become clear. TBAF.H₂O (706 mg, 2.70 mmol) was added to the system, and then the system was vacuumized and purged with argon, that was repeated three times. The system was stirred and reacted overnight at room temperature. The next day, TLC detection indicated completion of the reaction. The system was extracted by adding ethyl acetate and water. The organic phase was successively washed with water and saturated brine, and dried with anhydrous Na₂SO₄. The resultant solution was rotatory evaporated to remove the solvent and provide the crude product, which was separated and purified by Prep-TLC, to provide the target compound as off-white solid (140 mg, 43%).

### Step 4: Synthesis of ((1r,4r)-4-(((5-(1,3-dioxoisoindolin-2-yl)pentyl)oxy) methyl)cyclohexyl)methyl methanesulfonate

2-(5-(((1*r*,4*r*)-4-(hydroxylmethyl)cyclohexyl)methoxy)pentyl)isoindolin-1,3-dione (140 mg, 0.39 mmol) was weighed and dissolved in 5 mL of dichloromethane, to which were added triethylamine (52 mg, 0.51 mmol) and DMAP (5 mg, 0.04 mmol), and then the system was transferred into an ice water bath for cooling under stirring. After 15 min, methanesulfonyl choride (49 mg, 0.43 mmol) was added. Subsequently, the system was stirred and reacted at room temperature. After 2.5 h, the starting materials disappeared by TLC detection. To the system, were added dichloromethane and water, to carry out the extraction procedures. Th organic phase was dried with anhydrous Na₂SO₄. Then, the solvent was removed by rotatory evaporation, to obtain the target product as off-white solids (171 mg), which was directly used in the next step without further purification.

### Step 5: Synthesis of N-(4-((4-(2-(4-(((1r,4r)-4-(((5-(1,3-dioxopiperidin-2-yl)pentyl) oxy)methyl)cyclohexyl)methoxy)phenyl)isopropan-2-yl)phenoxy)methyl)pyrimidin-2-yl)methanesulfonamide

((1*r*,4*r*)-4-(((5-(1,3-dioxoisoindolin-2-yl)pentyl)oxy)methyl)cyclohexyl)methyl methanesulfonate (171 mg, 0.39 mmol), *N*-(4-((4-(2-(4-hydroxylphenyl)isopropan- 2-yl)phenoxy)methyl)pyrimidin-2-yl)methanesulfonamide (128 mg, 0.31 mmol), K₂CO₃ (108 mg, 0.78 mmol) and KI (98 mg, 0.59 mmol) were added into 5 mL of DMF, and then the system was vacuumized and purged with argon, that was repeated three times. After that, the system was transferred into an oil bath at 75 °C, and reacted overnight under stirring. After 17 h, TLC detection indicated completion of the reaction. The reaction solution was cooled to room temperature, and extracted by adding ethyl acetate and water. The organic phase was successively washed with water and saturated brine, and then dried with anhydrous Na₂SO₄. The solvent was removed by rotatory evaporation. The residue was separated and purified by Prep-TLC, to provide the target compound as off-white solid (78 mg, 33%). LC/MS (ESI⁺) calcd for C₄₂H₅₀N₄O₇S ([M+H]⁺) *m*/*z* 754.3; found, 755.3.

### Step 6: Synthesis of N-(4-((4-(2-(4-(((1r,4r)-4-(((5-aminopentyl)oxy)methyl) cyclohexyl)methoxy)phenyl)isopropan-2-yl)phenoxy)methyl)pyrimidin-2-yl)methanesulfonamide

*N*-(4-((4-(2-(4-(((1*r*,4*r*)-4-(((5-(1,3-dioxopiperidin-2-yl)pentyl)oxy)methyl) cyclohexyl)methoxy)phenyl)isopropan-2-yl)phenoxy)methyl)pyrimidin-2-yl) methanesulfonamide (78 mg, 0.10 mmol) was dissolved in 5 mL of ethanol, to which was then added hydrazine hydrate (54 mg, 1.00 mmol), and the system was allowed to react under reflux in an oil bath at 85°C. After 2 h, TLC indicated disappearnce of the starting material. ₒ The systme was cooled to room temperature, and then subjected to suction filtration. The filter cake was washed with a small amount of ethanol for a few times, , and then the filtrate was combined. The solvent was removed by rotatory evaporation to provide the crude product. To the system, was added dichloromethane, and then the system was transferred into an ice water bath, followed by stirring. After 30 min, the system was further subject to suction filtration, and the filter cake was rinsed with a small amount of dichloromethane for a few times, and then the filtrate was combined. The solvent was removed by rotatory evaporation, to obtain the target product as off-white solids (58 mg, 89%).

### Step 7: Synthesis of N-(4-((4-(2-(4-(((1r,4r)-4-(((5-((2-(2,6-dioxopiperidin-3- yl)-1,3-dioxoisoindolin-5-yl)amino)pentyl)oxy)methyl)cyclohexyl)methoxy)phenyl) isopropan-2-yl)phenoxy)methyl)pyrimidin-2-yl)methanesulfonamide

*N*-(4-((4-(2-(4-(((1*r*,4*r*)-4-(((5-aminopentyl)oxy)methyl)cyclohexyl)methoxy) phenyl)isopropan-2-yl)phenoxy)methyl)pyrimidin-2-yl)methanesulfonamide (58 mg, 0.09 mmol), 2-(2,6-dioxopiperidin-3-yl)-5-fluoroisoindolin-1,3-dione (30 mg, 0.11 mmol) and DIPEA(233 mg, 1.80 mmol) were added into 8 mL of dry DMSO, and then the system was vacuumized and purged with argon, that was repeated three times. After that, the system was placed in an oil bath at 90 °C, and reacted under stirring. The next day, TLC indicated almost disappearance of the starting materials. The reaction solution was cooled to room temperature, and extracted by adding ethyl acetate and water. The organic phase was successively washed with saturated NH₄Cl, water, and saturated brine, and then dried with anhydrous Na₂SO₄. The solvent was removed by rotatory evaporation, and the residue was separated and purified by Prep-TLC, to provide the target compound as light yellow solid (15 mg, 18%). ¹H NMR (400 MHz, Chloroform-*d*) *δ* 8.56 (d, *J* = 4.0 Hz, 1H), 8.07 (s, 1H), 7.60 (d, *J* = 7.4 Hz, 1H), 7.23 - 7.17 (m, 1H), 7.14 (d, *J* = 8.3 Hz, 2H), 7.07 (d, *J* = 8.2 Hz, 2H), 6.96 (s, 1H), 6.84 (d, *J* = 8.3 Hz, 2H), 6.73 (d, *J* = 8.3 Hz, 3H), 5.05 (s, 2H), 4.93 (dd, *J* = 11.7, 5.1 Hz, 1H), 4.01 (d, *J* = 6.6 Hz, 2H), 3.50 (s, 3H), 3.41 (t, *J* = 5.6 Hz, 2H), 3.21 (d, *J* = 6.0 Hz, 4H), 2.98 - 2.61 (m, 4H), 2.28 - 2.19 (m, 1H), 2.17 - 2.08 (m, 2H), 2.01 (dd, *J* = 11.8, 7.2 Hz, 1H), 1.78 (t, *J* = 13.0 Hz, 6H), 1.71 - 1.65 (m, 2H),1.63 (s, 6H), 1.53 - 1.44 (m, 3H), 1.10 - 0.96 (m, 3H). LC/MS (ESI⁺) calcd for C₄₇H₅₆N₆O₉S ([M+H]⁺) *m*/*z* 880.4; found, 881.2.

### Example 57: N-(4-((4-(3-(4-((8-(4-((2-(2,6-dioxopiperidin-3-yl)-1,3- dioxoisoindolin-5-yl)amino)butoxy)octyl)oxy)phenyl)pentan-3-yl)phenoxy)methyl)pyrimidin-2-yl)methanesulfonamide

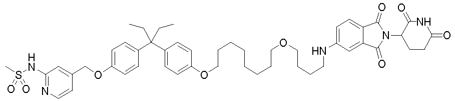

The target compound was synthesized using a method similar to that of Example 1. LC/MS (ESI⁺) calcd for C₄₈H₆₀N₆O₉S([M+H]⁺) *m*/*z* 896.4; found, 897.2.

### Example 58:N (4-((4-(1-(4-((8-(4-((2-(2,6-dioxopiperidin-3-yl)-1,3- dioxoisoindolin-5-yl)amino)butoxy)octyl)oxy)phenyl)cyclobutyl)phenoxy) methyl)pyrimidin-2-yl)methanesulfonamide

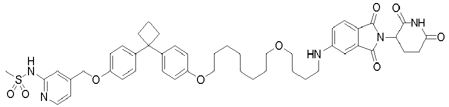

The title compound was synthesized using a method similar to that of Example 1. ¹H NMR (400 MHz, Chloroform-*d*) *δ* 8.62 (d, *J* = 15.1 Hz, 1H), 7.59 (d, *J* = 8.1 Hz, 1H), 7.25 - 7.10 (m, 6H), 6.94 (s, 1H), 6.82 (dd, *J* = 19.3, 8.6 Hz, 4H), 6.71 (d, *J* = 8.0 Hz, 1H), 5.38 - 5.32 (m, 1H), 5.07 (s, 2H), 4.92 (dd, *J* = 12.0, 5.1 Hz, 1H), 3.89 (t, *J* = 6.4 Hz, 2H), 3.56 - 3.31 (m, 7H), 3.24 (p, *J* = 6.1 Hz, 2H), 2.94 - 2.55 (m, 8H), 2.27 - 2.18 (m, 1H), 2.16 - 2.07 (m, 1H), 2.02 (dt, *J* = 11.8, 6.2 Hz, 2H), 1.94 (dt, *J* = 14.8, 7.5 Hz, 3H), 1.78 - 1.56 (m, 12H). LC/MS (ESI⁺) calcd for C₄₇H₅₆N₆O₉S ([M+H]⁺) *m*/*z* 879.4; found, 880.3.

### Example 59: 2-(2,6-dioxopiperidin-3-yl)-4-((3-(4-(2-(4-(2-morpholinepyrimidin- 4-yl)methoxy)phenyl)propan-2-yl)phenoxy)propyl)amino)isoindolin-1,3-dione

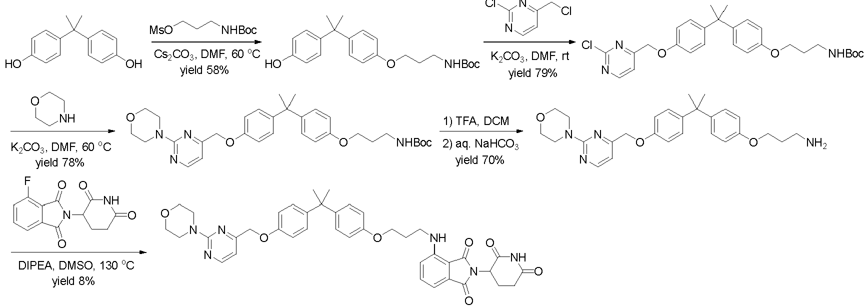

### Step 1: Synthesis of tert-butyl (3-(4-(2-(4-hydroxylphenyl)propan-2-yl) phenoxy)propyl)carbamate

Bisphenol A (11.4 g, 50 mmol), 3-((tert-butoxycarbonyl)amino)propyl methanesulfonate (12.6 g, 50 mmol) and cesium carbonate (32.5 g, 100 mmol) were successively added into 200 mL of DMF, and then the mixture was heated to 60 °C and reacted overnight. After completion of the reaction detected by TLC, the reaction solution was naturally cooled to room temperature, and extracted with 200 mL of ethyl acetate. The organic phase was washed with saturated brine (3*100 mL), dried with anhydrous Na₂SO₄, and concentrated. The residue was separated and purified by column chromatography, to provide 11.1 g of target compound, with a yield of 58%. Step 2: tert-butyl Synthesis of (3-(4-(2-(4-((2-chloropyrimidin-4-yl)methoxy)phenyl) propan-2-yl)phenoxy)propyl)carbamate

tert-butyl (3-(4-(2-(4-hydroxylphenyl)propan-2-yl)phenoxy)propyl)carbamate (3.85 g, 10 mmol), 2-chloro-4-(chloromethyl)pyrimidine (1.62 g, 10 mmol) and K₂CO₃ (2.76 g, 20 mmol) were successively added into 30 mL of DMF, and then the mixture was allowed to react overnight at room temperature. After completion of the reaction detected by TLC, the resultant solution was extracted with 20 mL of ethyl acetate. The organic phase was washed with saturated brine (3*10 mL), dried with anhydrous Na₂SO₄, and concentrated. The residue was separated and purified by column chromatography, to provide 4.05 g of target compound, with a yield of 79%.

### Step 3: Synthesis of tert-butyl (3-(4-(2-(4-((2-morpholinepyrimidin-4-yl)methoxy) phenyl)propan-2-yl)phenoxy)propyl)carbamate

tert-butyl (3-(4-(2-(4-((2-chloropyrimidin-4-yl)methoxy)phenyl)propan-2-yl) phenoxy)propyl)carbamate (102 mg, 0.2 mmol), morpholine (35 mg, 0.4 mmol) and K₂CO₃ (56 mg, 0.4 mmol) were successively added into 1 mL of DMF, and then the mixture was heated to 60 °C and reacted overnight. After completion of the reaction detected by TLC, the reaction solution was naturally cooled to room temperature, and extracted with 1 mL of ethyl acetate. The organic phase was washed with saturated brine (3*1 mL), dried with anhydrous Na₂SO₄, and concentrated. The residue was separated and purified by column chromatography, to provide 88 mg of target compound, with a yield of 78%.

### Step 4: Synthesis of (3-(4-(2-(4-((2-morpholinepyrimidin-4-yl)methoxy)phenyl) propan-2-yl)phenoxy)propan-1-amine

Compound tert-butyl (3-(4-(2-(4-((2-morpholinepyrimidin-4-yl)methoxy)phenyl) propan-2-yl)phenoxy)propyl)carbamate (88 mg, 0.16 mmol) was dissolved in 2 mL of dichloromethane, to which was added 1 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 52 mg of target compound, with a yield of 70%.

### Step 5: Synthesis of 2-(2,6-dioxopiperidin-3-yl)-4-((3-(4-(2-(4-(2- morpholinepyrimidin-4-yl)methoxy)phenyl)propan-2-yl)phenoxy)propyl)amino) isoindolin-1,3-dione

(3-(4-(2-(4-((2-morpholinepyrimidin-4-yl)methoxy)phenyl)propan-2-yl)phenoxy)propan-1-amine (52 mg, 0.11 mmol), 2-(2,6-dioxopiperidin-3-yl)-4- fluoroisoindolin-1,3-dione (33 mg, 0.12 mmol) and DIPEA (43 mg, 0.33 mmol) were successively added into 5 mL of DMSO, and then the system was heated to 130 °C and reacted overnight. The reaction solution was naturally cooled to room temperature, and then extracted with 10 mL of ethyl acetate. The organic phase was washed with saturated brine (3*10 mL), dried with anhydrous Na₂SO₄, and concentrated. The residue was separated and purified by column chromatography, to provide 6 mg of end product, with a yield of 7.6%. ¹H NMR (400 MHz, CDCl₃) *δ* 8.33 (s, 1H), 8.14 (s, 1H), 7.46 (t, *J* = 7.7 Hz, 1H), 7.11 (dd, *J* = 18.1, 7.6 Hz, 5H), 6.92 (d, *J* = 8.4 Hz, 1H), 6.83 (d, *J* = 8.2 Hz, 4H), 6.77 (s, 1H), 6.46 (d, *J* = 5.9 Hz, 1H), 4.94 (s, 2H), 4.90 (d, *J* = 4.5 Hz, 1H), 4.07 (t, *J* = 5.6 Hz, 2H), 3.80 (d, *J* = 17.9 Hz, 8H), 3.50 (d, *J* = 6.2 Hz, 2H), 3.00 - 2.62 (m, 3H), 2.18 - 2.03 (m, 3H), 1.63 (s, 6H). LC/MS (ESI+) calcd for C₄₀H₄₂N₆O₇( [M+H]⁺ ) *m*/*z:* 719.3; found 719.3.

### Example 60: 2-(2,6-dioxopiperidin-3-yl)-4-((3-(4-(2-(4-methylpiperazin-1-yl) pyrimidin-4-yl)methoxy)phenyl)propan-2-yl)phenoxy)propyl)amino)isoindolin-1,3-dione

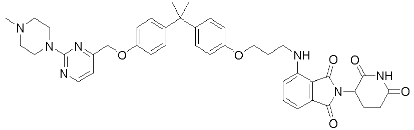

The target compound was synthesized by a method similar to that of Example 59. ¹H NMR (400 MHz, CDCl₃) *δ* 8.78 (s, 1H), 8.29 (d, *J* = 5.0 Hz, 1H), 7.46 (dd, *J* = 8.5, 7.1 Hz, 1H), 7.16 - 7.10 (m, 4H), 7.08 (d, *J* = 7.1 Hz, 1H), 6.91 (d, *J* = 8.6 Hz, 1H), 6.86 - 6.79 (m, 4H), 6.71 (d, *J* = 5.0 Hz, 1H), 6.45 (t, *J* = 5.8 Hz, 1H), 4.92 (s, 2H), 4.91 - 4.87 (m, 1H), 4.06 (t, *J* = 5.6 Hz, 2H), 3.89 (t, *J* = 4.9 Hz, 4H), 3.50 (q, *J* = 6.3 Hz, 2H), 2.91 - 2.71 (m, 3H), 2.55 (t, *J* = 5.2 Hz, 4H), 2.38 (s, 3H), 2.14 - 2.09 (m, 3H), 1.62 (s, 6H). LC/MS (ESI+) calcd for C₄₁H₄₅N₇O₆( [M+H]⁺ ) *m*/*z:* 732.4; found 732.4.

### Example 61: 5-((4-((8-(4-(2-(4-((2-(2-oxa-6-azaspiro[3.3]heptan-6-yl)pyrimidin- 4-yl)methoxy)phenyl)propan-2-yl)phenoxy)octyl)oxy)butyl)amino)-2-(2,6-dioxopiperidin-3-yl)isoindolin-1,3-dione

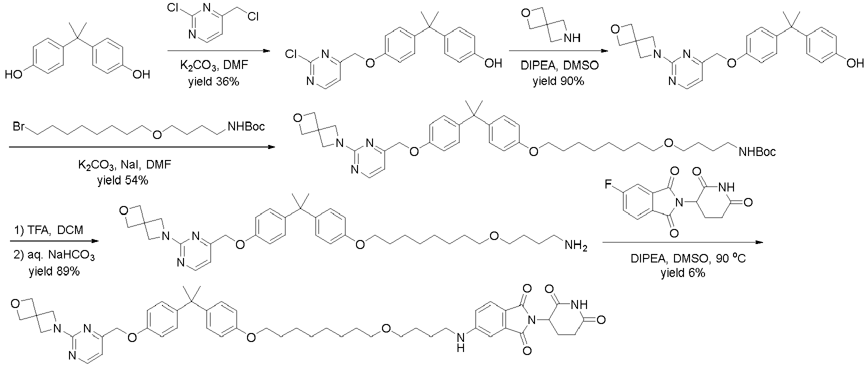

### Step 1: Synthesis of 4-(2-(4-((2-chloropyrimidin-4-yl)methoxy) phenyl)propan-2-yl) phenol

Bisphenol A (2.28 g, 10 mmol), 2-chloro-4-(chloromethyl)pyrimidine (0.81 g, 5 mmol) and K₂CO₃ (1.38 g, 10 mmol) were successively added into 20 mL of DMF, and then the mixture was allowed to react overnight at room temperature. After completion of the reaction detected by TLC, the resultant solution was extracted with 20 mL of ethyl acetate. The organic phase was washed with saturated brine (3*20 mL), dried with anhydrous Na₂SO₄, and concentrated. The residue was separated and purified by column chromatography, to provide 0.65 g of target compound, with a yield of 36%.

### Step 2: Synthesis of 4-(2-(4-((2-(2-oxa-6-azaspiro[3.3]heptan-6-yl)pyrimidin-4-yl) methoxy)phenyl)propan-2-yl)phenol

4-(2-(4-((2-chloropyrimidin-4-yl)methoxy)phenyl)propan-2-yl)phenol(650 mg, 1.8 mmol), 2-oxa-6-azaspiro[3.3]heptane (200 mg, 2 mmol) and DIPEA (702 mg, 5.4 mmol) were successively added into 10 mL of DMSO, and then allowed to react overnight at 80 °C. After completion of the reaction detected by TLC, the resultant solution was extracted with 10 mL of ethyl acetate. The organic phase was washed with saturated brine (3*10 mL), dried with anhydrous Na₂SO₄, and concentrated. The residue was separated and purified by column chromatography, to provide 680 mg of target compound, with a yield of 90%.

In the following steps, the target compound was synthesized by a method similar to that of Example 59. ¹H NMR (400 MHz, CDCl₃) *δ* 8.31 (s, 1H), 8.12 (s, 1H), 7.60 (d, *J* = 8.3 Hz, 1H), 7.18 - 7.08 (m, 4H), 6.95 (d, *J* = 2.1 Hz, 1H), 6.88 (s, 1H), 6.85 - 6.75 (m, 4H), 6.71 (dd, *J* = 8.3, 2.1 Hz, 1H), 4.97 (s, 2H), 4.95 - 4.84 (m, 5H), 4.40 (s, 4H), 3.91 (t, *J* = 6.5 Hz, 2H), 3.44 (dt, *J* = 13.2, 6.3 Hz, 4H), 3.24 (t, *J* = 6.6 Hz, 2H), 2.88 - 2.70 (m, 3H), 2.22 (t, *J* = 7.6 Hz, 1H), 1.72 (d, *J* = 7.5 Hz, 4H), 1.62 (s, 6H), 1.42 (d, *J* = 5.2 Hz, 4H), 1.34 (d, *J* = 5.5 Hz, 8H). LC/MS (ESI+) calcd for C₅₀H₆₀N₆O₈( [M+H]⁺ ) *m*/*z:* 873.5; found 873.5_{∘}

### Example 62: 5-((3-(3-((7-(4-(2-(4-((2-(2-oxa-6-azaspiro[3.3]heptan-6-yl) pyrimidin-4-yl)methoxy)phenyl)propan-2-yl)phenoxy)heptyl)oxy)propoxy) propyl) amino)-2-(2,6-dioxopiperidin-3-yl)isoindolin-1,3-dione

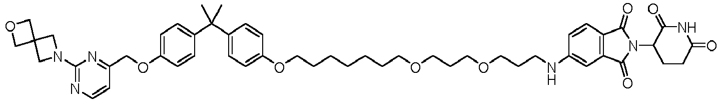

The target compound was synthesized by a method similar to that of Example 59. ¹H NMR (400 MHz, CDCl₃) *δ* 8.30 (s, 1H), 8.13 (s, 1H), 7.60 (d, *J* = 8.3 Hz, 1H), 7.17 - 7.08 (m, 4H), 6.95 (d, *J* = 2.1 Hz, 1H), 6.90 (s, 1H), 6.86 - 6.75 (m, 4H), 6.71 (dd, *J* = 8.3, 2.2 Hz, 1H), 4.97 (s, 2H), 4.95 - 4.78 (m, 5H), 4.43 (s, 4H), 3.91 (t, *J* = 6.5 Hz, 2H), 3.58 (t, *J* = 5.5 Hz, 2H), 3.52 (dt, *J* = 8.4, 6.3 Hz, 4H), 3.41 (t, *J* = 6.7 Hz, 2H), 3.33 (t, *J* = 6.3 Hz, 2H), 2.92 - 2.72 (m, 3H), 2.22 (t, *J* = 7.6 Hz, 1H), 1.92 - 1.85 (m, 4H), 1.62 (s, 6H), 1.44 - 1.40 (m, 4H), 1.30 - 1.28 (m, 6H). LC/MS (ESI+) calcd for C₅₁H₆₂N₆O₉( [M+H]⁺ ) *m*/*z:* 903.5; found 903.5.

### Example 63: 2-(2,6-dioxopiperidin-3-yl)-5-(4-((8-(4-(2-(2-(3-methylazetidin- 1-yl)pyrimidin-4-yl)methoxy)phenyl)propan-2-yl)phenoxy)octyl)oxy)butyl)amino) isoindolin-1,3-dione

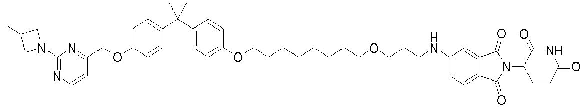

The target compound was synthesized by a method similar to that of Example 59. ¹H NMR (400 MHz, CDCl₃) *δ* 8.28 (s, 1H), 8.10 (d, *J* = 11.7 Hz, 1H), 7.59 (d, *J* = 7.9 Hz, 1H), 7.11 (td, *J* = 16.2, 7.5 Hz, 4H), 6.94 (s, 2H), 6.86 - 6.68 (m, 5H), 5.07 - 4.86 (m, 3H), 4.50 (s, 2H), 3.91 (t, *J* = 6.6 Hz, 3H), 3.61 - 3.17 (m, 7H), 2.94 - 2.74 (m, 3H), 2.38 - 2.17 (m, 1H), 2.11 (t, *J* = 7.6 Hz, 1H), 1.73 (dd, *J* = 17.7, 10.4 Hz, 6H), 1.63 (s, 6H), 1.39 (d, *J* = 36.1 Hz, 10H), 0.86 (q, *J* = 11.1, 8.6 Hz, 3H). LC/MS (ESI+) calcd for C₄₉H₆₀N₆O₇( [M+H]⁺ ) *m*/*z:* 845.5; found 845.5.

### Example 64: 2-(2,6-dioxopiperidin-3-yl)-5-((4-((8-(4-(2-(2-hydroxylethyl) (methyl)amino)pyrimidin-4-yl)methoxy)phenyl)propan-2-yl)phenoxy)octyl)oxy) butyl))isoindolin-1,3-dione

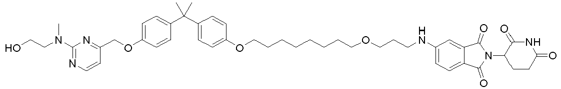

The target compound was synthesized by a method similar to that of Example 59. ¹H NMR (400 MHz, CDCl₃) *δ* 8.28 (s, 1H), 8.07 (s, 1H), 7.59 (d, *J* = 8.3 Hz, 1H), 7.17 (d, *J* = 8.7 Hz, 2H), 7.10 (t, *J* = 8.3 Hz, 4H), 6.95 (d, *J* = 2.1 Hz, 1H), 6.83 - 6.77 (m, 4H), 6.72 (dd, *J* = 8.3, 2.2 Hz, 1H), 5.06 (s, 2H), 4.92 (dd, *J* = 12.0, 5.2 Hz, 1H), 4.02 (d, *J* = 26.4 Hz, 4H), 3.91 (t, *J* = 6.5 Hz, 2H), 3.45 (dd, *J* = 11.8, 6.3 Hz, 4H), 3.37 (s, 3H), 3.24 (t, *J* = 6.6 Hz, 2H), 2.91 - 2.72 (m, 3H), 2.11 (dd, *J* = 12.2, 5.8 Hz, 1H), 1.73 (dq, *J* = 21.8, 7.0 Hz, 8H), 1.63 (s, 6H), 1.43 (d, *J* = 3.9 Hz, 2H), 1.34 (d, *J* = 5.4 Hz, 6H). LC/MS (ESI+) calcd for C₄₈H₆₀N₆O₈( [M+H]⁺ ) *m*/*z:* 849.5; found 849.5.

### Example 65: 5-((4-((8-(4-(2-(4-(2-(2-oxa-6-azaspiro[3.3]heptan-6-yl)-5,8-dihydropyrido[3,4-d]pyrimidin-7(6H)-yl)phenyl)propan-2-yl)phenoxy)octyl)oxy) butyl)amino)-2-(2,6-dioxopiperidin-3-yl)isoindolin-1,3-dione

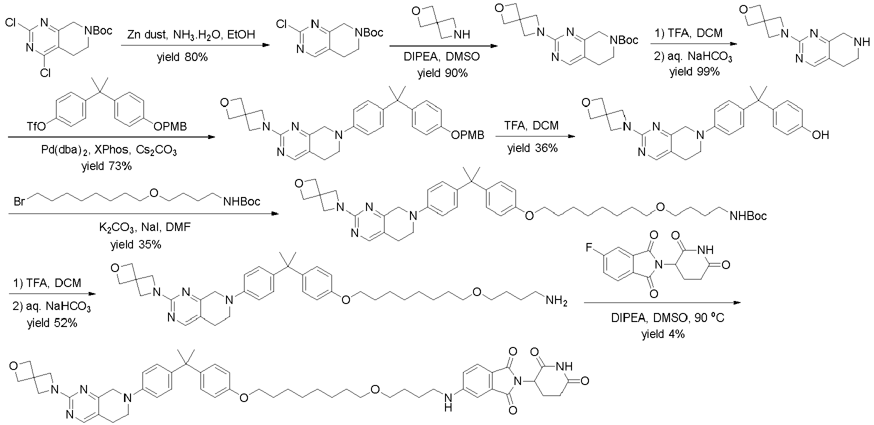

### Step 1: Synthesis of tert-butyl 2-chloro-5,8-dihydropyrido[3,4-d]pyrimidin- 7(6H)-carboxylate

tert-butyl 2,4-dichloro-5,8-dihydropyrido[3,4-*d*]pyrimidin-7(6*H*)-carboxylate (13 g, 42 mmol) was dissolved in 150 mL of absolute ethanol, to which were successively added zinc powder (3.2 g, 50 mmol) and 30 mL of ammonia, and then the system was allowed to react overnight at 80 °C. After completion of the reaction detected by TLC, the reaction solution was filtered with diatomite, and to the filtrate, was added 100 mL of water. The resultant solution was extracted twice with 100 mL of ethyl acetate. The organic phase was successively washed with 0.5 N HCl aqueous solution (2*50 mL), saturated NaHCO₃ solution (2*50 mL), and saturated brine (2*50 mL), dried with anhydrous Na₂SO₄, and concentrated. The residue was separated and purified by column chromatography, to provide 9 g of target compound, with a yield of 80%.

### Step 2: Synthesis of tert-butyl 2-(2-oxa-6-azaspiro[3.3]heptan-6-yl)-5,8- dihydropyrido[3,4-d]pyrimidin-7(6H)-carboxylate

tert-butyl 2-chloro-5,8-dihydropyrido[3,4-*d*]pyrimidin-7(6*H*)-carboxylate (1 g, 3.7 mmol), 2-oxa-6-azaspiro[3.3]heptane (380 mg, 3.7 mmol) and DIPEA (1.3 g, 10 mmol) were successively added into 10 mL of DMSO, and then allowed to react overnight at 80 °C. After completion of the reaction detected by TLC, the resultant solution was extracted with 10 mL of ethyl acetate. The organic phase was washed with saturated brine (3*10 mL), dried with anhydrous Na₂SO₄, and concentrated. The residue was separated and purified by column chromatography, to provide 1.1 g of target compound, with a yield of 90%.

### Step 3: Synthesis of 6-(5,6,7,8-tetrahydropyrido[3,4-d]pyrimidin-2-yl)-2-oxa-6-azaspiro[3.3]heptane

Compound tert-butyl 2-(2-oxa-6-azaspiro[3.3]heptan-6-yl)-5,8- dihydropyrido[3,4-*d*]pyrimidin-7(6*H*)-carboxylate (1.1 g, 3.3 mmol) was dissolved in 10 mL of dichloromethane, to which was added 5 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 800 mg of target compound, with a yield of 99%.

### Step 4: Synthesis of 6-(7-(4-(2-(4-(4-methoxybenzyl)oxy)phenyl)propan-2-yl) phenyl)-5,6,7,8-tetrahydropyrido[3,4-d]pyrimidin-2-yl)-2-oxa-6-azaspiro[3.3]heptan

Compound 6-(5,6,7,8-tetrahydropyrido[3,4-d]pyrimidin-2-yl)-2-oxa-6-azaspiro[3.3]heptane (400 mg, 1.7 mmol), 4-(2-(4-((4-methoxybenzyl)oxy)phenyl) propan-2-yl)phenyl trifluoromethanesulfonate (816 mg, 1.7 mmol), Pd(dba)₂ (96 mg, 0.17 mmol), XPhos (161 mg, 0.34 mmol), and cesium carbonate (1.1 g, 3.4 mmol) were added into 10 mL of dioxane, and then the reaction system was purged with helium and reacted overnight at 110 °C under helium atmosphere. After completion of the reaction detected by TLC, the reaction system was filtered over diatomaceous earth, and concentrated. The residue was separated and purified by column chromatography, to provide 700 mg of target compound, with a yield of 73%.

### Step 5: Synthesis of 4-(2-(2-oxa-6-azaspiro[3.3]heptan-6-yl)-5,8-dihydropyrido [3,4-d]pyrimidin-7(6H)-yl)phenyl)propan-2-yl)phenol

Compound 6-(7-(4-(2-(4-(4-methoxybenzyl)oxy)phenyl)propan-2-yl)phenyl)- 5,6,7,8-tetrahydropyrido[3,4-*d*]pyrimidin-2-yl)-2-oxa-6-azaspiro[3.3]heptan (700 mg, 1.2 mmol) was dissolved in 10 mL of dichloromethane, to which was added 5 mL of trifluoroacetic acid in an ice bath, and then the mixture was stirred for 0.5 h at room temperature. After completion of the reaction detected by TLC, the reaction system was concentrated. The residue was separated and purified by column chromatography, to provide 190 mg of target compound, with a yield of 36%.

In the following steps, the target compound was synthesized by a method similar to that of Example 59. ¹H NMR (400 MHz, CDCl₃) *δ* 7.58 (s, 1H), 7.20 - 7.07 (m, 4H), 6.97 (d, *J* = 19.3 Hz, 2H), 6.88 - 6.64 (m, 5H), 4.86 (s, 2H), 4.56 (s, 2H), 4.40 (s, 2H), 3.91 (d, *J* = 6.8 Hz, 2H), 3.85 (s, 2H), 3.76 (s, 2H), 3.57 (s, 2H), 3.44 (dd, *J* = 12.4, 6.0 Hz, 4H), 3.24 (s, 2H), 2.94 - 2.70 (m, 5H), 2.22 (t, *J* = 7.6 Hz, 1H), 1.75 (s, 8H), 1.63 (s, 6H), 1.42 (d, *J* = 5.3 Hz, 2H), 1.34 (d, *J* = 5.6 Hz, 6H). LC/MS (ESI+) calcd for C₅₂H₆₃N₇O₇( [M+H]⁺ ) *m*/*z:* 898.5; found 898.5.

### Example 66: 2-(2,6-dioxopiperidin-3-yl)-5-((3-(4-(2-(methylthio)pyrimidin-4-yl) methoxy)phenyl)propan-2-yl)phenoxy)propyl)amino)isoindolin-1,3-dione

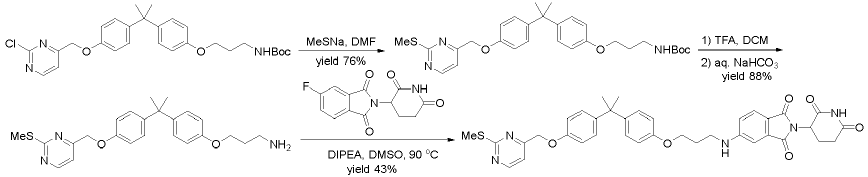

### Step 1: Synthesis of (3-(4-(2-(4-((2-(methylthio)pyrimidin-4-yl)methoxy)phenyl) propan-2-yl)phenoxy)propyl)carbamate tert-butyl

tert-butyl (3-(4-(2-(4-((2-chloropyrimidin-4-yl)methoxy)phenyl)propan-2-yl) phenoxy)propyl)carbamate (511 mg, 1 mmol) and sodium thiomethoxide (144 mg, 2 mmol) were successively added into 4 mL of DMF, and then the mixture was allowed to react overnight at room temperature. After completion of the reaction detected by TLC, the resultant solution was extracted with 4 mL of ethyl acetate. The organic phase was washed with saturated brine (3*4 mL), dried with anhydrous Na₂SO₄, and concentrated. The residue was separated and purified by column chromatography, to provide 400 mg of target compound, with a yield of 76%.

### Step 2: Synthesis of 3-(4-(2-(4-(2-(methylthio)pyrimidin-4-yl)methoxy)phenyl) propan-2-yl)phenoxy)propan-1-amine

Compound tert-butyl (3-(4-(2-(4-((2-(methylthio)pyrimidin-4-yl)methoxy)phenyl) propan-2-yl)phenoxy)propyl)carbamate (150 mg, 0.28 mmol) was dissolved in 2 mL of dichloromethane, to which was added 1 mL trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 104 mg of target compound, with a yield of 88%.

### Step 3: Synthesis of 2-(2,6-dioxopiperidin-3-yl)-5-((3-(4-(2-(methylthio)pyrimidin- 4-yl)methoxy)phenyl)propan-2-yl)phenoxy)propyl)amino)isoindolin-1,3-dione

3-(4-(2-(4-(2-(methylthio)pyrimidin-4-yl)methoxy)phenyl)propan-2-yl)phenoxy)propan-1-amine (104 mg, 0.24 mmol), 2-(2,6-dioxopiperidin-3-yl)-5-fluoroisoindolin- 1,3-dione (66 mg, 0.24 mmol) and DIPEA(94 mg, 0.72 mmol) were successively added into 5 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 70 mg of target compound, with a yield of 43%. LC/MS (ESI+) calcd for C₃₇H₃₇N₅O₆S( [M+H]⁺ ) *m*/*z:* 680.3; found 680.3.

### Example 67: 2-(2,6-dioxopiperidin-3-yl)-5-((3-(4-(2-(4-(4-(2-(methanesulfonyl) pyrimidin-4-yl)methoxy)phenyl)propan-2-yl)phenoxy)propyl)amino)isoindolin-1,3-dione

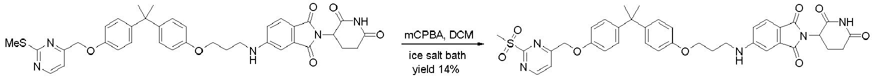

2-(2,6-dioxopiperidin-3-yl)-5-((3-(4-(2-(methylthio)pyrimidin-4-yl)methoxy)phenyl)propan-2-yl)phenoxy)propyl)amino)isoindolin-1,3-dione (50 mg, 0.07 mmol) was added into 5 mL of dichloromethane, to which was added m-chloroperoxybenzoic acid (24 mg, 0.14 mmol) in an ice-salt bath, and then the mixture was further stirred in the ice-salt bath for 1h. TLC detection indicated completion of the reaction, and then saturated NaHSO₃ solution was added to quench the reaction in the ice-salt bath. The resultant solution was extracted with 5 mL of dichloromethane. The organic phase was washed with saturated brine, dried with anhydrous Na₂SO₄, and concentrated. The residue was separated and purified by column chromatography to provide 7 mg compound 2-(2,6-dioxopiperidin-3-yl)-5-((3-(4-(2-(4-(4-(2-(methanesulfonyl)pyrimidin-4-yl)methoxy)phenyl)propan-2-yl)phenoxy)propyl)amino)isoindolin-1,3-dione, with a yield of 14%. ¹H NMR (400 MHz, CDCl₃) *δ* 8.91 (d, *J* = 5.1 Hz, 1H), 8.36 (s, 1H), 7.83 (d, *J* = 5.1 Hz, 1H), 7.21 - 7.11 (m, 4H), 6.97 (d, *J* = 2.1 Hz, 1H), 6.89 - 6.77 (m, 4H), 6.73 (dd, *J* = 8.3, 2.1 Hz, 1H), 5.26 (s, 2H), 4.93 (dd, *J* = 11.9, 5.2 Hz, 1H), 4.09 (t, *J* = 5.5 Hz, 2H), 3.46 (t, *J* = 6.5 Hz, 2H), 3.38 (s, 3H), 2.91 - 2.77 (m, 3H), 2.13 (t, *J* = 6.4 Hz, 3H), 1.64 (s, 6H). LC/MS (ESI+) calcd for C₃₇H₃₇N₅O₈S( [M+H]⁺ ) *m*/*z:* 712.2; found 712.2.

### Example 68: 2-(2,6-dioxopiperidin-3-yl)-5-((3-(4-(2-(4-((2-methoxypyrimidin- 4-yl)methoxy)phenyl)propan-2-yl)phenoxy)propyl)amino)isoindolin-1,3-dione

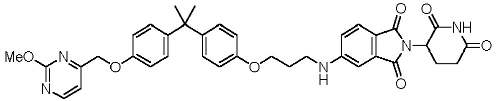

The target compound was synthesized by a method similar to that of Example 66. ¹H NMR (400 MHz, CDCl₃) *δ* 8.51 (d, *J* = 5.0 Hz, 1H), 8.07 (s, 1H), 7.60 (d, *J* = 8.3 Hz, 1H), 7.19 (dd, *J* = 5.0, 0.8 Hz, 1H), 7.17 - 7.11 (m, 4H), 6.97 (d, *J* = 2.2 Hz, 1H), 6.87 - 6.79 (m, 4H), 6.74 (dd, *J* = 8.3, 2.2 Hz, 1H), 5.06 (d, *J* = 0.7 Hz, 2H), 4.94 (d, *J* = 5.3 Hz, 1H), 4.13 - 4.05 (m, 2H), 4.02 (s, 3H), 3.46 (q, *J* = 6.2 Hz, 2H), 2.94 - 2.70 (m, 3H), 2.16 - 2.07 (m, 3H), 1.63 (s, 6H). LC/MS (ESI+) calcd for C₃₇H₃₇N₅O₇( [M+H]⁺ ) *m*/*z:* 664.3; found 664.3.

### Example 69: 2-(2,6-dioxopiperidin-3-yl)-5-((3-(4-(2-(4-((2-ethoxypyrimidin-4-yl) methoxy)phenyl)propan-2-yl)phenoxy)propyl)amino)isoindolin-1,3-dione

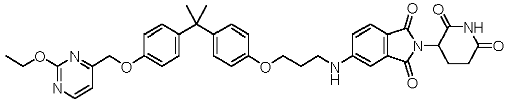

The target compound was synthesized by a method similar to that of Example 66. ¹H NMR (400 MHz, CDCl₃) *δ* 8.50 (s, 1H), 8.08 (s, 1H), 7.60 (d, *J* = 8.2 Hz, 1H), 7.15 (dd, *J* = 8.7, 2.3 Hz, 5H), 6.97 (d, *J* = 1.8 Hz, 1H), 6.83 (dd, *J* = 11.6, 8.6 Hz, 4H), 6.78 - 6.71 (m, 1H), 5.05 (s, 2H), 4.93 (dd, *J* = 12.1, 5.2 Hz, 1H), 4.44 (q, *J* = 7.0 Hz, 2H), 4.09 (t, *J* = 5.4 Hz, 2H), 3.46 (t, *J* = 6.3 Hz, 2H), 2.92 - 2.72 (m, 3H), 2.18 - 2.07 (m, 3H), 1.63 (s, 6H), 1.44 (t, *J* = 7.0 Hz, 3H). LC/MS (ESI+) calcd for C₃₈H₃₉N₅O₇( [M+H]⁺ ) *m*/*z:* 678.3; found 678.3.

### Example 70: 5-((3-(4-(2-(4-((2-cyclopropyloxypyrimidin-4-yl)methoxy)phenyl) propan-2-yl)phenoxy)propyl)amino)-2-(2,6-dioxopiperidin-3-yl)isoindolin-1,3-dione

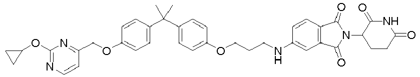

The target compound was synthesized by a method similar to that of Example 66. ¹H NMR (400 MHz, CDCl₃) *δ* 8.54 (s, 1H), 8.07 (s, 1H), 7.60 (d, *J* = 8.3 Hz, 1H), 7.22 (s, 1H), 7.15 (dd, *J* = 8.7, 2.1 Hz, 4H), 6.98 (d, *J* = 2.0 Hz, 1H), 6.91 - 6.77 (m, 4H), 6.78 - 6.70 (m, 1H), 5.06 (s, 2H), 4.93 (dd, *J* = 12.0, 5.2 Hz, 1H), 4.36 (d, *J* = 3.6 Hz, 1H), 4.09 (t, *J* = 5.4 Hz, 2H), 3.46 (t, *J* = 6.4 Hz, 2H), 2.94 - 2.67 (m, 3H), 2.12 (q, *J* = 6.7, 6.1 Hz, 3H), 1.63 (s, 6H), 0.88 - 0.81 (m, 4H). LC/MS (ESI+) calcd for C₃₉H₃₉N₅O₇( [M+H]⁺ ) *m*/*z:* 690.3; found 690.3.

### Example 71: 2-(2,6-dioxopiperidin-3-yl)-5-((3-(4-(2-(4-((2-morpholinepyrimidin- 4-yl)methoxy)phenyl)propan-2-yl)phenoxy)propyl)amino)isoindolin-1,3-dione

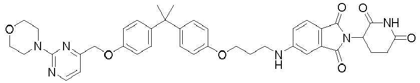

The target compound was synthesized by a method similar to that of Example 61. ¹H NMR (400 MHz, CDCl₃) *δ* 8.31 (d, *J* = 5.0 Hz, 1H), 8.13 (s, 1H), 7.60 (d, *J* = 8.3 Hz, 1H), 7.21 - 7.05 (m, 4H), 6.97 (d, *J* = 2.1 Hz, 1H), 6.89 - 6.78 (m, 4H), 6.78 - 6.71 (m, 2H), 4.98 - 4.89 (m, 3H), 4.08 (t, *J* = 5.5 Hz, 2H), 3.97 - 3.63 (m, 8H), 3.45 (d, *J* = 7.5 Hz, 2H), 2.92 - 2.66 (m, 3H), 2.13 (p, *J* = 7.6, 7.0 Hz, 3H), 1.63 (s, 6H). LC/MS (ESI+) calcd for C₄₀H₄₂N₆O₇( [M+H]⁺ ) *m*/*z:* 719.3; found 719.3.

### Example 72: 2-((2,6-dioxopiperidin-3-yl)-5-(3-(4-(2-(4-methylpiperazin-1-yl) pyrimidin-4-yl)methoxy)phenyl)propan-2-yl)phenoxy)propyl)amino)isoindolin-1,3-dione

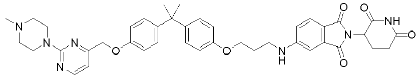

The target compound was synthesized by a method similar to that of Example 61. ¹H NMR (400 MHz, CDCl₃) *δ* 8.28 (d, *J* = 5.0 Hz, 1H), 7.58 (d*, J* = 8.3 Hz, 1H), 7.17 - 7.08 (m, 4H), 6.96 (d, *J* = 2.2 Hz, 1H), 6.81 (t, *J* = 8.6 Hz, 4H), 6.74 - 6.66 (m, 2H), 4.91 (s, 2H), 4.89 (d, *J* = 5.7 Hz, 1H), 4.07 (t, *J* = 5.5 Hz, 2H), 3.85 (t, *J* = 5.0 Hz, 4H), 3.43 (q, *J* = 6.2 Hz, 2H), 2.94 - 2.67 (m, 3H), 2.49 (t, *J* = 5.1 Hz, 4H), 2.34 (s, 3H), 2.12 (q, *J* = 5.8 Hz, 3H), 1.62 (s, 6H). LC/MS (ESI+) calcd for C₄₁H₄₅N₇O₆( [M+H]⁺ ) *m*/*z:* 732.4; found 732.4.

### Example 73: 5-((3-(4-(2-(4-((2-(2-oxa-6-azaspiro[3.3]heptan-6-yl)pyrimidin-4-yl) methoxy)phenyl)propan-2-yl)phenoxy)propyl)amino)-2-(2,6-dioxopiperidin-3-yl)isoindolin-1,3-dione

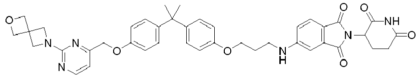

The target compound was synthesized by a method similar to that of Example 61. ¹H NMR (400 MHz, CDCl₃) *δ* 8.33 (s, 1H), 8.10 (s, 1H), 7.60 (d, *J* = 7.2 Hz, 1H), 7.14 (s, 4H), 6.98 (s, 1H), 6.82 (s, 4H), 6.76 (s, 1H), 4.94 (d, *J* = 29.0 Hz, 7H), 4.64 - 4.18 (m, 4H), 4.09 (s, 2H), 3.47 (s, 2H), 2.96 - 2.69 (m, 3H), 2.13 (s, 3H), 1.64 (s, 6H). LC/MS (ESI+) calcd for C₄₁H₄₂N₆O₇( [M+H]⁺ ) *m*/*z:* 731.3; found 731.3.

### Example 74: 5-((3-(4-(2-(4-((2-aminopyrimidin-4-yl)methoxy)phenyl)propan- 2-yl)phenoxy)propyl)amino)-2-(2,6-dioxopiperidin-3-yl)isoindolin-1,3-dione

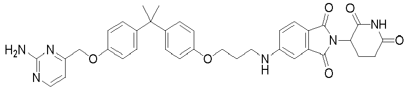

The target compound was synthesized by a method similar to that of Example 61. ¹H NMR (400 MHz, CDCl₃) *δ* 8.75 (s, 1H), 8.30 (s, 1H), 7.55 (dd, *J* = 27.3, 8.3 Hz, 1H), 7.20 - 7.02 (m, 4H), 6.99 - 6.86 (m, 2H), 6.87 - 6.67 (m, 5H), 5.05 - 4.84 (m, 3H), 4.15 - 3.98 (m, 2H), 3.48 (dt, *J* = 19.3, 6.7 Hz, 2H), 2.95 - 2.67 (m, 3H), 2.16 - 2.05 (m, 3H), 1.62 (d, *J* = 2.7 Hz, 6H). LC/MS (ESI+) calcd for C₃₆H₃₆N₆O₆( [M+H]⁺) *m*/*z:* 649.3; found 649.3.

### Example 75: 5-(((1r,3r)-3-(4-(2-(4-((2-(2-oxa-6-azaspiro[3.3]heptane-6-yl) pyrimidin-4-yl)methoxy)phenyl)propan-2-yl)phenoxy)cyclobutyl)amino)-2-(2,6-dioxopiperidin-3-yl)isoindolin-1,3-dione

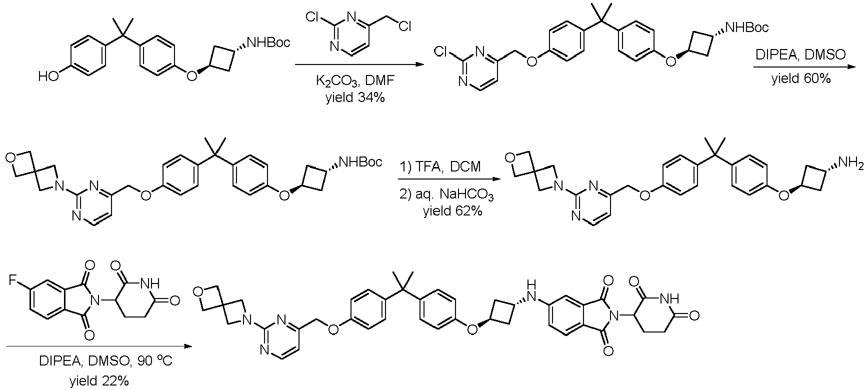

### Step 1: Synthesis of tert-butyl ((1r,3r)-3-(4-(2-(4-((2-chloropyrimidin-4-yl)methoxy) phenyl)propan-2-yl)phenoxy)cyclobutyl)carbamate

tert-butyl ((1*r*,3*r*)-3-(4-(2-(4-hydroxylphenyl)propan-2-yl)phenoxy)cyclobutyl) carbamate (400 mg, 1 mmol), 2-chloro-4-(chloromethyl)pyrimidine (162 mg, 1 mmol) and K₂CO₃ (276 mg, 2 mmol) were successively added into 5 mL of DMF, and then the mixture was allowed to react overnight at room temperature. After completion of the reaction detected by TLC, the resultant solution was extracted with 20 mL of ethyl acetate. The organic phase was washed with saturated brine (3*10 mL), dried with anhydrous Na₂SO₄, and concentrated. The residue was separated and purified by column chromatography, to provide 180 mg of target compound, with a yield of 34%.

### Step 2: Synthesis of tert-butyl ((1r,3r)-3-(4-(2-(4-((2-(2-oxa-6-azaspiro[3.3]heptan- 6-yl)pyrimidin-4-yl)methoxy)phenyl)propan-2-yl)phenoxy)cyclobutyl)carbamate

tert-butyl ((1*r*,3*r*)-3-(4-(2-(4-((2-chloropyrimidin-4-yl)methoxy)phenyl)propan- 2-yl)phenoxy)cyclobutyl)carbamate (180 mg, 0.34 mmol), 2-oxa-6- azaspiro[3.3]heptane (35 mg, 0.34 mmol) and DIPEA (90 mg, 0.7 mmol) were successively added into 2 mL of DMSO, and then allowed to react overnight at 80 °C. After completion of the reaction detected by TLC, the resultant solution was extracted with 5 mL of ethyl acetate. The organic phase was washed with saturated brine (3*5 mL), dried with anhydrous Na₂SO₄, and concentrated. The residue was separated and purified by column chromatography, to provide 120 mg of target compound, with a yield of 60%.

### Step 3: Synthesis of (1r,3r)-3-(4-(2-(4-((2-(2-oxa-6-azaspiro[3.3]heptan-6-yl) pyrimidin-4-yl)methoxy)phenyl)propan-2-yl)phenoxy)cyclobutylamine

Compound tert-butyl ((1*r*,3*r*)-3-(4-(2-(4-((2-(2-oxa-6-azaspiro[3.3]heptan-6-yl) pyrimidin-4-yl)methoxy)phenyl)propan-2-yl)phenoxy)cyclobutyl)carbamate (120 mg, 0.2 mmol) was dissolved in 2 mL of dichloromethane, to which was added 1 mL trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 60 mg of target compound, with a yield of 62%.

### Step 4: Synthesis of 5-(((1r,3r)-3-(4-(2-(4-((2-(2-oxa-6-azaspiro[3.3]heptane-6-yl) pyrimidin-4-yl)methoxy)phenyl)propan-2-yl)phenoxy)cyclobutyl)amino)-2-(2,6-dioxopiperidin-3-yl)isoindolin-1,3-dione

(1*r*,3*r*)-3-(4-(2-(4-((2-(2-oxa-6-azaspiro[3.3]heptan-6-yl)pyrimidin-4-yl)methoxy)phenyl)propan-2-yl)phenoxy)cyclobutylamine (60 mg, 0.12 mmol), 2-(2,6-dioxopiperidin-3-yl)-5-fluoroisoindolin-1,3-dione (30 mg, 0.12 mmol) and DIPEA (52 mg, 0.4 mmol) were successively added into 5 mL of DMSO, and then the mixture was heated to 60 °C and reacted overnight. The reaction solution was naturally cooled to room temperature, and then extracted with 5 mL of ethyl acetate. The organic phase was washed with saturated brine (3*5 mL), dried with anhydrous Na₂SO₄, and concentrated. The residue was separated and purified by column chromatography to provide 20 mg of target compound, with a yield of 22%. ¹H NMR (400 MHz, CDCl₃) *δ* 8.46 (s, 1H), 8.39 (s, 1H), 7.59 (d, *J* = 8.1 Hz, 1H), 7.12 (dd, *J* = 8.5, 4.9 Hz, 4H), 6.96 - 6.77 (m, 4H), 6.69 (t, *J* = 8.6 Hz, 3H), 4.90 (d, *J* = 34.4 Hz, 7H), 4.49 (p, *J* = 7.0 Hz, 1H), 4.31 (s, 4H), 3.74 (q, *J* = 7.4 Hz, 1H), 3.10 (dt, *J* = 13.1, 6.8 Hz, 2H), 2.94 - 2.64 (m, 3H), 2.08 (p, *J* = 7.7, 7.3 Hz, 3H), 1.62 (s, 6H). LC/MS (ESI+) calcd for C₄₂H₄₂N₆O₇( [M+H]⁺) *m*/*z:* 743.3; found 743.3.

### Example 76: 5-(((1s,3s)-3-(4-(2-(4-((2-(2-oxa-6-azaspiro[3.3]heptane-6-yl) pyrimidin-4-yl)methoxy)phenyl)propan-2-yl)phenoxy)cyclobutyl)amino)-2-(2,6-dioxopiperidin-3-yl)isoindolin-1,3-dione

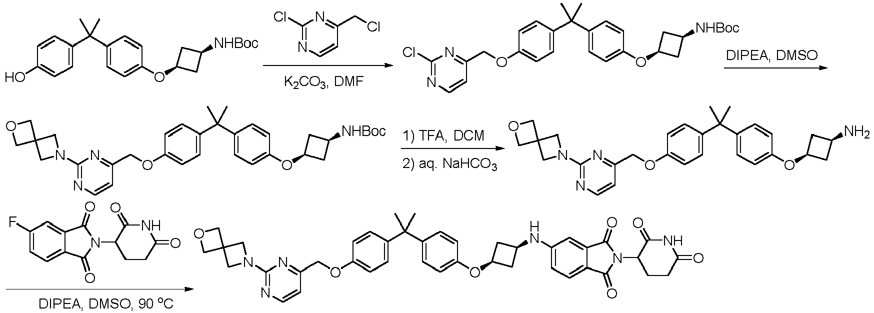

The target compound was synthesized by a method similar to that of Example 75. ¹H NMR (400 MHz, DMSO-*d*₆) *δ* 11.07 (s, 1H), 8.33 (d, *J* = 4.9 Hz, 1H), 7.59 (d, *J* = 8.3 Hz, 1H), 7.44 (d, *J* = 6.4 Hz, 1H), 7.15 - 7.03 (m, 4H), 6.89 (dd, *J* = 9.2, 2.3 Hz, 3H), 6.82 (dd, *J* = 8.3, 2.1 Hz, 1H), 6.76 (dd, *J* = 9.8, 3.5 Hz, 3H), 5.04 (dd, *J* = 13.0, 5.3 Hz, 1H), 4.95 (s, 2H), 4.71 (s, 4H), 4.48 (p, *J* = 7.1 Hz, 1H), 4.19 (s, 4H), 3.77 (q, *J* = 7.4 Hz, 1H), 3.14 - 2.98 (m, 2H), 2.87 (ddd, *J* = 17.3, 14.0, 5.4 Hz, 1H), 2.68 - 2.51 (m, 2H), 2.02 (d, *J* = 7.7 Hz, 1H), 1.95 (d, *J* = 9.9 Hz, 2H), 1.57 (s, 6H). LC/MS (ESI+) calcd for C₄₂H₄₂N₆O₇( [M+H]⁺) *m*/*z:* 743.3; found 743.3.

### Example 77: 5-((3-(4-(2-(4-((2-(2-oxa-7-azaspiro[3.5]nonan-7-yl)pyrimidin- 4-yl)methoxy)phenyl)propan-2-yl)phenoxy)propyl)amino)-2-(2,6-dioxopiperidin-3-yl)isoindolin-1,3-dione

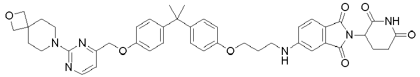

The target compound was synthesized by a method similar to that of Example 61. ¹H NMR (400 MHz, DMSO-d₆) *δ* 11.06 (s, 1H), 8.33 (d, *J* = 4.9 Hz, 1H), 7.56 (d, *J* = 8.4 Hz, 1H), 7.20 (t, *J* = 5.5 Hz, 1H), 7.16 - 7.06 (m, 4H), 6.98 (d, *J* = 2.1 Hz, 1H), 6.94 - 6.76 (m, 5H), 6.65 (d, *J* = 5.0 Hz, 1H), 5.03 (dd, *J* = 12.9, 5.4 Hz, 1H), 4.94 (s, 2H), 4.34 (s, 4H), 4.07 - 3.98 (m, 2H), 3.74 - 3.61 (m, 4H), 3.32 (d, *J* = 9.2 Hz, 2H), 2.87 (ddd, *J* = 17.4, 14.0, 5.4 Hz, 1H), 2.63 - 2.51 (m, 2H), 2.03 - 1.94 (m, 3H), 1.83 - 1.72 (m, 4H), 1.57 (s, 6H). LC/MS (ESI+) calcd for C₄₃H₄₆N₆O₇( [M+H]⁺) *m*/*z:* 759.4; found 759.4.

### Example 78: 5-((3-(4-(2-(4-((2-(2-oxa-7-azaspiro[3.5]nonan-7-yl)pyrimidin- 4-yl)methoxy)phenyl)propan-2-yl)phenoxy)cyclobutyl)amino)-2-(2,6-dioxopiperidin-3-yl)isoindolin-1,3-dione

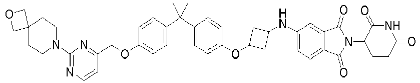

The target compound was synthesized by a method similar to that of Example 75. ¹H NMR (400 MHz, CDCl₃) *δ* 8.29 (d, *J* = 4.8 Hz, 1H), 8.15 (d, *J* = 5.5 Hz, 1H), 7.62 (dd, *J* = 8.2, 2.2 Hz, 1H), 7.13 (dd, *J* = 8.9, 5.8 Hz, 4H), 6.89 (dd, *J* = 9.5, 2.0 Hz, 1H), 6.83 (d, *J* = 8.4 Hz, 2H), 6.76 - 6.66 (m, 4H), 4.97 - 4.88 (m, 3H), 4.86 (s, 1H), 4.50 (s, 4H), 4.21 (s, 1H), 3.78 (t, *J* = 5.5 Hz, 4H), 3.18 - 3.05 (m, 1H), 2.92 - 2.64 (m, 4H), 2.40 (dd, *J* = 12.2, 6.3 Hz, 1H), 2.16 - 2.06 (m, 2H), 1.91 (t, *J* = 5.5 Hz, 4H), 1.63 (s, 6H). LC/MS (ESI+) calcd for C₄₄H₄₆N₆O₇( [M+H]⁺) *m*/*z*: 771.4; found 771.4.

### Example 79: 2-(2,6-dioxopiperidin-3-yl)-5-((3-(4-(2-(4-((2-(7-methyl-2,7-diazaspiro[4.4]nonan-2-yl)pyrimidin-4-yl)methoxy)phenyl)propan-2-yl)phenoxy)cyclobutyl)amino)isoindolin-1,3-dione

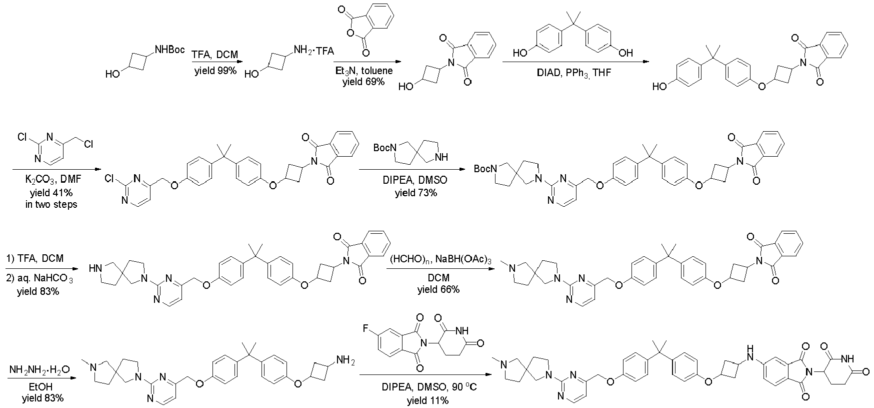

### Step 1: Synthesis of 3-aminocyclobutan-1-ol trifluoroacetic acid

Compound 3-hydroxylcyclobutylamine-1-butoxycarbonylamino (3.6 g, 20 mmol) was dissolved in 40 mL of dichloromethane, to which was added 20 mL of trifluoroacetic acid in an ice bath, and then the mixture was stirred at room temperature for 1h. After completion of the reaction detected by TLC, the reaction system was concentrated to provide 4.1 g of crude target compound.

### Step 2: Synthesis of 2-(3-hydroxylcyclobutyl)isoindolin-1,3-dione

Crude 3-aminocyclobutan-1-ol trifluoroacetate (4.1 g), phthalic anhydride (3 g, 20 mmol) and triethylamine (10 g, 100 mmol) were successively added into 50 mL of toluene, and then the mixture was allowed to react overnight at 100 °C. After completion of the reaction detected by TLC, the reaction solution was concentrated, to which was added 50 mL of water, and then the resultant solution was extracted with ethyl acetate (2*50 mL). The organic phase was washed with saturated brine (2*50 mL), dried with anhydrous Na₂SO₄, and concentrated. The residue was separated and purified by column chromatography, to provide 3 g of target compound, with a yield of 69% for two steps.

### Step 3: Synthesis of 2-(3-(4-(2-(4-hydroxylphenyl)propan-2-yl)phenoxy) cyclobutyl)isoindolin-1,3-dione

Compound 2-(3-hydroxylcyclobutyl)isoindolin-1,3-dione (3 g, 13.8 mmol), bisphenol A (3.1 g, 13.8 mmol), and triphenylphosphine (4 g, 15 mmol) were dissolved in 50 mL of tetrahydrofuran, to which was added DIAD (3 g, 15 mmol) in an ice bath, and then the mixture was stirred for half an hour in the ice bath. Subsequently, the mixture was allowed to react overnight at 65 °C. After completion of the reaction detected by TLC, the reaction solvent was removed by concentration. The residue was separated and purified by column chromatography, to provide 12 g of crude target compound.

### Step 4: Synthesis of 2-(3-(4-(2-(4-((2-chloropyrimidin-4-yl)methoxy)phenyl)propan- 2-yl)phenoxy)cyclobutyl)isoindolin-1,3-dione

Crude 2-(3-(4-(2-(4-hydroxylphenyl)propan-2-yl)phenoxy)cyclobutyl)isoindolin- 1,3-dione, 2-chloro-4-(chloromethyl)pyrimidine (2.2 g, 13.8 mmol) and K₂CO₃ (4.1 g, 30 mmol) were successively added into 100 mL of DMF, and then the mixture was allowed to react overnight at room temperature. After completion of the reaction detected by TLC, the resultant solution was extracted with 100 mL of ethyl acetate. The organic phase was washed with saturated brine (3*10 mL), dried with anhydrous Na₂SO₄, and concentrated. The residue was separated and purified by column chromatography, to provide 3.1 g of target compound, with a yield of 41% for two steps.

### Step 5: Synthesis of 7-(4-(2-(4-(4-(3-(1,3-dioxoisoindolin-2-yl)cyclobutyloxy)phenyl)propan-2-yl)phenoxy)pyrimidin-2-yl)-2,7-diazaspiro[4.4]nonan-2-carboxylate tert-butyl

2-(3-(4-(2-(4-((2-chloropyrimidin-4-yl)methoxy)phenyl)propan-2-yl)phenoxy)cyclobutyl)isoindolin-1,3-dione (500 mg, 0.9 mmol), tert-butyl 2,7-diazaspiro[4.4] nonane-2-carboxylate (226 mg, 1 mmol) and DIPEA (260 mg, 2 mmol) were successively added into 10 mL of DMSO, and then the mixture was allowed to react overnight at 80 °C. After completion of the reaction detected by TLC, the resultant solution was extracted with 10 mL of ethyl acetate. The organic phase was washed with saturated brine (3*10 mL), dried with anhydrous Na₂SO₄, and concentrated. The residue was separated and purified by column chromatography, to provide 488 mg of target compound, with a yield of 73%.

### Step 6: Synthesis of 2-(3-(4-(2-(4-((2-(2,7-diazaspiro[4.4]nonan-2-yl)pyrimidin- 4-yl)methoxy)phenyl)propan-2-yl)phenoxy)cyclobutyl)isoindolin-1,3-dione

tert-butyl 7-(4-(2-(4-(4-(3-(1,3-dioxoisoindolin-2-yl)cyclobutyloxy)phenyl) propan-2-yl)phenoxy)pyrimidin-2-yl)-2,7-diazaspiro[4.4]nonan-2-carboxylate (288 mg, 0.38 mmol) was dissolved in 2 mL of dichloromethane, to which was added 1 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and then extracted with dichloromethane/methanol (10:1) (3*10 mL). The organic phase was washed with saturated brine, dried with anhydrous Na₂SO₄, and concentrated. The residue was separated and purified by column chromatography, to provide 204 mg of target compound, with a yield of 83%.

### Step 7: Synthesis of 2-(3-(4-(2-(4-((2-(7-methyl-2,7-diazaspiro[4.4]nonan-2-yl) pyrimidin-4-yl)methoxy)phenyl)propan-2-yl)phenoxy)cyclobutyl)isoindolin-1,3-dione

Compound 2-(3-(4-(2-(4-((2-(2,7-diazaspiro[4.4]nonan-2-yl)pyrimidin-4-yl) methoxy)phenyl)propan-2-yl)phenoxy)cyclobutyl)isoindolin-1,3-dione (204 mg, 0.32 mmol) was added into 5 mL of dichloromethane, to which were successively added paraformaldehyde (270 mg, 3 mmol) and glacial acetic acid (2 mg, 0.03 mmol), and then the mixture was stirred for 10 min at room temperature, followed by addition of sodium triacetoxyborohydride (135 mg, 0.64 mmol). Subsequently, the mixture was stirred overnight at room temperature. After completion of the reaction detected by TLC, the reaction system was poured into saturated NH₄Cl solution in an ice bath, and then extracted with dichloromethane (3*10 mL). The organic phase was washed with saturated brine, dried with anhydrous Na₂SO₄, and concentrated. The residue was separated and purified by column chromatography, to provide 140 mg of target compound, with a yield of 66%.

### Step 8: Synthesis of 3-(4-(2-(4-((2-(7-methyl-2,7-diazaspiro[4.4]nonan-2-yl) pyrimidin-4-yl)methoxy)phenyl)propan-2-yl)phenoxy)cyclobutylamine

Compound 2-(3-(4-(2-(4-((2-(7-methyl-2,7-diazaspiro[4.4]nonan-2-yl)pyrimidin- 4-yl)methoxy)phenyl)propan-2-yl)phenoxy)cyclobutyl)isoindolin-1,3-dione (140 mg, 0.21 mmol) was dissolved in 5 mL of absolute ethanol, to which was added hydrazine hydrate (32 mg, 1 mmol), and then the mixture was stirred for 2 h at 80 °C. After completion of the reaction detected by TLC, the reaction system was concentrated. The residue was separated and purified by column chromatography, to provide 92 mg of target compound, with a yield of 83%.

### Step 9: Synthesis of 2-(2,6-dioxopiperidin-3-yl)-5-((3-(4-(2-(4-((2-(7-methyl- 2,7-diazaspiro[4.4]nonan-2-yl)pyrimidin-4-yl)methoxy)phenyl)propan-2-yl)phenoxy)cyclobutyl)amino)isoindolin-1,3-dione

3-(4-(2-(4-((2-(7-methyl-2,7-diazaspiro[4.4]nonan-2-yl)pyrimidin-4-yl)methoxy)phenyl)propan-2-yl)phenoxy)cyclobutylamine (92 mg, 0.17 mmol), 2-(2,6-dioxopiperidin-3-yl)-5-fluoroisoindolin-1,3-dione (50 mg, 0.17 mmol) and DIPEA (70 mg, 0.5 mmol) were successively added into 5 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 15 mg of target compound, with a yield of 11%. ¹H NMR (400 MHz, CDCl₃) *δ* 8.29 (d, *J* = 5.0 Hz, 1H), 7.62 (dd, *J* = 8.3, 2.2 Hz, 1H), 7.12 (dt, *J* = 8.7, 1.9 Hz, 4H), 6.90 (dd, *J* = 9.7, 2.2 Hz, 1H), 6.87 - 6.79 (m, 2H), 6.75 - 6.63 (m, 4H), 4.93 (s, 2H), 4.91 (s, 2H), 3.68 - 3.50 (m, 4H), 3.20 - 3.05 (m, 1H), 2.91 - 2.61 (m, 7H), 2.46 (s, 3H), 2.38 (dd, *J* = 13.4, 6.3 Hz, 1H), 2.15 - 1.94 (m, 8H), 1.62 (s, 6H). LC/MS (ESI+) calcd for C₄₅H₄₉N₇O₆( [M+H]⁺ ) *m*/*z:* 784.4; found 784.4.

### Example 80: 2-(2,6-dioxopiperidin-3-yl)-5-((3-(4-(2-(2-(hexahydropyrrolo[3,4-c] pyrrole-2(1H)-yl)pyrimidin-4-yl)methoxy)phenyl)propan-2-yl)phenoxy)cyclobutyl)amino)isoindolin-1,3-dione

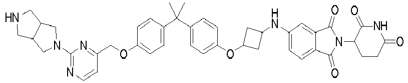

The title compound was synthesized by a method similar to that of Example 79. ¹H NMR (400 MHz, DMSO-d₆) *δ* 8.35 (d, *J* = 5.0 Hz, 1H), 7.65 - 7.40 (m, 2H), 7.11 (t, *J* = 8.6 Hz, 4H), 6.94 - 6.65 (m, 6H), 5.03 (dd, *J* = 13.0, 5.4 Hz, 1H), 4.95 (s, 2H), 4.56 - 4.39 (m, 1H), 3.82 - 3.52 (m, 5H), 3.10 - 2.71 (m, 8H), 2.69 - 2.51 (m, 2H), 2.42 (s, 2H), 1.96 (q, *J* = 20.1, 15.4 Hz, 3H), 1.57 (s, 6H). LC/MS (ESI+) calcd for C₄₃H₄₅N₇O₆( [M+H]⁺) *m*/*z:* 756.4; found 756.4.

### Example 81: 5-(((1r,3r)-3-(4-(2-(4-((2-(2,7-diazaspiro[4.4]nonan-2-yl)pyrimidin- 4-yl)methoxy)phenyl)propan-2-yl)phenoxy)cyclobutyl)amino)-2-(2,6-dioxopiperidin-3-yl)isoindolin-1,3-dione

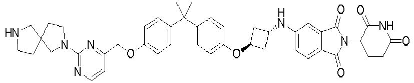

The target compound was synthesized by a method similar to that of Example 79. ¹H NMR (400 MHz, DMSO-d₆) *δ* 11.07 (s, 1H), 8.35 (d, *J* = 5.0 Hz, 1H), 7.57 (dd, *J* = 12.2, 6.9 Hz, 2H), 7.11 (t, *J* = 8.6 Hz, 4H), 6.94 - 6.68 (m, 6H), 5.03 (dd, *J =* 12.9, 5.4 Hz, 1H), 4.94 (s, 2H), 4.88 - 4.80 (m, 1H), 4.16 (d, *J =* 24.8 Hz, 1H), 3.52 (dd, *J* = 27.8, 10.2 Hz, 4H), 3.30 - 3.10 (m, 6H), 2.88 (dd, *J* = 22.4, 9.5 Hz, 1H), 2.70 - 2.53 (m, 2H), 2.47 - 2.31 (m, 3H), 1.98 (dqt, *J* = 20.4, 13.1, 7.3 Hz, 5H), 1.57 (s, 6H). LC/MS (ESI+) calcd for C₄₄H₄₇N₇O₆( [M+H]⁺) *m*/*z:* 770.4; found 770.4.

### Example 82: 5-(((1r,3r)-3-(4-(2-(4-((2-(2,6-diazaspiro[3.3]heptan -2-yl)pyrimidin-4-yl)methoxy)phenyl)propan-2-yl)phenoxy)cyclobutyl)amino)-2-(2,6-dioxopiperidin-3-yl)isoindolin-1,3-dione

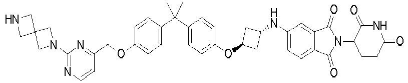

The target compound was synthesized by a method similar to that of Example 79. ¹H NMR (400 MHz, DMSO-*d*₆) *δ* 8.35 (d, *J* = 5.4 Hz, 1H), 7.66 - 7.39 (m, 2H), 7.10 (d, *J* = 8.4 Hz, 4H), 6.96 - 6.61 (m, 7H), 4.94 (s, 2H), 4.84 (d, *J* = 8.2 Hz, 1H), 4.47 (s, 1H), 4.15 (d, *J* = 29.4 Hz, 8H), 2.88 (dd, *J* = 20.5, 11.3 Hz, 2H), 2.63 (d, *J* = 30.5 Hz, 2H), 1.98 (s, 3H), 1.56 (s, 6H). LC/MS (ESI+) calcd for C₄₂H₄₃N₇O₆( [M+H]⁺) *m*/*z:* 742.3; found 742.3.

### Example 83: 5-(((1r,3r)-3-(4-(2-(4-((2-(2,6-diazaspiro[3.4]octan-6-yl)pyrimidin- 4-yl)methoxy)phenyl)propan-2-yl)phenoxy)cyclobutyl)amino)-2-(2,6-dioxopiperidin-3-yl)isoindolin-1,3-dione

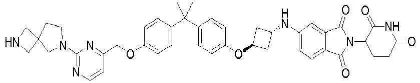

The target compound was synthesized by a method similar to that of Example 79. ¹H NMR (400 MHz, DMSO-d₆) *δ* 11.04 (s, 1H), 8.34 (d, *J* = 5.0 Hz, 1H), 7.63 (t, *J* = 5.6 Hz, 1H), 7.58 (d, *J* = 8.3 Hz, 1H), 7.10 (t, *J* = 8.1 Hz, 4H), 6.93 - 6.83 (m, 3H), 6.84 - 6.67 (m, 4H), 5.03 (dd, *J* = 12.9, 5.4 Hz, 1H), 4.94 (d, *J* = 4.1 Hz, 2H), 4.89 (s, 1H), 4.13 (dp, *J* = 12.6, 5.5 Hz, 1H), 4.03 - 3.85 (m, 4H), 3.49 (q, *J* = 8.5, 7.7 Hz, 4H), 2.87 (ddd, *J* = 17.5, 14.0, 5.4 Hz, 1H), 2.64 - 2.51 (m, 2H), 2.43 (dt, *J* = 10.7, 6.2 Hz, 3H), 2.23 (t, *J* = 6.9 Hz, 2H), 2.13 - 1.89 (m, 2H), 1.56 (d, *J* = 5.6 Hz, 6H). LC/MS (ESI+) calcd for C₄₃H₄₅N₇O₆( [M+H]⁺) *m*/*z:* 756.4; found 756.4.

### Example 84: 5-(((1s,3s)-3-(4-(2-(4-((2-(2,7-diazaspiro[3.5]nonan-7-yl)pyrimidin- 4-yl)methoxy)phenyl)propan-2-yl)phenoxy)cyclobutyl)amino)-2-(2,6-dioxopiperidin-3-yl)isoindolin-1,3-dione

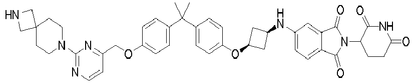

The target compound was synthesized by a method similar to that of Example 79. ¹H NMR (400 MHz, DMSO-d₆) *δ* 11.06 (s, 1H), 8.34 (d, *J* = 4.9 Hz, 1H), 7.58 (d, *J* = 8.3 Hz, 1H), 7.50 (d, *J* = 6.6 Hz, 1H), 7.10 (t, *J* = 8.7 Hz, 4H), 6.90 - 6.87 (m, 2H), 6.82 (dd, *J* = 8.5, 2.1 Hz, 1H), 6.76 (dd, *J* = 9.5, 2.8 Hz, 2H), 6.67 (d, *J* = 4.9 Hz, 1H), 5.03 (dd, *J* = 12.9, 5.4 Hz, 1H), 4.94 (s, 2H), 4.48 (p, *J =* 6.9 Hz, 1H), 3.95 - 3.44 (m, 9H), 3.13 - 2.98 (m, 2H), 2.87 (ddd, *J* = 18.1, 14.0, 5.6 Hz, 1H), 2.65 - 2.51 (m, 2H), 1.97 (td, *J* = 11.5, 11.0, 4.1 Hz, 3H), 1.77 (t, *J* = 5.7 Hz, 4H), 1.57 (s, 6H). LC/MS (ESI+) calcd for C₄₄H₄₇N₇O₆( [M+H]⁺) *m*/*z:* 770.4; found 770.4.

### Example 85: 5-(((1s,3s)-3-(4-(2-(4-((2-(2,7-diazaspiro[3.5]nonan-2-yl)pyrimidin- 4-yl)methoxy)phenyl)propan-2-yl)phenoxy)cyclobutyl)amino)-2-(2,6-dioxopiperidin-3-yl)isoindolin-1,3-dione

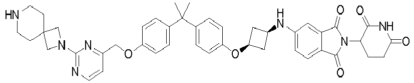

The target compound was synthesized by a method similar to that of Example 79. ¹H NMR (400 MHz, DMSO-d₆) δ 8.33 (d, *J* = 5.0 Hz, 1H), 7.58 (d, *J* = 8.3 Hz, 1H), 7.48 (d, *J* = 6.6 Hz, 1H), 7.10 (t, *J* = 8.6 Hz, 4H), 6.94 - 6.85 (m, 3H), 6.82 (dd, *J* = 8.5, 2.1 Hz, 1H), 6.74 (dd, *J* = 13.4, 6.7 Hz, 3H), 5.03 (dd, *J* = 12.9, 5.4 Hz, 1H), 4.94 (s, 2H), 4.48 (p, *J* = 7.1 Hz, 1H), 3.78 (s, 4H), 3.10 - 2.99 (m, 2H), 2.95 - 2.79 (m, 5H), 2.69 - 2.51 (m, 2H), 1.96 (p, *J* = 9.7, 8.9 Hz, 3H), 1.82 (d, *J* = 5.7 Hz, 4H), 1.57 (s, 6H). LC/MS (ESI+) calcd for C₄₄H₄₇N₇O₆( [M+H]⁺) *m*/*z*: 770.4; found 770.4.

### Example 86: 5-(((1s,3s)-3-(4-(2-(4-((2-(2,6-diazaspiro[3.3]-((2-(2,6-heptan-2-yl) pyrimidin-4-yl)methoxy)phenyl)propan-2-yl)phenoxy)cyclobutyl)amino)-2-(2,6-dioxopiperidin-3-yl)isoindolin-1,3-dione

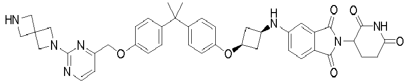

The target compound was synthesized by a method similar to that of Example 79. ¹H NMR (400 MHz, DMSO-*d*₆) *δ* 11.07 (s, 1H), 8.35 (d, *J* = 5.0 Hz, 1H), 7.58 (d, *J* = 8.3 Hz, 1H), 7.48 (d, *J* = 6.5 Hz, 1H), 7.10 (t, *J* = 8.6 Hz, 4H), 6.96 - 6.67 (m, 7H), 5.03 (dd, *J* = 12.8, 5.4 Hz, 1H), 4.95 (s, 2H), 4.48 (p, *J* = 6.9 Hz, 1H), 4.18 (d, *J* = 17.4 Hz, 8H), 3.78 (p, *J* = 7.7 Hz, 1H), 3.04 (ddd, *J* = 11.3, 8.6, 5.8 Hz, 2H), 2.95 - 2.80 (m, 1H), 2.61 - 2.52 (m, 2H), 1.96 (td, *J* = 19.1, 16.0, 8.1 Hz, 3H), 1.57 (s, 6H). LC/MS (ESI+) calcd for C₄₂H₄₃N₇O₆( [M+H]⁺) *m*/*z:* 742.3; found 742.3.

### Example 87: 5-(((1s,3s)-3-(4-(2-(4-((2-(2-oxa-7-azaspiro[3.5]nonan-7-yl) pyrimidin-4-yl)methoxy)phenyl)propan-2-yl)phenoxy)cyclobutyl)amino)-2-(2,6-dioxopiperidin-3-yl)isoindolin-1,3-dione

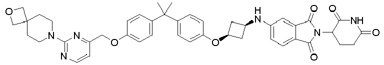

The target compound was synthesized by a method similar to that of Example 76. ¹H NMR (400 MHz, DMSO-*d*₆) *δ* 11.07 (s, 1H), 8.33 (d, *J* = 5.0 Hz, 1H), 7.58 (d, *J* = 8.4 Hz, 1H), 7.45 (d, *J* = 6.4 Hz, 1H), 7.17 - 7.05 (m, 4H), 6.96 - 6.85 (m, 3H), 6.82 (dd, *J* = 8.4, 2.1 Hz, 1H), 6.79 - 6.69 (m, 2H), 6.67 (d, *J* = 5.0 Hz, 1H), 5.03 (dd, *J* = 12.9, 5.4 Hz, 1H), 4.95 (s, 2H), 4.48 (p, *J=* 7.1 Hz, 1H), 3.86 - 3.74 (m, 3H), 3.58 - 3.42 (m, 6H), 3.18 - 3.00 (m, 2H), 2.92 - 2.81 (m, 1H), 2.69 - 2.52 (m, 2H), 2.00 - 1.83 (m, 7H), 1.57 (s, 6H). LC/MS (ESI+) calcd for C₄₄H₄₆N₆O₇( [M+H]⁺) *m*/*z:* 771.4; found 771.4.

### Example 88: 5-(((1s,3s)-3-(4-(2-(4-((2-(6-oxa-2-azaspiro[3.4]octan-2-yl)pyrimidin- 4-yl)methoxy)phenyl)propan-2-yl)phenoxy)cyclobutyl)amino)-2-(2,6-dioxopiperidin-3-yl)isoindolin-1,3-dione

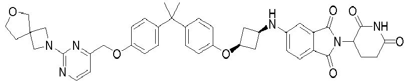

The target compound was synthesized by a method similar to that of Example 76. ¹H NMR (400 MHz, DMSO-d₆) *δ* 11.07 (s, 1H), 8.34 (d, *J* = 5.0 Hz, 1H), 7.58 (d, *J* = 8.3 Hz, 1H), 7.47 (d, *J* = 6.4 Hz, 1H), 7.10 (t, *J* = 8.5 Hz, 4H), 6.94 - 6.85 (m, 3H), 6.79 - 6.69 (m, 3H), 5.04 (dd, *J* = 12.9, 5.3 Hz, 1H), 4.95 (s, 2H), 4.48 (p, *J* = 7.0 Hz, 1H), 4.00 (s, 4H), 3.85 - 3.66 (m, 5H), 3.09 - 3.00 (m, 2H), 2.87 (ddd, *J* = 17.4, 14.0, 5.4 Hz, 1H), 2.63 - 2.51 (m, 2H), 2.14 (t, *J* = 6.9 Hz, 2H), 2.04 - 1.94 (m, 3H), 1.57 (s, 6H). LC/MS (ESI+) calcd for C₄₃H₄₄N₆O₇( [M+H]⁺) *m*/*z:* 757.3; found 757.3.

### Example 89: 5-(((1s,3s)-3-(4-(2-(4-((2-(7-oxa-2-azaspiro [3.5]nonan-2-yl) pyrimidin-4-yl)methoxy)phenyl)propan-2-yl)phenoxy)cyclobutyl)amino)-2-(2,6-dioxopiperidin-3-yl)isoindolin-1,3-dione

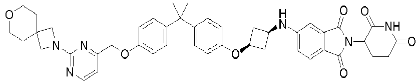

The target compound was synthesized by a method similar to that of Example 76. ¹H NMR (400 MHz, DMSO-d₆) *δ* 11.07 (s, 1H), 8.33 (d, *J* = 5.0 Hz, 1H), 7.58 (d, *J* = 8.3 Hz, 1H), 7.45 (d, *J* = 6.4 Hz, 1H), 7.10 (t, *J* = 8.4 Hz, 4H), 6.93 - 6.85 (m, 3H), 6.82 (dd, *J* = 8.4, 2.1 Hz, 1H), 6.79 - 6.73 (m, 2H), 6.72 (d, *J* = 5.0 Hz, 1H), 5.04 (dd, *J* = 12.9, 5.4 Hz, 1H), 4.94 (s, 2H), 4.48 (p, *J* = 7.0 Hz, 1H), 3.78 (s, 5H), 3.54 (t, *J* = 5.2 Hz, 4H), 3.10 - 3.01 (m, 2H), 2.87 (ddd, *J* = 17.4, 14.1, 5.5 Hz, 1H), 2.57 (dd, *J* = 19.3, 6.3 Hz, 2H), 1.99 - 1.91 (m, 3H), 1.72 (t, *J* = 5.2 Hz, 4H), 1.57 (s, 6H). LC/MS (ESI+) calcd for C₄₄H₄₆N₆O₇( [M+H]⁺) *m*/*z:* 771.4; found 771.4.

### Example 90: 5-(((1s,3s)-3-(4-(2-(4-((2-(2-oxa-6-azaspiro[3.3]heptane-6-yl) pyrimidin-4-yl)methoxy)phenyl)propan-2-yl)phenoxy)cyclobutyl)amino)-2-(2,6-dioxopiperidin-3-yl)-6-fluoroisoindolin-1,3-dione

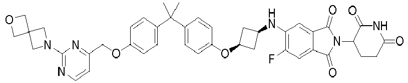

The target compound was synthesized by a method similar to that of Example 76. ¹H NMR (400 MHz, DMSO-d₆) *δ* 11.09 (s, 1H), 8.33 (d, *J* = 5.0 Hz, 1H), 7.59 (d, *J* = 10.2 Hz, 1H), 7.20 (dd, *J* = 6.8, 2.5 Hz, 1H), 7.14 - 7.07 (m, 4H), 7.05 (d, *J* = 7.2 Hz, 1H), 6.93 - 6.83 (m, 2H), 6.81 - 6.71 (m, 3H), 5.06 (dd, *J* = 12.8, 5.4 Hz, 1H), 4.95 (s, 2H), 4.71 (s, 4H), 4.45 (p, *J* = 7.0 Hz, 1H), 4.19 (s, 4H), 3.85 (p, *J* = 7.5 Hz, 1H), 3.07 - 2.82 (m, 3H), 2.64 - 2.51 (m, 2H), 2.13 (q, *J* = 9.1 Hz, 2H), 2.06 - 1.99 (m, 1H), 1.57 (s, 6H). LC/MS (ESI+) calcd for C₄₂H₄₁FN₆O₇ ([M+H]⁺) *m*/*z:* 761.3; found 761.3.

### Example 91: 3-(5-(((1s,3s)-3-(4-(2-(4-((2-(2-oxa-6-azaspiro[3.3]heptane-6-yl) pyrimidin-4-yl)methoxy)phenyl)propan-2-yl)phenoxy)cyclobutyl)amino)-1-oxoisoindolin-2-yl)piperidin-2,6-dione

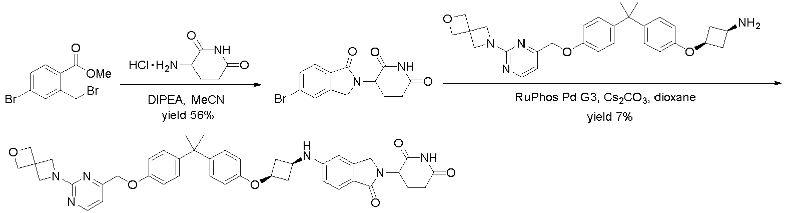

### Step 1: Synthesis of 3-(5-bromo-1-oxoisoindolin-2-yl)piperidin-2,6-dione

Methyl 4-bromo-2-(bromomethyl)benzoate (3 g, 10 mmol), 3-amino-2,6-dioxopiperidine hydrochloride (1.65 g, 10 mmol) and DIPEA (2.6 g, 20 mmol) were dissolved in 30 mL of acetonitrile, and then the mixture was allowed to react overnight at 60 °C. The reaction solution was naturally cooled to room temperature, and then the solution was stirred in an ice bath for 1h. The precipitated solid was filtered, and then rinsed with a small amount of acetonitrile, to provide 1.8 g of target compound, with a yield of 56%.

### Step 2: Synthesis of 3-(5-(((1s,3s)-3-(4-(2-(4-((2-(2-oxa-6-azaspiro[3.3]heptane-6-yl)pyrimidin-4-yl)methoxy)phenyl)propan-2-yl)phenoxy)cyclobutyl)amino)-1-oxoisoindolin-2-yl)piperidin-2,6-dione

Compound (1*s*,3*s*)-3-(4-(2-(4-((2-(2-oxa-6-azaspiro[3.3]heptan-6-yl)pyrimidin- 4-yl)methoxy)phenyl)propan-2-yl)phenoxy)cyclobutylamine (73 mg, 0.15 mmol), 3-(5-bromo-1-oxoisoindolin-2-yl)piperidin-2,6-dione (48 mg, 0.15 mmol), methanesulfonato(2-dicyclohexylphosphino-2',6'-diisopropyloxy-1,1'-biphenyl)(2-amino-1,1'-biphenyl-2-yl)palladium (II) (12 mg, 0.015 mmol), and cesium carbonate (100 mg, 0.3 mmol) were added into 2 mL of dioxane, and then the reaction system was purged with helium and reacted overnight at 110 °C under helium atmosphere. After completion of the reaction detected by TLC, the reaction system was filtered over diatomaceous earth, and concentrated. The residue was separated and purified by column chromatography, to provide 8 mg of target compound, with a yield of 7%. LC/MS (ESI+) calcd for C₄₂H₄₄N₆O₆([M+H]⁺) *m*/*z:* 729.3; found 729.3.

### Example 92: 3-(6-(((1s,3s)-3-(4-(2-(4-((2-(2-oxa-6-azaspiro[3.3]heptane- 6-yl)pyrimidin-4-yl)methoxy)phenyl)propan-2-yl)phenoxy)cyclobutyl)amino)-1-oxoisoindolin-2-yl)piperidin-2,6-dione

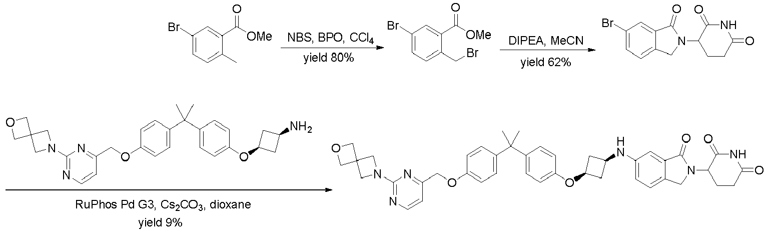

### Step 1: Synthesis of methyl 5-bromo-2-(bromomethyl)benzoate

Methyl 5-bromo-2-methylbenzoate (2.2 g, 10 mmol) was dissolved in 20 ml of CCl₄, to which were successively added NBS (1.8 g, 10 mmol) and BPO (242 mg, 1 mmol), and then the system was purged with nitrogen, followed by refluxing for 24 h. The reaction solution was cooled to room temperature, filtered, and concentrated under reduced pressure. The residue was separated and purified by column chromatography, to provide 2.4 g of target compound, with a yield of 80%.

In the following steps, the target compound was synthesized by the method similar to that of Example 91. LC/MS (ESI+) calcd for C₄₂H₄₄N₆O₆ ([M+H]⁺) *m*/*z:* 729.3; found 729.3.

### Example 93: 3-(5-((1r,3r)-3-(4-(2-(4-((6-(2H-1,2,3-triazol-2-yl)pyridin-3-yl)oxy) phenyl)propan-2-yl)phenoxy)cyclobutyl)amino)-1-oxoisoindolin-2-yl)piperidin-2,6-dione

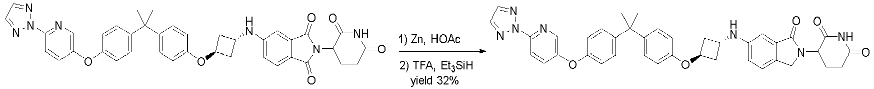

5-((1*r*,3*r*)-3-(4-(2-(4-((6-(2*H*-1,2,3-triazol-2-yl)pyridin-3-yl)oxy)phenyl)propan-2-ylphenoxy)cyclobutyl)amino)-2-(2,6-dioxopiperidin-3-yl)isoindolin-1,3-dione (35 mg, 0.05 mmol) was dissolved in 2 mL of glacial acetic acid, to which was added zinc powder (64 mg, 1 mmol), and then the mixture was allowed to react at 60 °C for 2h. TLC detection indicated disappearence of the starting material. The reaction solution was cooled to room temperature, filtered over diatomite, and concentrated to remove glacial acetic acid. The reaction solution was adjusted to be alkaline by adding saturated NaHCO₃ solution. The resultant solution was extracted several times with 5 mL of ethyl acetate. The organic phase was combined, washed with saturated brine, dried, and concentrated. The residue was dissolved in 2 mL of dichloromethane, to which were successively added 0.5 mL of trifluoroacetic acid and triethylsilane (12 mg, 0.1 mmol), and then the mixture was stirred overnight at room temperature. LC-MS indicated disappearance of hydroxylated compound, and then the reaction was stopped. Dichloromethane and most of trifluoroacetic acid were removed by concentation, and then the system was adjusted to be weakly alkaline by adding saturated NaHCO₃ solution. The resultant solution was extracted several times with 5 mL of ethyl acetate. The organic phase was combined, dried, and concentrated. The residue was separated and purified by column chromatography to provide 11 mg of compound 3-(5-((1*r*,3*r*)-3-(4-(2-(4-((6-(2*H*-1,2,3-triazol-2-yl)pyridin-3-yl)oxy)phenyl)propan-2-yl)phenoxy) cyclobutyl)amino)-1-oxoisoindolin-2-yl)piperidin-2,6-dione, with a yield of 32%. ¹H NMR (400 MHz, CDCl₃) *δ* 10.46 (s, 1H), 8.18 (s, 1H), 7.91 (s, 1H), 7.78 (m, 3H), 7.07 (m, 7H), 6.85 (m, 2H), 6.59 (m, 2H), 5.00 (m, 1H), 4.76 (s, 1H), 4.11 (m, 4H), 2.47 (m, 8H), 1.56 (s, 6H). LC/MS (ESI+) calcd for C₃₉H₃₇N₇O₅( [M+H]⁺) *m*/*z:* 684.3; found 684.3.

### Example 94: 3-(6-(((1r,3r)-3-(4-(2-(4-((6-(2H-1,2,3-triazol-2-yl)pyridin-3-yl)oxy) phenyl)propan-2-yl)phenoxy)cyclobutyl)amino)-1-oxoisoindolin-2-yl)piperidin-2,6-dione

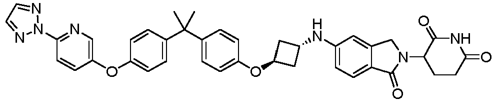

The target compound was obtained during the preparation process of Example 93. ¹H NMR (400 MHz, CDCl₃) *δ* 10.14 (s, 1H), 8.18 (s, 1H), 7.89 (s, 1H), 7.76 (s, 2H), 7.61 (t, *J* = 6.4 Hz, 1H), 7.37 (d, *J* = 8.7 Hz, 1H), 7.10 (dd, *J* = 8.6, 4.5 Hz, 2H), 7.06 - 6.87 (m, 4H), 6.82 (dd, *J* = 8.4, 4.4 Hz, 2H), 6.57 (dd, *J* = 8.6, 4.5 Hz, 2H), 5.08 - 4.96 (m, 1H), 4.83 (s, 1H), 4.38 - 3.93 (m, 4H), 2.69 (d, *J* = 14.2 Hz, 3H), 2.57 (s, 2H), 2.11 (d, *J* = 64.6 Hz, 3H), 1.53 (s, 6H). LC/MS (ESI+) calcd for C₃₉H₃₇N₇O₅( [M+H]⁺) *m*/*z:* 684.3; found 684.3.

### Example 95: 2-(2,6-dioxopiperidin-3-yl)-5-(((1r,3r)-3-(4-(2-(4-((5-fluoro-2-(5-methyl-1,3,4-oxadiazol-2-yl)pyridin-3-yl)oxy)phenyl)propan-2-yl)phenoxy) cyclobutyl)amino)isoindolin-1,3-dione

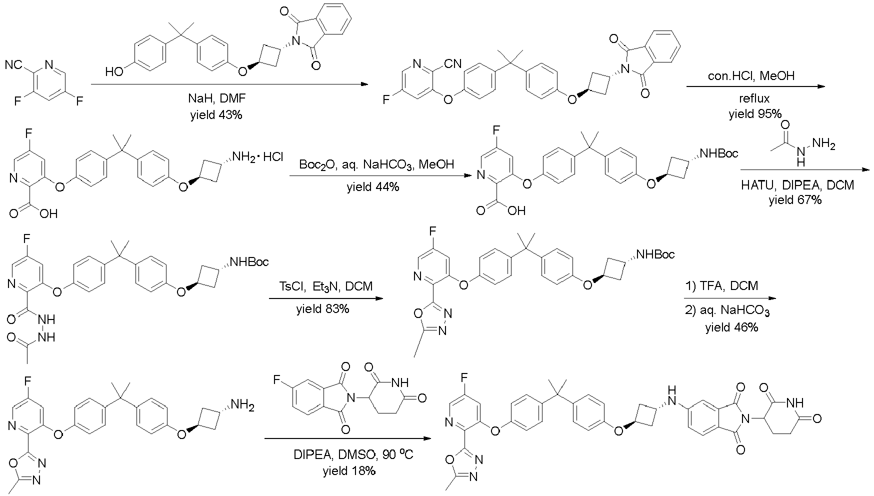

### Step 1: Synthesis of 3-(4-(2-(4-((1r,3r)-3-(1,3-dioxoisoindolin-2-yl)cyclobutyloxy)phenyl)propan-2-yl)phenoxy)-5-fluoropyridine-2-carbonitrile

60 % NaH (88 mg, 2.2 mmol) was dissolved in 10 mL DMF, to which was added 2-((1*r*,3*r*)-3-(4-(2-(4-hydroxylphenyl)propan-2-yl)phenoxy)cyclobutyl)isoindolin-1,3-dione (854 mg, 2 mmol) in an ice bath, and then the mixture was stirred at room temperature for 10 min, followed by addition of 3,5-difluoropyridine-2-carbonitrile (280 mg, 2 mmol). The mixture was slowly warmed to room temperature and reacted for 2h. After completion of the reaction detected by TLC, the reaction was quenched with saturated ammonium chloride solution in an ice bath, and then the resultant solution was extracted thrice with 10 mL of ethyl acetate. The organic phase was washed with saturated brine (3*10 mL), dried with anhydrous Na₂SO₄, and concentrated. The residue was separated and purified by column chromatography, to provide 470 mg of target compound, with a yield of 43%.

### Step 2: Synthesis of 3-(4-(2-(4-((1r,3r)-3-aminocyclobutyloxy)phenyl)propan-2-yl)phenoxy)-5-fluoropicolinic acid hydrochloride

Compound 3-(4-(2-(4-((1*r*,3*r*)-3-(1,3-dioxoisoindolin-2-yl)cyclobutyloxy) phenyl)propan-2-yl)phenoxy)-5-fluoropyridine-2-carbonitrile (460 mg, 0.84 mmol) was dissolved in 5 mL of methanol, to which was added concentrated hydrochloric acid (1 mL, 12 mmol), and then the mixture was allowed to react overnight at 90 °C. After completion of the reaction detected by TLC, the solvent was directly removed by concentration, to provide 380 mg of target compound, with a yield of 95%.

### Step 3: Synthesis of 3-(4-(2-(4-((1r,3r)-3-((tert-butoxycarbonyl)amino)cyclobutyloxy) phenyl)propan-2-yl)phenoxy)-5-fluoropicolinic acid

Compound 3-(4-(2-(4-((1*r*,3*r*)-3-aminocyclobutyloxy)phenyl)propan-2-yl) phenoxy)-5-fluoropicolinic acid hydrochloride (380 mg, 0.8 mmol) was dissolved in 10 mL of methanol, to which was added 3 mL of NaHCO₃ aqueous solution (2M), and then the mixture was stirred for 10 min at room temperature, followed by addition of di-tert-butyl dicarbonate (218 mg, 1 mmol). After completion of the reaction detected by TLC, saturated NaHCO₃ solution was poured into the reaction system, to quench the reaction. The resultant solution was further stirred for 10 min, and then extracted with ethyl acetate (3*10 mL). The organic phase was washed with saturated brine, dried with anhydrous Na₂SO₄, and concentrated. The residue was separated and purified by column chromatography, to provide 190 mg of target compound, with a yield of 44%.

### Step 4: Synthesis of tert-butyl ((1r,3r)-3-(4-(2-(4-((2-(2-acethydrazide-1-carbonyl)-5-fluoropyridin-3-yl)oxy)phenyl)propan-2-yl)phenoxy)cyclobutyl)carbamate

Compound 3-(4-(2-(4-((1*r*,3*r*)-3-((tert-butoxycarbonyl)amino)cyclobutyloxy) phenyl)propan-2-yl)phenoxy)-5-fluoropicolinic acid (190 mg, 0.35 mmol) was dissolved in 2 mL of dichloromethane, to which were added DIPEA (90 mg, 0.7 mmol) and HATU (133 mg, 0.35 mmol), and then the mixture was stirred for 10 min at room temperature, followed by addition of acethydrazide (51 mg, 0.7 mmol). The mixture was allowed to further react at room temperature for 6h. After completion of the reaction detected by TLC, saturated NH₄Cl solution was poured into the reaction system in an ice bath, to quench the reaction, and then extracted with dichloromethane. The organic phase was washed with saturated brine and concentrated. The residue was separated and purified by column chromatography, to provide 138 mg of target compound, with a yield of 67%.

### Step 5: Synthesis of tert-butyl ((1r,3r)-3-(4-(2-(4-((5-fluoro-2-(5-methyl-1,3,4-oxadiazol-2-yl)pyridin-3-yl)oxy)phenyl)propan-2-yl)phenoxy)cyclobutyl)carbamate

Compound tert-butyl ((1*r*,3*r*)-3-(4-(2-(4-((2-(2-acethydrazide-1-carbonyl)-5-fluoropyridin-3-yl)oxy)phenyl)propan-2-yl)phenoxy)cyclobutyl)carbamate (138 mg, 0.23 mmol) was added into 5 mL of dichloromethane, to which were successively added triethylamine (50 mg, 0.46 mmol) and p-toluenesulfonyl chloride (88 mg, 0.46 mmol), and then the mixture was allowed to react overnight at room temperature. After completion of the reaction detected by TLC, the reaction system was concentrated. The residue was separated and purified by column chromatography, to provide 110 mg of target compound, with a yield of 83%.

### Step 6: Synthesis of (1r,3r)-3-(4-(2-(4-((5-fluoro-2-(5-methyl-1,3,4-oxadiazol-2-yl)pyridin-3-yl)oxy)phenyl)propan-2-yl)phenoxy)cyclobutylamine

tert-butyl ((1*r*,3*r*)-3-(4-(2-(4-((5-fluoro-2-(5-methyl-1,3,4-oxadiazol-2-yl) pyridin-3-yl)oxy)phenyl)propan-2-yl)phenoxy)cyclobutyl)carbamate (110 mg, 0.19 mmol) was dissolved in 2 mL of dichloromethane, to which was added 1 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 42 mg of target compound, with a yield of 46%.

### Step 7: Synthesis of 2-(2,6-dioxopiperidin-3-yl)-5-(((1r,3r)-3-(4-(2-(4-((5-fluoro-2-(5-methyl-1,3,4-oxadiazol-2-yl)pyridin-3-yl)oxy)phenyl)propan-2-yl)phenoxy) cyclobutyl)amino)isoindolin-1,3-dione

(1*r*,3*r*)-3-(4-(2-(4-((5-fluoro-2-(5-methyl-1,3,4-oxadiazol-2-yl)pyridin-3 - yl)oxy)phenyl)propan-2-yl)phenoxy)cyclobutylamine (42 mg , 0.09 mmol), 2-(2,6-dioxopiperidin-3-yl)-5-fluoroisoindolin-1,3-dione (25 mg, 0.09 mmol) and DIPEA (40 mg, 0.3 mmol) were successively added into 10 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 12 mg of target compound, with a yield of 18%. ¹H NMR (400 MHz, CDCl₃) *δ* 8.35 (s, 1H), 8.09 (s, 1H), 7.62 (d, *J* = 7.9 Hz, 1H), 7.28 (d, *J* = 8.5 Hz, 2H), 7.13 (d, *J* = 8.2 Hz, 2H), 7.03 (t, *J* = 8.7 Hz, 3H), 6.88 (s, 1H), 6.71 (d, *J* = 8.1 Hz, 3H), 4.92 (dd, *J* = 11.9, 5.1 Hz, 1H), 4.87 (s, 1H), 4.22 (s, 1H), 2.93 - 2.68 (m, 5H), 2.65 (s, 3H), 2.42 (d, *J* = 11.0 Hz, 2H), 2.16 - 2.07 (m, 1H), 1.67 (s, 6H). LC/MS (ESI+) calcd for C₄₀H₃₅FN₆O₇( [M+H]⁺) *m*/*z:* 731.3; found 731.3.

### Example 96: 2-(2,6-dioxopiperidin-3-yl)-5-(((1r,3r)-3-(4-(2-(4-((5-(5-methyl-1,3,4-oxadiazol-2-yl)oxazol-2-yl)oxy)phenyl)propan-2-yl)phenoxy)cyclobutyl)amino) isoindolin-1,3-dione

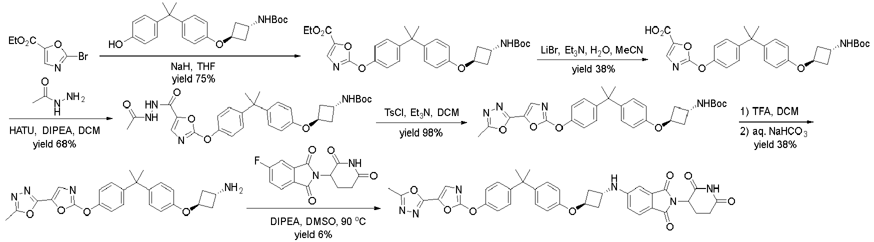

### Step 1: Synthesis of ethyl 2-(4-(2-(4-((1r,3r)-3-((tert-butoxycarbonyl)amino) cyclobutyloxy)phenyl)propan-2-yl)phenoxy)oxazol-5-carboxylate

60 % NaH (44 mg, 1.1 mmol) was dissolved in 5 mL DMF, to which was added 2-((1*r*,3*r*)-3-(4-(2-(4-hydroxylphenyl)propan-2-yl)phenoxy)cyclobutyl)isoindolin-1,3-dione (427 mg, 1 mmol) in an ice bath, and then the mixture was stirred at room temperature for 10 min, followed by addition of ethyl 2-bromooxazol-5-carboxylate (218 mg, 1 mmol). The mixture was slowly warmed to room temperature and reacted for 2 h. After completion of the reaction detected by TLC, the reaction was quenched with saturated ammonium chloride solution in an ice bath, and the resultant solution was extracted thrice with 10 mL of ethyl acetate. The organic phase was washed with saturated brine (3*10 mL), dried with anhydrous Na₂SO₄, and concentrated. The residue was separated and purified by column chromatography, to provide 402 mg of target compound, with a yield of 75%.

### Step 2: Synthesis of 2-(4-(2-(4-((1r,3r)-3-((tert-butoxycarbonyl)amino)cyclobutyloxy) phenyl)propan-2-yl)phenoxy)oxazol-5-carboxylic acid

Compound ethyl 2-(4-(2-(4-((1*r*,3*r*)-3-((tert-butoxycarbonyl)amino) cyclobutyloxy)phenyl)propan-2-yl)phenoxy)oxazol-5-carboxylate (402 mg, 0.75 mmol) was dissolved in 4 mL of acetonitrile, to which were successively added 2 mL of water, triethylamine (150 mg, 1.5 mmol), and LiBr (258 mg, 3 mmol), and then the mixture was allowed to react overnight at 90 °C. After completion of the reaction detected by TLC, the pH was adjusted to be less than 7 with 0.5 N hydrochloric acid. The resultant solution was extracted with ethyl acetate. The organic phase was combined, dried, and concentrated. The residue was separated and purified by column chromatography, to provide 146 mg of target compound, with a yield of 38%.

### Step 3: Synthesis of tert-butyl ((1r,3r)-3-(4-(2-(4-((5-(2-acethydrazide-1-carbonyl)oxazol-2-yl)oxy)phenyl)propan-2-yl)phenoxy)cyclobutyl)carbamate

Compound 2-(4-(2-(4-((1*r*,3*r*)-3-((tert-butoxycarbonyl)amino)cyclobutyloxy) phenyl)propan-2-yl)phenoxy)oxazol-5-carboxylic acid (146 mg, 0.29 mmol) was dissolved in 2 mL of dichloromethane, to which were successively added DIPEA (80 mg, 0.6 mmol) and HATU (114 mg, 0.3 mmol), and then the mixture was stirred for 10 min at room temperature, followed by addition of acethydrazide (45 mg, 0.6 mmol). The mixture was allowed to further react at room temperature for 6h. After completion of the reaction detected by TLC, saturated NH₄Cl solution was poured into the reaction system in an ice bath, to quench the reaction, and then extracted with dichloromethane. The organic phase was washed with saturated brine and concentrated. The residue was separated and purified by column chromatography, to provide 112 mg of target compound, with a yield of 68%.

### Step 4: Synthesis of tert-butyl ((1r,3r)-3-(4-(2-(4-((5-(5-methyl-1,3,4-oxadiazol-2-yl)oxazol-2-yl)oxy)phenyl)propan-2-yl)phenoxy)cyclobutyl)carbamate

Compound tert-butyl ((1*r*,3*r*)-3-(4-(2-(4-((5-(2-acethydrazide-1-carbonyl)oxazol-2-yl)oxy)phenyl)propan-2-yl)phenoxy)cyclobutyl)carbamate (112 mg, 0.2 mmol) was added into 5 mL of dichloromethane, to which were successively added triethylamine (40 mg, 0.4 mmol) and p-toluenesulfonyl chloride (76 mg, 0.4 mmol), and then the mixture was allowed to react overnight at room temperature. After completion of the reaction detected by TLC, the reaction system was concentrated. The residue was separated and purified by column chromatography, to provide 108 mg of target compound, with a yield of 98%.

### Step 5: Synthesis of (1r,3r)-3-(4-(2-(4-((5-(5-methyl-1,3,4-oxadiazol-2-yl)oxazol-2-yl)oxy)phenyl)propan-2-yl)phenoxy)cyclobutylamine-1-amine

tert-butyl ((1*r*,3*r*)-3-(4-(2-(4-((5-(5-methyl-1,3,4-oxadiazol-2-yl)oxazol-2-yl)oxy) phenyl)propan-2-yl)phenoxy)cyclobutyl)carbamate (108 mg, 0.19 mmol) was dissolved in 2 mL of dichloromethane, to which was added 1 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 32 mg of target compound, with a yield of 38%.

### Step 6: Synthesis of 2-(2,6-dioxopiperidin-3-yl)-5-(((1r,3r)-3-(4-(2-(4-((5-(5-methyl-1,3,4-oxadiazol-2-yl)oxazol-2-yl)oxy)phenyl)propan-2-yl)phenoxy)cyclobutyl)amino) isoindolin-1,3-dione

(1*r*,3*r*)-3 -(4-(2-(4-((5 -(5 -methyl-1,3,4-oxadiazol-2-yl)oxazol-2-yl)oxy)phenyl) propan-2-yl)phenoxy)cyclobutylamine-1-amine (32 mg, 0.07 mmol), 2-(2,6-dioxopiperidin-3-yl)-5-fluoroisoindolin-1,3-dione (20 mg, 0.07 mmol) and DIPEA(40 mg, 0.3 mmol) were successively added into 10 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 3 mg of target compound, with a yield of 6%. ¹H NMR (400 MHz, CDCl₃) *δ* 7.99 (s, 1H), 7.64 (d, *J* = 8.1 Hz, 1H), 7.30 (d, *J* = 8.4 Hz, 3H), 7.24 (s, 1H), 7.14 (d, *J* = 8.4 Hz, 2H), 6.90 (s, 1H), 6.71 (d, *J* = 8.6 Hz, 3H), 4.95 - 4.86 (m, 2H), 4.25 (s, 1H), 2.91 (s, 1H), 2.74 (d, *J* = 18.8 Hz, 3H), 2.56 (s, 3H), 2.41 (s, 2H), 2.12 (d, *J* = 9.2 Hz, 2H), 1.67 (s, 6H). LC/MS (ESI+) calcd for C₃₈H₃₄N₆O₈( [M+H]⁺) *m*/*z:* 703.3; found 703.3.

### Example 97: 5-(4-(2-(4-((1r,3r)-3-((2-(2,6-dioxopiperidin-3-yl)-1,3-dioxoisoindolin-5-yl)amino)cyclobutyloxy)phenyl)propan-2-yl)phenoxy) pyrimidin-2-carbonitrile

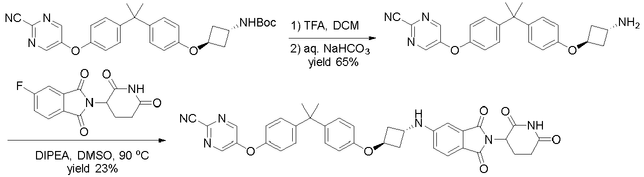

### Step 1: Synthesis of 5-(4-(2-(4-((1r,3r)-3-aminocyclobutyloxy)phenyl)propan-2-yl)phenoxy)pyrimidin-2-carbonitrile

tert-butyl ((1*r*,3*r*)-3-(4-(2-(4-((2-cyanopyrimidin-5-yl)oxy)phenyl)propan-2-yl) phenoxy)cyclobutyl)carbamate (100 mg, 0.2 mmol) was dissolved in 2 mL of dichloromethane, to which was added 1 mL trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 52 mg of target compound, with a yield of 65%.

### Step 2: Synthesis of 5-(4-(2-(4-((1r,3r)-3-((2-(2,6-dioxopiperidin-3-yl)-1,3-dioxoisoindolin-5-yl)amino)cyclobutyloxy)phenyl)propan-2-yl)phenoxy)pyrimidin-2-carbonitrile

5-(4-(2-(4-((1*r*,3*r*)-3-aminocyclobutyloxy)phenyl)propan-2-yl)phenoxy) pyrimidin-2-carbonitrile (52 mg, 0.13 mmol), 2-(2,6-dioxopiperidin-3-yl)-5-fluoroisoindolin-1,3-dione (38 mg, 0.13 mmol) and DIPEA (52 mg, 0.4 mmol) were successively added into 10 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 20 mg of target compound, with a yield of 23%. LC/MS (ESI+) calcd for C₃₇H₃₂N₆O₆( [M+H]⁺) *m*/*z:* 657.3; found 657.3.

### Example 98: 6-(4-(2-(4-((1r,3r)-3-((2-(2,6-dioxopiperidin-3-yl)-1,3-dioxoisoindolin-5-yl)amino)cyclobutyloxy)phenyl)propan-2-yl)phenoxy) pyridazine-3-carbonitrile

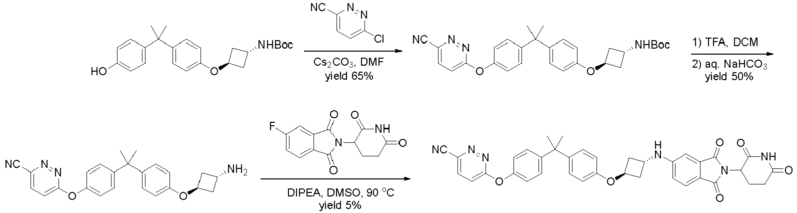

### Step 1: Synthesis of tert-butyl ((1r,3r)-3-(4-(2-(4-((6-cyanopyridin-3-yl)oxy)phenyl)propan-2-yl)phenoxy)cyclobutyl)carbamate

tert-butyl ((1*r*,3*r*)-3-(4-(2-(4-hydroxylphenyl)propan-2-phenoxy)cyclobutyl) carbamate (200 mg, 0.5 mmol), 6-chloropyridin-3-carbonitrile (70 mg, 0.5 mmol) and cesium carbonate (326 mg, 1 mmol) were successively added into 5 mL of DMF, and then the mixture was allowed to react overnight at room temperature. After completion of the reaction detected by TLC, the resultant solution was extracted with 5 mL of ethyl acetate. The organic phase was washed with saturated brine (3*5 mL), dried with anhydrous Na₂SO₄, and concentrated. The residue was separated and purified by column chromatography, to provide 162 mg of target compound, with a yield of 65%.

In following steps, the target compound was synthesized by a method similar to that of Example 97.

LC/MS (ESI+) calcd for C₃₇H₃₂N₆O₆( [M+H]⁺) *m*/*z:* 657.3; found 657.3.

### Example 99: 2-(2,6-dioxopiperidin-3-yl)-5-((1r,3r)-3-(4-(1-(4-((2-(5-methyl-1,2,4-oxadiazol-3-yl)pyrimidin-5-yl)oxy)phenyl)ethyl)phenoxy)cyclobutyl)amino) isoindolin-1,3-dione

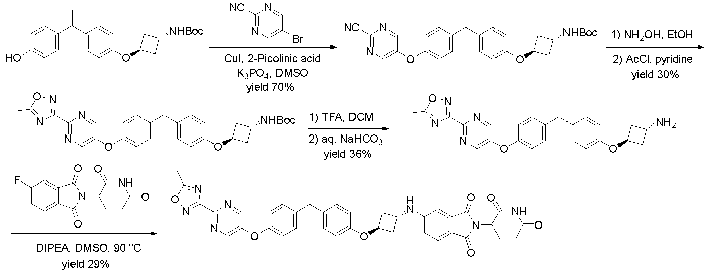

### Step 1: Synthesis of tert-butyl ((1r,3r)-3-(4-(1-(4-((2-cyanopyrimidin-5-yl)oxy)phenyl) ethyl)phenoxy)cyclobutyl)carbamate

Compound tert-butyl ((1*r*,3*r*)-3-(4-(1-(4-hydroxylphenyl)ethyl)phenoxy) cyclobutyl)carbamate (383 mg, 1 mmol), 5 -fluoropyrimidin-2-carbonitrile (123 mg, 1 mmol), CuI (19 mg, 0.1 mmol), 2-picolinic acid (24 mg, 0.2 mmol), and potassium phosphate (424 mg, 2 mmol) were placed in a 25 mL round-bottom flask, to which was added 5 mL of DMSO, and then the mixture was allowed to react overnight at 90 °C under argon atmosphere. After completion of the reaction detected by TLC, the reaction solution was cooled to room temperature, and then filtered over diatomite, followed by adding 5 mL of water. The resultant solution was extracted with ethyl acetate. The organic phase was combined, washed with saturated brine, and concentrated. The residue was separated and purified by column chromatography, to provide 340 mg of target compound, with a yield of 70%.

### Step 2: Synthesis of tert-butyl ((1r,3r)-3-(4-(1-(4-((2-(5-methyl-1,2,4-oxadiazol-3-yl) pyrimidin-5-yl)oxy)phenyl)ethyl)phenoxy)cyclobutyl)carbamate

Compound tert-butyl ((1*r*,3*r*)-3-(4-(1-(4-((2-cyanopyrimidin-5-yl)oxy)phenyl) ethyl)phenoxy)cyclobutyl)carbamate (340 mg, 0.7 mmol) was dissolved in 10 mL of absolute ethanol, to which was added hydroxylamine (46 mg, 1.4 mmol), and then the mixture was allowed to react for 2h at 70 °C. TLC indicated disappearance of the starting materials. The absolute alcohol was directly removed by concentration, followed by addition of ethyl actate. The organic phase was successively washed with water and saturated brine. The organic phase was combined and concentrated. The residue was dissolved in 5 mL of pyridine, to which was added acetyl chloride (109 mg, 1.4 mmol) in an ice bath, and then the mixture was allowed to react for 3h at 100 °C. After completion of the reaction detected by TLC, most of pyridine was removed by concentration, followed by washing with 0.5 N of hydrochloric acid. The resultant solution was extracted with ethyl acetate. The organic phase were successively washed with water and saturated brine. The organic phase was combined, dried and concentrated. The residue was separated and purified by column chromatography, to provide 115 mg of target compound, with a yield of 30%.

### Step 3: Synthesis of (1r,3r)-3-(4-(1-(4-((2-(5-methyl-1,2,4-oxadiazol-3-yl)pyrimidin-5-yl)oxy)phenyl)ethyl)phenoxy)cyclobutane-1-amine

tert-butyl ((1*r*,3*r*)-3-(4-(1-(4-((2-(5-methyl-1,2,4-oxadiazol-3-yl)pyrimidin-5-yl)oxy)phenyl)ethyl)phenoxy)cyclobutyl)carbamate (115 mg, 0.21 mmol) was dissolved in 2 mL of dichloromethane, to which was added 1 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*10 mL). The organic phase was washed with saturated brine, dried with anhydrous Na₂SO₄, and concentrated. The residue was separated and purified by column chromatography, to provide 33 mg of target compound, with a yield of 36%.

### Step 4: Synthesis of 2-(2,6-dioxopiperidin-3-yl)-5-((1r,3r)-3-(4-(1-(4-((2-(5-methyl-1,2,4-oxadiazol-3-yl)pyrimidin-5-yl)oxy)phenyl)ethyl)phenoxy)cyclobutyl)amino) isoindolin-1,3-dione

(1*r*,3*r*)-3-(4-(1-(4-((2-(5-methyl-1,2,4-oxadiazol-3-yl)pyrimidin-5-yl)oxy)phenyl) ethyl)phenoxy)cyclobutane-1-amine (33 mg, 0.074 mmol), 2-(2,6-dioxopiperidin-3-yl)-5-fluoroisoindolin-1,3-dione (22 mg, 0.074 mmol) and DIPEA (40 mg, 0.3 mmol) were successively added into 10 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 15 mg of target compound, with a yield of 29%. ¹H NMR (400 MHz, DMSO-*d*₆) δ 11.07 (s, 1H), 8.73 (s, 2H), 7.59 (d, *J* = 8.3 Hz, 1H), 7.53 (d, *J* = 5.5 Hz, 1H), 7.34 (d, *J* = 8.5 Hz, 2H), 7.21 - 7.14 (m, 4H), 6.85 (s, 1H), 6.83 - 6.71 (m, 3H), 5.04 (dd, *J* = 12.9, 5.4 Hz, 1H), 4.91 - 4.81 (m, 1H), 4.15 (d, *J* = 8.4 Hz, 2H), 2.94 - 2.80 (m, 1H), 2.69 (s, 3H), 2.63 - 2.51 (m, 3H), 2.47 - 2.39 (m, 2H), 2.19 - 1.86 (m, 2H), 1.56 (d, *J* = 7.2 Hz, 3H). LC/MS (ESI+) calcd for C₃₈H₃₃N₇O₇( [M+H]⁺) *m*/*z:* 700.3; found 700.3.

### Example 100: 5-((1r,3r)-3-(4-(2-(4-(4-((2-(2-oxa-6-azaspiro[3.3]heptane-6-yl)pyrimidin-4-yl)methoxy)phenyl)propan-2-yl)phenoxy)cyclobutyl)amino)-2-(1-methyl-2,6-dioxopiperidin-3-yl)isoindolin-1,3-dione

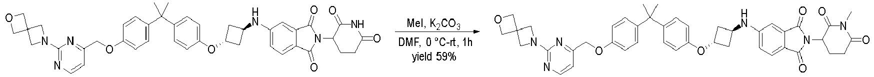

5-(((1*s*,3*s*)-3-(4-(2-(4-((2-(2-oxa-6-azaspiro[3.3]heptane-6-yl)pyrimidin-4-yl)methoxy)phenyl)propan-2-yl)phenoxy)cyclobutyl)amino)-2-(2,6-dioxopiperidin-3-yl)isoindolin-1,3-dione (70 mg, 0.09 mmol) was dissolved in 2 mL of DMF, to which was added K₂CO₃ (28 mg, 0.2 mmol), followed by addition of CH3I (15 mg, 0.1 mmol) in an ice bath. The mixture was allowed to react at room temperature for 1 h. After completion of the reaction detected by TLC, the resultant solution was extracted with 5 mL of ethyl acetate. The organic phase was washed with saturated brine (3*5 mL), dried with anhydrous Na₂SO₄, and concentrated. The residue was separated and purified by column chromatography, to provide 40 mg of compound 5-((1*r*,3*r*)-3-(4-(2-(4-(4-((2-(2-oxa-6-azaspiro[3.3]heptane-6-yl)pyrimidin-4-yl)methoxy)phenyl)propan-2-yl)phenoxy)cyclobutyl)amino)-2-(1-methyl-2,6-dioxopiperidin-3-yl)isoindolin-1,3-dione, with a yield of 59%. ¹H NMR (400 MHz, DMSO-d₆) *δ* 8.33 (d, *J=* 5.0 Hz, 1H), 7.59 (d, *J* = 8.3 Hz, 1H), 7.48 (d, *J* = 1.9 Hz, 1H), 7.12 - 7.08 (m, 4H), 6.92 - 6.86 (m, 3H), 6.82 (dd, *J* = 8.3, 2.0 Hz, 1H), 6.80 - 6.72 (m, 3H), 5.10 (dd, *J* = 13.0, 5.3 Hz, 1H), 4.95 (s, 2H), 4.71 (s, 4H), 4.49 (t, *J* = 6.9 Hz, 1H), 4.19 (s, 4H), 3.77 (d, *J* = 7.2 Hz, 1H), 3.08 - 3.02 (m, 2H), 3.00 (s, 3H), 2.92 (d, *J* = 12.5 Hz, 1H), 2.54 (d, *J* = 5.3 Hz, 2H), 2.02 - 1.94 (m, 3H), 1.57 (s, 6H). LC/MS (ESI+) calcd for C₄₃H₄₄N₆O₇( [M+H]⁺) *m*/*z:* 757.3; found 757.3.

### Example 101: (S) -3-(5-((1r,3S)-3-(4-(2-(5-methyl-1,2,4-oxadiazol-3-yl)pyrimidin-5-yl)oxy)phenyl)propan-2-yl)phenoxy)cyclobutyl)amino)-1-oxoindolin-2-yl)piperidin-2,6-dione

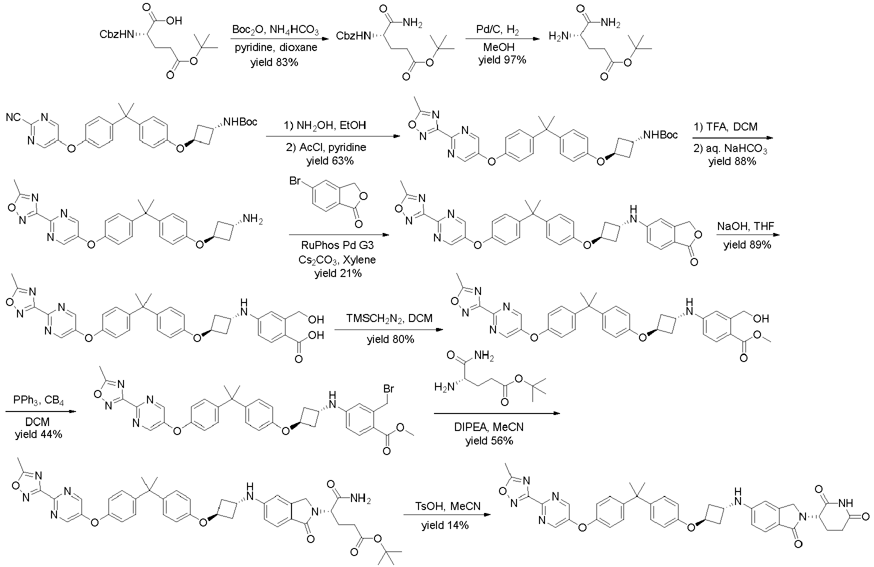

### Step 1: Synthesis of tert-butyl (S)-5-amino-4-((benzyloxy)carbonyl)amino)-5-oxopentanoate

(*S*)-2-((benzyloxy)carbonyl)amino)-5-(tert-butoxy)-5-oxopentanoic acid (10 g, 30 mmol) was dissolved in 100 mL of dioxane, to which were successively added di-tert-butyl dicarbonate (10.4 g, 48 mmol) and pyridine (4.5 g, 60 mmol), and then the reaction system was purged with argon, and allowed to react for half an hour at 0 °C under argon atmosphere. The system was kept at 0 °C, ammonium bicarbonate (7.1 g, 90 mmol) was added, and then the mixture was reacted overnight at room temperature. After completion of the reaction detected by TLC, the reaction was quenched by adding water, and then the resultant solution was extracted twice with 100 mL of ethyl acetate. The organic phase was successively washed with 0.5 N of hydrochloric acid (2*50 mL), saturated NaHCO₃ solution (2*50 mL), and saturated brine (2*50 mL), and then dried with anhydrous Na₂SO₄, and concentrated. The residue was triturated in ethyl acetate, to provide 8.2 g of target compound, with a yield of 83%.

### Step 2: Synthesis of tert-butyl (S)-4,5-diamino-5-oxopentanoate

tert-butyl (*S*)-5-amino-4-((benzyloxy)carbonyl)amino)-5-oxopentanoate (8.1 g, 24 mmol) was dissolved in 150 mL of methanol, to which was added 10% Pd/C (800 mg), and then the reaction system was purged with hydrogen using hydrogen balloons. The system was stirred overnight at room temperature. After completion of the reaction detected by TLC, the reaction system was concentrated. The residue was separated and purified by column chromatography, to provide 4.7 g of target compound, with a yield of 97%.

### Step 3: Synthesis of tert-butyl ((1r,3r)-3-(4-(2-(4-((2-(5-methyl-1,2,4-oxadiazol-3-yl)pyrimidin-5-yl)oxy)phenyl)propan-2-yl)phenoxy)cyclobutyl)carbamate

Compound tert-butyl ((1*r*,3*r*)-3-(4-(2-(4-((2-cyanopyrimidin-5-yl)oxy)phenyl) propan-2-yl)phenoxy)cyclobutyl)carbamate (2 g, 4 mmol) was dissolved in 30 mL of absolute ethanol, to which was added hydroxylamine (270 mg, 8 mmol), and then the mixture was allowed to react at 70 °C for 2h. TLC indicated disappearance of the starting materials. Absolute ethanol was removed by concentration. To the residue, was added ethyl acetate, followed by successively washing with water and saturated brine. The organic phase was combined and concentrated. The residue was dissolved in 20 mL of pyridine, to which was added acetyl chloride (630 mg, 8 mmol) in an ice bath, and then the mixture was allowed to react for 3h at 100 °C. After completion of the reaction detected by TLC, most of pyridine was removed by concentration, followed by washing with 0.5 N of hydrochloric acid. The resultant solution was extracted with ethyl acetate. The organic phase were successively washed with water and saturated brine. The combined organic phase was dried and concentrated. The residue was separated and purified by column chromatography, to provide 1.4 g of target compound, with a yield of 63%.

### Step 4: Synthesis of (1r,3r)-3-(4-(2-(4-((2-(5-methyl-1,2,4-oxadiazol-3-yl)pyrimidin-5-yl)oxy)phenyl)propan-2-yl)phenoxy)cyclobutylamine-1-amine

tert-butyl ((1*r*,3*r*)-3-(4-(2-(4-((2-(5-methyl-1,2,4-oxadiazol-3-yl)pyrimidin-5-yl)oxy)phenyl)propan-2-yl)phenoxy)cyclobutyl)carbamate (1.4 g, 2.5 mmol) was dissolved in 20 mL of dichloromethane, to which was added 10 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and extracted with dichloromethane/methanol (10:1) (3*20 mL). The organic phase was washed with saturated brine, dried with anhydrous Na₂SO₄, and concentrated. The residue was separated and purified by column chromatography, to provide 1.02 g of target compound, with a yield of 88%.

### Step 5: Synthesis of 5-((1r,3r)-3-(4-(2-(4-((2-(5-methyl-1,2,4-oxadiazol-3-yl)pyrimidin-5-yl)oxy)phenyl)propan-2-yl)phenoxy)cyclobutyl)amino)isobenzofuran-1(3H)-one

Compound (1*r*,3*r*)-3-(4-(2-(4-((2-(5-methyl-1,2,4-oxadiazol-3-yl)pyrimidin-5-yl)oxy)phenyl)propan-2-yl)phenoxy)cyclobutylamine-1-amine (300 mg, 0.65 mmol), 5-bromophthalide (152 mg, 0.71 mmol), methanesulfonato(2-dicyclohexylphosphino-2',6'-diisopropyloxy-1,1'-biphenyl)(2-amino-1,1'-biphenyl-2-yl)palladium (II) (54 mg, 0.065 mmol), and cesium carbonate (425 mg, 1.3 mmol) were added into 5 mL of dioxane, and then the reaction system was purged with helium and reacted overnight at 110 °C under helium atmosphere. After completion of the reaction detected by TLC, the reaction system was filtered over diatomaceous earth, and concentrated. The residue was separated and purified by column chromatography, to provide 79 mg of target compound, with a yield of 21%.

### Step 6: Synthesis of 2-(hydroxylmethyl)-4-((1r,3r)-3-(4-(2-(4-((2-(5-methyl-1,2,4-oxadiazol-3-yl)pyrimidin-5-yl)oxy)phenyl)propan-2-yl)phenoxy)cyclobutyl)amino) benzoic acid

5-((1*r*,3*r*)-3-(4-(2-(4-((2-(5-methyl-1,2,4-oxadiazol-3-yl)pyrimidin-5-yl)oxy) phenyl)propan-2-yl)phenoxy)cyclobutyl)amino)isobenzofuran-1(3H)-one (79 mg, 0.13 mmol) was added into 1 mL of THF, to which was added 1 mL of methanol, followed by addition of 5N NaOH solution (0.2 mL, 1 mmol), and then the mixture was heated to 40 °C and reacted for half an hour. After completion of the reaction detected by TLC, the reaction solution was naturally cooled to room temperature, and the pH was adjusted to acidity with 0.5 N dilute hydrochloric acid. The resultant solution was extracted with ethyl acetate. The organic phase was washed with saturated brine (3*4 mL), dried with anhydrous Na₂SO₄, and concentrated. The residue was separated and purified by column chromatography, to provide 70 mg of target compound, with a yield of 89%.

### Step 7: Synthesis of methyl 2-(hydroxylmethyl)-4-((1r,3r)-3-(4-(2-(4-((2-(5-methyl-1,2,4-oxadiazol-3-yl)pyrimidin-5-yl)oxy)phenyl)propan-2-yl)phenoxy)cyclobutyl) amino)benzoate

Compound 2-(hydroxylmethyl)-4-((1*r*,3*r*)-3-(4-(2-(4-((2-(5-methyl-1,2,4-oxadiazol-3-yl)pyrimidin-5-yl)oxy)phenyl)propan-2-yl)phenoxy)cyclobutyl)amino) benzoic acid (70 mg, 0.11 mmol) was dissolved in 1 mL of dichloromethane, to which was added (trimethylsilyl)diazomethane (0.5 mL, 0.5 mmol) in an ice bath, and then the mixture was stirred for 0.5 h at room temperature. After completion of the reaction detected by TLC, the solvent was removed by concentration. The residue was separated and purified by column chromatography, to provide 55 mg of target compound, with a yield of 80%.

### Step 8: Synthesis of methyl 2-(bromomethyl)-4-((1r,3r)-3-(4-(2-(4-((2-(5-methyl-1,2,4-oxadiazol-3-yl)pyrimidin-5-yl)oxy)phenyl)propan-2-yl)phenoxy)cyclobutyl) amino)benzoate

Compound methyl 2-(hydroxylmethyl)-4-((1*r*,3*r*)-3-(4-(2-(4-((2-(5-methyl-1,2,4-oxadiazol-3-yl)pyrimidin-5-yl)oxy)phenyl)propan-2-yl)phenoxy)cyclobutyl)amino) benzoate (55 mg, 0.09 mmol) was dissolved in 1 mL of dichloromethane, to which was added triphenylphosphine (47 mg, 0.18 mmol), followed by addition of CBr₄ (60 mg, 0.18 mmol), and then the mixture was stirred for 0.5 h at room temperature. After completion of the reaction detected by TLC, the solvent was removed by concentration. The residue was separated and purified by column chromatography, to provide 27 mg of target compound, with a yield of 44%.

### Step 9: Synthesis of tert-butyl (S)-5-amino-4-(5-((1r,3S)-3-(4-(2-(4-((2-(5-methyl-1,2,4-oxadiazol-3-yl)pyrimidin-5-yl)oxy)phenyl)propan-2-yl)phenoxy)cyclobutyl) amino)-1-oxoindolin-2-yl)-5-oxopentanedioate

Methyl 2-(bromomethyl)-4-((1*r*,3*r*)-3-(4-(2-(4-((2-(5-methyl-1,2,4-oxadiazol-3-yl)pyrimidin-5-yl)oxy)phenyl)propan-2-yl)phenoxy)cyclobutyl)amino)benzoate (27 mg, 0.04 mmol) was dissolved in 1 mL of acetonitrile, to which were successively added tert-butyl (*S*)-4,5-diamino-5-oxopentanoate (8 mg, 0.04 mmol) and DIPEA (10 mg, 0.08 mmol), and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 18 mg of target compound, with a yield of 56%.

### Step 10. Synthesis of (S) -3-(5-((1r,3S)-3-(4-(2-(5-methyl-1,2,4-oxadiazol-3-yl)pyrimidin-5-yl)oxy)phenyl)propan-2-yl)phenoxy)cyclobutyl)amino)-1-oxoindolin-2-yl)piperidin-2,6-dione

Methyl 2-((((*S*)-1-amino-5-(tert-butoxy)-1,5-dioxopentan-2-yl)amino)-4-((1*r*,3*S*)-3-(4-(2-(4-((2-(5-methyl-1,2,4-oxadiazol-3-yl)pyrimidin-5-yl)oxy)phenyl) propan-2-yl)phenoxy)amino)benzoate (18 mg, 0.02 mmol) was dissolved in 1 mL of acetonitrile, to which was added p-toluenesulfonic acid (17 mg, 0.1 mmol), and then the mixture was heated to 60 °C and reacted overnight. TLC indicated disappearance of the starting materials. The reaction solution was naturally cooled to room temperature, and then the reaction was quenched by adding water. The resultant solution was extracted with 5 mL of ethyl acetate. The organic phase was washed with saturated brine (3*5 mL), dried with anhydrous Na₂SO₄, and concentrated. The residue was separated and purified by column chromatography to provide 2 mg of compound, with a yield of 14%. LC/MS (ESI+) calcd for C₃₉H₃₇N₇O₆( [M+H]⁺) *m*/*z:* 700.3; found 700.3; 98% ee from chiral SFC analysis.

### Example 102: 2-(2,6-dioxopiperidin-3-yl)-5-fluoro-6-((1r,3r)-3-(4-(2-(4-((2-(5-methyl-1,2,4-oxadiazol-3-yl)pyrimidin-5-yl)oxy)phenyl)propan-2-yl)phenoxy) cyclobutyl)amino)isoindolin-1,3-dione

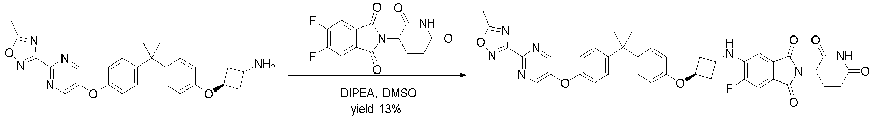

(1*r*,3*r*)-3-(4-(2-(4-((2-(5-methyl-1,2,4-oxadiazol-3-yl)pyrimidin-5-yl)oxy)phenyl) propan-2-yl)phenoxy)cyclobutylamine-1-amine (100 mg, 0.22 mmol), 2-(2,6-dioxopiperidin-3-yl)-5,6-difluoroisoindolin-1,3-dione (65 mg, 0.22 mmol) and DIPEA (86 mg, 0.66 mmol) were successively added into 10 mL of DMSO, and then the mixture was heated to 60 °C and reacted overnight. The reaction solution was naturally cooled to room temperature, and extracted with 5 mL of ethyl acetate. The organic phase was washed with saturated brine (3*5 mL), dried with anhydrous Na₂SO₄, and concentrated. The residue was separated and purified by column chromatography to provide 21 mg of compound 2-(2,6-dioxopiperidin-3-yl)-5-fluoro-6-((1*r*,3*r*)-3-(4-(2-(4-((2-(5-methyl-1,2,4-oxadiazol-3-yl)pyrimidin-5-yl)oxy)phenyl)propan-2-yl) phenoxy)cyclobutyl)amino)isoindolin-1,3-dione, with a yield of 13%. ¹H NMR (400 MHz, DMSO-d₆) *δ* 11.08 (s, 1H), 8.74 (s, 2H), 7.62 (d, *J* = 10.2 Hz, 1H), 7.33 - 7.28 (m, 2H), 7.28 - 7.24 (m, 1H), 7.18 - 7.12 (m, 4H), 6.92 (d, *J* = 7.1 Hz, 1H), 6.80 - 6.74 (m, 2H), 5.06 (dd, *J* = 12.8, 5.4 Hz, 1H), 4.88 (s, 1H), 4.23 (d, *J* = 5.3 Hz, 1H), 2.92 - 2.82 (m, 1H), 2.69 (s, 3H), 2.63 - 2.52 (m, 5H), 2.00 (d, *J* = 7.3 Hz, 2H), 1.63 (s, 6H). LC/MS (ESI+) calcd for C₃₉H₃₄FN₇O₇( [M+H]⁺) *m*/*z:* 732.3; found 732.3.

### Example 103: 2-(2,6-dioxopiperidin-3-yl)-5-((1r,3r)-3-(4-(1-(4-((6-(1-hydroxylethyl)pyridazine-3-yl)oxy)phenyl)cyclopentyl)phenoxy)cyclobutyl) amino)isoindolin-1,3-dione

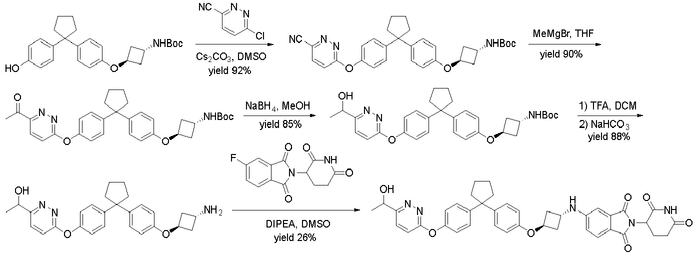

### Step 1: Synthesis of tert-butyl ((1r,3r)-3-(4-(1-(4-((6-cyanopyridin-3-yl)oxy)phenyl) cyclopentyl)phenoxy)cyclobutyl)carbamate

tert-butyl ((1*r*,3*r*)-3-(4-(1-(4-hydroxylphenyl)cyclopentyl)phenoxy)cyclobutyl) carbamate (423 mg, 1 mmol), 6-chloropyridin-3-carbonitrile (140 mg, 1 mmol) and cesium carbonate (652 mg, 2 mmol) were successively added to 10 mL of DMF, and then the mixture was allowed to react overnight at room temperature. After completion of the reaction detected by TLC, the resultant solution was extracted with 10 mL of ethyl acetate. The organic phase was washed with saturated brine (3*10 mL), dried with anhydrous Na₂SO₄, and concentrated. The residue was separated and purified by column chromatography, to provide 484 mg of target compound, with a yield of 92%.

### Step 2: Synthesis of tert-butyl ((1r,3r)-3-(4-(1-(4-((6-acetylpyridazine-3-yl)oxy)phenyl) cyclopentyl)phenoxy)cyclobutyl)carbamate

Compound tert-butyl ((1*r*,3*r*)-3-(4-(1-(4-((6-cyanopyridin-3-yl)oxy)phenyl) cyclopentyl)phenoxy)cyclobutyl)carbamate (484 mg, 0.92 mmol) was dissolved in 10 mL of anhydrous tetrahydrofuran, to which was added methylmagnesium bromide (5 mL, 5 mmol) in an ice bath, and then the mixture was stirred for 0.5 h at room temperature. After completion of the reaction detected by TLC, 0.5N hydrochloric acid was added, and then the mixture was stirred for 10 min at room temperature. The resultant solution was extracted with 10 mL of ethyl acetate. The organic phase was washed with saturated brine (3*10 mL), dried with anhydrous Na₂SO₄, and concentrated. The residue was separated and purified by column chromatography, to provide 450 mg of target compound, with a yield of 90%.

### Step 3: Synthesis of tert-butyl ((1r,3r)-3-(4-(1-(4-((6-(1-hydroxylethyl)pyridazine-3-yl)oxy)phenyl)cyclopentyl)phenoxy)cyclobutyl)carbamate

Compound tert-butyl ((1*r*,3*r*)-3-(4-(1-(4-((6-acetylpyridazine-3-yl)oxy)phenyl) cyclopentyl)phenoxy)cyclobutyl)carbamate (450 mg, 0.8 mmol) was dissolved in 10 mL of methanol, to which was added sodium borohydride (152 mg, 4 mmol) in an ice bath, and then the mixture was stirred at room temperature for 3h. After completion of the reaction detected by TLC, saturated NH₄Cl solution was added to quench the reaction. The resultant solution was extracted with 10 mL of ethyl acetate. The organic phase was washed with saturated brine (3 * 10 mL), dried with anhydrous Na₂SO₄, and concentrated. The residue was separated and purified by column chromatography, to provide 380 mg of target compound, with a yield of 85%.

### Step 4: Synthesis of 1-(6-(4-(1-(4-((1r,3r)-3-aminocyclobutyloxy)phenyl)cyclopentyl)phenoxy)pyridazine-3-yl)ethane-1-ol

tert-butyl ((1*r*,3*r*)-3-(4-(1-(4-((6-(1-hydroxylethyl)pyridazine-3-yl)oxy)phenyl) cyclopentyl)phenoxy)cyclobutyl)carbamate (380 mg, 0.7 mmol) was dissolved in 10 mL of dichloromethane, to which was added 5 mL of trifluoroacetic acid in an ice bath, and then the mixture was stirred for 0.5 h at room temperature. After completion of the reaction detected by TLC, the reaction system was poured into saturated NaHCO₃ solution in an ice bath. The system was alkaline, and then extracted with dichloromethane/methanol (10:1) (3*10 mL). The organic phase was washed with saturated brine, dried with anhydrous Na₂SO₄, and concentrated. The residue was separated and purified by column chromatography, to provide 276 mg of target compound, with a yield of 88%.

### Step 5: Synthesis of 2-(2,6-dioxopiperidin-3-yl)-5-((1r,3r)-3-(4-(1-(4-((6-(1-hydroxylethyl)pyridazine-3-yl)oxy)phenyl)cyclopentyl)phenoxy)cyclobutyl)amino) isoindolin-1,3-dione

1-(6-(4-(1-(4-((1*r*,3*r*)-3-aminocyclobutyloxy)phenyl)cyclopentyl)phenoxy) pyridazine-3-yl)ethane-1-ol (276 mg, 0.62 mmol), 2-(2,6-dioxopiperidin-3-yl)-5-fluoroisoindolin-1,3-dione (171 mg, 0.62 mmol) and DIPEA (390 mg, 3 mmol) were successively added into 30 mL of DMSO, and then the mixture was heated to 60 °C and reacted overnight. The reaction solution was naturally cooled to room temperature, and extracted with 30 mL of ethyl acetate. The organic phase was washed with saturated brine (3*30 mL), dried with anhydrous Na₂SO₄, and concentrated. The residue was separated and purified by column chromatography to provide 114 mg of target compound, with a yield of 26%. ¹H NMR (400 MHz, DMSO-*d*₆) *δ* 11.06 (s, 1H), 7.79 (d, *J* = 9.2 Hz, 1H), 7.58 (d, *J* = 8.4 Hz, 1H), 7.52 (d, *J* = 5.4 Hz, 1H), 7.43 - 7.31 (m, 3H), 7.29 - 7.19 (m, 2H), 7.11 - 7.03 (m, 2H), 6.86 (s, 1H), 6.76 (dd, *J* = 19.0, 8.6 Hz, 3H), 5.59 (d, *J* = 4.7 Hz, 1H), 5.03 (dd, *J* = 12.9, 5.4 Hz, 1H), 4.95 - 4.79 (m, 2H), 4.13 (d, *J* = 4.7 Hz, 1H), 2.87 (ddd, *J* = 17.3, 14.0, 5.5 Hz, 1H), 2.61 - 2.51 (m, 3H), 2.42 (q, *J* = 6.6 Hz, 2H), 2.27 (s, 4H), 2.04 - 1.95 (m, 1H), 1.62 (s, 4H), 1.39 (d, *J* = 6.5 Hz, 3H). LC/MS (ESI+) calcd for C₄₀H₃₉N₅O₇( [M+H]⁺) *m*/*z:* 702.3; found 702.3.

### Example 104: 5-((1r,3r)-3-(4-(1-(4-((6-acetylpyridazine-3-yl)oxy)phenyl) cyclopentyl)phenoxy)cyclobutyl)amino)-2-(2,6-dioxopiperidin-3-yl)isoindolin-1,3-dione

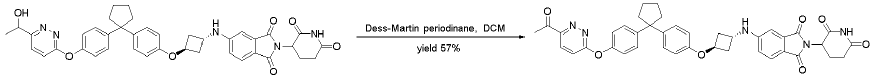

2-(2,6-dioxopiperidin-3-yl)-5-((1*r*,3*r*)-3-(4-(1-(4-((6-(1-hydroxylethyl) pyridazine-3-yl)oxy)phenyl)cyclopentyl)phenoxy)cyclobutyl)amino)isoindolin-1,3-dione (60 mg, 0.085 mmol) was dissolved in 2 mL of dichloromethane, to which was added Dess-Martin periodinane (106 mg, 0.25 mmol), and then the mixture was allowed to react at room temperature for 3h. TLC detection indicated completion of the reaction. The reaction solution was filtered over diatomite, and then the filtrate was concentrated. The residue was separated and purified by column chromatography to provide 34 mg of compound 5-((1*r*,3*r*)-3-(4-(1-(4-((6-acetylpyridazine-3-yl)oxy)phenyl)cyclopentyl) phenoxy)cyclobutyl)amino)-2-(2,6-dioxopiperidin-3-yl)isoindolin-1,3-dione, with a yield of 57%. ¹H NMR (400 MHz, DMSO-d₆) *δ* 11.06 (s, 1H), 8.14 (d, *J* = 9.2 Hz, 1H), 7.55 (dt, *J* = 14.5, 6.8 Hz, 3H), 7.47 - 7.35 (m, 2H), 7.35 - 7.22 (m, 2H), 7.24 - 7.09 (m, 2H), 6.86 (s, 1H), 6.77 (dd, *J* = 15.2, 8.8 Hz, 3H), 5.03 (dd, *J* = 12.9, 5.4 Hz, 1H), 4.93 - 4.78 (m, 1H), 4.13 (d, *J* = 5.1 Hz, 1H), 2.86 (d, *J* = 12.1 Hz, 1H), 2.67 (s, 3H), 2.62 - 2.51 (m, 3H), 2.43 (p, *J* = 6.0 Hz, 2H), 2.28 (s, 4H), 2.05 - 1.92 (m, 1H), 1.63 (s, 4H). LC/MS (ESI+) calcd for C₄₀H₃₇N₅O₇( [M+H]⁺) *m*/*z:* 700.3; found 700.3.

### Example 105: 2-(2,6-dioxopiperidin-3-yl)-5-(1r,3r)-3-(4-(4-(6-(1-hydroxylethyl)pyridazine-3-yl)oxy)phenyl)tetrohydro-2H-pyran-4-yl)phenoxy)cyclobutyl)amino)isoindolin-1,3-dione

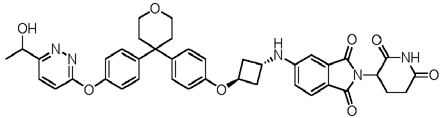

The target compound was synthesized by a method similar to that of Example 103. ¹H NMR (400 MHz, DMSO-d₆) *δ* 11.06 (s, 1H), 8.57 (s, 2H), 7.59 (d, *J* = 8.3 Hz, 1H), 7.52 (d, *J* = 5.4 Hz, 1H), 7.43 - 7.31 (m, 2H), 7.24 (d, *J* = 8.5 Hz, 2H), 7.07 - 6.95 (m, 2H), 6.85 (s, 1H), 6.78 (t, *J* = 8.8 Hz, 3H), 5.22 (d, *J* = 5.5 Hz, 1H), 5.03 (dd, *J* = 12.9, 5.4 Hz, 1H), 4.86 (s, 1H), 4.79 (q, *J* = 6.3 Hz, 1H), 4.13 (s, 1H), 3.58 (t, *J* = 5.0 Hz, 4H), 2.96 - 2.80 (m, 1H), 2.54 (s, 4H), 2.39 (d, *J* = 31.0 Hz, 6H), 1.99 (d, *J* = 12.3 Hz, 1H), 1.40 (d, *J* = 6.6 Hz, 3H). LC/MS (ESI+) calcd for C₄₀H₃₉N₅O₈ ([M+H]⁺) *m*/*z:* 718.3; found 718.3.

### Example 106: 5-((1r,3r)-3-(4-(4-((6-acetylpyridazine-3-yl)oxy)phenyl)tetrohydro-2H-pyran-4-yl)phenoxy)cyclobutyl)amino)-2-(2,6-dioxopiperidin-3-yl)isoindolin-1,3-dione

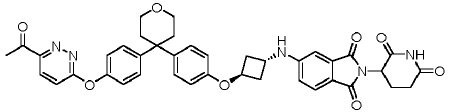

The target compound was synthesized by a method similar to that of Example 105. ¹H NMR (400 MHz, DMSO-d₆) *δ* 11.07 (s, 1H), 8.69 (s, 2H), 7.59 (d, *J* = 8.4 Hz, 1H), 7.52 (d, *J* = 5.4 Hz, 1H), 7.45 - 7.37 (m, 2H), 7.30 - 7.23 (m, 2H), 7.18 - 7.11 (m, 2H), 6.86 (s, 1H), 6.77 (d, *J* = 8.9 Hz, 3H), 5.03 (dd, *J* = 13.0, 5.4 Hz, 1H), 4.91 - 4.80 (m, 1H), 4.18 - 4.09 (m, 1H), 3.59 (d, *J* = 5.3 Hz, 4H), 3.17 (d, *J* = 5.2 Hz, 1H), 2.96 - 2.79 (m, 1H), 2.64 (s, 3H), 2.60 - 2.53 (m, 2H), 2.43 (d, *J* = 5.5 Hz, 2H), 2.37 (d, *J* = 5.9 Hz, 4H), 2.00 (d, *J* = 7.8 Hz, 2H). LC/MS (ESI+) calcd for C₄₀H₃₇N₅O₈ ([M+H]⁺) *m*/*z:* 716.3; found 716.3.

### Example 107: 5-(3-(4-(2-(2-acetylpyrimidin-4-yl)methoxy)phenyl)propan-2-yl)phenoxy)piperidin-1-yl)-2-(2,6-dioxopiperidin-3-yl)isoindolin-1,3-dione

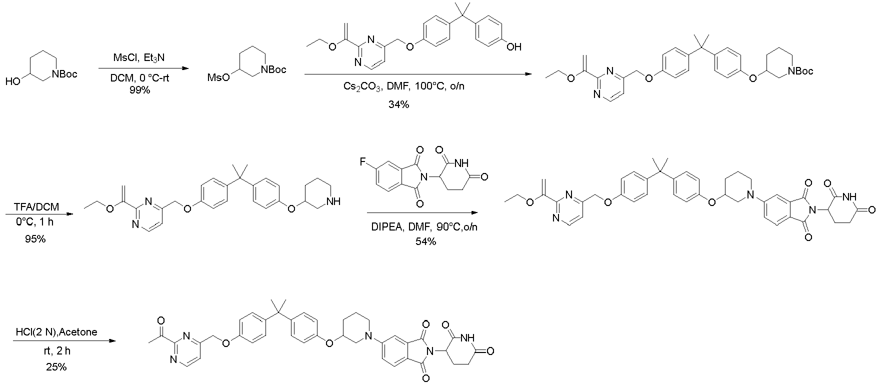

### Step 1: Synthesis of N-Boc-3-methylsulfonyloxypiperidine

*N*-Boc-3-hydroxylpiperidine (1.00 g, 5 mmol) was dissolved in 10 mL of dichloromethane, to which was added triethylamine (2.01 g, 20 mmol), followed by addition of methanesulfonyl choride (851 mg, 7.5 mmol) in an ice bath, and then the mixture was warmed to room temperature and reacted for 2 h. TLC indicated disappearance of starting materials, and then saturated NH₄Cl aqueous solution was added to quench the reaction. The resultant solution was extracted with 10 mL of dichloromethane. The organic phase was combined, washed with saturated brine, dried over anhydrous MgSO₄, and concentrated to dry under reduced pressure, to provide the crude product *N*-Boc-3-methylsulfonyloxypiperidine (1.50 g).

### Step 2: Synthesis of tert-butyl 3-(4-(2-(4-((2-(1-ethoxyvinyl)pyrimidin-4-yl)methoxy)phenyl)propan-2-yl)phenoxy)piperidin-1 -carboxylate

N Boc-3-methylsulfonyloxypiperidine (350 mg crude product, 0.78 mmol) and 4-(2-(4-((2-(1-ethoxyvinyl)pyrimidin-4-yl)methoxy)phenyl)propan-2-yl)phenol (100 mg, 0.26 mmol) were dissolved in 5 mL of DMF, to which was added Cs₂CO₃ (167 mg, 0.51 mmol), and then the mixture was allowed to react overnight at 100 °C. The reaction was quenched by adding water. The resultant solution was extracted with 10 mL of ethyl acetate. The organic phase was combined, washed with saturated brine, dried with anhydrous Na₂SO₄, and concentrated. The residue was separated and purified by prep-TLC, to provide compound tert-butyl 3-(4-(2-(4-((2-(1-ethoxyvinyl)pyrimidin-4-yl)methoxy)phenyl)propan-2-yl)phenoxy)piperidin-1-carboxylate (50 mg), with a yield of 34%. LC/MS (ESI+) calcd for C₃₄H₄₃N₃O₅ ([M+H]⁺) *m*/*z* 574.3; found 574.3.

### Step 3: Synthesis of 2-(1-ethoxyvinyl)-4-((4-(2-(4-(piperidin-3-oxy)phenyl)propan-2-yl)phenoxy)methyl)pyrimidine

tert-butyl 3-(4-(2-(4-((2-(1-ethoxyvinyl)pyrimidin-4-yl)methoxy)phenyl)propan-2-yl)phenoxy)piperidin-1-carboxylate (50 mg, 0.08 mmol) was dissolved in 4 mL of dichloromethane, to which was added 1 mL of trifluoroacetic acid in an ice bath, and then the reaction was further stirred in an ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 2-(1-ethoxyvinyl)-4-((4-(2-(4-(piperidin-3-oxy)phenyl)propan-2-yl)phenoxy)methyl) pyrimidine (40 mg), with a yield of 95%. LC/MS (ESI+) calcd for C₂₉H₃₅N₃O₃ ( [M+H]⁺ ) *m*/*z* 474.3; found 474.3.

### Step 4: Synthesis of 2-(2,6-dioxopiperidin-3-yl)-5-(3-(4-(2-(2-(1-ethoxyvinyl) pyrimidin-4-yl)methoxy)phenyl)propan-2-yl)phenoxy)piperidin-1-yl)isoindolin-1,3-dione

2-(1-ethoxyvinyl)-4-((4-(2-(4-(piperidin-3-oxy)phenyl)propan-2-yl)phenoxy) methyl)pyrimidine (40 mg, 0.085 mmol) was dissolved in 5 mL of DMSO, to which were added 3 drops of DIPEA and 2-(2,6-dioxopiperidin-3-yl)-5-fluoroisoindolin-1,3-dione (30 mg, 0.11 mmol), and then the mixture was allowed to react overnight at 90 °C. Water was added to quench the reaction. The resultant solution was extracted with ethyl acetate for three times. The organic phase was washed with saturated NaCl solution, dried with anhydrous Na₂SO₄, concentrated and rotatory evaporated to dry. The residue was separated and purified by prep-TLC, to provide 2-(2,6-dioxopiperidin-3-yl)-5-(3-(4-(2-(2-(1-ethoxyvinyl)pyrimidin-4-yl)methoxy)phenyl)propan-2-yl) phenoxy)piperidin-1-yl)isoindol-1,3-dione (34 mg), with a yield of 54%. LC/MS (ESI+) calcd for C₄₂H₄₃N₅O₇ ( [M+H]⁺) *m*/*z* 730.3; found 730.3.

### Step 5: Synthesis of 5-(3-(4-(2-(2-acetylpyrimidin-4-yl)methoxy)phenyl)propan-2-yl)phenoxy)piperidin-1-yl)-2-(2,6-dioxopiperidin-3-yl)isoindolin-1,3-dione

2-(2,6-dioxopiperidin-3-yl)-5-(3-(4-(2-(2-(1-ethoxyvinyl)pyrimidin-4-yl) methoxy)phenyl)propan-2-yl)phenoxy)piperidin-1-yl)isoindol-1,3-dione (34 mg, 0.046 mmol) was dissolved in 2 mL of acetone, to which was added 2 mL of 2N HCl aqueous solution, and then the mixture was allowed to react at room temperature for 2h. After completion of the reaction detected by TLC, 10 mL of water was added, and then the resultant solution was extracted with ethyl acetate for three times. The organic layers were combined, washed with saturated brine, dried with anhydrous Na₂SO₄, and concentrated to dry under reduced pressure. The residue was separated and purified by prep-TLC, to provide the product 5-(3-(4-(2-(2-acetylpyrimidin-4-yl)methoxy)phenyl)propan-2-yl)phenoxy)piperidin-1-yl)-2-(2,6-dioxopiperidin-3-yl)isoindolin-1,3-dione (8 mg, yellow solid), with a yield of 25%. ¹H NMR (400 MHz, DMSO-d₆) *δ* 11.06 (s, 1H), 9.01 (d, *J* = 5.1 Hz, 1H), 7.77 (d, *J* = 5.1 Hz, 1H), 7.62 (d, *J* = 8.5 Hz, 1H), 7.26 (dd, *J* = 5.4, 2.3 Hz, 1H), 7.22 - 7.17 (m, 1H), 7.17 - 7.12 (m, 2H), 7.09 (d, *J* = 8.6 Hz, 2H), 6.96 (d, *J* = 8.8 Hz, 2H), 6.82 (dd, *J* = 8.9, 2.4 Hz, 2H), 5.26 (s, 2H), 5.06 (dd, *J* = 12.8, 5.4 Hz, 1H), 4.48 (s, 1H), 3.83 (d, *J* = 13.3 Hz, 1H), 3.60 (s, 1H), 3.56 - 3.40 (m, 2H), 2.95 - 2.81 (m, 1H), 2.67 (s, 3H), 2.63 - 2.52 (m, 2H), 2.00 (dd, *J* = 14.9, 7.1 Hz, 3H), 1.83 (s, 1H), 1.72 (d, *J* = 8.8 Hz, 1H), 1.58 (s, 6H).
LC/MS (ESI+) calcd for C₄₀H₃₉N₅O₇ ([M+H]⁺) *m*/*z:* 702.3; found 702.3.

### Example 108: Synthesis of 5-(4-((4-(2-(4-((2-acetylpyrimidin-4-yl)methoxy)phenyl) propan-2-yl)phenoxy)methyl)piperidin-1-yl)-2-(2,6-dioxopiperidin-3-yl)isoindol-1,3-dione

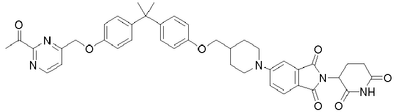

The target compound was synthesized by a method similar to that of Example 107. ¹H NMR (400 MHz, Chloroform-d) *δ* 8.92 (d, J = 5.1 Hz, 1H), 8.12 (s, 1H), 7.71 (dd, J = 22.0, 6.8 Hz, 2H), 7.30 (s, 1H), 7.20 - 7.03 (m, 4H), 6.83 (dd, J = 27.9, 8.4 Hz, 4H), 5.25 (s, 2H), 4.94 (dd, J = 12.2, 5.3 Hz, 1H), 4.00 (d, J = 13.0 Hz, 2H), 3.82 (d, J = 6.2 Hz, 2H), 3.03 (t, J = 12.8 Hz, 2H), 2.79 (d, J = 11.0 Hz, 3H), 2.23 - 2.10 (m, 2H), 1.99 (t, J = 11.3 Hz, 3H), 1.64 (s, 6H), 1.43 (d, J = 16.0 Hz, 4H). LC/MS (ESI+) calcd for C₄₁H₄₁N₅O₇ ([M+H]⁺) *m*/*z* 716, found 716.

### Example 109: Synthesis of 5-((2-(4-(2-(4-((2-acetylpyrimidin-4-yl)methoxy)phenyl)propan-2-yl)phenoxy)ethyl)amino)-2-(2,6-dioxopiperidin-3-yl) isoindolin-1,3-dione

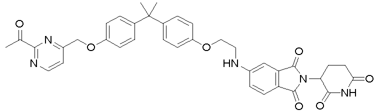

The target compound was synthesized by a method similar to that of Example 107. ¹H NMR (400 MHz, Chloroform-d) *δ* 8.92 (d, J = 5.1 Hz, 1H), 8.13 (s, 1H), 7.74 (d, J = 5.1 Hz, 1H), 7.63 (d, J = 8.3 Hz, 1H), 7.16 (dd, J = 8.8, 3.1 Hz, 4H), 7.03 (d, J = 2.2 Hz, 1H), 6.92 - 6.75 (m, 5H), 5.25 (s, 2H), 4.93 (dd, J = 12.1, 5.2 Hz, 1H), 4.23 - 4.12 (m, 2H), 3.63 (t, J = 5.1 Hz, 2H), 2.94 - 2.65 (m, 6H), 2.17 - 2.08 (m, 1H), 1.64 (s, 6H). LC/MS (ESI+) calcd for C₃₇H₃₅N₅O₇ ([M+H]⁺) *m*/*z* 662, found 662.

### Example 110: Synthesis of 5-(((trans)-3-(4-(2-(4-((2-acetylpyrimidin-4-yl)methoxy) phenyl)propan-2-yl)phenoxy)cyclobutyl)amino)-2-(2,6-dioxopiperidin-3-yl) isoindolin-1,3-dione

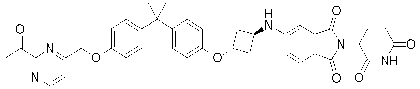

The target compound was synthesized by a method similar to that of Example 107. ¹H NMR (400 MHz, Chloroform-d) *δ* 8.92 (d, J = 5.1 Hz, 1H), 8.08 (s, 1H), 7.74 (dt, J = 5.1, 0.9 Hz, 1H), 7.62 (d, J = 8.2 Hz, 1H), 7.21 - 7.09 (m, 4H), 6.91 - 6.82 (m, 3H), 6.69 (dq, J = 7.1, 3.2, 2.6 Hz, 3H), 5.25 (d, J = 0.8 Hz, 2H), 4.93 (dd, J = 12.2, 5.2 Hz, 1H), 4.85 (td, J = 6.9, 3.5 Hz, 1H), 4.22 (td, J = 7.8, 3.9 Hz, 1H), 2.94 - 2.74 (m, 6H), 2.73 - 2.67 (m, 2H), 2.40 (dt, J = 12.9, 6.1 Hz, 2H), 2.12 (ddd, J = 13.2, 5.8, 2.9 Hz, 1H), 1.64 (s, 6H). LC/MS (ESI+) calcd for C₃₉H₃₇N₅O₇([M+H]⁺) *m*/*z* 688, found 688.

### Example 111: Synthesis of 5-(((trans)-3-(4-(2-(4-((2-acetylpyrimidin-4-yl)methoxy) phenyl)propan-2-yl)phenoxy)cyclobutyl)(methyl)amino)-2-(2,6-dioxopiperidin-3-yl)isoindolin-1,3-dione

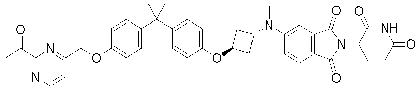

The target compound was synthesized by a method similar to that of Example 107. ¹H NMR (400 MHz, Chloroform-d) *δ* 9.01 - 8.85 (m, 1H), 8.19 (s, 1H), 7.80 - 7.72 (m, 1H), 7.67 (d, J = 8.4 Hz, 1H), 7.22 - 7.07 (m, 5H), 6.88 (td, J = 6.0, 2.8 Hz, 3H), 6.71 (d, J = 8.5 Hz, 2H), 5.25 (s, 2H), 4.94 (dd, J = 12.0, 5.2 Hz, 1H), 4.74 (d, J = 6.7 Hz, 1H), 4.61 (p, J = 7.8 Hz, 1H), 3.06 (s, 3H), 2.95 - 2.51 (m, 10H), 2.19 - 2.08 (m, 1H), 1.65 (s, 6H). LC/MS (ESI+) calcd for C₄₀H₃₉N₅O₇ ([M+H]⁺) *m*/*z* 702, found 702.

### Example 112: Synthesis of 5-((3-((4-(2-(4-((2-(2-oxa-6-azaspiro[3.3]heptan-6-yl)pyrimidin-4-yl)methoxy)phenyl)propan-2-yl)phenoxy)methyl)cyclobutyl) amino)-2-(2,6-dioxopiperidin-3-yl)isoindolin-1,3-dione

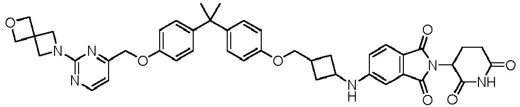

The target compound was synthesized by a method similar to that of Example 59. ¹H NMR (400 MHz, Chloroform-d) *δ* 8.30 (d, *J* = 5.3 Hz, 2H), 7.60 (d, *J* = 8.2 Hz, 1H), 7.14 (t, *J* = 8.4 Hz, 4H), 6.96 - 6.76 (m, 6H), 6.68 (td, *J* = 8.5, 2.1 Hz, 1H), 4.91 (d, *J* = 34.5 Hz, 8H), 4.32 (s, 4H), 3.93 (t, *J* = 8.7 Hz, 2H), 2.95 - 2.42 (m, 6H), 2.16 - 2.10 (m, 1H), 1.91 - 1.77 (m, 2H), 1.64 (d, *J* = 4.8 Hz, 6H). LC/MS (ESI+) calcd for C₄₃H₄₄N₆O₇ ([M+H]⁺) *m*/*z* 757, found 757.

### Example 113: Synthesis of 5-(3-(4-(2-(4-((2-(2-oxa-6-azaspiro[3.3]heptan-6-yl)pyrimidin-4-yl)methoxy)phenyl)propan-2-yl)phenoxy)propoxy)-2-(2,6-dioxopiperidin-3-yl)isoindolin-1,3-dione

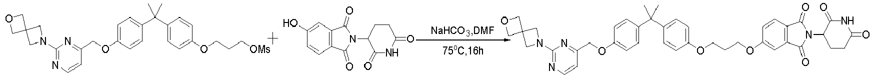

4-(2-(2-oxa-6-azaspiro[3.3]heptan-6-yl)pyrimidin-4-yl)methoxy)phenyl)propan-2-yl)phenoxy)propyl methanesulfonate (554 mg, 1 mmol), 2-(2,6-dioxopiperidin-3-yl)-5-hydroxyisoindolin-1,3-dione (274 mg, 1 mmol) and NaHCO₃ (252 mg, 3 mmol) were successively added into 15 mL of DMF, and then the mixture was heated to 75 °C and reacted for 16h. The reaction solution was naturally cooled to room temperature, and poured to water. The resultant solution was extracted with ethyl acetate. The organic phase was washed with saturated brine, dried with anhydrous Na₂SO₄, and concentrated. The residue was separated and purified by column chromatography to provide 439 mg of product, with a yield of 60%. ¹H NMR (400 MHz, DMSO-d₆) *δ* 11.11 (s, 1H), 8.33 (d, *J* = 5.0 Hz, 1H), 7.83 (d, *J* = 8.3 Hz, 1H), 7.45 (d, *J* = 2.3 Hz, 1H), 7.37 (dd, *J* = 8.3, 2.3 Hz, 1H), 7.09 (dd, *J* = 8.9, 2.5 Hz, 4H), 6.94 - 6.80 (m, 4H), 6.74 (d, *J* = 5.0 Hz, 1H), 5.11 (dd, *J* = 12.9, 5.4 Hz, 1H), 4.94 (s, 2H), 4.71 (s, 4H), 4.32 (t, *J* = 6.2 Hz, 2H), 4.19 (s, 4H), 4.10 (t, *J* = 6.2 Hz, 2H), 2.89 (s, 1H), 2.73 (s, 1H), 2.64 - 2.54 (m, 1H), 2.20 (q, *J* = 6.2 Hz, 2H), 2.10 - 1.99 (m, 1H), 1.56 (s, 6H). LC/MS (ESI+) calcd for C₄₁H₄₁N₅O₈ ([M+H]⁺) *m*/*z* 732, found 732.

### Example 114: Synthesis ofN (2-(4-(2-(4-((2-(2-oxa-6-azaspiro[3.3]heptan-6-yl)pyrimidin-4-yl)methoxy)phenyl)propan-2-yl)phenoxy)ethyl)-2-(2,6-dioxopiperidin-3-yl)-1,3-dioxoisoindolin-5-carbonylamine

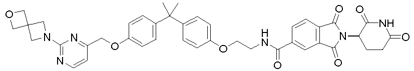

The target compound was synthesized using the similar method. ¹H NMR (400 MHz, DMSO-d₆) *δ* 11.16 (s, 1H), 9.12 (t, *J* = 5.4 Hz, 1H), 8.45 - 8.27 (m, 3H), 8.03 (d, *J* = 7.7 Hz, 1H), 7.10 (d, *J* = 8.5 Hz, 5H), 6.87 (dd, *J* = 8.6, 6.1 Hz, 4H), 6.74 (d, *J* = 5.0 Hz, 1H), 5.19 (dd, *J* = 12.8, 5.3 Hz, 1H), 4.94 (s, 2H), 4.71 (s, 4H), 4.15 (d, *J* = 29.8 Hz, 6H), 3.66 (q, *J* = 5.7 Hz, 2H), 2.89 (ddd, *J* = 17.0, 14.1, 5.2 Hz, 1H), 2.59 (td, *J* = 15.6, 14.9, 4.1 Hz, 2H), 2.08 (ddt, *J =* 12.3, 7.1, 4.3 Hz, 1H), 1.56 (s, 6H). LC/MS (ESI+) calcd for C₄₁H₄₀N₆O₈ ([M+H]⁺) *m*/*z* 745, found 745.

### Example 115: Synthesis of 5-(((trans)-3-(4-(2-(4-((2-(cyclopropanecarbonyl) pyrimidin-4-yl)methoxy)phenyl)propan-2-yl)phenoxy)cyclobutyl)amino)-2-(2,6-dioxopiperidin-3-yl)isoindolin-1,3-dione

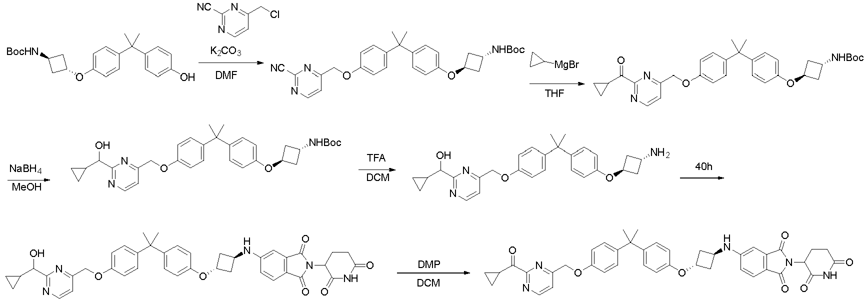

### Step 1: Synthesis of (1r,3r)-3-(4-(2-(4-((2-cyanopyrimidin-4-yl)methoxy)phenyl) propan-2-yl)phenoxy)cyclobutyl)carbamate

4-(chloromethyl)pyrimidin-2-carbonitrile (153 mg, 1 mmol) and tert-butyl ((1r,3r)-3-(4-(2-(4-hydroxylphenyl)propan-2-phenoxy)cyclobutyl)carbamate (397 mg, 1 mmol) were dissolved in DMF (8 mL), to which was added K₂CO₃ (276 mg, 2mmol), and then the mixture was allowed to react at room temperature for 15 h. The reaction solution was poured to water and extracted. The organic phase was combined, washed with saturated brine, and dried with anhydrous Na₂SO₄, followed by column chromatography, to provide 413 mg of product (yield 80%), LC/MS (ESI+) calcd for C₃₀H₃₄N₄O₄ ([M+H]⁺) *m*/*z* 515, found 515.

### Step 2: Synthesis of Synthesis of tert-butyl ((1r,3r)-3-(4-(2-(4-(2-(cyclopropanecarbonyl)pyrimidin-4-yl)methoxy)phenyl)propan-2-yl)phenoxy) cyclobutyl)carbamate

(1r,3r)-3-(4-(2-(4-((2-cyanopyrimidin-4-yl)methoxy)phenyl)propan-2-yl)phenoxy) cyclobutyl)carbamate (514 mg, 1 mmol) was dissolved in dry THF(10 mL), to which was added the solution of cyclopropylmagnesium bromide in THF (10 mL, 5 mmol) in an ice water bath under nitrogen protection, and then the mixture was allowed to react for 10 min in the ice water bath. The reaction solution was poured to 0.5N of cold dilute hydrochloric acid, and extracted. The organic phase was combined, washed with saturated brine, dried with anhydrous Na₂SO₄, followed by column chromatography, to provide 398 mg of product (yield 70%), LC/MS (ESI+) calcd for C₃₃H₃₉N₃O₅([M+H]⁺) *m*/*z* 558, found 558.

### Step 3: Synthesis of tert-butyl ((1r,3r)-3-(4-(2-(4-(2-(cyclopropyl(hydroxyl)methyl) pyrimidin-4-yl)methoxy)phenyl)propan-2-yl)phenoxy)cyclobutyl)carbamate

tert-butyl ((1r,3r)-3-(4-(2-(4-(2-(cyclopropanecarbonyl)pyrimidin-4-yl)methoxy) phenyl)propan-2-yl)phenoxy)cyclobutyl)carbamate (557 mg, 1 mmol) was dissolved in 15 mL of methanol, to which was added sodium borohydride (114 mg, 3 mmol) in an ice water bath in batches. After that, the mixture was allowed to react for half an hour. The reaction solution was poured to water, and extracted. The organic phase was combined, washed with saturated brine, dried with anhydrous Na₂SO₄, followed by column chromatography, to provide 502 mg of product (yield 90%), LC/MS (ESI+) calcd for C₃₃H₄₁N₃O₅ ([M+H]⁺) *m*/*z* 560, found 560. In following steps, the method, similar to that of Example 107, was used to synthesize 5-((1r,3r)-3-(4-(2-(2-(cyclopropyl(hydroxyl)methyl)pyrimidin-4-ylmethoxy)phenyl) propan-2-yl)phenoxy)cyclobutyl)amino)-2-(2,6-dioxopiperidin-3-yl)isoindol-1,3-dione. LC/MS (ESI+) calcd for C₄₁H₄₁N₅O₇ ([M+H]⁺) *m*/*z* 716, found 716.

### Step 6: Synthesis of 5-((1r,3r)-3-(4-(2-(4-(2-(cyclopropanecarbonyl)pyrimidin-4-yl)methoxy)phenyl)propan-2-yl)phenoxy)cyclobutyl)amino)-2-(2,6-dioxopyridin-3-yl)isoindolin-1,3-dione

5-((1r,3r)-3-(4-(2-(2-(cyclopropyl(hydroxyl)methyl)pyrimidin-4-ylmethoxy) phenyl)propan-2-yl)phenoxy)cyclobutyl)amino)-2-(2,6-dioxopiperidin-3-yl)isoindol-1,3-dione (715mg, 1 mmol) was dissolved in 30 mL of DCM, to which was added Dess-Martin periodinane (640mg, 1.5 mmol) in an ice water bath in portions, and then the mixture was naturally warmed to room temperature and reacted for 16 h. The reaction solution was poured to saturated NaHSO₃ aqueous solution, and then filtered over diatomite pad. The filtrate was extracted with ethyl acetate.The organic phase was washed with saturated brine, dried with anhydrous Na₂SO₄, and concentrated. The residue was separated and purified by column chromatography to provide 356 mg of product, with a yield of 50%. LC/MS (ESI+) calcd for C₄₁H₃₉N₅O₇ ([M+H]⁺) *m*/*z* 714, found 714.

### Example 116: Synthesis of 5-(((trans)-3-(4-(2-(4-((6-(cyclopropyl(hydroxyl) methyl)pyridin-2-yl)methoxy)phenyl)propan-2-yl)phenoxy) cyclobutyl)amino)-2-(2,6-dioxopiperidin-3-yl)isoindolin-1,3-dione

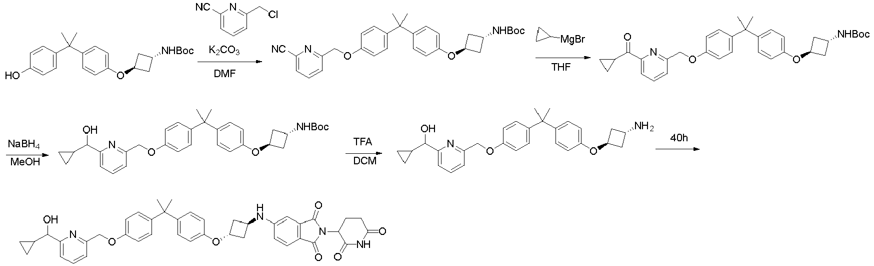

The target compound was synthesized by a method similar to that of Example 115. ¹H NMR (400 MHz, DMSO-d₆) *δ* 11.07 (s, 1H), 7.81 (t, *J* = 7.7 Hz, 1H), 7.56 (dd, *J* = 25.0, 6.9 Hz, 2H), 7.39 (dd, *J* = 20.2, 7.7 Hz, 2H), 7.11 (dd, *J* = 8.7, 4.8 Hz, 4H), 6.99 - 6.64 (m, 6H), 5.32 (d, *J* = 4.7 Hz, 1H), 5.18 - 4.98 (m, 3H), 4.86 (p, *J* = 6.1 Hz, 1H), 4.15 (dt, *J* = 9.8, 4.7 Hz, 2H), 2.88 (ddd, *J* = 17.5, 14.2, 5.5 Hz, 1H), 2.64 - 2.51 (m, 3H), 2.42 (dq, *J* = 12.5, 8.1, 6.3 Hz, 3H), 2.06 - 1.91 (m, 1H), 1.57 (s, 6H), 1.18 - 1.07 (m, 1H), 0.37 (t, *J* = 7.5 Hz, 4H). LC/MS (ESI+) calcd for C₄₂H₄₂N₄O₇ ([M+H]⁺) *m*/*z* 715, found 715.

### Example 117: Synthesis of 5-(((trans)-3-(4-(2-(4-((6-(cyclopropanecarbonyl) pyridin-2-yl)methoxy)phenyl)propan-2-yl)phenoxy)cyclobutyl)amino)-2-(2,6-dioxopiperidin-3-yl)isoindolin-1,3-dione

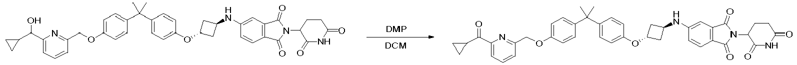

5-(((trans)-3-(4-(2-(4-((6-(cyclopropyl(hydroxyl)methyl)pyridin-2-yl)methoxy) phenyl)propan-2-yl)phenoxy)cyclobutyl)amino)-2-(2,6-dioxopiperidin-3-yl) isoindolin-1,3-dione (714 mg, 1 mmol) was dissolved in 30 mL of DCM, to which was added Dess-Martin periodinane (640 mg, 1.5 mmol) in an ice water bath in batches, and then the mixture was naturally warmed to room temperature and reacted for 16 h. The reaction solution was poured to saturated NaHSO₃ aqueous solution, and then filtered over diatomite pad. The filtrate was extracted with ethyl acetate. The organic phase was washed with saturated brine, dried with anhydrous Na₂SO₄, and concentrated. The residue was separated and purified by column chromatography to provide 356 mg of product, with a yield of 50%. LC/MS (ESI+) calcd for C₄₂H₄₀N₄O₇ ([M+H]⁺) *m*/*z* 713, found 713.

### Example 118: Synthesis of 2-(2,6-dioxopiperidin-3-yl)-5-(((trans)-3-(4-(2-(4-((2-(1-hydroxylethyl)pyrimidin-5-yl))oxy)phenyl)propan-2-yl)phenoxy)cyclobutyl) amino)isoindolin-1,3-dione

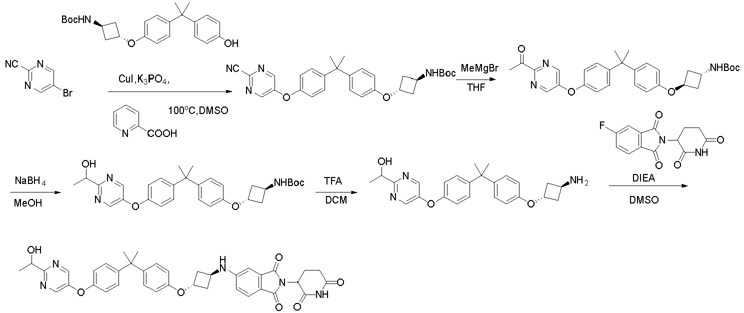

### Step 1: Synthesis of tert-butyl ((1r,3r)-3-(4-(2-(4-((2-cyanopyrimidin-5-yl)oxy)phenyl)propan-2-yl)phenoxy)cyclobutyl)carbamate

tert-butyl ((1r,3r)-3-(4-(2-(4-hydroxylphenyl)propan-2-phenoxy)cyclobutyl) carbamate (397 mg, 1 mmol) and 2-cyano-5-bromopyrimidine (183 mg, 1 mmol), CuI (26 mg, 0.2 mmol), dinicotinic acid (33 mg, 0.2 mmol), and potassium phosphate (424 mg, 2 mmol) were dissolved in DMSO (8 mL), and then the system was purged with nitrogen for three times. The reaction solution was heated to 90 °C and reacted for 5 h. The reaction solution was poured to water, and extracted. The organic phase was combined, washed with saturated brine, dried with anhydrous Na₂SO₄, followed by column chromatography, to provide 400 mg of product (yield 80%), LC/MS (ESI+) calcd for C₂₉H₃₂N₄O₄ ([M+H]⁺) *m*/*z* 501, found 501.

### Step 2: Synthesis of tert-butyl ((1r,3r)-3-(4-(2-(4-((2-acetylpyrimidin-5-yl)oxy)phenyl)propan-2-yl)phenoxy)cyclobutyl)carbamate

tert-butyl ((1r,3r)-3-(4-(2-(4-((2-cyanopyrimidin-5-yl)oxy)phenyl)propan-2-yl) phenoxy)cyclobutyl)carbamate (500 mg, 1 mmol) was dissolved in THF(10 mL), to which was added the solution of methylmagnesium bromide in THF (10 mL, 5 mmol) in an ice water bath under nitrogen protection, and then the mixture was allowed to react for 10 min in the ice water bath. The reaction solution was poured to 0.5N of cold dilute hydrochloric acid, and extracted. The organic phase was combined, washed with saturated brine, dried with anhydrous Na₂SO₄, followed by column chromatography, to provide 360 mg of product (yield 70%), LC/MS (ESI+) calcd for C₃₀H₃₅N₃O₅ ([M+H]⁺) *m*/*z* 518, found 518.

### Step 3: Synthesis of tert-butyl ((1r,3r)-3-(4-(2-(4-(2-(1-hydroxylethyl)pyrimidin-5-yl)oxy)phenyl)propan-2-yl)phenoxy)cyclobutyl)carbamate

tert-butyl ((1r,3r)-3-(4-(2-(4-((2-acetylpyrimidin-5-yl)oxy)phenyl)propan-2-yl)phenoxy)cyclobutyl)carbamate (517 mg, 1 mmol) was dissolved in 15 mL of methanol, to which was added sodium borohydride (114 mg, 3 mmol) in an ice water bath in batches. After that, the mixture was allowed to react for half an hour. The reaction solution was poured to water, and extracted. The organic phase was combined, washed with saturated brine, dried with anhydrous Na₂SO₄, followed by column chromatography, to provide 470 mg of product (yield 90%), LC/MS (ESI+) calcd for C₃₀H₃₇N₃O₅ ([M+H]⁺) *m*/*z* 520, found 520.

In following steps 4 and 5, the methods, similar to those of the last two steps Example 1, was used to synthesize the target compound, with a yield of 25%. ¹H NMR (400 MHz, Chloroform-d) *δ* 8.46 (s, 2H), 8.30 (s, 1H), 7.61 (d, *J* = 8.2 Hz, 1H), 7.25 (d, *J* = 2.2 Hz, 1H), 7.18 - 7.09 (m, 2H), 6.99 - 6.92 (m, 2H), 6.88 (d, *J* = 2.1 Hz, 1H), 6.77 - 6.65 (m, 3H), 5.02 - 4.82 (m, 3H), 4.21 (td, *J* = 7.7, 3.9 Hz, 1H), 2.93 - 2.65 (m, 5H), 2.40 (dt, *J* = 13.8, 5.9 Hz, 2H), 2.11 (td, *J* = 9.3, 8.6, 5.4 Hz, 1H), 1.67 (s, 6H), 1.57 (d, *J* = 6.6 Hz, 3H). LC/MS (ESI+) calcd for C₃₈H₃₇N₅O₇ ([M+H]⁺) *m*/*z* 676, found 676.

### Example 119: Synthesis of 5-(((trans)-3-(4-(2-(4-((2-acetylpyrimidin-5-yl)oxy)phenyl)propan-2-yl)phenoxy)cyclobutyl)amino)-2-(2,6-dioxopiperidin-3-yl) isoindolin-1,3-dione

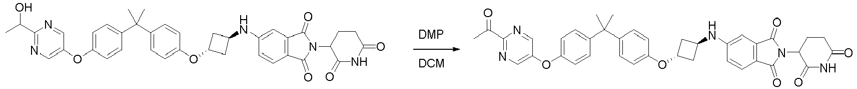

2-(2,6-dioxopiperidin-3-yl)-5-(((trans)-3-(4-(2-(4-((2-(1-hydroxylethyl) pyrimidin-5-yl))oxy)phenyl)propan-2-yl)phenoxy)cyclobutyl)amino) dihydroisoindole-1,3-dione (675 mg, 1 mmol) was dissolved in 30 mL of DCM, to which was added Dess-Martin periodinane (640 mg, 1.5 mmol) in an ice water bath in batches, and then the mixture was naturally warmed to room temperature and reacted for 16 h. The reaction solution was poured to saturated NaHSO₃ aqueous solution, and then filtered over diatomite pad. The filtrate was extracted with ethyl acetate. The organic phase was washed with saturated brine, dried with anhydrous Na₂SO₄, and concentrated. The residue was separated and purified by column chromatography to provide 510 mg of product, with a yield of 80%. ¹H NMR (400 MHz, Chloroform-d) *δ* 8.54 (s, 2H), 8.15 (s, 1H), 7.62 (d, *J* = 8.3 Hz, 1H), 7.33 - 7.28 (m, 2H), 7.20 - 7.08 (m, 2H), 7.05 - 6.97 (m, 2H), 6.90 (d, *J* = 2.1 Hz, 1H), 6.72 (dd, *J* = 8.8, 2.5 Hz, 3H), 4.99 - 4.82 (m, 2H), 4.22 (td, *J* = 7.8, 4.0 Hz, 1H), 2.93 - 2.66 (m, 8H), 2.42 (dt, *J* = 12.9, 6.2 Hz, 2H), 2.16 - 2.08 (m, 1H), 1.68 (s, 6H). LC/MS (ESI+) calcd for C₃₈H₃₅N₅O₇ ([M+H]⁺) *m*/*z* 674, found 674.

### Example 120: 3-(7-(((trans)-3-(4-(2-(4-((6-(5-methyl-1,3,4-methyl-1,3,4-oxadiazol-2-yl)pyridin-3-yl))oxy)phenyl)propan-2-yl)phenoxy)cyclobutyl)amino)-4-oxoquinazolin-3(4H)-yl)piperidin-2,6-dione

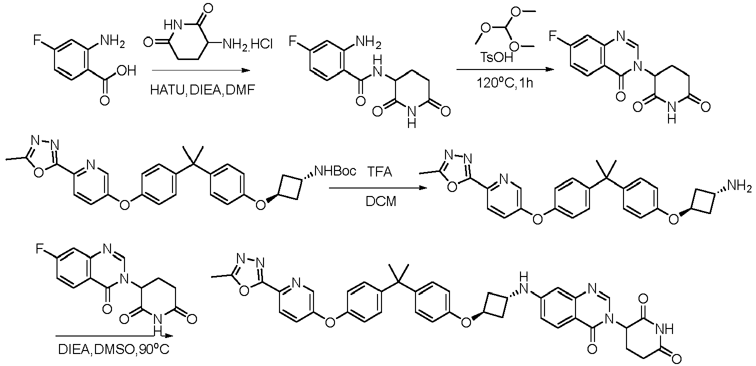

### Step 1: Synthesis of 2-amino-N-(2,6-dioxopiperidin-3-yl)-4-fluorobenzamide

2-amino-4-fluorobenzoic acid (155 mg, 1 mmol) and 3-aminopiperidin-2,6-dione hydrochloride (166 mg, 1 mmol) were dissolved in 6 mL of DMF, to wich were added DIEA (387 mg, 3 mmol) and HATU (380 mg, 1 mmol), and then the mixture was allowed to react for 2h under stirring at room temperature. The reaction system was poured to water and extracted. The organic phase was washed with saturated brine, dried with anhydrous Na₂SO₄, and concentrated. The residue was triturated to provide 160 mg of product, with a yield of 60%. LC/MS (ESI+) calcd for C₁₂H₁₂FN₃O₃ ([M+H]⁺) *m*/*z* 266, found 266.

### Step 2: Synthesis of 3-(7-fluoro-4-oxoquinazolin-3(4H)-yl)piperidin-2,6-dione

2-amino-N-(2,6-dioxopiperidin-3-yl)-4-fluorobenzamide (265 mg, 1 mmol) and TsOH (172 mg, 1 mmol) were dissolved in 5 mL of trimethyl orthoformate, and then the mixture was heated to 120 °C and reacted for 2h. After cooling, the reaction was triturated in EA and methanol, to provide 138 mg of product, with a yield of 50%. LC/MS (ESI+) calcd for C₁₃H₁₀FN₃O₃ ([M+H]⁺) *m*/*z* 276, found 276.

In following steps, the target compound was synthesized by a method similar to that of Example 116. ¹H NMR (400 MHz, DMSO-d₆) *δ* 11.06 (s, 1H), 8.52 (d, *J =* 2.8 Hz, 1H), 8.21 - 8.10 (m, 2H), 7.82 (d, *J* = 8.7 Hz, 1H), 7.51 (dd, *J* = 8.8, 2.9 Hz, 1H), 7.38 - 7.27 (m, 2H), 7.22 - 7.05 (m, 5H), 6.78 (t, *J* = 9.6 Hz, 3H), 6.44 (d, *J* = 2.2 Hz, 1H), 5.33 (s, 1H), 4.88 (h, *J* = 6.0, 5.5 Hz, 1H), 4.06 (dd, *J* = 26.5, 6.5 Hz, 1H), 2.81 (q, *J* = 14.3 Hz, 1H), 2.59 (s, 5H), 2.53 (d, *J* = 7.6 Hz, 2H), 2.45 (q, *J* = 5.7 Hz, 2H), 2.17 - 2.02 (m, 1H), 1.63 (s, 6H). LC/MS (ESI+) calcd for C₄₀H₃₇N₇O₆ ([M+H]⁺) *m*/*z* 712, found 712.

### Example 122: Synthesis of 5-(((trans)-3-(4-(4-((5-(2H-1,2,3-triazol-2-yl)pyrimidin-2-yl)oxy)phenoxy)phenoxy)cyclobutyl)amino)-2-(2,6-dioxopiperidin-3-yl) isoindolin-1,3-dione

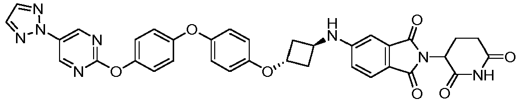

The target compound was synthesized by a method similar to that of Example 228. ¹H NMR (400 MHz, DMSO-d₆) *δ* 11.07 (s, 1H), 9.23 (s, 2H), 8.23 (s, 2H), 7.67 - 7.50 (m, 2H), 7.33 - 7.20 (m, 2H), 7.06 - 6.81 (m, 8H), 5.04 (dd, *J* = 13.0, 5.4 Hz, 1H), 4.96 - 4.82 (m, 1H), 4.17 (s, 1H), 2.93 - 2.80 (m, 1H), 2.57 (d, *J* = 17.3 Hz, 3H), 2.46 (d, *J* = 5.9 Hz, 2H), 1.99 (s, 2H). LC/MS (ESI+) calcd for C₃₅H₂₈N₈O₇ ([M+H]⁺) *m*/*z* 673, found 673.

### Example 123: Synthesis of 2-(2,6-dioxopiperidin-3-yl)-5-(((trans)-3-(4-(2-(4-((6-(1-hydroxylethyl)pyridin-3-yl))oxy)phenyl)propan-2-yl)phenoxy)cyclobutyl)amino) isoindolin-1,3-dione

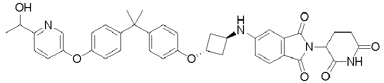

The target compound was synthesized by a method similar to that of Example 118, with a yield of 25%. ¹H NMR (400 MHz, Chloroform-d) *δ* 8.33 - 8.20 (m, 2H), 7.62 (d, *J* = 8.3 Hz, 1H), 7.41 (dd, *J* = 8.6, 2.7 Hz, 1H), 7.32 (d, *J* = 8.6 Hz, 1H), 7.25 - 7.20 (m, 2H), 7.18 -7.10 (m, 2H), 6.96 - 6.86 (m, 3H), 6.77 - 6.66 (m, 3H), 5.00 - 4.81 (m, 4H), 4.22 (s, 1H), 2.93 - 2.65 (m, 5H), 2.47 - 2.34 (m, 2H), 2.14 - 2.10 (m, 1H), 1.66 (s, 6H), 1.53 (d, *J* = 6.5 Hz, 3H). LC/MS (ESI+) calcd for C₃₉H₃₈N₄O₇ ([M+H]⁺) *m*/*z* 675, found 675.

### Example 124: Synthesis of 5-((((trans)-3-(4-(2-(4-((6-acetylpyridin-3-yl)oxy)phenyl)propan-2-yl)phenoxy)cyclobutyl)amino)-2-(2,6-dioxopiperidin-3-yl)isoindolin-1,3-dione

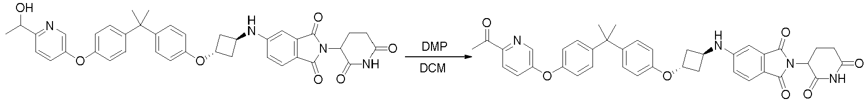

The target compound was synthesized by a method similar to that of Example 119, with a yield of 80%. LC/MS (ESI+) calcd for C₃₉H₃₆N₄O₇ ([M+H]⁺) *m*/*z* 673, found 673.

### Example 125: Synthesis of 2-(2,6-dioxopiperidin-3-yl)-5-(((trans)-3-(4-(2-(4-((6-(1-hydroxylethyl)pyridazine-3-yl))oxy)phenyl)propan-2-yl)phenoxy)cyclobutyl) amino)isoindolin-1,3-dione

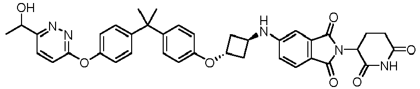

The target compound was synthesized by a method similar to that of Example 118, with a yield of 25%. ¹H NMR (400 MHz, Chloroform-d) *δ* 8.20 (s, 1H), 7.60 (t, *J* = 8.9 Hz, 2H), 7.22 (d, *J* = 8.7 Hz, 2H), 7.12 (dd, *J* = 28.8, 8.3 Hz, 4H), 6.93 - 6.85 (m, 1H), 6.70 (dd, *J* = 8.3, 4.7 Hz, 3H), 5.10 (d, *J* = 6.6 Hz, 1H), 4.99 - 4.80 (m, 2H), 4.21 (s, 1H), 2.99 (s, 2H), 2.91 - 2.75 (m, 2H), 2.69 (td, *J* = 9.4, 6.8, 3.8 Hz, 2H), 2.40 (p, *J* = 5.7 Hz, 2H), 2.14 - 2.09 (m, 1H), 1.67 (s, 6H), 1.56 (d, *J* = 6.3 Hz, 3H). LC/MS (ESI+) calcd for C₃₈H₃₇N₅O₇ ([M+H]⁺) m/z 676, found 676.

### Example 126: Synthesis of 5-(((trans)-3-(4-(2-(4-((6-acetylpyridazine-3-yl)oxy) phenyl)propan-2-yl)phenoxy)cyclobutyl)amino)-2-(2,6-dioxopiperidin-3-yl) isoindolin-1,3-dione

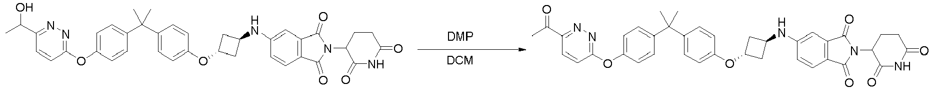

The target compound was synthesized by a method similar to that of Example 119, with a yield of 80%. ¹H NMR (400 MHz, DMSO-d6) *δ* 11.06 (s, 1H), 8.16 (d, *J* = 9.2 Hz, 1H), 7.56 (td, *J* = 10.9, 6.9 Hz, 3H), 7.37 - 7.27 (m, 2H), 7.22 - 7.13 (m, 4H), 6.90 - 6.71 (m, 4H), 5.03 (dd, *J* = 12.9, 5.4 Hz, 1H), 4.88 (p, *J* = 6.1 Hz, 1H), 4.15 (d, *J* = 5.0 Hz, 1H), 2.89 (s, 2H), 2.73 (d, *J* = 0.6 Hz, 2H), 2.68 (s, 3H), 2.57 - 2.52 (m, 2H), 2.45 (dd, *J* = 7.3, 4.7 Hz, 1H), 2.04 - 1.95 (m, 1H), 1.65 (s, 6H). LC/MS (ESI+) calcd for C₃₈H₃₅N₅O₇ ([M+H]+) *m*/*z* 674, found 674.

### Example 127: Synthesis of 2-(2,6-dioxopiperidin-3-yl)-5-(((cis)-3-(4-(2-(4-((2-(1-hydroxylethyl)pyrimidin-5-yl))oxy)phenyl)propan-2-yl)phenoxy)cyclobutyl) amino)isoindolin-1,3-dione

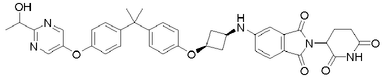

The target compound was synthesized by a method similar to that of Example 118, with a yield of 25%. ¹H NMR (400 MHz, DMSO-d₆) *δ* 11.08 (s, 1H), 8.58 (s, 2H), 7.59 (d, *J* = 8.3 Hz, 1H), 7.46 (d, *J* = 6.4 Hz, 1H), 7.32 - 7.20 (m, 2H), 7.19 - 7.09 (m, 2H), 7.07 - 6.97 (m, 2H), 6.90 (d, *J* = 2.0 Hz, 1H), 6.86 - 6.73 (m, 3H), 5.24 (d, *J* = 5.5 Hz, 1H), 5.04 (dd, *J* = 12.9, 5.4 Hz, 1H), 4.86 - 4.73 (m, 1H), 4.57 - 4.41 (m, 1H), 3.86 - 3.67 (m, 1H), 3.00 (s, 3H), 2.94 - 2.81 (m, 1H), 2.63 - 2.52 (m, 1H), 2.08 - 1.87 (m, 3H), 1.61 (s, 6H), 1.41 (d, *J* = 6.6 Hz, 3H). LC/MS (ESI+) calcd for C₃₈H₃₇N₅O₇([M+H]⁺) *m*/*z* 676, found 676.

### Example 128: Synthesis of 5-(((cis)-3-(4-(2-(4-((2-acetylpyrimidin-5-yl)oxy)phenyl) propan-2-yl)phenoxy)cyclobutyl)amino)-2- (2,6-dioxopiperidin-3-yl)isoindolin-1,3-dione

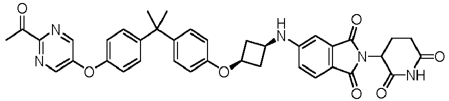

The target compound was synthesized by a method similar to that of Example 119, with a yield of 80%. ¹H NMR (400 MHz, DMSO-d₆) *δ* 11.08 (s, 1H), 8.70 (s, 2H), 7.59 (d, *J* = 8.3 Hz, 1H), 7.46 (d, *J* = 6.4 Hz, 1H), 7.37 - 7.25 (m, 2H), 7.22 - 7.08 (m, 4H), 6.95 - 6.73 (m, 4H), 5.04 (dd, *J* = 12.9, 5.4 Hz, 1H), 4.50 (t, *J* = 6.9 Hz, 1H), 3.78 (d, *J* = 7.3 Hz, 1H), 3.13 - 2.98 (m, 2H), 2.95 - 2.77 (m, 1H), 2.69 - 2.51 (m, 5H), 2.04 - 1.88 (m, 3H), 1.63 (s, 6H). LC/MS (ESI+) calcd for C₃₈H₃₅N₅O₇ ([M+H]⁺) *m*/*z* 674, found 674.

### Example 129: 2-(2,6-dioxopiperidin-3-yl)-5-((cis)-3-(4-(2-(4-((5-(1-hydroxylethyl) pyrazin-2-yl)oxy)phenyl)propan-2-yl)phenoxy)cyclobutyl)amino)isoindolin-1,3-dione

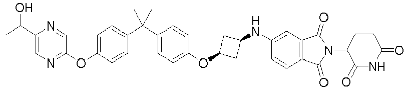

The target compound was synthesized by a method similar to that of Example 118, with a yield of 25%.¹H NMR (400 MHz, DMSO-d₆) δ 11.08 (s, 1H), 8.41 (d, *J* = 1.4 Hz, 1H), 8.29 - 8.19 (m, 1H), 7.59 (d, *J* = 8.3 Hz, 1H), 7.46 (d, *J* = 6.5 Hz, 1H), 7.33 - 7.22 (m, 2H), 7.21 - 7.12 (m, 2H), 7.12 - 7.04 (m, 2H), 6.95 - 6.74 (m, 4H), 5.49 (d, *J* = 4.7 Hz, 1H), 5.04 (dd, *J* = 12.9, 5.4 Hz, 1H), 4.79 (dd, *J* = 6.5, 4.7 Hz, 1H), 4.50 (t, *J* = 6.9 Hz, 1H), 3.78 (d, *J* = 7.2 Hz, 1H), 3.14 - 2.97 (m, 2H), 2.95 - 2.81 (m, 1H), 2.63 - 2.52 (m, 1H), 2.06 - 1.88 (m, 3H), 1.63 (s, 6H), 1.44 - 1.33 (m, 3H). LC/MS (ESI+) calcd for C₃₈H₃₇N₅O₇ ([M+H]⁺) m/z 676, found 676_{∘}

### Example 130: Synthesis of 5-((cis)-3-(4-(2-(4-((5-acetylpyrazin-2-yl)oxy)phenyl) propan-2-yl)phenoxy)cyclobutyl)amino)-2-(2,6-dioxopiperidin-3-yl)isoindolin-1,3-dione

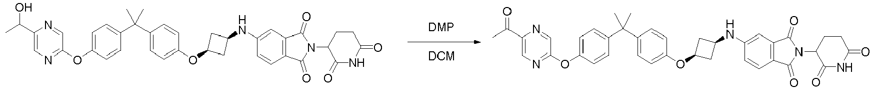

The target compound was synthesized by a method similar to that of Example 119, with a yield of 80%. ¹H NMR (400 MHz, DMSO-*d*₆) *δ* 11.08 (s, 1H), 8.65 (d, *J* = 25.9 Hz, 2H), 7.59 (d, *J* = 8.2 Hz, 1H), 7.46 (d, *J* = 6.4 Hz, 1H), 7.37 - 7.05 (m, 6H), 6.98 - 6.72 (m, 4H), 5.04 (dd, *J* = 13.2, 5.2 Hz, 1H), 4.60 - 4.39 (m, 1H), 3.78 (s, 1H), 3.06 (s, 2H), 2.88 (t, *J* = 14.5 Hz, 1H), 2.57 (d, *J* = 18.9 Hz, 4H), 1.96 (d, *J* = 16.5 Hz, 4H), 1.64 (s, 6H). LC/MS (ESI+) calcd for C₃₈H₃₅N₅O₇ ([M+H]⁺) *m*/*z* 674, found 674.

### Example 131: Synthesis of 2-(2,6-dioxopiperidin-3-yl)-5-((trans)-3-(4-(3-(4-((2-(1-hydroxylethyl)pyrimidin-5-yl)oxy)phenyl)pentan-3-yl)phenoxy)cyclobutyl)amino) isoindolin-1,3-dione

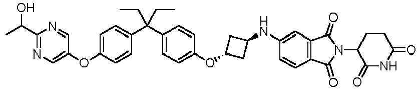

The target compound was synthesized by a method similar to that of Example 118, with a yield of 25%. ¹H NMR (400 MHz, DMSO-d₆) *δ* 11.08 (s, 1H), 8.56 (s, 2H), 7.56 (dd, *J=* 20.9, 6.8 Hz, 2H), 7.18 (d, *J=* 8.7 Hz, 2H), 7.09 - 6.96 (m, 4H), 6.90 - 6.71 (m, 4H), 5.24 (d, *J=* 5.5 Hz, 1H), 5.04 (dd, *J* = 12.9, 5.4 Hz, 1H), 4.93 - 4.71 (m, 2H), 4.14 (d, *J* = 5.7 Hz, 1H), 2.87 (td, *J* = 17.2, 15.4, 5.3 Hz, 1H), 2.63 - 2.51 (m, 3H), 2.41 (t, *J* = 6.1 Hz, 3H), 2.01 (dq, *J* = 13.7, 6.0, 5.4 Hz, 5H), 1.40 (d, *J* = 6.5 Hz, 3H), 0.56 (t, *J* = 7.1 Hz, 6H). LC/MS (ESI+) calcd for C₄₀H₄₁N₅O₇ ([M+H]⁺) *m*/*z* 704, found 704.

### Example 132: 5-((trans)-3-(4-(3-(4-((2-acetylpyrimidin-5-yl)oxy)phenyl)pentan-3-yl)phenoxy)cyclobutyl)amino)-2-(2,6-dioxopyridin-3-yl)isoindolin-1,3-dione

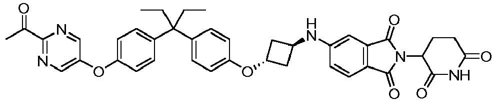

The target compound was synthesized by a method similar to that of Example 119, with a yield of 80%. ¹H NMR (400 MHz, DMSO-d₆) *δ* 11.03 (s, 1H), 8.68 (s, 2H), 7.56 (dd, *J=* 20.5, 6.8 Hz, 2H), 7.33 - 7.01 (m, 6H), 6.95 - 6.65 (m, 4H), 5.03 (dd, *J* = 13.1, 5.5 Hz, 1H), 4.94 - 4.83 (m, 1H), 4.14 (s, 1H), 2.97 - 2.79 (m, 1H), 2.64 (s, 3H), 2.43 (q, *J* = 7.3, 6.2 Hz, 2H), 2.03 (td, *J* = 14.9, 14.0, 7.1 Hz, 5H), 1.24 (d, *J* = 9.3 Hz, 4H), 0.57 (t, *J* = 7.2 Hz, 6H). LC/MS (ESI+) calcd for C₄₀H₃₉N₅O₇ ([M+H]⁺) *m*/*z* 702, found 702.

### Example 133: 2-(2,6-dioxopiperidin-3-yl)-5-((cis)-3-(4-(3-(4-((2-(1-hydroxylethyl) pyrimidin-5-yl)oxy)phenyl)pentan-3-yl)phenoxy)cyclobutyl)amino)isoindolin-1,3-dione

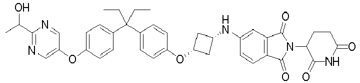

The target compound was synthesized by a method similar to that of Example 118, with a yield of 25%. ¹H NMR (400 MHz, DMSO-d₆) *δ* 11.08 (s, 1H), 8.56 (s, 2H), 7.59 (d, *J* = 8.3 Hz, 1H), 7.45 (d, *J* = 6.4 Hz, 1H), 7.24 - 7.15 (m, 2H), 7.10 - 6.99 (m, 4H), 6.90 (d, *J* = 2.0 Hz, 1H), 6.85 - 6.75 (m, 3H), 5.24 (d, *J* = 5.5 Hz, 1H), 5.04 (dd, *J* = 12.9, 5.4 Hz, 1H), 4.87 - 4.72 (m, 1H), 4.50 (p, *J* = 7.0 Hz, 1H), 3.78 (p, *J* = 7.5 Hz, 1H), 3.13 - 2.96 (m, 2H), 2.87 (ddd, *J=* 17.2, 14.0, 5.4 Hz, 1H), 2.63 - 2.51 (m, 2H), 2.11 - 1.85 (m, 7H), 1.40 (d, *J* = 6.5 Hz, 3H), 0.56 (t, *J* = 7.2 Hz, 6H). LC/MS (ESI+) calcd for C₄₀H₄₁N₅O₇([M+H]⁺) *m*/*z* 704, found 704.

### Example 134: 2-(2,6-dioxopiperidin-3-yl)-5-((trans)-3-(4-(1-(4-((2-(1-hydroxylethyl)pyrimidin-5-yl)oxy)phenyl)cyclopentyl)phenoxy)cyclobutyl)amino) isoindolin-1,3-dione

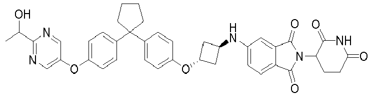

The target compound was synthesized by a method similar to that of Example 118, with a yield of 25%. ¹H NMR (400 MHz, DMSO-d₆) *δ* 11.08 (s, 1H), 8.56 (s, 2H), 7.56 (dd, *J=* 21.7, 6.9 Hz, 2H), 7.39 - 7.28 (m, 2H), 7.27 - 7.17 (m, 2H), 7.04 - 6.93 (m, 2H), 6.90 - 6.67 (m, 4H), 5.24 (d, *J* = 5.5 Hz, 1H), 5.04 (dd, *J* = 12.9, 5.4 Hz, 1H), 4.90 - 4.72 (m, 2H), 4.13 (d, *J* = 5.1 Hz, 1H), 2.88 (ddd, *J* = 19.5, 15.3, 5.5 Hz, 1H), 2.57 (d, *J* = 17.6 Hz, 3H), 2.42 (q, *J* = 6.1 Hz, 2H), 2.23 (s, 4H), 1.99 (q, *J =* 4.9, 4.2 Hz, 1H), 1.60 (t, *J* = 3.8 Hz, 4H), 1.40 (d, *J* = 6.6 Hz, 3H). LC/MS (ESI+) calcd for C₄₀H₃₉N₅O₇ ([M+H]⁺) *m*/*z* 702, found 702.

### Example 135: 5-((trans)-3-(4-(1-(4-((2-acetylpyrimidin-5-yl)oxy)phenyl) cyclopentyl)phenoxy)cyclobutyl)amino)-2-(2,6-dioxopiperidin-3-yl)isoindolin-1,3-dione

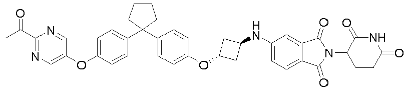

The target compound was synthesized by a method similar to that of Example 119, with a yield of 80%. ¹H NMR (400 MHz, DMSO-d₆) *δ* 11.08 (s, 1H), 8.68 (s, 2H), 7.56 (dd, *J* = 19.9, 6.8 Hz, 2H), 7.45 - 7.33 (m, 2H), 7.23 (d, *J* = 8.7 Hz, 2H), 7.16 - 7.06 (m, 2H), 6.91 - 6.67 (m, 4H), 5.04 (dd, *J* = 12.9, 5.4 Hz, 1H), 4.84 (dd, *J* = 8.9, 3.8 Hz, 1H), 4.12 (s, 1H), 2.96 - 2.78 (m, 1H), 2.64 (s, 3H), 2.59 (d, *J* = 3.5 Hz, 3H), 2.42 (q, *J* = 7.5, 7.0 Hz, 2H), 2.35 - 2.14 (m, 4H), 1.99 (q, *J* = 5.2, 4.1 Hz, 2H), 1.62 (d, *J* = 6.1 Hz, 4H). LC/MS (ESI+) calcd for C₄₀H₃₇N₅O₇ ([M+H]⁺) m/z 700, found 700.

### Example 136: 5-((4-((8-(4-(2-(4-((2-acetylpyrimidin-4-yl)methoxy)phenyl)propan-2-yl)phenoxy)octyl)oxy)butyl)amino)-2-(2,6-dioxopiperidin-3-yl)isoindolin-1,3-dione

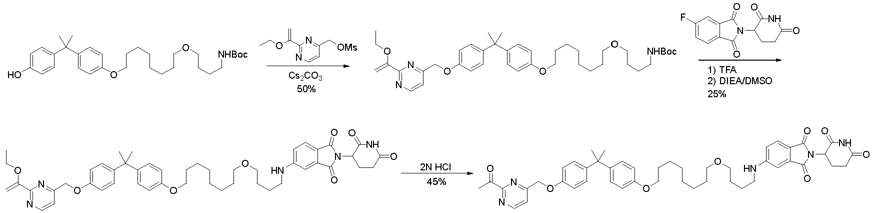

### Step 1: Synthesis of tert-butyl (4-((8-(4-(2-(2-(1-ethoxyvinyl)pyrimidin-4-yl)methoxy) phenyl)propan-2-yl)phenoxy)octyl)oxy)butanecarbamate

tert-butyl (4-((8-(4-(4-hydroxylphenyl)propan-2-phenoxy)octyl)oxy)carbamate (264 mg, 0.50 mmol) was dissolved in 5 mL of DMF, to which were added cesium carbonate (325 mg, 1.0 mmol) and (2-(1-ethoxyvinyl)pyrimidin-4-yl)methanesulfonate (129 mg, 0.5 mmol), and then the mixture was allowed to react at room temperature for 2h. To the reaction solution, were added 15 mL of ethyl acetate and 12 mL of water, and then the resultant solution was stirred and extracted. The water layer was re-extracted once with 10 mL of ethyl acetate. The organic phase was combined, successively washed with water and saturated brine, dried with anhydrous Na₂SO₄, and concentrated to dry under reduced pressure. The residue was separated and purified by TLC, to provide the product tert-butyl (4-((8-(4-(2-(2-(1-ethoxyvinyl)pyrimidin-4-yl)methoxy)phenyl)propan-2-yl)phenoxy)octyl)oxy)butanecarbamate (172 mg), with a yield of 50%. LC/MS (ESI+) calcd for C₄₁H₅₉N₃O₆ ([M+H]⁺) *m*/*z* 690.4, found 690.4.

### Step 2: Synthesis of 2-(2,6-dioxopiperidin-3-yl)-5-(4-((8-(4-(2-(2-(1-ethoxyvinyl) pyrimidin-4-yl)methoxy)phenyl)propan-2-yl)phenoxy)octyl)oxy)butyl)amino) isoindolin-1,3-dione

tert-butyl (4-((8-(4-(2-(2-(1-ethoxyvinyl)pyrimidin-4-yl)methoxy)phenyl)propan-2-yl)phenoxy)octyl)oxy)butanecarbamate (170 mg, 0.24 mmol) was dissolved in 4 mL of dichloromethane, to which was added 1 mL of trifluoroacetic acid dropwise in an ice water bath, and then the mixture was allowed to react for 1h in the ice water bath. The pH of the reaction solution was adjusted to alkaline with saturated Na₂CO₃ aqueous solution, and extracted with dichloromethane/methanol (10:1). The organic phase was dried over anhydrous MgSO₄, and concentrated to dry under reduced pressure, to obtain 130 mg of solid product. 65 mg of the product was dissolved in 5 mL of DMSO, to which was added 5 drops of N,N-diisopropylethylamine, 2-(2,6-dioxopiperidin-3-yl)-5-fluoroisoindolin-1,3-dione (48 mg, 0.16 mmol), and then the mixture was allowed to react overnight at 90 °C. The reaction solution was cooled, extracted with dichloromethane/water, dried, and concentrated. The residue was separated and purified by TLC, to provide the product 23 mg of 2-(2,6-dioxopiperidin-3-yl)-5-(4-((8-(4-(2-(2-(1-ethoxyvinyl)pyrimidin-4-yl)methoxy)phenyl)propan-2-yl)phenoxy)octyl)oxy)butyl) amino) soindolin-1,3-dione, with a yield of 25%. LC/MS (ESI+) calcd for C₄₉H₅₉N₅O₈ ([M+H]⁺) *m*/*z* 845.4, found 845.4.

### Step 3: Synthesis of 5-((4-((8-(4-(2-(4-((2-acetylpyrimidin-4-yl)methoxy)phenyl) propan-2-yl)phenoxy)octyl)oxy)butyl)amino)-2-(2,6-dioxopiperidin-3-yl)isoindolin-1,3-dione

2-(2,6-dioxopiperidin-3-yl)-5-(4-((8-(4-(2-(2-(1-ethoxyvinyl)pyrimidin-4-yl)methoxy)phenyl)propan-2-yl)phenoxy)octyl)oxy)butyl)amino)isoindolin-1,3-dione (23mg, 0.027 mmol) was dissolved in 4 mL of acetone, to which was added 2 mL of 2N HCl aqueous solution, and then the mixture was allowed to react at room temperature for 1.5h. To the reaction solution, were added 15 mL of ethyl acetate and 12 mL of water, and then the resultant solution was stirred and extracted. The water layer was re-extracted once with 10 mL of ethyl acetate. The organic phase was combined, successively washed with water and saturated brine, dried with anhydrous Na₂SO₄, and concentrated to dry under reduced pressure. The residue was separated and purified by TLC, to provide the product 5-((4-((8-(4-(2-(4-((2-acetylpyrimidin-4-yl)methoxy)phenyl)propan-2-yl)phenoxy)octyl)oxy)butyl)amino)-2-(2,6-dioxopiperidin-3-yl)isoindolin-1,3-dione (10 mg), with a yield of 45%. LC/MS (ESI+) calcd for C₄₇H₅₅N₅O₈ ([M+H]⁺) *m*/*z* 818.4, found 818.4. ¹H NMR (400 MHz, Chloroform-*d*) *δ* 8.92 (d, *J* = 5.0 Hz, 1H), 8.00 (s, 1H), 7.74 (d, *J* = 4.8 Hz, 1H), 7.61 (d, *J* = 8.1 Hz, 1H), 7.17 (d, *J* = 8.6 Hz, 2H), 7.14 - 7.09 (m, 2H), 6.99 (s, 1H), 6.86 (d, *J* = 8.5 Hz, 2H), 6.82 - 6.74 (m, 3H), 5.25 (s, 2H), 4.92 (dd, *J* = 12.0, 5.2 Hz, 1H), 3.91 (t, *J* = 6.5 Hz, 2H), 3.45 (dt, *J* = 13.3, 6.2 Hz, 4H), 3.25 (t, *J* = 6.5 Hz, 2H), 2.80 (s, 5H), 2.13 (d, *J* = 5.9 Hz, 2H), 2.01 (d, *J* = 4.6 Hz, 2H), 1.74 (dd, *J* = 13.7, 6.4 Hz, 6H), 1.63 (s, 6H), 1.46 - 1.41 (m, 2H), 1.26 (s, 6H).

### Example 137: 5-((4-(2-(4-(3-((2-(2,6-dioxopiperidin-3-yl)-1,3-dioxoisoindolin-5-yl)amino)propoxy)phenyl)propan-2-yl)phenoxy)methyl)-N-methyl-1,2,4-oxadiazol-3-carbonylaminomethane

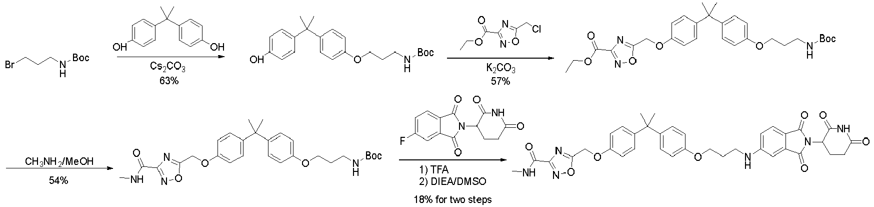

### Step 1: Synthesis of tert-butyl (3-(4-(2-(4-hydroxylphenyl)propan-2-yl)phenoxy) propyl)carbamate

Bisphenol A (17.1 g, 75 mmol) was dissolved in 100 mL of DMF, to which were added anhydrous cesium carbonate (24.4 g, 75 mmol) under stirring. N-Boc-3-aminopropyl bromide (11.9 g, 50 mmol) was dissolved in 30 mL of DMF, and then added to the reaction system dropwise. After that, the reaction solution was allowed to react at 60 °C for 5h, and then cooled to room temperature. To the reaction solution, were added 150 mL of ethyl acetate and 120 mL of water, and then the resultant solution was stirred and extracted. The water layer was re-extracted once with 100 mL of ethyl acetate. The organic phase was combined, successively washed with water and saturated brine, dried with anhydrous Na₂SO₄, and concentrated to dry under reduced pressure. The residue was separated and purified by column chromatography, to provide the product tert-butyl (3-(4-(2-(4-hydroxylphenyl)propan-2-yl)phenoxy)propyl) carbamate (12.2 g), with a yield of 63%. LC/MS (ESI+) calcd for C₂₃H₃₁NO₄ ([M+H]⁺) m/z 386.2, found 386.2_{∘}

### Step 2: Synthesis of ethyl 5-((4-(2-(4-(3-((tert-butoxycarbonyl)amino)propoxy)phenyl) propan-2-yl)phenoxy)methyl)-1,2,4-oxadiazol-3-carboxylate

tert-butyl (3-(4-(2-(4-hydroxylphenyl)propan-2-yl)phenoxy)propyl)carbamate (1930 mg, 5.0 mmol) was dissolved in 15 mL of DMF, to which were successively added anhydrous K₂CO₃ (1.38 g, 10 mmol) and ethyl 5-(chloromethyl)-1,2,4-oxadiazol-3-carboxylate (953 mg, 5.0 mmol), and then the mixture was allowed to react overnight at room temperature under stirring. To the reaction solution, were added 35 mL of ethyl acetate and 30 mL of water, and then the resultant solution was extracted. The water layer was re-extracted once with 25 mL of ethyl acetate. The organic layers were combined, successively washed with water and saturated brine, dried with anhydrous Na₂SO₄, and concentrated to dry under reduced pressure. The residue was separated and purified by column chromatography, to provide the product ethyl 5-((4-(2-(4-(3-((tert-butoxycarbonyl)amino)propoxy)phenyl)propan-2-yl)phenoxy)methyl)-1,2,4-oxadiazol-3-carboxylate (1.55 g), with a yield of 57%. LC/MS (ESI+) calcd for C₂₉H₃₇N₃O₇ ([M+H]⁺) *m*/*z* 540.2, found 540.2.

### Step 3: Synthesis of tert-butyl (3-(4-(2-(4-((3-(methylcarbamoyl)-1,2,4-oxadiazol-5-yl)methoxy)phenyl)propan-2-yl)phenoxy)propyl)carbamate

Ethyl 5-((4-(2-(4-(3-((tert-butoxycarbonyl)amino)propoxy)phenyl)propan-2-yl)phenoxy)methyl)-1,2,4-oxadiazol-3-carboxylate (225 mg, 0.42 mmol) was dissolved in 2 mL of methanol, to which was added 1 mL of 7 mmol/L methylamine ethanolic solution, and then the mixture was allowed to react in a sealed tube for 3h at 100 °C. The reaction solution was cooled to room temperature, followed by separation and purification by TLC, to provide tert-butyl (3-(4-(2-(4-((3-(methylcarbamoyl)-1,2,4-oxadiazol-5-yl)methoxy)phenyl)propan-2-yl)phenoxy)propyl)carbamate (120 mg), with a yield of 54%. LC/MS (ESI+) calcd for C₂₈H₃₆N₄O₆ ([M+H]⁺) *m*/*z* 525.3, found 525.3.

### Step 4: Synthesis of 5-((4-(2-(4-(3-((2-(2,6-dioxopiperidin-3-yl)-1,3-dioxoisoindolin-5-yl)amino)propoxy)phenyl)propan-2-yl)phenoxy)methyl)-N-methyl-1,2,4-oxadiazol-3-carbonylaminomethane

tert-butyl (3-(4-(2-(4-((3-(methylcarbamoyl)-1,2,4-oxadiazol-5-yl)methoxy) phenyl) propan-2-yl)phenoxy)propyl)carbamate (120 mg, 0.24 mmol) was dissolved in 4 mL of dichloromethane, to which was added 1 mL of trifluoroacetic acid dropwise in an ice water bath, and then the mixture was allowed to react at room temperature for 1h. The pH of the reaction solution was adjusted to alkaline with saturated Na₂CO₃ aqueous solution, and extracted with a mixed solution of dichloromethane/methanol (10:1). The organic phase was dried over anhydrous MgSO4, and concentrated to dry under reduced pressure. The residue was dissolved in 5 mL of DMSO, to which were added 5 drops of N,N-diisopropylethylamine and 2-(2,6-dioxopiperidin-3-yl)-5-fluoroisoindolin-1,3-dione (70 mg, 0.25 mmol), and then the mixture was allowed to react overnight at 95 °C. The reaction solution was cooled, extracted with dichloromethane/water, dried, and concentrated to dry. The residue was separated and purified by TLC, to provide the product 5-((4-(2-(4-(3-((2-(2,6-dioxopiperidin-3-yl)-1,3-dioxoisoindolin-5-yl)amino)propoxy)phenyl)propan-2-yl)phenoxy)methyl)-N-methyl-1,2,4-oxadiazol-3-carbonylaminomethane (29 mg), with a yield of 18%. LC/MS (ESI+) calcd for C₃₆H₃₆N₆O₈ ([M+H]⁺) *m*/*z* 681.3, found 681.3; ¹H NMR (400 MHz, Chloroform-*d*) *δ* 8.13 (s, 1H), 7.59 (d, *J* = 8.3 Hz, 1H), 7.20 - 7.10 (m, 4H), 7.06 (s, 1H), 6.98 (d, *J* = 2.1 Hz, 1H), 6.90 - 6.85 (m, 2H), 6.84 - 6.79 (m, 2H), 6.75 (dd, *J* = 8.3, 2.2 Hz, 1H), 5.31 (s, 2H), 5.01 (s, 1H), 4.93 (dd, *J* = 12.1, 5.2 Hz, 1H), 4.09 (t, *J* = 5.5 Hz, 2H), 3.47 (t, *J* = 6.5 Hz, 2H), 3.04 (d, *J* = 5.1 Hz, 3H), 2.93-2.68 (m, 4H), 2.15 - 2.11 (m, 2H), 1.64 (s, 6H).

Example 138: 5-((4-(2-(4-((8-(4-((2-(2,6-dioxopiperidin-3-yl)-1,3-dioxoisoindolin-5- yl)amino)butoxy)octoxy)phenyl)propan-2-yl)phenoxy)methyl)-1,2,4-oxadiazol-3-

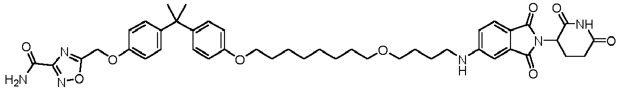 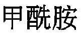

The target compound was synthesized by a method similar to that of Example 137. LC/MS (ESI+) calcd for C₄₄H₅₂N₆O₉([M+H]⁺) *m*/*z* 809.4, found 809.4; ¹H NMR (400 MHz, Chloroform-*d*) δ 8.07 (s, 1H), 7.61 (d, *J* = 8.0 Hz, 1H), 7.17 (d, *J* = 8.5 Hz, 2H), 7.11 (d, *J* = 8.6 Hz, 2H), 6.99 (d, *J* = 4.9 Hz, 1H), 6.87 (d, *J* = 8.6 Hz, 2H), 6.81 - 6.71 (m, 3H), 5.96 (s, 1H), 5.33 (s, 2H), 4.96 - 4.90 (m, 1H), 3.91 (t, *J* = 6.5 Hz, 2H), 3.45 (dt, *J* = 13.1, 6.1 Hz, 4H), 3.30 - 3.22 (m, 2H), 2.94 - 2.68 (m, 4H), 1.78 - 1.71 (m, 6H), 1.63 (s, 6H), 1.59 (d, *J* = 7.9 Hz, 2H), 1.46 - 1.41 (m, 2H), 1.26 (t, *J* = 4.7 Hz, 6H).

### Example 139: 5-((3-(4-(2-(4-((3-(azetidin-1-carbonyl)-1,2,4-oxadiazol-5-yl)methoxy)phenyl)propan-2-yl)phenoxy)propyl)amino)-2-(2,6-dioxopiperidin-3-yl)isoindolin-1,3-dione

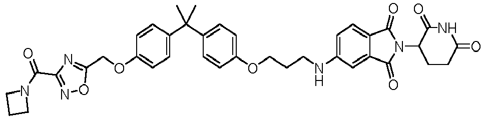

The target compound was synthesized by a method similar to that of Example 137. LC/MS (ESI+) calcd for C₃₈H₃₈N₆O₈ ([M+H]⁺) *m*/*z* 706.3, found 706.3. ¹H NMR (400 MHz, Chloroform-*d*) *δ* 8.14 (s, 1H), 7.59 (d, *J* = 8.3 Hz, 1H), 7.19 - 7.10 (m, 4H), 6.98 (d, *J* = 2.2 Hz, 1H), 6.91 - 6.85 (m, 2H), 6.84 - 6.78 (m, 2H), 6.74 (dd, *J=* 8.3, 2.2 Hz, 1H), 5.32 (s, 2H), 5.02 - 4.89 (m, 2H), 4.60 - 4.53 (m, 2H), 4.29 - 4.23 (m, 2H), 4.09 (t, *J* = 5.5 Hz, 2H), 3.46 (q, *J* = 7.2, 5.9 Hz, 2H), 2.93 - 2.69 (m, 3H), 2.46 - 2.35 (m, 2H), 2.18 - 2.07 (m, 3H), 1.63 (s, 6H).

### Example 140: 2-(2,6-dioxopiperidin-3-yl)-5-((3-(4-(2-(4-((3-(pyrrolidin-1-carbonyl)-1,2,4-oxadiazol-5-yl)methoxy)phenyl)propan-2-yl)phenoxy)propyl) amino)isoindolin-1,3-dione

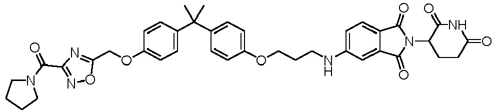

The target compound was synthesized by a method similar to that of Example 137. LC/MS (ESI+) calcd for C₃₉H₄₀N₆O₈ ([M+H]⁺) m/z 721.3, found 721.3. ¹H NMR (400 MHz, Chloroform-d) δ 8.14 (s, 1H), 7.59 (d, *J* = 8.3 Hz, 1H), 7.19 - 7.10 (m, 4H), 6.98 (d, *J* = 2.2 Hz, 1H), 6.92 - 6.87 (m, 2H), 6.84 - 6.79 (m, 2H), 6.74 (dd, *J* = 8.3, 2.2 Hz, 1H), 5.33 (s, 2H), 4.97 - 4.89 (m, 1H), 4.41 (s, 1H), 4.09 (t, *J* = 5.5 Hz, 2H), 3.77 (t, *J* = 6.5 Hz, 2H), 3.69 (t, *J* = 6.6 Hz, 2H), 3.51 - 3.42 (m, 2H), 2.93 - 2.69 (m, 3H), 2.17 - 2.08 (m, 3H), 1.98 (dtd, *J* = 9.6, 7.0, 5.2 Hz, 4H), 1.64 (s, 6H).

### Example 141: 2-(2,6-dioxopiperidin-3-yl)-5-(3-(4-(2-(4-((3-(4-methylpiperazin-1-carbonyl)-1,2,4-oxadiazol-5-yl)methoxy)phenyl)propan-2-yl)phenoxy)propyl) amino)isoindolin-1,3-dione

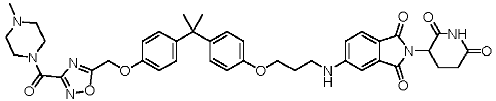

The target compound was synthesized by a method similar to that of Example 137. LC/MS (ESI+) calcd for C₄₀H₄₃N₇O₈ ([M+H]⁺) *m*/*z* 750.3, found 750.3. ¹H NMR (400 MHz, Chloroform-*d*) *δ* 8.53 (s, 1H), 7.59 (d, *J* = 8.3 Hz, 1H), 7.15 (dd, *J* = 8.8, 6.9 Hz, 4H), 6.98 (d, *J* = 2.2 Hz, 1H), 6.92 - 6.85 (m, 2H), 6.82 (d, *J* = 8.6 Hz, 2H), 6.74 (dd, *J* = 8.3, 2.2 Hz, 1H), 5.33 (s, 2H), 4.99 (s, 1H), 4.92 (dd, *J=* 12.1, 5.2 Hz, 1H), 4.09 (t, *J* = 5.5 Hz, 2H), 3.88 (s, 2H), 3.72 (s, 2H), 3.46 (q, *J* = 6.3 Hz, 2H), 2.91 - 2.72 (m, 3H), 2.54 (s, 3H), 2.39 (s, 3H), 2.12 (dt, *J* = 10.1, 4.8 Hz, 4H), 1.64 (s, 6H).

### Example 142: 2-(2,6-dioxopiperidin-3-yl)-5-((3-(4-(2-(4-((3-(morpholine-4-carbonyl)-1,2,4-oxadiazol-5-yl)methoxy)phenyl)propan-2-yl)phenoxy)propyl) amino)isoindolin-1,3-dione

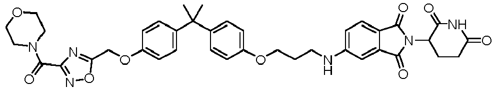

The target compound was synthesized by a method similar to that of Example 137. LC/MS (ESI+) calcd for C₃₉H₄₀N₆O₉ ([M+H]⁺) *m*/*z* 737.3, found 737.3. ¹H NMR (400 MHz, Chloroform-*d*) *δ* 8.13 (s, 1H), 7.59 (d, *J* = 8.3 Hz, 1H), 7.20 - 7.10 (m, 4H), 6.98 (d, *J* = 2.1 Hz, 1H), 6.92 - 6.86 (m, 2H), 6.84 - 6.79 (m, 2H), 6.75 (dd, *J* = 8.3, 2.1 Hz, 1H), 5.33 (s, 2H), 4.97 - 4.88 (m, 1H), 4.09 (t, *J* = 5.5 Hz, 2H), 3.86 - 3.76 (m, 4H), 3.74 - 3.65 (m, 4H), 3.46 (t, *J* = 6.4 Hz, 2H), 2.92 - 2.70 (m, 4H), 2.17 - 2.07 (m, 2H), 1.64 (s, 6H).

### Example 143: 5-((4-(2-(4-(3-((2-(2,6-dioxopiperidin-3-yl)-1,3-dioxoisoindolin-5-yl)amino)propoxy)phenyl)propan-2-yl)phenoxy)methyl)-1,2,4-oxadiazol-3-carbonylamine

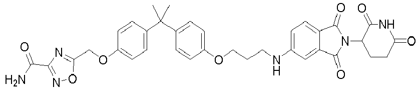

The target compound was synthesized by a method similar to that of Example 137. LC/MS (ESI+) calcd for C₃₅H₃₆N₆O₈([M+H]⁺) *m*/*z* 666.2, found 666.2. ¹H NMR (400 MHz, Chloroform-*d*) *δ* 8.10 (s, 1H), 7.59 (d, *J* = 8.3 Hz, 1H), 7.15 (dd, *J* = 8.7, 6.6 Hz, 4H), 6.98 (d, *J* = 2.0 Hz, 1H), 6.92 - 6.85 (m, 3H), 6.82 (d, *J* = 8.8 Hz, 2H), 6.76 - 6.72 (m, 1H), 5.33 (s, 2H), 4.93 (dd, *J* = 11.9, 5.2 Hz, 1H), 4.09 (t, *J* = 5.5 Hz, 2H), 3.47 (t, *J* = 6.5 Hz, 2H), 2.93 - 2.73 (m, 4H), 2.14 (d, *J* = 7.4 Hz, 2H), 1.29 - 1.23 (m, 6H).

### Example 144: 5-((R)-2-((4-(2-(4-((2-acetylpyrimidin-4-yl)methoxy)phenyl) propan-2-yl)phenoxy)methyl)pyrrolidin-1-yl)-2-(2,6-dioxopiperidin-3-yl) isoindolin-1,3-dione

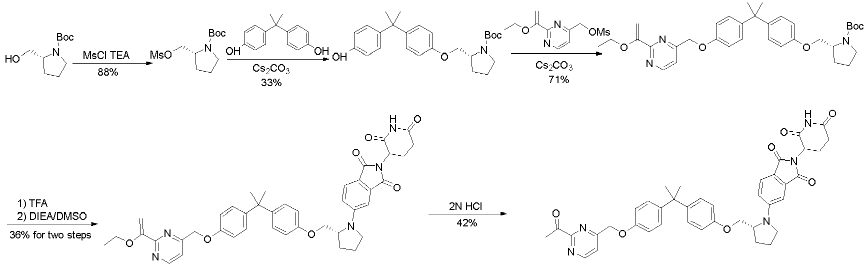

### Step 1: Synthesis of tert-butyl (R)-2-((methanesulfonyl)oxy)methyl)pyrrolidin-1-carboxylate

Boc-D-prolinol (1.05 g, 5.0 mmol) was dissolved in 15 mL of dichloromethane, to which was added triethylamine (1.01 g, 10.0 mmol), followed by addition of methanesulfonyl choride (684 mg, 6.0 mmol) in an ice water bath, and then the mixture was warmed to room temperature and allowed to react overnight at room temperature. The organic phase was successively washed with saturated NH₄Cl aqueous solution and saturated brine, dried over anhydrous MgSO₄, and concentrated to dry under reduced pressure, to provide the product tert-butyl (R)-2-((methanesulfonyl)oxy) methyl)pyrrolidin-1-carboxylate (1.22 g).

### Step 2: Synthesis of tert-butyl (R)-2-((4-(2-(4-hydroxylphenyl)propan-2-yl)phenoxy)methyl)pyrrolidin-1 -carboxylate

Bisphenol A (514 mg, 2.25 mmol) was dissolved in 6 mL of DMF, to which were sequentially added anhydrous cesium carbonate (975 mg, 3.0 mmol) and tert-butyl (R)-2-((methanesulfonyl)oxy)methyl)pyrrolidin-1-carboxylate (420 mg, 1.5 mmol), and then the mixture was allowed to react at 60 °C for 5 h. The reaction solution was cooled to room temperature, followed by addition of 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl (R)-2-((4-(2-(4-hydroxylphenyl)propan-2-yl)phenoxy)methyl) pyrrolidin-1-carboxylate (200 mg), with a yield of 33%. LC/MS (ESI+) calcd for C₂₅H₃₃NO₄ ([M+H]⁺) *m*/*z* 412.2, found 412.2.

### Step 3: Synthesis of tert-butyl (R)-2-((4-(2-(4-((2-(1-ethoxyvinyl)pyrimidin-4-yl)methoxy)phenyl)propan-2-yl)phenoxy)methyl)pyrrolidin-1-carboxylate

tert-butyl (R)-2-((4-(2-(4-hydroxylphenyl)propan-2-yl)phenoxy)methyl) pyrrolidin-1-carboxylate (112 mg, 0.27 mmol) was dissolved in 5 mL of DMF, to which was sequentially added anhydrous cesium carbonate (176 mg, 0.54 mmol) and (2-(1-ethoxyvinyl)pyrimidin-4-yl)methyl methanesulfonate (70 mg, 0.27 mmol), and then the mixture was allowed to react at 60 °C for 4 h. The reaction solution was cooled to room temperature, followed by addition of 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl (R)-2-((4-(2-(4-((2-(1-ethoxyvinyl)pyrimidin-4-yl)methoxy)phenyl) propan-2-yl)phenoxy)methyl)pyrrolidin-1-carboxylate (110 mg), with a yield of 71%. LC/MS (ESI+) calcd for C₃₄H₄₃N₃O₅ ([M+H]⁺) *m*/*z* 574.3, found 574.3.

### Step 4: Synthesis of 2-(2,6-dioxopiperidin-3-yl)-5-((R)-2-((4-(2-(1-ethoxyvinyl) pyrimidin-4-yl)methoxy)phenyl)propan-2-yl)phenoxy)methyl)pyrrolidin-1-yl)isoindolin-1,3-dione

tert-butyl (R)-2-((4-(2-(4-((2-(1-ethoxyvinyl)pyrimidin-4-yl)methoxy)phenyl) propan-2-yl)phenoxy)methyl)pyrrolidin-1-carboxylate (110 mg, 0.19 mmol) was dissolved in 4 mL of dichloromethane, to which was added 1 mL of trifluoroacetic acid dropwise in an ice-water bath, and then the mixture was allowed to react for 1h in the ice-water bath. The pH of the reaction solution was adjusted to alkaline with saturated Na₂CO₃ aqueous solution, followed by extraction with dichloromethane/methanol (10:1), dried over anhydrous MgSO₄, and concentrated to dry under reduced pressure. The residue was dissolved in 5 mL of DMSO, to which were added 5 drops of N,N-diisopropylethylamine and 2-(2,6-dioxopiperidin-3-yl)-5-fluoroisoindolin-1,3-dione (70 mg, 0.25 mmol), and then the mixture was allowed to react overnight at 90 °C. The reaction solution was cooled, extracted with dichloromethane/water, dried, and concentrated to dry. The residue was separated and purified by TLC, to provide the product 2-(2,6-dioxopiperidin-3-yl)-5-((R)-2-((4-(2-(1-ethoxyvinyl)pyrimidin-4-yl)methoxy)phenyl)propan-2-yl)phenoxy)methyl)pyrrolidin-1-yl)isoindolin-1,3-dione (50 mg), with a yield of 36%. LC/MS (ESI+) calcd for C₄₂H₄₃N₅O₇ ([M+H]⁺) *m*/*z* 730.3, found 730.3.

### Step 5: Synthesis of 5-((R)-2-((4-(2-(4-((2-acetylpyrimidin-4-yl)methoxy)phenyl) propan-2-yl)phenoxy)methyl)pyrrolidin-1-yl)-2-(2,6-dioxopiperidin-3-yl)isoindolin-1,3-dione

2-(2,6-dioxopiperidin-3-yl)-5-((R)-2-((4-(2-(1-ethoxyvinyl)pyrimidin-4-yl) methoxy)phenyl)propan-2-yl)phenoxy)methyl)pyrrolidin-1-yl)isoindolin-1,3-dione (50 mg, 0.069 mmol) was dissolved in 4 mL of acetone, to which was added 2 mL of 2N HCl aqueous solution, and then the mixture was allowed to react at room temperature for 1.5 h, followed by addition of 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product 5-((R)-2-((4-(2-(4-((2-acetylpyrimidin-4-yl)methoxy)phenyl)propan-2-yl) phenoxy)methyl)pyrrolidin-1-yl)-2-(2,6-dioxopiperidin-3-yl)isoindolin-1,3-dione (20 mg), with a yield of 42%. LC/MS (ESI+) calcd for C₄₀H₃₉N₅O₇ ([M+H]⁺) *m*/*z* 702.8, found 702.8; ¹H NMR (400 MHz, Chloroform-*d*) *δ* 8.91 (d, *J* = 5.1 Hz, 1H), 8.08 (d, *J* = 14.0 Hz, 1H), 7.73 (dd, *J=* 5.1, 1.1 Hz, 1H), 7.66 (d, *J* = 8.5 Hz, 1H), 7.20 - 7.06 (m, 5H), 6.89 - 6.80 (m, 3H), 6.79 - 6.73 (m, 2H), 5.24 (s, 2H), 4.94 (dd, *J* = 12.2, 5.2 Hz, 1H), 4.29 (s, 1H), 4.01 (dd, *J* = 9.2, 3.8 Hz, 1H), 3.84 (t, *J* = 8.4 Hz, 1H), 3.59 (t, *J* = 8.6 Hz, 1H), 3.34 (q, *J* = 8.6, 6.6 Hz, 1H), 2.93 - 2.83 (m, 1H), 2.80 (s, 3H), 2.79 - 2.59 (m, 2H), 2.28 - 2.08 (m, 5H), 1.63 (s, 6H).

### Example 145: 5-((S)-2-((4-(2-(4-((2-acetylpyrimidin-4-yl)methoxy)phenyl)propan-2-yl)phenoxy)methyl)pyrrolidin-1-yl)-2-(2,6-dioxopiperidin-3-yl)isoindolin-1,3-dione

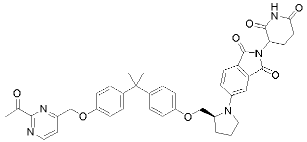

The target compound was synthesized by a method similar to that of Example 144. LC/MS (ESI+) calcd for C₄₀H₃₉N₅O₇ ([M+H]⁺) *m*/*z* 702.8, found 702.8. ¹H NMR (400 MHz, Chloroform-*d) δ* 8.92 (d, *J* = 4.9 Hz, 1H), 8.01 (d, *J* = 14.6 Hz, 1H), 7.73 (s, 1H), 7.66 (d, *J* = 8.4 Hz, 1H), 7.20 - 7.03 (m, 5H), 6.84 (t, *J* = 9.9 Hz, 3H), 6.77 (d, *J* = 8.5 Hz, 2H), 5.24 (s, 2H), 4.94 (dd, *J* = 12.3, 5.2 Hz, 1H), 4.29 (s, 1H), 4.01 (dd, *J* = 9.3, 3.6 Hz, 1H), 3.84 (t, *J* = 8.4 Hz, 1H), 3.59 (s, 1H), 3.34 (s, 1H), 2.93 - 2.69 (m, 6H), 2.27 - 2.09 (m, 5H), 1.63 (s, 6H).

### Example 146: 5-((1r,4r)-4-((4-(2-(4-((2-acetylpyrimidin-4-yl)methoxy)phenyl) propan-2-yl)phenoxy)methyl)cyclohexyl)amino)-2-(2,6-dioxopyridin-3-yl) isoindolin-1,3-dione

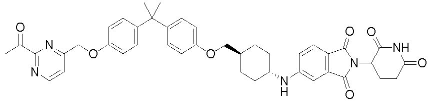

The target compound was synthesized by a method similar to that of Example 144. LC/MS (ESI+) calcd for C₄₂H₄₃N₅O₇ ([M+H]⁺) *m*/*z* 730.3, found 730.3. ¹H NMR (400 MHz, Chloroform-*d*) *δ* 8.92 (s, 1H), 7.97 (s, 1H), 7.74 (s, 1H), 7.61 (d, *J* = 8.1 Hz, 1H), 7.16 (dd, *J* = 15.3, 8.6 Hz, 4H), 6.96 (s, 1H), 6.87 (d, *J* = 8.5 Hz, 2H), 6.80 (d, *J* = 8.7 Hz, 2H), 6.73 (d, *J* = 8.3 Hz, 1H), 5.35 (t, *J* = 4.9 Hz, 2H), 5.25 (s, 2H), 4.93 (dd, *J* = 12.1, 5.5 Hz, 1H), 4.31 (t, *J* = 6.7 Hz, 1H), 3.78 (d, *J* = 6.1 Hz, 2H), 3.39 (s, 1H), 2.81 (s, 4H), 2.27 - 2.15 (m, 3H), 2.01 (d, *J* = 6.5 Hz, 4H), 1.42 (d, *J* = 6.6 Hz, 1H), 1.26 (s, 6H), 0.87 (d, *J* = 7.0 Hz, 2H).

### Example 147: 5-((4-(4-(2-(4-((2-(2-oxa-6-azaspiro[3.3]heptan-6-yl)pyrimidin-4-yl)methoxy)phenyl)propan-2-yl)phenoxy)piperidin-1-yl)methyl)-2-(2,6-dioxopiperidin-3-yl)isoindolin-1,3-dione

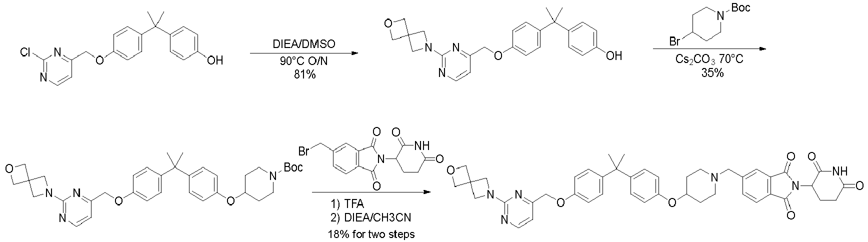

### Step 1: Synthesis of 4-(2-(4-((2-(2-oxa-6-azaspiro[3.3]heptan-6-yl)pyrimidin-4-yl)methoxy)phenyl)propan-2-yl)phenol

4-(2-(4-((2-chloropyrimidin-4-yl)methoxy)phenyl)propan-2-yl)phenol (1.76 g, 5.0 mmol) was dissolved in 13 mL of DMSO, to which were added DIEA (1.95 g, 15 mmol) and 2-oxa-6-azaspiro[3.3]heptane hemioxalate (865 mg, 3.0 mmol), and then the mixture was allowed to react overnight at 90 °C. The reaction solution was cooled to room temperature, followed by addition of 30 mL ethyl acetate and 25 mL water. The resultant solution was stirred and extracted. The water layer was re-extracted with 25 mL of ethyl acetate. The organic layers were combined, successively washed with water and saturated brine, dried with anhydrous Na₂SO₄, and concentrated to dry under reduced pressure. The residue was triturated in 10 mL of ethyl acetate for 30 min, and filtered. The filter cake was dried, to provide the product 4-(2-(4-((2-(2-oxa-6-azaspiro[3.3]heptan-6-yl)pyrimidin-4-yl)methoxy)phenyl)propan-2-yl)phenol (1.7 g), with a yield of 81%. LC/MS (ESI+) calcd for C₂₅H₂₇N₃O₃ ([M+H]⁺) *m*/*z* 418.2, found 418.2.

### Step 2: tert-butyl 4-(4-(2-(4-((2-(2-oxa-6-azaspiro[3.3]heptan-6-yl)pyrimidin-4-yl)methoxy)phenyl)propan-2-yl)phenoxy)piperidin-1 -carboxylate

4-(2-(4-((2-(2-oxa-6-azaspiro[3.3]heptan-6-yl)pyrimidin-4-yl)methoxy)phenyl) propan-2-yl)phenol (100 mg, 0.23 mmol) was dissolved in 5 mL of DMF, to which were successively added anhydrous cesium carbonate (325 mg, 1.0 mmol) and 1-N-Boc-4-bromopiperidine (132 mg, 0.5 mmol), and then the mixture was allowed to react overnight at 80 °C. The reaction solution was cooled to room temperature, followed by addition of 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl 4-(4-(2-(4-((2-(2-oxa-6-azaspiro[3.3]heptan-6-yl)pyrimidin-4-yl)methoxy)phenyl)propan-2-yl)phenoxy) piperidin-1-carboxylate (50 mg), with a yield of 35%. LC/MS (ESI+) calcd for C₃₅H₄₄N₄O₅ ([M+H]⁺) *m*/*z* 601.3, found 601.3.

### Step 3: 5-((4-(4-(2-(4-((2-(2-(2-oxa-6-azaspiro[3.3]heptan-6-yl)pyrimidin-4-yl) methoxy)phenyl)propan-2-yl)phenoxy)piperidin-1-yl)methyl)-2-(2,6-dioxopiperidin-3-yl)isoindolin-1,3-dione

tert-butyl 4-(4-(2-(4-((2-(2-oxa-6-azaspiro[3.3]heptan-6-yl)pyrimidin-4-yl) methoxy)phenyl)propan-2-yl)phenoxy)piperidin-1-carboxylate (50 mg, 0.083 mmol) was dissolved in 4 mL of dichloromethane, to which was added 1 mL of trifluoroacetic acid in the ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of acetonitrile, to which were added 3 drops of N,N-diisopropylethylamine and 5-(bromomethyl)-2-(2,6-dioxopiperidin-3-yl)isoindolin-1,3-dione (30 mg, 0.09 mmol), and then the mixture was allowed to react overnight at 40 °C. The reaction solution was cooled, extracted with dichloromethane/water, dried, and concentrated to dry. The residue was separated and purified by TLC, to provide the product 5-((4-(4-(2-(4-((2-(2-(2-oxa-6-azaspiro[3.3]heptan-6-yl)pyrimidin-4-yl)methoxy)phenyl)propan-2-yl)phenoxy) piperidin-1-yl)methyl)-2-(2,6-dioxopiperidin-3-yl)isoindolin-1,3-dione (12 mg), with a yield of 18%. LC/MS (ESI+) calcd for C₄₄H₄₆N₆O₇([M+H]⁺) *m*/*z* 771.3, found 771.3; ¹H NMR (400 MHz, Chloroform-*d*) *δ* 8.30 (d, *J* = 5.1 Hz, 1H), 8.22 (s, 1H), 7.93 (s, 1H), 7.88 (d, *J* = 4.6 Hz, 1H), 7.17 - 7.06 (m, 4H), 6.86 - 6.74 (m, 5H), 4.99 (dd, *J* = 12.3, 5.2 Hz, 1H), 4.94 (s, 2H), 4.86 (s, 4H), 4.41 (s, 1H), 4.29 (s, 4H), 3.84 (s, 1H), 2.97 - 2.68 (m, 5H), 2.54 (s, 1H), 2.17 (dt, *J* = 10.6, 6.6 Hz, 2H), 1.94 (s, 3H), 1.62 (s, 6H), 1.26 (s, 2H).

### Example 148: 5-((3-(4-(2-(4-(4-((2-(2-oxa-6-azaspiro[3.3]heptan-6-yl)pyrimidin-4-yl)methoxy)phenyl)propan-2-yl)phenoxy)azetidin-1-yl)methyl)-2-(2,6-dioxopiperidin-3-yl)isoindolin-1,3-dione

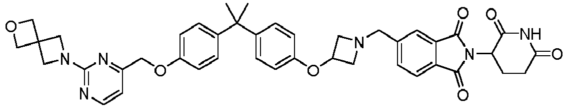

The target compound was synthesized by a method similar to that of Example 147. LC/MS (ESI+) calcd for C₄₂H₄₂N₆O₇([M+H]⁺) *m*/*z* 743.3, found 743.3. ¹H NMR (400 MHz, Chloroform-*d) δ* 8.30 (d, *J* = 5.0 Hz, 1H), 8.27 (s, 1H), 7.88 (s, 3H), 7.12 (dd, *J* = 8.8, 2.1 Hz, 4H), 6.86 - 6.78 (m, 3H), 6.68 - 6.61 (m, 2H), 4.98 (dd, *J* = 12.2, 5.3 Hz, 1H), 4.94 (s, 2H), 4.89 (d, *J* = 6.3 Hz, 1H), 4.86 (s, 4H), 4.29 (s, 4H), 4.11 (s, 1H), 4.04 (s, 2H), 3.40 (s, 2H), 2.96 - 2.69 (m, 4H), 2.19 - 2.11 (m, 1H), 1.61 (s, 6H).

### Example 149: 5-((R)-1-(4-(2-(4-((2-(2-oxa-6-azaspiro[3.3]heptane-6-yl)pyrimidin-4-yl)methoxy)phenyl)propan-2-yl)phenoxy)-3-methylbutan-2-yl)amino)-2-(2,6-dioxopiperidin-3-yl)isoindolin-1,3-dione

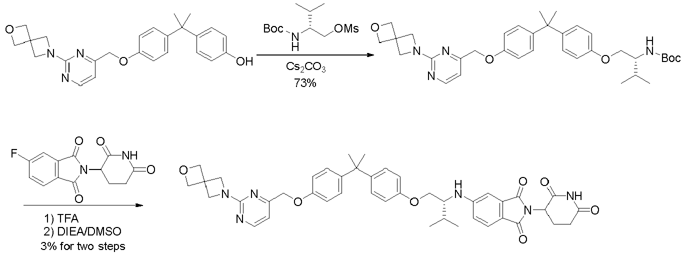

### Step 1: Synthesis of tert-butyl (R)-(1-(4-(2-(4-((2-(2-(2-oxa-6-azaspiro[3.3]heptan-6-yl)pyrimidin-4-yl)methoxy)phenyl)propan-2-yl)phenoxy)-3-methylbutan-2-yl) carbamate

4-(2-(4-((2-(2-oxa-6-azaspiro[3.3]heptan-6-yl)pyrimidin-4-yl)methoxy)phenyl) propan-2-yl)phenol (140 mg, 0.34 mmol) was dissolved in 5 mL of DMF, to which were added cesium carbonate (330 mg, 1.02 mmol) and (R)-2-((tert-butoxycarbonyl)amino)-3-methylbutyl methanesulfonate (191 mg, 0.68 mmol), and then the mixture was allowed to react overnight at 100 °C. The reaction solution was cooled to room temperature, followed by addition of 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl (R)-(1-(4-(2-(4-((2-(2-(2-oxa-6-azaspiro[3.3]heptan-6-yl)pyrimidin-4-yl) methoxy)phenyl)propan-2-yl)phenoxy)-3-methylbutan-2-yl)carbamate (150 mg), with a yield of 73%. LC/MS (ESI+) calcd for C₃₅H₄₆N₄O₅ ([M+H]⁺) *m*/*z* 603.3, found 603.3.

### Step 2: Synthesis of 5-((R)-1-(4-(2-(4-((2-(2-(2-oxa-6-azaspiro[3.3]heptan-6-yl) pyrimidin-4-yl)methoxy)phenyl)propan-2-yl)phenoxy)-3-methylbutan-2-yl)amino)-2-(2,6-dioxopiperidin-3-yl)isoindolin-1,3-dione

tert-butyl (R)-(1-(4-(2-(4-((2-(2-(2-oxa-6-azaspiro[3.3]heptan-6-yl)pyrimidin-4-yl)methoxy)phenyl)propan-2-yl)phenoxy)-3-methylbutan-2-yl)carbamate (80 mg, 0.13 mmol) was dissolved in 4 mL of dichloromethane, to which was added 1 mL of trifluoroacetic acid in the ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 3 drops of N,N-diisopropylethylamine and 2-(2,6-dioxopiperidin-3-yl)-5-fluoroisoindolin-1,3-dione (40 mg, 0.15 mmol), and then the mixture was allowed to react overnight at 90 °C. The reaction solution was cooled, extracted with dichloromethane/water, dried, and concentrated to dry. The residue was separated and purified by TLC, to provide the product 5-((R)-1-(4-(2-(4-((2-(2-(2-oxa-6-azaspiro[3.3]heptan-6-yl)pyrimidin-4-yl)methoxy)phenyl)propan-2-yl)phenoxy)-3-methylbutan-2-yl)amino)-2-(2,6-dioxopiperidin-3-yl)isoindolin-1,3-dione (3 mg), with a yield of 3%. LC/MS (ESI+) calcd for C₄₃H₄₆N₆O₇([M+H]⁺) *m*/*z* 759.3, found 759.3; ¹H NMR (400 MHz, Chloroform-*d*) *δ* 8.28 (s, 1H), 8.15 (s, 1H), 7.60 (d, *J* = 8.2 Hz, 1H), 7.11 (d, *J* = 8.4 Hz, 4H), 7.03 (s, 1H), 6.98 (s, 1H), 6.82 - 6.75 (m, 5H), 5.02 (s, 2H), 4.93 (s, 1H), 4.87 (s, 4H), 4.56 (s, 4H), 4.05 (s, 2H), 3.63 (s, 1H), 2.89 - 2.76 (m, 3H), 2.13 (s, 1H), 1.62 (s, 6H), 1.06 (dd, *J* = 6.7, 3.3 Hz, 6H).

### Example 150: 5-((S)-1-(4-(2-(2-oxa-6-azaspiro[3.3]heptan-6-yl)pyrimidin-4-yl)methoxy)phenyl)propan-2-yl)phenoxy)-3-methylbutan-2-yl)amino)-2-(2,6-dioxopiperidin-3-yl)isoindolin-1,3-dione

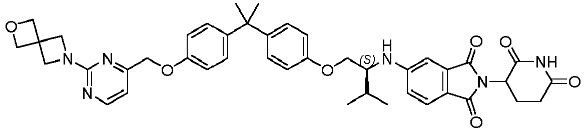

The target compound was synthesized by a method similar to that of Example 149. LC/MS (ESI+) calcd for C₄₃H₄₆N₆O₇([M+H]⁺) *m*/*z* 759.3, found 759.3.

### Example 151: 5-((1-(4-(2-(4-((2-(2-oxa-6-azaspiro[3.3]heptan-6-yl)pyrimidin-4-yl)methoxy)phenyl)propan-2-yl)phenoxy)propan-2-yl)amino)-2-(2,6-dioxopiperidin-3-yl)isoindolin-1,3-dione

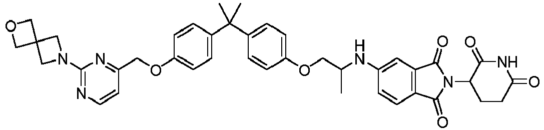

The target compound was synthesized by a method similar to that of Example 149. LC/MS (ESI+) calcd for C₄₁H₄₂N₆O₇([M+H]⁺) *m*/*z* 731.3, found 731.3. ¹H NMR (400 MHz, Chloroform-*d*) *δ* 8.31 (d, *J* = 4.7 Hz, 1H), 8.11 (d, *J* = 14.2 Hz, 1H), 7.62 (d, *J* = 8.3 Hz, 1H), 7.13 (d, *J* = 8.5 Hz, 4H), 7.02 (d, *J* = 2.1 Hz, 1H), 6.91 (s, 1H), 6.80 (t, *J* = 8.9 Hz, 5H), 4.99 (s, 2H), 4.96 - 4.91 (m, 1H), 4.86 (s, 4H), 4.45 (s, 4H), 4.02 (s, 2H), 3.97 (d, *J* = 5.3 Hz, 1H), 2.94 - 2.66 (m, 4H), 2.12 (d, *J* = 5.3 Hz, 1H), 1.63 (s, 6H), 1.41 (d, *J* = 6.0 Hz, 3H).

### Example 152: 5-((1R)-2-(4-(2-(2-(2-oxa-6-azaspiro[3.3]heptane-6-yl)pyrimidin-4-yl)methoxy)phenyl)propan-2-yl)phenoxy)cyclohexyl)amino)-2-(2,6-dioxopyridin-3-yl)isoindolin-1,3-dione

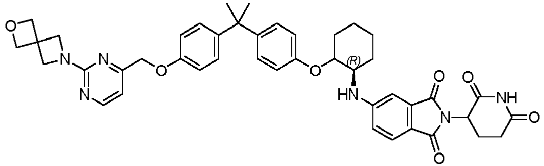

The target compound was synthesized by a method similar to that of Example 149. LC/MS (ESI+) calcd for C₄₄H₄₆N₆O₇([M+H]⁺) *m*/*z* 771.3, found 771.3.

### Example 153: 5-(((1s,4s)-4-(4-(2-(4-((2-(2-oxa-6-azaspiro[3.3|heptan-6-yl) pyrimidin-4-yl)methoxy)phenyl)propan-2-yl)phenoxy)cyclohexyl)amino)-2-(2,6-dioxopyridin-3-yl)isoindolin-1,3-dione

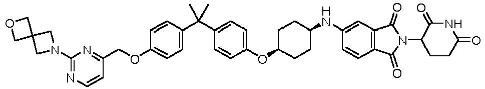

The target compound was synthesized by a method similar to that of Example 149. LC/MS (ESI+) calcd for C₄₄H₄₆N₆O₇([M+H]⁺) *m*/*z* 771.3, found 771.3. ¹H NMR (400 MHz, Chloroform-*d*) *δ* 8.31 (d, *J* = 5.0 Hz, 1H), 8.08 (s, 1H), 7.61 (d, *J* = 8.3 Hz, 1H), 7.18 - 7.09 (m, 4H), 6.97 (d, *J* = 2.2 Hz, 1H), 6.86 - 6.77 (m, 5H), 6.74 (dd, *J* = 8.3, 2.2 Hz, 1H), 4.94 (s, 2H), 4.94 - 4.89 (m, 1H), 4.86 (s, 4H), 4.51 (d, *J* = 11.9 Hz, 2H), 4.30 (s, 4H), 3.50 (s, 1H), 2.93 - 2.68 (m, 3H), 2.16 - 2.06 (m, 2H), 2.01 (d, *J* = 5.5 Hz, 1H), 1.89 (s, 2H), 1.72 (d, *J* = 9.8 Hz, 4H), 1.26 (s, 6H).

### Example 154: 5-((1-(4-(2-(4-(4-((2-(2-oxa-6-azaspiro[3.3]heptan-6-yl)pyrimidin-4-yl)methoxy)phenyl)propan-2-yl)phenyl)azetidin-3-yl)amino)-2-(2,6-dioxopiperidin-3-yl)isoindolin-1,3-dione

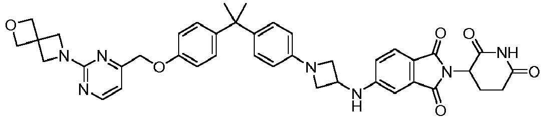

The target compound was synthesized by a method similar to that of Example 149. LC/MS (ESI+) calcd for C₄₁H₄₁N₇O₆([M+H]⁺) *m*/*z* 728.3, found728.3; ¹H NMR (400 MHz, Chloroform-*d*) δ 8.30 (d, *J* = 5.1 Hz, 1H), 8.13 (s, 1H), 7.65 (d, *J* = 8.2 Hz, 1H), 7.17 - 7.07 (m, 4H), 6.92 (d, *J* = 2.1 Hz, 1H), 6.86 - 6.79 (m, 3H), 6.75 (dd, *J* = 8.3, 2.2 Hz, 1H), 6.46 - 6.38 (m, 2H), 5.02 (d, *J* = 6.5 Hz, 1H), 4.94 (s, 2H), 4.92 (d, *J* = 5.4 Hz, 1H), 4.86 (s, 4H), 4.45 (q, *J* = 5.7 Hz, 1H), 4.31 (s, 6H), 3.68 (dd, *J* = 7.6, 4.6 Hz, 2H), 2.92 - 2.69 (m, 3H), 2.15 - 2.08 (m, 1H), 1.62 (s, 6H).

### Example 155: 5-((1s,3s)-3-(4-(2-(4-(2-oxa-6-azaspiro[3.3]heptane-6-yl)pyrimidin-2-yl)oxy)phenyl)propan-2-yl)phenoxy)cyclobutyl)amino)-2-(2,6-dioxopyridin-3-yl)isoindolin-1,3-dione

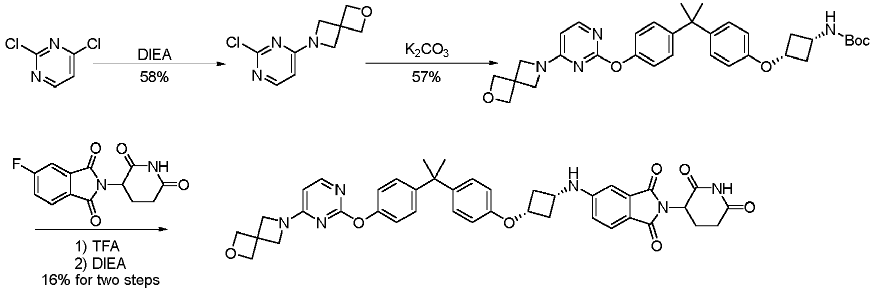

### Step 1: Synthesis of 6-(2-chloropyrimidin-4-yl)-2-oxa-6-azaspiro[3.3]heptane

2,4-dichloropyrimidine (1.19 g, 8.0 mmol) was dissolved in 20 mL, to which were successively added 2-oxa-6-azaspiro[3.3]heptanhemioxalate (577 mg, 2.0 mmol) and N,N-diisopropylethylamine (1.03 g, 8.0 mmol), and then the mixture was allowed to react at room temperature for 3h. The reaction solution was concentrated to dry under reduced pressure. The residue was separated and purified by TLC, to provide 6-(2-chloropyrimidin-4-yl)-2-oxa-6-azaspiro[3.3]heptane (490 mg), with a yield of 58%. LC/MS (ESI+) calcd for C₉H₁₀ClN₃O ([M+H]⁺) *m*/*z* 212.0, found 212.0.

### Step 2: Synthesis of tert-butyl ((1s,3s)-3-(4-(2-(4-((4-(2-oxa-6-azaspiro[3.3]heptane-6-yl)pyrimidin-2-yl)oxy)phenyl)propan-2-yl)phenoxy)cyclobutyl)carbamate

tert-butyl ((1s,3s)-3-(4-(2-(4-hydroxylphenyl)propan-2-phenoxy)cyclobutyl) carbamate (90 mg, 0.23 mmol) was dissolved in 5 mL of DMF, to which were successively added anhydrous K₂CO₃ (64 mg, 0.46 mmol) and 6-(2-chloropyrimidin-4-yl)-2-oxa-6-azaspiro[3.3]heptane (72 mg, 0.34 mmol), and then the mixture was allowed to react overnight at 100 °C. The reaction solution was cooled to room temperature, followed by addition of 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl ((1s,3s)-3-(4-(2-(4-((4-(2-oxa-6-azaspiro[3.3]heptane-6-yl) pyrimidin-2-yl)oxy)phenyl)propan-2-yl)phenoxy)cyclobutyl)carbamate (75 mg), with a yield of 57%. LC/MS (ESI+) calcd for C₃₃H₄₀N₄O₅ ([M+H]⁺) *m*/*z* 573.3, found 573.3.

### Step 3: Synthesis of 5-((1s,3s)-3-(4-(2-(4-(2-oxa-6-azaspiro[3.3]heptane-6-yl) pyrimidin-2-yl)oxy)phenyl)propan-2-yl)phenoxy)cyclobutyl)amino)-2-(2,6-dioxopyridin-3-yl)isoindolin-1,3-dione

tert-butyl ((1s,3s)-3-(4-(2-(4-((4-(2-oxa-6-azaspiro[3.3]heptane-6-yl)pyrimidin-2-yl)oxy)phenyl)propan-2-yl)phenoxy)cyclobutyl)carbamate (75 mg, 0.13 mmol) was dissolved in 4 mL of dichloromethane, to which was added 1.5 mL of trifluoroacetic acid in the ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 5 drops of N,N-diisopropylethylamine and 2-(2,6-dioxopiperidin-3-yl)-5-fluoroisoindolin-1,3-dione (45 mg, 0.16 mmol), and then the mixture was allowed to react overnight at 90 °C. The reaction solution was cooled, extracted with dichloromethane/water, dried, and concentrated to dry. The residue was separated and purified by TLC, to provide the product 5-((1s,3s)-3-(4-(2-(4-(2-oxa-6-azaspiro[3.3]heptane-6-yl)pyrimidin-2-yl)oxy)phenyl)propan-2-yl)phenoxy) cyclobutyl)amino)-2-(2,6-dioxopyridin-3-yl)isoindolin-1,3-dione (10 mg), with a yield of 16%. LC/MS (ESI+) calcd for C₄₁H₄₀N₆O₇ ([M+H]⁺) *m*/*z* 729.3, found 729.3; ¹H NMR (400 MHz, Chloroform-*d*) *δ* 8.12 (s, 1H), 7.98 (*d, J* = 5.8 Hz, 1H), 7.62 (d, *J* = 8.3 Hz, 1H), 7.24 - 7.20 (m, 2H), 7.18 - 7.14 (m, 2H), 7.07 - 7.04 (m, 2H), 6.92 (d, *J* = 2.2 Hz, 1H), 6.74 - 6.70 (m, 3H), 5.92 (d, *J* = 5.9 Hz, 1H), 4.93 (dd, *J* = 12.2, 5.3 Hz, 1H), 4.85 (s, 4H), 4.51 (t, *J* = 6.8 Hz, 1H), 4.26 (s, 4H), 3.77 (t, *J* = 8.3 Hz, 1H), 3.17 - 3.08 (m, 2H), 2.95 - 2.68 (m, 4H), 2.17 - 2.04 (m, 3H), 1.66 (d, *J* = 2.8 Hz, 6H).

### Example 156: 5-((1r,3r)-3-(4-(2-(4-(2-oxa-6-azaspiro[3.3]heptane-6-yl)pyrimidin-2-yl)oxy)phenyl)propan-2-yl)phenoxy)cyclobutyl)amino)-2-(2,6-dioxopyridin-3-yl)isoindolin-1,3-dione

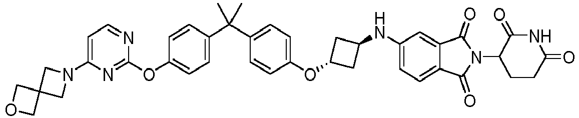

The target compound was synthesized by a method similar to that of Example 155. LC/MS (ESI+) calcd for C₄₁H₄₀N₆O₇ ([M+H]⁺) *m*/*z* 729.3, found 729.3.

### Example 157: 2-(2,6-dioxopiperidin-3-yl)-5-((ls,3s)-3-(4-(4-((5-fluoro-4-(2-oxa-6-azaspiro[3.3]heptan-6-yl)pyrimidin-2-yl)oxy)phenyl)propan-2-yl)phenoxy) cyclobutyl)amino)isoindolin-1,3-dione

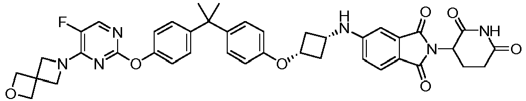

The target compound was synthesized by a method similar to that of Example 155. LC/MS (ESI+) calcd for C₄₁H₃₉FN₆O₇ ([M+H]⁺) *m*/*z* 747.3, found 747.3. ¹H NMR (400 MHz, Chloroform-*d*) *δ* 8.06 (s, 1H), 7.76 (d, *J* = 3.6 Hz, 1H), 7.62 (d, *J* = 8.3 Hz, 1H), 7.24 - 7.19 (m, 2H), 7.19 - 7.13 (m, 2H), 7.06 - 7.01 (m, 2H), 6.92 (d, *J* = 2.2 Hz, 1H), 6.72 (dd, *J* = 9.0, 2.5 Hz, 3H), 4.93 (dd, *J* = 12.1, 5.2 Hz, 1H), 4.85 (s, 4H), 4.77 (s, 1H), 4.51 (t, *J* = 6.8 Hz, 1H), 4.43 (s, 4H), 3.77 (s, 1H), 3.18 - 3.07 (m, 2H), 2.93 - 2.66 (m, 3H), 2.16 - 2.03 (m, 3H), 1.66 (s, 6H).

### Example 158: 2-(2,6-dioxopiperidin-3-yl)-5-((1s,3s)-3-(4-(4-((5-fluoro-2-(2-oxa-6-azaspiro[3.3]heptan-6-yl)pyrimidin-4-yl)oxy)phenyl)propan-2-yl)phenoxy) cyclobutyl)amino)isoindolin-1,3-dione

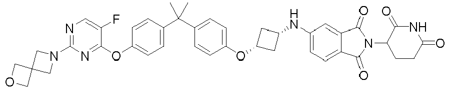

The target compound was synthesized by a method similar to that of Example 155. LC/MS (ESI+) calcd for C₄₁H₃₉FN₆O₇ ([M+H]⁺) *m*/*z* 747.3, found 747.3. ¹H NMR (400 MHz, Chloroform-*d*) *δ* 8.12 - 8.04 (m, 2H), 7.62 (d, *J* = 8.2 Hz, 1H), 7.24 (s, 2H), 7.18 - 7.11 (m, 2H), 7.08 (t, *J* = 6.5 Hz, 2H), 6.92 (d, *J* = 2.1 Hz, 1H), 6.77 - 6.69 (m, 3H), 4.93 (dd, *J* = 12.0, 5.3 Hz, 1H), 4.79 (s, 4H), 4.54 (q, *J* = 7.0 Hz, 1H), 4.12 (s, 4H), 3.79 (d, *J* = 9.4 Hz, 2H), 3.19 - 3.08 (m, 2H), 2.93 - 2.71 (m, 3H), 2.11 (dd, *J* = 10.4, 4.7 Hz, 3H), 1.69 (s, 6H).

### Example 159: 5-((1s,3s)-3-(4-(2-(4-((6-(2-oxa-6-azaspiro[3.3]heptan-6-yl)pyrazin-2-yl)oxy)phenyl)propan-2-yl)phenoxy)cyclobutyl)amino)-2-(2,6-dioxopyridin-3-yl)isoindolin-1,3-dione

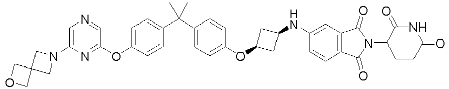

The target compound was synthesized by a method similar to that of Example 155. LC/MS (ESI+) calcd for C₄₁H₄₀N₆O₇ ([M+H]⁺) *m*/*z*729.3, found 729.3. ¹H NMR (400 MHz, Chloroform-*d*) δ 8.07 (s, 1H), 7.62 (d, *J* = 8.2 Hz, 1H), 7.46 (d, *J =* 2.1 Hz, 2H), 7.25 - 7.20 (m, 2H), 7.17 - 7.12 (m, 2H), 7.05 - 6.99 (m, 2H), 6.92 (d, *J* = 2.1 Hz, 1H), 6.76 - 6.70 (m, 3H), 4.93 (dd, *J* = 12.1, 5.2 Hz, 1H), 4.84 (s, 4H), 4.77 (d, *J* = 6.0 Hz, 1H), 4.53 (t, *J* = 6.8 Hz, 1H), 4.19 (s, 4H), 3.78 (d, *J* = 6.8 Hz, 1H), 3.18 - 3.09 (m, 2H), 2.93 - 2.70 (m, 3H), 2.16 - 2.04 (m, 3H), 1.67 (s, 6H).

### Example 160: 5-((1r,3r)-3-(4-(2-(4-(2-oxa-6-azaspiro[3.3]heptane-6-yl)-1,3,5-triazin-2-yl)oxy)phenyl)propan-2-yl)phenoxy)cyclobutyl)amino)-2-(2,6-dioxopiperidin-3-yl)isoindolin-1,3-dione

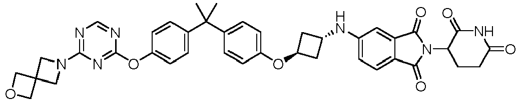

The target compound was synthesized by a method similar to that of Example 155. LC/MS (ESI+) calcd for C₄₀H₃₉N₇O₇ ([M+H]⁺) *m*/*z* 730.3, found 730.3. ¹H NMR (400 MHz, Chloroform-*d*) *δ* 8.35 (s, 1H), 8.07 (s, 1H), 7.63 (d, *J* = 8.3 Hz, 1H), 7.26 - 7.23 (m, 2H), 7.18 - 7.13 (m, 2H), 7.07 - 7.02 (m, 2H), 6.89 (d, *J* = 2.1 Hz, 1H), 6.73 - 6.67 (m, 3H), 4.96 - 4.90 (m, 1H), 4.89 - 4.86 (m, 1H), 4.85 (d, *J* = 2.5 Hz, 5H), 4.33 (d, *J* = 21.5 Hz, 4H), 4.22 (d, *J* = 6.9 Hz, 1H), 2.92 - 2.74 (m, 3H), 2.70 (dd, *J* = 13.2, 5.9 Hz, 2H), 2.40 (dt, *J* = 13.6, 6.3 Hz, 2H), 2.16 - 2.08 (m, 1H), 1.67 (s, 6H).

### Example 161: 2-(2,6-dioxopiperidin-3-yl)-5-((1r,3r)-3-(4-(2-(4-(2-hydroxylpropan-2-yl)pyrimidin-2-yl)oxy)phenyl)propan-2-yl)phenoxy)cyclobutyl)amino) isoindolin-1,3-dione

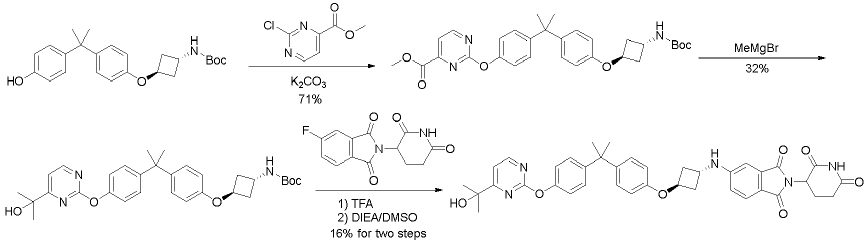

### Step 1: Synthesis of methyl 2-(4-(2-(4-((1r,3r)-3-((tert-butoxycarbonyl)amino) cyclobutyloxy)phenyl)propan-2-yl)phenoxy)pyrimidin-4-carboxylate

tert-butyl ((1r,3r)-3-(4-(2-(4-hydroxylphenyl)propan-2-phenoxy)cyclobutyl) carbamate (200 mg, 0.5 mmol) was dissolved in 5 mL of DMF, to which was added anhydrous K₂CO₃ (138 mg, 1.0 mmol), followed by addition of methyl 2-chloropyrimidin-4-carboxylate (86 mg, 0.5 mmol) under stirring, and then the mixture was allowed to react overnight at room temperature, followed by addition of 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product methyl 2-(4-(2-(4-((1r,3r)-3-((tert-butoxycarbonyl)amino)cyclobutyloxy)phenyl)propan-2-yl)phenoxy)pyrimidin-4-carboxylate (190 mg), with a yield of 71%. LC/MS (ESI+) calcd for C₃₀H₃₅N₃O₆ ([M+H]⁺) *m*/*z* 534.2, found 534.2.

### Step 2: Synthesis of tert-butyl ((1r,3r)-3-(4-(2-(4-((4-(2-hydroxylpropan-2-yl) pyrimidin-2-yl)oxy)phenyl)propan-2-yl)phenoxy)cyclobutyl)carbamate

Methyl 2-(4-(2-(4-((1r,3r)-3-((tert-butoxycarbonyl)amino)cyclobutyloxy)phenyl) propan-2-yl)phenoxy)pyrimidin-4-carboxylate (190 mg, 0.35 mmol) was dissolved in 5 mL of anhydrous tetrahydrofuran, to which was added 0.6 mL of 3N MeMgBr solution dropwise in an ice-water bath under nitrogen protection. After that, the mixture was allowed to react for 1.5 h in the ice-water bath, and then the reaction was quenched with saturated NH₄Cl aqueous solution, followed by addition of 15 mL dichloromethane and 10 mL water. The resultant solution was stirred and extracted. The water layer was re-extracted with 10 mL of dichloromethane. The organic layers were combined, successively washed with water and saturated brine, dried with anhydrous Na₂SO₄, and concentrated to dry under reduced pressure. The residue was separated and purified by TLC, to provide the product tert-butyl ((1r,3r)-3-(4-(2-(4-((4-(2-hydroxylpropan-2-yl)pyrimidin-2-yl)oxy)phenyl)propan-2-yl)phenoxy)cyclobutyl) carbamate (60 mg), with a yield of 32%. LC/MS (ESI+) calcd for C₃₁H₃₉N₃O₅ ([M+H]⁺) *m*/*z* 534.2, found 534.2.

### Step 3: Synthesis of 2-(2,6-dioxopiperidin-3-yl)-5-((1r,3r)-3-(4-(2-(4-(2-hydroxylpropan-2-yl)pyrimidin-2-yl)oxy)phenyl)propan-2-yl)phenoxy)cyclobutyl) amino)isoindolin-1,3-dione

tert-butyl ((1r,3r)-3-(4-(2-(4-((4-(2-hydroxylpropan-2-yl)pyrimidin-2-yl)oxy) phenyl)propan-2-yl)phenoxy)cyclobutyl)carbamate (60 mg, 0.11mmol) was dissolved in 4 mL of dichloromethane, to which was added 1.5 mL of trifluoroacetic acid in the ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 5 drops of N,N-diisopropylethylamine and 2-(2,6-dioxopiperidin-3-yl)-5-fluoroisoindolin-1,3-dione (45 mg, 0.16 mmol), and then the mixture was allowed to react overnight at 90 °C. The reaction solution was cooled, extracted with dichloromethane/water, dried, and concentrated to dry. The residue was separated and purified by TLC, to provide the product 2-(2,6-dioxopiperidin-3-yl)-5-((1r,3r)-3-(4-(2-(4-(2-hydroxylpropan-2-yl)pyrimidin-2-yl)oxy)phenyl)propan-2-yl)phenoxy)cyclobutyl)amino)isoindolin-1,3-dione (12 mg), with a yield of 16%. LC/MS (ESI+) calcd for C₃₉H₃₉N₅O₇ ([M+H]⁺) *m*/*z* 690.2, found 690.2; ¹H NMR (400 MHz, Chloroform-*d*) *δ* 8.51 (d, *J* = 5.1 Hz, 1H), 8.06 (s, 1H), 7.63 (d, *J* = 8.3 Hz, 1H), 7.28 (d, *J* = 2.2 Hz, 1H), 7.25 (d, *J* = 3.1 Hz, 1H), 7.17 (d, *J* = 8.8 Hz, 2H), 7.14 - 7.06 (m, 3H), 6.90 (d, *J* = 2.1 Hz, 1H), 6.71 (dd, *J* = 9.5, 2.4 Hz, 3H), 4.93 (dd, *J* = 12.2, 5.2 Hz, 1H), 4.88 (d, *J* = 11.1 Hz, 1H), 4.27 - 4.19 (m, 1H), 2.94 - 2.65 (m, 6H), 2.41 (dt, *J* = 12.9, 6.1 Hz, 2H), 2.15 - 2.08 (m, 1H), 1.68 (s, 6H), 1.53 (s, 6H).

### Example 162: 2-(2,6-dioxopiperidin-3-yl)-5-((1r,3r)-3-(4-(2-(4-((6-(2-hydroxylpropan-2-yl)pyrazin-2-yl)oxy)phenyl)propan-2-yl)phenoxy)cyclobutyl)amino)isoindolin-1,3-dione

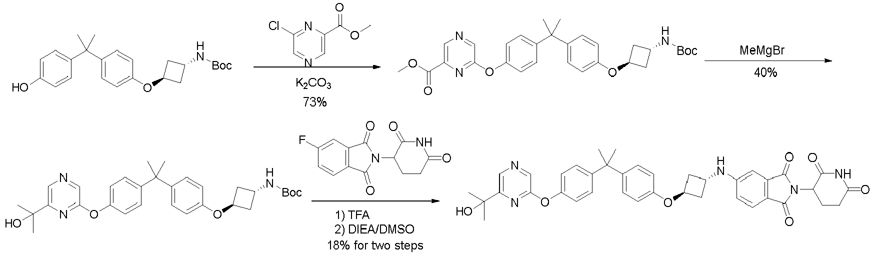

The target compound was synthesized by a method similar to that of Example 161. LC/MS (ESI+) calcd for C₃₉H₃₉N₅O₇ ([M+H]⁺) *m*/*z* 690.2, found 690.2. ¹H NMR (400 MHz, Chloroform-*d*) *δ* 8.42 (s, 1H), 8.24 (s, 1H), 8.04 (s, 1H), 7.64 (d, *J* = 8.3 Hz, 1H), 7.27 (s, 1H), 7.25 (d, *J* = 3.1 Hz, 1H), 7.18 - 7.11 (m, 2H), 7.07 - 7.02 (m, 2H), 6.90 (d, *J* = 2.1 Hz, 1H), 6.75 - 6.68 (m, 3H), 4.93 (dd, *J* = 12.2, 5.3 Hz, 1H), 4.90 - 4.83 (m, 1H), 4.77 (s, 1H), 4.23 (d, *J=* 4.6 Hz, 1H), 2.94 - 2.74 (m, 3H), 2.74 - 2.66 (m, 2H), 2.41 (dt, *J* = 12.9, 6.0 Hz, 2H), 2.16 - 2.09 (m, 1H), 1.68 (s, 6H), 1.54 (s, 6H).

### Example 163: 5-((1r,3r)-3-(4-(2-(2-(4-((1H-1,2,3-triazol-2-yl)pyridin-4-yl)oxy) phenyl)propan-2-ylphenoxy)cyclobutyl)amino)-2-(2,6-dioxopiperidin-3-yl) isoindolin-1,3-dione

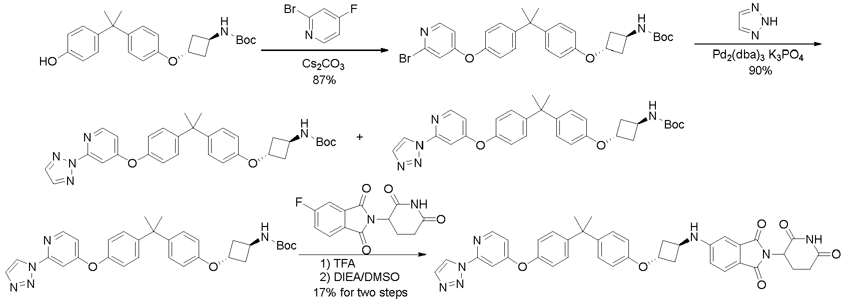

### Step 1: Synthesis of tert-butyl ((1r,3r)-3-(4-(2-(4-((2-bromopyridin-4-yl)oxy)phenyl) propan-2-yl)phenoxy)cyclobutyl)carbamate

tert-butyl ((1r,3r)-3-(4-(2-(4-hydroxylphenyl)propan-2-phenoxy)cyclobutyl) carbamate (200 mg, 0.5 mmol) was dissolvd in 5 mL of DMF, to which was added anhydrous cesium carbonate (325 mg, 1.0 mmol), followed by addition of 4-fluoro-2-bromopyridine (106 mg, 0.6 mmol) under stirring, and then the mixture was allowed to react for 4 h at 80 °C. The reaction solution was cooled to room temperature, to which were added 15 mL ethyl acetate and 12 mL water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl ((1r,3r)-3-(4-(2-(4-((2-bromopyridin-4-yl)oxy)phenyl)propan-2-yl)phenoxy)cyclobutyl)carbamate (240 mg), with a yield of 87%. LC/MS (ESI+) calcd for C₂₉H₃₃BrN₂O₄ ([M+H]⁺) *m*/*z* 553.1, found 553.1.

### Step 2: Synthesis of tert-butyl ((1r,3r)-3-(4-(2-(4-((2-(2H-l,2,3-triazol-2-yl)pyridin-4-yl)oxy)phenyl)propan-2-yl)phenoxy)cyclobutanecarbamate and tert-butyl ((1r,3r)-3-(4-(2-(4-(2-(1H-1,2,3-triazol-2-yl)pyridin-4-yl)oxy)phenyl)propan-2-yl)phenoxy) cyclobutanecarbamate

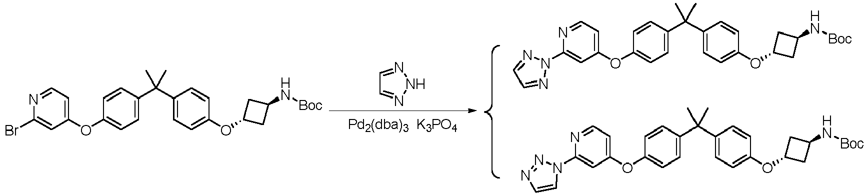

Pd₂(dba)₃ (19 mg, 0.02 mmol) and ligand (26 mg, 0.055 mmol) were added to a singlenecked round-bottom flask, to which was added 1 mL of toluene, and then the mixture was stirred at 120 °C for 3 min under argon gas protection, to produce pre-catalyst. The reaction solution was cooled to room temperature. tert-butyl ((1r,3r)-3-(4-(2-(4-((2-bromopyridin-4-yl)oxy)phenyl)propan-2-yl)phenoxy)cyclobutyl)carbamate (125 mg, 0.22 mmol) was dissolved in 3 mL of toluene, to which were successivle added anhydrous potassium phosphate and 2*H-*triazole, followed by addition of pre-catalyst under argon gas protection, and then the mixture was allowed to react overnight at 120 °C under argon gas protection. The reaction solution was cooled to room temperature, to which were added 15 mL of ethyl acetate and 12 mL of water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl ((1r,3r)-3-(4-(2-(4-(2-(2H-1,2,3-triazol-2-yl)pyridin-4-yl)oxy) phenyl)propan-2-yl)phenoxy)cyclobutanecarbamate (65 mg) and tert-butyl ((1r,3r)-3-(4-(2-(4-(2-(1H-1,2,3-triazol-2-yl)pyridin-4-yl)oxy)phenyl)propan-2-yl)phenoxy) cyclobutanecarbamate (45 mg), with an overall yield of 90%. LC/MS (ESI+) calcd for C₃₁H₃₅N₅O₄ ([M+H]⁺) *m*/*z* 542.2, found 542.2.

### Step 3: Synthesis of 5-(((1r,3r)-3-(4-(2-(4-((2-(1H-1,2,3-triazol-2-yl)pyridin-4-yl)oxy)phenyl)propan-2-ylphenoxy)cyclobutyl)amino)-2-(2,6-dioxopiperidin-3-yl)isoindolin-1,3-dione

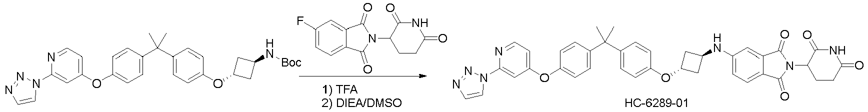

tert-butyl ((1r,3r)-3-(4-(2-(4-(2-(1H-1,2,3-triazol-2-yl)pyridin-4-yl)oxy)phenyl) propan-2-yl)phenoxy)cyclobutanecarbamate (45 mg, 0.083mmol) was dissolved in 4 mL of dichloromethane, to which was added 1.5 mL of trifluoroacetic acid in the ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 5 drops of N,N-diisopropylethylamine and 2-(2,6-dioxopiperidin-3-yl)-5-fluoroisoindolin-1,3-dione (30 mg, 0.11 mmol), and then the mixture was allowed to react overnight at 93 °C. The reaction solution was cooled, extracted with dichloromethane/water, dried, and concentrated to dry. The residue was separated and purified by TLC, to provide the product 5-(((1r,3r)-3-(4-(2-(4-((2-(1H-1,2,3-triazol-2-yl)pyridin-4-yl)oxy)phenyl) propan-2-ylphenoxy)cyclobutyl)amino)-2-(2,6-dioxopiperidin-3-yl)isoindolin-1,3-dione (20 mg), with a yield of 17%. LC/MS (ESI+) calcd for C₃₉H₃₅N₇O₆([M+H]⁺) *m*/*z* 698.2. ¹H NMR (400 MHz, Chloroform-*d*) *δ* 8.58 (s, 1H), 8.34 (d, *J* = 5.7 Hz, 1H), 8.04 (s, 1H), 7.81 (s, 1H), 7.69 (d, *J* = 2.3 Hz, 1H), 7.64 (d, *J* = 8.0 Hz, 1H), 7.30 (d, *J* = 8.6 Hz, 2H), 7.17 (d, *J* = 8.5 Hz, 2H), 7.06 - 7.00 (m, 2H), 6.93 (dd, *J* = 5.8, 2.3 Hz, 1H), 6.91 (s, 1H), 6.73 (t, *J* = 8.1 Hz, 3H), 4.93 (dd, *J* = 12.2, 5.2 Hz, 1H), 4.88 (s, 1H), 4.24 (s, 1H), 2.94 - 2.66 (m, 5H), 2.42 (s, 2H), 2.17 - 2.08 (m, 1H), 1.70 (s, 6H).

### Example 164: 5-(((1r,3r)-3-(4-(2-(4-((2-(2H-1,2,3-triazol-2-yl)pyridin-4-yl)oxy) phenyl)propan-2-ylphenoxy)cyclobutyl)amino)-2-(2,6-dioxopiperidin-3-yl) isoindolin-1,3-dione

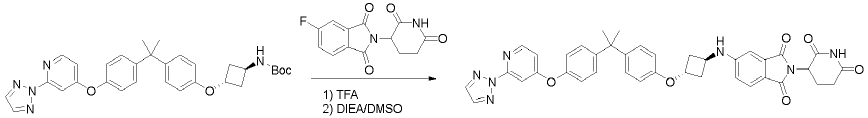

tert-butyl ((1r,3r)-3-(4-(2-(4-(2-(2H-1,2,3-triazol-2-yl)pyridin-4-yl)oxy)phenyl) propan-2-yl)phenoxy)cyclobutanecarbamate (65 mg, 0.12 mmol) was dissolved in 4 mL of dichloromethane, to which was added 1.5 mL of trifluoroacetic acid in an ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 5 drops of N,N-diisopropylethylamine and 2-(2,6-dioxopiperidin-3-yl)-5-fluoroisoindolin-1,3-dione (40 mg, 0.14 mmol), and then the mixture was allowed to react overnight at 93 °C. The reaction solution was cooled, extracted with dichloromethane/water, dried, and concentrated to dry. The residue was separated and purified by TLC, to provide the product 5-(((1r,3r)-3-(4-(2-(4-((2-(2H-1,2,3-triazol-2-yl)pyridin-4-yl)oxy)phenyl) propan-2-ylphenoxy)cyclobutyl)amino)-2-(2,6-dioxopiperidin-3-yl)isoindolin-1,3-dione 20 mg, with a yield of 24%. LC/MS (ESI+) calcd for C₃₉H₃₅N₇O₆ ([M+H]⁺) *m*/*z* 698.2. ¹H NMR (400 MHz, Chloroform-*d*) *δ* 8.43 (d, *J* = 5.7 Hz, 1H), 8.06 (s, 1H), 7.88 (d, *J* = 1.3 Hz, 2H), 7.66 - 7.59 (m, 2H), 7.30 (d, *J* = 8.2 Hz, 2H), 7.17 (d, *J* = 8.5 Hz, 2H), 7.04 (d, *J* = 8.3 Hz, 2H), 6.90 (d, *J* = 1.8 Hz, 1H), 6.88 - 6.83 (m, 1H), 6.77 - 6.67 (m, 3H), 4.95 - 4.91 (m, 1H), 4.88 (s, 1H), 4.25 (s, 1H), 2.94 - 2.65 (m, 5H), 2.48 - 2.36 (m, 2H), 2.12 (dd, *J* = 14.2, 6.7 Hz, 1H), 1.70 (s, 6H).

### Example 165: 5-(((1r,3r)-3-(4-(2-((4-(2H-1,2,3-triazol-2-yl)pyridin-2-yl)oxy) phenyl)propan-2-ylphenoxy)cyclobutyl)amino)-2-(2,6-dioxopyridin-3-yl)isoindol-1,3-dione

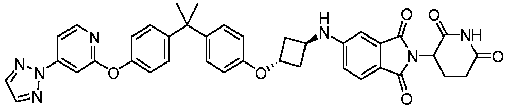

The target compound was synthesized by a method similar to that of Example 164. LC/MS (ESI+) calcd for C₃₉H₃₅N₇O₆([M+H]⁺) *m*/*z* 698.2, found 698.2. ¹H NMR (400 MHz, Chloroform-*d*) δ 8.92 (d, *J* = 2.7 Hz, 1H), 8.35 (dd, *J* = 8.9, 2.8 Hz, 1H), 8.07 (s, 1H), 7.83 (s, 2H), 7.62 (d, *J* = 8.3 Hz, 1H), 7.28 (d, *J* = 2.1 Hz, 1H), 7.26 (s, 1H), 7.20 - 7.15 (m, 2H), 7.09 - 7.04 (m, 2H), 7.01 (d, *J* = 8.9 Hz, 1H), 6.89 (d, *J* = 2.1 Hz, 1H), 6.71 (dq, *J* = 10.4, 2.7, 2.2 Hz, 3H), 4.93 (dd, *J* = 12.1, 5.2 Hz, 1H), 4.90 - 4.83 (m, 1H), 4.22 (td, *J* = 7.7, 3.8 Hz, 1H), 2.98 - 2.57 (m, 6H), 2.40 (dt, *J* = 12.8, 6.3 Hz, 2H), 2.12 (ddd, *J* = 10.4, 5.0, 2.7 Hz, 1H), 1.68 (s, 6H).

### Example 166: 2-(2,6-dioxopiperidin-3-yl)-5-((1r,3r)-3-(4-(2-(4-(4-(5-methyl-1,2,4-oxadiazol-3-yl)pyrimidin-2-yl)oxy)phenyl)propan-2-yl)phenoxy)cyclobutyl) amino)isoindolin-1,3-dione

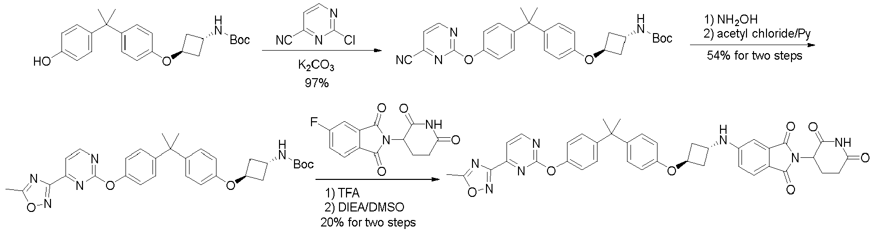

### Step 1: Synthesis of tert-butyl ((1r,3r)-3-(4-(2-(4-((4-cyanopyrimidin-2-yl)oxy)phenyl)propan-2-yl)phenoxy)cyclobutyl)carbamate

tert-butyl ((1r,3r)-3-(4-(2-(4-hydroxylphenyl)propan-2-phenoxy)cyclobutyl) carbamate (320 mg, 0.8 mmol) was dissolved in 5 mL DMF, to which was added anhydrous K₂CO₃ (221 mg, 1.6 mmol), followed by addition of 2-fluoro-4-cyanopyrimidine (140 mg, 1.0 mmol) under stirring, and then the mixture was allowed to react for 5 h at 66 °C. The reaction solution was cooled to room temperature, to which were added 15 mL of ethyl acetate and 12 mL of water. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product tert-butyl ((1r,3r)-3-(4-(2-(4-((4-cyanopyrimidin-2-yl)oxy)phenyl)propan-2-yl)phenoxy)cyclobutyl) carbamate (390 mg), with a yield of 97%. LC/MS (ESI+) calcd for C₂₉H₃₂N₄O₄ ([M+H]⁺) *m*/*z* 501.2, found 501.2.

### Step 2: Synthesis of tert-butyl ((1r,3r)-3-(4-(2-(4-((4-(5-methyl-1,2,4-oxadiazol-3-yl)pyrimidin-2-yl)oxy)phenyl)propan-2-yl)phenoxy)cyclobutyl)carbamate

tert-butyl ((1r,3r)-3-(4-(2-(4-((4-cyanopyrimidin-2-yl)oxy)phenyl)propan-2-yl) phenoxy)cyclobutyl)carbamate (390 mg, 0.78 mmol) was dissolved in 5 mL of ethanol, to which was added 50% hydroxylamine aqueous solution (106 mg, 1.6 mmol), and then the reaction solution was refluxed for 1h and cooled to room temperature, followed by filtration. The filter cake was rinsed with a small amount of ethyl acetate and dried, to provide the product as white solid, which was dissolved in 3 mL of pyridine. Then, acetyl chloride (173 mg, 2.2 mmol) was added dropwise in an ice-water bath. Subsequently, the reaction solution was stirred for 10 min, and the ice-water bath was removed. The mixture was heated to 105 °C, react overnight, and concentrated to dry under reduced pressure. The residue was separated and purified by TLC, to provide the product tert-butyl ((1r,3r)-3-(4-(2-(4-((4-(5-methyl-1,2,4-oxadiazol-3-yl)pyrimidin-2-yl)oxy)phenyl)propan-2-yl)phenoxy)cyclobutyl)carbamate (235 mg), with a yield of 54%. LC/MS (ESI+) calcd for C₃₁H₃₅N₅O₅ ([M+H]⁺) *m*/*z* 558.2, found 558.2.

### Step 3: Synthesis of 2-(2,6-dioxopiperidin-3-yl)-5-((1r,3r)-3-(4-(2-(4-(4-(5-methyl-1,2,4-oxadiazol-3-yl)pyrimidin-2-yl)oxy)phenyl)propan-2-yl)phenoxy)cyclobutyl) amino)isoindolin-1,3-dione

tert-butyl ((1r,3r)-3-(4-(2-(4-((4-(5-methyl-1,2,4-oxadiazol-3-yl)pyrimidin-2-yl) oxy)phenyl)propan-2-yl)phenoxy)cyclobutyl)carbamate (230 mg, 0.42 mmol) was dissolved in 4 mL of dichloromethane, to which was added 1.5 mL of trifluoroacetic acid in an ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 8 drops of N,N-diisopropylethylamine and 2-(2,6-dioxopiperidin-3-yl)-5-fluoroisoindolin-1,3-dione (152 mg, 0.55 mmol), and then the mixture was allowed to react overnight at 93 °C. The reaction solution was cooled, extracted with dichloromethane/water, dried, and concentrated to dry. The residue was separated and purified by TLC, to provide the product 2-(2,6-dioxopiperidin-3-yl)-5-((1r,3r)-3-(4-(2-(4-(4-(5-methyl-1,2,4-oxadiazol-3-yl)pyrimidin-2-yl)oxy)phenyl)propan-2-yl) phenoxy)cyclobutyl)amino)isoindolin-1,3-dione (60 mg), with a yield of 20%. LC/MS (ESI+) calcd for C₃₉H₃₅N₇O₇ ([M+H]⁺) *m*/*z* 714.2, found 714.2; ¹H NMR (400 MHz, Chloroform-*d*) *δ* 8.69 (d, *J* = 4.8 Hz, 1H), 8.01 (s, 1H), 7.74 (d, *J* = 4.7 Hz, 1H), 7.63 (d, *J* = 8.0 Hz, 1H), 7.28 (d, *J* = 8.4 Hz, 2H), 7.16 (dd, *J* = 16.4, 8.4 Hz, 4H), 6.90 (s, 1H), 6.71 (d, *J* = 8.2 Hz, 3H), 4.93 (dd, *J* = 12.0, 5.2 Hz, 1H), 4.87 (s, 1H), 4.24 (s, 1H), 2.93 - 2.72 (m, 5H), 2.72 (s, 3H), 2.41 (d, *J* = 10.1 Hz, 2H), 2.16 - 2.08 (m, 1H), 1.69 (s, 6H).

### Example 167: 2-(2,6-dioxopyridin-3-yl)-5-(((1r,3r)-3-(4-(2-((4-(2-(5-methyl-1,2,4-oxadiazol-3-yl)pyridin-4-yl)oxy)phenyl)propan-2-yl)phenoxy)cyclobutyl)amino) isoindolin-1,3-dione

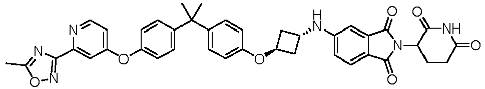

The target compound was synthesized by a method similar to that of Example 166. LC/MS (ESI+) calcd for C₄₀H₃₅N₆O₇([M+H]⁺) *m*/*z* 713.2, found 713.2. ¹H NMR (400 MHz, Chloroform-*d*) *δ* 8.62 (d, *J* = 5.2 Hz, 1H), 8.14 (s, 1H), 7.68 (d, *J* = 2.0 Hz, 1H), 7.62 (d, *J* = 8.2 Hz, 1H), 7.29 (d, *J* = 8.5 Hz, 2H), 7.17 (d, *J* = 8.4 Hz, 2H), 7.01 (d, *J* = 8.4 Hz, 2H), 6.95 (d, *J* = 4.9 Hz, 1H), 6.88 (d, *J* = 2.0 Hz, 1H), 6.71 (dd, *J* = 11.6, 7.5 Hz, 3H), 4.93 (dd, *J* = 12.2, 5.3 Hz, 1H), 4.88 (s, 1H), 4.22 (d, *J* = 4.9 Hz, 1H), 2.95 - 2.69 (m, 5H), 2.68 (s, 3H), 2.41 (dt, *J* = 12.8, 6.0 Hz, 2H), 2.17 - 2.08 (m, 1H), 1.69 (s, 6H).

### Example 168: 5-(((1r,3r)-3-(4-(2-(4-((4-(2H-1,2,3-triazol-2-yl)pyridin-2-yl)oxy) phenyl)propan-2-ylphenoxy)cyclobutyl)amino)-2-(2,6-dioxopyridin-3-yl) isoindolin-1,3-dione

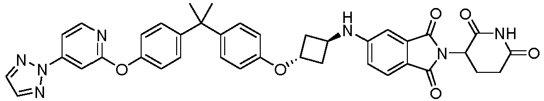

The target compound was synthesized by a method similar to that of Example 164. LC/MS (ESI+) calcd for C₃₉H₃₅N₇O₆ ([M+H]⁺) *m*/*z* 698.2, found 698.2. ¹H NMR (400 MHz, Chloroform-*d*) *δ* 8.31 (d, *J* = 5.6 Hz, 1H), 7.99 (s, 1H), 7.89 (s, 2H), 7.78 - 7.72 (m, 1H), 7.63 (d, *J=* 8.3 Hz, 1H), 7.59 (d, *J* = 1.7 Hz, 1H), 7.29 (s, 1H), 7.18 (d, *J* = 8.7 Hz, 2H), 7.08 (d, *J* = 8.5 Hz, 2H), 6.90 (d, *J* = 2.1 Hz, 1H), 6.71 (dd, *J* = 8.6, 2.0 Hz, 3H), 4.93 (dd, *J* = 12.1, 5.2 Hz, 1H), 4.87 (s, 1H), 4.23 (d, *J* = 4.8 Hz, 1H), 2.93 - 2.78 (m, 2H), 2.78 - 2.65 (m, 3H), 2.41 (dt, *J* = 12.9, 6.2 Hz, 2H), 2.15 - 2.09 (m, 1H), 1.69 (s, 6H).

### Example 169: 5-(((1r,3r)-3-(4-(2-(4-((4-(1H-1,2,3-triazol-1-yl)pyridin-2-yl)oxy) phenyl)propan-2-ylphenoxy)cyclobutyl)amino)-2-(2,6-dioxopyridin-3-yl) isoindolin-1,3-dione

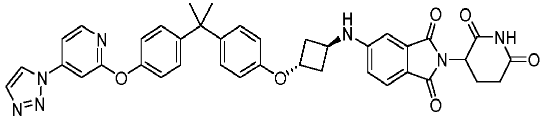

The target compound was synthesized by a method similar to that of Example 163. LC/MS (ESI+) calcd for C₃₉H₃₅N₇O₆ ([M+H]⁺) *m*/*z* 698.2, found 698.2. ¹H NMR (400 MHz, Chloroform-*d) δ* 8.34 (d, *J* = 5.2 Hz, 1H), 8.09 (s, 1H), 8.01 (s, 1H), 7.89 (s, 1H), 7.63 (d, *J* = 8.3 Hz, 1H), 7.44 (d, *J* = 5.1 Hz, 1H), 7.34 (s, 1H), 7.28 (d, *J* = 8.6 Hz, 2H), 7.18 (d, *J* = 8.7 Hz, 2H), 7.08 (d, *J* = 8.4 Hz, 2H), 6.90 (d, *J* = 2.0 Hz, 1H), 6.72 (d, *J* = 8.6 Hz, 3H), 4.93 (dd, *J* = 12.1, 5.2 Hz, 1H), 4.88 (s, 1H), 4.23 (d, *J* = 5.2 Hz, 1H), 2.94 - 2.74 (m, 3H), 2.74 - 2.66 (m, 2H), 2.41 (dt, *J* = 12.6, 6.1 Hz, 2H), 2.16 - 2.08 (m, 1H), 1.69 (s, 6H).

### Example 170: 5-(((1r,3r)-3-(4-(2-(4-((4-(2H-1,2,3-triazol-2-yl)pyrimidin-2-yl)oxy) phenyl)propan-2-yl)phenoxy)cyclobutyl)amino)-2-(2,6-dioxopiperidin-3-yl) isoindol-1,3-dione

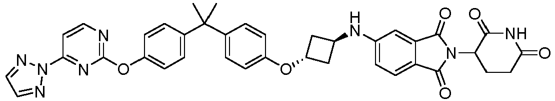

The target compound was synthesized by a method similar to that of Example 164. LC/MS (ESI+) calcd for C₃₈H₃₄N₈O₆ ([M+H]⁺) *m*/*z* 699.2, found 699.2. ¹H NMR (400 MHz, Chloroform-*d) δ* 8.62 (d, *J* = 5.4 Hz, 1H), 8.06 (s, 1H), 7.97 (s, 2H), 7.74 (d, *J* = 5.5 Hz, 1H), 7.63 (d, *J* = 8.3 Hz, 1H), 7.31 - 7.27 (m, 2H), 7.21 - 7.11 (m, 4H), 6.90 (d, *J* = 2.1 Hz, 1H), 6.75 - 6.68 (m, 3H), 4.93 (dd, *J* = 12.2, 5.2 Hz, 1H), 4.87 (d, *J* = 5.1 Hz, 1H), 4.27 - 4.19 (m, 1H), 2.93 - 2.67 (m, 5H), 2.41 (dt, *J* = 12.8, 6.0 Hz, 2H), 2.16 - 2.08 (m, 1H), 1.69 (s, 6H).

### Example 171: 2-(2,6-dioxopiperidin-3-yl)-5-(((1r,3r)-3-(4-(2-(4-(4-(5-methyl-1,3,4-oxadiazol-2-yl)pyrimidin-2-yl)oxy)phenyl)propan-2-yl)phenoxy)cyclobutyl) amino)isoindolin-1,3-dione

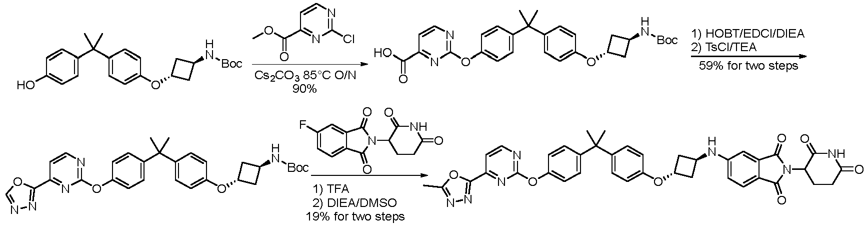

### Step 1: Synthesis of 2-(4-(2-(4-((1r,3r)-3-((tert-butoxycarbonyl)amino)cyclobutyloxy) phenyl)propan-2-yl)phenoxy)pyrimidin-4-carboxylic acid

tert-butyl ((1r,3r)-3-(4-(2-(4-hydroxylphenyl)propan-2-phenoxy)cyclobutyl) carbamate (300 mg, 0.75 mmol) was dissolved in 5 mL of DMF, to which was added anhydrous cesium carbonate (500 mg, 1.5 mmol), followed by addition of methyl 2-chloropyrimidin-4-carboxylate (130 mg, 0.75 mmol) under stirring, and then the mixture was allowed to react overnight at 85 °C. The reaction solution was cooled to room temperature, and the pH was adjusted to be 5.0 with citric acid aqueous solution, followed by addition of 15 mL ethyl acetate. The resultant solution was stirred and extracted. The water layer was re-extracted with 10 mL of ethyl acetate. The organic layers were combined, successively washed with water and saturated brine, dried with anhydrous Na₂SO₄, and concentrated to dry under reduced pressure. The residue was separated and purified by TLC, to provide the product 2-(4-(2-(4-((1r,3r)-3-((tert-butoxycarbonyl)amino)cyclobutyloxy)phenyl)propan-2-yl)phenoxy)pyrimidin-4-carboxylic acid (350 mg), with a yield of 90%.

### Step 2: Synthesis of tert-butyl ((1r,3r)-3-(4-(2-(4-((4-(5-methyl-1,3,4-oxadiazol-2-yl)pyrimidin-2-yl)oxy)phenyl)propan-2-yl)phenoxy)cyclobutyl)carbamate

2-(4-(2-(4-((1r,3r)-3-((tert-butoxycarbonyl)amino)cyclobutyloxy)phenyl)propan-2-yl)phenoxy)pyrimidin-4-carboxylic acid (350 mg, 0.61 mmol) was dissolved in 10 mL of dichloromethane, to which was added N,N-diisopropylethylamine (142 mg, 1.1 mmol), followed by addition of HOBT (119 mg, 0.88 mmol) in an ice-water bath, and then the mixture was stirred for 5 min. Acethydrazide (65 mg, 0.88 mmol) and EDCI (210 mg, 1.1 mmol) were added, and then the mixture was allowed to react overnight at room temperature, followed by washing with water, drying over anhydrous MgSO₄, and concentrating to dry. The residue was separated and purified by TLC, to provide the product, which was dissolved in 10 mL of dichloromethane, followed by addition of triethylamine (122 mg, 1.22 mmol) and p-toluenesulfonyl chloride (139 mg, 0.73mmol), and then the mixture was allowed to react overnight at room temperature. The reaction solution was washed with water, and then separated and purified by TLC, to provide the product tert-butyl ((1r,3r)-3-(4-(2-(4-((4-(5-methyl-1,3,4-oxadiazol-2-yl)pyrimidin-2-yl)oxy)phenyl)propan-2-yl)phenoxy)cyclobutyl)carbamate (200 mg), with a yield of 59%. LC/MS (ESI+) calcd for C₃₁H₃₅N₅O₅ ([M+H]⁺) *m*/*z* 558.2, found 558.2.

### Step 3: Synthesis of 2-(2,6-dioxopiperidin-3-yl)-5-(((1r,3r)-3-(4-(2-(4-(4-(5-methyl-1,3,4-oxadiazol-2-yl)pyrimidin-2-yl)oxy)phenyl)propan-2-yl)phenoxy)cyclobutyl) amino)isoindolin-1,3-dione

tert-butyl ((1r,3r)-3-(4-(2-(4-((4-(5-methyl-1,3,4-oxadiazol-2-yl)pyrimidin-2-yl) oxy)phenyl)propan-2-yl)phenoxy)cyclobutyl)carbamate (200 mg, 0.37mmol) was dissolved in 4 mL of dichloromethane, to which was added 1.5 mL of trifluoroacetic acid in an ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 10 drops of N,N-diisopropylethylamine and 2-(2,6-dioxopiperidin-3-yl)-5-fluoroisoindolin-1,3-dione (111 mg, 0.40 mmol), and then the mixture was allowed to react overnight at 93 °C. The reaction solution was cooled, extracted with dichloromethane/water, dried, and concentrated to dry. The residue was separated and purified by TLC, to provide the product 2-(2,6-dioxopiperidin-3-yl)-5-(((1r,3r)-3-(4-(2-(4-(4-(5-methyl-1,3,4-oxadiazol-2-yl)pyrimidin-2-yl)oxy)phenyl)propan-2-yl) phenoxy)cyclobutyl)amino)isoindolin-1,3-dione (50 mg), with a yield of 19%. LC/MS (ESI+) calcd for C₃₉H₃₅N₇O₇ ([M+H]⁺) *m*/*z* 714.2. ¹H NMR (400 MHz, Chloroform-*d*) *δ* 8.71 (d, *J* = 5.0 Hz, 1H), 8.21 (s, 1H), 7.87 (d, *J* = 5.0 Hz, 1H), 7.62 (d, *J* = 8.3 Hz, 1H), 7.34 - 7.27 (m, 2H), 7.22 - 7.16 (m, 2H), 7.16 - 7.10 (m, 2H), 6.89 (d, *J* = 2.1 Hz, 1H), 6.77 - 6.66 (m, 3H), 4.93 (dd, *J* = 12.1, 5.3 Hz, 1H), 4.87 (dt, *J* = 6.8, 2.9 Hz, 1H), 4.23 (d, *J* = 9.4 Hz, 1H), 2.93 - 2.69 (m, 5H), 2.68 (s, 3H), 2.41 (dt, *J* = 12.6, 6.2 Hz, 2H), 2.16 - 2.08 (m, 1H), 1.69 (s, 6H).

### Example 172: 2-(2,6-dioxopyridin-3-yl)-5-(((1r,3r)-3-(4-(2-(4-((2-(5-methyl-1,3,4-oxadiazol-2-yl)pyridin-4-yl)oxy)phenyl)propan-2-yl)phenoxy)cyclobutyl)amino) isoindolin-1,3-dione

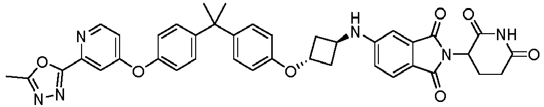

The target compound was synthesized by a method similar to that of Example 171. LC/MS (ESI+) calcd for C₄₀H₃₆N₆O₇ ([M+H]⁺) *m*/*z* 713.2, found 713.2. ¹H NMR (400 MHz, Chloroform-*d*) *δ* 8.60 (s, 1H), 8.05 (s, 1H), 7.76 (s, 1H), 7.64 (d, *J* = 8.1 Hz, 1H), 7.30 (d, *J* = 8.1 Hz, 2H), 7.17 (d, *J* = 8.4 Hz, 2H), 7.01 (d, *J* = 8.0 Hz, 3H), 6.90 (s, 1H), 6.73 (t, *J* = 7.9 Hz, 3H), 4.93 (dd, *J* = 12.2, 5.2 Hz, 1H), 4.89 (s, 1H), 4.24 (s, 1H), 2.94 - 2.68 (m, 6H), 2.66 (s, 3H), 2.42 (s, 2H), 2.12 (dd, *J* = 13.8, 6.6 Hz, 1H).

### Example 173: 2-(2,6-dioxopiperidin-3-yl)-5-(((1s,3s)-3-(4-(2-(4-((4-(5-methyl-1,2,4-oxadiazol-3-yl)pyrimidin-2-yl)oxy)phenyl)propan-2-yl)phenoxy)cyclobutyl) amino)isoindolin-1,3-dione

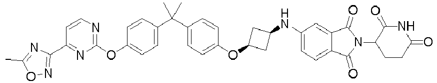

The target compound was synthesized by a method similar to that of Example 171. LC/MS (ESI+) calcd for C₃₉H₃₅N₇O₇ ([M+H]⁺) *m*/*z* 714.2, found 714.2. ¹H NMR (400 MHz, Chloroform-*d) δ* 8.69 (d, *J* = 4.9 Hz, 1H), 8.08 (s, 1H), 7.74 (d, *J* = 5.0 Hz, 1H), 7.62 (d, *J* = 8.3 Hz, 1H), 7.31 - 7.27 (m, 2H), 7.21 - 7.10 (m, 4H), 6.92 (d, *J* = 2.1 Hz, 1H), 6.78 - 6.69 (m, 3H), 5.30 (s, 1H), 4.93 (dd, *J* = 12.1, 5.2 Hz, 1H), 4.52 (p, *J* = 6.9 Hz, 1H), 3.78 (p, *J* = 7.6 Hz, 1H), 3.19 - 3.08 (m, 2H), 2.94 - 2.73 (m, 3H), 2.72 (s, 3H), 2.17 - 2.04 (m, 3H), 1.68 (s, 6H).

### Example 174: 2-(2,6-dioxopyridin-3-yl)-5-(((1s,3s)-3-(4-(2-(4-((2-(5-methyl-1,2,4-oxadiazol-3-yl)pyridin-4-yl)oxy)phenyl)propan-2-yl)phenoxy)cyclobutyl)amino) isoindolin-1,3-dione

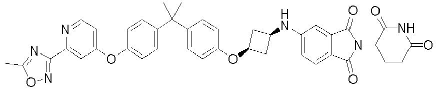

The target compound was synthesized by a method similar to that of Example 171. LC/MS (ESI+) calcd for C₄₀H₃₆N₆O₇ ([M+H]⁺) *m*/*z* 713.2, found 713.2. ¹H NMR (400 MHz, Chloroform-*d*) *δ* 8.61 (d, *J* = 5.6 Hz, 1H), 8.16 (s, 1H), 7.67 (d, *J* = 2.5 Hz, 1H), 7.62 (d, *J* = 8.3 Hz, 1H), 7.28 (d, *J* = 8.8 Hz, 2H), 7.20 - 7.13 (m, 2H), 7.03 - 6.98 (m, 2H), 6.94 (dd, *J* = 5.7, 2.5 Hz, 1H), 6.91 (d, *J* = 2.1 Hz, 1H), 6.78 - 6.74 (m, 2H), 6.72 (dd, *J* = 8.3, 2.2 Hz, 1H), 4.93 (dd, *J* = 12.2, 5.3 Hz, 1H), 4.80 (s, 1H), 4.53 (p, *J* = 6.8 Hz, 1H), 3.82 - 3.72 (m, 1H), 3.18 - 3.08 (m, 2H), 2.91 - 2.72 (m, 3H), 2.68 (s, 3H), 2.12 (ddt, *J* = 9.1, 6.7, 3.2 Hz, 3H), 1.69 (s, 6H).

### Example 175: 2-(2,6-dioxopiperidin-3-yl)-5-(((1r,3r)-3-(4-(3-(4-((6-(1-hydroxylethyl)pyridazine-3-yl)oxy)phenyl)pentan-3-yl)phenoxy)cyclobutyl) amino)isoindolin-1,3-dione

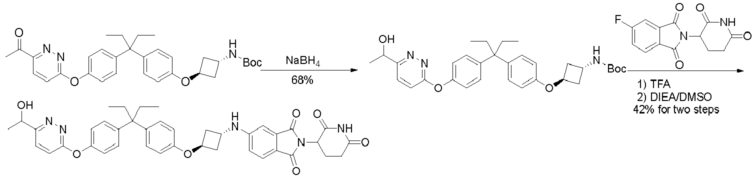

### Step 1: Synthesis of tert-butyl ((1r,3r)-3-(4-(3-(4-((6-(1-hydroxylethyl)pyridazine-3-yl)oxy)phenyl)pentan-3-yl)phenoxy)cyclobutyl)carbamate

tert-butyl ((1r,3r)-3-(4-(3-(4-((6-acetylpyridazine-3-yl)oxy)phenyl)pentan-3-yl) phenoxy)cyclobutyl)carbamate (320 mg, 0.59 mmol) was dissolved in 5 mL of anhydrous methanol, to which was added sodium borohydride (45 mg, 1.2 mmol) in an ice-water bath, and then the mixture was allowed to react for 20 min in the ice-water bath, followed by addition of 25 mL ethyl acetate and 20 mL water. The resultant solution was stirred and extracted. The water layer was re-extracted with 20 mL of ethyl acetate. The organic layers were combined, successively washed with 10 mL of saturated brine, dried with anhydrous Na₂SO₄, and concentrated to dry under reduced pressure. The residue was separated and purified by TLC, to provide the product tert-butyl ((1r,3r)-3-(4-(3-(4-((6-(1-hydroxylethyl)pyridazine-3-yl)oxy)phenyl)pentan-3-yl) phenoxy)cyclobutyl)carbamate (220 mg), with a yield of 68%. LC/MS (ESI+) calcd for C₃₂H₄₁N₃O₅ ([M+H]⁺) *m*/*z* 548.3, found 548.3.

### Step 2: Synthesis of 2-(2,6-dioxopiperidin-3-yl)-5-(((1r,3r)-3-(4-(3-(4-((6-(1-hydroxylethyl)pyridazine-3-yl)oxy)phenyl)pentan-3-yl)phenoxy)cyclobutyl)amino) isoindolin-1,3-dione

tert-butyl ((1r,3r)-3-(4-(3-(4-((6-(1-hydroxylethyl)pyridazine-3-yl)oxy)phenyl) pentan-3-yl)phenoxy)cyclobutyl)carbamate (220 mg, 0.40 mmol) was dissolved in 4 mL of dichloromethane, to which was added 1.5 mL of trifluoroacetic acid in an ice-water bath, and then the mixture was allowed to react in the ice-water bath for 1h. The pH of the reaction solution was adjusted to alkaline with saturated Na₂CO₃ aqueous solution, followed by extraction with dichloromethane/methanol (10:1). The organic phase was dried over anhydrous MgSO₄, and concentrated to dry under reduced pressure. The residue was dissolved in 5 mL of DMSO, to which were added 15 drops of N,N-diisopropylethylamine and 2-(2,6-dioxopiperidin-3-yl)-5-fluoroisoindolin-1,3-dione (170 mg, 0.6 mmol), and then the mixture was allowed to react overnight at 94 °C. The reaction solution was cooled, extracted with dichloromethane/water, dried, and concentrated to dry. The residue was separated and purified by TLC, to provide the product 2-(2,6-dioxopiperidin-3-yl)-5-(((1r,3r)-3-(4-(3-(4-((6-(1-hydroxylethyl) pyridazine-3-yl)oxy)phenyl)pentan-3-yl)phenoxy)cyclobutyl)amino)isoindolin-1,3-dione (120 mg), with a yield of 42%. LC/MS (ESI+) calcd for C₄₀H₄₁N₅O₇ ([M+H]⁺) *m*/*z* 704.3, found 704.3; ¹H NMR (400 MHz, Chloroform-*d*) *δ* 8.12 (s, 1H), 7.62 (dd, *J* = 8.3, 4.1 Hz, 2H), 7.26 - 7.17 (m, 3H), 7.13 - 7.04 (m, 4H), 6.89 (d, *J* = 2.1 Hz, 1H), 6.74 - 6.65 (m, 3H), 5.14 (d, *J* = 6.7 Hz, 1H), 4.93 (dd, *J* = 12.1, 5.3 Hz, 1H), 4.86 (s, 1H), 4.20 (d, *J* = 12.6 Hz, 1H), 2.93 - 2.74 (m, 3H), 2.73 - 2.67 (m, 2H), 2.41 (dd, *J* = 12.8, 6.5 Hz, 2H), 2.08 (q, *J* = 7.4 Hz, 6H), 1.57 (d, *J* = 6.4 Hz, 3H), 0.64 (t, *J* = 7.2 Hz, 6H).

### Example 176: 5-(((1r,3r)-3-(4-(3-(4-((6-acetylpyridazine-3-yl)oxy)phenyl)pentan-3-yl)phenoxy)cyclobutyl)amino)-2-(2,6-dioxopiperidin-3-yl)isoindolin-1,3-dione

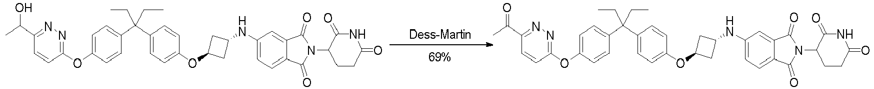

2-(2,6-dioxopiperidin-3-yl)-5-(((1r,3r)-3-(4-(3-(4-((6-(1-hydroxylethyl) pyridazine-3-yl)oxy)phenyl)pentan-3-yl)phenoxy)cyclobutyl)amino)isoindolin-1,3-dione (55 mg, 0.078 mmol) was dissolved in 5 mL of anhydrous dichloromethane, to which was added Dess-Martin periodinane (40 mg, 0.094 mmol), and then the reaction was stirred overnight at room temperature and filtered. The filtrate was concentrated to dry under reduced pressure. The residue was separated and purified by TLC, to provide the product 5-(((1r,3r)-3-(4-(3-(4-((6-acetylpyridazine-3-yl)oxy)phenyl)pentan-3-yl)phenoxy)cyclobutyl)amino)-2-(2,6-dioxopiperidin-3-yl)isoindolin-1,3-dione (38 mg), with a yield of 69%. LC/MS (ESI+) calcd for C₄₀H₃₉N₅0₇([M+H]⁺) *m*/*z* 702.3; ¹H NMR (400 MHz, Chloroform-*d*) *δ* 8.15 (d, *J =* 9.1 Hz, 1H), 8.12 (s, 1H), 7.63 (d, *J* = 8.3 Hz, 1H), 7.26 - 7.22 (m, 3H), 7.16 - 7.07 (m, 4H), 6.90 (d, *J* = 1.9 Hz, 1H), 6.71 (td, *J* = 5.7, 2.9 Hz, 3H), 4.93 (dd, *J* = 12.1, 5.2 Hz, 1H), 4.88 (d, *J* = 4.2 Hz, 1H), 4.29 - 4.20 (m, 1H), 2.93 - 2.82 (m, 2H), 2.81 (s, 3H), 2.76 (d, *J* = 5.3 Hz, 1H), 2.74 - 2.67 (m, 2H), 2.41 (dt, *J* = 12.8, 6.3 Hz, 2H), 2.09 (q, *J* = 7.5 Hz, 6H), 0.65 (t, *J* = 7.2 Hz, 6H).

### Example 177: 5-(((1r,3r)-3-(4-(1-(4-((5-acetylpyrazin-2-yl)oxy)phenyl)cyclopentyl) phenoxy)cyclobutyl)amino)-2-(2,6-dioxopiperidin-3-yl)isoindolin-1,3-dione

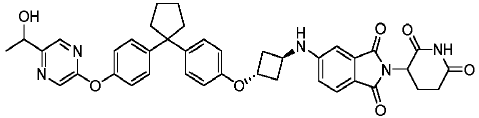

The target compound was synthesized by a method similar to that of Example 175. LC/MS (ESI+) calcd for C₄₀H₃₇N₅O₇ ([M+H]⁺) *m*/*z* 700.3, found 700.3. ¹H NMR (400 MHz, Chloroform-*d*) δ 8.78 (d, *J* = 1.3 Hz, 1H), 8.36 (d, *J* = 1.4 Hz, 1H), 8.11 (s, 1H), 7.63 (d, *J* = 8.3 Hz, 1H), 7.36 - 7.29 (m, 2H), 7.23 - 7.16 (m, 2H), 7.08 - 7.01 (m, 2H), 6.89 (d, *J* = 2.1 Hz, 1H), 6.70 (dd, *J* = 8.6, 2.3 Hz, 3H), 4.96 - 4.91 (m, 1H), 4.87 - 4.84 (m, 1H), 4.27 - 4.18 (m, 1H), 2.93 - 2.74 (m, 3H), 2.74 - 2.71 (m, 1H), 2.69 (d, *J* = 4.3 Hz, 1H), 2.67 (s, 3H), 2.43 - 2.35 (m, 2H), 2.27 (t, *J* = 10.0 Hz, 4H), 2.16 - 2.08 (m, 1H), 1.74 - 1.70 (m, 4H).

### Example 178: 2-(2,6-dioxopiperidin-3-yl)-5-(((1r,3r)-3-(4-(1-(4-((5-(1-hydroxylethyl)pyrazin-2-yl)oxy)phenyl)cyclopentyl)phenoxy)cyclobutyl)amino) isoindolin-1,3-dione

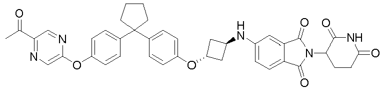

The target compound was synthesized by a method similar to that of Example 176. LC/MS (ESI+) calcd for C₄₀H₃₉N₅O₇ ([M+H]⁺) *m*/*z* 702.3, found 702.3. ¹H NMR (400 MHz, Chloroform-*d*) *δ* 8.29 (s, 1H), 8.13 (d, *J* = 19.4 Hz, 2H), 7.63 (d, *J* = 8.2 Hz, 1H), 7.33 - 7.27 (m, 2H), 7.19 (d, *J* = 8.7 Hz, 2H), 7.06 - 6.98 (m, 2H), 6.89 (d, *J* = 2.0 Hz, 1H), 6.69 (dd, *J* = 7.4, 4.7 Hz, 3H), 4.94 (dq, *J* = 11.3, 5.8, 5.2 Hz, 2H), 4.85 (s, 1H), 4.22 (s, 1H), 2.93 - 2.78 (m, 2H), 2.76 (d, *J* = 4.9 Hz, 1H), 2.70 (dd, *J* = 11.1, 4.5 Hz, 2H), 2.38 (dd, *J* = 13.3, 6.3 Hz, 2H), 2.28 (d, *J* = 5.8 Hz, 4H), 2.15 - 2.09 (m, 1H), 1.72 (s, 4H), 1.54 (d, *J* = 6.4 Hz, 3H).

### Example 179: 2-(2,6-dioxopiperidin-3-yl)-5-(1-(4-(2-(4-((2-(5-methyl-1,2,4-oxadiazol-3-yl)pyrimidin-5-yl)oxy)phenyl)propan-2-yl)phenyl)-1,6-diazaspiro[3.3] heptan-6-yl)isoindolin-1,3-dione

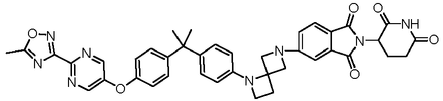

The target compound was synthesized by a method similar to that of Example 149. LC/MS (ESI+) calcd for C₄₀H₃₆N₈O₆ ([M+H]⁺) *m*/*z* 725.3, found 725.3. ¹H NMR (400 MHz, Chloroform-*d*) *δ* 8.59 (s, 2H), 8.09 (s, 1H), 7.69 (d, *J* = 8.2 Hz, 1H), 7.32 - 7.27 (m, 2H), 7.14 - 7.05 (m, 2H), 7.02 - 6.95 (m, 2H), 6.84 (d, *J* = 2.1 Hz, 1H), 6.59 (dd, *J* = 8.3, 2.2 Hz, 1H), 6.54 - 6.46 (m, 2H), 4.95 (dd, *J* = 12.2, 5.3 Hz, 1H), 4.70 (d, *J* = 9.4 Hz, 2H), 4.08 (d, *J* = 9.4 Hz, 2H), 3.80 (t, *J* = 6.8 Hz, 2H), 2.96 - 2.74 (m, 3H), 2.72 (s, 3H), 2.61 (t, *J* = 6.8 Hz, 2H), 2.17 - 2.10 (m, 1H), 1.66 (s, 6H).

### Example 180: 5-((4-(2-(4-(3-((2-(2,6-dioxopiperidin-3-yl)-1,3-dioxoisoindolin-5-yl)amino)propoxy)phenyl)propan-2-yl)phenoxy)methyl)-N,N-dimethyl-1,2,4-oxadiazol-3-carbonylamine

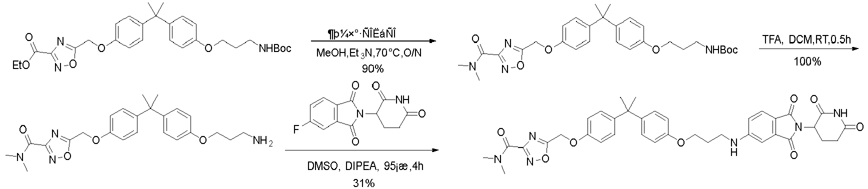

### Step 1: tert-butyl (3-(4-(2-(4-((3-(dimethylcarbamoyl)-1,2,4-oxadiazol-5-yl)methoxy)phenyl)propan-2-yl)phenoxy)propyl)carbamate

In a 100 mL round-bottom flask, ethyl 5-((4-(2-(4-(3-((tert-butoxycarbonyl)amino) propoxy)phenyl)propan-2-yl)phenoxy)methyl)-1,2,4-oxadiazol-3-carboxylate (200 mg, 0.37mmol) was dissolved in 30 mL of anhydrous methanol, to which were added triethylamine (4.0eq., 0.4mL, 1.50mmol) and dimethylamine hydrochloride (2.0eq., 62 mg, 0.74 mmol), and then the reaction solution was refluxed at 70 °C and stirred overnight. The reaction was directly rotatory evaporated to dry under reduced pressure. The residue was separated by column chromatography, to provide tert-butyl (3-(4-(2-(4-((3-(dimethylcarbamoyl)-1,2,4-oxadiazol-5-yl)methoxy)phenyl)propan-2-yl)phenoxy)propyl)carbamate (180 mg, 0.33 mmol, 90%). LC/MS (ESI+) calcd for C₂₉H₃₈N₄O₆ ([M+H]⁺) *m*/*z* 539.28, found 539.30.

### Step 2: Synthesis of 5-((4-(2-(4-(3-aminopropoxy)phenyl)propan-2-yl)phenoxy) methyl)-N,N-dimethyl-1,2,4-oxadiazol-3-carbonylamine

In a 50 mL round-bottom flask, tert-butyl (3-(4-(2-(4-((3-(dimethylcarbamoyl)-1,2,4-oxadiazol-5-yl)methoxy)phenyl)propan-2-yl)phenoxy)propyl)carbamate (180 mg, 0.33 mmol) was dissolved in 10 mL of DCM, to which was added 2 mL of TFA in an ice-water bath, and then stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated to dry. The residue was separated by TLC, to provide 5-((4-(2-(4-(3-aminopropoxy)phenyl)propan-2-yl)phenoxy)methyl)-N,N-dimethyl-1,2,4-oxadiazol-3-carbonylamine (180 mg, 0.36 mmol, 100%). LC/MS (ESI+) calcd for C₂₄H₃₀N₄O₄ ([M+H]⁺) *m*/*z* 439.23, found 439.20.

### Step 3: Synthesis of 5-((4-(2-(4-(3-((2-(2,6-dioxopiperidin-3-yl)-1,3-dioxoisoindolin-5-yl)amino)propoxy)phenyl)propan-2-yl)phenoxy)methyl)-N,N-dimethyl-1,2,4-oxadiazol-3-carbonylamine

In a 50 mL round-bottom flask, 5-((4-(2-(4-(3-aminopropoxy)phenyl)propan-2-yl)phenoxy)methyl)-N,N-dimethyl-1,2,4-oxadiazol-3-carbonylamine (100 mg, 0.23 mmol) was dissolved in 20 mLof anhydrous DMSO, to which were added DIPEA (4.0 eq., 0.15 mL, 0.92 mmol) and 2-(2,6-dioxo-piperidin-3-yl)-5-fluoro-isoindolin-1,3-dione (2.0 eq., 130 mg, 0.46 mmol), and then the system was purged with argon gas for three times, followed by reaction at 95 °C for 4h. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry. The residue was separated by TLC, to provide the target compound 5-((4-(2-(4-(3-((2-(2,6-dioxopiperidin-3-yl)-1,3-dioxoisoindolin-5-yl)amino)propoxy)phenyl)propan-2-yl)phenoxy)methyl)-N,N-dimethyl-1,2,4-oxadiazol-3-carbonylamine (50 mg, 0.072 mmol, 31%). ¹H NMR (400 MHz, DMSO-d₆) *δ* 11.06 (s, 1H), 7.56 (d, *J* = 8.4 Hz, 1H), 7.20 (t, *J* = 5.4 Hz, 1H), 7.12 (dd, *J* = 14.6, 8.8 Hz, 4H), 6.96 (dd, *J* = 10.0, 5.4 Hz, 3H), 6.86 (dd, *J* = 9.9, 5.4 Hz, 3H), 5.54 (s, 2H), 5.03 (dd, *J* = 12.9, 5.3 Hz, 1H), 4.01 (d, *J* = 7.2 Hz, 2H), 3.02 (s, 3H), 2.97 (s, 3H), 2.86 (td, *J* = 13.8, 7.0 Hz, 1H), 2.68 - 2.49 (m, 4H), 2.06 - 1.91 (m, 3H), 1.58 (s, 6H). LC/MS (ESI+) calcd for C₃₇H₃₈N₆O₈ ([M+H]⁺) *m*/*z* 694.28, found 694.20.

### Example 181: 5-((4-(2-(4-(3-((2-(2,6-dioxopiperidin-3-yl)-1,3-dioxoisoindolin-4-yl)amino)propoxy)phenyl)propan-2-yl)phenoxy)methyl)-N,N-dimethyl-1,2,4-oxadiazol-3-carbonylamine

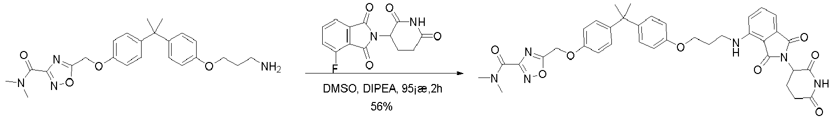

In a 50 mL round-bottom flask, 5-((4-(2-(4-(3-aminopropoxy)phenyl)propan-2-yl)phenoxy)methyl)-N,N-dimethyl-1,2,4-oxadiazol-3-carbonylamine (80 mg, 0.18 mmol) was dissolved in 20 mL of anhydrous DMSO, to which were added DIPEA (4.0 eq., 0.12 mL, 0.72 mmol) and 2-(2,6-dioxopiperidin-3-yl)-4-fluoroisoindolin-1,3-dione (2.0 eq., 100 mg, 0.36 mmol), and then the system was purged with argon gas for three times, followed by reaction at 95 °C for 2 h. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry. The residue was separated by TLC, to provide the target compound 5-((4-(2-(4-(3-((2-(2,6-dioxopiperidin-3-yl)-1,3-dioxoisoindolin-4-yl)amino)propoxy)phenyl)propan-2-yl)phenoxy)methyl)-N,N-dimethyl-1,2,4-oxadiazol-3-carbonylamine (70 mg, 0.10 mmol, 56%). ¹H NMR (400 MHz, CDCl₃) *δ* 8.29 (s, 1H), 7.45 (dt, *J* = 12.0, 6.0 Hz, 1H), 7.20 - 7.05 (m, 5H), 6.98 - 6.78 (m, 5H), 5.32 (s, 2H), 4.92 (dd, *J* = 11.8, 5.3 Hz, 1H), 4.07 (t, *J* = 5.6 Hz, 2H), 3.50 (s, 2H), 3.15 (d, *J* = 2.0 Hz, 6H), 2.92 - 2.65 (m, 4H), 2.22 - 2.06 (m, 3H), 1.63 (s, 6H). LC/MS (ESI+) calcd for C₃₇H₃₈N₆O₈ ([M+H]⁺) *m*/*z* 694.28, found 694.20.

### Example 182: 4-((4-(2-(4-(3-((2-(2,6-dioxopiperidin-3-yl)-1,3-dioxoisoindolin-5-yl)amino)propoxy)phenyl)propan-2-yl)phenoxy)methyl)pyrimidin-2-carbonylamine

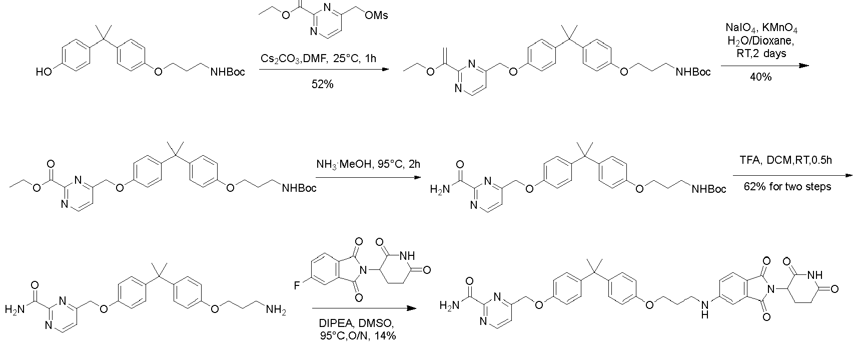

### Step 1: Synthesis of tert-butyl (3-(4-(2-(4-((2-(1-ethoxyvinyl)pyrimidin-4-yl)methoxy) phenyl)propan-2-yl)phenoxy)propyl)carbamate

In a 50 mL round-bottom flask, (2-(1-ethoxyvinyl)pyrimidin-4-yl)methanesulfonate(500mg, 1.94mmol) was dissolved in 20 mL of anhydrous DMF, to which was added tert-butyl (3-(4-(2-(4-hydroxylphenyl)propan-2-yl)phenoxy)propyl) carbamate (1.0 eq., 750 mg, 1.94 mmol), and then the mixture was allowed to dissolve before addition of cesium carbonate (2.0 eq., 1.26 g, 3.88 mmol). Subsequntly, the reaction solution was stirred at room temperature for 2h. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry. The residue was separated by column chromtography, to provide tert-butyl (3-(4-(2-(4-((2-(1-ethoxyvinyl)pyrimidin-4-yl)methoxy)phenyl)propan-2-yl)phenoxy)propyl)carbamate (550 mg, 1.01 mmol, 52%). LC/MS (ESI+) calcd for C₃₂H₄₁N₃O₅ ([M-55]) *m*/*z* 492.30, found 492.20.

### Step 2: Synthesis of ethyl 4-((4-(2-(4-(3-((tert-butoxycarbonyl)amino)propoxy)phenyl) propan-2-yl)phenoxy)methyl)pyrimidin-2-carboxylate

In a 50 mL round-bottom flask, tert-butyl (3-(4-(2-(4-((2-(1-ethoxyvinyl)pyrimidin-4-yl)methoxy)phenyl)propan-2-yl)phenoxy)propyl)carbamate (550 mg, 1.01 mmol) was dissolved in 1,4-dioxane/water (5/1), to which was added sodium periodate (2.0 eq., 440 mg, 2.02 mmol), followed by careful addition of potassium osmate dihydrate (0.1 eq., 10 mg, 0.01 mmol), and then the reaction solution was stirred at room temperature for two days. 20 mL of saturated sodium thiosulfate solution was added to quench the reaction, and the resultant solution was extracted with ethyl acetate (3*40 mL), followed by suction filtration. The filtrate was dried with anhydrous Na₂SO₄, and then rotatory evaporated to dry. The residue was separated by column chromtography, to provide ethyl 4-((4-(2-(4-(3-((tert-butoxycarbonyl)amino)propoxy)phenyl)propan-2-yl)phenoxy)methyl)pyrimidin-2-carboxylate (220 mg, 0.40 mmol, 40%). LC/MS (ESI+) calcd for C₃₁H₃₉N₃O₆ ([M-55]) *m*/*z* 494.28, found 494.20.

### Steps 3 and 4: Sythesis of 4-((4-(2-(4-(3-aminopropoxy)phenyl)propan-2-yl)phenoxy)methyl)pyrimidin-2-carbonylamine

In a 50 mL of sealed tube, ethyl 4-((4-(2-(4-(3-((tert-butoxycarbonyl)amino) propoxy)phenyl)propan-2-yl)phenoxy)methyl)pyrimidin-2-carboxylate (100 mg, 0.18 mmol) was added into ammonia/methanol solution (7.0 M, 10 mL), and then the solution was heated to 95 °C in the sealed tube and reacted for 2 h. The reaction solution was directly concentrated to dry under reduced pressure. The residue was separated by column chromatography, to provide the crude product tert-butyl (3-(4-(2-(4-((2-carbamoylpyrimidin-4-yl)methoxy)phenyl)propan-2-yl)phenoxy)propyl)carbamate, which was dissolved in 10 mL of anhydrous DCM, and then 2 mL of TFA was added in an ice-water bath. The resultant solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated to dry. The residue was separated by TLC, to provide 4-((4-(2-(4-(3-aminopropoxy)phenyl) propan-2-yl)phenoxy)methyl)pyrimidin-2-carbonylamine (47 mg, 0.11 mmol, 62% for two steps). LC/MS (ESI+) calcd for C₂₄H₂₈N₄O₃ ([M+H]⁺) *m*/*z* 421.22, found 421.20.

### Step 5: Synthesis of 4-((4-(2-(4-(3-((2-(2,6-dioxopiperidin-3-yl)-1,3-dioxoisoindolin-5-yl)amino)propoxy)phenyl)propan-2-yl)phenoxy)methyl)pyrimidin-2-carbonylamine

In a 50 mL round-bottom flask, 4-((4-(2-(4-(3-aminopropoxy)phenyl)propan-2-yl)phenoxy)methyl)pyrimidin-2-carbonylamine (47 mg, 0.11 mmol) was dissolved in 20 mL of anhydrous DMSO, to which were added DIPEA (4.0 eq., 0.07 mL, 0.44 mmol) and 2-(2,6-dioxopiperidin-3-yl)-4-fluoroisoindolin-1,3-dione (2.0 eq., 61 mg, 0.22 mmol), and then the system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry. The residue was separated by TLC, to provide the target compound 4-((4-(2-(4-(3-((2-(2,6-dioxopiperidin-3-yl)-1,3-dioxoisoindolin-5-yl)amino)propoxy) phenyl)propan-2-yl)phenoxy)methyl)pyrimidin-2-carbonylamine (10 mg, 0.015 mmol, 14%) as yellow solid. ¹H NMR (400 MHz, DMSO-d₆) *δ* 8.94 (d, *J* = 5.1 Hz, 1H), 8.19 (s, 1H), 7.84 (s, 1H), 7.70 (d, *J* = 5.0 Hz, 1H), 7.20 (s, 1H), 7.12 (dd, *J* = 12.2, 8.9 Hz, 4H), 7.05 - 6.90 (m, 3H), 6.84 (d, *J* = 8.8 Hz, 2H), 5.24 (s, 2H), 5.03 (d, *J* = 7.7 Hz, 1H), 4.03 (d, *J* = 6.3 Hz, 1H),2.86 (d, *J* = 19.7 Hz, 3H), 2.70 (d, *J* = 22.8 Hz, 3H), 2.00 (d, *J* = 7.4 Hz, 6H), 1.58 (s, 6H). LC/MS (ESI+) calcd for C₃₇H₃₆N₆O₇ ([M+H]⁺) *m*/*z* 677.26, found 677.10.

### Example 183: 4-((4-(2-(4-(3-((2-(2,6-dioxopiperidin-3-yl)-1,3-dioxoisoindolin-5-yl)amino)propoxy)phenyl)propan-2-yl)phenoxy)methyl)-N-methylpyrimidin-2-carbonylamine

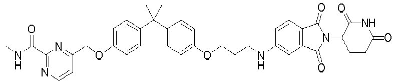

The target compound was synthesized by a method similar to that of Example 182. ¹H NMR (400 MHz, DMSO-d₆) *δ* 11.06 (s, 1H), 7.95 (s, 1H), 7.56 (d, *J* = 8.3 Hz, 1H), 7.22 (s, 1H), 7.11 (dd, *J* = 8.7, 5.6 Hz, 5H), 6.90 (dt, *J* = 17.3, 14.2 Hz, 7H), 5.13 (s, 2H), 5.05 - 4.98 (m, 1H), 4.36 (s, 2H), 4.03 (t, *J* = 6.0 Hz, 3H), 3.17 (s, 1H), 2.54 (s, 3H), 2.04 - 1.91 (m, 4H), 1.57 (s, 6H). LC/MS (ESI+) calcd for C₃₇H₃₆N₆O₇ ([M+H]⁺) *m*/*z* 677.26, found 677.10.

### Example 184: methyl 4-((4-(2-(4-(3-((2-(2,6-dioxopiperidin-3-yl)-1,3-dioxoisoindolin-5-yl)amino)propoxy)phenyl)propan-2-yl)phenoxy)pyrimidin-2-carboxylate

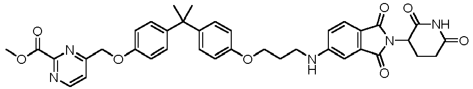

The target compound was synthesized by a method similar to that of Example 182. ¹H NMR (400 MHz, DMSO-*d*₆) *δ* 11.06 (s, 1H), 8.99 (d, *J* = 5.1 Hz, 1H), 7.79 (d, *J* = 5.1 Hz, 1H), 7.55 (d, *J* = 8.4 Hz, 1H), 7.21 (s, 1H), 7.12 (dd, *J* = 11.4, 8.8 Hz, 4H), 7.02 - 6.92 (m, 3H), 6.85 (t, *J=* 8.0 Hz, 3H), 5.26 (s, 2H), 5.03 (dd, *J* = 12.9, 5.2 Hz, 1H), 4.03 (t, *J* = 6.4 Hz, 2H), 3.92 (s, 3H), 2.86 (d, *J* = 17.9 Hz, 1H), 1.99 (t, *J* = 6.4 Hz, 4H), 1.58 (s, 6H), 1.23 (s, 3H). LC/MS (ESI+) calcd for C₃₈H₃₇N₅O₈ ([M+H]⁺) *m*/*z* 692.26, found 692.20.

### Example 185: 2-(2,6-dioxopiperidin-3-yl)-5-((3-(4-(2-(4-((4-(hydroxylmethyl) oxazol-2-yl)methoxy)phenyl)propan-2-yl)phenoxy)propyl)amino)isoindolin-1,3-dione

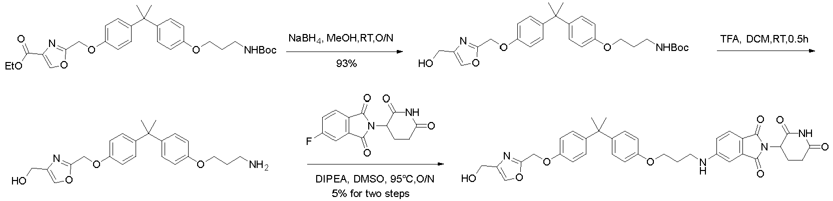

### Step 1: Synthesis of tert-butyl (3-(4-(2-(4-((4-(hydroxylmethyl)oxazol-2-yl)methoxy) phenyl)propan-2-yl)phenoxy)propyl)carbamate

In a 50 mL round-bottom flask, ethyl 2-((4-(2-(4-(3-((tert-butoxycarbonyl)amino) propoxy)phenyl)propan-2-yl)phenoxy)methyl)oxazol-4-carboxylate (200 mg, 0.37 mmol) was dissolved in 10 mL of anhydrous methanol, to which was added sodium borohydride (2.0 eq., 30 mg, 0.74 mmol) in an ice-water bath, and then the mixture was warmed to room temperature and reacted overnight. 20 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry. The residue was separated by column chromatography, to provide tert-butyl (3-(4-(2-(4-((4-(hydroxylmethyl)oxazol-2-yl)methoxy)phenyl)propan-2-yl)phenoxy)propyl) carbamate (170 mg, 0.34 mmol, 93%). LC/MS (ESI+) calcd for C₂₈H₃₆N₂O₆ ([M+H]⁺) *m*/*z* 497.26, found 497.20. Steps 2 and 3: Synthesis of 2-(2,6-dioxopiperidin-3-yl)-5-((3-(4-(2-(4-((4-(hydroxylmethyl)oxazol-2-yl)methoxy)phenyl)propan-2-yl)phenoxy)propyl)amino) isoindolin-1,3-dione

In a 50 mL round-bottom flask, tert-butyl (3-(4-(2-(4-((4-(hydroxylmethyl)oxazol-2-yl)methoxy)phenyl)propan-2-yl)phenoxy)propyl)carbamate (170 mg, 0.34 mmol) was dissolved in 10 mL of anhydrous DCM, to which was added 2 mL of TFA in an ice-water bath. The resultant solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry, to provide the crude product (2-((4-(2-(4-(3-aminopropoxy)phenyl)propan-2-yl)phenoxy)methyl)oxazol-4-yl)methanol, which (calculated at 0.34 mmol) was dissolved in 20 mL of anhydrous DMSO, followed by addition of DIPEA (4.0 eq., 0.22 mL, 1.36 mmol) and 2-(2,6-dioxopiperidin-3-yl)-4-fluoroisoindolin-1,3-dione (2.0 eq., 190 mg, 0.68 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry. The residue was separated by TLC, to provide the target compound 2-(2,6-dioxopiperidin-3-yl)-5-((3-(4-(2-(4-((4-(hydroxylmethyl)oxazol-2-yl)methoxy)phenyl)propan-2-yl)phenoxy) propyl)amino)isoindolin-1,3-dione (10 mg, 0.015 mmol, 5%). LC/MS (ESI+) calcd for C₃₆H₃₆N₄O₈ ([M+H]⁺) *m*/*z* 623.25, found 623.20.

### Example 186: 4-((4-(2-(4-((1r,3r)-3-((2-(2,6-dioxopiperidin-3-yl)-1,3-dioxoisoindolin-5-yl)amino)cyclobutyloxy)phenyl)propan-2-yl)phenoxy)methyl)-N-methylpyrimidin-2-carbonylamine

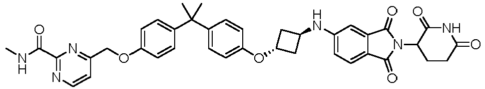

The target compound was synthesized by a method similar to that of Example 182. ¹H NMR (400 MHz, DMSO-d₆) *δ* 8.94 (d, *J* = 5.1 Hz, 1H), 8.86 (s, 1H), 7.69 (ddd, *J* = 19.1, 5.9, 3.3 Hz, 2H), 7.57 (dd, *J* = 17.8, 7.4 Hz, 2H), 7.22 (s, 1H), 7.13 (dd, *J* = 16.4, 8.8 Hz, 4H), 6.96 (dd, *J* = 16.2, 8.8 Hz, 2H), 6.73 (d, *J* = 8.7 Hz, 1H), 5.25 (s, 2H), 4.86 (m, 1H), 4.20 (dd, *J* = 24.4, 17.8 Hz, 1H), 4.16 - 4.07 (m, 1H), 3.45 (t, *J=* 9.2 Hz, 2H), 2.82 (d, *J=* 4.8 Hz, 4H), 1.99 (s, 2H), 1.70 - 1.61 (m, 2H), 1.57 (d, *J* = 9.2 Hz, 6H), 1.37 (dd, *J* = 16.5, 9.0 Hz, 3H). LC/MS (ESI+) calcd for C₃₉H₃₈N₆O₇ ([M+H]⁺) *m*/*z* 703.28, found 703.20.

### Example 187: 4-((4-(2-(4-((1s,3s)-3-((2-(2,6-dioxopiperidin-3-yl)-1,3-dioxoisoindolin-5-yl)amino)cyclobutyloxy)phenyl)propan-2-yl)phenoxy)methyl)-N-methylpyrimidin-2-carbonylamine

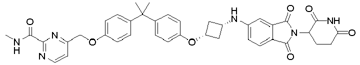

The target compound was synthesized by a method similar to that of Example 182. ¹H NMR (400 MHz, DMSO-d₆) *δ* 8.94 (d, *J* = 5.1 Hz, 1H), 8.84 (s, 1H), 7.52 (ddd, *J* = 19.1, 5.9, 3.3 Hz, 2H), 7.57 (dd, *J* = 17.8, 7.4 Hz, 2H), 7.23 (s, 1H), 7.13 (dd, *J* = 16.4, 8.8 Hz, 4H), 6.96 (dd, *J* = 16.2, 8.8 Hz, 2H), 6.69 (d, *J* = 8.7 Hz, 1H), 5.31 (s, 2H), 4.86 (m, 1H), 4.22 (dd, *J* = 24.4, 17.8 Hz, 1H), 4.13 - 4.05 (m, 1H), 3.48 (t, *J* = 9.2 Hz, 2H), 2.82 (d, *J* = 4.8 Hz, 4H), 2.03 (s, 2H), 1.70 - 1.61 (m, 2H), 1.57 (d, *J* = 9.2 Hz, 6H), 1.35 (dd, *J* = 16.5, 9.0 Hz, 3H). LC/MS (ESI+) calcd for C₃₉H₃₈N₆O₇ ([M+H]⁺) *m*/*z* 703.28, found 703.20.

### Example 188: 2-(2,6-dioxopiperidin-3-yl)-5-(3-(4-(2-(2-methoxy-5,8-dihydropyrido[3,4-d]pyrimidin-7(6H)-yl)phenyl)propan-2-yl)phenoxy)cyclobutyl) aminoisoindolin-1,3-dione

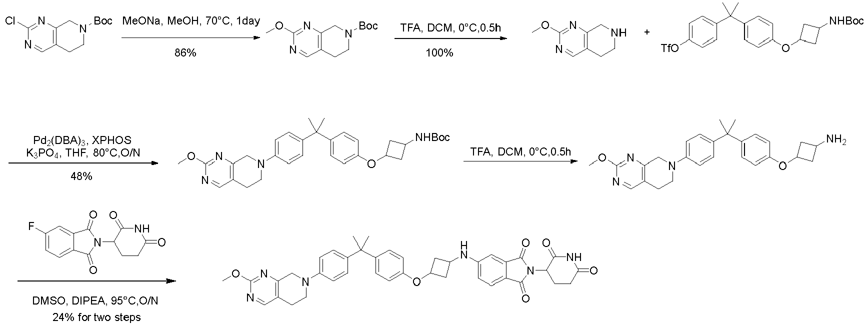

### Step 1: Synthesis of tert-butyl 2-methoxy-5,8-dihydropyrido[3,4-d]pyrimidin-7(6H)-carboxylate

In a 50 mL round-bottom flask, tert-butyl 2-chloro-5,8-dihydropyrido[3,4-d]pyrimidin-7(6H)-carboxylate (1.00 g, 3.72 mmol) was dissolved in 10 mL of anhydrous methanol, to which was added sodium methoxide (2.0 eq., 420 mg, 7.43 mmol), and then the reaction solution was allowed to react for one day under refluxing at 70 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated under reduced pressure to dry. The residue was separated by column chromatography, to provide tert-butyl 2-methoxy-5,8-dihydropyrido[3,4-d]pyrimidin-7(6H)-carboxylate (850 mg, 3.2 mmol, 86%). LC/MS (ESI+) calcd for C₁₃H₄₉N₃O₃ ([M+H]⁺) m/z 266.14, found 266.10.

### Step 2: Synthesis of 2-methoxy-5,6,7,8-tetrahydropyrido[3,4-d]pyrimidine

In a 50 mL round-bottom flask, tert-butyl 2-methoxy-5,8-dihydropyrido[3,4-d]pyrimidin-7(6H)-carboxylate (850 mg, 3.2 mmol) was dissolved in 10 mL of anhydrous DCM, to which was added 2 mL of TFA in an ice-water bath. The resultant solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide 2-methoxy-5,6,7,8-tetrahydropyrido[3,4-d]pyrimidine (700 mg, 4.20 mmol, 100%). LC/MS (ESI+) calcd for C₈HₙN₃O ([M+H]⁺) *m*/*z* 166.09, found 166.10.

### Step 3: Synthesis of tert-butyl (3-(4-(2-(4-(2-methoxy-5,8-dihydropyrido[3,4-d]pyrimidin-7(6H)-yl)phenyl)propan-2-yl)phenoxy)cyclobutyl)carbamate

In a 50 mL round-bottom flask, 2-methoxy-5,6,7,8-tetrahydropyrido[3,4-d]pyrimidine (1.2 eq., 800 mg, 0.45 mmol) and 4-(2-(4-(3-((tert-butoxycarbonyl)amino)cyclobutyloxy)phenyl)propan-2-yl)phenyl trifluoromethanesulfonate (200 mg, 0.38 mmol) was placed in a sealed tube, to which were added anhydrous THF (10 mL), potassium phosphate (2.0 eq., 160 mg, 0.76 mmol), tris(dibenzylideneacetone)dipalladium (0.10 eq., 35 mg, 0.038 mmol), and XPhos (0.2 eq., 38 mg, 0.076 mmol), and then the mixture was allowed to react overnight at 80 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated under reduced pressure to dry. The residue was separated by column chromatography, to provide tert-butyl (3-(4-(2-(4-(2-methoxy-5,8-dihydropyrido[3,4-d]pyrimidin-7(6H)-yl)phenyl)propan-2-yl)phenoxy)cyclobutyl)carbamate (100 mg, 0.18 mmol, 48%). LC/MS (ESI+) calcd for C₃₂H₄₀N₄O₄ ([M+H]⁺) *m*/*z* 545.30, found 545.20.

### Steps 4 and 5: Synthesis of 2-(2,6-dioxopiperidin-3-yl)-5-(3-(4-(2-(2-methoxy-5,8-dihydropyrido[3,4-d]pyrimidin-7(6H)-yl)phenyl)propan-2-yl)phenoxy)cyclobutyl) aminoisoindolin-1,3-dione

In a 50 mL round-bottom flask, tert-butyl (3-(4-(2-(4-(2-methoxy-5,8-dihydropyrido[3,4-d]pyrimidin-7(6H)-yl)phenyl)propan-2-yl)phenoxy)cyclobutyl) carbamate (100 mg, 0.18 mmol) was dissolved in 10 mL of anhydrous DCM, to which was added 2 mL of TFA in an ice-water bath. The resultant solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide 3-(4-(2-(2-methoxy-5,8-dihydropyrido[3,4-d]pyrimidin-7(6H)-phenyl)propan-2-yl)phenoxy)cyclobutylamine-1-amine, which (calculated at 0.18 mmol) was dissolved in 20 mL of anhydrous DMSO, followed by addition of DIPEA (4.0 eq., 0.22 mL, 1.36 mmol) and 2-(2,6-dioxopiperidin-3-yl)-4-fluoroisoindolin-1,3-dione (2.0 eq., 100 mg, 0.72 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the target compound 2-(2,6-dioxopiperidin-3-yl)-5-(3-(4-(2-(2-methoxy-5,8-dihydropyrido[3,4-d]pyrimidin-7(6H)-yl)phenyl)propan-2-yl)phenoxy) cyclobutyl)aminoisoindolin-1,3-dione (30 mg, 0.042 mmol, 24% for two steps). ¹H NMR (400 MHz, DMSO-d₆) *δ* 11.07 (s, 1H), 8.42 (s, 1H), 7.56 (dd, *J* = 22.2, 6.8 Hz, 2H), 7.09 (dd, *J* = 12.9, 8.8 Hz, 4H), 6.97 - 6.88 (m, 2H), 6.87 - 6.68 (m, 4H), 5.04 (dd, *J* = 12.9, 5.2 Hz, 1H), 4.86 (s, 1H), 4.26 (s, 2H), 4.16 (d, *J* = 18.6 Hz, 1H), 3.86 (d, *J* = 18.9 Hz, 3H), 3.51 (t, *J* = 5.7 Hz, 2H), 2.86 (d, *J* = 11.1 Hz, 1H), 2.82 (d, *J* = 5.5 Hz, 2H), 2.56 (d, *J* = 24.9 Hz, 3H), 2.45 (s, 2H), 1.99 (s, 2H), 1.57 (s, 6H). LC/MS (ESI+) calcd for C₄₀H₄₀N₆O₆ ([M+H]⁺) *m*/*z* 701.30, found 701.10.

### Example 189: 5-((1r,3r)-3-(4-(2-(2-(2-oxa-6-azaspiro[3.3]heptan-6-yl)-5,8-dihydropyrido[3,4-d]pyrimidin-7(6H)-yl)phenyl)propan-2-yl)phenoxy)cyclobutyl) amino)-2-(2,6-dioxopiperidin-3-yl)isoindolin-1,3-dione

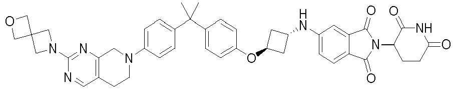

The target compound was synthesized by a method similar to that of Example 188. ¹H NMR (400 MHz, CDCl₃) *δ* 8.11 (d, *J* = 5.6 Hz, 2H), 7.63 (d, *J* = 8.1 Hz, 1H), 7.14 (d, *J* = 8.1 Hz, 4H), 6.89 (d, *J* = 7.9 Hz, 3H), 6.69 (t, *J* = 8.1 Hz, 4H), 4.93 (dd, *J* = 12.2, 5.4 Hz, 1H), 4.85 (s, 4H), 4.79 (s, 1H), 4.29 (s, 3H), 4.23 (s, 2H), 3.50 (d, *J* = 5.7 Hz, 2H), 2.89 (d, *J* = 16.8 Hz, 1H), 2.81 - 2.75 (m, 3H), 2.72 (d, *J* = 4.0 Hz, 3H), 2.40 (d, *J* = 6.2 Hz, 2H), 2.17 - 2.07 (m, 1H), 2.05 (s, 1H), 1.63 (s, 6H). LC/MS (ESI+) calcd for C₄₄H₄₅N₇O₆ ([M+H]⁺) *m*/*z* 768.34, found 768.20.

### Example 190: 5-((1s,3s)-3-(4-(2-(2-(2-oxa-6-azaspiro[3.3]heptan-6-yl)-5,8-dihydropyrido[3,4-d]pyrimidin-7(6H)-yl)phenyl)propan-2-yl)phenoxy)cyclobutyl) amino)-2-(2,6-dioxopiperidin-3-yl)isoindolin-1,3-dione

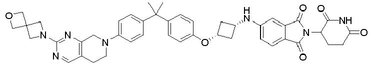

The target compound was synthesized by a method similar to that of Example 188. ¹H NMR (400 MHz, CDCl₃) *δ* 8.13 (d, *J* = 5.6 Hz, 2H), 7.60 (d, *J* = 8.1 Hz, 1H), 7.12 (d, *J* = 8.1 Hz, 4H), 6.90 (d, *J* = 7.9 Hz, 3H), 6.88 (t, *J* = 8.1 Hz, 4H), 4.90 (dd, *J* = 12.2, 5.4 Hz, 1H), 4.83 (s, 4H), 4.79 (s, 1H), 4.29 (s, 3H), 4.23 (s, 2H), 3.51 (d, *J* = 5.7 Hz, 2H), 2.89 (d, *J* = 16.8 Hz, 1H), 2.81 - 2.75 (m, 3H), 2.71 (d, *J* = 4.0 Hz, 3H), 2.40 (d, *J* = 6.2 Hz, 2H), 2.15 - 2.05 (m, 1H), 2.02 (s, 1H), 1.53 (s, 6H). LC/MS (ESI+) calcd for C₄₄H₄₅N₇O₆ ([M+H]⁺) *m*/*z* 768.34, found 768.20.

### Example 191: 2-(2,6-dioxopiperidin-3-yl)-5-((1s,3s)-3-(4-(2-(4-methylpiperazin-1-yl)-5,8-dihydropyrido[3,4-d]pyrimidin-7(6H)-phenyl)propan-2-ylphenoxy) cyclobutyl)amino)isoindolin-1,3-dione

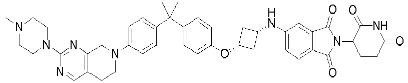

The target compound was synthesized by a method similar to that of Example 188. LC/MS (ESI+) calcd for C₄₄H₄₈N₈O₅ ([M+H]⁺) *m*/*z* 769.37, found 769.20. ¹H NMR (400 MHz, DMSO-*d₆*) *δ* 11.07 (s, 1H), 8.19 (s, 1H), 7.58 (d, *J* = 8.3 Hz, 1H), 7.41 (t, *J* = 22.4 Hz, 1H), 7.08 (dd, *J* = 15.5, 8.8 Hz, 4H), 6.91 (d, *J* = 8.8 Hz, 3H), 6.79 (dd, *J* = 25.8, 8.7 Hz, 3H), 5.03 (dd, *J* = 12.9, 5.3 Hz, 1H), 4.55 - 4.45 (m, 1H), 4.13 (s, 2H), 3.82 - 3.73 (m, 1H), 3.71 - 3.63 (m, 4H), 3.47 (t, *J=* 5.7 Hz, 2H), 3.04 (s, 2H), 2.86 (dd, *J* = 21.9, 9.9 Hz, 1H), 2.70 (t, *J* = 5.8 Hz, 2H), 2.62 - 2.54 (m, 1H), 2.38 - 2.26 (m, 4H), 2.16 (d, *J* = 27.0 Hz, 3H), 2.05 - 1.90 (m, 4H), 1.56 (s, 6H).

### Example 192: 2-(2,6-dioxopiperidin-3-yl)-5-((1r,3r)-3-(4-(2-(2-(4-methylpiperazin-1-yl)-5,8-dihydropyrido[3,4-d]pyrimidin-7(6H)-phenyl)propan-2-ylphenoxy)cyclobutyl)amino)isoindolin-1,3-dione

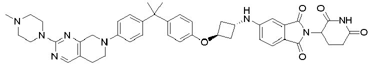

The target compound was synthesized by a method similar to that of Example 188. ¹H NMR (400 MHz, DMSO-*d*₆) *δ* 11.07 (s, 1H), 8.19 (s, 1H), 7.57 (dd, *J* = 15.8, 6.7 Hz, 2H), 7.08 (dd, *J* = 17.1, 8.7 Hz, 4H), 6.97 - 6.64 (m, 6H), 5.03 (dd, *J* = 13.0, 5.4 Hz, 1H), 4.86 (s, 1H), 4.13 (s, 2H), 3.68 (s, 4H), 3.47 (d, *J* = 3.4 Hz, 2H),3.04 (s, 2H), 2.70 (s, 2H), 2.57 (d, *J=* 16.2 Hz, 1H), 2.33 (s, 5H), 2.20 (s, 3H), 2.00 (d, *J* = 7.1 Hz, 2H), 1.75 (s, 3H), 1.56 (s, 6H). LC/MS (ESI+) calcd for C₄₄H₄₈N₈O₅ ([M+H]⁺) *m*/*z* 769.37, found 769.20.

### Example 193: 5-((1s,3s)-3-(4-(2-(2-oxa-6-azaspiro[3.3]heptan-6-yl)pyrimidin-5-yl)methoxy)phenyl)propan-2-yl)phenoxy)cyclobutyl)amino)-2-(2,6-dioxopiperidin-3-yl)isoindolin-1,3-dione

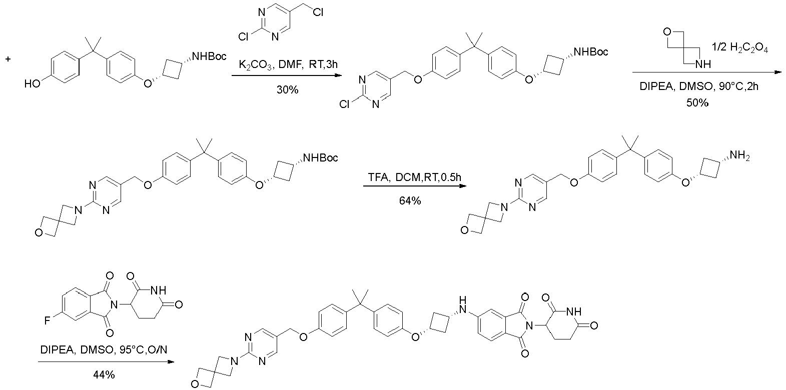

### Step 1: Synthesis of tert-butyl ((1s,3s)-3-(4-(2-(4-((2-chloropyrimidin-5-yl)methoxy) phenyl)propan-2-yl)phenoxy)cyclobutyl)carbamate

tert-butyl ((1s,3s)-3-(4-(2-(4-hydroxylphenyl)propan-2-phenoxy)cyclobutyl) carbamate (500 mg, 1.26 mmol) and 2-chloro-5-(chloromethyl)pyrimidine (1.1 eq., 230 mg, 1.38 mmol) were dissolved in 20 mL of anhydrous DMF, to which was added K₂CO₃ (2.0 eq., 350 mg, 2.52 mmol) at room temperature, and then the mixture was stirred for 3 h. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide tert-butyl ((1s,3s)-3-(4-(2-(4-((2-chloropyrimidin-5-yl)methoxy)phenyl)propan-2-yl)phenoxy)cyclobutyl)carbamate (200 mg, 0.38 mmol, 30%). LC/MS (ESI+) calcd for C₂₉H₃₄ClN₃O₄ ([M+H]⁺) *m*/*z* 524.22, found 524.20.

### Step 2: Synthesis of tert-butyl ((1s,3s)-3-(4-(2-(4-((2-(2-oxa-6-azaspiro[3.3]heptan-6-yl)pyrimidin-5-yl)methoxy)phenyl)propan-2-yl)phenoxy)cyclobutyl)carbamate

tert-butyl ((1s,3s)-3-(4-(2-(4-((2-chloropyrimidin-5-yl)methoxy)phenyl)propan-2-yl)phenoxy)cyclobutyl)carbamate (100 mg, 0.19mmol) was dissolved in 20 mL of anhydrous DMSO, to which were added 2-oxa-6-azaspiro[3.3]heptane hemioxalate (0.55 eq., 300 mg, 0.11 mmol) and DIPEA (4.0 eq., 0.15 mL, 0.76 mmol), and then the mixture was heated for 2 h at 90 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide tert-butyl ((1s,3s)-3-(4-(2-(4-((2-(2-oxa-6-azaspiro[3.3]heptan-6-yl)pyrimidin-5-yl)methoxy)phenyl)propan-2-yl)phenoxy) cyclobutyl)carbamate (56 mg, 0.096 mmol, 50%). LC/MS (ESI+) calcd for C₃₄H₄₂N₄O₅ ([M+H]⁺) *m*/*z* 587.32, found 587.20.

### Step 3: Synthesis of (1s,3s)-3-(4-(2-(4-((2-(2-oxa-6-azaspiro[3.3]heptan-6-yl) pyrimidin-5-yl)methoxy)phenyl)propan-2-yl)phenoxy)cyclobutylamine

tert-butyl ((1s,3s)-3-(4-(2-(4-((2-(2-oxa-6-azaspiro[3.3]heptan-6-yl)pyrimidin-5-yl)methoxy)phenyl)propan-2-yl)phenoxy)cyclobutyl)carbamate (56 mg, 0.096 mmol) was dissolved in 10 mL of anhydrous DCM, to which was added 2 mL of TFA in an ice-water bath. The resultant solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1s,3s)-3-(4-(2-(4-((2-(2-oxa-6-azaspiro[3.3]heptan-6-yl)pyrimidin-5-yl)methoxy)phenyl)propan-2-yl)phenoxy)cyclobutylamine (30 mg, 0.061 mmol, 64%). LC/MS (ESI+) calcd for C₂₉H₃₄N₄O₃ ([M+H]⁺) *m*/*z* 487.26, found 487.20.

### Step 4: Synthesis of 5-((1s,3s)-3-(4-(2-(2-oxa-6-azaspiro[3.3]heptan-6-yl)pyrimidin-5-yl)methoxy)phenyl)propan-2-yl)phenoxy)cyclobutyl)amino)-2-(2,6-dioxopiperidin-3-yl)isoindolin-1,3-dione

(1s,3s)-3-(4-(2-(4-((2-(2-oxa-6-azaspiro[3.3]heptan-6-yl)pyrimidin-5-yl)methoxy) phenyl)propan-2-yl)phenoxy)cyclobutylamine (30 mg, 0.061 mmol) was dissolved in 20 mL of anhydrous DMSO, to which were added DIPEA (4.0 eq., 0.04 mL, 0.24 mmol) and 2-(2,6-dioxopiperidin-3-yl)-4-fluoroisoindolin-1,3-dione (2 eq., 34 mg, 0.12 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the target compound 5-((1s,3s)-3-(4-(2-(2-oxa-6-azaspiro[3.3]heptan-6-yl)pyrimidin-5-yl)methoxy)phenyl)propan-2-yl)phenoxy)cyclobutyl)amino)-2-(2,6-dioxopiperidin-3-yl)isoindolin-1,3-dione (20 mg, 0.026 mmol, 44%) as yellow solid. ¹H NMR (400 MHz, CDCl₃) *δ* 8.37 (s, 2H),8.13 (d, *J* = 23.8 Hz, 1H), 7.62 (d, *J* = 8.3 Hz, 1H), 7.12 (dd, *J* = 16.6, 9.1 Hz, 4H), 6.91 (d, *J* = 2.0 Hz, 1H), 6.83 (d, *J* = 8.8 Hz, 2H), 6.74 - 6.59 (m, 3H), 4.98 - 4.86 (m, 1H), 4.86 (s, 4H), 4.83 (s, 2H), 4.74 (d, *J* = 6.2 Hz, 1H), 4.51 (p, *J* = 6.6 Hz, 1H), 4.31 (s, 4H), 3.19 - 3.05 (m, 2H), 2.96 - 2.69 (m, 2H), 2.62 (s, 3H), 2.11 (dd, *J* = 16.2, 6.2 Hz, 2H), 1.63 (s, 6H). LC/MS (ESI+) calcd for C₄₂H₄₂N₆O₇ ([M+H]⁺) *m*/*z* 743.31, found 743.20.

### Example 194: 5-((1s,3s)-3-(4-(2-(4-(2-oxa-6-azaspiro[3.3]heptan-6-yl)-7,8-dihydropyrido[4,3-d]pyrimidin-6(5H)-yl)phenyl)propan-2-ylphenoxy)cyclobutyl) amino)-2-(2,6-dioxopiperidin-3-yl)isoindolin-1,3-dione

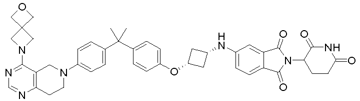

The target compound was synthesized by a method similar to that of Example 188. ¹H NMR (400 MHz, CDCl₃) *δ* 8.45 (s, 1H), 8.06 (s, 1H), 7.59 (t, *J* = 23.5 Hz, 1H), 7.15 (dd, *J* = 10.3, 8.8 Hz, 4H), 6.97 - 6.61 (m, 6H), 4.93 (dd, *J* = 12.2, 5.2 Hz, 1H), 4.86 (s, 4H), 4.74 (d, *J* = 6.3 Hz, 1H), 4.57 (s, 3H), 4.55 - 4.49 (m, 1H), 4.22 (s, 2H), 3.85 - 3.71 (m, 1H), 3.50 (t, *J* = 5.7 Hz, 2H), 3.19 - 3.02 (m, 4H), 2.95 - 2.66 (m, 3H), 2.19 - 2.04 (m, 3H), 1.64 (s, 6H). LC/MS (ESI+) calcd for C₄₄H₄₅N₇O₆ ([M+H]⁺) *m*/*z* 768.34, found 768.20.

### Example 195: 5-((1r,3r)-3-(4-(2-(2-(2-oxa-6-azaspiro[3.3]heptan-6-yl)-7,8-dihydropyrido[4,3-d]pyrimidin-6(5H)-yl)phenyl)propan-2-yl)phenoxy)cyclobutyl) amino)-2-(2,6-dioxopiperidin-3-yl)isoindolin-1,3-dione

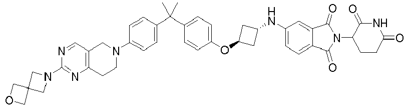

The target compound was synthesized by a method similar to that of Example 188. ¹H NMR (400 MHz, DMSO-d₆) *δ* 11.07 (s, 1H), 8.20 (s, 1H), 7.56 (dd, *J* = 23.9, 6.8 Hz, 2H), 7.08 (dd, *J* = 17.2, 8.8 Hz, 4H), 6.95 - 6.63 (m, 6H), 5.03 (dd, *J* = 12.8, 5.4 Hz, 1H), 4.85 (s, 1H), 4.70 (s, 4H), 4.19 (s, 2H), 4.14 (s, 5H), 3.52 (t, *J* = 5.9 Hz, 2H), 3.30 (s, 1H), 2.94 - 2.79 (m, 1H), 2.75 (t, *J* = 5.9 Hz, 2H), 2.43 (d, *J* = 5.0 Hz, 4H), 1.99 (s, 2H), 1.56 (s, 6H). LC/MS (ESI+) calcd for C₄₄H₄₅N₇O₆ ([M+H]⁺) *m*/*z* 768.34, found 768.20.

### Example 196: 5-((1s,3s)-3-(4-(2-(4-(2-(1-oxa-6-azaspiro[3.3]heptane-6-yl) pyrimidin-4-yl)methoxy)phenyl)propan-2-yl)phenoxy)cyclobutyl)amino)-2-(2,6-dioxopyridin-3-yl)isoindolin-1,3-dione

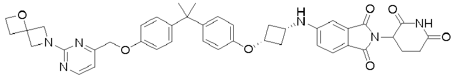

The target compound was synthesized by a method similar to that of Example 79. ¹H NMR (400 MHz, CDCl₃) *δ* 8.29 (d, *J* = 5.3 Hz, 1H), 8.03 (s, 1H), 7.62 (d, *J* = 8.3 Hz, 1H), 7.13 (t, *J* = 8.8 Hz, 4H), 6.92 (d, *J* = 2.0 Hz, 1H), 6.89 (s, 1H), 6.82 (d, *J* = 8.8 Hz, 2H), 6.75 - 6.66 (m, 3H), 4.99 (s, 2H), 4.93 (dd, *J* = 12.3, 5.3 Hz, 1H), 4.72 (s, 1H), 4.58 (t, *J* = 7.5 Hz, 2H), 4.55 - 4.42 (m, 4H), 3.83 - 3.73 (m, 1H), 3.19 - 3.06 (m, 2H), 2.93 (t, *J* = 7.6 Hz, 2H), 2.89 - 2.67 (m, 3H), 2.10 (ddd, *J* = 17.4, 11.6, 6.2 Hz, 3H), 1.63 (s, 6H). LC/MS (ESI+) calcd for C₄₂H₄₂N₆O₇([M+H]⁺) *m*/*z* 743.31, found 743.20.

### Example 197: 2-(2,6-dioxopiperidin-3-yl)-5-((1r,3r)-3-(4-(2-(4-((2-methylpyrimidin-4-yl)oxy)phenyl)propan-2-yl)phenoxy)cyclobutyl)amino) isoindolin-1,3-dione

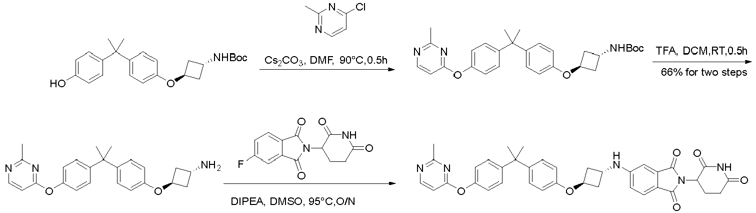

### Steps 1 and 2: Synthesis of (1r,3r)-3-(4-(2-(4-((2-methylpyrimidin-4-yl)oxy)phenyl)propan-2-yl)phenoxy)cyclobutane-1-amine

4-chloro-2-methylpyrimidine (50 mg, 0.39 mmol) and tert-butyl ((1r,3r)-3-(4-(2-(4-hydroxylphenyl)propan-2-phenoxy)cyclobutyl)carbamate (1.0 eq., 156 mg, 0.39 mmol) were dissolved in 10 mL of anhydrous DMF, to which was added cesium carbonate (2.0 eq., 250 mg, 0.78 mmol), and then the mixture was stirred at 90 °C for 2 h. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide the crude product tert-butyl ((1r,3r)-3-(4-(2-(4-((2-methylpyrimidin-4-yl)oxy)phenyl)propan-2-yl)phenoxy)cyclobutyl)carbamate, which (calculated at 0.39 mmol) was dissolved in 10 mL of anhydrous DCM, followed by addition of 2 mL TFA in an ice-water bath. The resultant solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-(4-(2-(4-((2-methylpyrimidin-4-yl)oxy)phenyl)propan-2-yl)phenoxy)cyclobutane-1-amine (100 mg, 0.26 mmol, 66% for two steps). LC/MS (ESI+) calcd for C₂₄H₂₇N₃O₂ ([M+H]⁺) *m*/*z* 390.21, found 390.20.

### Step 3: Synthesis of 2-(2,6-dioxopiperidin-3-yl)-5-((1r,3r)-3-(4-(2-(4-((2-methylpyrimidin-4-yl)oxy)phenyl)propan-2-yl)phenoxy)cyclobutyl)amino)isoindolin-1,3-dione

(1r,3r)-3-(4-(2-(4-((2-methylpyrimidin-4-yl)oxy)phenyl)propan-2-yl)phenoxy) cyclobutane-1-amine (100 mg, 0.26 mmol) was dissolved in 20 mL of anhydrous DMSO, to which were added DIPEA (4.0 eq., 0.17 mL, 1.04 mmol) and 2-(2,6-dioxopiperidin-3-yl)-4-fluoroisoindolin-1,3-dione (2.0 eq., 140 mg, 0.52 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the compound 2-(2,6-dioxopiperidin-3-yl)-5-((1r,3r)-3-(4-(2-(4-((2-methylpyrimidin-4-yl) oxy)phenyl)propan-2-yl)phenoxy)cyclobutyl)amino)isoindolin-1,3-dione (30 mg, 0.047 mmol, 18%) as yellow solid. ¹H NMR (400 MHz, CDCl₃) *δ* 8.46 (d, *J* = 5.5 Hz, 1H), 8.07 (s, 1H), 7.60 (t, *J* = 24.8 Hz, 1H), 7.28 (d, *J* = 5.6 Hz, 2H), 7.17 (d, *J* = 8.8 Hz, 2H), 7.03 (t, *J* = 11.3 Hz, 2H), 6.89 (d, *J* = 2.0 Hz, 1H), 6.77 - 6.65 (m, 3H), 6.60 (d, *J* = 5.2 Hz, 1H), 4.99 - 4.82 (m, 2H), 4.76 (s, 1H), 4.23 (s, 1H), 2.97 - 2.67 (m, 5H), 2.65 (s, 3H), 2.50 - 2.32 (m, 2H), 2.16 - 2.06 (m, 1H), 1.69 (s, 6H). LC/MS (ESI+) calcd for C₃₇H₃₅N₅O₆([M+H]⁺) *m*/*z* 646.26, found 646.20.

### Example 198: 2-(2,6-dioxopiperidin-3-yl)-5-((1s,3s)-3-(4-(2-(2-(2-methylpyrimidin-4-yl)oxy)phenyl)propan-2-yl)phenoxy)cyclobutyl)amino) isoindolin-1,3-dione

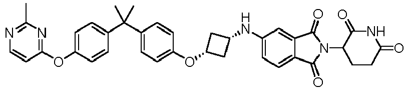

The target compound was synthesized by a method similar to that of Example 197. ¹H NMR (400 MHz, CDCl₃) *δ* 8.45 (d, *J* = 5.2 Hz, 1H), 8.15 (s, 1H), 7.59 (t, *J* = 21.7 Hz, 1H), 7.28 (d, *J* = 4.1 Hz, 2H), 7.16 (d, *J* = 8.7 Hz, 2H), 7.04 (d, *J* = 8.6 Hz, 2H), 6.92 (d, *J* = 1.9 Hz, 1H), 6.78 - 6.61 (m, 3H), 6.58 (d, *J* = 5.1 Hz, 1H), 4.93 (dd, *J* = 12.2, 5.2 Hz, 1H), 4.77 (d, *J* = 6.0 Hz, 1H), 4.52 (p, *J* = 6.7 Hz, 1H), 3.78 (dd, *J* = 14.2, 6.9 Hz, 1H), 3.24 - 2.97 (m, 2H), 2.95 - 2.67 (m, 3H), 2.63 (s, 3H), 2.19 - 2.06 (m, 3H), 1.68 (s, 6H). LC/MS (ESI+) calcd for C₃₇H₃₅N₅O₆ ([M+H]⁺) *m*/*z* 646.26, found 646.20.

### Example 199: 2-(2,6-dioxopiperidin-3-yl)-5-((1s,3s)-3-(4-(2-(4-((5-fluoro-2-(2-oxa-6-azaspiro[3.3]heptan-6-yl)pyrimidin-4-yl)methoxy)phenyl)propan-2-yl)phenoxy) cyclobutyl)amino)isoindolin-1,3-dione

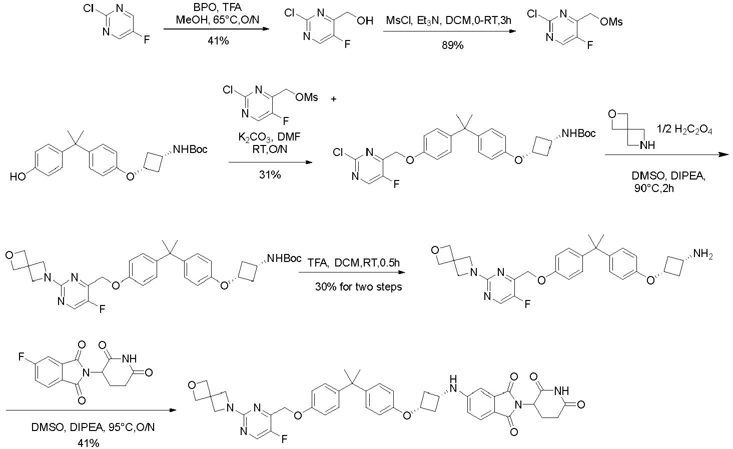

### Step 1: Synthesis of (2-chloro-5-fluoropyrimidin-4-yl)methyl methanesulfonate

2-chloro-5-fluoropyrimidine (0.50 mL, 5.45mmol) was dissolved in 20 mL of anhydrous methanol, to which were added TFA (2.0 eq., 0.82 mL, 10.90 mmol) and BPO (2.0 eq., 2.64 g, 10.90 mmol), and then the reaction solution was refluxed overnight at 65 °C. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by column chromatography, to provide (2-chloro-5-fluoropyrimidin-4-yl)methanol (366 mg, 2.25 mmol, 41%). LC/MS (ESI+) calcd for C₅H₄ClFN₂O ([M+H]⁺) *m*/*z* 163.00, found 162.99.

### Step 2: Synthesis of tert-butyl ((1s,3s)-3-(4-(2-(4-((2-chloro-5-fluoropyrimidin-4-yl)methoxy)phenyl)propan-2-yl)phenoxy)cyclobutyl)carbamate

(2-chloro-5-fluoropyrimidin-4-yl)methanol (366 mg, 2.25 mmol) was dissolved in 20 mL of anhydrous DCM, to which was added triethylamine (2.5 eq., 0.78 mL, 5.63 mmol), followed by addition of MsCl (2.0 eq., 0.50 mL, 4.50 mmol) in an ice-water bath, and then the reaction solution was warmed to room temperature and stirred for 3 h. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by column chromatography, to provide (2-chloro-5-fluoropyrimidin-4-yl)methyl methanesulfonate (480 mg, 2.00 mmol, 89%). LC/MS (ESI+) calcd for C₆H₆ClFN₂O₃S ([M+H]⁺) *m*/*z* 240.98, found 240.99. Step 3: Synthesis of tert-butyl ((1s,3s)-3-(4-(2-(4-((2-chloro-5-fluoropyrimidin-4-yl)methoxy)phenyl)propan-2-yl)phenoxy)cyclobutyl)carbamate

(2-chloro-5-fluoropyrimidin-4-yl)methyl methanesulfonate (160 mg, 0.66 mmol) and tert-butyl ((1s,3s)-3-(4-(2-(4-hydroxylphenyl)propan-2-phenoxy)cyclobutyl) carbamate (1.0 eq., 0.27 g, 0.67 mmol) were dissolved in 20 mL of anhydrous DMF, to which was added K₂CO₃ (2.0 eq., 180 mg, 1.34 mmol), and then the mixture was stirred overnight at room temperature. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide tert-butyl ((1s,3s)-3-(4-(2-(4-((2-chloro-5-fluoropyrimidin-4-yl)methoxy)phenyl)propan-2-yl)phenoxy)cyclobutyl)carbamate (112 mg, 0.21 mmol, 31%). LC/MS (ESI+) calcd for C₂₉H₃₃ClFN₃O₄ ([M+H]⁺) *m*/*z* 542.21, found 542.10. Steps 4 and 5: Synthesis of (1s,3s)-3-(4-(2-(4-((5-fluoro-2-(2-oxa-6-azaspiro[3.3]heptan-6-yl)pyrimidin-4-yl)methoxy)phenyl)propan-2-yl)phenoxy) cyclobutylamine

tert-butyl ((1s,3s)-3-(4-(2-(4-((2-chloro-5-fluoropyrimidin-4-yl)methoxy)phenyl) propan-2-yl)phenoxy)cyclobutyl)carbamate (112 mg, 0.21 mmol) was dissolved in 20 mL of anhydrous DMSO, to which were added 2-oxa-6-azaspiro[3.3]heptane hemioxalate (0.55 eq., 300 mg, 0.11 mmol) and DIPEA (4.0 eq., 0.15 mL, 0.84 mmol), and then the reaction solution was heated for 2h at 90 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide tert-butyl ((1s,3s)-3-(4-(2-(4-((5-fluoro-2-(2-oxa-6-azaspiro[3.3]heptan-6-yl)pyrimidin-4-yl)methoxy)phenyl) propan-2-yl)phenoxy)cyclobutyl)carbamate, which (calculated at 0.21 mmol) was dissolved in 10 mL of anhydrous DCM, followed by addition of 2 mL TFA in an ice-water bath. The resultant solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1s,3s)-3-(4-(2-(4-((5-fluoro-2-(2-oxa-6-azaspiro[3.3]heptan-6-yl)pyrimidin-4-yl)methoxy)phenyl) propan-2-yl)phenoxy)cyclobutylamine (32 mg, 0.063 mmol, 30% for two steps). LC/MS (ESI+) calcd for C₂₉H₃₃FN₄O₃ ([M+H]⁺) *m*/*z* 505.25, found 505.10.

### Step 6: Synthesis of 2-(2,6-dioxopiperidin-3-yl)-5-((1s,3s)-3-(4-(2-(4-((5-fluoro-2-(2-oxa-6-azaspiro[3.3]heptan-6-yl)pyrimidin-4-yl)methoxy)phenyl)propan-2-yl)phenoxy) cyclobutyl)amino)isoindolin-1,3-dione

(1s,3s)-3-(4-(2-(4-((5-fluoro-2-(2-oxa-6-azaspiro[3.3]heptan-6-yl)pyrimidin-4-yl)methoxy)phenyl)propan-2-yl)phenoxy)cyclobutylamine (32 mg, 0.063 mmol) was dissolved in 20 mL of anhydrous DMSO, to which were added DIPEA (4.0 eq., 0.05 mL, 0.25 mmol) and 2-(2,6-dioxopiperidin-3-yl)-4-fluoroisoindolin-1,3-dione (2.0 eq., 35 mg, 0.13 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the target compound 2-(2,6-dioxopiperidin-3-yl)-5-((1s,3s)-3-(4-(2-(4-((5-fluoro-2-(2-oxa-6-azaspiro[3.3]heptan-6-yl)pyrimidin-4-yl)methoxy)phenyl)propan-2-yl)phenoxy)cyclobutyl)amino)isoindolin-1,3-dione (20 mg, 0.026 mmol, 41%). ¹H NMR (400 MHz, CDCl₃) *δ* 8.19 (d, *J* = 1.8 Hz, 1H), 8.07 (s, 1H), 7.64 (d, *J* = 8.3 Hz, 1H), 7.13 (t, *J* = 8.7 Hz, 4H), 6.89 (d, *J* = 8.7 Hz, 3H), 6.70 (dd, *J* = 11.8, 5.5 Hz, 3H), 5.04 (d, *J* = 1.4 Hz, 2H), 4.93 (dd, *J* = 12.1, 5.3 Hz, 1H), 4.84 (s, 5H), 4.24 (s, 4H), 2.95 - 2.63 (m, 5H), 2.44 - 2.31 (m, 3H), 2.11 (d, *J* = 11.2 Hz, 2H), 1.63 (s, 6H). LC/MS (ESI+) calcd for C₄₂H₄₁FN₆O₇ ([M+H]⁺) *m*/*z* 761.30, found 761.10.

### Example 200: 2-(2,6-dioxopiperidin-3-yl)-5-((1r,3r)-3-(4-(2-(4-((5-fluoro-2-(2-oxa-6-azaspiro[3.3]heptan-6-yl)pyrimidin-4-yl)methoxy)phenyl)propan-2-yl)phenoxy) cyclobutyl)amino)isoindolin-1,3-dione

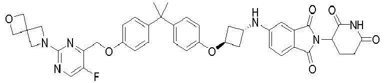

The target compound was synthesized by a method similar to that of Example 199. ¹H NMR (400 MHz, CDCl₃) *δ* 8.19 (d, *J =* 1.8 Hz, 1H), 8.07 (s, 1H), 7.64 (d, *J* = 8.3 Hz, 1H), 7.13 (t, *J =* 8.7 Hz, 4H), 6.89 (d, *J* = 8.7 Hz, 3H), 6.70 (dd, *J* = 11.8, 5.5 Hz, 3H), 5.04 (d, *J* = 1.4 Hz, 2H), 4.93 (dd, *J* = 12.1, 5.3 Hz, 1H), 4.84 (s, 5H), 4.24 (s, 4H), 2.95 - 2.63 (m, 5H), 2.44 - 2.31 (m, 3H), 2.11 (d, *J* = 11.2 Hz, 2H), 1.63 (s, 6H). LC/MS (ESI+) calcd for C₄₂H₄₁FN₆O₇ ([M+H]⁺) *m*/*z* 761.30, found 761.10.

### Example 201: 5-((1s,3s)-3-(4-(2-(4-((5-chloro-2-(2-oxa-6-azaspiro[3.3]heptan-6-yl)pyrimidin-4-methoxy)phenyl)propan-2-yl)phenoxy)cyclobutyl)amino)-2-(2,6-dioxopiperidin-3-yl)isoindolin-1,3-dione

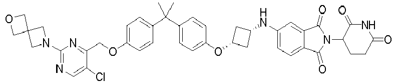

The target compound was synthesized by a method similar to that of Example 199. ¹H NMR (400 MHz, CDCl₃) *δ* 8.23 (s, 1H), 8.01 (s, 1H), 7.63 (d, *J* = 8.4 Hz, 1H), 7.13 (d, *J* = 8.6 Hz, 4H), 6.97 - 6.82 (m, 3H), 6.71 (d, *J* = 8.5 Hz, 3H), 5.06 (s, 2H), 4.93 (d, *J* = 7.0 Hz, 2H), 4.84 (d, *J=* 11.1 Hz, 5H), 4.25 (s, 4H), 3.13 (s, 2H), 2.94 - 2.69 (m, 5H), 2.07 (d, *J* = 21.0 Hz, 2H), 1.63 (s, 6H). LC/MS (ESI+) calcd for C₄₂H₄₁ClN₆O₇ ([M+H]⁺) *m*/*z* 777.27, found 777.10.

### Example 202: 5-((1r,3r)-3-(4-(2-(4-((5-chloro-2-(2-oxa-6-azaspiro[3.3]heptan-6-yl)pyrimidin-4-methoxy)phenyl)propan-2-yl)phenoxy)cyclobutyl)amino)-2-(2,6-dioxopiperidin-3-yl)isoindolin-1,3-dione

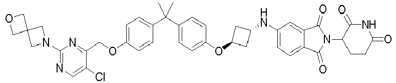

The target compound was synthesized by a method similar to that of Example 199. ¹H NMR (400 MHz, CDCl₃) *δ* 8.22 (s, 1H), 8.01 (s, 1H), 7.64 (d, *J* = 8.2 Hz, 1H), 7.13 (t, *J* = 6.9 Hz, 4H), 6.87 (d, *J* = 8.5 Hz, 3H), 6.69 (d, *J* = 7.5 Hz, 3H), 5.07 (d, *J* = 3.1 Hz, 2H), 4.98 - 4.90 (m, 1H), 4.83 (s, 5H), 4.25 (s, 4H), 2.96 - 2.66 (m, 5H), 2.42 (s,3H), 2.13 (s, 2H), 1.63 (s, 6H). LC/MS (ESI+) calcd for C₄₂H₄₁ClN₆O₇ ([M+H]⁺) *m*/*z* 777.27, found 777.10.

### Example 203: 2-(2,6-dioxopiperidin-3-yl)-5-((1s,3s)-3-(4-(2-(4-((5-methyl-2-(2-oxa-6-azaspiro[3.3]heptan-6-yl)pyrimidin-4-yl)methoxy)phenyl)propan-2-yl) phenoxy)cyclobutyl)amino)isoindolin-1,3-dione

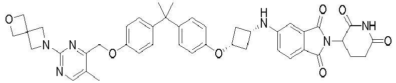

The target compound was synthesized by a method similar to that of Example 199. ¹H NMR (400 MHz, CDCl₃) *δ* 8.11 (s, 2H), 7.63 (d, *J* = 8.3 Hz, 1H), 7.13 (d, *J* = 8.6 Hz, 4H), 6.92 (d, *J* = 2.0 Hz, 1H), 6.86 (d, *J* = 8.8 Hz, 2H), 6.77 - 6.65 (m, 3H), 4.98 (s, 2H), 4.93 (dd, *J* = 12.3, 5.3 Hz, 1H), 4.84 (s, 4H), 4.76 (d, *J* = 5.9 Hz, 1H), 4.50 (dd, *J* = 13.5, 6.7 Hz, 1H), 4.28 (s, 4H), 3.78 (d, *J* = 7.0 Hz, 1H), 3.36 - 2.98 (m, 2H), 2.97 - 2.66 (m, 3H), 2.20 (s, 3H), 2.11 (dt, *J* = 16.8, 9.1 Hz, 3H), 1.63 (s, 6H). LC/MS (ESI+) calcd for C₄₃H₄₄N₆O₇ ([M+H]⁺) *m*/*z* 757.33, found 757.20.

### Example 204: 2-(2,6-dioxopiperidin-3-yl)-5-((1r,3r)-3-(4-(2-(4-((5-methyl-2-(2-oxa-6-azaspiro[3.3]heptan-6-yl)pyrimidin-4-yl)methoxy)phenyl)propan-2-yl) phenoxy)cyclobutyl)amino)isoindolin-1,3-dione

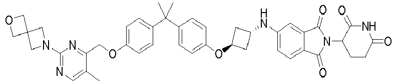

The target compound was synthesized by a method similar to that of Example 199. ¹H NMR (400 MHz, CDCl₃) *δ* 8.10 (d, *J* = 3.1 Hz, 2H), 7.63 (d, *J* = 8.3 Hz, 1H), 7.13 (dd, *J* = 8.8, 6.5 Hz, 4H), 6.87 (dd, *J* = 12.7, 5.4 Hz, 3H), 6.70 (dd, *J* = 10.3, 5.5 Hz, 3H), 5.00 (s, 2H), 4.93 (dd, *J* = 12.3, 5.3 Hz, 1H), 4.85 (d, *J* = 6.6 Hz, 6H), 4.32 (s, 4H), 4.22 (s, 1H), 2.97 - 2.64 (m, 5H), 2.49 - 2.34 (m, 2H), 2.21 (s, 3H), 2.16 - 2.07 (m, 1H), 1.63 (s, 6H). LC/MS (ESI+) calcd for C₄₃H₄₄N₆O₇ ([M+H]⁺) *m*/*z* 757.33, found 757.20.

### Example 205: 2-(2,6-dioxopiperidin-3-yl)-5-((1r,3r)-3-(4-(2-(4-((6-(1-methyl-1H-pyrazol-4-yl)pyridin-3-yl)oxy)phenyl)propan-2-yl)phenoxy)cyclobutyl)amino) isoindolin-1,3-dione

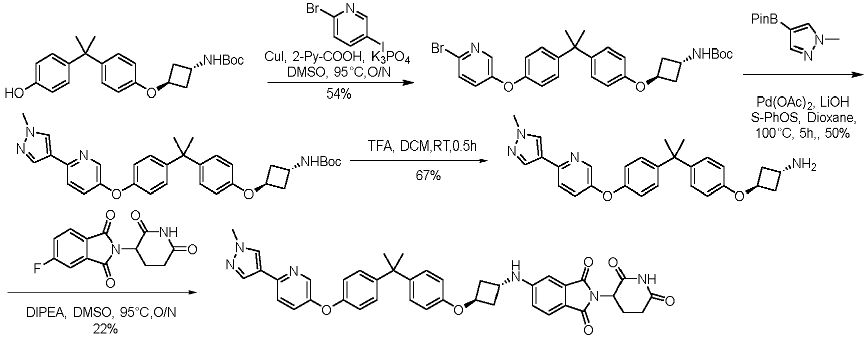

### Step 1: tert-butyl ((1r,3r)-3-(4-(2-(4-((6-bromopyridin-3-yl)oxy)phenyl)propan-2-yl)phenoxy)cyclobutyl)carbamate

2-bromo-5-iodopyridine (100 mg, 0.35 mmol) and tert-butyl ((1r,3r)-3-(4-(2-(4-hydroxylphenyl)propan-2-phenoxy)cyclobutyl)carbamate (1.0 eq., 140 mg, 0.35 mmol) were dissolved in 20 mL of anhydrous DMSO, to which were added CuI (0.30 eq., 20 mg, 0.10 mmol), 2-picolinic acid (0.3 eq., 13 mg, 0.10 mmol), and potassium phosphate (2.0 eq., 150 mg, 0.71 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide tert-butyl ((1r,3r)-3-(4-(2-(4-((6-bromopyridin-3-yl)oxy)phenyl)propan-2-yl)phenoxy)cyclobutyl)carbamate (110 mg, 0.19 mmol, 54%). LC/MS (ESI+) calcd for C₂₉H₃₃BrN₂O₄ ([M+H]⁺) *m*/*z* 553.16, 555.16, found 553.10, 555.10.

### Step 2: tert-butyl ((1r,3r)-3-(4-(2-(4-((6-(1-methyl-1H-pyrazol-4-yl)pyridin-3-yl)oxy) phenyl)propan-2-yl)phenoxy)cyclobutyl)carbamate

tert-butyl ((1r,3r)-3-(4-(2-(4-((6-bromopyridin-3-yl)oxy)phenyl)propan-2-yl) phenoxy)cyclobutyl)carbamate (110 mg, 0.19 mmol) was dissolved in 10 mL of 1,4-dioxane, to which were successively added 1-methyl-4-(4,4,5,5-tetramethyl-1,3,2-dioxaborolane-2-yl)-1H-pyrazol (2.0 eq., 800 g, 0.38 mmol), Pd(OAc)₂ (0.1 eq., 5 mg, 0.02 mmol), S-Phos (0.1 eq, 10 mg, 0.02 mmol), and LiOH aqueous solution (2.0 M, 0.10 mL). The system was purged with argon gas for three times, and allowed to react for 5h at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide tert-butyl ((1r,3r)-3-(4-(2-(4-((6-(1-methyl-1H-pyrazol-4-yl)pyridin-3-yl)oxy)phenyl)propan-2-yl)phenoxy)cyclobutyl)carbamate (53 mg, 0.095 mmol, 50%). LC/MS (ESI+) calcd for C₃₃H₃₈N₄O₄ ([M+H]⁺) *m*/*z* 555.29, found 555.10.

### Step 3: (1r,3r)-3-(4-(2-(4-((6-(1-methyl-1H-pyrazol-4-yl)pyridin-3-yl)oxy)phenyl) propan-2-yl)phenoxy)cyclobutylamine

tert-butyl ((1r,3r)-3-(4-(2-(4-((6-(1-methyl-1H-pyrazol-4-yl)pyridin-3-yl)oxy) phenyl)propan-2-yl)phenoxy)cyclobutyl)carbamate (53 mg, 0.095 mmol) was dissolved in 10 mL of anhydrous DCM, to which was added 2 mL of TFA in an ice-water bath. The reaction solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-(4-(2-(4-((6-(1-methyl-1H-pyrazol-4-yl)pyridin-3-yl)oxy)phenyl)propan-2-yl)phenoxy) cyclobutylamine (29 mg, 0.064 mmol, 67%). LC/MS (ESI+) calcd for C₂₈H₃₀N₄O₂ ([M+H]⁺) *m*/*z* 455.24, found 455.20.

### Step 4: 2-(2,6-dioxopiperidin-3-yl)-5-((1r,3r)-3-(4-(2-(4-((6-(1-methyl-1H-pyrazol-4-yl)pyridin-3-yl)oxy)phenyl)propan-2-yl)phenoxy)cyclobutyl)amino)isoindolin-1,3-dione

Compound (1r,3r)-3 -(4-(2-(4-((6-(1 -methyl-1H-pyrazol-4-yl)pyridin-3 -yl)oxy) phenyl)propan-2-yl)phenoxy)cyclobutylamine (29 mg, 0.064 mmol) was dissolved in 20 mL of anhydrous DMSO, to which were added DIPEA (4 eq., 0.05 mL, 0.25 mmol) and 2-(2,6-dioxopiperidin-3-yl)-4-fluoroisoindolin-1,3-dione (2 eq., 35 mg, 0.13 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide compound 2-(2,6-dioxopiperidin-3-yl)-5-((1r,3r)-3-(4-(2-(4-((6-(1-methyl-1H-pyrazol-4-yl)pyridin-3-yl)oxy)phenyl)propan-2-yl)phenoxy)cyclobutyl)amino) isoindolin-1,3-dione (10 mg, 0.014 mmol, 22%) as yellow solid. ¹H NMR (400 MHz, CDCl₃) *δ* 8.31 (d, *J* = 2.5 Hz, 1H), 8.22 (s, 1H), 7.90 (s, 2H), 7.63 (d, *J* = 8.2 Hz, 1H), 7.43 (d, *J* = 8.6 Hz, 1H), 7.37 - 7.30 (m, 1H), 7.21 (d, *J* = 8.7 Hz, 2H), 7.14 (d, *J* = 8.7 Hz, 2H), 6.91 (dd, *J* = 14.1, 5.2 Hz, 3H), 6.71 (d, *J* = 8.6 Hz, 3H), 4.93 (dd, *J* = 12.2, 5.2 Hz, 1H), 4.84 (d, *J* = 26.5 Hz, 2H), 4.23 (s, 1H), 3.96 (s, 3H), 2.95 - 2.65 (m, 5H), 2.49 - 2.34 (m, 2H), 2.19 - 2.06 (m, 1H), 1.66 (s, 6H). LC/MS (ESI+) calcd for C₄₁H₃₈N₆O₆ ([M+H]⁺) *m*/*z* 711.29, found 711.20.

### Example 206: 5-((1r,3r)-3-(4-(2-(4-((6-(2H-1,2,3-triazol-2-yl)pyridin-3-yl)oxy) phenyl)propan-2-ylphenoxy)cyclobutyl)amino)-2-(2,6-dioxopiperidin-3-yl) isoindolin-1,3-dione

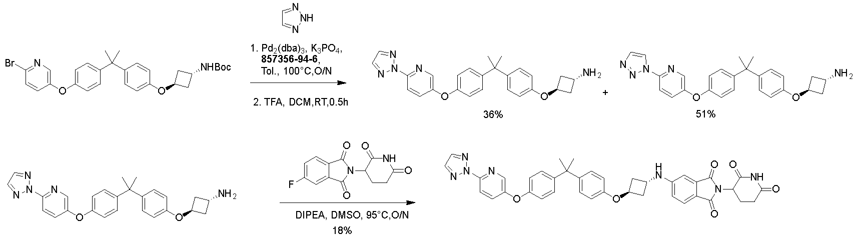

### Steps 1 and 2: Synthesis of (1r,3r)-3-(4-(2-(4-((6-(2H-1,2,3-triazol-2-yl)pyridin-3-yl)oxy)phenyl)propan-2-yl)phenoxy)cyclobutylamine and (1r,3r)-3-(4-(2-(4-((6-(1H-1,2,3-triazol-1-yl)pyridin-3-yl)oxy)phenyl)propan-2-yl)phenoxy)cyclobutylamine

tert-butyl ((1r,3r)-3-(4-(2-(4-((6-bromopyridin-3-yl)oxy)phenyl)propan-2-yl) phenoxy)cyclobutyl)carbamate (240 mg, 0.43 mmol) was dissolved in anhydrous toluene (20 mL), to which were added potassium phosphate (2.0 eq., 180 mg, 0.86 mmol), tris(dibenzylideneacetone)dipalladium (0.10 eq., 39 mg, 0.043 mmol), ligand 2-Di-tert-butylphosphine-3,4,5,6-tetramethyl-2',4',6'-triisopropylbiphenyl (857356-94-6, 0.2 eq., 42 mg, 0.086 mmol), 2H-triazole (2.0 eq., 60 mg, 0.86 mmol), and then the reaction solution was refluxed overnight at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was dissolved in 10 mL of anhydrous DCM, to which was added 2 mL of TFA in an ice-water bath. The reaction solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-(4-(2-(4-((6-(2H-1,2,3-triazol-2-yl)pyridin-3-yl)oxy)phenyl)propan-2-yl)phenoxy) cyclobutylamine (69 mg, 0.16 mmol, 36%) and (1r,3r)-3-(4-(2-(4-((6-(1H-1,2,3-triazol-1-yl)pyridin-3-yl)oxy)phenyl)propan-2-yl)phenoxy)cyclobutylamine (97 mg, 0.22 mmol, 51%). LC/MS (ESI+) calcd for C₂₆H₂₇N₅O₂ ([M+H]⁺) *m*/*z* 442.22, found 442.10. For both compounds, their retention times were different. Step 3: Synthesis of 5-((1r,3r)-3-(4-(2-(4-((6-(2H-1,2,3-triazol-2-yl)pyridin-3-yl)oxy)phenyl)propan-2-ylphenoxy)cyclobutyl)amino)-2-(2,6-dioxopiperidin-3-yl) isoindolin-1,3-dione

(1r,3r)-3-(4-(2-(4-((6-(2H-1,2,3-triazol-2-yl)pyridin-3-yl)oxy)phenyl)propan-2-yl)phenoxy)cyclobutylamine (69 mg, 0.16 mmol) was dissolved in 20 mL of anhydrous DMSO, to which were added DIPEA (4.0 eq., 0.11 mL, 0.64 mmol) and 2-(2,6-dioxopiperidin-3-yl)-4-fluoroisoindolin-1,3-dione (2.0 eq., 88 mg, 0.32 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide target compound 5-((1r,3r)-3-(4-(2-(4-((6-(2H-1,2,3-triazol-2-yl)pyridin-3-yl)oxy)phenyl) propan-2-ylphenoxy)cyclobutyl)amino)-2-(2,6-dioxopiperidin-3-yl)isoindolin-1,3-dione (20 mg, 0.029 mmol, 18%) as yellow solid. ¹H NMR (400 MHz, CDCl₃) *δ* 8.33 (s, 1H), 8.11 - 7.91 (m, 2H), 7.88 (s, 2H), 7.64 (d, *J=* 8.3 Hz, 1H), 7.57 - 7.40 (m, 1H), 7.24 (d, *J* = 8.8 Hz, 2H), 7.14 (d, *J* = 8.8 Hz, 2H), 7.01 - 6.86 (m, 3H), 6.72 (d, *J* = 8.8 Hz, 3H), 5.04 - 4.89 (m, 1H), 4.88 (s, 1H), 4.24 (t, *J* = 4.9 Hz, 1H), 2.96 - 2.62 (m, 5H), 2.50 - 2.35 (m, 2H), 2.20 - 2.08 (m, 2H), 1.67 (s, 6H). LC/MS (ESI+) calcd for C₃₉H₃₅N₇O₆ ([M+H]⁺) *m*/*z* 698.26, found 698.10.

### Example 207: 5-((1r,3r)-3-(4-(2-(4-((6-(1H-1,2,3-triazol-1-yl)pyridin-3-yl)oxy) phenyl)propan-2-ylphenoxy)cyclobutyl)amino)-2-(2,6-dioxopiperidin-3-yl) isoindolin-1,3-dione

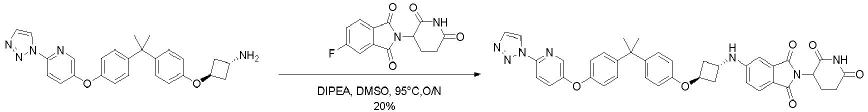

(1r,3r)-3-(4-(2-(4-((6-(1H-1,2,3-triazol-1-yl)pyridin-3-yl)oxy)phenyl)propan-2-yl)phenoxy)cyclobutylamine (97 mg, 0.22 mmol) was dissolved in 20 mLof anhydrous DMSO, to which were added DIPEA (4.0 eq., 0.15 mL, 0.88 mmol) and 2-(2,6-dioxopiperidin-3-yl)-4-fluoroisoindolin-1,3-dione (2.0 eq., 130 mg, 0.44 mmol). The system was purged with argon gas for three times, and allowed to react overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide target compound 5-((1r,3r)-3-(4-(2-(4-((6-(1H-1,2,3-triazol-1-yl)pyridin-3-yl)oxy)phenyl) propan-2-ylphenoxy)cyclobutyl)amino)-2-(2,6-dioxopiperidin-3-yl)isoindolin-1,3-dione (30 mg, 0.043 mmol, 20%) as yellow solid. ¹H NMR (400 MHz, CDCl₃) *δ* 8.52 (d, *J* = 1.1 Hz, 1H), 8.24 (d, *J* = 2.6 Hz, 1H), 8.15 (d, *J* = 8.9 Hz, 1H), 8.06 (s, 1H), 7.83 (d, *J* = 1.1 Hz, 1H), 7.63 (d, *J* = 8.3 Hz, 1H), 7.50 (dd, *J* = 8.9, 2.9 Hz, 1H), 7.27 (d, *J* = 0.9 Hz, 1H), 7.25 (d, *J* = 2.1 Hz, 1H), 7.15 (d, *J* = 8.8 Hz, 2H), 7.02 - 6.93 (m, 2H), 6.89 (d, *J* = 1.8 Hz, 1H), 6.79 - 6.58 (m, 3H), 4.93 (dd, *J* = 12.3, 5.3 Hz, 1H), 4.87 (s, 1H), 4.24 (s, 1H), 2.98 - 2.63 (m, 5H), 2.41 (dt, *J* = 13.4, 6.5 Hz, 2H), 2.17 - 2.08 (m, 2H), 1.68 (s, 6H). LC/MS (ESI+) calcd for C₃₉H₃₅N₇O₆ ([M+H]⁺) *m*/*z* 698.26, found 698.10.

### Example 208: 5-((1r,3r)-3-(4-(2-(4-((6-(1H-pyrazol-1-yl)pyridin-3-yl)oxy)phenyl) propan-2-yl)phenoxy)cyclobutyl)amino)-2-(2,6-dioxopiperidin-3-yl)isoindolin-1,3-dione

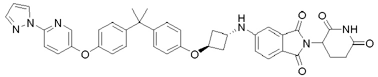

The target compound was synthesized by a method similar to that of Example 206. ¹H NMR (400 MHz, CDCl₃) *δ* 8.53 (d, *J* = 2.3 Hz, 1H), 8.16 (d, *J* = 2.7 Hz, 1H), 7.97 (d, *J* = 9.1 Hz, 2H), 7.75 (s, 1H), 7.64 (d, *J* = 8.3 Hz, 1H), 7.54 - 7.45 (m, 1H), 7.23 (d, *J* = 8.8 Hz, 2H), 7.15 (d, *J* = 8.8 Hz, 2H), 6.92 (dd, *J* = 13.2, 5.4 Hz, 3H), 6.72 (d, *J* = 8.8 Hz, 3H), 6.48 (s, 1H), 5.05 - 4.80 (m, 2H), 4.24 (s, 1H), 2.97 - 2.64 (m, 5H), 2.40 (dd, *J* = 12.1, 6.7 Hz, 2H), 2.19 - 2.06 (m, 2H), 1.67 (s, 6H). LC/MS (ESI+) calcd for C₄₀H₃₆N₆O₆ ([M+H]⁺) *m*/*z* 697.27, found 697.20.

### Example 209: 2-(2,6-dioxopiperidin-3-yl)-5-(1r,3r)-3-(4-(2-(4-(6-(5-methyl-1,2,4-oxadiazol-3-yl)pyridin-3-yl)oxy)phenyl)propan-2-yl)phenoxy)cyclobutyl)amino) isoindolin-1,3-dione

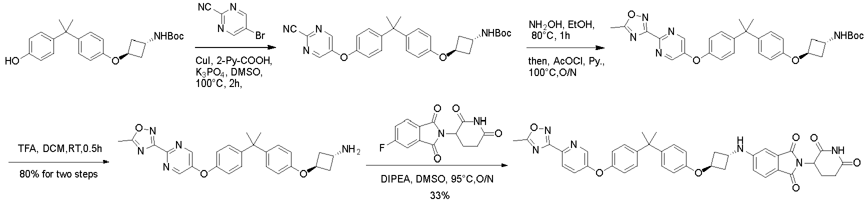

### Step 1: Synthesis of tert-butyl ((1r,3r)-3-(4-(2-(4-((2-cyanopyrimidin-5-yl)oxy) phenyl)propan-2-yl)phenoxy)cyclobutyl)carbamate

tert-butyl ((1r,3r)-3-(4-(2-(4-hydroxylphenyl)propan-2-phenoxy)cyclobutyl) carbamate (100 mg, mmol) and 5-bromo-2-cyanopyrimidine (1.2 eq., 55 mg, 0.30 mmol) were dissolved in 20 mL of anhydrous DMSO, to which were added CuI (0.30 eq., 20 mg, 0.075 mmol), 2-picolinic acid (0.3 eq., 10 mg, 0.075 mmol), and potassium phosphate (2.0 eq., 10 mg, 0.5 mmol). The system was purged with argon gas for three times, and reacted under stirring for 2 h at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide tert-butyl ((1r,3r)-3-(4-(2-(4-((2-cyanopyrimidin-5-yl)oxy)phenyl)propan-2-yl)phenoxy)cyclobutyl)carbamate (133 mg, 0.27 mmol, 100%). LC/MS (ESI+) calcd for C₂₉H₃₂N₄O₄ ([M+H]⁺) *m*/*z* 501.24, found 501.20.

### Step 2: Synthesis of tert-butyl ((1r,3r)-3-(4-(2-(4-((2-(5-methyl-1,2,4-oxadiazol-3-yl)pyrimidin-5-yl)oxy)phenyl)propan-2-yl)phenoxy)cyclobutyl)carbamate

tert-butyl ((1r,3r)-3-(4-(2-(4-((2-cyanopyrimidin-5-yl)oxy)phenyl)propan-2-yl) phenoxy)cyclobutyl)carbamate (133 mg, 0.27 mmol) was dissolved in 10 mL of absolute ethanol, to which was added hydroxylamine aqueous solution (2.0 eq., 50%, 0.03 mL, 0.54 mmol), and then the reaction was stirred for 1 h at 80 °C. The reaction solution was rotatory evaporated to dry under reduced pressure, followed by extraction with EA (3*20mL). The organic phase was dried over Na₂SO₄, suction filtered, and rotatory evaporated to dry, to provide the crude product, to which was added 10 mL of pyridine, followed by careful drop addition of acetyl chloride (2.0 eq., 0.04 mL, 0.54 mmol). The reaction solution was allowed to react overnight at 100 °C. 100 mL of 0.05 M diluted hydrochloric acid was added to quench the reaction. The resultant solution was extracted with EA (4*30 mL). The organic phase was dried with Na₂SO₄, and then rotatory evaporated to dry under reduced pressure. The residue was separated by column chromtography, to provide tert-butyl ((1r,3r)-3-(4-(2-(4-((2-(5-methyl-1,2,4-oxadiazol-3-yl)pyrimidin-5-yl)oxy)phenyl)propan-2-yl)phenoxy)cyclobutyl) carbamate (97 mg, 0.17 mmol). LC/MS (ESI+) calcd for C₃₁H₃₅N₅O₅ ([M+H]⁺) *m*/*z* 558.26, found 558.20.

### Step 3: Synthesis of (1r,3r)-3-(4-(2-(4-((2-(5-methyl-1,2,4-oxadiazol-3-yl)pyrimidin-5-yl)oxy)phenyl)propan-2-yl)phenoxy)cyclobutylamine-1-amine

tert-butyl ((1r,3r)-3-(4-(2-(4-((2-(5-methyl-1,2,4-oxadiazol-3-yl)pyrimidin-5-yl)oxy)phenyl)propan-2-yl)phenoxy)cyclobutyl)carbamate (97 mg, 0.17mmol) was dissolved in 10 mL of anhydrous DCM, to which was added 2 mL of TFA in an ice-water bath. The reaction solution was warmed to room temperature and stirred for 30 min. 20 mL of saturated NaHCO₃ solution was added to quench the reaction. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-(4-(2-(4-((2-(5-methyl-1,2,4-oxadiazol-3-yl)pyrimidin-5-yl)oxy)phenyl)propan-2-yl) phenoxy)cyclobutylamine-1-amine (100 mg, 0.21 mmol, 80% for two steps). LC/MS (ESI+) calcd for C₂₆H₂₇N₅O₃ ([M+H]⁺) *m*/*z* 458.21, found 458.10.

### Step 4: 2-(2,6-dioxopiperidin-3-yl)-5-(1r,3r)-3-(4-(2-(4-(6-(5-methyl-1,2,4-oxadiazol-3-yl)pyridin-3-yl)oxy)phenyl)propan-2-yl)phenoxy)cyclobutyl)amino)isoindolin-1,3-dione

(1r,3r)-3-(4-(2-(4-((2-(5-methyl-1,2,4-oxadiazol-3-yl)pyrimidin-5-yl)oxy)phenyl) propan-2-yl)phenoxy)cyclobutylamine-1-amine (100 mg, 0.21 mmol) was dissolved in 20 mL of anhydrous DMSO, to which were added DIPEA (4.0 eq., 0.10 mL, 0.60 mmol) and 2-(2,6-dioxopiperidin-3-yl)-4-fluoroisoindolin-1,3-dione (2.0 eq., 85 mg, 0.30 mmol). The system was purged with argon gas for three times, and reacted overnight at 95 °C. 50 mL of saturated NH₄Cl solution was added to quench the reaction. The resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide 2-(2,6-dioxopiperidin-3-yl)-5-(1r,3r)-3-(4-(2-(4-(6-(5-methyl-1,2,4-oxadiazol-3-yl)pyridin-3-yl)oxy)phenyl)propan-2-yl)phenoxy)cyclobutyl)amino)isoindolin-1,3-dione (50 mg, 0.07 mmol, 33%). ¹H NMR (400 MHz, CDCl₃) *δ* 8.60 (s, 2H), 7.98 (s, 1H), 7.64 (d, *J* = 8.3 Hz, 1H), 7.30 (d, *J* = 8.8 Hz, 2H), 7.13 (d, *J* = 8.8 Hz, 2H), 7.01 (d, *J* = 8.8 Hz, 2H), 6.90 (d, *J* = 2.0 Hz, 1H), 6.72 (d, *J* = 8.7 Hz, 3H), 5.02 - 4.79 (m, 2H), 4.24 (s, 1H), 2.95 - 2.65 (m, 9H), 2.42 (dt, *J* = 13.3, 6.6 Hz, 2H), 2.19 - 2.07 (m, 1H), 1.69 (s, 6H). LC/MS (ESI+) calcd for C₄₀H₃₀N₆O₇ ([M+H]⁺) *m*/*z* 713.26, found 713.10.

### Example 210: 2-(2,6-dioxopyridin-3-yl)-5-((1r,3r)-3-(4-(1-(4-((2-(5-methyl-1,2,4-oxadiazol-3-yl)pyrimidin-5-yl)oxy)phenyl)allyl)phenoxy)cyclobutyl)amino) isoindolin-1,3-dione

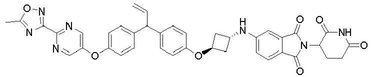

The target compound was synthesized by a method similar to that of Example 209. ¹H NMR (400 MHz, CDCl₃) *δ* 8.60 (s, 2H), 7.96 (s, 1H), 7.64 (d, *J* = 8.3 Hz, 1H), 7.24 (s, 1H), 7.07 (dd, *J* = 18.0, 8.6 Hz, 4H), 6.90 (s, 1H), 6.74 (dd, *J* = 17.8, 8.5 Hz, 3H), 6.27 (ddd, *J* = 17.0, 10.1, 7.1 Hz, 1H), 5.38 - 5.20 (m, 1H), 5.00 (d, *J* = 17.1 Hz, 1H), 4.96 - 4.83 (m, 2H), 4.72 (d, *J* = 6.6 Hz, 1H), 4.25 (s, 1H), 2.96 - 2.65 (m, 9H), 2.42 (s, 2H), 2.12 (d, J = 5.0 Hz, 1H). LC/MS (ESI+) calcd for C₃₉H₃₃N₇O₇ ([M+H]⁺) *m*/*z* 712.24, found 712.10.

### Example 211: 2-(2,6-dioxopyridin-3-yl)-5-((1r,3r)-3-(4-(2-(4-((6-(5-methyl-1,2,4-oxadiazol-3-yl)-5-(trifluoromethyl)pyridin-3-yl)oxy)phenyl)propan-2-yl)phenoxy) cyclobutyl)amino)isoindolin-1,3-dione

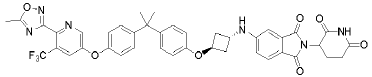

The target compound was synthesized by a method similar to that of Example 209. ¹H NMR (400 MHz, CDCl₃) *δ* 8.62 (s, 1H), 7.97 (s, 1H), 7.66 - 7.61 (m, 2H), 7.34 - 7.28 (m, 2H), 7.15 (d, *J* = 8.8 Hz, 2H), 7.00 (d, *J* = 8.8 Hz, 2H), 6.90 (d, *J* = 2.0 Hz, 1H), 6.72 (d, *J* = 8.8 Hz, 3H), 4.99 - 4.81 (m, 2H), 4.25 (s, 1H), 2.89 (d, *J* = 18.2 Hz, 1H), 2.84 - 2.79 (m, 1H), 2.78 - 2.67 (m, 7H), 2.47 - 2.36 (m, 2H), 2.18 - 2.08 (m, 1H), 1.69 (s, 6H). LC/MS (ESI+) calcd for C₄₁H₃₅F₃N₆O₇ ([M+H]⁺) *m*/*z* 781.25, found 781.10.

### Example 212: 2-(2,6-dioxopyridin-3-yl)-5-((1r,3r)-3-(4-(1-(4-((2-(5-methyl-1,2,4-oxadiazol-3-yl)pyrimidin-5-yl)oxy)phenyl)cyclopropyl)phenoxy)cyclobutyl) amino)isoindolin-1,3-dione

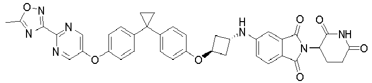

The target compound was synthesized by a method similar to that of Example 209. LC/MS (ESI+) calcd for C₃₉H₃₃N₇O₇ ([M+H]⁺) *m*/*z* 712.24, found 712.10. ¹H NMR (400 MHz, CDCl₃) *δ* 8.59 (s, 2H), 8.03 (s, 1H), 7.60 (t, *J* = 23.7 Hz, 1H), 7.26 (d, *J* = 7.9 Hz, 2H), 7.18 (d, *J* = 8.6 Hz, 2H), 7.00 (d, *J* = 8.6 Hz, 2H), 6.89 (d, *J* = 1.8 Hz, 1H), 6.72 (dd, *J* = 13.5, 5.3 Hz, 3H), 5.01 - 4.81 (m, 2H), 4.29 - 4.15 (m, 1H), 2.95 - 2.65 (m, 9H), 2.42 (dt, *J* = 12.7, 6.3 Hz, 2H), 2.18 - 2.07 (m, 1H), 1.28 (t, *J* = 7.9 Hz, 4H). LC/MS (ESI+) calcd for C₃₉H₃₃N₇O₇ ([M+H]⁺) m/z 712.24, found 712.10.

### Example 213: 2-(2,6-dioxopiperidin-3-yl)-5-(1s,3s)-3-(4-(2-(4-(5-(5-methyl-1,2,4-oxadiazol-3-yl)pyridin-2-yl)oxy)phenyl)propan-2-yl)phenoxy)cyclobutyl)amino) isoindolin-1,3-dione

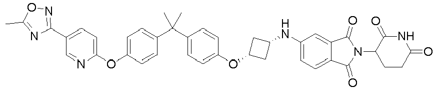

The target compound was synthesized by a method similar to that of Example 209. ¹H NMR (400 MHz, CDCl₃) *δ* 8.90 (d, *J* = 2.0 Hz, 1H), 8.33 (dd, *J* = 8.6, 2.2 Hz, 1H), 7.99 (s, 1H), 7.63 (d, *J* = 8.3 Hz, 1H), 7.28 (d, *J* = 6.8 Hz, 2H), 7.17 (d, *J* = 8.8 Hz, 2H), 7.07 (d, *J* = 8.7 Hz, 2H), 6.98 (d, *J* = 8.6 Hz, 1H), 6.93 (d, *J* = 2.0 Hz, 1H), 6.79 - 6.67 (m, 3H), 4.93 (dd, *J* = 12.3, 5.3 Hz, 1H), 4.52 (dd, *J* = 13.6, 6.8 Hz, 1H), 3.85 - 3.72 (m, 1H), 3.21 - 3.06 (m, 2H), 2.97 - 2.68 (m, 3H), 2.66 (s, 3H), 2.11 (dd, *J* = 16.5, 10.2 Hz, 4H), 1.68 (s, 6H). LC/MS (ESI+) calcd for C₄₀H₃₆N₆O₇ ([M+H]⁺) *m*/*z* 713.26, found 713.10.

### Example 214: 5-((1r,3r)-3-(4-(2-(4-((6-(2-oxa-6-azaspiro[3.3]heptane-6-yl)pyridin-3-yl)oxy)phenyl)propan-2-yl)phenoxy)cyclobutyl)amino)-2-(2,6-dioxopiperidin-3-yl)isoindolin-1,3-dione

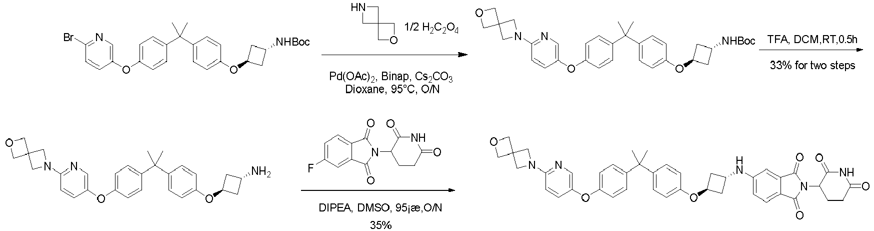

### Steps 1 and 2: Synthesis of (1r,3r)-3-(4-(2-(4-((6-(2-oxa-6-azaspiro[3.3]heptan-6-yl)pyridin-3-yl)oxy)phenyl)propan-2-yl)phenoxy)cyclobutylamine

tert-butyl ((1r,3r)-3-(4-(2-(4-((6-bromopyridin-3-yl)oxy)phenyl)propan-2-yl) phenoxy)cyclobutyl)carbamate (100 mg, 0.18 mmol) was dissolved in 20 mL of anhydrous 1,4-dioxane, to which were added palladium acetate (0.20 eq., 10 mg, 0.036 mmol), binap (0.20 eq, 0.020 g, 0.036 mmol), cesium carbonate (2.0 eq., 120 mg, 0.36 mmol), and 2-oxa-6-azaspiro[3.3]heptane hemioxalate (1.0 eq., 51 mg, 0.18 mmol), and the reaction was stirred overnight at 95 °C. 50 mL of saturated NH₄Cl solution was added to quench the reaction. The resultant solution was extracted with ethyl acetate (3*40 mL), followed by suction filtration. The filtrate was dried with anhydrous Na₂SO₄, and then rotatory evaporated to dry under reduced pressure, to provide the crude product tert-butyl ((1r,3r)-3-(4-(2-(4-((6-(2-oxa-6-azaspiro[3.3]heptan-6-yl)pyridin-3-yl)oxy)phenyl)propan-2-yl)phenoxy)cyclobutyl)carbamate, which was dissolved in 10 mL of anhydrous DCM, followed by adding 2 mL of TFA in an ice-water bath. The reaction solution was warmed to room temperature and stirred for 30 min. 20 mL of saturated NaHCO₃ solution was added to quench the reaction. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-(4-(2-(4-((6-(2-oxa-6-azaspiro[3.3]heptan-6-yl)pyridin-3-yl)oxy)phenyl)propan-2-yl)phenoxy) cyclobutylamine (28 mg, 0.059 mmol, 33% for two steps). LC/MS (ESI+) calcd for C₂₉H₃₃N₃O₃ ([M+H]⁺) *m*/*z* 472.25, found 472.20.

### Step 3: Synthesis of 5-((1r,3r)-3-(4-(2-(4-((6-(2-oxa-6-azaspiro[3.3]heptane-6-yl)pyridin-3-yl)oxy)phenyl)propan-2-yl)phenoxy)cyclobutyl)amino)-2-(2,6-dioxopiperidin-3-yl)isoindolin-1,3-dione

(1r,3r)-3-(4-(2-(4-((6-(2-oxa-6-azaspiro[3.3]heptan-6-yl)pyridin-3-yl)oxy)phenyl)propan-2-yl)phenoxy)cyclobutylamine (28 mg, 0.059 mmol) was dissolved in 20 mL of anhydrous DMSO, to which were added DIPEA (4.0 eq., 0.05 mL, 0.0.30 mmol) and 2-(2,6-dioxopiperidin-3-yl)-4-fluoroisoindolin-1,3-dione (2.0 eq., 33 mg, 0.12 mmol). The system was purged with argon gas for three times, and reacted overnight at 95 °C. 50 mL of saturated NH₄Cl solution was added to quench the reaction. The resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide target compound 5 -((1r,3r)-3-(4-(2-(4-((6-(2-oxa-6-azaspiro[3.3]heptane-6-yl)pyridin-3-yl)oxy)phenyl)propan-2-yl)phenoxy)cyclobutyl)amino)-2-(2,6-dioxopiperidin-3-yl)isoindolin-1,3-dione (15 mg, 0.021 mmol, 35%). ¹H NMR (400 MHz, CDCl₃) *δ* 8.24 (s, 1H), 7.95 (d, *J* = 2.6 Hz, 1H), 7.62 (d, *J* = 8.3 Hz, 1H), 7.25 - 7.21 (m, 1H), 7.12 (dd, *J* = 8.6, 5.2 Hz, 4H), 6.88 (d, *J* = 1.7 Hz, 1H), 6.79 (d, *J* = 8.7 Hz, 2H), 6.68 (d, *J* = 8.7 Hz, 3H), 6.30 (d, *J* = 8.9 Hz, 1H), 4.93 (dd, *J* = 12.3, 5.2 Hz, 1H), 4.85 (s, 6H), 4.28 - 4.04 (m, 5H), 2.97 - 2.62 (m, 5H), 2.47 - 2.30 (m, 2H), 2.15 - 2.05 (m, 1H), 1.63 (s, 6H). LC/MS (ESI+) calcd for C₄₂H₄₁N₅O₇ ([M+H]⁺) *m*/*z* 728.30, found 728.20.

### Example 215: 5-((1r,3r)-3-(4-(2-(4-((3-amino-1,2,4-triazin-6-yl)oxy)phenyl) propan-2-yl)phenoxy)cyclobutyl)amino)-2-(2,6-dioxopiperidin-3-yl)isoindolin-1,3-dione

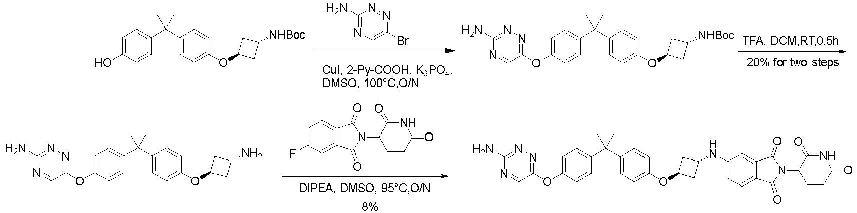

### Steps 1 and 2: Synthesis of 6-(4-(2-(4-((1r,3r)-3-aminocyclobutyloxy)phenyl)propan-2-yl)phenoxy)-1,2,4-triazin-3-amine

To tert-butyl ((1r,3r)-3-(4-(2-(4-hydroxylphenyl)propan-2-phenoxy)cyclobutyl) carbamate (300 mg, 0.76 mmol), were added 3-amino-6-bromo-1,2,4-triazine (2 eq., 269 mg, 1.52 mmol), CuI (0.3 eq., 40 mg, 0.23 mmol), 2-picolinic acid (0.3 eq., 29 mg, 0.23 mmol), and potassium phosphate (2 eq., 330 mg, 1.52 mmol). The system was purged with argon gas for three times, and reacted overnight under stirring at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the crude product tert-butyl ((1r,3r)-3-(4-(2-(4-((3-amino-1,2,4-triazin-6-yl)oxy)phenyl)propan-2-yl)phenoxy)cyclobutyl)carbamate, which was dissolved in 10 mL of anhydrous DCM, followed by adding 2 mL of TFA in an ice-water bath. The reaction solution was warmed to room temperature and stirred for 30 min. 20 mL of saturated NaHCO₃ solution was added to quench the reaction. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide 6-(4-(2-(4-((1r,3r)-3-aminocyclobutyloxy)phenyl) propan-2-yl) phenoxy)-1,2,4-triazin-3-amine (59 mg, 0.15 mmol, 20% for two steps). LC/MS (ESI+) calcd for C₂₂H₂₅N₅O₂ ([M+H]⁺) *m*/*z* 392.20, found 392.10.

### Step 3: Synthesis of 5-((1r,3r)-3-(4-(2-(4-((3-amino-1,2,4-triazin-6-yl)oxy)phenyl) propan-2-yl)phenoxy)cyclobutyl)amino)-2-(2,6-dioxopiperidin-3-yl)isoindolin-1,3-dione

6-(4-(2-(4-((1r,3r)-3-aminocyclobutyloxy)phenyl)propan-2-yl)phenoxy)-1,2,4-triazin-3-amine (59 mg, 0.15 mmol) was dissolved in 20 mL of anhydrous DMSO, to which were added DIPEA (4.0 eq., 0.05 mL, 0.0.30 mmol) and 2-(2,6-dioxopiperidin-3-yl)-4-fluoroisoindolin-1,3-dione (2.0 eq., 33 mg, 0.12 mmol). The system was purged with argon gas for three times, and reacted overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide target compound, 5-((1r,3r)-3-(4-(2-(4-((3-amino-1,2,4-triazin-6-yl)oxy)phenyl)propan-2-yl)phenoxy)cyclobutyl) amino)-2-(2,6-dioxopiperidin-3-yl)isoindolin-1,3-dione (8 mg, 0.012 mmol, 8%). LC/MS (ESI+) calcd for C₃₅H₃₃N₇O₆ ([M+H]⁺) *m*/*z* 648.25, found 648.10.

### Example 216: 2-(2,6-dioxopiperidin-3-yl)-5-(1r,3r)-3-(4-(2-(4-(5-(5-methyl-1,3,4-oxadiazol-2-yl)pyridin-2-yl)oxy)phenyl)propan-2-yl)phenoxy)cyclobutyl)amino) isoindolin-1,3-dione

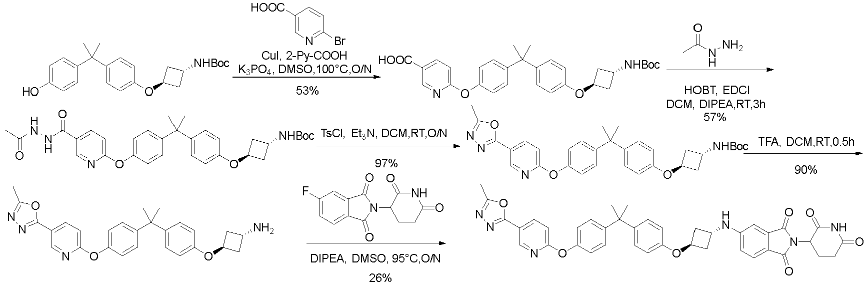

### Step 1: Synthesis of 6-(4-(2-(4-((1r,3r)-3-((tert-butoxycarbonyl)amino)cyclobutyloxy) phenyl)propan-2-yl)phenoxy)nicotinic acid

To tert-butyl ((1r,3r)-3-(4-(2-(4-hydroxylphenyl)propan-2-phenoxy)cyclobutyl) carbamate (320 mg, 0.80 mmol) and 6-bromonicotinic acid (1.5 eq., 240 mg, 1.20 mmol), were added CuI (0.30 eq., 45 mg, 0.24 mmol), 2-picolinic acid (0.3 eq., 29 mg, 0.23 mmol), and potassium phosphate (2.0 eq., 330 mg, 1.52 mmol). The system was purged with argon gas for three times, and reacted overnight under stirring at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide 6-(4-(2-(4-((1r,3r)-3-((tert-butoxycarbonyl)amino)cyclobutyloxy)phenyl)propan-2-yl)phenoxy)nicotinic acid (220 mg, 0.42 mmol, 53%). LC/MS (ESI+) calcd for C₃₀H₃₄N₂O₆ ([M-H]⁻) *m*/*z* 517.24, found 517.20.

### Step 2: Synthesis of tert-butyl ((1r,3r)-3-(4-(2-(4-((5-(2-acethydrazide-1-carbonyl) pyridin-2-yl)oxy)phenyl)propan-2-yl)phenoxy)cyclobutyl)carbamate

6-(4-(2-(4-((1r,3r)-3-((tert-butoxycarbonyl)amino)cyclobutyloxy)phenyl)propan-2-yl)phenoxy)nicotinic acid (220 mg, 0.42 mmol) was dissolved in 20 mL of anhydrous DCM, to which were added acethydrazide (2.0 eq., 65 mg, 0.84 mmol), DIPEA (2.0 eq., 0.14 mL, 0.84 mmol), HOBT (2.0 eq., 110 mg, 0.84 mmol), and EDCI (2.0 eq., 160 mg, 0.84 mmol), and then the reaction solution was stirred at room temperature for 3h. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide tert-butyl ((1r,3r)-3-(4-(2-(4-((5-(2-acethydrazide-1-carbonyl)pyridin-2-yl)oxy)phenyl)propan-2-yl) phenoxy)cyclobutyl)carbamate (140 mg, 0.24 mmol, 57%). LC/MS (ESI+) calcd for C₃₂H₃₈N₄O₆ ([M+H]⁺) *m*/*z* 575.28, found 575.20.

### Step 3: Synthesis of tert-butyl ((1r,3r)-3-(4-(2-(4-((5-(5-methyl-1,3,4-oxadiazol-2-yl)pyridin-2-yl)oxy)phenyl)propan-2-yl)phenoxy)cyclobutyl)carbamate

((1r,3r)-3-(4-(2-(4-((5-(2-acethydrazide-1-carbonyl)pyridin-2-yl)oxy)phenyl) propan-2-yl)phenoxy)cyclobutyl)carbamate (140 mg, 0.24 mmol) was dissolved in 20 mL of anhydrous DCM, to which were added triethylamine (2.0 eq., 0.065 mL, 0.48 mmol) and TsCl (2.0 eq., 91 mg, 0.48 mmol), and then the mixture was stirred overnight at room temperature. 50 mL of saturated NaHCO₃ solution was added to quench the reaction. The resultant solution was extracted with ethyl acetate (3*40 mL), followed by suction filtration. The filtrate was dried with anhydrous Na₂SO₄, and then rotatory evaporated under reduced pressure to dry. The residue was separated by TLC, to provide tert-butyl ((1r,3r)-3-(4-(2-(4-((5-(5-methyl-1,3,4-oxadiazol-2-yl)pyridin-2-yl) oxy)phenyl)propan-2-yl)phenoxy)cyclobutyl)carbamate (130 mg, 0.23 mmol, 97%). LC/MS (ESI+) calcd for C₃₂H₃₆N₄O₅ ([M+H]⁺) *m*/*z* 557.27, found 557.20.

### Step 4: Synthesis of (1r,3r)-3-(4-(2-(4-((5-(5-methyl-1,3,4-oxadiazol-2-yl)pyridin-2-yl)oxy)phenyl)propan-2-yl)phenoxy)cyclobutyl-1-amine

tert-butyl ((1r,3r)-3-(4-(2-(4-((5-(5-methyl-1,3,4-oxadiazol-2-yl)pyridin-2-yl)oxy) phenyl)propan-2-yl)phenoxy)cyclobutyl)carbamate (130 mg, 0.23 mmol) was dissolved in 10 mL of anhydrous DCM, to which was added 2 mL of TFA in an ice-water bath. The reaction solution was warmed to room temperature and stirred for 30 min. 20 mL of saturated NaHCO₃ solution was added to quench the reaction. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-(4-(2-(4-((5-(5-methyl-1,3,4-oxadiazol-2-yl)pyridin-2-yl)oxy)phenyl)propan-2-yl)phenoxy) cyclobutyl-1-amine (97 mg, 0.21 mmol, 90%). LC/MS (ESI+) calcd for C₂₇H₂₈N₄O₃ ([M+H]⁺) *m*/*z* 457.22, found 457.20.

### Step 5: Synthesis of 2-(2,6-dioxopiperidin-3-yl)-5-(1r,3r)-3-(4-(2-(4-(5-(5-methyl-1,3,4-oxadiazol-2-yl)pyridin-2-yl)oxy)phenyl)propan-2-yl)phenoxy)cyclobutyl)amino) isoindolin-1,3-dione

(1r,3r)-3-(4-(2-(4-((5-(5-methyl-1,3,4-oxadiazol-2-yl)pyridin-2-yl)oxy)phenyl) propan-2-yl)phenoxy)cyclobutyl-1-amine (97 mg, 90%, 0.21 mmol) was dissolved in 20 mLof anhydrous DMSO, to which were added DIPEA (4.0 eq., 0.05 mL, 0.30 mmol) and 2-(2,6-dioxopiperidin-3-yl)-4-fluoroisoindolin-1,3-dione (2.0 eq., 33 mg, 0.12 mmol). The system was purged with argon gas for three times, and reacted overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide 2-(2,6-dioxopiperidin-3-yl)-5-(1r,3r)-3-(4-(2-(4-(5-(5-methyl-1,3,4-oxadiazol-2-yl)pyridin-2-yl)oxy)phenyl)propan-2-yl)phenoxy)cyclobutyl)amino)isoindolin-1,3-dione (39 mg, 0.054 mmol, 26%). ¹H NMR (400 MHz, CDCl₃) *δ* 8.81 (s, 1H), 8.32 (d, *J* = 8.5 Hz, 1H), 8.00 (s, 1H), 7.64 (d, *J* = 8.2 Hz, 1H), 7.33 - 7.26 (m, 2H), 7.18 (d, *J* = 8.4 Hz, 2H), 7.07 (d, *J* = 8.0 Hz, 2H), 7.01 (d, *J* = 8.0 Hz, 1H), 6.90 (s, 1H), 6.72 (d, *J* = 8.6 Hz, 3H), 4.93 (dd, *J* = 12.3, 5.2 Hz, 2H), 4.24 (s, 1H), 2.98 - 2.66 (m, 6H), 2.63 (s, 3H), 2.43 (s, 2H), 2.16 - 2.08 (m, 1H), 1.69 (s, 6H). LC/MS (ESI+) calcd for C₄₀H₃₆N₆O₇ ([M+H]⁺) *m*/*z* 713.26, found 713.20.

### Example 217: 5-((1r,3r)-3-(4-(2-(4-((6-(2H-1,2,3-triazol-2-yl)pyridazine-4-yl)oxy) phenyl)propan-2-yl)phenoxy)cyclobutyl)amino)-2-(2,6-dioxopiperidin-3-yl) isoindolin-1,3-dione

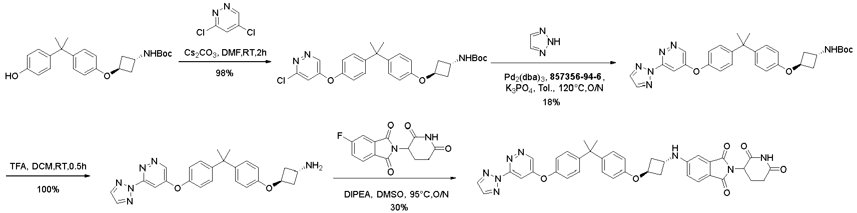

### Step 1: Synthesis of tert-butyl ((1r,3r)-3-(4-(2-(4-((6-chloropyridin-4-yl)oxy)phenyl) propan-2-yl)phenoxy)cyclobutyl)carbamate

tert-butyl ((1r,3r)-3-(4-(2-(4-hydroxylphenyl)propan-2-phenoxy)cyclobutyl) carbamate (230 mg, 0.58 mmol) was dissolved in 20 mL of anhydrous DMF, to which were added 3,5-dichloropyridazine (2.0 eq., 170 mg, 1.16 mmol) and cesium carbonate (2.0 eq., 380 mg, 1.16 mmol), and then the reaction solution was stirred at room temperature for 2 h. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide tert-butyl ((1r,3r)-3-(4-(2-(4-((6-chloropyridin-4-yl)oxy)phenyl)propan-2-yl) phenoxy)cyclobutyl)carbamate (290 mg, 0.56 mmol, 98%). LC/MS (ESI+) calcd for C₂₈H₃₂ClN₃O₄ ([M+H]⁺) *m*/*z* 510.21, found 510.10.

### Step 2: Synthesis of tert-butyl ((1r,3r)-3-(4-(2-(4-((6-(2H-1,2,3-triazol-2-yl)pyridazine-4-yl)oxy)phenyl)propan-2-yl)phenoxy)cyclobutyl)carbamate

tert-butyl ((1r,3r)-3-(4-(2-(4-((6-chloropyridin-4-yl)oxy)phenyl)propan-2-yl) phenoxy)cyclobutyl)carbamate (290 mg, 0.56 mmol) was dissolved in anhydrous toluene (20 mL), to which were added potassium phosphate (2.0 eq., 240 mg, 1.12 mmol), tris(dibenzylideneacetone)dipalladium (0.10 eq., 53 mg, 0.058 mmol), ligand 2-Di-tert-butylphosphine-3,4,5,6-tetramethyl-2',4',6'-triisopropylbiphenyl (857356-94-6, 0.2 eq., 0.054 g, 0.11mmol), and 2H-triazole (2.0 eq., 77 mg, 1.12 mmol), and then the reaction solution was refluxed overnight at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was dissolved in 10 mL of anhydrous DCM, to which was added 2 mL of TFA in an ice-water bath. The reaction solution was warmed to room temperature and stirred for 30 min. 20 mL of saturated NaHCO₃ solution was added to quench the reaction, and the resultant solution was extracted with DCM (3*40 mL), followed by suction filtration. The filtrate was dried with anhydrous Na₂SO₄, and then rotatory evaporated under reduced pressure to dry. The residue was separated by TLC, to provide tert-butyl ((1r,3r)-3-(4-(2-(4-((6-(2H-1,2,3-triazol-2-yl)pyridazine-4-yl)oxy)phenyl)propan-2-yl) phenoxy)cyclobutyl)carbamate (56 mg, 0.10 mmol, 18%). LC/MS (ESI+) calcd for C₃₀H₃₄N₆O₄ ([M+H]⁺) *m*/*z* 543.26, found 543.10.

### Step 3: Synthesis of (1r,3r)-3-(4-(2-(4-((6-(2H-1,2,3-triazol-2-yl)pyridazine-4-yl)oxy)phenyl)propan-2-yl)phenoxy)cyclobutylamine

tert-butyl ((1r,3r)-3-(4-(2-(4-((6-(2H-1,2,3-triazol-2-yl)pyridazine-4-yl)oxy) phenyl)propan-2-yl)phenoxy)cyclobutyl)carbamate (56 mg, 0.10 mmol) was dissolved in 10 mL of anhydrous DCM, to which was added 2 mL of TFA in an ice-water bath. The reaction solution was warmed to room temperature and stirred for 30 min. 20 mL of saturated NaHCO₃ solution was added to quench the reaction. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-(4-(2-(4-((6-(2H-1,2,3-triazol-2-yl)pyridazine-4-yl)oxy)phenyl)propan-2-yl)phenoxy)cyclobutylamine (45 mg, 0.10 mmol, 100%). LC/MS (ESI+) calcd for C₂₅H₂₆N₆O₂ ([M+H]⁺) *m*/*z* 443.21, found 443.10.

### Step 4: Synthesis of 5-((1r,3r)-3-(4-(2-(4-((6-(2H-1,2,3-triazol-2-yl)pyridazine-4-yl)oxy)phenyl)propan-2-yl)phenoxy)cyclobutyl)amino)-2-(2,6-dioxopiperidin-3-yl) isoindolin-1,3-dione

(1r,3r)-3-(4-(2-(4-((6-(2H-1,2,3-triazol-2-yl)pyridazine-4-yl)oxy)phenyl)propan-2-yl)phenoxy)cyclobutylamine (45 mg, 0.10 mmol) was dissolved in 20 mL of anhydrous DMSO, to which were added DIPEA (4.0 eq., 0.07 mL, 0.40 mmol) and 2-(2,6-dioxopiperidin-3-yl)-4-fluoroisoindolin-1,3-dione (2.0 eq., 110 mg, 0.40 mmol). The system was purged with argon gas for three times, and reacted overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the target compound 5-((1r,3r)-3-(4-(2-(4-((6-(2H-1,2,3-triazol-2-yl)pyridazine-4-yl)oxy)phenyl)propan-2-yl)phenoxy)cyclobutyl)amino)-2-(2,6-dioxopiperidin-3-yl)isoindolin-1,3-dione (21 mg, 0.03 mmol, 30%) as yellow solid. ¹H NMR (400 MHz, CDCl₃) *δ* 9.78 (d, *J* = 2.0 Hz, 1H), 8.00 (d, *J* = 5.2 Hz, 2H), 7.92 (s, 1H), 7.85 (d, *J* = 2.0 Hz, 1H), 7.64 (d, *J* = 8.3 Hz, 1H), 7.33 - 7.27 (m, 2H), 7.21 - 7.15 (m, 2H), 7.15 -7.10 (m, 2H), 6.92 (d, *J* = 2.0 Hz, 1H), 6.80 - 6.52 (m, 3H), 5.02 - 4.80 (m, 2H), 4.35 - 4.18 (m, 1H), 2.99 - 2.63 (m, 5H), 2.50 - 2.33 (m, 2H), 2.17 - 2.12 (m, 2H), 1.69 (s, 6H). LC/MS (ESI+) calcd for C₃₈H₃₄N₈O₆ ([M+H]⁺) m/z 699.26, found 699.20.

### Example 218: 2-(2,6-dioxopiperidin-3-yl)-5-(1r,3r)-3-(4-(2-(4-(5-(5-methyl-1,3,4-oxadiazol-2-yl)pyridazine-3-yl)oxy)phenyl)propan-2-yl)phenoxy)cyclobutyl) amino)isoindolin-1,3-dione

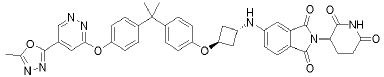

The target compound was synthesized by a method similar to that of Example 216. ¹H NMR (400 MHz, CDCl₃) *δ* 9.50 (s, 1H), 8.00 (s, 1H), 7.68 (d, *J* = 1.4 Hz, 1H), 7.64 (d, *J* = 8.2 Hz, 1H), 7.29 (d, *J* = 8.7 Hz, 2H), 7.15 (dd, *J* = 20.4, 8.7 Hz, 4H), 6.90 (s, 1H), 6.72 (d, *J* = 8.7 Hz, 3H), 5.02 - 4.80 (m, 2H), 4.24 (s, 1H), 2.97 - 2.78 (m, 2H), 2.74 (dd, *J* = 17.5, 4.6 Hz, 4H), 2.70 (s, 3H), 2.48 - 2.34 (m, 2H), 2.17 - 2.08 (m, 1H), 1.69 (s, 6H). LC/MS (ESI+) calcd for C₃₉H₃₅N₇O₇ ([M+H]⁺) *m*/*z* 714.26, found 714.10.

### Example 219: 2-(2,6-dioxopiperidin-3-yl)-5-((1r,3r)-3-((6-(1-(4-((2-(1-hydroxylethyl)pyrimidin-5-yl)oxy)phenyl)ethyl)pyridin-3-yl)oxy)cyclobutyl) amino)isoindolin-1,3-dione

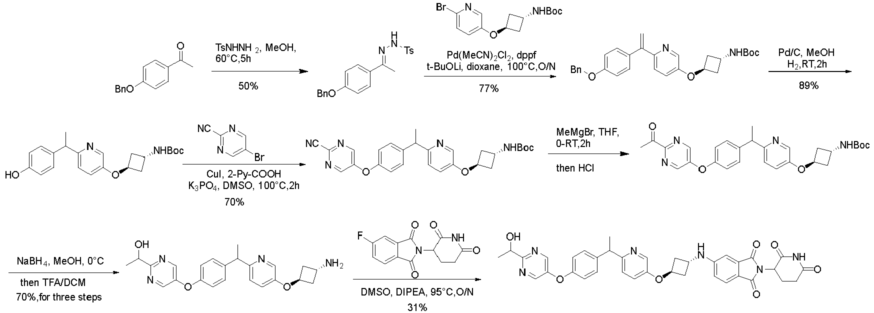

### Step 1: Synthesis of (Z/E)-N'-(1-(4-(benzyloxy)phenyl)ethylene)-4-methylbenzenesulfonhydrazide

4-benzyloxyacetophenone (1.5 g, 6.63 mmol) was dissolved in 30 mL of anhydrous methanol, to which was added p-methylbenzenesulfonhydrazide (1.0 eq., 1.20 g, 6.63 mmol), and then the solution was stirred at 60 °C for 5h. After cooling, the reaction solution was concentrated under reduced pressure to dry. The residue was separated by column chromatography, to provide (*Z*/*E*)-N'-(1-(4-(benzyloxy)phenyl) ethylene)-4-methylbenzenesulfonhydrazide (1.30 g, 3.30 mmol, 50%). LC/MS (ESI+) calcd for C₂₂H₂₂N₂O₃S ([M+H]⁺) *m*/*z* 395.14, found 395.10.

### Step 2: Synthesis of tert-butyl ((1r,3r)-3-((6-(1-(4-(benzyloxy)phenyl)vinyl)pyridin-3-yl)oxy)cyclobutyl)carbamate

(*Z*/*E*)-N'-(1-(4-(benzyloxy)phenyl)ethylene)-4-methylbenzenesulfonhydrazide (1.30 g, 3.30 mmol) and tert-butyl ((1r,3r)-3-((6-bromopyridin-3-yl)oxy)cyclobutyl) carbamate (1.0 eq., 1.13 g, 3.30 mmol) were dissolved in 30 mL of anhydrous 1,4-dioxane, to which were added bis(acetonitrile)palladium chloride (0.1eq., 83 mg, 0.33 mmol), DPPF (0.10 eq., 190 mg, 0.33 mmol), and lithium tert-butoxide (2.0 eq., 53 mg, 6.60 mmol), and then the reaction solution was stirred overnight at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separatd by column chromatography, to provide tert-butyl ((1r,3r)-3-((6-(1-(4-(benzyloxy)phenyl)vinyl)pyridin-3-yl)oxy)cyclobutyl)carbamate (1.21 g, 2.54 mmol, 77%). LC/MS (ESI+) calcd for C₂₉H₃₂N₂O₄ ([M+H]⁺) *m*/*z* 473.24, found 473.10. Step 3: Synthesis of tert-butyl ((1r,3r)-3-((6-(1-(4-hydroxylphenyl)ethyl)pyridin-3-yl)oxy)cyclobutyl)carbamate

tert-butyl ((1r,3r)-3-((6-(1-(4-(benzyloxy)phenyl)vinyl)pyridin-3-yl)oxy) cyclobutyl)carbamate (1.21 g, 2.54 mmol) was dissolved in 20 mL of anhydrous methanol, to which was added Pd/C (500 mg), and then the reaction system was purged with hydrogen for three times, followed by reaction under hydrogen atmosphere for 2h. The reaction solution was subjected to suction filtration over diatomite. The filtrate was concentrated to dry under reduced pressure. The residue was separated by column chromatography, to provide tert-butyl ((1r,3r)-3-((6-(1-(4-hydroxylphenyl) ethyl)pyridin-3-yl)oxy)cyclobutyl)carbamate (874 mg, 2.27 mmol, 89%). LC/MS (ESI+) calcd for C₂₂H₂₈N₂O₄ ([M+H]⁺) *m*/*z* 385.20, found 385.10.

### Step 4: Synthesis of tert-butyl ((1r,3r)-3-((6-(1-(4-((2-cyanopyrimidin-5-yl)oxy)phenyl) ethyl)pyridin-3-yl)oxy)cyclobutyl)carbamate

tert-butyl ((1r,3r)-3-((6-(1-(4-hydroxylphenyl)ethyl)pyridin-3-yl)oxy)cyclobutyl) carbamate (874 mg, 2.27 mmol) was dissolved in 20 mL of anhydrous DMSO, to which were added 5-bromo-2-cyanopyrimidine (2.0 eq., 830 mg, 4.54 mmol), CuI (0.30 eq., 130 mg, 0.68 mmol), 2-picolinic acid (0.3 eq., 830 mg, 0.68 mmol), and potassium phosphate (2.0 eq., 960 mg, 4.54 mmol). The system was purged with argon gas for three times, and reacted for 2 h under stirring at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide tert-butyl ((1r,3r)-3-((6-(1-(4-((2-cyanopyrimidin-5-yl)oxy)phenyl)ethyl)pyridin-3-yl)oxy)cyclobutyl)carbamate (670 mg, 1.38 mmol, 60%). LC/MS (ESI+) calcd for C₂₇H₂₉N₅O₄ ([M+H]⁺) *m*/*z* 488.22, found 488.10.

### Steps 5, 6, and 7: Synthesis of 1-(5-(4-(1-(5-((1r,3r)-3-aminocyclobutyloxy)pyridin-2-yl)ethyl)phenoxy)pyrimidin-2-yl)ethane-1-ol

tert-butyl ((1r,3r)-3-((6-(1-(4-((2-cyanopyrimidin-5-yl)oxy)phenyl)ethyl)pyridin-3-yl)oxy)cyclobutyl)carbamate (520 mg, 1.06 mmol) was dissolved in 20 mL of anhydrous THF, to which was added methylmagnesium bromide (4.0 eq., 3.0 M, 1.5 mL) in an ice-water bath, and then the mixture was warmed to room temperature and stirred for 2 h. 50 mL of diluted hydrochloric acid (0.05 M) was added to quench the reaction. The resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide the crude product tert-butyl ((1r,3r)-3-((6-(1-(4-((2-acetylpyrimidin-5-yl) oxy)phenyl)ethyl)pyridin-3-yl)oxy)cyclobutyl)carbamate, which was dissolved in 20 mL of anhydrous methanol, followed by slow addition of sodium borohydride (2.0 eq., 78 mg, 2.12 mmol) in an ice-water bath. The mixture was allowed to react for 30 min. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, followed by concentration to dry under reduced pressure. To the residue, was added 10 mL of anhydrous DCM, and then 2 mL of TFA was added in an ice-water bath. The reaction was stirred at room temperature for 30 min, and quenched by adding 20 mL of saturated NaHCO₃ solution. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide 1-(5-(4-(1-(5-((1r,3r)-3-aminocyclobutyloxy)pyridin-2-yl)ethyl)phenoxy) pyrimidin-2-yl)ethane-1-ol (300 mg, 0.74 mmol, 70% for three steps). LC/MS (ESI+) calcd for C₂₃H₂₆N₄O₃ ([M+H]⁺) *m*/*z* 407.20, found 407.10.

### Step 8: Synthesis of 2-(2,6-dioxopiperidin-3-yl)-5-((1r,3r)-3-((6-(1-(4-((2-(1-hydroxylethyl)pyrimidin-5-yl)oxy)phenyl)ethyl)pyridin-3-yl)oxy)cyclobutyl)amino) isoindolin-1,3-dione

1-(5-(4-(1-(5-((1r,3r)-3-aminocyclobutyloxy)pyridin-2-yl)ethyl)phenoxy) pyrimidin-2-yl)ethane-1-ol (300 mg, 0.74 mmol) was dissolved in 30 mL of anhydrous DMSO, to which were added DIPEA (4.0eq., 0.49 mL, 2.96 mmol) and 2-(2,6-dioxopiperidin-3-yl)-4-fluoroisoindolin-1,3-dione (2.0 eq., 410 mg, 1.48 mmol). The system was purged with argon gas for three times, and reacted overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the target compound 2-(2,6-dioxopiperidin-3-yl)-5-((1r,3r)-3-((6-(1-(4-((2-(1-hydroxylethyl)pyrimidin-5-yl)oxy)phenyl)ethyl)pyridin-3-yl)oxy)cyclobutyl)amino)isoindolin-1,3-dione (150 mg, 0.23 mmol, with a yield of 31%). ¹H NMR (400 MHz, CDCl₃) *δ* 8.44 (s, 2H), 8.30 (s, 1H), 8.19 (s, 1H), 7.59 (t, *J* = 20.7 Hz, 1H), 7.34 (d, *J* = 8.6 Hz, 2H), 7.16 (d, *J* = 8.8 Hz, 2H), 6.99 (d, *J* = 8.6 Hz, 2H), 6.88 (d, *J* = 1.8 Hz, 1H), 6.71 (dd, *J* = 8.3, 2.0 Hz, 1H), 5.03 (s, 1H), 5.00 - 4.87 (m, 3H), 4.41 (d, *J* = 6.9 Hz, 1H), 4.24 (s, 1H), 2.94 - 2.66 (m, 6H), 2.53 - 2.37 (m, 2H), 2.16 - 2.09 (m, 1H), 1.72 (d, *J* = 7.2 Hz, 3H), 1.56 (d, *J* = 6.6 Hz, 3H). LC/MS (ESI+) calcd for C₃₆H₃₄N₆O₇ ([M+H]⁺) *m*/*z* 663.25, found 663.10.

### Example 220: Synthesis of 5-((1r,3r)-3-((6-(1-(4-((2-acetylpyrimidin-5-yl)oxy)phenyl)ethyl)pyridin-3-yl)oxy)cyclobutyl)amino)-2-(2,6-dioxopyridin-3-yl)isoindolin-1,3-dione

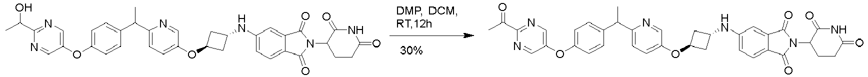

2-(2,6-dioxopiperidin-3-yl)-5-((1r,3r)-3-((6-(1-(4-((2-(1-hydroxylethyl) pyrimidin-5-yl)oxy)phenyl)ethyl)pyridin-3-yl)oxy)cyclobutyl)amino)isoindolin-1,3-dione (100 mg, 0.15 mmol) was dissolved in 20 mL of anhydrous DCM, to which was added DMP (3.0 eq., 130 mg, 0.30 mmol) in an ice-water bath, and then the mixture was warmed to room temperature and stirred overnight. 50 mL of saturated NaHCO₃ solution was added to quench the reaction. The resultant solution was extracted with ethy acetate (3*40 mL), followed by suction filtration. The filtrate was dried with anhydrous Na₂SO₄, and then rotatory evaporated under reduced pressure to dry. The residue was separated by TLC, to provide the target compound, 5-((1r,3r)-3-((6-(1-(4-((2-acetylpyrimidin-5-yl)oxy)phenyl)ethyl)pyridin-3-yl)oxy)cyclobutyl)amino*)*-2-(2,6-dioxopyridin-3-yl)isoindolin-1,3-dione (30 mg, 0.045 mmol, with a yield of 30%). ¹H NMR (400 MHz, CDCl₃) *δ* 8.54 (s, 2H), 8.20 (s, 1H), 8.13 (s, 1H), 7.63 (d, *J* = 8.3 Hz, 1H), 7.39 (d, *J* = 8.6 Hz, 2H), 7.17 (s, 2H), 7.05 (d, *J* = 8.6 Hz, 2H), 6.88 (d, *J* = 1.9 Hz, 1H), 6.71 (dd, *J* = 8.2, 2.1 Hz, 1H), 4.94 (dd, *J* = 12.0, 5.2 Hz, 3H), 4.42 (d, *J* = 6.7 Hz, 1H), 4.25 (s, 1H), 2.97 - 2.67 (m, 8H), 2.54 - 2.39 (m, 2H), 2.11 (dd, *J* = 24.2, 14.1 Hz, 1H), 1.73 (d, *J* = 7.2 Hz, 3H). LC/MS (ESI+) calcd for C₃₆H₃₂N₆O₇ ([M+H]⁺) *m*/*z* 661.23, found 661.10.

### Example 221: 2-(2,6-dioxopiperidin-3-yl)-5-((1r,3r)-3-((6-(2-(4-((2-(5-methyl-1,2,4-oxadiazol-3-yl)pyrimidin-5-yl)oxy)phenyl)propan-2-yl)pyridin-3-yl)oxy) cyclobutyl)amino)isoindolin-1,3-dione

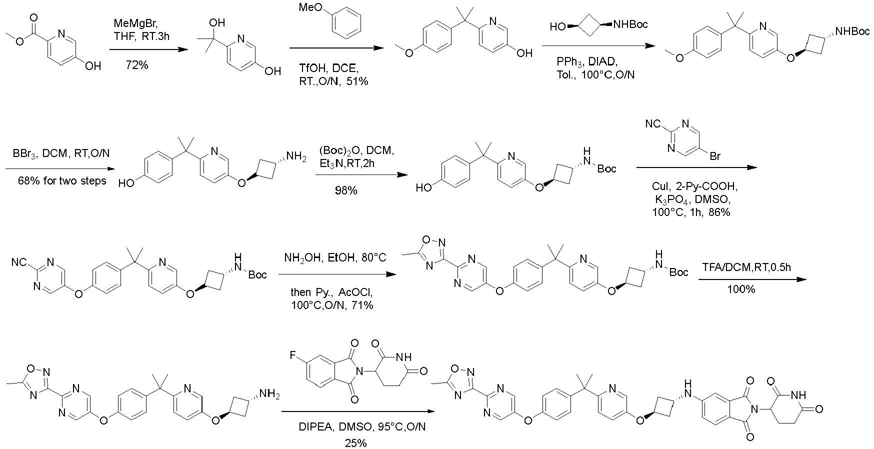

### Step 1: Synthesis of 6-(2-hydroxylpropan-2-yl)pyridin-3-ol

Methyl 5-hydroxyl-2-picolinate (1.00 g, 6.53 mmol) was dissolved in 30 mL of anhydrous THF, to which was slowly added methylmagnesium bromide (4.0 eq., 3.0 M, 1.5 mL) dropwise in an ice-water bath, and then the mixture was warmed to room temperature and stirred for 3 h. 50 mL of saturated NH₄Cl solution was added to quench the reaction. The resultant solution was extracted with ethyl acetate (3*40 mL), followed by suction filtration. The filtrate was dried with anhydrous Na₂SO₄, and then rotatory evaporated to dry under reduced pressure. The residue was separated by column chromatography, to provide 6-(2-hydroxylpropan-2-yl)pyridin-3-ol (720 mg, 4.7 mmol, 72%).

### Step 2: Synthesis of 6-(2-(4-methoxyphenyl)propan-2-yl)pyridin-3-ol

Compound 6-(2-hydroxylpropan-2-yl)pyridin-3-ol (360 mg, 2.40 mmol) was dissolved in 10 mL of anhydrous DCE, to which was added anisole (5.0 eq., 1.27 g, 12.0 mmol), followed by carefully adding TfDH (5.0 eq., 1.0 mL, 12.0 mmol) in an ice-water bath, and then the mixture was warmed to room temperature and stirred overnight. 50 mL of saturated NaHCO₃ solution was added to quench the reaction. The resultant solution was extracted with ethy acetate (3*40 mL), followed by suction filtration. The filtrate was dried with anhydrous Na₂SO₄, and then rotatory evaporated under reduced pressure to dry. The residue was separated by column chromatography, to provide 6-(2-(4-methoxyphenyl)propan-2-yl)pyridin-3-ol (300 mg, 1.23 mmol, 51%). LC/MS (ESI+) calcd for C₁₅H₁₇NO₂ ([M+H]⁺) *m*/*z* 244.13, found 244.10.

### Steps 3 and 4: Sythesis of 4-(2-(5-((1s,3s)-3-aminocyclobutyloxy)pyridin-2-yl)propan-2-yl)phenol

6-(2-(4-methoxyphenyl)propan-2-yl)pyridin-3-ol (300 mg, 1.23 mmol) and *cis*-3-BOC-aminocyclobutanol (1.2 eq., 280 mg, 1.50 mmol) were dissolved in 20 mL of anhydrous toluene, to which was added triphenylphosphine (2.0 eq., 640 mg, 2.46 mmol), followed by drop addition of DIAD (2.0 eq., 0.45 mL, 2.46 mmol) in an ice-water bath. The system was purged with argon gas for three times, and reacted overnight at 100 °C. The reaction solution was rotatory evaporated to dry. The residue was separated by column chromatography, to provide tert-butyl ((1s,3s)-3-((6-(2-(4-methoxyphenyl)propan-2-yl)pyridin-3-yl)oxy)cyclobutyl)carbamate, which was dissolved in 20 mL of anhydrous DCM, to which was added BBr₃ (3.0 eq., 0.35 mL, 3.69 mmol) dropwise in an ice-water bath, and then the mixture was warmed to room temperature and stirred overnight. 50 mL of saturated NaHCO₃ solution was added to quench the reaction. The resultant solution was extracted with DCM (3 *40 mL), followed by suction filtration. The filtrate was dried with anhydrous Na₂SO₄, and then rotatory evaporated under reduced pressure to dry. The residue was separated by TLC, to provide 4-(2-(5-((1s,3s)-3-aminocyclobutyloxy)pyridin-2-yl)propan-2-yl)phenol (250 mg, 0.84 mmol, 68% for two steps). LC/MS (ESI+) calcd for C₁₈H₂₂N₂O₂ ([M+H]⁺) *m*/*z* 299.17, found 299.10.

### Step 5: Synthesis of tert-butyl ((1s,3s)-3-((6-(2-(4-hydroxylphenyl)propan-2-yl) pyridin-3-yl)oxy)cyclobutyl)carbamate

4-(2-(5-((1s,3s)-3-aminocyclobutyloxy)pyridin-2-yl)propan-2-yl)phenol (250 mg, 0.84 mmol) was dissolved in 20 mL of anhydrous DCM, to which was added triethylamine (3.0 eq., 0.35 mL, 2.52 mmol), followed by addition of (Boc)₂O in an ice-water bath, and then the reaction solution was stirred at room temperature for 3h. 50 mL of saturated NaHCO₃ solution was added to quench the reaction. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide tert-butyl ((1s,3s)-3-((6-(2-(4-hydroxylphenyl)propan-2-yl)pyridin-3-yl)oxy)cyclobutyl)carbamate (330 mg, 0.83 mmol, 98%). LC/MS (ESI+) calcd for C₂₃H₃₀N₂O₄ ([M+H]⁺) *m*/*z* 399.22, found 399.10.

### Step 6: Synthesis of tert-butyl ((1s,3s)-3-((6-(2-(4-((2-cyanopyrimidin-5-yl)oxy)phenyl)propan-2-yl)pyridin-3-yl)oxy)cyclobutyl)carbamate

tert-butyl ((1s,3s)-3-((6-(2-(4-hydroxylphenyl)propan-2-yl)pyridin-3-yl)oxy) cyclobutyl)carbamate (330 mg, 0.83 mmol) was dissolved in 20 mL of anhydrous DMSO, to which were added 5-bromo-2-cyanopyrimidine (2.0 eq., 310 mg, 1.66 mmol), CuI (0.30 eq., 47 mg, 0.25 mmol), 2-picolinic acid (0.3 eq., 31 mg, 0.25 mmol), and potassium phosphate (2.0 eq., 350 mg, 1.66 mmol). The system was purged with argon gas for three times, and reacted for 1h under stirring at 100 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide tert-butyl ((1s,3s)-3-((6-(2-(4-((2-cyanopyrimidin-5-yl)oxy)phenyl)propan-2-yl)pyridin-3-yl)oxy)cyclobutyl) carbamate (360 mg, 0.72 mmol, 86%). LC/MS (ESI+) calcd for C₂₈H₃₁N₅O₄ ([M+H]⁺) *m*/*z* 502.24, found 502.10.

### Step 7: Synthesis of tert-butyl ((1r,3r)-3-((6-(2-(4-((2-(5-methyl-1,2,4-oxadiazol-3-yl)pyrimidin-5-yl)oxy)phenyl)propan-2-yl)pyridin-3-yl)oxy)cyclobutyl)carbamate

tert-butyl ((1s,3 s)-3 -((6-(2-(4-((2-cyanopyrimidin- 5 -yl)oxy)phenyl)propan-2-yl) pyridin-3-yl)oxy)cyclobutyl)carbamate (100 mg, 0.20 mmol) was dissolved in 10 mL of absolute ethanol, to which was added hydroxylamine aqueous solution (2.0 eq., 50%, 0.03 mL, 0.40 mmol), and then the reaction solution was stirred at 80 °C for 1h, and then rotatory evaporated to dry under reduced pressure, followed by extraction with EA (3*20 mL). The resultant solution was dried with anhydrous Na₂SO₄, suction filtered, and then rotatory evaporated to dry, to provide the crude product. Then, 10 mL of pyridine was added, followed by careful addition of acetyl chloride (2.0 eq., 0.03 Ml, 0.40 mmol) in an ice-water bath, and then the mixture was allowed to react overnight at 100 °C. 0.05 M diluted hydrochloric acid (100 mL) was added to quench the reaction. The resultant solution was extracted with EA (4*30mL). The organic phase was dried with Na₂SO₄, and concentrated to dry under reduced pressure. The residue was separated by column chromatography, to provide tert-butyl ((1r,3r)-3-((6-(2-(4-((2-(5-methyl-1,2,4-oxadiazol-3-yl)pyrimidin-5-yl)oxy)phenyl)propan-2-yl)pyridin-3-yl)oxy)cyclobutyl)carbamate (790 mg, 0.14 mmol, 71%). LC/MS (ESI+) calcd for C₃₀H₃₄N₆O₅ ([M+H]⁺) *m*/*z* 559.26, found 559.20.

### Step 8: Synthesis of (1r,3r)-3-((6-(2-(4-((2-(5-methyl-1,2,4-oxadiazol-3-yl)pyrimidin-5-yl)oxy)phenyl)propan-2-yl)pyridin-3-yl)oxy)cyclobutylamine

tert-butyl ((1r,3r)-3-((6-(2-(4-((2-(5-methyl-1,2,4-oxadiazol-3-yl)pyrimidin-5-yl) oxy)phenyl)propan-2-yl)pyridin-3-yl)oxy)cyclobutyl)carbamate (79 mg, 0.14 mmol) was dissolved in 10 mL of anhydrous DCM, to which was added 2 mL of TFA in an ice-water bath. The reaction was warmed to room temperature, stirred for 30 min, and then quenched by adding 20 mL of saturated NaHCO₃ solution. The resultant solution was extracted with DCM (3*40 mL), followed by suction filtration. The filtrate was dried with anhydrous Na₂SO₄, and then rotatory evaporated under reduced pressure to dry. The residue was separated by TLC, to provide (1r,3r)-3-((6-(2-(4-((2-(5-methyl-1,2,4-oxadiazol-3-yl)pyrimidin-5-yl)oxy)phenyl)propan-2-yl)pyridin-3-yl)oxy) cyclobutylamine (71 mg, 0.15 mmol, 100%). LC/MS (ESI+) calcd for C₂₅H₂₆N₆O₃ ([M+H]⁺) *m*/*z* 459.21, found 459.10.

### Step 9: Synthesis of 2-(2,6-dioxopiperidin-3-yl)-5-((1r,3r)-3-((6-(2-(4-((2-(5-methyl-1,2,4-oxadiazol-3-yl)pyrimidin-5-yl)oxy)phenyl)propan-2-yl)pyridin-3-yl)oxy) cyclobutyl)amino)isoindolin-1,3-dione

(1r,3r)-3-((6-(2-(4-((2-(5-methyl-1,2,4-oxadiazol-3-yl)pyrimidin-5-yl)oxy)phenyl) propan-2-yl)pyridin-3-yl)oxy)cyclobutylamine (71 mg, 0.15 mmol) was dissolved in 20 mL of anhydrous DMSO, to which were added DIPEA (4.0 eq., 0.10 mL, 0.60 mmol) and 2-(2,6-dioxopiperidin-3-yl)-4-fluoroisoindolin-1,3-dione (2.0 eq., 85 mg, 0.30 mmol). The system was purged with argon gas for three times, and reacted overnight at 95 °C. 50 mL of saturated NH₄Cl solution was added to quench the reaction, and the resultant solution was extracted with ethyl acetate (3*40 mL), followed by suction filtration. The filtrate was dried with anhydrous Na₂SO₄, and then rotatory evaporated to dry under reduced pressure. The residue was separated by TLC, to provide the target compound 2-(2,6-dioxopiperidin-3-yl)-5-((1r,3r)-3-((6-(2-(4-((2-(5-methyl-1,2,4-oxadiazol-3-yl)pyrimidin-5-yl)oxy)phenyl)propan-2-yl)pyridin-3-yl)oxy)cyclobutyl) amino)isoindolin-1,3-dione (27 mg, 0.038 mmol, 25%). ¹H NMR (400 MHz, CDCl₃) δ 8.60 (s, 2H), 8.16 (d, *J* = 2.3 Hz, 1H), 7.64 (d, *J* = 8.3 Hz, 1H), 7.35 - 7.27 (m, 2H), 7.11 - 6.98 (m, 4H), 6.90 (d, *J* = 2.1 Hz, 1H), 6.72 (dd, *J* = 8.3, 2.2 Hz, 1H), 4.99 - 4.86 (m, 2H), 4.78 (d, *J* = 4.9 Hz, 1H), 4.28 (dt, *J* = 12.7, 6.5 Hz, 1H), 2.95 - 2.65 (m, 8H), 2.44 (dt, *J* = 13.4, 6.6 Hz, 2H), 2.13 (ddd, *J* = 10.2, 8.6, 6.2 Hz, 1H), 1.77 - 1.69 (m, 6H). LC/MS (ESI+) calcd for C₃₈H₃₄N₈O₇ ([M+H]⁺) *m*/*z* 715.26, found 715.20.

### Example 222: 5-((1r,3r)-3-((6-(2-(4-((2-acetylpyrimidin-5-yl)oxy)phenyl)propan-2-yl)pyridin-3-yl)oxy)cyclobutyl)amino)-2-(2,6-dioxopyridin-3-yl)isoindolin-1,3-dione

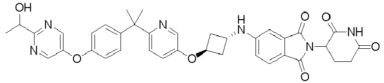

The target compound was synthesized by a method similar to that of Example 219. ¹H NMR (400 MHz, CDCl₃) *δ* 8.54 (s, 2H), 8.17 (d, *J* = 2.4 Hz, 1H), 7.64 (d, *J* = 8.3 Hz, 1H), 7.35 - 7.28 (m, 2H), 7.12 - 6.97 (m, 4H), 6.90 (d, *J* = 2.0 Hz, 1H), 6.72 (dd, *J* = 8.3, 2.2 Hz, 1H), 4.92 (dt, *J* = 6.5, 4.6 Hz, 2H), 4.75 (d, *J* = 4.9 Hz, 1H), 4.27 (d, *J* = 4.4 Hz, 1H), 2.93 - 2.68 (m, 8H), 2.51 - 2.35 (m, 2H), 2.19 - 2.08 (m, 1H), 1.75 (s, 6H). LC/MS (ESI+) calcd for C₃₇H₃₄N₆O₇ ([M+H]⁺) *m*/*z* 675.25, found 675.20.

### Example 223: 2-(2,6-dioxopiperidin-3-yl)-5-((1r,3r)-3-((6-(2-(4-((2-(1-hydroxylethyl)pyrimidin-5-yl)oxy)phenyl)propan-2-yl)pyridin-3-yl)oxy) cyclobutyl)amino)isoindolin-1,3-dione

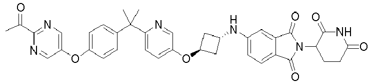

The target compound was synthesized by a method similar to that of Example 220. ¹H NMR (400 MHz, CDCl₃) *δ* 8.45 (s, 2H), 8.16 (d, *J* = 2.8 Hz, 1H), 7.64 (d, *J* = 8.3 Hz, 1H), 7.32 - 7.17 (m, 2H), 7.12 - 6.85 (m, 4H), 6.89 (d, *J* = 2.1 Hz, 1H), 6.72 (dd, *J* = 8.3, 2.2 Hz, 1H), 4.99 - 4.86 (m, 3H), 4.77 (d, *J* = 5.0 Hz, 1H), 4.27 (dt, *J* = 12.8, 6.4 Hz, 1H), 2.96 - 2.67 (m, 5H), 2.50 - 2.35 (m, 2H), 2.13 (ddd, *J* = 10.1, 8.7, 6.4 Hz, 1H), 1.73 (s, 6H), 1.56 (d, *J* = 6.6 Hz, 3H). LC/MS (ESI+) calcd for C₃₇H₃₆N₆O₇ ([M+H]⁺) *m*/*z* 677.26, found 676.20.

### Example 224: 2-(2,6-dioxopiperidin-3-yl)-4-((3-(4-(2-(4-((2-methylthiazol-4-yl) methoxy)phenyl)propan-2-yl)phenoxy)propyl)amino)isoindolin-1,3-dione

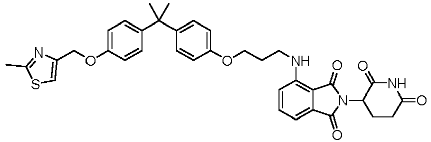

The target compound was synthesized by a method similar to that of Example 137. LC/MS (ESI+) calcd for C₃₆H₃₇N₄O₆S⁺ ([M+H]⁺) *m*/*z:* 653.2; found 653.2. ¹H NMR (400 MHz, CDCl₃) *δ* 8.14 (s, 1H), 7.46 (t, *J* = 7.8 Hz, 1H), 7.19 - 7.11 (m, 4H), 7.09 (d, *J* = 7.1 Hz, 1H), 6.92 (d, *J* = 8.6 Hz, 1H), 6.88 (d, *J* = 8.4 Hz, 2H), 6.86 - 6.78 (m, 2H), 6.46 (s, 1H), 5.15 (s, 2H), 4.91 (dd, *J* = 12.0, 5.3 Hz, 1H), 4.07 (t, *J* = 5.6 Hz, 2H), 3.51 (d, *J* = 6.6 Hz, 2H), 2.89 (d, *J* = 17.4 Hz, 1H), 2.77 (d, *J* = 6.1 Hz, 5H), 2.15 - 2.10 (m, 2H), 2.03 (dd, *J* = 9.8, 5.1 Hz, 1H), 1.63 (s, 6H).

### Example 225 : 2-(2,6-dioxopiperidin-3-yl)-5-((3-(4-(2-(4-((2-methylthiazol-4-yl) methoxy)phenyl)propan-2-yl)phenoxy)propyl)amino)isoindolin-1,3-dione

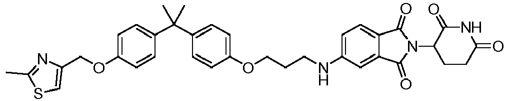

The target compound was synthesized by a method similar to that of Example 137. LC/MS (ESI+) calcd for C₃₆H₃₇N₄O₆S⁺ ([M+H]⁺) *m*/*z:* 653.2; found 653.2. ¹H NMR (400 MHz, CDCl₃) *δ* 8.12 (s, 1H), 7.60 (d, *J* = 8.2 Hz, 1H), 7.22 (s, 1H), 7.15 (dd, *J* = 8.4, 5.5 Hz, 4H), 6.98 (d, *J* = 2.0 Hz, 1H), 6.89 (d, *J* = 8.3 Hz, 2H), 6.81 (d, *J* = 8.4 Hz, 2H), 6.75 (dd, *J* = 8.3, 1.8 Hz, 1H), 5.19 (s, 2H), 4.93 (dd, *J* = 12.0, 5.2 Hz, 1H), 4.09 (t, *J* = 5.4 Hz, 2H), 3.47 (t, *J* = 6.4 Hz, 2H), 2.84 (d, *J* = 20.6 Hz, 6H), 2.13 (d, *J* = 5.6 Hz, 3H), 1.64 (s, 6H).

### Example 226: 2-(2,6-dioxopiperidin-3-yl)-5-(4-((8-(4-(2-(2-(oxazol-2-ylamino) pyrimidin-4-yl)methoxy)phenyl)propan-2-yl)phenoxy)octyl)oxy)butyl)amino) isoindolin-1,3-dione

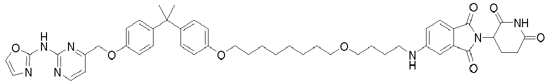

The target compound was synthesized by a method similar to that of Example 28. LC/MS (ESI+) calcd for C₄₈H₅₆N₇O₈⁺ ([M1/2+H]⁺) *m*/*z:* 429.7; found 429.8.

### Example 227 : N-(4-((4-(2-(4-((8-(2-(4-(2,6-dioxopiperidin-3-yl)-1,3-dioxoisoindolin-5-yl)piperazin-1-yl)ethoxy)octyl)oxy)phenyl)propan-2-yl)phenoxy)methyl)pyrimidin-2-yl)acrylamide

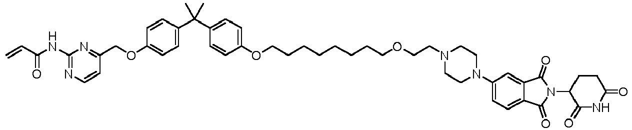

The target compound was synthesized by a method similar to that of Example 26. LC/MS (ESI+) calcd for C₅₀H₆₀N₇O₈⁺ ([M1/2+H]⁺) *m*/*z:* 443.7; found 443.9.

### Example 228 : 5-((3-(4-(2-(4-((2-(1H-1,2,3-triazol-1-yl)pyrimidin-4-yl)methoxy) phenyl)propan-2-yl)phenoxy)propyl)amino)-2-(2,6-dioxopiperidin-3-yl)isoindolin-1,3-dione

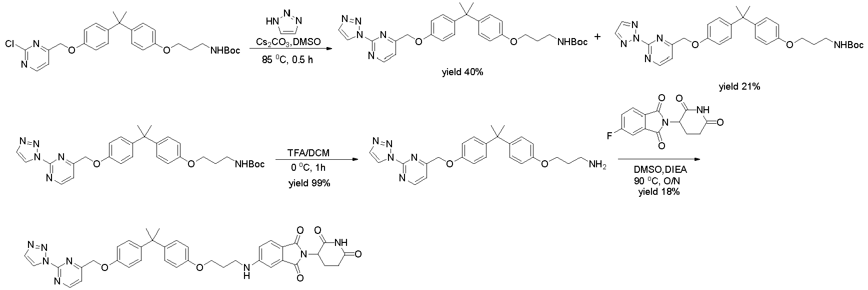

### Step 1: Synthesis of tert-butyl (3-(4-(2-(4-((2-(1H-1,2,3-triazol-1-yl)pyrimidin-4-yl)methoxy)phenyl)propan-2-yl)phenoxy)propyl)carbamate

In a 25 mL round-bottom flask, compound tert-butyl (3-(4-(2-(4-((2-chloropyrimidin-4-yl)methoxy)phenyl)propan-2-yl)phenoxy)propyl)carbamate (200 mg, 0.4 mmol), 1H-1,2,3-triazole (53.0 mg, 0.8 mmol), and cesium carbonate (254 mg, 0.8 mmol) were added into 4 mL of DMSO, and then the solution was heated to 85 °C and stirred for 0.5 h. The reaction solution was cooled to room temperature, and extracted by adding ethyl acetate and water. The organic layers were washed with saturated brine, dried, and rotatory evaporated. The residue was purified by prep.-TLC, to provide compound tert-butyl (3-(4-(2-(4-((2-(1H-1,2,3-triazol-1-yl)pyrimidin-4-yl)methoxy)phenyl)propan-2-yl)phenoxy)propyl)carbamate (85 mg, 0.15 mmol), with a yield of 40%; compound tert-butyl (3-(4-(2-(4-((2-(2H-1,2,3-triazol-2-yl)pyrimidin-4-yl)methoxy)phenyl)propan-2-yl)phenoxy)propyl)carbamate (45 mg, 0.08 mmol), with a yield of 21%.

### Step 2: Synthesis of 3-(4-(2-(4-((2-(1H-1,2,3-triazol-1-yl)pyrimidin-4-yl)methoxy) phenyl)propan-2-yl)phenoxy)propan-1 -amine

In a 25 mL round-bottom flask, compound tert-butyl (3-(4-(2-(4-((2-(1H-1,2,3-triazol-1-yl)pyrimidin-4-yl)methoxy)phenyl)propan-2-yl)phenoxy)propyl)carbamate (85.0 mg, 0.15 mmol) was added into 3 mL of CH₂Cl₂/CF₃COOH (2:1), and then the solution was stirred at 0 °C for 1h, followed by addition of dichloromethane. The pH was adjusted to 8-9 with NaHCO₃. The resultant solution was extracted. The organic phase was washed twice with brine, dried with anhydrous Na2SO4, and rotatory evaporated to dry, to provide the crude product 3-(4-(2-(4-((2-(1H-1,2,3-triazol-1-yl)pyrimidin-4-yl)methoxy)phenyl)propan-2-yl)phenoxy)propan-1-amin (69 mg, 0.15 mmol), with a yield of 99.0%.

### Step 3: Synthesis of 5-((3-(4-(2-(4-((2-(1H-1,2,3-triazol-1-yl)pyrimidin-4-yl)methoxy) phenyl)propan-2-yl)phenoxy)propyl)amino)-2-(2,6-dioxopiperidin-3-yl)isoindolin-1,3-dione

In a 25 mL round-bottom flask, compound (4-(2-(4-((2-(1H-1,2,3-triazol-1-yl)pyrimidin-4-yl)methoxy)phenyl)propan-2-yl)phenoxy)propan-1-amine (69 mg, 0.15 mmol), 2-(2,6-dioxopiperidin-3-yl)-5-fluoroisoindol-1,3-dione (64 mg, 0.23 mmol), and DIEA (51 mg, 0.3 mmol) were added into 3 mL of DMSO, and then the solution was heated to 95 °C and stirred overnight. The reaction solution was cooled to room temperature, and extracted by adding ethyl acetate and water. The organic layer was washed with saturated brine, dried, and rotatory evaporated. The residue was purified by prep.-TLC, to provide5-((3-(4-(2-(4-((2-(1H-1,2,3-triazol-1-yl)pyrimidin-4-yl)methoxy)phenyl)propan-2-yl)phenoxy)propyl)amino)-2-(2,6-dioxopiperidin-3-yl)isoindolin-1,3-dione (20 mg, 0.03 mmol) as yellow solid, with a yield of 18%. LC/MS (ESI+) calcd for C₃₈H₃₇N₈O₆⁺ ([M+H]⁺) *m*/*z:* 701.3; found 701.3. ¹H NMR (400 MHz, CDCl₃) δ 8.85 (d, *J* = 4.9 Hz, 1H), 8.64 (s, 1H), 8.09 (s, 1H), 7.87 (s, 1H), 7.68 (d, *J* = 4.8 Hz, 1H), 7.60 (d, *J* = 8.2 Hz, 1H), 7.17 (t, *J* = 9.1 Hz, 4H), 6.98 (s, 1H), 6.89 (d, *J* = 8.4 Hz, 2H), 6.82 (d, *J* = 8.4 Hz, 2H), 6.74 (d, *J* = 8.1 Hz, 1H), 5.28 (s, 2H), 4.93 (dd, *J* = 12.1, 5.2 Hz, 1H), 4.09 (t, *J* = 5.5 Hz, 2H), 3.46 (t, *J* = 6.5 Hz, 2H), 2.94 - 2.66 (m, 3H), 2.13 (dq, *J* = 9.8, 5.2, 3.6 Hz, 3H), 1.65 (s, 6H).

### Example 229: 5-((3-(4-(2-(4-((2-(2H-1,2,3-triazol-2-yl)pyrimidin-4-yl)methoxy) phenyl)propan-2-yl)phenoxy)propyl)amino)-2-(2,6-dioxopiperidin-3-yl) isoindolin-1,3-dione

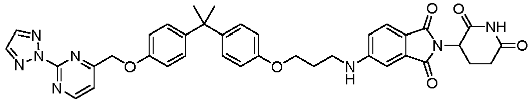

The target compound was synthesized by a method similar to that of Example 228. LC/MS (ESI+) calcd for C₃₈H₃₇N₈O₆⁺ ([M+H]⁺) *m*/*z:* 701.3; found 701.3. ¹H NMR (400 MHz, CDCl₃) *δ* 8.02 (s, 3H), 7.69 (s, 1H), 7.60 (d, *J* = 8.2 Hz, 1H), 7.17 (dd, *J* = 8.8, 6.9 Hz, 4H), 6.98 (d, *J* = 2.0 Hz, 1H), 6.89 (d, *J* = 8.7 Hz, 2H), 6.82 (d, *J* = 8.7 Hz, 2H), 6.75 (d, *J* = 7.7 Hz, 1H), 5.30 (s, 2H), 4.96 - 4.91 (m, 1H), 4.10 (t, *J* = 5.5 Hz, 2H), 3.47 (t, *J* = 6.3 Hz, 2H), 2.91 - 2.73 (m, 3H), 2.15 - 2.10 (m, 2H), 2.02 (s, 1H), 1.65 (s, 6H).

### Example 230: 5-((1r,3r)-3-(4-(2-(4-(2-(1H-1,2,3-triazol-1-yl)pyrimidin-4-yl) methoxy)phenyl)propan-2-yl)phenoxy)cyclobutyl)amino)-2-(2,6-dioxopiperidin-3-yl)isoindolin-1,3-dione

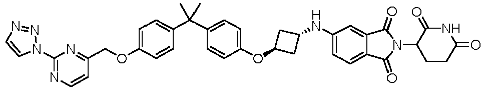

The target compound was synthesized by a method similar to that of Example 228. LC/MS (ESI+) calcd for C₃₉H₃₇N₈O₆⁺ ([M+H]⁺) *m*/*z:* 713.3; found 713.3. ¹H NMR (400 MHz, CDCl₃) *δ* 8.85 (s, 1H), 8.65 (s, 1H), 7.98 (s, 1H), 7.87 (s, 1H), 7.68 (d, *J* = 4.6 Hz, 1H), 7.64 (d, *J* = 8.2 Hz, 1H), 7.19 (d, *J* = 8.6 Hz, 2H), 7.13 (d, *J* = 8.6 Hz, 2H), 6.94 - 6.84 (m, 3H), 6.71 (dd, *J* = 13.7, 8.7 Hz, 3H), 5.28 (s, 2H), 4.93 (dd, *J* = 12.0, 5.2 Hz, 1H), 4.87 (s, 1H), 4.24 (s, 1H), 2.94 - 2.76 (m, 3H), 2.72 (d, *J* = 6.0 Hz, 2H), 2.41 (d, *J* = 9.5 Hz, 2H), 2.12 (d, *J* = 7.5 Hz, 1H), 1.64 (s, 6H).

### Example 231: 5-((1r,3r)-3-(4-(2-(4-(2-(2H-1,2,3-triazol-2-yl)pyrimidin-4-yl) methoxy)phenyl)propan-2-yl)phenoxy)cyclobutyl)amino)-2-(2,6-dioxopiperidin-3-yl)isoindolin-1,3-dione

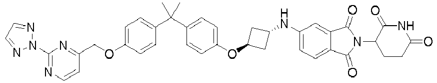

The target compound was synthesized by a method similar to that of Example 228. LC/MS (ESI+) calcd for C₃₉H₃₇N₈O₆⁺ ([M+H]⁺) *m*/*z:* 713.3; found 713.3. ¹H NMR (400 MHz, CDCl₃) *δ* 8.89 (d, *J* = 4.7 Hz, 1H), 8.05 (s, 1H), 8.01 (s, 2H), 7.67 (d, *J* = 4.7 Hz, 1H), 7.63 (d, *J* = 8.1 Hz, 1H), 7.22 - 7.15 (m, 2H), 7.15 - 7.08 (m, 2H), 6.94 - 6.83 (m, 3H), 6.69 (d, *J* = 8.5 Hz, 3H), 5.31 (s, 2H), 4.93 (dd, *J* = 12.0, 5.2 Hz, 1H), 4.86 (s, 1H), 4.23 (s, 1H), 3.00 - 2.75 (m, 3H), 2.72 (s, 2H), 2.40 (s, 2H), 2.12 (q, *J* = 6.7, 5.1 Hz, 1H), 1.64 (s, 6H).

### Example 232: 2-(2,6-dioxopiperidin-3-yl)-5-((1r,3r)-3-(4-(2-(4-((1-methylpiperidin-4-yl)amino)pyrimidin-4-yl)methoxy)phenyl)propan-2-yl) phenoxy)cyclobutyl)amino)isoindolin-1,3-dione

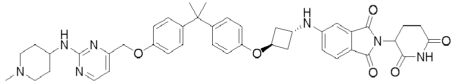

The target compound was synthesized by a method similar to that of Example 228. LC/MS (ESI+) calcd for C₄₃H₄₈N₇O₆⁺ ([M+H]⁺) *m*/*z:* 758.4; found 758.3. ¹H NMR (400 MHz, DMSO-*d*₆) *δ* 8.26 (d, *J* = 5.0 Hz, 1H), 7.56 (dt, *J* = 17.7, 9.0 Hz, 1H), 7.17 (s, 1H), 7.11 (dt, *J* = 8.3, 3.7 Hz, 4H), 6.87 (dd, *J* = 8.6, 4.4 Hz, 2H), 6.73 (q, *J* = 3.7 Hz, 2H), 6.64 - 6.59 (m, 1H), 6.50 - 6.37 (m, 1H), 4.91 (s, 2H), 4.84 (d, *J* = 7.6 Hz, 1H), 4.27 - 4.19 (m, 1H), 4.13 (s, 1H), 3.71 (s, 1H), 2.88 (dq, *J* = 16.6, 10.0, 8.2 Hz, 4H), 2.43 (d, *J* = 6.4 Hz, 2H), 2.36 (d, *J* = 6.0 Hz, 2H), 2.29 (s, 2H), 2.25 (s, 3H), 2.14 (d, *J* = 7.7 Hz, 1H), 1.84 (d, *J* = 7.8 Hz, 2H), 1.76 (d, *J* = 3.5 Hz, 2H), 1.57 (s, 6H).

### Example 233: 2-(2,6-dioxopiperidin-3-yl)-5-((1s,3s)-3-(4-(2-(2-(S)-3-hydroxylpyrrolidin-1-yl)pyrimidin-4-yl)methoxy)phenyl)propan-2-yl)phenoxy) cyclobutyl)amino)isoindolin-1,3-dione

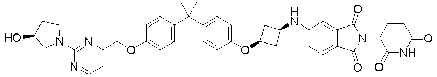

The target compound was synthesized by a method similar to that of Example 228. LC/MS (ESI+) calcd for C₄₁H₄₃N₆O₇⁺ ([M+H]⁺) *m*/*z:* 731.3; found 731.3. ¹H NMR (400 MHz, CDCl₃) *δ* 8.34 - 8.25 (m, 2H), 7.62 (d, *J* = 8.3 Hz, 1H), 7.13 (dd, *J* = 8.8, 3.3 Hz, 4H), 6.91 (d, *J* = 2.1 Hz, 1H), 6.87 - 6.81 (m, 2H), 6.76 (d, *J* = 5.2 Hz, 1H), 6.74 - 6.65 (m, 3H), 4.96 (s, 2H), 4.93 (s, 1H), 4.85 (s, 1H), 4.65 - 4.59 (m, 1H), 4.51 (t, *J* = 6.8 Hz, 1H), 3.77 (dt, *J* = 9.7, 3.6 Hz, 2H), 3.11 (ddt, *J* = 9.5, 6.9, 4.4 Hz, 2H), 2.93 - 2.72 (m, 3H), 2.62 (s, 4H), 2.12 - 2.07 (m, 3H), 1.63 (s, 6H).

### Example 234: 2-(2,6-dioxopiperidin-3-yl)-5-((1s,3s)-3-(4-(4-((2-((1-methylpiperidin-4-yl)oxy)pyrimidin-4-yl)methoxy)phenyl)propan-2-yl)phenoxy) cyclobutyl)amino)isoindolin-1,3-dione

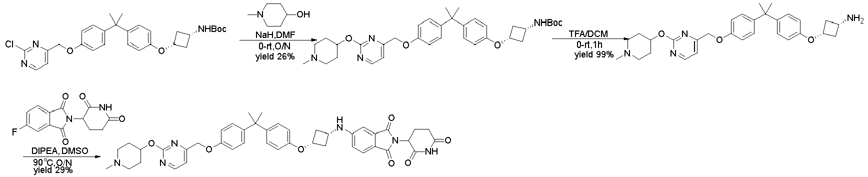

### Step 1: Synthesis of tert-butyl ((1s,3s)-3-(4-(2-(4-(2-((1-methylpiperidin-4-yl)oxy) pyrimidin-4-yl)methoxy)phenyl)propan-2-yl)phenoxy)cyclobutyl)carbamate

In a 50 mL round-bottom flask, compound 1-methylpiperidin-4-ol (263 mg, 2.2 mmol) was added into 10 mL of DMF, to which was added NaH (69 mg, 1.7 mmol), and then the solution was stirred at 0 °C for 10 min, followed by addition of tert-butyl ((1s,3s)-3-(4-(2-(4-((2-chloropyrimidin-4-yl)methoxy)phenyl)propan-2-yl)phenoxy) cyclobutyl)carbamate (300 mg, 0.6 mmol). The reaction was stirred overnight at room temperature, and extracted by adding ethyl acetate and water. The organic layer was washed twice with brine, dried over anhydrous Na₂SO₄, and rotatory evaporated to dry. The residue was purified by silica gel column chromatography, to provide tert-butyl ((1s,3s)-3-(4-(2-(4-(2-((1-methylpiperidin-4-yl)oxy)pyrimidin-4-yl)methoxy)phenyl) propan-2-yl)phenoxy)cyclobutyl)carbamate(90 mg, 0.15 mmol) as white solid, with a yield of 26%.

### Step 2: Synthesis of (1s,3s)-3-(4-(2-(4-((1-methylpiperidin-4-yl)oxy)pyrimidin-4-yl) methoxy)phenyl)propan-2-yl)phenoxy)cyclobutylamine

In a 25 mL round-bottom flask, compound tert-butyl ((1s,3s)-3-(4-(2-(4-(2-((1-methylpiperidin-4-yl)oxy)pyrimidin-4-yl)methoxy)phenyl)propan-2-yl)phenoxy) cyclobutyl)carbamate (90 mg, 0.15 mmol) was added into 3 mL of CH₂Cl₂/CF₃COOH (2:1), and then the solution was stirred at 0 °C for 1 h, followed by addition of dichloromethane. The pH was adjusted to be 8-9, followed by extraction. The organic layer was washed twice with brine, dried over anhydrous Na₂SO₄, and rotatory evaporated to dry, to provide the crude product (1s,3s)-3-(4-(2-(4-((1-methylpiperidin-4-yl)oxy)pyrimidin-4-yl)methoxy)phenyl)propan-2-yl)phenoxy)cyclobutylamine (74 mg, 0.15 mmol), with a yield of 99%.

### Step 3: Synthesis of 2-(2,6-dioxopiperidin-3-yl)-5-((1s,3s)-3-(4-(4-((2-((1-methylpiperidin-4-yl)oxy)pyrimidin-4-yl)methoxy)phenyl)propan-2-yl)phenoxy) cyclobutyl)amino)isoindolin-1,3-dione

In a 25 mL round-bottom flask, compound (1s,3s)-3-(4-(2-(4-((1-methylpiperidin-4-yl)oxy)pyrimidin-4-yl)methoxy)phenyl)propan-2-yl)phenoxy)cyclobutylamine (74 mg, 0.15 mmol), 2-(2,6-dioxopiperidin-3-yl)-5-fluoroisoindol-1,3-dione (61.0 mg, 0.23 mmol), and DIPEA (51 mg, 0.3 mmol) were added into 3 mL of DMSO, and then the solution was heated to 95 °C and stirred overnight. The reaction solution was cooled to room temperature, and extracted by adding ethyl acetate and water. The organic layer was washed with saturated brine, dried, and rotatory evaporated to dry. The residue was purified by prep.-TLC, to provide 2-(2,6-dioxopiperidin-3-yl)-5-((1s,3s)-3-(4-(4-((2-((1-methylpiperidin-4-yl)oxy)pyrimidin-4-yl)methoxy)phenyl)propan-2-yl)phenoxy) cyclobutyl)amino)isoindolin-1,3-dione (33 mg, 0.04 mmol) as yellow solid, with a yield of 29%. LC/MS (ESI+) calcd for C₄₃H₄₆N₆O₇⁺ ([M+H]⁺) *m*/*z*: 759.3; found 759.3. ¹H NMR (400 MHz, DMSO-*d*₆) *δ* 8.57 (d, *J* = 5.0 Hz, 1H), 7.56 (dd, *J* = 19.0, 8.0 Hz, 1H), 7.30 (dd, *J* = 37.0, 7.4 Hz, 1H), 7.20 - 7.00 (m, 6H), 6.90 (d, *J* = 7.7 Hz, 2H), 6.78 - 6.72 (m, 2H), 5.08 (s, 3H), 4.95 (s, 1H), 4.48 (dt, *J* = 15.9, 6.9 Hz, 1H), 3.80 - 3.76 (m, 1H), 3.14 - 2.80 (m, 5H), 2.66 (d, *J* = 7.6 Hz, 2H), 2.27 (d, *J* = 8.1 Hz, 4H), 2.22 (s, 3H), 2.14 (d, *J* = 7.5 Hz, 1H), 1.94 (t, *J* = 17.4 Hz, 4H), 1.57 (s, 6H).

### Example 235: benzyl 4-((4-((2-(4-((1s,3s)-3-((2-(2,6-dioxopiperidin-3-yl)-1,3-dioxoisoindolin-5-yl)amino)cyclobutyloxy)phenyl)propan-2-yl)phenoxy)methyl) pyrimidin-2-yl)oxy)piperidin-1-carboxylate

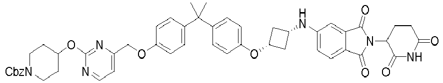

The target compound was synthesized by a method similar to that of Example 234. LC/MS (ESI+) calcd for C₅₀H₅₀N₆O₉ ([M+H]⁺) m/z:879.4; found 879.3. ¹H NMR (400 MHz, CDCl₃) *δ* 8.48 (d, *J* = 5.0 Hz, 1H), 8.22 (s, 1H), 7.62 (d, *J* = 8.3 Hz, 1H), 7.37 (d, *J* = 4.3 Hz, 5H), 7.19 - 7.09 (m, 5H), 6.91 (d, *J* = 2.1 Hz, 1H), 6.87 - 6.81 (m, 2H), 6.75 - 6.65 (m, 3H), 5.23 (tt, *J* = 7.3, 3.6 Hz, 1H), 5.15 (s, 2H), 5.05 (s, 2H), 4.93 (dd, *J* = 12.1, 5.2 Hz, 1H), 4.50 (p, *J* = 6.9 Hz, 1H), 3.87 - 3.83 (m, 2H), 3.76 (q, *J* = 7.5 Hz, 1H), 3.44 (ddd, *J* = 12.9, 7.9, 3.9 Hz, 2H), 3.13 (dt, *J* = 13.5, 3.9 Hz, 4H), 2.81 (ddt, *J* = 27.8, 17.0, 14.1 Hz, 3H), 2.18 - 1.97 (m, 6H), 1.63 (s, 6H).

### Example 236: 2-(2,6-dioxopiperidin-3-yl)-5-(1s,3s)-3-(4-(2-(4-(2-(piperidin-4-yloxy)pyrimidin-4-yl)methoxy)phenyl)propan-2-yl)phenoxy)cyclobutyl)amino) isoindolin-1,3-dione

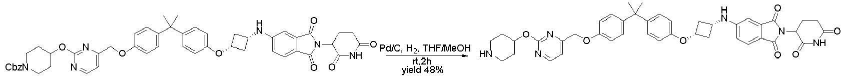

In a 25 mL round-bottom flask, compound benzyl 4-((4-((2-(4-((1s,3s)-3-((2-(2,6-dioxopiperidin-3-yl)-1,3-dioxoisoquinolin-5-yl)amino)cyclobutyloxy)phenyl)propan-2-yl)phenoxy)methyl)pyrimidin-2-yl)oxy)piperidin-1-carboxylate (22 mg, 23 umol) was dissolved in 1 mL of THF and 1 mL of methanol, to which was added Pd/C (5 mg), and then the system was purged thrice with hydrogen balloon, followed by stirring at room temperature for 2h. The reaction solution was filtered over diatomite pad. The filtrate was rotatory evaporated to dry. The residue was purified by prep.-TLC (silica gel), to provide2-(2,6-dioxopiperidin-3-yl)-5-((1s,3s)-3-(4-(4-((2-((1-methylpiperidin-4-yl)oxy)pyrimidin-4-yl)methoxy)phenyl)propan-2-yl)phenoxy)cyclobutyl)amino) isoindol-1,3-dione (10 mg, 12 umol) as yellow solid, with a yield of 48%.
LC/MS (ESI+) calcd for C₄₂H₄₅N₆O₇⁺ ([M+H]⁺) *m*/*z:* 745.3; found 745.3.

### Example 237: 2-(2,6-dioxopiperidin-3-yl)-5-((1s,3s)-3-(4-(2-(2-(oxetan-3-ylamino)pyrimidin-4-yl)methoxy)phenyl)propan-2-yl)phenoxy)cyclobutyl)amino) isoindolin-1,3-dione

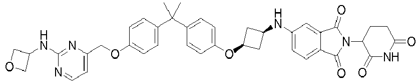

The target compound was synthesized by a method similar to that of Example 234. LC/MS (ESI+) calcd for C₄₀H₄₁N₆O₇⁺ ([M+H]⁺) *m*/*z*:717.3; found 717.3.

### Example 238: 5-((1s,3s)-3-(4-(2-(4-((2-((S)-3-(dimethylamino)pyrrolidin-1-yl) pyrimidin-4-yl)methoxy)phenyl)propan-2-yl)phenoxy)cyclobutyl)amino)-2-(2,6-dioxopiperidin-3-yl)isoindolin-1,3-dione

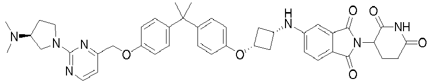

The target compound was synthesized by a method similar to that of Example 234. LC/MS (ESI+) calcd for C₄₃H₄₈N₇O₆⁺ ([M+H]⁺) *m*/*z:* 758.4; found 758.4. ¹H NMR (400 MHz, CDCl₃) *δ* 8.30 (d, *J* = 5.0 Hz, 1H), 7.62 (d, *J* = 8.3 Hz, 1H), 7.16 - 7.08 (m, 4H), 6.91 (d, *J* = 2.1 Hz, 1H), 6.87 - 6.80 (m, 2H), 6.75 - 6.65 (m, 4H), 5.01 - 4.88 (m, 3H), 4.77 (d, *J* = 6.2 Hz, 1H), 4.51 (p, *J* = 6.8 Hz, 1H), 3.95 (dd, *J* = 10.8, 7.1 Hz, 1H), 3.80 (ddd, *J* = 22.0, 13.4, 8.6 Hz, 2H), 3.50 (td, *J* = 10.7, 6.9 Hz, 1H), 3.42 - 3.33 (m, 1H), 3.12 (ddt, *J* = 13.6, 10.0, 5.0 Hz, 2H), 3.02 - 2.69 (m, 4H), 2.37 (s, 6H), 2.25 (dd, *J* = 12.1, 6.3 Hz, 1H), 2.12 - 2.02 (m, 3H), 1.63 (s, 6H).

### Example 239: 2-(2,6-dioxopiperidin-3-yl)-5-((1r,3r)-3-(4-(4-((2-((1-(4-hydroxylbutyl)piperidin-4-yl)amino)pyrimidin-4-yl)methoxy)phenyl)propan-2-yl)phenoxy)cyclobutyl)amino)isoindolin-1,3-dione

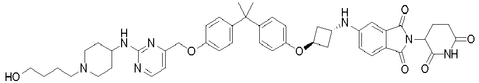

The target compound was synthesized by a method similar to that of Example 236. LC/MS (ESI+) calcd for C₄₆H₅₄N₇O₇⁺ ([M+H]⁺) *m*/*z:* 816.4; found 816.3. ¹H NMR (400 MHz, DMSO-*d*₆) *δ* 11.07 (s, 1H), 8.25 (d, *J* = 5.0 Hz, 1H), 7.59 (d, *J* = 8.3 Hz, 1H), 7.52 (d, *J* = 5.4 Hz, 1H), 7.17 - 7.02 (m, 5H), 6.92 - 6.83 (m, 3H), 6.79 (d, *J* = 8.3 Hz, 1H), 6.76 - 6.68 (m, 2H), 6.60 (d, *J* = 5.0 Hz, 1H), 5.04 (dd, *J* = 12.9, 5.4 Hz, 1H), 4.91 (s, 2H), 4.85 (dd, *J* = 8.7, 3.8 Hz, 1H), 4.18 - 4.06 (m, 1H), 3.67 (s, 1H), 3.42 - 3.36 (m, 2H), 2.98 - 2.75 (m, 3H), 2.64 - 2.51 (m, 4H), 2.47 - 2.33 (m, 3H), 2.25 (d, *J* = 6.8 Hz, 2H), 2.04 - 1.88 (m, 3H), 1.80 (d, *J* = 12.4 Hz, 2H), 1.57 (s, 6H), 1.51 - 1.35 (m, 6H).

### Example 240: 5-((3-(4-(2-(4-((2-(2-oxa-6-azaspiro[3.3]heptan-6-yl)pyrimidin-4-yl)oxy)phenyl)propan-2-yl)phenoxy)cyclobutyl)amino)-2-(2,6-dioxopiperidin-3-yl)isoindolin-1,3-dione

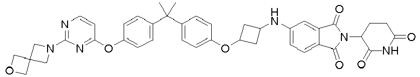

The target compound was synthesized by a method similar to that of Example 214. LC/MS (ESI+) calcd for C₄₁H₄₂N₆O₇⁺ ([M+H]⁺) *m*/*z*:729.3; found 729.3. ¹H NMR (400 MHz, CDCl₃) *δ* 8.10 (s, 2H), 7.67 - 7.60 (m, 1H), 7.30 (s, 1H), 7.13 (dd, *J* = 8.4, 5.7 Hz, 2H), 7.01 (d, *J* = 8.3 Hz, 2H), 6.94 - 6.86 (m, 1H), 6.73 (q, *J* = 6.6, 4.8 Hz, 3H), 6.25 (s, 1H), 4.93 (dd, *J* = 12.1, 5.3 Hz, 1H), 4.81 (s, 4H), 4.22 (s, 4H), 3.79 (s, 1H), 3.14 (s, 1H), 2.80 (ddd, *J* = 39.5, 30.4, 15.0 Hz, 4H), 2.42 (s, 1H), 2.13 (d, *J* = 12.7 Hz, 2H), 1.69 (s, 6H).

### Example 241: 2-(2,6-dioxopiperidin-3-yl)-5-(((1s,3s)-3-(4-(2-(4-((2-(4-(2-hydroxylethyl)piperazin-1-yl)pyrimidin-4-yl)methoxy)phenyl)propan-2-yl) phenoxy)cyclobutyl)amino)isoindolin-1,3-dione

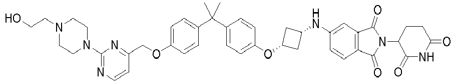

The target compound was synthesized by a method similar to that of Example 234. LC/MS (ESI+) calcd for C₄₃H₄₈N₇O₇⁺ ([M+H]⁺) *m*/*z:* 774.4; found 774.4. ¹H NMR (400 MHz, DMSO-*d*₆) *δ* 8.34 (d, *J* = 4.9 Hz, 1H), 7.60 - 7.47 (m, 1H), 7.10 (t, *J* = 8.6 Hz, 4H), 6.88 (d, *J* = 8.7 Hz, 3H), 6.76 (d, *J* = 8.2 Hz, 2H), 6.69 - 6.58 (m, 2H), 5.03 (dd, *J* = 12.9, 5.3 Hz, 1H), 4.95 (s, 2H), 4.53 - 4.45 (m, 1H), 3.70 (t, *J* = 5.0 Hz, 4H), 3.53 (s, 2H), 3.04 (s, 4H), 2.84 (q, *J* = 7.2 Hz, 5H), 2.44 (d, *J* = 5.9 Hz, 4H), 2.01 (s, 1H), 1.57 (s, 6H).

### Example 242: 2-(2,6-dioxopiperidin-3-yl)-5-(1s,3s)-3-(4-(2-(2-(R-pyrrolidin-3-yl)oxy)pyrimidin-4-yl)methoxy)phenyl)propan-2-yl)phenoxy)cyclobutyl)amino) isoindolin-1,3-dione

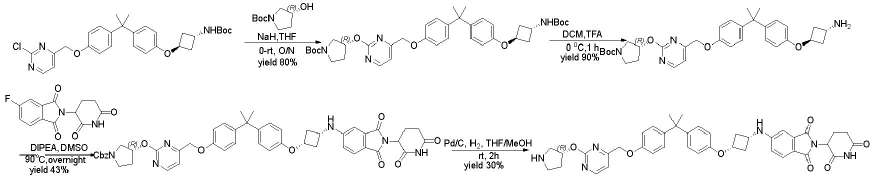

The target compound was synthesized by a method similar to that of Example 234. LC/MS (ESI+) calcd for C₄₁H₄₃N₆O₇⁺ ([M+H]⁺) *m*/*z:* 731.3; found 731.3.

### Example 243: 2-(2,6-dioxopiperidin-3-yl)-5-((1s,3s)-3-(4-(2-(2-((R)-1-methylpyrrolidin-3-yl)oxy)pyrimidin-4-yl)methoxy)phenyl)propan-2-yl)phenoxy) cyclobutyl)amino)isoindolin-1,3-dione

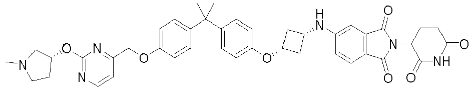

The target compound was synthesized by a method similar to that of Example 234. LC/MS (ESI+) calcd for C₄₂H₄₅N₆O₇⁺ ([M+H]⁺) *m*/*z:* 745.3; found 745.3.

### Example 244: 5-((1r,3r)-3-(4-(2-(4-(4-((2-(2-oxa-6-azaspiro[3.3]heptane-6-yl)pyrimidin-4-yl)oxy)phenyl)propan-2-yl)phenoxy)cyclobutyl)amino)-2-(2,6-dioxopiperidin-3-yl)isoindolin-1,3-dione

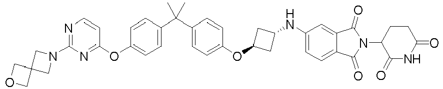

The target compound was synthesized by a method similar to that of Example 214. LC/MS (ESI+) calcd for C₄₁H₄₁N₆O₇⁺ ([M+H]⁺) *m*/*z:* 729.3; found 729.3. ¹H NMR (400 MHz, CDCl₃) δ 8.16 (s, 1H), 8.12 (d, *J* = 5.9 Hz, 1H), 7.62 (d, *J* = 8.2 Hz, 1H), 7.28 (s, 2H), 7.16 - 7.10 (m, 2H), 7.02 (d, *J* = 8.3 Hz, 2H), 6.88 (d, *J* = 2.1 Hz, 1H), 6.71 (dd, *J* = 8.1, 5.3 Hz, 3H), 6.09 (d, *J* = 5.6 Hz, 1H), 4.99 (d, *J* = 4.9 Hz, 1H), 4.93 (dd, *J* = 12.1, 5.3 Hz, 1H), 4.83 (s, 4H), 4.31 (s, 4H), 4.21 (d, *J* = 4.3 Hz, 1H), 2.94 - 2.73 (m, 3H), 2.73 - 2.67 (m, 2H), 2.46 - 2.36 (m, 2H), 2.16 - 2.09 (m, 1H), 1.69 (s, 6H).

### Example 245: N-(4-((4-(2-(4-((1s,3s)-3-((2-(2,6-dioxopiperidin-3-yl)-1,3-dioxoisoindolin-5-yl)amino)cyclobutyloxy)phenyl)propan-2-yl)phenoxy)methyl) pyrimidin-2-yl)cyanamide

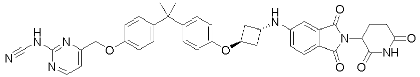

The target compound was synthesized by a method similar to that of Example 214. LC/MS (ESI+) calcd for C₃₈H₃₅N₇O₆⁺ ([M+H]⁺) *m*/*z:* 686.3; found 686.2. ¹H NMR (400 MHz, DMSO-*d*₆) *δ* 8.10 (d, *J* = 4.9 Hz, 1H), 7.58 (d, *J* = 8.3 Hz, 1H), 7.45 (d, *J* = 6.4 Hz, 1H), 7.10 (dd, *J* = 8.6, 4.8 Hz, 4H), 6.90 (s, 1H), 6.86 (d, *J* = 8.6 Hz, 2H), 6.82 (d, *J* = 8.6 Hz, 1H), 6.76 (d, *J* = 8.5 Hz, 2H), 6.42 (d, *J* = 4.9 Hz, 1H), 5.03 (dd, *J* = 12.8, 5.4 Hz, 1H), 4.81 (s, 2H), 4.49 (p, *J* = 7.1 Hz, 1H), 3.03 (td, *J* = 12.4, 6.1 Hz, 4H), 2.89 - 2.77 (m, 3H), 1.97 (s, 1H), 1.57 (s, 6H).

### Example 246 : 5-((1r,3r)-3-(4-(2-(4-((2-acetylpyrimidin-4-yl)oxy)phenyl)propan-2-yl)phenoxy)cyclobutyl)amino)-2-(2,6-dioxopiperidin-3-yl)isoindolin-1,3-dione

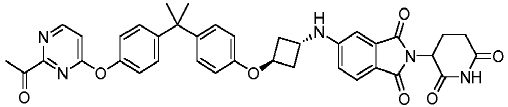

The target compound was synthesized by a method similar to that of Example 107. LC/MS (ESI+) calcd for C₃₈H₃₆N₅O₇⁺ ([M+H]⁺) *m*/*z:* 674.3; found 674.3_{∘} ¹H NMR (400 MHz, CDCl₃) δ 8.00 (s, 1H), 7.65 (s, 1H), 7.31 (s, 3H), 7.13 (d, *J* = 20.7 Hz, 4H), 6.94 (s, 2H), 6.72 (s, 2H), 4.92 (s, 2H), 4.25 (s, 1H), 2.94 - 2.77 (m, 3H), 2.73 (s, 2H), 2.64 (s, 3H), 2.44 (s, 2H), 2.13 (s, 1H), 1.69 (s, 6H).

### Example 247 : 2-(2,6-dioxopiperidin-3-yl)-5-((1r,3r)-3-(4-(2-(4-((5-fluoro-2-(2-oxa-6-azaspiro[3.3]heptan-6-yl)pyrimidin-4-yl)oxy)phenyl)propan-2-yl)phenoxy) cyclobutyl)amino)isoindolin-1,3-dione

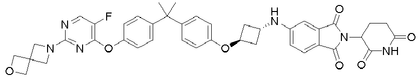

The target compound was synthesized by a method similar to that of Example 214. LC/MS (ESI+) calcd for C₄₁H₄₀FN₆O₇⁺ ([M+H]⁺) *m*/*z:* 747.3; found 747.3. ¹H NMR (400 MHz, CDCl₃) *δ* 8.08 (d, *J* = 3.0 Hz, 1H), 8.02 (s, 1H), 7.63 (d, *J* = 8.3 Hz, 1H), 7.28 (s, 2H), 7.17 - 7.04 (m, 4H), 6.88 (d, *J* = 2.1 Hz, 1H), 6.70 (dd, *J* = 8.3, 5.6 Hz, 3H), 4.96 - 4.91 (m, 1H), 4.88 (s, 1H), 4.81 (s, 4H), 4.20 (s, 1H), 4.10 (s, 4H), 2.85 (dd, *J* = 30.5, 16.4 Hz, 3H), 2.74 - 2.69 (m, 2H), 2.47 - 2.38 (m, 2H), 2.15 - 2.10 (m, 1H), 1.70 (s, 6H).

### Example 248 : 5-((1r,3r)-3-(4-(2-(4-(2-oxa-6-azaspiro[3.3]heptane-6-yl)pyrimidin-5-yl)oxy)phenyl)propan-2-yl)phenoxy)cyclobutyl)amino)-2-(2,6-dioxopyridin-3-yl)isoindolin-1,3-dione

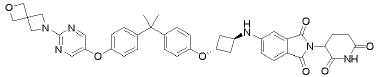

The target compound was synthesized by a method similar to that of Example 214. LC/MS (ESI+) calcd for C₄₁H₄₁N₆O₇⁺ ([M+H]⁺) *m*/*z:* 729.3; found 729.3. ¹H NMR (400 MHz, CDCl₃) *δ* 8.18 (s, 3H), 7.62 (d, *J* = 8.2 Hz, 1H), 7.17 (d, *J* = 8.7 Hz, 2H), 7.11 (d, *J* = 8.7 Hz, 2H), 6.89 (d, *J* = 2.1 Hz, 1H), 6.85 - 6.79 (m, 2H), 6.70 (dd, *J* = 8.6, 2.4 Hz, 3H), 4.93 (dd, *J* = 12.1, 5.2 Hz, 1H), 4.86 (s, 5H), 4.38 (s, 4H), 4.22 (d, *J* = 4.6 Hz, 1H), 2.88 (dd, *J* = 16.3, 3.6 Hz, 1H), 2.84 - 2.74 (m, 2H), 2.70 (dd, *J* = 11.7, 6.0 Hz, 2H), 2.40 (dt, *J* = 13.1, 6.6 Hz, 2H), 2.15 - 2.09 (m, 1H), 1.64 (s, 6H).

### Example 249: 5-((1r,3r)-3-(4-(2-(2-(1-(azetidin-1-yl)ethyl)pyrimidin-4-yl)methoxy) phenyl)propan-2-yl)phenoxy)cyclobutyl)amino)-2-(2,6-dioxopiperidin-3-yl) isoindolin-1,3-dione

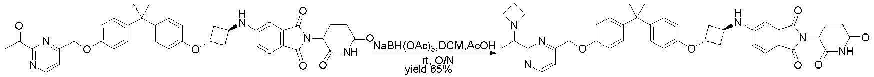

In a 25 mL round-bottom flask, compound 5-((1r,3r)-3-(4-(2-(4-((2-acetylpyrimidin-4-yl)methoxy)phenyl)propan-2-yl)phenoxy)cyclobutyl)amino)-2-(2,6-dioxopiperidin-3-yl)isoindol-1,3-dione (25.0 mg, 0.04 mmol) and cyclobutylamine (7.0 mg, 0.1 mmol) were added into 2 mL of dichloromethane, to which was added catalytic amount of acetic acid, and then the reaction was stirred overnight at room temperature, rotatory evaporated, and purified by prep.-TLC (silica gel), to provide 5-((1r,3r)-3-(4-(2-(2-(1-(azetidin-1-yl)ethyl)pyrimidin-4-yl)methoxy) phenyl)propan-2-yl)phenoxy)cyclobutyl)amino)-2-(2,6-dioxopiperidin-3-yl) isoindolin-1,3-dione (16 mg, 24 □mol) as yellow solid, with a yield of 65%. LC/MS (ESI+) calcd for C₄₁H₄₁N₆O₇⁺ ([M+H]⁺) *m*/*z:* 729.3; found 729.3. ¹H NMR (400 MHz, CDCl₃) *δ* 8.77 (d, *J* = 5.1 Hz, 1H), 8.10 (s, 1H), 7.63 (dd, *J* = 8.3, 2.6 Hz, 1H), 7.54 (d, *J* = 5.1 Hz, 1H), 7.16 (d, *J* = 8.7 Hz, 2H), 7.13 -7.10 (m, 2H), 6.90 (d, *J* = 2.1 Hz, 1H), 6.86 (d, *J* = 8.8 Hz, 2H), 6.73 (dd, *J* = 8.3, 2.2 Hz, 1H), 6.69 (d, *J* = 8.7 Hz, 2H), 5.22 - 5.12 (m, 2H), 5.01 (s, 1H), 4.95 - 4.91 (m, 1H), 4.86 (d, *J* = 6.9 Hz, 1H), 4.33 (s, 1H), 4.21 (d, *J* = 6.3 Hz, 2H), 4.00 (t, *J* = 7.1 Hz, 2H), 2.92 - 2.77 (m, 3H), 2.76 - 2.59 (m, 5H), 2.47 - 2.32 (m, 4H), 2.14 - 2.10 (m, 1H), 1.63 (s, 6H).

### Example 250 : 2-(2,6-dioxopiperidin-3-yl)-5-((1r,3r)-3-(4-(2-(4-((2-(3-methyl-1,2,4-oxadiazol-5-yl)pyrimidin-4-yl)methoxy)phenyl)propan-2-yl)phenoxy)cyclobutyl) amino)isoindolin-1,3-dione

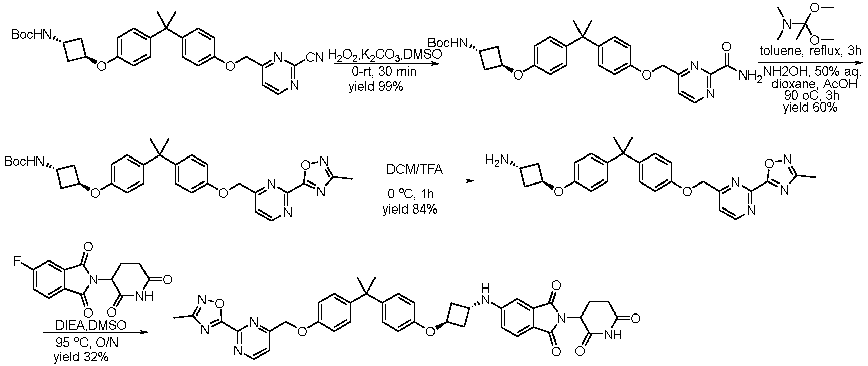

### Step 1: Synthesis of tert-butyl ((1r,3r)-3-(4-(2-(4-((2-carbamoyl pyrimidin-4-yl)methoxy)phenyl)propan-2-yl)phenoxy)cyclobutyl)carbamate

In a 25 mL round-bottom flask, compound tert-butyl ((1r,3r)-3-(4-(2-(4-((2-cyanopyrimidin-4-yl)methoxy)phenyl)propan-2-yl)phenoxy)cyclobutyl)carbamate (400 mg, 0.77 mmol) and K₂CO₃ (214 mg, 1.54 mmol) were added into 4 mL of DMSO, to which was added 0.5 mL of H₂O₂ at 0 °C, and then the mixture was stirred at room temperature for 0.5 h. White solid precipitated, and was collected by suction filtration. The filter cake was rinsed with water and dried, to provide tert-butyl ((1r,3r)-3-(4-(2-(4-((2-carbamoylpyrimidin-4-yl)methoxy)phenyl)propan-2-yl)phenoxy)cyclobutyl) carbamate (410 mg, 0.76 mmol), with a yield of 99%.

### Step 2: Synthesis of tert-butyl ((1r,3r)-3-(4-(2-(4-((2-(3-methyl-1,2,4-oxadiazol-5-yl)pyrimidin-4-yl)methoxy)phenyl)propan-2-yl)phenoxy)cyclobutyl)carbamate

In a 25 mL round-bottom flask, compound tert-butyl ((1r,3r)-3-(4-(2-(4-((2-carbamoylpyrimidin-4-yl)methoxy)phenyl)propan-2-yl)phenoxy)cyclobutyl) carbamate (200 mg, 0.37 mmol) was added into 4 mL of toluene, and then the solution was heated and refluxed for 3 h. Toluene was removed by rotatory evaporation *in vacuum,* to provide the crude product, which was added into 4 mL of dioxane and 1 mL of acetic acid, followed by addition of hydroxylamine solution (37 mg, 50% aq., 0.56 mmol). The reaction solution was heated to 90 °C and stirred for 3 h. After cooling, ethyl acetate and water were added, and the resultant solution was extracted. The organic layer was washed twice with brine, dried over anhydrous Na2SO4, and rotatory evaporated to dry, to provide tert-butyl ((1r,3r)-3-(4-(2-(4-((2-(3-methyl-1,2,4-oxadiazol-5-yl)pyrimidin-4-yl)methoxy)phenyl)propan-2-yl)phenoxy)cyclobutyl) carbamate (130 mg, 0.22 mmol), with a yield of 60%.

### Step 3: Synthesis of (1r,3r)-3-(4-(2-(4-((2-(3-methyl-1,2,4-oxadiazol-5-yl)pyrimidin-4-yl)methoxy)phenyl)propan-2-yl)phenoxy)cyclobutyl-1 -amine

In a 25 mL round-bottom flask, compound tert-butyl ((1r,3r)-3-(4-(2-(4-((2-(3-methyl-1,2,4-oxadiazol-5-yl)pyrimidin-4-yl)methoxy)phenyl)propan-2-yl)phenoxy) cyclobutyl)carbamate (130 mg, 0.22 mmol) was added into 3 mL of CH₂Cl₂/CF₃COOH (2:1), and then the solution was stirred at 0 °C for 1 h, followed by addition of dichloromethane. The pH was adjusted to be 8-9 with NaHCO₃, followed by extraction. The organic layer was washed twice with brine, dried over anhydrous Na₂SO₄, and rotatory evaporated to dry, to provide (1r,3r)-3-(4-(2-(4-((2-(3-methyl-1,2,4-oxadiazol-5-yl)pyrimidin-4-yl)methoxy)phenyl)propan-2-yl)phenoxy)cyclobutyl-1-amine (90 mg, 0.19 mmol), with a yield of 84%.

### Step 4: Synthesis of 2-(2,6-dioxopiperidin-3-yl)-5-((1r,3r)-3-(4-(2-(4-((2-(3-methyl-1,2,4-oxadiazol-5-yl)pyrimidin-4-yl)methoxy)phenyl)propan-2-yl)phenoxy) cyclobutyl)amino)isoindol-1,3-dione

In a 25 mL round-bottom flask, compound (1r,3r)-3-(4-(2-(4-((2-(3-methyl-1,2,4-oxadiazol-5-yl)pyrimidin-4-yl)methoxy)phenyl)propan-2-yl)phenoxy)cyclobutyl-1-amine(90.0 mg, 0.19 mmol), 2-(2,6-dioxopiperidin-3-yl)-5-fluoroisoindol-1,3-dione (79.0 mg, 0.28 mmol), and DIEA (61.0 mg, 0.38 mmol) were added into 4 mL of DMSO, and then the solution was heated to 95 °C and stirred overnight. The reaction solution was cooled to room temperature, and extracted by adding ethyl acetate and water. The organic layer was washed with saturated brine, dried, and rotatory evaporated to dry. The residue was purified by prep.-TLC (silica gel), to provide 2-(2,6-dioxopiperidin-3-yl)-5-((1r,3r)-3-(4-(2-(4-((2-(3-methyl-1,2,4-oxadiazol-5-yl)pyrimidin-4-yl)methoxy) phenyl)propan-2-yl)phenoxy)cyclobutyl)amino)isoindolin-1,3-dione (45 mg, 0.06 mmol), with a yield of 32%. LC/MS (ESI+) calcd for C₄₀H₃₈N₇O₇⁺ ([M+H]⁺) *m*/*z:* 728.3; found 728.3. ¹H NMR (400 MHz, CDCl₃) *δ* 8.99 (d, *J* = 5.1 Hz, 1H), 8.03 (s, 1H), 7.80 (d, *J* = 5.0 Hz, 1H), 7.64 (d, *J* = 8.0 Hz, 1H), 7.23 - 7.15 (m, 2H), 7.13 (d, *J* = 8.5 Hz, 2H), 6.94 - 6.84 (m, 3H), 6.71 (dd, *J* = 11.9, 8.2 Hz, 3H), 5.30 (s, 2H), 4.93 (dd, *J* = 11.9, 5.1 Hz, 1H), 4.87 (s, 1H), 4.23 (s, 1H), 2.96 - 2.75 (m, 3H), 2.72 (s, 2H), 2.57 (s, 3H), 2.41 (s, 2H), 2.16 - 2.10 (m, 1H), 1.64 (s, 6H).

### Example 251: 2-(2,6-dioxopiperidin-3-yl)-5-(1s,3s)-3-(4-(2-(4-(2-(3-methyl-1,2,4-oxadiazol-5-yl)pyrimidin-4-yl)methoxy)phenyl)propan-2-yl)phenoxy)cyclobutyl) amino)isoindolin-1,3-dione

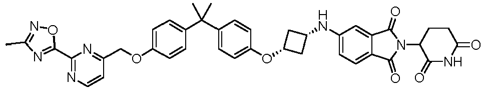

The target compound was synthesized by a method similar to that of Example 250. LC/MS (ESI+) calcd for C₄₀H₃₈N₇O₇⁺ ([M+H]⁺) *m*/*z:* 728.3; found 729.3. ¹H NMR (400 MHz, CDCl₃) *δ* 8.99 (s, 1H), 8.00 (s, 1H), 7.80 (d, *J* = 4.8 Hz, 1H), 7.63 (d, *J* = 7.9 Hz, 1H), 7.18 (d, *J* = 8.5 Hz, 2H), 7.13 (d, *J* = 8.4 Hz, 2H), 6.92 (s, 1H), 6.88 (d, *J* = 8.6 Hz, 2H), 6.72 (d, *J* = 8.2 Hz, 3H), 5.30 (s, 2H), 4.93 (dd, *J* = 11.9, 5.2 Hz, 1H), 4.52 (s, 1H), 3.78 (s, 1H), 3.13 (s, 2H), 2.94 - 2.73 (m, 3H), 2.11 (s, 3H), 1.64 (s, 6H).

### Example 252: 5-((1s,3s)-3-(4-(2-(2-(1,2,4-oxadiazol-5-yl)pyrimidin-4-yl)methoxy) phenyl)propan-2-yl)phenoxy)cyclobutyl)amino)-2-(2,6-dioxopiperidin-3-yl) isoindolin-1,3-dione

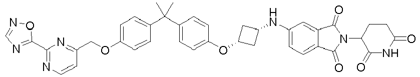

The target compound was synthesized by a method similar to that of Example 250. LC/MS (ESI+) calcd for C₃₉H₃₆N₇O₇⁺ ([M+H]⁺) *m*/*z:* 714.3; found 714.3. ¹H NMR (400 MHz, CDCl₃) *δ* 8.98 (s, 1H), 8.85 (s, 1H), 8.01 (s, 1H), 7.80 (d, *J* = 4.8 Hz, 1H), 7.62(d, *J* = 7.9 Hz, 1H), 7.18 (d, *J* = 8.5 Hz, 2H), 7.13 (d, *J* = 8.4 Hz, 2H), 6.93 (s, 1H), 6.87 (d, *J* = 8.6 Hz, 2H), 6.72 (d, *J* = 8.2 Hz, 3H), 5.29 (s, 2H), 4.93 (dd, *J* = 11.9, 5.2 Hz, 1H), 4.52 (s, 1H), 3.78 (s, 1H), 3.13 (s, 2H), 2.94 - 2.72 (m, 3H), 2.57 (s, 3H), 2.10 (s, 3H), 1.63 (s, 6H).

### Example 253: 5-((1r,3r)-3-(4-(2-(4-((6-(1H-pyrazol-1-yl)pyridin-2-yl)methoxy) phenyl)propan-2-yl)phenoxy)cyclobutyl)amino)-2-(2,6-dioxopiperidin-3-yl) isoindolin-1,3-dione

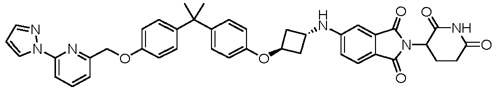

The target compound was synthesized by a method similar to that of Example 228. LC/MS (ESI+) calcd for C₄₁H₃₉N₆O₆⁺ ([M+H]⁺) *m*/*z:* 711.3; found 711.3. ¹H NMR (400 MHz, CDCl₃) δ 8.60 (s, 1H), 8.08 (s, 1H), 7.91 (d, *J* = 7.7 Hz, 1H), 7.85 (d, *J* = 7.5 Hz, 1H), 7.76 (s, 1H), 7.63 (d, *J* = 7.9 Hz, 1H), 7.42 (d, *J* = 7.2 Hz, 1H), 7.14 (t, *J* = 9.0 Hz, 4H), 6.90 (d, *J* = 8.7 Hz, 3H), 6.73 (s, 1H), 6.69 (d, *J* = 8.2 Hz, 2H), 6.48 (s, 1H), 5.18 (s, 2H), 4.93 (dd, *J* = 12.0, 5.1 Hz, 1H), 4.86 (s, 1H), 4.22 (s, 1H), 3.04 - 2.76 (m, 3H), 2.74 - 2.66 (m, 2H), 2.42 (d, J = 10.4 Hz, 2H), 2.18 - 2.07 (m, 1H), 1.64 (s, 6H).

### Example 254: 2-(2,6-dioxopiperidin-3-yl)-5-(1r,3r)-3-(4-(2-(2-(3-methyl-1,2,4-oxadiazol-5-yl)pyrimidin-5-yl)oxy)phenyl)propan-2-yl)phenoxy)cyclobutyl) amino)isoindolin-1,3-dione

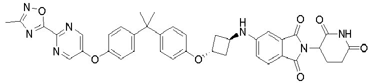

The target compound was synthesized by a method similar to that of Example 250. LC/MS (ESI+) calcd for C₃₉H₃₆N₇O₇⁺ ([M+H]⁺) *m*/*z:* 714.3; found 714.3. ¹H NMR (400 MHz, CDCl₃) *δ* 8.61 (s, 2H), 8.07 (s, 1H), 7.65 (d, *J* = 7.8 Hz, 1H), 7.32 (d, *J* = 8.4 Hz, 2H), 7.14 (d, *J* = 8.3 Hz, 2H), 7.03 (d, *J* = 8.3 Hz, 2H), 6.95 (s, 1H), 6.77 (d, *J* = 7.8 Hz, 1H), 6.72 (d, *J* = 8.4 Hz, 2H), 4.98 - 4.82 (m, 2H), 4.24 (s, 1H), 2.96 - 2.76 (m, 3H), 2.73 (d, *J* = 4.7 Hz, 2H), 2.54 (s, 3H), 2.45 (s, 2H), 2.13 (s, 1H), 1.69 (s, 6H).

### Example 255: 2-(2,6-dioxopiperidin-3-yl)-5-(1r,3r)-3-(4-(2-(2-(3-methyl-1,2,4-oxadiazol-5-yl)pyrimidin-5-yl)oxy)phenyl)propan-2-yl)phenoxy)cyclobutyl) amino)isoindolin-1,3-dione

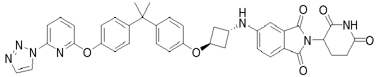

The target compound was synthesized by a method similar to that of Example 228. LC/MS (ESI+) calcd for C₃₉H₃₆N₇O₆⁺ ([M+H]⁺) *m*/*z:* 698.3; found 698.3. ¹H NMR (400 MHz, CDCl₃) *δ* 8.29 - 8.25 (m, 1H), 8.23 (s, 1H), 7.90 - 7.85 (m, 2H), 7.76 (d, *J* = 1.2 Hz, 1H), 7.60 (d, *J* = 8.3 Hz, 1H), 7.31 - 7.27 (m, 2H), 7.19 - 7.14 (m, 2H), 7.10 - 7.05 (m, 2H), 6.87 (dd, *J* = 5.1, 3.4 Hz, 2H), 6.77 - 6.66 (m, 3H), 4.99 - 4.90 (m, 1H), 4.87 (dt, *J* = 6.8, 2.8 Hz, 1H), 4.21 (td, *J* = 7.6, 7.1, 3.8 Hz, 1H), 2.94 - 2.74 (m, 3H), 2.71 (dq, *J* = 8.3, 3.8 Hz, 2H), 2.41 (dt, *J* = 13.1, 6.3 Hz, 2H), 2.15 - 2.08 (m, 1H), 1.70 (s, 6H).

### Example 256: 5-((1r,3r)-3-(4-(2-(4-((6-(2H-1,2,3-triazol-2-yl)pyridin-2-yl)oxy) phenyl)propan-2-ylphenoxy)cyclobutyl)amino)-2-(2,6-dioxopiperidin-3-yl) isoindolin-1,3-dione

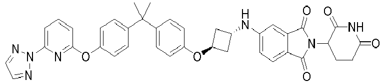

The target compound was synthesized by a method similar to that of Example 228. LC/MS (ESI+) calcd for C₃₉H₃₆N₇O₆⁺ ([M+H]⁺) *m*/*z:* 698.3; found 698.3. ¹H NMR (400 MHz, CDCl₃) *δ* 8.00 (s, 1H), 7.87 (s, 2H), 7.82 (t, *J* = 7.9 Hz, 1H), 7.75 (d, *J* = 7.8 Hz, 1H), 7.64 (d, *J* = 8.2 Hz, 1H), 7.25 (s, 2H), 7.19 - 7.14 (m, 2H), 7.14 - 7.08 (m, 2H), 6.94 (d, *J* = 2.1 Hz, 1H), 6.80 - 6.73 (m, 2H), 6.71 (d, *J* = 8.7 Hz, 2H), 4.93 (dd, *J* = 12.2, 5.3 Hz, 1H), 4.90 - 4.83 (m, 1H), 4.28 - 4.20 (m, 1H), 2.95 - 2.75 (m, 3H), 2.74 - 2.68 (m, 2H), 2.43 (dt, *J* = 12.9, 6.4 Hz, 2H), 2.15 - 2.10 (m, 1H), 1.68 (s, 6H).

### Example 257: 2-(2,6-dioxopiperidin-3-yl)-5-(1r,3r)-3-(4-(2-(4-(4-(5-methyl-1,3,4-oxadiazol-2-yl)pyridin-2-yl)oxy)phenyl)propan-2-yl)phenoxy)cyclobutyl)amino) isoindolin-1,3-dione

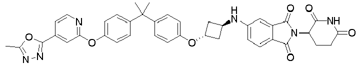

The target compound was synthesized by a method similar to that of Example 216. LC/MS (ESI+) calcd for C₄₀H₃₆N₆O₇⁺ ([M+H]⁺) *m*/*z:* 713.3; found 713.3. ¹H NMR (400 MHz, CDCl₃) *δ* 8.35 (d, *J* = 4.9 Hz, 1H), 8.09 (s, 1H), 7.63 (d, *J* = 8.2 Hz, 1H), 7.58 (d, *J* = 4.7 Hz, 1H), 7.49 (s, 1H), 7.28 (s, 2H), 7.18 (d, *J* = 8.4 Hz, 2H), 7.06 (d, *J* = 8.2 Hz, 2H), 6.89 (d, *J* = 1.9 Hz, 1H), 6.71 (dd, *J* = 8.2, 4.3 Hz, 3H), 4.93 (dd, *J* = 12.1, 5.2 Hz, 1H), 4.87 (s, 1H), 4.23 (s, 1H), 2.97 - 2.74 (m, 3H), 2.71 (dq, *J* = 7.2, 4.0, 3.6 Hz, 2H), 2.66 (s, 3H), 2.41 (d, *J* = 11.1 Hz, 2H), 2.16 - 2.09 (m, 1H), 1.68 (s, 6H).

### Example 258 : 2-(2,6-dioxopiperidin-3-yl)-5-((1r,3r)-3-(4-(2-(4-((6-(3-fluoro-[1,3'-diazetidino]-1'-yl)pyridazine-3-yl)oxy)phenyl)propan-2-yl)phenoxy)cyclobutyl) amino)isoindolin-1,3-dione

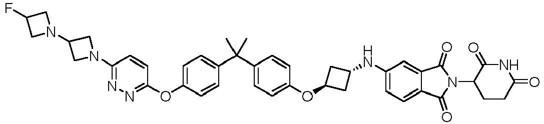

The target compound was synthesized by a method similar to that of Example 214. LC/MS (ESI+) calcd for C₄₂H₄₃FN₇O₆⁺ ([M+H]⁺) *m*/*z:* 760.3; found 760.3. ¹H NMR (400 MHz, CDCl₃) δ 8.55 (s, 1H), 7.62 (d, *J* = 8.3 Hz, 1H), 7.23 - 7.16 (m, 2H), 7.13 (d, *J* = 8.3 Hz, 2H), 7.02 (t, *J* = 8.5 Hz, 3H), 6.89 (d, *J* = 2.2 Hz, 1H), 6.70 (t, *J* = 8.4 Hz, 4H), 5.33 - 5.11 (m, 1H), 4.96 - 4.87 (m, 2H), 4.28 - 4.17 (m, 1H), 4.12 (d, *J* = 7.9 Hz, 2H), 3.94 (dd, *J* = 8.0, 4.4 Hz, 2H), 3.85 - 3.68 (m, 3H), 3.38 (d, *J* = 24.2 Hz, 2H), 2.83 (ddd, *J* = 26.6, 18.4, 14.9 Hz, 3H), 2.70 (d, *J* = 12.5 Hz, 2H), 2.40 (d, *J* = 12.9 Hz, 2H), 2.11 (d, *J* = 10.4 Hz, 1H), 1.64 (s, 6H).

### Example 259 : 2-(2,6-dioxopiperidin-3-yl)-5-((1r,3r)-3-(4-(1-(4-((6-(5-methyl-1,2,4-oxadiazol-3-yl)pyridazine-3-yl)oxy)phenyl)allyl)phenoxy)cyclobutyl)amino) isoindolin-1,3-dione

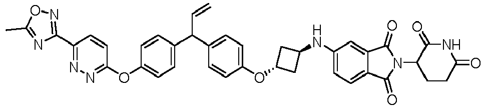

The target compound was synthesized by a method similar to that of Example 221. LC/MS (ESI+) calcd for C₃₉H₃₄FN₇O₆⁺ ([M+H]⁺) *m*/*z:* 712.3; found 712.2. ¹H NMR (400 MHz, CDCl₃) *δ* 8.18 (d, *J* = 9.1 Hz, 1H), 7.99 (s, 1H), 7.64 (d, *J* = 8.1 Hz, 1H), 7.23 (s, 2H), 7.17 (d, *J* = 8.4 Hz, 2H), 7.11 (d, *J* = 8.2 Hz, 2H), 6.91 (s, 1H), 6.81 - 6.64 (m, 3H), 6.27 (ddd, *J* = 17.2, 10.1, 7.1 Hz, 1H), 5.24 (d, *J* = 10.0 Hz, 1H), 5.01 (d, *J* = 17.1 Hz, 1H), 4.93 (dd, *J* = 12.0, 5.2 Hz, 1H), 4.88 (s, 1H), 4.71 (d, *J* = 7.2 Hz, 1H), 4.24 (s, 1H), 2.94 - 2.76 (m, 3H), 2.71 (s, 5H), 2.42 (s, 2H), 2.16 - 2.09 (m, 1H), 1.26 (s, 6H).

### Example 260: Syntheis of 5-(((1s,3s)-3-(4-(2-(4-((2-((1S,4S)-2,5-diazabicyclo[2.2.1]heptan-2-yl)pyrimidin-4-yl)methoxy)phenyl)propan-2-yl)phenoxy)cyclobutyl)amino)-2-(2,6-dioxopiperidin-3-yl)isoindolin-1,3-dione

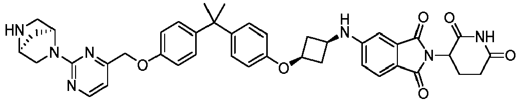

The target compound was synthesized by a method similar to that of Example 59. LC/MS (ESI+) Calcd for C₄₂H₄₃N₇O₆ (M+H⁺) *m*/*z* 741.3; found 741.3.

### Example 261: 5-((1r,3r)-3-(4-(2-(4-(2-(cyclobutyl(hydroxyl)methyl)pyrimidin-5-yl)oxy)phenyl)propan-2-yl)phenoxy)cyclobutyl)amino)-2-(2,6-dioxopiperidin-3-yl)isoindolin-1,3-dione

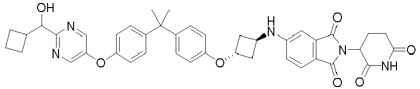

The target compound was synthesized by a method similar to that of Example 219. LC/MS (ESI+) calcd for C₄₁H₄₂N₅O₇⁺ ([M+H]⁺) *m*/*z:* 716.3; found 716.1. ¹H NMR (400 MHz, Chloroform-*d*) *δ* 8.57 (s, 2H), 7.98 (s, 1H), 7.64 (d, *J* = 8.3 Hz, 1H), 7.32 (d, *J* = 8.7 Hz, 2H), 7.15 (d, *J* = 8.8 Hz, 2H), 7.03 (d, *J* = 8.7 Hz, 2H), 6.93 (d, *J* = 2.1 Hz, 1H), 6.75-6.78 (m, 3H), 4.93 (m, 1H), 4.81 (m, 1H),4.37 (s, 1H), 4.27 (s, 1H), 3.78 (s, 1H), 2.95 - 2.66 (m, 5H), 2.36-2.45 (m, 4H), 2.10 (s, 4H),1.92 (s, 2H), 1.69 (s, 6H)

### Example 262: 5-((1r,3r)-3-(4-(2-(2-(cyclobutylcarbonyl)pyrimidin-5-yl)oxy) phenyl)propan-2-yl)phenoxy)cyclobutyl)amino)-2-(2,6-dioxopiperidin-3-yl) isoindolin-1,3-dione

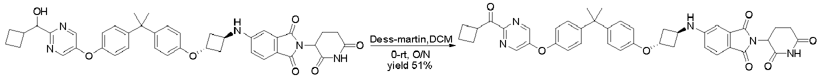

The target compound was synthesized by a method similar to that of Example 220. LC/MS (ESI+) calcd for C₄₁H₄₀N₅O₇⁺ ([M+H]⁺) *m*/*z:* 714.3; found 714.0. ¹H NMR (400 MHz, Chloroform-*d*) *δ* 8.57 (s, 2H), 7.98 (s, 1H), 7.67 (s, 1H), 7.32 (m, 2H), 7.15 (m, 2H), 6.99-7.04 (m, 3H), 6.83(m, 1H), 6.83-6.79(m, 2H),4.93 (s, 2H), 4.37 (s, 1H), 4.27 (s, 1H), 2.95 - 2.66 (m, 5H), 2.36 (d, *J* = 47.6 Hz, 4H), 2.10 (s, 4H),1.92 (s, 2H), 1.69 (s, 6H).

### Example 263:2-(2,6-dioxopiperidin-3-yl)-5-(1s,3s)-3-(4-(2-(4-(2-(3-methyl-1,2,4-oxadiazol-5-yl)pyrimidin-5-yl)oxy)phenyl)propan-2-yl)phenoxy)cyclobutyl) amino)isoindolin-1,3-dione

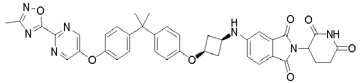

The target compound was synthesized by a method similar to that of Example 220. LC/MS (ESI+) calcd for C₃₉H₃₆N₇O₇⁺ ([M+H]⁺) *m*/*z:* 714.3; found 714.2. ¹H NMR (400 MHz, Chloroform-*d*) *δ* 8.61 (s, 2H), 8.11 (s, 1H), 7.62 (d, *J* = 8.3 Hz, 1H), 7.32 (d, *J* = 8.7 Hz, 2H), 7.14 (d, *J* = 8.8 Hz, 2H), 7.03 (d, *J* = 8.7 Hz, 2H), 6.93 (d, *J* = 2.1 Hz, 1H), 6.74 (dd, *J* = 8.9, 7.0 Hz, 3H), 4.93 (dd, *J* = 12.2, 5.3 Hz, 1H), 4.53 (t, *J* = 6.8 Hz, 1H), 3.85 - 3.73 (m, 1H), 3.14 (ddt, *J* = 13.6, 9.9, 5.0 Hz, 2H), 2.93 - 2.72 (m, 3H), 2.54 (s, 3H), 2.16 - 2.07 (m, 3H), 1.69 (s, 6H).

### Example 264: 2-(2,6-dioxopiperidin-3-yl)-5-((1r,3r)-3-(4-(2-(4-((2-(trifluoromethyl) pyrimidin-5-yl)oxy)phenyl)propan-2-yl)phenoxy)cyclobutyl)amino)isoindolin-1,3-dione

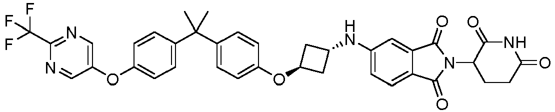

The target compound was synthesized by a method similar to that of Example 197. LC/MS (ESI+) calcd for C₃₇H₃₄F₃N₅O₆ ([M+H]⁺) *m*/*z:* 700.2; found 700.2. ¹H NMR (400 MHz, Chloroform-*d*) *δ* 8.52 (s, 2H), 8.01 (s, 1H), 7.64 (d, *J* = 8.2 Hz, 1H), 7.31 (d, *J* = 8.8 Hz, 2H), 7.14 (d, *J* = 8.8 Hz, 2H), 7.00 (d, *J* = 8.7 Hz, 2H), 6.92 (s, 1H), 6.76 - 6.70 (m, 3H), 4.94 (dd, *J* = 12.2, 5.3 Hz, 1H), 4.88 (s, 1H), 4.29 - 4.19 (m, 1H), 2.96 - 2.78 (m, 3H), 2.74 - 2.69 (m, 2H), 2.43 (dt, *J* = 12.9, 6.1 Hz, 2H), 2.13 (dd, *J* = 7.7, 5.2 Hz, 1H), 1.68 (s, 6H).

### Example 265: 5-(4-(2-(4-((1r,3r)-3-((2-(2,6-dioxopiperidin-3-yl)-1,3-dioxoisoindolin-5-yl)amino)cyclobutyloxy)phenyl)propan-2-yl)phenoxy) pyrimidin-2-carbonylamine

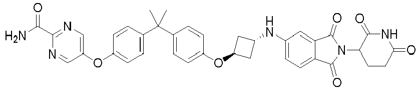

The target compound was synthesized by a method similar to that of Example 197. LC/MS (ESI+) calcd for C₃₇H₃₅N₆O₇⁺ ([M+H]⁺) *m*/*z:* 675.2; found 675.2. ¹H NMR (400 MHz, DMSO-*d*₆) *δ* 11.08 (s, 1H), 8.64 (s, 2H), 8.12 (s, 1H), 7.76 (s, 1H), 7.59 (d, *J* = 8.3 Hz, 1H), 7.54 (d, *J* = 5.4 Hz, 1H), 7.29 (d, *J* = 8.8 Hz, 2H), 7.13 (t, *J* = 8.8 Hz, 4H), 6.86 (s, 1H), 6.78 (dd, *J* = 13.1, 8.9 Hz, 3H), 5.04 (dd, *J* = 12.9, 5.4 Hz, 1H), 4.86 (q, *J* = 5.8 Hz, 1H), 4.14 (d, *J* = 6.5 Hz, 1H), 2.93 - 2.81 (m, 1H), 2.55 (d, *J* = 4.5 Hz, 4H), 2.44 (d, *J* = 6.3 Hz, 2H), 2.04 - 1.94 (m, 1H), 1.63 (s, 6H).

### Example 266: methyl 5-(4-(2-(4-((1r,3r)-3-((2-(2,6-dioxopiperidin-3-yl)-1,3-dioxoisoindolin-5-yl)amino)cyclobutyloxy)phenyl)propan-2-yl)phenoxy) pyrimidin-2-carboxylate

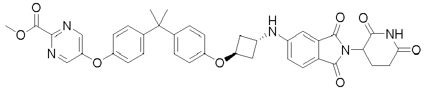

The target compound was synthesized by a method similar to that of Example 197. LC/MS (ESI+) calcd for C₃₈H₃₇N₅O₈⁺ ([M+H]⁺) *m*/*z:* 690.2; found 690.2. ¹H NMR (400 MHz, DMSO-*d*₆) *δ* 11.05 (s, 1H), 8.49 (s, 2H), 7.58 (d, *J* = 8.3 Hz, 1H), 7.53 (d, *J* = 5.6 Hz, 1H), 7.25 (d, *J* = 8.4 Hz, 2H), 7.11 (d, *J* = 8.1 Hz, 4H), 6.86 (s, 1H), 6.77 (t, *J* = 10.5 Hz, 3H), 5.03 (dd, *J* = 12.9, 5.4 Hz, 1H), 4.91 - 4.81 (m, 1H), 4.12 (s, 1H), 3.92 (s, 3H), 3.01 - 2.79 (m, 3H), 2.58 (m, 2H), 2.44 - 2.38 (m, 2H), 2.01 - 1.93 (m, 1H), 1.61 (s, 6H).

### Example 267: 5-(4-(2-(4-((1r,3r)-3-((2-(2,6-dioxopiperidin-3-yl)-1,3-dioxoisoindolin-5-yl)amino)cyclobutyloxy)phenyl)propan-2-yl)phenoxy)pyrimidin-2-carboxylic acid

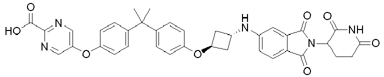

The title compound was prepared by hydrolysis of methyl 5-(4-(2-(4-((1r,3r)-3-((2-(2,6-dioxopiperidin-3-yl)-1,3-dioxoisoindolin-5-yl)amino)cyclobutyloxy)phenyl) propan-2-yl)phenoxy)pyrimidin-2-carboxylate. LC/MS (ESI+) calcd for C₃₇H₃₅N₅O₈⁺ ([M+H]⁺) *m*/*z:* 676.2; found 676.2. ¹H NMR (400 MHz, DMSO-*d*₆) *δ* 11.08 (s, 1H), 8.48 (s, 2H), 7.58 (d, *J* = 8.3 Hz, 1H), 7.55 (d, *J* = 5.7 Hz, 1H), 7.27 (d, *J* = 8.4 Hz, 2H), 7.12 (d, *J* = 8.1 Hz, 4H), 6.86 (s, 1H), 6.77 (t, *J* = 10.5 Hz, 3H), 5.03 (dd, *J* = 12.9, 5.4 Hz, 1H), 4.91 - 4.81 (m, 1H), 4.12 (s, 1H), 3.00 - 2.79 (m, 3H), 2.56 (d, *J* = 18.4 Hz, 2H), 2.46 - 2.37 (m, 2H), 2.04 - 1.95 (m, 1H), 1.61 (s, 6H).

### Example 268: 5-((1s,3s)-3-(4-(2-(2-(3-amino-3-methylazetidin-1-yl)pyrimidin-4-yl)methoxy)phenyl)propan-2-yl)phenoxy)cyclobutyl)amino)-2-(2,6-dioxopiperidin-3-yl)isoindolin-1,3-dione

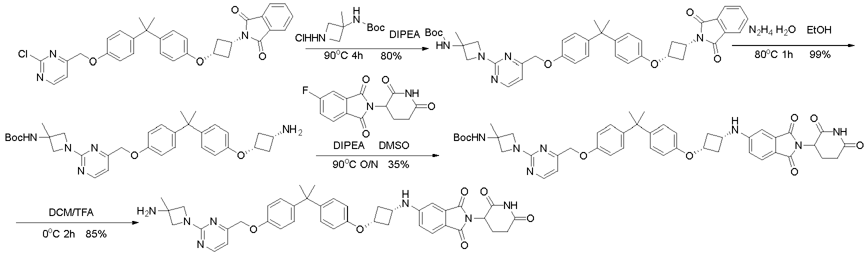

### Step 1: Synthesis of tert-butyl (1-(4-((4-(2-(4-((1s,3s)-3-(1,3-dioxoisoquinolin-2-yl)cyclobutyloxy)phenyl)propan-2-yl)phenoxy)methyl)pyrimidin-2-yl)-3-methylazetidin-3-yl)carbamate

In a 25 mL round-bottom flask, 2-((1s,3s)-3-(4-(2-(4-((2-chloropyrimidin-4-yl)methoxy)phenyl)propan-2-yl)phenoxy)cyclobutyl)isoindolin-1,3-dione (200 mg, 0.36 mmol) was dissolved in 4 mL of DMSO, to which were added tert-butyl (3-methylazetidin-3-yl)carbamate hydrochloride (161 mg, 0.72 mmol) and DIPEA (233 mg, 1.8 mmol) under nitrogen protection, and then the solution was heated to 90 °C and reacted for 4 h. The reaction was quenched by adding water. The resultant solution was extracted with ethyl acetate (10 mL) for three times. The organic phase was combined, and dried over anhydrous Na₂SO₄. The crude product was further purified by silica gel column chromatography, to provide the target product as white solid (162 mg, yield 80%). LC/MS (ESI+) Calcd for C₄₁H₄₆N₅O₆ ([M+H]⁺) *m*/*z* 704.3, found 704.3.

### Step 2: Synthesis of tert-butyl (1-(4-((4-(2-(4-((1s,3s)-3-aminocyclobutyloxy)phenyl) propan-2-yl)phenoxy)methyl)pyrimidin-2-yl)-3-methylazetidin-3-yl)carbamate

(1,3-dioxoisoindolin-2-yl)cyclobutyloxy)phenyl)propan-2-yl)phenoxy)methyl) pyrimidin-2-yl)-3-methylazetidin-3-yl)carbamate (162 mg, 0.23mmol) was dissolved in 4 mL of absolute ethanol, to which was added 1 mL of hydrazine hydrate, and then the mixture was allowed to react under refluxing for 1 h. The reaction solution was rotatory evaporated to dry. To the residue, were added water and DCM, and the resultant solution was extracted twice. The organic phase was combined, and dried over anhydrous Na₂SO₄. The crude product was further purified by silica gel column chromatography, to provide the target product as white solid (130 mg, yield 99%). LC/MS (ESI+) Calcd for C₃₃H₄₃N₅O₄ ([M+H]⁺) *m*/*z* 574.3; found 574.3.

### Step 3: Synthesis of tert-butyl (1-(4-((4-(2-(4-((1s,3s)-3-((2-(2,6-dioxopiperidin-3-yl)-1,3-dioxoisoindolin-5-yl)amino)cyclobutyloxy)phenyl)propan-2-yl)phenoxy)methyl) pyrimidin-2-yl)-3-methylazetidin-3-yl)carbamate

tert-butyl (1-(4-((4-(2-(4-((1s,3s)-3-aminocyclobutyloxy)phenyl)propan-2-yl)phenoxy)methyl)pyrimidin-2-yl)-3-methylazetidin-3-yl)carbamate (160 mg, 0.28mmol) was dissolved in 3 mL of DMSO, to which was added DIEA (108 mg, 0.84 mmol), and then the solution was stirred well, followed by addition of 2-(2,6-dioxopiperidin-3-yl)-5-fluoroisoindolin-1,3-dione (93 mg, 0.33 mmol). The mixture was heated to 90 °C and reacted overnight. After completion of the reaction by detection, the reaction solution was cooled to room temperature, and extracted by adding water and ethyl acetate. The organic phase was washed with saturated brine. The organic phase was combined, and dried over anhydrous Na₂SO₄. The crude product was further purified by silica gel column chromatography, to provide the target product as yellow solid (80 mg, yield 35%). LC/MS (ESI+) Calcd for C₄₆H₅₁N₇O₈ ([M+H]⁺) *m*/*z* 830.3; found 830.3. Step 4: Synthesis of 5-((1*s*,3*s*)-3-(4-(2-(2-(3-amino-3-methylazetidin-1-yl)pyrimidin-4-yl)methoxy)phenyl)propan-2-yl)phenoxy)cyclobutyl)amino)-2-(2,6-dioxopiperidin-3-yl)isoindolin-1,3-dione

Intermediate tert-butyl (1-(4-((4-(2-(4-((1*s*,3*s*)-3-((2-(2,6-dioxopiperidin-3-yl)-1,3-dioxoisoindolin-5-yl)amino)cyclobutyloxy)phenyl)propan-2-yl)phenoxy)methyl) pyrimidin-2-yl)-3-methylazetidin-3-yl)carbamate (80 mg, 0.96 mmol) was dissolved in 3 mL of DCM, to which was added 3 mL of TFA at 0 °C, and then the mixture was allowed to react under stirring for 2 h. The reaction was completed by detection. To the reaction solution, was added saturated NaHCO₃ aqueous solution, to adjust its pH to about 7. The resultant solution was extracted with DCM for three times. The organic phase was combined, and dried over anhydrous Na₂SO₄. The crude product was further purified by silica gel column chromatography, to provide the target product as white solid (60 mg, yield 85%). ¹H NMR (400 MHz, CDCl3) *δ* 8.28 (d, *J* = 5.0 Hz, 1H), 7.61 (d, *J* = 8.3 Hz, 1H), 7.13 (dd, *J* = 8.8, 3.7 Hz, 4H), 6.92 (d, *J* = 2.2 Hz, 1H), 6.87 - 6.80 (m, 3H), 6.72 (dd, *J* = 8.5, 2.3 Hz, 3H), 4.98 - 4.88 (m, 3H), 4.56 - 4.46 (m, 1H), 4.03 (q, *J* = 9.2 Hz, 4H), 3.75 (dd, *J* = 16.2, 8.6 Hz, 1H), 3.16 - 3.04 (m, 2H), 2.92 - 2.71 (m, 3H), 2.12 (d, *J* = 13.1 Hz, 3H), 1.63 (s, 6H), 1.58 (s, 3H). LC/MS (ESI+) Calcd for C₄₁H₄₃N₇O₆ ([M+H]⁺) *m*/*z* 730.3; found 730.3.

### Example 269: 2-((1s,3s)-3-(4-(2-(4-((3-methyloxetan-3-yl)methyl)amino) pyrimidin-4-yl)methoxy)phenyl)propan-2-yl)phenoxy)cyclobutyl)isoindolin-1,3-dione

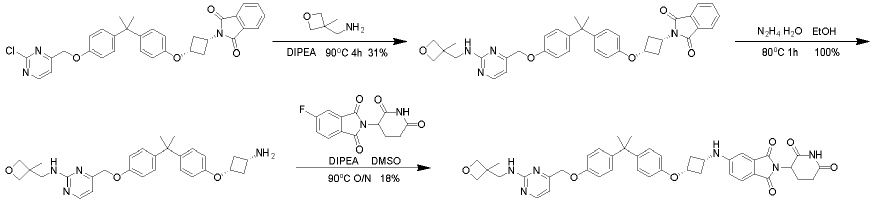

### Step 1: Synthesis of 2-((1s,3s)-3-(4-(2-(4-((3-methyloxetan-3-yl)methyl)amino) pyrimidin-4-yl)methoxy)phenyl)propan-2-yl)phenoxy)cyclobutyl)isoindolin-1,3-dione

2-((1s,3s)-3-(4-(2-(4-((2-chloropyrimidin-4-yl)methoxy)phenyl)propan-2-yl) phenoxy)cyclobutyl)isoindolin-1,3-dione (200 mg, 0.36 mmol) was dissolved in 4 mL of DMSO, to which were added tert-butyl (3-methyloxetan-3-yl)carbonylamine (109 mg, 0.58 mmol) and DIPEA(148 mg, 1.14 mmol), and then the solution was heated to 90 °C and allowed to react overnight. To the system, was added water, and then the resultant solution was extracted with ethyl acetate. The organic phase was combined, and dried over anhydrous Na₂SO₄. The crude product was further purified by silica gel column chromatography, to provide the target product as white solid (70 mg, yield 31%). LC/MS (ESI+) Calcd for C₃₇H₃₈N₄O₅ ([M+H]⁺) *m*/*z* 619.3; found 619.3.

### Step 2: Synthesis of 4-((4-(2-(4-((1s,3s)-3-aminocyclobutyloxy)phenyl)propan-2-yl)phenoxy)methyl)-N-((3-methyloxetan-3-yl)methyl)pyrimidin-2-amine

Intermediate 2-((1*s*,3*s*)-3-(4-(2-(4-((3-methyloxetan-3-yl)methyl)amino) pyrimidin-4-yl)methoxy)phenyl)propan-2-yl)phenoxy)cyclobutyl)isoindolin-1,3-dione (70 mg, 0.11 mmol) was dissolved in 3 mL of DCM, to which was added 3 mL of TFA at 0 °C, and then the mixture was allowed to react under stirring for 2h. The reaction was completed by detection. To the reaction solution, was added saturated NaHCO₃ aqueous solution, to adjust its pH to about 7. The resultant solution was extracted with DCM for three times. The organic phase was combined, and dried over anhydrous Na₂SO₄. The crude product was further purified by silica gel column chromatography, to provide the target product as white solid (55 mg, yield 100%). LC/MS (ESI+) Calcd for C₂₉H₃₆N₄O₃ ([M+H]⁺) *m*/*z* 489.3; found 489.3.

### Step 3: Synthesis of 2-((1s,3s)-3-(4-(2-(4-((3-methyloxetan-3-yl)methyl)amino) pyrimidin-4-yl)methoxy)phenyl)propan-2-yl)phenoxy)cyclobutyl)isoindolin-1,3-dione

4-((4-(2-(4-((1*s*,3*s*)-3-aminocyclobutyloxy)phenyl)propan-2-yl)phenoxy) methyl)-N-((3-methyloxetan-3-yl)methyl)pyrimidin-2-amine (60 mg, 0.12 mmol) was dissolved in 3mL of DMSO, to which was added DIEA (48 mg, 0.37 mmol), and then the solution was stirred well, followed by addition of 2-(2,6-dioxopiperidin-3-yl)-5-fluoroisoindolin-1,3-dione (41 mg, 0.15 mmol). The mixture was heated to 90 °C and reacted overnight. After completion of the reaction by detection, the reaction solution was cooled to room temperature, and extracted by adding water and ethyl acetate. The organic phase was washed with saturated brine. The organic phase was combined, and dried over anhydrous Na₂SO₄. The crude product was further purified by silica gel column chromatography, to provide the target product as yellow solid (16 mg, yield 18%). ¹H NMR (400 MHz, DMSO-*d*₆) *δ* 11.07 (s, 1H), 8.27 (d, *J* = 5.0 Hz, 1H), 7.59 (d, *J* = 8.3 Hz, 1H), 7.51 - 7.42 (m, 2H), 7.17 - 7.06 (m, 4H), 6.93 - 6.85 (m, 3H), 6.82 (dd, *J* = 8.5, 2.1 Hz, 1H), 6.79 - 6.72 (m, 2H), 6.64 (d, *J* = 5.1 Hz, 1H), 5.04 (dd, *J* = 12.9, 5.4 Hz, 1H), 4.92 (s, 2H), 4.49 (q, *J* = 7.0 Hz, 1H), 4.44 (d, *J* = 5.8 Hz, 2H), 4.17 (d, *J* = 5.7 Hz, 2H), 3.77 (q, *J* = 7.4 Hz, 1H), 3.50 (d, *J* = 6.2 Hz, 2H), 3.10 - 3.00 (m, 2H), 2.87 (ddd, *J* = 17.2, 14.1, 5.4 Hz, 1H), 2.56 (dd, *J* = 20.1, 6.5 Hz, 1H), 2.02 - 1.89 (m, 3H), 1.57 (s, 6H), 1.23 (s, 4H). LC/MS (ESI+) Calcd for C₄₂H₄₄N₆O₇ ([M+H]⁺) *m*/*z* 745.3; found 745.3.

### Example 270: 2-(2,6-dioxopiperidin-3-yl)-5-((1s,3s)-3-(4-(2-(2-(1-methyl-1H-pyrazol-4-yl)pyrimidin-4-yl)methoxy)phenyl)propan-2-yl)phenoxy)cyclobutyl) amino)isoindolin-1,3-dione

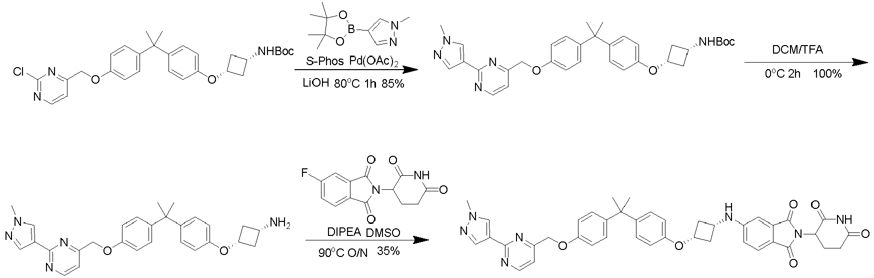

### Step 1: Synthesis of tert-butyl ((1s,3s)-3-(4-(2-(4-(2-(1-methyl-1H-pyrazol-4-yl)pyrimidin-4-yl)methoxy)phenyl)propan-2-yl)phenoxy)cyclobutyl)carbamate

tert-butyl ((1*s*,3*s*)-3-(4-(2-(4-((2-chloropyrimidin-4-yl)methoxy)phenyl)propan-2-yl)phenoxy)cyclobutyl)carbamate (100 mg, 0.19 mmol) was dissolved in 4mL of 1,4-dioxane, to which were added 1-methyl-1H-pyrazol-4-boronic acid pinacol ester (48 mg, 0.23 mmol), S-Phos (8 mg, 19 µmol), and palladium acetate (2 mg, 9 µmol), followed by addition of LiOH (18 mg, 0.73 mmol) dissolved in 0.4 ml of pure water, under nitrogen protection. The reaction solution was heated to 80 °C and allowed to react overnight. To the system, was added water, and then the resultant solution was extracted with ethyl acetate. The organic phase was combined, and dried over anhydrous Na₂SO₄. The crude product was further purified by silica gel column chromatography, to provide the target product as white solid (95 mg, yield 85%). LC/MS (ESI+) Calcd for C₃₃H₃₉N₅O₄ ([M+H]⁺) *m*/*z* 570.3; found 570.3.

### Step 2: Synthesis of (1s,3s)-3-(4-(2-(4-((2-(1-methyl-1H-pyrazol-4-yl)pyrimidin-4-yl)methoxy)phenyl)propan-2-yl)phenoxy)cyclobutyl-1-amine

Intermediate 2-((1*s*,3*s*)-3-(4-(2-(4-((3-methyloxetan-3-yl)methyl)amino) pyrimidin-4-yl)methoxy)phenyl)propan-2-yl)phenoxy)cyclobutyl)isoindolin-1,3-dione (95 mg, 30.16 mmol) was dissolved in 3mL of DCM, to which was added 1 mL of TFA at 0 °C, and then the mixture was allowed to react under stirring for 2h. The reaction was completed by detection. To the reaction solution, was added saturated NaHCO₃ aqueous solution, to adjust its pH to about 7. The resultant solution was extracted with DCM for three times. The organic phase was combined, and dried over anhydrous Na₂SO₄. The crude product was further purified by silica gel column chromatography, to provide the target product as white solid (78 mg, yield 100%). LC/MS (ESI+) Calcd for C₂₈H₃₁N₅O₂ ([M+H]⁺) *m*/*z* 470.3; found 470.3.

### Step 3: Synthesis of 2-(2,6-dioxopiperidin-3-yl)-5-((1s,3s)-3-(4-(2-(2-(1-methyl-1H-pyrazol-4-yl)pyrimidin-4-yl)methoxy)phenyl)propan-2-yl)phenoxy)cyclobutyl)amino) isoindolin-1,3-dione

(1*s*,3*s*)-3-(4-(2-(4-((2-(1-methyl-1H-pyrazol-4-yl)pyrimidin-4-yl)methoxy) phenyl)propan-2-yl)phenoxy)cyclobutyl-1-amine (78 mg, 30.16 mmol) was dissolved in 3 mL of DMSO, to which was added DIEA (66 mg, 0.51 mmol), and then the solution was stirred well, followed by addition of 2-(2,6-dioxopiperidin-3-yl)-5-fluoroisoindolin-1,3-dione (57 mg, 0.20 mmol). The mixture was heated to 90 °C and reacted overnight. After completion of the reaction by detection, the reaction solution was cooled to room temperature, and extracted by adding water and ethyl acetate. The organic phase was washed with saturated brine. The organic phase was combined, and dried over anhydrous Na₂SO₄. The crude product was further purified by silica gel column chromatography, to provide the target product as yellow solid (43 mg, yield 35%). ¹H NMR (400 MHz, Chloroform-*d*) *δ* 8.67 (d, *J* = 5.2 Hz, 1H), 8.39 (s, 1H), 8.24 (s, 1H), 8.13 (s, 1H), 7.62 (d, *J* = 8.3 Hz, 1H), 7.40 (d, *J* = 5.1 Hz, 1H), 7.20 - 7.10 (m, 4H), 6.95 - 6.83 (m, 3H), 6.76 - 6.67 (m, 3H), 5.16 (s, 2H), 4.93 (dd, *J* = 12.2, 5.2 Hz, 1H), 4.74 (s, 1H), 4.51 (p, *J* = 6.8 Hz, 1H), 3.99 (s, 3H), 3.79 (q, *J* = 7.6 Hz, 1H), 3.20 - 3.04 (m, 2H), 2.93 - 2.69 (m, 3H), 2.11 (q, *J* = 10.7, 9.3 Hz, 3H), 1.64 (s, 6H). LC/MS (ESI+) Calcd for C₄₁H₃₉N₇O₆([M+H]⁺) *m*/*z* 726.3; found 726.3.

### Example 271: 5-((1r,3r)-3-(4-(2-(3,5-dimethylisooxazol-4-yl)pyrimidin-4-yl)methoxy)phenyl)propan-2-ylphenoxy)cyclobutyl)amino)-2-(2-oxopiperidin-3-yl)isoindolin-1,3-dione

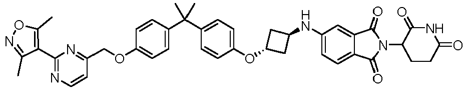

The target compound was synthesized by a method similar to that of Example 270. ¹H NMR (400 MHz, Chloroform-*d*) *δ* 8.74 (d, *J* = 5.1 Hz, 1H), 7.96 (s, 1H), 7.64 (d, *J* = 8.3 Hz, 1H), 7.36 (d, *J* = 5.1 Hz, 1H), 7.20 - 7.09 (m, 4H), 6.92 - 6.84 (m, 3H), 6.70 (t, *J* = 9.4 Hz, 3H), 5.14 (s, 2H), 4.93 (dd, *J* = 12.1, 5.2 Hz, 1H), 4.86 (s, 1H), 4.24 (s, 1H), 2.95 - 2.79 (m, 3H), 2.77 (s, 3H), 2.71 (dd, *J* = 10.2, 3.7 Hz, 3H), 2.59 (s, 3H), 2.40 (dt, *J* = 12.7, 6.2 Hz, 3H), 2.22 (t, *J* = 7.6 Hz, 1H), 2.12 (dd, *J* = 11.7, 6.4 Hz, 1H), 2.01 (d, *J* = 5.9 Hz, 1H), 1.64 (s, 6H). LC/MS (ESI+) Calcd for C₄₂H₄₂N₆O₆ ([M+H]⁺) *m*/*z* 741.3; found 741.3.

### Example 272: 2-(2,6-dioxopiperidin-3-yl)-5-((1r,3r)-3-(4-(2-(4-((2-(thiazol-5-yl) pyrimidin-4-yl)methoxy)phenyl)propan-2-yl)phenoxy)cyclobutyl)amino) isoindolin-1,3-dione

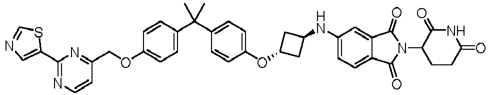

The target compound was synthesized by a method similar to that of Example 270. ¹H NMR (400 MHz, DMSO-*d*₆) *δ* 11.07 (s, 1H), 9.27 (d, *J* = 0.8 Hz, 1H), 8.86 (d, *J* = 5.2 Hz, 1H), 8.64 (d, *J* = 0.8 Hz, 1H), 7.58 (d, *J* = 8.3 Hz, 1H), 7.50 (d, *J* = 5.2 Hz, 1H), 7.45 (d, *J* = 6.5 Hz, 1H), 7.18 - 7.06 (m, 4H), 7.01 - 6.92 (m, 2H), 6.90 (d, *J* = 2.0 Hz, 1H), 6.82 (dd, *J* = 8.3, 2.1 Hz, 1H), 6.80 - 6.73 (m, 2H), 5.23 (s, 2H), 5.04 (dd, *J* = 12.9, 5.4 Hz, 1H), 4.48 (p, *J* = 6.8 Hz, 1H), 3.77 (q, *J* = 7.4 Hz, 1H), 3.10 - 3.00 (m, 2H), 2.86 (d, *J* = 11.8 Hz, 1H), 2.62 - 2.51 (m, 2H), 1.96 (dq, *J* = 18.6, 8.6, 7.7 Hz, 3H), 1.58 (s, 6H). LC/MS (ESI+) Calcd for C₄₀H₃₆N₆O₆S ([M+H]⁺) *m*/*z* 730.3; found 730.3.

### Example 273: 5-((1s,3s)-3-(4-(2-(1H-pyrazol-4-yl)pyrimidin-4-yl)methoxy)phenyl) propan-2-yl)phenoxy)cyclobutyl)amino)-2-(2,6-dioxopiperidin-3-yl)isoindolin-1,3-dione

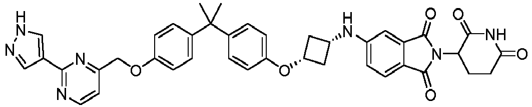

The target compound was synthesized by a method similar to that of Example 270. ¹H NMR (400 MHz, Chloroform-*d*) *δ* 8.71 (s, 1H), 8.45 (s, 2H), 7.62 (d, *J* = 8.2 Hz, 1H), 7.44 (s, 1H), 7.15 (dd, *J* = 14.3, 8.3 Hz, 4H), 6.96 - 6.84 (m, 3H), 6.72 (d, *J* = 8.1 Hz, 3H), 5.17 (s, 2H), 4.92 (dd, *J* = 11.9, 5.3 Hz, 1H), 4.51 (t, *J* = 6.8 Hz, 1H), 3.82 - 3.71 (m, 1H), 3.41 (d, *J* = 2.1 Hz, 1H), 3.11 (s, 2H), 2.92 - 2.70 (m, 3H), 2.10 (s, 3H), 1.64 (s, 6H). LC/MS (ESI+) Calcd for C₄₀H₃₇N₇O₆ ([M+H]⁺) *m*/*z* 712.3; found 712.3.

### Example 274: 5-((1s,3s)-3-(4-(2-(2-(1H-pyrazol-3-yl)pyrimidin-4-yl)methoxy) phenyl)propan-2-yl)phenoxy)cyclobutyl)amino)-2-(2,6-dioxopiperidin-3-yl) isoindolin-1,3-dione

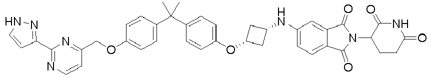

The target compound was synthesized by a method similar to that of Example 270. ¹H NMR (400 MHz, Chloroform-*d*) *δ* 8.73 (d, *J* = 5.0 Hz, 1H), 8.62 (d, *J* = 2.7 Hz, 1H), 7.99 (s, 1H), 7.88 - 7.82 (m, 1H), 7.63 (d, *J* = 8.3 Hz, 1H), 7.48 (d, *J* = 5.0 Hz, 1H), 7.12 (d, *J* = 2.0 Hz, 4H), 6.92 (d, *J* = 2.0 Hz, 1H), 6.91 - 6.83 (m, 2H), 6.77 - 6.65 (m, 3H), 6.52 (s, 1H), 5.23 (s, 2H), 4.93 (dd, *J* = 12.0, 5.2 Hz, 1H), 4.51 (p, *J* = 6.9 Hz, 1H), 3.86 - 3.71 (m, 1H), 3.24 - 3.05 (m, 1H), 2.94 - 2.69 (m, 3H), 2.17 - 2.03 (m, 3H), 1.64 (s, 6H). LC/MS (ESI+) Calcd for C₄₀H₃₇N₇O₆ ([M+H]⁺) *m*/*z* 712.3; found 712.3.

### Example 275: 5-((1r,3r)-3-(4-(2-(4-(3-amino-3-methylazetidin-1-yl)pyrimidin-4-yl)methoxy)phenyl)propan-2-yl)phenoxy)cyclobutyl)amino)-2-(2,6-dioxopiperidin-3-yl)isoindolin-1,3-dione

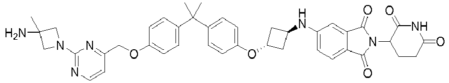

The target compound was synthesized by a method similar to that of Example 269. ¹H NMR (400 MHz, Chloroform-*d*) *δ* 8.28 (d, *J* = 5.1 Hz, 1H), 7.62 (d, *J* = 8.3 Hz, 1H), 7.18 - 7.10 (m, 4H), 6.90 (d, *J* = 2.1 Hz, 1H), 6.86 - 6.80 (m, 3H), 6.70 (dd, *J* = 8.6, 2.3 Hz, 3H), 4.95 (s, 2H), 4.94 - 4.90 (m, 1H), 4.86 (td, *J* = 7.0, 3.5 Hz, 1H), 4.20 (ddd, *J* = 10.0, 7.8, 4.8 Hz, 1H), 4.05 (d, *J* = 9.1 Hz, 2H), 3.99 (d, *J* = 9.1 Hz, 2H), 2.90 - 2.74 (m, 5H), 2.45 - 2.37 (m, 2H), 2.11 (ddd, *J* = 9.6, 4.9, 2.4 Hz, 1H), 1.63 (s, 6H), 1.56 (s, 3H). LC/MS (ESI+) Calcd for C₄₁H₄₃N₇O₆([M+H]⁺) *m*/*z* 730.3; found 730.3_{∘}

### Example 276: 5-((1s,3s)-3-(4-(2-(2-(1,2,4-oxadiazol-3-yl)pyrimidin-4-yl)methoxy) phenyl)propan-2-yl)phenoxy)cyclobutyl)amino)-2-(2,6-dioxopiperidin-3-yl) isoindolin-1,3-dione

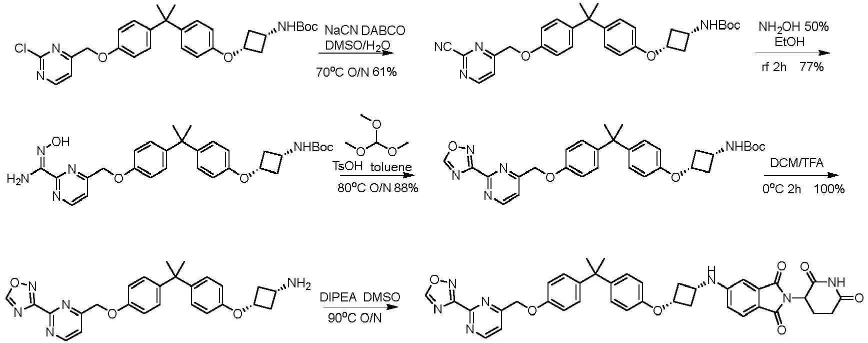

### Step 1: Synthesis of tert-butyl ((1s,3s)-3-(4-(2-(4-((2-cyanopyrimidin-4-yl)methoxy)phenyl)propan-2-yl)phenoxy)cyclobutyl)carbamate

tert-butyl ((1*s*,3*s*)-3-(4-(2-(4-((2-chloropyrimidin-4-yl)methoxy)phenyl)propan-2-yl)phenoxy)cyclobutyl)carbamate (3 g, 5.72 mmol) was dissolved in 60 mL of DMSO, to which were added sodium cyanide (308 mg, 6.30 mmol) and DABCO (83 mg, 0.74 mmol) dissolved in 15 mL of pure water. The reaction solution was heated to 70 °C and allowed to react overnight. To the system, was added water, and then the resultant solution was extracted with ethyl acetate. The organic phase was combined, and dried over anhydrous Na₂SO₄. The crude product was further purified by silica gel column chromatography, to provide the target product as white solid (1.8 g, yield 61%). LC/MS (ESI+) Calcd for C₃₀H₃₄N₄O₄([M+H]⁺) *m*/*z* 514.3; found 514.3.

### Step 2: Synthesis of tert-butyl ((1s,3s)-3-(4-(2-(4-((E)-N'-hydroxylcarbamoyl) pyrimidin-4-yl)methoxy)phenyl)propan-2-yl)phenoxy)cyclobutyl)carbamate

tert-butyl ((1*s*,3*s*)-3-(4-(2-(4-((2-cyanopyrimidin-4-yl)methoxy)phenyl)propan-2-yl)phenoxy)cyclobutyl)carbamate (55 mg, 0.11 mmol) was dissolved in 2 mL of absolute ethanol, to which was added 50% hydroxylamine aqueous solution (14 mg, 0.21 mmol), and then the mixture was refluxed and reacted for 1 h. The reaction solution was filtered, dried, and rotatory evaporated to dry, to provide white solid (45 mg, yield 77%). LC/MS (ESI+) Calcd for C₃₀H₃₇N₅O₅ ([M+H]⁺) *m*/*z* 548.3; found 548.3.

### Step 3: Synthesis of tert-butyl ((1s,3s)-3-(4-(2-(4-(2-(1,2,4-oxadiazol-3-yl)pyrimidin-4-yl)methoxy)phenyl)propan-2-yl)phenoxy)cyclobutyl)carbamate

Intermediate tert-butyl ((1*s*,3*s*)-3-(4-(2-(4-((E)-N'-hydroxylcarbamoyl)pyrimidin-4-yl)methoxy)phenyl)propan-2-yl)phenoxy)cyclobutyl)carbamate (250 mg, 0.45 mmol) was dissolved in 3 mL of toluene, to which were added trimethyl orthoformate (97 mg, 0.91 mmol) and p-toluenesulfonic acid monohydrate (9 mg, 0.04 mmol), and then the mixture was allowed to react overnight at 80 °C. The reaction was completed by detection. The reaction solution was rotatory evaporated to dry. The residue was further purified over silica gel column chromatography, to provide the target product as white solid (223 mg, yield 88%). LC/MS (ESI+) Calcd for C₃₁H₃₅N₅O₅([M+H]⁺) *m*/*z* 558.3; found 558.3.

### Step 4: Synthesis of (1s,3s)-3-(4-(2-(4-((2-(1,2,4-oxadiazol-3-yl)pyrimidin-4-yl)methoxy)phenyl)propan-2-yl)phenoxy)cyclobutyl-1-amine

Intermediate tert-butyl ((1*s*,3*s*)-3-(4-(2-(4-(2-(1,2,4-oxadiazol-3-yl)pyrimidin-4-yl)methoxy)phenyl)propan-2-yl)phenoxy)cyclobutyl)carbamate (200 mg, 0.36 mmol) was dissolved in 3 mL of DCM, to which was added 1 mL of TFA at 0 °C, and then the mixture was allowed to react under stirring for 2h. The reaction was completed by detection. To the reaction solution, was added saturated NaHCO₃ aqueous solution, to adjust its pH to about 7. The resultant solution was extracted with DCM for three times. The organic phase was combined, and dried over anhydrous Na₂SO₄. The crude product was further purified by silica gel column chromatography, to provide the target product as white solid (164 mg, yield 100%). LC/MS (ESI+) Calcd for C₂₆H₂₇N₅O₃ ([M+H]⁺) *m*/*z* 458.3; found 458.3.

### Step 5: Synthesis of 5-((1s,3s)-3-(4-(2-(2-(3-amino-3-methylazetidin-1-yl)pyrimidin-4-yl)methoxy)phenyl)propan-2-yl)phenoxy)cyclobutyl)amino)-2-(2,6-dioxopiperidin-3-yl)isoindolin-1,3-dione

(1*s*,3*s*)-3-(4-(2-(4-((2-(1,2,4-oxadiazol-3-yl)pyrimidin-4-yl)methoxy)phenyl) propan-2-yl)phenoxy)cyclobutyl-1-amine (120 mg, 0.26 mmol) was dissolved in 3 mL of DMSO, to which was added DIEA (105 mg, 0.79 mmol), and then the solution was stirred well, followed by addition of 2-(2,6-dioxopiperidin-3-yl)-5-fluoroisoindolin-1,3-dione (87 mg, 0.31 mmol). The mixture was heated to 90 °C and reacted overnight. After completion of the reaction by detection, the reaction solution was cooled to room temperature, and extracted by adding water and ethyl acetate. The organic phase was washed with saturated brine. The organic phase was combined, and dried over anhydrous Na₂SO₄. The crude product was further purified by silica gel column chromatography, to provide 5-((1s,3s)-3-(4-(2-(2-(3-amino-3-methylazetidin-1-yl)pyrimidin-4-yl)methoxy)phenyl)propan-2-yl)phenoxy)cyclobutyl)amino)-2-(2,6-dioxopiperidin-3-yl)isoindolin-1,3-dione as yellow solid (2 mg, yield 1%). LC/MS (ESI+) Calcd for C₃₉H₃₅N₇O₇ ([M+H]⁺) *m*/*z* 714.3; found 714.3.

### Example 277: 4-((4-(2-(4-((1s,3s)-3-((2-(2,6-dioxopiperidin-3-yl)-1,3-dioxoisoindolin-5-yl)amino)cyclobutyloxy)phenyl)propan-2-yl)phenoxy)methyl) pyrimidin-2-carbonitrile

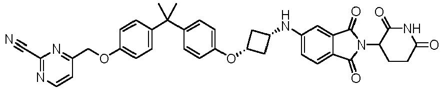

The target compound was synthesized by a method similar to that of Example 276. ¹H NMR (400 MHz, Chloroform-*d*) *δ* 8.85 (d, *J* = 5.2 Hz, 1H), 8.01 (s, 1H), 7.80 (d, *J* = 5.2 Hz, 1H), 7.63 (d, *J* = 8.3 Hz, 1H), 7.22 - 7.15 (m, 2H), 7.14 - 7.08 (m, 2H), 6.92 (d, *J* = 2.0 Hz, 1H), 6.88 - 6.82 (m, 2H), 6.72 (dd, *J* = 8.7, 2.5 Hz, 3H), 5.18 (s, 2H), 4.93 (dd, *J* = 12.0, 5.2 Hz, 1H), 4.71 (s, 1H), 4.50 (q, *J* = 6.9 Hz, 1H), 3.83 - 3.73 (m, 1H), 3.18 - 3.08 (m, 2H), 2.97 - 2.66 (m, 3H), 2.16 - 2.04 (m, 3H), 1.64 (s, 6H). LC/MS (ESI+) Calcd for C₃₈H₃₄N₆O₆([M+H]⁺) *m*/*z* 671.3; found 671.3.

### Example 278: 2-(2,6-dioxopiperidin-3-yl)-5-(1s,3s)-3-(4-(2-(4-(2-(5-methyl-1,2,4-oxadiazol-3-yl)pyrimidin-4-yl)methoxy)phenyl)propan-2-yl)phenoxy)cyclobutyl) amino)isoindolin-1,3-dione

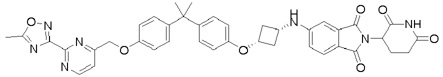

The target compound was synthesized by a method similar to that of Example 276. ¹H NMR (400 MHz, Chloroform-*d*) *δ* 8.95 (d, *J* = 5.1 Hz, 1H), 7.99 (s, 1H), 7.73 (d, *J* = 5.0 Hz, 1H), 7.63 (d, *J* = 8.2 Hz, 1H), 7.15 (dd, *J* = 16.0, 8.8 Hz, 4H), 6.92 (d, *J* = 2.1 Hz, 1H), 6.87 (d, *J* = 8.8 Hz, 2H), 6.72 (d, *J* = 8.8 Hz, 3H), 5.31 - 5.28 (m, 2H), 4.93 (dd, *J* = 12.2, 5.3 Hz, 1H), 4.70 (s, 1H), 4.52 (q, *J* = 6.8 Hz, 1H), 3.78 (t, *J* = 7.5 Hz, 1H), 3.18 - 3.09 (m, 2H), 2.94 - 2.76 (m, 3H), 2.75 (s, 3H), 2.16 - 2.04 (m, 3H), 1.64 (s, 6H). LC/MS (ESI+) Calcd for C₄₀H₃₇N₇O₇([M+Ht) m/z 728.3; found 728.3.

### Example 279 : 2-(2,6-dioxopiperidin-3-yl)-5-((1r,3r)-3-(4-(2-(4-((2-(5-methyl-1,2,4-oxadiazol-3-yl)pyrimidin-4-yl)methoxy)phenyl)propan-2-yl)phenoxy)cyclobutyl) amino)isoindolin-1,3-dione

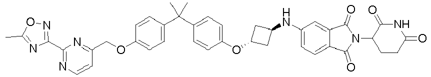

The target compound was synthesized by a method similar to that of Example 276. ¹H NMR (400 MHz, Chloroform-*d*) *δ* 8.95 (d, *J* = 5.1 Hz, 1H), 8.00 (s, 1H), 7.73 (d, *J* = 4.8 Hz, 1H), 7.63 (d, *J* = 8.2 Hz, 1H), 7.22 - 7.10 (m, 4H), 6.88 (dd, *J* = 9.5, 2.7 Hz, 3H), 6.70 (dd, *J* = 7.2, 4.9 Hz, 3H), 5.30 (d, *J* = 2.0 Hz, 2H), 4.93 (dd, *J* = 12.1, 5.2 Hz, 1H), 4.86 (s, 1H), 4.76 (s, 1H), 4.23 (s, 1H), 2.92 - 2.80 (m, 2H), 2.75 (s, 6H), 2.46 - 2.34 (m, 2H), 2.13 (dd, *J* = 8.3, 5.8 Hz, 1H), 1.64 (s, 6H). LC/MS (ESI+) Calcd for C₄₀H₃₇N₇O₇ ([M+H]⁺) *m*/*z* 728.3; found 728.3.

### Example 280: 2-(2,6-dioxopiperidin-3-yl)-5-((1r,3r)-3-(4-(2-(4-(1-(5-fluoro-6-(2-oxa-6-azaspiro[3.3]heptan-6-yl)pyrimidin-4-yl)ethoxy)phenyl)propan-2-yl) phenoxy)cyclobutyl)amino)isoindolin-1,3-dione

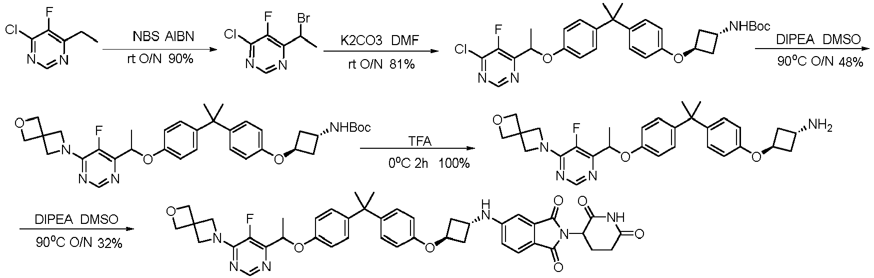

### Step 1: Synthesis of 4-(1-bromoethyl)-6-chloro-5-fluoropyrimidine

4-chloro-6-ethyl-5-fluoropyrimidine (1.00 g, 2.52 mmol) was dissolved in 10 mL of DCM, to which were successivel added NBS (423 mg, 2.77 mmol) and AIBN (1.29 g, 7.55mmol), and then the mixture was allowed to react overnight at room temperature. The system was added to water, and then extracted with DCM. The organic phase was combined, and dried over anhydrous Na2SO4. The crude product was further purified by silica gel column chromatography, to provide the target product as white solid (1.16 g, yield 90%). LC/MS (ESI+) Calcd for C₆H₅BrClFN₂([M+H]⁺) *m*/*z* 238.9; found 238.9.

### Step 2: Synthesis of tert-butyl ((1r,3r)-3-(4-(2-(4-(1-(6-chloro-5-fluoropyrimidin-4-yl)ethoxy)phenyl)propan-2-yl)phenoxy)cyclobutyl)carbamate

4-(1-bromoethyl)-6-chloro-5-fluoropyrimidine (330 g, 1.38mmol) was dissolved in 6 mL of DMF, to which were successively added tert-butyl ((1*r*,3*r*)-3-(4-(2-(4-hydroxylphenyl)propan-2-phenoxy)cyclobutyl)carbamate (504 mg, 1.27 mmol) and K₂CO₃ (381 mg, 2.76 mmol), and then the mixture was allowed to react overnight at room temperature. The system was added to water, and then extracted with ethyl acetate. The organic phase was combined, and dried over anhydrous Na2SO4. The crude product was further purified by silica gel column chromatography, to provide the target product as white solid (616 mg, yield 81%). LC/MS (ESI+) Calcd for C₃₀H₃₅ClFN₃O₄ ([M+H]⁺) *m*/*z* 556.2; found 556.2.

### Step 3: Synthesis of tert-butyl ((1r,3r)-3-(4-(2-(4-(1-(5-fluoro-6-(2-oxa-6-azaspiro[3.3] heptan-6-yl)pyrimidin-4-yl)ethoxy)phenyl)propan-2-yl)phenoxy)cyclobutyl) carbamate

tert-butyl ((1*r*,3*r*)-3-(4-(2-(4-(1-(6-chloro-5-fluoropyrimidin-4-yl)ethoxy)phenyl) propan-2-yl)phenoxy)cyclobutyl)carbamate (200 mg, 0.36 mmol) was dissolved in 3 mL of DMSO, to which was added DIEA (140 mg, 1.08 mmol), and then the solution was stirred well, followed by addition of 2-oxa-6-azaspiro[3.3]heptane oxalate (104 mg, 0.36 mmol). The mixture was heated to 90 °C and allowed to react overnight. After completion of the reaction by detection, the reaction solution was cooled to room temperature, and extracted by adding water and ethyl acetate. The organic phase was washed with saturated brine. The organic phase was combined, and dried over anhydrous Na₂SO₄. The crude product was further purified by silica gel column chromatography, to provide the target product as white solid (107 mg, yield 48%). LC/MS (ESI+) Calcd for C₃₅H₄₃FN₄O₅ ([M+H]⁺) *m*/*z* 619.3; found 619.3.

### Step 4: Synthesis of (1r,3r)-3-(4-(2-(4-(1-(5-fluoro-6-(2-oxa-6-azaspiro[3.3]heptan-6-yl)pyrimidin-4-yl)ethoxy)phenyl)propan-2-yl)phenoxy)cyclobutylamine

Intermediate tert-butyl ((1*r*,3*r*)-3-(4-(2-(4-(1-(5-fluoro-6-(2-oxa-6-azaspiro[3.3] heptan-6-yl)pyrimidin-4-yl)ethoxy)phenyl)propan-2-yl)phenoxy)cyclobutyl) carbamate (105 mg, 0.17 mmol) was dissolved in 3 mL of DCM, to which was added 1 mL of TFA at 0 °C, and then the mixture was allowed to react under stirring for 2 h. The reaction was completed by detection. To the reaction solution, was added saturated NaHCO₃ aqueous solution, to adjust its pH to about 7. The resultant solution was extracted with DCM for three times. The organic phase was combined, and dried over anhydrous Na₂SO₄. The crude product was further purified by silica gel column chromatography, to provide the target product as white solid (88 mg, yield 100%). LC/MS (ESI+) Calcd for C₃₀H₃₅FN₄O₃ ([M+H]⁺) *m*/*z* 519.3; found 519.3.

### Step 5: Synthesis of 2-(2,6-dioxopiperidin-3-yl)-5-((1r,3r)-3-(4-(2-(4-(1-(5-fluoro-6-(2-oxa-6-azaspiro[3.3]heptan-6-yl)pyrimidin-4-yl)ethoxy)phenyl)propan-2-yl) phenoxy)cyclobutyl)amino)isoindolin-1,3-dione

(1*r*,3*r*)-3-(4-(2-(4-(1-(5-fluoro-6-(2-oxa-6-azaspiro[3.3]]heptan-6-yl)pyrimidin-4-yl)ethoxy)phenyl)propan-2-yl)phenoxy)cyclobutylamine (90 mg, 0.19 mmol) was dissolved in 3 mL of DMSO, to which was added DIEA (74 mg, 0.57 mmol), and then the solution was stirred well, followed by addition of 2-(2,6-dioxopiperidin-3-yl)-5-fluoroisoindolin-1,3-dione (63 mg, 0.23 mmol). The mixture was heated to 90 °C and reacted overnight. After completion of the reaction by detection, the reaction solution was cooled to room temperature, and extracted by adding water and ethyl acetate. The organic phase was washed with saturated brine. Then, the organic phase was combined, and dried over anhydrous Na₂SO₄. The crude product was further purified by silica gel column chromatography, to provide the target product as yellow solid (45 mg, yield 32%). ¹H NMR (400 MHz, Chloroform-*d*) *δ* 8.37 (s, 1H), 8.10 (d, *J* = 13.8 Hz, 1H), 7.63 (d, *J* = 8.2 Hz, 1H), 7.09 (t, *J* = 9.0 Hz, 4H), 6.89 (s, 1H), 6.82 (d, *J* = 8.0 Hz, 2H), 6.71 (d, *J* = 8.4 Hz, 1H), 6.67 (d, *J* = 8.2 Hz, 2H), 5.51 (s, 1H), 4.93 (dd, *J* = 12.1, 5.3 Hz, 1H), 4.86 (s, 5H), 4.48 (s, 4H), 4.23 (s, 1H), 2.93 - 2.78 (m, 2H), 2.78 - 2.65 (m, 3H), 2.40 (d, *J* = 10.0 Hz, 2H), 2.17 - 2.09 (m, 1H), 1.68 (s, 3H), 1.60 (s, 6H). LC/MS (ESI+) Calcd for C₄₃H₄₃FN₆O₇([M+H]⁺) *m*/*z* 775.3; found 775.3.

### Example 281: 5-((1r,3r)-3-(4-(2-(2-(1,2,4-oxadiazol-3-yl)pyrimidin-4-yl)methoxy) phenyl)propan-2-yl)phenoxy)cyclobutyl)amino)-2-(2,6-dioxopiperidin-3-yl) isoindolin-1,3-dione

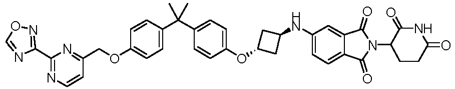

The target compound was synthesized by a method similar to that of Example 276. ¹H NMR (400 MHz, Chloroform-*d*) *δ* 8.98 (d, *J* = 5.1 Hz, 1H), 8.95 (s, 1H), 7.99 (s, 1H), 7.76 (d, *J* = 5.1 Hz, 1H), 7.64 (d, *J* = 8.3 Hz, 1H), 7.22 - 7.12 (m, 2H), 7.12 (s, 2H), 6.92 - 6.83 (m, 3H), 6.74-6.66 (m, 3H), 5.33 - 5.27 (m, 2H), 4.93 (dd, *J* = 12.1, 5.2 Hz, 1H), 4.86 (s, 1H), 4.74 (s, 1H), 4.24 (d, *J* = 5.0 Hz, 1H), 2.94 - 2.78 (m, 2H), 2.79 - 2.66 (m, 3H), 2.40 (dt, *J* = 12.9, 6.2 Hz, 2H), 2.17 - 2.08 (m, 1H), 1.64 (s, 6H). LC/MS (ESI+) Calcd for C₃₉H₃₅N₇O₇ ([M+H]⁺) *m*/*z* 714.3; found 714.3.

### Example 282: 2-(2,6-dioxopiperidin-3-yl)-5-((1r,3r)-3-(4-(2-(4-((6-(3-methyl-1,2,4-oxadiazol-5-yl)pyridin-2-yl)methoxy)phenyl)propan-2-yl)phenoxy)cyclobutyl) amino)isoindolin-1,3-dione

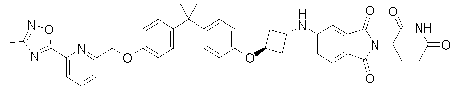

The target compound was synthesized by a method similar to that of Example 276. ¹H NMR (400 MHz, Chloroform-*d*) *δ* 8.14 - 8.08 (m, 2H), 7.94 (t, *J* = 7.9 Hz, 1H), 7.80 (d, *J* = 7.9 Hz, 1H), 7.62 (d, *J* = 8.0 Hz, 1H), 7.15 (dd, *J* = 11.8, 8.3 Hz, 4H), 6.89 (d, *J* = 8.3 Hz, 3H), 6.69 (d, *J* = 8.3 Hz, 3H), 5.32 (s, 2H), 4.97 - 4.91 (m, 1H), 4.86 (s, 1H), 4.22 (s, 1H), 2.85 (dd, *J* = 30.4, 15.1 Hz, 3H), 2.72 (s, 3H), 2.53 (s, 3H), 2.40 (s, 2H), 2.16 - 2.08 (m, 1H), 1.64 (s, 6H). LC/MS (ESI+) Calcd for C₄₁H₃₈N₆O₇([M+H]⁺) *m*/*z* 727.3; found 727.3.

### Example 283: 2-(2,6-dioxopiperidin-3-yl)-5-((1r,3r)-3-(4-(2-(4-((6-(5-methyl-1,2,4-oxadiazol-3-yl)pyridin-2-yl)methoxy)phenyl)propan-2-yl)phenoxy)cyclobutyl) amino)isoindolin-1,3-dione

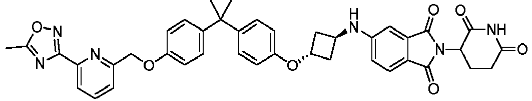

The target compound was synthesized by a method similar to that of Example 276. ¹H NMR (400 MHz, Chloroform-*d*) *δ* 8.09 - 8.01 (m, 2H), 7.88 (t, *J* = 7.8 Hz, 1H), 7.71 (d, *J* = 7.8 Hz, 1H), 7.63 (d, *J* = 8.2 Hz, 1H), 7.14 (t, *J* = 8.3 Hz, 4H), 6.94 - 6.86 (m, 3H), 6.70 (td, *J* = 6.3, 5.8, 2.8 Hz, 3H), 5.32 (s, 2H), 4.93 (dd, *J* = 12.0, 5.2 Hz, 1H), 4.86 (s, 1H), 4.79 (s, 1H), 4.23 (s, 1H), 2.93 - 2.75 (m, 3H), 2.71 (s, 5H), 2.40 (dt, *J* = 12.9, 6.1 Hz, 2H), 2.16 - 2.09 (m, 1H), 1.63 (d, *J* = 4.2 Hz, 6H). LC/MS (ESI+) Calcd for C₄₁H₃₈N₆O₇([M+H]⁺) *m*/*z* 728.3; found 728.3.

### Example 284: 2-(2,6-dioxopiperidin-3-yl)-5-((1r,3r)-3-(4-(2-(4-((3-fluoro-6-(2H-1,2,3-triazol-2-yl)pyridin-2-yl)methoxy)phenyl)propan-2-yl)phenoxy)cyclobutyl) amino)isoindolin-1,3-dione

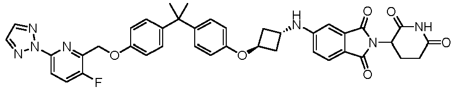

The target compound was synthesized by a method similar to that of Example 276. ¹H NMR (400 MHz, Chloroform-*d*) δ 8.11 (dd, *J* = 8.9, 3.3 Hz, 1H), 8.01 (s, 1H), 7.90 (s, 2H), 7.70 - 7.61 (m, 2H), 7.18 - 7.10 (m, 4H), 6.96 - 6.89 (m, 3H), 6.70 (td, *J* = 7.5, 6.6, 2.1 Hz, 3H), 5.28 (d, *J* = 1.9 Hz, 2H), 4.93 (dd, *J* = 12.1, 5.2 Hz, 1H), 4.86 (s, 1H), 4.76 (d, *J* = 4.9 Hz, 1H), 4.23 (d, *J* = 6.3 Hz, 1H), 2.94 - 2.79 (m, 2H), 2.79 - 2.66 (m, 3H), 2.40 (dt, *J* = 13.0, 6.1 Hz, 2H), 2.15 - 2.09 (m, 1H), 1.63 (s, 6H). LC/MS (ESI+) Calcd for C₄₀H₃₆FN₇O₆ ([M+H]⁺) *m*/*z* 730.3; found 730.3.

### Example 285: 2-(2,6-dioxopiperidin-3-yl)-5-((1r,3r)-3-(4-(2-(4-((3-fluoro-6-(1H-1,2,3-triazol-1-yl)pyridin-2-yl)methoxy)phenyl)propan-2-yl)phenoxy)cyclobutyl) amino)isoindolin-1,3-dione

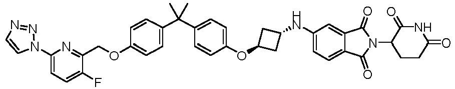

The target compound was synthesized by a method similar to that of Example 276. ¹H NMR (400 MHz, Chloroform-*d*) *δ* 8.48 (d, *J* = 1.0 Hz, 1H), 8.22 (dd, *J* = 8.9, 3.3 Hz, 1H), 8.04 (s, 1H), 7.82 (d, *J* = 1.2 Hz, 1H), 7.72 - 7.60 (m, 2H), 7.20 - 7.14 (m, 2H), 7.14 - 7.08 (m, 2H), 6.96-6.88 (m, 3H), 6.69 (dd, *J* = 13.4, 8.7 Hz, 3H), 5.25 (d, *J* = 2.0 Hz, 2H), 4.93 (dd, *J* = 12.1, 5.2 Hz, 1H), 4.85 (m, 2H), 4.23 (s, 1H), 2.93 - 2.79 (m, 2H), 2.77 - 2.66 (m, 3H), 2.45 - 2.36 (m, 2H), 2.16 - 2.09 (m, 1H), 1.64 (s, 6H). LC/MS (ESI+) Calcd for C₄₀H₃₆FN₇O₆([M+H]⁺) *m*/*z* 730.3; found 730.3.

### Example 286: 2-(2,6-dioxopiperidin-3-yl)-5-((1r,3r)-3-(4-(2-(4-((5-fluoro-6-(1H-1,2,3-triazol-2-yl)pyridin-2-yl)methoxy)phenyl)propan-2-yl)phenoxy)cyclobutyl) amino)isoindolin-1,3-dione

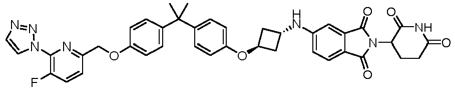

The target compound was synthesized by a method similar to that of Example 276. ¹H NMR (400 MHz, Chloroform-*d*) *δ* 8.38 (s, 1H), 7.99 (s, 1H), 7.89 (s, 1H), 7.76 (t, *J* = 9.1 Hz, 1H), 7.68 (dd, *J* = 8.4, 3.1 Hz, 1H), 7.64 (d, *J* = 8.0 Hz, 1H), 7.15 (dd, *J* = 15.8, 8.5 Hz, 4H), 6.88 (d, *J* = 8.8 Hz, 3H), 6.70 (t, *J* = 8.9 Hz, 3H), 5.20 (s, 2H), 4.93 (dd, *J* = 12.0, 5.2 Hz, 1H), 4.86 (s, 1H), 4.24 (s, 1H), 2.94 - 2.79 (m, 2H), 2.74 (d, *J* = 21.5 Hz, 3H), 2.40 (d, *J* = 11.3 Hz, 2H), 2.12 (dd, *J* = 12.9, 6.3 Hz, 1H), 1.64 (s, 6H). LC/MS (ESI+) Calcd for C₄₀H₃₆FN₇O₆ ([M+H]⁺) *m*/*z* 730.3; found 730.3.

### Example 287: 2-(2,6-dioxopiperidin-3-yl)-5-((1r,3r)-3-(4-(2-(4-((5-fluoro-6-(2H-1,2,3-triazol-1-yl)pyridin-2-yl)methoxy)phenyl)propan-2-yl)phenoxy)cyclobutyl) amino)isoindolin-1,3-dione

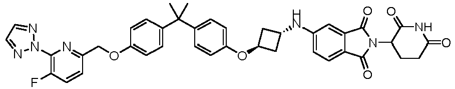

The target compound was synthesized by a method similar to that of Example 276. ¹H NMR (400 MHz, Chloroform-*d*) *δ* 8.04 (s, 1H), 7.99 (s, 2H), 7.63 (d, *J* = 8.2 Hz, 1H), 7.19 - 7.10 (m, 4H), 6.91 - 6.85 (m, 3H), 6.69 (dq, *J* = 7.4, 3.3, 2.7 Hz, 3H), 5.25 (d, *J* = 1.3 Hz, 2H), 4.93 (dd, *J* = 12.1, 5.1 Hz, 1H), 4.86 (d, *J* = 4.1 Hz, 1H), 4.22 (q, *J* = 7.3, 6.1 Hz, 1H), 2.93 - 2.79 (m, 2H), 2.78 - 2.67 (m, 3H), 2.40 (dt, *J* = 13.0, 6.2 Hz, 2H), 2.13 (ddd, *J* = 10.3, 5.0, 2.7 Hz, 1H), 2.04-1.98 (m, 1H), 1.64 (s, 6H). LC/MS (ESI+) Calcd for C₄₀H₃₆FN₇O₆ ([M+H]⁺) m/z 730.3; found 730.3.

### Example 288: 2-(2,6-dioxopiperidin-3-yl)-5-(1r,3r)-3-(4-(2-(2-(5-methyl-1,2,4-oxadiazol-3-yl)pyrimidin-4-yl)oxy)phenyl)propan-2-yl)phenoxy)cyclobutyl) amino)isoindolin-1,3-dione

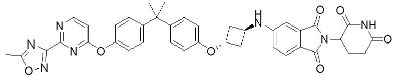

The target compound was synthesized by a method similar to that of Example 276. ¹H NMR (400 MHz, Chloroform-*d*) *δ* 8.78 - 8.68 (m, 1H), 8.06 (s, 1H), 7.63 (d, *J* = 8.2 Hz, 1H), 7.35-7.28 (m, 2H), 7.21 - 7.09 (m, 4H), 6.90 (s, 1H), 6.85 (d, *J* = 5.5 Hz, 1H), 6.77 - 6.67 (m, 3H), 4.97 - 4.84 (m, 2H), 4.24 (s,1H), 2.95 - 2.78 (m, 2H), 2.70 (s, 6H), 2.41 (t, *J* = 8.5 Hz, 2H), 2.16 - 2.08 (m, 1H), 1.69 (s, 6H). LC/MS (ESI+) Calcd for C₃₉H₃₅N₇O₇ ([M+H]⁺) m/z 714.3; found 714.3.

### Example 289: 2-(2,6-dioxopiperidin-3-yl)-5-(1r,3r)-3-(4-(2-(2-(5-methyl-1,3,4-oxadiazol-2-yl)pyrimidin-4-yl)oxy)phenyl)propan-2-yl)phenoxy)cyclobutyl) amino)isoindolin-1,3-dione

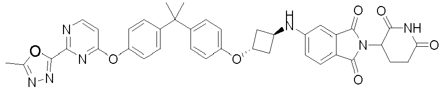

The target compound was synthesized by a method similar to that of Example 276. ¹H NMR (400 MHz, Chloroform-*d*) *δ* 8.73 (d, *J* = 5.7 Hz, 1H), 8.06 (s, 1H), 7.64 (d, *J* = 8.3 Hz, 1H), 7.37 - 7.30 (m, 2H), 7.21 - 7.15 (m, 2H), 7.12 (d, *J* = 8.7 Hz, 2H), 6.93 - 6.85 (m, 2H), 6.78 - 6.68 (m, 3H), 4.98 - 4.84 (m, 2H), 4.24 (s, 1H), 2.89 (d, *J* = 17.0 Hz, 1H), 2.84 - 2.69 (m, 3H), 2.66 (s, 3H), 2.47 - 2.37 (m, 2H), 2.16 - 2.09 (m, 1H), 2.06 - 1.97 (m, 1H), 1.70 (s, 6H). LC/MS (ESI+) Calcd for C₃₉H₃₅N₇O₇ ([M+H]⁺) *m*/*z* 714.3; found 714.3.

### Example 290: 2-(2,6-dioxopyridin-3-yl)-5-(1r,3r)-3-(4-(2-(4-(2-(1-hydroxyl-2-methylpropyl)pyrimidin-5-yl)oxyphenyl)propan-2-yl)phenoxy)cyclobutyl) amino)isoindolin-1,3-dione

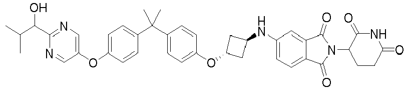

The target compound was synthesized by a method similar to that of Example 118. ¹H NMR (400 MHz, Chloroform-*d*) *δ* 8.46 (s, 2H), 8.12 (d, *J* = 4.0 Hz, 1H), 7.63 (d, *J* = 8.2 Hz, 1H), 7.25 (d, *J* = 4.5 Hz, 2H), 7.17 -7.11 (m, 2H), 6.98 - 6.92 (m, 2H), 6.89 (d, *J* = 2.1 Hz, 1H), 6.71 (dd, *J* = 8.8, 2.5 Hz, 3H), 4.93 (dd, *J* = 12.1, 5.2 Hz, 1H), 4.89 (dd, *J* = 7.0, 4.1 Hz, 1H), 4.68 (d, *J* = 3.9 Hz, 1H), 4.29 - 4.19 (m, 1H), 2.93 - 2.79 (m, 2H), 2.77 - 2.67 (m, 3H), 2.41 (dt, *J* = 13.0, 6.3 Hz, 2H), 2.25 (ddd, *J* = 9.7, 6.8, 3.3 Hz, 2H), 2.16 - 2.09 (m, 1H), 1.67 (s, 6H), 1.06 (d, *J* = 6.9 Hz, 3H), 0.76 (d, *J* = 6.8 Hz, 3H). LC/MS (ESI+) Calcd for C₄₀H₄₁N₅O₇([M+H]⁺) m/z 704.3; found 704.3.

### Example 291: 2-(2,6-dioxopiperidin-3-yl)-5-((1r,3r)-3-(4-(2-(4-((2-isobutylpyrimidin-5-yl)oxy)phenyl)propan-2-yl)phenoxy)cyclobutyl)amino) isoindolin-1,3-dione

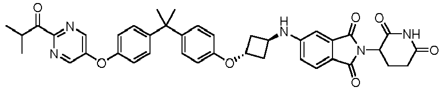

The target compound was synthesized by a method similar to that of Example 119. ¹H NMR (400 MHz, Chloroform-*d*) *δ* 8.54 (s, 2H), 8.05 (s, 1H), 7.63 (d, *J* = 8.2 Hz, 1H), 7.30 (d, *J* = 8.1 Hz, 2H), 7.14 (d, *J* = 8.2 Hz, 2H), 7.01 (d, *J* = 8.1 Hz, 2H), 6.89 (d, *J* = 1.9 Hz, 1H), 6.72 (dd, *J* = 9.1, 5.0 Hz, 3H), 4.93 (dd, *J* = 12.1, 5.2 Hz, 1H), 4.88 (s, 1H), 4.24 (s, 1H), 3.96 (p, *J* = 6.8 Hz, 1H), 2.93 - 2.78 (m, 2H), 2.78 - 2.68 (m, 3H), 2.47 - 2.34 (m, 2H), 2.16 - 2.09 (m, 1H), 1.68 (s, 6H), 1.24 (d, *J=* 6.9 Hz, 6H). LC/MS (ESI+) Calcd for C₄₀H₃₉N₅O₇ ([M+H]⁺) *m*/*z* 702.3; found 702.3.

### Example 292: 2-(2,6-dioxopiperidin-3-yl)-5-(1s,3s)-3-(4-(2-(2-(5-methyl-1,2,4-oxadiazol-3-yl)pyrimidin-4-yl)oxy)phenyl)propan-2-yl)phenoxy)cyclobutyl) amino)isoindolin-1,3-dione

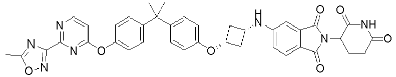

The target compound was synthesized by a method similar to that of Example 276. ¹H NMR (400 MHz, DMSO-*d*₆) *δ* 11.07 (s, 1H), 8.85 (d, *J* = 5.8 Hz, 1H), 7.59 (d, *J* = 8.3 Hz, 1H), 7.45 (d, *J* = 6.5 Hz, 1H), 7.36 - 7.27 (m, 2H), 7.24 - 7.13 (m, 5H), 6.90 (s, 1H), 6.86 - 6.77 (m, 3H), 5.04 (dd, *J* = 12.9, 5.4 Hz, 1H), 4.55 - 4.46 (m, 1H), 3.79 (q, *J* = 7.4 Hz, 1H), 3.06 (s, 2H), 2.66 (s, 3H), 2.51 (d, *J* = 1.9 Hz, 4H), 1.98 (q, *J* = 10.5, 8.5 Hz, 3H), 1.65 (s, 6H). LC/MS (ESI+) Calcd for C₃₉H₃₅N₇O₇ ([M+H]⁺) *m*/*z* 714.3; found 714.3.

### Example 293: 2-(2,6-dioxopyridin-3-yl)-5-((1r,3r)-3-(4-(1-(4-((2-(5-methyl-1,2,4-oxadiazol-3-yl)pyrimidin-5-yl)oxy)phenyl)cyclopentyl)phenoxy)cyclobutyl) amino)isoindolin-1,3-dione

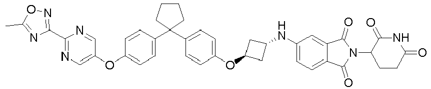

The target compound was synthesized by a method similar to that of Example 276. ¹H NMR (400 MHz, DMSO-*d*₆) *δ* 11.07 (s, 1H), 8.73 (s, 2H), 7.59 (d, *J* = 8.3 Hz, 1H), 7.52 (d, *J* = 5.4 Hz, 1H), 7.41 - 7.35 (m, 2H), 7.24 (d, *J* = 8.8 Hz, 2H), 7.17 - 7.10 (m, 2H), 6.86 (s, 1H), 6.79 (d, *J* = 8.1 Hz, 1H), 6.77 - 6.71 (m, 2H), 5.04 (dd, *J* = 12.9, 5.4 Hz, 1H), 4.90 - 4.81 (m, 1H), 4.13 (s, 1H), 2.93 - 2.81 (m, 1H), 2.69 (s, 3H), 2.59 (s, 1H), 2.54 (d, *J* = 9.0 Hz, 3H), 2.43 (q, *J* = 5.8 Hz, 2H), 2.26 (s, 4H), 2.04 - 1.95 (m, 1H), 1.62 (s, 4H). LC/MS (ESI+) Calcd for C₄₁H₃₇N₇O₇([M+H]⁺) *m*/*z* 740.3; found 740.3.

### Example 294: 5-((1s,3s)-3-(4-(3-(4-((5-acetylpyrazin-2-yl)oxy)phenyl)pentan-3-yl)phenoxy)cyclobutyl)amino)-2-(2,6-dioxopyridin-3-yl)isoindolin-1,3-dione

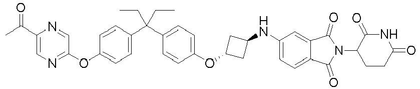

The target compound was synthesized by a method similar to that of Example 119. ¹H NMR (400 MHz, DMSO-*d*₆) *δ* 11.07 (s, 1H), 8.70 (d, *J* = 1.3 Hz, 1H), 8.60 (d, *J* = 1.3 Hz, 1H), 7.59 (d, *J* = 8.3 Hz, 1H), 7.53 (d, *J* = 5.4 Hz, 1H), 7.25 - 7.19 (m, 2H), 7.19 - 7.13 (m, 2H), 7.10 (d, *J* = 8.8 Hz, 2H), 6.89 - 6.73 (m, 4H), 5.04 (dd, *J* = 12.9, 5.4 Hz, 1H), 4.92 - 4.82 (m, 1H), 4.14 (d, *J* = 8.3 Hz, 1H), 2.95 - 2.81 (m, 1H), 2.60 (s, 3H), 2.58 - 2.52 (m, 4H), 2.47 - 2.38 (m, 2H), 2.07 (q, *J* = 7.1 Hz, 4H), 2.00 (d, *J* = 7.3 Hz, 1H), 1.23 (d, *J* = 3.6 Hz, 1H), 0.58 (t, *J* = 7.2 Hz, 6H). LC/MS (ESI+) Calcd for C₄₀H₃₉N₅O₇ ([M+H]⁺) *m*/*z* 702.3; found 702.3.

### Example 295: 2-(2,6-dioxopiperidin-3-yl)-5-((1s,3s)-3-(4-(3-(4-((5-(1-hydroxylethyl)pyrazin-2-yl)oxy)phenyl)pentan-3-yl)phenoxy)cyclobutyl)amino) isoindolin-1,3-dione

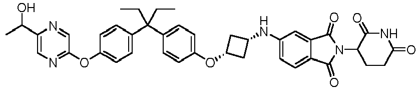

The target compound was synthesized by a method similar to that of Example 118. ¹H NMR (400 MHz, DMSO-*d*₆) *δ* 11.08 (s, 1H), 8.39 (d, *J* = 1.3 Hz, 1H), 8.25 (d, *J* = 1.4 Hz, 1H), 7.59 (d, *J* = 8.3 Hz, 1H), 7.46 (d, *J* = 6.4 Hz, 1H), 7.21 - 7.15 (m, 2H), 7.12 - 7.03 (m, 4H), 6.90 (d, *J* = 2.1 Hz, 1H), 6.81 (td, *J* = 8.7, 1.9 Hz, 3H), 5.49 (d, *J* = 4.7 Hz, 1H), 5.04 (dd, *J* = 12.9, 5.4 Hz, 1H), 4.84 - 4.75 (m, 1H), 4.50 (p, *J* = 7.1 Hz, 1H), 3.78 (q, *J* = 7.4 Hz, 1H), 3.06 (s, 2H), 2.93-2.81 (m, 1H), 2.63 - 2.52 (m, 1H), 2.06 (q, *J* = 7.4 Hz, 4H), 1.98 (d, *J* = 6.9 Hz, 4H), 1.39 (d, *J* = 6.5 Hz, 3H), 0.57 (t, *J* = 7.2 Hz, 6H). LC/MS (ESI+) Calcd for C₄₀H₄₁N₅O₇ ([M+H]⁺) *m*/*z* 704.3; found 704.3.

### Example 296: 5-((1s,3s)-3-(4-(3-(4-((5-acetylpyrazin-2-yl)oxy)phenyl)pentan-3-yl)phenoxy)cyclobutyl)amino)-2-(2,6-dioxopyridin-3-yl)isoindolin-1,3-dione

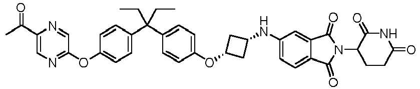

The target compound was synthesized by a method similar to that of Example 119. ¹H NMR (400 MHz, DMSO-*d*₆) *δ* 11.07 (s, 1H), 8.70 (d, *J* = 1.3 Hz, 1H), 8.60 (d, *J* = 1.3 Hz, 1H), 7.59 (d, *J* = 8.3 Hz, 1H), 7.45 (d, *J=* 6.4 Hz, 1H), 7.25 - 7.18 (m, 2H), 7.18 - 7.12 (m, 4H), 7.09 (d, *J* = 8.8 Hz, 2H), 6.90 (d, *J* = 2.1 Hz, 1H), 6.81 (t, *J* = 9.1 Hz, 3H), 5.04 (dd, *J* = 12.9, 5.4 Hz, 1H), 4.50 (p, *J* = 7.1 Hz, 1H), 3.78 (q, *J* = 7.4 Hz, 1H), 3.06 (d, *J* = 7.0 Hz, 1H), 2.94 - 2.81 (m, 1H), 2.60 (s, 3H), 2.54 (d, *J* = 9.5 Hz, 1H), 2.07 (q, *J* = 7.2 Hz, 4H), 1.98 (dd, *J* = 15.8, 8.1 Hz, 4H), 0.57 (t, *J* = 7.2 Hz, 6H). LC/MS (ESI+) Calcd for C₄₀H₃₉N₅O₇ ([M+H]⁺) *m*/*z* 702.3; found 702.3.

### Example 297: 5-((1r,3r)-3-(4-(2-(2-(3-amino-3-methylazetidin-1-yl)pyrimidin-4-yl)methoxy)phenyl)propan-2-yl)phenoxy)cyclobutyl)amino)-2-(2,6-dioxopiperidin-3-yl)isoindolin-1,3-dione

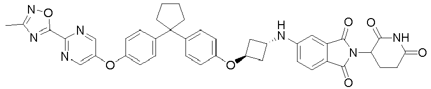

The target compound was synthesized by a method similar to that of Example 276. ¹H NMR (400 MHz, DMSO-*d*₆) *δ* 11.07 (s, 1H), 8.77 (s, 2H), 7.59 (d, *J* = 8.3 Hz, 1H), 7.53 (d, *J* = 5.4 Hz, 1H), 7.44 - 7.37 (m, 2H), 7.24 (d, *J* = 8.8 Hz, 2H), 7.20 - 7.11 (m, 2H), 6.85 (s, 1H), 6.79 (d, *J* = 8.4 Hz, 1H), 6.74 (d, *J* = 8.7 Hz, 2H), 5.04 (dd, *J* = 12.9, 5.4 Hz, 1H), 4.84 (q, *J* = 5.8 Hz, 1H), 4.13 (s, 1H), 2.93 - 2.81 (m, 1H), 2.57 (d, *J* = 16.6 Hz, 3H), 2.46 (m, 5H), 2.26 (s, 4H), 2.09 - 1.92 (m, 1H), 1.62 (s, 4H), 1.23 (s, 1H). LC/MS (ESI+) Calcd for C₄₁H₃₇N₇O₇([M+H]⁺) m/z 740.3; found 740.3.

### Example 298: 5-(3-((8-(4-(2-(4-((2-acetylpyrimidin-4-yl)methoxy)phenyl)propan-2-yl)phenoxy)octyl)oxy)methyl)azetidin-1-yl)-2-(2,6-dioxopiperidin-3-yl) isoindolin-1,3-dione

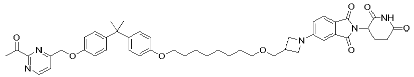

The target compound was synthesized by a method similar to that of Example 136. ¹H NMR (400 MHz, DMSO-*d*₆) *δ* 11.08 (s, 1H), 9.01 (d, *J* = 5.1 Hz, 1H), 8.01 (dd, *J* = 8.3, 4.5 Hz, 1H), 7.86 (dd, *J* = 7.4, 2.3 Hz, 1H), 7.76 (dd, *J* = 5.6, 3.7 Hz, 1H), 7.62 (d, *J* = 8.3 Hz, 1H), 7.15 - 7.05 (m, 4H), 6.97 - 6.92 (m, 2H), 6.77 (d, *J* = 2.1 Hz, 1H), 6.62 (dd, *J* = 8.4, 2.1 Hz, 1H), 5.26 (s, 2H), 5.17 (dd, *J* = 12.9, 5.3 Hz, 2H), 5.05 (dd, *J* = 12.9, 5.4 Hz, 1H), 4.07 (t, *J* = 8.3 Hz, 2H), 3.89 (t, *J* = 6.4 Hz, 2H), 3.74 (dd, *J* = 8.4, 5.3 Hz, 2H), 3.56 (d, *J* = 6.4 Hz, 2H), 3.42 (d, *J* = 6.5 Hz, 2H), 2.98 (d, *J* = 6.8 Hz, 1H), 2.91 - 2.83 (m, 2H), 2.67 (s, 3H), 2.63 - 2.56 (m, 2H), 2.07 (ddd, *J* = 12.8, 5.6, 3.3 Hz, 2H), 2.02 - 1.94 (m, 1H), 1.65 (q, *J* = 7.2 Hz, 2H), 1.56 (d, *J* = 6.6 Hz, 6H), 1.49 (s, 2H), 1.35 (dd, *J* = 9.9, 5.5 Hz, 2H), 1.23 (s, 1H). LC/MS (ESI+) calcd for C₄₇H₅₃N₅O₈ ( [M+H]⁺ ) *m*/*z* 815.97; found 816.2.

### Example 299: 5-(4-(2-((8-(4-(2-(4-((2-acetylpyrimidin-4-yl)methoxy)phenyl) propan-2-yl)phenoxy)octyl)oxy)ethyl)piperazin-1-yl)-2-(2,6-dioxopiperidin-3-yl)isoindolin-1,3-dione

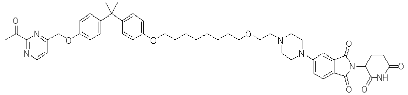

The target compound was synthesized by a method similar to that of Example 136. ¹H NMR (400 MHz, DMSO-*d*₆) *δ* 11.09 (s, 1H), 9.01 (d, *J* = 5.1 Hz, 1H), 7.76 (d, *J* = 5.1 Hz, 1H), 7.66 (d, *J* = 8.5 Hz, 1H), 7.33 (d, *J* = 2.3 Hz, 1H), 7.24 (dd, *J* = 8.6, 2.4 Hz, 1H), 7.16 - 7.10 (m, 2H), 7.10 - 7.03 (m, 2H), 6.98 - 6.91 (m, 2H), 6.84 - 6.74 (m, 2H), 5.26 (s, 2H), 5.07 (dd, *J* = 12.9, 5.4 Hz, 1H), 3.89 (t, *J* = 6.4 Hz, 2H), 3.50 (t, *J* = 5.7 Hz, 2H), 3.41 (d, *J* = 10.3 Hz, 4H), 3.38 (s, 2H), 3.16 (d, *J* = 4.7 Hz, 1H), 2.94 - 2.81 (m, 1H), 2.67 (s, 3H), 2.63 - 2.52 (m, 6H), 2.04 - 1.96 (m, 1H), 1.66 (q, *J* = 6.9 Hz, 2H), 1.56 (s, 6H), 1.48 (t, *J* = 6.6 Hz, 2H), 1.42 - 1.35 (m, 2H), 1.26 (d, *J* = 24.1 Hz, 7H). LC/MS (ESI+) calcd for C₄₉H₅₈N₆O₈ ( [M+H]⁺ ) *m*/*z* 859.04; found 859.3.

### Example 300: 2-(2,6-dioxopiperidin-3-yl)-5-(4-(2-((8-(4-(2-(1-hydroxylethyl) pyrimidin-4-yl)methoxy)phenyl)propan-2-yl)phenoxy)octyl)oxy)ethyl)piperazin-1-yl)isoindolin-1,3-dione

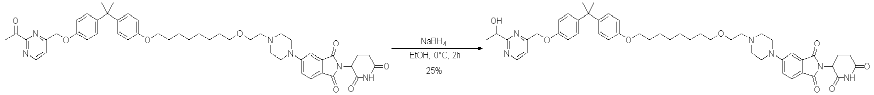

Compound 5-(4-(2-((8-(4-(2-(4-((2-acetylpyrimidin-4-yl)methoxy)phenyl) propan-2-yl)phenoxy)octyl)oxy)ethyl)piperazin-1-yl)-2-(2,6-dioxopiperidin-3-yl) isoindolin-1,3-dione (100 mg, 0.12 mmol) was added into 6 mL of ethanol, to which was added sodium borohydride (18 mg, 0.48 mmol) in an ice bath, and then the solution was stirred in the ice bath for 2 h, and then the resultant solution was extracted with ethyl acetate for three times. The organic phase was dried with anhydrous Na₂SO₄, and then rotatory evaporated to dry, to provide compound 2-(2,6-dioxopiperidin-3-yl)-5-(4-(2-((8-(4-(2-(1-hydroxylethyl)pyrimidin-4-yl)methoxy)phenyl)propan-2-yl)phenoxy) octyl)oxy)ethyl)piperazin-1-yl)isoindolin-1,3-dione (26 mg, 0.03 mmol), with a yield of 25%. ¹H NMR (400 MHz, DMSO-*d*₆) *δ* 11.10 (s, 1H), 8.80 (d, *J* = 5.1 Hz, 1H), 7.66 (d, *J* = 8.5 Hz, 1H), 7.45 (d, *J* = 5.1 Hz, 1H), 7.33 (d, *J* = 2.2 Hz, 1H), 7.24 (d, *J* = 8.8 Hz, 1H), 7.10 (ddd, *J* = 18.1, 8.8, 3.5 Hz, 4H), 6.96 - 6.89 (m, 2H), 6.79 (dd, *J* = 8.9, 2.9 Hz, 2H), 5.26 (d, *J* = 5.3 Hz, 1H), 5.16 (s, 2H), 5.07 (dd, *J* = 12.9, 5.4 Hz, 1H), 4.81-4.74 (m, 1H), 3.89 (t, *J* = 6.4 Hz, 2H), 3.50 (t, *J* = 5.6 Hz, 2H), 3.41 (dd, *J* = 20.5, 10.1 Hz, 5H), 2.56 (q, *J* = 10.1, 6.5 Hz, 5H), 2.33 (s, 1H), 1.99 (q, *J* = 7.0 Hz, 3H), 1.67 (t, *J* = 7.1 Hz, 2H), 1.62 (s, 1H), 1.57 (d, *J* = 3.1 Hz, 6H), 1.52 - 1.44 (m, 3H), 1.41 (d, *J* = 6.6 Hz, 3H), 1.38 (d, *J* = 3.3 Hz, 3H), 1.23 (s, 4H). LC/MS (ESI+) calcd for C₄₉H₆₀N₄O₈ ( [M+H]⁺ ) *m*/*z* 861.05; found 861.3.

### Example 301: 4-(3-((4-(2-(4-((2-acetylpyrimidin-4-yl)methoxy)phenyl)propan-2-yl)phenoxy)methyl)pyrrolidin-1-yl)-2-(2,6-dioxopiperidin-3-yl)isoindolin-1,3-dione

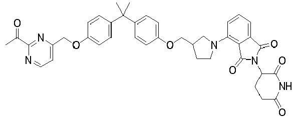

The target compound was synthesized by a method similar to that of Example 136. ¹H NMR (400 MHz, CDCl₃) *δ* 8.92 (d, *J* = 5.1 Hz, 1H), 8.06 (d, *J* = 6.8 Hz, 1H), 7.74 (d, *J* = 5.1 Hz, 1H), 7.47 (dd, *J* = 8.6, 6.9 Hz, 1H), 7.22 (d, *J* = 6.9 Hz, 1H), 7.14 (ddd, *J* = 9.8, 7.1, 5.0 Hz, 4H), 6.96 (d, *J* = 8.7 Hz, 1H), 6.89 - 6.83 (m, 2H), 6.83 - 6.77 (m, 2H), 5.25 (s, 2H), 4.95 (dd, *J* = 11.8, 5.3 Hz, 1H), 3.97 (dd, *J* = 6.9, 2.4 Hz, 2H), 3.83 - 3.75 (m, 1H), 3.76 - 3.67 (m, 2H), 3.60 (t, *J* = 8.6 Hz, 1H), 2.94 - 2.82 (m, 2H), 2.81 (s, 3H), 2.80 - 2.69 (m, 2H), 2.31 - 2.17 (m, 1H), 2.13 (dd, *J* = 8.8, 6.2 Hz, 1H), 1.95 (dd, *J* = 12.6, 7.6 Hz, 1H), 1.64 (s, 6H). LC/MS (ESI+) calcd for C₄₀H₃₉N₅O₇ ( [M+H]⁺ ) *m*/*z* 701.78; found 702.3.

### Example 302: 5-(3-((4-(2-(4-((2-acetylpyrimidin-4-yl)methoxy)phenyl)propan-2-yl)phenoxy)methyl)pyrrolidin-1-yl)-2-(2,6-dioxopiperidin-3-yl)isoindolin-1,3-dione

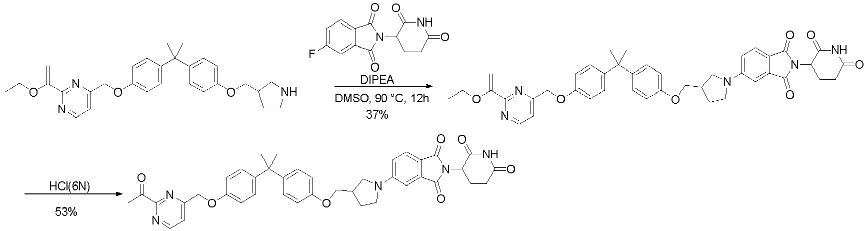

The target compound was synthesized by a method similar to that of Example 136. ¹H NMR (400 MHz, DMSO-*d*₆) *δ* 11.08 (s, 1H), 9.02 (d, *J* = 5.1 Hz, 1H), 7.77 (d, *J* = 5.1 Hz, 1H), 7.65 (d, *J* = 8.4 Hz, 1H), 7.16 - 7.08 (m, 4H), 6.98 - 6.90 (m, 3H), 6.85 (t, *J* = 8.7 Hz, 3H), 5.26 (s, 2H), 5.05 (dd, *J* = 12.9, 5.5 Hz, 1H), 4.06 - 3.91 (m, 3H), 3.63 (t, *J* = 9.0 Hz, 1H), 3.55 (s, 1H), 3.46 (d, *J* = 8.4 Hz, 1H), 2.94 - 2.77 (m, 3H), 2.67 (s, 3H), 2.21 (d, *J* = 6.4 Hz, 1H), 2.04 - 1.89 (m, 3H), 1.58 (s, 6H). LC/MS (ESI+) calcd for C₄₀H₃₉N₅O₇ ( [M+H]⁺ ) *m*/*z* 701.78; found 702.3.

### Example 303: 4-(3-((4-(2-(4-((2-acetylpyrimidin-4-yl)methoxy)phenyl)propan-2-yl)phenoxy)methyl)piperidin-1-yl)-2-(2,6-dioxopiperidin-3-yl)isoindolin-1,3-dione

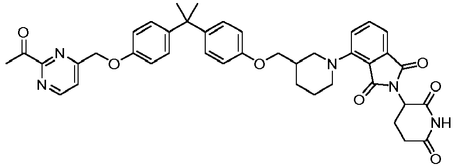

The target compound was synthesized by a method similar to that of Example 136. ¹H NMR (400 MHz, CDCl₃) *δ* 8.92 (d, *J* = 5.1 Hz, 1H), 8.09 (d, *J* = 8.7 Hz, 1H), 7.74 (d, *J* = 4.9 Hz, 1H), 7.56 (d, *J* = 8.3 Hz, 1H), 7.37 (d, *J* = 7.3 Hz, 1H), 7.14 (dd, *J* = 17.0, 8.4 Hz, 5H), 6.86 (d, *J* = 8.8 Hz, 2H), 6.79 (d, *J* = 8.6 Hz, 2H), 5.25 (s, 2H), 4.96 (d, *J* = 11.5 Hz, 1H), 3.89 (t, *J* = 8.7 Hz, 2H), 3.81 (d, *J* = 12.1 Hz, 1H), 3.68 (s, 2H), 2.87 (s, 2H), 2.80 (s, 3H), 2.72 (s, 2H), 2.34 (s, 1H), 2.12 (s, 1H), 1.64 - 1.63 (m, 6H). LC/MS (ESI+) calcd for C₄₁H₄₁N₅O₇ ( [M+H]⁺ ) *m*/*z* 715.81; found 716.3.

### Example 304: 5-(3-((4-(2-(4-((2-acetylpyrimidin-4-yl)methoxy)phenyl)propan-2-yl)phenoxy)methyl)piperidin-1-yl)-2-(2,6-dioxopiperidin-3-yl)isoindolin-1,3-dione

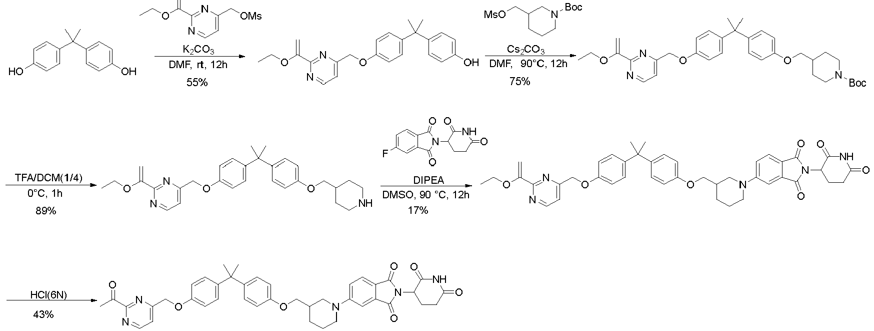

### Step 1: Synthesis of 4-(2-(4-((2-(1-ethoxyvinyl)pyrimidin-4-yl)methoxy)phenyl) propan-2-yl)phenol

Bisphenol A (1.88 g, 8.2 mmol) and (2-(1-ethoxyvinyl)pyrimidin-4-yl)methanesulfonate (1.42 g, 5.5 mmol) were dissolved in 25 mL of DMF, to which was added K₂CO₃ (1.51 g, 10.9 mmol), and then the mixture was allowed to react overnight at room temperature. The reaction was quenched by adding water. The resultant solution was extracted with ethyl acetate, and the water layer was re-extracted once with ethyl acetate. The organic layers were combined, washed with saturated brine, dried with anhydrous Na₂SO₄, and concentrated. The residue was separated and purified by prep-TLC, to provide compound 4-(2-(4-((2-(1-ethoxyvinyl)pyrimidin-4-yl)methoxy)phenyl)propan-2-yl)phenol (1.17 g, yield 55%). LC/MS (ESI+) calcd for C₂₄H₂₆N₂O₃ ( [M+H]⁺ ) *m*/*z* 390.48; found 391.3.

### Step 2: Synthesis of 4-(4-(2-(4-((2-(1-ethoxyvinyl)pyrimidin-4-yl)methoxy)phenyl) propan-2-yl)phenoxy)methyl)piperidin-1-carboxylate tert-butyl

4-(2-(4-((2-(1-ethoxyvinyl)pyrimidin-4-yl)methoxy)phenyl)propan-2-yl)phenol (1.17 g, 3.0 mmol) and tert-butyl 3-((methanesulfonyl)oxy)methyl)piperidin-1-carboxylate (0.97 g, 3.3 mmol) were dissolved in 20 mL of DMF, to which was added Cs₂CO₃ (2.93 g, 9.0 mmol), and then the mixture was heated to 60 °C and reacted overnight. The reaction solution was cooled to room temperature, and then the reaction was quenched by adding water. The resultant solution was extracted with ethyl acetate, and the water layer was re-extracted once with ethyl acetate. The organic layers were combined, washed with saturated brine, dried with anhydrous Na₂SO₄, and concentrated. The residue was separated and purified by prep-TLC, to provide compound tert-butyl 4-(4-(2-(4-((2-(1-ethoxyvinyl)pyrimidin-4-yl)methoxy)phenyl) propan-2-yl)phenoxy)methyl)piperidin-1-carboxylate (1.32 g, yield 75%). LC/MS (ESI+) calcd for C₃₅H₄₅N₃O₅ ( [M+H]⁺ ) *m*/*z* 587.76; found 588.3.

### Step 3: Synthesis of 2-(1-ethoxyvinyl)-4-(4-(2-(4-(piperidin-4-yl)methoxy)phenyl) propan-2-ylphenoxy)methyl)pyrimidine

tert-butyl 4-(4-(2-(4-((2-(1-ethoxyvinyl)pyrimidin-4-yl)methoxy)phenyl)propan-2-yl) phenoxy)methyl)piperidin-1-carboxylate (300 mg, 0.51 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 2-(1-ethoxyvinyl)-4-(4-(2-(4-(piperidin-4-yl)methoxy)phenyl)propan-2-ylphenoxy)methyl)pyrimidine (221 mg, yield 89%).

### Step 4: Synthesis of 2-(2,6-dioxopiperidin-3-yl)-5-(3-(4-(2-(4-(2-(1-ethoxyvinyl) pyrimidin-4-yl)methoxy)phenyl)propan-2-yl)phenoxy)methyl)piperidin-1-yl)isoindol-1,3-dione

2-(1-ethoxyvinyl)-4-(4-(2-(4-(piperidin-4-yl)methoxy)phenyl)propan-2-yl phenoxy)methyl)pyrimidine (221 mg, 0.45 mmol) was dissolved in 5 mL of DMSO, to which were successively added DIPEA (173 mg, 1.35 mmol) and 2-(2,6-dioxopiperidin-3-yl)-5-fluoroisoindolin-1,3-dione (150 mg, 0.54 mmol), and then the mixture was heated to 60 °C and reacted overnight under N₂ protection. The reaction solution was cooled to room temperature, and water was added to quench the reaction. The resultant solution was extracted with ethyl acetate for three times. The organic phase was washed with saturated NaCl solution, dried over anhydrous Na₂SO₄, concentrated and rotatory evaporated to dry. The residue was separated and purified by prep-TLC, to provide 2-(2,6-dioxopiperidin-3-yl)-5-(3-(4-(2-(4-(2-(1-ethoxyvinyl) pyrimidin-4-yl)methoxy)phenyl)propan-2-yl)phenoxy)methyl)piperidin-1-yl)isoindol-1,3-dione (56 mg, with a yield of 17%). LC/MS (ESI+) calcd for C₄₃H₄₅N₅O₇ ( [M+H]⁺ ) *m*/*z* 743.33; found 744.3.

### Step 5: Synthesis of 5-(3-((4-(2-(4-((2-acetylpyrimidin-4-yl)methoxy)phenyl)propan-2-yl)phenoxy)methyl)piperidin-1-yl)-2-(2,6-dioxopiperidin-3-yl)isoindolin-1,3-dione

2-(2,6-dioxopiperidin-3-yl)-5-(3-(4-(2-(4-(2-(1-ethoxyvinyl)pyrimidin-4-yl) methoxy)phenyl)propan-2-yl)phenoxy)methyl)piperidin-1-yl)isoindol-1,3-dione (56 mg, 0.075 mmol) was dissolved in 3 mL of acetone, to which was added 2 mL of conc. HCl solution, and then the mixture was allowed to react at room temperature for 3 h. After completion of the reaction detected by TLC, 10 mL of water was added, and then the resultant solution was extracted with ethyl acetate for three times. The organic layers were combined, washed with saturated brine, dried with anhydrous Na₂SO₄, and concentrated to dry under reduced pressure. The residue was separated and purified by prep-TLC, to provide the product 5-(3-((4-(2-(4-((2-acetylpyrimidin-4-yl)methoxy) phenyl)propan-2-yl)phenoxy)methyl)piperidin-1-yl)-2-(2,6-dioxopiperidin-3-yl) isoindolin-1,3-dione (23 mg, yield 43%). ¹H NMR (400 MHz, DMSO-*d*₆) *δ* 11.09 (s, 1H), 9.02 (d, *J* = 5.1 Hz, 1H), 7.77 (d, *J* = 5.1 Hz, 1H), 7.64 (d, *J* = 3.3 Hz, 1H), 7.13-7.09 (m, 4H), 6.94 (dd, *J* = 14.3, 8.8 Hz, 3H), 6.85 (q, *J* = 3.3 Hz, 3H), 5.27 (s, 2H), 4.98 (d, *J* = 8.5 Hz, 1H), 4.36 (t, *J* = 5.0 Hz, 1H), 4.11 (q, *J* = 5.3 Hz, 1H), 3.48 - 3.40 (m, 2H), 3.16 (d, *J* = 5.2 Hz, 1H), 2.88 (d, *J* = 5.4 Hz, 2H), 2.67 (s, 3H), 1.84 (d, *J* = 21.8 Hz, 4H), 1.72 (s, 4H), 1.58 (s, 6H). LC/MS (ESI+) calcd for C₄₁H₄₁N₅O₇ ( [M+H]⁺ ) ***m*/*z*** 715.81; found 716.3.

### Example 305: 5-(7-(4-(2-(2-acetylpyrimidin-4-yl)methoxy)phenyl)propan-2-yl)phenyl)-2,7-diazaspiro[3.5]nonan-2-yl)-2-(2,6-dioxopiperidin-3-yl)isoindolin-1,3-dione

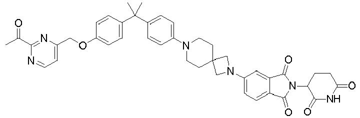

The target compound was synthesized by a method similar to that of Example 304. ¹H NMR (400 MHz, CDCl₃) *δ* 8.92 (d, *J* = 5.1 Hz, 1H), 8.06 (s, 1H), 7.74 (d, *J* = 5.1 Hz, 1H), 7.66 (d, *J* = 8.2 Hz, 1H), 7.17 (d, *J* = 8.8 Hz, 4H), 6.95 - 6.84 (m, 3H), 6.80 (d, *J* = 2.1 Hz, 1H), 6.57 - 6.51 (m, 1H), 5.25 (s, 2H), 4.94 (dd, *J* = 12.2, 5.2 Hz, 2H), 3.81 (s, 4H), 3.19 (s, 4H), 2.94 - 2.82 (m, 2H), 2.75 (d, *J* = 17.4 Hz, 3H), 2.12 (d, *J* = 6.7 Hz, 2H), 2.03 (s, 3H), 1.64 (s, 6H). LC/MS (ESI+) calcd for C₄₂H₄₂N₆O₆ ( [M+H]⁺ ) *m*/*z* 726.83; found 727.3.

### Example 306: 5-((R)-1-(4-(2-(4-((2-acetylpyrimidin-4-yl)methoxy)phenyl)propan-2-yl)phenoxy)-4-chlorobutan-2-yl)amino)-2-(2,6-dioxopiperidin-3-yl)isoindolin-1,3-dione

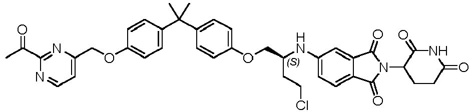

The target compound was synthesized by a method similar to that of Example 136. ¹H NMR (400 MHz, CDCl₃) *δ* 8.92 (d, *J* = 5.1 Hz, 1H), 8.23 - 8.08 (m, 1H), 7.73 (d, *J* = 5.1 Hz, 1H), 7.63 (d, *J=* 8.3 Hz, 1H), 7.22 - 7.00 (m, 5H), 6.86 (dd, *J* = 8.8, 2.8 Hz, 2H), 6.83 - 6.71 (m, 2H), 5.25 (s, 2H), 4.94 (td, *J* = 11.8, 11.0, 4.9 Hz, 1H), 4.78 (d, *J* = 9.6 Hz, 1H), 4.15 - 3.95 (m, 3H), 3.78 - 3.61 (m, 2H), 2.89 (d, *J* = 18.2 Hz, 1H), 2.80 (s, 3H), 2.78 - 2.71 (m, 1H), 2.24 (h, *J* = 7.0, 6.5 Hz, 2H), 2.18 - 2.08 (m, 1H), 1.65 (s, 6H). LC/MS (ESI+) calcd for C₃₉H₃₈ClN₅O₇ ( [M+H]⁺ ) *m*/*z* 724.21; found 724.3.

### Example 307: 5-((R)-2-((4-(2-(4-((2-acetylpyrimidin-4-yl)methoxy)phenyl) propan-2-yl)phenoxy)methyl)azetidin-1-yl)-2-(2,6-dioxopiperidin-3-yl)isoindolin-1,3-dione

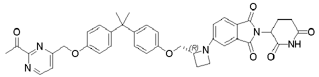

The target compound was synthesized by a method similar to that of Example 304. ¹H NMR (400 MHz, CDCl₃) *δ* 8.92 (d, *J* = 5.2 Hz, 1H), 8.05 (d, *J* = 21.6 Hz, 1H), 7.75 - 7.71 (m, 1H), 7.64 (d, *J* = 8.3 Hz, 1H), 7.19 - 7.11 (m, 5H), 6.85 (dd, *J* = 8.9, 2.7 Hz, 4H), 6.78 (d, *J* = 8.2 Hz, 1H), 5.25 (s, 2H), 4.96 - 4.91 (m, 1H), 4.64 (s, 1H), 4.25 - 4.20 (m, 2H), 4.20 - 4.14 (m, 1H), 3.87 (q, *J* = 8.0 Hz, 1H), 2.87 (s, 1H), 2.80 (s, 3H), 2.74 (d, *J* = 15.6 Hz, 2H), 2.60 (s, 1H), 2.39 (s, 1H), 2.17 - 2.09 (m, 1H), 1.63 (s, 6H). LC/MS (ESI+) calcd for C₃₉H₃₇N₅O₇ ( [M+H]⁺ ) *m*/*z* 687.75; found 688.3.

### Example 308: 5-((S)-2-((4-(2-(4-((2-acetylpyrimidin-4-yl)methoxy)phenyl)propan-2-yl)phenoxy)methyl)azetidin-1-yl)-2-(2,6-dioxopiperidin-3-yl)isoindolin-1,3-dione

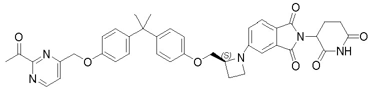

The target compound was synthesized by a method similar to that of Example 304. ¹H NMR (400 MHz, CDCl₃) *δ* 8.92 (d, *J* = 5.0 Hz, 1H), 8.13 (d, *J=* 20.6 Hz, 1H), 7.73 (dd, *J* = 5.1, 1.8 Hz, 1H), 7.64 (d, *J* = 8.3 Hz, 1H), 7.15 (dq, *J* = 9.2, 3.7, 3.2 Hz, 5H), 6.88 - 6.82 (m, 4H), 6.78 (dd, *J* = 8.3, 2.1 Hz, 1H), 5.28 - 5.22 (m, 2H), 4.97 - 4.89 (m, 1H), 4.63 (dq, *J* = 8.9, 5.6 Hz, 1H), 4.22 (dd, *J* = 5.0, 2.2 Hz, 2H), 4.16 (td, *J* = 8.5, 5.0 Hz, 1H), 3.87 (q, *J* = 7.8 Hz, 1H), 2.93 - 2.85 (m, 1H), 2.80 (s, 3H), 2.79 - 2.70 (m, 2H), 2.66 - 2.53 (m, 1H), 2.38 (ddt, *J* = 11.6, 8.9, 6.5 Hz, 1H), 2.12 (ddd, *J* = 10.5, 4.8, 2.5 Hz, 1H), 1.63 (s, 6H). LC/MS (ESI+) calcd for C₃₉H₃₇N₅O₇ ( [M+H]⁺ ) *m*/*z* 687.75; found 688.3.

### Example 309: 5-((3-(4-(2-(4-((2-(2-oxa-6-azaspiro[3.3]heptan-6-yl)pyrimidin-4-yl)methoxy)phenyl)propan-2-yl)phenyl)prop-2-yn-1-yl)amino)-2-(2,6-dioxopiperidin-3-yl)isoindolin-1,3-dione

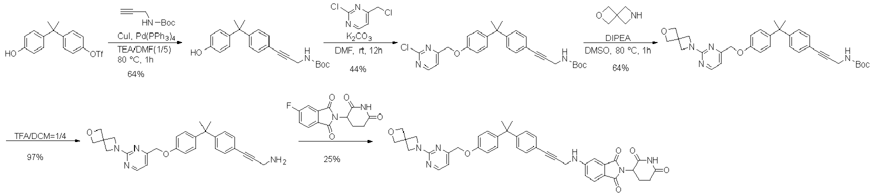

### Step 1: Synthesis of tert-butyl (3-(4-(2-(4-hydroxylphenyl)propan-2-yl)phenyl)prop-2-yn-1-yl)carbamate

4-(2-(4-hydroxylphenyl)propan-2-yl)phenyl trifluoromethanesulfonate (500 mg, 1.39 mmol), tert-butyl propylcarbamate (431 mg, 2.78 mmol), tetrakis(triphenylphosphine)palladium (98 mg, 0.14 mmol), CuI (27 mg, 0.14 mmol) and 1mL of triethylamine were successively added to 4 mL of DMF, and then the mixture was heated to 60 °C and reacted overnight. The reaction solution was cooled to room temperature, and extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl (3-(4-(2-(4-hydroxylphenyl)propan-2-yl)phenyl)prop-2-yn-1-yl)carbamate (323 mg, 0.88 mmol), with a yield of 64%. LC/MS (ESI+) calcd for C₂₃H₂₇NO₃ ( [M+H]⁺ ) *m*/*z* 365.47; found 310.2.

### Step 2: Synthesis of tert-butyl (3-(4-(2-(4-((2-chloropyrimidin-4-yl)methoxy)phenyl) propan-2-yl)phenyl)prop-2-yn-1-yl)carbamate

tert-butyl (3-(4-(2-(4-hydroxylphenyl)propan-2-yl)phenyl)prop-2-yn-1-yl) carbamate (323 mg, 0.88 mmol) and 2-chloro-4-(chloromethyl)pyrimidine (143 mg, 0.88 mmol) were dissolved in 10 mL of DMF, to which was added K₂CO₃ (242 mg, 1.76 mmol), and then the solution was stirred overnight at room temperature. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl (3-(4-(2-(4-((2-chloropyrimidin-4-yl)methoxy)phenyl)propan-2-yl)phenyl)prop-2-yn-1-yl)carbamate (190 mg, 0.39 mmol), with a yield of 44%. LC/MS (ESI+) calcd for C₂₈H₃₀ClN₃O₃ ( [M+H]⁺ ) *m*/*z* 492.02; found 436.1.

### Step 3: Synthesis of tert-butyl (3-(4-(2-(4-((2-(2-oxa-6-azaspiro[3.3]heptan-6-yl)pyrimidin-4-yl)methoxy)phenyl)propan-2-yl)phenyl)prop-2-yn-1-yl)carbamate

tert-butyl (3-(4-(2-(4-((2-chloropyrimidin-4-yl)methoxy)phenyl)propan-2-yl) phenyl)prop-2-yn-1-yl)carbamate (190 mg, 0.39 mmol), 2-oxa-6-azaspiro[3.3]heptane hemioxalate (61 mg, 0.21 mmol), and diisopropylethylamine (150 mg, 1.16 mmol) were successively added into 5 mL of DMSO, and then the mixture was heated to 60 °C and reacted overnight. The reaction solution was cooled to room temperature, and extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl (3-(4-(2-(4-((2-(2-oxa-6-azaspiro[3.3]heptan-6-yl)pyrimidin-4-yl)methoxy)phenyl)propan-2-yl)phenyl)prop-2-yn-1-yl)carbamate (136 mg, 0.25 mmol), with a yield of 64%. LC/MS (ESI+) calcd for C₃₃H₃₈N₄O₄ ( [M+H]⁺ ) *m*/*z* 554.29; found 555.3.

### Step 4: Synthesis of 3-(4-(2-(2-oxa-6-azaspiro[3.3]heptan-6-yl)pyrimidin-4-yl) methoxy)phenyl)propan-2-yl)phenyl)prop-2-yn-1-amine

tert-butyl (3 -(4-(2-(4-((2-(2-oxa-6-azaspiro[3.3]heptan-6-yl)pyrimidin-4-yl) methoxy)phenyl)propan-2-yl)phenyl)prop-2-yn-1-yl)carbamate (136 mg, 0.25 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 3-(4-(2-(2-oxa-6-azaspiro[3.3]heptan-6-yl)pyrimidin-4-yl)methoxy)phenyl) propan-2-yl)phenyl)prop-2-yn-1-amine (110 mg, 0.24 mmol), with a yield of 97%.

### Step 5: Synthesis of 5-((3-(4-(2-(4-((2-(2-oxa-6-azaspiro[3.3]heptan-6-yl)pyrimidin-4-yl)methoxy)phenyl)propan-2-yl)phenyl)prop-2-yn-1-yl)amino)-2-(2,6-dioxopiperidin-3-yl)isoindolin-1,3-dione

3-(4-(2-(2-oxa-6-azaspiro[3.3]heptan-6-yl)pyrimidin-4-yl)methoxy)phenyl) propan-2-yl)phenyl)prop-2-yn-1-amine (110 mg, 0.24 mmol) was dissolved in 6 mL of DMSO, to which were successively added DIPEA (93 mg, 0.72 mmol) and 2-(2,6-dioxopiperidin-3-yl)-5-fluoroisoindolin-1,3-dione (100 mg, 0.36 mmol), and then the mixture was heated to 60 °C and reacted overnight under N₂ protection. The reaction solution was cooled to room temperature, and water was added to quench the reaction. The resultant solution was extracted with ethyl acetate for three times. The organic phase was washed with saturated NaCl solution, dried over anhydrous Na₂SO₄, concentrated and rotatory evaporated to dry. The residue was separated and purified by prep-TLC, to provide 5-((3-(4-(2-(4-((2-(2-oxa-6-azaspiro[3.3]heptan-6-yl)pyrimidin-4-yl)methoxy)phenyl)propan-2-yl)phenyl)prop-2-yn-1-yl)amino)-2-(2,6-dioxopiperidin-3-yl)isoindolin-1,3-dione (48 mg, 0.07 mmol), with a yield of 25%. ¹H NMR (400 MHz, CDCl₃) *δ* 8.29 (dd, *J* = 5.3, 2.6 Hz, 1H), 8.20 (s, 1H), 8.14 - 8.10 (m, 1H), 7.68 (d, *J* = 8.3 Hz, 1H), 7.31 - 7.27 (m, 2H), 7.17 - 7.07 (m, 5H), 6.92 - 6.85 (m, 2H), 6.83 - 6.78 (m, 2H), 4.97 (s, 2H), 4.94 (dd, *J* = 12.3, 5.4 Hz, 1H), 4.86 (s, 3H), 4.85 (s, 1H), 4.41 (s, 4H), 4.37 - 4.34 (m, 1H), 4.25 (s, 2H), 2.94 - 2.67 (m, 4H), 2.14 (dd, *J* = 7.8, 5.2 Hz, 1H), 1.62 (s, 6H). LC/MS (ESI+) calcd for C₄₁H₃₈N₆O₆ ( [M+H]⁺ ) *m*/*z* 710.79; found 711.3.

### Example 310: 5-(3-(4-(2-(2-oxa-6-azaspiro[3.3]heptan-6-yl)pyrimidin-4-yl) methoxy)phenyl)propan-2-yl)phenoxy)prop-1-yn-1-yl)-2-(2,6-dioxopiperidin-3-yl)isoindolin-1,3-dione

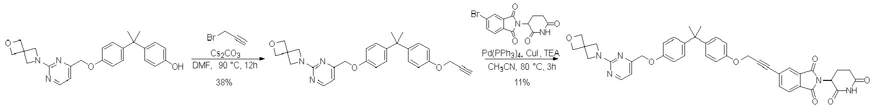

### Step 1: Synthesis of 6-(4-((4-(2-(4-(prop-2-yn-1-propoxy)phenyl)propan-2-yl)phenoxy)methyl)pyrimidin-2-yl)-2-oxa-6-azaspiro[3.3]heptane

4-(2-(4-((2-(2-oxa-6-azaspiro[3.3]heptan-6-yl)pyrimidin-4-yl)methoxy)phenyl) propan-2-yl)phenol (50 mg, 0.12 mmol) and 3-bromopropyne (28 mg, 0.24 mmol) was dissolved in 10 mL of DMF, to which was added Cs₂CO₃ (156 mg, 0.48 mmol), and then the mixture was heated to 60 °C and reacted overnight. The reaction solution was cooled to room temperature, and extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound 6-(4-((4-(2-(4-(prop-2-yn-1-propoxy)phenyl)propan-2-yl)phenoxy)methyl)pyrimidin-2-yl)-2-oxa-6-azaspiro[3.3]heptane (21 mg, 0.05 mmol), with a yield of 38%. LC/MS (ESI+) calcd for C₂₈H₂₉N₃O₃ ( [M+H]⁺ ) *m*/*z* 455.56; found 456.2.

### Step 2: Synthesis of 5-(3-(4-(2-(2-oxa-6-azaspiro[3.3]heptan-6-yl)pyrimidin-4-yl)methoxy)phenyl)propan-2-yl)phenoxy)prop-1-yn-1-yl)-2-(2,6-dioxopiperidin-3-yl)isoindolin-1,3-dione

6-(4-((4-(2-(4-(prop-2-yn-1-propoxy)phenyl)propan-2-yl)phenoxy)methyl) pyrimidin-2-yl)-2-oxa-6-azaspiro[3.3]heptane (21 mg, 0.05 mmol), 5-bromo-2-(2,6-dioxopiperidin-3-yl)isoindolin-1,3-dione (20 mg, 0.06 mmol), tetrakis(triphenylphosphine)palladium (7 mg, 0.01 mmol), CuI (3 mg, 0.01 mmol), and 1 mL of triethylamine were successively added into 4 mL of DMF, and then the mixture was heated to 60 °C and reacted overnight. The reaction solution was cooled to room temperature, and extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound 5-(3-(4-(2-(2-oxa-6-azaspiro[3.3]heptan-6-yl) pyrimidin-4-yl)methoxy)phenyl)propan-2-yl)phenoxy)prop-1-yn-1-yl)-2-(2,6-dioxopiperidin-3-yl)isoindolin-1,3-dione (4 mg, 0.006 mmol), with a yield of 11%. ¹H NMR (400 MHz, CDCl₃) *δ* 8.31 (d, *J* = 5.1 Hz, 1H), 8.25 (s, 1H), 7.89 (s, 1H), 7.86 - 7.76 (m, 2H), 7.21 - 7.11 (m, 4H), 6.92 (d, *J* = 8.8 Hz, 2H), 6.87 - 6.81 (m, 3H), 4.94 (s, 2H), 4.92 (s, 1H), 4.86 (s, 4H), 4.30 (s, 4H), 2.97 - 2.88 (m, 2H), 2.87 - 2.73 (m, 2H), 2.10 (s, 2H), 1.65 (s, 6H). LC/MS (ESI+) calcd for C₄₁H₃₇N₅O₇ ( [M+H]⁺ ) m/z 711.78; found 712.2.

### Example 311: 5-((1r,3r)-3-(4-(2-(4-((5-(2-oxa-6-azaspiro[3.3]heptan-6-yl) pyrimidin-2-yl)oxy)phenyl)propan-2-yl)phenoxy)cyclobutyl)amino)-2-(2,6-dioxopyridin-3-yl)isoindolin-1,3-dione

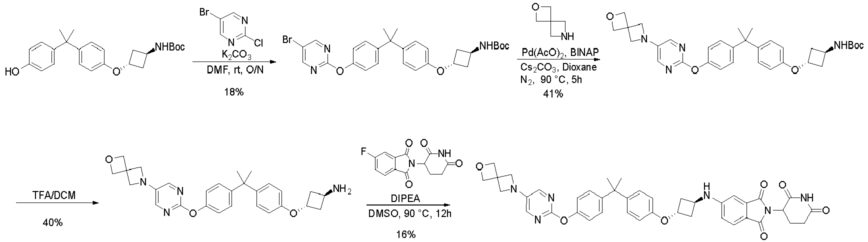

### Step 1: Synthesis of tert-butyl ((1r,3r)-3-(4-(2-(4-((5-bromopyrimidin-2-yl)oxy)phenyl) propan-2-yl)phenoxy)cyclobutyl)carbamate

tert-butyl ((1*r*,3*r*)-3-(4-(2-(4-hydroxylphenyl)propan-2-phenoxy)cyclobutyl) carbamate (2.02 g, 5.07 mmol) and 5-bromo-2-chloropyrimidine (1.08 g, 5.58 mmol) were dissolved in 20 mL of DMF, to which was added K₂CO₃ (2.1 g, 15.2 mmol), and then the mixture was stirred overnight. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-((5-bromopyrimidin-2-yl)oxy)phenyl)propan-2-yl)phenoxy)cyclobutyl)carbamate (501 mg, 0.90 mmol), with a yield of 18%. LC/MS (ESI+) calcd for C₂₈H₃₂BrN₃O₅ ( [M+H]⁺ ) *m*/*z* 554.49; found 498.0.

### Step 2: Synthesis of tert-butyl ((1r,3r)-3-(4-(2-(4-((5-(2-oxa-6-azaspiro[3.3]heptane-6-yl)pyrimidin-2-yl)oxy)phenyl)propan-2-yl)phenoxy)cyclobutyl)carbamate

tert-butyl ((1*r*,3*r*)-3-(4-(2-(4-((5-bromopyrimidin-2-yl)oxy)phenyl)propan-2-yl)phenoxy)cyclobutyl)carbamate (501 mg, 0.90 mmol), 2-oxa-6-azaspiro[3.3]heptane hemioxalate (261 mg, 0.90 mmol), palladium acetate (25 mg, 0.10 mmol), BINAP (113 mg, 0.18 mmol), and cesium carbonate (890 mg, 2.73 mmol) were successively added into 20 mL of 1,4-dioxane, and then the mixture was heated to 60 °C and reacted overnight. The reaction solution was cooled to room temperature, and extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-((5-(2-oxa-6-azaspiro[3.3]heptane-6-yl) pyrimidin-2-yl)oxy)phenyl)propan-2-yl)phenoxy)cyclobutyl)carbamate (210 mg, 0.37 mmol), with a yield of 41%. LC/MS (ESI+) calcd for C₃₃H₄₀N₄O₅ ( [M+H]⁺ ) *m*/*z* 572.71; found 517.2.

### Step 3: Synthesis of (1r,3r)-3-(4-(2-(4-((5-(2-oxa-6-azaspiro[3.3]heptan-6-yl) pyrimidin-2-yl)oxy)phenyl)propan-2-yl)phenoxy)cyclobutylamine

tert-butyl ((1*r*,3*r*)-3-(4-(2-(4-((5-(2-oxa-6-azaspiro[3.3]heptane-6-yl)pyrimidin-2-yl)oxy)phenyl)propan-2-yl)phenoxy)cyclobutyl)carbamate (210 mg, 0.37 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1*r*,3*r*)-3-(4-(2-(4-((5-(2-oxa-6-azaspiro[3.3]heptan-6-yl)pyrimidin-2-yl) oxy)phenyl)propan-2-yl)phenoxy)cyclobutylamine (70 mg, 0.15 mmol), with a yield of 40%.

### Step 4: Synthesis of 5-((1r,3r)-3-(4-(2-(4-((5-(2-oxa-6-azaspiro[3.3]heptan-6-yl)pyrimidin-2-yl)oxy)phenyl)propan-2-yl)phenoxy)cyclobutyl)amino)-2-(2,6-dioxopyridin-3-yl)isoindolin-1,3-dione

(1*r*,3*r*)-3-(4-(2-(-((5-(2-oxa-6-azaspiro[3.3]heptan-6-yl)pyrimidin-2-yl)oxy) phenyl)propan-2-yl)phenoxy)cyclobutylamine (70 mg, 0.15 mmol) was dissolved in 6 mL of DMSO, to which were successively added DIPEA (60 mg, 0.47 mmol) and 2-(2,6-dioxopiperidin-3-yl)-5-fluoroisoindolin-1,3-dione (45 mg, 0.16 mmol), and then the mixture was heated to 60 °C and reacted overnight under N₂ protection. The reaction solution was cooled to room temperature, and water was added to quench the reaction. The resultant solution was extracted with ethyl acetate for three times. The organic phase was washed with saturated NaCl solution, dried over anhydrous Na₂SO₄, concentrated and rotatory evaporated to dry. The residue was separated and purified by prep-TLC, to provide 5-((1*r*,3*r*)-3-(4-(2-(4-((5-(2-oxa-6-azaspiro[3.3]heptan-6-yl) pyrimidin-2-yl)oxy)phenyl)propan-2-yl)phenoxy)cyclobutyl)amino)-2-(2,6-dioxopyridin-3-yl)isoindolin-1,3-dione (18 mg, 0.02 mmol), with a yield of 16%. ¹H NMR (400 MHz, CDCl₃)) *δ* 8.11 (s, 1H), 7.63 (d, *J* = 8.3 Hz, 1H), 7.20 - 7.13 (m, 4H), 7.06 - 7.01 (m, 3H), 6.92 (d, *J* = 2.2 Hz, 1H), 6.76 - 6.67 (m, 3H), 4.93 (dd, *J* = 12.2, 5.3 Hz, 1H), 4.85 (s, 4H), 4.52 (t, *J* = 6.8 Hz, 1H), 4.26 (s, 4H), 3.77 (q, *J* = 7.6 Hz, 1H), 3.17 - 3.10 (m, 2H), 2.94 - 2.70 (m, 3H), 2.10 (m, 3H), 1.64 (s, 6H). LC/MS (ESI+) calcd for C₄₁H₄₀N₆O₇ ( [M+H]⁺ ) *m*/*z* 728.81; found 729.3.

### Example 312: 5-((S)-2-((4-(2-(4-((2-(2-oxa-6-azaspiro[3.3]heptan-6-yl)pyrimidin-4-yl)methoxy)phenyl)propan-2-yl)phenoxy)methyl)azetidin-1-yl)-2-(2,6-dioxopiperidin-3-yl)isoindolin-1,3-dione

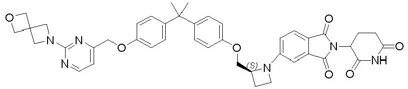

The target compound was synthesized by a method similar to that of Example 311. ¹H NMR (400 MHz, CDCl₃) *δ* 8.28 (s, 1H), 8.19 (d, *J* = 49.4 Hz, 1H), 7.64 (d, *J* = 8.3 Hz, 1H), 7.13 (dd, *J* = 9.0, 2.8 Hz, 5H), 6.93 (s, 1H), 6.88 - 6.70 (m, 5H), 5.00 (s, 2H), 4.93 (dd, *J* = 12.1, 5.3 Hz, 1H), 4.86 (s, 4H), 4.64 (s, 1H), 4.49 (s, 4H), 4.22 (d, *J* = 6.4 Hz, 2H), 4.18 (d, *J* = 7.9 Hz, 1H), 3.87 (q, *J* = 8.0 Hz, 1H), 2.93 - 2.71 (m, 3H), 2.39 (s, 1H), 2.16 - 2.09 (m, 1H), 1.63 (s, 6H). LC/MS (ESI+) calcd for C₄₂H₄₂N₆O₇ ( [M+H]⁺ ) *m*/*z* 742.83; found 743.3.

### Example 313: 5-((S)-2-((4-(2-(4-((2-(2-oxa-6-azaspiro[3.3]heptane-6-yl)pyrimidin-4-yl)methoxy)phenyl)propan-2-yl)phenoxy)methyl)pyrrolidin-1-yl)-2-(2,6-dioxopiperidin-3-yl)isoindolin-1,3-dione

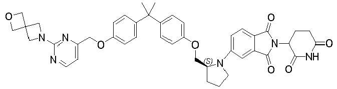

The target compound was synthesized by a method similar to that of Example 311. ¹H NMR (400 MHz, CDCl₃) *δ* 8.30 (s, 1H), 8.25 (s, 1H), 7.66 (d, *J* = 8.2 Hz, 1H), 7.12 (d, *J* = 8.4 Hz, 4H), 7.07 (d, *J* = 9.0 Hz, 1H), 6.93 (s, 1H), 6.81 (s, 3H), 6.76 (d, *J* = 8.8 Hz, 2H), 4.99 (s, 2H), 4.92 (s, 1H), 4.87 (s, 3H), 4.48 (s, 4H), 4.30 (s, 1H), 4.00 (s, 1H), 3.85 (s, 1H), 3.58 (d, *J* = 8.2 Hz, 1H), 3.34 (s, 1H), 2.82 (d, *J* = 40.5 Hz, 3H), 2.25 - 2.14 (m, 5H), 1.62 (s, 6H). LC/MS (ESI+) calcd for C₄₃H₄₄N₆O₇ ([M+H]⁺) *m*/*z* 756.86; found 757.3.

### Example 314: 5-((R)-2-((4-(2-(4-((2-(2-oxa-6-azaspiro[3.3]heptan-6-yl)pyrimidin-4-yl)methoxy)phenyl)propan-2-yl)phenoxy)methyl)pyrrolidin-1-yl)-2-(2,6-dioxopiperidin-3-yl)isoindolin-1,3-dione

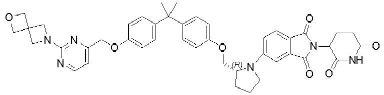

The target compound was synthesized by a method similar to that of Example 311. ¹H NMR (400 MHz, CDCl₃) *δ* 8.29 (s, 1H), 8.17 (d, *J* = 42.5 Hz, 1H), 7.66 (d, *J* = 8.4 Hz, 1H), 7.17 - 7.10 (m, 4H), 7.09 (s, 1H), 6.92 (s, 1H), 6.82 (d, *J* = 7.7 Hz, 3H), 6.76 (d, *J* = 8.5 Hz, 2H), 4.99 (s, 2H), 4.93 (dd, *J* = 12.2, 5.1 Hz, 1H), 4.87 (s, 4H), 4.48 (s, 4H), 4.29 (s, 1H), 4.01 (d, *J* = 9.5 Hz, 1H), 3.85 (s, 1H), 3.59 (s, 1H), 3.34 (s, 1H), 2.82 (dt, *J* = 44.9, 16.0 Hz, 3H), 2.25 - 2.11 (m, 5H), 1.62 (s, 6H). LC/MS (ESI+) calcd for C₄₃H₄₄N₆O₇ ( [M+H]⁺ ) *m*/*z* 756.86; found 757.3.

### Example 315: 5-((1R,2S)-2-(4-(2-(2-oxa-6-azaspiro[3.3]heptane-6-yl)pyrimidin-4-yl)methoxy)phenyl)propan-2-yl)phenoxy)cyclopentyl)amino)-2-(2,6-dioxopiperidin-3-yl)isoindolin-1,3-dione

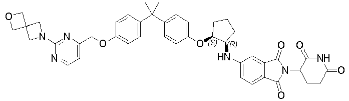

The target compound was synthesized by a method similar to that of Example 311. LC/MS (ESI+) calcd for C₄₃H₄₄N₆O₇ ( [M+H]⁺ ) *m*/*z* 756.86; found 757.3.

### Example 316: 2-(2,6-dioxopiperidin-3-yl)-5-((1s,3s)-3-(4-(2-(4-((5-iodopyrimidin-2-yl)oxy)phenyl)propan-2-yl)phenoxy)cyclobutyl)amino)isoindolin-1,3-dione

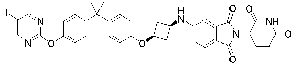

The target compound was synthesized by a method similar to that of Example 311. ¹H NMR (400 MHz, CDCl₃) *δ* 8.68 (s, 1H), 8.02 (s, 1H), 7.93 (d, *J* = 8.4 Hz, 1H), 7.63 (d, *J* = 8.2 Hz, 1H), 7.29 (d, *J* = 8.7 Hz, 2H), 7.17 (d, *J* = 8.7 Hz, 2H), 7.12 (d, *J* = 8.8 Hz, 1H), 7.08 - 7.05 (m, 2H), 6.92 (d, *J* = 2.1 Hz, 1H), 6.84 (d, *J* = 8.9 Hz, 1H), 6.75 - 6.72 (m, 2H), 4.57 - 4.47 (m, 1H), 3.17 - 3.09 (m, 2H), 2.93 - 2.72 (m, 3H), 2.12 (dq, *J* = 8.5, 4.6, 3.7 Hz, 3H), 2.00 (t, *J* = 6.2 Hz, 2H), 1.68 (s, 6H). LC/MS (ESI+) calcd for C₃₆H₃₂IN₅O₆ ( [M+H]⁺ ) *m*/*z* 757.59; found 758.1.

### Example 317: 5-((1s,3s)-3-(4-(2-(4-((6-(2-oxa-6-azaspiro[3.3]heptane-6-yl) pyridazine-3-yl)oxy)phenyl)propan-2-yl)phenoxy)cyclobutyl)amino)-2-(2,6-dioxopiperidin-3-yl)isoindolin-1,3-dione

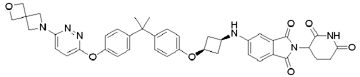

The target compound was synthesized by a method similar to that of Example 311. ¹H NMR (400 MHz, CDCl₃) *δ* 8.08 (s, 1H), 7.82 (s, 1H), 7.64 (d, *J* = 8.2 Hz, 1H), 7.23 (d, *J* = 8.7 Hz, 2H), 7.16 (d, *J* = 8.7 Hz, 2H), 7.07 - 7.01 (m, 2H), 6.90 (d, *J* = 2.1 Hz, 1H), 6.71 (dd, *J* = 8.5, 6.3 Hz, 3H), 4.93 (dd, *J* = 12.1, 5.2 Hz, 1H), 4.87 (s, 1H), 4.85 (s, 4H), 4.24 (s, 1H), 4.06 (s, 4H), 2.89 (d, *J* = 16.8 Hz, 1H), 2.85 - 2.79 (m, 1H), 2.79 - 2.64 (m, 3H), 2.41 (dd, *J* = 13.0, 6.4 Hz, 2H), 2.19 - 2.07 (m, 1H), 1.66 (s, 6H). LC/MS (ESI+) calcd for C₄₁H₄₀N₆O₇ ( [M+H]⁺ ) *m*/*z* 728.81; found 729.3.

### Example 318: 5-((4-(2-(4-((2-(2-oxa-6-azaspiro[3.3]heptan-6-yl)pyrimidin-4-yl) methoxy)phenyl)propan-2-yl)phenylethyl)amino)-2-(2,6-dioxopiperidin-3-yl) isoindolin-1,3-dione Synthesis route:

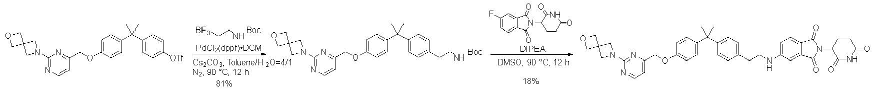

### Step 1: Synthesis of tert-butyl (4-(2-(4-((2-(2-oxa-6-azaspiro[3.3]heptan-6-yl)pyrimidin-4-yl)methoxy)phenyl)propan-2-yl)phenylethyl)carbamate

4-(2-(4-((2-(2-oxa-6-azaspiro[3.3]heptan-6-yl)pyrimidin-4-yl)methoxy)phenyl) propan-2-yl)phenyl trifluoromethanesulfonate (150 mg, 0.27 mmol), tert-butyl potassium N [2-(BF₃)ethyl]carbamate (117 mg, 0.41 mmol), 1,1'-[1,1'-bis(diphenylphosphino)ferrocene]palladium(II) dichloride dichloromethane complex (20 mg, 0.03 mmol), and cesium carbonate (270 mg, 0.82 mmol) were successively added into 5 mL mixed solution of toluene and water (4/1), and then the mixture was heated to 60 °C and reacted overnight under N₂ protection. The reaction solution was cooled to room temperature, and extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl (4-(2-(4-((2-(2-oxa-6-azaspiro[3.3]heptan-6-yl)pyrimidin-4-yl)methoxy)phenyl)propan-2-yl) phenylethyl)carbamate (119 mg, 0.22 mmol), with a yield of 81%. LC/MS (ESI+) calcd for C₃₂H₄₀N₄O₄ ( [M+H]⁺ ) *m*/*z* 544.70; found 545.3.

### Step 2: Synthesis of 5-((4-(2-(4-((2-(2-oxa-6-azaspiro[3.3]heptan-6-yl)pyrimidin-4-yl)methoxy)phenyl)propan-2-yl)phenylethyl)amino)-2-(2,6-dioxopiperidin-3-yl) isoindolin-1,3-dione

tert-butyl (4-(2-(4-((2-(2-oxa-6-azaspiro[3.3]heptan-6-yl)pyrimidin-4-yl)methoxy) phenyl)propan-2-yl)phenylethyl)carbamate (119 mg, 0.22 mmol) was dissolved in 6 mL of DMSO, to which were successively added DIPEA (86 mg, 0.67 mmol) and 2-(2,6-dioxopiperidin-3-yl)-5-fluoroisoindolin-1,3-dione (65 mg, 0.22 mmol), and then the mixture was heated to 60 °C and reacted overnight under N₂ protection. The reaction solution was cooled to room temperature, and water was added to quench the reaction. The resultant solution was extracted with ethyl acetate. The organic phase was washed with saturated brine, dried with anhydrous Na₂SO₄, concentrated and rotatory evaporated to dry. The residue was separated and purified by prep-TLC, to provide 5-((4-(2-(4-((2-(2-oxa-6-azaspiro[3.3]heptan-6-yl)pyrimidin-4-yl)methoxy)phenyl) propan-2-yl)phenylethyl)amino)-2-(2,6-dioxopiperidin-3-yl)isoindolin-1,3-dione (28 mg, 0.04 mmol), with a yield of 18%. ¹H NMR (400 MHz, CDCl₃) *δ* 8.31 (s, 1H), 8.06 (s, 1H), 7.62 (d, *J* = 8.2 Hz, 1H), 7.17 (t, *J* = 10.0 Hz, 4H), 7.10 (d, *J* = 7.8 Hz, 2H), 6.96 (s, 1H), 6.88 (s, 1H), 6.84 (d, *J* = 8.1 Hz, 2H), 6.73 (d, *J=* 8.2 Hz, 1H), 4.98 (s, 2H), 4.93 (dd, *J=* 11.8, 5.2 Hz, 1H), 4.87 (s, 4H), 4.40 (s, 4H), 3.50 (t, *J=* 6.7 Hz, 2H), 2.96 - 2.70 (m, 5H), 2.13 (s, 1H), 1.65 (s, 6H). LC/MS (ESI+) calcd for C₄₀H₄₀N₆O₆ ( [M+H]⁺ ) *m*/*z* 700.8; found 701.3.

### Example 319: 2-(2,6-dioxopiperidin-3-yl)-5-((1r,3r)-3-(4-(2-(4-((3-fluoro-6-(1H-1,2,3-triazol-1-yl)pyridin-2-yl)oxy)phenyl)propan-2-yl)phenoxy)cyclobutyl)amino) isoindolin-1,3-dione

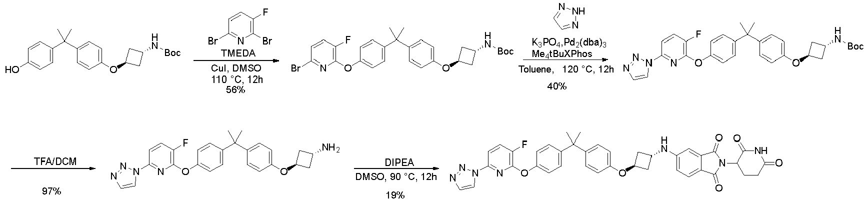

### Step 1: Synthesis of tert-butyl ((1r,3r)-3-(4-(2-(4-((6-bromo-3-fluoropyridin-2-yl)oxy)phenyl)propan-2-yl)phenoxy)cyclobutyl)carbamate

tert-butyl ((1*r*,3*r*)-3-(4-(2-(4-hydroxylphenyl)propan-2-phenoxy)cyclobutyl) carbamate (437 mg, 1.10 mmol) and 2,6-dibromo-3-fluoropyridine (280 mg, 1.10 mmol) were dissolved in 10 mL of DMSO, to which were successively added CuI (19 mg, 0.1 mmol) and N,N,N',N'-tetramethylethylenediamine (12 mg, 0.1 mmol), and then the mixture was heated to 110 °C and reacted overnight under N₂ protection. The reaction solution was cooled to room temperature, and water was added to quench the reaction. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-((6-bromo-3-fluoropyridin-2-yl)oxy)phenyl)propan-2-yl)phenoxy)cyclobutyl)carbamate (353 mg, 0.62 mmol), with a yield of 56%. LC/MS (ESI+) calcd for C₂₉H₃₂BrFN₂O₄ ( [M+H]⁺ ) *m*/*z* 571.49; found 471.1.

### Step 2: Synthesis of tert-butyl ((1r,3r)-3-(4-(2-(4-((3-fluoro-6-(1H-1,2,3-triazol-1-yl)pyridin-2-yl)oxy)phenyl)propan-2-yl)phenoxy)cyclobutyl)carbamate

tert-butyl ((1*r*,3*r*)-3-(4-(2-(4-((6-bromo-3-fluoropyridin-2-yl)oxy)phenyl)propan-2-yl)phenoxy)cyclobutyl)carbamate (300 mg, 0.52 mmol), 2H-1,2,3-triazole (44 mg, 0.63 mmol) and K₃PO₄ (223 mg, 1.05 mmol) were dissolved in 10 mL of toluene in a single-necked flask A, and then the reaction solution was stirred at room temperature under N₂ protection. In another single-necked flask B, Pd₂(dba)₃ (48 mg, 0.05 mmol) and L1 (63 mg, 0.13 mmol) were dissolved in 10 mL of toluene, and then the reaction solution was heated to 120 °C and stirred for 3 min under N₂ protection. The solution in flask B was transferred to flask A, and then the reaction solution was heated to 120 °C and stirred overnight under N₂ protection. The reaction solution was cooled to room temperature, and water was added to quench the reaction. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-((3-fluoro-6-(1*H*-1,2,3-triazol-1-yl)pyridin-2-yl)oxy)phenyl)propan-2-yl)phenoxy)cyclobutyl)carbamate (116 mg, 0.21 mmol), with a yield of 40%. LC/MS (ESI+) calcd for C₃₁H₃₄FN₅O₄ ( [M+H]⁺ ) *m*/*z* 559.64; found 504.1.

### Step 3: Synthesis of (1r,3r)-3-(4-(2-(4-((3-fluoro-6-(1H-1,2,3-triazol-1-yl) pyridin-2-yl)oxy)phenyl)propan-2-yl)phenoxy) cyclobutane-1-amine

tert-butyl ((1*r*,3*r*)-3 -(4-(2-(4-((3 -fluoro-6-(1*H*-1,2,3 -triazol-1-yl)pyridin-2-yl)oxy) phenyl)propan-2-yl)phenoxy)cyclobutyl)carbamate (116 mg, 0.21 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1*r*,3*r*)-3-(4-(2-(4-((3-fluoro-6-(1*H*-1,2,3-triazol-1-yl)pyridin-2-yl)oxy) phenyl) propan-2-yl)phenoxy) cyclobutane-1-amine (94 mg, 0.20 mmol), with a yield of 97%.

### Step 4: Synthesis of 2-(2,6-dioxopiperidin-3-yl)-5-((1r,3r)-3-(4-(2-(4-((3-fluoro-6-(1H-1,2,3-triazol-1-yl)pyridin-2-yl)oxy)phenyl)propan-2-yl)phenoxy)cyclobutyl) amino)isoindolin-1,3-dione

(1*r*,3*r*)-3-(4-(2-(4-((3-fluoro-6-(1*H*-1,2,3-triazol-1-yl)pyridin-2-yl)oxy)phenyl) propan-2-yl)phenoxy)cyclobutane-1-amine (94 mg, 0.20 mmol) was dissolved in 10 mL of DMSO, to which were successively added DIPEA(88 mg, 0.69 mmol) and 2-(2,6-dioxopiperidin-3-yl)-5-fluoroisoindolin-1,3-dione (69 mg, 0.25 mmol), and then the reaction solution was heated to 60 °C and reacted overnight under N₂ protection. The reaction solution was cooled to room temperature, and water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 2-(2,6-dioxopiperidin-3-yl)-5-((1*r*,3*r*)-3-(4-(2-(4-((3-fluoro-6-(1*H*-1,2,3-triazol-1-yl)pyridin-2-yl)oxy)phenyl)propan-2-yl)phenoxy)cyclobutyl) amino)isoindolin-1,3-dione (27 mg, 0.04 mmol), with a yield of 19%. ¹H NMR (400 MHz, CDCl₃) *δ* 8.13 (s, 1H), 7.91 (dd, *J* = 8.3, 4.4 Hz, 2H), 7.82 (s, 1H), 7.75 (dd, *J* = 8.5, 2.7 Hz, 1H), 7.69 - 7.62 (m, 1H), 7.57 (dd, *J* = 7.0, 2.3 Hz, 2H), 7.44 (td, *J* = 8.5, 2.3 Hz, 2H), 7.24 (s, 1H), 7.20 (s, 1H), 7.19 - 7.14 (m, 2H), 6.90 (d, *J* = 1.6 Hz, 1H), 6.70 (d, *J* = 8.4 Hz, 2H), 4.87 (s, 1H), 4.23 (s, 1H), 2.98 - 2.89 (m, 2H), 2.84 (dd, *J* = 12.6, 3.9 Hz, 2H), 2.77 - 2.71 (m, 2H), 2.46 - 2.36 (m, 1H), 2.22 - 2.13 (m, 2H), 1.67 (s, 6H). LC/MS (ESI+) calcd for C₃₉H₃₄FN₇O₆ ( [M+H]⁺ ) *m*/*z* 715.74; found 716.3.

### Example 320: 2-(2,6-dioxopiperidin-3-yl)-5-((1r,3r)-3-(4-(2-(4-((3-fluoro-6-(2H-1,2,3-triazol-2-yl)pyridin-2-yl)oxy)phenyl)propan-2-yl)phenoxy)cyclobutyl)amino) isoindolin-1,3-dione

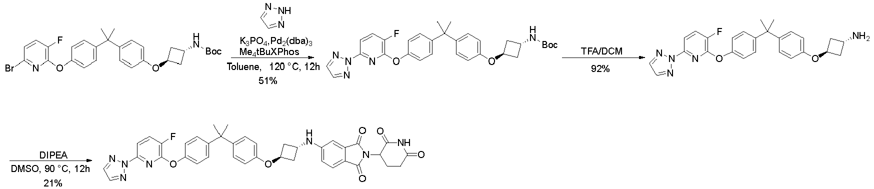

### Step 1: Synthesis of tert-butyl ((1r,3r)-3-(4-(2-(4-((3-fluoro-6-(2H,2,3-triazol-2-yl)pyridin-2-yl)oxy)phenyl)propan-2-yl)phenoxy)cyclobutyl)carbamate

tert-butyl ((1*r*,3*r*)-3-(4-(2-(4-((6-bromo-3-fluoropyridin-2-yl)oxy)phenyl)propan-2-yl)phenoxy)cyclobutyl)carbamate (300 mg, 0.52 mmol), 2*H*-1,2,3-triazole (44 mg, 0.63 mmol) and K₃PO₄ (223 mg, 1.05 mmol) was dissolved in 10 mL of toluene in a single-necked flask A, and then the reaction solution was stirred at room temperature under N₂ protection. In another single-necked flask B, Pd₂(dba)₃ (48 mg, 0.05 mmol) and L1 (63 mg, 0.13 mmol) were dissolved in 10 mL of toluene, and then the reaction solution was heated to 120 °C and stirred for 3 min under N₂ protection. The solution in flask B was transferred to flask A, and then the reaction solution was heated to 120 °C and stirred overnight under N₂ protection. The reaction solution was cooled to room temperature, and water was added to quench the reaction. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-((3-fluoro-6-(2*H*-1,2,3-triazol-2-yl)pyridin-2-yl)oxy)phenyl)propan-2-yl)phenoxy)cyclobutyl)carbamate (147 mg, 0.26 mmol), with a yield of 51%. LC/MS (ESI+) calcd for C₃₁R₃₄FN₅O₄( [M+H]⁺ ) *m*/*z* 559.64; found 504.1.

### Step 2: Synthesis of (1r,3r)-3-(4-(2-(4-((3-fluoro-6-(2H-1,2,3-triazol-2-yl) pyridin-2-yl)oxy)phenyl)propan-2-yl)phenoxy) cyclobutane-1-amine

tert-butyl ((1*r*,3*r*)-3-(4-(2-(4-((3-fluoro-6-(2*H*-1,2,3-triazol-2-yl)pyridin-2-yl)oxy) phenyl)propan-2-yl)phenoxy)cyclobutyl)carbamate (147 mg, 0.26 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1*r*,3*r*)-3-(4-(2-(4-((3-fluoro-6-(2*H*-1,2,3-triazol-2-yl)pyridin-2-yl)oxy) phenyl)propan-2-yl)phenoxy) cyclobutane-1-amine (109 mg, 0.24 mmol), with a yield of 92%.

### Step 3: Synthesis of target compound

(1*r*,3*r*)-3-(4-(2-(4-((3-fluoro-6-(2*H*-1,2,3-triazol-2-yl)pyridin-2-yl)oxy)phenyl) propan-2-yl)phenoxy)cyclobutane-1-amine (109 mg, 0.24 mmol) was dissolved in 10 mL of DMSO, to which were successively added DIPEA (108 mg, 0.84 mmol) and 2-(2,6-dioxopiperidin-3-yl)-5-fluoroisoindolin-1,3-dione (86 mg, 0.31 mmol), and then the reaction solution was heated to 60 °C and reacted overnight under N₂ protection. The reaction solution was cooled to room temperature, and water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 2-(2,6-dioxopiperidin-3-yl)-5-((1*r*,3*r*)-3-(4-(2-(4-((3-fluoro-6-(2*H*-1,2,3-triazol-2-yl)pyridin-2-yl)oxy)phenyl)propan-2-yl)phenoxy)cyclobutyl) amino)isoindolin-1,3-dione (36 mg, 0.05 mmol), with a yield of 21%. ¹H NMR (400 MHz, CDCl₃) *δ* 8.00 (d, *J* = 9.9 Hz, 2H), 7.91 (dd, *J* = 8.2, 4.4 Hz, 1H), 7.85 (d, *J* = 2.5 Hz, 1H), 7.72 (d, *J* = 5.6 Hz, 1H), 7.67-7.62 (m, 1H), 7.57 (dd, *J* = 6.9, 2.2 Hz, 1H), 7.44 (td, *J* = 8.5, 2.3 Hz, 1H), 7.29 (d, *J* = 8.7 Hz, 1H), 7.16 (d, *J* = 8.5 Hz, 2H), 7.11 (d, *J* = 8.6 Hz, 2H), 6.90 (s, 1H), 6.72 (d, *J* = 8.4 Hz, 2H), 4.98 (dd, *J* = 12.3, 5.4 Hz, 1H), 4.88 (s, 1H), 4.25 (s, 1H), 2.97 - 2.85 (m, 2H), 2.84 - 2.79 (m, 1H), 2.74 (d, *J* = 11.7 Hz, 3H), 2.41 (s, 1H), 2.21 - 2.13 (m, 1H), 1.70 (s, 6H). LC/MS (ESI+) calcd for C₃₉H₃₄FN₇O₆ ( [M+H]⁺ ) *m*/*z* 715.74; found 716.2.

### Example 321: 5-((1r,3r)-3-(4-(2-(4-((5-(2H-1,2,3-triazol-2-yl)pyrimidin-2-yl)oxy) phenyl)propan-2-yl)phenoxy)cyclobutyl)amino)-2-(2,6-dioxopiperidin-3-yl) isoindolin-1,3-dione

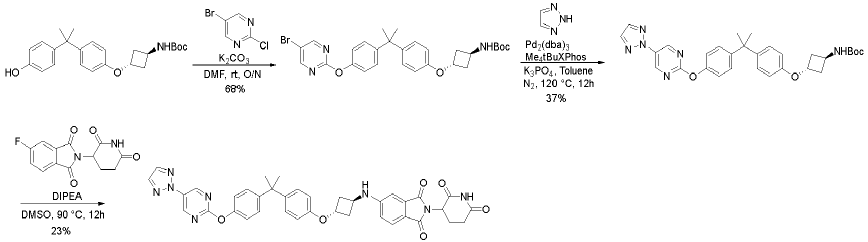

The target compound was synthesized by a method similar to that of Example 320. ¹H NMR (400 MHz, CDCl₃) *δ* 9.24 (s, 2H), 7.98 (s, 1H), 7.87 (s, 2H), 7.64 (d, *J* = 8.3 Hz, 1H), 7.33 - 7.28 (m, 2H), 7.21 - 7.16 (m, 2H), 7.16 - 7.11 (m, 2H), 6.90 (d, *J* = 2.2 Hz, 1H), 6.74 - 6.71 (m, 2H), 4.93 (dd, *J* = 12.2, 5.3 Hz, 1H), 4.90 - 4.82 (m, 1H), 4.24 (t, *J* = 6.3 Hz, 1H), 2.94 - 2.86 (m, 1H), 2.82 (dd, *J* = 12.6, 3.9 Hz, 1H), 2.73 (ddd, *J* = 17.4, 7.9, 4.1 Hz, 3H), 2.41 (dt, *J* = 13.0, 6.2 Hz, 2H), 2.13 (dd, *J* = 8.0, 5.3 Hz, 1H), 1.70 (s, 6H). LC/MS (ESI+) calcd for C₃₈H₃₄N₈O₆ ( [M+H]⁺ ) *m*/*z* 698.74; found 699.3.

### Example 322: 2-(2,6-dioxopiperidin-3-yl)-5-((1r,3r)-3-(4-(2-(4-(4-(5-methyl-1,3,4-oxadiazol-2-yl)phenoxy)phenyl)propan-2-yl)phenoxy)cyclobutyl) aminoisoindolin-1,3-dione

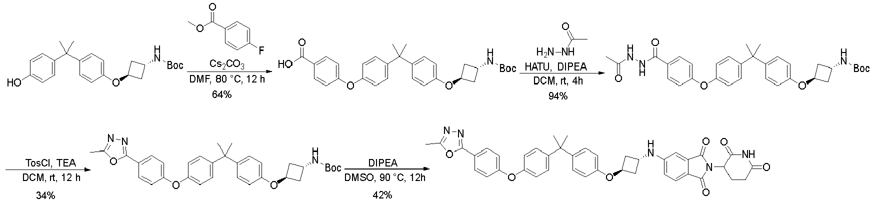

### Step 1: Synthesis of 4-(4-(2-(4-((1r,3r)-3-((tert-butoxycarbonyl)amino)cyclobutyloxy) phenyl)propan-2-yl)phenoxy)benzoic acid

tert-butyl ((1*r*,3*r*)-3-(4-(2-(4-hydroxylphenyl)propan-2-phenoxy)cyclobutyl) carbamate (720 mg, 1.81 mmol) and methyl 4-fluorobenzoate (308 mg, 2.0 mmol) were dissolved in 15 mL of DMF, to which was added Cs₂CO₃ (1.78 g, 5.43 mmol), and then the mixture was heated to 80 °C and reacted overnight. The reaction solution was cooled to room temperature, and water was added to quench the reaction. The resultant solution was adjusted to neutral with diluted hydrochloric acid (2 M), and then extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound 4-(4-(2-(4-((1*r*,3*r*)-3-((tert-butoxycarbonyl)amino) cyclobutyloxy)phenyl) propan-2-yl)phenoxy)benzoic acid (600 mg, 1.16 mmol), with a yield of 64%. LC/MS (ESI+) calcd for C₃₁H₃₅NO₆ ( [M+H]⁺ ) *m*/*z* 517.62; found 518.6.

### Step 2: Synthesis of tert-butyl ((1r,3r)-3-(4-(2-(4-(4-(2-acethydrazide-1-carbonyl) phenoxy)phenyl)propan-2-yl)phenoxy)cyclobutyl)carbamate

4-(4-(2-(4-((1*r*,3*r*)-3-((tert-butoxycarbonyl)amino)cyclobutyloxy)phenyl)propan-2-yl)phenoxy)benzoic acid (600 mg, 1.16 mmol) was dissolved in 10 mL of DCM, to which were added diisopropylethylamine (372 mg, 2.88 mmol) and HATU (655 mg, 1.73 mmol), and then the reaction solution was stirred at room temperature for 30 min, followed by addition of acethydrazide (172 mg, 2.30 mmol). The reaction solution was further stirred at room temperature for 4 h. The reaction was quenched by adding water, and the resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-(4-(2-acethydrazide-1-carbonyl)phenoxy)phenyl)propan-2-yl)phenoxy)cyclobutyl) carbamate (626 mg, 1.09mmol), with a yield of 94%. LC/MS (ESI+) calcd for C₃₃H₃₉N₃O₆ ( [M+H]⁺ ) *m*/*z* 573.69; found 474.2.

### Step 3: Synthesis of tert-butyl ((1r,3r)-3-(4-(2-(4-(4-(5-methyl-1,3,4-oxadiazol-2-yl)phenoxy)phenyl)propan-2-yl)phenoxy)cyclobutyl)carbamate

tert-butyl ((1*r*,3*r*)-3-(4-(2-(4-(4-(2-acethydrazide-1-carbonyl)phenoxy)phenyl) propan-2-yl)phenoxy)cyclobutyl)carbamate (626 mg, 1.09 mmol) and triethylamine (165 mg, 1.63 mmol) were dissolved in 15 mL of dichloromethane, to which was added p-toluenesulfonyl chloride (252 mg, 1.32 mmol), and then the mixture was stirred overnight. The reaction was quenched by adding water. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-(4-(5-methyl-1,3,4-oxadiazol-2-yl)phenoxy)phenyl)propan-2-yl)phenoxy)cyclobutyl)carbamate (206 mg, 0.37 mmol), with a yield of 34%. LC/MS (ESI+) calcd for C₃₃H₃₇N₃O₅ ( [M+H]⁺ ) *m*/*z* 555.68; found 500.2.

### Step 4: Synthesis of (1r,3r)-3-(4-(2-(4-(4-(5-methyl-1,3,4-oxadiazol-2-yl)phenoxy) phenyl)propan-2-yl)phenoxy)cyclobutyl-1-amine

tert-butyl ((1*r*,3*r*)-3-(4-(2-(4-(4-(5-methyl-1,3,4-oxadiazol-2-yl)phenoxy)phenyl) propan-2-yl)phenoxy)cyclobutyl)carbamate (206 mg, 0.37 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1*r*,3*r*)-3-(4-(2-(4-(4-(5-methyl-1,3,4-oxadiazol-2-yl)phenoxy)phenyl) propan-2-yl)phenoxy)cyclobutyl-1-amine (110 mg, 0.24 mmol), with a yield of 65%.

### Step 5: Synthesis of 2-(2,6-dioxopiperidin-3-yl)-5-((1r,3r)-3-(4-(2-(4-(4-(5-methyl-1,3,4-oxadiazol-2-yl)phenoxy)phenyl)propan-2-yl)phenoxy)cyclobutyl) aminoisoindolin-1,3-dione

(1*r*,3*r*)-3-(4-(2-(4-(4-(5-methyl-1,3,4-oxadiazol-2-yl)phenoxy)phenyl)propan-2-yl)phenoxy)cyclobutyl-1-amine (110 mg, 0.24 mmol) was dissolved in 10 mL of DMSO, to which were successively added DIPEA (92 mg, 0.72 mmol) and 2-(2,6-dioxopiperidin-3-yl)-5-fluoroisoindolin-1,3-dione (73 mg, 0.26 mmol), and then the reaction solution was heated to 60 °C and reacted overnight under N₂ protection. The reaction solution was cooled to room temperature, and water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 2-(2,6-dioxopiperidin-3-yl)-5-((1*r*,3*r*)-3-(4-(2-(4-(4-(5-methyl-1,3,4-oxadiazol-2-yl)phenoxy)phenyl)propan-2-yl)phenoxy)cyclobutyl) aminoisoindolin-1,3-dione (72 mg, 0.1 mmol), with a yield of 42%. ¹H NMR (400 MHz, CDCl₃) *δ* 8.06 (s, 1H), 7.97 (d, *J* = 7.7 Hz, 2H), 7.88 (d, *J* = 8.3 Hz, 1H), 7.64 (d, *J* = 7.3 Hz, 1H), 7.23 (s, 2H), 7.16 (d, *J* = 7.4 Hz, 2H), 7.06 (s, 2H), 6.97 (d, *J* = 8.2 Hz, 2H), 6.72 (d, *J* = 7.8 Hz, 3H), 4.91 (s, 2H), 4.25 (s, 1H), 2.93 - 2.71 (m, 6H), 2.61 (s, 3H), 2.43 (s, 1H), 2.16 - 2.08 (m, 1H),1.68 (s, 6H). LC/MS (ESI+) calcd for C₄₁H₃₇N₅O₇ ( [M+H]⁺ ) *m*/*z* 711.78; found 712.3.

### Example 323: 2-(2,6-dioxopiperidin-3-yl)-5-(1r,3r)-3-(4-(2-(4-(6-(5-methyl-1,3,4-oxadiazol-2-yl)pyridin-3-yl)oxy)phenyl)propan-2-yl)phenoxy)cyclobutyl)amino) isoindolin-1,3-dione

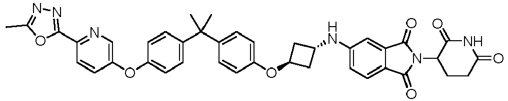

The target compound was synthesized by a method similar to that of Example 322. ¹H NMR (400 MHz, CDCl₃) *δ* 8.48 (s, 1H), 8.17 (d, *J* = 8.7 Hz, 1H), 8.03 (s, 1H), 7.64 (d, *J* = 8.2 Hz, 1H), 7.37 (dd, *J* = 8.8, 2.7 Hz, 1H), 7.29 (d, *J* = 2.2 Hz, 1H), 7.15 (d, *J* = 8.6 Hz, 2H), 7.02 - 6.96 (m, 2H), 6.90 (d, *J* = 2.1 Hz, 1H), 6.77 - 6.67 (m, 3H), 4.93 (dd, *J* = 12.2, 5.3 Hz, 1H), 4.88 (s, 1H), 4.24 (s, 1H), 2.89 (d, *J* = 16.7 Hz, 1H), 2.85 - 2.79 (m, 1H), 2.74 (dd, *J* = 20.5, 8.2 Hz, 3H), 2.65 (s, 3H), 2.47 - 2.37 (m, 2H), 2.13 (dd, *J* = 8.5, 5.9 Hz, 1H), 1.68 (s, 6H). LC/MS (ESI+) calcd for C₄₀H₃₆N₆O₇ ( [M+H]⁺ ) m/z 712.76; found 713.3.

### Example 324: 5-((1r,3r)-3-(4-(2-(4-((6-(2H-1,2,3-triazol-2-yl)pyridazine-3-yl)oxy) phenyl)propan-2-yl)phenoxy)cyclobutyl)amino)-2-(2,6-dioxopiperidin-3-yl) isoindolin-1,3-dione

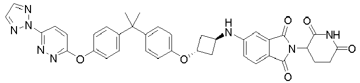

The target compound was synthesized by a method similar to that of Example 320. ¹H NMR (400 MHz, CDCl₃) *δ* 8.26 (d, *J* = 9.3 Hz, 1H), 7.99 (s, 1H), 7.95 (s, 2H), 7.64 (d, *J* = 7.8 Hz, 1H), 7.35 (d, *J* = 9.3 Hz, 1H), 7.29 (s, 1H), 7.15 (t, *J* = 8.7 Hz, 4H), 6.91 (s, 1H), 6.72 (d, *J* = 8.2 Hz, 3H), 4.93 (dd, *J* = 12.1, 5.2 Hz, 1H), 4.88 (s, 1H), 4.24 (s, 1H), 2.89 (d, *J* = 17.0 Hz, 1H), 2.85-2.78 (m, 1H), 2.74 (d, *J* = 17.8 Hz, 3H), 2.42 (s, 2H), 2.17 - 2.10 (m, 1H), 1.68 (s, 6H). LC/MS (ESI+) calcd for C₃₈H₃₄N₈O₆ ( [M+H]⁺ ) *m*/*z* 698.74; found 699.2.

### Example 325: 5-((1r,3r)-3-(4-(2-(4-((6-(1H-1,2,3-triazol-1-yl)pyridazine-3-yl)oxy) phenyl)propan-2-yl)phenoxy)cyclobutyl)amino)-2-(2,6-dioxopiperidin-3-yl) isoindolin-1,3-dione

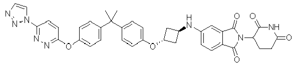

The target compound was synthesized by a method similar to that of Example 319. ¹H NMR (400 MHz, CDCl₃) *δ* 8.68 (d, *J* = 1.2 Hz, 1H), 8.45 (d, *J* = 9.4 Hz, 1H), 7.99 (s, 1H), 7.87 (d, *J* = 1.2 Hz, 1H), 7.64 (d, *J* = 8.2 Hz, 1H), 7.44 (d, *J* = 9.4 Hz, 1H), 7.33 - 7.28 (m, 2H), 7.20 - 7.11 (m, 4H), 6.90 (d, *J* = 2.1 Hz, 1H), 6.73 - 6.70 (m, 2H), 4.93 (dd, *J* = 12.2, 5.3 Hz, 1H), 4.88 (t, *J* = 6.8 Hz, 1H), 4.25 (q, *J* = 6.6, 5.6 Hz, 1H), 2.89 (d, *J* = 19.8 Hz, 1H), 2.86 - 2.79 (m, 1H), 2.78 - 2.69 (m, 3H), 2.41 (dt, *J* = 12.8, 6.2 Hz, 2H), 2.13 (dd, *J* = 8.0, 5.4 Hz, 1H), 1.69 (s, 6H). LC/MS (ESI+) calcd for C₃₈H₃₄N₈O₆ ( [M+H]⁺ ) *m*/*z* 698.74; found 699.3.

### Example 326: 2-(2,6-dioxopiperidin-3-yl)-5-((S)-2-((4-(2-(4-((2-(5-methyl-1,2,4-oxadiazol-3-yl)pyrimidin-5-yl)oxy)phenyl)propan-2-yl)phenoxy)methyl)azetidin-1-yl)isoindolin-1,3-dione

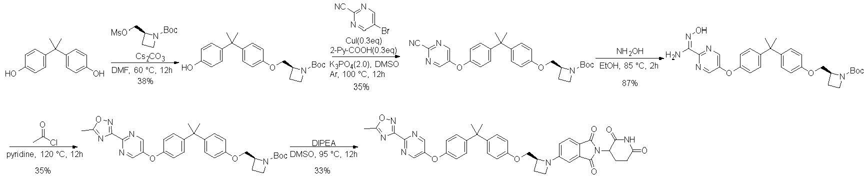

### Step 1: Synthesis of tert-butyl (S)-2-((4-(2-(4-hydroxylphenyl)propan-2-yl)phenoxy) methyl)azetidine-1-carboxylate

Bisphenol A (914 mg, 4.0 mmol) and tert-butyl (*S*)-2-((methanesulfonyl)oxy) methyl)azetidin-1-carboxylate (709 mg, 2.7 mmol) were dissolved in 15 mL of DMF, to which was added Cs₂CO₃ (1.74 g, 5.3 mmol), and then the mixture was heated to 60 °C and reacted overnight. The reaction solution was quenched by adding water. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl (*S*)-2-((4-(2-(4-hydroxylphenyl)propan-2-yl)phenoxy)methyl)azetidine-1-carboxylate (600 mg, 1.51 mmol), with a yield of 38%. LC/MS (ESI+) calcd for C₂₄H₃₁NO₃ ( [M+H]⁺ ) *m*/*z* 397.52; found 342.1.

### Step 2: Synthesis of (S)-2-((4-(2-(4-((2-cyanopyrimidin-5-yl)oxy)phenyl)propan-2-yl)phenoxy)methyl)azetidin-1 -carboxylate

tert-butyl (*S*)-2-((4-(2-(4-hydroxylphenyl)propan-2-yl)phenoxy)methyl) azetidine-1-carboxylate (600 mg, 1.51 mmol) and 5-bromopyrimidin-2-carbonitrile (417 mg, 3.26 mmol) were dissolved in 15 mL of DMSO, to which were successively added K₃PO₄ (641 mg, 3.02 mmol), 2-picolinic acid (56 mg, 0.45 mmol) and CuI (87 mg, 0.45 mmol), and then the reaction solution was heated to 100 °C and reacted overnight under N₂ protection. The reaction solution was cooled to room temperature, and water was added to quench the reaction. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound (*S*)-2-((4-(2-(4-((2-cyanopyrimidin-5-yl)oxy)phenyl)propan-2-yl)phenoxy) methyl)azetidin-1-carboxylate (263 mg, 0.53 mmol), with a yield of 35%. LC/MS (ESI+) calcd for C₂₉H₃₂N₄O₄ ( [M+H]⁺ ) *m*/*z* 500.60; found 401.1.

### Step 3: Synthesis of (S,E)-2-((4-(2-(4-((2-(N'-hydroxylcarbamoyl)pyrimidin-5-yl)oxy)phenyl)propan-2-yl)phenoxy)methyl)azetidin-1-carboxylate

(*S*)-2-((4-(2-(4-((2-cyanopyrimidin-5-yl)oxy)phenyl)propan-2-yl)phenoxy) methyl)azetidin-1-carboxylate (263 mg, 0.53 mmol) was dissolved in 10 mL of ethanol, to which was then added 50% hydroxylamine aqueous solution (70 mg, 1.05 mmol), and then the solution was heated to 85 °C and reacted for 2h. The reaction solution was cooled to room temperature, and concentrated under reduced pressure, to provide compound (*S*,*E*)-2-((4-(2-(4-((2-(*N*'-hydroxylcarbamoyl)pyrimidin-5-yl)oxy)phenyl) propan-2-yl)phenoxy)methyl)azetidin-1-carboxylate (246 mg, 0.46 mmol), with a yield of 87%. LC/MS (ESI+) calcd for C₂₉H₃₅N₅O₅ ( [M+H]⁺ ) *m*/*z* 533.63; found 534.2.

### Step 4: Synthesis of tert-butyl (S)-2-((4-(2-(4-((2-(5-methyl-1,2,4-oxadiazol-3-yl)pyrimidin-5-yl)oxy)phenyl)propan-2-yl)phenoxy)methyl)azetidine-1-carboxylate

(*S*,*E*)-2-((4-(2-(4-((2-(*N*'-hydroxylcarbamoyl)pyrimidin-5-yl)oxy)phenyl)propan-2-yl)phenoxy)methyl)azetidin-1-carboxylate (246 mg, 0.46 mmol) was dissolved in 5 mL of pyridine, to which was added acetyl chloride (144 mg, 1.84 mmol) in an ice bath, and then the solution was heated to 120 °C and refluxed overnight. The reaction solution was cooled to room temperature, and pyridine was removed by concentration under reduced pressure. The residue was adjusted to alkaline with NaHCO₃ solution, and extracted with ethyl acetate. The organic phase was washed with saturated brine, dried with anhydrous Na₂SO₄, and concentrated. The residue was separated and purified by prep-TLC, to provide compound tert-butyl (*S*)-2-((4-(2-(4-((2-(5-methyl-1,2,4-oxadiazol-3-yl)pyrimidin-5-yl)oxy)phenyl)propan-2-yl)phenoxy) methyl)azetidine-1-carboxylate (91 mg, 0.16 mmol), with a yield of 35%. LC/MS (ESI+) calcd for C₃₁H₃₅N₅O₅ ( [M+H]⁺ ) *m*/*z* 557.65; found 458.1.

### Step 5: Synthesis of (S)-3-(5-(4-(2-(4-(azetidin-2-ylmethoxy)phenyl)propan-2-yl) phenoxy)pyrimidin-2-yl)-5-methyl-1,2,4-oxadiazole

tert-butyl (*S*)-2-((4-(2-(4-((2-(5-methyl-1,2,4-oxadiazol-3-yl)pyrimidin-5-yl)oxy) phenyl)propan-2-yl)phenoxy)methyl)azetidine-1-carboxylate (91 mg, 0.16 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (*S*)-3-(5-(4-(2-(4-(azetidin-2-ylmethoxy)phenyl)propan-2-yl)phenoxy) pyrimidin-2-yl)-5-methyl-1,2,4-oxadiazole (68 mg, 0.15 mmol), with a yield of 94%.

### Step 6: Synthesis of 2-(2,6-dioxopiperidin-3-yl)-5-((S)-2-((4-(2-(4-((2-(5-methyl-1,2,4-oxadiazol-3-yl)pyrimidin-5-yl)oxy)phenyl)propan-2-yl)phenoxy)methyl) azetidin-1-yl)isoindolin-1,3-dione

(*S*)-3-(5-(4-(2-(4-(azetidin-2-ylmethoxy)phenyl)propan-2-yl)phenoxy)pyrimidin-2-yl)-5-methyl-1,2,4-oxadiazole (68 mg, 0.15 mmol) was dissolved in 10 mL of DMSO, to which were successively added DIPEA (75 mg, 0.59 mmol) and 2-(2,6-dioxopiperidin-3-yl)-5-fluoroisoindolin-1,3-dione (60 mg, 0.22 mmol), and then the reaction solution was heated to 60 °C and reacted overnight under N₂ protection. The reaction solution was cooled to room temperature, and water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 2-(2,6-dioxopiperidin-3-yl)-5-((*S*)-2-((4-(2-(4-((2-(5-methyl-1,2,4-oxadiazol-3-yl)pyrimidin-5-yl)oxy)phenyl)propan-2-yl)phenoxy)methyl) azetidin-1-yl)isoindolin-1,3-dione (36 mg, 0.05 mmol), with a yield of 33%. ¹H NMR (400 MHz, CDCl₃) *δ* 8.60 (s, 2H), 8.08 (d, *J* = 18.0 Hz, 1H), 7.65 (d, *J* = 8.3 Hz, 1H), 7.29 (s, 1H), 7.16 (d, *J* = 8.3 Hz, 3H), 7.00 (d, *J* = 8.5 Hz, 2H), 6.88 (d, *J* = 8.6 Hz, 2H), 6.78 (d, *J* = 8.7 Hz, 1H), 4.93 (dd, *J* = 12.0, 5.3 Hz, 1H), 4.65 (s, 1H), 4.24 (dd, *J* = 4.9, 2.8 Hz, 2H), 4.18 (dd, *J* = 13.0, 4.7 Hz, 1H), 3.88 (q, *J* = 8.0 Hz, 1H), 2.93 - 2.76 (m, 2H), 2.72 (s, 3H), 2.60 (d, *J* = 7.3 Hz, 1H), 2.45 - 2.35 (m, 1H), 2.17 - 2.08 (m, 1H), 1.69 (s, 6H). LC/MS (ESI+) calcd for C₃₉H₃₅N₇O₇ ( [M+H]⁺) *m*/*z* 713.75; found 714.1.

### Example 327: 2-(2,6-dioxopiperidin-3-yl)-5-(1r,3r)-3-(4-(2-(4-(5-(5-methyl-1,2,4-oxadiazol-3-yl)pyrimidin-2-yl)oxy)phenyl)propan-2-yl)phenoxy)cyclobutyl) amino)isoindolin-1,3-dione

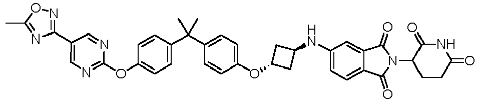

The target compound was synthesized by a method similar to that of Example 326. ¹H NMR (400 MHz, CDCl₃) *δ* 9.18 (s, 1H), 7.97 (s, 1H), 7.64 (d, *J* = 8.1 Hz, 1H), 7.30 (d, *J* = 8.6 Hz, 1H), 7.18 (d, *J* = 8.6 Hz, 1H), 7.16 - 7.07 (m, 3H), 6.90 (s, 1H), 6.76 - 6.66 (m, 4H), 4.93 (dd, *J* = 12.1, 5.2 Hz, 1H), 4.87 (s, 1H), 4.24 (s, 1H), 2.90 (d, *J* = 16.7 Hz, 1H), 2.85 - 2.79 (m, 1H), 2.78 - 2.69 (m, 3H), 2.68 (s, 3H), 2.40 (d, *J* = 12.4 Hz, 2H), 2.16 - 2.11 (m, 1H), 1.69 (s, 6H). LC/MS (ESI+) calcd for C₃₉H₃₅N₇O₇ ( [M+H]⁺ ) *m*/*z* 713.75; found 714.3.

### Example 328: 2-(2,6-dioxopiperidin-3-yl)-5-((1r,3r)-3-(4-(2-(4-((6-(5-methyl-1,2,4-oxadiazol-3-yl)pyridazine-3-yl)oxy)phenyl)propan-2-yl)phenoxy)cyclobutyl) amino)isoindolin-1,3-dione

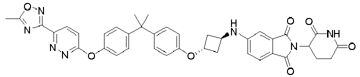

The target compound was synthesized by a method similar to that of Example 326. ¹H NMR (400 MHz, CDCl₃) *δ* 8.18 (d, *J* = 8.5 Hz, 1H), 7.98 (s, 1H), 7.76 (s, 1H), 7.64 (s, 1H), 7.15 (s, 4H), 6.90 (s, 1H), 6.72 (d, *J* = 7.8 Hz, 3H), 4.91 (s, 3H), 4.26 (s, 1H), 2.89 (d, *J* = 16.7 Hz, 2H), 2.78 (d, *J* = 14.6 Hz, 3H), 2.72 (s, 3H), 2.45 (s, 2H), 2.13 (s, 1H), 1.68 (s, 6H). LC/MS (ESI+) calcd for C₃₉H₃₅N₇O₇ ( [M+H]⁺ ) *m*/*z* 713.75; found 714.3.

### Example 329: 5-((1s,3s)-3-(4-(2-(4-(5-(2H-1,2,3-triazol-2-yl)pyrimidin-2-yl)oxy) phenyl)propan-2-yl)phenoxy)cyclobutyl)amino)-2-(2,6-dioxopiperidin-3-yl) isoindolin-1,3-dione

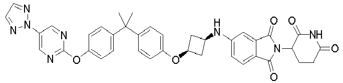

The target compound was synthesized by a method similar to that of Example 320. ¹H NMR (400 MHz, CDCl₃) *δ* 9.24 (s, 1H), 8.01 (s, 1H), 7.87 (s, 1H), 7.63 (d, *J* = 8.0 Hz, 1H), 7.29 (d, *J* = 8.6 Hz, 3H), 7.21 - 7.16 (m, 2H), 7.16 -7.10 (m, 3H), 6.92 (d, *J* = 6.8 Hz, 1H), 6.74 (t, *J* = 7.2 Hz, 4H), 4.93 (dd, *J* = 12.1, 5.2 Hz, 1H), 4.60 - 4.47 (m, 1H), 3.79 (s, 1H), 3.14 (s, 2H), 2.91-2.72 (m, 4H), 2.11 (s, 2H), 1.69 (s, 6H). LC/MS (ESI+) calcd for C₃₈H₃₄N₈O₆ ( [M+H]⁺ ) m/z 698.74; found 699.3.

### Example 330: 2-(2,6-dioxopiperidin-3-yl)-5-(1r,3r)-3-(4-(2-(4-(6-(5-methyl-1,3,4-oxadiazol-2-yl)pyridazine-3-yl)oxy)phenyl)propan-2-yl)phenoxy)cyclobutyl) amino)isoindolin-1,3-dione

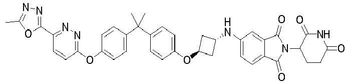

The target compound was synthesized by a method similar to that of Example 322. ¹H NMR (400 MHz, CDCl₃) *δ* 8.34 (d, *J* = 9.2 Hz, 1H), 8.01 (s, 1H), 7.64 (d, *J* = 8.3 Hz, 1H), 7.30 (dd, *J* = 8.9, 3.4 Hz, 3H), 7.15 (dd, *J* = 13.7, 8.7 Hz, 4H), 6.91 (d, *J* = 2.1 Hz, 1H), 6.72 (d, *J* = 8.6 Hz, 3H), 4.98 - 4.84 (m, 2H), 4.24 (s, 1H), 2.89 (d, *J* = 17.0 Hz, 1H), 2.85 - 2.70 (m, 4H), 2.67 (s, 3H), 2.46 - 2.37 (m, 2H), 2.13 (dd, *J* = 8.0, 5.3 Hz, 1H), 1.69 (s, 6H). LC/MS (ESI+) calcd for C₃₉H₃₅N₇O₇ ( [M+H]⁺ ) *m*/*z* 713.75; found 714.3.

### Example 331: 2-(2,6-dioxopiperidin-3-yl)-5-(1r,3r)-3-(4-(2-(4-(6-(3-methyl-1,2,4-oxadiazol-5-yl)pyridazine-3-yl)oxy)phenyl)propan-2-yl)phenoxy)cyclobutyl) amino)isoindolin-1,3-dione

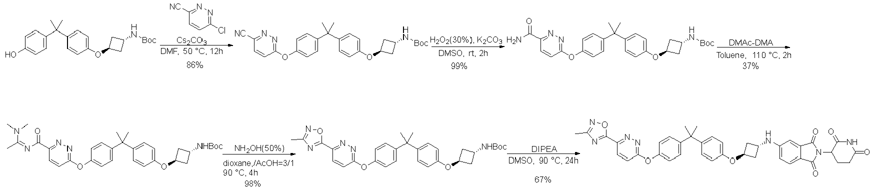

### Step 1: Synthesis of tert-butyl ((1r,3r)-3-(4-(2-(4-((6-carbamoyl pyridazine-3-yl)oxy)phenyl)propan-2-yl)phenoxy)cyclobutyl)carbamate

tert-butyl ((1*r*,3*r*)-3-(4-(2-(4-((6-cyanopyridin-3-yl)oxy)phenyl)propan-2-yl) phenoxy)cyclobutyl)carbamate (618 mg, 1.23 mmol) and K₂CO₃ (340 mg, 2.46 mmol) were dissolved in 6 mL of DMSO, to which was added 30% H₂O₂ (0.6 mL, 4.92 mmol) dropwise in an ice bath, and then the solution was naturally warmed and reacted under stirring for 1 h. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-((6-carbamoylpyridazine-3-yl)oxy)phenyl)propan-2-yl)phenoxy)cyclobutyl) carbamate (550 mg, 1.06 mmol), with a yield of 86%. LC/MS (ESI+) calcd for C₂₉H₃₄N₄O₅ ( [M+H]⁺ ) *m*/*z* 518.61; found 419.2.

### Step 2: Synthesis of tert-butyl ((1r,3r)-3-(4-(2-(4-((6-((dimethylamino)ethylene) carbamoyl)pyridazine-3-yl)oxy)phenyl)propan-2-yl)phenoxy)cyclobutyl)carbamate

tert-butyl ((1*r*,3*r*)-3-(4-(2-(4-((6-carbamoyl pyridazine-3-yl)oxy)phenyl)propan-2-yl)phenoxy)cyclobutyl)carbamate (550 mg, 1.06 mmol) was dissolved in 10 mL of toluene, to which was added DMAc-DMA (425 mg, 3.18 mmol), and then the solution was heated to 110 °C and reacted for 2h. The reaction solution was cooled to room temperature, and then water was added to quench the reaction. The resultant solution was extracted with ethyl acetate. The water layer was re-extracted once with ethyl acetate. The organic layers were combined, washed with saturated brine, dried with anhydrous Na₂SO₄, and concentrated, to provide compound tert-butyl ((1*r*,3*r*)-3-(4-(2-(4-((6-((dimethylamino)ethylene)carbamoyl)pyridazine-3-yl)oxy)phenyl)propan-2-yl)phenoxy)cyclobutyl)carbamate (600 mg, 1.05 mmol), with a yield of 99%.

### Step 3: Synthesis of tert-butyl ((1r,3r)-3-(4-(2-(4-((6-(3-methyl-1,2,4-oxadiazol-5-yl)pyridazine-3-yl)oxy)phenyl)propan-2-yl)phenoxy)cyclobutyl)carbamate

tert-butyl ((1*r*,3*r*)-3-(4-(2-(4-((6-((dimethylamino)ethylene)carbamoyl) pyridazine-3-yl)oxy)phenyl)propan-2-yl)phenoxy)cyclobutyl)carbamate (600 mg, 1.05 mmol) was dissolved in 4 mL mixed solution of 1,4-dioxane and glacial acetic acid (3/1), to which was added 50% hydroxylamine aqueous solution (105 mg, 1.58 mmol), and then the solution was heated to 90 °C and reacted for 4h. The reaction solution was cooled to room temperature, and then water was added to quench the reaction. The resultant solution was extracted with ethyl acetate. The water layer was re-extracted once with ethyl acetate. The organic layers were combined, washed with saturated brine, dried with anhydrous Na₂SO₄, and concentrated, to provide compound tert-butyl ((1*r*,3*r*)-3-(4-(2-(4-((6-(3-methyl-1,2,4-oxadiazol-5-yl)pyridazine-3-yl)oxy)phenyl) propan-2-yl)phenoxy)cyclobutyl)carbamate (215 mg, 0.39 mmol), with a yield of 37%. LC/MS (ESI+) calcd for C₃₁H₃₅N₅O₅ ( [M+H]⁺ ) *m*/*z* 557.65; found 458.2.

### Step 4: Synthesis of (1r,3r)-3-(4-(2-(4-((6-(3-methyl-1,2,4-oxadiazol-5-yl)pyridazine-3-yl)oxy)phenyl)propan-2-yl)phenoxy)cyclobutyl-1-amine

tert-butyl ((1*r*,3*r*)-3-(4-(2-(4-((6-(3-methyl-1,2,4-oxadiazol-5-yl)pyridazine-3-yl) oxy)phenyl)propan-2-yl)phenoxy)cyclobutyl)carbamate (215 mg, 0.39 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1*r*,3*r*)-3-(4-(2-(4-((6-(3-methyl-1,2,4-oxadiazol-5-yl)pyridazine-3-yl)oxy) phenyl)propan-2-yl)phenoxy)cyclobutyl-1-amine (175 mg, 0.24 mmol), with a yield of 98%. LC/MS (ESI+) calcd for C₂₆H₂₇N₅O₃ ( [M+H]⁺ ) *m*/*z* 457.53; found 458.2.

### Step 5: Synthesis of 2-(2,6-dioxopiperidin-3-yl)-5-(1r,3r)-3-(4-(2-(4-(6-(3-methyl-1,2,4-oxadiazol-5-yl)pyridazine-3-yl)oxy)phenyl)propan-2-yl)phenoxy)cyclobutyl) amino)isoindolin-1,3-dione

(1*r*,3*r*)-3-(4-(2-(4-((6-(3-methyl-1,2,4-oxadiazol-5-yl)pyridazine-3-yl)oxy)phenyl) propan-2-yl)phenoxy)cyclobutyl-1-amine (175 mg, 0.24 mmol) was dissolved in 6 mL of DMSO, to which were successively added DIPEA (121 mg, 0.94 mmol) and 2-(2,6-dioxopiperidin-3-yl)-5-fluoroisoindolin-1,3-dione (97 mg, 0.35 mmol), and then the reaction solution was heated to 60 °C and reacted overnight under N₂ protection. The reaction solution was cooled to room temperature, and water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 2-(2,6-dioxopiperidin-3-yl)-5-(1*r*,3*r*)-3-(4-(2-(4-(6-(3-methyl-1,2,4-oxadiazol-5-yl)pyridazine-3-yl)oxy)phenyl)propan-2-yl)phenoxy)cyclobutyl) amino)isoindolin-1,3-dione (115 mg, 0.16 mmol), with a yield of 67%. ¹H NMR (400 MHz, CDCl₃) *δ* 8.26 (d, *J* = 9.2 Hz, 1H), 8.05 (s, 1H), 7.64 (d, *J* = 8.2 Hz, 1H), 7.31 (dd, *J* = 8.9, 6.1 Hz, 2H), 7.18 - 7.11 (m, 4H), 6.90 (d, *J* = 2.2 Hz, 1H), 6.75 - 6.69 (m, 3H), 4.97 - 4.83 (m, 2H), 4.79 (s, 1H), 4.24 (s, 1H), 2.95 - 2.79 (m, 2H), 2.78 - 2.68 (m, 3H), 2.53 (s, 3H), 2.41 (dt, *J* = 12.7, 6.3 Hz, 2H), 2.17-2.08 (m, 1H), 1.69 (s, 6H). LC/MS (ESI+) calcd for C₃₉H₃₅N₇O₇ ( [M+H]⁺ ) *m*/*z* 713.75; found 714.2.

### Example 332: 2-(2,6-dioxopiperidin-3-yl)-5-((1r,3r)-3-(4-(2-(4-(4-(5-methyl-1,2,4-oxadiazol-3-yl)phenoxy)phenyl)propan-2-yl)phenoxy)cyclobutyl)aminoisoindolin-1,3-dione

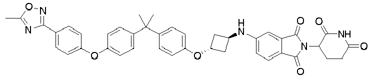

The target compound was synthesized by a method similar to that of Example 326. ¹H NMR (400 MHz, CDCl₃) *δ* 8.06 (s, 1H), 8.03 (s, 1H), 8.02 - 7.97 (m, 2H), 7.90 (dd, *J* = 8.3, 4.4 Hz, 1H), 7.64 (d, *J* = 8.3 Hz, 1H), 7.57 (dd, *J* = 6.9, 2.3 Hz, 1H), 7.44 (td, *J* = 8.5, 2.3 Hz, 1H), 7.23 (d, *J* = 8.6 Hz, 1H), 7.16 (d, *J* = 8.6 Hz, 1H), 7.06 (d, *J* = 8.7 Hz, 1H), 6.96 (d, *J* = 8.6 Hz, 1H), 6.90 (d, *J* = 2.1 Hz, 1H), 6.74 - 6.69 (m, 2H), 4.96 (ddd, *J* = 20.9, 12.3, 5.3 Hz, 2H), 4.24 (s, 1H), 2.96 - 2.86 (m, 2H), 2.84 - 2.71 (m, 4H), 2.65 (s, 3H), 2.41 (dt, *J* = 12.7, 6.1 Hz, 1H), 2.19-2.14 (m, 1H), 1.67 (s, 6H). LC/MS (ESI+) calcd for C₄₁H₃₇N₅O₇ ( [M+H]⁺ ) *m*/*z* 711.78; found 712.2.

### Example 333: 2-(2,6-dioxopiperidin-3-yl)-5-((1r,3r)-3-(4-(2-(4-(pyridazine-3-oxy) phenyl)propan-2-yl)phenoxy)cyclobutyl)aminoisoindolin-1,3-dione

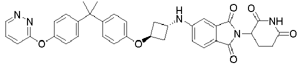

The target compound was synthesized by a method similar to that of Example 331. ¹H NMR (400 MHz, CDCl₃) *δ* 9.06 (s, 1H), 8.01 (s, 1H), 7.76 (s, 1H), 7.63 (d, *J* = 8.1 Hz, 1H), 7.44 (s, 1H), 7.29 (s, 2H), 7.16 (d, *J* = 8.2 Hz, 2H), 7.08 (d, *J* = 7.7 Hz, 2H), 6.91 (s, 1H), 6.72 (d, *J* = 8.3 Hz, 3H), 4.97 - 4.86 (m, 2H), 4.23 (s, 1H), 2.93 - 2.68 (m, 6H), 2.46 - 2.40 (m, 2H), 2.12 (d, *J* = 9.4 Hz, 1H), 1.68 (s, 6H). LC/MS (ESI+) calcd for C₃₆H₃₃N₅O₆ ( [M+H]⁺ ) *m*/*z* 631.69; found 632.3.

### Example 334: 5-((1r,3r)-3-(4-(2-(4-(4-(2H-1,2,3-triazol-2-yl)phenoxy)phenyl) propan-2-yl)phenoxy)cyclobutyl)amino)-2-(2,6-dioxopiperidin-3-yl)isoindolin-1,3-dione

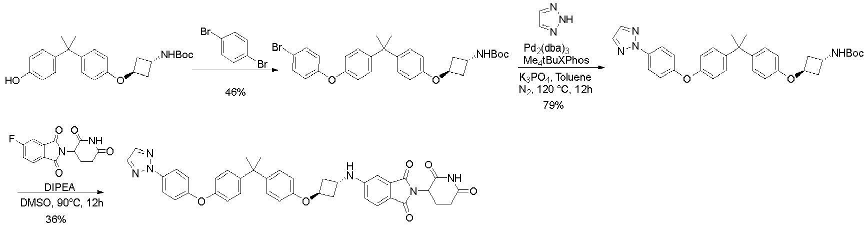

### Step 1: Synthesis of tert-butyl ((1r,3r)-3-(4-(2-(4-(4-bromophenoxy)phenyl)propan-2-yl)phenoxy)cyclobutyl)carbamate

tert-butyl ((1*r*,3*r*)-3-(4-(2-(4-hydroxylphenyl)propan-2-phenoxy)cyclobutyl) carbamate (333 mg, 0.84 mmol) and 1,4-dibromobenzene (175 mg, 1.0 mmol) were dissolved in 10 mL of DMF, to which was added Cs₂CO₃ (822 mg, 2.52 mmol), and then the mixture was heated to 60 °C and reacted overnight. The reaction was cooled to room temperature, and quenched by adding water. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-(4-bromophenoxy)phenyl)propan-2-yl) phenoxy)cyclobutyl)carbamate (212 mg, 0.38 mmol), with a yield of 46%. LC/MS (ESI+) calcd for C₃₀H₃₄BrNO₄ ( [M+H]⁺ ) *m*/*z* 551.51; found 552.2.

### Step 2: Synthesis of tert-butyl ((1r,3r)-3-(4-(2-(4-(4-(2H-1,2,3-triazol-2-yl)phenoxy) phenyl)propan-2-yl)phenoxy)cyclobutyl)carbamate

tert-butyl ((1*r*,3*r*)-3-(4-(2-(4-(4-bromophenoxy)phenyl)propan-2-yl)phenoxy) cyclobutyl)carbamate (212 mg, 0.38 mmol), 2H-1,2,3-triazole (33 mg, 0.47 mmol) and K₃PO₄ (166 mg, 0.78 mmol) were dissolved in 6 mL of toluene in a single-necked flask A, and then the reaction solution was stirred at room temperature under N₂ protection. In another single-necked flask B, Pd₂(dba)₃ (36 mg, 0.04 mmol) and L1 (48 mg, 0.1 mmol) were dissolved in 6 mL of toluene, and then the reaction solution was heated to 120 °C and stirred for 3 min under N₂ protection. The solution in flask B was transferred to flask A, and then the reaction solution was heated to 120 °C and stirred overnight under N₂ protection. The reaction solution was cooled to room temperature, and water was added to quench the reaction. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl ((1*r*,3*r*)-3-(4-(2-(4-(4-(2*H*-1,2,3-triazol-2-yl)phenoxy)phenyl) propan-2-yl)phenoxy)cyclobutyl)carbamate (161 mg, 0.30 mmol), with a yield of 79%. LC/MS (ESI+) calcd for C₃₂H₃₆N₄O₄( [M+H]⁺ ) *m*/*z* 540.66; found 485.3.

### Step 3: Synthesis of (1r,3r)-3-(4-(2-(4-(2H-1,2,3-triazol-2-yl)phenoxy)phenyl)propan-2-yl)phenoxy)cyclobutane-1-amine

tert-butyl ((1*r*,3*r*)-3-(4-(2-(4-(4-(2*H*-1,2,3-triazol-2-yl)phenoxy)phenyl)propan-2-yl)phenoxy)cyclobutyl)carbamate (161 mg, 0.30 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1*r*,3*r*)-3-(4-(2-(4-(2*H*-1,2,3-triazol-2-yl)phenoxy)phenyl)propan-2-yl)phenoxy)cyclobutane-1-amine (121 mg, 0.28 mmol), with a yield of 94%.

### Step 4: Synthesis of 5-((1r,3r)-3-(4-(2-(4-(4-(2H-1,2,3-triazol-2-yl)phenoxy) phenyl)propan-2-yl)phenoxy)cyclobutyl)amino)-2-(2,6-dioxopiperidin-3-yl) isoindolin-1,3-dione

(1*r*,3*r*)-3-(4-(2-(4-(2*H*-1,2,3-triazol-2-yl)phenoxy)phenyl)propan-2-yl)phenoxy) cyclobutane-1-amine (121 mg, 0.28 mmol) was dissolved in 10 mL of DMSO, to which were successively added DIPEA (106 mg, 0.83 mmol) and 2-(2,6-dioxopiperidin-3-yl)-5-fluoroisoindolin-1,3-dione (83 mg, 0.30 mmol), and then the reaction solution was heated to 60 °C and reacted overnight under N₂ protection. The reaction solution was cooled to room temperature, and water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 5-((1*r*,3*r*)-3-(4-(2-(4-(4-(2*H*-1,2,3-triazol-2-yl)phenoxy)phenyl)propan-2-yl)phenoxy) cyclobutyl)amino)-2-(2,6-dioxopiperidin-3-yl)isoindolin-1,3-dione (70 mg, 0.1 mmol), with a yield of 36%. ¹H NMR (400 MHz, CDCl₃) *δ* 8.06 - 7.98 (m, 3H), 7.80 (s, 2H), 7.63 (d, *J* = 8.3 Hz, 1H), 7.23 - 7.19 (m, 2H), 7.18 - 7.14 (m, 2H), 7.12 - 7.08 (m, 2H), 6.96-6.92 (m, 2H), 6.90 (d, *J* = 2.1 Hz, 1H), 6.71 (dd, *J* = 8.8, 2.3 Hz, 3H), 4.93 (dd, *J* = 12.1, 5.2 Hz, 1H), 4.86 (q, *J* = 6.6, 5.4 Hz, 1H), 4.24 (dd, *J* = 9.0, 4.4 Hz, 1H), 2.95 - 2.86 (m, 1H), 2.82 (dd, *J* = 12.6, 3.9 Hz, 1H), 2.78 - 2.69 (m, 3H), 2.41 (dt, *J* = 13.0, 6.3 Hz, 2H), 2.17 - 2.08 (m, 1H), 1.67 (s, 6H). LC/MS (ESI+) calcd for C₄₀H₃₆N₆O₆ ( [M+H]⁺ ) *m*/*z* 696.76; found 697.0.

### Example 335: 5-((1r,3r)-3-(4-(2-(4-((6-(5-methyl-1,2,4-oxadiazol-3-yl)pyridazine-3-yl)oxy)phenyl)propan-2-yl)phenoxy)cyclobutyl)amino)-2-(1-methyl-2,6-dioxopiperidin-3-yl)isoindolin-1,3-dione

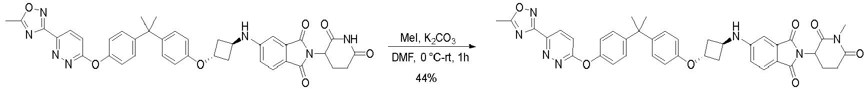

2-(2,6-dioxopiperidin-3-yl)-5-((1*r*,3*r*)-3-(4-(2-(4-((6-(5-methyl-1,2,4-oxadiazol-3-yl)pyridazine-3-yl)oxy)phenyl)propan-2-yl)phenoxy)cyclobutyl)amino)isoindolin-1,3-dione (71 mg, 0.10 mmol) was dissolved in 5 mL of DMF, to which were successively added K₂CO₃ (42 mg, 0.30 mmol) and CH₃I (20 mg, 0.20 mmol), and then the mixture was allowed to react overnight at room temperature. Water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 5-((1*r*,3*r*)-3-(4-(2-(4-((6-(5-methyl-1,2,4-oxadiazol-3-yl)pyridazine-3-yl)oxy)phenyl)propan-2-yl)phenoxy)cyclobutyl)amino)-2-(1-methyl-2,6-dioxopiperidin-3-yl)isoindolin-1,3-dione (32 mg, 0.04 mmol), with a yield of 44%. ¹H NMR (400 MHz, DMSO-*d*₆) *δ* 8.28 (d, *J* = 9.2 Hz, 1H), 7.60 (dd, *J* = 8.8, 6.0 Hz, 2H), 7.55 (d, *J* = 5.5 Hz, 1H), 7.31 (d, *J* = 8.7 Hz, 2H), 7.22 - 7.15 (m, 4H), 6.86 (s, 1H), 6.79 (t, *J* = 8.8 Hz, 3H), 5.10 (dd, *J* = 12.9, 5.4 Hz, 1H), 4.88 (s, 1H), 4.15 (s, 1H), 3.17 (d, *J* = 5.3 Hz, 1H), 3.00 (s, 3H), 2.76 (d, *J* = 4.1 Hz, 1H), 2.71 (s, 3H), 2.54 (s, 3H), 2.44 (d, *J* = 7.1 Hz, 2H), 2.01 (d, *J* = 13.6 Hz, 1H), 1.65 (s, 6H). LC/MS (ESI+) calcd for C₄₀H₃₇N₇O₇ ( [M+H]⁺ ) *m*/*z* 727.8; found 728.2.

### Example 336: 2-(2,6-dioxopiperidin-3-yl)-5-((1s,3s)-3-(4-(2-((5-(5-methyl-1,2,4-oxadiazol-3-yl)pyrimidin-2-yl)oxy)phenyl)propan-2-yl)phenoxy)cyclobutyl) amino)isoindolin-1,3-dione

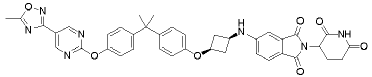

The target compound was synthesized by a method similar to that of Example 326. ¹H NMR (400 MHz, CDCl₃) *δ* 9.17 (s, 2H), 8.03 (s, 1H), 7.63 (d, *J* = 8.2 Hz, 1H), 7.33 - 7.28 (m, 2H), 7.21 - 7.15 (m, 2H), 7.16 - 7.09 (m, 2H), 6.92 (d, *J* = 2.1 Hz, 1H), 6.77 - 6.73 (m, 2H), 6.71 (d, *J* = 2.1 Hz, 1H), 4.93 (dd, *J* = 12.0, 5.2 Hz, 1H), 4.52 (p, *J* = 6.8 Hz, 1H), 3.78 (p, *J* = 7.5 Hz, 1H), 3.19 - 3.08 (m, 2H), 2.89 (d, *J* = 17.0 Hz, 1H), 2.85 - 2.71 (m, 2H), 2.68 (s, 3H), 2.16-2.06 (m, 3H), 1.69 (s, 6H). LC/MS (ESI+) calcd for C₃₉H₃₅N₇O₇ ( [M+H]⁺ ) *m*/*z* 713.8; found 714.2.

### Example 337: 2-(2,6-dioxopiperidin-3-yl)-5-(1s,3s)-3-(4-(2-(4-(6-(5-methyl-1,2,4-oxadiazol-3-yl)pyridazine-3-yl)oxy)phenyl)propan-2-yl)phenoxy)cyclobutyl) amino)isoindolin-1,3-dione

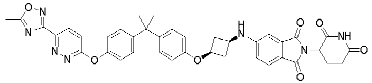

The target compound was synthesized by a method similar to that of Example 326. ¹H NMR (400 MHz, CDCl₃) *δ* 8.18 (d, *J* = 9.1 Hz, 1H), 8.01 (s, 1H), 7.63 (d, *J* = 8.3 Hz, 1H), 7.30 - 7.28 (m, 1H), 7.24 (s, 1H), 7.14 (dd, *J* = 12.9, 8.6 Hz, 4H), 6.93 (d, *J* = 2.0 Hz, 1H), 6.76 - 6.72 (m, 3H), 4.93 (dd, *J* = 12.0, 5.2 Hz, 1H), 4.54 (q, *J* = 6.9 Hz, 1H), 3.79 (t, *J* = 7.5 Hz, 1H), 3.13 (d, *J* = 6.0 Hz, 2H), 2.94 - 2.74 (m, 3H), 2.71 (s, 3H), 2.09 (s, 3H), 1.68 (s, 6H). LC/MS (ESI+) calcd for C₃₉H₃₅N₇O₇ ( [M+H]⁺ ) *m*/*z* 713.8; found 714.2.

### Example 338: 2-(2,6-dioxopiperidin-3-yl)-5-((1r,3r)-3-(4-(1-(4-((6-(5-methyl-1,2,4-oxadiazol-3-yl)pyridazine-3-yl)oxy)phenyl)cyclopentyl)phenoxy)cyclobutyl) amino)isoindolin-1,3-dione

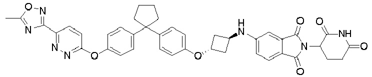

The target compound was synthesized by a method similar to that of Example 326. ¹H NMR (400 MHz, CDCl₃) *δ* 8.16 (d, *J* = 8.9 Hz, 1H), 8.01 (s, 1H), 7.63 (d, *J* = 8.0 Hz, 1H), 7.31 (d, *J* = 8.2 Hz, 2H), 7.23 (d, *J* = 9.0 Hz, 1H), 7.19 (d, *J* = 8.2 Hz, 2H), 7.11 (d, *J* = 8.2 Hz, 2H), 6.90 (s, 1H), 6.70 (t, *J* = 9.6 Hz, 3H), 5.30 (s, 1H), 4.93 (dd, *J* = 12.1, 5.1 Hz, 1H), 4.85 (s, 1H), 4.22 (s, 1H), 2.94 - 2.74 (m, 3H), 2.71 (s, 3H), 2.41 (s, 2H), 2.28 (s, 4H), 2.15 - 2.09 (m, 1H), 1.73 (m, 6H).
LC/MS (ESI+) calcd for C₄₁H₃₇N₇O₇ ( [M+H]⁺ ) *m*/*z* 739.8; found 740.2.

### Example 339: 2-(2,6-dioxopiperidin-3-yl)-5-((1r,3r)-3-(4-(1-(4-((2-(5-methyl-1,2,4-oxadiazol-3-yl)pyrimidin-5-yl)oxy)phenyl)cyclohexyl)phenoxy)cyclobutyl)amino) isoindolin-1,3-dione

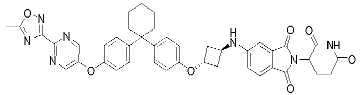

The target compound was synthesized by a method similar to that of Example 326. ¹H NMR (400 MHz, CDCl₃) *δ* 8.57 (s, 2H), 8.05 (s, 1H), 7.63 (d, *J* = 8.1 Hz, 1H), 7.33 (d, *J* = 8.4 Hz, 2H), 7.17 (d, *J* = 8.3 Hz, 2H), 7.01 (d, *J* = 8.5 Hz, 2H), 6.95 - 6.84 (m, 1H), 6.72 (d, *J* = 8.1 Hz, 3H), 4.93 (dd, *J* = 12.0, 5.2 Hz, 1H), 4.86 (s, 1H), 4.23 (s, 1H), 2.93 - 2.73 (m, 3H), 2.73 - 2.71 (m, 3H), 2.41 (d, *J* = 9.3 Hz, 2H), 2.25 (s, 4H), 2.16 - 2.09 (m, 1H), 1.54 (d, *J* = 14.9 Hz, 8H). LC/MS (ESI+) calcd for C₄₂H₃₉N₇O₇ ( [M+H]⁺ ) *m*/*z* 753.82; found 754.3.

### Example 340: 2-(2,6-dioxopiperidin-3-yl)-5-((2S)-2-((4-(2-(2-(1-hydroxylethyl) pyrimidin-5-yl)oxy)phenyl)propan-2-yl)phenoxy)methyl)azetidin-1-yl)isoindolin-1,3-dione

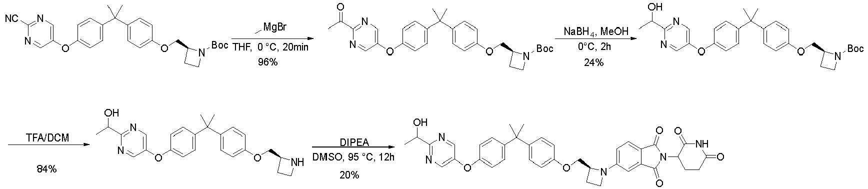

### Step 1: Synthesis of tert-butyl (S)-2-((4-(2-(4-((2-acetylpyrimidin-5-yl)oxy)phenyl) propan-2-yl)phenoxy)methyl)azetidin-1-carboxylate

(*S*)-2-((4-(2-(4-((2-cyanopyrimidin-5-yl)oxy)phenyl)propan-2-yl)phenoxy) methyl)azetidin-1-carboxylate (500 mg, 1.0 mmol) was dissolved in 10 mL of dry tetrahydrofuran, and then the solution was cooled to about 0 °C in an ice-water bath, followed by addition of methylmagnesium bromide (1N) solution (4 mL, 4 mmol) in one portion. The reaction was allowed to react for 1 h at 0 °C. After disappearance of starting materials by TLC detection, 20 mL of diluted hydrochloric acid (0.5N) was added, and then the reaction was stirred for 5 min. After that, the reaction solution was extracted with ethyl acetate, and concentrated under reduced pressure. The residue was separated and purified by prep-TLC, to provide compound tert-butyl (*S*)-2-((4-(2-(4-((2-acetylpyrimidin-5-yl)oxy)phenyl)propan-2-yl)phenoxy)methyl)azetidin-1-carboxylate (498 mg, 0.96 mmol), with a yield of 96%. LC/MS (ESI+) calcd for C₃₀H₃₅N₃O₅ ( [M+H]⁺ ) *m*/*z* 517.63; found 518.2.

### Step 2: Synthesis of tert-butyl (2S)-2-((4-(2-(4-((2-(1-hydroxylethyl)pyrimidin-5-yl)oxy)phenyl)propan-2-yl)phenoxy)methyl)azetidin-1-carboxylate

tert-butyl (*S*)-2-((4-(2-(4-((2-acetylpyrimidin-5-yl)oxy)phenyl)propan-2-yl) phenoxy)methyl)azetidin-1-carboxylate (498 mg, 0.96 mmol) was dissolved in 20 mL of methanol, and then the solution was cooled to about 0 °C in an ice-water bath, to which was added sodium borohydride (152 mg, 3.84 mmol). The mixture was allowed to react at 0 °C for 1 h, followed by addition of ice water. The resultant solution was extracted with ethyl acetate. The water layer was re-extracted once with ethyl acetate. The organic layers were combined, washed with saturated brine, dried with anhydrous Na₂SO₄, and concentrated. The residue was separated and purified by prep-TLC, to provide compound tert-butyl (2*S*)-2-((4-(2-(4-((2-(1-hydroxylethyl)pyrimidin-5-yl)oxy)phenyl)propan-2-yl)phenoxy)methyl)azetidin-1-carboxylate (121 mg, 0.23 mmol), with a yield of 24%. LC/MS (ESI+) calcd for C₃₀H₃₇N₃O₅ ( [M+H]⁺ ) *m*/*z* 519.64; found 520.2.

### Step 3: Synthesis of 1-(5-(4-(2-(4-((S)-azetidin-2-yl)methoxy)phenyl)propan-2-yl)phenoxy)pyrimidin-2-yl)ethane-1-ol

tert-butyl (2*S*)-2-((4-(2-(4-((2-(1-hydroxylethyl)pyrimidin-5-yl)oxy)phenyl) propan-2-yl)phenoxy)methyl)azetidin-1-carboxylate (121 mg, 0.23 mmol) was dissolved in 8 mL of dichloromethane, to which was added 2 mL of trifluoroacetic acid dropwise in an ice bath, and then the reaction was further stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 1-(5-(4-(2-(4-((*S*)-azetidin-2-yl)methoxy)phenyl)propan-2-yl)phenoxy) pyrimidin-2-yl)ethane-1-ol (81 mg, 0.19 mmol), with a yield of 84%.

### Step 4: Synthesis of 2-(2,6-dioxopiperidin-3-yl)-5-((2S)-2-((4-(2-(2-(1-hydroxylethyl) pyrimidin-5-yl)oxy)phenyl)propan-2-yl)phenoxy)methyl)azetidin-1-yl)isoindolin-1,3-dione

(1-(5-(4-(2-(4-((*S*)-azetidin-2-yl)methoxy)phenyl)propan-2-yl)phenoxy) pyrimidin-2-yl)ethane-1-ol (81 mg, 0.19 mmol) was dissolved in 10 mL ofDMSO, to which were successively added DIPEA (75 mg, 0.58 mmol) and 2-(2,6-dioxopiperidin-3-yl)-5-fluoroisoindolin-1,3-dione (60 mg, 0.22 mmol), and then the reaction solution was heated to 60 °C and reacted overnight under N₂ protection. The reaction solution was cooled to room temperature, and water was added to quench the reaction. The resultant solution was extracted with ethyl acetate for three times. The organic phase was washed with saturated brine, dried with anhydrous Na₂SO₄, concentrated and rotatory evaporated to dry. The residue was separated and purified by prep-TLC, to provide 2-(2,6-dioxopiperidin-3-yl)-5-((2*S*)-2-((4-(2-(2-(1-hydroxylethyl)pyrimidin-5-yl)oxy)phenyl)propan-2-yl)phenoxy)methyl)azetidin-1-yl)isoindol-1,3-dione (26 mg, 0.04 mmol), with a yield of 20%. ¹H NMR (400 MHz, CDCl₃) *δ* 8.46 (d, *J* = 2.5 Hz, 2H), 7.64 (d, *J* = 8.3 Hz, 1H), 7.22 (t, *J* = 10.1 Hz, 3H), 7.15 (dd, *J* = 8.9, 2.4 Hz, 2H), 6.94 (d, *J* = 8.7 Hz, 2H), 6.90 - 6.84 (m, 2H), 6.79 - 6.71 (m, 1H), 5.03 - 4.89 (m, 2H), 4.65 (s, 1H), 4.29 - 4.19 (m, 2H), 3.88 (q, *J* = 7.9 Hz, 1H), 2.94 - 2.71 (m, 3H), 2.67 - 2.55 (m, 1H), 2.44 - 2.33 (m, 1H), 2.18-2.07 (m, 2H), 1.66 (s, 6H), 1.58 (d, *J* = 6.5 Hz, 3H). LC/MS (ESI+) calcd for C₃₈H₃₇N₅O₇ ( [M+H]⁺ ) *m*/*z* 675.74; found 676.2.

### Example 341: 5-((5)-2-((4-(2-(4-((2-acetylpyrimidin-5-yl)oxy)phenyl)propan-2-yl)phenoxy)methyl)azetidin-1-yl)-2-(2,6-dioxopiperidin-3-yl)isoindolin-1,3-dione

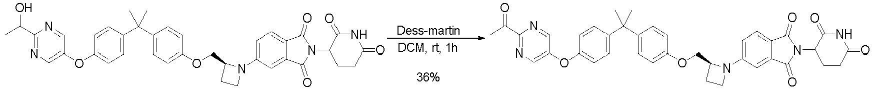

2-(2,6-dioxopiperidin-3-yl)-5-((2*S*)-2-((4-(2-(2-(1-hydroxylethyl)pyrimidin-5-yl)oxy)phenyl)propan-2-yl)phenoxy)methyl)azetidin-1-yl)isoindolin-1,3-dione (93 mg, 0.14 mmol) was dissolved in 20 mL of dichloromethane, to which was added Dess-Martin periodinane (117 mg, 0.28 mmol), and then the mixture was allowed to react at room temperature for 1 h. The reaction solution was filtered over diatomite, and the filter cake was rinsed with dichloromethane. The organic phase was combined and concentrated. The residue was separated and purified by prep-TLC, to provide compound 5-((*S*)-2-((4-(2-(4-((2-acetylpyrimidin-5-yl)oxy)phenyl)propan-2-yl) phenoxy)methyl)azetidin-1-yl)-2-(2,6-dioxopiperidin-3-yl)isoindolin-1,3-dione (32 mg, 0.05 mmol), with a yield of 36%. ¹H NMR (400 MHz, CDCl₃) *δ* 8.54 (s, 2H), 8.12 (d, *J* = 30.7 Hz, 1H), 7.65 (d, *J* = 8.3 Hz, 1H), 7.31 - 7.27 (m, 2H), 7.19 - 7.12 (m, 3H), 7.02 - 6.96 (m, 2H), 6.91 - 6.85 (m, 2H), 6.78 (d, *J* = 8.3 Hz, 1H), 4.93 (dd, *J* = 12.2, 5.3 Hz, 1H), 4.65 (t, *J* = 6.5 Hz, 1H), 4.24 (d, *J* = 6. 0 Hz, 2H), 4.17 (td, *J* = 8.4, 4.8 Hz, 1H), 3.88 (q, *J* = 7.8 Hz, 1H), 2.93 - 2.77 (m, 2H), 2.75 (s, 3H), 2.74 - 2.69 (m, 1H), 2.66 - 2.53 (m, 1H), 2.47 - 2.33 (m, 1H), 2.14 (d, *J* = 5.4 Hz, 1H), 1.68 (s, 6H). LC/MS (ESI+) calcd for C₃₈H₃₅N₅O₇ ( [M+H]⁺ ) *m*/*z* 673.73; found 674.2.

### Example 342: 5-((5-(4-(2-(4-((2-acetyl pyrimidin-4-yl)methoxy)phenyl)isopropan-2-yl)phenoxy)pentyl)amino)-2-(2,6-dioxopiperidin-3-yl)isoindolin-1,3-dione

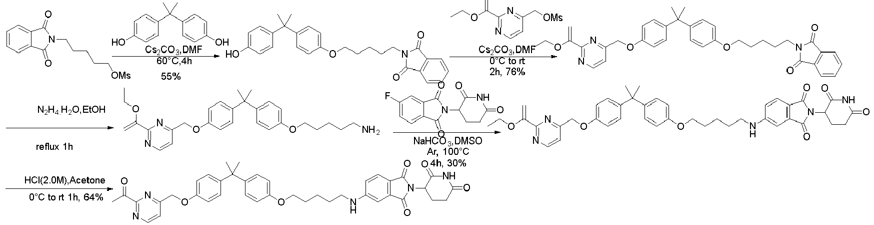

### Step 1: Synthesis of 2-(5-(4-(2-(4-hydroxylphenyl)isopropan-2-yl)phenoxy)pentyl) isoindolin-1,3-dione

Bisphenol A (600 mg, 2.63 mmol) was weighed and placed in a single-necked round-bottome flask, to which was added DMF (20 mL), and then the mixture was stirred to dissolve and become clear, followed by addition of cesium carbonate (857 mg, 2.63 mmol). The reaction solution was stirred for 15 min, and then 5-(1,3-dioxoisoindolin-2-yl)phenyl methanesulfonate (545 mg, 1.75 mmol) was added. Subsequently, the reaction solution was heated to 60 °C, and reacted under stirring. After 4 h, the reaction was completed by TLC detection, and then heating was removed. The reaction solution was cooled to room temperature, and extracted by adding ethyl acetate and water. The organic phase was successively washed with 0.01 N HCl solution, water, and saturated brine, and dried over anhydrous Na₂SO₄. The resultant solution was rotatory evaporated to remove the solvent and provide the crude product, which was separated and purified by column chromatography, to obtain the target product as white solid (425 mg, 55%). LC/MS (ESI⁺) calcd for C₂₈H₂₉NO₄ ([M-H]⁻) *m*/*z* 443.2; found, 442.2.

### Step 2: Synthesis of 2-(5-(4-(2-(4-((3-(1-ethoxyvinyl)phenyl)oxy)phenyl)isopropan-2-yl)phenoxy)pentyl)isoindolin-1,3-dione

2-(5-(4-(2-(4-hydroxylphenyl)isopropan-2-yl)phenoxy)pentyl)isoindolin-1,3-dione (144 mg, 0.32 mmol), (2-(1-ethoxyvinyl)pyrimidin-4-yl)methyl methanesulfonate (90 mg, 0.35 mmol), and cesium carbonate (114 mg, 0.35 mmol) were added into 8 mL of DMF, and then the solution was heated to 25 °C, and reacted under stirring. After 2 h, TLC detection indicated the starting materials disappeared, and heating was removed. The reaction solution was cooled to room temperature, and extracted by adding ethyl acetate and water. The organic phase was successively washed with water and saturated brine, and dried over anhydrous Na₂SO₄. The resultant solution was rotatory evaporated to remove the solvent and provide the crude product, which was separated and purified by Prep-TLC, to provide the target compound as white solid (150 mg, 76%). LC/MS (ESI⁺) calcd for C₃₇H₃₉N₃O₅ ([M+H]⁺) *m*/*z* 605.3; found, 606.3.

### Step 3: Synthesis of 5-(4-(2-(4-((2-(1-ethoxyvinyl)pyrimidin-4-yl)methoxy)phenyl) isopropan-2-yl)phenoxy)pentan-1-amine

Compound 2-(5-(4-(2-(4-((3-(1-ethoxyvinyl)phenyl)oxy)phenyl)isopropan-2-yl) phenoxy)pentyl)isoindolin-1,3-dione (150 mg, 0.25 mmol) was dissolved in 5 mL of ethanol, to which was added hydrazine hydrate (135 mg, 2.50 mmol), and then the system was moved to an oil bath at 85°C and reacted under refluxing. After 1 h, TLC detection indicated completion of the reaction, and heating was removed. The solvent and excessive hydrazine hydrate were removed by rotatory evaporation, followed by addition of dichloromethane for several times, to remove the residual ethanol by co-evaporation. To the residue, was added dichloromethane (15 mL), and then the system was stirred at room temperature, presenting a white and turbid appearance. After 30 min, the reaction solution was subjected to suction filtration, and the filter cake was rinsed many times with a small amount of dichloromethane. The filtrate was collected, and dried with anhydrous Na₂SO₄. The solvent was removed by rotatory evaporation, to obtain the target product as off-white solids (84 mg), which was directly used in the next step without further purification. LC/MS (ESI⁺) calcd for C₂₉H₃₇N₃O₃([M+H]⁺) *m*/*z* 475.3; found, 476.3.

### Step 4: Synthesis of 2-(2,6-dioxopiperidin-3-yl)-5-((5-(4-(2-(4-((2-(1-ethoxyvinyl) pyrimidin-4-yl)methoxy)phenyl)isopropan-2-yl)phenoxy)pentyl)amino)isoindolin-1,3-dione

5-(4-(2-(4-((2-(1-ethoxyvinyl)pyrimidin-4-yl)methoxy)phenyl)isopropan-2-yl)phenoxy)pentan-1-amine (84 mg, 0.18 mmol), 2-(2,6-dioxopiperidin-3-yl)-5-fluoroisoindolin-1,3-dione (75 mg, 0.27 mmol) and NaHCO₃ (38 mg, 0.45 mmol) were added to 4 mL of dry DMSO, and then the system was vacuumized and purged with argon, that was repeated three times. After addition, the system was transferred into an oil bath at 100 °C and reacted under stirring. After 5 h, TLC indicated almost disappearance of the starting materials. The reaction solution was cooled to room temperature, and extracted by adding ethyl acetate and water. The organic phase was washed with saturated NH₄Cl solution, water, and saturated brine, and then dried with anhydrous Na₂SO₄. The solvent was removed by rotatory evaporation. The residue was separated and purified by Prep-TLC, to provide the target compound as light yellow solid (39 mg, 30%). LC/MS (ESI⁺) calcd for C₄₂H₄₅N₅O₇ ([M+H]⁺) *m*/*z* 731.3; found, 732.8.

### Step 5: Synthesis of 5-((5-(4-(2-(4-((2-acetylpyrimidin-4-yl)methoxy)phenyl) isopropan-2-yl)phenoxy)pentyl)amino)-2-(2,6-dioxopiperidin-3-yl)isoindolin-1,3-dione

2-(2,6-dioxopiperidin-3-yl)-5-((5-(4-(2-(4-((2-(1-ethoxyvinyl)pyrimidin-4-yl) methoxy)phenyl)isopropan-2-yl)phenoxy)pentyl)amino)isoindolin-1,3-dione (39 mg, 0.05 mmol) was dissolved in 3 mL of acetone, to which was added HCl (100 µL, 2.0 M), and then the solution was reacted at room temperature under stirring. After 1 h, TLC detection indicated the starting materials disappeared. The solvent was rotatory evaporated at low temperature, followed by addition of dichloromethane and water. The pH of the system was adjusted to about 8 with saturated NaHCO₃ solution, and then the extraction procedure was performed. The organic phase was successively washed with water and saturated brine, and dried with anhydrous Na₂SO₄. The resultant solution was rotatory evaporated to remove the solvent and provide the crude product, which was separated and purified by Prep-TLC, to provide the target compound as light yellow solid (24 mg, 64%). ¹H NMR (400 MHz, Chloroform-*d*) *δ* 8.92 (d, *J* = 5.1 Hz, 1H), 8.03 (s, 1H), 7.74 (d, *J* = 4.8 Hz, 1H), 7.61 (d, *J* = 8.3 Hz, 1H), 7.15 (dd, *J* = 15.7, 8.6 Hz, 4H), 6.97 (s, 1H), 6.83 (dd, *J* = 27.3, 8.6 Hz, 4H), 6.74 (d, *J* = 8.0 Hz, 1H), 5.25 (s, 2H), 4.93 (dd, *J* = 11.8, 4.9 Hz, 1H), 3.96 (t, *J* = 5.9 Hz, 2H), 3.26 (t, *J* = 6.8 Hz, 2H), 2.94 - 2.63 (m, 7H), 2.26 - 2.20 (m, 1H), 2.16 - 2.09 (m, 1H), 2.04 - 1.97 (m, 1H), 1.82 (dd, *J=* 13.3, 6.6 Hz, 2H), 1.79 - 1.68 (m, 2H) , 1.64 (s, 6H). LC/MS (ESI⁺) calcd for C₄₀H₄₁N₅O₇ ([M+H]⁺) *m*/*z* 703.3; found, 704.2.

### Example 343: 5-((3-(4-(2-(4-((2-acetylpyrimidin-4-yl)methoxy)phenyl)isopropan-2-yl)phenoxy)propyl)amino)-2-(2,6-dioxopiperidin-3-yl)isoindolin-1,3-dione

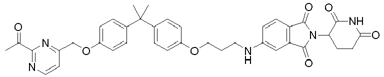

The target compound was synthesized by a method similar to that of Example 342. ¹H NMR (400 MHz, Chloroform-*d*) *δ* 8.92 (d, *J* = 5.0 Hz, 1H), 8.11 (s, 1H), 7.74 (d, *J* = 5.0 Hz, 1H), 7.60 (d, *J* = 8.3 Hz, 1H), 7.21 - 7.12 (m, 4H), 6.98 (d, *J* = 1.8 Hz, 1H), 6.87 (d, *J* = 8.9 Hz, 2H), 6.82 (d, *J* = 8.8 Hz, 2H), 6.75 (dd, *J* = 8.3, 1.9 Hz, 1H), 5.25 (s, 2H), 4.93 (dd, *J* = 12.2, 5.3 Hz, 1H), 4.09 (t, *J* = 5.4 Hz, 2H), 3.46 (t, *J* = 6.4 Hz, 2H), 2.93 - 2.63 (m, 7H), 2.17 - 2.09 (m, 3H), 1.64 (s, 6H). LC/MS (ESI⁺) calcd for C₃₈H₃₇N₅O₇ ([M+H]⁺) *m*/*z* 675.3; found, 676.2.

### Example 344: 4-((3-(4-(2-(4-((2-acetylpyrimidin-4-yl)methoxy)phenyl)isopropan-2-yl)phenoxy)propyl)amino)-2-(2,6-dioxopiperidin-3-yl)isoindolin-1,3-dione

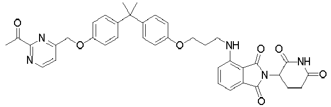

The target compound was synthesized by a method similar to that of Example 342. ¹H NMR (400 MHz, Chloroform-*d*) *δ* 8.92 (d, *J=* 4.9 Hz, 1H), 8.01 (s, 1H), 7.74 (d, *J* = 4.8 Hz, 1H), 7.47 (dd, *J* = 8.5, 7.2 Hz, 1H), 7.15 (dd, *J* = 11.9, 8.8 Hz, 4H), 7.09 (d, *J* = 7.0 Hz, 1H), 6.92 (d, *J* = 8.6 Hz, 1H), 6.85 (t, *J* = 9.1 Hz, 4H), 5.25 (s, 2H), 4.92 (dd, *J* = 12.0, 5.4 Hz, 1H), 4.07 (t, *J* = 5.6 Hz, 2H), 3.51 (t, *J* = 6.6 Hz, 2H), 2.94 - 2.67 (m, 7H), 2.13 (p, *J* = 5.2, 4.6 Hz, 3H), 1.64 (s, 6H). LC/MS (ESI⁺) calcd for C₃₈H₃₇N₅O₇ ([M+H]⁺) *m*/*z* 675.3; found, 676.3.

### Example 345: 2-(2,6-dioxopiperidin-3-yl)-5-((3-(4-(2-(4-((2-(1-hydroxylethyl) pyrimidin-4-yl)methoxy)phenyl)isopropan-2-yl)phenoxy)propyl)amino) isoindolin-1,3-dione

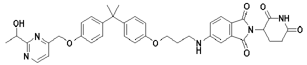

The target compound was synthesized by a method similar to that of Example 342. ¹H NMR (400 MHz, Chloroform-*d*) *δ* 8.78 (s, 1H), 8.26 - 8.11 (m, 1H), 7.60 (d, *J* = 8.0 Hz, 1H), 7.55 (s, 1H), 7.15 (d, *J* = 7.7 Hz, 4H), 6.98 (s, 1H), 6.84 (dd, *J* = 17.8, 7.9 Hz, 4H), 6.75 (d, *J* = 7.9 Hz, 1H), 5.17 (s, 2H), 5.01 (s, 1H), 4.93 (dd, *J* = 11.1, 4.2 Hz, 1H), 4.09 (s, 2H), 3.47 (t, *J* = 5.4 Hz, 2H), 2.94 - 2.65 (m, 4H), 2.19 - 2.06 (m, 4H), 1.65 (s, 6H), 1.62 (s, 3H). LC/MS (ESI⁺) calcd for C₃₈H₃₉N₅O₇ ([M+H]⁺) *m*/*z* 677.3; found, 678.2.

### Example 346:2-(2,6-dioxopiperidin-3-yl)-4-((3-(4-(2-(4-((2-(1-hydroxylethyl) pyrimidin-4-yl)methoxy)phenyl)isopropan-2-yl)phenoxy)propyl)amino) isoindolin-1,3-dione

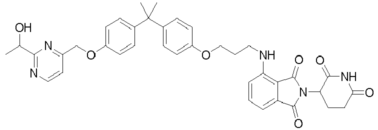

The target compound was synthesized by a method similar to that of Example 342. ¹H NMR (400 MHz, Chloroform-*d*) *δ* 8.78 (s, 1H), 8.43 (s, 1H), 7.57 - 7.50 (m, 1H), 7.46 (dd, *J* = 8.5, 7.2 Hz, 1H), 7.15 (dd, *J* = 10.5, 8.7 Hz, 4H), 7.09 (d, *J* = 7.0 Hz, 1H), 6.92 (d, *J* = 8.5 Hz, 1H), 6.84 (dd, *J* = 8.3, 6.3 Hz, 4H), 5.17 (s, 2H), 5.01 (d, *J* = 6.0 Hz, 1H), 4.92 (dd, *J* = 11.8, 5.2 Hz, 1H), 4.07 (t, *J* = 5.6 Hz, 2H), 3.50 (t, *J* = 6.4 Hz, 2H), 2.94 - 2.66 (m, 4H), 2.18 - 2.08 (m, 4H), 1.64 (s, 6H), 1.60 (d, *J=* 6.1 Hz, 3H). LC/MS (ESI⁺) calcd for C₃₈H₃₉N₅O₇ ([M+H]⁺) *m*/*z* 677.3; found, 678.2.

### Example 347: 5-((4-((8-(4-(2-(4-((2-acetylpyridin-4-yl)methoxy)phenyl)isopropan-2-yl)phenoxy)octyl)oxy)butyl)amino)-2-(2,6-dioxopiperidin-3-yl)isoindolin-1,3-dione

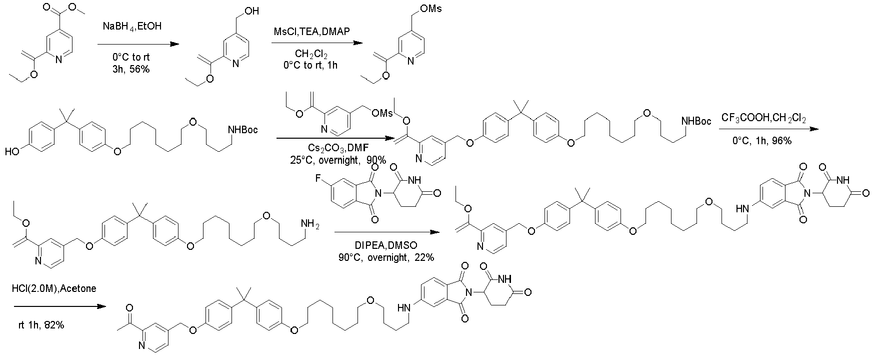

### Step 1: Synthesis of (2-(1-ethoxyvinyl)pyridin-4-yl)methanol

Methyl 2-(1-ethoxyvinyl)isonicotinate (920 mg, 4.44 mmol) was added into a single-necked round-bottome flask, to which was added ethanol (15 mL), and then the mixture was stirred to dissolve and become clear. Subsequently, the system was transferred into an ice water bath and cooled under stirring. After 15 min, to the system, was added sodium borohydride (420 mg, 11.10 mmol), and then the system was allowed to react in the ice water bath for 10 min, followed by reaction at room temperature under stirring. After 3 h, the starting materials disappeared by TLC detection. To the system, was added saturated NH₄Cl solution in the ice-water bath to quench the reaction. The resultant solution was extracted after adding ethyl acetate and water. The organic phase was successively washed with water and saturated brine, dried with anhydrous Na₂SO₄, and rotatory evaporated to remove the solvent and provide the crude product, which was separated and purified by column chromatography, to obtain the target product as off-white solid (445 mg, 56%).

### Step 2: Synthesis of (2-(1-ethoxyvinyl)pyridin-4-yl)methyl methanesulfonate

(2-(1-ethoxyvinyl)pyridin-4-yl)methanol (452 mg, 2.52 mmol) was dissolved in 20 mL of dichloromethane, to which was added triethylamine (765 mg, 7.56 mmol) and DMAP (31 mg, 0.25 mmol), and then the system was transferred into an ice water bath for cooling under stirring. After 15 min, methanesulfonyl choride (577 mg, 5.04 mmol) was added. Subsequently, the system was stirred and reacted at room temperature. After 1 h, the starting materials disappeared by TLC detection. To the system, were added dichloromethane and water, to carry out the extraction procedures. The organic phase was dried with anhydrous Na₂SO₄. The solvent was removed by rotatory evaporation, to provide the target product as oily liquid (243 mg), which was directly used in the next step without further purification. LC/MS (ESI⁺) calcd for C₁₁H₁₅NO₄S ([M+H]⁺) *m*/*z* 257.1; found 258.1.

### Step 3: Synthesis of tert-butyl (4-((8-(4-(2-(4-((2-(1-ethoxyvinyl)pyridin-4-yl)methoxy)phenyl)isopropan-2-yl)phenoxy)octyl)oxy)butyl)carbamate

(2-(1-ethoxyvinyl)pyridin-4-yl)methyl methanesulfonate (157 mg, 0.61 mmol), tert-butyl (4-((8-(4-(2-(4-hydroxylphenyl)isopropan-2-yl)phenoxy)octyl)oxy)tert-butyl)carbamate (248 mg, 0.47 mmol) and cesium carbonate (169 mg, 0.52 mmol) were added into 10 mL of DMF, and then the system was moved into an oil bath at 25 °C and reacted overnight under stirring. The next day, TLC detection indicated completion of the reaction. The reaction solution was cooled to room temperature, and extracted by adding ethyl acetate and water. The organic phase was successively washed with water and saturated brine, and dried over anhydrous Na₂SO₄. The solvent was removed by rotatory evaporation. The residue was separated and purified by Prep-TLC, to provide the target compound as off-white solid (293 mg, 90%). LC/MS (ESI⁺) calcd for C₄₂H₆₀N₂O₆ ([M+H]⁺) *m*/*z* 688.4; found, 689.5.
In the following steps, the target compound was synthesized by a method similar to that of Example 342.
¹H NMR (400 MHz, Chloroform-*d*) *δ* 8.69 (d, *J* = 4.4 Hz, 1H), 8.08 (s, 2H), 7.59 (d, *J* = 8.8 Hz, 2H), 7.13 (dd, *J=* 13.4, 8.5 Hz, 4H), 6.94 (s, 1H), 6.81 (dd, *J=* 23.2, 8.3 Hz, 4H), 6.71 (d, *J=* 7.8 Hz, 1H), 5.11 (s, 2H), 4.92 (dd, *J=* 11.3, 5.0 Hz, 1H), 4.89 - 4.77 (m, 1H), 3.91 (t, *J*= 6.0 Hz, 2H), 3.44 (dt, *J* = 12.9, 5.8 Hz, 4H), 3.31 - 3.15 (m, 2H), 2.96 - 2.59 (m, 6H), 2.16 - 2.07 (m, 1H), 2.06 - 1.95 (m, 1H), 1.80 - 1.65 (m, 7H), 1.63 (s, 6H), 1.50 - 1.40 (m, 4H), 1.11 - 1.00 (m, 4H). LC/MS (ESI⁺) calcd for C₄₈H₅₆N₄O₈ ([M+H]⁺) *m*/*z* 816.4; found 817.3.

### Example 348: 4-((4-((8-(4-(2-(4-((2-acetylpyridin-4-yl)methoxy)phenyl)isopropan-2-yl)phenoxy)octyl)oxy)butyl)amino)-2-(2,6-dioxopiperidin-3-yl)isoindolin-1,3-dione

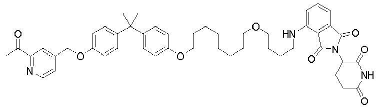

The target compound was synthesized by a method similar to that of Example 342. ¹H NMR (400 MHz, Chloroform-*d*) *δ* 8.69 (d, *J=* 4.9 Hz, 1H), 8.10 (d, *J=* 3.5 Hz, 2H), 7.58 (d, *J* = 4.0 Hz, 1H), 7.53 - 7.44 (m, 1H), 7.13 (dd, *J* = 14.4, 8.7 Hz, 4H), 7.08 (d, *J =* 7.1 Hz, 1H), 6.89 (d, *J* = 8.6 Hz, 1H), 6.81 (dd, *J* = 23.2, 8.7 Hz, 4H), 5.11 (s, 2H), 4.91 (dd, *J* = 12.0, 5.1 Hz, 1H), 3.91 (t, *J* = 6.5 Hz, 2H), 3.43 (dt, *J* = 19.7, 6.3 Hz, 4H), 3.36 - 3.21 (m, 2H), 2.96 - 2.62 (m, 7H), 2.16 - 2.09 (m, 1H), 1.94 - 1.66 (m, 9H), 1.63 (s, 6H), 1.60 - 1.53 (m, 3H), 1.49 - 1.39 (m, 4H). LC/MS (ESI⁺) calcd for C₄₈H₅₆N₄O₈([M+H]⁺) *m*/*z* 816.4; found 817.3.

### Example 349: 5-((4-((8-(4-(2-(4-((6-acetylpyridin-2-yl)methoxy)phenyl)isopropan-2-yl)phenoxy)octyl)oxy)tert-butyl)amino)-2-(2,6-dioxopiperidin-3-yl)isoindolin-1,3-dione

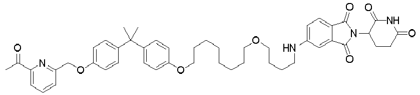

The target compound was synthesized by a method similar to that of Example 342. ¹H NMR (400 MHz, Chloroform-*d*) *δ* 8.11 (s, 1H), 7.96 (d, *J* = 7.6 Hz, 1H), 7.85 (t, *J* = 7.7 Hz, 1H), 7.71 (d, *J* = 7.6 Hz, 1H), 7.60 (d, *J* = 8.2 Hz, 1H), 7.14 (dd, *J=* 13.1, 8.8 Hz, 4H), 6.97 (s, 1H), 6.90 (d, *J* = 8.7 Hz, 2H), 6.79 (d, *J* = 8.7 Hz, 2H), 6.74 (d, *J* = 8.7 Hz, 1H), 5.23 (s, 2H), 4.92 (dd, *J* = 12.1, 4.8 Hz, 1H), 3.91 (t, *J* = 6.4 Hz, 2H), 3.45 (dt, *J* = 13.4, 6.3 Hz, 4H), 3.25 (t, *J=* 6.4 Hz, 2H), 2.92 - 2.62 (m, 7H), 2.15 - 2.08 (m, 1H), 2.03 - 1.98 (m, 1H), 1.81 - 1.67 (m, 8H), 1.63 (s, 6H), 1.61 - 1.53 (m, 3H), 1.48 - 1.38 (m, 4H). LC/MS (ESI⁺) calcd for C₄₈H₅₆N₄O₈ ([M+H]⁺) *m*/*z* 816.4; found 817.2.

### Example 350: 5-((3-(4-(3-(4-((2-acetylpyrimidin-4-yl)methoxy)phenyl)pentan-3-yl)phenoxy)propyl)amino)-2-(2,6-dioxopiperidin-3-yl)isoindolin-1,3-dione

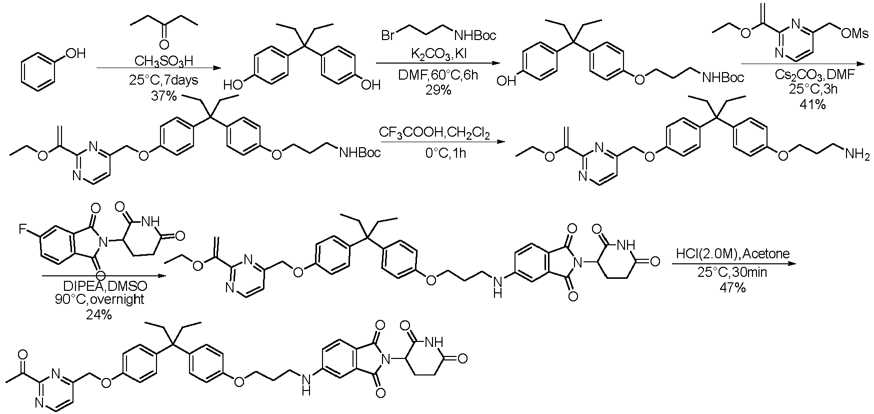

### Step 1: Synthesis of 4,4'-(pentan-3,3-diyl)bisphenol

3-pentanone (3.50 mL) and phenol (9.98 g, 106 mmol) were added into a 25 mL single-necked round-bottome flask, and then the mixture was stirred to dissolve and become clear, followed by addition of methanesulfonic acid (2.60 mL). After that, the system was moved into an oil bath at 25°C and reacted under stirring. After 7 days, LCMS indicated completion of the reaction, and then heating was removed. The system was cooled to room temperature, to which was added ethyl acetate, and then vigorously stirred. The organic phase was successively washed with water and saturated brine, and dried with anhydrous Na₂SO₄. The resultant solution was rotatory evaporated to remove the solvent and provide the crude product, which was triturated in dichloromethane/n-hexane (1:5) for purification, to provide the target product as white solid (3.12 g, 37%). LC/MS (ESI⁺) calcd for C₁₇H₂₀O₂ ([M-H]⁻) *m*/*z* 256.1; found, 255.2.

### Step 2: Synthesis of (3-(4-(3-(4-hydroxylphenyl)pentan-3-yl)phenoxy)propyl) carbamate

4,4'-(pentan-3,3-diyl)bisphenol (850 mg, 3.32 mmol), tert-butyl (3-bromopropyl) carbamate (712 mg, 2.99 mmol), K₂CO₃ (1.38 g, 9.96 mmol) and KI (496 mg, 2.99 mmol) were added into 15 mL of DMF, and then the solution was heated to 60 °C and reacted under stirring. After 6 h, the reaction was completed by TLC detection, and heating was removed. The reaction solution was cooled to room temperature, and extracted by adding ethyl acetate and water. The organic phase was successively washed with 0.01 N HCl solution, water, and saturated brine, and dried over anhydrous Na₂SO₄. The resultant solution was rotatory evaporated to remove the solvent and provide the crude product, which was separated and purified by column chromatography, to obtain the target product as off-white solid (400 mg, 29%). LC/MS (ESI⁺) calcd for C₂₅H₃₅NO₄ ([M-H]⁻) *m*/*z* 413.3; found, 412.2.

### Step 3: Synthesis of tert-butyl (3-(4-(3-(4-((2-(1-ethoxyvinyl)pyrimidin-4-yl)methoxy) phenyl)pentan-3-yl)phenoxy)propyl)carbamate

tert-butyl (3-(4-(3-(4-hydroxylphenyl)pentan-3-yl)phenoxy)propyl)carbamate (400 mg, 0.97 mmol), (2-(1-ethoxyvinyl)pyrimidin-4-yl)methyl methanesulfonate (276 mg, 1.07 mmol) and cesium carbonate (349 mg, 1.07 mmol) were added into 10 mL of DMF, and then the solution was heated to 25 °C, and reacted under stirring. After 3 h, TLC detection indicated the starting materials disappeared, and heating was removed. The reaction solution was cooled to room temperature, and extracted by adding ethyl acetate and water. The organic phase was successively washed with water and saturated brine, and dried over anhydrous Na₂SO₄. The resultant solution was rotatory evaporated to remove the solvent and provide the crude product, which was separated and purified by Prep-TLC, to provide the target compound as off-white solid (230 mg, 41%). LC/MS (ESI⁺) calcd for C₃₄H₄₅N₃O₅ ([M+H]⁺) *m*/*z* 575.3; found 576.3.

### Step 4: Synthesis of 3-(4-(3-(4-((2-(1-ethoxyvinyl)pyrimidin-4-yl)methoxy)phenyl) pentan-3-yl)phenoxy)propan-1-amine

Compound tert-butyl (3-(4-(3-(4-((2-(1-ethoxyvinyl)pyrimidin-4-yl)methoxy) phenyl)pentan-3-yl)phenoxy)propyl)carbamate (230 mg, 0.40 mmol) was dissolved in 3 mL of dichloromethane, and then the system was transferred into an ice water bath for cooling under stirring. After 15 min, trifluoroacetic acid (0.75 mL) was added, and subsequently, the mixture was allowed to react under stirring. After 1 h, TLC detection indicated completion of the reaction. The pH of the system was adjusted to about 8 by slowly adding saturated Na₂CO₃ solution dropwise in the ice-water bath, and then the extraction procedure was performed by adding the mixed solvent of dichloromethane and methanol (10:1). The organic phase was dried with anhydrous Na₂SO₄. The solvent was removed by rotatory evaporation, to provide the target compound as off-white solid (30 mg), which was directly used in the next step without further purification.

### Step 5: Synthesis of 2-(2,6-dioxopiperidin-3-yl)-5-((3-(4-(3-(4-((2-(1-ethoxyvinyl) pyrimidin-4-yl)methoxy)phenyl)pentan-3-yl)phenoxy)propyl)amino)isoindolin-1,3-dione

3-(4-(3-(4-((2-(1-ethoxyvinyl)pyrimidin-4-yl)methoxy)phenyl)pentan-3-yl) phenoxy)propan-1-amine (30 mg, 0.06 mmol), 2-(2,6-dioxopiperidin-3-yl)-5-fluoroisoindole-1,3-dione (19 mg, 0.07 mmol) and diisopropylethylamine (23 mg, 0.18 mmol) were added into 3 mL of DMSO, and then the system was placed in an oil bath at 90 °C and reacted overnight under stirring. The next day, the reaction solution was cooled to room temperature, and extracted by adding ethyl acetate and water. The organic layers were successively washed with saturated NH₄Cl solution, water, and saturated brine, and then dried with anhydrous Na₂SO₄. The solvent was removed by rotatory evaporation. The residue was separated and purified by Prep-TLC, to provide the target compound as light yellow solid (11 mg, 24%). LC/MS (ESI⁺) calcd for C₄₂H₄₅N₅O₇ ([M+H]⁺) *m*/*z* 731.3; found 732.3.

### Step 6: Synthesis of 5-((3-(4-(3-(4-((2-acetylpyrimidin-4-yl)methoxy)phenyl)pentan-3-yl)phenoxy)propyl)amino)-2-(2,6-dioxopiperidin-3-yl)isoindolin-1,3-dione

2-(2,6-dioxopiperidin-3-yl)-5-((3-(4-(3-(4-((2-(1-ethoxyvinyl)pyrimidin-4-yl)methoxy)phenyl)pentan-3-yl)phenoxy)propyl)amino)isoindolin-1,3-dione (11 mg, 0.02 mmol) was dissolved in 3 mL of acetone, to which was added HCl (50 µL, 2.0 M), and then the solution was reacted at room temperature under stirring. After 30 min, TLC detection indicated the starting materials disappeared. The solvent was rotatory evaporated at low temperature, followed by addition of dichloromethane and water. The pH of the system was adjusted to about 8 with saturated NaHCO₃ solution, and then the extraction procedure was performed. The organic phase was successively washed with water and saturated brine, and dried with anhydrous Na₂SO₄. The resultant solution was rotatory evaporated to remove the solvent and provide the crude product, which was separated and purified by Prep-TLC, to provide the target compound as light yellow solid (5 mg, 47%). ¹H NMR (400 MHz, Chloroform-*d*) *δ* 8.92 (d, *J =* 5.1 Hz, 1H), 8.02 (s, 1H), 7.75 (d, *J* = 5.0 Hz, 1H), 7.60 (d, *J* = 8.3 Hz, 1H), 7.10 (t, *J* = 8.3 Hz, 4H), 6.99 (d, *J* = 2.0 Hz, 1H), 6.86 (d, *J* = 8.8 Hz, 2H), 6.80 (d, *J* = 8.8 Hz, 2H), 6.78 - 6.74 (m, 1H), 5.25 (s, 2H), 4.93 (dd, *J* = 12.3, 5.3 Hz, 1H), 4.09 (t, *J* = 5.5 Hz, 2H), 3.47 (t, *J* = 6.6 Hz, 2H), 2.93 - 2.82 (m, 2H), 2.80 (s, 3H), 2.78 - 2.74 (m, 1H), 2.16 - 2.12 (m, 2H), 2.04 (ddd, *J=* 15.5, 7.6, 4.9 Hz, 6H), 0.61 (t, *J=* 7.3 Hz, 6H). LC/MS (ESI⁺) calcd for C₄₀H₄₁N₅O₇ ([M+H]⁺) *m*/*z* 703.3; found, 704.3.

### Example 351: 5-((3-(4-(1-(4-((2-acetylpyrimidin-4-yl)methoxy)phenyl)cyclobutyl) phenoxy)propyl)amino)-2-(2,6-dioxopiperidin-3-yl)isoindolin-1,3-dione

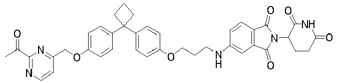

The target compound was synthesized by a method similar to that of Example 350. ¹H NMR (400 MHz, Chloroform-*d*) *δ* 8.90 (d, *J* = 4.8 Hz, 1H), 8.10 (s, 1H), 7.71 (d, *J* = 4.6 Hz, 1H), 7.60 (d, *J* = 8.2 Hz, 1H), 7.22 (dd, *J* = 7.9, 5.2 Hz, 4H), 7.01 (s, 1H), 6.85 (dd, *J* = 18.7, 8.5 Hz, 4H), 6.78 (d, *J* = 7.8 Hz, 1H), 5.23 (s, 2H), 4.92 (dd, *J* = 11.6, 4.9 Hz, 1H), 4.07 (t, *J* = 5.0 Hz, 2H), 3.45 (t, *J* = 6.1 Hz, 2H), 2.99 - 2.81 (m, 3H), 2.79 (s, 3H), 2.78 - 2.59 (m, 6H), 2.15 - 2.09 (m, 2H), 1.95 (p, *J* = 7.4 Hz, 2H). LC/MS (ESI⁺) calcd for C₃₉H₃₇N₅O₇([M+H]⁺) *m*/*z* 687.3; found 688.3.

### Example 352: 5-((4-(1-(4-(3-((2-(2,6-dioxopiperidin-3-yl)-1,3-dioxoisoindolin-5-yl) amino)propoxy)phenyl)cyclobutyl)phenoxy)methyl)-1,2,4-oxadiazol-3-carbonylamine

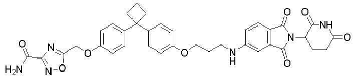

The target compound was synthesized by a method similar to that of Example 331. ¹H NMR (400 MHz, Chloroform-*d*) δ 7.58 (d, *J* = 8.3 Hz, 1H), 7.22 (t, *J* = 8.9 Hz, 4H), 6.97 (d, *J* = 2.0 Hz, 1H), 6.89 (d, *J* = 8.8 Hz, 2H), 6.84 (d, *J* = 8.7 Hz, 2H), 6.74 (dd, *J* = 8.4, 2.1 Hz, 1H), 5.32 (s, 2H), 4.96 - 4.87 (m, 1H), 4.07 (t, *J* = 5.6 Hz, 2H), 3.43 (t, *J* = 6.5 Hz, 2H), 3.39 (dt, *J* = 3.2, 1.6 Hz, 1H), 2.80 (dddd, *J* = 29.3, 14.2, 7.3, 3.3 Hz, 3H), 2.68 (t, *J* = 7.3 Hz, 4H), 2.11 (p, *J* = 6.1 Hz, 3H), 1.95 (p, *J =* 7.5 Hz, 2H). LC/MS (ESI⁺) calcd for C₃₆H₃₄N₆O₈ ([M+H]⁺) *m*/*z* 678.2; found 679.3.

### Example 353: 5-(((1r,4r)-4-(4-(2-(4-((2-acetylpyrimidin-4-yl)methoxy)phenyl) isopropan-2-yl)phenoxy)cyclohexyl)amino)-2-(2,6-dioxopiperidin-3-yl)isoindolin-1,3-dione

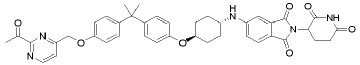

The target compound was synthesized by a method similar to that of Example 350. ¹H NMR (400 MHz, Chloroform-*d*) δ 8.92 (d, *J* = 5.0 Hz, 1H), 8.09 (s, 1H), 7.74 (d, *J* = 4.8 Hz, 1H), 7.63 (d, *J* = 8.2 Hz, 1H), 7.15 (dd, *J* = 20.5, 8.7 Hz, 4H), 7.01 (s, 1H), 6.87 (d, *J* = 8.8 Hz, 2H), 6.80 (d, *J* = 8.7 Hz, 3H), 5.25 (s, 2H), 4.94 (dd, *J* = 12.1, 5.1 Hz, 1H), 4.22 (t, *J* = 9.8 Hz, 1H), 3.48 (t, *J* = 9.7 Hz, 1H), 2.94 - 2.68 (m, 7H), 2.30 - 2.08 (m, 6H), 1.64 (s, 9H). LC/MS (ESI⁺) calcd for C₄₁H₄₁N₅O₇ ([M+H]⁺) m/z 715.3; found, 716.2.

### Example 354: 2-(2,6-dioxopiperidin-3-yl)-5-(((1r,4r)-4-(4-(2-(4-((2-(1-hydroxylethyl)pyrimidin-4-yl)methoxy)phenyl)isopropan-2-yl)phenoxy) cyclohexyl)amino)isoindolin-1,3-dione

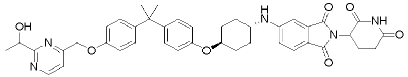

The target compound was synthesized by a method similar to that of Example 342. ¹H NMR (400 MHz, Chloroform-*d*) *δ* 8.76 (br, 1H), 8.23 (s, 1H), 7.61 (d, *J* = 7.4 Hz, 1H), 7.51 (s, 1H), 7.15 (dd, *J* = 18.5, 7.9 Hz, 4H), 6.97 (s, 1H), 6.86 (d, *J* = 7.6 Hz, 2H), 6.81 (d, *J=* 8.2 Hz, 2H), 6.74 (d, *J* = 7.2 Hz, 1H), 5.16 (s, 2H), 4.95 (td, *J* = 12.5, 11.5, 3.8 Hz, 2H), 4.22 (br, 1H), 3.49 (br, 1H), 2.82 (dt, *J =* 44.1, 16.4 Hz, 4H), 2.17 (dd, *J* = 26.2, 5.9 Hz, 6H), 1.62 (d, *J* = 14.7 Hz, 13H). LC/MS (ESI⁺) calcd for C₄₁H₄₃N₅O₇ ([M+H]⁺) *m*/*z* 717.3; found, 718.2.

### Example 355: Synthesis of 5-(((1r,3r)-3-(4-(3-(4-((2-(2-oxa-6-azaspiro[3.3] heptane-6-yl)pyrimidin-4-yl)methoxy)phenyl)oxetane-3-yl)phenoxy)cyclobutyl) amino)-2-(2,6-dioxopiperidin-3-yl)isoindolin-1,3-dione

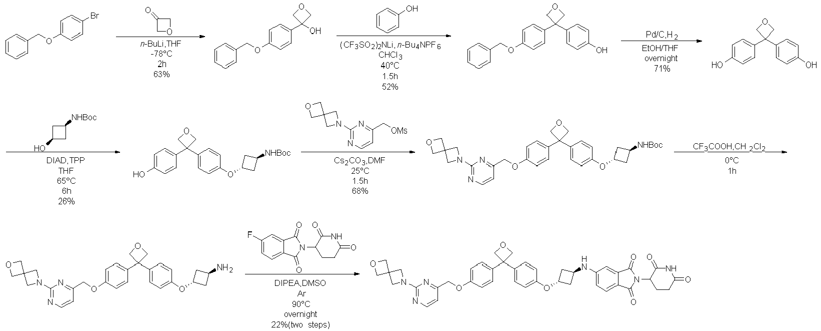

### Step 1: Synthesis of 3-(4-(benzyloxy)phenyl)oxetane-3-ol

1-(Benzyloxy)-4-bromobenzene (8500 mg, 32.30 mmol) was weighed and placed in a three-necked flask, to which was added THF (90 mL), and then the mixture was stirred to dissolve and become clear. The system was vacuumized and purged with argon, that was repeated three times. Subsequently, the system was moved to a dry ice ethanol bath, and cooled under stirring. When the internal temperature was reduced to about -78 °C, to the system, was slowly added n-butyl lithium (13 mL, 32.30 mmol, 2.5 M) dropwise, and the dropping rate was controlled to ensure that the internal temperature did not exceed -70 °C. After that, the system was allowed to react at -78 °C. After 30 min, to the system was slowly added 10 mL solution of oxetan-3-one (1800 mg, 24.85 mmol) in dry THF dropwise. Subsequently, the system was allowed to further react at - 78 °C for 1 h. Then, the system was naturally warmed to room temperature. TLC detection indicated completion of the reaction. To the system, was added saturated NH₄Cl solution at low temperature to quench the reaction. The reaction was extracted by adding ethyl acetate and water. The organic phase was successively washed with water and saturated brine, and dried with anhydrous Na₂SO₄. The resultant solution was rotatory evaporated to remove the solvent and provide the crude product, which was triturated in n-hexane/dichloromethane (5:1) for purification, to obtain the target product as off-white solids (4000 mg, 63%). LC/MS (ESI⁺) calcd for C₁₆H₁₆O₃ ([M+23]⁺) *m*/*z* 256.1; found 279.3.

### Step 2: Synthesis of 4-(3-(4-(benzyloxy)phenyl)oxetane-3-yl)phenol

3-(4-(benzyloxy)phenyl)oxetane-3-ol (1920 mg, 7.50 mmol), phenol (3529 mg, 37.50 mmol), bistrifluoromethanesulfonimide lithium salt (238 mg, 0.83 mmol) and tetrabutylammonium hexafluorophosphate (163 mg, 0.42 mmol) were weighed and placed in a single-necked round-bottome flask, to which was added chloroform (15 mL), and then the mixture was heated to 40 °C and reacted under stirring. After 1.5 h, TLC detection indicated the starting materials disappeared, and heating was removed. The reaction solution was cooled to room temperature, and extracted by adding chloroform and water. The organic phase was successively washed with water and saturated brine, and then dried over anhydrous Na₂SO₄. The resultant solution was rotatory evaporated to remove the solvent and provide the crude product, which was separated and purified by column chromatography, to provide the target product as white solid (1300 mg, 52%). LC/MS (ESI⁺) calcd for C₂₂H₂₀O₃ ([M-H]⁻) *m*/*z* 332.1; found 331.1.

### Step 3: Synthesis of 4,4'-(oxetane-3,3-diyl)bisphenol

4-(3-(4-(benzyloxy)phenyl)oxetane-3-yl)phenol (1300 mg, 3.91 mmol) was weighed and dissolved in a mixed solvent of ethanol (12 mL) and THF (6 mL), to which was added Pd/C (260 mg), and then the system was vacuumed and purged with hydrogen, that was repeated several times, to ensure the hydrogen atmosphere in the system. Subsequently, the system was stirred and reacted at room temperature. The next day, TLC indicated disappearance of starting materials. The reaction was stopped, and the system was subjected to suction filtration. The filter cake was washed with a small amount of ethanol for a few times, and then the filtrate was combined. The solvent was removed by rotatory evaporation. To the residue, were added ethyl acetate and water, followed by extraction. The organic layers were combined, successively washed with water and saturated brine, and then dried over anhydrous Na₂SO₄. The resultant solution was rotatory evaporated to remove the solvent and provide the crude product, which was separated and purified by column chromatography, to provide the target product as white solid (670 mg, 71%). LC/MS (ESI⁺) calcd for C₁₅H₁₄O₃ ([M-H]⁻) *m*/*z* 242.1; found 241.1.

### Step 4: Synthesis of tert-butyl ((1r,3r)-3-(4-(3-(4-hydroxylphenyl)oxetane-3-yl)phenoxy)cyclobutyl)carbamate

4,4'-(oxetane-3,3-diyl)bisphenol (300 mg, 1.24 mmol), tert-butyl ((1*s*,3*s*)-3-hydroxylcyclobutyl)carbamate (178 mg, 0.95 mmol), and triphenylphosphine (325 mg, 1.24 mmol) were weighed and added into a three-necked round-bottom flask, to which was added dry THF (10 mL), and then the mixture was stirred to dissolve and become clear. The system was transferred into an ice water bath and cooled under stirring. After 15 min, to the system, was added 5 mL solution of DIAD (250 mg, 1.24 mmol) in THF dropwise, and then, the reaction solution was heated to 65 °C and reacted under stirring. After 6 h, TLC detection indicated completion of the reaction, and heating was removed. The reaction solution was cooled to room temperature, and extracted by adding ethyl acetate and water. The organic layer was successively washed with water and saturated brine, and then dried over anhydrous Na₂SO₄. The solvent was removed by rotatory evaporation. The residue was separated and purified by column chromatography, to provide the target product as white solid (100 mg, 26%). LC/MS (ESI⁺) calcd for C₂₄H₂₉NO₅ ([M-100]⁺) *m*/*z* 411.2; found 311.1.

### Step 5: Synthesis of tert-butyl ((1r,3r)-3-(4-(3-(4-((2-(2-oxa-6-azaspiro[3.3]heptane-6-yl)pyrimidin-4-yl)methoxy)phenyl)oxetane-3-yl)phenoxy)cyclobutyl)carbamate

tert-butyl ((1*r*,3*r*)-3-(4-(3-(4-hydroxylphenyl)oxetane-3-yl)phenoxy)cyclobutyl) carbamate (100 mg, 0.24 mmol) and cesium carbonate (117 mg, 0.36 mmol) were added into 5 mL of DMF, and then the mixture was stirred at room temperature. After 5 min, (2-(2-oxa-6-azaspiro[3.3]heptane-6-yl)pyrimidin-4-yl)methyl methanesulfonate (83 mg, 0.29 mmol) was added, and then the reaction solution was heated to 25 °C and reacted under stirring. After 1.5 h, TLC detection indicated completion of the reaction, and heating was removed. The reaction solution was extracted by adding ethyl acetate and water. The organic phase was successively washed with water and saturated brine, and then dried over anhydrous Na₂SO₄. The resultant solution was rotatory evaporated to remove the solvent and provide the crude product, which was separated and purified by Prep-TLC, to provide the target compound as off-white solid (100 mg, 68%). LC/MS (ESI⁺) calcd for C₄₀H₄₁N₅O₇ ([M+H]⁺) *m*/*z* 600.3; found 601.3.

### Step 6: Synthesis of (1r,3r)-3-(4-(3-(4-((2-(2-oxa-6-azaspiro[3.3]heptane-6-yl) pyrimidin-4-yl)methoxy)phenyl)oxetane-3-yl)phenoxy)cyclobutyl-1-amine

tert-butyl ((1*r*,3*r*)-3-(4-(3-(4-((2-(2-oxa-6-azaspiro[3.3]heptane-6-yl)pyrimidin-4-yl)methoxy)phenyl)oxetane-3-yl)phenoxy)cyclobutyl)carbamate (100 mg, 0.17 mmol) was dissolved in 2 mL of dichloromethane, and then transferred into an ice water bath for cooling under stirring. After 15 min, trifluoroacetic acid (0.5 mL) was added, and then the system was allowed to react under stirring in the ice water bath. After 1 h, TLC detection indicated completion of the reaction. To the system in the ice water bath, was slowly added saturated Na₂CO₃ solution dropwise, to adjust pH to be about 8.0, and then the mixed solvent of dichloromethane and methanol (10:1) was added to carry out the extraction procedure. The organic phase was dried with anhydrous Na₂SO₄. The solvent was removed by rotatory evaporation, to obtain the target product as off-white solids (80 mg), which was directly used in the next step without further purification.

### Step 7: Synthesis of 5-(((1r,3r)-3-(4-(3-(4-((2-(2-oxa-6-azaspiro[3.3]heptane-6-yl)pyrimidin-4-yl)methoxy)phenyl)oxetane-3-yl)phenoxy)cyclobutyl)amino)-2-(2,6-dioxopiperidin-3-yl)isoindolin-1,3-dione

(1r,3r)-3-(4-(3-(4-((2-(2-oxa-6-azaspiro[3.3]heptane-6-yl)pyrimidin-4-yl) methoxy)phenyl)oxetane-3-yl)phenoxy)cyclobutyl-1-amine (80 mg, 0.16 mmol), 2-(2,6-dioxopiperidin-3-yl)-5-fluoroisoindole-1,3-dione (66 mg, 0.24 mmol), and diisopropylethylamine (62 mg, 0.48 mmol) were added into 5 mL of DMSO, and then the system was placed in an oil bath at 90 °C, and reacted overnight under stirring. The next day, the reaction solution was cooled to room temperature, and extracted by adding ethyl acetate and water. The organic layers were successively washed with saturated NH₄Cl solution, water, and saturated brine, and then dried with anhydrous Na₂SO₄. The solvent was removed by rotatory evaporation. The residue was separated and purified by Prep-TLC, to provide the target compound as light yellow solid (28 mg, 22%). ¹H NMR (400 MHz, Chloroform-*d*) δ 8.31 (d, *J* = 5.0 Hz, 1H), 8.18 (s, 1H), 7.63 (d, *J* = 8.2 Hz, 1H), 7.12 (t, *J* = 9.1 Hz, 4H), 6.95 - 6.87 (m, 3H), 6.85 - 6.80 (m, 1H), 6.77 (d, *J* = 8.7 Hz, 2H), 6.71 (dd, *J* = 8.2, 1.9 Hz, 1H), 5.18 (d, *J* = 5.8 Hz, 4H), 4.99 (s, 2H), 4.96 - 4.88 (m, 2H), 4.87 (s, 4H), 4.82 (s, 1H), 4.34 (s, 4H), 4.24 (br, 1H), 2.94 - 2.65 (m, 5H), 2.42 (dt, *J* = 12.7, 5.7 Hz, 2H), 2.16 - 2.09 (m, 1H). LC/MS (ESI⁺) calcd for C₄₂H₄₀N₆O₈ ([M+H]⁺) *m*/*z* 756.3; found, 757.3.

### Example 356: 5-((3-(4-(1-(4-((2-(2-oxa-6-azaspiro[3.3]heptane-6-yl)pyrimidin-4-yl)methoxy)phenyl)cyclobutyl)phenoxy)cyclobutyl)amino)-2-(2,6-dioxopiperidin-3-yl)isoindolin-1,3-dione

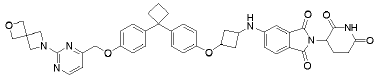

The target compound was synthesized by a method similar to that of Example 355. ¹H NMR (400 MHz, Chloroform-*d*) δ 8.29 (d, *J* = 5.2 Hz, 1H), 8.11 (d, *J* = 13.1 Hz, 1H), 7.62 (dd, *J* = 8.3, 2.8 Hz, 1H), 7.18 (ddd, *J* = 9.1, 5.6, 2.7 Hz, 4H), 6.89 (dd, *J* = 9.5, 2.0 Hz, 1H), 6.87 - 6.77 (m, 3H), 6.71 (ddt, *J* = 8.1, 5.8, 3.5 Hz, 3H), 4.94 (s, 2H), 4.86 (s, 4H), 4.49 (p, *J* = 6.9 Hz, 1H), 4.34 (s, 4H), 3.76 (dq, *J* = 13.0, 7.1, 6.5 Hz, 1H), 3.11 (dtd, *J* = 9.6, 6.8, 2.9 Hz, 2H), 2.93 - 2.70 (m, 3H), 2.66 (t, *J* = 7.5 Hz, 4H), 2.44 - 2.32 (m, 1H), 2.19 - 1.98 (m, 4H), 1.94 (p, *J* = 7.7 Hz, 2H). LC/MS (ESI⁺) calcd for C₄₃H₄₂N₆O₇ ([M+H]⁺) *m*/*z* 754.3; found, 755.3.

### Example 357: 2-(2,6-dioxopiperidin-3-yl)-5-(((1r,3r)-3-(4-(1-(4-((2-methoxypyrimidin-4-yl)methoxy)phenyl)cyclobutyl)phenoxy)cyclobutyl)amino) isoindolin-1,3-dione

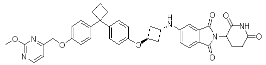

The target compound was synthesized by a method similar to that of Example 355. ¹H NMR (400 MHz, Chloroform-*d*) *δ* 8.53 (s, 1H), 8.04 (s, 1H), 7.63 (d, *J* = 8.2 Hz, 1H), 7.24 - 7.14 (m, 5H), 6.93 - 6.81 (m, 3H), 6.70 (d, *J* = 8.3 Hz, 3H), 5.06 (s, 2H), 4.93 (dd, *J* = 12.0, 5.2 Hz, 1H), 4.84 (br, 1H), 4.21 (br, 1H), 4.05 (s, 3H), 2.93 - 2.71 (m, 4H), 2.67 (t, *J* = 7.5 Hz, 6H), 2.39 (dt, *J* = 12.2, 6.2 Hz, 2H), 1.95 (dt, *J* = 15.0, 7.7 Hz, 3H). LC/MS (ESI⁺) calcd for C₃₉H₃₇N₅O₇ ([M+H]⁺) *m*/*z* 687.3; found, 688.2.

### Example 358: 5-(((1r,3r)-3-(4-(1-(4-((2-(2-oxa-6-azaspiro[3.3]heptane-6-yl) pyrimidin-4-yl)methoxy)phenyl)cyclobutyl)phenoxy)cyclobutyl)amino)-2-(2,6-dioxopiperidin-3-yl)isoindolin-1,3-dione

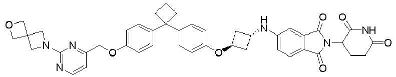

The target compound was synthesized by a method similar to that of Example 355. ¹H NMR (400 MHz, Chloroform-*d*) *δ* 8.29 (d, *J* = 4.8 Hz, 1H), 8.15 (s, 1H), 7.62 (d, *J* = 8.3 Hz, 1H), 7.19 (t, *J* = 8.0 Hz, 4H), 6.88 (d, *J* = 1.9 Hz, 1H), 6.84 (d, *J* = 8.5 Hz, 3H), 6.74 - 6.66 (m, 3H), 5.00 - 4.89 (m, 3H), 4.86 (s, 4H), 4.84 - 4.76 (m, 1H), 4.37 (s, 4H), 4.21 (br, 1H), 2.93 - 2.70 (m, 4H), 2.67 (t, *J* = 7.5 Hz, 6H), 2.38 (dt, *J* = 13.9, 6.4 Hz, 2H), 2.16 - 2.08 (m, 1H), 1.95 (p, *J* = 7.6 Hz, 2H). LC/MS (ESI⁺) calcd for C₄₃H₄₂N₆O₇ ([M+H]⁺) *m*/*z* 754.3; found, 755.3.

### Example 359: 5-(((1s,3s)-3-(4-(1-(4-((2-(2-oxa-6-azaspiro[3.3]heptane-6-yl) pyrimidin-4-yl)methoxy)phenyl)cyclobutyl)phenoxy)cyclobutyl)amino)-2-(2,6-dioxopiperidin-3-yl)isoindolin-1,3-dione

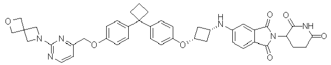

The target compound was synthesized by a method similar to that of Example 355. ¹H NMR (400 MHz, Chloroform-*d*) *δ* 8.30 (d, *J=* 4.2 Hz, 1H), 8.14 (s, 1H), 7.62 (d, *J=* 8.0 Hz, 1H), 7.19 (dd, *J=* 6.9, 4.0 Hz, 4H), 6.90 (s, 1H), 6.84 (d, *J=* 8.2 Hz, 2H), 6.81 - 6.77 (m, 1H), 6.71 (dd, *J* = 9.1, 4.4 Hz, 3H), 5.00 - 4.89 (m, 2H), 4.86 (s, 4H), 4.74 (d, *J* = 5.2 Hz, 1H), 4.53 - 4.43 (m, 1H), 4.31 (s, 4H), 3.84 - 3.68 (m, 1H), 3.11 (br, 2H), 2.94 - 2.70 (m, 3H), 2.66 (t, *J* = 7.2 Hz, 4H), 2.18 - 1.99 (m, 4H), 1.99 - 1.86 (m, 2H). LC/MS (ESI⁺) calcd for C₄₃H₄₂N₆O₇ ([M+H]⁺) *m*/*z* 754.3; found, 755.3.

### Example 360: 5-(((1r,3r)-3-(4-(1-(4-((2-(2-oxa-6-azaspiro[3.3]heptane-6-yl) pyrimidin-4-yl)methoxy)phenyl)cyclopentyl)phenoxy)cyclobutyl)amino)-2-(2,6-dioxopiperidin-3-yl)isoindolin-1,3-dione

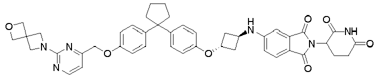

The target compound was synthesized by a method similar to that of Example 355. ¹H NMR (400 MHz, Chloroform-*d*) *δ* 8.32 (s, 1H), 8.18 (s, 1H), 7.62 (d, *J=* 7.3 Hz, 1H), 7.23 - 7.07 (m, 4H), 6.95 - 6.75 (m, 4H), 6.68 (t, *J* = 9.5 Hz, 3H), 5.09 - 4.65 (m, 9H), 4.52 - 4.27 (m, 3H), 4.27 - 4.13 (m, 1H), 2.94 - 2.61 (m, 5H), 2.47 - 2.31 (m, 2H), 2.22 (br, 4H), 2.16 - 2.08 (m, 1H), 2.04 - 1.98 (m, 1H), 1.69 (br, 4H). LC/MS (ESI⁺) calcd for C₄₄H₄₄N₆O₇ ([M+H]⁺) *m*/*z* 768.3; found, 769.3.

### Example 361: 5-(((1s,3s)-3-(4-(1-(4-((2-(2-oxa-6-azaspiro[3.3]heptane-6-yl) pyrimidin-4-yl)methoxy)phenyl)cyclopentyl)phenoxy)cyclobutyl)amino)-2-(2,6-dioxopiperidin-3-yl)isoindolin-1,3-dione

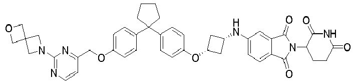

The target compound was synthesized by a method similar to that of Example 355. ¹H NMR (400 MHz, Chloroform-*d*) *δ* 8.29 (d, *J=* 4.8 Hz, 1H), 8.09 (s, 1H), 7.62 (d, *J=* 8.3 Hz, 1H), 7.21 - 7.11 (m, 4H), 6.91 (d, *J* = 1.8 Hz, 1H), 6.83 (d, *J* = 4.7 Hz, 1H), 6.80 (d, *J* = 8.7 Hz, 2H), 6.70 (t, *J=* 8.6 Hz, 3H), 4.94 (s, 2H), 4.90 (dd, *J=* 8.1, 5.3 Hz, 1H), 4.86 (s, 4H), 4.73 (d, *J=* 5.1 Hz, 1H), 4.49 (p, *J* = 7.0 Hz, 1H), 4.36 (s, 4H), 3.82 - 3.70 (m, 1H), 3.11 (dtd, *J* = 9.7, 6.9, 2.7 Hz, 2H), 2.93 - 2.67 (m, 3H), 2.22 (br, 4H), 2.16 - 1.97 (m, 3H), 1.68 (br, 4H). LC/MS (ESI⁺) calcd for C₄₄H₄₄N₆O₇ ([M+H]⁺) *m*/*z* 768.3; found, 769.3.

### Example 362: 5-(((1r,3r)-3-(4-((4-((2-(2-oxa-6-azaspiro[3.3]heptane-6-yl) pyrimidin-4-yl)methoxy)phenyl)sulfonyl)phenoxy)cyclobutyl)amino)-2-(2,6-dioxopiperidin-3-yl)isoindolin-1,3-dione

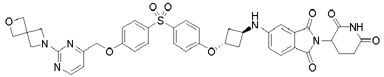

The target compound was synthesized by a method similar to that of Example 355. ¹H NMR (400 MHz, Chloroform-*d*) δ 8.30 (d, *J* = 5.3 Hz, 1H), 8.15 (s, 1H), 7.85 (dd, *J* = 8.8, 6.8 Hz, 4H), 7.63 (d, *J* = 8.3 Hz, 1H), 7.00 (dd, *J* = 7.8, 3.8 Hz, 2H), 6.89 - 6.82 (m, 3H), 6.76 (d, *J* = 5.6 Hz, 1H), 6.71 (dd, *J=* 8.3, 2.1 Hz, 1H), 5.03 (s, 2H), 4.97 - 4.88 (m, 2H), 4.86 (s, 4H), 4.79 (s, 1H), 4.38 (s, 4H), 4.28 - 4.20 (m, 1H), 2.93 - 2.75 (m, 3H), 2.75 - 2.66 (m, 2H), 2.49 - 2.38 (m, 2H), 2.16 - 2.09 (m, 1H). LC/MS (ESI⁺) calcd for C₃₉H₃₆N₆O₉S ([M+H]⁺) *m*/*z* 764.2; found, 765.2.

### Example 363: 5-(((1s,3s)-3-(4-((4-((2-(2-oxa-6-azaspiro[3.3]heptane-6-yl) pyrimidin-4-yl)methoxy)phenyl)sulfonyl)phenoxy)cyclobutyl)amino)-2-(2,6-dioxopiperidin-3-yl)isoindolin-1,3-dione

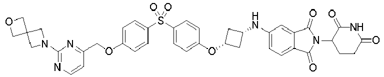

The target compound was synthesized by a method similar to that of Example 355. ¹H NMR (400 MHz, Chloroform-*d*) *δ* 8.35 (s, 1H), 8.30 (d, *J* = 5.1 Hz, 1H), 7.91 - 7.79 (m, 4H), 7.61 (d, *J* = 8.3 Hz, 1H), 7.07 - 6.95 (m, 2H), 6.89 (d, *J* = 2.0 Hz, 1H), 6.88 - 6.81 (m, 2H), 6.74 - 6.67 (m, 2H), 4.99 (s, 2H), 4.93 (dd, *J* = 12.2, 5.3 Hz, 2H), 4.86 (s, 4H), 4.57 (p, *J=* 6.8 Hz, 1H), 4.29 (s, 4H), 3.79 (h, *J=* 7.8 Hz, 1H), 3.15 (dtd, *J* = 9.8, 6.8, 3.0 Hz, 2H), 2.94 - 2.66 (m, 3H), 2.10 (dddq, *J* = 16.8, 10.0, 6.7, 2.9 Hz, 3H). LC/MS (ESI⁺) calcd for C₃₉H₃₆N₆O₉S ([M+H]⁺) *m*/*z* 764.2; found, 765.2.

### Example 364: Synthesis of 5-(((1r,3r)-3-(4-(2-(4-((2-(2-oxa-6-azaspiro[3.3] heptane-6-yl)pyrimidin-4-yl)methoxy)phenyl)isopropan-2-yl)-2-fluorophenoxy) cyclobutyl)amino)-2-(2,6-dioxopiperidin-3-yl)isoindolin-1,3-dione

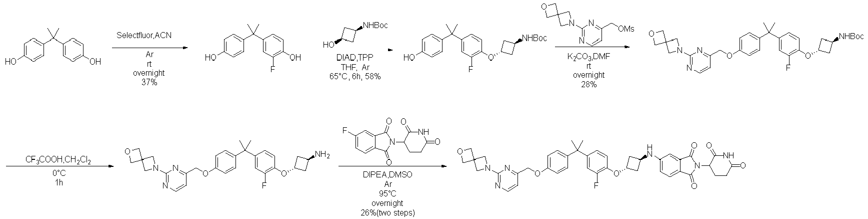

### Step 1: Synthesis of 2-fluoro-4-(2-(4-hydroxylphenyl)isopropan-2-yl)phenol

Bisphenol A (500 mg, 2.19 mmol) was weighed and placed in a single-necked round-bottome flask, to which was added dry acetonitrile (12 mL), and then the mixture was stirred to dissolve and become clear. Subsequently, selectfluor (737 mg, 2.08 mmol) was added. The system was vacuumized and purged with argon, that was repeated three times, and then the system was allowed to react overnight at room temperature. The next day, the reaction was detected by LCMS. The reaction solution was extracted by adding ethyl acetate and water. The organic phase was successively washed with water and saturated brine, and then dried over anhydrous Na₂SO₄. The resultant solution was rotatory evaporated to remove the solvent and provide the crude product, which was separated and purified by Prep-TLC, to provide the target compound as off-white solid (200 mg, 37%). LC/MS (ESI⁺) calcd for C₁₆H₁₆O₃ ([M-H]⁻) *m*/*z* 246.1; found, 245.1.

The target compound was synthesized by a method similar to that of Example 355. ¹H NMR (400 MHz, Chloroform-*d*) δ 8.31 (d, *J* = 4.9 Hz, 1H), 8.20 (s, 1H), 7.63 (d, *J* = 8.2 Hz, 1H), 7.13 (d, *J* = 8.7 Hz, 2H), 6.95 (dd, *J* = 13.2, 2.1 Hz, 1H), 6.91 - 6.78 (m, 7H), 6.70 (dd, *J* = 8.3, 1.8 Hz, 1H), 6.65 (t, *J=* 8.6 Hz, 1H), 4.96 (s, 2H), 4.95 - 4.80 (m, 6H), 4.33 (s, 4H), 2.94 - 2.67 (m, 4H), 2.45 - 2.34 (m, 2H), 2.16 - 2.09 (m, 1H), 2.04 - 1.97 (m, 1H), 1.62 (s, 6H). LC/MS (ESI⁺) calcd for C₄₂H₄₁FN₆O₇([M+H]⁺) *m*/*z* 760.3; found, 761.3.

### Example 365: 5-(((1r,3r)-3-(4-(2-(4-((2-(2-oxa-6-azaspiro[3.3]heptane-6-yl)pyrimidin-4-yl)methoxy)-3-fluorophenyl)isopropan-2-yl)-2-fluorophenoxy) cyclobutyl)amino)-2-(2,6-dioxopiperidin-3-yl)isoindolin-1,3-dione

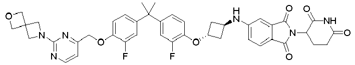

The target compound was synthesized by a method similar to that of Example 364. ¹H NMR (400 MHz, Chloroform-*d*) δ 8.33 (d, *J* = 5.0 Hz, 1H), 8.25 (s, 1H), 7.62 (d, *J* = 8.2 Hz, 1H), 6.94 (ddd, *J* = 13.0, 7.9, 1.9 Hz, 2H), 6.85 (dd, *J* = 15.6, 7.1 Hz, 5H), 6.74 - 6.61 (m, 2H), 5.02 (s, 2H), 4.98 - 4.78 (m, 7H), 4.32 (s, 4H), 2.79 (dddd, *J* = 33.8, 24.1, 18.1, 9.6 Hz, 5H), 2.48 - 2.35 (m, 2H), 2.12 (dq, *J* = 9.6, 3.3, 2.8 Hz, 1H), 2.04 - 1.97 (m, 1H), 1.60 (s, 6H). LC/MS (ESI⁺) calcd for C₄₂H₄₀F₂N₆O₇ ([M+H]⁺) *m*/*z* 778.3; found, 779.3.

### Example 366: 5-(((1s,3s)-3-(4-(2-(4-((2-(2-oxa-6-azaspiro[3.3]heptane-6-yl) pyrimidin-4-yl)methoxy)-3-fluorophenyl)isopropan-2-yl)-2-fluorophenoxy) cyclobutyl)amino)-2-(2,6-dioxopiperidin-3-yl)isoindolin-1,3-dione

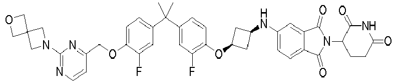

The target compound was synthesized by a method similar to that of Example 364. ¹H NMR (400 MHz, Chloroform-*d*) *δ* 8.33 (d, *J* = 4.4 Hz, 1H), 8.22 (s, 1H), 7.62 (d, *J* = 8.2 Hz, 1H), 6.99 - 6.78 (m, 7H), 6.73 (t, *J* = 8.6 Hz, 2H), 5.01 (s, 2H), 4.98 - 4.78 (m, 6H), 4.61 - 4.48 (m, 1H), 4.32 (s, 4H), 3.84 - 3.68 (m, 1H), 3.20 - 3.03 (m, 2H), 2.95 - 2.67 (m, 3H), 2.25 - 2.08 (m, 2H), 2.01 (dd, *J* = 10.9, 5.6 Hz, 1H), 1.60 (s, 6H). LC/MS (ESI⁺) calcd for C₄₂H₄₀F₂N₆O₇ ([M+H]⁺) *m*/*z* 778.3; found 779.3.

### Example 367: Synthesis of 5-(((1r,3r)-3-(4-(1-(4-((2-(2-oxa-6-azaspiro[3.3] heptane-6-yl)pyrimidin-4-yl)methoxy)phenyl)allyl)phenoxy)cyclobutyl)amino)-2-(2,6-dioxopiperidin-3-yl)isoindolin-1,3-dione

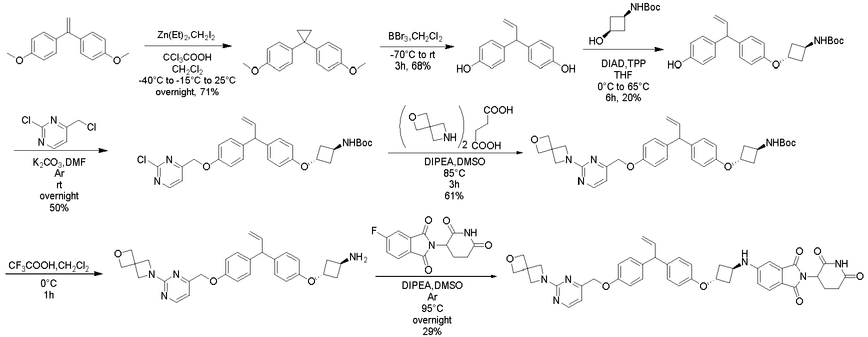

### Step 1: Synthesis of 4,4'-(cyclopropan-1,1-diyl)bis(methoxybenzene)

To a three-necked round-bottom flask, was added dichloromethane (6 mL), and then the system was vacuumized and purged with argon, that was repeated three times, followed by addition of diethylzinc (6 mL, 6.00 mmol, 1.0 M) solution. Subsequently, the system was moved to a dry ice ethanol bath, and cooled under stirring. When the internal temperature was reduced to about -40 °C, to the system, was slowly added 6 mL solution of diiodomethane (967 mL, 12.00 mmol) in dichloromethane dropwise, and then the system was allowed to react at -40 °C for 1 h. After that, to the system, was added 6 mL solution of trichloroacetic acid (980 mg, 6.00 mmol) in dichloromethane dropwise, and then the system was naturally warmed to -15 °C, and allowed to react at this temperature for 1 h. To the system, was added 6 mL solution of 4,4'-(ethylene-1,1-diyl)bis(methoxybenzene) (721 mg, 3.00 mmol) in dichloromethane dropwise. Subsequently, the system was warmed to room temperature and reacted overnight under stirring. The next day, TLC detection indicated completion of the reaction. To the system in the ice water bath, was added saturated NH₄Cl solution to quench the reaction. The resultant solution was extracted by adding ethyl acetate and water. The organic phase was successively washed with water and saturated brine, and dried over anhydrous Na₂SO₄. The resultant solution was rotatory evaporated to remove the solvent and provide the crude product, which was separated and purified by column chromatography, to obtain the target product as off-white solids (527 mg, 71%). LC/MS (ESI⁺) calcd for C₁₇H₁₈O₂ ([M+H]⁺) *m*/*z* 254.3; found 255.1.

### Step 2: Synthesis of 4,4'-(prop-2-en-1,1-diyl)diphenol

4,4'-(cyclopropan-1,1-diyl)bis(methoxybenzene) (520 mg, 2.04 mmol) was weighed and added into a three-necked round-bottom flask, to which was added dichloromethane (15 mL), and then the mixture was stirred to dissolve and become clear. Subsequently, the system was moved to a dry ice ethanol bath, and cooled under stirring. When the internal temperature was reduced to about -70 °C, to the system, was slowly added 5 mL solution of BBr₃ (4098 mg, 16.36 mmol) in dichloromethane dropwise, and then the system was allowed to react at -70 °C for 30 min. After that, the system was moved out from the bath and placed at room temperature to react under stirring. After 3h, TLC detection indicated the starting materials disappeared. The reaction was stopped. Under the conditions of cooling in an ice bath, the reaction solution was slowly added dropwise to a round-bottom flask containing saturated NaHCO₃ solution, to quench the reaction. Then, the reaction was extracted by adding dichloromethane and water. The organic phase was successively washed with water and saturated brine, and dried over anhydrous Na₂SO₄. The resultant solution was rotatory evaporated to remove the solvent and provide the crude product, which was separated and purified by column chromatography, to obtain the target product as white solid (315 mg, 68%). LC/MS (ESI⁺) calcd for C₁₅H₁₄O₂ ([M-H]⁻) *m*/*z* 226.1; found 225.1.

In the following steps, the target compound was synthesized by a method similar to that of Example 355. ¹H NMR (400 MHz, Chloroform-*d*) *δ* 8.30 (d, *J* = 5.1 Hz, 1H), 8.24 (d, *J* = 8.5 Hz, 1H), 7.62 (d, *J* = 8.3 Hz, 1H), 7.08 (dd, *J* = 8.4, 5.5 Hz, 4H), 6.86 (d, *J* = 8.8 Hz, 3H), 6.80 (d, *J* = 5.0 Hz, 1H), 6.71 (dd, *J* = 10.2, 7.6 Hz, 3H), 6.23 (ddd, *J* = 17.1, 10.1, 7.2 Hz, 1H), 5.19 (d, *J* = 10.1 Hz, 1H), 4.95 (q, *J* = 5.2, 3.5 Hz, 4H), 4.86 (s, 6H), 4.63 (d, *J* = 7.0 Hz, 1H), 4.31 (s, 4H), 4.26 - 4.17 (m, 1H), 2.94 - 2.60 (m, 5H), 2.40 (dt, *J* = 13.6, 6.5 Hz, 2H), 2.16 - 2.09 (m, 1H). LC/MS (ESI⁺) calcd for C₄₂H₄₀N₆O₇ ([M+H]⁺) *m*/*z* 740.3; found, 741.3.

### Example 368: 2-(2,6-dioxopiperidin-3-yl)-5-(((1r,3r)-3-(4-(2-(4-((2-((tetrahydro-2H-pyran-4-yl)oxy)pyrimidin-4-yl)methoxy)phenyl)isopropan-2-yl)phenoxy) cyclobutyl)amino)isoindolin-1,3-dione

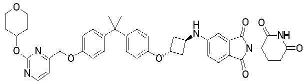

The target compound was synthesized by a method similar to that of Example 355. ¹H NMR (400 MHz, Chloroform-*d*) *δ* 8.49 (d, *J* = 5.0 Hz, 1H), 8.10 (s, 1H), 7.64 (d, *J* = 8.2 Hz, 1H), 7.21 - 7.11 (m, 5H), 6.89 (d, *J* = 1.7 Hz, 1H), 6.84 (d, *J* = 8.7 Hz, 2H), 6.74 - 6.63 (m, 3H), 5.23 (tt, *J* = 8.2, 3.8 Hz, 1H), 5.05 (s, 2H), 4.93 (dd, *J* = 12.2, 5.3 Hz, 1H), 4.86 (tt, *J* = 6.9, 4.0 Hz, 1H), 4.82 - 4.73 (m, 1H), 4.23 (br, 1H), 4.03 (dt, *J* = 10.0, 4.5 Hz, 2H), 3.62 (ddd, *J*= 11.8, 8.8, 3.1 Hz, 2H), 2.94 - 2.75 (m, 3H), 2.71 (ddt, *J* = 11.3, 7.0, 4.3 Hz, 2H), 2.40 (dt, *J*= 13.1, 6.0 Hz, 2H), 2.13 - 2.07 (m, 2H), 1.89 (ddt, *J=* 17.1, 8.5, 3.9 Hz, 3H), 1.63 (s, 6H). LC/MS (ESI⁺) calcd for C₄₂H₄₃N₅O₈ ([M+H]⁺) *m*/*z* 745.3; found, 746.3.

### Example 369: 2-(2,6-dioxopiperidin-3-yl)-5-(((1r,3r)-3-(4-(2-(4-((2-(oxetane-3-yloxy)pyrimidin-4-yl)methoxy)phenyl)isopropan-2-yl)phenoxy)cyclobutyl)amino) isoindolin-1,3-dione

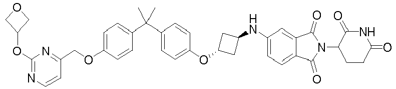

The target compound was synthesized by a method similar to that of Example 355. ¹H NMR (400 MHz, Chloroform-*d*) *δ* 8.49 (d, *J=* 5.1 Hz, 1H), 7.61 (d, *J* = 8.3 Hz, 1H), 7.34 (d, *J* = 5.0 Hz, 1H),7.26 (d, *J* = 5.0 Hz, 1H), 7.14 (dd, *J* = 13.9, 8.7 Hz, 4H), 6.97 - 6.88 (m, 1H), 6.84 (d, *J* = 8.6 Hz, 2H), 6.70 (d, *J=* 8.7 Hz, 3H), 5.64 (p, *J=* 5.6 Hz, 1H), 5.05 (s, 2H), 5.00 (t, *J=* 6.8 Hz, 2H), 4.93 (dd, *J* = 11.7, 5.2 Hz, 1H), 4.90 - 4.84 (m, 1H), 4.81 (q, *J* = 7.5, 6.2 Hz, 2H), 4.26 - 4.13 (m, 1H), 2.91 - 2.72 (m, 3H), 2.68 (dq, *J* = 12.0, 4.0 Hz, 2H), 2.43 (dt, *J* = 11.9, 6.0 Hz, 2H), 2.16 - 2.08 (m, 1H), 2.01 (q, *J* = 5.8 Hz, 1H), 1.64 (s, 6H). LC/MS (ESI⁺) calcd for C₄₀H₃₉N₅O₈ ([M+H]⁺) *m*/*z* 717.3; found 718.3.

### Example 370: 5-(((1r,3r)-3-(4-(1-(4-((5-(2H-1,2,3-triazole-2-yl)pyrimidin-2-yl)oxy) phenyl)allyl)phenoxy)cyclobutyl)amino)-2-(2,6-dioxopiperidin-3-yl)isoindolin-1,3-dione

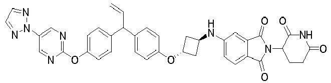

The target compound was synthesized by a method similar to that of Example 367. ¹H NMR (400 MHz, Chloroform-*d*) *δ* 9.23 (s, 2H), 8.03 (s, 1H), 7.87 (s, 2H), 7.64 (d, *J* = 8.2 Hz, 1H), 7.26 (d, *J* = 8.5 Hz, 2H),7.16 (d, *J* = 8.5 Hz, 2H), 7.13 (d, *J* = 8.5 Hz, 2H), 6.92 - 6.88 (m, 1H), 6.76 (d, *J* = 8.6 Hz, 2H), 6.73 - 6.68 (m, 1H), 6.29 (ddd, *J* = 17.2, 10.1, 7.3 Hz, 1H), 5.23 (d, *J =* 10.1 Hz, 1H), 5.02 (d, *J=* 17.0 Hz, 1H), 4.93 (dd, *J=* 12.2, 5.3 Hz, 1H), 4.90 - 4.83 (m, 1H), 4.72 (d, *J* = 7.1 Hz, 1H), 4.24 (ddd, *J=* 12.1, 7.5, 4.9 Hz, 1H), 2.94 - 2.65 (m, 5H), 2.41 (dt, *J* = 12.3, 6.2 Hz, 2H), 2.12 (dt, *J* = 10.3, 5.0 Hz, 1H), 2.01 (q, *J =* 6.3, 5.8 Hz, 1H). LC/MS (ESI⁺) calcd for C₃₈H₃₂N₈O₆ ([M+H]⁺) *m*/*z* 696.2; found, 697.2.

### Example 371: 5-(((1r,3r)-3-(4-(1-(4-((6-(2H-1,2,3-triazole-2-yl)pyridazine-3-yl)oxy) phenyl)allyl)phenoxy)cyclobutyl)amino)-2-(2,6-dioxopiperidin-3-yl)isoindolin-1,3-dione

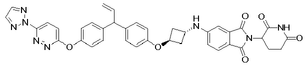

The target compound was synthesized by a method similar to that of Example 367. ¹H NMR (400 MHz, Chloroform-*d*) *δ* 8.26 (d, *J* = 9.3 Hz, 1H), 7.95 (s, 2H), 7.64 (d, *J* = 8.3 Hz, 1H), 7.35 (d, *J* = 9.5 Hz, 1H), 7.24 (d, *J* = 8.6 Hz, 2H), 7.18 (d, *J* = 8.6 Hz, 2H), 7.11 (d, *J* = 8.5 Hz, 2H), 6.94 - 6.89 (m, 1H), 6.78 - 6.70 (m, 3H), 6.27 (ddd, *J=* 17.1, 10.3, 7.3 Hz, 1H), 5.35 (t, *J* = 4.5 Hz, 1H), 5.24 (d, *J* = 10.2 Hz, 1H), 5.01 (d, *J* = 17.1 Hz, 1H), 4.93 (dd, *J* = 12.2, 5.2 Hz, 1H), 4.90 - 4.84 (m, 1H), 4.71 (d, *J* = 7.0 Hz, 1H), 4.24 (ddd, *J* = 12.4, 7.6, 4.9 Hz, 1H), 4.04 (dd, *J* = 8.0, 3.6 Hz, 1H), 2.94 - 2.66 (m, 5H), 2.26 - 2.20 (m, 1H), 2.05 - 1.97 (m, 2H). LC/MS (ESI⁺) calcd for C₃₈H₃₂N₈O₆ ([M+H]⁺) *m*/*z* 696.2; found, 697.2.

### Example 372: 5-(((1r,3r)-3-(4-(1-(4-((2-(2H-1,2,3-triazole-2-yl)pyrimidin-5-yl)oxy) phenyl)allyl)phenoxy)cyclobutyl)amino)-2-(2,6-dioxopiperidin-3-yl)isoindolin-1,3-dione

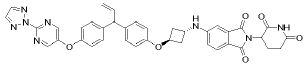

The target compound was synthesized by a method similar to that of Example 367. ¹H NMR (400 MHz, Chloroform-*d*) *δ* 8.57 (s, 2H), 7.97 (s, 3H), 7.64 (d, *J* = 8.2 Hz, 1H), 7.24 (d, *J* = 8.5 Hz, 2H), 7.09 (d, *J* = 8.4 Hz, 2H), 7.03 (d, *J* = 8.5 Hz, 2H), 6.94 - 6.89 (m, 1H), 6.76 (d, *J* = 8.6 Hz, 2H), 6.73 (d, *J* = 8.5 Hz, 1H), 6.27 (ddd, *J* = 17.1, 10.1, 7.2 Hz, 1H), 5.35 (t, *J* = 4.7 Hz, 1H), 5.25 (d, *J* = 10.2 Hz, 1H), 4.99 (d, *J* = 17.1 Hz, 1H), 4.93 (dd, *J* = 12.1, 5.2 Hz, 1H), 4.88 (br, 1H), 4.71 (d, *J* = 7.0 Hz, 1H), 4.29 - 4.20 (m, 1H), 2.95 - 2.65 (m, 5H), 2.28 - 2.20 (m, 1H), 2.04 - 1.98 (m, 2H). LC/MS (ESI⁺) calcd for C₃₈H₃₂N₈O₆ ([M+H]⁺) *m*/*z* 696.2; found, 697.2.

### Example 373:2-(2,6-dioxopiperidin-3-yl)-5-(((1r,3r)-3-(4-(1-(4-((2-(5-methyl-1,3,4-oxadiazol-2-yl)pyrimidin-5-yl)oxy)phenyl)allyl)phenoxy)cyclobutyl)amino) isoindolin-1,3-dione

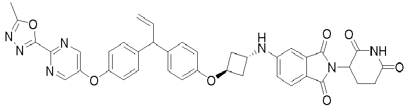

The target compound was synthesized by a method similar to that of Example 367. ¹H NMR (400 MHz, Chloroform-*d*) *δ* 8.59 (s, 2H), 8.02 (s, 1H), 7.64 (d, *J* = 7.9 Hz, 1H), 7.08 (dd, *J* = 14.5, 8.0 Hz, 4H), 6.91 (s, 1H), 6.74 (dd, *J=* 15.3, 7.9 Hz, 3H), 6.27 (ddd, *J=* 17.5, 10.3, 7.5 Hz, 1H), 5.26 (d, *J* = 9.7 Hz, 1H), 5.00 (d, *J=* 17.0 Hz, 1H), 4.93 (dd, *J* = 12.1, 5.2 Hz, 1H), 4.89 (br, 1H), 4.73 (d, *J* = 6.6 Hz, 1H), 4.25 (br, 1H), 2.97 - 2.63 (m, 8H), 2.50 - 2.36 (m, 2H), 2.17 - 2.08 (m, 1H), 1.64 (d, *J=* 7.0 Hz, 2H). LC/MS (ESI⁺) calcd for C₃₉H₃₃N₇O₇([M+H]⁺) *m*/*z* 711.2; found, 712.3.

### Example 374: 5-(((1r,3r)-3-(4-(1-(4-((6-(2H-1,2,3-triazole-2-yl)pyridazine-3-yl)oxy) phenyl)cyclopropyl)phenoxy)cyclobutyl)amino)-2-(2,6-dioxopiperidin-3-yl)isoindolin-1,3-dione

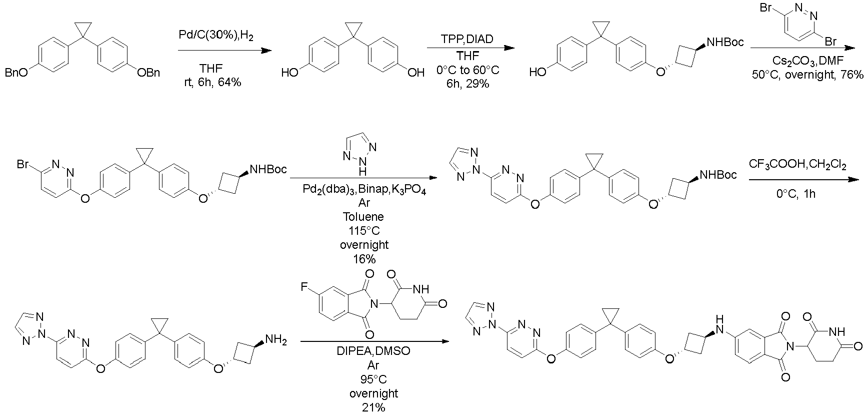

### Step 1: Synthesis of compound 4,4'-(cyclopropan-1,1-diyl)diphenol

4,4'-(cyclopropan-1,1-diyl)bis((benzyloxy)benzene) (1400 mg, 3.44 mmol) was weighed and added into a three-necked round-bottom flask, to which was added THF (42 mL), and then the mixture was stirred to dissolve and become clear. To the system, was added Pd/C (420 mg). Subsequently, the system was vacuumed and purged with hydrogen, that was repeated several times, to ensure the hydrogen atmosphere in the system. Then, the system was stirred and reacted at room temperature. After 6 h, LCMS indicated disappearance of starting materials. The reaction was stopped, and the system was subjected to suction filtration. The filter cake was washed with a small amount of THF for a few times, and then the filtrate was combined. The solvent was removed by rotatory evaporation, provide the crude product, which was separated and purified by column chromatography, to obtain the target product as white solid (500 mg, 64%). LC/MS (ESI⁺) calcd for C₁₅H₁₄O₂ ([M-H]⁻) *m*/*z* 226.1; found 225.1.

In the following steps, the target compound was synthesized by a method similar to that of Example 320. LC/MS (ESI⁺) calcd for C₃₈H₃₂N₈O₆ ([M+H]⁺) *m*/*z* 696.2; found 697.3.

### Example 375: 5-(((1r,3r)-3-(4-(1-(4-((6-(1H-1,2,3-triazole-1-yl)pyridazine-3-yl)oxy) phenyl)cyclopropyl)phenoxy)cyclobutyl)amino)-2-(2,6-dioxopiperidin-3-yl) isoindolin-1,3-dione

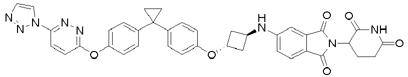

The target compound was synthesized by a method similar to that of Example 320. ¹H NMR (400 MHz, Chloroform-*d*) *δ* 8.71 - 8.64 (m, 1H), 8.44 (d, *J* = 9.4 Hz, 1H), 8.03 - 7.96 (m, 1H), 7.90 - 7.84 (m, 1H), 7.64 (d, *J* = 8.2 Hz, 1H), 7.43 (d, *J =* 9.4 Hz, 1H), 7.28 (d, *J* = 8.7 Hz, 2H),7.20 (d, *J* = 8.7 Hz, 2H), 7.12 (d, *J* = 8.7 Hz, 2H), 6.94 - 6.89 (m, 1H), 6.72 (d, *J* = 8.7 Hz, 3H), 4.93 (dd, *J* = 12.3, 5.2 Hz, 1H), 4.90 - 4.82 (m, 1H), 4.29 - 4.20 (m, 1H), 2.93 - 2.66 (m, 6H), 2.47 - 2.37 (m, 1H), 2.04 - 1.97 (m, 1H), 1.64 (dt, *J* = 13.2, 6.6 Hz, 1H), 0.88 (t, *J* = 6.8 Hz, 4H). LC/MS (ESI⁺) calcd for C₃₈H₃₂N₈O₆ ([M+H]⁺) *m*/*z* 696.2; found, 697.3.

### Example 376: Synthesis of 2-(2,6-dioxopiperidin-3-yl)-5-(((1r,3r)-3-(4-(1-(4-((6-(5-methyl-1,2,4-oxadiazol-3-yl)pyridazine-3-yl)oxy)phenyl)cyclopropyl)phenoxy) cyclobutyl)amino)isoindolin-1,3-dione

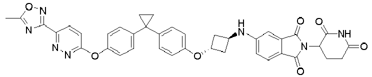

The target compound was synthesized by a method similar to that of Example 326. ¹H NMR (400 MHz, Chloroform-*d*) *δ* 8.17 (d, *J=* 8.8 Hz, 1H), 8.00 (s, 1H), 7.64 (d, *J=* 7.6 Hz, 1H), 7.24 (d, *J=* 7.6 Hz, 1H), 7.15 (dd, *J=* 23.4, 8.0 Hz, 4H), 6.91 (s, 1H), 6.72 (d, *J=* 7.9 Hz, 3H), 4.93 (dd, *J=* 12.0, 5.4 Hz, 1H), 4.88 (br, 1H), 4.24 (br, 1H), 2.94 - 2.65 (m, 8H), 2.43 (s, 2H), 2.17 - 2.09 (m, 1H), 2.01 (h, *J* = 5.1 Hz, 2H), 0.88 (t, *J* = 6.8 Hz, 4H). LC/MS (ESI⁺) calcd for C₃₉H₃₃N₇O₇([M+H]⁺) *m*/*z* 711.2; found, 712.2.

### Example 377: 2-(2,6-dioxopiperidin-3-yl)-5-(((1r,3r)-3-(4-(1-(4-((2-(1-hydroxylethyl)pyrimidin-5-yl)oxy)phenyl)cyclopropyl)phenoxy)cyclobutyl) amino)isoindolin-1,3-dione

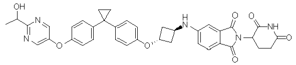

The target compound was synthesized by a method similar to that of Example 342. ¹H NMR (400 MHz, Chloroform-*d*) *δ* 8.46 (s, 2H), 8.11 (s, 1H), 7.63 (d, *J* = 8.3 Hz, 1H), 7.23 (d, *J* = 8.6 Hz, 2H), 7.20 - 7.15 (m, 2H), 6.94 (d, *J* = 8.7 Hz, 2H), 6.89 (d, *J* = 2.0 Hz, 1H), 6.76 - 6.68 (m, 3H), 4.99 (q, *J=* 6.7 Hz, 1H), 4.93 (dd, *J* = 12.3, 5.3 Hz, 1H), 4.90 - 4.82 (m, 1H), 4.23 (ddd, *J* = 12.8, 7.6, 5.1 Hz, 1H), 3.49 (s, 1H), 2.94 - 2.75 (m, 3H), 2.70 (dq, *J* = 11.8, 4.1 Hz, 3H), 2.47 - 2.35 (m, 2H), 2.13 (ddd, *J* = 10.1, 4.2, 2.4 Hz, 1H), 1.58 (d, *J* = 6.6 Hz, 3H), 1.27 (q, *J* = 8.7 Hz, 4H). LC/MS (ESI⁺) calcd for C₃₈H₃₅N₅O₇ ([M+H]⁺) *m*/*z* 673.3; found, 674.2.

### Example 378: 5-(((1r,3r)-3-(4-(1-(4-((2-acetylpyrimidin-5-yl)oxy)phenyl) cyclopropyl)phenoxy)cyclobutyl)amino)-2-(2,6-dioxopiperidin-3-yl)isoindolin-1,3-dione

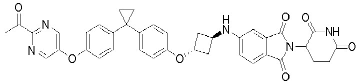

The target compound was synthesized by a method similar to that of Example 350. ¹H NMR (400 MHz, Chloroform-*d*) *δ* 8.53 (s, 2H), 8.04 (s, 1H), 7.63 (d, *J* = 8.1 Hz, 1H), 7.26 (d, *J* = 8.3 Hz, 2H), 7.18 (d, *J* = 8.3 Hz, 2H), 6.99 (d, *J* = 8.3 Hz, 2H), 6.90 (s, 1H), 6.72 (t, *J* = 8.2 Hz, 3H), 4.93 (dd, *J* = 12.0, 5.2 Hz, 1H), 4.88 (s, 1H), 4.24 (s, 1H), 3.00 - 2.61 (m, 9H), 2.48 - 2.36 (m, 2H), 2.17 - 2.09 (m, 1H), 0.99 - 0.58 (m, 4H). LC/MS (ESI⁺) calcd for C₃₈H₃₃N₅O₇ ([M+H]⁺) m/z 671.2; found 672.2.

### Example 379:2-(2,6-dioxopiperidin-3-yl)-5-(((1r,3r)-3-(4-(1-(4-((2-(5-methyl-1,3,4-oxadiazol-2-yl)pyrimidin-5-yl)oxy)phenyl)cyclopropyl)phenoxy)cyclobutyl) amino)isoindolin-1,3-dione

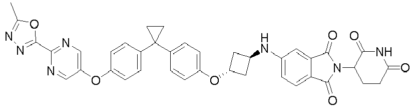

The target compound was synthesized by a method similar to that of Example 322. ¹H NMR (400 MHz, Chloroform-*d*) *δ* 8.58 (s, 2H), 8.04 (s, 1H), 7.63 (d, *J* = 8.3 Hz, 1H), 7.27 (d, *J* = 8.5 Hz, 2H), 7.18 (d, *J* = 8.6 Hz, 2H), 7.04 - 6.96 (m, 2H), 6.89 (d, *J* = 2.0 Hz, 1H), 6.76 - 6.68 (m, 3H), 4.93 (dd, *J* = 12.2, 5.3 Hz, 1H), 4.90 - 4.84 (m, 1H), 4.23 (ddd, *J* = 12.8, 7.6, 5.4 Hz, 1H), 2.94 - 2.63 (m, 9H), 2.41 (dt, *J* = 12.9, 6.5 Hz, 2H), 2.16 - 2.09 (m, 1H), 1.01 - 0.63 (m, 4H). LC/MS (ESI⁺) calcd for C₃₉H₃₃N₇O₇([M+H]⁺) *m*/*z* 711.2; found, 712.2.

### Example 380: 2-(2,6-dioxopiperidin-3-yl)-5-(((1r,3r)-3-(4-(3-(4-((2-(5-methyl-1,2,4-oxadiazol-3-yl)pyrimidin-5-yl)oxy)phenyl)pentan-3-yl)phenoxy)cyclobutyl) amino)isoindolin-1,3-dione

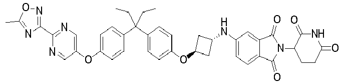

The target compound was synthesized by a method similar to that of Example 326. ¹H NMR (400 MHz, Chloroform-*d*) *δ* 8.57 (s, 2H), 8.01 (s, 1H), 7.64 (d, *J* = 8.3 Hz, 1H), 7.25 (d, *J* = 8.8 Hz, 2H), 7.07 (d, *J* = 8.8 Hz, 2H), 7.03 - 6.97 (m, 2H), 6.91 (d, *J* = 1.8 Hz, 1H), 6.75 - 6.67 (m, 3H), 4.93 (dd, *J* = 12.3, 5.3 Hz, 1H), 4.87 (dq, *J* = 7.2, 4.4, 3.6 Hz, 1H), 4.24 (ddd, *J* = 12.6, 7.6, 5.2 Hz, 1H), 2.94 - 2.79 (m, 2H), 2.79 - 2.66 (m, 6H), 2.42 (dt, *J* = 12.2, 6.3 Hz, 2H), 2.16 - 2.04 (m, 6H), 0.64 (t, *J* = 7.3 Hz, 6H). LC/MS (ESI⁺) calcd for C₄₁H₃₉N₇O₇ ([M+H]⁺) *m*/*z* 741.3; found, 742.2.

### Example 381: ethyl 5-(4-(2-(4-((1r,3r)-3-((2-(2,6-dioxopiperidin-3-yl)-1,3-dioxoisoindolin-5-yl)amino)cyclobutyloxy)phenyl)isopropan-2-yl)phenoxy) pyrimidin-2-carboxylate

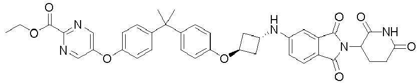

The target compound was synthesized by a method similar to that of Example 326. ¹H NMR (400 MHz, Chloroform-*d*) *δ* 8.55 (s, 2H), 8.01 (s, 1H), 7.63 (d, *J=* 8.3 Hz, 1H), 7.30 (dt, *J=* 9.5, 2.9 Hz, 2H), 7.12 (d, *J* = 8.8 Hz, 2H), 7.03 - 6.96 (m, 2H), 6.90 (d, *J =* 1.9 Hz, 1H), 6.72 (d, *J* = 8.7 Hz, 3H), 4.93 (dd, *J=* 12.3, 5.3 Hz, 1H), 4.90 - 4.83 (m, 1H), 4.53 (q, *J* = 7.1 Hz, 2H), 4.24 (ddd, *J* = 12.3, 7.5, 4.7 Hz, 1H), 2.85 (ddd, *J* = 27.4, 15.1, 3.1 Hz, 2H), 2.73 (dtd, *J* = 12.3, 7.4, 6.4, 2.9 Hz, 3H), 2.42 (dt, *J* = 12.7, 6.4 Hz, 2H), 2.16 - 2.08 (m, 1H), 2.05 - 1.98 (m, 1H), 1.68 (s, 6H), 1.46 (t, *J=* 7.1 Hz, 3H). LC/MS (ESI⁺) calcd for C₃₉H₃₇N₈O₈ ([M+H]⁺) *m*/*z* 703.3; found, 704.2.

### Example 382: 2-(2,6-dioxopiperidin-3-yl)-5-(((1r,3r)-3-(4-(4-(4-((2-(5-methyl-1,2,4-oxadiazol-3-yl)pyrimidin-5-yl)oxy)phenyl)tetrohydro-2H-pyran-4-yl) phenoxy)cyclobutyl)amino)isoindolin-1,3-dione

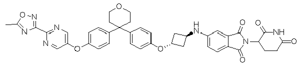

The target compound was synthesized by a method similar to that of Example 326. ¹H NMR (400 MHz, Chloroform-*d*) *δ* 8.58 (s, 2H), 8.19 (s, 1H), 7.62 (d, *J* = 7.8 Hz, 1H), 7.30 (d, *J* = 8.2 Hz, 2H), 7.16 (d, *J* = 7.8 Hz, 2H), 7.03 (d, *J* = 8.2 Hz, 2H), 6.88 (s, 1H), 6.75 (d, *J* = 8.0 Hz, 2H), 6.70 (d, *J* = 7.5 Hz, 1H), 4.94 (dd, *J* = 11.6, 4.9 Hz, 1H), 4.87 (s, 1H), 4.23 (s, 1H), 3.77 (s, 4H), 2.86 (dd, *J=* 29.1, 14.5 Hz, 2H), 2.72 (s, 6H), 2.49 - 2.30 (m, 6H), 2.18 - 2.07 (m, 1H). LC/MS (ESI⁺) calcd for C₄₁H₃₇N₇O₈ ([M+H]⁺) *m*/*z* 755.3; found, 756.2.

### Example 383: 5-((3-(4-(2-(2-(azetidin-1-yl)pyrimidin-4-yl)methoxy)phenyl) propan-2-yl)phenoxy)propyl)amino)-2-(2,6-dioxopiperidin-3-yl)isoindol-1,3-dione

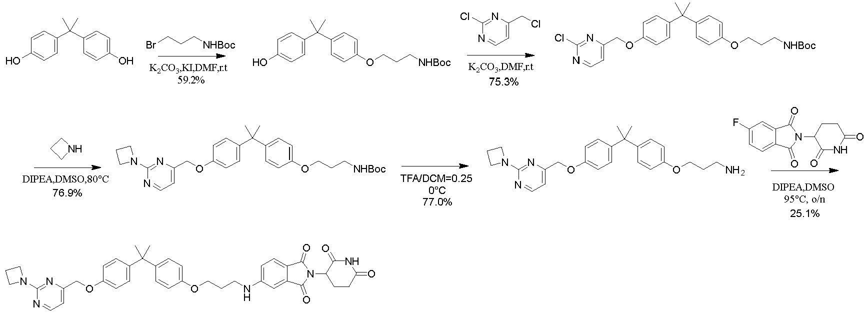

### Step 1: Synthesis of tert-butyl (3-(4-(2-(4-hydroxylphenyl)propan-2-yl)phenoxy) propyl)carbamate

In a 25 mL conical flask, 4,4'-(propan-2,2-diyl)bisphenol (1.000 g, 4.380 mmol) was dissolved in 10 mL of DMF, to which were added tert-butyl (3-bromopropyl)carbamate (1.560 g, 6.570 mmol), K₂CO₃ (1.210 g, 8.760 mmol), and KI (218 mg,1.210 mmol) under nitrogen protection, and then the mixture was stirred for 12 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (500 mg, yield 59.2%).

### Step 2: Synthesis of tert-butyl (3-(4-(2-(4-((2-chloropyrimidin-4-yl)methoxy)phenyl) propan-2-yl)phenoxy)propyl)carbamate

In a 25 mL conical flask, tert-butyl (3-(4-(2-(4-hydroxylphenyl)propan-2-yl)phenoxy)propyl)carbamate (500 mg, 1.300 mmol) was dissolved in 5 mL of DMF, to which were added 2-chloro-4-(chloromethyl)pyrimidine (254 mg, 1.580 mmL) and K₂CO₃ (359 mg, 2.590 mmol) under nitrogen protection, and then the mixture was stirred for 12 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (500 mg, yield 75.3%).

### Step 3: Synthesis of tert-butyl (3-(4-(2-(4-((2-(azetidin-1-yl)pyrimidin-4-yl)methoxy) phenyl)propan-2-yl)phenoxy)propyl)carbamate

In a 25 mL conical flask, tert-butyl (3-(4-(2-(4-((2-chloropyrimidin-4-yl)methoxy) phenyl)propan-2-yl)phenoxy)propyl)carbamate (500 mg, 0.977 mmol) was dissolved in 5 mL of DMSO, to which were added cyclobutylamine (84 mg, 1.460 mmol) and N,N-diisopropylethylamine (379 mg, 2.930 mmol) under nitrogen protection, and then the mixture was stirred for 2 h at 80 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (400 mg, yield 76.9%).

### Step 4: Synthesis of 3-(4-(2-(2-(azetidin-1-yl)pyrimidin-4-yl)methoxy)phenyl)propan-2-yl)phenoxy)propan-1-amine

In a 25 mL conical flask, tert-butyl (3-(4-(2-(4-((2-(azetidin-1-yl)pyrimidin-4-yl)methoxy)phenyl)propan-2-yl)phenoxy)propyl)carbamate (400 mg,0.751 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (250 mg, yield 77.0%).

### Step 5: Synthesis of 5-((3-(4-(2-(2-(azetidin-1-yl)pyrimidin-4-yl)methoxy)phenyl) propan-2-yl)phenoxy)propyl)amino)-2-(2,6-dioxopiperidin-3-yl)isoindol-1,3-dione

In a 25 mL conical flask, 3-(4-(2-(2-(azetidin-1-yl)pyrimidin-4-yl)methoxy) phenyl)propan-2-yl)phenoxy)propan-1-amine (250 mg, 0.578 mmol) was dissolved in 5 mL of DMSO, to which were added 2-(2,6-dioxopiperidin-3-yl)-5-fluoroisoindolin-1,3-dione (224 mg, 0.809 mmol) and N,N-diisopropylethylamine (299 mg, 2.310 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 25.1%). ¹H NMR (400 MHz, Chloroform-*d*) *δ* 8.28 (d, *J* = 5.5 Hz, 1H), 8.06 (s, 1H), 7.60 (d, *J* = 8.3 Hz, 1H), 7.15 (dd, *J* = 8.9, 2.1 Hz, 4H), 6.98 (d, *J* = 2.1 Hz, 1H), 6.82 (dd, *J* = 8.8, 5.4 Hz, 5H), 6.75 (dd, *J* = 8.3, 2.2 Hz, 1H), 4.98 (s, 2H), 4.96 - 4.91 (m, 1H), 4.36 - 4.25 (m, 4H), 4.09 (t, *J* = 5.5 Hz, 2H), 3.50 - 3.41 (m, 2H), 2.93 - 2.69 (m, 3H), 2.43 (q, *J=* 7.6 Hz, 2H), 2.14 (dt, *J* = 12.2, 6.5 Hz, 3H), 1.64 (s, 6H). LC/MS (ESI+) calcd for C₃₉H₄₀N₆O₆ ([M+H]⁺) *m*/*z* 689.3, found 689.3.

### Example 384: 2-(2,6-dioxopiperidin-3-yl)-5-(4-((8-(4-(2-(4-((2-(7-methyl-2,7-diazaspiro[4.4]nonan-2-yl)pyrimidin-4-yl)methoxy)phenyl)propan-2-yl) phenoxy)octyl)oxy)butyl)amino)isoindolin-1,3-dione

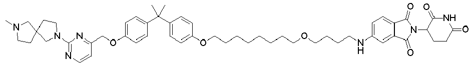

The target compound was synthesized by a method similar to that of Example 383. ¹H NMR (400 MHz, DMSO-*d₆*) *δ* 8.36 - 8.28 (m, 1H), 7.66 (s, 1H), 7.26 (d, *J=* 8.4 Hz, 1H), 7.14 - 7.03 (m, 4H), 6.88 (d, *J* = 8.7 Hz, 2H), 6.79 (d, *J* = 8.5 Hz, 2H), 6.65 (d, *J* = 4.3 Hz, 1H), 6.44 (d, *J* = 8.9 Hz, 1H), 4.93 (s, 2H), 4.25 - 4.17 (m, 1H), 3.89 (t, *J* = 6.3 Hz, 2H), 2.86 (dd, *J* = 14.2, 7.0 Hz, 2H), 2.50 (s, 20H), 2.25 (d, *J* = 9.3 Hz, 3H), 1.97 (dt, *J* = 11.8, 7.0 Hz, 4H), 1.82 - 1.73 (m, 2H), 1.69 - 1.62 (m, 2H), 1.56 (s, 8H). LC/MS (ESI+) calcd for C₅₃H₆₇N₇O₇ ( [M+H]⁺ ) *m*/*z*: 914.5, found 914.5.

### Example 385: 5-((4-((8-(4-(2-(4-((2-acetylpyrimidin-5-yl)methoxy)phenyl) propan-2-yl)phenoxy)octyl)oxy)butyl)amino)-2-(2,6-dioxopiperidin-3-yl)isoindol-1,3-dione

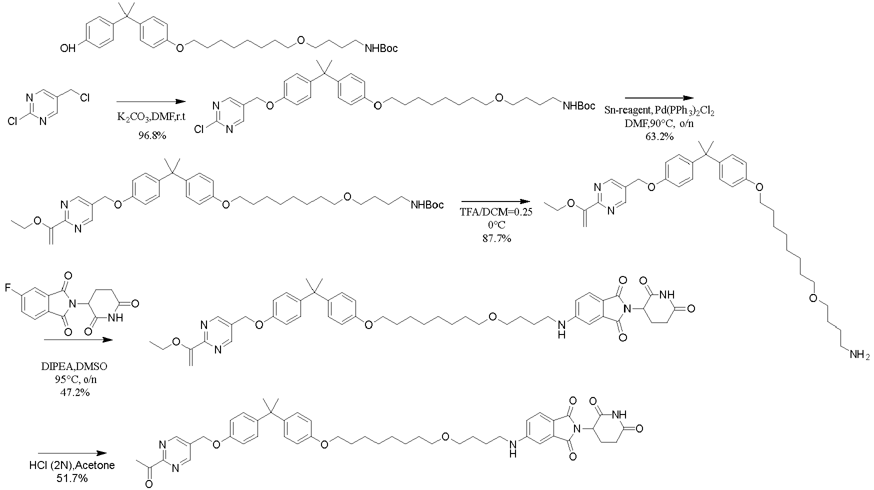

### Step 1: Synthesis of tert-butyl (4-((8-(4-(2-(4-((2-chloropyrimidin-5-yl)methoxy) phenyl)propan-2-yl)phenoxy)octyl)oxy)butylcarbamate

In a 25 mL conical flask, tert-butyl (4-((8-(4-(2-(4-hydroxylphenyl)propan-2-phenoxy)octyl)oxy)butyl)carbamate (500 mg, 0.947 mmol) was dissolved in 5 mL of DMF, to which were added 5-(chloromethyl)pyrimidine (185 mg,1.140 mmol) and K₂CO₃ (393 mg, 2.840 mmol) under nitrogen protection, and then the mixture was stirred for 12 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (600 mg, yield 96.8%).

### Step 2: Synthesis of tert-butyl (4-((8-(4-(2-(2-(1-ethoxyvinyl)pyrimidin-5-yl) methoxy phenyl)propan-2-yl)phenoxy)octyl)oxy)butylcarbamate

In a 25 mL conical flask, tert-butyl (4-((8-(4-(2-(4-((2-chloropyrimidin-5-yl)methoxy)phenyl)propan-2-yl)phenoxy)octyl)oxy)butylcarbamate (600 mg, 0.917 mmol) was dissolved in 5 mL of DMF, to which were added Pd(PPh₃)₂Cl₂ (32 mg, 0.046 mmol) and 1-ethoxyvinyltri-n-butyltin (348 mg, 0.963 mmol) under nitrogen protection, and then the mixture was stirred for 12 h at 90 °C. Subsequently, saturated KF aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (400 mg, yield 63.2%).

### Step 3: Synthesis of 4-((8-(4-(2-(4-((2-(1-ethoxyvinyl)pyrimidin-5-yl)methoxy) phenyl)propan-2-yl)phenoxy)octyl)oxy)butylamine

In a 25 mL conical flask, tert-butyl (4-((8-(4-(2-(2-(1-ethoxyvinyl)pyrimidin-5-yl)methoxy)phenyl)propan-2-yl)phenoxy)octyl)oxy)butylcarbamate (400 mg, 0.580 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (600 mg, yield 87.7%).

### Step 4: Synthesis of 2-(2,6-dioxopiperidin-3-yl)-5-((4-((8-(4-(2-(2-(1-ethoxyvinyl) pyrimidin-5-yl)methoxy)phenyl)propan-2-yl)phenoxy)octyl)oxy)butyl)aminoisoindol-1,3-dione

In a 25 mL conical flask, 4-((8-(4-(2-(4-((2-(1-ethoxyvinyl)pyrimidin-5-yl) methoxy)phenyl)propan-2-yl)phenoxy)octyl)oxy)butylamine (300 mg, 0.509 mmol) was dissolved in 5 mL of DMSO, to which were added 2-(2,6-dioxopiperidin-3-yl)-5-fluoroisoindolin-1,3-dione (196 mg, 0.712 mmol) and N,N-diisopropylethylamine (263 mg, 2.030 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (200 mg, yield 47.2%).

### Step 5: Synthesis of 5-((4-((8-(4-(2-(4-((2-acetylpyrimidin-5-yl)methoxy)phenyl) propan-2-yl)phenoxy)octyl)oxy)butyl)amino)-2-(2,6-dioxopiperidin-3-yl)isoindol-1,3-dione

In a 25 mL conical flask, 5-((3-(4-(2-(2-(azetidin-1-yl)pyrimidin-4-yl)methoxy) phenyl)propan-2-yl)phenoxy)propyl)amino)-2-(2,6-dioxopiperidin-3-yl)isoindol-1,3-dione (100 mg, 0.118 mmol) was dissolved in 4 mL of acetone, to which was added 4 mL of 2 mol/L HCl aqueous solution under nitrogen protection, and then the mixture was stirred for 1 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (50 mg, yield 51.7%). ¹H NMR (400 MHz, Chloroform-*d*) δ 8.97 (s, 2H), 8.00 (s, 1H), 7.59 (d, *J* = 8.3 Hz, 1H), 7.17 (d, *J* = 8.8 Hz, 2H), 7.10 (d, *J* = 8.8 Hz, 2H), 6.93 (d, *J =* 2.0 Hz, 1H), 6.86 (d, *J* = 8.8 Hz, 2H), 6.78 (d, *J* = 8.8 Hz, 2H), 6.70 (dd, *J* = 8.3, 2.2 Hz, 1H), 5.13 (s, 2H), 4.91 (dd, *J* = 12.3, 5.2 Hz, 1H), 3.90 (t, *J* = 6.5 Hz, 2H), 3.43 (dt, *J* = 13.2, 6.3 Hz, 4H), 3.23 (q, *J* = 6.5 Hz, 2H), 2.91 - 2.81 (m, 2H), 2.79 (s, 3H), 2.77 - 2.67 (m, 2H), 1.63 (s, 8H), 1.45 (dd, *J* = 15.1, 7.5 Hz, 10H), 1.34 (s, 8H). LC/MS (ESI+) calcd for C₄₇H₅₅N₅O₈ ( [M+H]⁺ ) *m*/*z* 817.4, found 817.4.

### Example 386: 4-((9-(4-(2-(4-((2-acetylpyrimidin-4-yl)methoxy)phenyl)propan-2-yl)phenoxy)nonyl)amino)-2-(2,6-dioxopyridin-3-yl)isoindol-1,3-dione

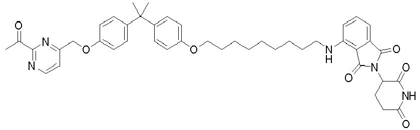

The target compound was synthesized by a method similar to that of Example 385. ¹H NMR (400 MHz, Chloroform-*d*) *δ* 8.92 (d, *J* = 5.1 Hz, 1H), 8.05 (s, 1H), 7.74 (d, *J* = 5.0 Hz, 1H), 7.49 (dd, *J* = 8.6, 7.1 Hz, 1H), 7.21 - 7.05 (m, 5H), 6.87 (dd, *J* = 9.2, 7.6 Hz, 3H), 6.81 - 6.77 (m, 2H), 6.23 (t, *J* = 5.5 Hz, 1H), 5.25 (d, *J* = 0.9 Hz, 2H), 4.91 (dd, *J* = 12.1, 5.4 Hz, 1H), 3.92 (t, *J* = 6.5 Hz, 2H), 3.26 (q, *J* = 6.9 Hz, 2H), 2.81 (s, 3H), 2.77 - 2.70 (m, 1H), 2.12 (ddd, *J* = 9.9, 5.1, 2.2 Hz, 1H), 1.76 (p, *J* = 6.6 Hz, 2H), 1.67 (d, *J* = 7.0 Hz, 2H), 1.64 (s, 6H), 1.60 (s, 5H), 1.30 (d, *J* = 35.5 Hz, 14H). LC/MS (ESI+) calcd for C₄₄H₄₉N₅O₇ ( [M+H]⁺ ) *m*/*z* 760.4, found 760.4

### Example 387: 5-((3-(4-(2-(4-((2-(1H-pyrazol-1-yl)pyrimidin-4-yl)methoxy) phenyl)propan-2-yl)phenoxy)propyl)amino)-2-(2,6-dioxopiperidin-3-yl)isoindol-1,3-dione

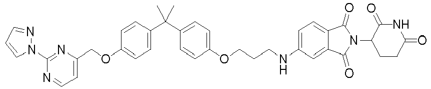

The target compound was synthesized by a method similar to that of Example 383. ¹H NMR (400 MHz, Chloroform-*d*) *δ* 8.73 (d, *J* = 5.1 Hz, 1H), 8.62 (d, *J* = 2.7 Hz, 1H), 8.03 (s, 1H), 7.87 - 7.81 (m, 1H), 7.60 (d, *J* = 8.3 Hz, 1H), 7.48 (d, *J* = 5.1 Hz, 1H), 7.20 - 7.13 (m, 4H), 6.98 (d, *J* = 2.1 Hz, 1H), 6.91 - 6.85 (m, 2H), 6.84 - 6.79 (m, 2H), 6.74 (dd, *J=* 8.3, 2.2 Hz, 1H), 6.52 (dd, *J* = 2.7, 1.6 Hz, 1H), 5.23 (s, 2H), 4.93 (dd, *J* = 12.2, 5.3 Hz, 2H), 4.09 (t, *J* = 5.5 Hz, 2H), 3.46 (t, *J* = 5.8 Hz, 2H), 2.93 - 2.66 (m, 3H), 2.16 - 2.10 (m, 2H), 1.64 (s, 6H). LC/MS (ESI+) calcd for C₃₉H₃₇N₇O₆ ( [M+H]⁺ ) *m*/*z* 700.3, found 700.3.

### Example 388: 2-((4-(2-(4-(3-((2-(2,6-dioxopiperidin-3-yl)-1,3-dioxoisoquinolin-5-yl)amino)propoxy)phenyl)propan-2-yl)phenoxy)methyl)oxazol-4-carbonylamine

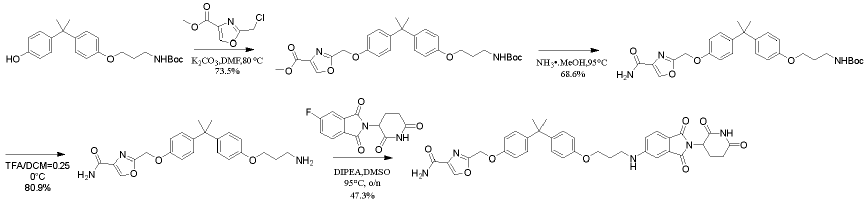

### Step 1: Synthesis of methyl 2-((4-(2-(4-(3-((tert-butoxycarbonyl)amino)propoxy) phenyl)propan-2-yl)phenoxy)methyl)oxazol-4-carboxylate

In a 25 mL conical flask, tert-butyl (3-(4-(2-(4-hydroxylphenyl)propan-2-yl)phenoxy)propyl)carbamate (300 mg, 0.778 mmol) was dissolved in 5 mL of DMF, to which were added methyl 2-(chloromethyl)oxazol-4-carboxylate (205 mg, 1.170 mmol) and anhydrous K₂CO₃ (323 mg, 2.330 mmol) under nitrogen protection, and then the mixture was stirred for 12 h at 80 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (300 mg, yield 73.5%).

### Step 2: Synthesis of tert-butyl (3-(4-(2-(4-((4-carbamoyl oxazol-2-yl)methoxy) phenyl)propan-2-yl)phenoxy)propyl)carbamate

In a 25 mL conical flask, methyl 2-((4-(2-(4-(3-((tert-butoxycarbonyl)amino) propoxy)phenyl)propan-2-yl)phenoxy)methyl)oxazol-4-carboxylate (300 mg, 0.572 mmol) was dissolved in 5 mL solutiono f ammonia in methanol, and then the mixture was stirred for 12 h at 95 °C in a sealed tube under nitrogen protection. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (200 mg, yield 68.6%).

### Step 3: Synthesis of 2-((4-(2-(4-(3-aminopropoxy)phenyl)propan-2-yl)phenoxy) methyl)oxazol-4-carbonylamine

In a 25 mL conical flask, tert-butyl (3-(4-(2-(4-((4-carbamoyloxazol-2-yl)methoxy)phenyl)propan-2-yl)phenoxy)propyl)carbamate (200 mg, 0.393 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (130 mg, yield 80.9%).

### Step 4: Synthesis of 2-((4-(2-(4-(3-((2-(2,6-dioxopiperidin-3-yl)-1,3-dioxoisoquinolin-5-yl)amino)propoxy)phenyl)propan-2-yl)phenoxy)methyl)oxazol-4-carbonylamine

In a 25 mL conical flask, 2-((4-(2-(4-(3-aminopropoxy)phenyl)propan-2-yl)phenoxy)methyl)oxazol-4-carbonylamine (130 mg, 0.318 mmol) was dissolved in 5 mL of DMSO, to which were added 2-(2,6-dioxopiperidin-3-yl)-5-fluoroisoindolin-1,3-dione (123 mg, 0.445 mmol) and N,N-diisopropylethylamine (164 mg, 1.270 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 47.3%). ¹H NMR (400 MHz, Chloroform-*d*) *δ* 8.41 (s, 1H), 8.26 (s, 1H), 8.09 (s, 1H), 7.59 (d, *J* = 8.3 Hz, 1H), 7.15 (d, *J* = 8.8 Hz, 5H), 6.98 (s, 1H), 6.89 (d, *J* = 8.9 Hz, 3H), 6.74 (d, *J* = 8.3 Hz, 1H), 5.74 (s, 1H), 5.12 (s, 2H), 4.09 (t, *J* = 5.4 Hz, 2H), 3.47 (d, *J* = 5.0 Hz, 2H), 2.92 - 2.71 (m, 3H), 2.17 - 2.08 (m, 3H), 1.25 (s, 6H). LC/MS (ESI+) calcd for C₃₆H₃₅N₅O₈ ([M+H]⁺) *m*/*z* 666.3, found 666.3.

### Example 389: 5-((3-(4-(2-(4-((2-(1H-1,2,4-triazol-1-yl)pyrimidin-4-yl)methoxy) phenyl)propan-2-yl)phenoxy)propyl)amino)-2-(2,6-dioxopiperidin-3-yl)isoindol-1,3-dione

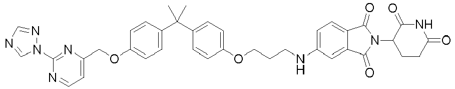

The target compound was synthesized by a method similar to that of Example 383. ¹H NMR (400 MHz, Chloroform-*d*) *δ* 9.27 (s, 1H), 8.80 (d, *J* = 5.0 Hz, 1H), 8.19 (s, 1H), 8.12 (d, *J* = 10.8 Hz, 1H), 7.63 (d, *J* = 5.0 Hz, 1H), 7.60 (d, *J* = 8.3 Hz, 1H), 7.20 - 7.11 (m, 4H), 6.98 (d, *J* = 2.1 Hz, 1H), 6.91 - 6.85 (m, 2H), 6.85 - 6.80 (m, 2H), 6.74 (dd, *J=* 8.3, 2.2 Hz, 1H), 5.25 (d, *J* = 0.9 Hz, 2H), 4.93 (dd, *J* = 12.1, 5.2 Hz, 2H), 4.09 (t, *J* = 5.5 Hz, 2H), 3.46 (q, *J* = 6.1 Hz, 2H), 2.93 - 2.65 (m, 3H), 2.14 (dt, *J=* 12.1, 6.5 Hz, 3H), 1.65 (s, 6H). LC/MS (ESI+) calcd for C₃₈H₃₆N₈O₆ ( [M+H]⁺ ) *m*/*z*: 701.3; found 701.3.

### Example 390: 2-(2,6-dioxopiperidin-3-yl)-5-((3-(4-(2-(methylamino)pyrimidin-4-yl)methoxy)phenyl)propan-2-yl)phenoxy)propyl)amino)isoindol-1,3-dione

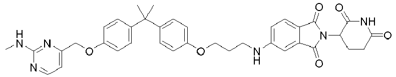

The target compound was synthesized by a method similar to that of Example 383. ¹H NMR (400 MHz, Chloroform-*d*) *δ* 8.25 (s, 1H), 7.56 (d, *J* = 8.3 Hz, 1H), 7.20 - 7.06 (m, 5H), 6.97 (d, *J* = 2.2 Hz, 1H), 6.86 - 6.80 (m, 5H), 6.74 (dd, *J* = 8.4, 2.2 Hz, 1H), 4.98 (d, *J* = 5.1 Hz, 2H), 4.92 (dt, *J* = 10.0, 5.0 Hz, 2H), 4.12 - 4.05 (m, 2H), 3.47 (h, *J=* 6.4 Hz, 2H), 3.04 (d, J = 4.9 Hz, 3H), 2.90 - 2.72 (m, 3H), 2.22 (t, *J* = 7.6 Hz, 2H), 1.64 (s, 6H). LC/MS (ESI+) calcd for C₃₇H₃₈N₆O₆ ( [M+H]⁺ ) *m*/*z* 663.3, found 663.3.

### Example 391: 5-((3-(4-(2-(4-((2-(1H-imidazol-1-yl)pyrimidin-4-yl)methoxy) phenyl)propan-2-yl)phenoxy)propyl)amino)-2-(2,6-dioxopiperidin-3-yl)isoindol-1,3-dione

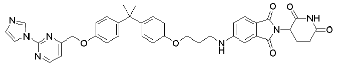

The target compound was synthesized by a method similar to that of Example 383. ¹H NMR (400 MHz, DMSO-*d*₆) *δ* 11.07 (s, 1H), 8.87 (d, *J=* 5.1 Hz, 1H), 8.58 (s, 1H), 7.94 (s, 1H), 7.59 - 7.50 (m, 2H), 7.21 (t, *J* = 5.4 Hz, 1H), 7.19 - 7.07 (m, 5H), 6.96 (d, *J* = 8.8 Hz, 3H), 6.85 (t, *J* = 7.7 Hz, 3H), 5.25 (s, 2H), 5.03 (dd, *J* = 13.0, 5.5 Hz, 1H), 4.03 (t, *J* = 6.1 Hz, 2H), 2.97 - 2.76 (m, 2H), 2.57 (d, *J=* 20.3 Hz, 2H), 2.33 (s, 1H), 2.05 - 1.92 (m, 4H), 1.58 (s, 6H). LC/MS (ESI+) calcd for C₃₉H₃₇N₇O₆ ( [M+H]⁺ ) *m*/*z* 700.3, found 700.3.

### Example 392: 2-(2,6-dioxopiperidin-3-yl)-5-((3-(4-(2-(methylamino)pyrimidin-4-yl)methoxy)phenyl)propan-2-yl)phenoxy)cyclobutyl)amino)isoindol-1,3-dione

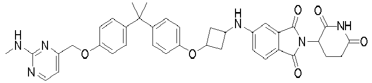

The target compound was synthesized by a method similar to that of Example 383. ¹H NMR (400 MHz, DMSO-*d*₆) *δ* 11.07 (s, 1H), 8.34 (d, *J=* 5.4 Hz, 1H), 8.01 (dd, *J* = 8.2, 4.5 Hz, 1H), 7.85 (d, *J* = 7.3 Hz, 1H), 7.72 (t, *J* = 9.4 Hz, 1H), 7.58 (d, *J* = 8.2 Hz, 1H), 7.11 (dt, *J* = 10.8, 5.3 Hz, 6H), 6.89 (d, *J* = 8.4 Hz, 4H), 5.05 (d, *J* = 5.3 Hz, 1H), 5.01 (s, 2H), 4.92 - 4.79 (m, 1H), 4.54 - 4.42 (m, 1H), 2.56 (d, *J* = 20.1 Hz, 2H), 2.45 (d, *J* = 13.0 Hz, 2H), 2.12 - 1.77 (m, 4H), 1.57 (s, 9H). LC/MS (ESI+) calcd for C₃₈H₃₆N₆O₆ ( [M+H]⁺ ) *m*/*z* 675.3, found 675.3.

### Example 393: 2-(2,6-dioxopiperidin-3-yl)-5-(3-(4-(2-(4-methylpiperazin-1-yl) pyrimidin-4-yl)methoxy)phenyl)propan-2-yl)phenoxy)cyclobutyl)amino)isoindol-1,3-dione

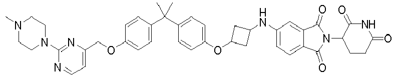

The target compound was synthesized by a method similar to that of Example 383. ¹H NMR (400 MHz, DMSO-*d*₆) *δ* 11.06 (s, 1H), 8.35 (d, *J* = 4.9 Hz, 1H), 7.59 (d, *J* = 8.3 Hz, 1H), 7.11 (t, *J* = 7.9 Hz, 4H), 6.91 - 6.84 (m, 3H), 6.83 - 6.70 (m, 3H), 6.68 (d, *J* = 4.9 Hz, 1H), 5.03 (dd, *J* = 12.9, 5.4 Hz, 1H), 4.95 (s, 2H), 4.86 (dt, *J* = 11.5, 5.7 Hz, 1H), 4.54 - 4.39 (m, 1H), 4.14 (d, *J* = 4.8 Hz, 1H), 3.73 - 3.65 (m, 4H), 2.62 - 2.52 (m, 2H), 2.43 (dd, *J* = 11.8, 6.7 Hz, 2H), 2.37 - 2.29 (m, 4H), 2.20 (s, 3H), 2.00 (d, *J=* 10.6 Hz, 2H), 1.57 (s, 6H), 1.23 (s, 2H). LC/MS (ESI+) calcd for C₄₂H₄₅N₇O₆ ([M+H]⁺ ) *m*/*z* 744.3, found 744.3.

### Example 394: 2-(2,6-dioxopiperidin-3-yl)-5-(3-(4-(2-(4-((2-morpholinylpyridin-4-yl)methoxy)phenyl)propan-2-yl)phenoxy)cyclobutyl)amino)isoindol-1,3-dione

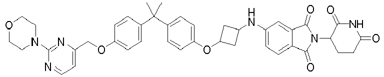

The target compound was synthesized by a method similar to that of Example 383. ¹H NMR (400 MHz, Chloroform-*d*) *δ* 8.32 (d, *J* = 5.2 Hz, 1H), 8.07 (s, 1H), 7.63 (d, *J* = 8.3 Hz, 1H), 7.15 - 7.10 (m, 4H), 6.93 (d, *J* = 2.1 Hz, 1H), 6.85 - 6.80 (m, 3H), 6.73 (dq, *J* = 8.4, 1.9 Hz, 3H), 4.96 (d, *J* = 3.0 Hz, 2H), 4.95 - 4.90 (m, 1H), 4.51 (p, *J* = 6.8 Hz, 1H), 3.85 (d, *J* = 5.0 Hz, 4H), 3.78 (dd, *J* = 5.6, 3.7 Hz, 5H), 3.16 - 3.08 (m, 2H), 2.91 - 2.71 (m, 3H), 2.13 (td, *J* = 10.6, 9.3, 6.4 Hz, 3H), 1.63 (s, 6H). LC/MS (ESI+) calcd for C₄₁H₄₂N₆O₇ ( [M+H]⁺ ) *m*/*z* 731.3, found: 731.3.

### Example 395: 2-(2,6-dioxopiperidin-3-yl)-5-((1r,3r)-3-(4-(2-(4-((2-(methylamino) pyrimidin-4-yl)methoxy)phenyl)propan-2-yl)phenoxy)cyclobutyl)amino)isoindol-1,3-dione

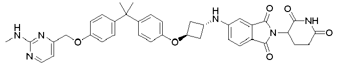

The target compound was synthesized by a method similar to that of Example 383. ¹H NMR (400 MHz, DMSO-*d*₆) *δ* 11.03 (s, 1H), 8.26 (s, 1H), 7.59 (d, *J* = 8.3 Hz, 1H), 7.53 (d, *J* = 5.4 Hz, 1H), 7.11 (dd, *J* = 8.8, 6.5 Hz, 5H), 6.93 - 6.83 (m, 3H), 6.79 (d, *J* = 9.5 Hz, 1H), 6.73 (d, *J* = 8.8 Hz, 2H), 6.62 (d, *J* = 4.9 Hz, 1H), 5.03 (dd, *J* = 12.8, 5.4 Hz, 1H), 4.92 (s, 2H), 4.85 (q, *J* = 7.9, 6.4 Hz, 1H), 4.19 - 4.07 (m, 1H), 2.87 (s, 1H), 2.79 (d, *J* = 4.8 Hz, 3H), 2.42 (dt, *J* = 12.4, 6.6 Hz, 2H), 2.00 (d, *J=* 7.8 Hz, 2H), 1.57 (s, 6H), 1.23 (s, 3H). LC/MS (ESI+) calcd for C₃₈H₃₆N₆O₆ ( [M+H]⁺ ) *m*/*z* 675.3, found 675.3.

### Example 396: 2-(2,6-dioxopyridin-3-yl)-5-(1s,3s)-3-(4-(2-(4-(2-morpholinylpyrimidin-4-yl)methoxy)phenyl)propan-2-yl)phenoxy)cyclobutyl) amino)isoindol-1,3-dione

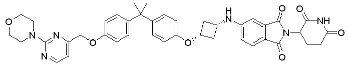

The target compound was synthesized by a method similar to that of Example 383. ¹H NMR (400 MHz, Chloroform-*d*) *δ* 8.33 (d, *J* = 5.2 Hz, 1H), 8.06 (s, 1H), 7.62 (d, *J* = 8.3 Hz, 1H), 7.16 - 7.11 (m, 4H), 6.92 (d, *J* = 2.1 Hz, 1H), 6.86 - 6.80 (m, 3H), 6.72 (dq, *J* = 8.4, 1.9 Hz, 3H), 4.96 (d, *J* = 3.0 Hz, 2H), 4.95 - 4.90 (m, 1H), 4.51 (p, *J* = 6.8 Hz, 1H), 3.87 (d, *J* = 5.0 Hz, 4H), 3.79 (dd, *J* = 5.6, 3.7 Hz, 5H), 3.16 - 3.08 (m, 2H), 2.91 - 2.71 (m, 3H), 2.11 (td, *J* = 10.6, 9.3, 6.4 Hz, 3H), 1.63 (s, 6H). LC/MS (ESI+) calcd for C₄₁H₄₂N₆O₇ ( [M+H]⁺ ) *m*/*z* 731.3, found 731.3.

### Example 397: 2-(2,6-dioxopiperidin-3-yl)-5-((1s,3s)-3-(4-(2-(4-((2-((R)-3-hydroxylpiperidin-1-yl)pyrimidin-4-yl)methoxy)phenyl)propan-2-yl)phenoxy) cyclobutyl)amino)isoindol-1,3-dione

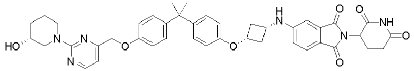

The target compound was synthesized by a method similar to that of Example 404. ¹H NMR (400 MHz, Chloroform-*d*) *δ* 8.27 (d, *J=* 5.3 Hz, 1H), 8.07 (s, 1H), 7.62 (d, *J* = 8.3 Hz, 1H), 7.17 - 7.10 (m, 4H), 6.91 (d, *J* = 2.1 Hz, 1H), 6.86 - 6.80 (m, 2H), 6.78 (d, *J* = 5.3 Hz, 1H), 6.71 (dq, *J=* 7.5, 3.3, 2.7 Hz, 3H), 4.96 (s, 2H), 4.95 - 4.88 (m, 1H), 4.74 (d, *J* = 6.2 Hz, 1H), 4.51 (p, *J* = 6.8 Hz, 1H), 3.94 (d, *J* = 27.1 Hz, 4H), 3.77 (d, *J* = 6.8 Hz, 2H), 3.17 - 3.08 (m, 2H), 2.92 - 2.71 (m, 3H), 2.11 (td, *J* = 12.2, 10.0, 7.3 Hz, 3H), 1.91 (d, *J* = 9.3 Hz, 2H), 1.81 - 1.70 (m, 2H), 1.63 (s, 6H). LC/MS (ESI+) calcd for C₄₂H₄₄N₆O₇ ( [M+H]⁺ ) *m*/*z* 745.3, found 745.3.

### Example 398: 2-(2,6-dioxopiperidin-3-yl)-5-(1s,3s)-3-(4-(2-(2-(R)-3-hydroxylpyrrolidin-1-yl)pyrimidin-4-yl)methoxy)phenyl)propan-2-yl)phenoxy) cyclobutyl)amino)isoindol-1,3-dione

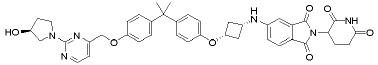

The target compound was synthesized by a method similar to that of Example 383. ¹H NMR (400 MHz, Chloroform-*d*) *δ* 8.37 (s, 1H), 8.30 (d, *J* = 5.1 Hz, 1H), 7.61 (d, *J* = 8.3 Hz, 1H), 7.12 (dq, *J* = 8.1, 3.3 Hz, 4H), 6.91 (d, *J* = 2.1 Hz, 1H), 6.87 - 6.81 (m, 2H), 6.74 (d, *J* = 5.1 Hz, 1H), 6.70 (dd, *J* = 8.6, 2.2 Hz, 3H), 4.96 (s, 2H), 4.91 (dd, *J* = 12.3, 6.8 Hz, 2H), 4.61 (d, *J* = 4.5 Hz, 1H), 4.50 (p, *J=* 6.9 Hz, 1H), 3.80 - 3.69 (m, 5H), 3.15 - 3.07 (m, 2H), 2.90 - 2.71 (m, 3H), 2.13 - 2.06 (m, 5H), 1.62 (s, 6H). LC/MS (ESI+) calcd for C₄₁H₄₂N₆O₇ ([M+H]⁺) *m*/*z* 731.3, found 731.3.

### Example 399: 2-(2,6-dioxopiperidin-3-yl)-5-((1s,3s)-3-(4-(2-(4-((2-((S)-3-hydroxylpiperidin-1-yl)pyrimidin-4-yl)methoxy)phenyl)propan-2-yl)phenoxy) cyclobutyl)amino)isoindol-1,3-dione

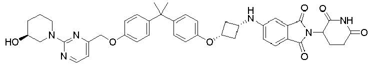

The target compound was synthesized by a method similar to that of Example 383. ¹H NMR (400 MHz, Chloroform-*d*) *δ* 8.27 (d, *J* = 5.3 Hz, 1H), 8.10 (s, 1H), 7.62 (d, *J* = 8.2 Hz, 1H), 7.16 - 7.10 (m, 4H), 6.91 (d, *J* = 2.1 Hz, 1H), 6.86 - 6.80 (m, 2H), 6.78 (d, *J* = 5.3 Hz, 1H), 6.74 - 6.67 (m, 3H), 4.96 (s, 2H), 4.94 - 4.87 (m, 1H), 4.76 (d, *J* = 6.2 Hz, 1H), 4.51 (p, *J* = 6.8 Hz, 1H), 3.94 (d, *J* = 29.5 Hz, 4H), 3.82 - 3.72 (m, 2H), 3.17 - 3.07 (m, 2H), 2.92 - 2.71 (m, 3H), 2.16 - 2.01 (m, 4H), 1.91 (d, *J* = 9.4 Hz, 3H), 1.63 (s, 6H). LC/MS (ESI+) calcd for C₄₂H₄₄N₆O₇ ( [M+H]⁺ ) *m*/*z* 745.3, found 745.3.

### Example 400: 5-((1s,3s)-3-(4-(2-(2-(3-(dimethylamino)azetidin-1-yl)pyrimidin-4-yl)methoxy)phenyl)propan-2-yl)phenoxy)cyclobutyl)amino)-2-(2,6-dioxopiperidin-3-yl)isoindolin-1,3-dione

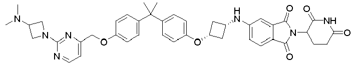

The target compound was synthesized by a method similar to that of Example 383. ¹H NMR (400 MHz, Chloroform-*d*) *δ* 8.23 (dd, *J* = 5.0, 1.3 Hz, 1H), 8.20 (s, 1H), 7.55 (d, *J* = 8.1 Hz, 1H), 7.10 - 7.00 (m, 4H), 6.85 (d, *J* = 2.3 Hz, 1H), 6.79 - 6.70 (m, 3H), 6.64 (d, *J* = 8.3 Hz, 3H), 4.87 (d, *J* = 3.7 Hz, 2H), 4.84 (d, *J* = 5.6 Hz, 1H), 4.74 (d, *J* = 6.2 Hz, 1H), 4.50 - 4.39 (m, 1H), 4.14 (t, *J* = 8.1 Hz, 2H), 4.02 (t, *J* = 7.2 Hz, 2H), 3.69 (q, *J* = 7.4 Hz, 1H), 3.28 (s, 1H), 3.10 - 2.98 (m, 2H), 2.84 - 2.64 (m, 3H), 2.26 (s, 6H), 2.02 (s, 2H), 1.56 (s, 6H). LC/MS (ESI+) calcd for C₄₂H₄₅N₇O₆ ([M+H]⁺) *m*/*z* 744.3, found 744.3.

### Example 401: 2-(2,6-dioxopiperidin-3-yl)-5-((1s,3s)-3-(4-(2-(4-((2-((R)-3-(methylamino)piperidin-1-yl)pyrimidin-4-yl)methoxy)phenyl)propan-2-yl) phenoxy)cyclobutyl)amino)isoindol-1,3-dione

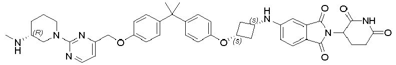

The target compound was synthesized by a method similar to that of Example 383. ¹H NMR (400 MHz, Chloroform-*d*) *δ* 8.28 (d, *J* = 5.0 Hz, 1H), 7.61 (d, *J* = 8.3 Hz, 1H), 7.16 - 7.10 (m, 4H), 6.91 (d, *J* = 2.1 Hz, 1H), 6.86 - 6.80 (m, 2H), 6.74 - 6.68 (m, 4H), 4.96 - 4.90 (m, 3H), 4.83 (d, *J* = 6.2 Hz, 1H), 4.71 (d, *J* = 13.3 Hz, 1H), 4.51 (t, *J* = 6.8 Hz, 1H), 4.36 (d, *J* = 13.3 Hz, 1H), 3.76 (q, *J* = 7.2 Hz, 1H), 3.39 - 3.32 (m, 1H), 3.23 (t, *J* = 11.0 Hz, 1H), 3.15 - 3.08 (m, 2H), 2.92 - 2.86 (m, 2H), 2.84 - 2.71 (m, 3H), 2.66 (s, 3H), 2.23 (s, 1H), 2.10 (t, *J* = 5.1 Hz, 2H), 1.86 (d, *J* = 10.2 Hz, 2H), 1.63 (s, 6H). LC/MS (ESI+) calcd for C₄₃H₄₇N₇O₆ ([M+H]⁺) *m*/*z* 758.4, found 758.4.

### Example 402: 5-((1s,3s)-3-(4-(2-(4-((2-(dimethylamino)piperidin-1-yl)pyrimidin-4-yl)methoxy)phenyl)propan-2-yl)phenoxy)cyclobutyl)amino)-2-(2,6-dioxopiperidin-3-yl)isoindol-1,3-dione

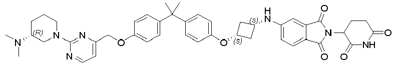

The target compound was synthesized by a method similar to that of Example 383. ¹H NMR (400 MHz, Chloroform-*d*) *δ* 8.28 (d, *J* = 5.0 Hz, 1H), 7.62 (d, *J* = 8.3 Hz, 1H), 7.13 (d, *J* = 8.8 Hz, 4H), 6.92 (d, *J* = 2.0 Hz, 1H), 6.83 (d, *J* = 8.8 Hz, 2H), 6.77 - 6.61 (m, 4H), 4.93 (d, *J* = 4.4 Hz, 4H), 4.79 (d, *J* = 6.3 Hz, 1H), 4.62 (d, *J* = 13.7 Hz, 1H), 4.52 (p, *J* = 6.9 Hz, 1H), 3.78 (q, *J* = 7.0 Hz, 1H), 3.19 - 3.07 (m, 2H), 3.04 - 2.68 (m, 6H), 2.53 (s, 6H), 2.17 - 2.05 (m, 4H), 1.86 (d, *J* = 13.4 Hz, 2H), 1.63 (s, 7H), 1.59 - 1.45 (m, 2H), 1.25 (s, 3H). LC/MS (ESI+) calcd for C₄₄H₄₉N₇O₆ ([M+H]⁺) *m*/*z* 772.4, found 772.4.

### Example 403: 2-(2,6-dioxopiperidin-3-yl)-5-((1s,3s)-3-(4-(2-(4-((2-((S)-3-(methylamino)piperidin-1-yl)pyrimidin-4-yl)methoxy)phenyl)propan-2-yl) phenoxy)cyclobutyl)amino)isoindol-1,3-dione

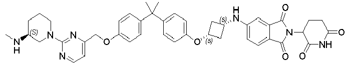

The target compound was synthesized by a method similar to that of Example 383. ¹H NMR (400 MHz, Chloroform-*d*) *δ* 8.28 (d, *J* = 5.0 Hz, 1H), 7.62 (d, *J* = 8.3 Hz, 1H), 7.12 (d, *J* = 8.8 Hz, 4H), 6.91 (d, *J* = 2.0 Hz, 1H), 6.83 (d, *J* = 8.8 Hz, 2H), 6.71 (dq, *J* = 7.6, 3.1, 2.7 Hz, 5H), 4.92 (s, 2H), 4.79 (d, *J=* 6.2 Hz, 1H), 4.65 (d, *J* = 12.6 Hz, 1H), 4.51 (p, *J* = 6.8, 6.4 Hz, 1H), 4.34 (d, *J=* 13.6 Hz, 1H), 3.81 - 3.73 (m, 1H), 3.32 - 3.20 (m, 2H), 3.15 - 3.07 (m, 2H), 2.92 - 2.76 (m, 4H), 2.61 (s, 3H), 2.15 - 2.06 (m, 4H), 1.63 (s, 6H), 1.55 (d, *J* = 14.0 Hz, 2H), 0.87 (d, *J* = 11.4 Hz, 2H). LC/MS (ESI+) calcd for C₄₃H₄₇N₇O₆ ( [M+H]⁺ ) *m*/*z:* 758.4; found 758.4.

### Example 404: 5-((1s,3s)-3-(4-(2-(4-((2-(dimethylamino)piperidin-1-yl)pyrimidin-4-yl)methoxy)phenyl)propan-2-yl)phenoxy)cyclobutyl)amino)-2-(2,6-dioxopiperidin-3-yl)isoindol-1,3-dione

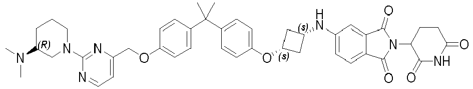

The target compound was synthesized by a method similar to that of Example 383. ¹H NMR (400 MHz, DMSO-*d*₆) *δ* 11.07 (s, 1H), 8.32 (d, *J* = 4.9 Hz, 1H), 7.58 (d, *J* = 8.3 Hz, 1H), 7.44 (d, *J* = 6.4 Hz, 1H), 7.16 - 7.05 (m, 4H), 6.88 (d, *J* = 8.9 Hz, 3H), 6.82 (d, *J* = 8.4 Hz, 1H), 6.76 (d, *J* = 8.8 Hz, 2H), 6.63 (d, *J* = 4.9 Hz, 1H), 5.03 (dd, *J* = 12.9, 5.3 Hz, 1H), 4.94 (s, 2H), 4.58 (d, *J* = 9.8 Hz, 1H), 4.49 (p, *J* = 7.1 Hz, 1H), 4.39 (d, *J* = 13.0 Hz, 1H), 3.10 - 3.00 (m, 2H), 3.00 - 2.78 (m, 4H), 2.63 - 2.51 (m, 2H), 2.22 (s, 6H), 2.02 - 1.85 (m, 4H), 1.57 (s, 6H), 1.49 - 1.32 (m, 2H), 1.23 (s, 2H). LC/MS (ESI+) calcd for C₄₄H₄₉N₇O₆ ( [M+H]⁺ ) *m*/*z* 772.4, found 772.4.

### Example 405: 5-((1s,3s)-3-(4-(2-(2-(1H-imidazol-1-yl)pyrimidin-4-yl)methoxy) phenyl)propan-2-yl)phenoxy)cyclobutyl)amino)-2-(2,6-dioxopiperidin-3-yl) isoindol-1,3-dione

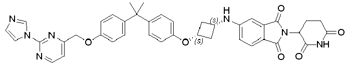

The target compound was synthesized by a method similar to that of Example 383. ¹H NMR (400 MHz, DMSO-*d*₆) *δ* 11.07 (s, 1H), 8.87 (d, *J* = 5.1 Hz, 1H), 8.57 (s, 1H), 7.93 (s, 1H), 7.58 (d, *J* = 8.3 Hz, 1H), 7.54 (d, *J* = 5.1 Hz, 1H), 7.44 (d, *J* = 6.4 Hz, 1H), 7.19 - 7.06 (m, 6H), 6.96 (d, *J* = 8.9 Hz, 2H), 6.90 (s, 1H), 6.82 (d, *J* = 6.6 Hz, 1H), 6.76 (d, *J* = 8.8 Hz, 2H), 5.25 (s, 2H), 5.04 (dd, *J* = 12.9, 5.4 Hz, 1H), 4.48 (p, *J* = 7.0 Hz, 1H), 3.77 (h, *J* = 6.9, 6.0 Hz, 1H), 3.05 (d, *J* = 9.3 Hz, 2H), 2.58 (d, *J* = 16.5 Hz, 2H), 1.96 (dt, *J* = 19.1, 8.2 Hz, 4H), 1.58 (s, 6H). LC/MS (ESI+) calcd for C₄₀H₃₇N₇O₆ ( [M+H]⁺) *m*/*z* 712.3, found 712.3.

### Example 406: 5-((1s,3s)-3-(4-(2-(2-(azetidin-3-ylmethyl)amino)pyrimidin-4-ylmethoxy)phenyl)propan-2-yl)phenoxy)cyclobutyl)amino)-2-(2,6-dioxopiperidin-3-yl)isoindolin-1,3-dione

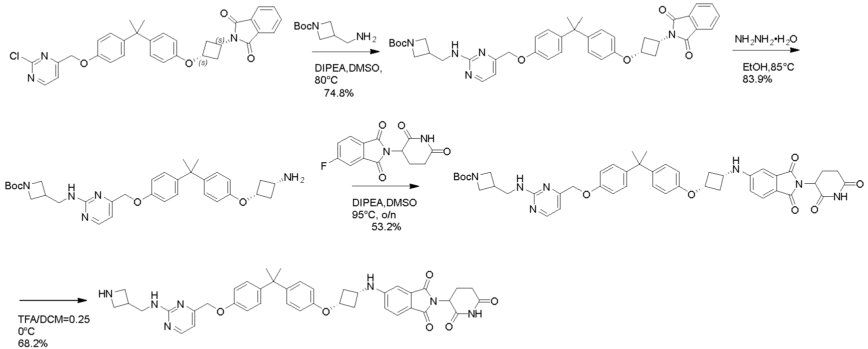

### Step 1: Synthesis of tert-butyl 3-((4-((4-(2-(4-((1s,3s)-3-(1,3-dioxoisoquinolin-2-yl)cyclobutyloxy)phenyl)propan-2-yl)phenoxy)methyl)pyrimidin-2-yl)amino)methyl) azetidine-1-carboxylate

In a 25 mL conical flask, 2-((1s,3s)-3-(4-(2-(4-hydroxylphenyl)propan-2-yl)phenoxy)cyclobutyl)isoindol-1,3-dione (200 mg, 0.361 mmol) was dissolved in 5 mL of DMSO, to which were added 3-(aminomethyl)azetidin-1-carboxylate tert-butyl(135 mg,0.722 mmol) and N,N-diisopropylethylamine (140 mg,1.080 mmol) under nitrogen protection, and then the mixture was stirred for 2 h at 80 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (190 mg, yield 74.8%).

### Step 2: Synthesis of tert-butyl 3-((4-((4-(2-(4-((1s,3s)-3-aminocyclobutyloxy)phenyl) propan-2-yl)phenoxy)methyl)pyrimidin-2-yl)amino)methyl)azetidin-1-carboxylate

In a 25 mL conical flask, tert-butyl 3-((4-((4-(2-(4-((1s,3s)-3-(1,3-dioxoisoquinolin-2-yl)cyclobutyloxy)phenyl)propan-2-yl)phenoxy)methyl)pyrimidin-2-yl)amino)methyl)azetidine-1-carboxylate (190 mg, 0.270 mmol) was dissolved in 10 mL of EtOH, to which was added 85% hydrazine hydrate (159 mg, 2.700 mmol) under nitrogen protection, and then the mixture was stirred for 1 h under refluxing at 85 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as off-white solid (130 mg, yield 83.9%).

### Step 3: Synthesis of tert-butyl 3-((4-((4-(2-(4-((1s,3s)-3-((2-(2,6-dioxopiperidin-3-yl)-1,3-dioxoisoindolin-5-yl)amino)cyclobutyloxy)phenyl)propan-2-yl)phenoxy)methyl) pyrimidin-2-yl)amino)methyl)azetidin-1-carboxylate

In a 25 mL conical flask, tert-butyl 3-((4-((4-(2-(4-((1s,3s)-3-aminocyclobutyloxy)phenyl)propan-2-yl)phenoxy)methyl)pyrimidin-2-yl)amino) methyl)azetidin-1-carboxylate (130 mg, 0.227 mmol) was dissolved in 5 mL of DMSO, to which were added 2-(2,6-dioxopiperidin-3-yl)-5-fluoroisoindolin-1,3-dione (88 mg, 0.317 mmol) and N,N-diisopropylethylamine (117 mg, 0.906 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 53.2%).

### Step 4: Synthesis of 5-((1s,3s)-3-(4-(2-(2-(azetidin-3-ylmethyl)amino)pyrimidin-4-ylmethoxy)phenyl)propan-2-yl)phenoxy)cyclobutyl)amino)-2-(2,6-dioxopiperidin-3-yl)isoindolin-1,3-dione

In a 25 mL conical flask, tert-butyl 3-((4-((4-(2-(4-((1s,3s)-3-((2-(2,6-dioxopiperidin-3-yl)-1,3-dioxoisoindolin-5-yl)amino)cyclobutyloxy)phenyl)propan-2-yl)phenoxy)methyl)pyrimidin-2-yl)amino)methyl)azetidin-1-carboxylate (50 mg, 0.061 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (30 mg, yield 68.2%). ¹H NMR (400 MHz, DMSO-*d*₆) *δ* 11.08 (s, 1H), 8.33 (d, J = 5.1 Hz, 1H), 7.58 (d, J = 8.3 Hz, 1H), 7.10 (t, J = 8.3 Hz, 5H), 6.89 (d, J = 8.7 Hz, 3H), 6.85 - 6.64 (m, 5H), 5.03 (dd, J = 12.9, 5.3 Hz, 1H), 4.97 (s, 2H), 4.90 - 4.80 (m, 1H), 4.53 - 4.42 (m, 1H), 4.13 (s, 1H), 3.83 - 3.71 (m, 1H), 2.94 - 2.81 (m, 2H), 2.61 - 2.53 (m, 2H), 1.95 (d, J = 21.7 Hz, 7H), 1.57 (s, 6H), 1.23 (s, 2H), 1.17 (t, J = 7.3 Hz, 1H). LC/MS (ESI+) calcd for C₄₁H₄₃N₇O₆ ([M+H]⁺) m/z 730.3, found 730.3.

### Example 407: 2-(2,6-dioxopiperidin-3-yl)-5-((1s,3s)-3-(4-(2-(2-((1-methylazetidin-3-yl)oxy)pyrimidin-4-yl)methoxy)phenyl)propan-2-yl)phenoxy)cyclobutyl) amino)isoindolin-1,3-dione

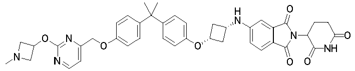

The target compound was synthesized by a method similar to that of Example 383. LC/MS (ESI+) calcd for C₄₁H₄₂N₆O₇ ([M+H]⁺) *m*/*z* 731.3, found 731.3.

### Example 408: 5-((1s,3s)-3-(4-(2-(4-((2-((2R,5R)-2,5-dimethylpiperazin-1-yl) pyrimidin-4-yl)methoxy)phenyl)propan-2-yl)phenoxy)cyclobutyl)amino)-2-(2,6-dioxopiperidin-3-yl)isoindol-1,3-dione

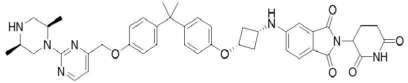

The target compound was synthesized by a method similar to that of Example 383. ¹H NMR (400 MHz, DMSO-*d*₆) δ 11.06 (s, 1H), 8.34 (d, *J* = 4.9 Hz, 1H), 7.59 (d, *J* = 8.3 Hz, 1H), 7.44 (d, *J* = 6.6 Hz, 1H), 7.10 (t, *J* = 8.4 Hz, 4H), 6.89 (d, *J* = 8.8 Hz, 3H), 6.82 (dd, *J* = 8.4, 1.8 Hz, 1H), 6.76 (d, *J* = 8.8 Hz, 2H), 6.66 (d, *J =* 4.9 Hz, 1H), 5.03 (dd, *J* = 12.8, 5.4 Hz, 1H), 4.95 (s, 2H), 4.75 - 4.66 (m, 1H), 4.53 - 4.44 (m, 1H), 4.36 (dd, *J* = 12.6, 2.5 Hz, 1H), 3.77 (q, *J* = 7.3 Hz, 1H), 3.05 (d, *J* = 11.3 Hz, 2H), 2.83 (s, 4H), 2.74 - 2.52 (m, 4H), 1.57 (s, 6H), 1.23 (s, 2H), 1.14 (d, *J =* 6.7 Hz, 3H), 1.05 (d, *J =* 6.1 Hz, 3H), 0.90 - 0.78 (m, 1H). LC/MS (ESI+) calcd for C₄₃H₄₇N₇O₆ ( [M+H]⁺ ) *m*/*z* 758.4, found 758.4.

### Example 409: 2-(2,6-dioxopiperidin-3-yl)-5-((1s,3s)-3-(4-(4-((2-((2R,5R)-2,4,5-trimethylpiperazin-1-yl)pyrimidin-4-yl)methoxy)phenyl)propan-2-yl)phenoxy) cyclobutyl)amino)isoindol-1,3-dione

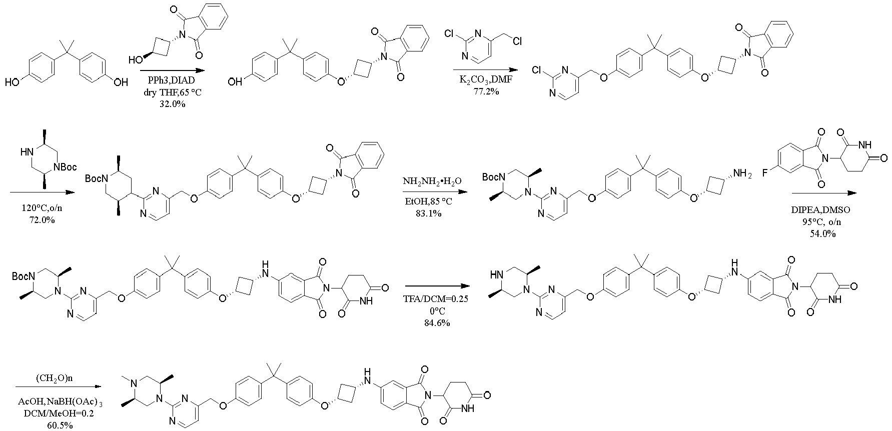

### Step 1: Synthesis of 2-((1s,3s)-3-(4-(2-(4-hydroxylphenyl)propan-2-yl)phenoxy) cyclobutyl)isoindol-1,3-dione

In a 25 mL conical flask, 4,4'-(propan-2,2-diyl)diphenol (2.000 g, 8.760 mmol) was dissolved in 10 mL of THF, and then the solution was cooled to 0 °C in an ice water bath, to which were added 2-((1r,3r)-3-hydroxylcyclobutyl)isoindolin-1,3-dione (2.280 g, 10.510 mmol), triphenylphosphine (3.450 g, 13.140 mmol), and diisopropyl azodicarboxylate (2.660 g, 13.140 mmol) under nitrogen protection. The reaction solution was stirred for 12 h at 65 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (1.200 g, yield 32.0%).

### Step 2: Synthesis of 2-((1s,3s)-3-(4-(2-(4-hydroxylphenyl)propan-2-yl)phenoxy) cyclobutyl)isoindol-1,3-dione

In a 25 mL conical flask, 2-((1s,3s)-3-(4-(2-(4-hydroxylphenyl)propan-2-yl)phenoxy)cyclobutyl)isoindol-1,3-dione (1.200 g, 2.810 mmol) was dissolved in 5 mL of DMF, to which were added 2-chloro-4-(chloromethyl)pyrimidine (549 mg, 3.370 mmol) and K₂CO₃ (776 mg, 5.610 mmol) under nitrogen protection. The reaction solution was stirred for 12 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (1.200 g, yield 77.2%).

### Step 3: Synthesis of tert-butyl (2S,5R)-4-(4-(4-(2-(4-((1s,3s)-3-(1,3-dioxoisoquinolin-2-yl)cyclobutyloxy)phenyl)propan-2-yl)phenoxy)methyl)pyrimidin-2-yl)-2,5-dimethylpiperidin-1-carboxylate

To a 25 mL conical flask, were added 2-((1s,3s)-3-(4-(2-(4-hydroxylphenyl)propan-2-yl)phenoxy)cyclobutyl)isoindol-1,3-dione (200 mg, 0.361 mmol) and tert-butyl (2S,5S)-2,5-dimethylpiperazin-1-carboxylate (116 mg, 0.542 mmol), and then the reaction solution was stirred for 12 h at 120 °C under nitrogen protection. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (190 mg, yield 72.0%).

### Step 4: Synthesis of tert-butyl (2R,5R)-4-(4-(4-(2-(4-((1s,3s)-3-aminocyclobutyloxy) phenyl)propan-2-yl)phenoxy)methyl)pyrimidin-2-yl)-2,5-dimethylpiperazin-1-carboxylate

In a 25 mL conical flask, tert-butyl (2S,5R)-4-(4-(4-(2-(4-((1s,3s)-3-(1,3-dioxoisoquinolin-2- yl)cyclobutyloxy)phenyl)propan-2-yl)phenoxy)methyl)pyrimidin-2-yl)-2,5-dimethylpiperidin-1-carboxylate (190 mg, 0.260 mmol) was dissolved in 10 mL of EtOH, to which was added 85% hydrazine hydrate (153 mg, 2.600 mmol) under nitrogen protection, and then the mixture was stirred for 1 h under refluxing at 85 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (130 mg, yield 83.1%).

### Step 5: Synthesis of tert-butyl (2R,5R)-4-(4-(4-(2-(4-((1s,3s)-3-((2-(2,6-dioxopiperidin-3-yl)-1,3-dioxoisoquinolin-5-yl)amino)cyclobutyloxy)phenyl)propan-2-yl)phenoxy)methyl)pyrimidin-2-yl)-2,5-dimethylpiperazin-1-carboxylate

In a 25 mL conical flask, tert-butyl (2R,5R)-4-(4-(4-(2-(4-((1s,3s)-3-aminocyclobutyloxy)phenyl)propan-2-yl)phenoxy)methyl)pyrimidin-2-yl)-2,5-dimethylpiperazin-1-carboxylate (130 mg, 0.216 mmol) was dissolved in 5 mL of DMSO, to which were added 2-(2,6-dioxopiperidin-3-yl)-5-fluoroisoindolin-1,3-dione (88 mg, 0.302 mmol) and N,N-diisopropylethylamine (112 mg, 0.864 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 54.0%).

### Step 6: Synthesis of 5-((1s,3s)-3-(4-(2-(4-((2R,5R)-2,5-dimethylpiperazin-1-yl) pyrimidin-4-yl)methoxy)phenyl)propan-2-yl)phenoxy)cyclobutyl)amino)-2-(2,6-dioxopiperidin-3-yl)isoindolin-1,3-dione

In a 25 mL conical flask, tert-butyl (2R,5R)-4-(4-(4-(2-(4-((1s,3s)-3-((2-(2,6-dioxopiperidin-3-yl)-1,3-dioxoisoquinolin-5-yl)amino)cyclobutyloxy)phenyl)propan-2-yl)phenoxy)methyl)pyrimidin-2-yl)-2,5-dimethylpiperazin-1-carboxylate (100 mg, 0.117 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (75 mg, yield 84.6%).

### Step 7: Synthesis of 2-(2,6-dioxopiperidin-3-yl)-5-((1s,3s)-3-(4-(4-((2-((2R,5R)-2,4,5-trimethylpiperazin-1-yl)pyrimidin-4-yl)methoxy)phenyl)propan-2-yl)phenoxy) cyclobutyl)amino)isoindol-1,3-dione

In a 25 mL conical flask, 5-((1s,3s)-3-(4-(2-(4-((2R,5R)-2,5-dimethylpiperazin-1-yl)pyrimidin-4-yl)methoxy)phenyl)propan-2-yl)phenoxy)cyclobutyl)amino)-2-(2,6-dioxopiperidin-3-yl)isoindolin-1,3-dione (40 mg, 0.053 mmol) was dissolved in 2 mL of MeOH, and then the solution was cooled to 0 °C in an ice water bath, to which were added 0.4 mL of DCM, paraformaldehyde (16 mg, 0.530 mmol), and acetic acid (4 mg, 0.064 mmol) under nitrogen protection. The reaction solution was stirred for 30 min at 0 °C, to which was added sodium triacetoxyborohydride (15 mg,0.069 mmol), and then the solution was stirred for 12 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (25 mg, yield 60.4%). ¹H NMR (400 MHz, DMSO-*d*₆) *δ* 11.07 (s, 1H), 8.34 (d, *J* = 4.9 Hz, 1H), 7.58 (d, *J* = 8.3 Hz, 1H), 7.45 (d, *J* = 6.4 Hz, 1H), 7.10 (t, *J* = 8.2 Hz, 4H), 6.89 (d, *J* = 8.9 Hz, 3H), 6.82 (d, *J* = 10.3 Hz, 1H), 6.76 (d, *J* = 8.8 Hz, 2H), 6.67 (d, *J* = 4.9 Hz, 1H), 5.03 (dd, *J* = 12.9, 5.4 Hz, 1H), 4.95 (s, 2H), 4.79 - 4.69 (m, 1H), 4.53 - 4.43 (m, 1H), 4.31 (dd, *J* = 13.2, 3.0 Hz, 1H), 3.77 (q, *J* = 7.2 Hz, 1H), 3.04 (d, *J* = 4.3 Hz, 2H), 2.94 - 2.79 (m, 1H), 2.72 - 2.63 (m, 2H), 2.57 (d, *J* = 16.4 Hz, 2H), 2.19 (dd, *J=* 11.5, 4.0 Hz, 1H), 2.16 (s, 3H), 2.02 - 1.94 (m, 2H), 1.57 (s, 6H), 1.16 (d, *J* = 6.7 Hz, 3H), 1.05 (d, *J* = 6.1 Hz, 3H). LC/MS (ESI+) calcd for C₄₄H₄₉N₇O₆ ( [M+H]⁺ ) *m*/*z* 772.4, found 772.4.

### Example 410: 5-((1s,3s)-3-(4-(2-(2-(2-([1,3'-diazetidine]-1'-yl)pyrimidin-4-yl) methoxy)phenyl)propan-2-yl)phenoxy)cyclobutyl)amino)-2-(2,6-dioxopiperidin-3-yl)isoindol-1,3-dione

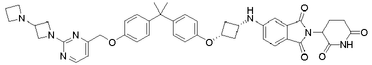

The target compound was synthesized by a method similar to that of Example 383. ¹H NMR (400 MHz, DMSO-*d*₆) *δ* 11.07 (s, 1H), 8.32 (d, *J*= 5.0 Hz, 1H), 7.58 (d, *J*= 8.3 Hz, 1H), 7.45 (d, *J*= 6.5 Hz, 1H), 7.10 (t, *J*= 8.5 Hz, 4H), 6.89 (d, *J* = 8.9 Hz, 3H), 6.82 (d, *J* = 10.2 Hz, 1H), 6.76 (d, *J* = 8.8 Hz, 2H), 6.72 (d, *J* = 5.0 Hz, 1H), 5.03 (dd, *J* = 12.9, 5.4 Hz, 1H), 4.94 (s, 2H), 4.49 (p, *J* = 7.1 Hz, 1H), 4.04 - 3.95 (m, 2H), 3.76 (dd, *J* = 9.2, 4.4 Hz, 3H), 3.18 (t, *J* = 6.9 Hz, 4H), 2.94 - 2.80 (m, 1H), 2.09 - 1.84 (m, 6H), 1.57 (s, 6H), 1.25 (d, *J=* 14.3 Hz, 4H). LC/MS (ESI+) calcd for C₄₃H₄₅N₇O₆ ([M+H]⁺) *m*/*z* 756.3, found 756.3.

### Example 411: 5-((1s,3s)-3-(4-(2-(4-((2-((2R,5S)-2,5-dimethylpiperazin-1-yl) pyrimidin-4-yl)methoxy)phenyl)propan-2-yl)phenoxy)cyclobutyl)amino)-2-(2,6-dioxopiperidin-3-yl)isoindol-1,3-dione

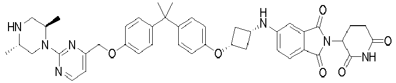

The target compound was synthesized by a method similar to that of Example 409. ¹H NMR (400 MHz, DMSO-*d*₆) *δ* 11.07 (s, 1H), 8.34 (d, *J*= 4.9 Hz, 1H), 7.59 (d, *J*= 8.3 Hz, 1H), 7.45 (d, *J* = 6.4 Hz, 1H), 7.10 (t, *J*= 8.1 Hz, 5H), 6.89 (d, *J*= 8.8 Hz, 3H), 6.82 (d, *J*= 10.3 Hz, 1H), 6.76 (d, *J=* 8.8 Hz, 2H), 6.67 (d, *J=* 4.9 Hz, 1H), 5.03 (dd, *J=* 12.9, 5.4 Hz, 1H), 4.95 (s, 2H), 4.77 - 4.68 (m, 1H), 4.48 (p, *J* = 7.2, 6.5 Hz, 2H), 4.22 (d, *J* = 12.5 Hz, 1H), 3.82 - 3.71 (m, 2H), 2.96 - 2.79 (m, 2H), 2.69 - 2.51 (m, 4H), 2.05 - 1.80 (m, 4H), 1.57 (s, 6H), 1.23 (s, 1H), 1. 17 (d, *J* = 6.8 Hz, 3H), 1.08 (d, *J*= 6.6 Hz, 3H). LC/MS (ESI+) calcd for C₄₃H₄₇N₇O₆ ( [M+H]⁺ ) *m*/*z* 758.4, found 758.4.

### Example 412: 2-(2,6-dioxopiperidin-3-yl)-5-((1s,3s)-3-(4-(4-((2-((2R,5S)-2,4,5-trimethylpiperazin-1-yl)pyrimidin-4-yl)methoxy)phenyl)propan-2-yl)phenoxy) cyclobutyl)amino)isoindol-1,3-dione

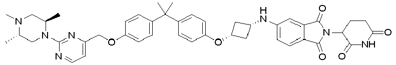

The target compound was synthesized by a method similar to that of Example 409. ¹H NMR (400 MHz, DMSO-*d*₆) *δ* 11.07 (s, 1H), 8.33 (d, *J*= 4.9 Hz, 1H), 7.58 (d, *J*= 8.3 Hz, 1H), 7.45 (d, *J*= 6.5 Hz, 1H), 7.10 (dd, *J=* 8.8, 6.7 Hz, 4H), 6.89 (d, *J* = 8.8 Hz, 3H), 6.82 (d, *J* = 8.3 Hz, 1H), 6.76 (d, *J* = 8.8 Hz, 2H), 6.64 (d, *J =* 4.9 Hz, 1H), 5.03 (dd, *J* = 12.9, 5.4 Hz, 1H), 4.94 (s, 2H), 4.75 (dt, *J=* 9.9, 5.1 Hz, 1H), 4.49 (p, *J* = 7.0 Hz, 1H), 4.30 (d, *J=* 13.2 Hz, 1H), 3.77 (q, *J=* 7.3 Hz, 1H), 3.04 (d, *J* = 6.8 Hz, 2H), 2.96 - 2.83 (m, 2H), 2.66 (dd, *J* = 11.7, 4.5 Hz, 1H), 2.63 - 2.52 (m, 2H), 2.29 (d, *J* = 12.1 Hz, 1H), 2.22 (s, 3H), 2.04 - 1.88 (m, 4H), 1.57 (s, 6H), 1.16 (d, *J=* 6.7 Hz, 3H), 0.82 (d, *J* = 6.5 Hz, 3H).
LC/MS (ESI+) calcd for C₄₄H₄₉N₇O₆ ( [M+H]⁺ ) *m*/*z* 772.4, found 772.4.

### Example 413: 5-((1r,3r)-3-(4-(2-(4-((6-(2-oxa-6-azaspiro[3.3]heptane-6-yl) pyridin-2-yl)methoxy)phenyl)propan-2-yl)phenoxy)cyclobutyl)amino)-2-(2,6-dioxopyridin-3-yl)isoindol-1,3-dione

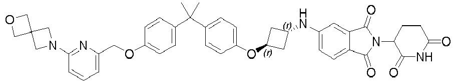

The target compound was synthesized by a method similar to that of Example 383. ¹H NMR (400 MHz, DMSO-*d*₆) *δ* 11.07 (s, 1H), 7.59 (d, *J* = 8.3 Hz, 1H), 7.56 - 7.48 (m, 2H), 7.10 (d, *J* = 8.2 Hz, 4H), 6.94 - 6.84 (m, 3H), 6.79 (d, *J*= 9.2 Hz, 1H), 6.73 (dd, *J*= 8.0, 2.9 Hz, 3H), 6.31 (d, *J* = 8.2 Hz, 1H), 5.04 (dd, *J* = 12.9, 5.4 Hz, 1H), 4.93 (s, 2H), 4.86 (p, *J* = 6.6 Hz, 1H), 4.71 (s, 4H), 4.17 - 4.12 (m, 1H), 4.09 (s, 4H), 2.55 (d, *J* = 5.8 Hz, 2H), 2.43 (dd, *J =* 11.1, 6.3 Hz, 2H), 2.00 (d, *J=* 7.8 Hz, 2H), 1.57 (s, 6H), 1.23 (s, 2H). LC/MS (ESI+) calcd for C₄₃H₄₃N₅O₆ ( [M+H]⁺ ) *m*/*z* 742.3, found 742.3

### Example 414: 5-((1r,3r)-3-(4-(2-(4-(2-([1,3'-diazetidine]-1'-yl)pyrimidin-4-yl)methoxy)phenyl)propan-2-yl)phenoxy)cyclobutyl)amino)-2-(2,6-dioxopiperidin-3-yl)isoindol-1,3-dione

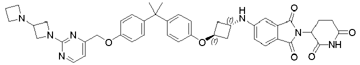

The target compound was synthesized by a method similar to that of Example 383. ¹H NMR (400 MHz, DMSO-*d*₆) *δ* 11.06 (s, 1H), 8.32 (d, *J*= 5.0 Hz, 1H), 7.59 (d, *J*= 8.3 Hz, 1H), 7.53 (d, *J* = 5.4 Hz, 1H), 7.11 (dd, *J* = 8.6, 6.6 Hz, 4H), 6.93 - 6.83 (m, 3H), 6.79 (d, *J* = 8.1 Hz, 1H), 6.75 - 6.66 (m, 3H), 5.03 (dd, *J* = 12.9, 5.4 Hz, 1H), 4.94 (s, 2H), 4.89 - 4.81 (m, 1H), 4.14 (d, *J* = 5.0 Hz, 1H), 4.03 - 3.94 (m, 2H), 3.75 (dd, *J* = 9.2, 4.3 Hz, 2H), 3.44 (s, 1H), 3.15 (t, *J*= 7.0 Hz, 4H), 2.94 - 2.80 (m, 1H), 2.60 - 2.52 (m, 3H), 2.43 (dd, *J* = 12.2, 5.5 Hz, 3H), 1.97 (p, *J* = 7.1 Hz, 3H), 1.57 (s, 6H). LC/MS (ESI+) calcd for C₄₃H₄₅N₇O₆ ( [M+H]⁺ ) *m*/*z* 756.3, found 756.3.

### Example 415: 5-((1r,3r)-3-(4-(2-(4-(6-(1H-1,2,3-triazol-1-yl)pyridin-2-yl) methoxy)phenyl)propan-2-yl)phenoxy)cyclobutyl)amino)-2-(2,6-dioxopiperidin-3-yl)isoindolin-1,3-dione

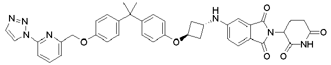

The target compound was synthesized by a method similar to that of Example 383. ¹H NMR (400 MHz, DMSO-*d*₆) *δ* 11.05 (s, 1H), 8.81 (d, *J* = 1.0 Hz, 1H), 8.16 (t, *J*= 7.8 Hz, 1H), 8.07 (d, *J* = 8.0 Hz, 1H), 8.00 (s, 1H), 7.65 (d, *J* = 7.5 Hz, 1H), 7.59 (d, *J* = 8.3 Hz, 1H), 7.54 (d, *J* = 5.4 Hz, 1H), 7.18 - 7.06 (m, 4H), 6.96 (d, *J* = 8.8 Hz, 2H), 6.86 (s, 1H), 6.79 (d, *J* = 9.1 Hz, 1H), 6.73 (d, *J*= 8.7 Hz, 2H), 5.23 (s, 2H), 5.03 (dd, *J*= 12.9, 5.4 Hz, 1H), 4.90 - 4.78 (m, 1H), 4.14 (d, *J*= 4.5 Hz, 1H), 3.30 (s, 1H), 2.99 - 2.71 (m, 1H), 2.62 - 2.51 (m, 2H), 2.43 (dd, *J*= 11.1, 6.3 Hz, 2H), 2.07 - 1.88 (m, 1H), 1.58 (s, 6H), 1.23 (s, 1H).
LC/MS (ESI+) calcd for C₄₀H₃₇N₇O₆ ([M+H]⁺) *m*/*z* 712.3, found 712.3.

### Example 416: 2-(2,6-dioxopiperidin-3-yl)-5-((1r,3r)-3-(4-(2-(4-((6-(1-methyl-1H-pyrazol-4-yl)pyridin-2-yl)methoxy)phenyl)propan-2-yl)phenoxy)cyclobutyl) amino)isoindolin-1,3-dione

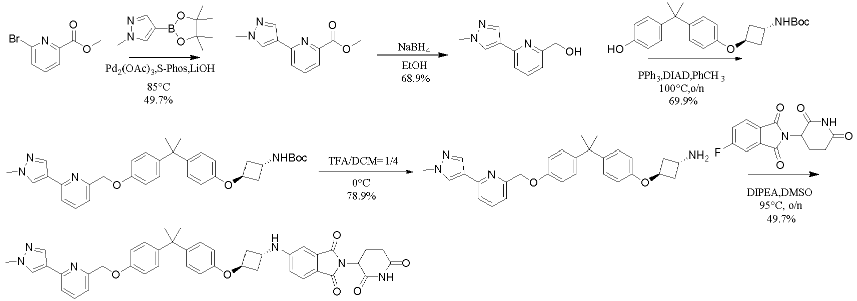

### Step 1: Synthesis of methyl 6-(1-methyl-1H-pyrazol-4-yl)picolinate

In a 25 mL conical flask, methyl 6-bromopicolinate (1.000 g, 4.629 mmol) was dissolved in 10 mL of 1,4-dioxane, to which were added 1-methyl-4-(4,4,5,5-tetramethyl-1,3,2-2-dioxaborolan-2-yl)-1H-pyrazole (1.156 g, 5.555 mmol), palladium acetate (52 mg, 0.231 mmol), S-phos (190 mg,0.462 mmol), and 2 mol/L LiOH (2 mL) under nitrogen protection, and then the reaction solution was stirred for 12 h at 85 °C, followed by filtration. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (500 mg, yield 49.7%).

### Step 2: Synthesis of (6-(1-methyl-1H-pyrazol-4-yl)pyridin-2-yl)methanol

In a 25 mL conical flask, methyl 6-(1-methyl-1H-pyrazol-4-yl)picolinate (0.500 g, 2.300 mmol) was dissolved in 5 mL of EtOH, and then the solution was cooled to 0 °C in an ice water bath, to which was added sodium borohydride (435 mg, 11.51 mmol) under nitrogen protection. The reaction solution was stirred for 2 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (300 mg, yield 68.9%).

### Step 3: Synthesis of tert-butyl ((1r,3r)-3-(4-(2-(4-((6-(1-methyl-1H-pyrazol-4-yl)pyridin-2-yl)methoxy)phenyl)propan-2-yl)phenoxy)cyclobutyl)carbamate

In a 25 mL conical flask, tert-butyl ((1r,3r)-3-(4-(2-(4-hydroxylphenyl)propan-2-phenoxy)cyclobutyl)carbamate (200 mg, 0.503 mmol) was dissolved in 10 mL of THF, and then the solution was cooled to 0 °C in an ice water bath, to which were added (6-(1-methyl-1H-pyrazol-4-yl)pyridin-2-yl)methanol (114 mg, 10.604 mmol), triphenylphosphine (198 mg, 0.755 mmol), and diisopropyl azodicarboxylate (153 g, 0.755 mmol) under nitrogen protection. The reaction solution was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (200 mg, yield 69.9%).

### Step 4: Synthesis of (1r,3r)-3-(4-(2-(4-((6-(1-methyl-1H-pyrazol-4-yl)pyridin-2-yl)methoxy)phenyl)propan-2-yl)phenoxy)cyclobutylamine

In a 25 mL conical flask, tert-butyl ((1r,3r)-3-(4-(2-(4-((6-(1-methyl-1H-pyrazol-4-yl)pyridin-2-yl)methoxy)phenyl)propan-2-yl)phenoxy)cyclobutyl)carbamate(200 mg,0.352 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (130 mg, yield 78.9%).

### Step 5: Synthesis of 2-(2,6-dioxopiperidin-3-yl)-5-((1r,3r)-3-(4-(2-(4-((6-(1-methyl-1H-pyrazol-4-yl)pyridin-2-yl)methoxy)phenyl)propan-2-yl)phenoxy)cyclobutyl) amino)isoindolin-1,3-dione

In a 25 mL conical flask, (1r,3r)-3-(4-(2-(4-((6-(1-methyl-1H-pyrazol-4-yl)pyridin-2-yl)methoxy)phenyl)propan-2-yl)phenoxy)cyclobutylamine(130 mg,0.277 mmol) was dissolved in 5 mL of DMSO, to which were added 2-(2,6-dioxopiperidin-3-yl)-5-fluoroisoindolin-1,3-dione (107 mg, 0.388 mmol) and N,N-diisopropylethylamine (143 mg, 1.110 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 49.7%). ¹H NMR (400 MHz, DMSO-*d*₆) *δ* 11.07 (s, 1H), 8.28 (s, 1H), 7.99 (s, 1H), 7.78 (t, *J* = 7.8 Hz, 1H), 7.58 (dd, *J* = 7.8, 5.3 Hz, 2H), 7.53 (d, *J* = 5.5 Hz, 1H), 7.27 (d, *J* = 7.4 Hz, 1H), 7.12 (t, *J* = 8.6 Hz, 4H), 6.93 (d, *J* = 8.9 Hz, 2H), 6.86 (s, 1H), 6.79 (d, *J* = 9.6 Hz, 1H), 6.73 (d, *J*= 8.8 Hz, 2H), 5.12 (s, 2H), 5.03 (dd, *J*= 12.9, 5.3 Hz, 1H), 4.90 - 4.81 (m, 1H), 4.14 (d, *J*= 5.1 Hz, 1H), 3.88 (s, 3H), 2.62 - 2.53 (m, 2H), 2.00 (d, *J*= 7.8 Hz, 2H), 1.58 (s, 6H), 1.23 (s, 4H). LC/MS (ESI+) calcd for C₄₂H₄₀N₆O₆ ( [M+H]⁺ ) *m*/*z* 725.3, found 725.3.

### Example 417: 2-(2,6-dioxopiperidin-3-yl)-5-((1s,3s)-3-(4-(2-(2-(1-methyl-1H-pyrazol-5-yl)pyrimidin-4-yl)methoxy)phenyl)propan-2-yl)phenoxy)cyclobutyl) amino)isoindol-1,3-dione

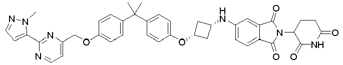

The target compound was synthesized by a method similar to that of Example 416. ¹H NMR (400 MHz, DMSO-*d*₆) *δ* 11.07 (s, 1H), 8.91 (d, *J*= 5.1 Hz, 1H), 7.59 (d, *J*= 8.4 Hz, 1H), 7.53 (d, *J* = 1.9 Hz, 1H), 7.50 (d, *J* = 5.1 Hz, 1H), 7.44 (d, *J* = 6.6 Hz, 1H), 7.12 (dd, *J* = 16.7, 8.8 Hz, 4H), 6.99 (d, *J* = 1.9 Hz, 1H), 6.95 (d, *J* = 8.9 Hz, 2H), 6.90 (s, 1H), 6.82 (d, *J* = 8.0 Hz, 1H), 6.76 (d, *J* = 8.8 Hz, 2H), 5.26 (s, 2H), 5.03 (dd, *J* = 13.0, 5.4 Hz, 1H), 4.53 - 4.44 (m, 1H), 4.21 (s, 3H), 3.82 - 3.74 (m, 1H), 3.11 - 3.00 (m, 2H), 1.58 (s, 6H), 1.30 - 1.13 (m, 6H). LC/MS (ESI+) calcd for C₄₁H₃₉N₇O₆ ( [M+H]⁺ ) *m*/*z* 726.30, found 726.3.

### Example 418: 2-(2,6-dioxopiperidin-3-yl)-5-((1s,3s)-3-(4-(2-(4-((6-(1-methyl-1H-pyrazol-4-yl)pyridin-2-yl)methoxy)phenyl)propan-2-yl)phenoxy)cyclobutyl) amino)isoindolin-1,3-dione

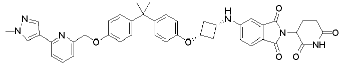

The target compound was synthesized by a method similar to that of Example 416. ¹H NMR (400 MHz, DMSO-*d*₆) *δ* 11.06 (s, 1H), 8.27 (s, 1H), 7.98 (s, 1H), 7.78 (t, *J*= 7.8 Hz, 1H), 7.58 (dd, *J* = 7.9, 4.4 Hz, 2H), 7.44 (d, *J* = 6.4 Hz, 1H), 7.27 (d, *J* = 7.4 Hz, 1H), 7.11 (t, *J* = 9.4 Hz, 4H), 6.96 - 6.87 (m, 3H), 6.82 (d, *J=* 10.3 Hz, 1H), 6.76 (d, *J=* 8.8 Hz, 2H), 5.12 (s, 2H), 5.03 (dd, *J* = 13.0, 5.4 Hz, 1H), 4.52 - 4.44 (m, 1H), 3.88 (s, 3H), 3.82 - 3.73 (m, 1H), 1.58 (s, 6H), 1.25 (d, *J =* 13.9 Hz, 8H). LC/MS (ESI+) calcd for C₄₂H₄₀N₆O₆ ( [M+H]⁺ ) *m*/*z* 725.3, found 725.3.

### Example 419: 5-((1r,3r)-3-(4-(2-(4-((6-(2H-1,2,3-triazol-2-yl)pyridin-2-yl) methoxy)phenyl)propan-2-yl)phenoxy)cyclobutyl)amino)-2-(2,6-dioxopiperidin-3-yl)isoindolin-1,3-dione

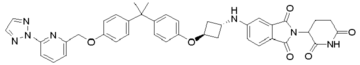

The target compound was synthesized by a method similar to that of Example 383. ¹H NMR (400 MHz, DMSO-*d*₆) *δ* 11.07 (s, 1H), 8.19 (s, 2H), 8.10 (t, *J*= 7.9 Hz, 1H), 7.97 (d, *J*= 8.0 Hz, 1H), 7.63 - 7.56 (m, 2H), 7.53 (d, *J*= 5.4 Hz, 1H), 7.12 (dd, *J*= 13.0, 8.8 Hz, 4H), 6.96 (d, *J*= 8.9 Hz, 2H), 6.86 (s, 1H), 6.79 (d, *J* = 8.7 Hz, 1H), 6.73 (d, *J* = 8.8 Hz, 2H), 5.22 (s, 2H), 5.03 (dd, *J* = 12.9, 5.4 Hz, 1H), 4.86 (p, *J* = 5.9, 5.1 Hz, 1H), 4.19 - 4.08 (m, 1H), 2.43 (dd, *J* = 11.1, 6.3 Hz, 2H), 1.99 (d, *J* = 10.1 Hz, 2H), 1.58 (s, 6H), 1.23 (s, 2H). LC/MS (ESI+) calcd for C₄₀H₃₇N₇O₆ ( [M+H]⁺ ) *m*/*z* 712.3, found 712.3.

### Example 420: 2-(2,6-dioxopiperidin-3-yl)-5-((1r,3r)-3-(4-(2-(4-((2-(3-fluoro-[1,3'-diazetidine]-1'-yl)pyrimidin-4-yl)methoxy)phenyl)propan-2-yl)phenoxy) cyclobutyl)amino)isoindol-1,3-dione

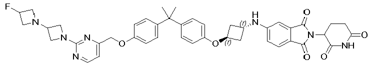

The target compound was synthesized by a method similar to that of Example 383. ¹H NMR (400 MHz, DMSO-*d*₆) *δ* 11.06 (s, 1H), 8.33 (d, *J*= 5.0 Hz, 1H), 7.59 (d, *J*= 8.3 Hz, 1H), 7.52 (d, *J* = 5.5 Hz, 1H), 7.11 (dd, *J* = 8.7, 6.8 Hz, 4H), 6.91 - 6.84 (m, 3H), 6.79 (d, *J* = 9.7 Hz, 1H), 6.73 (dd, *J* = 6.9, 1.6 Hz, 3H), 5.31 - 5.22 (m, 1H), 5.18 - 5.08 (m, 1H), 5.03 (dd, *J* = 12.9, 5.4 Hz, 1H), 4.95 (s, 2H), 4.90 - 4.81 (m, 1H), 4.14 (d, *J* = 4.5 Hz, 1H), 4.04 (dd, *J*= 9.0, 7.1 Hz, 2H), 3.76 (dd, *J=* 9.3, 4.3 Hz, 2H), 3.65 - 3.51 (m, 3H), 3.24 (dd, *J*= 9.5, 4.4 Hz, 1H), 3.18 (dd, *J* = 9.4, 4.3 Hz, 1H), 2.94 - 2.79 (m, 1H), 2.57 (d, *J* = 15.9 Hz, 1H), 2.43 (d, *J* = 7.0 Hz, 3H), 2.05 - 1.93 (m, 1H), 1.57 (s, 6H), 1.23 (s, 1H). LC/MS (ESI+) calcd for C₄₃H₄₄FN₇O₆ ( [M+H]⁺ ) *m*/*z* 774.3, found 774.3.

### Example 421: 2-(2,6-dioxopiperidin-3-yl)-5-(1r,3r)-3-(4-(2-(4-(6-(5-methyl-1,2,4-oxadiazol-3-yl)pyridin-2-yl)oxy)phenyl)propan-2-yl)phenoxy)cyclobutyl)amino) isoindolin-1,3-dione

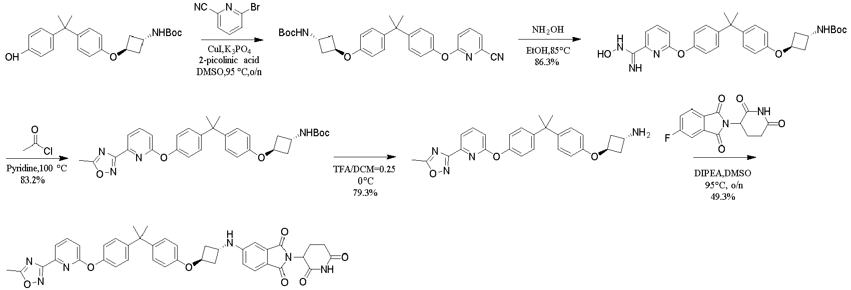

### Step 1: Synthesis of tert-butyl ((1r,3r)-3-(4-(2-(4-((6-(N-hydroxylcarbamoyl)pyridin-2-yl)oxy)phenyl)propan-2-yl)phenoxy)cyclobutyl)carbamate

In a 25 mL conical flask, tert-butyl ((1r,3r)-3-(4-(2-(4-((6-cyanopyridin-2-yl)oxy)phenyl)propan-2-yl)phenoxy)cyclobutyl)carbamate (250 mg, 0.500 mmol) was dissolved in 5 mL of EtOH, to which was added 50% hydroxylamine (99 mg, 1.500 mmol) aqueous solution, and then the reaction solution was stirred for 12 h under refluxing at 85 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (230 mg, yield 86.3%).

### Step 2: Synthesis of tert-butyl ((1r,3r)-3-(4-(2-(4-((6-(5-methyl-1,2,4-oxadiazol-3-yl)pyridin-2-yl)oxy)phenyl)propan-2-yl)phenoxy)cyclobutyl)carbamate

In a 25 mL conical flask, tert-butyl ((1r,3r)-3-(4-(2-(4-((6-(N-hydroxylcarbamoyl)pyridin-2-yl)oxy)phenyl)propan-2-yl)phenoxy)cyclobutyl) carbamate (230 mg, 0.432 mmol) was dissolved in 5 mL of pyridine, and then the solution was cooled to 0 °C in an ice water bath, to which was added acetyl chloride (230 mg, 1.300 mmol) under nitrogen protection. The reaction solution was stirred for 12 h at 100 °C, and cooled to 0 °C in an ice water bath, to which was added 0.5 mol/L HCl to quench the reaction. The resultant solution was extracted with 10 mL of DCM for three times. The combined organic phase was dried over anhydrous Na₂SO₄, and filtered. The solvent was rotatory evaporated under reduced pressure to provide the crude product, which was further purified by silica gel column chromatography, to obtain the target product as pale yellow solid (200 mg, yield 83.2%).

### Step 3: Synthesis of (1r,3r)-3-(4-(2-(4-((6-(5-methyl-1,2,4-oxadiazol-3-yl)pyridin-2-yl)oxy)phenyl)propan-2-yl)phenoxy)cyclobutyl-1-amine

In a 25 mL conical flask, tert-butyl ((1r,3r)-3-(4-(2-(4-((6-(5-methyl-1,2,4-oxadiazol-3-yl)pyridin-2-yl)oxy)phenyl)propan-2-yl)phenoxy)cyclobutyl)carbamate (200 mg, 0.359 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of DCM for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (130 mg, yield 79.3%).

### Step 4: Synthesis of 2-(2,6-dioxopiperidin-3-yl)-5-(1r,3r)-3-(4-(2-(4-(6-(5-methyl-1,2,4-oxadiazol-3-yl)pyridin-2-yl)oxy)phenyl)propan-2-yl)phenoxy)cyclobutyl) amino)isoindolin-1,3-dione

In a 25 mL conical flask, (1r,3r)-3-(4-(2-(4-((6-(5-methyl-1,2,4-oxadiazol-3-yl)pyridin-2-yl)oxy)phenyl)propan-2-yl)phenoxy)cyclobutyl-1-amine (130 mg, 0.285 mmol) was dissolved in 5 mL of DMSO, to which were added 2-(2,6-dioxopiperidin-3-yl)-5-fluoroisoindolin-1,3-dione (110 mg, 0.399 mmol) and N,N-diisopropylethylamine (147 mg, 1.140 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 49.3%). ¹H NMR (400 MHz, DMSO-*d*₆) *δ* 11.07 (s, 1H), 8.07 - 8.00 (m, 1H), 7.81 (d, *J* = 7.0 Hz, 1H), 7.59 (d, *J* = 8.3 Hz, 1H), 7.54 (d, *J* = 5.4 Hz, 1H), 7.26 (d, *J* = 8.8 Hz, 2H), 7.15 (t, *J* = 8.0 Hz, 3H), 7.10 (d, *J*= 8.7 Hz, 2H), 6.86 (s, 1H), 6.82 - 6.73 (m, 3H), 5.04 (dd, *J* = 12.8, 5.4 Hz, 1H), 4.87 (p, *J*= 6.4 Hz, 1H), 4.14 (d, *J*= 4.9 Hz, 1H), 2.65 (s, 3H), 2.44 (dd, *J* = 12.2, 5.4 Hz, 2H), 1.99 (d, *J* = 8.9 Hz, 2H), 1.64 (s, 6H), 1.23 (s, 4H). LC/MS (ESI+) calcd for C₄₀H₃₆N₆O₇ ([M+H]⁺) *m*/*z* 713.3, found 713.3.

### Example 422: 2-(2,6-dioxopiperidin-3-yl)-5-(1s,3s)-3-(4-(2-(4-(6-(5-methyl-1,2,4-oxadiazol-3-yl)pyridin-2-yl)oxy)phenyl)propan-2-yl)phenoxy)cyclobutyl)amino) isoindol-1,3-dione

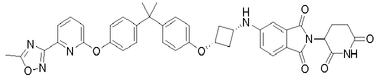

The target compound was synthesized by a method similar to that of Example 421. ¹H NMR (400 MHz, DMSO-*d*₆) *δ* 11.07 (s, 1H), 8.07 - 8.01 (m, 1H), 7.81 (d, *J=* 7.9 Hz, 1H), 7.59 (d, *J* = 8.3 Hz, 1H), 7.45 (d, *J* = 6.4 Hz, 1H), 7.26 (d, *J*= 8.8 Hz, 3H), 7.15 (dd, *J*= 8.3, 5.1 Hz, 4H), 7.10 (d, *J*= 8.8 Hz, 3H), 6.90 (s, 1H), 5.04 (dd, *J*= 12.9, 5.3 Hz, 1H), 4.55 - 4.45 (m, 1H), 3.78 (d, *J*= 7.1 Hz, 1H), 2.65 (s, 4H), 1.64 (s, 9H), 1.23 (s, 4H). LC/MS (ESI+) calcd for C₄₀H₃₆N₆O₇ ( [M+H]⁺ ) *m*/*z* 713.3, found 713.3.

### Example 423: 2-(2,6-dioxopiperidin-3-yl)-5-((1r,3r)-3-(4-(2-(4-((6-(5-methyl-1,3,4-oxadiazol-2-yl)pyridin-2-yl)oxy)phenyl)propan-2-yl)phenoxy)cyclobutyl) amino)isoindol-1,3-dione

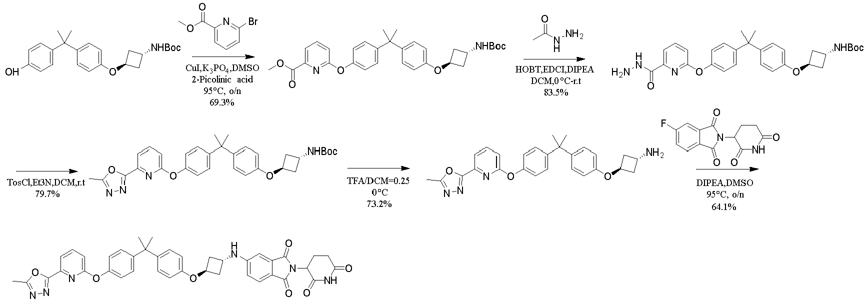

### Step 1: Synthesis of methyl 6-(4-(2-(4-((1r,3r)-3-((tert-butoxycarbonyl)amino) cyclobutyloxy)phenyl)propan-2-yl)phenoxy)picolinate

In a 25 mL conical flask, tert-butyl ((1r,3r)-3-(4-(2-(4-hydroxylphenyl)propan-2-phenoxy)cyclobutyl)carbamate (300 mg, 0.755 mmol) was dissolved in 5 mL of DMSO, to which were added methyl 6-bromopicolinate (212 mg, 0.981 mmol), K₃PO₄ (320 mg, 1.510 mmol), CuI (43 mg, 0.226 mmol), and 2-picolinic acid (28 mg, 0.226 mmol) under nitrogen protection, and then the mixture was stirred for 12 h at 100 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (300 mg, yield 69.3%).

### Step 2: Synthesis of tert-butyl ((1r,3r)-3-(4-(2-(4-(6-(hydrazinecarbonyl)pyridin-2-yl)oxy)phenyl)propan-2-yl)phenoxy)cyclobutyl)carbamate

In a 25 mL conical flask, 6-(4-(2-(4-((1s,3s)-3-((tert-butoxycarbonyl)amino) cyclobutyloxy)phenyl)propan-2-yl)phenoxy)picolinic acid (210 mg, 0.405 mmol) was dissolved in 5 mL of DMSO, and then the solution was cooled to 0 °C in an ice water bath, to which were added HOBT (82 mg, 0.607 mmol), EDCI (116 mg, 0.607 mmol), acethydrazide (33 mg, 0.445 mmol), and DIPEA (115 mg, 0.891 mmol) under nitrogen protection. The reaction solution was stirred for 12 h at 25 °C, and then saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (180 mg, yield 83.2%).

### Step 3: Synthesis of tert-butyl ((1r,3r)-3-(4-(2-(4-((6-(5-methyl-1,3,4-oxadiazol-2-yl)pyridin-2-yl)oxy)phenyl)propan-2-yl)phenoxy)cyclobutyl)carbamate

In a 25 mL conical flask, tert-butyl ((1r,3r)-3-(4-(2-(4-(6-(hydrazinecarbonyl) pyridin-2-yl)oxy)phenyl)propan-2-yl)phenoxy)cyclobutyl)carbamate (180 mg, 0.338 mmol) was dissolved in 5 mL of DCM, to which were added p-toluenesulfonyl chloride (77 mg, 0.406 mmol) and triethylamine (171 mg, 1.691 mmol) under nitrogen protection. The reaction solution was stirred for 12 h at 25 °C. Subsequently, saturated brine was added to quench the reaction. The resultant solution was extracted with 10 mL of DCM for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (150 mg, yield 79.7%).

### Step 4: Synthesis of (1r,3r)-3-(4-(2-(4-((6-(5-methyl-1,3,4-oxadiazol-2-yl)pyridin-2-yl)oxy)phenyl)propan-2-yl)phenoxy)cyclobutyl-1-amine

In a 25 mL conical flask, tert-butyl ((1r,3r)-3-(4-(2-(4-((6-(5-methyl-1,3,4-oxadiazol-2-yl)pyridin-2-yl)oxy)phenyl)propan-2-yl)phenoxy)cyclobutyl)carbamate (150 mg, 0.270 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of DCM for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (90 mg, yield 73.2%).

### Step 5: Synthesis of 2-(2,6-dioxopiperidin-3-yl)-5-((1r,3r)-3-(4-(2-(4-((6-(5-methyl-1,3,4-oxadiazol-2-yl)pyridin-2-yl)oxy)phenyl)propan-2-yl)phenoxy)cyclobutyl)amino) isoindolin-1,3-dione

In a 25 mL conical flask, (1r,3r)-3-(4-(2-(4-((6-(5-methyl-1,3,4-oxadiazol-2-yl)pyridin-2-yl)oxy)phenyl)propan-2-yl)phenoxy)cyclobutyl-1-amine (90 mg, 0.197 mmol) was dissolved in 5 mL of DMSO, to which were added 2-(2,6-dioxopiperidin-3-yl)-5-fluoroisoindolin-1,3-dione (76 mg, 0.276 mmol) and N,N-diisopropylethylamine (102 mg, 0.789 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (90 mg, yield 64.1%). ¹H NMR (400 MHz, DMSO-*d*₆) *δ* 11.06 (s, 1H), 8.09 - 8.03 (m, 1H), 7.88 (d, *J* = 7.4 Hz, 1H), 7.59 (d, *J* = 8.2 Hz, 1H), 7.53 (d, *J* = 5.3 Hz, 1H), 7.27 (d, *J* = 8.8 Hz, 2H), 7.16 (dd, *J* = 8.5, 4.0 Hz, 3H), 7.10 (d, *J* = 8.7 Hz, 2H), 6.86 (s, 1H), 6.80 (d, *J* = 8.9 Hz, 1H), 6.77 (d, *J* = 8.8 Hz, 2H), 5.33 (d, *J* = 4.8 Hz, 1H), 5.03 (dd, *J* = 12.8, 5.3 Hz, 1H), 4.87 (s, 1H), 2.56 (s, 3H), 1.64 (s, 6H), 1.23 (s, 8H). LC/MS (ESI+) calcd for C₄₀H₃₆N₆O₇ ( [M+H]⁺ ) m/z 713.3, found 713.3.

### Example 424: 5-((1r,3r)-3-(4-(2-(4-((6-(3,3-difluoro-[1,3'-diazetidin]-1'-yl) pyridazine-3-yl)oxy)phenyl)propan-2-yl)phenoxy)cyclobutyl)amino)-2-(2,6-dioxopiperidin-3-yl)isoindol-1,3-dione

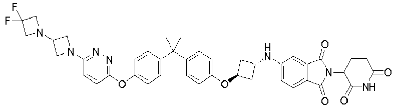

The target compound was synthesized by a method similar to that of Example 383. ¹H NMR (400 MHz, DMSO-*d*₆) *δ* 11.06 (s, 1H), 7.59 (d, *J* = 8.3 Hz, 1H), 7.53 (d, *J* = 5.5 Hz, 1H), 7.22 (dd, *J* = 9.0, 7.5 Hz, 4H), 7.14 (d, *J* = 8.8 Hz, 2H), 7.00 - 6.94 (m, 4H), 6.86 (s, 1H), 6.79 (d, *J* = 7.9 Hz, 1H), 5.03 (dd, *J* = 12.9, 5.4 Hz, 1H), 4.87 (dt, *J* = 10.8, 5.8 Hz, 1H), 4.14 (d, *J =* 5.0 Hz, 1H), 3.79 - 3.63 (m, 9H), 2.63 - 2.52 (m, 4H), 1.61 (s, 6H), 1.59 (s, 2H), 1.25 (d, *J* = 13.9 Hz, 3H). LC/MS (ESI+) calcd for C₄₂H₄₁F₂N₇O₆ ( [M+H]⁺ ) *m*/*z* 778.3, found 778.3.

### Example 425: 2-(2,6-dioxopiperidin-3-yl)-5-((1r,3r)-3-(4-(2-(4-((6-(4-methylpiperazin-1-yl)pyridazine-3-yl)oxy)phenyl)propan-2-yl)phenoxy) cyclobutyl)amino)isoindol-1,3-dione

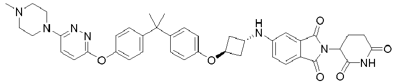

The target compound was synthesized by a method similar to that of Example 383. ¹H NMR (400 MHz, DMSO-*d*₆) *δ* 11.07 (s, 1H), 7.59 (d, *J=* 8.3 Hz, 1H), 7.53 (d, *J*= 5.4 Hz, 1H), 7.46 (d, *J*= 9.7 Hz, 1H), 7.22 (dd, *J* = 9.1, 6.7 Hz, 3H), 7.15 (d, *J* = 8.7 Hz, 2H), 6.99 (d, *J*= 8.7 Hz, 2H), 6.86 (s, 1H), 6.76 (d, *J* = 8.8 Hz, 2H), 5.03 (dd, *J* = 12.9, 5.4 Hz, 1H), 4.87 (p, *J* = 5.5, 4.8 Hz, 1H), 4.14 (d, .7= 4.8 Hz, 1H), 3.49 - 3.44 (m, 4H), 2.93 - 2.81 (m, 1H), 2.44 - 2.38 (m, 6H), 2.21 (s, 4H), 2.06 - 1.93 (m, 2H), 1.62 (s, 6H), 1.23 (s, 3H). LC/MS (ESI+) calcd for C₄₁H₄₃N₇O₆ ([M+H]⁺) *m*/*z* 730.3, found 730.3.

### Example 426: 2-(2,6-dioxopiperidin-3-yl)-5-(1r,3r)-3-(4-(2-(2-(3-methyl-1,2,4-oxadiazol-5-yl)pyrimidin-4-yl)oxy)phenyl)propan-2-yl)phenoxy)cyclobutyl) amino)isoindol-1,3-dione

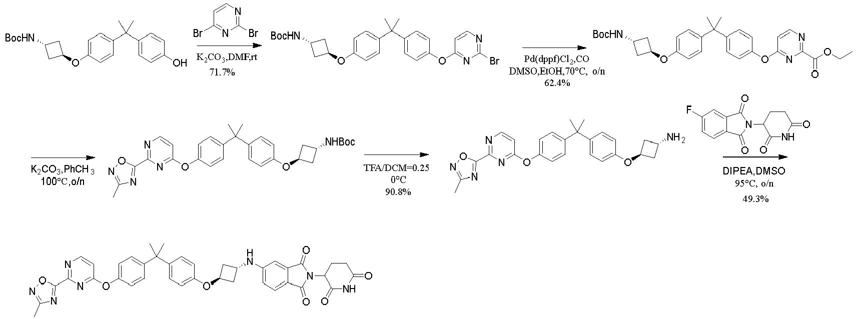

### Step 1: Synthesis of tert-butyl ((1r,3r)-3-(4-(2-(4-((2-bromopyrimidin-4-yl)oxy) phenyl)propan-2-yl)phenoxy)cyclobutyl)carbamate

In a 25 mL conical flask, tert-butyl ((1r,3r)-3-(4-(2-(4-hydroxylphenyl)propan-2-phenoxy)cyclobutyl)carbamate (500 mg,1.260 mmol) was dissolved in 5 mL of DMF, to which were added 2,4-dibromopyrimidine (359 mg, 1.510 mmL) and K₂CO₃ (348 mg, 2.520 mmol) under nitrogen protection, and then the mixture was stirred for 12 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (500 mg, yield 71.7%).

### Step 2: Synthesis of methyl 4-(4-(2-(4-((1r,3r)-3-((tert-butoxycarbonyl)amino) cyclobutyloxy) phenyl)propan-2-yl)phenoxy)pyrimidin-2-carboxylate

In a 25 mL conical flask, tert-butyl ((1r,3r)-3-(4-(2-(4-((2-bromopyrimidin-4-yl)oxy)phenyl)propan-2-yl)phenoxy)cyclobutyl)carbamate (0.500 g, 0.902 mmol) was dissolved in 5 mL of DMSO:MeOH (1:1), to which was added Pd(dppf)Cl₂ (0.066 g, 0.090 mmol), and the system was purged with CO for three times. The reaction solution was stirred for 12 h at 70 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (300 mg, yield 62.4%).

### Step 4: Synthesis of (1r,3r)-3-(4-(2-(4-((2-(3-methyl-1,2,4-oxadiazol-5-yl)pyrimidin-4-yl)oxy)phenyl)propan-2-yl)phenoxy)cyclobutyl-1-amine

In a 25 mL conical flask, tert-butyl ((1r,3r)-3-(4-(2-(4-((2-(3-methyl-1,2,4-oxadiazol-5-yl)pyrimidin-4-yl)oxy)phenyl)propan-2-yl)phenoxy)cyclobutyl) carbamate (180 mg, 0.323 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of DCM for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (130 mg, yield 90.8%).

### Step 5: Synthesis of 2-(2,6-dioxopiperidin-3-yl)-5-((1r,3r)-3-(4-(2-(4-((2-(3-methyl-1,2,4-oxadiazol-5-yl)pyrimidin-4-yl)oxy)phenyl)propan-2-yl)phenoxy)cyclobutyl) amino)isoindolin-1,3-dione

In a 25 mL conical flask, (1r,3r)-3-(4-(2-(4-((2-(3-methyl-1,2,4-oxadiazol-5-yl)pyrimidin-4-yl)oxy)phenyl)propan-2-yl)phenoxy)cyclobutyl-1-amine (130 mg, 0.293 mmol) was dissolved in 5 mL of DMSO, to which were added 2-(2,6-dioxopiperidin-3-yl)-5-fluoroisoindolin-1,3-dione (110 mg, 0.398 mmol) and N,N-diisopropylethylamine (147 mg, 1.140 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 49.3%). ¹H NMR (400 MHz, DMSO-*d*₆) *δ* 11.08 (s, 1H), 8.91 (d, *J*= 5.8 Hz, 1H), 7.59 (d, *J* = 8.1 Hz, 1H), 7.54 (d, *J* = 5.6 Hz, 1H), 7.36 - 7.28 (m, 2H), 7.19 (dd, *J* = 16.0, 8.8 Hz, 4H), 6.86 (s, 1H), 6.81 (s, 1H), 6.77 (d, *J=* 8.7 Hz, 2H), 5.35 - 5.29 (m, 1H), 5.04 (dd, *J=* 13.3, 5.1 Hz, 1H), 4.88 (d, *J=* 11.9 Hz, 1H), 4.18 - 4.10 (m, 1H), 2.43 (s, 4H), 1.66 (s, 4H), 1.23 (s, 9H). LC/MS (ESI+) calcd for C₃₉H₃₅N₇O₇ ([M+H]⁺) *m*/*z* 714.3, found 714.3.

### Example 427: 2-(2,6-dioxopiperidin-3-yl)-5-((1r,3r)-3-(4-(2-(4-((2-propionylpyridin-5-yl)oxy)phenyl)propan-2-yl)phenoxy)cyclobutyl)amino) isoindol-1,3-dione

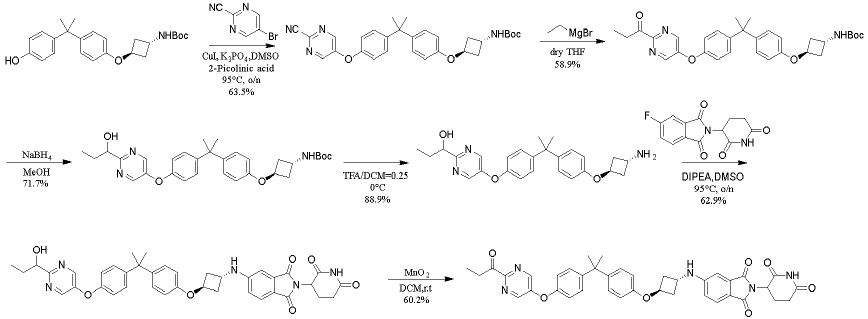

### Step 1: Synthesis of tert-butyl ((1r,3r)-3-(4-(2-(4-((2-cyanopyrimidin-5-yl)oxy) phenyl)propan-2-yl)phenoxy)cyclobutyl)carbamate

In a 25 mL conical flask, tert-butyl ((1s,3s)-3-(4-(2-(4-hydroxylphenyl)propan-2-phenoxy)cyclobutyl)carbamate (500 mg, 1.260 mmol) was dissolved in 5 mL of DMSO, to which were added 5-bromo-2-cyanopyrimidine (301 mg, 1.640 mmol), K₃PO₄ (534 mg, 2.520 mmol), CuI (72 mg, 0.377 mmol), and 2-picolinic acid (47 mg, 0.377 mmol) under nitrogen protection, and then the mixture was stirred for 12 h at 100 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (400 mg, yield 63.5%).

### Step 2: Synthesis of tert-butyl ((1r,3r)-3-(4-(2-(4-((2-propionylpyridin-5-yl)oxy) phenyl)propan-2-yl)phenoxy)cyclobutyl)carbamate

In a 25 mL conical flask, tert-butyl ((1r,3r)-3-(4-(2-(4-((2-cyanopyrimidin-5-yl)oxy)phenyl)propan-2-yl)phenoxy)cyclobutyl)carbamate (400 mg, 0.799 mmol) was dissolved in 5 mL of THF, and then the solution was cooled to 0 °C in an ice water bath, to which was added ethylmagnesium bromide (533 mg, 4.000 mmol) under nitrogen protection. The reaction solution was stirred for 12 h at 25 °C, and then saturated NaHCO₃ aqueous solution was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (250 mg, yield 58.9%).

### Step 3: Synthesis of tert-butyl ((1r,3r)-3-(4-(2-(4-(2-(1-hydroxylpropyl)pyrimidin-5-yl)oxy)phenyl)propan-2-yl)phenoxy)cyclobutyl)carbamate

In a 25 mL conical flask, tert-butyl ((1r,3r)-3-(4-(2-(4-((2-propionylpyridin-5-yl)oxy)phenyl)propan-2-yl)phenoxy)cyclobutyl)carbamate (250 mg,0.470 mmol) was dissolved in 5 mL of MeOH, and then the solution was cooled to 0 °C in an ice water bath, to which was added sodium borohydride (53 mg, 1.410 mmol) under nitrogen protection. The reaction solution was stirred for 2 h at 25 °C, and then saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (180 mg, yield 71.7%).

### Step 4: Synthesis of 1-(5-(4-(2-(4-((1r,3r)-3-aminocyclobutyloxy)phenyl)propan-2-yl)phenoxy)pyrimidin-2-yl)propan-1 -ol

In a 25 mL conical flask, tert-butyl ((1r,3r)-3-(4-(2-(4-(2-(1-hydroxylpropyl) pyrimidin-5-yl)oxy)phenyl)propan-2-yl)phenoxy)cyclobutyl)carbamate (180 mg, 0.337 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of DCM for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (130 mg, yield 88.9%).

### Step 5: Synthesis of 2-(2,6-dioxopiperidin-3-yl)-5-((1r,3r)-3-(4-(2-(4-((2-(1-hydroxylpropyl)pyrimidin-5-yl)oxy)phenyl)propan-2-yl)phenoxy)cyclobutyl) amino) isoindol-1,3-dione

In a 25 mL conical flask, 1-(5-(4-(2-(4-((1r,3r)-3-aminocyclobutyloxy)phenyl) propan-2-yl)phenoxy)pyrimidin-2-yl)propan-1-ol (130 mg, 0.300 mmol) was dissolved in 5 mL of DMSO, to which were added 2-(2,6-dioxopiperidin-3-yl)-5-fluoroisoindolin-1,3-dione (116 mg, 0.420 mmol) and N,N-diisopropylethylamine (155 mg, 1.200 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (130 mg, yield 62.9%).

### Step 6: Synthesis of 2-(2,6-dioxopiperidin-3-yl)-5-((1r,3r)-3-(4-(2-(4-((2-propionylpyridin-5-yl)oxy)phenyl)propan-2-yl)phenoxy)cyclobutyl)amino)isoindol-1,3-dione

In a 25 mL conical flask, 2-(2,6-dioxopiperidin-3-yl)-5-((1r,3r)-3-(4-(2-(4-((2-(1-hydroxylpropyl)pyrimidin-5-yl)oxy)phenyl)propan-2-yl)phenoxy)cyclobutyl)amino) isoindol-1,3-dione (50 mg, 0.073 mmol) was dissolved in 5 mL of DCM, to which was added MnO₂ (32 mg,0.362 mmol) under nitrogen protection, and then the mixture was stirred for 2 h at 25 °C. The reaction solution was filtered, and then the filtrate was rotatory evaporated under reduced pressure to remove the solvent and provide the crude product, which was further purified by silica gel column chromatography, to obtain the target product as light yellow solid (30 mg, yield 60.2%). ¹H NMR (400 MHz, DMSO-*d*₆) *δ* 11.08 (s, 1H), 8.58 (s, 2H), 7.59 (d, *J* = 8.5 Hz, 1H), 7.54 (d, *J* = 5.3 Hz, 1H), 7.25 (d, *J* = 8.8 Hz, 2H), 7.13 (d, *J* = 8.7 Hz, 2H), 7.01 (d, *J* = 8.8 Hz, 2H), 6.85 (s, 1H), 6.79 (d, *J* = 8.2 Hz, 1H), 6.75 (d, *J* = 8.8 Hz, 2H), 5.17 (d, *J* = 5.9 Hz, 1H), 5.04 (dd, *J* = 12.7, 5.1 Hz, 1H), 4.90 - 4.82 (m, 1H), 4.57 - 4.49 (m, 1H), 2.57 (d, *J* = 17.3 Hz, 2H), 2.04 - 1.93 (m, 2H), 1.77 (ddd, *J*= 28.9, 14.2, 6.7 Hz, 2H), 1.61 (s, 4H), 1.23 (s, 6H), 0.83 (t, *J* = 7.5 Hz, 3H). LC/MS (ESI+) calcd for C₃₉H₃₇N₅O₇ ([M+H]⁺) *m*/*z* 688.3, found 688.3.

### Example 428: 2-(2,6-dioxopiperidin-3-yl)-5-((1r,3r)-3-(4-(2-(4-((2-(1-hydroxylpropyl)pyrimidin-5-yl)oxy)phenyl)propan-2-yl)phenoxy)cyclobutyl) amino)isoindol-1,3-dione

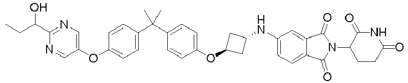

The target compound was synthesized by a method similar to that of Example 427. ¹H NMR (400 MHz, DMSO-*d*₆) *δ* 11.08 (s, 1H), 8.58 (s, 2H), 7.59 (d, *J*= 8.5 Hz, 1H), 7.54 (d, *J*= 5.3 Hz, 1H), 7.25 (d, *J* = 8.8 Hz, 2H), 7.13 (d, *J* = 8.7 Hz, 2H), 7.01 (d, *J* = 8.8 Hz, 2H), 6.85 (s, 1H), 6.79 (d, *J* = 8.2 Hz, 1H), 6.75 (d, *J* = 8.8 Hz, 2H), 5.32 (t, *J* = 4.2 Hz, 1H), 5.17 (d, *J* = 5.9 Hz, 1H), 5.04 (dd, *J* = 12.7, 5.1 Hz, 1H), 4.90 - 4.82 (m, 1H), 4.57 - 4.49 (m, 1H), 2.57 (d, *J* = 17.3 Hz, 2H), 2.04 - 1.93 (m, 2H), 1.77 (ddd, *J* = 28.9, 14.2, 6.7 Hz, 2H), 1.61 (s, 4H), 1.23 (s, 6H), 0.83 (t, *J* = 7.5 Hz, 3H). LC/MS (ESI+) calcd for C₃₉H₃₇N₅O₇ ([M+H]⁺) *m*/*z* 688.3, found 688.3.

### Example 429: 2-(2,6-dioxopiperidin-3-yl)-5-((1r,3r)-3-(4-(2-(4-((5-methyl-1,3,4-oxadiazol-2-yl)oxy)phenyl)propan-2-yl)phenoxy)cyclobutyl)aminoisoindol-1,3-dione

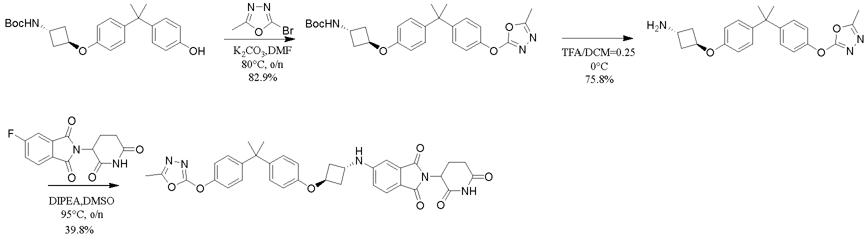

### Step 1: Synthesis of tert-butyl ((1r,3r)-3-(4-(2-(4-((5-methyl-1,3,4-oxadiazol-2-yl) oxy)phenyl)propan-2-yl)phenoxy)cyclobutyl)carbamate

In a 25 mL conical flask, tert-butyl ((1r,3r)-3-(4-(2-(4-hydroxylphenyl)propan-2-phenoxy)cyclobutyl)carbamate (500 mg, 1.260 mmol) was dissolved in 5 mL of DMF, to which were added 2-bromo-5-methyl-1,3,4-oxadiazole (246 mg, 1.510 mmL) and K₂CO₃(348 mg,2.520 mmol) under nitrogen protection, and then the mixture was stirred for 12 h at 25 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (500 mg, yield 82.9%).

### Step 2: Synthesis of (1r,3r)-3-(4-(2-(4-((5-methyl-1,3,4-oxadiazol-2-yl)oxy)phenyl) propan-2-yl)phenoxy)cyclobutyl-1-amine

In a 25 mL conical flask, tert-butyl ((1r,3r)-3-(4-(2-(4-((5-methyl-1,3,4-oxadiazol-2-yl)oxy)phenyl)propan-2-yl)phenoxy)cyclobutyl)carbamate (500 mg, 1.040 mmol) was dissolved in 2 mL of DCM, and then the solution was cooled to 0 °C in an ice water bath, to which was added 0.5 mL of trifluoroacetic acid under nitrogen protection. The reaction solution was stirred for 2 h at 0 °C. Subsequently, saturated NaHCO₃ aqueous solution was added to quench the reaction. The resultant solution was extracted with 10 mL of DCM for three times. The combined organic phase was dried over anhydrous Na₂SO₄, and filtered. The solvent was rotatory evaporated under reduced pressure to provide the crude product, which was further purified by silica gel column chromatography, to obtain the target product as white solid (300 mg, yield 75.8%).

### Step 3: Synthesis of 2-(2,6-dioxopiperidin-3-yl)-5-((1r,3r)-3-(4-(2-(4-((5-methyl-1,3,4-oxadiazol-2-yl)oxy)phenyl)propan-2-yl)phenoxy)cyclobutyl)aminoisoindol-1,3-dione

In a 25 mL conical flask, (1r,3r)-3-(4-(2-(4-((5-methyl-1,3,4-oxadiazol-2-yl)oxy)phenyl)propan-2-yl)phenoxy)cyclobutyl-1-amine (150 mg, 0.395 mmol) was dissolved in 5 mL of DMSO, to which were added 2-(2,6-dioxopiperidin-3-yl)-5-fluoroisoindolin-1,3-dione (153 mg, 0.553 mmol) and N,N-diisopropylethylamine (204 mg, 1.580 mmol) under nitrogen protection, and then the mixture was stirred for 12 h at 95 °C. Subsequently, saturated brine was added to quench the reaction. The resultant solution was extracted with 10 mL of EtOAc for three times. The combined organic phase was dried over anhydrous Na₂SO₄, and filtered. The solvent was rotatory evaporated under reduced pressure to provide the crude product, which was further purified by silica gel column chromatography, to obtain the target product as light yellow solid (100 mg, yield 39.8%). ¹H NMR (400 MHz, DMSO-*d*₆) *δ* 11.07 (s, 1H), 7.59 (d, *J*= 8.4 Hz, 1H), 7.54 (d, *J* = 5.4 Hz, 1H), 7.36 - 7.26 (m, 3H), 7.14 (d, *J*= 8.8 Hz, 2H), 6.85 (s, 1H), 6.78 (dd, *J* = 12.7, 8.8 Hz, 3H), 5.35 - 5.29 (m, 1H), 5.04 (dd, *J* = 13.2, 5.2 Hz, 1H), 4.91 - 4.82 (m, 1H), 4.14 (dd, *J* = 8.6, 3.3 Hz, 1H), 2.42 (s, 4H), 1.62 (s, 4H), 1.23 (s, 9H). LC/MS (ESI+) calcd for C₃₅H₃₃N₅O₇ ( [M+H]⁺ ) *m*/*z* 636.2, found 636.2.

### Example 430: 2-(2,6-dioxopiperidin-3-yl)-5-(1s,3s)-3-(4-(3-(4-(6-(1-hydroxylethyl) pyridazine-3-yl)oxy)phenyl)pentan-3-yl)phenoxy)cyclobutyl)amino)isoindol-1,3-dione

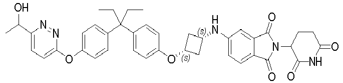

The target compound was synthesized by a method similar to that of Example 427. ¹H NMR (400 MHz, DMSO-*d*₆) *δ* 11.08 (s, 1H), 7.81 (d, *J*= 9.1 Hz, 1H), 7.59 (d, *J*= 8.4 Hz, 1H), 7.46 (d, *J* = 6.4 Hz, 1H), 7.41 (d, *J* = 9.1 Hz, 1H), 7.19 (d, *J* = 8.8 Hz, 2H), 7.08 (d, *J* = 7.7 Hz, 4H), 6.90 (s, 1H), 6.81 (t, *J* = 9.0 Hz, 3H), 5.38 - 5.27 (m, 1H), 5.04 (dd, *J=* 12.9, 5.2 Hz, 1H), 4.90 (q, *J* = 7.3 Hz, 1H), 4.50 (dt, *J* = 14.9, 7.8 Hz, 1H), 3.82 - 3.72 (m, 1H), 2.54 (s, 4H), 2.06 (d, *J* = 7.1 Hz, 4H), 2.01 - 1.90 (m, 4H), 1.39 (d, *J* = 6.5 Hz, 3H), 0.58 (t, *J* = 7.1 Hz, 6H). LC/MS (ESI+) calcd for C₄₀H₄₁N₅O₇ ( [M+H]⁺ ) *m*/*z* 704.3, found 704.3.

### Example 431: 5-((1s,3s)-3-(4-(3-(4-((6-acetylpyridazine-3-yl)oxy)phenyl)pentan-3-yl)phenoxy)cyclobutyl)amino)-2-(2,6-dioxopyridin-3-yl)isoindol-1,3-dione

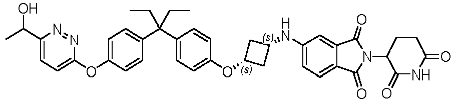

The target compound was synthesized by a method similar to that of Example 427. ¹H NMR (400 MHz, DMSO-*d*₆) *δ* 11.07 (s, 1H), 8.16 (d, *J* = 9.2 Hz, 1H), 7.57 (dd, *J* = 12.4, 8.8 Hz, 2H), 7.46 (d, *J* = 6.4 Hz, 1H), 7.24 (d, *J* = 8.8 Hz, 2H), 7.17 (d, *J* = 8.8 Hz, 2H), 7.10 (d, *J* = 8.8 Hz, 2H), 6.90 (s, 1H), 6.85 - 6.76 (m, 3H), 5.04 (dd, *J=* 12.9, 5.3 Hz, 1H), 4.57 - 4.45 (m, 1H), 3.85 - 3.70 (m, 1H), 3.07 (s, 2H), 2.69 (s, 3H), 2.63 - 2.52 (m, 2H), 2.08 (d, *J* = 7.3 Hz, 4H), 2.02 - 1.89 (m, 4H), 0.59 (t, *J=* 7.2 Hz, 6H). LC/MS (ESI+) calcd for C₄₀H₃₉N₅O₇ ([M+H]⁺) *m*/*z* 702.3, found 702.3.

### Example 432: 5-(4-(2-(4-((1r,3r)-3-((2-(2,6-dioxopiperidin-3-yl)-1,3-dioxoisoquinolin-5-yl)amino)cyclobutyloxy)phenyl)propan-2-yl)phenoxy)-N-methylpyrimidin-2-carbonylamine

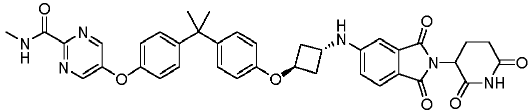

The target compound was synthesized by a method similar to that of Example 429. ¹H NMR (400 MHz, DMSO-*d*₆) *δ* 11.07 (s, 1H), 8.78 (d, *J*= 3.3 Hz, 1H), 8.65 (s, 2H), 7.59 (d, *J*= 8.2 Hz, 1H), 7.54 (d, *J* = 5.4 Hz, 1H), 7.29 (d, *J* = 8.7 Hz, 2H), 7.13 (t, *J* = 9.5 Hz, 4H), 6.85 (s, 1H), 6.78 (dd, *J* = 13.1, 8.7 Hz, 3H), 5.35 - 5.30 (m, 1H), 5.04 (dd, *J* = 13.0, 5.5 Hz, 1H), 4.89 - 4.83 (m, 1H), 2.80 (d, *J* = 4.8 Hz, 3H), 2.57 (d, *J* = 17.2 Hz, 2H), 2.43 (s, 2H), 2.05 - 1.95 (m, 4H), 1.63 (s, 6H). LC/MS (ESI+) calcd for C₃₈H₃₆N₆O₇ ([M+H]⁺) *m*/*z* 689.3, found 689.3.

### Example 433: Synthesis of 5-(((1s,3s)-3-(4-(2-(4-((2-((3s,5r)-3,5-dimethylpiperazin-1-yl)pyrimidin-4-yl)methoxy)phenyl)propan-2-yl)phenoxy) cyclobutyl)amino)-2-(2,6-dioxopiperidin-3-yl)isoindolin-1,3-dione

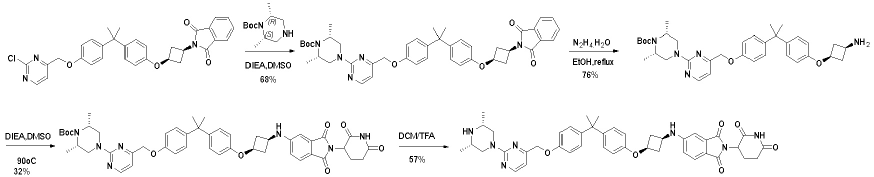

### Step 1: Synthesis of compound tert-butyl (2r,6s)-4-(4-((4-(2-(4-((1s,3s)-3-(1,3-dioxoisoindolin-2-yl)cyclobutyloxy)phenyl) propan-2-yl)phenoxy)methyl)pyrimidin-2-yl)-2,6-dimethylpiperazin-1 -carboxylate

2-((1s,3s)-3-(4-(2-(4-((2-chloropyrimidin-4-yl)methoxy)phenyl)propan-2-yl) phenoxy)cyclobutyl)isoindol-1,3-dione (200 mg, 0.36 mmol) was dissolved in 5 mL of DMS, to which were added tert-butyl (2r,6s)-2,6-dimethylpiperazin-1-carboxylate (154 mg, 0.72 mmol) and DIEA (248 mg, 1.8 mmol), and then the mixture was heated to 90 °C and allowed to react overnight. The next day, the system was added to water, and extracted with EA. The organic phase was washed with saturated brine, dried and concentrated. The residue was purified to provide tert-butyl (2r,6s)-4-(4-((4-(2-(4-((1s,3s)-3-(1,3-dioxoisoindolin-2-yl)cyclobutyloxy)phenyl)propan-2-yl)phenoxy) methyl)pyrimidin-2-yl)-2,6-dimethylpiperazin-1-carboxylate (185 mg), with a yield of 68%. LC/MS (ESI+) Calcd for C₄₃H₄₉N₅O₆ (M+H⁺) *m*/*z* 731.4; found 731.8.

### Step 2: Synthesis of tert-butyl (2R,6S)-4-(4-((4-(2-(4-((1s,3s)-3-aminocyclobutyloxy)phenyl)propan-2-yl)phenoxy)methyl)pyrimidin-2-yl)-2,6-dimethylpiperazin-1 -carboxylate

tert-butyl (2r,6s)-4-(4-((4-(2-(4-((1s,3s)-3-(1,3-dioxoisoindolin-2-yl) cyclobutyloxy)phenyl)propan-2-yl)phenoxy)methyl)pyrimidin-2-yl)-2,6-dimethylpiperazin-1-carboxylate (180 mg, 0.24 mmol) was dissolved in 8 mL of ethanol, to which was added hydrazine hydrate (2 mL), and then the solution was refluxed for 1 h. The system was moved to an ice bath and stirred for 1h, and then lots of white flocculent solid was precipitated, followed by filtration. The filtrate was concentrated, and then 5 mL of dichloromethane was added. A small amount of white solid was insoluble, which was further filtered, and then the filtrate was concentrated, to provide tert-butyl (2R,6S)-4-(4-((4-(2-(4-((1s,3s)-3-aminocyclobutyloxy)phenyl) propan-2-yl)phenoxy)methyl)pyrimidin-2-yl)-2,6-dimethylpiperazin-1-carboxylate (104 mg).

### Step 3: Synthesis of compound tert-butyl (2R,6S)-4-(4-((4-(2-(4-((1s,3s)-3-((2-(2,6-dioxopiperidin-3-yl)-)1,3-dioxoisoindolin-5-yl)amino)cyclobutyloxy)phenyl)propan-2-yl)phenoxy)methyl)pyrimidin-2-yl)-2,6-dimethylpiperazin-1-carboxylate

tert-butyl (2R,6S)-4-(4-((4-(2-(4-((1s,3s)-3-aminocyclobutyloxy)phenyl)propan-2-yl)phenoxy)methyl)pyrimidin-2-yl)-2,6-dimethylpiperazin-1-carboxylate (100 mg, 0.18 mmol) was dissolved in 6 mL of DMSO, to which was added 2-(2,6-dioxopiperidin-3-yl)-5-fluoroisoindolin-1,3-dione (200.0 mg, 0.7 mmol), followed by adding 0.1 mL ofDIEAdropwise, and then the mixture was allowed to react overnight at 95 °C. The reaction solution was cooled to room temperature, and extracted by adding water and ethyl acetate. The organic phase was washed with saturated brine, dried and concentrated. The residue was subjected to column chromatography, to provide tert-butyl (2R,6S)-4-(4-((4-(2-(4-((1s,3s)-3-((2-(2,6-dioxopiperidin-3-yl)-)1,3-dioxoisoindolin-5-yl)amino)cyclobutyloxy)phenyl)propan-2-yl)phenoxy)methyl) pyrimidin-2-yl)-2,6-dimethylpiperazin-1-carboxylate (38 mg), with a yield of 32%. LC/MS (ESI+) Calcd for C₄₈H₅₅N₇O₈ (M+H⁺) *m*/*z* 857.4; found 857.4.

### Step 4: Synthesis of compound 5-(((1s,3s)-3-(4-(2-(4-((2-((3S,5R)-3,5-dimethylpiperazin-1-yl)pyrimidin-4-yl)methoxy)phenyl)propan-2-yl)phenoxy) cyclobutyl)amino)-2-(2,6-dioxopiperidin-3 -yl)isoindolin-1,3 -dione

tert-butyl (2R,6S)-4-(4-((4-(2-(4-((1s,3s)-3-((2-(2,6-dioxopiperidin-3-yl)-)1,3-dioxoisoindolin-5-yl)amino)cyclobutyloxy)phenyl)propan-2-yl)phenoxy)methyl) pyrimidin-2-yl)-2,6-dimethylpiperazin-1-carboxylate (30 mg, 0.04 mmol) was dissolved in 10 mL of DCM, to which was added 2 mL of trifluoroacetic acid dropwise in an ice bath, and then the ice bath was removed. The reaction solution was warmed to room temperature and reacted for 2 h, to which was added saturated Na₂CO₃ solution, to adjust the pH to neutral. The reaction mixture was poured into a separatory funnel and separated. The organic phase was washed with saturated brine, dried over anhydrous Na₂SO₄, filtered and concentrated. The residue was purified by TLC, to provide 5-(((1s,3s)-3-(4-(2-(4-((2-((3S,5R)-3,5-dimethylpiperazin-1-yl)pyrimidin-4-yl)methoxy) phenyl)propan-2-yl)phenoxy)cyclobutyl)amino)-2-(2,6-dioxopiperidin-3-yl) isoindolin-1,3-dione (15 mg), with a yield of 57%. LC/MS (ESI+) Calcd for C₄₃H₄₇N₇O₆ (M+H⁺) *m*/*z* 757.4; found 757.4.
¹H NMR (400 MHz, Chloroform-*d*) *δ* 8.30 (d, *J* = 5.0 Hz, 1H), 7.62 (d, *J* = 8.2 Hz, 1H), 7.20 - 7.09 (m, 4H), 6.89 (d, *J=* 2.1 Hz, 1H), 6.84 (d, *J* = 8.6 Hz, 2H), 6.74 (d, *J* = 5.0 Hz, 1H), 6.70 (td, *J* = 6.5, 3.3 Hz, 3H), 5.30 (s, 2H), 4.94 (s, 2H), 4.86 (d, *J* = 5.3 Hz, 2H), 4.76 (d, *J* = 13.2 Hz, 2H), 4.22 (d, *J* = 5.3 Hz, 1H), 3.05 (s, 2H), 2.89 (d, *J* = 17.5 Hz, 1H), 2.71 (dd, *J* = 9.3, 4.4 Hz, 2H), 2.45 - 2.36 (m, 2H), 2.11 (d, *J* = 6.5 Hz, 2H), 1.63 (s, 6H), 1.36 (d, *J* = 6.2 Hz, 6H).

### Example 434: Synthesis of 2-(2,6-dioxopiperidin-3-yl)-5-((3-(4-(2-(4-((2-morpholinethiazol-5-yl)methoxy)phenyl)propan-2-yl)phenoxy)propyl)amino) isoindolin-1,3-dione

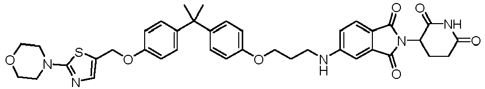

The target compound was synthesized by a method similar to that of Example 433. LC/MS (ESI+) calcd for C₃₉H₄₁N₅O₇S⁺ ([M+H]⁺) *m*/*z:* 723.3; found 723.3. ¹H NMR (400 MHz, Chloroform-*d*) *δ* 8.18 (s, 1H), 7.41 (t, *J* = 7.8 Hz, 2H), 7.13 - 7.09 (m, 4H), 7.01 (d, *J* = 2.4 Hz, 1H), 6.90 (d, *J* = 2.5 Hz, 2H), 6.85 - 6.82 (m, 2H), 6.73 - 6.66 (m, 2H), 4.91 (dd, *J* = 12.0, 5.2 Hz, 2H), 4.07 (t, *J* = 5.6 Hz, 3H), 3.92 (s, 2H), 3.78 (d, *J* = 4.8 Hz, 4H), 3.50 (d, *J* = 6.3 Hz, 3H), 3.38 (t, *J=* 4.9 Hz, 4H), 2.12 (d, *J=* 6.2 Hz, 4H), 1.65 (d, *J=* 2.0 Hz, 6H).

### Example 435: Synthesis of 2-(2,6-dioxopiperidin-3-yl)-4-((3-(4-(2-(4-((2-morpholinethiazol-5-yl)methoxy)phenyl)propan-2-yl)phenoxy)propyl)amino) isoindolin-1,3-dione

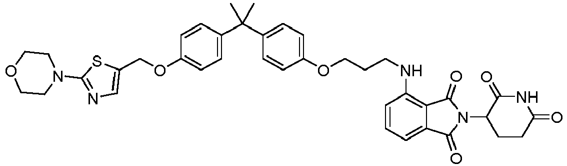

The target compound was synthesized by a method similar to that of Example 433. LC/MS (ESI+) calcd for C₃₉H₄₁N₅O₇S⁺ ( [M+H]⁺ ) *m*/*z:* 723.3; found 723.3. ¹H NMR (400 MHz, Chloroform-*d*) *δ* 8.40 (s, 1H), 7.46 (t, *J* = 7.8 Hz, 2H), 7.13 - 7.09 (m, 4H), 7.01 (d, *J* = 2.4 Hz, 1H), 6.90 (d, *J* = 2.5 Hz, 2H), 6.85 - 6.82 (m, 2H), 6.73 - 6.66 (m, 2H), 4.91 (dd, *J* = 12.0, 5.2 Hz, 2H), 4.07 (t, *J* = 5.6 Hz, 3H), 3.92 (s, 2H), 3.78 (d, *J* = 4.8 Hz, 4H), 3.50 (d, *J* = 6.3 Hz, 3H), 3.38 (t, *J* = 4.9 Hz, 4H), 2.12 (d, *J=* 6.2 Hz, 4H), 1.61 (d, *J* = 2.0 Hz, 6H).

### Example 436: Synthesis of 5-(((1r,3r)-3-(4-(2-(4-((2-(2H-1,2,3-triazol-2-yl)pyrimidin-5-yl)oxy)phenyl)propan-2-yl)phenoxy)cyclobutyl)amino)-2-(2,6-dioxopiperidin-3-yl)isoindolin-1,3-dione

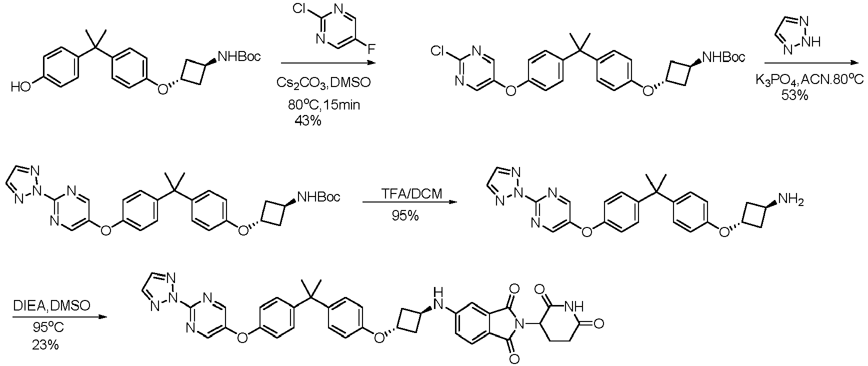

### Step 1: Preparation of tert-butyl ((1r,3r)-3-(4-(2-(4-((2-chloropyrimidin-5-yl)oxy) phenyl)propan-2-yl)phenoxy)cyclobutyl)carbamate

2-chloro-5-fluoropyrimidine (1.0 g, 7.6 mmol) was dissolved in 50 mL of DMF, to which were added tert-butyl ((1r,3r)-3-(4-(2-(4-hydroxylphenyl)propan-2-yl)phenoxy)cyclobutyl)carbamate (4.1 g, 11.4 mmol) and Cs₂CO₃ (2.4 g, 17.4 mmol), and then the reaction solution was heated to 80 °C and allowed to react for 2 h. The system was added to water, and then extracted with ethyl acetate. The organic phase was dried over anhydrous Na₂SO₄, followed by column chromatography, to provide tert-butyl ((1r,3r)-3-(4-(2-(4-((2-chloropyrimidin-5-yl)oxy)phenyl)propan-2-yl) phenoxy)cyclobutyl)carbamate (0.6 g), with a yield of 43%. LC/MS (ESI+) Calcd for C₂₈H₃₂ClN₃O₄ (M+H⁺) *m*/*z* 509.2; found 509.2.

### Step 2: Preparation of tert-butyl ((1r,3r)-3-(4-(2-(4-((2-(2H-1,2,3-triazol-2-yl) pyrimidin-5-yl)oxy)phenyl) propan-2-yl)phenoxy)cyclobutyl)carbamate

tert-butyl ((1r,3r)-3-(4-(2-(4-((2-chloropyrimidin-5-yl)oxy)phenyl)propan-2-yl) phenoxy)cyclobutyl)carbamate (0.6 g, 1.2 mmol) was dissolved in 24 mL of acetonitrile, to which was added potassium phosphate (0.5 g, 2.4 mmol), followed by addition of 2H-1,2,3-triazole (0.1 g, 1.4 mmol). The reaction solution was heated to 80 °C and then allowed to react overnight. Water was added to quench the reaction. The resultant solution was extracted with EA, followed by column chromatography, to provide tert-butyl ((1r,3r)-3-(4-(2-(4-((2-(2H-1,2,3-triazol-2-yl)pyrimidin-5-yl)oxy)phenyl) propan-2-yl)phenoxy)cyclobutyl)carbamate (0.2 g). LC/MS (ESI+) Calcd for C₃₀H₃₄N₆O₄ (M+H⁺) *m*/*z* 542.3; found 542.5.

### Step 3: Preparation of (1r,3r)-3-(4-(2-(4-((2-(2H-1,2,3-triazol-2-yl)pyrimidin-5-yl)oxy) phenyl)propan-2-yl) phenoxy)cyclobutane-1-amine

tert-butyl ((1r,3r)-3-(4-(2-(4-((2-(2H-1,2,3-triazol-2-yl)pyrimidin-5-yl)oxy)phenyl)propan-2-yl)phenoxy)cyclobutyl)carbamate (140.0 mg, 0.2 mmol) was dissolved in 8 mL of dichloromethane, to which was added 2 mL of trifluoroacetic acid in an ice bath, and then the mixture was allowed to react for 30 min, to which was added saturated Na₂CO₃ solution in the ice bath, to adjust the pH to neutral. The reaction solution was extracted with dichloromethane, dried over anhydrous Na₂SO₄, and concentrated under reduced pressure, to provide (1r,3r)-3-(4-(2-(4-((2-(2H-1,2,3-triazol-2-yl)pyrimidin-5-yl)oxy)phenyl)propan-2-yl)phenoxy)cyclobutane-1-amine (110.3 mg).
LC/MS (ESI+) Calcd for C₂₅H₂₆N₆O₂ (M+H⁺) *m*/*z* 442.2; found 442.2.

### Step 4: Preparation of 5-(((1r,3r)-3-(4-(2-(4-((2-(2H-1,2,3-triazol-2-yl)pyrimidin-5-yl)oxy)phenyl)propan-2-yl)phenoxy)cyclobutyl)amino)-2-(2,6-dioxopiperidin-3-yl) isoindolin-1,3-dione

Intermediate (1r,3r)-3-(4-(2-(4-((2-(2H-1,2,3-triazol-2-yl)pyrimidin-5-yl)oxy) phenyl)propan-2-yl)phenoxy)cyclobutane-1-amine (110.0 mg, 0.2 mmol) was dissolved in 5 mL of DMSO, to which was added DIEA(97.3 mg, 0.8mmol), and then the solution was stirred well, followed by addition of 2-(2,6-dioxopiperidin-3-yl)-5-fluoroisoindolin-1,3-dione (49.9 mg, 0.2 mmol). The reaction solution was heated to 120 °C and reacted for 2 h. The reaction was completed by TLC detection. The reaction solution was cooled to room temperature, and then extracted by adding water and ethyl acetate. The organic phase was washed with saturated brine, dried over anhydrous Na₂SO₄, and then concentrated, followed by column chromatography to provide 5-(((1r,3r)-3-(4-(2-(4-((2-(2H-1,2,3-triazol-2-yl)pyrimidin-5-yl)oxy)phenyl)propan-2-yl)phenoxy)cyclobutyl)amino)-2-(2,6-dioxopiperidin-3-yl)isoindolin-1,3-dione (21.2 mg), with a yield of 23%. LC/MS (ESI+) Calcd for C₃₈H₃₄N₈O₆ (M+H⁺) *m*/*z* 698.3; found 698.8. ¹H NMR (400 MHz, CDCl₃) *δ* 8.00 (s, 1H), 7.87 (s, 2H), 7.82 (t, *J* = 7.9 Hz, 1H), 7.64 (d, *J* = 8.2 Hz, 1H), 7.25 (s, 2H), 7.19 - 7.14 (m, 2H), 7.14 - 7.08 (m, 2H), 6.94 (d, *J* = 2.1 Hz, 1H), 6.80 - 6.73 (m, 2H), 6.71 (d, *J* = 8.7 Hz, 2H), 4.93 (dd, *J* = 12.2, 5.3 Hz, 1H), 4.90 - 4.83 (m, 1H), 4.28 - 4.20 (m, 1H), 2.95 - 2.75 (m, 3H), 2.74 - 2.68 (m, 2H), 2.43 (dt, *J* = 12.9, 6.4 Hz, 2H), 2.15 - 2.10 (m, 1H), 1.68 (s, 6H).

### Example 437: Synthesis of 5-((4-((8-(4-(2-(4-((2-(1H-pyrazol-1-yl)pyrimidin-4-yl)methoxy)phenyl)propan-2-yl)phenoxy)octyl)oxy)butyl)amino)-2-(2,6-dioxopiperidin-3-yl)isoindolin-1,3-dione

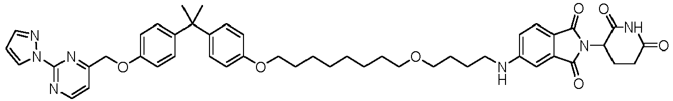

The target compound was synthesized by a method similar to that of Example 436. LC/MS (ESI+) calcd for C₄₈H₅₅N₇O₇ ([M+H]⁺) *m*/*z:* 841.4; found 841.4 ¹H NMR (400 MHz, CDCl₃) δ 8.66 (d, *J* = 4.8 Hz, 1H), 8.08 (s, 1H), 7.60 (d, *J* = 7.8 Hz, 1H), 7.15 (dd, *J* = 10.6, 6.7 Hz, 3H), 7.07 (d, *J* = 8.1 Hz, 2H), 6.99 (s, 1H), 6.83 (d, *J* = 8.3 Hz, 2H), 6.73 (d, *J* = 8.4 Hz, 3H), 5.04 (s, 2H), 4.98 - 4.88 (m, 1H), 4.09 - 4.01 (m, 2H), 3.45 (dt, *J* = 13.0, 6.2 Hz, 4H), 3.24 (s, 2H), 2.94 - 2.74 (m, 3H), 2.11 (d, *J* = 8.8 Hz, 1H), 1.72 (d, *J* = 24.2 Hz, 6H), 1.62 (s, 6H), 1.57 (s, 2H), 1.34 (d, *J* = 7.3 Hz, 11H).

### Example 438: Synthesis of 2-(2,6-dioxopiperidin-3-yl)-5-((4-((8-(4-(2-(4-hydroxylphenyl)propan-2-yl)phenoxy)octyl)oxy)butyl)amino)isoindolin-1,3-dione

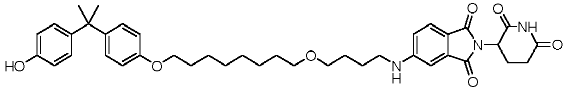

The target compound was synthesized by a method similar to that of Example 31. LC/MS (ESI+) Calcd for C₄₀H₄₉N₃O₇ (M +H⁺) *m*/*z* 683.4; Found 683.4. ¹H NMR (400 MHz, Chloroform-*d*) *δ* 9.12 (s, 1H), 8.66 (d, *J* = 7.5 Hz, 1H), 7.81 (d, *J* = 7.5 Hz, 1H), 7.62 (d, *J* = 7.3 Hz, 1H), 7.20 (d, *J=* 1.5 Hz, 1H), 7.16 - 7.10 (m, 4H), 7.06 (dd, *J* = 7.5, 1.5 Hz, 1H), 6.92 - 6.86 (m, 4H), 6.36 (t, *J* = 5.7 Hz, 1H), 5.43 (t, *J* = 7.1 Hz, 1H), 5.32 - 5.12 (m, 2H), 4.09 - 3.81 (m, 2H), 3.38 (ddtd, *J* = 40.1, 12.7, 7.1, 5.7 Hz, 2H), 2.72 (s, 3H), 2.66 - 2.56 (m, 2H), 2.24 - 2.02 (m, 4H), 2.00 - 1.82 (m, 4H), 0.85 (t, *J=* 8.0 Hz, 6H).

### Example 439: Synthesis of 5-((3-(4-(3-(4-((2-acetylpyrimidin-4-yl)methoxy)phenyl) pentan-3-yl)phenoxy)propyl)amino)-2-(2,6-dioxopiperidine-3-amino)isoindolin-1,3-dione

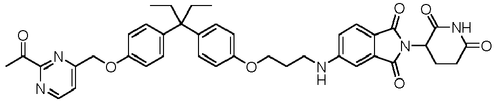

The target compound was synthesized by a method similar to that of Example 350. LC/MS (ESI+) Calcd for C₄₀H₄₁N₅O₇ (M +H⁺) *m*/*z* 703.3; Found 703.3. ¹H NMR (400 MHz, Chloroform-*d*) *δ* 9.12 (s, 1H), 8.66 (d, *J* = 7.5 Hz, 1H), 7.81 (d, *J* = 7.5 Hz, 1H), 7.62 (d, *J* = 7.3 Hz, 1H), 7.20 (d, *J* = 1.5 Hz, 1H), 7.16 - 7.10 (m, 4H), 7.06 (dd, *J* = 7.5, 1.5 Hz, 1H), 6.92 - 6.86 (m, 4H), 6.36 (t, *J* = 5.7 Hz, 1H), 5.43 (t, *J=* 7.1 Hz, 1H), 5.32 - 5.12 (m, 2H), 4.09 - 3.81 (m, 2H), 3.38 (ddtd, *J* = 40.1, 12.7, 7.1, 5.7 Hz, 2H), 2.72 (s, 3H), 2.66 - 2.56 (m, 2H), 2.24 - 2.02 (m, 4H), 2.00 - 1.82 (m, 4H), 0.85 (t, *J* = 8.0 Hz, 6H).

### Example 440: Synthesis of 2-(2,6-dioxopiperidin-3-yl)-5-((3-(4-(2-(4-((2-(pyrrolidin-1-yl)pyrimidin-4-yl)methoxy)phenyl)propan-2-yl)phenoxy) propyl)amino)isoindolin-1,3-dione

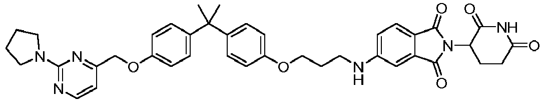

The target compound was synthesized by a method similar to that of Example 59. LC/MS (ESI+) Calcd for C₄₀H₄₂N₆O₆ (M +H⁺) *m*/*z* 702.3; Found, 702.3. ¹H NMR (400 MHz, Chloroform-*d*) *δ* 9.12 (s, 1H), 8.15 (d, *J=* 7.5 Hz, 1H), 7.81 (d, *J=* 7.5 Hz, 1H), 7.20 (d, *J=* 1.5 Hz, 1H), 7.20 - 7.13 (m, 4H), 7.13 - 7.03 (m, 2H), 6.95 - 6.78 (m, 4H), 6.36 (t, *J=* 5.7 Hz, 1H), 5.44 (d, *J=* 7.0 Hz, 1H), 5.22 - 5.03 (m, 2H), 4.15 - 3.95 (m, 2H), 3.92 - 3.76 (m, 4H), 3.38 (ddtd, *J* = 40.1, 12.7, 7.1, 5.7 Hz, 2H), 2.69 - 2.55 (m, 2H), 2.27 - 2.00 (m, 4H), 2.00 - 1.90 (m, 4H), 1.62 (s, 6H).

### Example 441: Synthesis of 2-((4-(2-(4-(3-((2-(2,6-dioxopiperidin-3-yl)-1,3-dioxoisoindolin-5-yl)amino)propoxy)phenyl)propan-2-yl)phenoxy)methyl) thiazol-4-carbonylamine

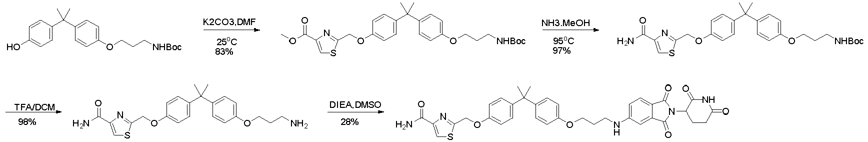

### Step 1: Synthesis of methyl 2-((4-(2-(4-(3-((tert-butoxycarbonyl)amino)propoxy) phenyl)propan-2-yl)phenoxy)methyl)thiazol-4-carboxylate

tert-butyl (3-(4-(2-(4-hydroxylphenyl)propan-2-yl)phenoxy)propyl)carbamate (200 mg, 0.27 mmol) was dissolved in 10 mL of DMF, to which was added K₂CO₃ (75 mg, 0.54 mmol), and then the system was cooled to 0 °C, followed by addition of methyl 2-(chloromethyl)thiazol-4-carboxylate (62 mg, 0.32 mmol). The ice bath was removed, and then the mixture was allowed to react at room temperature for 2 h. Water was added to quench the reaction. The resultant solution was extracted with ethyl acetate. The organic phase was dried and concentrated, followed by TLC, to provide methyl 2-((4-(2-(4-(3-((tert-butoxycarbonyl)amino)propoxy)phenyl)propan-2-yl)phenoxy)methyl) thiazol-4-carboxylate (220 mg).

### Step 2: Synthesis of tert-butyl (3-(4-(2-(4-((4-carbamoylthiazol-2-yl)methoxy)phenyl) propan-2-yl)phenoxy)propyl)carbamate

Methyl 2-((4-(2-(4-(3-((tert-butoxycarbonyl)amino)propoxy)phenyl)propan-2-yl) phenoxy)methyl)thiazol-4-carboxylate (200 mg, 0.37 mmol) was mixed with 5 mL solution of ammonia in methanol, and then the solution was warmed to 95 °C and reacted overnight. The reaction solution was concentrated, to provide crude tert-butyl (3-(4-(2-(4-((4-carbamoylthiazol-2-yl)methoxy)phenyl)propan-2-yl)phenoxy)propyl) carbamate (220 mg).
The following steps were performed by referring to that of Example 59. LC/MS (ESI+) Calcd for C₃₆H₃₅N₅O₇S (M +H⁺) *m*/*z* 681.2; Found, 681.2. ¹H NMR (400 MHz, Chloroform-*d*) *δ* 9.12 (s, 1H), 7.81 (d, *J=* 7.5 Hz, 1H), 7.43 (s, 1H), 7.29 (d, *J=* 12.3 Hz, 1H), 7.26 - 7.16 (m, 4H), 7.07 (dd, *J* = 7.4, 1.5 Hz, 1H), 6.88 (ddt, *J* = 6.2, 5.0, 1.5 Hz, 4H), 6.36 (t, *J =* 5.7 Hz, 1H), 5.61 - 5.39 (m, 3H), 4.10 - 3.94 (m, 2H), 3.39 (ddtd, *J* = 43.6, 12.7, 7.1, 5.7 Hz, 2H), 2.69 - 2.52 (m, 2H), 2.37 - 1.93 (m, 4H), 1.61 (s, 6H).

### Example 442: Synthesis of 2-(2,6-dioxopiperidin-3-yl)-5-((3-(4-(2-(4-(oxazolo[4,5-b] pyridin-2-ylmethoxy)phenyl)propan-2-yl)phenoxy)propyl)amino)isoindolin-1,3-dione:

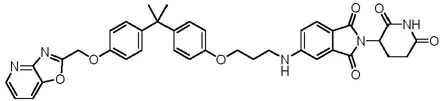

The target compound was synthesized by a method similar to that of Example 59. LC/MS (ESI+) Calcd for C₃₈H₃₅N₅O₇ (M +H⁺) *m*/*z* 673.3; Found 673.3. ¹H NMR (400 MHz, Chloroform-*d) δ* 9.12 (s, 1H), 8.27 (dd, *J=* 7.5, 1.6 Hz, 1H), 8.05 (dd, *J=* 7.5, 1.5 Hz, 1H), 7.82 (s, 1H), 7.40 (t, *J=* 7.5 Hz, 1H), 7.25 - 7.12 (m, 5H), 7.07 (d, *J=* 1.5 Hz, 1H), 6.97 - 6.82 (m, 4H), 6.36 (t, *J* = 5.7 Hz, 1H), 5.43 (t, *J=* 7.1 Hz, 1H), 5.35 - 5.23 (m, 2H), 4.09 - 3.89 (m, 2H), 3.39 (ddtd, *J=* 43.6, 12.7, 7.1, 5.7 Hz, 2H), 2.76 - 2.55 (m, 2H), 2.29 - 1.99 (m, 4H), 1.61 (s, 6H).

### Example 443: Synthesis of 2-(2,6-dioxopiperidin-3-yl)-4-((3-(4-(2-(4-(oxazolo[4,5-b] pyridin-2-ylmethoxy)phenyl)propan-2-yl)phenoxy)propyl)amino)isoindolin-1,3-dione

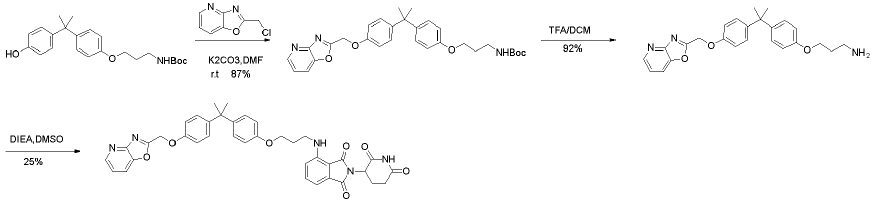

The target compound was synthesized by a method similar to that of Example 59. LC/MS (ESI+) Calcd for C₃₈H₃₅N₅O₇ (M +H⁺) *m*/*z* 673.3; Found 673.3. ¹H NMR (400 MHz, Chloroform-*d*) *δ* 8.99 (s, 1H), 8.21 (d, *J=* 7.5 Hz, 1H), 7.90 (dd, *J=* 7.4, 1.5 Hz, 1H), 7.51 (t, *J =* 7.5 Hz, 1H), 7.22 (dd, *J* = 7.5, 1.6 Hz, 1H), 7.16 (ddd, *J=* 7.7, 6.3, 1.4 Hz, 5H), 6.93 - 6.81 (m, 4H), 6.36 (t, *J=* 5.7 Hz, 1H), 5.43 (t, *J=* 7.1 Hz, 1H), 5.35 - 5.23 (m, 2H), 4.09 - 3.89 (m, 2H), 3.39 (ddtd, *J* = 43.6, 12.7, 7.1, 5.7 Hz, 2H), 2.77 - 2.56 (m, 2H), 2.29 - 1.99 (m, 4H), 1.65 (s, 6H).

### Example 444: Synthesis of 5-((3-(4-(2-(4-((2-(cyclopropylamino)pyrimidin-4-yl)methoxy)phenyl)propan-2-yl)phenoxy)propyl)amino)-2-(2,6-dioxopiperidin-3-yl)isoindolin-1,3-dione

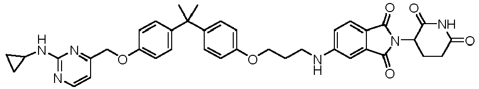

The target compound was synthesized by a method similar to that of Example 59. LC/MS (ESI+) Calcd for C₃₉H₄₀N₆O₆ (M +H⁺) *m*/*z* 688.3; Found 688.3. ¹H NMR (400 MHz, Chloroform-*d*) *δ* 9.12 (s, 1H), 8.19 (d, *J* = 7.5 Hz, 1H), 7.81 (d, *J* = 7.5 Hz, 1H), 7.20 (d, *J* = 1.5 Hz, 1H), 7.19 - 7.12 (m, 4H), 7.11 - 7.01 (m, 2H), 6.95 - 6.78 (m, 4H), 6.36 (t, *J* = 5.7 Hz, 1H), 5.22 - 5.03 (m, 2H), 4.15 - 3.95 (m, 2H), 3.92 - 3.76 (m, 4H), 3.38 (ddtd, *J* = 40.1, 12.7, 7.1, 5.7 Hz, 2H), 2.69 - 2.55 (m, 2H), 2.27 - 2.00 (m, 4H), 2.00 - 1.90 (m, 4H), 1.62 (s, 6H), 1.57 - 1.51 (m, 2H), 1.48 - 1.36 (m, 2H).

### Example 445: 5-(((1s,3s)-3-(4-(2-(4-((2-(1H-1,2,3-triazol-1-yl)pyrimidin-4-yl) methoxy)phenyl)propan-2-yl)phenoxy)cyclobutyl)amino)-2-(2,6-dioxopiperidin-3-yl)isoindolin-1,3-dione

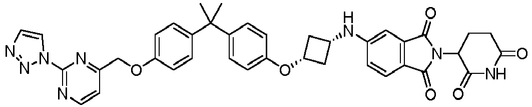

The target compound was synthesized by a method similar to that of Example 436. LC/MS (ESI+) Calcd for C₃₉H₃₆N₈O₆ (M +H⁺) *m*/*z* 712.8; Found 712.8. ¹H NMR (400 MHz, Chloroform-*d) δ* 8.29 - 8.25 (m, 1H), 8.23 (s, 1H), 7.90 - 7.85 (m, 2H), 7.60 (d, *J=* 8.3 Hz, 1H), 7.31 - 7.27 (m, 2H), 7.19 - 7.14 (m, 2H), 7.10 - 7.05 (m, 2H), 6.87 (dd, *J* = 5.1, 3.4 Hz, 2H), 6.77 - 6.66 (m, 3H), 5.30 (d, *J=* 2.7 Hz, 2H), 4.93 (dd, *J=* 12.1, 5.2 Hz, 1H), 4.51 (t, *J=* 6.8 Hz, 1H), 3.78 (t, *J* = 7.6 Hz, 1H), 3.17 - 3.08 (m, 2H), 2.96 - 2.67 (m, 4H), 2.10 (dd, *J=* 10.6, 7.5 Hz, 2H), 1.64 (s, 7H).

### Example 446: Synthesis of 5-(((1s,3s)-3-(4-(2-(4-((2-(2H-1,2,3-triazol-2-yl) pyrimidin-4-yl)methoxy)phenyl)propan-2-yl)phenoxy)cyclobutyl)amino)-2-(2,6-dioxopiperidin-3-yl)isoindolin-1,3-dione

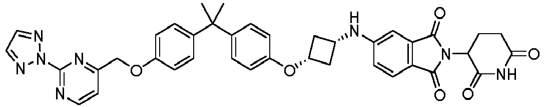

The target compound was synthesized by a method similar to that of Example 436. LC/MS (ESI+) Calcd for C₃₉H₃₆N₈O₆ (M +H⁺) *m*/*z* 712.8; Found 712.8. ¹H NMR (400 MHz, Chloroform-*d*) δ 8.89 (d, *J* = 5.1 Hz, 1H), 8.02 (d, *J* = 6.4 Hz, 2H), 7.65 (dd, *J* = 18.6, 6.7 Hz, 2H), 7.19 - 7.11 (m, 4H), 6.92 (d, *J* = 2.1 Hz, 1H), 6.91 - 6.86 (m, 2H), 6.72 (dd, *J* = 8.8, 2.6 Hz, 3H), 5.30 (d, *J=* 2.7 Hz, 2H), 4.93 (dd, *J=* 12.1, 5.2 Hz, 1H), 4.51 (t, *J=* 6.8 Hz, 1H), 3.78 (t, *J=* 7.6 Hz, 1H), 3.17 - 3.08 (m, 2H), 2.96 - 2.67 (m, 4H), 2.10 (dd, *J* = 10.6, 7.5 Hz, 2H), 1.64 (s, 7H).

### Example 447: Synthesis of 2-(2,6-dioxopiperidin-3-yl)-5-((3-(4-(2-(4-((2-methoxypyrimidin-4-yl)methoxy)phenyl)propan-2-yl)phenoxy)cyclobutyl)amino) isoindolin-1,3-dione

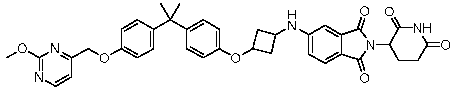

The target compound was synthesized by a method similar to that of Example 66. LC/MS (ESI+) Calcd for C₃₈H₃₇N₅O₇ (M+H⁺) *m*/*z* 675.3; found 675.3.

### Example 448: Synthesis of 5-3-(4-(2-(4-((2-(3,4-dimethylpiperazin-1-yl)pyrimidin-4-yl)methoxy)phenyl)propan-2-yl)phenoxy)cyclobutyl)amino)-2-(2,6-dioxopiperidin-3-yl)isoindolin-1,3-dione

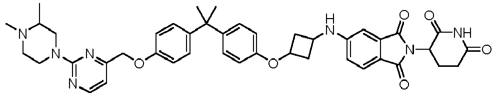

The target compound was synthesized by a method similar to that of Example 59. LC/MS (ESI+) Calcd for C₄₃H₄₇N₇O₆ (M+H⁺) *m*/*z* 757.4; Found, 757.4. ¹H NMR (400 MHz, Chloroform-*d) δ* 8.31 (dd, *J* = 5.0, 1.7 Hz, 1H), 7.60 (d, *J* = 8.3 Hz, 1H), 7.15 -7.10 (m, 4H), 6.89 (d, *J* = 2.0 Hz, 1H), 6.85 - 6.81 (m, 2H), 6.70 (ddd, *J* = 8.9, 5.9, 2.4 Hz, 4H), 4.93 (s, 3H), 4.91 - 4.84 (m, 2H), 4.55 (dd, *J=* 26.0, 13.3 Hz, 3H), 4.22 (d, *J=* 6.4 Hz, 1H), 3.24 (d, *J=* 13.7 Hz, 1H), 2.99 - 2.83 (m, 4H), 2.81 - 2.75 (m, 1H), 2.70 (ddd, *J=* 13.2, 7.5, 3.9 Hz, 3H), 2.40 (s, 3H), 2.26 (s, 2H), 2.16 - 2.08 (m, 2H), 1.94 (s, 2H), 1.65 (s, 6H).

### Example 449: Synthesis of 2-(2,6-dioxopiperidin-3-yl)-5-(((1r,3r)-3-(4-(2-(4-((2-methoxypyrimidin-4-yl)methoxy)phenyl)propan-2-yl)phenoxy)cyclobutyl)amino) isoindolin-1,3-dione

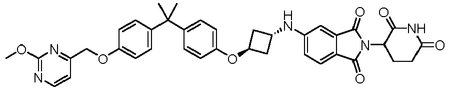

The target compound was synthesized by a method similar to that of Example 66. LC/MS (ESI+) Calcd for C₃₈H₃₇N₅O₇ (M+H⁺) *m*/*z* 675.3; found 675.3. ¹H NMR (400 MHz, Chloroform-*d) δ* 8.54 (s, 1H), 8.09 (s, 1H), 7.63 (d, *J=* 8.2 Hz, 1H), 7.23 (s, 1H), 7.17 - 7.08 (m, 4H), 6.90 (d, *J=* 3.7 Hz, 1H), 6.85 (d, *J=* 8.1 Hz, 2H), 6.69 (d, *J=* 8.7 Hz, 3H), 6.36 (s, 2H), 5.08 (s, 2H), 4.99 - 4.94 (m, 2H), 4.23 (s, 1H), 4.05 (s, 3H), 3.00 - 2.67 (m, 5H), 2.47 - 2.35 (m, 2H), 2.15 - 2.09 (m, 1H), 1.63 (s, 6H).

### Example 450: Synthesis of 5-(((1r,3r)-3-(4-(2-(4-((2-(3,4-dimethylpiperazin-1-yl)pyrimidin-4-yl)methoxy)phenyl)propan-2-yl)phenoxy)cyclobutyl)amino)-2-(2,6-dioxopiperidin-3-yl)isoindolin-1,3-dione

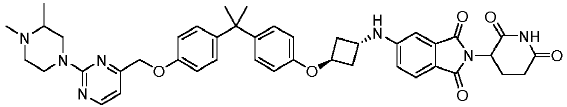

The target compound was synthesized by a method similar to that of Example 59. LC/MS (ESI+) Calcd for C₄₃H₄₇N₇O₆ (M+H⁺) *m*/*z* 757.4; Found, 757.4. ¹H NMR (400 MHz, Chloroform-*d) δ* 8.29 (dd, *J* = 5.0, 1.7 Hz, 1H), 7.62 (d, *J* = 8.3 Hz, 1H), 7.15 - 7.10 (m, 4H), 6.88 (d, *J* = 2.0 Hz, 1H), 6.85 - 6.81 (m, 2H), 6.70 (ddd, *J* = 8.9, 5.9, 2.4 Hz, 4H), 4.93 (s, 3H), 4.91 - 4.84 (m, 2H), 4.55 (dd, *J=* 26.0, 13.3 Hz, 3H), 4.22 (d, *J=* 6.4 Hz, 1H), 3.24 (d, *J=* 13.7 Hz, 1H), 2.99 - 2.83 (m, 4H), 2.81 - 2.75 (m, 1H), 2.70 (ddd, *J* = 13.2, 7.5, 3.9 Hz, 3H), 2.40 (s, 3H), 2.26 (s, 2H), 2.16 - 2.08 (m, 2H), 1.94 (s, 2H), 1.63 (s, 6H).

### Example 451: Synthesis of 2-(2,6-dioxopiperidin-3-yl)-5-((3-(4-(2-(4-((2-(piperazin-1-yl)pyrimidin-4-yl)methoxy)phenyl)propan-2-yl)phenoxy)cyclobutyl) amino)isoindolin-1,3-dione

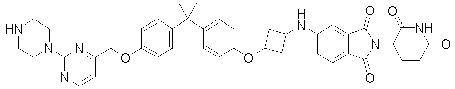

The target compound was synthesized by a method similar to that of Example 59. LC/MS (ESI+) Calcd for C₄₁H₄₃N₇O₆ (M +H⁺) *m*/*z* 729.3; Found, 729.3. ¹H NMR (400 MHz, Chloroform-*d*) *δ* 9.11 (d, *J* = 5.0 Hz, 1H), 7.34 (dd, *J=* 8.3, 3.3 Hz, 1H), 7.11 (ddd, *J* = 8.3, 5.1, 2.8 Hz, 4H), 6.91 (dd, *J* = 8.6, 2.1 Hz, 1H), 6.86 - 6.78 (m, 3H), 6.70 (dd, *J* = 8.7, 6.3 Hz, 3H), 4.95 (d, *J=* 3.8 Hz, 2H), 4.87 (s, 1H), 4.51 (t, *J* = 6.7 Hz, 1H), 4.22 (s, 1H), 3.77 (s, 1H), 3.25 (s, 2H), 3.16 (t, *J=* 12.4 Hz, 3H), 3.08 - 3.01 (m, 1H), 2.80 (ddd, *J=* 34.3, 27.9, 13.8 Hz, 5H), 2.52 (s, 1H), 2.17 - 2.04 (m, 2H), 1.62 (s, 6H).

### Example 452: Synthesis of 5-((3-(4-(2-(4-((2-(3,5-dimethylpiperazin-1-yl) pyrimidin-4-yl)methoxy)phenyl)propan-2-yl)phenoxy)cyclobutyl)amino)-2-(2,6-dioxopiperidin-3-yl)isoindolin-1,3-dione

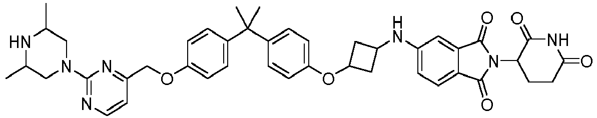

The target compound was synthesized by a method similar to that of Example 59. LC/MS (ESI+) Calcd for C₄₁H₄₇N₇O₆ (M +H⁺) *m*/*z* 757.4; Found, 757.4. ¹H NMR (400 MHz, Chloroform-*d*) *δ* 8.32 (d, *J* = 5.0 Hz, 1H), 7.62 (dd, *J* = 8.3, 3.3 Hz, 1H), 7.13 (ddd, *J* = 8.3, 5.1, 2.8 Hz, 4H), 6.91 (dd, *J* = 8.6, 2.1 Hz, 1H), 6.86 - 6.78 (m, 3H), 6.70 (dd, *J* = 8.7, 6.3 Hz, 3H), 4.95 (d, *J=* 3.8 Hz, 2H), 4.87 (s, 1H), 4.51 (t, *J=* 6.7 Hz, 1H), 4.22 (s, 1H), 3.77 (s, 1H), 3.25 (s, 2H), 3.16 (t, *J=* 12.4 Hz, 3H), 3.08 - 3.01 (m, 1H), 2.80 (ddd, *J=* 34.3, 27.9, 13.8 Hz, 5H), 2.42 (s, 1H), 2.17 - 2.04 (m, 2H), 1.63 (s, 12H).

### Example 453: 2-(2,6-dioxopiperidin-3-yl)-5-(((1s,3s)-3-(4-(2-(4-((2-(3-hydroxylazetidine)-1-yl)pyrimidin-4-yl)methoxy)phenyl)propan-2-yl)phenoxy) cyclobutyl)amino)isoindolin-1,3-dione

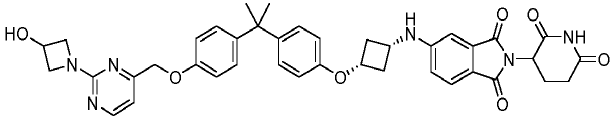

The target compound was synthesized by a method similar to that of Example 59. LC/MS (ESI+) Calcd for C₄₁H₄₂N₆ O₇ (M+H⁺) *m*/*z* 716.3; Found, 716.3. ¹H NMR (400 MHz, DMSO-*d*₆) *δ* 11.01 (s, 1H), 8.33 (d, *J* = 5.0 Hz, 1H), 7.59 (d, *J* = 8.3 Hz, 1H), 7.44 (d, *J* = 6.5 Hz, 1H), 7.11 (t, *J* = 8.5 Hz, 4H), 6.94 - 6.86 (m, 3H), 6.82 (dd, *J* = 8.4, 2.1 Hz, 1H), 6.79 - 6.70 (m, 3H), 5.61 (s, 1H), 5.03 (dd, *J* = 12.9, 5.4 Hz, 1H), 4.95 (s, 2H), 4.49 (t, *J* = 6.9 Hz, 1H), 3.88 (d, *J* = 2.4 Hz, 4H), 3.77 (q, *J=* 7.4 Hz, 1H), 3.05 (dq, *J=* 11.3, 6.4 Hz, 2H), 2.93 - 2.82 (m, 1H), 2.57 (dd, *J* = 19.8, 6.2 Hz, 2H), 2.03 - 1.90 (m, 3H), 1.53 (s, 6H).

### Example 454: 2-(2,6-dioxopiperidin-3-yl)-5-(((1s,3s)-3-(4-(2-(4-((2-((S)-3-methylpiperazin-1-))yl)pyrimidin-4-yl)methoxy)phenyl)propan-2-yl)phenoxy) cyclobutyl)amino)isoindolin-1,3-dione

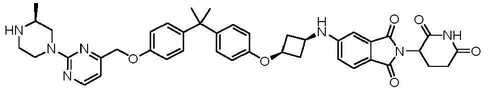

The target compound was synthesized by a method similar to that of Example 59. LC/MS (ESI+) Calcd for C₄₂H₄₅N₇O₆ (M+H+) *m*/*z* 743.3; Found, 743.3. ¹H NMR (400 MHz, Chloroform-*d*) *δ* 8.75 (d, *J* = 5.0 Hz, 1H), 7.53 (dd, *J=* 8.3, 3.3 Hz, 1H), 7.11 (ddd, *J=* 8.3, 5.1, 2.8 Hz, 4H), 6.91 (dd, *J=* 8.6, 2.1 Hz, 1H), 6.83 - 6.75 (m, 3H), 6.51 (dd, *J=* 8.7, 6.3 Hz, 3H), 4.95 (d, *J* = 3.8 Hz, 2H), 4.87 (s, 1H), 4.51 (t, *J* = 6.7 Hz, 1H), 4.22 (s, 1H), 3.77 (s, 1H), 3.25 (s, 2H), 3.16 (t, *J=* 12.4 Hz, 3H), 3.08 - 3.01 (m, 1H), 2.80 (ddd, *J* = 34.3, 27.9, 13.8 Hz, 5H), 2.41 (s, 1H), 2.17 - 2.04 (m, 2H), 1.63 (s, 6H), 1.57 (s, 3H).

### Example 455: Synthesis of compound 2-(2,6-dioxopiperidin-3-yl)-5-(((1s,3s)-3-(4-(2-(4-((2-(3-hydroxyl-3-methylazetidine)-1-yl)pyrimidin-4-yl)methoxy)phenyl) propan-2-yl)phenoxy)cyclobutyl)amino)isoindolin-1,3-dione

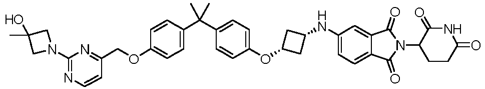

The target compound was synthesized by a method similar to that of Example 59. LC/MS (ESI+) Calcd for C₄₁H₄₂N₆O₇ (M+H⁺) *m*/*z* 730.3; Found, 730.3. ¹H NMR (400 MHz, DMSO-*d*₆) *δ* 11.05 (s, 1H), 8.33 (d, *J* = 5.0 Hz, 1H), 7.59 (d, *J* = 8.3 Hz, 1H), 7.44 (d, *J* = 6.5 Hz, 1H), 7.11 (t, *J*= 8.5 Hz, 4H), 6.94 - 6.86 (m, 3H), 6.82 (dd, *J* = 8.4, 2.1 Hz, 1H), 6.79 - 6.70 (m, 3H), 5.61 (s, 1H), 5.03 (dd, *J* = 12.9, 5.4 Hz, 1H), 4.95 (s, 2H), 4.49 (t, *J*= 6.9 Hz, 1H), 3.88 (d, *J*= 2.4 Hz, 4H), 3.77 (q, *J* = 7.4 Hz, 1H), 3.05 (dq, *J*= 11.3, 6.4 Hz, 2H), 2.93 - 2.82 (m, 1H), 2.57 (dd, *J*= 19.8, 6.2 Hz, 2H), 2.03 - 1.90 (m, 3H), 1.57 (s, 6H), 1.42 (s, 3H).

### Example 456: 5-(((1s,3s)-3-(4-(2-(4-((2-((R)-3-(dimethylamino)pyrrolidin-1-yl)pyrimidin-4-yl)methoxy)phenyl))propan-2-yl)phenoxy)cyclobutyl)amino)-2-(2,6-dioxopiperidin-3-yl)isoindolin-1,3-dione

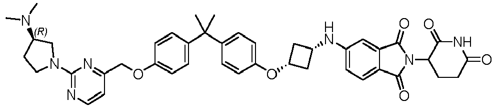

The target compound was synthesized by a method similar to that of Example 59. LC/MS (ESI+) Calcd for C₄₃H₄₇N₇O₆ (M+H⁺) m/z 757.4; found 757.4. ¹H NMR (400 MHz, CDCl₃) *δ* 8.27 (d, *J*= 5.0 Hz, 1H), 7.62 (d, *J*= 8.3 Hz, 1H), 7.16 - 7.08 (m, 4H), 6.91 (d, *J*= 2.1 Hz, 1H), 6.87 - 6.80 (m, 2H), 6.75 - 6.65 (m, 4H), 5.01 - 4.88 (m, 3H), 4.77 (d, *J*= 6.2 Hz, 1H), 4.51 (p, *J* = 6.8 Hz, 1H), 3.95 (dd, *J*= 10.8, 7.1 Hz, 1H), 3.80 (ddd, *J* = 22.0, 13.4, 8.6 Hz, 2H), 3.50 (td, *J* = 10.7, 6.9 Hz, 1H), 3.42 - 3.33 (m, 1H), 3.12 (ddt, *J=* 13.6, 10.0, 5.0 Hz, 2H), 3.02 - 2.69 (m, 4H), 2.37 (s, 6H), 2.25 (dd, *J* = 12.1, 6.3 Hz, 1H), 2.12 - 2.02 (m, 3H), 1.65 (s, 6H).

### Example 457: 2-(2,6-dioxopiperidin-3-yl)-5-(((1s,3s)-3-(4-(2-(4-((2-(((3-methyl) -3-yl)methyl)amino)pyrimidin-4-yl)methoxy)phenyl)propan-2-yl)phenoxy) cyclobutyl)amino)isoindolin-1,3-dione

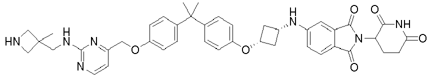

The target compound was synthesized by a method similar to that of Example 59. LC/MS (ESI+) Calcd for C₄₂H₄₅N₇O₆ (M+H⁺) *m*/*z* 743.3; found 743.3.

### Example 458: 5-(((1s,3s)-3-(4-(2-(4-((2-(3,3-dimethylpiperazin-1-yl)pyrimidin-4-yl)methoxy)phenyl)propan-2-yl)phenoxy)cyclobutyl)amino)-2-(2,6-dioxopiperidin-3-yl)isoindolin-1,3-dione

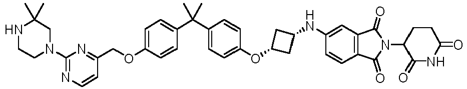

The target compound was synthesized by a method similar to that of Example 59. LC/MS (ESI+) Calcd for C₄₃H₄₇N₇O₆ (M+H⁺) *m*/*z* 757.4; found 757.4. ¹H NMR (400 MHz, Chloroform-*d) δ* 8.32 (d, *J* = 5.0 Hz, 1H), 7.62 (dd, *J* = 8.3, 3.3 Hz, 1H), 7.13 (ddd, *J* = 8.3, 5.1, 2.8 Hz, 4H), 6.91 (dd, *J* = 8.6, 2.1 Hz, 1H), 6.86 - 6.78 (m, 3H), 6.70 (dd, *J* = 8.7, 6.3 Hz, 3H), 4.95 (d, *J* = 3.8 Hz, 2H), 4.87 (s, 1H), 4.51 (t, *J=* 6.7 Hz, 1H), 4.22 (s, 1H), 3.77 (s, 1H), 3.25 (s, 2H), 3.16 (t, *J=* 12.4 Hz, 3H), 3.08 - 3.01 (m, 1H), 2.80 (ddd, *J=* 34.3, 27.9, 13.8 Hz, 5H), 2.63 - 2.55 (m, 3H), 2.42 (s, 1H), 2.17 - 2.04 (m, 2H), 1.63 (s, 6H).

### Example 459: 5-((1s,3s)-3-(4-(2-(2-oxa-6-azaspiro[3.3]heptan-6-yl)pyrimidin-4-yl)oxy)phenyl)propan-2-yl)phenoxy)cyclobutyl)amino)-2-(2,6-dioxopyridin-3-yl)isoindolin-1,3-dione

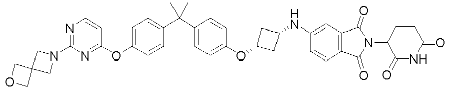

The target compound was synthesized by a method similar to that of Example 59. LC/MS (ESI+) Calcd for C₄₁H₄₀N₆O₇(M+H⁺) *m*/*z* 728.3; found 728.3 _{∘} ¹H NMR (400 MHz, Chloroform-*d*) *δ* 8.11 (d, *J*= 6.0 Hz, 1H), 8.07 (s, 1H), 7.63 (d, *J* = 8.3 Hz, 1H), 7.24 (s, 1H), 7.14 (d, *J=* 8.7 Hz, 2H), 7.01 (d, *J* = 8.7 Hz, 2H), 6.92 (d, *J* = 2.2 Hz, 1H), 6.73 (td, *J* = 8.0, 7.2, 2.1 Hz, 3H), 6.08 (d, *J* = 5.7 Hz, 1H), 5.30 (s, 2H), 4.93 (dd, *J* = 12.0, 5.3 Hz, 1H), 4.77 (d, *J* = 6.5 Hz, 1H), 4.53 (t, *J* = 6.8 Hz, 1H), 4.32 (s, 3H), 3.78 (q, *J=* 7.3 Hz, 1H), 3.18 - 3.10 (m, 2H), 2.95 - 2.65 (m, 4H), 2.11 (d, *J* = 9.3 Hz, 3H), 1.68 (s, 6H).

### Example 460: 2-(2,6-dioxopiperidin-3-yl)-5-(((1s,3s)-3-(4-(2-(4-((2-(4-fluoro-1H-pyrazol)-1-yl)pyrimidin-4-yl)methoxy)phenyl)propan-2-yl)phenoxy)cyclobutyl) amino)isoindolin-1,3-dione

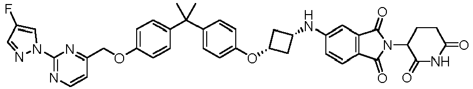

The target compound was synthesized by a method similar to that of Example 59. LC/MS (ESI+) Calcd for C₄₀H₃₆FN₇O₆ (M+H⁺) *m*/*z* 729.3; found 729.3. ¹H NMR (400 MHz, Chloroform-*d*) *δ* 8.54 (d, *J=* 5.1 Hz, 1H), 8.01 (s, 1H), 7.75 (d, *J=* 5.1 Hz, 1H), 7.63 (d, *J=* 8.3 Hz, 1H), 7.20 - 7.10 (m, 4H), 6.94 - 6.85 (m, 4H), 6.75 - 6.69 (m, 3H), 5.29 (s, 2H), 4.93 (dd, *J* = 12.1, 5.3 Hz, 1H), 4.51 (t, *J* = 6.8 Hz, 1H), 3.78 (t, *J* = 7.6 Hz, 1H), 3.17 - 3.08 (m, 2H), 2.94 - 2.82 (m, 2H), 2.78 (d, *J=* 11.3 Hz, 1H), 2.72 (s, 3H), 1.63 (s, 6H).

### Example 461: Synthesis of 5-(((1s,3s)-3-(4-(2-(4-((2-(4H-1,2,4-triazol-4-yl) pyrimidin-4-yl)methoxy)phenyl)propan-2-yl)phenoxy)cyclobutyl)amino)-2-(2,6-dioxopiperidin-3-yl)isoindolin-1,3-dione

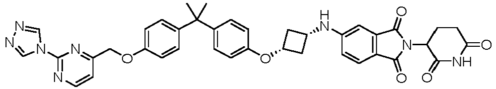

The target compound was synthesized by a method similar to that of Example 436. LC/MS (ESI+) Calcd for C₃₉H₃₆N₈O₆ (M+H⁺) *m*/*z* 712.3; found 712.3. ¹H NMR (400 MHz, CDCl₃) *δ* 8.10 (s, 1H), 8.00 (d, *J* = 9.5 Hz, 1H), 7.86 (d, *J* = 8.2 Hz, 1H), 7.56 (d, *J* = 8.7 Hz, 1H), 7.45 (d, *J* = 11.8 Hz, 1H), 7.17 (d, *J* = 6.7 Hz, 1H), 7.00 (dd, *J* = 5.9, 3.6 Hz, 2H), 6.85 (dd, *J=* 8.7, 2.4 Hz, 1H), 4.92 (dd, *J* = 12.3, 5.3 Hz, 1H), 4.49 (s, 2H), 4.32 (t, *J* = 10.1 Hz, 2H), 4.05 (d, *J* = 8.2 Hz, 2H), 3.53 (s, 4H), 3.14 (s, 4H), 2.91 - 2.66 (m, 6H), 2.20 - 2.10 (m, 7H), 1.54 - 1.38 (m, 4H).

### Example 462: Synthesis of 2-(2,6-dioxopiperidin-3-yl)-5-(((1s,3s)-3-(4-(2-(4-((2-(1-methyl-3-(trifluoromethyl)))1H-pyrazol-5-yl)pyrimidin-4-yl)methoxy)phenyl) propan-2-yl)phenoxy)cyclobutyl)amino)isoindolin-1,3-dione

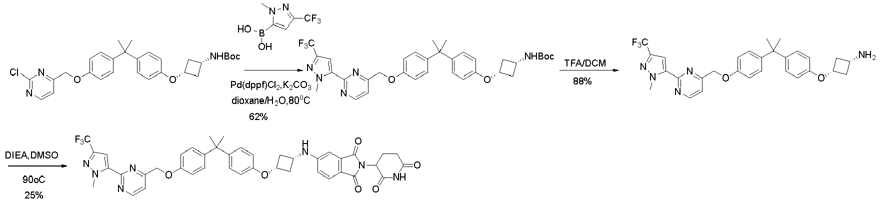

### Step 1: Synthesis of compound tert-butyl ((1s,3s)-3-(4-(2-(4-((2-(1-methyl-3-(trifluoromethyl)-1H-pyrazol-5-yl)pyrimidin-4-yl))methoxy)phenyl)propan-2-yl) phenoxy)cyclobutyl)carbamate

tert-butyl ((1s,3s)-3-(4-(2-(4-((2-chloropyrimidin-4-yl)methoxy)phenyl)propan-2-yl)phenoxy)cyclobutyl)carbamate (200 mg, 0.37 mmol) and (1-methyl-3-(trifluoromethyl)-1H-pyrazol-5-yl)boric acid (110 mg, 0.56 mmol) were dissolved in a mixed solvent of dioxane and water (10:1), to which were added 0.05% Pd(dppf)Cl₂ and K₂CO₃ (103 mg, 0.74 mmol), and then under nitrogen protection, the reaction solution was heated to 80 °C and reacted for 2h. The system was cooled to room temperature, and filtered over diatomite pad. The filtrate was concentrated, and the residue was purified by TLC, to provide tert-butyl ((1s,3s)-3-(4-(2-(4-((2-(1-methyl-3-(trifluoromethyl)-1H-pyrazol-5-yl)pyrimidin-4-yl))methoxy)phenyl)propan-2-yl) phenoxy)cyclobutyl)carbamate (193 mg), with a yield of 62%. LC/MS (ESI+) Calcd for C₃₄H₃₈F₃N₅O₄ (M+H⁺) *m*/*z* 637.3; found 637.3.

The method in the following steps were similar to that of Example 446. LC/MS (ESI+) Calcd for C₄₃H₄₅ClN₈O₆ (M+H⁺) *m*/*z* 805.3; found 805.3. ¹H NMR (400 MHz, Chloroform-*d*) *δ* 8.54 (d, *J* = 5.1 Hz, 1H), 8.01 (s, 1H), 7.75 (d, *J=* 5.1 Hz, 1H), 7.63 (d, *J* = 8.3 Hz, 1H), 7.20 - 7.10 (m, 4H), 6.94 - 6.85 (m, 4H), 6.75 - 6.69 (m, 3H), 5.29 (s, 2H), 4.93 (dd, *J=* 12.1, 5.3 Hz, 1H), 4.51 (t, *J* = 6.8 Hz, 1H), 3.78 (t, *J* = 7.6 Hz, 1H), 3.17 - 3.08 (m, 2H), 2.94 - 2.82 (m, 2H), 2.78 (d, *J* = 11.3 Hz, 1H), 2.72 (s, 3H), 2.11 (d, *J* = 10.3 Hz, 3H), 1.63 (s, 6H).

### Example 463: Synthesis of 2-(2,6-dioxopiperidin-3-yl)-5-(((1r,3r)-3-(4-(2-(4-((6-(5-methyl-1,3,4-oxadiazole)-2-yl)pyridin-2-yl)methoxy)phenyl)propan-2-yl)phenoxy) cyclobutyl)amino)isoindolin-1,3-dione

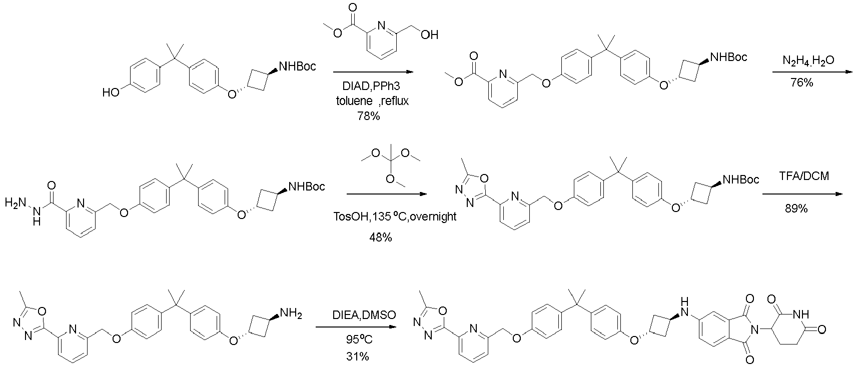

### Step 1: Synthesis of methyl 6-((4-(2-(4-((1r,3r)-3-((tert-butoxycarbonyl)amino) cyclobutyloxy)phenyl)propan-2-yl)phenoxy)methyl)picolinate

tert-butyl ((1r,3r)-3-(4-(2-(4-hydroxylphenyl)propan-2-yl)phenoxy)cyclobutyl) carbamate (0.5 g, 1.26 mmol) and methyl 6-(hydroxylmethyl)picolinate (0.2 g, 1.26 mmol) were dissolved in 20 mL of toluene, to which was added triphenylphosphine (0.4 g, 1.51 mmol), followed by adding DIAD (0.2 g, 1.51 mmol) dropwise in an ice bath. Subsequently, the ice bath was removed, and the system was transferred to an oil bath at 95 °C, then allowed to react overnight. The reaction solution was concentrated to dry, and to the residue, were added EA and water. The reaction mixture was poured into a separatory funnel and separated. The organic phase was washed with saturated brine, and dried, followed by column chromatography, to provide methyl 6-((4-(2-(4-((1r,3r)-3-((tert-butoxycarbonyl)amino)cyclobutyloxy)phenyl)propan-2-yl)phenoxy) methyl)picolinate (0.5 g), with a yield of 78%. LC/MS (ESI+) Calcd for C₃₂H₃₈N₂O₆ (M+H⁺) *m*/*z* 546.3; found 546.3.

### Step 2: Synthesis of tert-butyl ((1r,3r)-3-(4-(2-(4-((6-(hydrazinecarbonyl)pyridin-2-yl)methoxy)phenyl)propan-2-yl)phenoxy)cyclobutyl)carbamate

Intermediate methyl 6-((4-(2-(4-((1r,3r)-3-((tert-butoxycarbonyl)amino) cyclobutyloxy)phenyl)propan-2-yl)phenoxy)methyl)picolinate (500 mg, 0.95 mmol) was dissolved in 10 mL and 2 mL hydrazine hydrate, and then the solution was heated and refluxed for 1h, followed by TLC, to provide tert-butyl ((1r,3r)-3-(4-(2-(4-((6-(hydrazinecarbonyl)pyridin-2-yl)methoxy)phenyl)propan-2-yl)phenoxy)cyclobutyl) carbamate (350 mg).

### Step 3: Synthesis of tert-butyl ((1r,3r)-3-(4-(2-(4-((6-(5-methyl-1,3,4-oxadiazol-2-yl)pyridin-2-yl)methoxy)phenyl)propan-2-yl)phenoxy)cyclobutyl)carbamate

Intermediate tert-butyl ((1r,3r)-3-(4-(2-(4-((6-(hydrazinecarbonyl)pyridin-2-yl) methoxy)phenyl)propan-2-yl)phenoxy)cyclobutyl)carbamate (350 mg, 0.64 mmol) was dissolved in 6 mL of triethyl orthoacetate, to which was added a catalytic amount of TosOH, and then the solution was heated to 135 °C and allowed to react overnight. The reaction solution was cooled to room temperature, to which were added EA and water. The reaction mixture was poured into a separatory funnel and separated. The organic phase was washed with saturated brine, dried, and concentrated, followed by column chromatography to provide tert-butyl ((1r,3r)-3-(4-(2-(4-((6-(5-methyl-1,3,4-oxadiazol-2-yl)pyridin-2-yl)methoxy)phenyl)propan-2-yl)phenoxy)cyclobutyl) carbamate (120 mg). LC/MS (ESI+) Calcd for C₃₃H₃₈N₄O₅ (M+H⁺) *m*/*z* 570.3; found 570.3.
The method in the following steps were similar to that of Example 446. LC/MS (ESI+) Calcd for C₄₁H₃₈N₆O₇ (M+H⁺) *m*/*z* 726.3; found 726.3. ¹H NMR (400 MHz, Chloroform-*d*) *δ* 8.12 (d, *J* = 7.7 Hz, 1H), 8.09 (s, 1H), 7.96 (t, *J=* 7.8 Hz, 1H), 7.80 (d, *J=* 7.9 Hz, 1H), 7.62 (d, *J=* 8.3 Hz, 1H), 7.14 (dd, *J=* 10.5, 8.0 Hz, 4H), 6.94 - 6.85 (m, 3H), 6.72 (dd, *J=* 8.6, 2.3 Hz, 3H), 5.32 (s, 2H), 5.30 (s, 1H), 4.93 (dd, *J=* 12.1, 5.2 Hz, 1H), 4.51 (t, *J=* 6.8 Hz, 1H), 3.82 - 3.72 (m, 1H), 3.17 - 3.06 (m, 2H), 2.97 - 2.65 (m, 4H), 2.53 (s, 3H), 2.13 - 2.07 (m, 2H), 1.63 (s, 6H).

### Example 464: Synthesis of 5-(((1s,3s)-3-(4-(2-(4-((2-(1,3,4-oxadiazol-2-yl) pyrimidin-4-yl)methoxy)phenyl)propan-2-yl)phenoxy)cyclobutyl)amino)-2-(2,6-dioxopiperidin-3-yl)isoindolin-1,3-dione

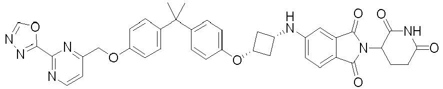

The target compound was synthesized by a method similar to that of Example 463. LC/MS (ESI+) Calcd for C₃₉H₃₅N₇O₇ (M+H⁺) *m*/*z* 713.3; found 713.3. ¹H NMR (400 MHz, Chloroform-*d) δ* 8.97 (d, *J=* 5.1 Hz, 1H), 8.64 (s, 1H), 8.00 (s, 1H), 7.79 (d, *J=* 5.2 Hz, 1H), 7.64 (d, *J=* 8.3 Hz, 1H), 7.18 (d, *J* = 8.8 Hz, 2H), 7.13 (d, *J* = 8.8 Hz, 2H), 6.88 (d, *J* = 8.6 Hz, 3H), 6.75 - 6.66 (m, 3H), 4.93 (dd, *J=* 12.1, 5.2 Hz, 1H), 4.86 (s, 1H), 4.25 (d, *J=* 12.7 Hz, 1H), 2.94 - 2.76 (m, 3H), 2.71 (dd, *J=* 11.5, 6.2 Hz, 2H), 2.40 (dt, *J=* 13.0, 6.6 Hz, 2H), 2.16 - 2.09 (m, 1H), 1.64 (s, 6H).

### Example 465: Synthesis of 2-(2,6-dioxopiperidin-3-yl)-5-(((1s,3s)-3-(4-(2-(4-((2-(5-methyl-1,3,4oxadiazole)-2-yl)pyrimidin-4-yl)methoxy)phenyl)propan-2-yl) phenoxy)cyclobutyl)amino)isoindolin-1,3-dione

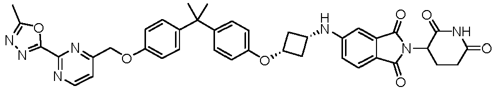

The target compound was synthesized by a method similar to that of Example 463. LC/MS (ESI+) Calcd for C₄₀H₃₇N₇O₇ (M+H⁺) *m*/*z* 727.3; found 727.3. ¹H NMR (400 MHz, Chloroform-*d) δ* 8.94 (d, *J=* 5.1 Hz, 1H), 8.01 (s, 1H), 7.75 (d, *J=* 5.1 Hz, 1H), 7.63 (d, *J* = 8.3 Hz, 1H), 7.20 -7.10 (m, 4H), 6.94 - 6.85 (m, 3H), 6.75 - 6.69 (m, 3H), 5.29 (s, 2H), 4.93 (dd, *J=* 12.1, 5.3 Hz, 1H), 4.51 (t, *J* = 6.8 Hz, 1H), 3.78 (t, *J* = 7.6 Hz, 1H), 3.17 - 3.08 (m, 2H), 2.94 - 2.82 (m, 2H), 2.78 (d, *J* = 11.3 Hz, 1H), 2.72 (s, 3H), 2.11 (d, *J* = 10.3 Hz, 3H), 1.64 (s, 6H).

### Example 466: Synthesis of 2-(2,6-dioxopiperidin-3-yl)-5-((1s,3s)-3-(4-(2-(2-(1-ethylazetidin-3-yl)oxy)pyrimidin-4-yl)methoxy)phenyl)propan-2-yl)phenoxy) cyclobutyl)amino)isoindolin-1,3-dione

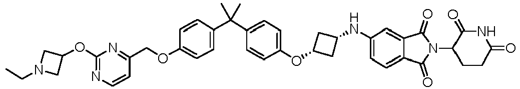

The target compound was synthesized by a method similar to that of Example 66. LC/MS (ESI+) Calcd for C₄₂H₄₄N₆O₇ (M+H⁺) *m*/*z* 744.3; found 744.3. ¹H NMR (400 MHz, Chloroform-*d) δ* 9.12 (s, 1H), 8.47 (d, *J* = 7.5 Hz, 1H), 7.78 (d, *J* = 5.1 Hz, 1H), 7.29 (d, *J* = 8.3 Hz, 1H), 7.20 - 7.10 (m, 5H), 7.07 (dd, *J* = 7.5, 1.5 Hz,1H), 6.91 - 6.84 (m, 4H), 5.45 (t, *J* = 7.0 Hz, 1H), 5.35 (m, 1H), 5.26 - 5.10 (m, 2H), 4.79 (p, *J* = 7.0 Hz, 1H), 4.71 (p, *J* = 7.0 Hz, 1H), 3.74 (t, *J* = 6.8 Hz, 1H), 3.46 (t, *J=* 7.6 Hz, 1H), 3.44 - 3.32 (m, 3H), 2.69 - 2.58 (m, 2H), 2.48 - 2.23 (m, 4H), 2.27 (p, *J=* 7.0 Hz, 1H), 2.72 (s, 3H), 2.11 (d, *J=* 10.3 Hz, 3H), 1.64 (s, 6H).

### Example 467: Synthesis of 5-(((1s,3s)-3-(4-(2-(4-((2-((1-cyclopropylazetidin-3-yl)oxy)pyrimidin-4-yl)methoxy)phenyl)propan-2-yl)phenoxy)cyclobutyl)amino)-2-(2,6-dioxopiperidin-3-yl)isoindolin-1,3-dione

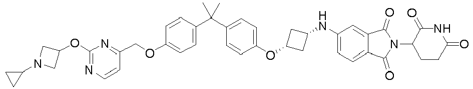

The target compound was synthesized by a method similar to that of Example 66. LC/MS (ESI+) Calcd for C₄₃H₄₄N₆O₇ (M+H⁺) *m*/*z* 756.3; found 756.3. ¹H NMR (400 MHz, DMSO-*d*₆) *δ* 8.59 (d, *J=* 5.0 Hz, 1H), 7.58 (d, *J=* 8.3 Hz, 1H), 7.45 (d, *J=* 6.5 Hz, 1H), 7.20 (d, *J=* 5.0 Hz, 1H), 7.15 - 7.07 (m, 4H), 6.89 (td, *J* = 5.3, 2.2 Hz, 3H), 6.82 (dd, *J* = 8.3, 2.1 Hz, 1H), 6.79 - 6.73 (m, 2H), 5.10 - 5.01 (m, 4H), 4.49 (t, *J* = 6.9 Hz, 1H), 3.77 (q, *J=* 7.3 Hz, 1H), 3.67 (td, *J* = 6.3, 1.9 Hz, 2H), 3.19 - 3.13 (m, 2H), 3.08 - 3.00 (m, 2H), 2.54 (s, 2H), 2.07 - 1.86 (m, 5H), 1.57 (s, 6H), 1.55 (s, 1H), 1.23 (s, 3H).

### Example 468: Synthesis of 5-(((1r,3r)-3-(4-(2-(4-((2-(1,3,4-oxadiazol-2-yl) pyrimidin-4-yl)methoxy)phenyl)propan-2-yl)phenoxy)cyclobutyl)amino)-2-(2,6-dioxopiperidin-3-yl)isoindolin-1,3-dione

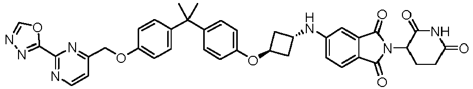

The target compound was synthesized by a method similar to that of Example 463. LC/MS (ESI+) Calcd for C₃₉H₃₅N₇O₇ (M+H⁺) *m*/*z* 713.3; found 713.3. ¹H NMR (400 MHz, Chloroform-*d) δ* 8.97 (d, *J=* 5.1 Hz, 1H), 8.64 (s, 1H), 8.00 (s, 1H), 7.79 (d, *J=* 5.2 Hz, 1H), 7.64 (d, *J=* 8.3 Hz, 1H), 7.18 (d, *J* = 8.8 Hz, 2H), 7.13 (d, *J* = 8.8 Hz, 2H), 6.88 (d, *J* = 8.6 Hz, 3H), 6.75 - 6.66 (m, 3H), 4.93 (dd, *J=* 12.1, 5.2 Hz, 1H), 4.86 (s, 1H), 4.25 (d, *J=* 12.7 Hz, 1H), 2.94 - 2.76 (m, 3H), 2.71 (dd, *J=* 11.5, 6.2 Hz, 2H), 2.40 (dt, *J=* 13.0, 6.6 Hz, 2H), 2.16 - 2.09 (m, 1H), 1.64 (s, 6H).

### Example 469: Synthesis of 5-(((1r,3r)-3-(4-(2-(4-((2-(2H-1,2,3-triazol-2-yl) pyrimidin-4-yl)oxy)phenyl)propan-2-yl)phenoxy)cyclobutyl)amino)-2-(2,6-dioxopiperidin-3-yl)isoindolin-1,3-dione

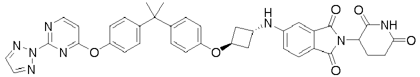

The target compound was synthesized by a method similar to that of Example 163. LC/MS (ESI+) Calcd for C₄₂H₄₃ClFN₉O₆ (M+H⁺) *m*/*z* 826.3; found 826.3. ¹H NMR (400 MHz, CDCl₃) *δ* 8.10 (s, 1H), 8.00 (d, *J* = 9.5 Hz, 1H), 7.86 (d, *J* = 8.2 Hz, 1H), 7.56 (d, *J* = 8.7 Hz, 1H), 7.45 (d, *J* = 11.8 Hz, 1H), 7.17 (d, *J* = 6.7 Hz, 1H), 7.00 (dd, *J* = 5.9, 3.6 Hz, 2H), 6.85 (dd, *J* = 8.7, 2.4 Hz, 1H), 4.92 (dd, *J* = 12.3, 5.3 Hz, 1H), 4.49 (s, 2H), 4.32 (t, *J* = 10.1 Hz, 2H), 4.05 (d, *J* = 8.2 Hz, 2H), 3.53 (s, 4H), 3.14 (s, 4H), 2.91 -2.66 (m, 6H), 2.20 - 2.10 (m, 7H), 1.54 - 1.38 (m, 4H).

### Example 470: Synthesis of 5-(((1r,3r)-3-(4-(2-(4-((6-(1,3,4-oxadiazol-2-yl)pyridin-2-yl)methoxy)phenyl)propan-2-yl)phenoxy)cyclobutyl)amino)-2-(2,6-dioxopiperidin-3-yl)isoindolin-1,3-dione

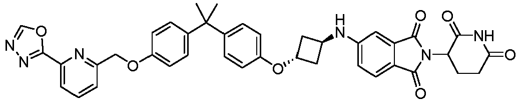

The target compound was synthesized by a method similar to that of Example 463. LC/MS (ESI+) Calcd for C₄₀H₃₆N₆O₇ (M+H⁺) *m*/*z* 712.3; found 712.3. ¹H NMR (400 MHz, Chloroform-*d) δ* 8.57 (s, 1H), 8.20 (d, *J=* 7.7 Hz, 1H), 8.02 (s, 1H), 7.94 (t, *J=* 7.8 Hz, 1H), 7.77 (d, *J=* 7.9 Hz, 1H), 7.63 (d, *J* = 8.2 Hz, 1H), 7.18 - 7.11 (m, 4H), 6.94 - 6.86 (m, 3H), 6.70 (td, *J=* 7.7, 6.9, 2.1 Hz, 3H), 5.30 (d, *J=* 2.5 Hz, 2H), 4.93 (dd, *J=* 12.1, 5.2 Hz, 1H), 4.86 (s, 1H), 4.23 (t, *J* = 5.3 Hz, 1H), 2.94 - 2.81 (m, 2H), 2.76 (d, *J=* 5.1 Hz, 1H), 2.70 (d, *J=* 9.7 Hz, 2H), 2.40 (dt, *J=* 13.0, 6.4 Hz, 2H), 2.15 - 2.09 (m, 1H), 1.64 (s, 6H).

### Example 471: Synthesis of 5-(((1r,3r)-3-(4-(2-(4-((2-(1H-1,2,3-triazol-1-yl)pyrimidin-4-yl)oxy)phenyl)propan-2-yl)phenoxy)cyclobutyl)amino)-2-(2,6-dioxopiperidin-3-yl)isoindolin-1,3-dione

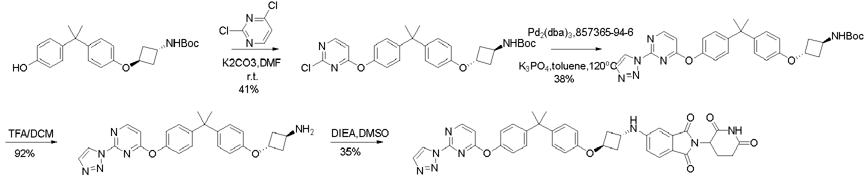

The target compound was synthesized by a method similar to that of Example 163. LC/MS (ESI+) Calcd for C₄₂H₄₃ClFN₉O₆ (M+H⁺) *m*/*z* 826.3; found 826.3. ¹H NMR (400 MHz, CDCl₃) *δ* 8.29 - 8.25 (m, 1H), 8.23 (s, 1H), 7.90 - 7.85 (m, 2H), 7.60 (d, *J=* 8.3 Hz, 1H), 7.31 - 7.27 (m, 2H), 7.19 - 7.14 (m, 2H), 7.10 - 7.05 (m, 2H), 6.87 (dd, *J* = 5.1, 3.4 Hz, 2H), 6.77 - 6.66 (m, 3H), 4.99 - 4.90 (m, 1H), 4.87 (dt, *J=* 6.8, 2.8 Hz, 1H), 4.21 (td, *J=* 7.6, 7.1, 3.8 Hz, 1H), 2.94 - 2.74 (m, 3H), 2.71 (dq, *J* = 8.3, 3.8 Hz, 2H), 2.41 (dt, *J* = 13.1, 6.3 Hz, 2H), 2.15 - 2.08 (m, 1H), 1.70 (s, 6H).

### Example 472: Synthesis of 2-(2,6-dioxopiperidin-3-yl)-5-(((1s,3s)-3-(4-(2-(4-((6-(3-methyl-1,2,4-oxadiazole)-5-yl)pyridin-2-yl)methoxy)phenyl)propan-2-yl)phenoxy) cyclobutyl)amino)isoindolin-1,3-dione

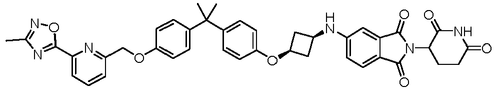

The target compound was synthesized by a method similar to that of Example 250. LC/MS (ESI+) Calcd for C₄₁H₃₈N₆O₇ (M+H⁺) *m*/*z* 726.3; found 726.3. ¹H NMR (400 MHz, Chloroform-*d) δ* 8.11 (d, *J* = 7.7 Hz, 1H), 8.06 (s, 1H), 7.94 (t, *J* = 7.8 Hz, 1H), 7.80 (d, *J* = 7.9 Hz, 1H), 7.62 (d, *J* = 8.3 Hz, 1H), 7.14 (dd, *J* = 10.5, 8.0 Hz, 4H), 6.94 - 6.85 (m, 3H), 6.72 (dd, *J* = 8.6, 2.3 Hz, 3H), 5.32 (s, 2H), 5.30 (s, 1H), 4.93 (dd, *J* = 12.1, 5.2 Hz, 1H), 4.51 (t, *J=* 6.8 Hz, 1H), 3.82 - 3.72 (m, 1H), 3.17 - 3.06 (m, 2H), 2.97 - 2.65 (m, 4H), 2.53 (s, 3H), 2.13 - 2.07 (m, 2H), 1.63 (s, 6H).

### Example 473: Synthesis of 2-(2,6-dioxopiperidin-3-yl)-5-(((1r,3r)-3-(4-(2-(4-((2-(5-methyl-1,3,4)-oxadiazol-2-yl)pyrimidin-5-yl)oxy)phenyl)propan-2-yl)phenoxy) cyclobutyl)amino)isoindolin-1,3-dione

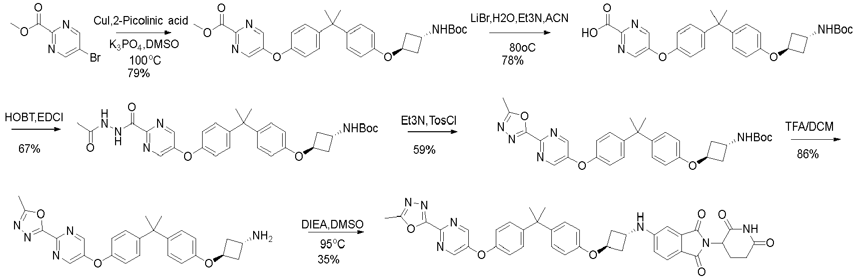

### Step 1: Synthesis of tert-butyl ((1r,3r)-3-(4-(2-(4-hydroxylphenyl)propan-2-yl)phenoxy) cyclobutyl)carbamate

tert-butyl ((1r,3r)-3-(4-(2-(4-hydroxylphenyl)propan-2-yl)phenoxy)cyclobutyl) carbamate (400 mg, 1.02 mmol) was dissolved in 10 mL of DMSO, to which were added methyl 5-bromopyrimidin-2-carboxylate (246 mg, 1.23 mmol), potassium phosphate (661 mg, 5.11 mmol), and 2-carboxylic acid of pyridine (14 mg, 0.11 mmol), and then the solution was heated to 100 °C and allowed to react for 2h. Water was added to quench the reaction. The resultant solution was extracted with EA. The organic phase was washed with saturated citric acid aqueous solution, followed by purification, to provide tert-butyl ((1r,3r)-3-(4-(2-(4-hydroxylphenyl)propan-2-yl)phenoxy)cyclobutyl) carbamate (450 mg), with a yield of 79%. LC/MS (ESI+) Calcd for C₃₀H₃₅N₃O₆ (M-56+H⁺) *m*/*z* 533.3; found 533.6.

### Step 2: Synthesis of 5-(4-(2-(4-((1r,3r)-3-((tert-butoxycarbonyl)amino)cyclobutyloxy) phenyl)propan-2-yl)phenoxy)pyrimidin-2-carboxylic acid

tert-butyl ((1r,3r)-3-(4-(2-(4-hydroxylphenyl)propan-2-yl)phenoxy)cyclobutyl) carbamate (400 mg, 0.64 mmol) was dissolved in 8 mL of acetonitrile, to which were added 0.1 mL of trifluoroacetic acid and 0.1 mL of water, and then the solution was heated to 80 °C and reacted for 1h. The reaction solution was concentrated to dry, and then the residue was dissolved in DCM. Subsequently, the pH of the solution was adjusted to 3 with 0.1M HCl, followed by column chromatography to provide 5-(4-(2-(4-((1r,3r)-3-((tert-butoxycarbonyl)amino)cyclobutyloxy)phenyl)propan-2-yl)phenoxy) pyrimidin-2-carboxylic acid (325 mg).

### Step 3: Preparation of tert-butyl ((1r,3r)-3-(4-(2-(4-((2-(2-acethydrazide-1-carbonyl) pyrimidin-5-yl)oxy)phenyl)propan-2-yl) phenoxy)cyclobutyl)carbamate

5-(4-(2-(4-((1r,3r)-3-((tert-butoxycarbonyl)amino)cyclobutyloxy)phenyl)propan-2-yl)phenoxy)pyrimidin-2-carboxylic acid (320 mg, 0.62 mmol) was dissolved in 8 mL of dichloromethane, to which were then added DIEA (320 mg, 2.48 mmol), HOBT (88 mg, 0.65 mmol), and EDCI (125mg,0.65mmol) in an ice bath. The reaction solution was warmed to room temperature and allowed to react at for 2h. The DCM phase was successively washed with saturated citric acid aqueous solution and saturated brine, dried, and concentrated, followed by purification over column chromatography to provide tert-butyl ((1r,3r)-3-(4-(2-(4-((2-(2-acethydrazide-1-carbonyl)pyrimidin-5-yl) oxy)phenyl)propan-2-yl) phenoxy)cyclobutyl)carbamate (220 mg).

### Step 4: Synthesis of tert-butyl ((1r,3r)-3-(4-(2-(4-(2-(5-methyl-1,3,4-oxadiazol-2-yl)pyrimidin-5-yl)oxy)phenyl)propan-2-yl)phenoxy)cyclobutyl)carbamate

tert-butyl ((1r,3r)-3-(4-(2-(4-((2-(2-acethydrazide-1-carbonyl)pyrimidin-5-yl)oxy) phenyl)propan-2-yl)phenoxy)cyclobutyl)carbamate (200 mg, 0.22 mmol) was dissolved in 5 mL of DCM, to which was added p-toluenesulfonyl chloride (186 mg, 0.44 mmol), and then the mixture was allowed to react overnight at room temperature, followed by purification with TLC (silica gel) to provide tert-butyl ((1r,3r)-3-(4-(2-(4-(2-(5-methyl-1,3,4-oxadiazol-2-yl)pyrimidin-5-yl)oxy)phenyl)propan-2-yl)phenoxy) cyclobutyl)carbamate (130 mg). LC/MS (ESI+) Calcd for C₃₁H₃₅N₅O₅ (M+H⁺) *m*/*z* 557.3; found 557.6.

### Step 5: Preparation of ((1r,3r)-3-(4-(2-(4-(2-(5-methyl-1,3,4-oxadiazol-2-yl)pyrimidin-5-yl)oxy)phenyl)propan-2-yl)phenoxy)cyclobutyl-1-amine

tert-butyl ((1r,3r)-3-(4-(2-(4-(2-(5-methyl-1,3,4-oxadiazol-2-yl)pyrimidin-5-yl) oxy)phenyl)propan-2-yl)phenoxy)cyclobutyl)carbamate (130 mg, 0.15 mmol) was dissolved in 8 mL of dichloromethane, to which was added 2 mL of trifluoroacetic acid in an ice bath, and then the mixture was allowed to react for 30 min. The pH of the reaction solution was adjusted to be neutral with saturated Na₂CO₃ solution in an ice bath, extracted with dichloromethane, dried over anhydrous Na₂SO₄, and concentrated under reduced pressure to provide ((1r,3r)-3-(4-(2-(4-(2-(5-methyl-1,3,4-oxadiazol-2-yl)pyrimidin-5-yl)oxy)phenyl)propan-2-yl)phenoxy)cyclobutyl-1-amine (101 mg). LC/MS (ESI+) Calcd for C₂₇H₃₀FN₄O₂ (M+H⁺) *m*/*z* 462.6; found 462.6.

### Step 6: Preparation of 2-(2,6-dioxopiperidin-3-yl)-5-(((1r,3r)-3-(4-(2-(4-((2-(5-methyl-1,3,4oxadiazol-2-yl)pyrimidin-5-yl)oxy)phenyl)propan-2-yl)phenoxy)cyclobutyl) amino)isoindolin-1,3-dione

Intermediate ((1r,3r)-3-(4-(2-(4-(2-(5-methyl-1,3,4-oxadiazol-2-yl)pyrimidin-5-yl)oxy)phenyl)propan-2-yl)phenoxy)cyclobutyl-1-amine (101 mg, 0.13 mmol) was dissolved in 5 mL of DMSO, to which was added DIEA (96.3 mg, 0.8 mmol), and then the solution was stirred well, followed by addition of 2-(2,6-dioxopiperidin-3-yl)-5-fluoroisoindolin-1,3-dione (47.3 mg, 0.2 mmol). The reaction solution was heated to 95 °C and then allowed to react overnight. After the reaction was completed by TLC detection, the reaction solution was cooled to room temperature, and extracted by adding water and ethyl acetate. The organic phase was washed with saturated brine, dried over anhydrous Na₂SO₄, and concentrated, followed by column chromatography to provide 2-(2,6-dioxopiperidin-3-yl)-5-(((1r,3r)-3-(4-(2-(4-((2-(5-methyl-1,3,4oxadiazol-2-yl)pyrimidin-5-yl)oxy)phenyl)propan-2-yl)phenoxy)cyclobutyl) amino)isoindolin-1,3-dione (43.2 mg), with a yield of 35%. LC/MS (ESI+) Calcd for C₃₉H₃₅N₇O₇ (M+H⁺) *m*/*z* 713.3; found 713.8. ¹H NMR (400 MHz, Chloroform-*d*) *δ* 8.59 (s, 2H), 8.03 (s, 1H), 7.63 (d, *J* = 8.3 Hz, 1H), 7.31 (d, *J* = 8.6 Hz, 2H), 7.14 (d, *J* = 8.6 Hz, 2H), 7.04 - 6.99 (m, 2H), 6.90 (d, *J* = 2.1 Hz, 1H), 6.72 (dd, *J* = 9.6, 3.9 Hz, 3H), 4.93 (dd, *J* = 12.1, 5.4 Hz, 1H), 4.88 (d, *J* = 4.0 Hz, 1H), 4.80 (s, 1H), 4.24 (s, 1H), 2.94 - 2.79 (m, 2H), 2.76 (d, *J* = 5.8 Hz, 1H), 2.75 - 2.70 (m, 2H), 2.69 (s, 3H), 2.42 (dt, *J=* 13.0, 6.1 Hz, 2H), 2.16 - 2.09 (m, 1H), 1.69 (s, 6H).

### Example 474: Synthesis of 2-(2,6-dioxopiperidin-3-yl)-5-(((1r,3r)-3-(4-(2-(4-((4-(5-methyl-1,2,4oxadiazol-)3-yl)pyridin-2-yl)oxy)phenyl)propan-2-yl)phenoxy) cyclobutyl)amino) soindolin-1,3-dione

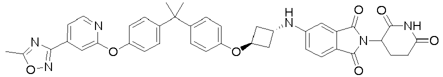

The target compound was synthesized by a method similar to that of Example 221. LC/MS (ESI+) Calcd for C₄₀H₃₆N₆O₇ (M+H⁺) *m*/*z* 712.3; found 712.3. ¹H NMR (400 MHz, Chloroform-*d) δ* 8.35 (s, 1H), 7.98 (s, 1H), 7.64 (d, *J=* 8.1 Hz, 2H), 7.56 (s, 1H), 7.28 (s, 1H), 7.18 (d, *J=* 8.5 Hz, 2H), 7.06 (d, *J* = 8.2 Hz, 2H), 6.91 (d, *J* = 2.0 Hz, 1H), 6.71 (d, *J* = 8.4 Hz, 3H), 4.93 (dd, *J* = 12.1, 5.2 Hz, 1H), 4.88 (s, 1H), 4.24 (s, 1H), 2.95 - 2.82 (m, 2H), 2.74 (d, *J* = 12.8 Hz, 2H), 2.69 (s, 3H), 2.45 - 2.37 (m, 2H), 2.17 - 2.06 (m, 2H), 1.68 (s, 6H).

### Example 475: Synthesis of 2-(2,6-dioxopiperidin-3-yl)-5-(((1r,3r)-3-(4-(2-(4-((5-(5-methyl-1,3,4oxadiazol-2-yl)pyrimidin-2-yl)oxy)phenyl)propan-2-yl)phenoxy) cyclobutyl)amino)isoindolin-1,3-dione

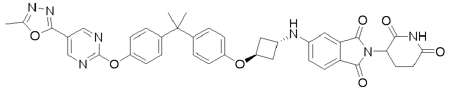

The target compound was synthesized by a method similar to that of Example 473. LC/MS (ESI+) Calcd for C₃₉H₃₅N₇O₇ (M+H⁺) *m*/*z* 713.3; found 713.3. ¹H NMR (400 MHz, Chloroform-*d) δ* 8.56 (s, 2H), 8.03 (s, 1H), 7.62 (d, *J=* 8.3 Hz, 1H), 7.31 (d, *J=* 8.6 Hz, 2H), 7.08 (d, *J=* 8.6 Hz, 2H), 7.04 - 6.99 (m, 2H), 6.90 (d, *J* = 2.1 Hz, 1H), 6.72 (dd, *J* = 9.6, 3.9 Hz, 3H), 4.93 (dd, *J* = 12.1, 5.4 Hz, 1H), 4.88 (d, *J* = 4.0 Hz, 1H), 4.80 (s, 1H), 4.24 (s, 1H), 2.94 - 2.79 (m, 2H), 2.76 (d, *J* = 5.8 Hz, 1H), 2.75 - 2.70 (m, 2H), 2.69 (s, 3H), 2.42 (dt, *J* = 13.0, 6.1 Hz, 2H), 2.16 - 2.09 (m, 1H), 1.69 (s, 6H).

### Example 476: Synthesis of 5-((((1r,3r)-3-(4-(2-(4-((2-(1H-1,2,3-triazol-1-yl)pyrimidin-5-yl)oxy)phenyl)propan-2-yl)phenoxy)cyclobutyl)amino)-2-(2,6-dioxopiperidin-3-yl)isoindolin-1,3-dione

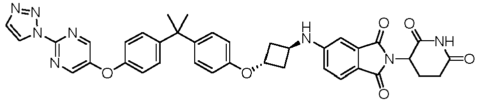

The target compound was synthesized by a method similar to that of Example 436. LC/MS (ESI+) Calcd for C₄₂H₄₃ClFN₉O₆ (M+H⁺) *m*/*z* 824.3; found 824.3. ¹H NMR (400 MHz, CDCl₃) *δ* 8.29 - 8.25 (m, 1H), 8.23 (s, 1H), 7.90 - 7.85 (m, 2H), 7.60 (d, *J=* 8.3 Hz, 1H), 7.31 - 7.27 (m, 2H), 7.19 - 7.14 (m, 2H), 7.10 - 7.05 (m, 2H), 6.87 (dd, *J* = 5.1, 3.4 Hz, 2H), 6.77 - 6.66 (m, 3H), 4.99 - 4.90 (m, 1H), 4.87 (dt, *J* = 6.8, 2.8 Hz, 1H), 4.21 (td, *J=* 7.6, 7.1, 3.8 Hz, 1H), 2.94 - 2.74 (m, 3H), 2.71 (dq, *J* = 8.3, 3.8 Hz, 2H), 2.41 (dt, *J* = 13.1, 6.3 Hz, 2H), 2.15 - 2.08 (m, 1H), 1.70 (s, 6H).

### Example 477: Synthesis of 2-(2,6-dioxopiperidin-3-yl)-5-(((1r,3r)-3-(4-(2-(4-(pyrimidin-5-yloxy)phenyl)propan-2-yl)phenoxy)cyclobutyl)amino)isoindolin-1,3-dione

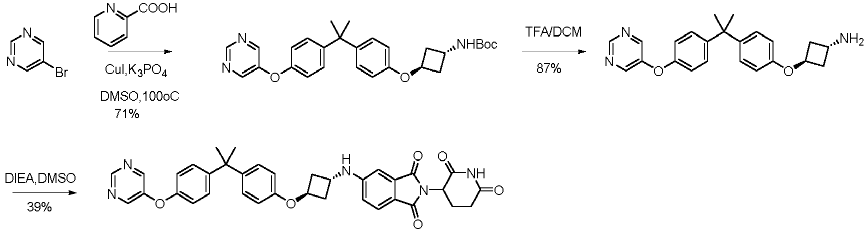

### Step 1: Synthesis of tert-butyl ((1r,3r)-3-(4-(2-(4-(pyrimidin-5-yloxy)phenyl)propan-2-yl)phenoxy)cyclobutyl)carbamate

tert-butyl ((1r,3r)-3-(4-(2-(4-hydroxylphenyl)propan-2-yl)phenoxy)cyclobutyl) carbamate (400 mg, 1.02 mmol) was dissolved in 10 mL of DMSO, to which were added 5-bromopyrimidine (200 mg, 1.26 mmol), potassium phosphate (661 mg, 5.11 mmol), and 2-pyridinecarboxylic acid (14 mg, 0.11 mmol), and then the solution was heated to 100 °C and reacted for 2 h. Water was added to quench the reaction. The resultant solution was extracted with EA. The organic phase was washed with saturated citric acid aqueous solution, followed by purification, to provide tert-butyl ((1r,3r)-3-(4-(2-(4-hydroxylphenyl)propan-2-yl)phenoxy)cyclobutyl)carbamate (410 mg), with a yield of 71%. LC/MS (ESI+) Calcd for C₃₀H₃₅N₃O₆ (M-56+H⁺) *m*/*z* 475.3; found 420.3.

### Step 2: Synthesis of ((1r,3r)-3-(4-(2-(4-(pyrimidin-5-yloxy)phenyl)propan-2-yl) phenoxy)cyclobutyl)amine

tert-butyl ((1r,3r)-3-(4-(2-(4-(pyrimidin-5-yloxy)phenyl)propan-2-yl)phenoxy) cyclobutyl)carbamate (200 mg, 0.42 mmol) was dissolved in 10 mL of dichloromethane, to which was added 3 mL of trifluoroacetic acid in an ice bath, and then the mixture was warmed to room temperature and allowed to react for 1.5 h, to which was added saturated Na₂CO₃ solution, to adjust the pH to weak alkalinity. The reaction mixture was poured into a separatory funnel and separated. The reaction solution was dried over anhydrous Na₂SO₄, and concentrated to dry, to provide ((1r,3r)-3-(4-(2-(4-(pyrimidin-5-yloxy)phenyl)propan-2-yl)phenoxy)cyclobutyl)amine (153 mg).

### Step 3: Synthesis of 2-(2,6-dioxopiperidin-3-yl)-5-(((1r,3r)-3-(4-(2-(4-(pyrimidin-5-yloxy)phenyl)propan-2-yl) phenoxy)cyclobutyl)amino)isoindolin-1,3-dione

Intermediate ((1r,3r)-3-(4-(2-(4-(pyrimidin-5-yloxy)phenyl)propan-2-yl)phenoxy) cyclobutyl)amine (150 mg, 0.13 mmol) was dissolved in 10 mL of DMSO, to which was added DIEA (96.3 mg, 0.8 mmol), and then the solution was stirred well, followed by addition of 2-(2,6-dioxopiperidin-3-yl)-5-fluoroisoindolin-1,3-dione (47.3 mg, 0.2 mmol). The reaction solution was heated to 95 °C and then allowed to react overnight. After the reaction was completed by TLC detection, the reaction solution was cooled to room temperature, and extracted with water and ethyl acetate. The organic phase was washed with saturated brine, dried over anhydrous Na₂SO₄, and concentrated, followed by column chromatography to provide 2-(2,6-dioxopiperidin-3-yl)-5-(((1r,3r)-3-(4-(2-(4-(pyrimidin-5-yloxy)phenyl)propan-2-yl) phenoxy)cyclobutyl)amino)isoindolin-1,3-dione (53.2 mg), with a yield of 39%. LC/MS (ESI+) Calcd for C36H35N5O6 (M+H+) *m*/*z* 631.3; found 631.2. ¹H NMR (400 MHz, Chloroform-d) *δ* 8.59 (s, 2H), 8.03 (s, 1H), 7.63 (d, *J* = 8.3 Hz, 2H), 7.31 (d, *J* = 8.6 Hz, 2H), 7.14 (d, *J* = 8.6 Hz, 2H), 7.04 - 6.99 (m, 2H), 6.90 (d, *J =* 2.1 Hz, 1H), 6.72 (dd, *J* = 9.6, 3.9 Hz, 3H), 4.93 (dd, *J* = 12.1, 5.4 Hz, 1H), 4.88 (d, *J* = 4.0 Hz, 1H), 4.80 (s, 1H), 4.24 (s, 1H), 2.94 - 2.79 (m, 2H), 2.76 (d, *J* = 5.8 Hz, 1H), 2.75 - 2.70 (m, 2H), 2.42 (dt, *J* = 13.0, 6.1 Hz, 2H), 2.16 - 2.09 (m, 1H), 1.69 (s, 6H).

### Example 478: 5-(((1r,3r)-3-(4-(2-(4-((2-((3-fluoroazetidin-1-yl)methyl)pyrimidin-5-yl)oxy)phenyl)propan-2-yl)phenoxy)cyclobutyl)amino)-2-(2,6-dioxopiperidin-3-yl)isoindolin-1,3-dione

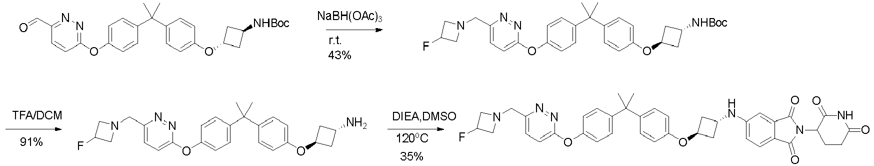

### Step 1: Preparation of tert-butyl ((1r,3r)-3-(4-(2-(4-((6-((3-fluoroazetidin-1-yl)methyl)pyridazine-3-yl)oxy)phenyl) propan-2-yl)phenoxy)cyclobutyl)carbamate

tert-butyl ((1r,3r)-3-(4-(2-(4-((6-formylpyridazine-3-yl)oxy)phenyl)propan-2-yl)phenoxy)cyclobutyl)carbamate (300 mg, 0.6 mmol) was dissolved in a mixed solvent of DCM/MeOH (4 mL/2 mL), to which was added 3,3-difluoroazetidine hydrochloride (105.2 mg, 0.9 mmol), followed by addition of NaBH(OAc)₃ (222.3 mg, 1.2 mmol) in an ice bath. After 2h, saturated NH₄Cl aqueous solution was added. The resultant solution was extracted with DCM and dried, followed by purification to provide tert-butyl ((1r,3r)-3-(4-(2-(4-((6-((3-fluoroazetidin-1-yl)methyl)pyridazine-3-yl)oxy)phenyl)propan-2-yl)phenoxy)cyclobutyl)carbamate (115.3 mg), with a yield of 43%. LC/MS (ESI+) Calcd for C₃₂H₃₈FN₄O₄ (M+H⁺) *m*/*z* 562.7; found 562.7.

### Step 2: Preparation of (1r,3r)-3-(4-(2-(4-((6-((3-fluoroazetidin-1-yl)methyl)pyridazine-3-yl)oxy)phenyl) propan-2-yl)phenoxy)cyclobutane-1-amine

tert-butyl ((1r,3r)-3-(4-(2-(4-((6-((3-fluoroazetidin-1-yl)methyl)pyridazine-3-yl) oxy)phenyl)propan-2-yl)phenoxy)cyclobutyl)carbamate (115.0 mg, 0.2 mmol) was dissolved in 8 mL of dichloromethane, to which was added 2 mL of trifluoroacetic acid in an ice bath, and then the mixture was allowed to react for 30 min. The pH of the reaction solution was adjusted to be neutral with saturated Na₂CO₃ solution in an ice bath, extracted with dichloromethane, dried over anhydrous Na₂SO₄, and concentrated under reduced pressure to provide (1r,3r)-3-(4-(2-(4-((6-((3 -fluoroazetidin-1-yl)methyl)pyridazine-3-yl)oxy)phenyl)propan-2-yl)phenoxy)cyclobutane-1-amine (101.3 mg).
LC/MS (ESI+) Calcd for C₂₇H₃₀FN₄O₂ (M+H⁺) *m*/*z* 462.6; found 462.6.

### Step 3: Preparation of 5-(((1r,3r)-3-(4-(2-(4-((2-((3-fluoroazetidin-1-yl)methyl) pyrimidin-5-yl)oxy)phenyl)propan-2-yl)phenoxy)cyclobutyl)amino)-2-(2,6-dioxopiperidin-3-yl)isoindolin-1,3-dione

Intermediate (1r,3r)-3-(4-(2-(4-((6-((3-fluoroazetidin-1-yl)methyl)pyridazine-3-yl) oxy)phenyl)propan-2-yl)phenoxy)cyclobutane-1-amine (101.0 mg, 0.2 mmol) was dissolved in 5 mL of DMSO, to which was added DIEA (96.3 mg, 0.8 mmol), and then the solution was stirred well, followed by addition of 2-(2,6-dioxopiperidin-3-yl)-5-fluoroisoindolin-1,3-dione (47.3 mg, 0.2 mmol). The reaction solution was heated to 120 °C and then allowed to react for 2h. After the reaction was completed by TLC detection, the reaction solution was cooled to room temperature, and extracted with water and ethyl acetate. The organic phase was washed with saturated brine, dried over anhydrous Na₂SO₄, and concentrated, followed by column chromatography to provide 5-(((1r,3r)-3-(4-(2-(4-((2-((3-fluoroazetidin-1-yl)methyl)pyrimidin-5-yl)oxy)phenyl) propan-2-yl)phenoxy)cyclobutyl)amino)-2-(2,6-dioxopiperidin-3-yl)isoindolin-1,3-dione (43.2 mg), with a yield of 35%. LC/MS (ESI+) Calcd for C₄₀H₃₈FN₆O₆ (M+H⁺) *m*/*z* 718.8; found 718.8. ¹H NMR (400 MHz, CDCl₃) *δ* 8.55 (s, 1H), 7.62 (d, *J* = 8.3 Hz, 1H), 7.23 - 7.16 (m, 2H), 7.13 (d, *J* = 8.3 Hz, 2H), 7.02 (t, *J* = 8.5 Hz, 3H), 6.89 (d, *J* = 2.2 Hz, 1H), 6.70 (t, *J* = 8.4 Hz, 4H), 5.33 - 5.11 (m, 1H), 4.96 - 4.87 (m, 2H), 4.12 (d, *J* = 7.9 Hz, 2H), 3.94 (dd, *J* = 8.0, 4.4 Hz, 2H), 3.85 - 3.68 (m, 3H), 3.38 (d, *J* = 24.2 Hz, 2H), 2.83 (ddd, *J* = 26.6, 18.4, 14.9 Hz, 3H), 2.11 (d, *J* = 10.4 Hz, 1H), 1.64 (s, 6H).

### Example 479: 5-(((1r,3r)-3-(4-(2-(4-((2-((3,3-difluoroazetidin-1-yl)methyl) pyrimidin-5-yl)oxy)phenyl)propan-2-yl)phenoxy)cyclobutyl)amino)-2-(2,6-dioxopiperidin-3-yl)isoindolin-1,3-dione

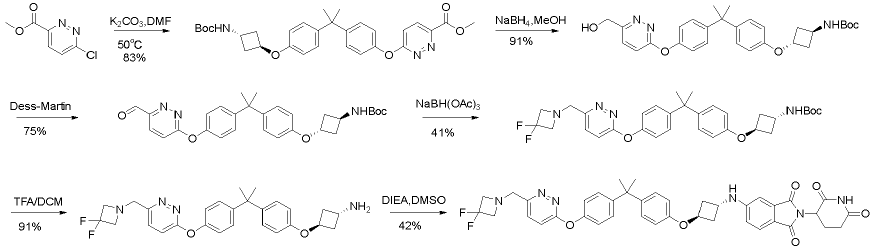

### Step 1: Methyl 6-(4-(2-(4-((1r,3r)-3-((tert-butoxycarbonyl)amino)cyclobutyloxy) phenyl)propan-2-yl)phenoxy)pyridazine-3-carboxylate

Methyl 6-chloropyridazine-3-carboxylate (1.50 g, 8.69 mmol) was dissolved in 50 mL of DMF, to which were added tert-butyl ((1r,3r)-3-(4-(2-(4-hydroxylphenyl)propan-2-yl)phenoxy)cyclobutyl)carbamate (4.1 g, 10.4 mmol) and K₂CO₃ (2.4 g, 17.4 mmol), and then the solution was heated to 50 °C and reacted for 2 h. The system was added to water, and then extracted with ethyl acetate. The organic phase was dried over anhydrous Na₂SO₄, followed by column chromatography to provide methyl 6-(4-(2-(4-((1r,3r)-3-((tert-butoxycarbonyl)amino)cyclobutyloxy) phenyl)propan-2-yl)phenoxy)pyridazine-3-carboxylate (1.4 g), with a yield of 83%. LC/MS (ESI+) Calcd for C₃₀H₃₅N₃O₆ (M+H⁺) *m*/*z* 533.25; found 478.25.

### Step 2: Preparation of tert-butyl ((1r,3r)-3-(4-(2-(4-((6-(hydroxylmethyl)pyridazine-3-yl)oxy)phenyl)propan-2-yl)phenoxy)cyclobutyl)carbamate

Methyl 6-(4-(2-(4-((1r,3r)-3-((tert-butoxycarbonyl)amino)cyclobutyloxy)phenyl) propan-2-yl)phenoxy)pyridazine-3-carboxylate (1.2 g, 2.4 mmol) was dissolved in 24 mL of methanol, to which was added sodium borohydride (1.1 g, 28.5 mmol) in portions, accompanied by release of many bubbles. After 5h, the system was added to cold water, and extracted with DCM, to provide crude tert-butyl ((1r,3r)-3-(4-(2-(4-((6-(hydroxylmethyl)pyridazine-3-yl)oxy)phenyl)propan-2-yl)phenoxy)cyclobutyl) carbamate (1.2 g), with a yield of 91%. LC/MS (ESI+) Calcd for C₂₉H₃₅N₃O₅ (M+H⁺) *m*/*z* 505.3; found 450.3.

### Step 3: Preparation of tert-butyl ((1r,3r)-3-(4-(2-(4-((6-formylpyridazine-3-yl)oxy)phenyl)propan-2-yl)phenoxy)cyclobutyl)carbamate

tert-butyl ((1r,3r)-3-(4-(2-(4-((6-(hydroxylmethyl)pyridazine-3-yl)oxy)phenyl) propan-2-yl)phenoxy)cyclobutyl)carbamate (1g,2.0mmol) was dissolved in 20 mL of dichloromethane, to which was added Dess-Martin (1.0 g, 2.2 mmol), and then the mixture was allowed to react at room temperature for 2h, followed by washing with saturated NaHSO₃ solution and saturated Na₂CO₃ aqueous solution. The resultant solution was dried and purified by column chromatography, to provide tert-butyl ((1r,3r)-3-(4-(2-(4-((6-formylpyridazine-3-yl)oxy)phenyl)propan-2-yl)phenoxy) cyclobutyl)carbamate (601.5 mg), with a yield of 75%. LC/MS (ESI+) Calcd for C₂₉H₃₃N₃O₅ (M-55+H⁺) *m*/*z* 503.3; found 448.3.

### Step 4: Preparation of tert-butyl ((1r,3r)-3-(4-(2-(4-((6-((3,3-difluoroazetidin-1-yl)methyl)pyridazine-3-yl)oxy)phenyl) propan-2-yl)phenoxy)cyclobutyl)carbamate

tert-butyl ((1r,3r)-3-(4-(2-(4-((6-formylpyridazine-3-yl)oxy)phenyl)propan-2-yl)phenoxy)cyclobutyl)carbamate (300 mg, 0.6 mmol) was dissolved in a mixed solvent of DCM/MeOH (4mL /2mL), to which was added 3,3-difluoroazetidine hydrochloride (116.2 mg, 0.9 mmol), followed by addition of NaBH(OAc)₃ (254.3 mg, 1.2 mmol) in an ice bath. After 2 h, saturated NH₄Cl aqueous solution was added to the reaction solution. The resultant solution was extracted with DCM. The organic phase was dried and purified, to provide tert-butyl ((1r,3r)-3-(4-(2-(4-((6-((3,3-difluoroazetidin-1-yl)methyl)pyridazine-3 -yl)oxy)phenyl)propan-2-yl)phenoxy) cyclobutyl)carbamate (140 mg), with a yield of 41%. LC/MS (ESI+) Calcd for C₃₂H₃₈F₂N₄O₄ (M+H⁺) *m*/*z* 580.3; found 580.3.

### Step 5: Preparation of (1r,3r)-3-(4-(2-(4-((6-((3,3-difluoroazetidin-1-yl)methyl) pyridazine-3-yl)oxy)phenyl) propan-2-yl)phenoxy)cyclobutane-1-amine

tert-butyl ((1r,3r)-3-(4-(2-(4-((6-((3,3-difluoroazetidin-1-yl)methyl)pyridazine-3-yl)oxy)phenyl)propan-2-yl)phenoxy)cyclobutyl)carbamate (140.0 mg, 0.2 mmol) was dissolved in 8 mL of dichloromethane, to which was added 2 mL of trifluoroacetic acid in an ice bath, and then the mixture was allowed to react for 30 min. The pH of the reaction solution was adjusted to be neutral with saturated Na₂CO₃ solution in an ice bath. The resultant solution was extracted with dichloromethane, dried over anhydrous Na₂SO₄, and concentrated under reduced pressure to provide (1r,3r)-3-(4-(2-(4-((6-((3,3-difluoroazetidin-1-yl)methyl)pyridazine-3-yl)oxy)phenyl)propan-2-yl)phenoxy) cyclobutane-1-amine (110.3 mg). LC/MS (ESI+) Calcd for C₂₇H₃₀F₂N₄O₂ (M+H⁺) *m*/*z* 480.2; found 480.2.

### Step 6: Preparation of 5-(((1r,3r)-3-(4-(2-(4-((2-((3,3-difluoroazetidin-1-yl)methyl) pyrimidin-5-yl)oxy)phenyl)propan-2-yl)phenoxy)cyclobutyl)amino)-2-(2,6-dioxopiperidin-3-yl)isoindolin-1,3-dione

Intermediate (1r,3r)-3-(4-(2-(4-((6-((3,3-difluoroazetidin-1-yl)methyl)pyridazine-3-yl)oxy)phenyl) propan-2-yl)phenoxy)cyclobutane-1-amine (110 mg, 0.2 mmol) was dissolved in 5 mL of DMSO, to which was added DIEA (97.3 mg, 0.8 mmol), and then the solution was stirred well, followed by addition of 2-(2,6-dioxopiperidin-3-yl)-5-fluoroisoindolin-1,3-dione (49.9 mg, 0.2 mmol). The reaction solution was heated to 120 °C and then allowed to react for 2 h. After the reaction was completed by TLC detection, the reaction solution was cooled to room temperature, and extracted by adding water and ethyl acetate. The organic phase was washed with saturated brine, dried over anhydrous Na₂SO₄, and concentrated, followed by column chromatography to provide 5-(((1r,3r)-3-(4-(2-(4-((2-((3,3-difluoroazetidin-1-yl)methyl)pyrimidin-5-yl)oxy)phenyl)propan-2-yl)phenoxy)cyclobutyl)amino)-2-(2,6-dioxopiperidin-3-yl) isoindolin-1,3-dione (55.2 mg), with a yield of 42%. LC/MS (ESI+) Calcd for C₄₀H₃₈F₂N₆O₆ (M+H⁺) *m*/*z* 736.3; found 736.8. ¹H NMR (400 MHz, CDCl₃) *δ* 8.68 (s, 1H), 7.62 (d, *J* = 8.3 Hz, 1H), 7.23 - 7.16 (m, 2H), 7.13 (d, *J* = 8.3 Hz, 2H), 7.02 (t, *J* = 8.5 Hz, 3H), 6.89 (d, *J* = 2.2 Hz, 1H), 6.70 (t, *J* = 8.4 Hz, 4H), 5.33 - 5.11 (m, 1H), 4.96 - 4.87 (m, 1H), 4.12 (d, *J* = 7.9 Hz, 2H), 3.94 (dd, *J* = 8.0, 4.4 Hz, 2H), 3.85 - 3.68 (m, 3H), 3.38 (d, *J* = 24.2 Hz, 2H), 2.83 (ddd, *J* = 26.6, 18.4, 14.9 Hz, 3H), 2.08 (d, *J* = 10.4 Hz, 1H), 1.63 (s, 6H).

### Example 480: Synthesis of 2-(2,6-dioxopiperidin-3-yl)-5-(((1r,3r)-3-(4-(1-(4-((2-(5-methyl-1,2,4oxadiazol-)3-yl)pyrimidin-5-yl)oxy)phenyl)ethyl)phenoxy)cyclobutyl) amino)isoindolin-1,3-dione

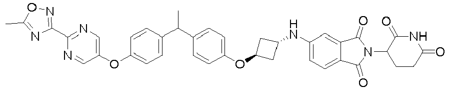

The target compound was synthesized by a method similar to that of Example 221. LC/MS (ESI+) Calcd for C₃₈H₃₃N₇O₇(M+H⁺) *m*/*z* 699.2; found 699.3. ¹H NMR (400 MHz, Chloroform-*d*) *δ* 9.12 (s, 1H), 8.60 (s, 2H), 7.79 (d, *J* = 7.5 Hz, 1H), 7.28 (d, *J* = 1.5 Hz, 1H), 7.11 (dd, *J* = 7.5, 1.5 Hz, 3H), 7.05 - 7.01 (m, 2H), 6.91 - 6.85 (m, 4H), 5.43 (t, *J* = 7.1 Hz, 1H), 5.35 (d, *J* = 11.0 Hz, 1H), 4.76 (pd, *J* = 7.0, 2.4 Hz, 1H), 4.53 - 4.45 (m, 1H), 3.80 - 3.69 (m, 1H), 2.64 - 2.60 (m, 2H), 2.59 (s, 3H), 2.52 (dt, *J=* 12.5, 7.0 Hz, 2H), 2.28 (dt, *J* = 12.4, 7.0 Hz, 2H), 2.20 - 2.07 (m, 2H), 1.46 (d, *J* = 6.8 Hz, 3H).

### Example 481: Synthesis of 2-(2,6-dioxopiperidin-3-yl)-5-(((1s,3s)-3-(4-(2-(4-((4-(5-methyl-1,2,4oxadiazol-)3-yl)pyridin-2-yl)oxy)phenyl)propan-2-yl)phenoxy) cyclobutyl)amino)isoindolin-1,3-dione

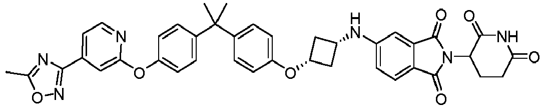

The target compound was synthesized by a method similar to that of Example 221. LC/MS (ESI+) Calcd for C₄₀H₃₆N₆O₇ (M+H⁺) *m*/*z* 712.3; found 712.3. ¹H NMR (400 MHz, Chloroform-*d*) *δ* 8.11 (d, *J* = 7.7 Hz, 1H), 8.06 (s, 1H), 7.88 (t, *J* = 7.8 Hz, 1H), 7.80 (d, *J* = 7.9 Hz, 1H), 7.62 (d, *J* = 8.3 Hz, 1H), 7.14 (dd, *J* = 10.5, 8.0 Hz, 4H), 6.94 - 6.85 (m, 3H), 6.72 (dd, *J* = 8.6, 2.3 Hz, 3H), 5.32 (s, 2H), 5.30 (s, 1H), 4.93 (dd, *J* = 12.1, 5.2 Hz, 1H), 4.51 (t, *J* = 6.8 Hz, 1H), 3.82 - 3.72 (m, 1H), 2.97 - 2.65 (m, 4H), 2.53 (s, 3H), 2.13 - 2.07 (m, 2H), 1.63 (s, 6H).

### Example 482: Synthesis of 5-(((1s,3s)-3-(4-(2-(4-((2-(2H-1,2,3-triazol-2-yl) pyrimidin-5-yl)oxy)phenyl)propan-2-yl)phenoxy)cyclobutyl)amino)-2-(2,6-dioxopiperidin-3-yl)isoindolin-1,3-dione

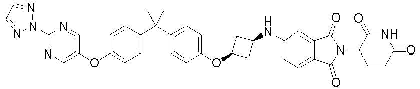

The target compound was synthesized by a method similar to that of Example 228. LC/MS (ESI+) Calcd for C₃₈H₃₄N₈O₆(M+H⁺) m/z 698.3; found 698.3. ¹H NMR (400 MHz, CDCl₃) *δ* 8.00 (s, 1H), 7.87 (s, 2H), 7.82 (t, *J* = 7.9 Hz, 1H), 7.64 (d, *J* = 8.2 Hz, 1H), 7.25 (s, 2H), 7.19 - 7.14 (m, 2H), 7.14 - 7.08 (m, 2H), 6.94 (d, *J* = 2.1 Hz, 1H), 6.80 - 6.73 (m, 2H), 6.71 (d, *J* = 8.7 Hz, 2H), 4.93 (dd, *J* = 12.2, 5.3 Hz, 1H), 4.90 - 4.83 (m, 1H), 4.28 - 4.20 (m, 1H), 2.95 - 2.75 (m, 3H), 2.74 - 2.68 (m, 2H), 2.43 (dt, *J* = 12.9, 6.4 Hz, 2H), 2.15 - 2.10 (m, 1H), 1.68 (s, 6H).

### Example 483: Synthesis of 2-(2,6-dioxopiperidin-3-yl)-5-(((1r,3r)-3-(4-(1-(4-((2-(5-methyl-1,3,4-oxadiazol-)2-yl)pyrimidin-5-yl)oxy)phenyl)cyclopentyl)phenoxy) cyclobutyl)amino)isoindolin-1,3-dione

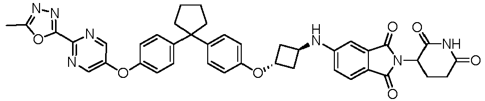

The target compound was synthesized by a method similar to that of Example 436. LC/MS (ESI+) Calcd for C₄₁H₃₇N₇O₇(M+H⁺) *m*/*z* 739.3; found 739.3. ¹H NMR (400 MHz, Chloroform-*d*) *δ* 8.61 (s, 2H), 8.11 (s, 1H), 7.61 (d, *J* = 8.3 Hz, 1H), 7.34 (d, *J* = 8.7 Hz, 2H), 7.14 (d, *J* = 8.8 Hz, 2H), 7.03 (d, *J* = 8.7 Hz, 2H), 6.93 (d, *J* = 2.1 Hz, 1H), 6.74 (dd, *J* = 8.9, 7.0 Hz, 3H), 4.91 (dd, *J* = 12.2, 5.3 Hz, 1H), 4.53 (t, *J* = 6.8 Hz, 1H), 3.85 - 3.73 (m, 1H), 3.14 (ddt, *J* = 13.6, 9.9, 5.0 Hz, 2H), 2.93 - 2.72 (m, 3H), 2.54 (s, 3H), 2.21 - 1.96 (m, 4H), 1.73 - 1.63 (m, 4H).

### Example 484: Synthesis of 2-(2,6-dioxopiperidin-3-yl)-5-(((1s,3s)-3-(4-(2-(4-((2-(5-methyl-1,3,4-oxadiazole)-2-yl)pyrimidin-5-yl)oxy)phenyl)propan-2-yl)phenoxy) cyclobutyl)amino)isoindolin-1,3-dione

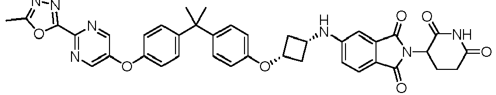

The target compound was synthesized by a method similar to that of Example 436. LC/MS (ESI+) Calcd for C₃₉H₃₅N₇O₇(M+H⁺) *m*/*z* 713.3; found 713.3. ¹H NMR (400 MHz, Chloroform-*d*) *δ* 8.61 (s, 2H), 8.11 (s, 1H), 7.61 (d, *J* = 8.3 Hz, 1H), 7.34 (d, *J* = 8.7 Hz, 2H), 7.14 (d, *J* = 8.8 Hz, 2H), 7.03 (d, *J* = 8.7 Hz, 2H), 6.93 (d, *J* = 2.1 Hz, 1H), 6.74 (dd, *J* = 8.9, 7.0 Hz, 3H), 4.91 (dd, *J* = 12.2, 5.3 Hz, 1H), 4.53 (t, *J* = 6.8 Hz, 1H), 3.85 - 3.73 (m, 1H), 3.14 (ddt, *J* = 13.6, 9.9, 5.0 Hz, 2H), 2.93 - 2.72 (m, 3H), 2.54 (s, 3H), 2.16 - 2.07 (m, 3H), 1.69 (s, 6H).

### Example 485: Synthesis of 5-(3-((4-(2-(4-((2-acetylpyrimidin-4-yl)methoxy)phenyl) propan-2-yl)phenoxy)methyl)azetidin-1-yl)-2-(2,6-dioxopiperidin-3-yl) isoindolin-1,3-dione

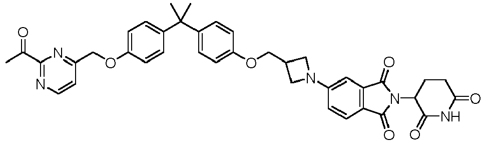

The target compound was synthesized by a method similar to that of Example 107. ¹H NMR (400 MHz, Chloroform-d) *δ* 9.14 - 8.74 (m, 1H), 8.34 (d, J = 35.8 Hz, 1H), 7.86 - 7.52 (m, 2H), 7.16 (s, 3H), 6.98 - 6.67 (m, 5H), 6.53 (d, J = 8.1 Hz, 1H), 5.25 (s, 2H), 4.94 (t, J = 8.3 Hz, 1H), 4.33 - 3.70 (m, 6H), 3.52 (s, 1H), 3.25 (s, 1H), 2.83 (d, J = 22.2 Hz, 5H), 2.12 (d, J = 11.9 Hz, 1H), 1.64 (s, 6H). LC/MS (ESI+) calcd for C₃₉H₃₇N₅O₇ ([M+H]⁺) *m*/*z* 688, found 688.

### Example 486: 5-(3-(4-(2-(2-acetylpyrimidin-4-yl)methoxy)phenyl)propan-2-yl)phenoxy)pyrrolidin-1-yl)-2-(2,6-dioxopiperidin-3-yl)isoindolin-1,3-dione

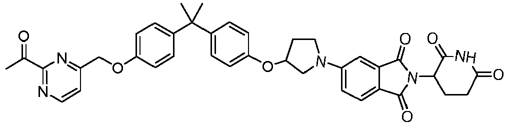

The target compound was synthesized by a method similar to that of Example 485. ¹H NMR (400 MHz, DMSO-*d*₆) *δ* 11.07 (s, 1H), 9.02 (d, *J* = 5.1 Hz, 1H), 7.77 (d, *J* = 5.1 Hz, 1H), 7.65 (d, *J* = 8.4 Hz, 1H), 7.21 - 7.09 (m, 4H), 7.01 - 6.93 (m, 3H), 6.92 - 6.82 (m, 3H), 5.27 (s, 2H), 5.16 (s, 1H), 5.06 (dd, *J* = 12.9, 5.4 Hz, 1H), 3.80 (dd, *J* = 12.0, 4.6 Hz, 1H), 3.55 (dd, *J* = 16.2, 8.8 Hz, 3H), 2.88 (ddd, *J* = 17.6, 14.1, 5.5 Hz, 1H), 2.67 (s, 3H), 2.64 - 2.52 (m, 2H), 2.32 (dt, *J* = 9.4, 4.6 Hz, 1H), 2.24 (s, 1H), 2.05 - 1.97 (m, 1H), 1.59 (s, 6H).LC/MS (ESI+) calcd for C₃₉H₃₇N₅O₇ ( [M+H]⁺ ) *m*/*z:* 688.3; found 688.3.

### Example 487: 5-(4-(4-(2-(2-acetylpyrimidin-4-yl)methoxy)phenyl)propan-2-yl) phenyl)piperazin-1-yl)-2-(2,6-dioxopiperidin-3-yl)isoindolin-1,3-dione

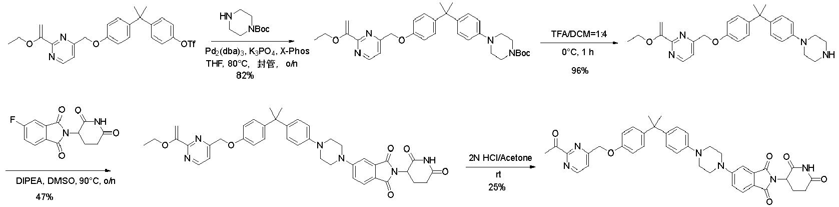

### Step 1: Synthesis of tert-butyl 4-(4-(2-(4-((2-(1-ethoxyvinyl)pyrimidin-4-yl)methoxy)phenyl)propan-2-yl)phenyl)piperazin-1-carboxylate

4-(2-(4-((2-(1-ethoxyvinyl)pyrimidin-4-yl)methoxy)phenyl)propan-2-yl)phenyl trifluoromethanesulfonate (80 mg, 0.15 mmol), 1-Boc-piperazine (113 mg, 0.61 mmol), Pd₂(dba)₃ (14 mg, 0.015 mmol), X-Phos (36 mg, 0.076 mmol), and K₃PO₄ (80 mg, 0.38 mmol) were placed in a sealed tube, to which was added 5 mL of THF, and then the mixture was allowed to react overnight at 80 °C under Ar gas protection. After completion of the reaction, the reaction was quenched by adding water. The resultant solution was extracted with ethyl acetate for three times. The combined organic layer was washed with saturated brine, dried over anhydrous Na₂SO₄, and concentrated. The residue was separated and purified by prep-TLC, to provide compound tert-butyl 4-(4-(2-(4-((2-(1-ethoxyvinyl)pyrimidin-4-yl)methoxy)phenyl)propan-2-yl)phenyl) piperazin-1-carboxylate (70 mg), with a yield of 82%. LC/MS (ESI+) calcd for C₃₃H₄₂N₄O₄ ( [M+H]⁺ ) *m*/*z* 559.3; found 559.3.

### Step 2: Synthesis of 2-(1-ethoxyvinyl)-4-((4-(2-(4-(piperazin-1-yl)phenyl)propan-2-yl)phenoxy)methyl)pyrimidine

tert-butyl 4-(4-(2-(4-((2-(1-ethoxyvinyl)pyrimidin-4-yl)methoxy)phenyl)propan-2-yl)phenyl)piperazin-1-carboxylate (70 mg, 0.13 mmol) was dissolved in 4 mL of dichloromethane, to which was added 1 mL of trifluoroacetic acid dropwise in an ice bath. Subsequently, the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 2-(1-ethoxyvinyl)-4-((4-(2-(4-(piperazin-1-yl)phenyl)propan-2-yl)phenoxy)methyl) pyrimidine (55 mg), with a yield of 96%. LC/MS (ESI+) calcd for C₂₈H₃₄N₄O₂ ( [M+H]⁺ ) *m*/*z* 458.3; found 459.3.

### Step 3: Synthesis of 2-(2,6-dioxopiperidin-3-yl)-5-(4-(2-(4-(2-(1-ethoxyvinyl) pyrimidin-4-yl)methoxy)phenyl)propan-2-yl)phenyl)piperazin-1-yl)isoindolin-1,3-dione

2-(1-ethoxyvinyl)-4-((4-(2-(4-(piperazin-1-yl)phenyl)propan-2-yl)phenoxy) methyl)pyrimidine (55 mg, 0.12 mmol) was dissolved in 5 mL of DMSO, to which were added three drops of DIPEA and 2-(2,6-dioxopiperidin-3-yl)-5-fluoroisoindolin-1,3-dione (40 mg, 0.14 mmol), and then the mixture was allowed to react overnight at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-(4-(2-(4-(2-(1-ethoxyvinyl)pyrimidin-4-yl)methoxy)phenyl)propan-2-yl)phenyl) piperazin-1-yl)isoindol-1,3-dione (40 mg), with a yield of 47%. LC/MS (ESI+) calcd for C₄₁H₄₂N₆O₆ ( [M+H]⁺ ) *m*/*z* 715.3; found 715.3.

### Step 4: Synthesis of 5-(4-(4-(2-(2-acetylpyrimidin-4-yl)methoxy)phenyl)propan-2-yl)phenyl)piperazin-1-yl)-2-(2,6-dioxopiperidin-3-yl)isoindolin-1,3-dione

2-(2,6-dioxopiperidin-3-yl)-5-(4-(2-(4-(2-(1-ethoxyvinyl)pyrimidin-4-yl) methoxy)phenyl)propan-2-yl)phenyl)piperazin-1-yl)isoindolin-1,3-dione (40 mg, 0.046 mmol) was dissolved in 2 mL of acetone, to which was added 2 mL of 2N HCl aqueous solution, and then the mixture was allowed to react at room temperature for 2 h. After completion of the reaction detected by TLC, 10 mL of water was added, and then the resultant solution was extracted with ethyl acetate for three times. The combined organic layer was washed with saturated brine, dried over anhydrous Na₂SO₄, and concentrated to dry under reduced pressure. The residue was separated and purified by prep-TLC, to provide the product 5-(4-(4-(2-(2-acetylpyrimidin-4-yl)methoxy)phenyl)propan-2-yl)phenyl)piperazin-1-yl)-2-(2,6-dioxopiperidin-3-yl)isoindolin-1,3-dione (20 mg, yellow solid), with a yield of 25%. ¹H NMR (400 MHz, Chloroform-*d*) *δ* 8.92 (d, *J* = 5.1 Hz, 1H), 8.09 (s, 1H), 7.78 - 7.68 (m, 2H), 7.34 (d, *J* = 2.3 Hz, 1H), 7.22 - 7.14 (m, 4H), 7.11 (dd, *J* = 8.6, 2.4 Hz, 1H), 6.92 (s, 1H), 6.89 - 6.85 (m, 2H), 5.25 (s, 2H), 4.95 (dd, *J* = 12.3, 5.3 Hz, 1H), 3.61 (s, 4H), 3.35 (d, *J* = 5.5 Hz, 4H), 2.94 - 2.69 (m, 6H), 2.14 (ddd, *J* = 10.5, 4.8, 2.6 Hz, 1H), 1.65 (s, 6H).
LC/MS (ESI+) calcd for C₃₉H₃₈N₆O₆ ([M+H]⁺) *m*/*z:* 687.3; found 687.3.

### Example 488: 5-(6-(4-(2-(2-acetylpyrimidin-4-yl)methoxy)phenyl)propan-2-yl)phenyl)-2,6-diazaspiro[3.3]heptan-2-yl)-2-(2,6-dioxopiperidin-3-yl)isoindolin-1,3-dione

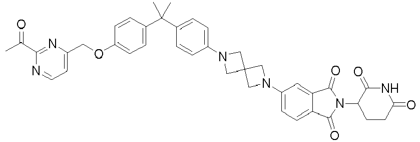

The target compound was synthesized by a method similar to that of Example 487. ¹H NMR (400 MHz, Chloroform-*d*) *δ* 8.92 (dd, *J* = 5.1, 1.7 Hz, 1H), 8.43 (s, 1H), 7.74 (d, *J* = 5.1 Hz, 1H), 7.66 (dd, *J* = 8.3, 2.1 Hz, 1H), 7.22 - 7.14 (m, 2H), 7.08 (dd, *J* = 18.1, 8.6 Hz, 2H), 6.93 - 6.77 (m, 3H), 6.67 - 6.38 (m, 3H), 5.25 (s, 2H), 4.93 (dd, *J* = 12.2, 5.3 Hz, 1H), 4.19 (s, 3H), 4.07 (s, 3H), 3.96 - 3.36 (m, 2H), 2.98 - 2.61 (m, 6H), 2.12 (ddd, *J* = 10.4, 4.9, 2.7 Hz, 1H), 1.63 (d, *J* = 2.3 Hz, 6H). LC/MS (ESI+) calcd for C₄₀H₃₈N₆O₆ ([M+H]⁺) *m*/*z:* 699.3; found 699.3.

### Example 489: 5-(2-(4-(2-(2-acetylpyrimidin-4-yl)methoxy)phenyl)propan-2-yl)phenoxy)-7-azaspiro[3.5]nonan-7-yl)-2-(2,6-dioxopiperidin-3-yl)isoindolin-1,3-dione

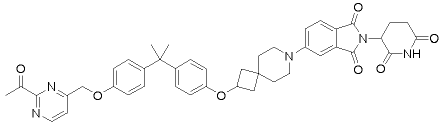

The target compound was synthesized by a method similar to that of Example 485. ¹H NMR (400 MHz, Chloroform-*d*) *δ* 8.92 (d, *J* = 5.1 Hz, 1H), 8.18 (s, 1H), 7.74 (d, *J* = 5.1 Hz, 1H), 7.67 (d, *J* = 8.5 Hz, 1H), 7.28 (d, *J* = 2.4 Hz, 1H), 7.17 (dq, *J* = 8.9, 2.5, 1.6 Hz, 2H), 7.14 - 7.10 (m, 2H), 7.04 (dd, *J* = 8.6, 2.4 Hz, 1H), 6.91 - 6.81 (m, 2H), 6.76 - 6.60 (m, 2H), 5.25 (s, 2H), 4.99 - 4.90 (m, 1H), 4.68 (p, *J* = 6.5 Hz, 1H), 3.45 - 3.34 (m, 4H), 2.91 - 2.70 (m, 6H), 2.47 - 2.40 (m, 2H), 2.12 (ddd, *J* = 10.3, 4.7, 2.3 Hz, 1H), 2.04 - 1.98 (m, 2H), 1.76 (q, *J* = 5.7 Hz, 4H), 1.65 - 1.61 (m, 6H). LC/MS (ESI+) calcd for C₄₃H₄₃N₅O₇ ([M+H]⁺) *m*/*z:* 742.3; found 742.3.

### Example 490: 5-(6-(4-(2-(2-(2-acetylpyrimidin-4-yl)methoxy)phenyl)propan-2-yl)phenoxy)-2-azaspiro[3.4]octane-2-yl)-2-(2,6-dioxopiperidin-3-yl)isoindolin-1,3-dione

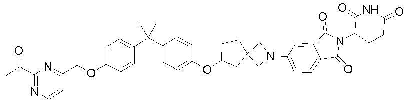

The target compound was synthesized by a method similar to that of Example 485. ¹H NMR (400 MHz, Chloroform-*d*) *δ* 8.92 (d, *J* = 5.0 Hz, 1H), 8.36 - 8.14 (m, 1H), 7.74 (d, *J* = 4.9 Hz, 1H), 7.63 (d, *J* = 8.2 Hz, 1H), 7.21 - 7.15 (m, 3H), 7.13 (d, *J* = 8.5 Hz, 2H), 6.90 - 6.82 (m, 3H), 6.76 (d, *J* = 3.0 Hz, 2H), 5.25 (s, 2H), 4.93 (dd, *J* = 12.0, 5.0 Hz, 1H), 4.81 (s, 1H), 4.13 - 3.88 (m, 4H), 2.88 - 2.73 (m, 6H), 2.13 (td, *J* = 12.2, 10.4, 5.1 Hz, 3H), 2.01 (d, *J* = 6.4 Hz, 2H), 1.64 (s, 6H), 1.33 - 1.31 (m, 2H). LC/MS (ESI+) calcd for C₄₂H₄₁N₅O₇ ([M+H]⁺) *m*/*z:* 728.3; found 728.3.

### Example 491: 5-((1s,3s)-3-(4-(2-(4-((2-acetylpyrimidin-4-yl)methoxy)phenyl) propan-2-yl)phenoxy)cyclobutyl)amino)-2-(2,6-dioxopyridin-3-yl)isoindolin-1,3-dione

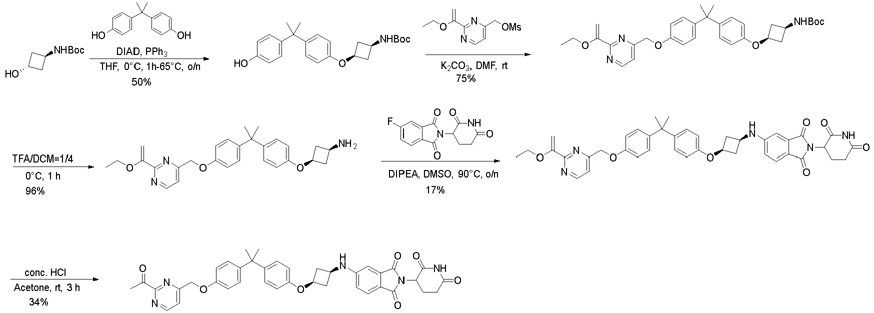

### Step 1: Synthesis of tert-butyl ((1s,3s)-3-(4-(2-(4-hydroxylphenyl)propan-2-phenoxy)cyclobutyl)carbamate

*N*-Boc-trans-3-aminocyclobutanol (1.87 g, 10 mmol), bisphenol A (2.74 g, 12 mmol), and PPh₃ (2.75 g, 10.5 mmol) were added into a three-necked flask under Ar gas protection, to which was added 30 mL of dry THF, and then the solution was stirred well, followed by addition of DIAD (2.12 g, 10.5 mmol) dropwise in an ice bath. Subsequently, the reaction solution was still reacted in the ice bath for 1 h, and then reacted overnight at 65 °C. The reaction solution was cooled to room temperature, to which was then added anhydrous ZiCl₂ (1.43 g, 10.5 mmol), followed by stirring for 1h. The reaction solution was directly filtered, and the filtrate was washed with 2 N NaOH solution. The resultant solution was extracted with ethyl acetate.The organic phase was washed with saturated brine, dried over anhydrous Na₂SO₄, and concentrated to dry under reduced pressure. The residue was separated and purified by column chromatography (PE:EA:Et₃N = 5:1:0.5), to provide compound tert-butyl ((1s,3s)-3-(4-(2-(4-hydroxylphenyl)propan-2-phenoxy)cyclobutyl)carbamate (2.00 g), with a yield of 50%. LC/MS (ESI+) calcd for C₂₄H₃₁NO₄( [M]⁺ ) *m*/*z:* 397.2; found 298.3.

### Step 2: Synthesis of tert-butyl ((1s,3s)-3-(4-(2-(4-(2-(1-ethoxyvinyl)pyrimidin-4-yl)methoxy)phenyl)propan-2-yl)phenoxy)cyclobutyl)carbamate

tert-butyl ((1s,3s)-3-(4-(2-(4-hydroxylphenyl)propan-2-phenoxy)cyclobutyl) carbamate (273 mg, 0.69 mmol) and (2-(1-ethoxyvinyl)pyrimidin-4-yl) methanesulfonate (180 mg, 0.69 mmol) were dissolved in 5 mL of DMF, to which was added K₂CO₃ (192 mg, 1.39 mmol), and then the mixture was allowed to react overnight at room temperature. The reaction was quenched by adding water, and the resultant solution was extracted with ethyl acetate. The water layer was re-extracted once. The combined organic layer was washed with saturated brine, dried over anhydrous Na₂SO₄, and concentrated. The residue was separated and purified by prep-TLC, to provide compound tert-butyl ((1*s*,3*s*)-3-(4-(2-(4-(2-(1-ethoxyvinyl)pyrimidin-4-yl)methoxy) phenyl)propan-2-yl)phenoxy)cyclobutyl)carbamate (290 mg), with a yield of 75%.

### Step 3: Synthesis of (1s,3s)-3-(4-(2-(4-(2-(1-ethoxyvinyl)pyrimidin-4-yl)methoxy) phenyl)propan-2-yl)phenoxy)cyclobutyl-1-amine

tert-butyl ((1*s*,3*s*)-3-(4-(2-(4-(2-(1-ethoxyvinyl)pyrimidin-4-yl)methoxy)phenyl) propan-2-yl)phenoxy)cyclobutyl)carbamate (290 mg, 0.52 mmol) was dissolved in 8 mL of dichloromethane, to which was added 2 mL of trifluoroacetic acid dropwise in an ice bath. Subsequently, the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1s,3s)-3-(4-(2-(4-(2-(1-ethoxyvinyl)pyrimidin-4-yl)methoxy)phenyl) propan-2-yl)phenoxy)cyclobutyl-1-amine (230 mg), with a yield of 96%.

### Step 4: 2-(2,6-dioxopiperidin-3-yl)-5-((1s,3s)-3-(4-(2-(2-(1-ethoxyvinyl)pyrimidin-4-yl)methoxy)phenyl)propan-2-yl)phenoxy)cyclobutyl)amino)isoindol-1,3-dione

(1*s*,3*s*)-3-(4-(2-(4-(2-(1-ethoxyvinyl)pyrimidin-4-yl)methoxy)phenyl)propan-2-yl)phenoxy)cyclobutyl-1-amine (230 mg, 0.50 mmol) was dissolved in 5 mL of DMSO, to which were added DIPEA(194 mg, 1.50 mmol) and 2-(2,6-dioxopiperidin-3-yl)-5-fluoroisoindolin-1,3-dione (166 mg, 0.60 mmol), and then the mixture was allowed to react overnight at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-((1s,3s)-3-(4-(2-(2-(1-ethoxyvinyl)pyrimidin-4-yl)methoxy) phenyl)propan-2-yl)phenoxy)cyclobutyl)amino)isoindol-1,3-dione (60 mg), with a yield of 17%.

### Step 5: Synthesis of 5-((1s,3s)-3-(4-(2-(4-((2-acetylpyrimidin-4-yl)methoxy)phenyl) propan-2-yl)phenoxy)cyclobutyl)amino)-2-(2,6-dioxopyridin-3-yl)isoindolin-1,3-dione

2-(2,6-dioxopiperidin-3-yl)-5-((1s,3s)-3-(4-(2-(2-(1-ethoxyvinyl)pyrimidin-4-yl)methoxy)phenyl)propan-2-yl)phenoxy)cyclobutyl)amino)isoindol-1,3-dione (60 mg, 0.084 mmol) was dissolved in 3 mL of acetone, to which was added 2 mL of conc. HCl solution, and then the mixture was allowed to react at room temperature for 3h. After completion of the reaction detected by TLC, 10 mL of water was added, and then the resultant solution was extracted with ethyl acetate for three times. The combined organic layer was washed with saturated brine, dried over anhydrous Na₂SO₄, and concentrated to dry under reduced pressure. The residue was separated and purified by prep-TLC, to provide the product 5-((1*s*,3*s*)-3-(4-(2-(4-((2-acetylpyrimidin-4-yl)methoxy)phenyl)propan-2-yl)phenoxy)cyclobutyl)amino)-2-(2,6-dioxopyridin-3-yl)isoindol-1,3-dione (20 mg, yellow solid), with a yield of 34%.
¹H NMR (400 MHz, Chloroform-*d*) *δ* 8.92 (d, *J* = 5.1 Hz, 1H), 8.11 (s, 1H), 7.73 (d, *J* = 5.1 Hz, 1H), 7.62 (d, *J* = 8.2 Hz, 1H), 7.15 (dd, *J* = 16.3, 8.3 Hz, 4H), 6.91 (d, *J* = 2.1 Hz, 1H), 6.87 (d, *J* = 8.4 Hz, 2H), 6.72 (d, *J* = 8.1 Hz, 3H), 5.25 (s, 2H), 4.93 (dd, *J* = 12.0, 5.3 Hz, 1H), 4.51 (p, *J* = 6.8 Hz, 1H), 3.77 (d, *J* = 7.1 Hz, 1H), 3.19 - 3.06 (m, 2H), 2.92 - 2.70 (m, 6H), 2.09 (ddt, *J* = 14.8, 10.4, 4.4 Hz, 3H), 1.64 (s, 6H).
LC/MS (ESI+) calcd for C₃₉H₃₇N₅O₇ ([M+H]⁺) *m*/*z:* 688.3; found 688.3.

### Example 492: 2-(2,6-dioxopiperidin-3-yl)-5-((1s,3s)-3-(4-(2-(2-(1-hydroxylethyl) pyrimidin-4-yl)methoxy)phenyl)propan-2-yl)phenoxy)cyclobutyl)amino) isoindolin-1,3-dione

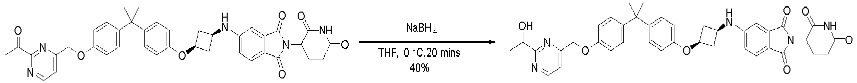

5-((1*s*,3*s*)-3-(4-(2-(4-((2-acetylpyrimidin-4-yl)methoxy)phenyl)propan-2-yl)phenoxy)cyclobutyl)amino)-2-(2,6-dioxopyridin-3-yl)isoindol-1,3-dione (15 mg, 0.022 mmol) was dissolved in 5 mL of acetone, to which was added NaBH₄ (3 mg, 0.08 mmol) in an ice bath, and then the mixture was still reacted at the ice bath for 20 min. After completion of the reaction detected by TLC, 10 mL of water was added, and then the resultant solution was extracted with ethyl acetate for three times. The combined organic layer was washed with saturated brine, dried over anhydrous Na₂SO₄, and concentrated to dry under reduced pressure. The residue was separated and purified by prep-TLC, to provide the product 2-(2,6-dioxopiperidin-3-yl)-5-((1s,3s)-3-(4-(2-(2-(1-hydroxylethyl)pyrimidin-4-yl)methoxy)phenyl)propan-2-yl)phenoxy)cyclobutyl) amino)isoindol-1,3-dione (6 mg, yellow solid), with a yield of 40%. ¹H NMR (400 MHz, Chloroform-*d*) *δ* 8.73 (d*, J* = 5.1 Hz, 1H), 8.41 (s, 1H), 7.60 (d*, J* = 8.3 Hz, 1H), 7.47 (d, *J* = 5.1 Hz, 1H), 7.18 -7.10 (m, 4H), 6.90 (d, *J* = 2.2 Hz, 1H), 6.88 - 6.83 (m, 2H), 6.75 - 6.67 (m, 3H), 5.14 (s, 2H), 5.00 - 4.91 (m, 2H), 4.86 (s, 1H), 4.50 (p, *J* = 6.9 Hz, 1H), 3.74 (d, *J* = 8.2 Hz, 1H), 3.11 (ddt, *J* = 12.1, 8.5, 4.3 Hz, 2H), 2.90 - 2.71 (m, 3H), 2.11 (tdd, *J* = 11.9, 6.7, 3.4 Hz, 3H), 1.63 (s, 6H), 1.58 (d, *J* = 6.6 Hz, 3H). LC/MS (ESI+) calcd for C₃₉H₃₉N₅O₇ ([M+H]⁺) *m*/*z:* 690.3; found 690.3.

### Example 493: 5-((3-(4-(2-(4-((2-(2-oxa-6-azaspiro[3.3]heptan-6-yl)pyrimidin-4-yl)methoxy)phenyl)propan-2-yl)phenoxy)cyclohexyl)amino)-2-(2,6-dioxopiperidin-3-yl)isoindolin-1,3-dione

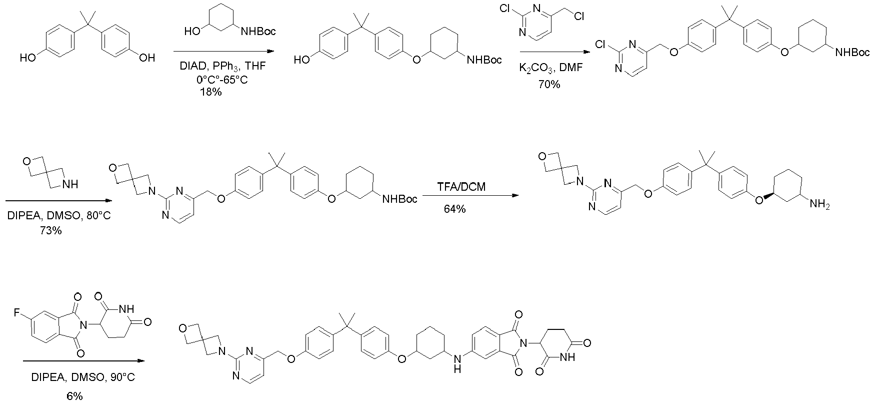

### Step 1: Synthesis of tert-butyl (3-(4-(2-(4-hydroxylphenyl)propan-2-phenoxy) cyclohexyl)carbamate

tert-butyl 3-hydroxylcyclohexylcarbamate (500 mg, 2.32 mmol), bisphenol A (530 mg, 2.32 mmol) and PPh₃(609 mg, 2.32 mmol) were added into a three-necked flask under Ar gas protection, to which was added 10 mL of dry THF, and then the solution was stirred well, followed by addition of DIAD (469 mg, 2.32 mmol) dropwise in an ice bath. Subsequently, the reaction solution was still reacted in the ice bath for 1 h, and then reacted overnight at 65 °C. The reaction solution was cooled to room temperature, to which was then added anhydrous ZiCl₂ (315 mg, 2.32 mmol), followed by stirring for 1h. The reaction solution was directly filtered, and the filtrate was washed with 2 N NaOH solution. The resultant solution was extracted with ethyl acetate.The organic phase was washed with saturated brine, dried over anhydrous Na₂SO₄, and concentrated to dry under reduced pressure. The residue was separated and purified by prep-TLC, to provide compound tert-butyl (3-(4-(2-(4-hydroxylphenyl)propan-2-phenoxy) cyclohexyl)carbamate (180 mg), with a yield of 18%. LC/MS (ESI+) calcd for C₂₆H₃₅NO₄ ( [M]⁺ ) *m*/*z:* 425.2; found 326.2. Step 2: Synthesis of tert-butyl (3-(4-(2-(4-((2-chloropyrimidin-4-yl)methoxy)phenyl) propan-2-yl)phenoxy)cyclohexyl)carbamate

tert-butyl (3-(4-(2-(4-hydroxylphenyl)propan-2-phenoxy)cyclohexyl)carbamate (180 mg, 0.42 mmol) and 2-chloro-4-(chloromethyl)pyrimidine (90 mg, 0.55 mmol) were dissolved in 10 mL of DMF, to which was added K₂CO₃ (116 mg, 0.84 mmol), and then the mixture was allowed to react overnight at room temperature. The reaction was quenched by adding water, and the resultant solution was extracted with ethyl acetate. The combined organic layer was washed with saturated brine, dried over anhydrous Na₂SO₄, and concentrated. The residue was separated and purified by prep-TLC, to provide compound tert-butyl (3-(4-(2-(4-((2-chloropyrimidin-4-yl)methoxy)phenyl)propan-2-yl)phenoxy)cyclohexyl)carbamate (160 mg), with a yield of 70%. LC/MS (ESI+) calcd for C₃₁H₃₈ClN₃O₄ ( [M+H]⁺ ) *m*/*z* 552.2; found 452.2.

### Step 3: Synthesis of tert-butyl (3-(4-(2-(4-((2-(2-oxa-6-azaspiro[3.3]heptan-6-yl)pyrimidin-4-yl)methoxy)phenyl)propan-2-yl)phenoxy)cyclohexyl)carbamate

tert-butyl (3-(4-(2-(4-((2-chloropyrimidin-4-yl)methoxy)phenyl)propan-2-yl) phenoxy)cyclohexyl)carbamate (160 mg, 0.29 mmol) and 2-oxa-6-azaspiro[3.3]heptane hemioxalate (54 mg, 0.38 mmol) were dissolved in 5 mL of DMSO, to which was added DIPEA (75 mg, 0.58 mmol), and then the mixture was allowed to react overnight at 80 °C. Water was added to quench the reaction. The resultant solution was extracted with ethyl acetate for three times. The organic phase was washed with saturated NaCl solution, dried over anhydrous Na₂SO₄, and concentrated. The residue was separated and purified by prep-TLC, to provide compound tert-butyl (3-(4-(2-(4-((2-(2-oxa-6-azaspiro[3.3]heptan-6-yl)pyrimidin-4-yl)methoxy)phenyl)propan-2-yl)phenoxy)cyclohexyl)carbamate (130 mg), with a yield of 73%. LC/MS (ESI+) calcd for C₃₆H₄₆N₄O₅ ( [M+H]⁺ ) *m*/*z* 615.4; found 615.3.

### Step 4: Synthesis of 3-(4-(2-(2-oxa-6-azaspiro[3.3]heptan-6-yl)pyrimidin-4-yl) methoxy)phenyl)propan-2-yl)phenoxy)cyclohexane-1-amine

tert-butyl (3 -(4-(2-(4-((2-(2-oxa-6-azaspiro[3.3]heptan-6-yl)pyrimidin-4-yl) methoxy)phenyl)propan-2-yl)phenoxy)cyclohexyl)carbamate (130 mg, 0.21 mmol) was dissolved in 8 mL of dichloromethane, to which was added 2 mL of trifluoroacetic acid in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound compound 3-(4-(2-(2-oxa-6-azaspiro[3.3]heptan-6-yl)pyrimidin-4-yl) methoxy)phenyl)propan-2-yl)phenoxy)cyclohexane-1-amine (70 mg), with a yield of 64%.

### Step 5: Synthesis of 5-((3-(4-(2-(4-((2-(2-oxa-6-azaspiro[3.3]heptan-6-yl)pyrimidin-4-yl)methoxy)phenyl)propan-2-yl)phenoxy)cyclohexyl)amino)-2-(2,6-dioxopiperidin-3-yl)isoindolin-1,3-dione

3-(4-(2-(2-oxa-6-azaspiro[3.3]heptan-6-yl)pyrimidin-4-yl)methoxy)phenyl) propan-2-yl) phenoxy)cyclohexane-1-amine (70 mg, 0.14 mmol) was dissolved in 5 mL of DMSO, to which were added DIPEA (35 mg, 0.27 mmol) and 2-(2,6-dioxopiperidin-3-yl)-5-fluoroisoindolin-1,3-dione (45 mg, 0.16 mmol), and then the mixture was allowed to react overnight at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 5-((3-(4-(2-(4-((2-(2-oxa-6-azaspiro[3.3]heptan-6-yl)pyrimidin-4-yl)methoxy)phenyl)propan-2-yl)phenoxy)cyclohexyl)amino)-2-(2,6-dioxopiperidin-3-yl)isoindolin-1,3-dione (6 mg), with a yield of 6%. ¹H NMR (400 MHz, Chloroform-*d*) *δ* 8.30 (s, 1H), 7.56 (dd, *J* = 8.2, 4.0 Hz, 1H), 7.14 (dd, *J* = 8.6, 3.2 Hz, 4H), 6.96 (d, *J* = 3.0 Hz, 1H), 6.90 - 6.78 (m, 5H), 6.72 - 6.60 (m, 1H), 4.95 (d, *J* = 2.2 Hz, 2H), 4.94 - 4.89 (m, 1H), 4.86 (s, 4H), 4.69 (s, 1H), 4.32 (s, 4H), 3.89 (s, 1H), 2.79 (ddd, *J* = 39.8, 30.1, 14.9 Hz, 3H), 2.38 (d, *J* = 13.2 Hz, 1H), 2.12 (s, 2H), 2.02 (d, *J* = 14.2 Hz, 1H), 1.88 (t, *J* = 12.9 Hz, 2H), 1.64 (s, 6H), 1.52 (dd, *J* = 26.5, 13.6 Hz, 3H). LC/MS (ESI+) calcd for C₄₄H₄₆N₆O₇ ([M+H]⁺) *m*/*z* 771.3; found 771.2.

### Example 494: 5-((3-((4-(2-(4-((2-(2-oxa-6-azaspiro[3.3]heptan-6-yl)pyrimidin-4-yl)methoxy)phenyl)propan-2-yl)phenyl)amino)cyclobutyl)amino)-2-(2,6-dioxopiperidin-3-yl)isoindolin-1,3-dione

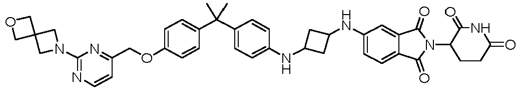

The target compound was synthesized by a method similar to that of Example 487. ¹H NMR (400 MHz, Chloroform-*d*) *δ* 8.30 (d, *J* = 5.1 Hz, 1H), 8.24 (s, 1H), 7.60 (dd, *J* = 8.3, 5.0 Hz, 1H), 7.20 - 7.11 (m, 2H), 7.05 (dd, *J* = 8.9, 2.7 Hz, 2H), 6.93 - 6.85 (m, 1H), 6.85 - 6.74 (m, 3H), 6.68 (dd, *J* = 8.4, 2.2 Hz, 1H), 6.50 (d, *J* = 8.2 Hz, 1H), 4.95 (d, *J* = 2.9 Hz, 2H), 4.91 (d, *J* = 5.3 Hz, 1H), 4.86 (s, 4H), 4.30 (s, 4H), 4.18 (s, 1H), 4.13 - 4.02 (m, 1H), 2.92 - 2.70 (m, 3H), 2.43 (d, *J* = 6.0 Hz, 2H), 2.36 (t, *J* = 6.3 Hz, 2H), 2.15 - 2.08 (m, 1H), 1.61 (s, 6H). LC/MS (ESI+) calcd for C₄₂H₄₃N₇O₆ ([M+H]⁺) *m*/*z:* 742.3; found 742.3.

### Example 495: 5-((1-((4-(2-(4-((2-(2-oxa-6-azaspiro[3.3]heptan-6-yl)pyrimidin-4-yl)methoxy)phenyl)propan-2-yl)phenoxy)methyl)cyclopropyl)methyl)amino)-2-(2,6-dioxopiperidin-3-yl)isoindolin-1,3-dione

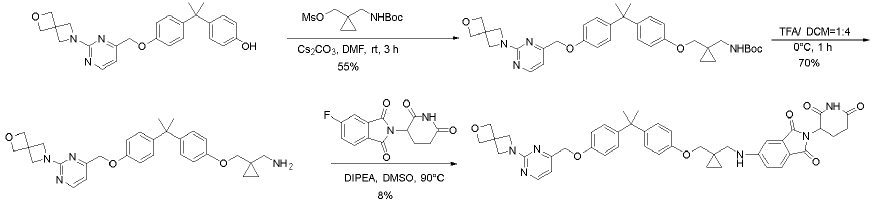

### Step 1: Synthesis of tert-butyl ((1-((4-(2-(4-((2-(2-oxa-6-azaspiro[3.3]heptan-6-yl)pyrimidin-4-yl)methoxy)phenyl)propan-2-yl)phenoxy)methyl)cyclopropyl) carbamate

4-(2-(4-((2-(2-oxa-6-azaspiro[3.3]heptan-6-yl)pyrimidin-4-yl)methoxy)phenyl) propan-2-yl)phenol (150 mg, 0.36 mmol) and (1-((tert-butoxycarbonyl)amino)methyl) cyclopropyl)methyl methanesulfonate (301 mg, 1.08 mmol) were dissolved in 5 mL of DMF, to which was added Cs₂CO₃ (234 mg, 0.72 mmol), and then the mixture was allowed to react at room temperature for 3 h. TLC indicated disappearance of starting materials, and subsequently, saturated NH₄Cl aqueous solution was added to quench the reaction. The resultant solution was extracted with ethyl acetate. The organic phase was combined, washed with saturated brine, dried over anhydrous Na₂SO₄, and concentrated to dry under reduced pressure. The residue was separated and purified by prep-TLC, to provide the product tert-butyl ((1-((4-(2-(4-((2-(2-oxa-6-azaspiro[3.3] heptan-6-yl)pyrimidin-4-yl)methoxy)phenyl)propan-2-yl)phenoxy)methyl) cyclopropyl)carbamate (120 mg), with a yield of 55%. LC/MS (ESI+) calcd for C₃₅H₄₄N₄O₅ ( [M+H]⁺ ) *m*/*z* 601.3; found 601.2.

### Step 2: Synthesis of (1-((4-(2-(4-((2-(2-oxa-6-azaspiro[3.3]heptan-6-yl) pyrimidin-4-yl)methoxy)phenyl)propan-2-yl)phenoxy)methyl)cyclopropyl)methylamine

tert-butyl ((1-((4-(2-(4-((2-(2-oxa-6-azaspiro[3.3]heptan-6-yl)pyrimidin-4-yl) methoxy)phenyl)propan-2-yl)phenoxy)methyl)cyclopropyl)carbamate (120 mg, 0.20 mmol) was dissolved in 4 mL of dichloromethane, to which was added 1 mL of trifluoroacetic acid in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1-((4-(2-(4-((2-(2-oxa-6-azaspiro[3.3]heptan-6-yl)pyrimidin-4-yl)methoxy)phenyl)propan-2-yl)phenoxy)methyl)cyclopropyl) methylamine (70 mg), with a yield of 70%. LC/MS (ESI+) calcd for C₃₀H₃₆N₄O₃ ( [M+H]⁺ ) *m*/*z* 501.3; found 501.2.

### Step 3: Synthesis of 5-((1-((4-(2-(4-((2-(2-oxa-6-azaspiro[3.3]heptan-6-yl)pyrimidin-4-yl)methoxy)phenyl)propan-2-yl)phenoxy)methyl)cyclopropyl)methyl)amino)-2-(2,6-dioxopiperidin-3 -yl)isoindol-1,3 -dione

(1-((4-(2-(4-((2-(2-oxa-6-azaspiro[3.3]heptan-6-yl)pyrimidin-4-yl)methoxy) phenyl)propan-2-yl)phenoxy)methyl)cyclopropyl)methylamine (70 mg, 0.14 mmol) was dissolved in 5 mL of DMSO, to which were added DIPEA (54 mg, 0.42 mmol) and 2-(2,6-dioxopiperidin-3-yl)-5-fluoroisoindolin-1,3-dione (46 mg, 0.17 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 5-((1-((4-(2-(4-((2-(2-oxa-6-azaspiro[3.3]heptan-6-yl)pyrimidin-4-yl)methoxy)phenyl)propan-2-yl)phenoxy)methyl)cyclopropyl)methyl)amino)-2-(2,6-dioxopiperidin-3-yl)isoindol-1,3-dione (8 mg), with a yield of 8%. ¹H NMR (400 MHz, Chloroform-*d*) *δ* 8.30 (d, *J* = 5.1 Hz, 1H), 8.25 (s, 1H), 7.57 (d, *J* = 8.3 Hz, 1H), 7.16 - 7.10 (m, 4H), 6.94 (d, *J* = 2.2 Hz, 1H), 6.85 - 6.79 (m, 4H), 6.78 (d, *J* = 2.1 Hz, 1H), 6.72 (dd, *J* = 8.4, 2.2 Hz, 1H), 4.94 (s, 2H), 4.93 - 4.89 (m, 1H), 4.86 (s, 4H), 4.31 (s, 4H), 3.92 - 3.83 (m, 2H), 3.34 - 3.25 (m, 2H), 2.90 - 2.69 (m, 3H), 2.11 (ddd, *J* = 10.3, 4.9, 2.6 Hz, 1H), 1.62 (s, 6H), 0.77 - 0.68 (m, 4H). LC/MS (ESI+) calcd for C₄₃H₄₄N₆O₇ ([M+H]⁺) *m*/*z* 757.3; found 757.3.

### Example 496: 5-((3-(4-(2-(4-((2-(2-oxa-6-azaspiro[3.3]heptan-6-yl)pyrimidin-4-yl)methoxy)phenyl)propan-2-yl)phenoxy)-2-methylpropyl)amino)-2-(2,6-dioxopiperidin-3-yl)isoindolin-1,3-dione

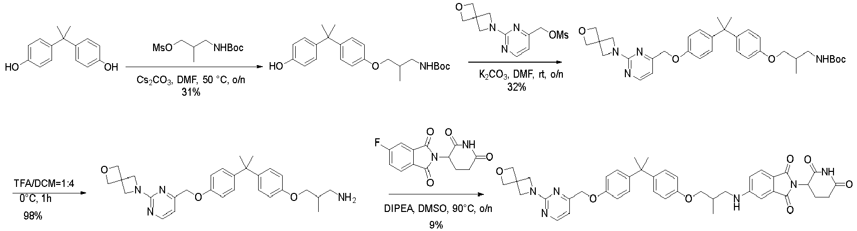

### Step 1: Synthesis of tert-butyl (3-(4-(2-(4-hydroxylphenyl)propan-2-yl)phenoxy)-2-methylpropyl)carbamate

Bisphenol A (456 mg, 2.0 mmol) and 3-((tert-butoxycarbonyl)amino)-2-methylpropyl methanesulfonate (534 mg, 2.0 mmol) was dissolved in 10 mL of DMF, to which was added Cs₂CO₃ (1.30 g, 4.0 mmol), and then the mixture was allowed to react overnight at 50 °C. The reaction was quenched by adding water. The resultant solution was extracted with ethyl acetate. The combined organic layer was washed with saturated brine, dried over anhydrous Na₂SO₄, and concentrated. The residue was separated and purified by prep-TLC, to provide compound tert-butyl (3-(4-(2-(4-hydroxylphenyl)propan-2-yl)phenoxy)-2-methylpropyl)carbamate (250 mg), with a yield of 31%. LC/MS (ESI+) calcd for C₂₄H₃₃NO₄ ( [M+H]⁺ ) m/z 400.2; found 300.3.

### Step 2: Synthesis of tert-butyl (3-(4-(2-(4-((2-(2-oxa-6-azaspiro[3.3]heptan-6-yl)pyrimidin-4-yl)methoxy)phenyl)propan-2-yl)phenoxy)-2-methylpropyl)carbamate

tert-butyl 3-(4-(2-(4-hydroxylphenyl)propan-2-yl)phenoxy)-2-methylpropyl) carbamate (150 mg, 0.38 mmol) and (2-(2-oxa-6-azaspiro[3.3]heptan-6-yl)pyrimidin-4-yl)methyl methanesulfonate (107 mg, 0.38 mmol) were dissolved in 5 mL of DMF, to which was added K₂CO₃ (103 mg, 0.76 mmol), and then the mixture was allowed to react overnight at room temperature. The reaction was quenched by adding water. The resultant solution was extracted with ethyl acetate. The combined organic layer was washed with saturated brine, dried over anhydrous Na₂SO₄, and concentrated. The residue was separated and purified by prep-TLC, to provide compound tert-butyl (3-(4-(2-(4-((2-(2-oxa-6-azaspiro[3.3]heptan-6-yl)pyrimidin-4-yl)methoxy)phenyl)propan-2-yl)phenoxy)-2-methylpropyl)carbamate (70 mg), with a yield of 32%. LC/MS (ESI+) calcd for C₃₄H₄₄N₄O₅ ( [M+H]⁺ ) *m*/*z* 589.3; found 589.3.

### Step 3: Synthesis of 3-(4-(2-(4-((2-(2-oxa-6-azaspiro[3.3]heptan-6-yl)pyrimidin-4-yl)methoxy)phenyl)propan-2-yl)phenoxy)-2-methylpropan-1-amine

tert-butyl (3 -(4-(2-(4-((2-(2-oxa-6-azaspiro[3.3]heptan-6-yl)pyrimidin-4-yl) methoxy)phenyl)propan-2-yl)phenoxy)-2-methylpropyl)carbamate (70 mg, 0.12 mmol) was dissolved in 4 mL of dichloromethane, to which was added 1 mL of trifluoroacetic acid in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 3-(4-(2-(4-((2-(2-oxa-6-azaspiro[3.3]heptan-6-yl)pyrimidin-4-yl)methoxy) phenyl)propan-2-yl)phenoxy)-2-methylpropan-1-amine (57 mg), with a yield of 98%. LC/MS (ESI+) calcd for C₂₉H₃₆N₄O₃ ( [M+H]⁺ ) *m*/*z* 489.3; found 489.3.

### Step 4: Synthesis of 5-((3-(4-(2-(4-((2-(2-oxa-6-azaspiro[3.3]heptan-6-yl)pyrimidin-4-yl)methoxy)phenyl)propan-2-yl)phenoxy)-2-methylpropyl)amino)-2-(2,6-dioxopiperidin-3-yl)isoindolin-1,3-dione

(3-(4-(2-(4-((2-(2-oxa-6-azaspiro[3.3]heptan-6-yl)pyrimidin-4-yl)methoxy) phenyl)propan-2-yl)phenoxy)-2-methylpropan-1-amine (57 mg, 0.11 mmol) was dissolved in 5 mL of DMSO, to which were added DIPEA(43 mg, 0.33 mmol) and 2-(2,6-dioxopiperidin-3-yl)-5-fluoroisoindolin-1,3-dione (36 mg, 0.13 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 5-((3-(4-(2-(4-((2-(2-oxa-6-azaspiro[3.3]heptan-6-yl)pyrimidin-4-yl)methoxy)phenyl)propan-2-yl)phenoxy)-2-methylpropyl)amino)-2-(2,6-dioxopiperidin-3-yl)isoindolin-1,3-dione (8 mg), with a yield of 9%. ¹H NMR (400 MHz, Chloroform-*d*) *δ* 8.38 - 8.28 (m, 1H), 8.22 (s, 1H), 7.59 (d, *J* = 8.2 Hz, 1H), 7.14 (dd, *J* = 8.4, 6.0 Hz, 4H), 6.98 (d, *J* = 2.1 Hz, 1H), 6.82 (t, *J* = 7.3 Hz, 5H), 6.74 (dd, *J* = 8.4, 2.0 Hz, 1H), 4.96 (s, 2H), 4.94 - 4.89 (m, 1H), 4.86 (s, 4H), 4.33 (s, 4H), 3.99 (dd, *J* = 9.2, 4.0 Hz, 1H), 3.85 (t, *J* = 8.1 Hz, 1H), 3.33 (m, 2H), 2.92 - 2.67 (m, 3H), 2.34 (s, 1H), 2.16 - 2.08 (m, 1H), 1.63 (s, 6H), 1.13 (d, *J* = 6.9 Hz, 3H). LC/MS (ESI+) calcd for C₄₂H₄₄N₆O₇ ([M+H]⁺) *m*/*z:* 745.3; found 745.3.

### Example 497: 5-(4-(4-(2-(2-oxa-6-azaspiro[3.3]heptan-6-yl)pyrimidin-4-yl) methoxy)phenyl)propan-2-yl)phenoxy)but-1-yn-1-yl)-2-(2,6-dioxopiperidin-3-yl)isoindolin-1,3-dione

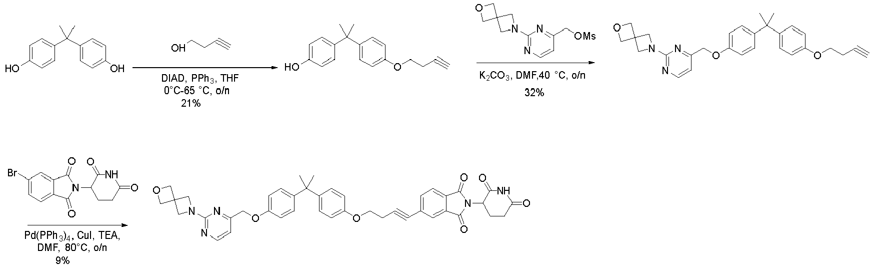

### Step 1: Synthesis of 4-(2-(4-(but-3-yn-1-oxy)phenyl)propan-2-yl)phenol

3-Butyn-1-ol (256 mg, 3.65 mmol), bisphenol A (1.00 g, 4.38 mmol), and PPh₃(958 mg, 3.65 mmol) were added into a three-necked flask under Ar gas protection, to which was added 30 mL of dry THF, and then the solution was stirred well, followed by addition of DIAD (738 mg, 3.65 mmol) dropwise in an ice bath. Subsequently, the reaction solution was still reacted in the ice bath for 1 h, and then reacted overnight at 65 °C. The reaction solution was cooled to room temperature, to which was then added anhydrous ZiCl₂ (315 mg, 2.32 mmol), followed by stirring for 1h. The reaction solution was directly filtered, and the filtrate was washed with 2 N NaOH solution. The resultant solution was extracted with ethyl acetate.The organic phase was washed with saturated brine, dried over anhydrous Na₂SO₄, and concentrated to dry under reduced pressure. The residue was separated and purified by prep-TLC, to provide compound 4-(2-(4-(but-3-yn-1-oxy)phenyl)propan-2-yl)phenol(220 mg), with a yield of 21%. LC/MS (ESI+) calcd for C₁₉H₂₀O₂ ( [M]⁺ ) *m*/*z:* 280.2; found 298.2.

### Step 2: Synthesis of 6-(4-((4-(2-(4-(but-3-yn-1-oxy)phenyl)propan-2-yl)phenoxy) methyl)pyrimidin-2-yl)-2-oxa-6-azaspiro[3.3]heptane

4-(2-(4-(but-3-yn-1-oxy)phenyl)propan-2-yl)phenol (80 mg, 0.28 mmol) and (2-(2-oxa-6-azaspiro[3.3]heptan-6-yl)pyrimidin-4-yl)methyl methanesulfonate (82 mg, 0.28 mmol) were dissolved in 5 mL of DMF, to which was added K₂CO₃ (75 mg, 0.56 mmol), and then the mixture was allowed to react overnight at 40 °C. The reaction was quenched by adding water. The resultant solution was extracted with ethyl acetate. The combined organic layer was washed with saturated brine, dried over anhydrous Na₂SO₄, and concentrated. The residue was separated and purified by prep-TLC, to provide compound 6-(4-((4-(2-(4-(but-3-yn-1-oxy)phenyl)propan-2-yl)phenoxy)methyl) pyrimidin-2-yl)-2-oxa-6-azaspiro[3.3]heptane (70 mg), with a yield of 32%. LC/MS (ESI+) calcd for C₂₉H₃₁N₃O₃ ( [M+H]⁺ ) *m*/*z* 470.2; found 470.2.

### Step 3: Synthesis of 5-(4-(4-(2-(2-oxa-6-azaspiro[3.3]heptan-6-yl)pyrimidin-4-yl) methoxy)phenyl)propan-2-yl)phenoxy)but-1-yn-1-yl)-2-(2,6-dioxopiperidin-3-yl)isoindolin-1,3-dione

6-(4-((4-(2-(4-(but-3-yn-1-oxy)phenyl)propan-2-yl)phenoxy)methyl)pyrimidin-2-yl)-2-oxa-6-azaspiro[3.3]heptane (70 mg, 0.15 mmol), 5-bromo-2-(2,6-dioxopiperidin-3-yl)isoindolin-1,3-dione (60 mg, 0.18 mmol), Pd(PPh₃)₄ (13 mg, 0.015 mmol), CuI (3 mg, 0.015 mmol), and Et₃N(1 mL) were dissolved in 5 mL of DMF, and then the mixture was allowed to react overnight at 80 °C under Ar gas protection. The reaction solution was filtered, and then to the filtrate, was added water to quench the reaction. The resultant solution was extracted with ethyl acetate. The combined organic layer was washed with saturated brine, dried over anhydrous Na₂SO₄, and concentrated. The residue was separated and purified by prep-TLC, to provide compound 5-(4-(4-(2-(2-oxa-6-azaspiro[3.3]heptan-6-yl)pyrimidin-4-yl)methoxy)phenyl)propan-2-yl) phenoxy)but-1-yn-1-yl)-2-(2,6-dioxopiperidin-3-yl)isoindolin-1,3-dione (10 mg), with a yield of 9%. ¹H NMR (400 MHz, Chloroform-*d*) *δ* 8.34 - 8.28 (m, 1H), 8.20 (s, 1H), 7.88 (d, *J* = 2.8 Hz, 1H), 7.81 (dd, *J* = 7.9, 2.8 Hz, 1H), 7.75 (t, *J* = 5.0 Hz, 1H), 7.27 (s, 1H), 7.15 (ddd, *J* = 8.6, 5.2, 2.6 Hz, 4H), 6.84 (dq, *J* = 12.2, 5.3, 4.3 Hz, 4H), 4.99 (dd, *J* = 6.4, 3.4 Hz, 1H), 4.95 (d, *J* = 2.9 Hz, 2H), 4.87 (d, *J* = 2.8 Hz, 4H), 4.30 (d, *J* = 2.8 Hz, 4H), 4.17 (td, *J* = 6.7, 2.7 Hz, 2H), 2.94 (td, *J* = 6.8, 2.8 Hz, 2H), 2.80 (dddd, *J* = 31.6, 17.4, 14.2, 10.5 Hz, 3H), 2.26 - 2.18 (m, 1H), 1.64 (d, *J* = 2.8 Hz, 6H). LC/MS (ESI+) calcd for C₄₂H₃₉N₅O₇ ([M+H]⁺) *m*/*z:* 726.3; found 726.3.

### Example 498: 5-((1-((4-(2-(4-((2-(2-oxa-6-azaspiro[3.3]heptan-6-yl)pyrimidin-4-yl) methoxy)phenyl)propan-2-yl)phenoxy)methyl)cyclobutyl)amino)-2-(2,6-dioxopiperidin-3-yl)isoindolin-1,3-dione

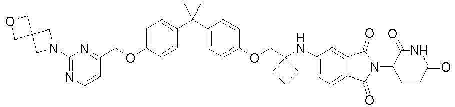

The target compound was synthesized by a method similar to that of Example 496. ¹H NMR (400 MHz, Chloroform-*d*) *δ* 8.36 - 8.28 (m, 1H), 8.18 (d, *J* = 32.0 Hz, 1H), 7.59 (dd, *J* = 11.0, 8.3 Hz, 1H), 7.12 (dd, *J* = 11.3, 7.7 Hz, 4H), 6.98 (d, *J* = 2.1 Hz, 1H), 6.90 (d, *J* = 2.1 Hz, 1H), 6.84 - 6.70 (m, 5H), 4.96 - 4.92 (m, 2H), 4.86 (s, 4H), 4.32 (s, 4H), 4.15 (s, 1H), 2.93 - 2.69 (m, 3H), 2.45 (q, *J* = 9.9, 9.2 Hz, 2H), 2.33 (q, *J* = 10.0, 9.4 Hz, 2H), 2.22 (t, *J* = 7.6 Hz, 2H), 2.15 - 2.09 (m, 1H), 2.04 - 1.97 (m, 2H), 1.60 (s, 6H). LC/MS (ESI+) calcd for C₄₃H₄₄N₆O₇ ([M+H]⁺) *m*/*z:* 757.3; found 757.3.

### Example 499: 5-((1r,3r)-3-(4-(2-(4-(4-((2-(2-oxa-6-azaspiro[3.3]heptane-6-yl) pyrimidin-4-yl)methoxy)phenyl)propan-2-yl)phenoxy)-3-methylcyclobutyl) amino)-2-(2,6-dioxopiperidin-3-yl)isoindolin-1,3-dione

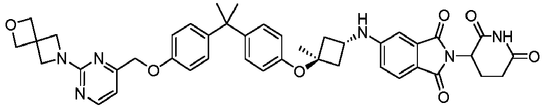

The target compound was synthesized by a method similar to that of Example 493. ¹H NMR (400 MHz, Chloroform-*d*) *δ* 8.42 - 8.20 (m, 2H), 7.61 (d, *J* = 8.3 Hz, 1H), 7.23 - 7.04 (m, 4H), 6.94 - 6.77 (m, 4H), 6.76 - 6.60 (m, 3H), 4.95 (s, 2H), 4.94 - 4.90 (m, 1H), 4.86 (s, 4H), 4.30 (s, 4H), 4.10 (q, *J* = 5.4, 4.0 Hz, 1H), 3.02 (dd, *J* = 13.1, 8.2 Hz, 2H), 2.93 - 2.66 (m, 3H), 2.09 (ddd, *J* = 18.1, 11.6, 6.2 Hz, 3H), 1.63 (d, *J* = 6.8 Hz, 9H). LC/MS (ESI+) calcd for C₄₃H₄₄N₆O₇ ([M+H]⁺) *m*/*z:* 757.3; found 757.3.

### Example 500: 5-((1s,3s)-3-(4-(2-(4-(4-((2-(2,8-diazaspiro[4.5]decan-8-yl) pyrimidin-4-yl)methoxy)phenyl)propan-2-yl)phenoxy)cyclobutyl)amino)-2-(2,6-dioxopyridin-3-yl)isoindolin-1,3-dione

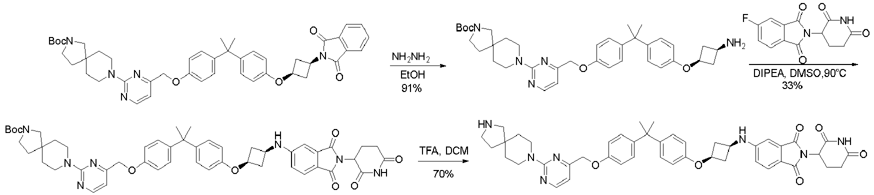

### Step 1: Synthesis of tert-butyl 8-(4-(2-(4-((1s,3s)-3-aminocyclobutyloxy)phenyl) propan-2-yl)phenoxy)methyl)pyrimidin-2-yl)-2,8-diazaspiro[4.5]decan-2-carboxylate

tert-butyl **8-(4-(2-(4-((1*s*,3*s*)-3-(1,3-dioxoisoquinolin-2-yl)cyclobutyloxy)phenyl)** propan-2-yl)phenoxy)methyl)pyrimidin-2-yl)-2,8-diazaspiro[4.5]decan-2-carboxylate (224 mg, 0.29 mmol) was dissolved in ethanol, to which was added hydrazine hydrate (10 drops), and then the mixture was allowed to react for 1 h under refluxing. After completion of the reaction, ethanol was removed by rotatory evaporation. The resultant solution was extracted with ethyl acetate. The combined organic layer was washed with saturated brine, dried over anhydrous Na2SO4, and concentrated. The residue was separated and purified by prep-TLC, to provide compound tert-butyl 8-(4-(2-(4-((1s,3s)-3-aminocyclobutyloxy)phenyl)propan-2-yl)phenoxy)methyl)pyrimidin-2-yl)-2,8-diazaspiro[4.5]decan-2-carboxylate (170 mg), with a yield of 91%. LC/MS (ESI+) calcd for C₃₇H₄₉N₅O₄( [M+H]⁺ ) *m*/*z* 628.3; found 528.3.

### Step 2: Synthesis of tert-butyl 8-(4-(2-(4-((1s,3s)-3-((2-(2,6-dioxopiperidin-3-yl)-1,3-dioxoisoquinolin-5-yl)amino)cyclobutyloxy)phenyl)propan-2-yl)phenoxy)methyl) pyrimidin-2-yl)-2,8-diazaspiro[4.5]decan-2-carboxylate

tert-butyl 8-(4-(2-(4-((1s,3s)-3-aminocyclobutyloxy)phenyl)propan-2-yl)phenoxy) methyl) pyrimidin-2-yl)-2,8-diazaspiro[4.5]decan-2-carboxylate (170 mg, 0.27 mmol) was dissolved in 5 mL of DMSO, to which were added DIPEA (105 mg, 0.81 mmol) and 2-(2,6-dioxopiperidin-3-yl)-5-fluoroisoindolin-1,3-dione (89 mg, 0.32 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide tert-butyl 8-(4-(2-(4-((1s,3s)-3-((2-(2,6-dioxopiperidin-3-yl)-1,3-dioxoisoquinolin-5-yl)amino)cyclobutyloxy)phenyl)propan-2-yl)phenoxy)methyl) pyrimidin-2-yl)-2,8-diazaspiro[4.5]decan-2-carboxylate (80 mg), with a yield of 33%. LC/MS (ESI+) calcd for C₅₀H₅₇N₇O₈ ( [M+H]⁺) *m*/*z*: 883.4; found 442.5.

### Step 3: Synthesis of 5-((1s,3s)-3-(4-(2-(4-(4-((2-(2,8-diazaspiro[4.5]decan-8-yl)pyrimidin-4-yl)methoxy)phenyl)propan-2-yl)phenoxy)cyclobutyl)amino)-2-(2,6-dioxopyridin-3-yl)isoindol-1,3-dione

tert-butyl 8-(4-(2-(4-((1s,3s)-3-((2-(2,6-dioxopiperidin-3-yl)-1,3-dioxoisoquinolin-5-yl)amino)cyclobutyloxy)phenyl)propan-2-yl)phenoxy)methyl) pyrimidin-2-yl)-2,8-diazaspiro[4.5]decan-2-carboxylate (80 mg, 0.09 mmol) was dissolved in 4 mL of dichloromethane, to which was added 1 mL of trifluoroacetic acid in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 5-((1s,3s)-3-(4-(2-(4-(4-((2-(2,8-diazaspiro[4.5]decan-8-yl)pyrimidin-4-yl) methoxy)phenyl)propan-2-yl)phenoxy)cyclobutyl)amino)-2-(2,6-dioxopyridin-3-yl)isoindolin-1,3-dione (50 mg), with a yield of 70%. ¹H NMR (400 MHz, DMSO-*d*₆) δ 8.34 (d, *J* = 5.0 Hz, 1H), 7.59 (d, *J* = 8.3 Hz, 1H), 7.46 (d, *J* = 6.4 Hz, 1H), 7.10 (t, *J* = 8.6 Hz, 4H), 6.89 (d, *J* = 8.7 Hz, 3H), 6.82 (d, *J* = 8.5 Hz, 1H), 6.76 (d, *J* = 8.4 Hz, 2H), 6.67 (d, *J* = 4.9 Hz, 1H), 5.04 (dd, *J* = 12.9, 5.4 Hz, 1H), 4.95 (s, 2H), 4.49 (h, *J* = 7.1, 6.1 Hz, 1H), 3.76 (dtd, *J* = 19.6, 14.2, 13.7, 6.5 Hz, 5H), 3.19 (s, 2H), 3.10 - 2.99 (m, 2H), 2.98 (s, 2H), 2.87 (ddd, *J* = 17.9, 14.4, 5.4 Hz, 1H), 2.57 (d, *J* = 17.0 Hz, 2H), 2.08 - 1.89 (m, 3H), 1.80 (t, *J* = 7.4 Hz, 2H), 1.55 (d, *J* = 11.9 Hz, 10H). LC/MS (ESI+) calcd for C₄₅H₄₉N₇O₆ ([M+H]⁺) *m*/*z* 784.4; found 784.3.

### Example 501: 4-(2-(4-((2-(2-oxa-6-azaspiro[3.3]heptan-6-yl)pyrimidin-4-yl) methoxy)phenyl)propan-2-yl)-N-(2-(2,6-dioxopiperidin-3-yl)-1,3-dioxoisoindolin-5-yl)amino)ethyl)benzamide

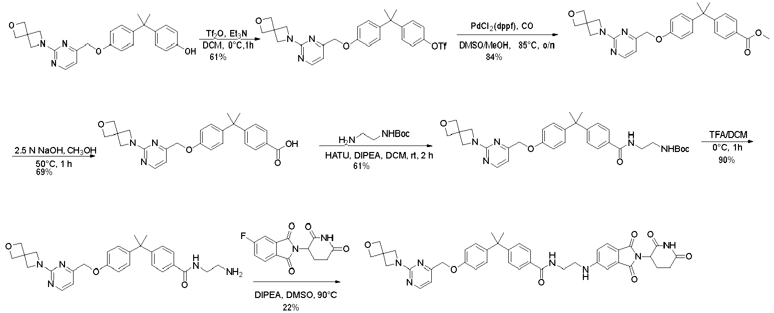

### Step 1: Synthesis of 4-(2-(4-((2-(2-oxa-6-azaspiro[3.3]heptan-6-yl)pyrimidin-4-yl)methoxy)phenyl)propan-2-yl)phenyl trifluoromethanesulfonate

4-(2-(4-((2-(2-oxa-6-azaspiro[3.3]heptan-6-yl)pyrimidin-4-yl)methoxy)phenyl) propan-2-yl)phenol (1.00 g, 2.4 mmol) was dissolved in 20 mL of dichloromethane, to which was added triethylamine (727 mg, 7.2 mmol), followed by addition of trifluoromethanesulfonic anhydride (1.35 g, 4.8 mmol) in an ice water bath, and then the solution was warmed to room temperature and allowed to react for 2 h. TLC indicated disappearance of starting materials, and then saturated NH₄Cl aqueous solution was added to quench the reaction. The resultant solution was extracted with dichloromethane. The combined organic phase was washed with saturated brine, dried over anhydrous MgSO₄, and concentrated to dry under reduced pressure. The residue was separated and purified by column chromatography, to provide the product 4-(2-(4-((2-(2-oxa-6-azaspiro[3.3]heptan-6-yl)pyrimidin-4-yl)methoxy)phenyl)propan-2-yl)phenyl trifluoromethanesulfonate (800 mg), with a yield of 61%. LC/MS (ESI+) calcd for C₂₆H₂₆F₃N₃O₅S ( [M+H]⁺ ) *m*/*z* 550.2; found 550.1.

### Step 2: Synthesis of methyl 4-(2-(4-((2-(2-oxa-6-azaspiro[3.3]heptan-6-yl)pyrimidin-4-yl)methoxy)phenyl)propan-2-yl)benzoate

4-(2-(4-((2-(2-oxa-6-azaspiro[3.3]heptan-6-yl)pyrimidin-4-yl)methoxy)phenyl) propan-2-yl)phenyl trifluoromethanesulfonate (500 mg, 0.91 mmol) was dissolved in a mixed solvent of 6 mL DMSO and 6 mL methanol, to which were added PdCl₂(dppf) (67 mg, 0.09 mmol) and Et₃N(184 mg, 1.82 mmol), and then the system was purged with CO gas for three times. The reaction solution was allowed to react overnight under refluxing at 85 °C. After completion of the reaction, the reaction solution was filtered, and the filtrate was rotatory evaporated to remove methanol. Water was added to quench the reaction. The resultant solution was extracted with ethyl acetate. The combined organic layer was washed with saturated brine, dried over anhydrous Na₂SO₄, and concentrated. The residue was separated and purified by prep-TLC, to provide compound methyl 4-(2-(4-((2-(2-oxa-6-azaspiro[3.3]heptan-6-yl)pyrimidin-4-yl) methoxy)phenyl)propan-2-yl)benzoate (350 mg), with a yield of 84%. LC/MS (ESI+) calcd for C₂₇H₂₉N₃O₄ ( [M+H]⁺) *m*/*z:* 460.2; found 460.1.

### Step 3: Synthesis of 4-(2-(4-((2-(2-oxa-6-azaspiro[3.3]heptan-6-yl)pyrimidin-4-yl)methoxy)phenyl)propan-2-yl)benzoic acid

Methyl 4-(2-(4-((2-(2-oxa-6-azaspiro[3.3]heptan-6-yl)pyrimidin-4-yl)methoxy) phenyl)propan-2-yl)benzoate (300 mg, 0.66 mmol) was dissolved in 10 mL of methanol, to which was added 2.5N NaOH (4 mL), and then the mixture was allowed to react for 1 h at 50 °C. After completion of the reaction, methanol was removed by rotatory evaporation. Water was added to quench the reaction, followed by addition of 2N HCl, to adjust the solution to be alkaline. The resultant solution was extracted with ethyl acetate. The combined organic layer was washed with saturated brine, dried over anhydrous Na₂SO₄, and concentrated under reduced pressure, to provide compound 4-(2-(4-((2-(2-oxa-6-azaspiro[3.3]heptan-6-yl)pyrimidin-4-yl)methoxy)phenyl)propan-2-yl)benzoic acid (200 mg), with a yield of 69%. LC/MS (ESI+) calcd for C₂₆H₂₇N₃O₄ ( [M+H]⁺) *m*/*z:* 446.2; found 446.2.

### Step 4: Synthesis of tert-butyl (2-(4-(2-(4-((2-(2-oxa-6-azaspiro[3.3]heptan-6-yl) pyrimidin-4-yl)methoxy)phenyl)propan-2-yl)benzamido)ethyl)carbamate

4-(2-(4-((2-(2-oxa-6-azaspiro[3.3]heptan-6-yl)pyrimidin-4-yl)methoxy)phenyl) propan-2-yl)benzoic acid (150 mg, 0.34 mmol) was dissolved in 10 mL of dichloromethane, to which were added DIPEA (130.mg, 1.01 mmol) and HATU (194 mg, 0.51mmol), and then the mixture was allowed to react at room temperature for 30 mins, followed by addition of *N*-Boc-ethylenediamine (109 mg, 0.68 mmol). The reaction was allowed to react for additional 2 h at room temperature. After completion of the reaction, water was added to quench the reaction. The resultant solution was extracted with ethyl acetate. The combined organic layer was washed with saturated brine, dried over anhydrous Na₂SO₄, and concentrated under reduced pressure. The residue was separated and purified by prep-TLC, to provide compound tert-butyl (2-(4-(2-(4-((2-(2-oxa-6-azaspiro[3.3]heptan-6-yl)pyrimidin-4-yl)methoxy)phenyl)propan-2-yl)benzamido)ethyl)carbamate (120 mg), with a yield of 61%. LC/MS (ESI+) calcd for C₃₃H₄₁N₅O₅ ([M+H]⁺) *m*/*z:* 588.3; found 588.3.

### Step 5: Synthesis of 4-(2-(4-((2-(2-oxa-6-azaspiro[3.3]heptan-6-yl)pyrimidin-4-yl)methoxy)phenyl)propan-2-yl)-N-(2-aminoethyl)benzamide

(2-(4-(2-(4-((2-(2-oxa-6-azaspiro[3.3]heptan-6-yl)pyrimidin-4-yl)methoxy) phenyl)propan-2-yl)benzamido)ethyl)carbamate (120 mg, 0.20 mmol) was dissolved in 4 mL of dichloromethane, to which was added 1 mL of trifluoroacetic acid in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 4-(2-(4-((2-(2-oxa-6-azaspiro[3.3]heptan-6-yl)pyrimidin-4-yl)methoxy)phenyl)propan-2-yl)-N-(2-aminoethyl)benzamide (90 mg), with a yield of 90%. LC/MS (ESI+) calcd for C₂₈H₃₃N₅O₃ ([M+H]⁺) *m*/*z* 488.3; found 488.3.

### Step 6: Synthesis of 4-(2-(4-((2-(2-oxa-6-azaspiro[3.3]heptan-6-yl)pyrimidin-4-yl)methoxy)phenyl)propan-2-yl)-N-(2-(2,6-dioxopiperidin-3-yl)-1,3-dioxoisoindolin-5-yl)amino)ethyl)benzamide

(4-(2-(4-((2-(2-oxa-6-azaspiro[3.3]heptan-6-yl)pyrimidin-4-yl)methoxy)phenyl) propan-2-yl)-N-(2-aminoethyl)benzamide (90 mg, 0.18 mmol) was dissolved in 5 mL of DMSO, to which were added DIPEA (71 mg, 0.55 mmol) and 2-(2,6-dioxopiperidin-3-yl)-5-fluoroisoindolin-1,3-dione (61 mg, 0.22 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 4-(2-(4-((2-(2-oxa-6-azaspiro[3.3]heptan-6-yl)pyrimidin-4-yl)methoxy)phenyl) propan-2-yl)-N-(2-(2,6-dioxopiperidin-3-yl)-1,3-dioxoisoindolin-5-yl)amino)ethyl) benzamide (30 mg), with a yield of 22%. ¹H NMR (400 MHz, Chloroform-*d*) *δ* 8.30 (d, *J* = 5.0 Hz, 1H), 8.16 (s, 1H), 7.67 (d, *J* = 8.4 Hz, 2H), 7.57 (d, *J* = 8.3 Hz, 1H), 7.33 - 7.28 (m, 2H), 7.16 - 7.06 (m, 2H), 6.95 (d, *J* = 2.2 Hz, 1H), 6.88 - 6.81 (m, 2H), 6.81 - 6.72 (m, 2H), 6.57 (t, *J* = 6.1 Hz, 1H), 4.95 (s, 2H), 4.93 - 4.89 (m, 1H), 4.86 (s, 4H), 4.29 (s, 4H), 3.77 (q, *J* = 5.8 Hz, 2H), 3.44 (s, 2H), 2.92 - 2.68 (m, 3H), 2.01 (d, *J* = 5.7 Hz, 1H), 1.66 (s, 6H). LC/MS (ESI+) calcd for C₄₁H₄₁N₇O₇ ([M+H]⁺) *m*/*z*:744.3; found 744.3.

### Example 502: 5-((1-(4-(2-(4-(4-((2-(2-oxa-6-azaspiro[3.3]heptane-6-yl)pyrimidin-4-yl)methoxy)phenyl)propan-2-yl)benzyl)azetidin-3-yl)amino)-2-(2,6-dioxopiperidin-3-yl)isoindolin-1,3-dione

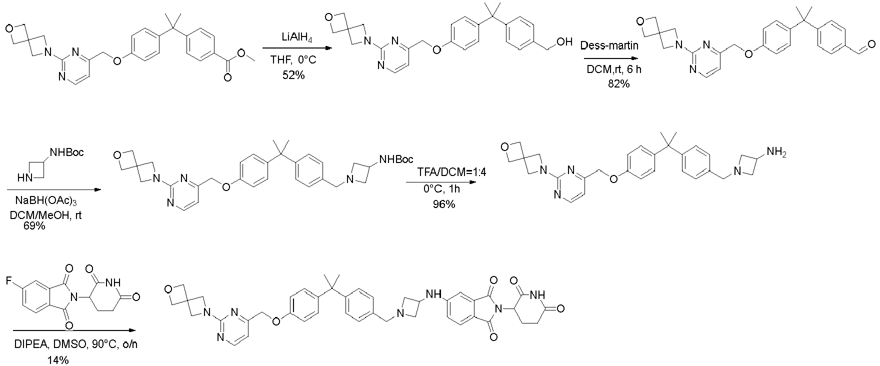

### Step 1: Synthesis of (4-(2-(4-((2-(2-oxa-6-azaspiro[3.3]heptan-6-yl)pyrimidin-4-yl)methoxy)phenyl)propan-2-yl)phenyl)methanol

LiAlH₄ (87 mg, 2.28 mmol) was added to 15 mL of THF under Ar gas protection, to which was slowly added the solution of methyl 4-(2-(4-((2-(2-oxa-6-azaspiro[3.3]heptan-6-yl) pyrimidin-4-yl)methoxy)phenyl)propan-2-yl)benzoate (350 mg, 0.76 mmol) in THF (5 mL) at 0 °C, and then the mixture was further reacted at 0 °C for 20 min. TLC indicated disappearance of starting materials, and then the reaction solution was diluted with diethyl ether, followed by addition of Na₂SO₄·10H₂O to quench the reaction. The solution was stirred in an ice bath for 20 min, and then stirred at room temperature for 30 min, followed by filtration. The filtrate was concentrated to dry under reduced pressure, to obtain the crude product, which was separated and purified by prep-TLC, to obtain (4-(2-(4-((2-(2-oxa-6-azaspiro[3.3]heptan-6-yl)pyrimidin-4-yl)methoxy)phenyl)propan-2-yl)phenyl) methanol (170 mg), with a yield of 52%. LC/MS (ESI+) calcd for C₂₆H₂₉N₃O₃ ( [M+H]⁺ ) *m*/*z* 432.2; found 432.3.

### Step 2: Synthesis of 4-(2-(4-((2-(2-oxa-6-azaspiro[3.3]heptan-6-yl)pyrimidin-4-yl)methoxy)phenyl)propan-2-yl)benzaldehyde

(4-(2-(4-((2-(2-oxa-6-azaspiro[3.3]heptan-6-yl)pyrimidin-4-yl)methoxy)phenyl) propan-2-yl)phenyl)methanol (170 mg, 0.39 mmol) was dissolved in 15 mL of dichloromethane, to which was added Dess-Martin (200 mg, 0.47 mmol), and then the mixture was allowed to react overnight at room temperature. TLC indicated disappearance of starting materials, and then water was added to quench the reaction. The resultant solution was extracted with dichloromethane. The combined organic layer was washed with saturated brine, dried over anhydrous Na₂SO₄, and concentrated, to provide 4-(2-(4-((2-(2-oxa-6-azaspiro[3.3]heptan-6-yl)pyrimidin-4-yl)methoxy) phenyl)propan-2-yl)benzaldehyde (140 mg), with a yield of 82%. LC/MS (ESI+) calcd for C₂₆H₂₇N₃O₃ ( [M+H]⁺ ) *m*/*z* 430.2; found 430.2.

### Step 3: Synthesis of tert-butyl (1-(4-(2-(4-((2-(2-oxa-6-azaspiro[3.3]heptan-6-yl)pyrimidin-4-yl)methoxy)phenyl)propan-2-yl)benzyl)azetidine-3-yl)carbamate

4-(2-(4-((2-(2-oxa-6-azaspiro[3.3]heptan-6-yl)pyrimidin-4-yl)methoxy)phenyl) propan-2-yl)benzaldehyde (160 mg, 0.37 mmol) and 3-(Boc-amino)azetidine (77 mg, 0.45mmol) were dissolved in a mixed solvent of 3 mL dichloromethane and 3 mL methanol, followed by addition of one drop of glacial acetic acid, and then the reaction solution was stirred at room temperature for 30 mins. Subsequently, NaBH(OAc)₃ (236 mg, 1.12 mmol) was added, and then the mixture was allowed to react overnight at room temperature. TLC indicated disappearance of starting materials, and water was added to quench the reaction. The resultant solution was extracted with ethyl acetate. The combined organic layer was washed with saturated brine, dried over anhydrous Na₂SO₄, and concentrated. The residue was separated and purified by prep-TLC, to provide tert-butyl (1-(4-(2-(4-((2-(2-oxa-6-azaspiro[3.3]heptan-6-yl)pyrimidin-4-yl) methoxy)phenyl)propan-2-yl)benzyl)azetidine-3-yl)carbamate (150 mg), with a yield of 69%. LC/MS (ESI+) calcd for C₃₄H₄₃N₅O₄ ( [M+H]⁺ ) *m*/*z* 586.3; found 586.3.

### Step 4: Synthesis of 1-(4-(2-(2-oxa-6-azaspiro[3.3]heptan-6-yl)pyrimidin-4-yl) methoxy)phenyl)propan-2-yl)benzyl)azacyclobutylamine

tert-butyl (1-(4-(2-(4-((2-(2-oxa-6-azaspiro[3.3]heptan-6-yl)pyrimidin-4-yl) methoxy)phenyl)propan-2-yl)benzyl)azetidine-3-yl)carbamate (150 mg, 0.26 mmol) was dissolved in 8 mL of dichloromethane, to which was added 2 mL of TFA in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound 1-(4-(2-(2-oxa-6-azaspiro[3.3]heptan-6-yl)pyrimidin-4-yl)methoxy)phenyl)propan-2-yl)benzyl) azacyclobutylamine (120 mg), with a yield of 96%. LC/MS (ESI+) calcd for C₂₉H₃₅N₅O₂ ( [M+H]⁺ ) *m*/*z* 486.3; found 486.3.

### Step 5: Synthesis of 5-((1-(4-(2-(4-(4-((2-(2-oxa-6-azaspiro[3.3]heptane-6-yl)pyrimidin-4-yl)methoxy)phenyl)propan-2-yl)benzyl )azetidin-3-yl)amino)-2-(2,6-dioxopiperidin-3-yl)isoindolin-1,3-dione

1-(4-(2-(2-oxa-6-azaspiro[3.3]heptan-6-yl)pyrimidin-4-yl)methoxy)phenyl) propan-2-yl)benzyl)azacyclobutylamine (120 mg, 0.25 mmol) was dissolved in 5 mL of DMSO, to which were added DIPEA (96 mg, 0.74 mmol) and 2-(2,6-dioxopiperidin-3-yl)-5-fluoroisoindolin-1,3-dione (82 mg, 0.30 mmol), and then the mixture was allowed to react overnight at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 5-((1-(4-(2-(4-(4-((2-(2-oxa-6-azaspiro[3.3]heptane-6-yl)pyrimidin-4-yl)methoxy)phenyl)propan-2-yl)benzyl)azetidin-3-yl)amino)-2-(2,6-dioxopiperidin-3-yl)isoindolin-1,3-dione (25 mg), with a yield of 14%. ¹H NMR (400 MHz, Chloroform-*d*) *δ* 8.30 (d, *J* = 5.0 Hz, 1H), 7.61 (d, *J* = 8.3 Hz, 1H), 7.21 - 7.09 (m, 6H), 6.88 (d, *J* = 2.1 Hz, 1H), 6.86 - 6.79 (m, 3H), 6.68 (dd, *J* = 8.3, 2.2 Hz, 1H), 4.94 (s, 2H), 4.93 - 4.89 (m, 1H), 4.86 (s, 4H), 4.29 (s, 4H), 4.20 (q, *J* = 5.9 Hz, 1H), 3.75 (t, *J* = 5.3 Hz, 2H), 3.67 (s, 2H), 3.13 (dt, *J* = 8.8, 4.8 Hz, 2H), 2.91 - 2.69 (m, 3H), 2.15 - 2.08 (m, 1H), 1.64 (s, 6H). LC/MS (ESI+) calcd for C₄₂H₄₃N₇O₆ ([M+H]⁺) *m*/*z* 742.3; found 742.3.

### Example 503: 5-((1-(4-(2-(4-(4-((2-(2-oxa-6-azaspiro[3.3]heptane-6-yl)pyrimidin-4-yl)methoxy)phenyl)propan-2-yl)benzoyl)azetidine-3-yl)amino)-2-(2,6-dioxopiperidin-3-yl)isoindolin-1,3-dione

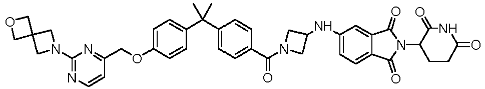

The target compound was synthesized by a method similar to that of Example 501. ¹H NMR (400 MHz, Chloroform-*d*) *δ* 8.30 (d, *J* = 5.1 Hz, 1H), 7.61 (d, *J* = 8.2 Hz, 1H), 7.55 - 7.48 (m, 2H), 7.27 - 7.22 (m, 2H), 7.13 - 7.06 (m, 2H), 6.86 - 6.77 (m, 4H), 6.68 (dd, *J* = 8.3, 2.1 Hz, 1H), 4.97 - 4.91 (m, 3H), 4.86 (s, 4H), 4.66 (s, 2H), 4.36 (d, *J* = 5.5 Hz, 1H), 4.29 (s, 4H), 4.10 (d, *J* = 10.1 Hz, 2H), 2.92 - 2.69 (m, 3H), 2.15 - 2.07 (m, 1H), 1.64 (s, 6H). LC/MS (ESI+) calcd for C₄₂H₄₁N₇O₇ ([M+H]⁺) *m*/*z* 756.3; found 756.3.

### Example 504: 4-(2-(4-((2-(2-oxa-6-azaspiro[3.3]heptan-6-yl)pyrimidin-4-yl) methoxy)phenyl)propan-2-yl)-N-(2-(2,6-dioxopiperidin-3-yl)-1,3-dioxoisoindolin-5-yl)benzamide

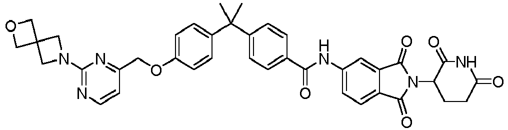

4-(2-(4-(2-(2-oxa-6-azaspiro[3.3]heptan-6-yl)pyrimidin-4-yl)methoxy)phenyl) propan-2-yl)benzoic acid (100 mg, 0.22 mmol), 5-amino-2-(2,6-dioxoporphyrin-3-yl)isoindolin-1,3-dione (63 mg, 0.24 mmol), N-methylimidazol (55 mg, 0.67 mmol), and N,N,N',N'-tetramethylchloroformamidinium hexafluorophosphate (71 mg, 0.26 mmol) were added into 3 mL of DMF, and then the mixture was stirred at room temperature for 5h. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 4-(2-(4-((2-(2-oxa-6-azaspiro[3.3]heptan-6-yl)pyrimidin-4-yl)methoxy)phenyl)propan-2-yl)-N-(2-(2,6-dioxopiperidin-3-yl)-1,3-dioxoisoindolin-5-yl)benzamide (14 mg), with a yield of 9%.

¹H NMR (400 MHz, Chloroform-d) *δ* 8.50 (s, 1H), 8.30 (d, *J* = 5.0 Hz, 1H), 8.25 (s, 1H), 8.18 (d, *J* = 1.7 Hz, 1H), 8.06 (d, *J* = 8.3 Hz, 1H), 7.81 (dd, *J* = 8.2, 2.0 Hz, 3H), 7.36 (d, *J* = 8.0 Hz, 2H), 7.13 (d, *J* = 8.4 Hz, 2H), 6.86 (d, *J* = 8.3 Hz, 2H), 6.81 (d, *J* = 4.8 Hz, 1H), 5.02 - 4.92 (m, 3H), 4.86 (s, 4H), 4.30 (s, 4H), 2.88 - 2.66 (m, 3H), 2.19 - 2.11 (m, 1H), 1.69 (s, 6H). LC/MS (ESI+) calcd for C₃₉H₃₆N₆O₇ ([M+H]+) *m*/*z* 701.3; found 701.2.

### Example 505: 5-((1-(1-(4-(2-(4-(4-)4-((2-(2-oxa-6-azaspiro[3.3]heptane-6-yl) pyrimidin-4-yl)methoxy)phenyl)propan-2-yl)phenyl)ethyl)azetidin-3-yl)amino)-2-(2,6-dioxopiperidin-3-yl)isoindolin-1,3-dione

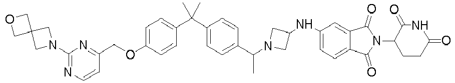

The target compound was synthesized by a method similar to that of Example 502. ¹H NMR (400 MHz, Chloroform-*d*) *δ* 8.31 (d, *J* = 5.1 Hz, 1H), 7.60 (d, *J* = 8.3 Hz, 1H), 7.23 - 7.06 (m, 6H), 6.89 - 6.77 (m, 4H), 6.71 (d, *J* = 8.2 Hz, 1H), 4.94 (s, 2H), 4.94 - 4.88 (m, 1H), 4.86 (s, 4H), 4.29 (s, 4H), 4.18 - 4.10 (m, 1H), 3.78 (t, *J* = 7.1 Hz, 1H), 3.63 (t, *J* = 7.2 Hz, 1H), 3.41 (d, *J* = 6.5 Hz, 1H), 3.14 (s, 1H), 3.04 (s, 1H), 2.91 - 2.69 (m, 3H), 2.15 - 2.09 (m, 1H), 1.64 (s, 6H), 1.30 (t, *J* = 5.3 Hz, 3H). LC/MS (ESI+) calcd for C₄₃H₄₅N₇O₆ ([M+H]⁺) *m*/*z* 756.3; found 756.3.

### Example 506: 4-(2-(4-((2-(2-oxa-6-azaspiro[3.3]heptan-6-yl)pyrimidin-4-yl) methoxy)phenyl)propan-2-yl)-N-(1-(2-(2,6-dioxopiperidin-3-yl)-1,3-dioxoisoindolin-5-yl)azetidin-3-yl)benzamide

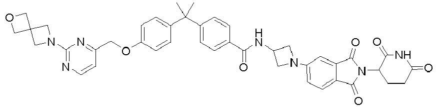

The target compound was synthesized by a method similar to that of Example 501. ¹H NMR (400 MHz, Chloroform-*d*) *δ* 8.54 (s, 1H), 8.29 (d, *J* = 5.1 Hz, 1H), 7.71 (d, *J* = 8.0 Hz, 2H), 7.60 (d, *J* = 8.2 Hz, 1H), 7.29 (s, 1H), 7.11 (dd, *J* = 9.0, 3.0 Hz, 3H), 6.90 - 6.81 (m, 2H), 6.78 (dd, *J* = 5.7, 3.6 Hz, 2H), 6.49 (dd, *J* = 8.3, 2.1 Hz, 1H), 5.12 - 5.01 (m, 1H), 4.94 (s, 2H), 4.91 (dd, *J* = 12.0, 5.3 Hz, 1H), 4.85 (s, 4H), 4.41 (t, *J* = 8.0 Hz, 2H), 4.28 (s, 4H), 3.90 (t, *J* = 7.5 Hz, 2H), 2.89 - 2.65 (m, 3H), 2.10 (dt, *J* = 10.5, 3.9 Hz, 1H), 1.66 (s, 6H). LC/MS (ESI+) calcd for C₄₂H₄₁N₇O₇ ([M+H]⁺) *m*/*z* 756.3; found 756.3.

### Example 507: 5-((4-(2-(4-((2-(2-oxa-6-azaspiro[3.3]heptan-6-yl)pyrimidin-4-yl)methoxy)phenyl)propan-2-yl)benzyl)amino)-2-(2,6-dioxopiperidin-3-yl) isoindolin-1,3-dione

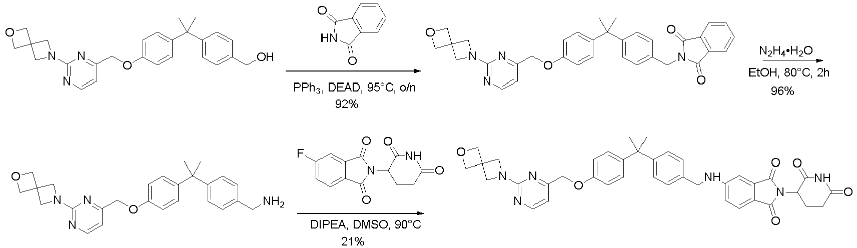

### Step 1: Synthesis of 2-(4-(2-(2-oxa-6-azaspiro[3.3]heptan-6-yl)pyrimidin-4-yl) methoxy)phenyl)propan-2-yl)benzyl )isoindolin-1,3-dione

(4-(2-(4-((2-(2-oxa-6-azaspiro[3.3]heptan-6-yl)pyrimidin-4-yl)methoxy)phenyl) propan-2-yl)phenyl)methanol (100 mg, 0.23 mmol) and PPh₃ (79 mg, 0.30 mmol) were added into a three-necked flask under Ar gas protection, to which was added 5 mL of toluene, and then the solution was heated to 95 °C, followed by adding the solution of phthalimide (41 mg, 0.28 mmol) and DEAD (53 mg, 0.30 mmol) in 3 mL of toluene, and then the mixture was allowed to react overnight at 95 °C. The reaction solution was cooled to room temperature, and subsequently, water was added to quench the reaction. The resultant solution was extracted with ethyl acetate.The organic phase was washed with saturated brine, dried over anhydrous Na₂SO₄, and concentrated to dry under reduced pressure. The residue was separated and purified by prep-TLC, to provide compound 2-(4-(2-(2-oxa-6-azaspiro[3.3]heptan-6-yl)pyrimidin-4-yl)methoxy)phenyl) propan-2-yl)benzyl )isoindol-1,3-dione (120 mg), with a yield of 92%. LC/MS (ESI+) calcd for C₃₄H₃₂N₄O₄ ( [M]⁺) *m*/*z:* 561.2; found 561.2.

### Step 2: Synthesis of (4-(2-(4-((2-(2-oxa-6-azaspiro[3.3]heptan-6-yl)pyrimidin-4-yl)methoxy)phenyl)propan-2-yl)phenyl)carbonylamine

2-(4-(2-(2-oxa-6-azaspiro[3.3]heptan-6-yl)pyrimidin-4-yl)methoxy)phenyl) propan-2-yl)benzyl )isoindol-1,3-dione (120 mg, 0.21 mmol) was dissolved in ethanol, to which was added hydrazine hydrate (10 drops), and then the mixture was allowed to react under refluxing for 1 h. After completion of the reaction, ethanol was removed by rotatory evaporation. The resultant solution was extracted with ethyl acetate. The combined organic layer was washed with saturated brine, dried over anhydrous Na₂SO₄, and concentrated. The residue was separated and purified by prep-TLC, to provide compound (4-(2-(4-((2-(2-oxa-6-azaspiro[3.3]heptan-6-yl)pyrimidin-4-yl)methoxy) phenyl)propan-2-yl)phenyl)carbonylamine (90 mg), with a yield of 96%. LC/MS (ESI+) calcd for C₂₆H₃₀N₄O₂ ( [M+H]⁺ ) *m*/*z* 431.2; found 431.3.

### Step 3: Synthesis of 5-((4-(2-(4-((2-(2-oxa-6-azaspiro[3.3]heptan-6-yl)pyrimidin-4-yl)methoxy)phenyl)propan-2-yl)benzyl)amino)-2-(2,6-dioxopiperidin-3-yl)isoindolin-1,3-dione

(4-(2-(4-((2-(2-oxa-6-azaspiro[3.3]heptan-6-yl)pyrimidin-4-yl)methoxy)phenyl) propan-2-yl)phenyl)carbonylamine (90 mg, 0.21 mmol) was dissolved in 5 mL of DMSO, to which were added DIPEA(81 mg, 0.63 mmol) and 2-(2,6-dioxopiperidin-3-yl)-5-fluoroisoindolin-1,3-dione (69 mg, 0.25 mmol), and then the mixture was allowed to react overnight at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 5-((4-(2-(4-((2-(2-oxa-6-azaspiro[3.3]heptan-6-yl)pyrimidin-4-yl)methoxy)phenyl)propan-2-yl)benzyl)amino)-2-(2,6-dioxopiperidin-3-yl)isoindolin-1,3-dione (30 mg), with a yield of 21%. ¹H NMR (400 MHz, Chloroform-*d*) *δ* 8.30 (d, *J* = 5.1 Hz, 1H), 8.20 (s, 1H), 7.62 (d, *J* = 8.3 Hz, 1H), 7.22 (s, 4H), 7.17 - 7.10 (m, 2H), 6.99 (d, *J* = 2.2 Hz, 1H), 6.88 - 6.80 (m, 3H), 6.78 (dd, *J* = 8.3, 2.2 Hz, 1H), 4.95 (s, 2H), 4.93 - 4.90 (m, 1H), 4.86 (s, 4H), 4.40 (d, *J* = 4.6 Hz, 2H), 4.32 (s, 4H), 2.91 -2.68 (m, 3H), 2.11 (ddd, *J* = 12.5, 5.3, 2.4 Hz, 1H), 1.65 (s, 6H). LC/MS (ESI+) calcd for C₃₉H₃₈N₆O₆ ([M+H]⁺) *m*/*z*:687.3; found 687.3.

### Example 508: 4-(2-(4-((2-(2-oxa-6-azaspiro[3.3]heptan-6-yl)pyrimidin-4-yl) methoxy)phenyl)propan-2-yl)-N-(1-(2-(2,6-dioxopiperidin-3-yl)-1,3-dioxoisoindolin-5-yl)azetidin-3-yl)-N-methylbenzamide

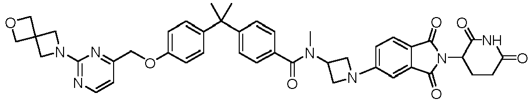

The target compound was synthesized by a method similar to that of Example 501. ¹H NMR (400 MHz, Chloroform-*d*) *δ* 8.38 - 8.24 (m, 2H), 7.68 (d, *J* = 8.2 Hz, 1H), 7.30 (d, *J* = 10.1 Hz, 3H), 7.17 - 7.06 (m, 2H), 6.93 - 6.75 (m, 4H), 6.57 (dd, *J* = 8.3, 2.1 Hz, 1H), 5.30 (s, 1H), 4.95 (s, 2H), 4.92 (d, *J* = 5.5 Hz, 1H), 4.86 (s, 4H), 4.31 (s, 6H), 4.17 (d, *J* = 6.6 Hz, 2H), 3.31 - 3.03 (m, 3H), 2.93 - 2.67 (m, 3H), 2.15 - 2.08 (m, 1H), 1.67 (s, 6H).
LC/MS (ESI+) calcd for C₄₃H₄₃N₇O₇ ([M+H]⁺) *m*/*z*:770.3; found 770.3.

### Example 509: 5-(3-((4-(2-(4-)((2-(2-oxa-6-azaspiro[3.3]heptane-6-yl)pyrimidin-4-yl)methoxy)phenyl)propan-2-yl)benzyl)amino)azetidin-1-yl)-2-(2,6-dioxopiperidin-3-yl)isoindolin-1,3-dione

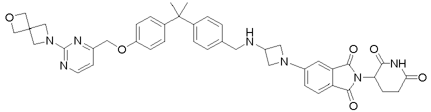

The target compound was synthesized by a method similar to that of Example 502. ¹H NMR (400 MHz, Chloroform-*d*) *δ* 8.30 (d, *J* = 5.1 Hz, 1H), 7.63 (d, *J* = 8.3 Hz, 1H), 7.21 (d, *J* = 1.7 Hz, 4H), 7.17 - 7.08 (m, 2H), 6.93 - 6.78 (m, 3H), 6.75 (d, *J* = 2.1 Hz, 1H), 6.49 (dd, *J* = 8.4, 2.1 Hz, 1H), 4.94 (s, 2H), 4.93 - 4.88 (m, 1H), 4.85 (s, 4H), 4.29 (s, 4H), 4.19 (t, *J* = 7.7 Hz, 2H), 3.90 (dq, *J* = 9.0, 3.5, 2.2 Hz, 1H), 3.78 (s, 2H), 3.71 (dd, *J* = 8.4, 5.0 Hz, 2H), 2.90 - 2.68 (m, 3H), 2.09 (s, 1H), 1.65 (s, 6H). LC/MS (ESI+) calcd for C₄₂H₄₃N₇O₆ ([M+H]⁺) *m*/*z*:742.3; found 742.3.

### Example 511: 2-(2,6-dioxopiperidin-3-yl)-5-((1r,3r)-3-(4-(4-((5-fluoro-6-(5-methyl-1,2,4-oxadiazol-3-yl)pyridin-3-yl)oxy)phenyl)propan-2-yl)phenoxy) cyclobutyl)amino)isoindolin-1,3-dione

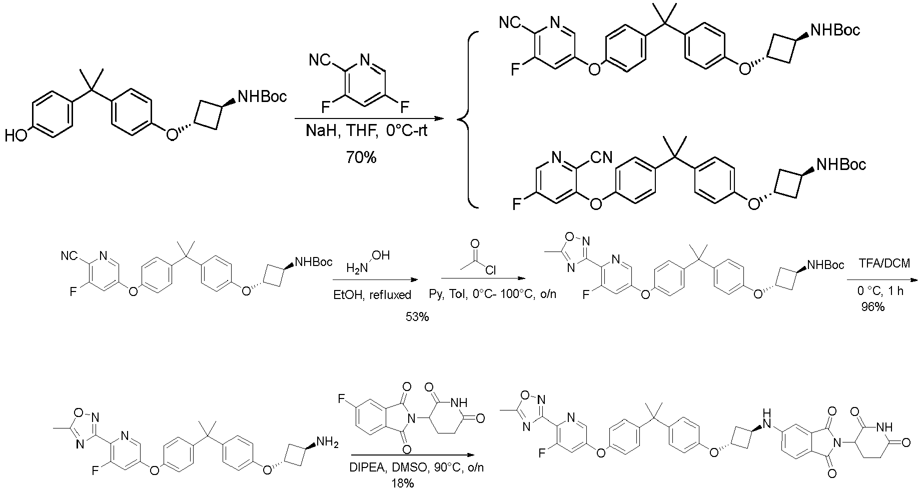

### Step 1: Synthesis of tert-butyl ((1r,3r)-3-(4-(2-(4-((6-cyano-5-fluoropyridin-3-yl)oxy)phenyl)propan-2-yl)phenoxy)cyclobutyl)carbamate and tert-butyl ((1r,3r)-3-(4-(2-(4-((2-cyano-5-fluoropyridin-3-yl)oxy)phenyl)propan-2-yl)phenoxy)cyclobutyl) carbamate

tert-butyl ((1r,3r)-3-(4-(2-(4-hydroxylphenyl)propan-2-phenoxy)cyclobutyl) carbamate (3.97 g, 10 mmol) was dissolved in 15 mL of THF, to which was added 60% NaH (480 mg, 12 mmol) in an ice bath, and then the solution was stirred in the ice bath for 30 min. Subsequently, 2-cyano-3,5-difluoropyridine (1.68 g, 12 mmol) was added. The reaction solution was slowly warmed to room temperature, and allowed to react for additional 2 h. After completion of the reaction detected by TLC, saturated NH₄Cl solution was added to the reaction solution in an ice bath, to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by column chromatography, to provide two isomers: tert-butyl ((1r,3r)-3-(4-(2-(4-((6-cyano-5-fluoropyridin-3-yl)oxy)phenyl)propan-2-yl)phenoxy)cyclobutyl)carbamate (720 mg); tert-butyl ((1r,3r)-3-(4-(2-(4-((2-cyano-5-fluoropyridin-3-yl)oxy)phenyl) propan-2-yl)phenoxy)cyclobutyl)carbamate (2.90 g), with a total yield of 70%.

### Step 2: Synthesis of tert-butyl ((1r,3r)-3-(4-(2-(4-((5-fluoro-6-(5-methyl-1,2,4-oxadiazol-3-yl)pyridin-3-yl)oxy)phenyl)propan-2-yl)phenoxy)cyclobutyl)carbamate

tert-butyl ((1r,3r)-3-(4-(2-(4-((6-cyano-5-fluoropyridin-3-yl)oxy)phenyl)propan-2-yl)phenoxy)cyclobutyl)carbamate (120 mg, 0.23 mmol) was dissolved in 5 mL of ethanol, to which was added 50% hydroxylamine aqueous solution (30 mg, 0.46 mmol), and then the mixture was allowed to react under refluxing for 1 h. After completion of the reaction, a small amount of ethanol was removed by rotatory evaporation. The resultant solution was extracted with ethyl acetate. The combined organic layer was washed with saturated brine, dried over anhydrous Na₂SO₄, and concentrated by rotatory evaporation, to obtain the product as white solid, which was dissolved in 3 mL of pyridine. The system was transferred to an ice water bath for cooling, to which was added the solution of acetyl chloride (24 mg, 0.30 mmol) in 1 mL of toluene, and subsequently, the reaction solution was stirred for 10 min. The ice water bath was removed. The reaction solution was heated to 100 °C, reacted overnight, and then concentrated to dry under reduced pressure. The residue was separated and purified by TLC, to provide the product tert-butyl ((1r,3r)-3-(4-(2-(4-((5-fluoro-6-(5-methyl-1,2,4-oxadiazol-3-yl)pyridin-3-yl)oxy)phenyl)propan-2-yl)phenoxy)cyclobutyl)carbamate (70 mg), with a yield of 53%, LC/MS (ESI+) calcd for C₃₂H₃₅FN₄O₅ ([M+H]⁺) *m*/*z:* 575.3; found 519.2.

### Step 3: Synthesis of (1r,3r)-3-(4-(2-(4-((5-fluoro-6-(5-methyl-1,2,4-oxadiazol-3-yl) pyridin-3-yl)oxy)phenyl)propan-2-yl)phenoxy)cyclobutylamine

tert-butyl ((1r,3r)-3-(4-(2-(4-((5-fluoro-6-(5-methyl-1,2,4-oxadiazol-3-yl)pyridin-3-yl)oxy)phenyl)propan-2-yl)phenoxy)cyclobutyl)carbamate (70 mg, 0.12 mmol) was dissolved in 4 mL of dichloromethane, to which was added 1 mL of TFA in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound (1r,3r)-3-(4-(2-(4-((5-fluoro-6-(5-methyl-1,2,4-oxadiazol-3-yl)pyridin-3-yl)oxy)phenyl)propan-2-yl)phenoxy)cyclobutylamine (55 mg), with a yield of 96%.

### Step 4: Synthesis of 2-(2,6-dioxopiperidin-3-yl)-5-((1r,3r)-3-(4-(4-((5-fluoro-6-(5-methyl-1,2,4-oxadiazol-3-yl)pyridin-3-yl)oxy)phenyl)propan-2-yl)phenoxy) cyclobutyl)amino)isoindolin-1,3-dione

((1r,3r)-3-(4-(2-(4-((5-fluoro-6-(5-methyl-1,2,4-oxadiazol-3-yl)pyridin-3-yl)oxy) phenyl)propan-2-yl)phenoxy)cyclobutylamine (55 mg, 0.12 mmol) was dissolved in 5 mL of DMSO, to which were added DIPEA (45 mg, 0.35 mmol) and 2-(2,6-dioxopiperidin-3-yl)-5-fluoroisoindolin-1,3-dione (38 mg,0.14 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-((1r,3r)-3-(4-(4-((5-fluoro-6-(5-methyl-1,2,4-oxadiazol-3-yl)pyridin-3-yl)oxy)phenyl)propan-2-yl)phenoxy)cyclobutyl)amino) isoindolin-1,3-dione (15 mg), with a yield of 18%.
¹H NMR (400 MHz, DMSO-*d*₆) *δ* 11.07 (s, 1H), 8.46 - 8.34 (m, 1H), 7.61 - 7.55 (m, 2H), 7.53 (d, *J* = 5.4 Hz, 1H), 7.34 - 7.28 (m, 2H), 7.18 - 7.12 (m, 4H), 6.91 - 6.84 (m, 1H), 6.83 - 6.79 (m, 1H), 6.77 (d, *J* = 8.7 Hz, 2H), 5.04 (dd, *J* = 12.9, 5.4 Hz, 1H), 4.88 (p, *J* = 6.2 Hz, 1H), 4.15 (d, *J* = 4.9 Hz, 1H), 2.88 (ddd, *J* = 17.5, 14.1, 5.5 Hz, 1H), 2.69 (s, 3H), 2.62 - 2.51 (m, 4H), 2.44 (q, *J* = 6.7, 6.0 Hz, 2H), 2.03 - 1.96 (m, 1H), 1.64 (s, 6H). LC/MS (ESI+) calcd for C₄₀H₃₅FN₆O₇ ([M+H]⁺) *m*/*z*:731.3; found 731.2.

### Example 512: 2-(2,6-dioxopiperidin-3-yl)-5-((1r,3r)-3-(4-(4-((5-fluoro-2-(5-methyl-1,2,4-oxadiazol-3-yl)pyridin-3-yl)oxy)phenyl)propan-2-yl)phenoxy) cyclobutyl)amino)isoindolin-1,3-dione

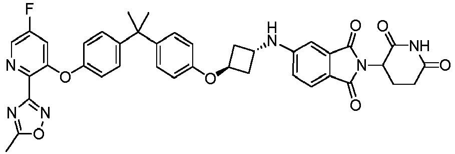

The target compound was synthesized by a method similar to that of Example 511. ¹H NMR (400 MHz, DMSO-*d*₆) *δ* 11.07 (s, 1H), 8.58 (d, *J* = 2.4 Hz, 1H), 7.59 (d, *J* = 8.3 Hz, 1H), 7.57 - 7.50 (m, 2H), 7.30 - 7.21 (m, 2H), 7.13 (d, *J* = 8.7 Hz, 2H), 7.06 - 6.94 (m, 2H), 6.86 (s, 1H), 6.79 (d, *J* = 8.6 Hz, 1H), 6.77 - 6.72 (m, 2H), 5.04 (dd, *J* = 12.9, 5.3 Hz, 1H), 4.86 (h, *J* = 5.6, 5.0 Hz, 1H), 4.14 (d, *J* = 6.1 Hz, 1H), 2.88 (ddd, *J* = 17.1, 14.0, 5.5 Hz, 1H), 2.65 (s, 3H), 2.61 - 2.51 (m, 4H), 2.43 (dd, *J* = 11.5, 6.1 Hz, 2H), 1.99 (d, *J* = 12.1 Hz, 1H), 1.61 (s, 6H). LC/MS (ESI+) calcd for C₄₀H₃₅FN₆O₇ ([M+H]⁺) *m*/*z*:731.3; found 731.3.

### Example 513: 2-(2,6-dioxopiperidin-3-yl)-5-((1r,3r)-3-(4-(2-(4-((5-morpholino-1,3,4-thiadiazol-2-yl)oxy)phenyl)propan-2-yl)phenoxy)cyclobutyl)amino) isoindolin-1,3-dione

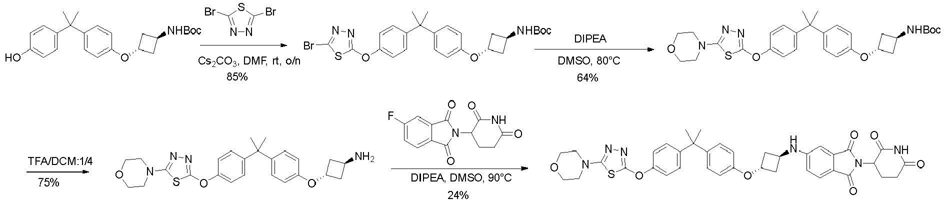

### Step 1: Synthesis of tert-butyl ((1r,3r)-3-(4-(2-(4-((5-bromo-1,3,4-thiadiazol-2-yl)oxy) phenyl)propan-2-yl)phenoxy)cyclobutyl)carbamate

tert-butyl ((1r,3r)-3-(4-(2-(4-hydroxylphenyl)propan-2-phenoxy)cyclobutyl) carbamate (250 mg, 0.63 mmol) and 2,5-dibromo-1,3,4-thiadiazole (184 mg, 0.76 mmol) were dissolved in 5 mL of DMF, to which was added Cs₂CO₃ (400 mg, 1.26 mmol), and then the mixture was allowed to react overnight at room temperature. The reaction was quenched by adding water. The resultant solution was extracted with ethyl acetate. The combined organic layer was washed with saturated brine, dried over anhydrous Na₂SO₄, and concentrated. The residue was separated and purified by prep-TLC, to provide compound tert-butyl ((1r,3r)-3-(4-(2-(4-((5-bromo-1,3,4-thiadiazol-2-yl)oxy)phenyl)propan-2-yl)phenoxy)cyclobutyl)carbamate (300 mg), with a yield of 85%. LC/MS (ESI+) calcd for C₂₆H₃₀BrN₃O₄S( [M+H]⁺ ) *m*/*z* 560.1; found 504.0.

### Step 2: Synthesis of tert-butyl ((1r,3r)-3-(4-(2-(4-((5-morpholino-1,3,4-thiadiazol-2-yl)oxy)phenyl)propan-2-yl)phenoxy)cyclobutyl)carbamate

tert-butyl ((1r,3r)-3-(4-(2-(4-((5-bromo-1,3,4-thiadiazol-2-yl)oxy)phenyl)propan-2-yl)phenoxy)cyclobutyl)carbamate (200 mg, 0.36 mmol) and morpholine (62 mg, 0.71 mmol) were dissolved in 5 mL of DMSO, to which was added DIPEA(138 mg, 1.07 mmol), and then the mixture was allowed to react overnight at 80 °C. The reaction was quenched by adding water. The resultant solution was extracted with ethyl acetate. The combined organic layer was washed with saturated brine, dried over anhydrous Na₂SO₄, and concentrated. The residue was separated and purified by prep-TLC, to provide compound tert-butyl ((1r,3r)-3-(4-(2-(4-((5-morpholino-1,3,4-thiadiazol-2-yl)oxy) phenyl)propan-2-yl)phenoxy)cyclobutyl)carbamate (130 mg), with a yield of 64%. LC/MS (ESI+) calcd for C₃₀H₃₈N₄O₅S( [M+H]⁺ ) *m*/*z* 567.3; found 567.3.

### Step 3: Synthesis of ((1r,3r)-3-(4-(2-(4-((5-morpholino-1,3,4-thiadiazol-2-yl)oxy)phenyl)propan-2-yl)phenoxy)cyclobutylamine

tert-butyl ((1r,3r)-3-(4-(2-(4-((5-morpholino-1,3,4-thiadiazol-2-yl)oxy)phenyl) propan-2-yl)phenoxy)cyclobutyl)carbamate (130 mg, 0.23 mmol) was dissolved in 4 mL of dichloromethane, to which was added 1 mL of trifluoroacetic acid in an ice bath, and then the reaction was still stirred in the ice bath for 1 h. After completion of the reaction detected by TLC, the pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide compound ((1r,3r)-3-(4-(2-(4-((5-morpholino-1,3,4-thiadiazol-2-yl)oxy)phenyl)propan-2-yl)phenoxy) cyclobutylamine (80 mg), with a yield of 75%.

### Step 4: Synthesis of 2-(2,6-dioxopiperidin-3-yl)-5-((1r,3r)-3-(4-(2-(4-((5-morpholino-1,3,4-thiadiazol-2-yl)oxy)phenyl)propan-2-yl)phenoxy)cyclobutyl)amino)isoindolin-1,3-dione

((1r,3r)-3-(4-(2-(4-((5-morpholino-1,3,4-thiadiazol-2-yl)oxy)phenyl)propan-2-yl)phenoxy)cyclobutylamine (80 mg, 0.17 mmol) was dissolved in 3 mL of DMSO, to which were added DIPEA (66 mg, 0.52 mmol) and 2-(2,6-dioxopiperidin-3-yl)-5-fluoroisoindolin-1,3-dione (57 mg, 0.21 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-((1r,3r)-3-(4-(2-(4-((5-morpholino-1,3,4-thiadiazol-2-yl)oxy) phenyl)propan-2-yl)phenoxy)cyclobutyl)amino)isoindolin-1,3-dione (30 mg), with a yield of 24%. ¹H NMR (400 MHz, Chloroform-*d*) *δ* 8.15 (s, 1H), 7.62 (d, *J* = 8.2 Hz, 1H), 7.22 (d, *J* = 8.0 Hz, 2H), 7.13 (t, *J* = 8.8 Hz, 4H), 6.90 (d, *J* = 2.0 Hz, 1H), 6.70 (dd, *J* = 8.6, 3.6 Hz, 3H), 4.93 (dd, *J* = 12.1, 5.2 Hz, 1H), 4.86 (s, 1H), 4.18 (d, *J* = 26.6 Hz, 1H), 3.82 (s, 4H), 3.50 (s, 4H), 2.93 - 2.75 (m, 3H), 2.73 - 2.67 (m, 2H), 2.45 - 2.38 (m, 2H), 2.13 (d, *J* = 7.5 Hz, 1H), 1.65 (s, 6H). LC/MS (ESI+) calcd for C₃₈H₃₈N₆O₇S ([M+H]⁺) *m*/*z:* 723.3; found 723.2.

### Example 514: 2-(2,6-dioxopiperidin-3-yl)-5-(1r,3r)-3-(4-(2-(4-(4-(5-methyl-1,3,4-oxadiazol-2-yl)oxazol-2-yl)oxy)phenyl)propan-2-yl)phenoxy)cyclobutyl)amino) isoindolin-1,3-dione

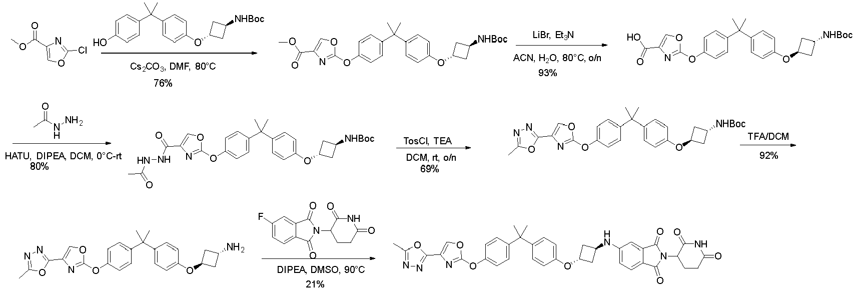

### Step 1: Synthesis of methyl 2-(4-(2-(4-((1r,3r)-3-((tert-butoxycarbonyl)amino) cyclobutyloxy)phenyl)propan-2-yl)phenoxy)oxazol-4-carboxylate

tert-butyl ((1r,3r)-3-(4-(2-(4-hydroxylphenyl)propan-2-phenoxy)cyclobutyl) carbamate (300 mg, 0.75 mmol) and methyl 2-chlorooxazol-4-carboxylate (122 mg, 0.75 mmol) were dissolved in 5 mL of DMF, to which was added Cs₂CO₃ (493 mg, 1.51 mmol), and then the mixture was allowed to react overnight at 80 °C. The reaction was quenched by adding water. The resultant solution was extracted with ethyl acetate. The combined organic layer was washed with saturated brine, dried over anhydrous Na₂SO₄, and concentrated. The residue was separated and purified by prep-TLC, to provide compound methyl 2-(4-(2-(4-((1r,3r)-3-((tert-butoxycarbonyl)amino) cyclobutyloxy)phenyl)propan-2-yl)phenoxy)oxazol-4-carboxylate (300 mg), with a yield of 76%. LC/MS (ESI+) calcd for C₂₉H₃₄N₂O₇ ( [M+H]⁺ ) *m*/*z* 523.2; found 423.1.

### Step 2: Synthesis of 2-(4-(2-(4-((1r,3r)-3-((tert-butoxycarbonyl)amino)cyclobutyloxy) phenyl)propan-2-yl)phenoxy)oxazol-4-carboxylic acid

Methyl 2-(4-(2-(4-((1r,3r)-3-((tert-butoxycarbonyl)amino)cyclobutyloxy)phenyl) propan-2-yl)phenoxy)oxazol-4-carboxylate (300 mg, 0.57 mmol) was dissolved in 10 mL mixed solvent of acetonitrile/water (1/1), to which were added LiBr (99 mg, 1.15 mmol) and Et₃N (116 mg, 1.15 mmol), and then the mixture was allowed to react overnight at 80 °C. The reaction solution was cooled to room temperature, and extracted with ethyl acetate. The combined organic layer was washed with saturated brine, dried over anhydrous Na₂SO₄, and concentrated, to provide compound 2-(4-(2-(4-((1r,3r)-3-((tert-butoxycarbonyl)amino)cyclobutyloxy)phenyl)propan-2-yl)phenoxy)oxazol-4-carboxylic acid (270 mg), with a yield of 93%. LC/MS (ESI+) calcd for C₂₈H₃₂N₂O₇ ( [M+H]⁺ ) *m*/*z* 509.2; found 409.1.

### Step 3: Synthesis of compound tert-butyl ((1r,3r)-3-(4-(2-(4-((4-(2-acethydrazide-1-carbonyl)oxazol-2-yl)oxy)phenyl)propan-2-yl)phenoxy)cyclobutyl)carbamate

2-(4-(2-(4-((1r,3r)-3-((tert-butoxycarbonyl)amino)cyclobutyloxy)phenyl)propan-2-yl)phenoxy)oxazol-4-carboxylic acid (270 mg, 0.53 mmol) was dissolved in 10 mL of dichloromethane, to which were added DIPEA (130 mg, 1.06 mmol) and HATU (300 mg, 0.80 mmol) at 0 °C, and then the mixture was allowed to react at room temperature for 30 min, followed by addition of acethydrazide (79 mg, 1.06 mmol). The mixture was allowed to react for additional 2 h at room temperature. After completion of the reaction, water was added to quench the reaction. The resultant solution was extracted with ethyl acetate. The combined organic layer was washed with saturated brine, dried over anhydrous Na₂SO₄, and concentrated under reduced pressure, to provide compound tert-butyl ((1r,3r)-3-(4-(2-(4-((4-(2-acethydrazide-1-carbonyl)oxazol-2-yl)oxy)phenyl)propan-2-yl)phenoxy)cyclobutyl)carbamate (240 mg), with a yield of 80%. LC/MS (ESI+) calcd for C₃₀H₃₆N₄O₇ ( [M+H]⁺) *m*/*z:* 565.3; found 465.2.

### Step 4: Synthesis of tert-butyl ((1r,3r)-3-(4-(2-(4-((4-(5-methyl-1,3,4-oxadiazol-2-yl)oxazol-2-yl)oxy)phenyl)propan-2-yl)phenoxy)cyclobutyl)carbamate

tert-butyl ((1r,3r)-3-(4-(2-(4-((4-(2-acethydrazide-1-carbonyl)oxazol-2-yl)oxy) phenyl)propan-2-yl)phenoxy)cyclobutyl)carbamate (240 mg, 0.43 mmol) was dissolved in 10 mL of dichloromethane, to which were added triethylamine (65 mg, 0.64 mmol) and p-toluenesulfonyl chloride (98 mg, 0.52mmol), and then the mixture was allowed to react overnight at room temperature. The resultant solution was washed with water, and then extracted with ethyl acetate. The combined organic layer was washed with saturated brine, dried over anhydrous Na₂SO₄, and concentrated under reduced pressure. The residue was separated and purified by prep-TLC, to provide the product tert-butyl ((1r,3r)-3-(4-(2-(4-((4-(5-methyl-1,3,4-oxadiazol-2-yl)oxazol-2-yl)oxy)phenyl)propan-2-yl)phenoxy)cyclobutyl)carbamate (160 mg), with a yield of 69%, LC/MS (ESI+) calcd for C₃₀H₃₄N₄O₆ ( [M+H]⁺) *m*/*z:* 547.2; found 447.2.

### Step 5: Synthesis of (1r,3r)-3-(4-(2-(4-((4-(5-methyl-1,3,4-oxadiazol-2-yl)oxazol-2-yl)oxy)phenyl)propan-2-yl)phenoxy)cyclobutylamine

tert-butyl ((1r,3r)-3-(4-(2-(4-((4-(5-methyl-1,3,4-oxadiazol-2-yl)oxazol-2-yl)oxy) phenyl)propan-2-yl)phenoxy)cyclobutyl)carbamate (160 mg, 0.29 mmol) was dissolved in 4 mL of dichloromethane, to which was added 1.5 mL of TFA in an ice water bath, and then the reaction was still stirred in the ice water bath for 1 h. The pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide (1r,3r)-3-(4-(2-(4-((4-(5-methyl-1,3,4-oxadiazol-2-yl) oxazol-2-yl)oxy)phenyl)propan-2-yl)phenoxy)cyclobutylamine (120 mg), with a yield of 92%.

### Step 6: Synthesis of 2-(2,6-dioxopiperidin-3-yl)-5-(1r,3r)-3-(4-(2-(4-(4-(5-methyl-1,3,4-oxadiazol-2-yl)oxazol-2-yl)oxy)phenyl)propan-2-yl)phenoxy)cyclobutyl)amino) isoindolin-1,3-dione

(1*r*,3*r*)-3-(4-(2-(4-((4-(5-methyl-1,3,4-oxadiazol-2-yl)oxazol-2-yl)oxy)phenyl) propan-2-yl) phenoxy)cyclobutylamine (120 mg, 0.27 mmol) was dissolved in 4 mL of DMSO, to which were added DIPEA(104 mg, 0.81 mmol) and 2-(2,6-dioxopiperidin-3-yl)-5-fluoroisoindolin-1,3-dione (89 mg, 0.32 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-(1r,3r)-3-(4-(2-(4-(4-(5-methyl-1,3,4-oxadiazol-2-yl) oxazol-2-yl)oxy)phenyl)propan-2-yl)phenoxy)cyclobutyl)amino)isoindolin-1,3-dione (40 mg), with a yield of 21%. ¹H NMR (400 MHz, Chloroform-*d*) *δ* 8.13 (s, 1H), 7.98 (d, *J* = 1.9 Hz, 1H), 7.67 - 7.56 (m, 1H), 7.27 (d, *J* = 2.1 Hz, 3H), 7.18 - 7.08 (m, 2H), 6.92 (s, 1H), 6.72 (dt, *J* = 8.1, 4.7 Hz, 3H), 4.92 (dd, *J* = 20.2, 12.6 Hz, 2H), 4.23 (s, 1H), 2.91 - 2.76 (m, 3H), 2.70 (s, 2H), 2.59 (d, *J* = 1.9 Hz, 3H), 2.42 (s, 2H), 2.12 (d, *J* = 6.9 Hz, 1H), 1.66 (s, 6H). LC/MS (ESI+) calcd for C₃₈H₃₄N₆O₈ ([M+H]⁺) *m*/*z*:703.2; found 703.2.

### Example 515: 5-((1r,3r)-3-(4-(2-(4-((5-chloro-6-(2H-1,2,3-triazol-2-yl)pyridin-3-yl)oxy)phenyl)propan-2-yl)phenoxy)cyclobutyl)amino)-2-(2,6-dioxopiperidin-3-yl)isoindolin-1,3-dione

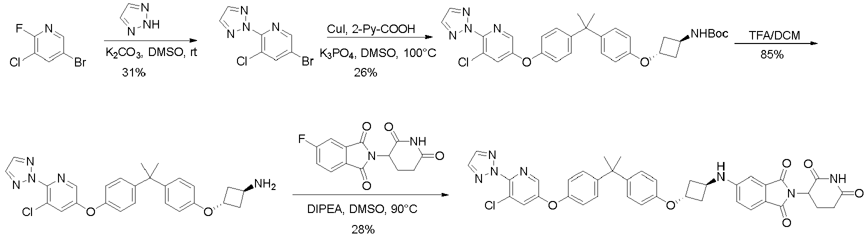

### Step 1: Synthesis of 5-bromo-3-chloro-2-(2H-1,2,3-triazol-2-yl)pyridine

5-bromo-3-chloro-2-fluoropyridine (1.00 g, 4.76 mmol) was dissolved in 10 mL of DMF, to which was added K₂CO₃ (1.31 g, 9.52 mmol), followed by addition of 2H-1,2,3-triazole (295 mg, 4.28 mmol) under stirring, and then the mixture was allowed to react overnight at room temperature. Water was added to quench the reaction. The resultant solution was extracted with ethyl acetate. The combined organic layer was washed with saturated brine, dried over anhydrous Na₂SO₄, and concentrated to dry under reduced pressure. The residue was separated and purified by column chromatography, to provide the product 5-bromo-3-chloro-2-(2H-1,2,3-triazol-2-yl) pyridine (380 mg), with a yield of 31%. LC/MS (ESI+) calcd for C₇H₄BrClN₄ ( [M+H]⁺) *m*/*z*: 258.9; found 259.0.

### Step 2: Synthesis of tert-butyl ((1r,3r)-3-(4-(2-(4-((5-chloro-6-(2H-1,2,3-triazol-2-yl)pyridin-3-yl)oxy)phenyl)propan-2-yl)phenoxy)cyclobutyl)carbamate

5-bromo-3-chloro-2-(2H-1,2,3-triazol-2-yl)pyridine (380 mg, 1.47 mmol) was dissolved in 10 mL of DMSO, to which were added tert-butyl ((1r,3r)-3-(4-(2-(4-hydroxylphenyl)propan-2-phenoxy)cyclobutyl)carbamate (583 mg, 1.47 mmol), potassium phosphate (624 mg, 2.95 mmol), CuI (84 mg, 0.44 mmol), and 2-picolinic acid (54 mg, 0.44 mmol), and then the mixture was allowed to react overnight at 100 °C. The reaction solution was cooled to room temperature, and water was added to quench the reaction. The resultant solution was extracted with ethyl acetate. The combined organic layer was washed with saturated brine, dried over anhydrous Na₂SO₄, and concentrated to dry under reduced pressure. The residue was separated and purified by column chromatography, to provide tert-butyl ((1r,3r)-3-(4-(2-(4-((5-chloro-6-(2H-1,2,3-triazol-2-yl)pyridin-3-yl)oxy)phenyl)propan-2-yl)phenoxy)cyclobutyl) carbamate (220 mg), with a yield of 26%. LC/MS (ESI+) calcd for C₃₁H₃₄ClN₅O₄ ( [M+H]⁺) *m*/*z:* 576.2; found 520.1.

### Step 3: Synthesis of (1r,3r)-3-(4-(2-(4-((5-chloro-6-(2H-1,2,3-triazol-2-yl)pyridin-3-yl)oxy)phenyl)propan-2-yl)phenoxy)cyclobutyl-1-amine

tert-butyl ((1r,3r)-3-(4-(2-(4-((5-chloro-6-(2H-1,2,3-triazol-2-yl)pyridin-3-yl)oxy) phenyl)propan-2-yl)phenoxy)cyclobutyl)carbamate (100 mg, 0.17 mmol) was dissolved in 4 mL of dichloromethane, to which was added 1.5 mL of TFA in an ice water bath, and then the reaction was still stirred in the ice water bath for 1 h. The pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide (1r,3r)-3-(4-(2-(4-((5-chloro-6-(2H-1,2,3-triazol-2-yl)pyridin-3-yl)oxy)phenyl)propan-2-yl)phenoxy)cyclobutyl-1-amine (70 mg), with a yield of 85%.

### Step 4: Synthesis of 5-((1r,3r)-3-(4-(2-(4-((5-chloro-6-(2H-1,2,3-triazol-2-yl)pyridin-3-yl)oxy)phenyl)propan-2-yl)phenoxy)cyclobutyl)amino)-2-(2,6-dioxopiperidin-3-yl)isoindolin-1,3-dione

(1r,3r)-3-(4-(2-(4-((5-chloro-6-(2H-1,2,3-triazol-2-yl)pyridin-3-yl)oxy)phenyl) propan-2-yl)phenoxy)cyclobutyl-1-amine (70 mg, 0.15 mmol) was dissolved in 4 mL of DMSO, to which were added DIPEA (57 mg, 0.44 mmol) and 2-(2,6-dioxopiperidin-3-yl)-5-fluoroisoindolin-1,3-dione (49 mg, 0.18 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 5-((1r,3r)-3-(4-(2-(4-((5-chloro-6-(2H-1,2,3-triazol-2-yl)pyridin-3-yl)oxy)phenyl) propan-2-yl)phenoxy)cyclobutyl)amino)-2-(2,6-dioxopiperidin-3-yl)isoindolin-1,3-dione (30 mg), with a yield of 28%. ¹H NMR (400 MHz, Chloroform-*d*) *δ* 8.29 - 8.22 (m, 1H), 8.13 (s, 1H), 7.92 (s, 2H), 7.62 (d, *J* = 7.8 Hz, 1H), 7.49 (d, *J* = 2.3 Hz, 1H), 7.33 - 7.27 (m, 2H), 7.15 (d, *J* = 8.4 Hz, 2H), 7.04 - 6.97 (m, 2H), 6.92 (s, 1H), 6.72 (d, *J* = 8.5 Hz, 3H), 4.93 (dd, *J* = 12.1, 5.2 Hz, 1H), 4.88 (s, 1H), 4.23 (s, 1H), 2.93 - 2.76 (m, 3H), 2.73 - 2.68 (m, 2H), 2.43 (s, 2H), 2.16 - 2.07 (m, 1H), 1.69 (s, 6H). LC/MS (ESI+) calcd for C₃₉H₃₄ClN₇O₆ ([M+H]⁺) *m*/*z:* 732.2; found 732.2.

### Example 516: 2-(2,6-dioxopiperidin-3-yl)-5-((1r,3r)-3-(4-(2-(4-((5-fluoro-2-(2H-1,2,3-triazol-2-yl)pyridin-3-yl)oxy)phenyl)propan-2-yl)phenoxy)cyclobutyl)amino) isoindolin-1,3-dione

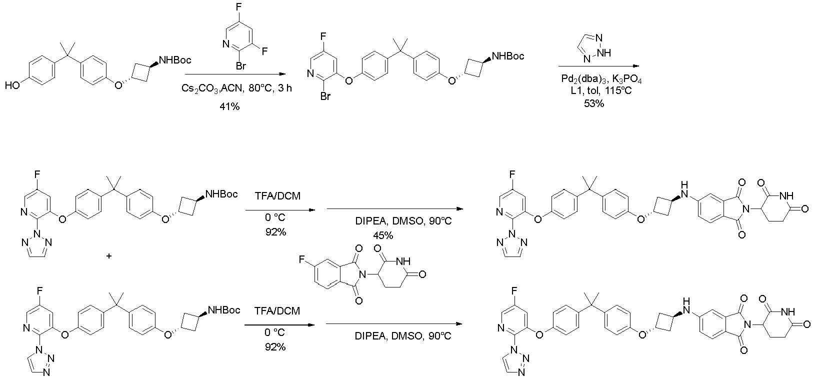

### Step 1: Synthesis of tert-butyl ((1r,3r)-3-(4-(2-(4-((2-bromo-5-fluoropyridin-3-yl)oxy)phenyl)propan-2-yl)phenoxy)cyclobutyl)carbamate

tert-butyl ((1r,3r)-3-(4-(2-(4-hydroxylphenyl)propan-2-phenoxy)cyclobutyl) carbamate (1.70g, 4.29 mmol) and 2-bromo-3,5-difluoropyridine (1.00 g, 5.15 mmol) were dissolved in 15 mL of acetonitrile, to which was added Cs₂CO₃(2.80 g, 8.58 mmol), and then the mixture was allowed to react overnight at 80 °C. The reaction was quenched by adding water. The resultant solution was extracted with ethyl acetate. The combined organic layer was washed with saturated brine, dried over anhydrous Na₂SO₄, and concentrated. The residue was separated and purified by prep-TLC, to provide compound tert-butyl ((1r,3r)-3-(4-(2-(4-((2-bromo-5-fluoropyridin-3-yl)oxy)phenyl) propan-2-yl)phenoxy)cyclobutyl)carbamate (1.00 g), with a yield of 41%. LC/MS (ESI+) calcd for C₂₉H₃₂BrFN₂O₄ ( [M+H]⁺ ) *m*/*z* 571.5; found 516.0.

### Step 2: Synthesis of tert-butyl ((1r,3r)-3-(4-(2-(4-((5-fluoro-2-(2H-1,2,3-triazol-2-yl)pyridin-3-yl)oxy)phenyl)propan-2-yl)phenoxy)cyclobutyl)carbamate (150 mg) and tert-butyl ((1r,3r)-3-(4-(2-(4-((5-fluoro-2-(1H-1,2,3 -triazol-1 -yl)pyri din-3 -yl)oxy) phenyl)propan-2-yl)phenoxy)cyclobutyl)carbamate

Pd₂(dba)₃ (128 mg, 0.14 mmol) and Me₄tBuXPhos (**L1**, 67 mg, 0.28 mmol) were added into 4 mL of toluene, and then reacted at 120 °C for 10 min under Ar gas protection. The reaction solution was cooled to room temperature, for future use.

tert-butyl ((1r,3r)-3-(4-(2-(4-((2-bromo-5-fluoropyridin-3-yl)oxy)phenyl)propan-2-yl)phenoxy)cyclobutyl)carbamate (800 mg, 1.40 mmol), 2-*H*-1,2,3-triazole (97 mg, 1.40 mmol), and K₃PO₄ (594 mg, 2.80 mmol) were added to another single-necked flask, to which were added the above reaction solution and 4 mL of toluene under Ar gas protection, and then the mixture was allowed to react overnight at 120 °C. TLC indicated disappearance of starting materials. Water was added to quench the reaction. The resultant solution was extracted with ethyl acetate. The organic phase was combined, washed with saturated brine, dried over anhydrous Na₂SO₄, and concentrated to dry under reduced pressure. The residue was separated and purified by prep-TLC, to provide tert-butyl ((1r,3r)-3-(4-(2-(4-((5-fluoro-2-(2H-1,2,3-triazol-2-yl)pyridin-3-yl)oxy)phenyl)propan-2-yl)phenoxy)cyclobutyl)carbamate (150 mg) and the isomer tert-butyl ((1r,3r)-3-(4-(2-(4-((5-fluoro-2-(1H-1,2,3-triazol-1-yl)pyridin-3-yl)oxy)phenyl)propan-2-yl)phenoxy)cyclobutyl)carbamate (270 mg), with a total yield of 53%. LC/MS (ESI+) calcd for C₃₁H₃₄FN₅O₄ ( [M+H]⁺ ) *m*/*z* 560.3; found 560.3.

### Step 3: Synthesis of 2-(2,6-dioxopiperidin-3-yl)-5-((1r,3r)-3-(4-(2-(4-((5-fluoro-2-(2H-1,2,3-triazol-2-yl)pyridin-3-yl)oxy)phenyl)propan-2-yl)phenoxy)cyclobutyl)amino) isoindol-1,3-dione

tert-butyl ((1r,3r)-3-(4-(2-(4-((5-fluoro-2-(2H-1,2,3-triazol-2-yl)pyridin-3-yl)oxy) phenyl)propan-2-yl)phenoxy)cyclobutyl)carbamate (150 mg, 0.27 mmol) was dissolved in 4 mL of dichloromethane, to which was added 1.5 mL of TFA in an ice water bath, and then the reaction was still stirred in the ice water bath for 1 h. The pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide (1r,3r)-3-(4-(2-(4-((5-fluoro-2-(2H-1,2,3-triazol-2-yl)pyridin-3-yl)oxy)phenyl)propan-2-yl)phenoxy)cyclobutylamine(115 mg), with a yield of 92%.

((1r,3r)-3-(4-(2-(4-((5-fluoro-2-(2H-1,2,3-triazol-2-yl)pyridin-3-yl)oxy)phenyl) propan-2-yl)phenoxy)cyclobutylamine (115 mg, 0.25 mmol) was dissolved in 4 mL of DMSO, to which were added DIPEA (97 mg, 0.75 mmol) and 2-(2,6-dioxopiperidin-3-yl)-5-fluoroisoindolin-1,3-dione (76 mg, 0.28 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-((1r,3r)-3-(4-(2-(4-((5-fluoro-2-(2H-1,2,3-triazol-2-yl)pyridin-3-yl)oxy)phenyl)propan-2-yl)phenoxy)cyclobutyl)amino)isoindolin-1,3-dione (80 mg), with a yield of 45%. LC/MS (ESI+) calcd for C₃₉H₃₄FN₇O₆ ([M+H]⁺) *m*/*z:* 716.3; found 716.3.
¹H NMR (400 MHz, Chloroform-*d*) *δ* 8.17 (s, 1H), 7.89 (s, 2H), 7.61 (d, *J* = 8.2 Hz, 1H), 7.26 - 7.20 (m, 2H), 7.17 - 7.08 (m, 3H), 7.00 - 6.94 (m, 2H), 6.87 (d, *J* = 2.0 Hz, 1H), 6.70 (dd, *J* = 8.4, 6.1 Hz, 3H), 4.93 (dd, *J* = 12.1, 5.2 Hz, 1H), 4.89 - 4.84 (m, 1H), 4.22 (t, *J* = 5.8 Hz, 1H), 2.84 (dtd, *J* = 27.7, 15.3, 14.5, 4.0 Hz, 3H), 2.69 (dt, *J* = 7.9, 4.5 Hz, 2H), 2.40 (dt, *J* = 12.7, 5.8 Hz, 2H), 2.19 - 2.08 (m, 1H), 1.65 (s, 6H).

### Example 517: 2-(2,6-dioxopiperidin-3-yl)-5-((1r,3r)-3-(4-(2-(4-((5-fluoro-2-(1H-1,2,3-triazol-1-yl)pyridin-3-yl)oxy)phenyl)propan-2-yl)phenoxy)cyclobutyl) amino)isoindolin-1,3-dione

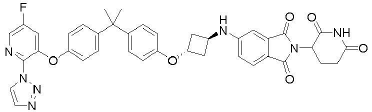

The target compound was synthesized by a method similar to that of Example 506. ¹H NMR (400 MHz, Chloroform-*d*) *δ* 8.27 (s, 1H), 8.23 (d, *J* = 1.1 Hz, 1H), 8.20 (d, *J* = 2.5 Hz, 1H), 7.83 (d, *J* = 1.2 Hz, 1H), 7.59 (d, *J* = 8.2 Hz, 1H), 7.28 - 7.25 (m, 2H), 7.15 (dd, *J* = 8.6, 2.4 Hz, 1H), 7.14 - 7.06 (m, 2H), 7.00 - 6.94 (m, 2H), 6.86 (d, *J* = 2.0 Hz, 1H), 6.74 - 6.70 (m, 2H), 6.70 - 6.64 (m, 1H), 4.93 (dd, *J* = 12.0, 5.2 Hz, 1H), 4.86 (dq, *J* = 7.0, 4.2, 3.4 Hz, 1H), 4.20 (td, *J* = 7.7, 3.9 Hz, 1H), 2.90 - 2.72 (m, 3H), 2.68 (td, *J* = 8.1, 7.6, 3.8 Hz, 2H), 2.41 (dt, *J* = 12.9, 6.2 Hz, 2H), 2.15 - 2.08 (m, 1H), 1.66 (s, 6H).
LC/MS (ESI+) calcd for C₃₉H₃₄FN₇O₆ ([M+H]⁺) *m*/*z*:716.3; found 716.3.

### Example 518: 5-((1r,3r)-3-(4-(1-(4-((2-(2H-1,2,3-triazol-2-yl)pyrimidin-5-yl)oxy) phenyl)ethyl)phenoxy)cyclobutyl)amino)-2-(2,6-dioxopiperidin-3-yl)isoindolin-1,3-dione

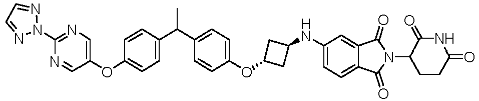

The target compound was synthesized by a method similar to that of Example 506. ¹H NMR (400 MHz, Chloroform-*d*) *δ* 8.54 (d, *J* = 13.6 Hz, 2H), 8.10 (s, 1H), 7.97 (s, 2H), 7.62 (d, *J* = 8.3 Hz, 1H), 7.25 (d, *J* = 2.2 Hz, 2H), 7.13 (dd, *J* = 8.9, 2.9 Hz, 2H), 7.01 (dq, *J* = 9.4, 2.9, 2.3 Hz, 2H), 6.89 (d, *J* = 2.1 Hz, 1H), 6.77 - 6.72 (m, 2H), 6.70 (dd, *J* = 8.3, 2.1 Hz, 1H), 4.93 (dd, *J* = 12.2, 5.2 Hz, 1H), 4.88 (d, *J* = 5.8 Hz, 1H), 4.22 (td, *J* = 7.7, 3.8 Hz, 1H), 4.12 (tt, *J* = 7.1, 3.5 Hz, 1H), 2.94 - 2.73 (m, 3H), 2.73 - 2.63 (m, 2H), 2.41 (dt, *J* = 13.0, 6.4 Hz, 2H), 2.12 (ddd, *J* = 10.3, 4.9, 2.5 Hz, 1H), 1.63 (d, *J* = 7.2 Hz, 3H). LC/MS (ESI+) calcd for C₃₇H₃₂N₈O₆ ([M+H]⁺) *m*/*z:* 685.2; found 685.0.

### Example 519: 5-((1r,3r)-3-(4-(1-(4-((2-(1H-1,2,3-triazol-1-yl)pyrimidin-5-yl)oxy) phenyl)ethyl)phenoxy)cyclobutyl)amino)-2-(2,6-dioxopiperidin-3-yl)isoindolin-1,3-dione

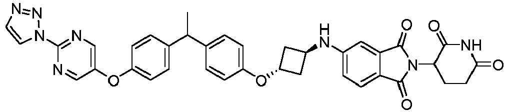

The target compound was synthesized by a method similar to that of Example 506. ¹H NMR (400 MHz, Chloroform-*d*) *δ* 8.61 - 8.46 (m, 3H), 8.16 (s, 1H), 7.85 (d, *J* = 1.2 Hz, 1H), 7.61 (d, *J* = 8.3 Hz, 1H), 7.28 (d, *J* = 2.0 Hz, 2H), 7.17 - 7.10 (m, 2H), 7.06 - 6.99 (m, 2H), 6.88 (d, *J* = 2.0 Hz, 1H), 6.80 - 6.72 (m, 2H), 6.70 (dd, *J* = 8.3, 2.1 Hz, 1H), 4.93 (dd, *J* = 12.1, 5.2 Hz, 1H), 4.88 (dt, *J* = 9.3, 4.7 Hz, 1H), 4.21 (td, *J* = 7.8, 4.0 Hz, 1H), 4.14 (q, *J* = 7.1 Hz, 1H), 2.93 - 2.73 (m, 3H), 2.72 - 2.65 (m, 2H), 2.42 (dt, *J* = 12.8, 6.1 Hz, 2H), 2.12 (ddd, *J* = 10.0, 4.7, 2.4 Hz, 1H), 1.63 (d, *J* = 7.2 Hz, 3H). LC/MS (ESI+) calcd for C₃₇H₃₂N₈O₆ ([M+H]⁺) *m*/*z:* 685.2; found 685.0.

### Example 520: 5-((1r,3r)-3-(4-(4-(2-(2H-1,2,3-triazol-2-yl)pyrimidin-5-yl)oxy) benzyl)phenoxy)cyclobutyl)amino)-2-(2,6-dioxopiperidin-3-yl)isoindolin-1,3-dione

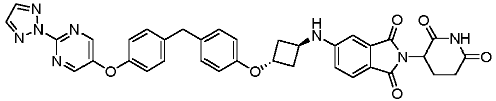

The target compound was synthesized by a method similar to that of Example 506. ¹H NMR (400 MHz, Chloroform-*d*) *δ* 8.55 (d, *J* = 13.8 Hz, 2H), 8.08 (s, 1H), 7.97 (s, 2H), 7.63 (d, *J* = 8.3 Hz, 1H), 7.26 - 7.19 (m, 2H), 7.15 - 7.07 (m, 2H), 7.05 - 6.98 (m, 2H), 6.89 (d, *J* = 2.0 Hz, 1H), 6.78 - 6.72 (m, 2H), 6.70 (dd, *J* = 8.2, 2.0 Hz, 1H), 4.93 (dd, *J* = 12.2, 5.3 Hz, 1H), 4.88 (s, 1H), 4.30 - 4.19 (m, 1H), 3.95 (s, 2H), 2.94 - 2.73 (m, 3H), 2.71 (td, *J* = 7.0, 6.5, 2.8 Hz, 2H), 2.41 (dt, *J* = 13.0, 6.2 Hz, 2H), 2.15 - 2.09 (m, 1H).
LC/MS (ESI+) calcd for C₃₆H₃₀N₈O₆ ([M+H]⁺) *m*/*z:* 671.2; found 671.0.

### Example 521: 5-((1r,3r)-3-(4-(4-(2-(1H-1,2,3-triazol-1-yl)pyrimidin-5-yl)oxy) benzyl)phenoxy)cyclobutyl)amino)-2-(2,6-dioxopiperidin-3-yl)isoindolin-1,3-dione

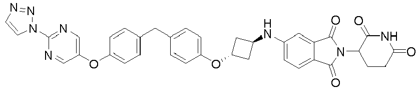

The target compound was synthesized by a method similar to that of Example 506. ¹H NMR (400 MHz, Chloroform-*d*) *δ* 8.61 - 8.44 (m, 3H), 8.10 (s, 1H), 7.85 (d, *J* = 1.2 Hz, 1H), 7.62 (d, *J* = 8.3 Hz, 1H), 7.26 - 7.21 (m, 2H), 7.14 - 7.07 (m, 2H), 7.06 - 6.99 (m, 2H), 6.89 (d, *J* = 2.1 Hz, 1H), 6.79 - 6.73 (m, 2H), 6.71 (dd, *J* = 8.3, 2.1 Hz, 1H), 4.93 (dd, *J* = 12.2, 5.2 Hz, 1H), 4.88 (t, *J* = 4.2 Hz, 1H), 4.29 - 4.18 (m, 1H), 3.95 (s, 2H), 2.96 - 2.73 (m, 3H), 2.73 - 2.66 (m, 2H), 2.42 (dt, *J* = 12.9, 6.2 Hz, 2H), 2.15 - 2.08 (m, 1H). LC/MS (ESI+) calcd for C₃₆H₃₀N₈O₆ ([M+H]⁺) *m*/*z:* 671.2; found 671.0.

### Example 522: 2-(2,6-dioxopiperidin-3-yl)-5-((1s,3s)-3-(4-(2-(4-((6-(1-hydroxylethyl)pyridazine-3-yl)oxy)phenyl)propan-2-yl)phenoxy)cyclobutyl) amino)isoindolin-1,3-dione

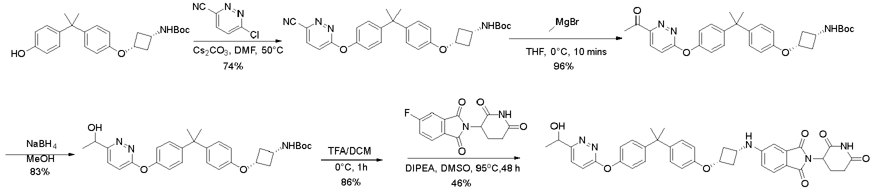

### Step 1: Synthesis of tert-butyl ((1s,3s)-3-(4-(2-(4-((6-cyanopyridin-3-yl)oxy)phenyl) propan-2-yl)phenoxy)cyclobutyl)carbamate

tert-butyl ((1s,3s)-3-(4-(2-(4-hydroxylphenyl)propan-2-phenoxy)cyclobutyl) carbamate (1.00 g, 2.52 mmol) and 3-cyano-6-chloropyridazine (350 mg, 2.52 mmol) were dissolved in 5 mL of DMF, to which was added Cs₂CO₃ (1.64 g, 5.04 mmol), and then the mixture was allowed to react overnight at 50 °C. TLC indicated disappearance of starting materials, and the reaction was quenched by adding water. The resultant solution was extracted with ethyl acetate. The combined organic layer was washed with saturated brine, dried over anhydrous Na₂SO₄, and concentrated to dry under reduced pressure. The residue was separated and purified by prep-TLC, to provide the product tert-butyl ((1s,3s)-3-(4-(2-(4-((6-cyanopyridin-3-yl)oxy)phenyl)propan-2-yl)phenoxy) cyclobutyl)carbamate (930 mg), with a yield of 74%. LC/MS (ESI+) calcd for C₂₉H₃₂N₄O₄ ( [M+H]⁺ ) *m*/*z* 501.2; found 401.1.

### Step 2: Synthesis of tert-butyl ((1s,3s)-3-(4-(2-(4-((6-acetylpyridazine-3-yl)oxy)phenyl) propan-2-yl)phenoxy)cyclobutyl)carbamate

tert-butyl ((1s,3s)-3-(4-(2-(4-((6-cyanopyridin-3-yl)oxy)phenyl)propan-2-yl) phenoxy)cyclobutyl)carbamate (600 mg, 1.20 mmol) was added to a three-necked flask, to which was added 10 mL of dry THF under Ar gas protection. The reaction was transferred to an ice water bath, to which was added methylmagnesium bromide (3M, 3.6 mmol, 1.2 mL) dropwise at 0 °C, and then the mixture was allowed to react at 0 °C for 10 min. TLC indicated disappearance of starting materials. To the reaction solution, was added 0.5N HCl (20 mL) to quench the reaction. The resultant solution was extracted with ethyl acetate. The combined organic layer was washed with saturated brine, dried over anhydrous Na₂SO₄, and concentrated to dry under reduced pressure, to provide the product tert-butyl ((1s,3s)-3-(4-(2-(4-((6-acetylpyridazine-3-yl)oxy) phenyl)propan-2-yl)phenoxy)cyclobutyl)carbamate (600 mg), with a yield of 96%. LC/MS (ESI+) calcd for C₃₀H₃₅N₃O₅ ( [M+H]⁺ ) *m*/*z* 518.3; found 418.2.

### Step 3: Synthesis of tert-butyl ((1s,3s)-3-(4-(2-(4-(6-(1-hydroxylethyl)pyridazine-3-yl)oxy)phenyl)propan-2-yl)phenoxy)cyclobutyl)carbamate

tert-butyl ((1s,3s)-3-(4-(2-(4-((6-acetylpyridazine-3-yl)oxy)phenyl)propan-2-yl)phenoxy)cyclobutyl)carbamate (600 mg, 1.16 mmol) was dissolved in 10 mL of methanol, to which was added NaBH₄ (132 mg, 3.48 mmol) dropwise at 0°C, and then the mixture was allowed to react at 0 °C for 30 min. TLC indicated disappearance of starting materials. The reaction solution was slowly poured to a suitable amount of ice water, to quench the reaction. The resultant solution was extracted with ethyl acetate. The combined organic phase was washed with saturated brine, dried over anhydrous Na₂SO₄, and concentrated to dry under reduced pressure, to provide the product tert-butyl ((1s,3s)-3-(4-(2-(4-(6-(1-hydroxylethyl)pyridazine-3-yl)oxy)phenyl)propan-2-yl)phenoxy)cyclobutyl)carbamate (500 mg), with a yield of 83%. LC/MS (ESI+) calcd for C₃₀H₃₇N₃O₅ ( [M+H]⁺ ) *m*/*z* 520.3; found 520.2.

### Step 4: Synthesis of 2-(2,6-dioxopiperidin-3-yl)-5-((1s,3s)-3-(4-(2-(4-((6-(1-hydroxylethyl)pyridazine-3-yl)oxy)phenyl)propan-2-yl)phenoxy)cyclobutyl)amino) isoindol-1,3-dione

tert-butyl ((1s,3s)-3-(4-(2-(4-(6-(1-hydroxylethyl)pyridazine-3 -yl)oxy)phenyl) propan-2-yl)phenoxy)cyclobutyl)carbamate (500 mg, 0.96 mmol) was dissolved in 10 mL of dichloromethane, to which was added 3 mL of TFA in an ice water bath, and then the reaction was allowed to react in the ice water bath for 1 h. The pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide 1-(6-(4-(2-(4-(1s,3s)-3-aminocyclobutyloxy)phenyl)propan-2-yl)phenoxy) pyridazine-3-yl)ethane-1-ol (350 mg), with a yield of 86%.

1-(6-(4-(2-(4-(1s,3s)-3-aminocyclobutyloxy)phenyl)propan-2-yl)phenoxy) pyridazine-3-yl)ethane-1-ol (350 mg, 0.83 mmol) was dissolved in 15 mL of DMSO, to which were added DIPEA (323 mg, 2.50 mmol) and 2-(2,6-dioxopiperidin-3-yl)-5-fluoroisoindolin-1,3-dione (252 mg, 0.91 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-((1s,3s)-3-(4-(2-(4-((6-(1-hydroxylethyl)pyridazine-3-yl)oxy) phenyl)propan-2-yl)phenoxy)cyclobutyl)amino)isoindolin-1,3-dione (260 mg), with a yield of 46%. ¹H NMR (400 MHz, Chloroform-*d*) *δ* 8.22 (s, 1H), 7.60 (d, *J* = 8.3 Hz, 1H), 7.56 (d, *J* = 9.1 Hz, 1H), 7.26 (d, *J* = 7.3 Hz, 2H), 7.20 (d, *J* = 9.1 Hz, 1H), 7.18 - 7.13 (m, 2H), 7.11 - 7.05 (m, 2H), 6.91 (d, *J* = 2.1 Hz, 1H), 6.77 - 6.67 (m, 3H), 5.08 (q, *J* = 6.6 Hz, 1H), 4.96 - 4.89 (m, 1H), 4.51 (p, *J* = 6.8 Hz, 1H), 3.76 (p, *J* = 7.6 Hz, 1H), 3.11 (qd, *J* = 8.2, 6.7, 4.6 Hz, 2H), 2.88 - 2.69 (m, 3H), 2.10 (ddt, *J* = 10.0, 7.2, 4.0 Hz, 3H), 1.66 (s, 6H), 1.56 (d, *J* = 6.5 Hz, 3H). LC/MS (ESI+) calcd for C₃₈H₃₇N₅O₇ ([M+H]⁺) *m*/*z:* 676.3; found 676.3.

### Example 523: 5-((1s,3s)-3-(4-(2-(4-((6-acetylpyridazine-3-yl)oxy)phenyl)propan-2-yl)phenoxy)cyclobutyl)amino)-2-(2,6-dioxopiperidin-3-yl)isoindolin-1,3-dione

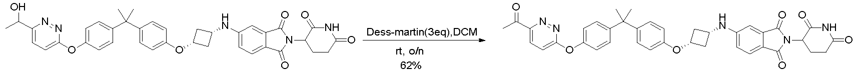

2-(2,6-dioxopiperidin-3-yl)-5-((1s,3s)-3-(4-(2-(4-((6-(1-hydroxylethyl) pyridazine-3-yl)oxy)phenyl)propan-2-yl)phenoxy)cyclobutyl)amino)isoindol-1,3-dione (70 mg, 0.10 mmol) was dissolved in 5 mL of DCM, to which was added Dess-Martin (75 mg, 0.18 mmol) at 0 °C, and then the mixture was allowed to react overnight at room temperature. After completion of the reaction, the reaction solution was filtered to remove the solid, and the filtrate was concentrated by rotatory evaporation. The residue was separated and purified by prep-TLC, to provide 5-((1s,3s)-3-(4-(2-(4-((6-acetylpyridazine-3-yl)oxy)phenyl)propan-2-yl)phenoxy)cyclobutyl)amino)-2-(2,6-dioxopiperidin-3-yl)isoindolin-1,3-dione (43 mg), with a yield of 62%. ¹H NMR (400 MHz, Chloroform-*d*) *δ* 8.15 (d, *J* = 9.1 Hz, 1H), 8.05 (s, 1H), 7.62 (d, *J* = 8.3 Hz, 1H), 7.30 (d, *J* = 2.0 Hz, 2H), 7.25 (s, 1H), 7.21 - 7.15 (m, 2H), 7.14 - 7.08 (m, 2H), 6.92 (d, *J=* 2.1 Hz, 1H), 6.73 (td, *J* = 8.2, 7.4, 2.1 Hz, 3H), 4.98 - 4.89 (m, 1H), 4.52 (p, *J* = 6.9 Hz, 1H), 3.78 (p, *J* = 7.6 Hz, 1H), 3.13 (td, *J* = 9.9, 6.9 Hz, 2H), 2.91 - 2.70 (m, 6H), 2.14 - 2.03 (m, 3H), 1.68 (s, 6H). LC/MS (ESI+) calcd for C₃₈H₃₅N₅O₇ ([M+H]⁺) *m*/*z*:674.2; found 674.2.

### Example 524: 2-(2,6-dioxopiperidin-3-yl)-5-((1s,3s)-3-(4-(2-(4-((6-(1-hydroxylethyl)pyridin-3-yl)oxy)phenyl)propan-2-yl)phenoxy)cyclobutyl)amino) isoindolin-1,3-dione

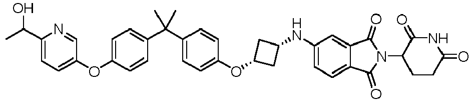

The target compound was synthesized by a method similar to that of Example 522. ¹H NMR (400 MHz, Chloroform-*d*) *δ* 8.31 (s, 1H), 8.29 (d, *J* = 2.7 Hz, 1H), 7.61 (d, *J* = 8.2 Hz, 1H), 7.35 (dd, *J* = 8.5, 2.7 Hz, 1H), 7.28 (s, 1H), 7.23 - 7.18 (m, 2H), 7.17 - 7.09 (m, 2H), 6.94 - 6.86 (m, 3H), 6.78 - 6.66 (m, 3H), 4.97 - 4.87 (m, 2H), 4.84 (d, *J* = 6.2 Hz, 1H), 4.51 (p, *J* = 6.9 Hz, 1H), 3.76 (h, *J* = 7.5 Hz, 1H), 3.18 - 3.06 (m, 2H), 2.92 - 2.68 (m, 3H), 2.15 - 2.06 (m, 3H), 1.66 (s, 6H), 1.51 (d, *J* = 6.5 Hz, 3H). LC/MS (ESI+) calcd for C₃₉H₃₈N₄O₇ ([M+H]⁺) *m*/*z*:675.3; found 675.3.

### Example 525: 5-((1s,3s)-3-(4-(2-(4-((6-acetylpyridin-3-yl)oxy)phenyl)propan-2-yl)phenoxy)cyclobutyl)amino)-2-(2,6-dioxopyridin-3-yl)isoindolin-1,3-dione

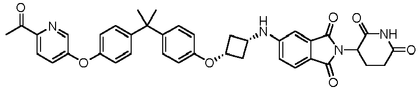

The target compound was synthesized by a method similar to that of Example 523. ¹H NMR (400 MHz, Chloroform-*d*) δ 8.37 (d, *J* = 2.6 Hz, 1H), 8.11 (s, 1H), 8.03 (d, *J* = 8.6 Hz, 1H), 7.62 (d, *J* = 8.2 Hz, 1H), 7.32 (dd, *J* = 8.7, 2.6 Hz, 1H), 7.26 (d, *J* = 8.4 Hz, 2H), 7.18 - 7.12 (m, 2H), 7.01 - 6.94 (m, 2H), 6.91 (d, *J* = 2.1 Hz, 1H), 6.79 - 6.68 (m, 3H), 4.98 - 4.88 (m, 1H), 4.52 (p, *J* = 6.9 Hz, 1H), 3.78 (p, *J* = 7.6 Hz, 1H), 3.14 (ddt, *J* = 12.8, 9.3, 4.5 Hz, 2H), 2.93 - 2.72 (m, 3H), 2.70 (s, 3H), 2.11 (dtd, *J* = 10.2, 5.0, 2.3 Hz, 3H), 1.67 (s, 6H).LC/MS (ESI+) calcd for C₃₉H₃₆N₄O₇([M+H]⁺) *m*/*z*:673.3; found 673.2.

### Example 526: 2-(2,6-dioxopiperidin-3-yl)-5-(1s,3s)-3-(4-(3-(4-(6-(5-methyl-1,2,4-oxadiazol-3-yl)pyridazine-3-yl)oxy)phenyl)pentan-3-yl)phenoxy)cyclobutyl) amino)isoindolin-1,3-dione

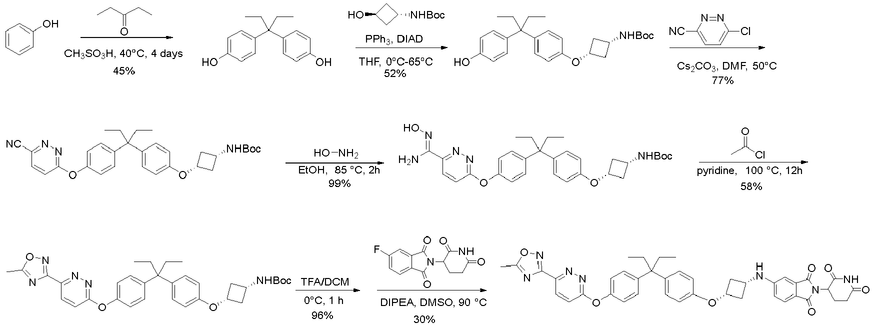

### Step 1: Synthesis of 4,4'-(pentan-3,3-diyl)diphenol

Phenol (40.0 g, 420 mmol) and 3-pentanone (11.4 g, 130 mmol) were added into a three-necked flask, to which was added methanesulfonic acid (15.4 g, 160 mmol) at 0 °C, and then the solution was slowly heated to 40 °C and reacted for 4 days. The reaction was detected by TLC. To the reaction solution, was added water to quench the reaction. The resultant solution was extracted with ethyl acetate. The combined organic phase was washed with saturated brine, dried over anhydrous Na₂SO₄, and concentrated to dry under reduced pressure, to provide the crude product, which was triturated, to obtain the target product 4,4'-(pentan-3,3-diyl)diphenol (15.0 g), with a yield of 45%.

### Step 2: Synthesis of tert-butyl ((1s,3s)-3-(4-(3-(4-hydroxylphenyl)pentan-3-yl)phenoxy)cyclobutyl)carbamate

*N*-Boc-trans-3-aminocyclobutanol (3.1 g, 16 mmol), 4,4'-(pentan-3,3-diyl) diphenol (5.0 g, 19 mmol), and PPh₃(4.5 g, 17 mmol) were added into a three-necked flask under Ar gas protection, to which was added 30 mL of dry THF, and then the reaction solution was stirred to dissolve. To the reaction system, was slowly added DIAD (3.5 g, 17 mmol) dropwise in an ice bath, and then the mixture was allowed to react for additional 1 h in the ice bath and then react overnight at 65 °C. The reaction solution was cooled to room temperature, followed by addition of anhydrous ZiCl₂ (2.1 g, 15 mmol), and then the solution was stirred for 1 h. The reaction solution was filtered to remove the solid. A suitable amount of water was added to the filtrate. The resultant solution was extracted with ethyl acetate.The organic phase was washed with saturated brine, dried over anhydrous Na₂SO₄, and concentrated to dry under reduced pressure. The residue was separated and purified by column chromatography, to provide compound tert-butyl ((1s,3s)-3-(4-(3-(4-hydroxylphenyl)pentan-3-yl)phenoxy) cyclobutyl)carbamate (3.6 g), with a yield of 52%. LC/MS (ESI+) calcd for C₂₆H₃₅NO₄( [M]⁺ ) *m*/*z:* 425.3; found 424.3.

### Step 3: Synthesis of tert-butyl ((1s,3s)-3-(4-(3-(4-((6-cyanopyridin-3-yl)oxy)phenyl) pentan-3-yl)phenoxy)cyclobutyl)carbamate

tert-butyl ((1s,3s)-3-(4-(3-(4-hydroxylphenyl)pentan-3-yl)phenoxy)cyclobutyl) carbamate (1.00 g, 2.4 mmol) and 3-cyano-6-chloropyridazine (359 mg, 2.6 mmol) were dissolved in 10 mL of DMF, to which was added Cs₂CO₃(1.5 g, 4.7 mmol), and then the mixture was allowed to react overnight at 50 °C. TLC indicated disappearance of starting materials. Water was added to quench the reaction. The resultant solution was extracted with ethyl acetate. The organic phase was combined, washed with saturated brine, dried over anhydrous Na₂SO₄, and concentrated to dry under reduced pressure. The residue was separated and purified by prep-TLC, to provide the product tert-butyl ((1s,3s)-3-(4-(3-(4-((6-cyanopyridin-3-yl)oxy)phenyl)pentan-3-yl)phenoxy) cyclobutyl)carbamate (950 mg), with a yield of 77%. LC/MS (ESI+) calcd for C₃₁H₃₆N₄O₄ ( [M+H]⁺ ) *m*/*z* 529.3; found 429.2.

### Step 4: Synthesis of tert-butyl ((1s,3s)-3-(4-(3-(4-((6-((Z)-N'-hydroxylcarbamoyl) pyridazine-3-yl)oxy)phenyl)pentan-3-yl)phenoxy)cyclobutyl)carbamate

tert-butyl ((1s,3s)-3-(4-(3 -(4-((6-cyanopyridin-3 -yl)oxy)phenyl)pentan-3 -yl) phenoxy)cyclobutyl)carbamate (300 mg, 0.57 mmol) was dissolved in 10 mL of ethanol, to which was added 50% hydroxylamine aqueous solution (56 mg, 0.85 mmol), and then the mixture was allowed to react under refluxing for 1 h. A small amount of ethanol was removed by rotatory evaporation. Water was added to quench the reaction. The resultant solution was extracted with ethyl acetate. The organic phase was combined, washed with saturated brine, dried over anhydrous Na₂SO₄, and concentrated by rotatory evaporation, to provide the white solid product tert-butyl ((1s,3s)-3-(4-(3-(4-((6-((Z)-N'-hydroxylcarbamoyl)pyridazine-3-yl)oxy)phenyl)pentan-3-yl)phenoxy) cyclobutyl)carbamate(350 mg), which was directly used in the next step.

### Step 5: Synthesis of tert-butyl ((1s,3s)-3-(4-(3-(4-((6-(5-methyl-1,2,4-oxadiazol-3-yl)pyridazine-3-yl)oxy)phenyl)pentan-3-yl)phenoxy)cyclobutyl)carbamate

The product in the previous step was dissolved in 5 mL of pyridine, and then cooled in an ice water bath, to which was added the solution of acetyl chloride (78 mg, 1.00 mmol) in 1 mL toluene, and then the solution was stirred for 10 min. The ice water bath was removed, and the reaction was heated to 100 °C and allowed to react overnight, followed by concentration to dry under reduced pressure. Water was added to quench the reaction. The resultant solution was extracted with ethyl acetate. The organic phase was combined, washed with saturated brine, dried over anhydrous Na₂SO₄, and concentrated by rotatory evaporation. The residue was separated and purified by TLC, to provide the product tert-butyl ((1s,3s)-3-(4-(3-(4-((6-(5-methyl-1,2,4-oxadiazol-3-yl)pyridazine-3-yl)oxy)phenyl)pentan-3-yl)phenoxy)cyclobutyl)carbamate (210 mg), with a yield of 58%, LC/MS (ESI+) calcd for C₃₃H₃₉N₅O₅ ([M+H]⁺) *m*/*z:* 586.3; found 486.2.

### Step 6: Synthesis of 2-(2,6-dioxopiperidin-3-yl)-5-(1s,3s)-3-(4-(3-(4-(6-(5-methyl-1,2,4-oxadiazol-3-yl)pyridazine-3-yl)oxy)phenyl)pentan-3-yl)phenoxy)cyclobutyl) amino)isoindolin-1,3-dione

tert-butyl ((1s,3s)-3-(4-(3-(4-((6-(5-methyl-1,2,4-oxadiazol-3-yl)pyridazine-3-yl) oxy)phenyl)pentan-3-yl)phenoxy)cyclobutyl)carbamate (160 mg, 0.27mmol) was dissolved in 8 mL of dichloromethane, to which was added 2 mL of TFA in an ice water bath, and then the reaction was allowed to react in the ice water bath for 1 h. The pH of the reaction solution was adjusted to alkaline with saturated Na₂CO₃ aqueous solution. The resultant solution was extracted with dichloromethane/methanol (10:1). The organic phase was dried over anhydrous MgSO₄, and concentrated to dry under reduced pressure, to provide the crude compound (1s,3s)-3-(4-(3-(4-((6-(5-methyl-1,2,4-oxadiazol-3-yl)pyridazine-3-yl)oxy)phenyl)pentan-3-yl)phenoxy)cyclobutylamine, which was directly used in the next step.

The above product was dissolved in 5 mL of DMSO, to which were added DIPEA(104 mg, 0.81 mmol) and 2-(2,6-dioxopiperidin-3-yl)-5-fluoroisoindolin-1,3-dione (83 mg, 0.30 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-5-(1s,3s)-3-(4-(3-(4-(6-(5-methyl-1,2,4-oxadiazol-3-yl)pyridazine-3-yl)oxy)phenyl) pentan-3-yl)phenoxy)cyclobutyl)amino)isoindolin-1,3-dione (60 mg), with a yield of 30%. ¹H NMR (400 MHz, DMSO-*d*₆) *δ* 11.07 (s, 1H), 8.28 (d, *J* = 9.2 Hz, 1H), 7.59 (dd, *J* = 8.8, 4.2 Hz, 2H), 7.46 (d, *J* = 6.5 Hz, 1H), 7.31 - 7.15 (m, 4H), 7.14 - 7.04 (m, 2H), 6.90 (d, *J* = 2.0 Hz, 1H), 6.81 (dd, *J* = 8.5, 6.4 Hz, 3H), 5.04 (dd, *J* = 12.9, 5.4 Hz, 1H), 4.51 (p, *J* = 7.1 Hz, 1H), 3.78 (q, *J* = 7.3 Hz, 1H), 3.13 - 2.99 (m, 2H), 2.95 - 2.80 (m, 1H), 2.71 (s, 3H), 2.63 - 2.51 (m, 2H), 2.16 - 1.88 (m, 7H), 0.59 (t, *J* = 7.2 Hz, 6H). LC/MS (ESI+) calcd for C₄₁H₃₉N₇O₇ ([M+H]⁺) *m*/*z:* 742.3; found 742.2.

### Example 527: 2-(2,6-dioxopiperidin-3-yl)-5-((1r,3r)-3-(4-(3-(4-((6-(5-methyl-1,2,4-oxadiazol-3-yl)pyridazine-3-yl)oxy)phenyl)pentan-3-yl)phenoxy)cyclobutyl) amino)isoindolin-1,3-dione

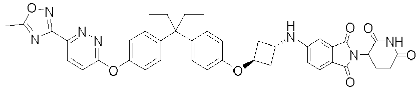

The target compound was synthesized by a method similar to that of Example 526. ¹H NMR (400 MHz, DMSO-*d*₆) *δ* 11.07 (s, 1H), 8.28 (d, *J* = 9.3 Hz, 1H), 7.59 (dd, *J* = 8.8, 3.1 Hz, 2H), 7.54 (d, *J* = 5.4 Hz, 1H), 7.27 - 7.15 (m, 4H), 7.14 - 7.03 (m, 2H), 6.86 (s, 1H), 6.78 (t, *J* = 8.6 Hz, 3H), 5.04 (dd, *J* = 12.9, 5.4 Hz, 1H), 4.87 (q, *J* = 5.9 Hz, 1H), 4.14 (d, *J* = 6.9 Hz, 1H), 2.94 - 2.81 (m, 1H), 2.71 (s, 3H), 2.57 (d, *J=* 21.5 Hz, 4H), 2.44 (q, *J=* 7.2 Hz, 2H), 2.13 - 1.95 (m, 5H), 0.59 (t, *J* = 7.1 Hz, 6H). LC/MS (ESI+) calcd for C₄₁H₃₉N₇O₇ ( [M+H]⁺) *m*/*z:* 742.3; found 742.2.

### Example 528: 2-(2,6-dioxopiperidin-3-yl)-5-((1r,3r)-3-(4-(1-(4-((2-(1-hydroxylethyl)pyrimidin-5-yl)oxy)phenyl)cyclohexyl)phenoxy)cyclobutyl) amino)isoindolin-1,3-dione

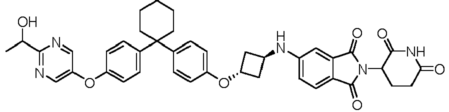

The target compound was synthesized by a method similar to that of Example 522. ¹H NMR (400 MHz, DMSO-*d*₆) *δ* 11.07 (s, 1H), 8.56 (s, 2H), 7.59 (d, *J* = 8.3 Hz, 1H), 7.53 (d, *J* = 5.3 Hz, 1H), 7.37 - 7.26 (m, 2H), 7.20 (d, *J* = 8.4 Hz, 2H), 7.00 (d, *J* = 8.7 Hz, 2H), 6.85 (s, 1H), 6.77 (dd, *J* = 15.8, 8.4 Hz, 3H), 5.24 (d, *J* = 5.5 Hz, 1H), 5.03 (dd, *J* = 12.9, 5.3 Hz, 1H), 4.83 (d, *J* = 12.9 Hz, 1H), 4.78 (q, *J* = 6.3 Hz, 1H), 4.13 (s, 1H), 2.86 (d, *J* = 12.7 Hz, 1H), 2.57 (d, *J* = 16.9 Hz, 4H), 2.42 (d, *J* = 5.9 Hz, 2H), 2.21 (s, 4H), 1.99 (d, *J* = 8.7 Hz, 1H), 1.54 - 1.35 (m, 9H). LC/MS (ESI+) calcd for C₄₁H₄₁N₅O₇ ([M+H]⁺) *m*/*z:* 716.3; found 716.2.

### Example 529: 5-((1r,3r)-3-(4-(1-(4-((2-acetylpyrimidin-5-yl)oxy)phenyl) cyclohexyl)phenoxy)cyclobutyl)amino)-2-(2,6-dioxopiperidin-3-yl)isoindolin-1,3-dione

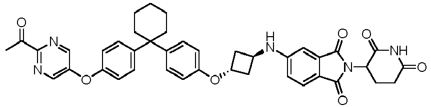

The target compound was synthesized by a method similar to that of Example 523. ¹H NMR (400 MHz, DMSO-*d*₆) *δ* 11.08 (s, 1H), 8.68 (s, 2H), 7.58 (d, *J* = 8.3 Hz, 1H), 7.54 (d, *J* = 5.4 Hz, 1H), 7.41 - 7.33 (m, 2H), 7.26 - 7.18 (m, 2H), 7.18 - 7.08 (m, 2H), 6.85 (s, 1H), 6.77 (t, *J* = 10.7 Hz, 3H), 5.04 (dd, *J* = 12.9, 5.4 Hz, 1H), 4.92 - 4.80 (m, 1H), 4.13 (d, *J* = 4.8 Hz, 1H), 2.93 - 2.79 (m, 1H), 2.64 (s, 3H), 2.61 - 2.50 (m, 4H), 2.42 (q, *J* = 6.9 Hz, 2H), 2.23 (s, 4H), 2.03 - 1.98 (m, 1H), 1.50 - 1.39 (m, 6H). LC/MS (ESI+) calcd for C₄₁H₃₉N₅O₇ ([M+H]⁺) *m*/*z*:714.3; found 714.3.

### Example 530: 2-(2,6-dioxopiperidin-3-yl)-6-((1r,3r)-3-(4-(2-(4-((2-(5-methyl-1,2,4-oxadiazol-3-yl)pyrimidin-5-yl)oxy)phenyl)propan-2-yl)phenoxy)cyclobutyl) amino)-1H-pyrrolo[3,4-c]pyridin-1,3(2H)-dione

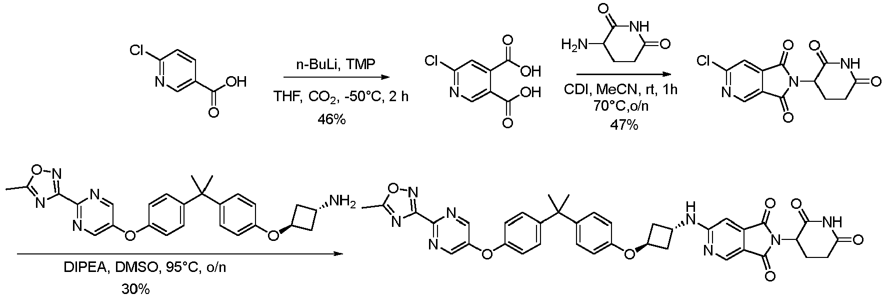

### Step 1: Synthesis of 6-chloropyridin-3,4-dicarboxylic acid

Under Ar gas protection, 6-chloronicotinic acid (5.0 g, 32 mmol) was dissolved in 150 mL of dry THF, to which was added TMP (13.4 g, 95 mmol), and then the mixture was stirred for 30 min at -50 °C, followed by addition of n-BuLi (1.6 M, 127 mmol, 192 mL) dropwise at -78 °C, and then the reaction solution was warmed to -50 °C and reacted for 2 h. After the starting material disappeared, the reaction solution was slowly poured to dry ice, and further stirred for 1 h. Subsequently, 2N HCl was added to adjust the solution to be neutral. The resultant solution was extracted with ethyl acetate. The organic phase was washed with saturated NaCl solution, dried over anhydrous Na₂SO₄, and concentrated by rotatory evaporation. The residue was separated and purified by column chromatography, to provide the target product 6-chloropyridin-3,4-dicarboxylic acid (3.0 g), with a yield of 46%. LC/MS (ESI+) calcd for C₇H₄ClNO₄ ([M+H]⁺) *m*/*z:* 202.0; found 202.0.

### Step 2: Synthesis of 6-chloro-2-(2,6-dioxopiperidin-3-yl)-1H-pyrrolo[3,4-c]pyridin-1,3(2H)-dione

6-chloropyridin-3,4-dicarboxylic acid (1.0 g, 5 mmol) was dissolved in 20 mL of acetonitrile, to which was added CDI (1.9 g, 11.5 mmol), and then the reaction solution was stirred at room temperature for 1 h, followed by addition of 3-aminopiperidin-2,6-dione (819 mg, 4.98 mmol). The reaction solution was slowly warmed to 70 °C, and then allowed to react overnight. After disappearance of starting materials, acetonitrile was removed by rotatory evaporation, and to the residue, was added 10 mL of water, followed by stirring for 20 min. The resultant solution was filtered to collect the white solid, which was separated and purified by column chromatography, to provide the target product 6-chloro-2-(2,6-dioxopiperidin-3-yl)-1H-pyrrolo[3,4-c]pyridin-1,3(2H)-dione (700 mg), with a yield of 47%. LC/MS (ESI+) calcd for C₁₂H₈ClN₃O₄ ([M+H]⁺) *m*/*z:* 294.0; found 294.0.

### Step 3: Synthesis of 2-(2,6-dioxopiperidin-3-yl)-6-((1r,3r)-3-(4-(2-(4-((2-(5-methyl-1,2,4-oxadiazol-3-yl)pyrimidin-5-yl)oxy)phenyl)propan-2-yl)phenoxy)cyclobutyl) amino)-1H-pyrrolo[3,4-c]pyridin-1,3(2H)-dione

6-Chloro-2-(2,6-dioxopiperidin-3-yl)-1H-pyrrolo[3,4-c]pyridin-1,3(2H)-dione (75 mg, 0.26 mmol) was dissolved in 6 mL of DMSO, to which were added (1r,3r)-3-(4-(2-(4-((2-(5-methyl-1,2,4-oxadiazol-3-yl)pyrimidin-5-yl)oxy)phenyl)propan-2-yl) phenoxy)cyclobutyl-1-amine (105 mg, 0.23 mmol) and DIPEA (90 mg, 0.70 mmol), and then the mixture was allowed to react for 48 h at 90 °C. Water was added to quench the reaction. The resultant solution was extracted with ethyl acetate for three times. The organic phase was washed with saturated NaCl solution, dried over anhydrous Na₂SO₄, and concentrated by rotatory evaporation. The residue was separated and purified by prep-TLC, to provide 2-(2,6-dioxopiperidin-3-yl)-6-((1r,3r)-3-(4-(2-(4-((2-(5-methyl-1,2,4-oxadiazol-3-yl)pyrimidin-5-yl)oxy)phenyl)propan-2-yl)phenoxy)cyclobutyl) amino)-1*H*-pyrrolo[3,4-c]pyridin-1,3(2H)-dione (50 mg), with a yield of 30%. ¹H NMR (400 MHz, DMSO-*d*₆) *δ* 11.11 (s, 1H), 8.74 (s, 2H), 8.61 - 8.46 (m, 2H), 7.40 - 7.25 (m, 2H), 7.18 - 7.11 (m, 4H), 6.83 (d, *J* = 16.9 Hz, 1H), 6.78 - 6.71 (m, 2H), 5.08 (dd, *J* = 12.7, 5.4 Hz, 1H), 4.87 (p, *J* = 5.7 Hz, 1H), 4.57 (s, 1H), 2.86 (ddd, *J* = 13.6, 10.5, 6.7 Hz, 1H), 2.69 (s, 3H), 2.63 - 2.50 (m, 4H), 2.46 (d, *J* = 8.8 Hz, 2H), 2.04 - 1.98 (m, 1H), 1.63 (s, 6H). LC/MS (ESI+) calcd for C₃₈H₃₄N₈O₇ ([M+H]⁺) *m*/*z:* 714.3; found 614.1.

### Example 531: 4-((4-(2-(4-(3-(2,6-dioxopiperidin-3-yl)-1,3-dioxoisoindolin-5-yl) amino)cyclobutyloxy)phenyl)propan-2-yl)phenoxy)methyl)-N-ethylpyrimidin-2-carbonylamine

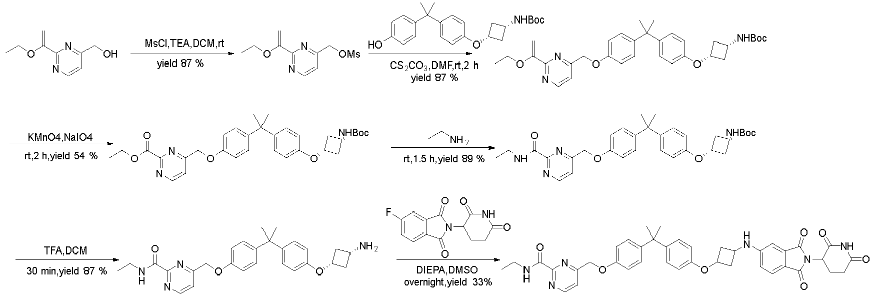

### Step 1: Synthesis of (2-(1-ethoxyvinyl)pyrimidin-4-yl)methyl methanesulfonate

(2-(1-ethoxyvinyl)pyrimidin-4-yl)methanol (2 g, 11.10 mmol) was dissolved in dichloromethane (15 mL), to which was added methanesulfonyl choride (2.7 g, 22.20 mmol) in an ice bath, and then the mixture was allowed to react at room temperature for 6 h. The reaction solution was added into water, and then extracted with ethyl acetate (30 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as colorless oil (2.5 g, yield 87%). LC/MS (ESI+) calcd for C₁₀H₁₄N₂O₄S ([M+H]⁺) *m*/*z* 259.1, found 259.2.

### Step 2: Synthesis of tert-butyl ((1s,3s)-3-(4-(2-(4-(2-(1-ethoxyvinyl)pyrimidin-4-yl)methoxy)phenyl)propan-2-yl)phenoxy)cyclobutyl)carbamate

(2-(1-ethoxyvinyl)pyrimidin-4-yl)methyl methanesulfonate (900 mg, 3.48 mmol) was dissolved in DMF (15 mL), to which were added tert-butyl ((1*s*,3*s*)-3-(4-(2-(4-hydroxylphenyl)propan-2-yl)phenoxy)cyclobutyl)carbamate (1.39 g, 3.48 mmol) and anhydrous cesium carbonate (1.42 g, 10.45 mmol), and then the mixture was allowed to react at room temperature for 2 h. The reaction solution was quenched by adding water, and then the resultant solution was extracted with ethy acetate (50 mL) for three time. The organic phase was successively washed three times with water and saturated brine. The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (1.70 g, yield 87%). LC/MS (ESI+) calcd for C₃₃H₄₁N₃O₅ ([M+H]⁺) *m*/*z* 560.3, found 560.1.

### Step 3: Synthesis of ethyl 4-((4-(2-(4-((1s,3s)-3-((tert-butoxycarbonyl)amino) cyclobutyloxy)phenyl)propan-2-yl)phenoxy)methyl)pyrimidin-2-carboxylate

tert-butyl ((1*s*,3*s*)-3-(4-(2-(4-(2-(1-ethoxyvinyl)pyrimidin-4-yl)methoxy)phenyl) propan-2-yl)phenoxy)cyclobutyl)carbamate (750 mg, 1.34 mmol) was dissolved in 1,4-dioxane/water (45 mL/15 mL), to which were added potassium permanganate (423.52 mg, 2.68 mmol) and sodium periodate (859.83 mg, 4.02 mmol), and then the mixture was allowed to react at room temperature for 2 h. The reaction solution was added into saturated sodium thiosulfate solution (80 mL), and then the reaction solution was filtered over diatomite to remove the black solid. The filtrate was extracted with ethyl acetate (50 mL) for three times. The organic phase was washed with water for three times. The combined organic phase was dried over anhydrous Na₂SO₄. After filtration, the solvent was removed by rotatory evaporation under reduced pressure to obtain the crude product, which was further purified by silica gel column chromatography, to provide the target product as colorless oil (410 mg, 54%). LC/MS (ESI+) calcd for C₃₂H₃₉N₃O₆ ([M+H]⁺) *m*/*z* 562.2, found 562.3.

### Step 4: Synthesis of tert-butyl ((1s,3s)-3-(4-(2-(4-(2-(ethylcarbamoyl)pyrimidin-4-yl)methoxy)phenyl)propan-2-yl)phenoxy)cyclobutyl)carbamate

Ethyl 4-((4-(2-(4-((1*s*,3*s*)-3-((tert-butoxycarbonyl)amino)cyclobutyloxy)phenyl) propan-2-yl)phenoxy)methyl)pyrimidin-2-carboxylate (150 mg, 0.28 mmol) was dissolved in 10 mL of ethylamine, and then the mixture was stirred at room temperature for 1.5 h. The reaction solution was rotatory evaporated to dry, followed by extraction with ethyl acetate for three times. The organic phase was washed once with saturated brine. The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (143 mg, 89%). LC/MS (ESI+) calcd for C32H40N4O5 ([M+H]⁺) *m*/*z* 561.3, found 561.4.

### Step 5: Synthesis of 4-((4-(2-(4-((1s,3s)-3-aminocyclobutyloxy)phenyl)propan-2-yl)phenoxy)methyl)-N-ethylpyrimidin-2-carbonylamine

tert-butyl ((1*s*,3*s*)-3-(4-(2-(4-(2-(ethylcarbamoyl)pyrimidin-4-yl)methoxy)phenyl) propan-2-yl)phenoxy)cyclobutyl)carbamate (143 mg, 0.255 mmol) was dissolved in anhydrous dichloromethane (10 mL), to which was added trifluoroacetic acid (2 mL) in an ice bath. Subsequently, the ice bath was removed, and then the mixture was allowed to react for half an hour at room temperature. The reaction solution was added into saturated Na₂CO₃ solution to quench the reaction, and then the resultant solution was extracted with dichloromethane: methanol (10:1, 50 mL). The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (96 mg, 81.7%). LC/MS (ESI+) calcd for C₂₇H₃₂N₄O₃ ([M+H]⁺) *m*/*z* 461.2, found 461.3.

### Step 6: Synthesis of 4-((4-(2-(4-(3-(2,6-dioxopiperidin-3-yl)-1,3-dioxoisoindolin-5-yl)amino)cyclobutyloxy)phenyl)propan-2-yl)phenoxy)methyl)-N-ethylpyrimidin-2-carbonyl amine

4-((4-(2-(4-((1*s*,3*s*)-3-aminocyclobutyloxy)phenyl)propan-2-yl)phenoxy)methyl)-N-ethylpyrimidin-2-carbonylamine (96 mg, 0.208 mmol) was dissolved in anhydrous dimethylsulfoxide (20 mL), to which were added 2-(2,6-dioxopiperidin-3-yl)-5-fluoroisoindolin-1,3-dione (115.15 mg, 0.416 mmol) and N,N-diisopropylethylamine (107.76 mg, 0.833 mmol), and then the mixture was allowed to react overnight at 95 °C under argon protection. The reaction solution was quenched with saturated NH₄Cl solution. The resultant solution was extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as yellow oil (52 mg, 33%). LC/MS (ESI+) calcd for C₄₀H₄₀N₆O₇ ([M+H]⁺) *m*/*z* 717.3, found 717.2. ¹H NMR (400 MHz, Chloroform-*d*) *δ* 8.84 (d, *J* = 5.1 Hz, 1H), 8.19 (d, *J* = 4.2 Hz, 1H), 8.03 (s, 1H), 7.70 (d, *J* = 5.1 Hz, 1H), 7.62 (dd, *J* = 8.2, 2.5 Hz, 1H), 7.21 - 7.09 (m, 4H), 6.93 - 6.81 (m, 3H), 6.70 (td, *J* = 5.9, 5.2, 2.5 Hz, 2H), 5.25 (s, 2H), 5.00 - 4.79 (m, 3H), 3.61 - 3.53 (m, 2H), 3.19 - 3.05 (m, 1H), 2.94 - 2.65 (m, 4H), 2.62 (s, 2H), 2.41 (dd, *J* = 12.6, 6.8 Hz, 1H), 2.19 - 2.07 (m, 2H), 1.63 (d, *J* = 4.2 Hz, 6H), 1.29 (t, *J* = 7.3 Hz, 3H).

### Example 532: Synthesis of 5-((3-(4-(2-(4-(6-(2-oxa-6-azaspiro[3.3]heptan-6-yl) pyrimidin-4-yl)methoxy)phenyl)propan-2-yl)phenoxy)cyclobutyl)amino)-2-(2,6-dioxopiperidin-3-yl)isoindolin-1,3-dione

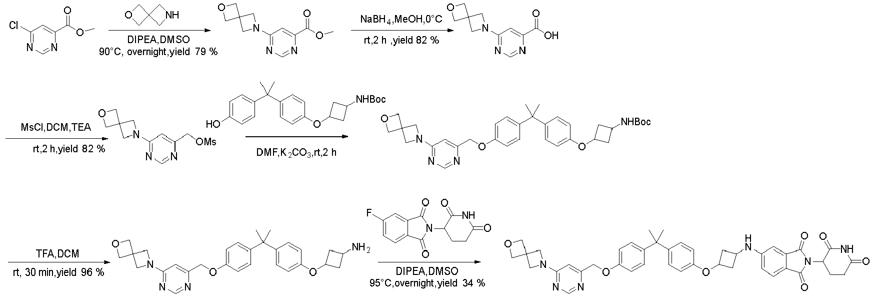

### Step 1: Synthesis of 6-(2-oxa-6-azaspiro[3.3]heptan-6-yl)pyrimidin-4-carboxylic acid

Methyl 6-chloropyrimidin-4-carboxylate (500 mg, 2.90 mmol) was dissolved in anhydrous dichloromethane, to which were added 2-oxa-6-azaspiro[3.3]heptane hemioxalate (315.95 mg, 3.19 mmol) and N,N-diisopropylethylamine (1.12 g, 8.69 mmol), and then the mixture was allowed to react overnight at 90 °C. The reaction solution was added into saturated NH₄Cl solution. The resultant solution was extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (540 mg, 79%). LC/MS (ESI+) calcd for C₁₀H₁₁N₃O₃ ([M+H]⁺) *m*/*z* 221.1, found 221.2.

### Step 2: Synthesis of 6-(2-oxa-6-azaspiro[3.3]heptan-6-yl)pyrimidin-4-carboxylic acid

6-(2-oxa-6-azaspiro[3.3]heptan-6-yl)pyrimidin-4-carboxylic acid (540 mg, 2.30 mmol) was dissolved in methanol, to which was added sodium borohydride (173.69 mg, 4.59 mmol) in an ice bath, and then the mixture was allowed to react at room temperature for 2 h. The reaction solution was added into acetone, and then extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (420 mg, 82.7%). LC/MS (ESI+) calcd for C₁₁H₁₃N₃O₃ 236.10 ([M+H]⁺) *m*/*z* found 236.2.

### Step 3: Synthesis of (6-(2-oxa-6-azaspiro[3.3]heptan-6-yl)pyrimidin-4-yl) methanesulfonate

6-(2-oxa-6-azaspiro[3.3]heptan-6-yl)pyrimidin-4-carboxylic acid (64.00 mg, 0.28 mmol) was dissolved in anhydrous dichloromethane (10 mL), to which was added triethylamine (87.83 mg, 0.867 mmol), followed by addition of methanesulfonyl choride (66.28 mg, 0.578 mmol) in an ice bath. The reaction solution was added into saturated NH₄Cl solution to quench the reaction. The resultant solution was extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as colorless oil (65 mg, 82%). LC/MS (ESI+) calcd for C₁₁H₁₅N₃O₄S1 ([M+H]⁺) *m*/*z* 286.1, found 286.1.

### Step 4: Synthesis of tert-butyl (3-(4-(2-(4-((6-(2-oxa-6-azaspiro[3.3]heptan-6-yl)pyrimidin-4-yl)methoxy)phenyl)propan-2-yl)phenoxy)cyclobutyl)carbamate

(6-(2-oxa-6-azaspiro[3.3]heptan-6-yl)pyrimidin-4-yl)methanesulfonate (65 mg, 0.227 mmol) was dissolved in anhydrous DMF, to which were added tert-butyl ((1*s*,3*s*)-3-(4-(2-(4-hydroxylphenyl)propan-2-yl)phenoxy)cyclobutyl)carbamate (181.12 mg, 0.455 mmol) and anhydrous Na₂CO₃ (94.46 mg, 0.68 mmol), and then the mixture was allowed to react at room temperature for 2 h. The reaction solution was added into water, and then the resultant solution was extracted with ethy acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as colorless oil (70 mg, 52%).
LC/MS (ESI+) calcd for C₃₄H₄₂N₄O₅ ([M+H]⁺) *m*/*z* 587.3, found 587.4.

### Step 5: Synthesis of 3-(4-(2-(4-(6-(2-oxa-6-azaspiro[3.3]heptan-6-yl)pyrimidin-4-yl) methoxy)phenyl)propan-2-yl)phenoxy)cyclobutan-1-amine

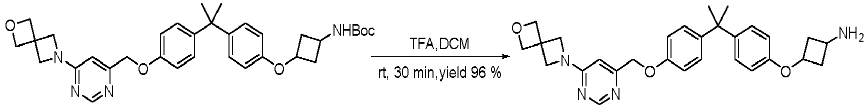

tert-butyl (3-(4-(2-(4-((6-(2-oxa-6-azaspiro[3.3]heptan-6-yl)pyrimidin-4-yl) methoxy)phenyl)propan-2-yl)phenoxy)cyclobutyl)carbamate (70 mg) was dissolved in anhydrous dichloromethane, to which was added trifluoroacetic acid (6 mL) in an ice bath. Subsequently, the ice bath was removed, and then the mixture was allowed to react for 30 min at room temperature. The reaction solution was added into saturated Na₂CO₃ solution to quench the reaction, and then the resultant solution was extracted with dichloromethane (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as yellow oil (60 mg, 96%). LC/MS (ESI+) calcd for C₂₉H₃₄N₄O₃ ([M+H]⁺) *m*/*z* 587.3, found 587.4.

### Step 6: Synthesis of 5-((3-(4-(2-(4-(6-(2-oxa-6-azaspiro[3.3]heptan-6-yl)pyrimidin-4-yl)methoxy)phenyl)propan-2-yl)phenoxy)cyclobutyl)amino)-2-(2,6-dioxopiperidin-3-yl)isoindolin-1,3-dione

3-(4-(2-(4-(6-(2-oxa-6-azaspiro[3.3]heptan-6-yl)pyrimidin-4-yl)methoxy)phenyl) propan-2-yl)phenoxy)cyclobutan-1-amine (60 mg, 0.12 mmol) was dissolved in anhydrous dimethylsulfoxide (20 mL), to which were added 2-(2,6-dioxopiperidin-3-yl)-5-fluoroisoindolin-1,3-dione (68.12 mg, 0.246 mmol) and N,N-diisopropylethylamine (63.74 mg, 0.493 mmol), and then the mixture was allowed to react overnight at 95 °C under argon protection. The reaction solution was quenched with saturated NH₄Cl solution. The resultant solution was extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as yellow solid (32 mg, 34%). LC/MS (ESI+) calcd for C₄₂H₄₂N₆O₇ ([M+H]⁺) *m*/*z* 743.3, found 732.1. ¹H NMR (400 MHz, Chloroform-*d*) *δ* 9.12 (s, 1H), 8.50 (d, *J* = 1.4 Hz, 1H), 7.78 (d, *J* = 7.5 Hz, 1H), 7.29 (d, *J* = 1.4 Hz, 1H), 7.23 - 7.11 (m, 4H), 7.07 (dd, *J* = 7.5, 1.5 Hz, 1H), 6.90 - 6.84 (m, 4H), 6.45 (d, *J* = 1.4 Hz, 1H), 5.45 (t, *J* = 7.0 Hz, 1H), 5.35 (d, *J* = 10.4 Hz, 1H), 5.07 (q, *J* = 12.4 Hz, 2H), 4.58 (p, *J* = 7.0 Hz, 1H), 3.84 (s, 4H), 3.77 - 3.64 (m, 5H), 2.67 - 2.58 (m, 2H), 2.44 - 2.25 (m, 4H), 2.23 - 2.05 (m, 2H), 1.62 (s, 6H).

### Example 533: 5-((3-(4-(2-(2-oxa-6-azaspiro[3.3]heptan-6-yl)pyrimidin-5-yl) methoxy)phenyl)propan-2-yl)phenoxy)cyclobutyl)amino)-2-(2,6-dioxopiperidin-3-yl)isoindolin-1,3-dione

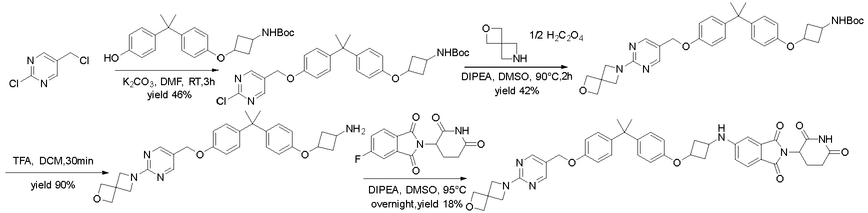

### Step 1: tert-butyl Synthesis of (3-(4-(2-(4-((2-chloropyrimidin-5-yl)methoxy) phenyl)propan-2-yl)phenoxy)cyclobutyl)carbamate

tert-butyl (3-(4-(2-(4-hydroxylphenyl)propan-2-yl)phenoxy)cyclobutyl)carbamate (0.5 g, 1.26 mmol) and 2-chloro-5-(chloromethyl)pyrimidine (230 mg, 1.38 mmol) were dissolved in 20 mL of anhydrous DMF, to which was added anhydrous K₂CO₃ (350 mg, 2.52 mmol) at room temperature, and then the mixture was stirred for 3 h. 50 mL of saturated NH₄Cl solution was added to quench the reaction. The resultant solution was extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as faint colorless oil (750 mg, 46%). LC/MS (ESI+) calcd for C₂₉H₃₄ClN₃O₄ ([M+H]⁺) *m*/*z* 524.2, found 524.4.

### Step 2: Synthesis of tert-butyl (3-(4-(2-(4-((2-(2-oxa-6-azaspiro[3.3]heptan-6-yl)pyrimidin-5-yl)methoxy)phenyl)propan-2-yl)phenoxy)cyclobutyl)carbamate

tert-butyl (3-(4-(2-(4-((2-chloropyrimidin-5-yl)methoxy)phenyl)propan-2-yl) phenoxy)cyclobutyl)carbamate (100 mg, 0.19 mmol) was dissolved in 20 mL of anhydrous DMSO, to which were added 2-oxa-6-azaspiro[3.3]heptane (300 mg, 0.11 mmol) and DIPEA (0.15 mL, 0.76 mmol), and then the mixture was heated to 90 °C and allowed to react overnight. 50 mL of saturated NH₄Cl solution was added to quench the reaction. The resultant solution was extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as faint colorless oil (48 mg, 42%). LC/MS (ESI+) calcd for C₃₄H₄₂N₄O₅ ([M+H]⁺) *m*/*z* 587.3, found 587.2.

### Step 3: Synthesis of (1s,3s)-3-(4-(2-(4-(2-(2-oxa-6-azaspiro[3.3]heptan-6-yl) pyrimidin-5-yl)methoxy)phenyl)propan-2-yl)phenoxy)cyclobutan-1-amine

tert-butyl (3-(4-(2-(4-((2-(2-oxa-6-azaspiro[3.3]heptane-6-yl)pyrimidin-5-yl) methoxy)phenyl)propan-2-yl)phenoxy)cyclobutyl)carbamate (48 mg, 0.081mmol) was dissolved in 10 mL of dichloromethane, to which was added 2 mL of TFA in an ice-water bath, and then the mixture was warmed to room temperature and stirred for 30 min. 20 mL of saturated NaHCO₃ solution was added to quench the reaction, and then the resultant solution was extracted with 50 mL of dichloromethane for three times. The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (48 mg, 90%). LC/MS (ESI+) calcd for C₂₉H₃₄N₄O₃ ([M+H]⁺) *m*/*z* 487.2, found 487.3.

### Step 4: Synthesis of 5-((3-(4-(2-(2-oxa-6-azaspiro[3.3]heptan-6-yl)pyrimidin-5-yl) methoxy)phenyl)propan-2-yl)phenoxy)cyclobutyl)amino)-2-(2,6-dioxopiperidin-3-yl)isoindolin-1,3-dione

(1*s*,3*s*)-3-(4-(2-(4-(2-(2-oxa-6-azaspiro[3.3]heptan-6-yl)pyrimidin-5-yl)methoxy) phenyl)propan-2-yl)phenoxy)cyclobutan-1-amine (36 mg, 0.073 mmol) was dissolved in 20 mL of anhydrous DMSO, to which were added DIPEA (38 mg, 0.29 mmol) and 2-(2,6-dioxopiperidin-3-yl)-4-fluoroisoindolin-1,3-dione (40.87 mg, 0.147 mmol), and then the mixture was allowed to react overnight at 95 °C under argon protection for three times. The reaction solution was quenched with 50 mL of saturated NH₄Cl solution. The resultant solution was extracted with ethyl acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as yellow solid (10 mg, yield 18%). LC/MS (ESI+) calcd for C₄₂H₄₂N₆O₇ ([M+H]⁺) *m*/*z* 743.3, found 743.1. ¹H NMR (400 MHz, Chloroform-*d*) *δ* 9.12 (s, 1H), 8.29 (s, 2H), 7.78 (d, *J* = 7.5 Hz, 1H), 7.29 (d, *J* = 1.4 Hz, 1H), 7.23 - 7.15 (m, 4H), 7.07 (dd, *J* = 7.5, 1.5 Hz, 1H), 6.92 - 6.82 (m, 4H), 5.45 (t, *J* = 7.0 Hz, 1H), 5.35 (d, *J* = 10.4 Hz, 1H), 5.27 (s, 2H), 4.58 (p, *J* = 7.0 Hz, 1H), 3.91 (s, 4H), 3.81 (d, *J* = 6.0 Hz, 2H), 3.79 - 3.65 (m, 3H), 2.66 - 2.56 (m, 2H), 2.43 - 2.25 (m, 4H), 2.25 - 2.04 (m, 2H), 1.62 (s, 6H).

### Example 534: 5-((3-(4-(2-(2-oxa-6-azaspiro[3.3]heptan-6-yl)pyrimidin-5-yl) methoxy)phenyl)propan-2-yl)phenoxy)cyclobutyl)amino)-2-(2,6-dioxopiperidin-3-yl)isoindolin-1,3-dione

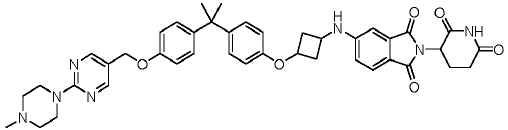

The target compound was synthesized by a method similar to that of Example 533. LC/MS (ESI+) calcd for C₄₂H₄₅N₇O₆ ([M+H]⁺) *m*/*z* 744.3, found 744.4. ¹H NMR (400 MHz, Chloroform-*d*) *δ* 9.12 (s, 1H), 8.29 (s, 2H), 7.78 (d, *J* = 7.5 Hz, 1H), 7.29 (d, *J* = 1.4 Hz, 1H), 7.21 - 7.13 (m, 4H), 7.07 (dd, *J* = 7.5, 1.5 Hz, 1H), 6.92 - 6.82 (m, 4H), 5.45 (t, *J* = 7.0 Hz, 1H), 5.35 (d, *J* = 10.4 Hz, 1H), 5.27 (s, 2H), 4.58 (p, *J* = 7.0 Hz, 1H), 3.70 (dp, *J* = 10.4, 7.0 Hz, 1H), 3.56 - 3.44 (m, 6H), 3.41 - 3.28 (m, 2H), 2.62 (t, *J* = 6.9 Hz, 2H), 2.44 - 2.36 (m, 2H), 2.34 (d, *J* = 7.2 Hz, 1H), 2.33 - 2.25 (m, 5H), 2.23 - 2.05 (m, 2H), 1.62 (s, 6H).

### Example 535: 5-((1r,3r)-3-(4-(2-(4-(2-oxa-6-azaspiro[3.3]heptan-6-yl)pyrimidin-5-yl)methoxy)phenyl)propan-2-yl)phenoxy)cyclobutylamino)-2-(2,6-dioxopiperidin-3-yl)isoindolin-1,3-dione

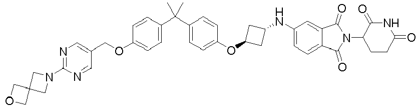

The target compound was synthesized by a method similar to that of Example 533. LC/MS (ESI+) calcd for C₄₂H₄₂N₆O₇ ([M+H]⁺) *m*/*z* 743.3, found 743.1. ¹H NMR (400 MHz, Chloroform-*d) δ* 9.12 (s, 1H), 8.29 (s, 2H), 7.78 (d, *J* = 7.5 Hz, 1H), 7.29 (d, *J* = 1.4 Hz, 1H), 7.21 - 7.13 (m, 4H), 7.07 (dd, *J* = 7.5, 1.5 Hz, 1H), 6.92 - 6.82 (m, 4H), 5.45 (t, *J* = 7.0 Hz, 1H), 5.35 (d, *J* = 10.8 Hz, 1H), 5.27 (s, 2H), 4.74 (pd, *J* = 7.0, 2.5 Hz, 1H), 3.91 (s, 4H), 3.84 - 3.77 (m, 2H), 3.77 - 3.67 (m, 3H), 2.66 - 2.58 (m, 2H), 2.53 (dt, *J* = 12.4, 7.0 Hz, 2H), 2.28 (dt, *J* = 12.3, 6.9 Hz, 2H), 2.23 - 2.05 (m, 2H), 1.62 (s, 6H).

### Example 536: 2-(2,6-dioxopiperidin-3-yl)-5-(((1r,3r)-3-(4-(4-((6-methylpyrazin-2-yl)oxy)phenyl)propan-2-yl)phenoxy)amino)isoindolin-1,3-dione

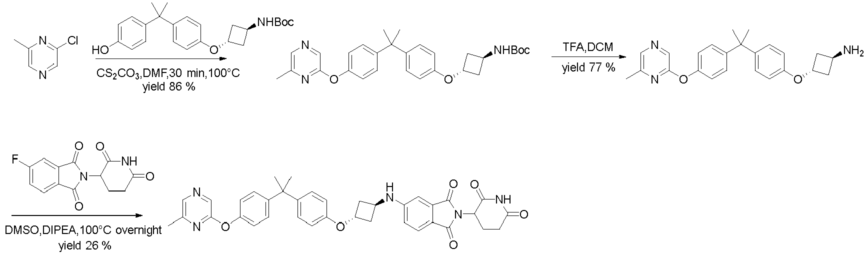

### Step 1: tert-butyl Synthesis of ((1r,3r)-3-(4-(2-(4-((6-methylpyrazin-2-yl)oxy)phenyl) propan-2-yl)phenoxy)cyclobutyl)carbamate

2-chloro-6-methylpyrazine (50 mg, 0.388 mmol) was added into 15 mL of DMF, to which were added tert-butyl ((1*s*,3*s*)-3-(4-(2-(4-hydroxylphenyl)propan-2-yl)phenoxy) cyclobutyl)carbamate (140.55 mg, 0.353 mmol) and anhydrous cesium carbonate (96.27 mg, 0.707 mmol), and then the solution was heated to100 °C and allowed to react for 30 min. The reaction solution was added into water to quench the reaction, and then the resultant solution was extracted with ethy acetate (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (150 mg, 86%). LC/MS (ESI+) calcd for C₂₉H₃₅N₃O₄ ([M+H]⁺) *m*/*z* 489.3, found 489.3.

### Step 2: Synthesis of (1r,3r)-3-(4-(2-(4-((6-methylpyrazin-2-yl)oxy)phenyl)propan-2-yl)phenoxy)cyclobutane-1-amine

tert-butyl (1*r*,3*r*)-3-(4-(2-(4-((6-methylpyrazin-2-yl)oxy)phenyl)propan-2-yl) phenoxy)cyclobutyl)carbamate (150 mg, 0.30 mmol) was dissolved in anhydrous dichloromethane (10 mL), to which was added trifluoroacetic acid (2 mL) in an ice bath, and then the mixture was warmed to room temperature and allowed to react for 30 min. 20 mL of saturated Na₂CO₃ solution was added to quench the reaction, and then the resultant solution was extracted with dichloromethane (50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as faint yellow oil (93 mg, 77%). LC/MS (ESI+) calcd for C₂₄H₂₇N₃O₂ ([M+H]⁺) *m*/*z* 389.2, found 389.2.

### Step 3: Synthesis of 2-(2,6-dioxopiperidin-3-yl)-5-(((1r,3r)-3-(4-(4-((6-methylpyrazin-2-yl)oxy)phenyl)propan-2-yl)phenoxy)amino)isoindolin-1,3-dione

(1*r*,3*r*)-3-(4-(2-(4-((6-methylpyrazin-2-yl)oxy)phenyl)propan-2-yl)phenoxy) cyclobutane-1-amine (93 mg, 0.238 mmol) was dissolved in 20 mL of anhydrous DMSO, to which was added DIPEA (123.44 mg, 0.95 mmol), followed by addition of 2-(2,6-dioxopiperidin-3-yl)-4-fluoroisoindolin-1,3-dione (131.91 mg, 0.47 mmol), and then the system was purged with argon for three times. The mixture was allowed to react overnight at 95 °C. The reaction solution was quenched with 50 mL of saturated NH₄Cl solution. The resultant solution was extracted with ethyl acetate (3*40 mL). After suction filtration, the combined organic phase was dried over anhydrous Na₂SO₄. The reaction solution was rotatory evaporated to dry under reduced pressure, followed by separation with TLC, to provide the compound (41 mg, yield 26.5%). LC/MS (ESI+) calcd for C₃₇H₃₅N₅O₆ ([M+H]⁺) *m*/*z* 655.2, found 655.3. ¹H NMR (400 MHz, Chloroform-*d*) *δ* 8.15 (s, 2H), 8.09 (s, 1H), 7.62 (d, *J* = 8.2 Hz, 1H), 7.25 (d, *J* = 8.3 Hz, 3H), 7.19 - 7.13 (m, 2H), 7.05 (d, *J* = 8.3 Hz, 2H), 6.88 (d, *J* = 1.9 Hz, 1H), 6.75 - 6.66 (m, 3H), 4.93 (dd, *J* = 12.1, 5.2 Hz, 1H), 4.87 (*s*, 1H), 4.22 (*s,* 1H), 2.93 - 2.64 (m, 5H), 2.48 - 2.35 (m, 5H), 2.16 - 2.09 (m, 1H), 1.68 (*s*, 6H).

### Example 537: 2-(2,6-dioxopiperidin-3-yl)-5-(((1s,3s)-3-(4-(4-((6-methylpyrazin-2-yl)oxy)phenyl)propan-2-yl)phenoxy)cyclobutyl)amino)isoindolin-1,3-dione

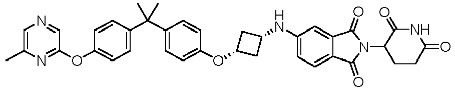

The target compound was synthesized by a method similar to that of Example 536. LC/MS (ESI+) calcd for C₃₇H₃₅N₅O₆ ([M+H]⁺) *m*/*z* 655.2 , found 655.4. ¹H NMR (400 MHz, Chloroform-*d*) *δ* 8.15 (d, *J* = 5.4 Hz, 2H), 8.09 (*s,* 1H), 7.62 (d, *J* = 8.3 Hz, 1H), 7.27 - 7.23 (m, 3H), 7.19 - 7.13 (m, 2H), 7.07 - 7.02 (m, 2H), 6.91 (d, *J* = 2.1 Hz, 1H), 6.77 - 6.68 (m, 3H), 4.96 - 4.89 (m, 1H), 4.52 (p, *J* = 6.9 Hz, 1H), 3.77 (p, *J* = 7.6 Hz, 1H), 3.13 (dtd, *J* = 9.8, 6.8, 2.9 Hz, 2H), 2.92 - 2.68 (m, 3H), 2.46 (*s,* 3H), 2.16 - 2.04 (m, 3H), 1.68 (*s,* 6H).

### Example 538: Synthesis of 5-((1s,3s)-3-(4-(2-(4-(6-(2-oxa-6-azaspiro[3.3]heptan-6-yl)pyrazin-2-yl)methoxy)phenyl)propan-2-yl)phenoxy)cyclobutylamino)-2-(2,6-dioxopiperidin-3-yl)isoindolin-1,3-dione

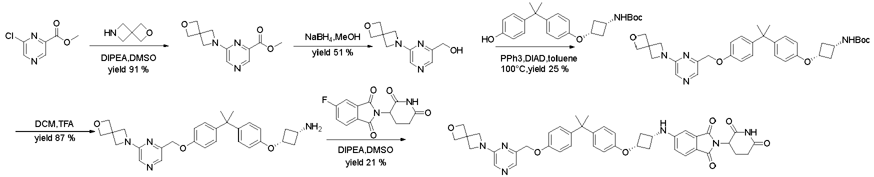

### Step 1: Synthesis of methyl 6-(2-oxa-6-azaspiro[3.3]heptan-6-yl)pyrazin-2-carboxylate

Methyl 6-chloropyrazin-2-carboxylate (2 g, 11.59 mmol) was dissolved in anhydrous dimethylsulfoxide (20 mL), to which were added 2-oxa-6-azaspiro[3.3]heptane hemioxalate (1.15 g, 11.59 mmol) and N,N-diisopropylethylamine (4.49 g, 34.77 mmol), and then the mixture was allowed to react overnight at 90 °C. The reaction solution was added into saturated NH₄Cl solution. The resultant solution was extracted with ethyl acetate (40 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as white solid (2.5 g, 91%). LC/MS (ESI+) calcd for C₁₁H₁₃N₃O₃ ([M+H]⁺) *m*/*z* 235.1, found 235.1.

### Step 2: Synthesis of (6-(2-oxa-6azaspiro[3.3]heptan-6-yl)pyrazin-2-yl)methanol

Methyl 6-(2-oxa-6-azaspiro[3.3]heptan-6-yl)pyrazin-2-carboxylate (2.50 g, 10.63 mmol) was dissolved in anhydrous methanol, to which was added sodium borohydride (2.01 g, 53.14 mmol) in an ice bath, and then the mixture was allowed to react overnight in the ice bath. The reaction solution was extracted with n-butanol (50 mL) for five times. The combined organic phase was dried over anhydrous Na₂SO₄. After filtration, the solvent was removed by rotatory evaporation under reduced pressure to obtain the crude product, which was further purified by silica gel column chromatography, to provide the target product as white solid (300 mg). LC/MS (ESI+) calcd for C₁₀H₁₃N₃O₂ ([M+H]⁺) *m*/*z* 207.1, found 207.1.

### Step 3: Synthesis of ((1s,3s)-3-(4-(2-(4-(6-(2-oxa-6-azaspiro[3.3]heptan-6-yl)pyrazin-2-yl)methoxy)phenyl)propan-2-yl)phenoxy)cyclobutyl)carbamate tert-butyl

(6-(2-oxa-6azaspiro[3.3]heptan-6-yl)pyrazin-2-yl)methanol (200 mg, 0.965 mmol) was dissolved in anhydrous toluene, to which were added tert-butyl ((1*s*,3*s*)-3-(4-(2-(4-hydroxylphenyl)propan-2-yl)phenoxy)cyclobutyl)carbamate (364.46 mg, 0.916 mmol) and triphenylphosphine (379.71 mg, 1.45 mmol), followed by adding diisopropyl azodicarboxylate (292.73 mg, 1.45 mmol) dropwise in an ice bath, and then the mixture was allowed to react at room temperature for half an hour. Subsequently, the reaction solution was heated to 100 °C and allowed to react overnight. The reaction solution was rotatory evaporated to dry, and then the residue was separated by column chromatography, to provide faint yellow oil (120 mg, 25 %). LC/MS (ESI+) calcd for C₃₄H₄₂N₄O₅ ([M+H]⁺) *m*/*z* 587.3, found 587.2.

### Step 4: Synthesis of (1s,3s)-3-(4-(2-(4-(6-(2-oxa-6-azaspiro[3.3]heptan-6-yl)pyrazin-2-yl)methoxy)phenyl)propan-2-yl)phenoxy)cyclobutane-1-amine

tert-butyl ((1*s*,3*s*)-3-(4-(2-(4-(6-(2-oxa-6-azaspiro[3.3]heptan-6-yl)pyrazin-2-yl) methoxy)phenyl)propan-2-yl)phenoxy)cyclobutyl)carbamate (120 mg) was dissolved in 10 mL of anhydrous dichloromethane, to which was added 2 mL of trifluoroacetic acid at 0 °C. Then, the ice bath was removed, and the reaction was allowed to react at room temperature for 30 min. The reaction solution was added into saturatd Na₂CO₃ solution, and then extracted with a mixed solvent of DCM and MeOH (10:1, 50 mL) for three times. The combined organic phase was dried over anhydrous Na₂SO₄. After filtration, the solvent was removed by rotatory evaporation under reduced pressure to obtain the crude product, which was further purified by silica gel column chromatography, to provide the target product as yellow oil (87 mg, 87%). LC/MS (ESI+) calcd for C₂₉H₃₄N₄O₃ ([M+H]⁺) *m*/*z* 487.3, found 487.4.

### Step 5: Synthesis of 5-((1s,3s)-3-(4-(2-(4-(6-(2-oxa-6-azaspiro[3.3]heptan-6-yl) pyrazin-2-yl)methoxy)phenyl)propan-2-yl)phenoxy)cyclobutylamino)-2-(2,6-dioxopiperidin-3-yl)isoindolin-1,3-dione

(1*s*,3*s*)-3-(4-(2-(4-(6-(2-oxa-6-azaspiro[3.3]heptan-6-yl)pyrazin-2-yl)methoxy) phenyl)propan-2-yl)phenoxy)cyclobutane-1-amine (87 mg, 0.178mmol) was dissolved in anhydrous dimethylsulfoxide (20 mL), to which were added 2-(2,6-dioxopiperidin-3-yl)-5-fluoroisoindolin-1,3-dione (98.77 mg, 0.357 mmol) and N,N-diisopropylethylamine (92.43 mg, 0.715 mmol), and then the mixture was allowed to react overnight at 95 °C under argon protection. The reaction solution was quenched with saturated NH₄Cl solution, and then extracted with ethyl acetate (50 mL) for three times. The combined organic phase was dried over anhydrous Na₂SO₄. After filtration, the reaction solution was rotatory evaporate under reduced pressure to remove the solvent and obtain the crude product, which was further purified by column chromatography, to provide the target product as yellow solid (28 mg, 21%).
LC/MS (ESI+) calcd for C₄₂H₄₂N₆O₇ ([M+H]⁺) *m*/*z* 743.31, found 743.42.
¹H NMR (400 MHz, Chloroform-*d*) *δ* 8.08 (d, *J* = 13.1 Hz, 2H), 7.72 (*s,* 1H), 7.63 (d, *J* = 8.3 Hz, 1H), 7.13 (dd, *J* = 8.6, 5.5 Hz, 4H), 6.92 (d, *J* = 2.1 Hz, 1H), 6.86 (d, *J* = 8.5 Hz, 2H), 6.75 - 6.68 (m, 3H), 5.03 (*s,* 2H), 4.93 (dd, *J* = 12.2, 5.2 Hz, 1H), 4.87 (*s,* 3H), 4.51 (p, *J* = 6.9 Hz, 1H), 4.29 (s, 4H), 3.95 (s, 1H), 3.78 (t, *J* = 7.5 Hz, 1H), 3.19 - 3.03 (m, 2H), 2.93 - 2.70 (m, 3H), 2.11 (q, *J* = 11.5, 9.7 Hz, 3H), 1.63 *(s,* 6H).

### Example 539: 5-((1r,3r)-3-(4-(2-(4-(6-(2-oxa-6-azaspiro[3.3]heptan-6-yl)pyrazin-2-yl)methoxy)phenyl)propan-2-yl)phenoxy)cyclobutyl)amino)-2-(2,6-dioxopiperidin-3-yl)isoindolin-1,3-dione

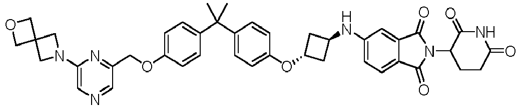

The target compound was synthesized by a method similar to that of Example 538. LC/MS (ESI+) calcd for C₄₂H₄₂N₆O₇ ([M+H]⁺) *m*/*z* 743.31, found 743.35. ¹H NMR (400 MHz, Chloroform-*d*) *δ* 8.37 (s, 2H), 7.62 (dd, *J* = 8.3, 2.6 Hz, 1H), 7.14 (td, *J* = 8.7, 1.9 Hz, 4H), 6.91 (dd, *J* = 8.0, 2.1 Hz, 1H), 6.84 (d, *J* = 8.7 Hz, 2H), 6.76 - 6.66 (m, 3H), 4.93 (dd, *J* = 12.1, 5.3 Hz, 1H), 4.85 (d, *J* = 2.5 Hz, 2H), 4.03 (*s,* 4H), 3.20 - 3.07 (m, 1H), 2.94 - 2.78 (m, 2H), 2.74 (d, *J* = 16.6 Hz, 6H), 2.44 - 2.37 (m, 1H), 2.11 (d, *J* = 7.8 Hz, 3H), 1.63 (*s,* 6H).

### Example 540: 5-((1s,3s)-3-(4-(2-(4-(6-(2H-1,2,3-triazol-2-yl)pyridin-3-yl)oxy) phenyl)propan-2-yl)phenoxy)cyclobutyl)amino)-2-(2,6-dioxopiperidin-3-yl) isoindolin-1,3-dione

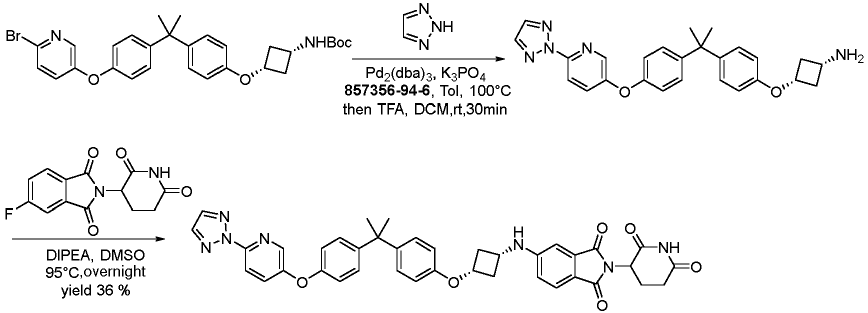

### Step 1: Synthesis of (1s,3s)-3-(4-(2-(4-((6-(2H-1,2,3-triazol-2-yl)pyridin-3-yl)oxy) phenyl)propan-2-yl)phenoxy)cyclobutylamine

tert-butyl ((1*s*,3*s*)-3-(4-(2-(4-((6-bromopyridin-3-yl)oxy)phenyl)propan-2-yl) phenoxy)cyclobutyl)carbamate (380 mg, 0.55 mmol) was dissolved in anhydrous toluene (20 mL), to which were added potassium phosphate (76 mg, 0.86 mmol) and tris(dibenzylideneacetone)dipalladium (50 mg, 0.055 mmol), followed by the ligand 2-di-tert-butylphosphine-3,4,5,6-tetramethyl-2',4',6'-triisopropylbiphenyl (52 mg, 0.011mmol) and 2H-triazole (76 mg, 1.1 mmol), and then the reaction solution was refluxed overnight at 100 °C. The reaction solution was quenched with 50 mL of saturated NH₄Cl solution. The resultant solution was extracted with ethyl acetate (40 mL) for three times. After suction filtration, the combined organic phase was dried over anhydrous Na₂SO₄. The reaction solution was rotatory evaporated to dry under reduced pressure, and then the residue was dissolved in 10 mL of anhydrous dichloromethane, followed by addition of 2 mL TFA in an ice-water bath. Subsequently, the reaction solution was warmed to room temperature and stirred for 30 min. The reaction was quenched with 20 mL of saturated NaHCO₃ solution, and the resultant solution was extracted with dichloromethane (40 mL) for three times. After suction filtration, the combined organic phase was dried over anhydrous Na₂SO₄. The reaction solution was rotatory evaporated to dry under reduced pressure. The residue was separated by TLC, to provide faint yellow solid (69 mg, 36%). LC/MS (ESI+) calcd for C₂₆H₂₇N₅O₂ ([M+H]⁺) *m*/*z* 442.2, found 442.1.

### Step 2: Synthesis of 5-((1s,3s)-3-(4-(2-(4-(6-(2H-1,2,3-triazol-2-yl)pyridin-3-yl)oxy)phenyl)propan-2-yl)phenoxy)cyclobutyl)amino)-2-(2,6-dioxopiperidin-3-yl)isoindolin-1,3-dione

(1s,3s)-3-(4-(2-(4-((6-(2H-1,2,3-triazol-2-yl)pyridin-3-yl)oxy)phenyl)propan-2-yl)phenoxy)cyclobutylamine (87 mg, 0.19 mmol) was dissolved in 20 mL of anhydrous DMSO, to which were added DIPEA (4.0 eq., 0.101 mg, 0.67 mmol) and 2-(2,6-dioxopiperidin-3-yl)-4-fluoroisoindolin-1,3-dione (2.0 eq., 0.107 g, 0.39 mmol), and then the system was purged with argon for three times. The mixture was allowed to react overnight at 95 °C. The reaction solution was quenched with 50 mL of saturated NH₄Cl solution. The resultant solution was extracted with ethyl acetate (3*40 mL). After suction filtration, the combined organic phase was dried over anhydrous Na₂SO₄. The reaction solution was rotatory evaporated to dry under reduced pressure, and then the residue was separated with TLC, to provide yellow solid (35 mg, 25%). LC/MS (ESI+) calcd for C₃₉H₃₅N₇O₆ ([M+H]⁺) *m*/*z* 698.2, found 698.3.
¹H NMR (400 MHz, Chloroform-*d*) *δ* 8.33 (d, *J* = 2.8 Hz, 1H), 8.16 (s, 1H), 8.02 (d, *J* = 8.9 Hz, 1H), 7.88 (s, 2H), 7.61 (d, *J* = 8.3 Hz, 1H), 7.49 (dd, *J* = 8.9, 2.9 Hz, 1H), 7.26 - 7.22 (m, 2H), 7.17 - 7.12 (m, 2H), 6.98 - 6.90 (m, 3H), 6.78 - 6.69 (m, 3H), 4.97 - 4.91 (m, 1H), 4.52 (p, *J* = 6.8 Hz, 1H), 3.78 (q, *J* = 7.6 Hz, 1H), 3.13 (dtd, *J* = 9.8, 6.9, 3.0 Hz, 2H), 2.97 - 2.65 (m, 3H), 2.11 (dtd, *J* = 10.4, 5.3, 2.6 Hz, 3H), 1.67 (s, 6H).

### Example 541: 5-((1s,3s)-3-(4-(2-(4-((6-(1H-1,2,3-triazol-1-yl)pyridin-3-yl)oxy) phenyl)propan-2-ylphenoxy)cyclobutyl)amino)-2-(2,6-dioxopiperidin-3-yl) isoindolin-1,3-dione

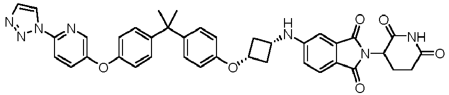

The target compound was synthesized by a method similar to that of Example 540. LC/MS (ESI+) calcd for C₃₉H₃₅N₇O₆ ([M+H]⁺) *m*/*z* 698.2, found 698.4. ¹H NMR (400 MHz, Chloroform-*d*) *δ* 8.33 (d, *J* = 2.9 Hz, 1H), 8.13 (s, 1H), 8.02 (d, *J* = 8.9 Hz, 1H), 7.88 (s, 2H), 7.62 (d, *J* = 8.3 Hz, 1H), 7.49 (dd, *J* = 8.9, 2.9 Hz, 1H), 7.26 - 7.22 (m, 2H), 7.17 - 7.12 (m, 2H), 6.98 - 6.90 (m, 3H), 6.76 - 6.69 (m, 3H), 4.93 (dd, *J* = 12.2, 5.3 Hz, 1H), 4.53 (q, *J* = 6.8 Hz, 1H), 3.77 (p, *J* = 7.6 Hz, 1H), 3.13 (dtd, *J* = 9.9, 6.9, 3.0 Hz, 2H), 2.92 - 2.86 (m, 1H), 2.81 (dd, *J* = 12.6, 3.9 Hz, 1H), 2.77 - 2.71 (m, 1H), 2.11 (ddt, *J* = 10.3, 7.9, 2.4 Hz, 3H), 1.67 (s, 6H).

### Example 542: 5-((1s,3s)-3-(4-(2-(4-((6-(1H-pyrazol-1-yl)pyridin-3-yl)oxy)phenyl) propan-2-yl)phenoxy)cyclobutyl)amino)-2-(2,6-dioxopiperidin-3-yl)isoindolin-1,3-dione

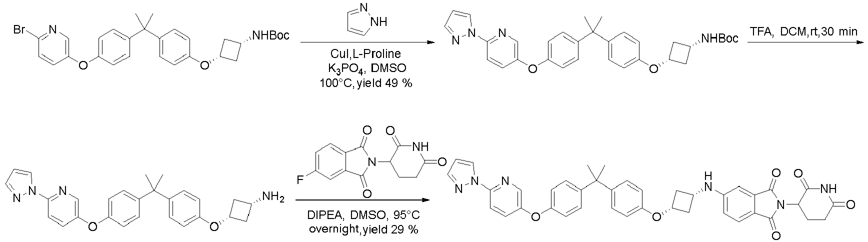

### Step 1: Synthesis of tert-butyl ((1s,3s)-3-(4-(2-(4-((6-(1H-pyrazol-1-yl)pyridin-3-yl)oxy)phenyl)propan-2-yl)phenoxy)cyclobutyl)carbamate

tert-butyl ((1s,3s)-3-(4-(2-(4-((6-bromopyridin-3 -yl)oxy)phenyl)propan-2-yl) phenoxy)cyclobutyl)carbamate (300 mg, 0.54 mmol) was dissolved in 20 mL of anhydrous DMSO, to which were added CuI (30 mg, 0.162 mmol), L-proline (18 mg, 0.162 mmol), potassium phosphate (228 mg, 1.08 mmol), and pyrazol(110 mg, 1.62 mmol), and then the mixture was allowed to react overnight at 100 °C. The reaction solution was quenched with 50 mL of saturated NH₄Cl solution. The resultant solution was extracted with ethyl acetate (40 mL) for three times. The combined organic phase was dried over anhydrous Na₂SO₄. After filtration, the reaction solution was rotatory evaporated under reduced pressure to remove the solvent and obtain the crude product, which was further purified by column chromatography, to provide the target product as faint yellow oil (123 mg, yield 42%). LC/MS (ESI+) calcd for C₃₂H₃₆N₄O₄ ([M+H]⁺) *m*/*z* 541.2, found 541.3.

### Step 2: Synthesis of (1s,3s)-3-(4-(2-(4-((6-(1H-pyrazol-1-yl)pyridin-3-yl)oxy)phenyl) propan-2-yl)phenoxy)cyclobutyl-1-amine

tert-butyl ((1s,3s)-3-(4-(2-(4-((6-(1H-pyrazol-1-yl)pyridin-3 -yl)oxy)phenyl) propan-2-yl)phenoxy)cyclobutyl)carbamate (123 mg, 0.227 mmol) was dissolved in 10 mL of anhydrous dichloromethane, to which was added 2 mL of TFA in an ice-water bath, and then the mixture was warmed to room temperature and stirred for 30 min. 20 mL of saturated Na₂CO₃ solution was added to quench the reaction, and then the resultant solution was extracted with dichloromethane (40 mL) for three times. After suction filtration, the combined organic phase was dried over anhydrous Na₂SO₄. After filtration, the reaction solution was rotatory evaporated to dry under reduced pressure, and the residue was separated by TLC, to provide the target product as faint yellow oil (67 mg, yield 90%). LC/MS (ESI+) calcd for C₂₇H₂₈N₄O₂ ([M+H]⁺) *m*/*z* 441.2, found 441.4.

### Step 3: Synthesis of 5-((1s,3s)-3-(4-(2-(4-((6-(1H-pyrazol-1-yl)pyridin-3-yl)oxy) phenyl)propan-2-yl)phenoxy)cyclobutyl)amino)-2-(2,6-dioxopiperidin-3-yl) isoindolin-1,3-dione

(1s,3s)-3-(4-(2-(4-((6-(1H-pyrazol-1-yl)pyridin-3 -yl)oxy)phenyl)propan-2-yl) phenoxy)cyclobutyl-1-amine (52 mg, 0.15 mmol) was dissolved in 20 mL of anhydrous DMSO, to which were added DIPEA(56 mg, 0.44 mmol) and 2-(2,6-dioxopiperidin-3-yl)-4-fluoroisoindolin-1,3-dione (65 mg, 0.23 mmol), and then the system was purged with argon for three times. The mixture was allowed to react overnight at 95 °C. The reaction solution was quenched with 50 mL of saturated NH₄Cl solution. The resultant solution was extracted with ethyl acetate (40 mL) for three times. The combined organic phase was dried over anhydrous Na₂SO₄. After filtration, the solvent was removed by rotatory evaporation under reduced pressure to obtain the crude product, which was separated with TLC, to provide the target product as yellow solid (30 mg, 29%). LC/MS (ESI+) calcd for C₄₀H₃₆N₆O₆ ([M+H]⁺) *m*/*z* 697.2, found 697.3. ¹H NMR (400 MHz, Chloroform-*d*) *δ* 8.49 (d, *J* = 2.6 Hz, 1H), 8.16 (d, *J* = 2.9 Hz, 1H), 8.10 (d, *J* = 11.6 Hz, 2H), 7.96 - 7.88 (m, 2H), 7.72 (d, *J* = 1.7 Hz, 1H), 7.57 (dd, *J* = 7.0, 2.3 Hz, 1H), 7.48 - 7.41 (m, 2H), 7.23 - 7.13 (m, 3H), 6.94 - 6.90 (m, 2H), 6.75 - 6.71 (m, 2H), 6.46 (t, *J* = 2.1 Hz, 1H), 4.52 (p, *J* = 6.9 Hz, 1H), 3.78 (p, *J* = 7.6 Hz, 1H), 2.94 - 2.76 (m, 5H), 2.20 - 2.09 (m, 3H), 1.66 (*s*, 6H).

### Example 543: 2-(2,6-dioxopiperidin-3-yl)-5-(1s,3s)-3-(4-(2-(4-(6-(5-methyl-1,2,4-oxadiazol-3-yl)pyridin-3-yl)oxy)phenyl)propan-2-yl)phenoxy)cyclobutyl)amino) isoindolin-1,3-dione

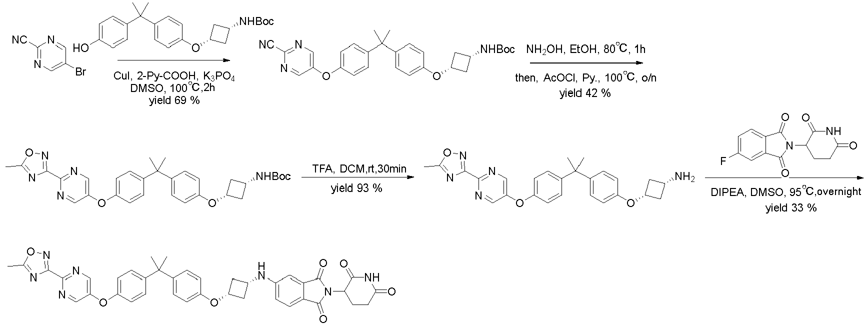

### Step 1: Synthesis of tert-butyl ((1s,3s)-3-(4-(2-(4-((2-cyanopyrimidin-5-yl)oxy)phenyl) propan-2-yl)phenoxy)cyclobutyl)carbamate

tert-butyl ((1s,3s)-3-(4-(2-(4-hydroxylphenyl)propan-2-phenoxy)cyclobutyl) carbamate (200 mg, 0.5 mmol) and 5-bromo-2-cyanopyrimidine (110 mg, 0.6 mmol) were dissolved in 20 mL of anhydrous DMSO, to which were added CuI (297 mg, 0.15 mmol), 2-picolinic acid (18 mg, 0.15 mmol), and potassium phosphate (242 mg, 1.2 mmol), and then the system was purged with argon for three times. The mixture was stirred and reacted for 2 h at 100 °C. The reaction was quenched with 50 mL of saturated NH₄Cl solution. The resultant solution was extracted with ethyl acetate (40 mL) for three times. After suction filtration, the combined organic phase was dried over anhydrous Na₂SO₄, and rotatory evaporated to dry under reduced pressure. The residue was separated with TLC, to provide the target product as faint yellow oil (208 mg, 69%). LC/MS (ESI+) calcd for C₂₉H₃₂N₄O₄ ([M+H]⁺) *m*/*z* 501.2, found 501.3.

### Step 2: Synthesis of tert-butyl ((1s,3s)-3-(4-(2-(4-((2-(5-methyl-1,2,4-oxadiazol-3-yl)pyrimidin-5-yl)oxy)phenyl)propan-2-yl)phenoxy)cyclobutyl)carbamate

tert-butyl ((1s,3s)-3-(4-(2-(4-((2-cyanopyrimidin-5-yl)oxy)phenyl)propan-2-yl) phenoxy)cyclobutyl)carbamate (0.208 g, 0.416 mmol) was dissolved in 10 mL of absolute ethanol, to which was added hydroxylamine aqueous solution (2.0 eq., 50%, 0.05 mL, 0.832 mmol), and then the solution was stirred for 1h at 80 °C. The reaction solution was directly rotatory evaporated, followed by extraction with EA (3*20 mL). The combined organic phase was dried over anhydrous Na₂SO₄, suction filtered and rotatory evaporated to dry, to obtain the crude product, to which was 10 mL of pyridine, followed by carefully adding acetyl chloride (2.0 eq., 0.10 mL, 1.46 mmol) dropwise in an ice-water bath. The reaction solution was allowed to react overnight at 100 °C. 0.05 M diluted hydrochloric acid (100 mL) was added to quench the reaction. The resultant solution was extracted with EA (4*30mL). The combined organic phase was dried over Na₂SO₄, and concentrated under reduced pressure to dry. The residue was separated by column chromatography, to provide the target product as colorless oil (123 mg, 42 %). LC/MS (ESI+) calcd for C₃₁H₃₅N₅O₅ ([M+H]⁺) *m*/*z* 558.2, found 558.1.

### Step 3: Synthesis of (1s,3s)-3-(4-(2-(4-((2-(5-methyl-1,2,4-oxadiazol-3-yl)pyrimidin-5-yl)oxy)phenyl)propan-2-yl)phenoxy)cyclobutyl-1-amine

tert-butyl ((1s,3s)-3-(4-(2-(4-((2-(5-methyl-1,2,4-oxadiazol-3-yl)pyrimidin-5-yl)oxy)phenyl)propan-2-yl)phenoxy)cyclobutyl)carbamate (123 mg, 0.22 mmol) was dissolved in 10 mL of anhydrous dichloromethane, to which was added 2 mL of TFA in an ice-water bath, and then the mixture was warmed to room temperature and stirred for 30 min. 20 mL of saturated NaHCO₃ solution was added to quench the reaction, and then the resultant solution was extracted with dichloromethane (40 mL) for three times. After suction filtration, the combined organic phase was dried over anhydrous Na₂SO₄, and rotatory evaporated to dry under reduced pressure. The residue was separated by TLC, to provide the target product as faint yellow oil (108 mg, yield 93%). LC/MS (ESI+) calcd for C₂₆H₂₇N₅O₃ ([M+H]⁺) *m*/*z* 458.2, found 458.3.

### Step 4: Synthesis of 2-(2,6-dioxopiperidin-3-yl)-5-(1s,3s)-3-(4-(2-(4-(6-(5-methyl-1,2,4-oxadiazol-3-yl)pyridin-3-yl)oxy)phenyl)propan-2-yl)phenoxy)cyclobutyl)amino) isoindolin-1,3-dione

(1s,3s)-3-(4-(2-(4-((2-(5-methyl-1,2,4-oxadiazol-3-yl)pyrimidin-5-yl)oxy)phenyl) propan-2-yl)phenoxy)cyclobutyl-1-amine (108 mg, 0.23 mmol) was dissolved in 20 mL of anhydrous DMSO, to which were added DIPEA (118 mg, 0.92 mmol) and 2-(2,6-dioxopiperidin-3-yl)-4-fluoroisoindolin-1,3-dione (132 mg, 0.47 mmol), and then the system was purged with argon for three times. The mixture was allowed to react overnight at 95 °C. The reaction solution was quenched with 50 mL of saturated NH₄Cl solution. The resultant solution was extracted with ethyl acetate (40 mL) for three times. After suction filtration, the combined organic phase was dried over anhydrous Na₂SO₄, and rotatory evaporated to dry under reduced pressure. The residue was separated with TLC, to provide the target product as yellow solid (32 mg, 33%). LC/MS (ESI+) calcd for C₄₀H₃₀N₆O₇ ([M+H]⁺) *m*/*z* 713.2, found 713.1. ¹H NMR (400 MHz, Chloroform-*d*) *δ* 8.60 (*s*, 2H), 8.07 *(s,* 1H), 7.62 (d, *J* = 8.3 Hz, 1H), 7.31 - 7.26 (m, 3H), 7.16 - 7.10 (m, 2H), 7.04 - 6.98 (m, 2H), 6.92 (d, *J* = 2.1 Hz, 1H), 6.80 - 6.68 (m, 3H), 4.93 (dd, *J* = 12.1, 5.2 Hz, 1H), 4.53 (t, *J* = 6.8 Hz, 1H), 3.78 (t, *J* = 7.5 Hz, 1H), 3.14 (dtd, *J* = 9.9, 6.9, 2.9 Hz, 2H), 2.92 - 2.74 (m, 3H), 2.72 (s, 3H), 2.17 - 2.06 (m, 3H), 1.68 (*s,* 6H).

### Example 544: 5-((1r,3r)-3-(4-(2-(4-(2-oxa-6-azaspiro[3.3]heptan-6-yl)pyrimidin-2-yl)methoxy)phenyl)propan-2-yl)phenoxy)cyclobutylamino)-2-(2,6-dioxopiperidin-3-yl)isoindolin-1,3-dione

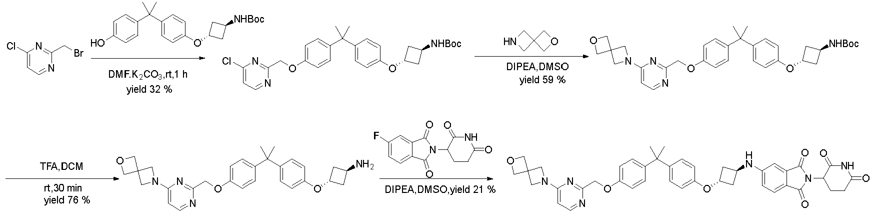

### Step 1: Synthesis of tert-butyl ((1r,3r)-3-(4-(2-(4-((4-chloropyrimidin-2-yl)methoxy) phenyl)propan-2-yl)phenoxy)cyclobutyl) carbamate

2-(bromomethyl)-4-chloropyrimidine (100 mg,0.482 mmol) was dissolved in anhydrous DMF, to which were added tert-butyl ((1s,3s)-3-(4-(2-(4-hydroxylphenyl) propan-2-phenoxy)cyclobutyl)carbamate (191.62 mg, 0.482 mmol) and anhydrous K₂CO₃ (133.2 4 mg, 0.964 mmol), and then the mixture was allowed to react at room temperature for 1h. The reaction solution was added into water to quench the reaction, and then the resultant solution was extracted with ethy acetate (50 mL). The organic phase was washed with saturated brine for three times. After suction filtration, the organic phase was dried over anhydrous Na₂SO₄, and rotatory evaporated to dry under reduced pressure. The residue was separated by TLC, to provide the target product as faint yellow oil (83 mg, 32%). LC/MS (ESI+) calcd for C₂₉H₃₄ClN₃O₄ ([M+H]⁺) *m*/*z* 524.2, found 524.2.

### Step 2: Synthesis of tert-butyl ((1r,3r)-3-(4-(2-(4-(2-oxa-6-azaspiro[3.3]heptan-6-yl)pyrimidin-2-yl)methoxy)phenyl)propan-2-yl)phenoxy)cyclobutyl)carbamate

tert-butyl ((1*r*,3*r*)-3-(4-(2-(4-((4-chloropyrimidin-2-yl)methoxy)phenyl)propan-2-yl)phenoxy)cyclobutyl)carbamate (83 mg, 0.158 mmol) was dissolved in anhydrous DMSO, to which were added 2-oxa-6-azaspiro[3.3]heptane hemioxalate (25 mg, 0.08 mmol) and DIPEA (40 mg, 0.31 mmol), and then the solution was heated to 90 °C and allowed to react overnight at 90 °C. The reaction solution was quenched with 50 mL of saturated NH₄Cl solution. The resultant solution was extracted with ethyl acetate (40 mL) for three times. After suction filtration, the combined organic phase was dried over anhydrous Na₂SO₄, and rotatory evaporated to dry under reduced pressure. The residue was separated by column chromatography, to provide the target product as faint yellow solid (55 mg, yield 59%). LC/MS (ESI+) calcd for C₃₄H₄₂N₄O₅ ([M+H]⁺) *m*/*z* 586.3, found 586.4.

### Step 3: Synthesis of (1r,3r)-3-(4-(2-(4-(2-oxa-6-azaspiro[3.3]heptan-6-yl)pyrimidin-2-yl)methoxy)phenyl)propan-2-yl)phenoxy)cyclobutan-1-amine

tert-butyl ((1*r*,3*r*)-3-(4-(2-(4-(2-oxa-6-azaspiro[3.3]heptan-6-yl)pyrimidin-2-yl) methoxy)phenyl)propan-2-yl)phenoxy)cyclobutyl)carbamate (55 mg, 0.093 mmol) was dissolved in anhydrous dichloromethane, to which was added trifluoroacetic acid at 0 °C in an ice-water bath. 20 mL of saturated NaHCO₃ solution was added to quench the reaction, and then the resultant solution was extracted with dichloromethane (40 mL) for three times. After suction filtration, the combined organic phase was dried over anhydrous Na₂SO₄, and rotatory evaporated to dry under reduced pressure. The residue was separated by TLC, to provide the target product as faint yellow oil (35 mg, yield 76%). LC/MS (ESI+) calcd for C₂₉H₃₄N₄O₃ ([M+H]⁺) *m*/*z* 486.3, found 486.2.

### Step 4: Synthesis of 5-((1r,3r)-3-(4-(2-(4-(2-oxa-6-azaspiro[3.3]heptan-6-yl)pyrimidin-2-yl)methoxy)phenyl)propan-2-yl)phenoxy)cyclobutylamino)-2-(2,6-dioxopiperidin-3-yl)isoindolin-1,3-dione

(1*r*,3*r*)-3-(4-(2-(4-(2-oxa-6-azaspiro[3.3]heptan-6-yl)pyrimidin-2-yl)methoxy) phenyl)propan-2-yl)phenoxy)cyclobutan-1-amine (35 mg, 0.07 mmol) was dissolved in anhydrous DMSO, to which were added DIPEA (36 mg, 0.28 mmol) and 2-(2,6-dioxopiperidin-3-yl)-4-fluoroisoindolin-1,3-dione (40 mg, 0.14 mmol), and then the system was purged with argon for three times. The mixture was allowed to react overnight at 95 °C. The reaction solution was quenched with 50 mL of saturated NH₄Cl solution. The resultant solution was extracted with ethyl acetate (40 mL) for three times. After suction filtration, the combined organic phase was dried over anhydrous Na₂SO₄, and rotatory evaporated to dry under reduced pressure. The residue was separated with TLC, to provide the compound (11 mg, yield 21%). LC/MS (ESI+) calcd for C₄₂H₄₂N₆O₇, ([M+H]⁺) *m*/*z* 744.3, found 744.4. ¹H NMR (400 MHz, Chloroform-*d*) *δ* 8.32 (*s*, 1H), 8.23 (d, *J* = 6.0 Hz, 1H), 7.58 (d, *J* = 8.3 Hz, 1H), 7.16 - 7.08 (m, 4H), 6.75 - 6.65 (m, 3H), 6.68 (dd, *J* = 8.6, 2.6 Hz, 3H), 6.12 (d, *J* = 5.9 Hz, 1H), 5.03 (*s,* 2H), 4.94 - 4.86 (m, 2H), 4.82 (*s*, 4H), 4.48 (p, *J* = 6.8 Hz, 1H), 4.14 (*s,* 4H), 3.75 (p, *J* = 7.3 Hz, 1H), 3.12 - 3.05 (m, 2H), 2.91 - 2.68 (m, 3H), 2.01 (ddd, *J* = 19.0, 9.4, 5.3 Hz, 3H), 1.62 (*s,* 6H).

### Example 545: 5-((1s,3s)-3-(4-(2-(4-(2-oxa-6-azaspiro[3.3]heptan-6-yl)pyrimidin-2-yl)methoxy)phenyl)propan-2-yl)phenoxy)cyclobutyl)amino)-2-(2,6-dioxopiperidin-3-yl)isoindolin-1,3-dione .

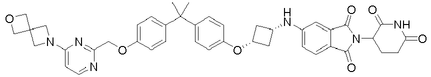

The target compound was synthesized by a method similar to that of Example 544. LC/MS (ESI+) calcd for C₄₂H₄₂N₆O₇, ([M+H]⁺) *m*/*z* 744.4, found 744.3. ¹H NMR (400 MHz, Chloroform-*d*) *δ* 8.34 (*s,* 1H), 8.22 (d, *J* = 6.0 Hz, 1H), 7.61 (d, *J* = 8.3 Hz, 1H), 7.16 - 7.08 (m, 4H), 6.95 - 6.85 (m, 3H), 6.70 (dd, *J* = 8.6, 2.6 Hz, 3H), 6.10 (d, *J* = 5.9 Hz, 1H), 5.04 *(s,* 2H), 4.95 - 4.87 (m, 2H), 4.84 (*s,* 4H), 4.50 (p, *J* = 6.8 Hz, 1H), 4.24 (*s,* 4H), 3.74 (p, *J* = 7.3 Hz, 1H), 3.16 - 3.05 (m, 2H), 2.90 - 2.68 (m, 3H), 2.11 (ddd, *J* = 19.0, 9.4, 5.3 Hz, 3H), 1.62 (*s*, 6H).

### Example 546: 2-(2,6-dioxopiperidin-3-yl)-5-(((1r,3r)-3-(4-(4-((5-(5-methyl-1,2,4-oxadiazol-3-yl)pyridin-2-yl)oxy)phenyl)propan-2-yl)phenoxy)cyclobutyl)amino) isoindolin-1,3-dione

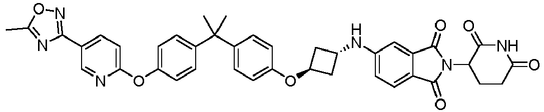

The target compound was synthesized by a method similar to that of Example 543. LC/MS (ESI+) calcd for C₄₀H₃₆N₆O₇, ([M+H]⁺) *m*/*z* 713.2, found 713.3. ¹H NMR (400 MHz, Chloroform-*d*) *δ* 8.88 (dd, *J* = 2.4, 0.8 Hz, 1H), 8.30 (dd, *J* = 8.7, 2.4 Hz, 1H), 8.00 (s, 1H), 7.64 (d, *J* = 8.3 Hz, 1H), 7.28 (d, *J* = 2.1 Hz, 1H), 7.20 - 7.16 (m, 2H), 7.09 - 7.04 (m, 2H), 6.98 (dd, *J* = 8.7, 0.8 Hz, 1H), 6.91 (d, *J* = 2.1 Hz, 1H), 6.75 - 6.68 (m, 3H), 4.96 - 4.86 (m, 2H), 4.76 (d, *J* = 4.9 Hz, 1H), 3.58 (t, *J* = 5.0 Hz, 1H), 2.84 - 2.69 (m, 4H), 2.66 (s, 3H), 2.40 (d, *J* = 6.3 Hz, 1H), 2.24 - 2.18 (m, 1H), 2.14 - 2.09 (m, 1H), 2.04 - 1.99 (m, 1H), 1.69 (s, 6H).

### Example 547: 2-(2,6-dioxopiperidin-3-yl)-5-(((1r,3r)-3-(4-(4-(pyridin-3-oxy) phenyl)propan-2-yl)phenoxy)cyclobutyl)amino)isoindolin-1,3-dione

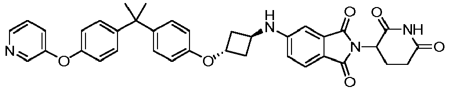

The target compound was synthesized by a method similar to that of Example 543. LC/MS (ESI+) calcd for C₃₇H₃₄N₄O₆, ([M+H]⁺) *m*/*z* 631.2, found 631.3. ¹H NMR (400 MHz, Chloroform-*d*) *δ* 8.36 (d, *J* = 2.8 Hz, 1H), 8.14 (s, 1H), 8.01 (d, *J* = 8.9 Hz, 1H), 7.84 (s, 2H), 7.42 (d, *J* = 8.3 Hz, 1H), 7.39 (dd, *J* = 8.9, 2.9 Hz, 2H), 7.24 - 7.22 (m, 2H), 7.16 - 7.12 (m, 2H), 6.91 - 6.87 (m, 3H), 4.23 (p, *J* = 6.8 Hz, 1H), 3.56 (q, *J* = 7.6 Hz, 1H), 3.01 (dtd, *J* = 9.8, 6.9, 3.0 Hz, 2H), 2.87 - 2.68 (m, 3H), 2.12 (dtd, *J* = 10.4, 5.3, 2.6 Hz, 3H). 1.64 (s, 6H).

### Example 548: 2-(2,6-dioxopiperidin-3-yl)-5-(((1r,3r)-3-(4-(2-(2-hydroxylpropan-2-yl)pyrimidin-5-yl)oxy)phenyl)propan-2-yl)phenoxy)cyclobutyl)amino)isoindolin-1,3-dione

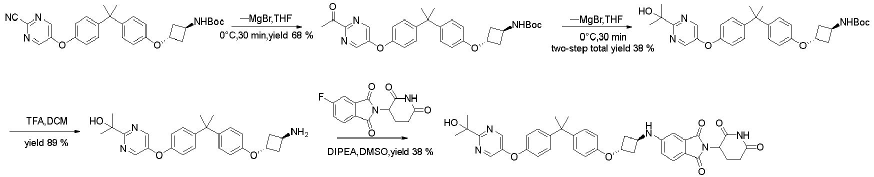

### Step 1: Synthesis of tert-butyl ((1r,3r)-3-(4-(2-(4-((2-acetylpyrimidin-5-yl)oxy)phenyl)propan-2-yl)phenoxy)cyclobutyl)carbamate

tert-butyl ((1*r*,3*r*)-3-(4-(2-(4-((2-cyanopyrimidin-5-yl)oxy)phenyl)propan-2-yl) phenoxy)cyclobutyl)carbamate (200 mg, 0.399 mmol) was dissolved in anhydrous tetrahydrofuran, to which was added methylmagnesium bromide (4 mL, 1.99 mmol) in an ice bath at -78 °C, and then the reaction was allowed to react for half an hour at 0 °C, to provide the target product as colorless oil (206 mg, 68%). LC/MS (ESI+) calcd for C₃₇H₃₄N₄O₆, ([M+H]⁺) *m*/*z* 518.3, found 518.4.

### Step 2: Synthesis of tert-butyl ((1r,3r)-3-(4-(2-(4-(2-hydroxylpropan-2-yl)pyrimidin-5-yl)oxy)phenyl)propan-2-yl)phenoxy)cyclobutyl)carbamate

tert-butyl ((1*r*,3*r*)-3-(4-(2-(4-((2-acetylpyrimidin-5-yl)oxy)phenyl)propan-2-yl) phenoxy)cyclobutyl)carbamate (206 mg) was dissolved in anhydrous tetrahydrofuran, to which was added methylmagnesium bromide (4 mL, 1 mol/L) in an ice bath at -78 °C, and then the reaction was allowed to react for half an hour at 0 °C, to provide the compound (80 mg, two-step total yield 68%). LC/MS (ESI+) calcd for C₃₁H₃₉N₃O₅, ([M+H]⁺) *m*/*z* 534.3, found 534.1.

### Step 3: Synthesis of 2-(5-(4-(4-((1r,3r)-3-aminocyclobutyloxy)phenyl)propan-2-yl)phenoxy)pyrimidin-2-yl)propan-2-ol

tert-butyl ((1*r*,3*r*)-3-(4-(2-(4-(2-hydroxylpropan-2-yl)pyrimidin-5-yl)oxy)phenyl) propan-2-yl)phenoxy)cyclobutyl)carbamate (80 mg, 0.149 mmol) was dissolved in anhydrous dichloromethane, to which was added 20 mL of trifluoroacetic acid at 0 °C in an ice-water bath. 20 mL of saturated NaHCO₃ solution was added to quench the reaction, and then the resultant solution was extracted with dichloromethane (40 mL) for three times. After suction filtration, the combined organic phase was dried over anhydrous Na₂SO₄, and rotatory evaporated to dry under reduced pressure. The residue was separated by TLC, to provide the target product as faint yellow oil (60 mg, 89%). LC/MS (ESI+) calcd for C₂₆H₃₁N₃O₃ ([M+H]⁺) m/z 434.2, found 434.3.

### Step 4: Synthesis of 2-(2,6-dioxopiperidin-3-yl)-5-(((1r,3r)-3-(4-(2-(2-hydroxylpropan-2-yl)pyrimidin-5-yl)oxy)phenyl)propan-2-yl)phenoxy)cyclobutyl) amino)isoindolin-1,3-dione

2-(5-(4-(4-((1*r*,3*r*)-3-aminocyclobutyloxy)phenyl)propan-2-yl)phenoxy) pyrimidin-2-yl)propan-2-ol (60 mg, 0.184 mmol) was dissolved in anhydrous DMSO, to which were added DIPEA (75.4 mg, 0.553mmol) and 2-(2,6-dioxopiperidin-3-yl)-4-fluoroisoindolin-1,3-dione (76 mg, 0.276 mmol), and then the system was purged with argon for three times. The mixture was allowed to react overnight at 95 °C. The reaction solution was quenched with 50 mL of saturated NH₄Cl solution. The resultant solution was extracted with ethyl acetate (40 mL) for three times. After suction filtration, the combined organic phase was dried over anhydrous Na₂SO₄, and rotatory evaporated to dry under reduced pressure. The residue was separated with TLC, to provide the target product as yellow solid (40 mg, 41 %). LC/MS (ESI+) calcd for C₃₉H₃₉N₅O₇, ([M+H]⁺) m/z 690.2, found 690.1. ¹H NMR (400 MHz, Chloroform-*d*) *δ* 9.12 (s, 1H), 8.28 (s, 2H), 7.79 (d, *J* = 7.5 Hz, 1H), 7.28 (d, *J* = 1.5 Hz, 1H), 7.22 - 7.08 (m, 5H), 6.95 - 6.83 (m, 4H), 5.43 (t, *J* = 7.0 Hz, 1H), 5.35 (d, *J* = 10.8 Hz, 1H), 4.76 (pd, *J* = 7.0, 2.5 Hz, 1H), 4.05 (s, 1H), 3.82 - 3.67 (m, 1H), 2.66 - 2.57 (m, 2H), 2.44 (dt, *J* = 12.3, 6.9 Hz, 2H), 2.23 - 2.05 (m, 4H), 1.83 (s, 6H), 1.61 (s, 6H).

### Example 549: 2-(2,6-dioxopiperidin-3-yl)-5-(((1r,3r)-3-(4-(2-(hydroxylmethyl) pyrimidin-5-yl)oxy)phenyl)propan-2-yl)phenoxy)cyclobutyl)amino)isoindolin-1,3-dione

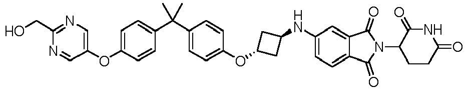

The target compound was synthesized by a method similar to that of Example 543. LC/MS (ESI+) calcd for C₃₇H₃₅N₅O₇, ([M+H]⁺) *m*/*z* 662.2, found 662.3. ¹H NMR (400 MHz, Chloroform-*d*) *δ* 8.33 (d, *J* = 5.3 Hz, 1H), 8.72 (s, 1H), 7.51 (d, *J* = 8.2 Hz, 1H), 7.13 (t, *J* = 8.3 Hz, 4H), 6.73 (d, *J* = 2.1 Hz, 1H), 6.88 - 6.80 (m, 2H), 6.74 - 6.61 (m, 3H), 4.93 (dd, *J* = 12.1, 5.2 Hz, 1H), 4.79 (s, 2H), 4.31 - 4.11 (m, 1H), 3.1 - 2.8(m,1H),2.91 - 2.79 (m, 2H), 2.79 - 2.71 (m, 1H), 2.31 (t, *J* = 6.8 Hz, 2H), 2.12 (t, *J* = 10.4 Hz, 3H), 1.65 (s, 6H).

### Example 550: 5-((1r,3r)-3-(4-(2-(4-(5-(dimethylamino)pyrazin-2-yl)oxy)phenyl) propan-2-yl)phenoxy)cyclobutylamino)-2-(2,6-dioxopiperidin-3-yl)isoindolin-1,3-dione

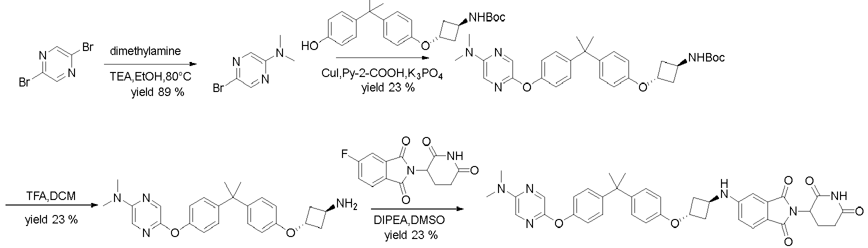

### Step 1: Synthesis of 5-bromo-N,N-dimethylpyrazin-2-amine

2,5-dibromopyrazine (500 mg, 2.1 mmol) was dissolved in absolute ethanol, to which were added dimethylamine (283 mg, 6.3 mmol) and triethylamine (1.27 g, 12.6 mmol), and then the mixture was allowed to react overnight at 80 °C. The reaction solution was rotatory evaporated to dry, and the residue was separated by TLC, to provide the target product as yellow solid (380 mg, 89 %). LC/MS (ESI+) calcd for C₆H₈BrN₃, ([M+H]⁺) *m*/*z* 201.0, found 201.1.

### Step 2: tert-butyl ((1r,3r)-3-(4-(2-(4-((5-(dimethylamino)pyrazin-2-yl)oxy)phenyl) propan-2-yl)phenoxy)cyclobutyl)carbamate

5-Bromo-N,N-dimethylpyrazin-2-amine (100 mg, 0.49 mmol) was dissolved in anhydrous DMSO, to which were added tert-butyl ((1*s*,3*s*)-3-(4-(2-(4-hydroxylphenyl) propan-2-yl)phenoxy)cyclobutyl)carbamate (178 mg, 0.45 mmol), CuI (267 mg, 0.135 mmol), 2-picolinic acid (16 mg, 0.135 mmol), and potassium phosphate (272 mg, 1.35 mmol), and then the system was purged with argon for three times. The mixture was stirred and reacted for 1.5 h at 100 °C. The reaction solution was quenched with 50 mL of saturated NH₄Cl solution. The resultant solution was extracted with ethyl acetate (40 mL). After suction filtration, the combined organic phase was dried over anhydrous Na₂SO₄, and rotatory evaporated to dry under reduced pressure. The residue was separated with TLC, to provide the target product as faint yellow solid (63 mg, 23%). LC/MS (ESI+) calcd for C₃₀H₃₈N₄O₄, ([M+H]⁺) *m*/*z* 519.3, found 591.2.

### Step 3: Synthesis of 5-(4-(2-(4-((1r,3r)-3-aminocyclobutyloxy)phenyl)propan-2-yl)phenoxy)-N,N-dimethylpyrazin-2-amine

tert-butyl ((1*r*,3*r*)-3-(4-(2-(4-((5-(dimethylamino)pyrazin-2-yl)oxy)phenyl) propan-2-yl)phenoxy)cyclobutyl)carbamate (53 mg, 0.09 mmol) was dissolved in anhydrous dichloromethane (20 mL), to which was added trifluoroacetic acid (4 mL) at 0 °C in an ice bath. 20 mL of saturated NaHCO₃ solution was added to quench the reaction, and then the resultant solution was extracted with dichloromethane (40 mL) for three times. After suction filtration, the combined organic phase was dried over anhydrous Na₂SO₄, and rotatory evaporated to dry under reduced pressure. The residue was separated by TLC, to provide the target product as faint yellow oil (13 mg, 32%). LC/MS (ESI+) calcd for C₂₅H₃₀N₄O₂, ([M+H]⁺) *m*/*z* 419.2, found 419.1.

### Step 4: Synthesis of 5-((1r,3r)-3-(4-(2-(4-(5-(dimethylamino)pyrazin-2-yl)oxy) phenyl)propan-2-yl)phenoxy)cyclobutylamino)-2-(2,6-dioxopiperidin-3-yl)isoindolin-1,3-dione

5-(4-(2-(4-((1*r*,3*r*)-3-aminocyclobutyloxy)phenyl)propan-2-yl)phenoxy)-N,N-dimethylpyrazin-2-amine (13 mg, 0.03 mmol) was dissolved in anhydrous DMSO, to which were added DIPEA (0.1 mL) and 2-(2,6-dioxopiperidin-3-yl)-4-fluoroisoindolin-1,3-dione (17 mg, 0.06 mmol), and then the system was purged with argon for three times. The mixture was allowed to react overnight at 95 °C. The reaction solution was quenched with 50 mL of saturated NH₄Cl solution. The resultant solution was extracted with ethyl acetate (40 mL) for three times. After suction filtration, the combined organic phase was dried over anhydrous Na₂SO₄, and rotatory evaporated to dry under reduced pressure. The residue was separated with TLC, to provide the target product as yellow solid (5 mg, 23%).
LC/MS (ESI+) calcd for C₃₈H₃₈N₆O₆, ([M+H]⁺) *m*/*z* 675.29, found 675.26.
¹H NMR (400 MHz, Chloroform-*d*) *δ* 9.12 (s, 1H), 7.79 (d, *J* = 7.5 Hz, 1H), 7.68 (s, 1H), 7.45 (s, 1H), 7.28 (d, *J* = 1.5 Hz, 1H), 7.23 -7.14 (m, 4H), 7.11 (dd, *J* = 7.5, 1.5 Hz, 1H), 6.90-6.84 (m, 2H), 6.84 - 6.76 (m, 2H), 5.43 (t, *J* = 7.0 Hz, 1H), 5.35 (d, *J* = 10.8 Hz, 1H), 4.76 (pd, *J* = 7.0, 2.5 Hz, 1H), 3.82 - 3.67 (m, 1H), 3.13 (s, 6H), 2.66 - 2.57 (m, 2H), 2.44 (dt, *J* = 12.3, 6.9 Hz, 2H), 2.23 - 2.05 (m, 4H), 1.61 (s, 6H).

### Example 551: 2-(2,6-dioxopiperidin-3-yl)-5-(((1r,3r)-3-(4-(2-((5-(methylamino) pyrazin-2-yl)oxy)phenyl)propan-2-yl)phenoxy)cyclobutyl)amino)isoindolin-1,3-dione

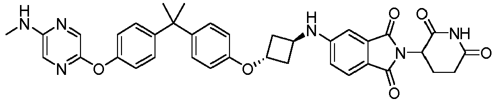

The target compound was synthesized by a method similar to that of Example 550. LC/MS (ESI+) calcd for C₃₇H₃₆N₆O₆, ([M+H]⁺) *m*/*z* 660.2, found 660.4. ¹H NMR (400 MHz, Chloroform-*d*) *δ* 8.05 (s, 1H), 7.85 (s, 1H), 7.66 - 7.62 (m, 2H), 7.22 (dd, *J* = 9.1, 2.6 Hz, 2H), 7.14 (d, *J* = 8.8 Hz, 2H), 6.94 (d, *J* = 8.7 Hz, 2H), 6.73 - 6.69 (m, 3H), 4.97 - 4.83 (m, 3H), 4.22 (d, *J* = 4.9 Hz, 1H), 3.04 (s, 3H), 2.92 - 2.83 (m, 2H), 2.75 - 2.68 (m, 3H), 2.41 (dt, *J* = 13.5, 6.0 Hz, 3H), 2.16 - 2.09 (m, 2H), 1.66 (s, 6H).

### Example 552: 2-(2,6-dioxopiperidin-3-yl)-5-(((1r,3r)-3-(4-(4-((5-methoxypyrazin-2-yl)oxy)phenyl)propan-2-yl)phenoxy)amino)isoindolin-1,3-dione

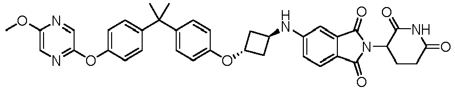

The target compound was synthesized by a method similar to that of Example 550. LC/MS (ESI+) calcd for C₃₇H₃₅N₅O₇ ([M+H]⁺) *m*/*z* 662.2 found 662.1. ¹H NMR (400 MHz, Chloroform-*d*) *δ* 8.12 (s, 2H), 7.91 (d, *J* = 4.4 Hz, 2H), 7.57 (dd, *J* = 6.9, 2.3 Hz, 3H), 7.22 (d, *J* = 8.8 Hz, 2H), 6.97 (d, *J* = 8.8 Hz, 2H), 6.70 (d, *J* = 8.8 Hz, 2H), 5.00 (d, *J* = 5.5 Hz, 2H), 3.95 (s, 3H), 2.96 - 2.94 (m, 1H), 2.48 - 2.33 (m, 2H), 2.24 - 2.09 (m, 6H), 1.66 (s, 6H).

### Example 553: 2-(2,6-dioxopiperidin-3-yl)-5-(((1s,3s)-3-(4-(4-((2-(5-methyl-1,2,4-oxadiazol-3-yl)pyrimidin-5-yl)oxy)phenyl)pentan-3-yl)phenoxy)cyclobutyl)amino) isoindolin-1,3-dione

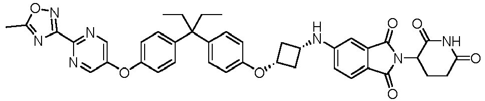

The target compound was synthesized by a method similar to that of Example 221. LC/MS (ESI+) calcd for C₄₁H₃₉N₇O₇ ([M+H]⁺) *m*/*z* 742.2 found 742.3. ¹H NMR (400 MHz, Chloroform-*d*) *δ* 9.12 (s, 1H), 8.60 (s, 2H), 7.78 (d, *J* = 7.5 Hz, 1H), 7.29 (d, *J* = 1.4 Hz, 1H), 7.16 - 7.04 (m, 5H), 6.92 - 6.83 (m, 4H), 5.45 (t, *J* = 7.0 Hz, 1H), 5.35 (d, *J* = 10.8 Hz, 1H), 4.71 (p, *J* = 7.0 Hz, 1H), 3.74 (dp, *J* = 10.8, 7.0 Hz, 1H), 2.66 - 2.57 (m, 5H), 2.50 - 2.31 (m, 4H), 2.23 - 2.05 (m, 2H), 2.03 - 1.80 (m, 4H), 0.85 (t, *J* = 8.0 Hz, 6H).

### Example 554: 4-((3-(4-(2-(4-((2-(azetidin-1-yl)pyrimidin-4-yl)methoxy)phenyl) propan-2-yl)phenoxy)propyl)amino)-2-(2,6-dioxopiperidin-3-yl)isoindolin-1,3-dione

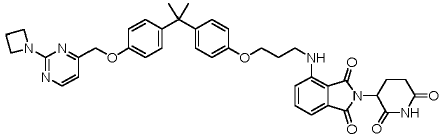

The target compound was synthesized by a method similar to that of Example 61. ¹H NMR (400 MHz, DMSO-*d*₆) *δ* 11.10 (s, 1H), 8.32 (d, *J* = 5.0 Hz, 1H), 7.55 (dd, *J* = 8.6, 7.1 Hz, 1H), 7.10 (dq, *J* = 8.9, 3.0 Hz, 5H), 7.02 (d, *J* = 7.0 Hz, 1H), 6.86 (tt, *J* = 9.9, 2.7 Hz, 4H), 6.72 (dd, *J* = 9.3, 5.5 Hz, 2H), 5.05 (dd, *J* = 12.9, 5.4 Hz, 1H), 4.94 (s, 2H), 4.02 (dd, *J* = 9.9, 5.1 Hz, 6H), 3.47 (d, *J* = 6.3 Hz, 2H), 2.85 (t, *J* = 7.0 Hz, 1H), 2.63 - 2.52 (m, 2H), 2.35 - 2.23 (m, 2H), 2.07 - 1.96 (m, 3H), 1.55 (d, *J* = 9.1 Hz, 6H). LC/MS (ESI+) calcd for C₃₉H₄₀N₆O₆ (M + H⁺) *m*/*z* 689.3; found, 689.3.

### Example 555: 2-(2,6-dioxopiperidin-3-yl)-4-((3-(4-(2-(2-(piperidin-1-yl) pyrimidin-4-yl)methoxy)phenyl)propan-2-yl)phenoxy)propyl)amino)isoindolin-1,3-dione

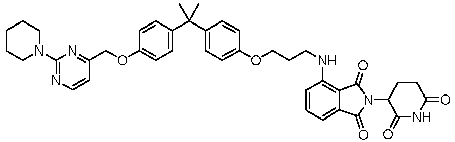

The target compound was synthesized by a method similar to that of Example 61. ¹H NMR (400 MHz, DMSO-*d*₆) *δ* 11.10 (s, 1H), 8.32 (d, *J* = 4.9 Hz, 1H), 7.55 (dd, *J* = 8.6, 7.1 Hz, 1H), 7.10 (dq, *J* = 8.8, 3.0 Hz, 5H), 7.02 (d, *J* = 7.0 Hz, 1H), 6.90 - 6.83 (m, 4H), 6.74 (t, *J* = 5.9 Hz, 1H), 6.62 (d, *J* = 4.9 Hz, 1H), 5.05 (dd, *J* = 12.9, 5.4 Hz, 1H), 4.94 (s, 2H), 4.03 (t, *J* = 5.9 Hz, 2H), 3.72 (t, *J* = 5.5 Hz, 4H), 3.47 (q, *J* = 6.5 Hz, 2H), 2.88 (ddd, *J* = 17.4, 14.0, 5.4 Hz, 1H), 2.63 - 2.52 (m, 2H), 2.04 - 1.98 (m, 3H), 1.61 (d, *J* = 5.5 Hz, 2H), 1.57 (s, 6H), 1.48 (tt, *J* = 7.6, 5.1, 4.0 Hz, 4H). LC/MS (ESI+) calcd for C₄₀H₄₂N₆O₆(M + H⁺) *m*/*z* 703.3; found, 703.3.

### Example 557: 5-(4-(4-(2-(2-acetylpyrimidin-4-yl)methoxy)phenyl)propan-2-yl) phenoxy)piperidin-1-yl)-2-(2,6-dioxopiperidin-3-yl)isoindolin-1,3-dione

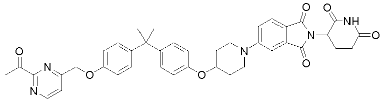

The target compound was synthesized by a method similar to that of Example 485. ¹H NMR (400 MHz, DMSO-*d*₆) *δ* 11.08 (s, 1H), 9.02 (d, *J* = 5.1 Hz, 1H), 7.77 (d, *J* = 5.1 Hz, 1H), 7.67 (d, *J* = 8.6 Hz, 1H), 7.36 (d, *J* = 2.3 Hz, 1H), 7.27 (dd, *J* = 8.6, 2.4 Hz, 1H), 7.17 - 7.09 (m, 4H), 6.99 - 6.94 (m, 2H), 6.92 - 6.86 (m, 2H), 5.27 (s, 2H), 5.07 (dd, *J* = 12.9, 5.4 Hz, 1H), 4.60 (t, *J* = 3.8 Hz, 1H), 3.86 - 3.74 (m, 2H), 3.44 - 3.36 (m, 2H), 2.67 (s, 3H), 2.59 - 2.53 (m, 2H), 2.10 - 1.95 (m, 4H), 1.72 - 1.63 (m, 2H), 1.59 (s, 6H). LC/MS (ESI+) calcd for C₄₂H₄₃N₅O₇S (M + H⁺) *m*/*z* 511.2; found, 511.2.

### Example 558: 5-(3-(4-(2-(2-acetylpyrimidin-4-yl)methoxy)phenyl)propan-2-yl) phenoxy)azetidin-1-yl)-2-(2,6-dioxopiperidin-3-yl)isoindolin-1,3-dione

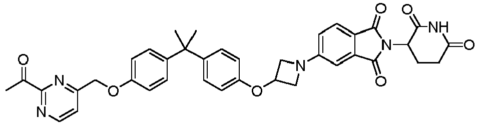

The target compound was synthesized by a method similar to that of Example 485. ¹H NMR (400 MHz, DMSO-*d*₆) *δ* 11.09 (s, 1H), 9.02 (d, *J* = 5.1 Hz, 1H), 7.77 (d, *J* = 5.1 Hz, 1H), 7.66 (d, *J* = 8.2 Hz, 1H), 7.15 (dd, *J* = 8.8, 3.6 Hz, 4H), 7.02 - 6.93 (m, 2H), 6.87 (d, *J* = 2.1 Hz, 1H), 6.79 (d, *J* = 8.4 Hz, 2H), 6.72 (dd, *J* = 8.4, 2.1 Hz, 1H), 5.27 (s, 2H), 5.18 (t, *J* = 6.2 Hz, 1H), 5.06 (dd, *J* = 12.9, 5.4 Hz, 1H), 4.49 (dd, *J* = 9.6, 6.3 Hz, 2H), 4.01 (dd, *J* = 9.6, 3.7 Hz, 2H), 2.67 (s, 3H), 2.54 (s, 2H), 2.14 - 1.94 (m, 2H), 1.59 (s, 6H). LC/MS (ESI+) calcd for C₃₈H₃₅N₅O₇ (M + H⁺) *m*/*z* 674.2; found, 674.2.

### Example 559: 5-(2-(4-(2-(2-acetylpyrimidin-4-yl)methoxy)phenyl)propan-2-yl) phenyl)-2,7-diazaspiro[3.5]nonan-7-yl)-2-(2,6-dioxopiperidin-3-yl)isoindolin-1,3-dione

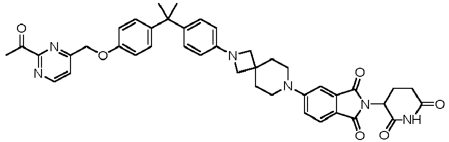

The target compound was synthesized by a method similar to that of Example 487. ¹H NMR (400 MHz, DMSO-*d*₆) *δ* 11.08 (s, 1H), 9.01 (d, *J* = 5.1 Hz, 1H), 7.76 (d, *J* = 5.1 Hz, 1H), 7.66 (d, *J* = 8.6 Hz, 1H), 7.35 (d, *J* = 2.3 Hz, 1H), 7.27 (dd, *J* = 8.8, 2.3 Hz, 1H), 7.16 - 7.09 (m, 2H), 7.04 - 6.91 (m, 4H), 6.38 - 6.30 (m, 2H), 5.26 (s, 2H), 5.07 (dd, *J* = 12.8, 5.4 Hz, 1H), 3.56 (s, 4H), 3.49 (d, *J* = 6.4 Hz, 4H), 2.95 - 2.82 (m, 1H), 2.67 (s, 3H), 2.63 - 2.53 (m, 2H), 2.07 - 1.96 (m, 1H), 1.87 - 1.76 (m, 4H), 1.55 (s, 6H). LC/MS (ESI+) calcd for C₄₂H₄₂N₆O₆(M + H⁺) *m*/*z* 727.3; found,727.3.

### Example 560: 5-(7-(4-(2-(2-acetylpyrimidin-4-yl)methoxy)phenyl)propan-2-yl) phenyl)-2,7-diazaspiro[4.4]nonan-2-yl)-2-(2,6-dioxopiperidin-3-yl)isoindolin-1,3-dione

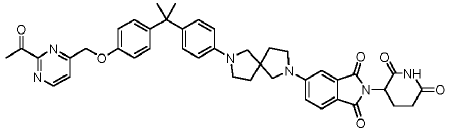

The target compound was synthesized by a method similar to that of Example 487. ¹H NMR (400 MHz, DMSO-*d*₆) *δ* 11.07 (s, 1H), 9.01 (d, *J* = 5.0 Hz, 1H), 7.76 (d, *J* = 5.1 Hz, 1H), 7.64 (d, *J* = 8.4 Hz, 1H), 7.15 - 7.09 (m, 2H), 7.02 - 6.97 (m, 2H), 6.93 (dd, *J* = 9.4, 2.5 Hz, 3H), 6.82 (dd, *J* = 8.5, 2.2 Hz, 1H), 6.48 - 6.38 (m, 2H), 5.26 (s, 2H), 5.05 (dd, *J* = 12.9, 5.4 Hz, 1H), 3.55 (t, *J* = 6.8 Hz, 2H), 3.49 - 3.40 (m, 2H), 3.27 - 3.19 (m, 2H), 2.95 - 2.81 (m, 1H), 2.67 (s, 3H), 2.57 (dd, *J* = 20.5, 3.1 Hz, 2H), 2.12 - 1.92 (m, 6H), 1.55 (s, 7H). LC/MS (ESI+) calcd for C₄₂H₄₂N₆O₆ (M + H⁺) *m*/*z* 727.3; found,727.3.

### Example 561:5-(6-(4-(2-(2-(2-acetylpyrimidin-4-yl)methoxy)phenyl)propan-2-yl) phenoxy)-2-azaspiro[3.3]heptan-2-yl)-2-(2,6-dioxopiperidin-3-yl)isoindolin-1,3-dione

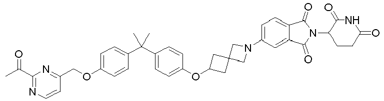

The target compound was synthesized by a method similar to that of Example 485. ¹H NMR (400 MHz, DMSO-*d*₆) *δ* 11.07 (s, 1H), 9.02 (d, *J* = 5.1 Hz, 1H), 7.77 (d, *J* = 5.1 Hz, 1H), 7.64 (d, *J* = 8.3 Hz, 1H), 7.17 - 7.06 (m, 4H), 6.99 - 6.93 (m, 2H), 6.79 - 6.71 (m, 3H), 6.63 (dd, *J* = 8.4, 2.1 Hz, 1H), 5.27 (s, 2H), 5.05 (dd, *J* = 12.9, 5.4 Hz, 1H), 4.64 (p, *J* = 6.7 Hz, 1H), 4.07 (d, *J* = 27.6 Hz, 4H), 2.94 - 2.74 (m, 3H), 2.67 (d, *J* = 2.3 Hz, 3H), 2.62 - 2.54 (m, 1H), 2.34 - 2.26 (m, 2H), 2.06 - 1.94 (m, 2H), 1.58 (s, 6H). LC/MS (ESI+) calcd for C₄₁H₃₉N₅O₇(M + H⁺) m/z 714.3; found, 714.3.

### Example 562: 2-(2,6-dioxopiperidin-3-yl)-5-(2-(2-(2-(1-ethoxyvinyl)pyrimidin-4-yl)methoxy)phenyl)propan-2-yl)phenoxy)-6-azaspiro[3.4]octane-6-yl)isoindolin-1,3-dione

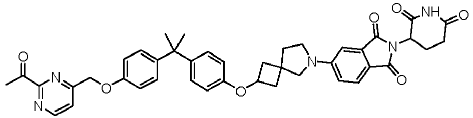

The target compound was synthesized by a method similar to that of Example 485. ¹H NMR (400 MHz, DMSO-*d*₆) *δ* 11.07 (s, 1H), 9.01 (d, *J* = 5.1 Hz, 1H), 7.76 (d, *J* = 5.1 Hz, 1H), 7.65 - 7.61 (m, 1H), 7.11 (ddd, *J* = 17.5, 8.9, 2.5 Hz, 4H), 6.96 (dt, *J* = 6.6, 3.4 Hz, 2H), 6.89 (d, *J* = 10.2 Hz, 1H), 6.76 (dt, *J* = 8.7, 6.5 Hz, 3H), 5.26 (s, 2H), 5.13 - 5.00 (m, 1H), 4.76 (dd, *J* = 9.8, 5.8 Hz, 1H), 3.57 - 3.40 (m, 4H), 2.67 (s, 3H), 2.57 (d, *J* = 22.1 Hz, 2H), 2.19 - 1.93 (m, 6H), 1.57 (s, 6H), 1.35 (d, *J* = 12.5 Hz, 2H). LC/MS (ESI+) calcd for C₄₂H₄₁N₅O₇(M + H⁺) *m*/*z* 728.3; found, 728.3.

### Example 563: 5-((3-(4-(2-(4-((2-(2-oxa-6-azaspiro[3.3]heptan-6-yl)pyrimidin-4-yl) methoxy)phenyl)propan-2-yl)phenoxy)cyclopentyl)amino)-2-(2,6-dioxopiperidin-3-yl)isoindolin-1,3-dione

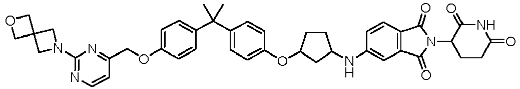

The target compound was synthesized by a method similar to that of Example 61. ¹H NMR (400 MHz, DMSO-*d*₆) *δ* 11.06 (s, 1H), 8.33 (d, *J* = 4.9 Hz, 1H), 7.57 (d, *J* = 8.4 Hz, 1H), 7.21 (d, *J* = 6.4 Hz, 1H), 7.14 - 7.07 (m, 4H), 6.94 (d, *J* = 2.1 Hz, 1H), 6.91 - 6.86 (m, 2H), 6.83 - 6.78 (m, 2H), 6.74 (d, *J* = 5.0 Hz, 1H), 5.32 (t, *J* = 4.8 Hz, 1H), 5.03 (dd, *J* = 12.9, 5.4 Hz, 1H), 4.95 (s, 2H), 4.88 (s, 1H), 4.71 (s, 4H), 4.19 (s, 4H), 4.13 - 3.96 (m, 2H), 2.95 - 2.79 (m, 2H), 2.24 - 2.11 (m, 3H), 1.99 (p, *J* = 7.4 Hz, 4H), 1.57 (s, 6H). LC/MS (ESI+) calcd for C₄₃H₄₄N₆O₇(M + H⁺) *m*/*z* 757.3; found,757.3.

### Example 564:5-((1-((4-(2-(4-((2-(2-oxa-6-azaspiro[3.3]heptan-6-yl)pyrimidin-4-yl)methoxy)phenyl)propan-2-yl)phenoxy)methyl)cyclopropyl)amino)-2-(2,6-dioxopiperidin-3-yl)isoindolin-1,3-dione

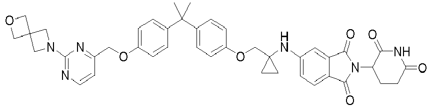

The target compound was synthesized by a method similar to that of Example 75. ¹H NMR (400 MHz, DMSO-*d*₆) *δ* 11.06 (s, 1H), 8.33 (dd, *J* = 5.0, 2.4 Hz, 1H), 7.75 (s, 1H), 7.58 (d, *J* = 8.3 Hz, 1H), 7.18 - 7.14 (m, 1H), 7.05 (ddd, *J* = 9.7, 5.9, 2.9 Hz, 4H), 6.89 - 6.83 (m, 2H), 6.80 - 6.71 (m, 3H), 5.03 (dd, *J* = 12.9, 5.4 Hz, 1H), 4.93 (s, 2H), 4.71 (s, 4H), 4.19 (s, 4H), 4.04 - 3.97 (m, 2H), 2.87 (ddd, *J* = 17.3, 13.7, 5.4 Hz, 1H), 2.57 (d, *J* = 16.6 Hz, 1H), 1.99 (s, 2H), 1.54 (s, 6H), 1.23 (s, 2H), 1.17 (t, *J=* 7.1 Hz, 1H), 1.06 (q, *J* = 4.6, 4.1 Hz, 2H). LC/MS (ESI+) calcd for C₄₂H₄₂N₆O₇(M + H⁺) *m*/*z* 743.3; found, 743.3.

### Example 565: 5-((R)-2-((4-(2-(4-((2-(2-oxa-6-azaspiro[3.3]heptan-6-yl)pyrimidin-4-yl)methoxy)phenyl)propan-2-yl)phenoxy)methyl)pyrrolidin-1-yl)methyl)-2-(2,6-dioxopiperidin-3-yl)isoindolin-1,3-dione

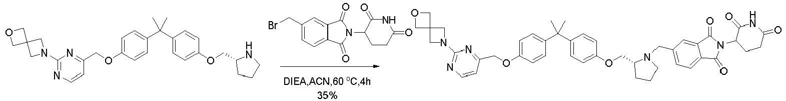

Compound (*R*)-6-(4-((4-(2-(4-(pyrrolidin-2-ylmethoxy)phenyl)propan-2-yl) phenoxy)methyl)pyrimidin-2-yl)-2-oxa-6-azaspiro[3.3]heptane (75 mg, 0.15 mmol), 5-(bromomethyl)-2-(2,6-dioxopiperidin-3-yl)isoindolin-1,3-dione (52 mg, 0.15 mmol) and DIEA (58 mg, 0.45 mmol) were added into 6 mL of ACN, and then the solution was stirred overnight at 60 °C. The reaction solution was added with water, and the extracted with water. The resultant solution was extracted with EA for three times. The organic phase was combined, washed with saturated NaCl solution for three times, dried over anhydrous Na₂SO₄, and rotatory evaporated to dry. The residue was purified by column chromatography, to obtain the target compound (39 mg, 0.05 mmol), with a yield of 35 %. ¹H NMR (400 MHz, DMSO-*d*₆) *δ* 11.13 (s, 1H), 8.33 (d, *J* = 5.0 Hz, 1H), 7.82 (dd, *J* = 18.3, 8.2 Hz, 3H), 7.09 (t, *J* = 8.8 Hz, 4H), 6.91 - 6.79 (m, 4H), 6.74 (d, *J* = 5.0 Hz, 1H), 5.14 (dd, *J* = 12.9, 5.3 Hz, 1H), 4.94 (s, 2H), 4.71 (s, 4H), 4.29 (d, *J* = 14.1 Hz, 1H), 4.19 (s, 4H), 4.06 - 3.97 (m, 2H), 3.85 (dd, *J* = 9.6, 6.0 Hz, 1H), 3.64 (d, *J* = 14.1 Hz, 1H), 3.04 - 2.77 (m, 3H), 2.60 (d, *J* = 17.8 Hz, 1H), 2.26 (q, *J* = 8.4 Hz, 1H), 2.08 - 2.00 (m, 1H), 1.92 (d, *J* = 12.1 Hz, 1H), 1.68 (dt, *J* = 18.5, 10.4 Hz, 3H), 1.56 (s, 6H). LC/MS (ESI+) calcd for C₄₄H₄₆N₆O₇(M + H⁺) *m*/*z* 771.3; found 771.3.

### Example 566:5-((S)-2-((4-(2-(4-((2-(2-oxa-6-azaspiro[3.3]heptane-6-yl)pyrimidin-4-yl)methoxy)phenyl)propan-2-yl)phenoxy)methyl)azetidine-1-yl)methyl)-2-(2,6-dioxopiperidin-3-yl)isoindolin-1,3-dione

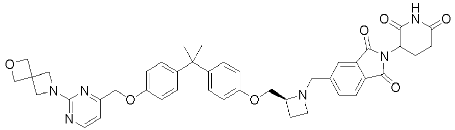

The target compound was synthesized by a method similar to that of Example 565. LC/MS (ESI+) calcd for C₄₃H₄₄N₆O₇(M + H⁺) *m*/*z* 757.3; found 757.3. ¹H NMR (400 MHz, DMSO-*d*₆) *δ* 11.13 (s, 1H), 8.33 (d, *J* = 5.0 Hz, 1H), 7.86 - 7.72 (m, 3H), 7.13 - 7.02 (m, 4H), 6.87 (d, *J* = 8.5 Hz, 2H), 6.78 - 6.69 (m, 3H), 5.13 (dd, *J* = 12.9, 5.4 Hz, 1H), 4.94 (s, 2H), 4.71 (s, 4H), 4.19 (s, 4H), 4.06 - 3.90 (m, 4H), 3.68 (d, *J* = 14.2 Hz, 1H), 3.62 (dd, *J* = 9.7, 4.5 Hz, 1H), 3.22 (d, *J* = 7.4 Hz, 1H), 2.96 - 2.81 (m, 2H), 2.64 - 2.56 (m, 1H), 2.13 - 2.01 (m, 2H), 1.96 (d, *J* = 9.9 Hz, 1H), 1.55 (s, 6H).

### Example 567: 5-((6-(4-(2-(4-((2-(2-oxa-6-azaspiro[3.3]heptan-6-yl)pyrimidin-4-yl) methoxy)phenyl)propan-2-yl)phenoxy)spiro[3.3]heptan-2-yl)amino)-2-(2,6-dioxopiperidin-3-yl)isoindolin-1,3-dione

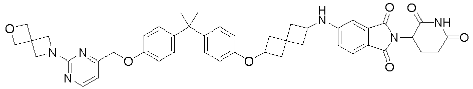

The target compound was synthesized by a method similar to that of Example 61. ¹H NMR (400 MHz, DMSO-*d*₆) *δ* 11.06 (s, 1H), 8.33 (d, *J* = 5.0 Hz, 1H), 7.56 (d, *J* = 8.3 Hz, 1H), 7.36 (d, *J* = 6.0 Hz, 1H), 7.14 - 7.04 (m, 4H), 6.92 - 6.83 (m, 3H), 6.81 - 6.67 (m, 4H), 5.03 (dd, *J* = 12.9, 5.4 Hz, 1H), 4.95 (s, 2H), 4.71 (s, 4H), 4.58 (p, *J* = 6.9 Hz, 1H), 4.19 (s, 4H), 3.93 (q, *J* = 7.1 Hz, 1H), 2.94 - 2.82 (m, 1H), 2.68 (dt, *J* = 11.2, 5.8 Hz, 1H), 2.64 - 2.54 (m, 2H), 2.15 - 1.91 (m, 6H), 1.55 (d, *J* = 7.5 Hz, 6H), 1.17 (t, *J* = 7.1 Hz, 2H). LC/MS (ESI+) calcd for C₄₅H₄₆N₆O₇(M + H⁺) *m*/*z* 783.3; found,783.3.

### Example 568: 2-(4-(2-(2-oxa-6-azaspiro[3.3]heptan-6-yl)pyrimidin-4-yl)methoxy) phenyl)propan-2-yl)phenoxy)-N-(2-(2,6-dioxopiperidin-3-yl)-1,3-dioxoisoindolin-5-yl)acetamide

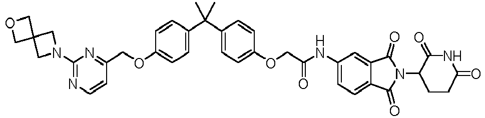

The target compound was synthesized by a method similar to that of Example 504. ¹H NMR (400 MHz, DMSO-*d*₆) *δ* 11.13 (s, 1H), 10.74 (s, 1H), 8.38 - 8.25 (m, 2H), 8.02 (dd, *J* = 8.2, 1.9 Hz, 1H), 7.90 (d, *J* = 8.2 Hz, 1H), 7.19 - 7.06 (m, 4H), 6.95 - 6.85 (m, 4H), 6.74 (d, *J* = 5.0 Hz, 1H), 5.13 (dd, *J* = 12.8, 5.4 Hz, 1H), 4.94 (s, 2H), 4.75 (s, 2H), 4.71 (s, 4H), 4.19 (s, 4H), 2.95 - 2.81 (m, 1H), 2.60 (d, *J* = 17.9 Hz, 1H), 2.10 - 1.92 (m, 2H), 1.58 (s, 6H). LC/MS (ESI+) calcd for C₄₀H₃₈N₆O₈(M + H⁺) *m*/*z* 731.3; found, 731.3.

### Example 569: 5-((2-(4-(2-(4-((2-(2-oxa-6-azaspiro[3.3]heptan-6-yl)pyrimidin-4-yl) methoxy)phenyl)propan-2-yl)phenoxy)cyclohexyl)amino)-2-(2,6-dioxopiperidin-3-yl)isoindolin-1,3-dione

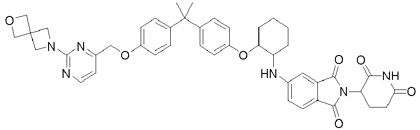

The target compound was synthesized by a method similar to that of Example 61. LC/MS (ESI+) calcd for C₄₄H₄₆N₆O₇(M + H⁺) *m*/*z* 771.3; found,771.3.

### Example 570:5-((1-(4-(2-(4-((2-(2-oxa-6-azaspiro[3.3]heptan-6-yl)pyrimidin-4-yl) methoxy)phenyl)propan-2-yl)phenyl)azetidin-3-yl)methyl)amino)-2-(2,6-dioxopiperidin-3-yl)isoindolin-1,3-dione

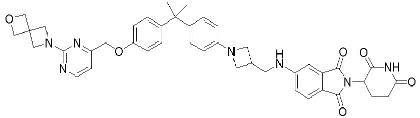

The target compound was synthesized by a method similar to that of Example 487. ¹H NMR (400 MHz, DMSO-*d*₆) *δ* 11.06 (s, 1H), 8.33 (d, *J* = 5.0 Hz, 1H), 7.57 (d, *J* = 8.3 Hz, 1H), 7.27 (t, *J* = 5.4 Hz, 1H), 7.09 (d, *J* = 8.5 Hz, 2H), 7.03 - 6.95 (m, 3H), 6.93 - 6.84 (m, 3H), 6.74 (d, *J* = 5.0 Hz, 1H), 6.33 (d, *J* = 8.2 Hz, 2H), 5.03 (dd, *J* = 12.9, 5.4 Hz, 1H), 4.94 (s, 2H), 4.71 (s, 4H), 4.19 (s, 4H), 3.85 (t, *J* = 7.4 Hz, 2H), 3.49 (dt, *J* = 19.7, 6.2 Hz, 4H), 2.96 - 2.81 (m, 2H), 2.57 (d, *J* = 16.7 Hz, 2H), 2.00 (d, *J* = 8.8 Hz, 1H), 1.54 (s, 6H). LC/MS (ESI+) calcd for C₄₂H₄₃N₇0₆(M + H⁺) *m*/*z* 742.3; found, 742.3.

### Example 571: 5-((1s,3s)-3-(4-(2-(4-((6-(2-oxa-6-azaspiro[3.3]heptane-6-yl) pyrimidin-4-yl)oxy)phenyl)propan-2-yl)phenoxy)cyclobutyl)amino)-2-(2,6-dioxapiperidin-3-yl)isoindolin-1,3-dione

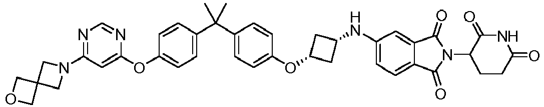

The target compound was synthesized by a method similar to that of Example 75. ¹H NMR (400 MHz, DMSO-*d*₆) *δ* 11.07 (s, 1H), 8.14 (d, *J* = 0.9 Hz, 1H), 7.59 (d, *J* = 8.4 Hz, 1H), 7.45 (d, *J* = 6.5 Hz, 1H), 7.25 - 7.11 (m, 4H), 7.04 - 6.96 (m, 2H), 6.90 (d, *J* = 2.1 Hz, 1H), 6.81 (dd, *J* = 12.1, 8.7 Hz, 3H), 5.04 (dd, *J* = 12.9, 5.4 Hz, 1H), 4.71 (s, 4H), 4.50 (p, *J* = 7.0 Hz, 1H), 4.17 (s, 4H), 3.11 - 3.00 (m, 2H), 2.87 (ddd, *J* = 17.3, 14.0, 5.4 Hz, 1H), 2.62 - 2.52 (m, 2H), 1.98 (d, *J* = 8.8 Hz, 4H), 1.62 (s, 6H). LC/MS (ESI+) calcd for C₄₁H₄₀N₆O₇(M + H⁺) *m*/*z* 729.3; found, 729.3.

### Example 572:2-(2,6-dioxopiperidin-3-yl)-5-((1r,3r)-3-(4-(2-(4-((5-fluoro-6-(2-oxa-6-azaspiro[3.3]heptan-6-yl)pyrimidin-4-yl)oxy)phenyl)propan-2-yl)phenoxy) cyclobutyl)amino)isoindolin-1,3-dione

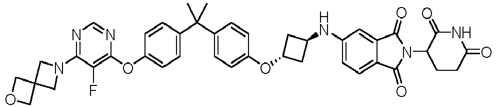

The target compound was synthesized by a method similar to that of Example 75. ¹H NMR (400 MHz, DMSO-*d*₆) *δ* 11.07 (s, 1H), 7.93 (d, *J* = 1.1 Hz, 1H), 7.59 (d, *J* = 8.3 Hz, 1H), 7.53 (d, *J* = 5.5 Hz, 1H), 7.26 - 7.21 (m, 2H), 7.18 - 7.13 (m, 2H), 7.07 - 7.02 (m, 2H), 6.86 (s, 1H), 6.82 - 6.72 (m, 3H), 5.03 (dd, *J* = 12.9, 5.4 Hz, 1H), 4.87 (p, *J* = 6.0 Hz, 1H), 4.72 (s, 4H), 4.39 (d, *J* = 1.9 Hz, 4H), 2.94 - 2.79 (m, 1H), 2.62 - 2.52 (m, 3H), 2.43 (q, *J* = 5.6 Hz, 3H), 2.00 (d, *J* = 7.7 Hz, 2H), 1.62 (s, 6H). LC/MS (ESI+) calcd for C₄₁H₃₉FN₆O₇(M + H⁺) *m*/*z* 747.3; found 747.3.

### Example 573:5-((1r,3r)-3-(4-(2-(4-((5-(2-oxa-6-azaspiro[3.3]heptane-6-yl)pyrazin-2-yl)oxy)phenyl)propan-2-yl)phenoxy)cyclobutyl)amino)-2-(2,6-dioxapiperidin-3-yl)isoindolin-1,3-dione

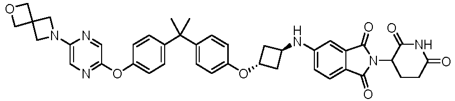

The target compound was synthesized by a method similar to that of Example 75. ¹H NMR (400 MHz, DMSO-*d*₆) *δ* 11.07 (s, 1H), 7.98 (d, *J* = 1.4 Hz, 1H), 7.59 (d, *J* = 8.3 Hz, 1H), 7.53 (q, *J* = 2.2, 1.7 Hz, 2H), 7.20 - 7.09 (m, 4H), 6.92 - 6.84 (m, 3H), 6.81 - 6.77 (m, 1H), 6.76 - 6.72 (m, 2H), 5.04 (dd, *J* = 12.9, 5.4 Hz, 1H), 4.90 - 4.82 (m, 1H), 4.71 (s, 4H), 4.14 (s, 4H), 2.87 (ddd, *J* = 17.4, 14.0, 5.4 Hz, 1H), 2.62 - 2.52 (m, 3H), 2.43 (dt, *J* = 10.8, 5.1 Hz, 3H), 2.06 - 1.91 (m, 2H), 1.59 (s, 6H). LC/MS (ESI+) calcd for C₄₁H₄₀N₆O₇(M + H⁺) *m*/*z* 729.3; found 729.3.

### Example 574: 2-(2,6-dioxopiperidin-3-yl)-5-((1s,3s)-3-(4-(2-(4-((5-fluoro-6-(2-oxa-6-azaspiro[3.3]heptan-6-yl)pyrimidin-4-yl)oxy)phenyl)propan-2-yl)phenoxy) cyclobutyl)amino)isoindolin-1,3-dione

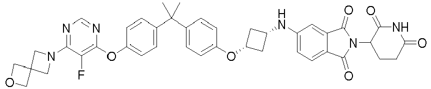

The target compound was synthesized by a method similar to that of Example 75. ¹H NMR (400 MHz, DMSO-*d*₆) *δ* 11.07 (s, 1H), 7.93 (d, *J* = 1.1 Hz, 1H), 7.59 (d, *J* = 8.3 Hz, 1H), 7.45 (d, *J* = 6.5 Hz, 1H), 7.26 - 7.19 (m, 2H), 7.16 - 7.10 (m, 2H), 7.06 - 7.00 (m, 2H), 6.90 (d, *J* = 2.0 Hz, 1H), 6.85 - 6.74 (m, 3H), 5.03 (dd, *J* = 12.9, 5.3 Hz, 1H), 4.72 (s, 4H), 4.50 (p, *J* = 6.9 Hz, 1H), 4.39 (d, *J* = 2.0 Hz, 4H), 3.05 (s, 2H), 2.87 (ddd, *J* = 17.8, 13.9, 5.4 Hz, 1H), 2.63 - 2.53 (m, 2H), 1.97 (dd, *J* = 18.1, 10.2 Hz, 4H), 1.62 (s, 6H). LC/MS (ESI+) calcd for C₄₁H₃₉FN₆O₇(M + H⁺) *m*/*z* 747.3; found 747.3.

### Example 575: 5-((1s,3s)-3-(4-(2-(4-((6-([1,3'-diazetidine]-1'-yl)-5-fluoropyrimidin-4-yl)oxy)phenyl)propan-2-yl)phenoxy)cyclobutyl)amino)-2-(2,6-dioxopyridin-3-yl)isoindolin-1,3-dione

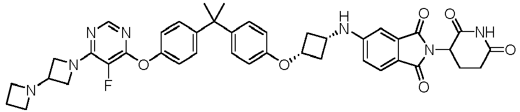

The target compound was synthesized by a method similar to that of Example 75. ¹H NMR (400 MHz, DMSO-*d*₆) *δ* 11.07 (s, 1H), 7.91 (d, *J* = 1.0 Hz, 1H), 7.59 (d, *J* = 8.3 Hz, 1H), 7.45 (d, *J* = 6.5 Hz, 1H), 7.25 - 7.20 (m, 2H), 7.18 - 7.12 (m, 2H), 7.07 - 7.02 (m, 2H), 6.90 (d, *J* = 2.1 Hz, 1H), 6.85 - 6.77 (m, 3H), 5.03 (dd, *J* = 12.9, 5.4 Hz, 1H), 4.50 (p, *J* = 7.0 Hz, 1H), 4.19 (t, *J* = 8.1 Hz, 2H), 3.93 (dd, *J* = 9.6, 4.2 Hz, 2H), 3.78 (q, *J* = 7.4 Hz, 1H), 3.19 (t, *J* = 7.0 Hz, 4H), 3.10 - 3.00 (m, 2H), 2.87 (ddd, *J* = 17.5, 14.1, 5.5 Hz, 1H), 2.57 (dd, *J* = 19.7, 6.3 Hz, 2H), 1.97 (dt, *J* = 12.9, 8.6 Hz, 6H), 1.62 (s, 6H). LC/MS (ESI+) calcd for C₄₂H₄₂FN₇O₆(M + H⁺) *m*/*z* 760.3; found 760.3.

### Example 576: 2-(2,6-dioxopiperidin-3-yl)-5-(1s,3s)-3-(4-(2-(4-((6-((1-ethylazetidin-3-yl)oxy)-5-fluoropyrimidin-4-yl)oxy)phenyl)propan-2-yl)phenoxy) cyclobutyl)amino)isoindolin-1,3-dione

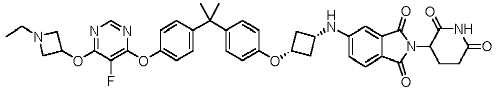

The target compound was synthesized by a method similar to that of Example 234. ¹H NMR (400 MHz, DMSO-*d*₆) *δ* 11.07 (s, 1H), 8.18 (s, 1H), 7.59 (d, *J* = 8.3 Hz, 1H), 7.45 (d, *J* = 6.4 Hz, 1H), 7.28 - 7.23 (m, 2H), 7.18 -7.10 (m, 4H), 6.90 (d, *J* = 2.1 Hz, 1H), 6.84 - 6.76 (m, 3H), 5.26 (p, *J* = 5.6 Hz, 1H), 5.04 (dd, *J* = 12.9, 5.4 Hz, 1H), 4.50 (p, *J* = 7.0 Hz, 1H), 3.77 (p, *J* = 7.4 Hz, 1H), 3.71 - 3.61 (m, 2H), 3.06 (ddd, *J* = 10.8, 5.4, 2.7 Hz, 4H), 2.62 - 2.53 (m, 2H), 2.45 (t, *J* = 7.2 Hz, 2H), 2.04 - 1.90 (m, 4H), 1.63 (s, 6H), 0.89 (t, *J* = 7.1 Hz, 3H). LC/MS (ESI+) calcd for C₄₁H₄₁FN₆O₇(M + H⁺) *m*/*z* 749.3; found 749.3.

### Example 577: 5-((1s,3s)-3-(4-(2-(4-((6-(2H-1,2,3-triazol-2-yl)pyrimidin-4-yl)oxy) phenyl)propan-2-yl)phenoxy)cyclobutyl)amino)-2-(2,6-dioxopiperidin-3-yl)isoindolin-1,3-dione

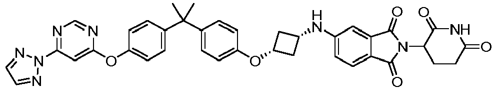

The target compound was synthesized by a method similar to that of Example 228. ¹H NMR (400 MHz, DMSO-*d*₆) *δ* 11.07 (s, 1H), 8.84 - 8.77 (m, 1H), 8.30 (s, 2H), 7.59 (d, *J* = 8.3 Hz, 1H), 7.50 - 7.42 (m, 2H), 7.35 - 7.29 (m, 2H), 7.18 (dd, *J* = 8.8, 2.6 Hz, 4H), 6.90 (d, *J* = 2.1 Hz, 1H), 6.82 (dd, *J* = 8.5, 6.6 Hz, 3H), 5.04 (dd, *J* = 12.9, 5.4 Hz, 1H), 4.51 (p, *J* = 7.0 Hz, 1H), 3.78 (q, *J* = 7.4 Hz, 1H), 3.13 - 3.00 (m, 2H), 2.64 - 2.52 (m, 2H), 1.98 (q, *J* = 9.6 Hz, 4H), 1.65 (s, 6H). LC/MS (ESI+) calcd for C₃₀H₃₄N₈O₆(M + H⁺) *m*/*z* 699.3; found 699.3.

### Example 578: 5-((1r,3r)-3-(4-(2-(4-((6-(2H-1,2,3-triazol-2-yl)pyrimidin-4-yl)oxy) phenyl)propan-2-yl)phenoxy)cyclobutyl)amino)-2-(2,6-dioxopiperidin-3-yl) isoindolin-1,3-dione

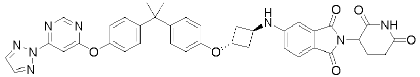

The target compound was synthesized by a method similar to that of Example 228. ¹H NMR (400 MHz, DMSO-*d*₆) *δ* 11.07 (s, 1H), 8.80 (d, *J* = 1.0 Hz, 1H), 8.30 (s, 2H), 7.59 (d, *J* = 8.3 Hz, 1H), 7.54 (d, *J* = 5.5 Hz, 1H), 7.48 (d, *J* = 1.0 Hz, 1H), 7.35 - 7.29 (m, 2H), 7.21 - 7.14 (m, 4H), 6.86 (s, 1H), 6.79 (dd, *J* = 9.1, 7.0 Hz, 3H), 5.04 (dd, *J* = 12.9, 5.4 Hz, 1H), 4.89 (q, *J* = 6.0 Hz, 1H), 4.15 (d, *J* = 5.0 Hz, 1H), 2.68 - 2.52 (m, 4H), 2.44 (q, *J* = 5.7 Hz, 2H), 2.00 (d, *J* = 7.9 Hz, 2H), 1.65 (s, 6H). LC/MS (ESI+) calcd for C₃₈H₃₄N₈O₆(M + H⁺) *m*/*z* 699.3; found 699.3.

### Example 579: 5-((1r,3r)-3-(4-(2-(4-((6-(2H-1,2,3-triazol-2-yl)pyrazin-2-yl)oxy) phenyl)propan-2-yl)phenoxy)cyclobutyl)amino)-2-(2,6-dioxopiperidin-3-yl) isoindolin-1,3-dione

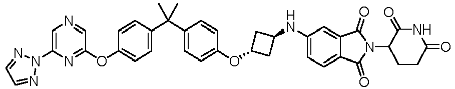

The target compound was synthesized by a method similar to that of Example 228. ¹H NMR (400 MHz, DMSO-*d*₆) *δ* 11.07 (s, 1H), 9.00 (d, *J* = 0.6 Hz, 1H), 8.53 (d, *J* = 0.6 Hz, 1H), 8.23 (s, 2H), 7.59 (d, *J* = 8.3 Hz, 1H), 7.53 (d, *J* = 5.4 Hz, 1H), 7.33 - 7.27 (m, 2H), 7.25 - 7.15 (m, 4H), 6.86 (s, 1H), 6.82 - 6.73 (m, 3H), 5.03 (dd, *J* = 12.9, 5.4 Hz, 1H), 4.87 (p, *J* = 6.5 Hz, 1H), 4.14 (d, *J* = 5.0 Hz, 1H), 2.65 - 2.52 (m, 4H), 2.44 (q, *J* = 5.9 Hz, 2H), 1.99 (s, 2H), 1.65 (s, 6H). LC/MS (ESI+) calcd for C₃₈H₃₄N₈O₆(M + H⁺) *m*/*z* 699.3; found 699.3.

### Example 580: 5-((1r,3r)-3-(4-(2-(4-((6-(1H-1,2,3-triazol-1-yl)pyrazin-2-yl)oxy) phenyl)propan-2-yl)phenoxy)cyclobutyl)amino)-2-(2,6-dioxopiperidin-3-yl) isoindolin-1,3-dione

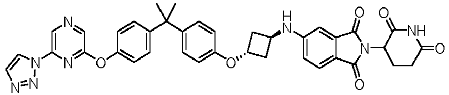

The target compound was synthesized by a method similar to that of Example 228. ¹H NMR (400 MHz, DMSO-*d*₆) *δ* 11.06 (s, 1H), 9.12 - 9.04 (m, 1H), 8.60 - 8.54 (m, 1H), 8.48 (d, *J* = 1.3 Hz, 1H), 8.01 (d, *J* = 1.3 Hz, 1H), 7.59 (d, *J* = 8.3 Hz, 1H), 7.53 (d, *J* = 5.4 Hz, 1H), 7.34 - 7.23 (m, 4H), 7.18 - 7.13 (m, 2H), 6.86 (s, 1H), 6.78 (t, *J* = 9.7 Hz, 3H), 5.03 (dd, *J* = 12.9, 5.4 Hz, 1H), 4.93 - 4.83 (m, 1H), 4.14 (d, *J* = 4.9 Hz, 1H), 2.69 - 2.52 (m, 3H), 2.44 (q, *J* = 6.8 Hz, 3H), 1.99 (t, *J* = 5.9 Hz, 2H), 1.65 (s, 6H). LC/MS (ESI+) calcd for C₃₈H₃₄N₈O₆(M + H⁺) *m*/*z* 699.3; found 699.3.

### Example 581: 2-(2,6-dioxopiperidin-3-yl)-5-(1r,3r)-3-(4-(2-(4-(6-(5-methyl-1,2,4-oxadiazol-3-yl)pyridin-3-yl)oxy)phenyl)propan-2-yl)phenoxy)cyclobutyl)amino) isoindolin-1,3-dione

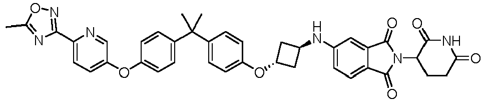

The target compound was synthesized by a method similar to that of Example 221. ¹H NMR (400 MHz, DMSO-*d*₆) *δ* 11.07 (s, 1H), 8.51 (d, *J* = 2.8 Hz, 1H), 8.05 (d, *J* = 8.7 Hz, 1H), 7.62 - 7.46 (m, 3H), 7.33 - 7.25 (m, 2H), 7.18 - 7.05 (m, 4H), 6.86 (s, 1H), 6.78 (dd, *J* = 11.8, 8.4 Hz, 3H), 5.03 (dd, *J* = 12.9, 5.4 Hz, 1H), 4.87 (p, *J* = 6.2 Hz, 1H), 4.14 (d, *J* = 5.3 Hz, 1H), 2.67 (s, 3H), 2.57 (dd, *J* = 19.4, 5.5 Hz, 3H), 2.47 - 2.33 (m, 3H), 1.99 (s, 2H), 1.63 (s, 6H). LC/MS (ESI+) calcd for C₄₀H₃₆N₆O₇(M + H⁺) *m*/*z* 713.2; found 713.2.

### Example 582:2-(2,6-dioxopiperidin-3-yl)-5-((1r,3r)-3-(4-(2-(4-((6-(3-methyl-1,2,4-oxadiazol-5-yl)pyridin-3-yl)oxy)phenyl)propan-2-yl)phenoxy)cyclobutyl)amino) isoindolin-1,3-dione

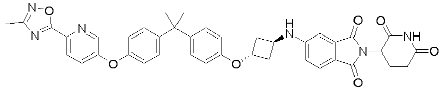

The target compound was synthesized by a method similar to that of Example 250. ¹H NMR (400 MHz, DMSO-*d*₆) *δ* 11.06 (s, 1H), 8.57 (d, *J=* 2.9 Hz, 1H), 8.21 (d, *J=* 8.7 Hz, 1H), 7.61 - 7.46 (m, 3H), 7.35 - 7.29 (m, 2H), 7.18 - 7.10 (m, 4H), 6.86 (s, 1H), 6.82 - 6.73 (m, 3H), 5.03 (dd, *J* = 12.9, 5.4 Hz, 1H), 4.94 - 4.82 (m, 1H), 4.14 (d, *J* = 5.5 Hz, 1H), 2.57 (dd, *J* = 19.1, 5.6 Hz, 3H), 2.43 (s, 6H), 2.06 - 1.90 (m, 2H), 1.63 (s, 6H). LC/MS (ESI+) calcd for C₄₀H₃₆N₆O₇(M + H⁺) *m*/*z* 713.2; found 713.

### Example 583: 5-((1r,3r)-3-(4-(2-(4-((5-(2H-1,2,3-triazol-2-yl)pyrazin-2-yl)oxy) phenyl)propan-2-yl)phenoxy)cyclobutyl)amino)-2-(2,6-dioxopiperidin-3-yl)isoindolin-1,3-dione

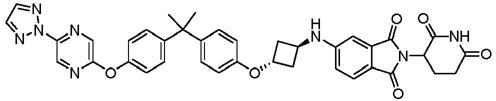

The target compound was synthesized by a method similar to that of Example 228. ¹H NMR (400 MHz, DMSO-*d*₆) *δ* 11.07 (s, 1H), 8.82 (d, *J* = 1.3 Hz, 1H), 8.53 (d, *J* = 1.3 Hz, 1H), 8.22 (s, 2H), 7.56 (dd, *J* = 22.5, 6.9 Hz, 2H), 7.31 - 7.25 (m, 2H), 7.21 - 7.12 (m, 4H), 6.86 (s, 1H), 6.82 - 6.73 (m, 3H), 5.03 (dd, *J* = 12.9, 5.4 Hz, 1H), 4.88 (p, *J* = 6.0 Hz, 1H), 4.14 (d, *J* = 4.9 Hz, 1H), 2.54 (s, 4H), 2.44 (q, *J* = 5.8 Hz, 2H), 1.99 (s, 2H), 1.64 (s, 6H). LC/MS (ESI+) calcd for C₃₈H₃₄N₈O₆ (M + H⁺) *m*/*z* 699.3; found 699.3.

### Example 584: 5-((1r,3r)-3-(4-(2-(4-((5-(1H-1,2,3-triazol-1-yl)pyrazin-2-yl)oxy) phenyl)propan-2-yl)phenoxy)cyclobutyl)amino)-2-(2,6-dioxopiperidin-3-yl) isoindolin-1,3-dione

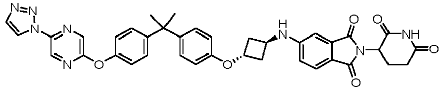

The target compound was synthesized by a method similar to that of Example 228. ¹H NMR (400 MHz, DMSO-*d*₆) δ 11.06(s, 1H), 8.91 (d, *J* = 1.3 Hz, 1H), 8.82 (d, *J* = 1.2 Hz, 1H), 8.57 (d, *J* = 1.3 Hz, 1H), 8.04 (d, *J* = 1.3 Hz, 1H), 7.56 (dd, *J=* 22.5, 6.9 Hz, 2H), 7.33 - 7.27 (m, 2H), 7.21 - 7.13 (m, 4H), 6.86 (s, 1H), 6.78 (dq, *J* = 10.0, 3.2 Hz, 3H), 5.03 (dd, *J* = 12.9, 5.4 Hz, 1H), 4.88 (q, *J* = 6.0 Hz, 1H), 4.15 (d, *J* = 4.8 Hz, 1H), 2.54 (s, 4H), 2.47 - 2.41 (m, 2H), 1.99 (s, 2H), 1.65 (s, 6H). LC/MS (ESI+) calcd for C₃₈H₃₄N₈O₆(M + H⁺) *m*/*z* 699.3; found 699.

### Example 585: 2-(2,6-dioxopiperidin-3-yl)-5-(1r,3r)-3-(4-(2-(4-(6-(5-methyl-1,2,4-oxadiazol-3-yl)pyrazin-2-yl)oxy)phenyl)propan-2-yl)phenoxy)cyclobutyl)amino) isoindolin-1,3-dione

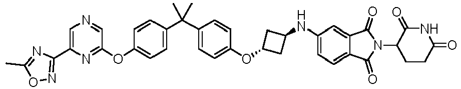

The target compound was synthesized by a method similar to that of Example 221. ¹H NMR (400 MHz, DMSO-*d*₆) *δ* 11.07 (s, 1H), 8.94 (s, 1H), 8.69 (s, 1H), 7.56 (dd, *J* = 22.6, 6.9 Hz, 2H), 7.31 - 7.26 (m, 2H), 7.22 - 7.13 (m, 4H), 6.86 (s, 1H), 6.81 - 6.73 (m, 3H), 5.03 (dd, *J* = 12.9, 5.4 Hz, 1H), 4.91 - 4.80 (m, 1H), 4.14 (d, *J* = 5.5 Hz, 1H), 2.67 (s, 3H), 2.61 - 2.52 (m, 4H), 2.44 (q, *J* = 5.8 Hz, 2H), 1.99 (s, 2H), 1.65 (s, 6H). LC/MS (ESI+) calcd for C₃₉H₃₅N₇O₇(M + H⁺) m/z 714.3; found 714.3.

### Example 586: 2-(2,6-dioxopiperidin-3-yl)-5-(1r,3r)-3-(4-(2-(4-(5-(5-methyl-1,2,4-oxadiazol-3-yl)pyrazin-2-yl)oxy)phenyl)propan-2-yl)phenoxy)cyclobutyl)amino) isoindolin-1,3-dione

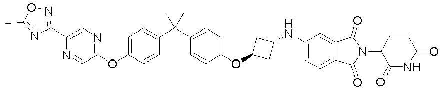

The target compound was synthesized by a method similar to that of Example 221. ¹H NMR (400 MHz, DMSO-*d*₆) *δ* 11.06 (s, 1H), 8.76 (d, *J* = 1.4 Hz, 1H), 8.69 (d, *J* = 1.3 Hz, 1H), 7.56 (dd, *J* = 21.0, 7.0 Hz, 2H), 7.32 - 7.27 (m, 2H), 7.21 - 7.14 (m, 4H), 6.86 (s, 1H), 6.78 (t, *J* = 8.7 Hz, 3H), 5.03 (dd, *J* = 12.9, 5.4 Hz, 1H), 4.93 - 4.82 (m, 1H), 4.14 (s, 1H), 2.69 (s, 3H), 2.61 - 2.53 (m, 3H), 2.44 (dd, *J* = 12.4, 5.4 Hz, 3H), 1.99 (d, *J* = 10.0 Hz, 2H), 1.64 (s, 6H). LC/MS (ESI+) calcd for C₃₉H₃₅N₇O₅(M + H⁺) *m*/*z* 714.3; found 714.3.

### Example 587: 2-(2,6-dioxopiperidin-3-yl)-5-(1s,3s)-3-(4-(2-(4-(6-(5-methyl-1,2,4-oxadiazol-3-yl)pyridin-3-yl)oxy)phenyl)propan-2-yl)phenoxy)cyclobutyl)amino) isoindolin-1,3-dione

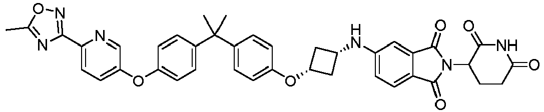

The target compound was synthesized by a method similar to that of Example 221. ¹H NMR (400 MHz, DMSO-*d*₆) *δ* 11.07 (s, 1H), 8.52 (d, *J* = 2.9 Hz, 1H), 8.05 (d, *J* = 8.6 Hz, 1H), 7.59 (d, *J* = 8.3 Hz, 1H), 7.52 - 7.42 (m, 2H), 7.32 - 7.25 (m, 2H), 7.17 - 7.07 (m, 4H), 6.90 (d, *J* = 2.1 Hz, 1H), 6.85 - 6.71 (m, 3H), 5.04 (dd, *J* = 12.9, 5.4 Hz, 1H), 4.50 (p, *J* = 6.9 Hz, 1H), 3.78 (q, *J* = 7.4 Hz, 1H), 3.11 - 3.00 (m, 2H), 2.67 (s, 3H), 2.57 (dd, *J* = 19.7, 6.0 Hz, 2H), 2.08 - 1.88 (m, 4H), 1.63 (s, 6H). LC/MS (ESI+) calcd for C₄₀H₃₆N₆O₇(M + H⁺) *m*/*z* 713.3; found,713.

### Example 588:2-(2,6-dioxopiperidin-3-yl)-5-((1r,3r)-3-(4-(2-(4-((6-(5-methyl-1,2,4-oxadiazol-3-yl)pyrimidin-4-yl)oxy)phenyl)propan-2-yl)phenoxy)cyclobutyl) amino)isoindolin-1,3-dione

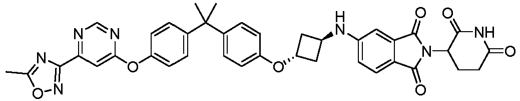

The target compound was synthesized by a method similar to that of Example 221. ¹H NMR (400 MHz, DMSO-*d*₆) *δ* 11.07 (s, 1H), 8.92 (d, *J* = 1.1 Hz, 1H), 7.62 - 7.51 (m, 3H), 7.33 - 7.27 (m, 2H), 7.22 - 7.13 (m, 4H), 6.86 (s, 1H), 6.78 (dd, *J* = 9.2, 7.2 Hz, 3H), 5.04 (dd, *J* = 12.9, 5.4 Hz, 1H), 4.92 - 4.83 (m, 1H), 4.14 (d, *J* = 4.9 Hz, 1H), 2.71 (s, 3H), 2.62 - 2.52 (m, 3H), 2.44 (d, *J* = 6.4 Hz, 3H), 2.00 (q, *J* = 6.0, 3.2 Hz, 2H), 1.65 (s, 6H). LC/MS (ESI+) calcd for C₃₉H₃₅N₇O₇(M + H⁺) *m*/*z* 714.3; found 714.3.

### Example 589: 2-(2,6-dioxopiperidin-3-yl)-5-(1r,3r)-3-(4-(2-(4-(5-(5-methyl-1,3,4-oxadiazol-2-yl)pyrazin-2-yl)oxy)phenyl)propan-2-yl)phenoxy)cyclobutyl)amino) isoindolin-1,3-dione

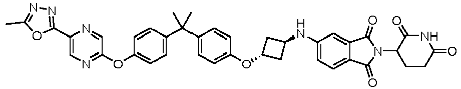

The target compound was synthesized by a method similar to that of Example 216. ¹H NMR (400 MHz, DMSO-*d*₆) *δ* 11.07 (s, 1H), 8.87 (d, *J* = 1.4 Hz, 1H), 8.70 (d, *J* = 1.4 Hz, 1H), 7.56 (dd, *J* = 23.5, 6.9 Hz, 2H), 7.34 - 7.28 (m, 2H), 7.22 - 7.15 (m, 4H), 6.90 - 6.84 (m, 1H), 6.83 - 6.75 (m, 3H), 5.04 (dd, *J* = 12.9, 5.4 Hz, 1H), 4.93 - 4.84 (m, 1H), 4.15 (d, *J* = 5.3 Hz, 1H), 2.61 (s, 3H), 2.60 - 2.52 (m, 3H), 2.44 (q, *J* = 6.7 Hz, 3H), 2.00 (d, *J* = 8.7 Hz, 2H), 1.65 (s, 6H). LC/MS (ESI+) calcd for C₃₉H₃₅N₇O₇(M + H⁺) *m*/*z* 714.3; found 714.3.

### Example 590: 2-(2,6-dioxopiperidin-3-yl)-5-(1r,3r)-3-(4-(2-(4-(6-(5-methyl-1,3,4-oxadiazol-2-yl)pyrimidin-4-yl)oxy)phenyl)propan-2-yl)phenoxy)cyclobutyl) amino)isoindolin-1,3-dione

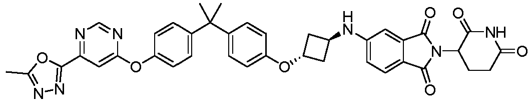

The target compound was synthesized by a method similar to that of Example 216. ¹H NMR (400 MHz, DMSO-*d*₆) *δ* 11.07 (s, 1H), 8.94 (d, *J* = 1.2 Hz, 1H), 7.66 (d, *J* = 1.2 Hz, 1H), 7.56 (dd, *J* = 23.2, 6.9 Hz, 2H), 7.35 - 7.29 (m, 2H), 7.21 - 7.12 (m, 4H), 6.86 (s, 1H), 6.79 (dd, *J* = 8.9, 6.8 Hz, 3H), 5.04 (dd, *J* = 12.9, 5.4 Hz, 1H), 4.88 (h, *J* = 5.4, 4.8 Hz, 1H), 4.15 (d, *J* = 5.2 Hz, 1H), 2.64 (s, 3H), 2.62 - 2.53 (m, 3H), 2.44 (dt, *J* = 12.3, 6.0 Hz, 3H), 2.00 (d, *J* = 8.6 Hz, 2H), 1.66 (s, 6H). LC/MS (ESI+) calcd for C₃₉H₃₅N₇O₇(M + H⁺) *m*/*z* 714.3; found 714.3.

### Example 591:2-(2,6-dioxopiperidin-3-yl)-5-((1r,3r)-3-(4-(2-(4-((3-methyl-1,2,4-thiadiazol-5-yl)oxy)phenyl)propan-2-yl)phenoxy)cyclobutyl)aminoisoindolin-1,3-dione

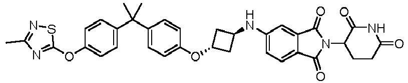

The target compound was synthesized by a method similar to that of Example 276. ¹H NMR (400 MHz, DMSO-*d*₆) *δ* 11.06 (s, 1H), 7.56 (dd, *J* = 25.5, 6.9 Hz, 2H), 7.35 (d, *J* = 0.9 Hz, 4H), 7.18 - 7.12 (m, 2H), 6.86 (s, 1H), 6.78 (t, *J* = 8.8 Hz, 3H), 5.04 (dd, *J* = 12.9, 5.4 Hz, 1H), 4.85 (dt, *J* = 20.8, 5.7 Hz, 1H), 4.14 (d, *J* = 4.9 Hz, 1H), 2.63 - 2.52 (m, 3H), 2.45 (q, *J* = 5.0, 4.0 Hz, 3H), 2.40 (s, 3H), 2.00 (d, *J* = 8.3 Hz, 2H), 1.64 (s, 6H). LC/MS (ESI+) calcd for C₃₅H₃₃N₅O₆S(M + H⁺) *m*/*z* 652.2; found 652.2.

### Example 592: 5-((1r,3r)-3-(4-(2-(4-((1,3,4-thiadiazol-2-yl)oxy)phenyl)propan-2-yl) phenoxy)cyclobutyl)amino)-2-(2,6-dioxopiperidin-3-yl)isoindolin-1,3-dione

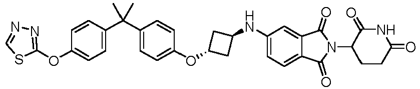

The target compound was synthesized by a method similar to that of Example 277. ¹H NMR (400 MHz, DMSO-*d*₆) *δ* 11.06 (s, 1H), 9.14 (s, 1H), 7.59 (d, *J* = 8.3 Hz, 1H), 7.53 (d, *J* = 5.4 Hz, 1H), 7.34 - 7.28 (m, 4H), 7.18 - 7.12 (m, 2H), 6.86 (s, 1H), 6.78 (dd, *J* = 11.7, 8.3 Hz, 3H), 5.03 (dd, *J* = 12.9, 5.4 Hz, 1H), 4.87 (p, *J* = 6.0 Hz, 1H), 4.14 (s, 1H), 2.62 - 2.52 (m, 3H), 2.44 (q, *J* = 5.7 Hz, 3H), 2.00 (d, *J* = 7.7 Hz, 2H), 1.63 (s, 6H). LC/MS (ESI+) calcd for C₃₄H₃₁N₅O₆S(M + H⁺) *m*/*z* 638.2; found 638.2.

### Example 593: 2-(2,6-dioxopiperidin-3-yl)-5-((1r,3r)-3-(4-(2-(4-((5-(5-methyl-1,3,4-oxadiazol-2-yl)-1,3,4-thiadiazol-2-yl)oxy)phenyl)propan-2-yl)phenoxy)cyclobutyl) amino)isoindolin-1,3-dione

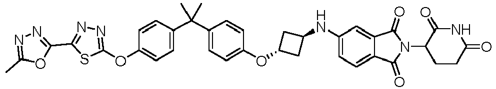

The target compound was synthesized by a method similar to that of Example 216. ¹H NMR (400 MHz, DMSO-*d*₆) *δ* 11.07 (s, 1H), 9.14 (s, 1H), 7.56 (dd, *J* = 20.7, 6.9 Hz, 2H), 7.31 (d, *J* = 1.6 Hz, 4H), 7.14 (d, *J* = 8.7 Hz, 2H), 6.78 (t, *J* = 9.9 Hz, 3H), 5.03 (dd, *J* = 12.9, 5.4 Hz, 1H), 4.95 - 4.83 (m, 1H), 4.13 (d, *J* = 7.7 Hz, 1H), 2.95 - 2.80 (m, 1H), 2.62 - 2.52 (m, 3H), 2.47 - 2.39 (m, 2H), 1.99 (d, *J* = 9.2 Hz, 2H), 1.63 (s, 6H), 1.23 (s, 3H). LC/MS (ESI+) calcd for C₃₇H₃₃N₇O₇S(M + H⁺) *m*/*z* 720.2; found 720.2.

### Example 594: 5-(4-(2-(4-((1r,3r)-3-((2-(2,6-dioxopiperidin-3-yl)-1,3-dioxoisoindolin-5-yl)amino)cyclobutyloxy) phenyl)propan-2-yl)phenoxy)pyrazin-2-nitrile

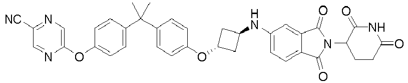

The target compound was synthesized by a method similar to that of Example 216. ¹H NMR (400 MHz, DMSO-*d*₆) *δ* 11.07 (s, 1H), 8.78 (d, *J* = 1.4 Hz, 1H), 8.71 (d, *J* = 1.3 Hz, 1H), 7.56 (dd, *J* = 22.1, 6.8 Hz, 2H), 7.34 - 7.25 (m, 2H), 7.16 (dd, *J* = 8.8, 3.3 Hz, 4H), 6.94 - 6.72 (m, 4H), 5.32 (t, *J* = 4.9 Hz, 1H), 5.03 (dd, *J* = 12.9, 5.4 Hz, 1H), 4.87 (t, *J* = 6.0 Hz, 1H), 4.14 (s, 1H), 2.96 - 2.81 (m, 1H), 2.06 - 1.92 (m, 3H), 1.64 (s, 6H), 1.34 (d, *J* = 5.9 Hz, 3H). LC/MS (ESI+) calcd for C₃₇H₃₂N₆O₆(M + H⁺) *m*/*z* 657.2; found 657.2.

### Example 595: 2-(2,6-dioxopiperidin-3-yl)-5-((1r,3r)-3-(4-(2-(4-((5-(1-hydroxylethyl)pyrazin-2-yl)oxy)phenyl)propan-2-yl)phenoxy)cyclobutyl)amino) isoindolin-1,3-dione

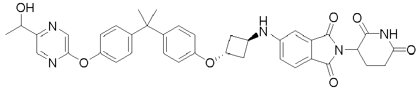

The target compound was synthesized by a method similar to that of Example 118. ¹H NMR (400 MHz, DMSO-*d*₆) *δ* 11.07 (s, 1H), 8.40 (d, *J* = 1.4 Hz, 1H), 8.23 (d, *J* = 1.1 Hz, 1H), 7.56 (dd, *J* = 22.9, 6.8 Hz, 2H), 7.30 - 7.22 (m, 2H), 7.19 - 7.12 (m, 2H), 7.11 - 7.03 (m, 2H), 6.86 (s, 1H), 6.78 (t, *J* = 10.2 Hz, 3H), 5.48 (d, *J* = 4.7 Hz, 1H), 5.03 (dd, *J* = 12.9, 5.4 Hz, 1H), 4.86 (d, *J* = 5.8 Hz, 1H), 4.79 (p, *J* = 6.5 Hz, 1H), 4.14 (d, *J* = 9.3 Hz, 1H), 2.96 - 2.80 (m, 1H), 2.57 (d, *J* = 16.9 Hz, 2H), 2.44 (d, *J* = 6.3 Hz, 3H), 2.00 (d, *J* = 7.6 Hz, 2H), 1.63 (s, 6H), 1.39 (d, *J* = 6.4 Hz, 4H). LC/MS (ESI+) calcd for C₃₈H₃₇N₅O₇ (M + H⁺) *m*/*z* 720.2; found 720.2.

### Example 596: 5-((1r,3r)-3-(4-(2-(4-((5-acetylpyrazin-2-yl)oxy)phenyl)propan-2-yl)phenoxy)cyclobutyl)amino)-2-(2,6-dioxopiperidin-3-yl)isoindolin-1,3-dione

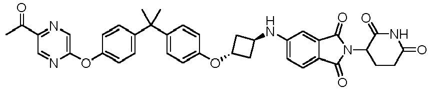

The target compound was synthesized by a method similar to that of Example 118. ¹H NMR (400 MHz, DMSO-*d*₆) *δ* 11.07 (s, 1H), 8.69 (d, *J* = 1.3 Hz, 1H), 8.62 (d, *J* = 1.3 Hz, 1H), 7.59 (d, *J* = 8.3 Hz, 1H), 7.53 (d, *J* = 5.4 Hz, 1H), 7.32 - 7.25 (m, 2H), 7.16 (dd, *J* = 11.4, 8.7 Hz, 4H), 6.86 (s, 1H), 6.78 (t, *J* = 9.4 Hz, 3H), 5.04 (dd, *J* = 12.9, 5.4 Hz, 1H), 4.91 - 4.81 (m, 1H), 4.14 (d, *J* = 8.8 Hz, 1H), 2.90 - 2.81 (m, 1H), 2.54 (d, *J* = 6.0 Hz, 3H), 2.47 - 2.39 (m, 3H), 2.00 (d, *J* = 8.7 Hz, 3H), 1.64 (s, 6H), 1.41-1.31 (m, 2H). LC/MS (ESI+) calcd for C₃₈H₃₅N₅O₇(M + H⁺) *m*/*z* 674.2; found 674.2.

### Example 597: 2-(2,6-dioxopiperidin-3-yl)-5-(((1s,3s)-3-(4-(2-(4-((6-(5-methyl-1,2,4-oxadiazol-3-yl)pyridin-3-yl)oxy)phenyl)propan-2-yl)phenoxy)cyclobutyl) amino)isoindolin-1,3-dione

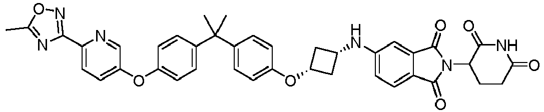

The target compound was synthesized by a method similar to that of Example 221. ¹H NMR (400 MHz, DMSO-*d*₆) *δ* 11.08 (s, 1H), 8.55 - 8.48 (m, 1H), 8.08 - 8.00 (m, 1H), 7.59 (d, *J* = 8.3 Hz, 1H), 7.51 - 7.43 (m, 2H), 7.32 - 7.26 (m, 2H), 7.18 - 7.12 (m, 2H), 7.11 - 7.06 (m, 2H), 6.90 (d, *J* = 2.0 Hz, 1H), 6.85 - 6.77 (m, 3H), 5.04 (dd, *J* = 12.9, 5.4 Hz, 1H), 4.50 (p, *J* = 7.0 Hz, 1H), 3.77 (p, *J* = 7.5 Hz, 1H), 3.11 - 3.00 (m, 2H), 2.88 (ddd, *J* = 17.3, 14.0, 5.5 Hz, 1H), 2.67 (s, 3H), 2.63 - 2.55 (m, 1H), 1.98 (q, *J* = 10.8 Hz, 4H), 1.63 (s, 6H). LC/MS (ESI+) calcd for C₄₀H₃₆N₆O₇(M + H⁺) *m*/*z* 713.3; found 713.3.

### Example 598: 2-(2,6-dioxopiperidin-3-yl)-5-(((1s,3s)-3-(4-(2-(4-((5-(5-methyl-1,2,4-oxadiazol-3-yl)pyrazin-2-yl)oxy)phenyl)propan-2-yl)phenoxy)cyclobutyl) amino)isoindolin-1,3-dione

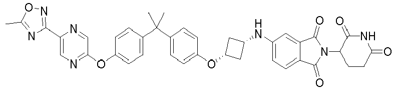

The target compound was synthesized by a method similar to that of Example 221. ¹H NMR (400 MHz, DMSO-*d*₆) *δ* 11.08 (s, 1H), 8.72 (dd, *J* = 27.4, 1.3 Hz, 2H), 7.59 (d, *J* = 8.3 Hz, 1H), 7.46 (d, *J* = 6.5 Hz, 1H), 7.35 - 7.25 (m, 2H), 7.21 - 7.14 (m, 4H), 6.90 (d, *J* = 2.0 Hz, 1H), 6.86 - 6.72 (m, 3H), 5.04 (dd, *J* = 12.9, 5.4 Hz, 1H), 4.50 (p, *J* = 6.9 Hz, 1H), 3.78 (h, *J* = 7.7 Hz, 1H), 3.14 - 3.00 (m, 2H), 2.88 (ddd, *J* = 17.3, 14.0, 5.5 Hz, 1H), 2.68 (s, 3H), 2.61 - 2.54 (m, 1H), 2.07 - 1.89 (m, 4H), 1.64 (s, 6H). LC/MS (ESI+) calcd for C₃₉H₃₅N₇O₇(M + H⁺) *m*/*z* 714.3; found 714.3.

### Example 599: 2-(2,6-dioxopiperidin-3-yl)-5-(1s,3s)-3-(4-(2-(4-(6-(5-methyl-1,2,4-oxadiazol-3-yl)pyrazin-2-yl)oxy)phenyl)propan-2-yl)phenoxy)cyclobutyl)amino) isoindolin-1,3-dione

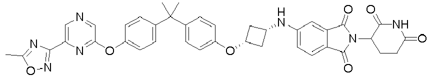

The target compound was synthesized by a method similar to that of Example 221. ¹H NMR (400 MHz, DMSO-*d*₆) *δ* 11.08 (s, 1H), 8.94 (s, 1H), 8.69 (s, 1H), 7.58 (d, *J* = 8.3 Hz, 1H), 7.46 (d, *J* = 6.5 Hz, 1H), 7.32 - 7.26 (m, 2H), 7.21 - 7.13 (m, 4H), 6.90 (d, *J* = 2.0 Hz, 1H), 6.81 (t, *J* = 9.0 Hz, 3H), 5.04 (dd, *J* = 12.9, 5.4 Hz, 1H), 4.50 (p, *J* = 7.0 Hz, 1H), 3.78 (h, *J* = 7.5 Hz, 1H), 3.12 - 3.01 (m, 2H), 2.88 (ddd, *J* = 17.0, 13.8, 5.4 Hz, 1H), 2.67 (s, 3H), 2.62 - 2.55 (m, 1H), 2.05 - 1.90 (m, 4H), 1.64 (s, 6H). LC/MS (ESI+) calcd for C₃₉H₃₅N₇O₇(M + H⁺) *m*/*z* 714.3; found 714.3.

### Example 600: 2-(2,6-dioxopiperidin-3-yl)-5-((1s,3s)-3-(4-(2-(4-((6-(5-methyl-1,3,4-oxadiazol-2-yl)pyrimidin-4-yl)oxy)phenyl)propan-2-yl)phenoxy)cyclobutyl) amino)isoindolin-1,3-dione

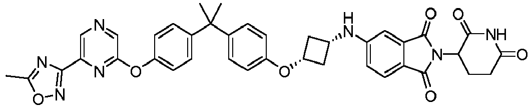

The target compound was synthesized by a method similar to that of Example 216. ¹H NMR (400 MHz, DMSO-*d*₆) *δ* 11.08 (s, 1H), 8.92 (d, *J* = 1.1 Hz, 1H), 7.62 - 7.56 (m, 2H), 7.46 (d, *J* = 6.5 Hz, 1H), 7.34 - 7.28 (m, 2H), 7.20 - 7.13 (m, 4H), 6.92 - 6.77 (m, 4H), 5.04 (dd, *J* = 12.9, 5.4 Hz, 1H), 4.50 (p, *J* = 7.0 Hz, 1H), 3.78 (h, *J* = 7.7 Hz, 1H), 3.06 (ddd, *J* = 10.9, 8.3, 5.6 Hz, 2H), 2.87 (ddd, *J* = 17.2, 14.1, 5.5 Hz, 1H), 2.71 (s, 3H), 2.62 - 2.54 (m, 1H), 1.97 (td, *J* = 11.2, 5.2 Hz, 4H), 1.65 (s, 6H). LC/MS (ESI+) calcd for C₃₉H₃₅N₇O₇(M + H⁺) *m*/*z* 714.3; found 714.3.

### Example 601:2-(2,6-dioxopiperidin-3-yl)-5-((1r,3r)-3-(4-(1-(4-((5-(5-methyl-1,2,4-oxadiazol-3-yl)pyrazin-2-yl)oxy)phenyl)cyclopentyl)phenoxy)cyclobutyl)amino) isoindolin-1,3-dione

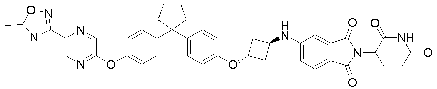

The target compound was synthesized by a method similar to that of Example 221. ¹H NMR (400 MHz, DMSO-*d*₆) *δ* 11.04 (s, 1H), 8.71 (dd, *J* = 23.4, 1.4 Hz, 2H), 7.58 (d, *J* = 8.3 Hz, 1H), 7.54 (d, *J* = 5.4 Hz, 1H), 7.41 - 7.36 (m, 2H), 7.28 (d, *J* = 8.6 Hz, 2H), 7.18 - 7.11 (m, 2H), 6.87 (d, *J* = 15.3 Hz, 1H), 6.82 - 6.71 (m, 3H), 5.03 (dd, *J* = 12.9, 5.4 Hz, 1H), 4.90 - 4.81 (m, 1H), 4.13 (d, *J* = 5.6 Hz, 1H), 3.04 (s, 1H), 2.68 (s, 3H), 2.44 (dd, *J* = 11.2, 5.5 Hz, 2H), 2.28 (d, *J* = 7.4 Hz, 5H), 2.00 (dd, *J* = 14.4, 7.1 Hz, 3H), 1.67 - 1.56 (m, 5H). LC/MS (ESI+) calcd for C₄₁H₃₇N₇O₇(M + H⁺) *m*/*z* 740.3; found 740.3.

In the following, the beneficial effect of the compound according to the present invention was demonstrated by experimental examples.

### Experimental example 1. The inhibitory activity of the compound according to the present invention on the proliferation of prostate cancer 22RV1 cell lines

### 1. Experimental materials

22RV1 cell lines (Cobioer, CBP6034)
Penicillin-Streptomycin (Hyclone, Cat. No. SV30010)
Cell counting kit-8 (Signalway Antibody, Cat. No. CP002)

### 2. Experimental procedures

(1) 22RV1 cells were subcultured in cell culture media, and then the cells in good growth condition were seeded in a 96-well plate, and cultured overnight in a 37 °C, 5% CO₂ cell incubator.
(2) The drug was prepared into a 10 mM stock solution with dimethyl sulfoxide (DMSO). Prior to use, the stock solution was diluted with medium in a ratio of 1:66.67, and then diluted at a 3-fold gradient to obtain 9 serial concentrations. Then, two wells were set for each concentration. 20 µL of the diluted compound solution was added to the cell culture well, and then the plate was gently shaken to mix. In addition, the experiment included 3 negative control wells only containing cells and 3 blank control wells only containing culture medium.

### 3. Result detection:

1) After culturing for 6 days, 10 µL of CCK-8 was added to each well, and the plate was further incubated for 2.5 h in a 37°C, 5% CO₂ cell incubator.
2) The absorbance (OD value) was measured at 450 nm with a multifunctional microplate reader.
3) The data was analyzed by the Dose-response-inhibition equation in the software GraphPad Prism8, and the IC₅₀ value was obtained.

The inhibitory results of the compounds according to the present invention on 22RV1 cells were listed in Table 1:

**Table 1. The inhibitory results of the compounds on 22RV1 cells.**

| **Example** | **22RV1 IC50 (nM)** | **Example** | **22RV1 IC50 (nM)** | **Example** | **22RV1 IC50 (nM)** |
|---|---|---|---|---|---|
| **61** | 4000 | **89** | 16 | **110** | 3 |
| **62** | 1500 | **91** | 2 | **115** | 5 |
| **66** | 70 | **94** | 4 | **116** | 17 |
| **68** | 30 | **95** | 6 | **118** | 3 |
| **69** | 50 | **96** | 25 | **119** | 2 |
| **70** | 30 | **97** | 15 | **123** | 7 |
| **71** | 35 | **98** | 4 | **124** | 8 |
| **72** | 37 | **99** | 17 | **125** | 10 |
| **73** | 25 | **101** | 2.4 | **126** | 6 |
| **74** | 31 | **103** | 2 | **128** | 7 |
| **75** | 8 | **104** | 1 | **130** | 13 |
| **76** | 11 | **105** | 0.2 | **131** | 4 |
| **77** | 44 | **106** | 0.3 | **132** | 5 |
| **88** | 15 | **109** | 9 | **133** | 9 |
| **134** | <1 | **206** | 10 | **277** | 5 |
| **135** | 2 | **207** | 16 | **278** | 6 |
| **141** | 85 | **209** | 5 | **279** | 4 |
| **142** | 61 | **210** | 7 | **280** | 7 |
| **143** | 160 | **212** | 14 | **281** | 5 |
| **154** | 7 | **217** | 15 | **288** | 4 |
| **155** | 39 | **218** | 6 | **289** | 7 |
| **156** | 10 | **219** | 6 | **290** | 7 |
| **160** | 8 | **220** | 8 | **291** | 16 |
| **161** | 10 | **221** | 6 | **293** | 2.5 |
| **162** | 10 | **222** | 3 | **294** | 4.2 |
| **166** | 6 | **223** | 0.5 | **295** | 11 |
| **168** | 18 | **225** | 60 | **296** | 7 |
| **169** | 10 | **228** | 22 | **297** | 3 |
| **170** | 9 | **229** | 31 | **306** | 13 |
| **172** | 19 | **230** | 9 | **308** | 19 |
| **173** | 13 | **231** | 8.6 | **311** | 18 |
| **175** | 4 | **232** | 8 | **312** | 22 |
| **176** | 2 | **233** | 16 | **321** | 8 |
| **177** | 2 | **237** | 9 | **323** | 11 |
| **178** | 5 | **239** | 3 | **324** | 4 |
| **181** | 140 | **240** | 12 | **328** | 5 |
| **182** | 50 | **244** | 6 | **331** | 18 |
| **183** | 20 | **246** | 5 | **333** | 3 |
| **185** | 56 | **248** | 23 | **338** | 2 |
| **186** | 32 | **249** | 6 | **339** | 2 |
| **187** | 6 | **251** | 6 | **343** | 19 |
| **188** | 12 | **254** | 6 | **345** | 10 |
| **189** | 9 | **256** | 2 | **350** | 30 |
| **190** | 12 | **258** | 8 | **351** | 7 |
| **193** | 36 | **259** | 7 | **355** | 2 |
| **194** | 33 | **265** | 4 | **356** | 16 |
| **195** | 15 | **268** | 9 | **357** | 5 |
| **196** | 10 | **270** | 10 | **358** | 9 |
| **197** | 5 | **273** | 10 | **359** | 6 |
| **200** | 12 | **275** | 4 | **360** | 4 |
| **204** | 6 | **276** | 6 | **361** | 8 |
| **362** | 20 | **429** | 9 | **522** | 7 |
| **363** | 24 | **430** | 9 | **523** | 6 |
| **364** | 12 | **431** | 4 | **525** | 8 |
| **365** | 9 | **432** | 7 | **526** | 5 |
| **366** | 3 | **436** | 6 | **527** | 3 |
| **367** | 8 | **444** | 62 | **528** | 2 |
| **369** | 6 | **445** | 5 | **529** | 2 |
| **371** | 9 | **446** | 9 | **532** | 9 |
| **372** | 8 | **447** | 8 | **535** | 35 |
| **373** | 13 | **448** | 11 | **536** | 5 |
| **377** | 12 | **449** | 10 | **538** | 12 |
| **378** | 7 | **450** | 8 | **539** | 8 |
| **379** | 17 | **451** | 11 | **543** | 8 |
| **380** | 5 | **452** | 8 | **544** | 12 |
| **382** | <1 | **453** | 6 | **547** | 9 |
| **383** | 34 | **455** | 5 | **548** | 8 |
| | | **456** | 12 | **549** | 7 |
| **389** | 54 | **461** | 11 | **551** | 12 |
| **390** | 26 | **463** | 11 | **553** | 15 |
| **391** | 56 | **464** | 4 | **564** | 40 |
| **392** | 21 | **465** | 3 | **571** | 5 |
| **393** | 10 | **468** | 5 | **572** | 16 |
| **395** | 10 | **470** | 7 | **574** | 5 |
| **396** | 23 | **471** | 17 | **578** | 8 |
| **397** | 13 | **473** | 5 | **579** | 7 |
| **398** | 9 | **476** | 14 | **580** | 14 |
| **399** | 16 | **477** | 9 | **581** | 10 |
| **400** | 10 | **478** | 6 | **583** | 10 |
| **405** | 19 | **480** | 12 | **584** | 4 |
| **410** | 6 | **483** | 2 | **588** | 13 |
| **413** | 11 | **491** | 4 | **589** | 7 |
| **414** | 6 | **492** | 7 | **592** | 8 |
| **416** | 22 | **494** | 7 | **595** | 4 |
| **419** | 8 | **496** | 61 | **596** | 10 |
| **420** | 5 | **502** | 12 | **601** | 11 |
| **421** | 3 | **506** | 4 | | |
| **423** | 3 | **512** | 8 | | |
| **425** | 16 | **513** | 12 | | |
| **426** | 7 | **516** | 10 | | |
| **427** | 6 | **517** | 16 | | |
| **428** | 6 | **518** | 13 | | |

### Experimental example 2. Determination of the degradation activity of the compound according to the present invention on androgen receptor (AR) splicing variant AR-v7

The degradation activity of the compound of the present invention on the androgen receptor splicing variant AR-V7 was analyzed using enzyme-linked immunosorbent assay (ELISA).

### 1. Experimenta materials:

22RV1 cell lines (Cobioer, CBP6034)
Penicillin-Streptomycin liquid (Hyclone, Cat. No. SV30010)
FastScan^{™} Androgen Receptor (AR-V7 Specific) ELISA kit (CST, Cat. No. 93577)

### 2. Experimental procedures

1) After 22RV1 cells were subcultured in cell culture medium, the cells in good growth condition were inoculated in a 96 well plate at 80 µl/well. The plate was cultured in a 5% CO₂ cell incubator at 37 °C for 2 days until the cells adhered to the wall.
2) The drug was prepared into 10 mM stock solution with dimethylsulfoxide (DMSO). Prior to use, the stock solution was diluted with cell culture medium, and 20 µl of the diluted compound solution was transferred to a cell culture well, to obtain 6 serial concentrations. The plate was gently shaken and mixed. In addition, negative control wells (only containing culture medium) and positive control wells (only containing cells and DMSO) were set.
3) After the cells were cultured in an incubator for 48 h, the medium was removed and the cells were rinsed once with ice cold PBS. Then, ice-cold 1x cell lysis buffer was added to each well of the cell culture plate. After the plate was incubated on ice for 15 min, the plate was tapped to mix.
4) The supernatant of the cell lysate was transferred from the cell culture plate to a 94-well plate for ELISA.
5) The newly prepared Capture antibody (green) was mixed with the newly prepared HRP-labeled antibody (red), and then the antibody mixture was added to the above 96-well ELISA plate.
6) The ELISA plate was sealed with plastic film, and then incubated in a 25 °C incubator for 1 h.
7) The washing buffer was added to each well, and then TMB substrate was added to each well of the ELISA plate. Subsequntly, the plate was cultured in an incubator at 37 ° C for 5 min.
8) 100 µl of stop solution was added to each well of the ELISA plate, and the plate was gently tapped for several seconds. The absorbance was read at 450 nm within 30 min. The data were analyzed by the Dose-response equation in the software GraphPad Prism8, to obtain DC₅₀ and Dₘₐₓ values.

The degradation activity of the compound according to the present invention on AR-v7 is shown in Table 2.

**Table 2. The degradation activity of compounds on AR-v7 in 22RV1 cells.**

| **Example** | **22RV1 DC50 (nM)** | **Example** | **22RV1 DC50 (nM)** | **Example** | **22RV1 DC50 (nM)** |
|---|---|---|---|---|---|
| **1** | C | **6** | 6332 | **11** | D |
| **2** | C | **7** | >10000 | **12** | D |
| **3** | >10000 | **8** | 5273 | **13** | 6973 |
| **4** | >10000 | **9** | D | **14** | >10000 |
| **5** | >10000 | **10** | D | **15** | >10000 |
| **16** | >10000 | **54** | C | **92** | C |
| **17** | D | **55** | | **93** | C |
| **18** | >10000 | **56** | 5200 | **94** | A |
| **19** | 6857 | **57** | C | **95** | A |
| **20** | D | **58** | D | **96** | A |
| **21** | 5773 | **59** | B | **97** | A |
| **22** | C | **60** | C | **98** | A |
| **23** | D | **61** | D | **99** | A |
| **24** | >10000 | **62** | D | **100** | C |
| **25** | >10000 | **63** | D | **101** | A |
| **26** | D | **64** | >10000 | **102** | B |
| **27** | 7580 | **65** | >10000 | **103** | A |
| **28** | 5273 | **66** | B | **104** | A |
| **29** | 6332 | **67** | D | **105** | A |
| **30** | >10000 | **68** | B | **106** | A |
| **31** | >10000 | **69** | B | **107** | >10000 |
| **32** | >10000 | **70** | B | **108** | >10000 |
| **33** | C | **71** | B | **109** | A |
| **34** | >10000 | **72** | B | **110** | A |
| **35** | >10000 | **73** | B | **111** | C |
| **36** | >10000 | **74** | B | **112** | B |
| **37** | D | **75** | A | **113** | D |
| **38** | >5000 | **76** | A | **114** | D |
| **39** | >10000 | **77** | B | **115** | A |
| **40** | >10000 | **78** | B | **116** | A |
| **41** | >10000 | **79** | B | **117** | B |
| **42** | >10000 | **80** | B | **118** | A |
| **43** | >10000 | **81** | B | **119** | A |
| **44** | C | **82** | B | **120** | B |
| **45** | 8759 | **83** | B | | |
| **46** | >5000 | **84** | B | **122** | B |
| **47** | C | **85** | B | **123** | A |
| **48** | >10000 | **86** | B | **124** | A |
| **49** | D | **87** | B | **125** | A |
| **50** | C | **88** | A | **126** | A |
| **51** | C | **89** | A | **127** | B |
| **52** | >10000 | **90** | B | **128** | B |
| **53** | D | **91** | A | **129** | B |
| **130** | A | **169** | A | **208** | B |
| **131** | A | **170** | A | **209** | A |
| **132** | A | **171** | B | **210** | A |
| **133** | A | **172** | A | **211** | B |
| **134** | A | **173** | B | **212** | A |
| **135** | A | **174** | B | **213** | B |
| **136** | C | **175** | A | **214** | B |
| **137** | B | **176** | A | **215** | B |
| **138** | D | **177** | A | **216** | B |
| **139** | C | **178** | A | **217** | A |
| **140** | C | **179** | C | **218** | A |
| **141** | B | **180** | D | **219** | A |
| **142** | B | **181** | D | **220** | A |
| **143** | B | **182** | B | **221** | A |
| **144** | D | **183** | B | **222** | A |
| **145** | C | **184** | D | **223** | A |
| **146** | D | **185** | B | **224** | C |
| **147** | D | **186** | B | **225** | B |
| **148** | C | **187** | A | **226** | >10000 |
| **149** | D | **188** | A | **227** | >10000 |
| **150** | B | **189** | A | **228** | B |
| **151** | B | **190** | A | **229** | B |
| **152** | D | **191** | B | **230** | A |
| **153** | C | **192** | B | **231** | B |
| **154** | A | **193** | B | **232** | A |
| **155** | A | **194** | B | **233** | A |
| **156** | A | **195** | A | **234** | B |
| **157** | B | **196** | A | **235** | C |
| **158** | B | **197** | A | **236** | B |
| **159** | B | **198** | B | **237** | A |
| **160** | A | **199** | B | **238** | B |
| **161** | A | **200** | A | **239** | A |
| **162** | A | **201** | B | **240** | A |
| **163** | B | **202** | B | **241** | B |
| **164** | B | **203** | B | **242** | B |
| **165** | B | **204** | A | **243** | A |
| **166** | A | **205** | B | **244** | A |
| **167** | B | **206** | A | **245** | C |
| **168** | B | **207** | A | **246** | A |
| **247** | B | **286** | B | **325** | B |
| **248** | A | **287** | B | **326** | B |
| **249** | A | **288** | A | **327** | B |
| **250** | B | **289** | A | **328** | B |
| **251** | A | **290** | A | **329** | B |
| **252** | D | **291** | A | **330** | B |
| **253** | B | **292** | B | **331** | A |
| **254** | A | **293** | A | **332** | B |
| **255** | B | **294** | A | **333** | A |
| **256** | A | **295** | A | **334** | B |
| **257** | B | **296** | A | **335** | C |
| **258** | C | **297** | A | **336** | B |
| **259** | A | **298** | >10000 | **337** | B |
| **260** | B | **299** | >10000 | **338** | A |
| **261** | A | **300** | >10000 | **339** | A |
| **262** | B | **301** | >10000 | **340** | B |
| **263** | B | **302** | 10000 | **341** | B |
| **264** | B | **303** | >10000 | **342** | >10000 |
| **265** | A | **304** | >2500 | **343** | B |
| **266** | B | **305** | D | **344** | D |
| **267** | C | **306** | A | **345** | B |
| **268** | A | **307** | D | **346** | D |
| **269** | C | **308** | B | **347** | D |
| **270** | A | **309** | B | **348** | D |
| **271** | B | **310** | C | **349** | >10000 |
| **272** | B | **311** | A | **350** | B |
| **273** | A | **312** | A | **351** | A |
| **274** | B | **313** | C | **352** | B |
| **275** | A | **314** | D | **353** | D |
| **276** | A | **315** | D | **354** | D |
| **277** | A | **316** | B | **355** | A |
| **278** | A | **317** | B | **356** | A |
| **279** | A | **318** | C | **357** | A |
| **280** | A | **319** | B | **358** | A |
| **281** | A | **320** | B | **359** | A |
| **282** | B | **321** | A | **360** | A |
| **283** | B | **322** | A | **361** | A |
| **284** | B | **323** | A | **362** | A |
| **285** | B | **324** | A | **363** | A |
| **364** | A | **403** | B | **442** | B |
| **365** | A | **404** | B | **443** | B |
| **366** | A | **405** | A | **444** | B |
| **367** | A | **406** | | **445** | A |
| **368** | B | **407** | B | **446** | B |
| **369** | A | **408** | B | **447** | A |
| **370** | B | **409** | B | **448** | B |
| **371** | A | **410** | A | **449** | B |
| **372** | A | **411** | B | **450** | B |
| **373** | A | **412** | B | **451** | A |
| **374** | B | **413** | A | **452** | B |
| **375** | B | **414** | A | **453** | A |
| **376** | B | **415** | B | **454** | B |
| **377** | A | **416** | A | **455** | A |
| **378** | B | **417** | B | **456** | A |
| **379** | B | **418** | B | **457** | B |
| **380** | A | **419** | A | **458** | B |
| **381** | B | **420** | A | **459** | B |
| **382** | A | **421** | A | **460** | B |
| **383** | B | **422** | B | **461** | A |
| **384** | >5000 | **423** | A | **462** | C |
| **385** | >2000 | **424** | B | **463** | A |
| **386** | D | **425** | A | **464** | A |
| **387** | B | **426** | A | **465** | A |
| **388** | B | **427** | A | **466** | B |
| **389** | B | **428** | A | **467** | B |
| **390** | B | **429** | A | **468** | A |
| **391** | B | **430** | A | **469** | B |
| **392** | B | **431** | A | **470** | A |
| **393** | B | **432** | A | **471** | A |
| **394** | B | **433** | B | **472** | B |
| **395** | A | **434** | - | **473** | B |
| **396** | A | **435** | D | **474** | B |
| **397** | A | **436** | A | **475** | B |
| **398** | A | **437** | >10000 | **476** | B |
| **399** | A | **438** | >2000 | **477** | A |
| **400** | A | **439** | B | **478** | A |
| **401** | B | **440** | B | **479** | B |
| **402** | B | **441** | B | **480** | A |
| **481** | B | **520** | B | **559** | D |
| **482** | B | **521** | B | **560** | D |
| **483** | A | **522** | B | **561** | B |
| **484** | B | **523** | A | **562** | D |
| **485** | B | **524** | B | **563** | B |
| **486** | C | **525** | B | **564** | B |
| **487** | D | **526** | A | **565** | D |
| **488** | B | **527** | A | **566** | D |
| **489** | D | **528** | A | **567** | C |
| **490** | C | **529** | A | **568** | B |
| **491** | A | **530** | C | **569** | D |
| **492** | A | **531** | B | **570** | C |
| **493** | C | **532** | A | **571** | A |
| **494** | A | **533** | A | **572** | A |
| **495** | C | **534** | B | **573** | B |
| **496** | A | **535** | B | **574** | A |
| **497** | D | **536** | A | **575** | B |
| **498** | B | **537** | B | **576** | B |
| **499** | B | **538** | A | **577** | B |
| **500** | B | **539** | A | **578** | A |
| **501** | B | **540** | B | **579** | A |
| **502** | A | **541** | B | **580** | A |
| **503** | B | **542** | B | **581** | A |
| **504** | C | **543** | B | **582** | B |
| **505** | D | **544** | A | **583** | B |
| **506** | A | **545** | B | **584** | A |
| **507** | B | **546** | B | **585** | B |
| **508** | C | **547** | A | **586** | B |
| **509** | B | **548** | A | **587** | B |
| | | **549** | A | **588** | A |
| **511** | B | **550** | B | **589** | A |
| **512** | A | **551** | B | **590** | B |
| **513** | A | **552** | B | **591** | B |
| **514** | B | **553** | A | **592** | A |
| **515** | B | **554** | C | **593** | B |
| **516** | A | **555** | C | **594** | B |
| **517** | A | | | **595** | A |
| **518** | A | **557** | >10000 | **596** | A |
| **519** | B | **558** | >10000 | **597** | B |
| **598** | **B** | | | | |
| **599** | **B** | | | | |
| **600** | **B** | | | | |
| **601** | **A** | | | | |

| | | | | | |
|---|---|---|---|---|---|
| A: < 10 nM, B: 11 nM - 100 nM, C: 101 nM - 1000 nM, D: 1001 nM - 5000 nM. | | | | | |

### Experimental example 3. Pharmacokinetics of the compound according to the present invention in mice

1) Experimental materials and instruments:
   LC-20AD high performance liquid chromatography (SHIMADZU, Japan)
   API4000 triple quadrupole mass spectrometer (Applied Biosystem, USA)
   PhenixWinnolin Pharmacokinetic software (Version 6.3, Certara, USA)
   High speed freezing centrifuge (Thermo Fisher Scientific)
   Analytical balance (Sartorius, SECURA225D-1CN)
   ICR mice (Chengdu Dossy Experimental Animals CO., LTD.)
2) Experimental methods and results

A suitable amount of drug was accurately weighed, and then the medium (5% DMSO, 15 % HS-15, 80% D5W) was added to prepare the solution at a concentration of 0.1 mg/ml (i.v.) and 0.3 mg/ml (i.g.).

18 healthy adult ICR mice were divided into two groups (9 mice for i.v. group; 9 mice for i.g. group), and administered by tail vein or by gavage after fasting overnight (free drinking water); using the satellite blood collection method, 3 animals were collected at each time point, and then 0.1 ml of blood obtained at different time points was anticoagulated with EDTA-K2, centrifuged at 4 °C for 5 min to separate the plasma, and stored at -80 °C for detection. The sampling time points after i.v. administration were 5 min, 15 min, 0.5 h, 1 h, 2 h, 4 h, 8 h, 12 h, and 24 h; while the sampling time points for i.g. group were before administration (0 h) as well as 0.5 h, 1 h, 2 h, 4 h, 6 h, 8 h, 12 h, and 24 h after administration. Then, the drug concentration in plasma was determined by LC/MS/MS.

The experimental results showed that the compound of the present invention had good pharmacokinetics. The exposure dose AUC_{inf} of the compound according to the present invention in mouse plasma by oral administration (dose 3 mg/kg) is shown in the following table [A: 30-5 µg * h/mL, B: 5-1 µg * h/mL, C: 1-0.1 µg * h/mL]:

| Example | After oral administration of 3 mg/kg, mouse plasma exposure AUC_{inf} (µg*h/mL) | Example | After oral administration of 3 mg/kg, mouse plasma exposure AUC_{inf} (µg*h/mL) |
|---|---|---|---|
| **75** | **A** | **321** | **A** |
| **76** | **B** | **322** | **A** |
| **82** | **C** | **323** | **A** |
| **90** | **B** | **324** | **A** |
| **94** | **B** | **328** | **A** |
| **97** | **A** | **330** | **A** |
| **98** | **C** | **338** | **B** |
| **99** | **A** | **355** | **A** |
| **110** | **A** | **358** | **B** |
| **118** | **A** | **359** | **B** |
| **119** | **A** | **360** | **B** |
| **122** | **A** | **361** | **B** |
| **125** | **A** | **362** | **C** |
| **126** | **A** | **367** | **B** |
| **148** | **B** | **370** | **A** |
| **154** | **B** | **371** | **A** |
| **164** | **A** | **372** | **A** |
| **166** | **A** | **373** | **A** |
| **171** | **A** | **377** | **A** |
| **175** | **B** | **406** | **C** |
| **183** | **C** | **415** | **A** |
| **187** | **C** | **423** | **B** |
| **188** | **B** | **426** | **B** |
| **189** | **A** | **438** | **A** |
| **190** | **A** | **446** | **A** |
| **193** | **B** | **455** | **B** |
| **195** | **B** | **456** | **A** |
| **196** | **B** | **460** | **C** |
| **206** | **A** | **464** | **C** |
| **209** | **A** | **475** | **A** |
| **210** | **A** | **483** | **A** |
| **212** | **A** | **501** | **B** |
| **223** | **B** | **502** | **B** |
| **231** | **A** | **504** | **C** |
| **232** | **C** | **516** | **B** |
| **239** | **C** | **521** | **A** |
| **240** | **B** | **528** | **A** |
| **242** | **C** | **553** | **A** |
| **248** | **B** | **558** | **A** |
| **251** | **B** | **582** | **B** |
| **254** | **A** | **584** | **B** |
| **259** | **A** | **588** | **A** |
| **268** | **B** | **591** | **A** |
| **275** | **B** | **593** | **A** |
| **279** | **A** | **598** | **A** |
| **282** | **B** | **599** | **A** |
| **312** | **C** | **605** | **A** |

As demonstrated by the experimental results, the compound of the present invention could target the degradation of AR, downregulate the expression level of AR, and particularly have significant degradation activity and inhibitory effect on the AR splicing variant AR-v7. The compound of the present invention could also effectively inhibit the proliferation of drug-resistant prostate cancer cells, and exhibit good metabolic stability and oral pharmacokinetic properties, therby having good application prospects in the manufacturer of protein degradation agents targeting AR as well as medicaments for the treatment of diseases regulated by AR.

## Claims

1. A compound represented by formula I, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof: 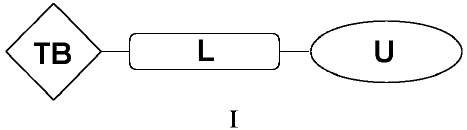
wherein TB is a target recognition/binding moiety, L is a linker, and U is a ubiquitin protease recognition/binding moiety; the three moieties are linked by a chemical bond.
Wherein, the above TB moiety has a structure represented by formula I-A: 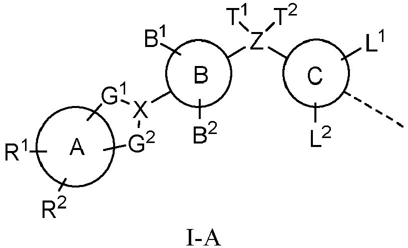
wherein, A is absent, aromatic ring, heteroaromatic ring, non-aromatic heterocycle, non-aromatic carbon ring, bridged ring, spiral ring, fused heterocycle, and fused heteroaromatic ring; the aromatic ring comprises a benzene ring; the heteroaromatic ring comprises 5-6 membered heteroaromatic rings; the non-aromatic heterocycles comprise 3-7 membered non-aromatic heterocycles; the non-aromatic carbon ring comprises 3-7 membered non-aromatic carbon rings; the fused heterocycle comprises a (5-6-membered heterocycle)-fused 5-6-membered heterocycle; the fused heteroaromatic ring comprises a (5-6-membered heteroaromatic ring)-fused 5-6-membered heterocycle, and a (5-6-membered heteroaromatic ring)-fused 5-6-membered heteroaromatic ring;
wherein, R¹ and R² are each independently selected from the group consisting of absence, hydrogen, halogen, cyano, amino, hydroxyl, C1-C6 alkoxy, C1-C6 alkylamino, C1-C6 alkylthiol, C1-C6 alkylsulfonyl, C1-C6 alkylsulfinyl, C1-C6 alkylcarbonyl, C1-C6 alkylaminocarbonyl, -NR³R⁴, -CR^{a}R³R⁴, -NR³-CO-R⁴, -CO-NR³R⁴, -NR³-SO₂-R⁴, -SO₂-NR³R⁴, -CO-R³, -SO-R³, -SO₂-R³, -CR³=CH₂, -OR³, -SR³, substituted or unsubstituted C1-C6 alkyl, substituted or unsubstituted 3-8-membered cycloalkyl, substituted or unsubstituted heterocyclyl, substituted or unsubstituted fused heterocyclyl, substituted or unsubstituted spiro-heterocyclyl, substituted or unsubstituted aromatic heterocyclyl, and substituted or unsubstituted phenyl; R¹ and R² can be linked to form a ring;
wherein, the substituted substituents are R^{a}, R³, and R⁴, which are each independently selected from the group consisting of hydrogen, halogen, hydroxyl, amino, cyano, C1-C6 alkenyl, C1-C6 alkoxy, C1-C6 alkylamino, C1-C6 alkylsulfonyl, substituted or unsubstituted C1-C6 alkyl, substituted or unsubstituted 3-8-membered cycloalkyl, substituted or unsubstituted 3-8-membered heterocyclyl, substituted or unsubstituted aromatic heterocyclyl, substituted or unsubstituted phenylcarbonyl, and sulfonyl; any two of R^{a}, R³, and R⁴ can be linked to form a 3-8-membered ring; the substituted substituents are selected from the group consisting of halogen, amino, hydroxyl, and hydroxyl-substituted C1-6 alkyl;
wherein, G¹ and G² are each independently linked to A by a chemical bond; wherein G¹ is selected from the group consisting of absence, -(CH₂)_{y}-, -(CH₂)ₘ-CR^{g1}R^{g2}-, -(CH₂)ₘ-CO-, O, S, SO, SO₂, and NR^{g1}; G² is selected from the group consisting of absence, -(CH₂)ₙ-CR^{g3}R^{g4}-, -(CH₂)ₙ-O-, O, S, SO, SO₂, and NR^{g2}; wherein y, m, and n are integers from 0 to 3; R^{g1}, R^{g2}, R^{g3}, and R^{g4} are each independently selected from the group consisting of hydrogen, C1-C6 alkyl, halogen, and hydroxyl; R^{g1} and R^{g2}, R^{g3} and R^{g4} can be linked to form a ring; when G1 and G2 are both absent, X is directly linked to A by a chemical bond;
wherein, X is selected from the group consisting of -N-, -NR⁵-, -O-, -S-, -CO-, - SO-, -SO₂-, -CONR⁵-, -NR⁵CO-, -CH-, -CHR⁵-, and -CR⁵R^{5'}-; said R⁵ and R^{5'} are each independently selected from the group consisting of H and C1-C6 alkyl;
wherein, rings B and C are each independently selected from the group consisting of substituted or unsubstituted benzene ring, thiophene, pyridine, pyrimidine, 5-6-membered aromatic heterocycle, 3-8-membered cycloalkane, and 3-8-membered heterocycle; the substituted substituents are halogen and cyano;
wherein, B¹ and B² are each independently selected from the group consisting of H, halogen, and C1-C6 alkyl; or B¹, B² and X are linked to form a ring;
wherein, Z is selected from the group consisting of -C-, -CO-, -CH-, -CH₂-, -O-, - N-, -S-, -SO-, and -SO₂-;
wherein, T¹ and T² are each independently selected from the group consisting of absence, H, hydroxyl, amino, substituted or unsubstituted C1-C6 alkyl, C1-C6 oxaalkyl, C1-C6 azaalkyl, C3-C6 cycloalkyl, acyl, C1-C6 alkoxy, and C1-C6 alkylamino; or T¹ and T² can be linked to each other to form a ring; the substituted substituent is hydroxyl or amino;
wherein, L¹ and L² are each independently selected from the group consisting of H, halogen, cyano, amino, hydroxyl, and C1-C6 alkyl;
wherein, said L moiety has a structure as represented by formula I-L: 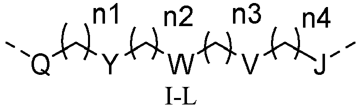
wherein, Q, J, Y, W, and V are each independently selected from the group consisting of absence, -O-, -S-, -SO-, -SO₂-, -NR^{q1}-, -NR^{j1}-, -C≡C-, -C=C-, -NR^{q1}CO-, -NR^{j1}CO-, -CO-, -CONH-, -NR^{q11}SO₂-, -CR^{q1}R^{q2}-, -CR^{y1}R^{y2}-, -CR^{w1}R^{w2}-, -CR^{v1}R^{v2}-, - CR^{j1}R^{j2}-, -[(OCH₂CH₂O)₂]ₙ₅-, -[(OCH₂CH₂O)₃]ₙ₆-, (R^{c},R^{d})-substituted or unsubstituted 3-7-membered cycloalkyl, (R^{c},R^{d})-substituted or unsubstituted 3-7-membered heterocyclyl, (R^{c},R^{d})-substituted or unsubstituted phenyl, (R^{c},R^{d})-substituted or unsubstituted aromatic heterocyclyl, (R^{c},R^{d})-substituted or unsubstituted fused aromatic heterocyclyl; said R^{c} and R^{d} are each independently selected from H, halogen, and C1-3 alkyl; or R^{c} and R^{d} can be linked to each other to form a ring;
wherein, R^{q1}, R^{q2}, R^{y1}, R^{y2}, R^{w1}, R^{w2}, R^{v1}, R^{v2}, R^{j1}, and R^{j2} are each independently selected from the group consisting of H; halo-substituted or unsubstituted C1-C6 alkyl, C3-C6 cycloalkyl, C1-C6 oxaalkyl, C1-C6 azaalkyl, C3-C6 oxacycloalkyl, and C3-C6 azacycloalkyl;
wherein, R^{q1} and R^{q2}, R^{y1} and R^{y2}, R^{w1} and R^{w2}, R^{v1} and R^{v2}, and R^{j1} and R^{j2} can be linked to each other to form a ring;
wherein, n1, n2, n3, n4, n5, and n6 are each independently selected from an integer of 0 to 6;
wherein, Q and J can freely linked to TB moiety or U moiety;
wherein, said U moiety has a structure as represented by formula I-U: 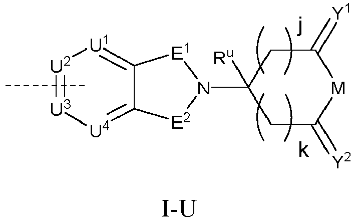
wherein, M is selected from the group consisting of -O-, -S-, -CR^{m}-, and -NR^{m}-; wherein, R^{m} is selected from the group consisting of H, C₁₋₆ alkyl, C₃₋₆ cycloalkyl, and C₃₋₆ heteocyclyl, and 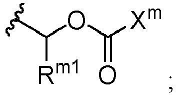
said R^{m1} is selected from the group consisting of H, C₁₋₆ alkyl, and C₃₋₆ cycloalkyl;
X^{m} is selected from the group consisting of -CR^{m2}R^{m3}-, -OR^{m2}-, and -NR^{m2}R^{m3}-, wherein, R^{m2} and R^{m3} are each independently selected from the group consisting of H, C₁₋₆ alkyl, C₃₋₆ cycloalkyl, and C₃₋₆ heteocyclyl, and C₁₋₆ oxaalkyl; R^{m2} and R^{m3} can be linked to form a ring;
E¹ and E² are each independently selected from the group consisting of -CO-, - CS-, -NR^{e1}-, -O-, -S-, -SO₂-, -CH₂-, -CD₂-, -CR^{e2}R^{e3}-, 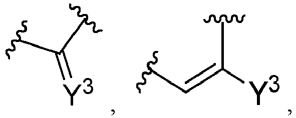 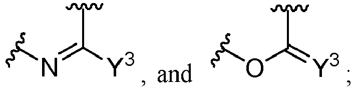 R^{e1}, R^{e2}, and R^{e3} are each independently selected from the group consisting of C₁₋₆ alkyl, H, halogen, hydroxyl, and amino;
Y¹, Y², and Y³ are each independently selected from the group consisting of H, O, S, and C₁₋₃ alkyl;
J and k are each independently selected from an integer of 0 to 3, and J and k are not both 0;
U¹, U², U³, and U⁴ are each independently selected from the group consisting of O, S, N, -CR^{g1}-, -CR^{g2}-, -CR^{g3}-, and -CR^{g4}-, wherein R^{g1}, R^{g2}, R^{g3}, and R^{g4} are each independently selected from the group consisting of H, halogen, hydroxyl, amino, thiol, sulfonyl, sulfinyl, nitro, cyano, CF₃, heterocyclyl, C₁₋₆ alkyl, C₃₋₆ cycloalkyl, C₁₋₆ alkoxy, C₁₋₆ alkylamino, C₂₋₆ alkenyl, and C₂₋₆ alkynyl.

2. The compound according to claim 1, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** G¹ and G² in TB moiety are linked to different atoms in A by a chemical bond, as represented by formula II-A; or, G¹ and G² in TB moiety are linked to a same atom in A by a chemical bond, as represented by formula II-B:

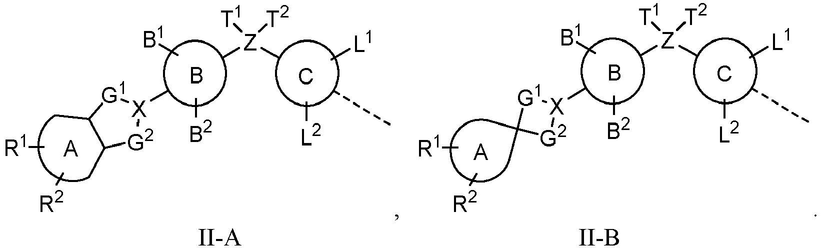

3. The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** A is an aromatic heterocycle, G¹ and G² are not absent, and the TB moiety in the compound has a structure as represented by formula III-A: 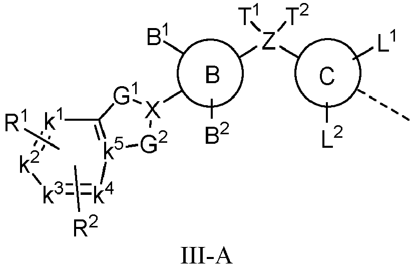 wherein, k¹, k², k³, k⁴, and k⁵ are each independently selected from CH or N, but they are not CH at the same time.

4. The compound according to claim 3, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** G¹ is - CH₂-, G² is -CH₂- or -CH₂CH₂-, and the TB moiety in the compound has a structure as represented by formula IV-A or IV-B: 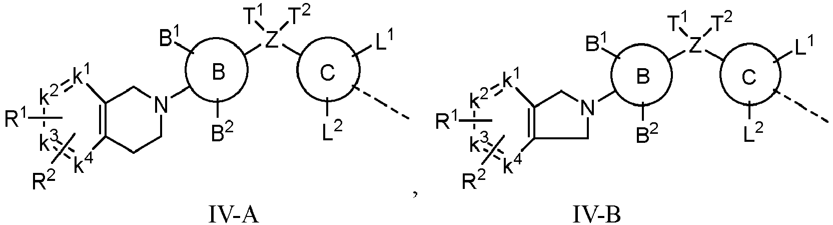 wherein, k¹, k², k³, and k⁴ are each independently selected from CH or N, but they are not CH at the same time.

5. The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** A is an aromatic heterocycle, G² is absent, and the TB moiety in the compound has a structure as represented by formula V-A: 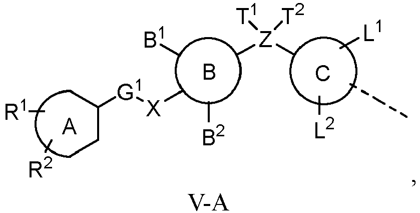
wherein, G¹ is not absent;
alternatively, the TB moiety in the compound has a structure as represented by formula V-B:

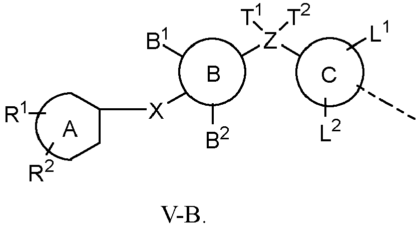

6. The compound according to claim 5, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** A is a 6-membered aromatic heterocycle, G² is absent, and the TB moiety in the compound has a structure as represented by formula VI-A: 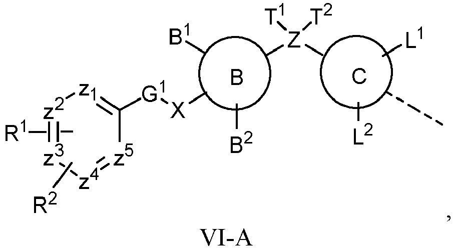
wherein, G¹ is not absent; z¹, z², z³, z⁴, and z⁵ are each independently selected from CH or N, but they are not CH at the same time;
alternatively, the TB moiety in the compound has a structure as represented by formula VI-B: 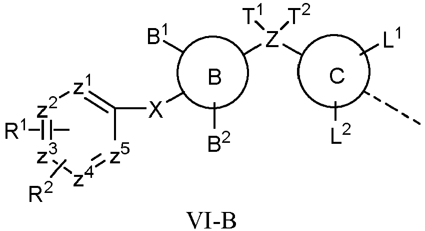
wherein, z¹, z², z³, z⁴, and z⁵ are each independently selected from CH or N, but they are not CH at the same time;

7. The compound according to claim 5, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** A is a 5-membered aromatic heterocycle, and G² is absent;
the TB moiety in the compound has a structure as represented by formula VII-A: 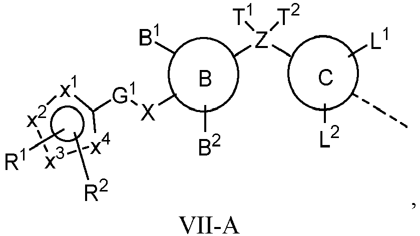
wherein, G¹ is not absent; x¹, x², x³, and x⁴ are each independently selected from CH, N, O or S, but they are not CH at the same time;
alternatively, the TB moiety in the compound has a structure as represented by formula VII-B: 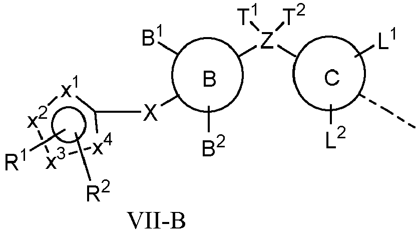
wherein, x¹, x², x³, and x⁴ are each independently selected from CH, N, O or S, and they are not CH at the same time.

8. The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** A is a (5-membered aromatic heterocycle)-fused 6-membered aromatic heterocycle, G² is absent, and the TB moiety in the compound has a structure as represented by formula VIII-A: 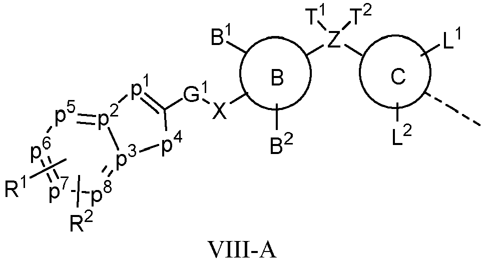 wherein, p¹, p², p³, p⁴, p⁵, p⁶, p⁷, and p⁸ are each independently selected from CH, N, O or S, and at least one of p¹, p², p³, and p⁴ is not CH, and at least one of p², p³, p⁵, p⁶, p⁷, and p⁸ is not CH.

9. The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** A is absent, G¹ and G² are absent, R¹ is H, R² is absent, X is -O-, and the TB moiety in the compound has a structure as represented by formula IX-A:

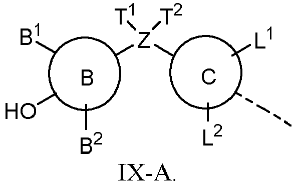

10. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** 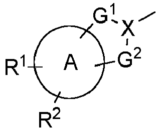 in the TB moiety of the compound has any one of the structures as represented in the following, wherein, X, X¹, and X² are each independently selected from the group consisting of -N-, -NR⁵-, -O-, -S-, -CO-, -SO-, - SO₂-, -CONR⁵-, -NR⁵CO-, -CH-, -CHR⁵-, and -CR⁵R^{5'}-; said R⁵ and R^{5'} are each independently selected from the group consisting of H and C1-C6 alkyl:

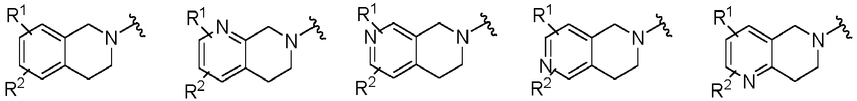

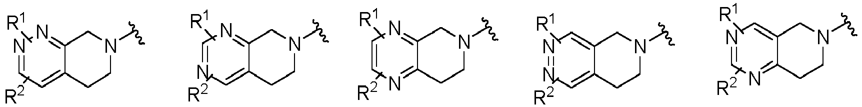

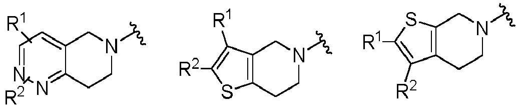

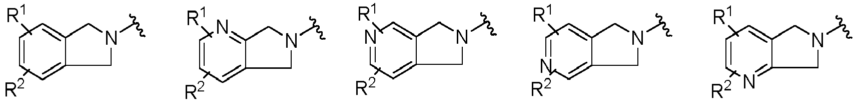

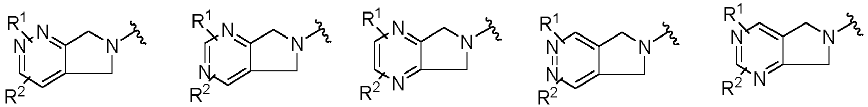

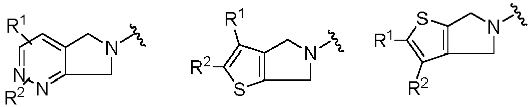

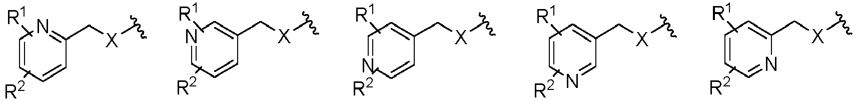

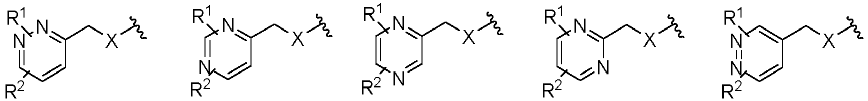

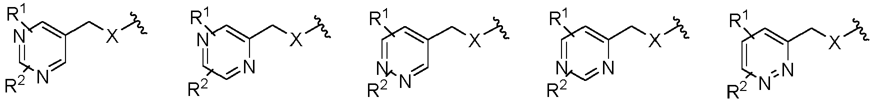

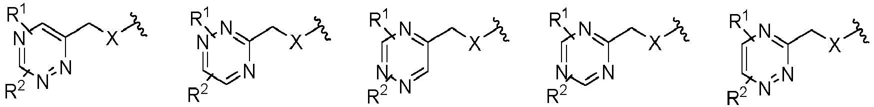

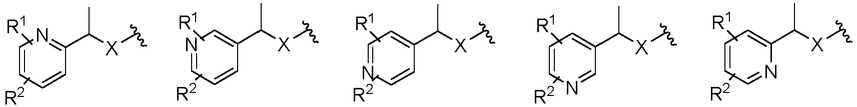

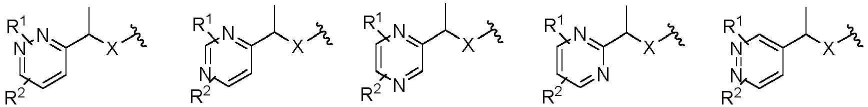

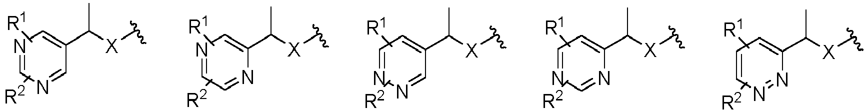

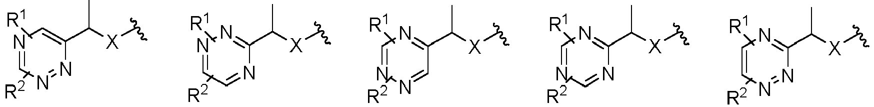

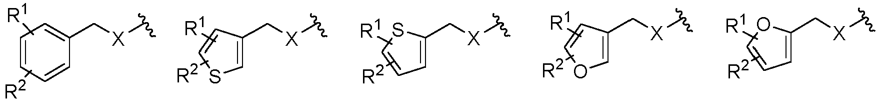

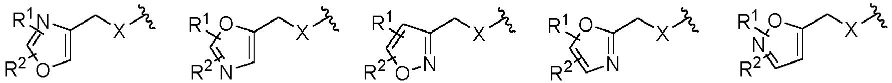

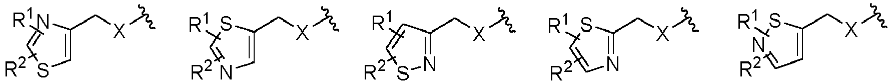

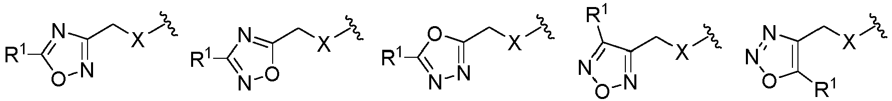

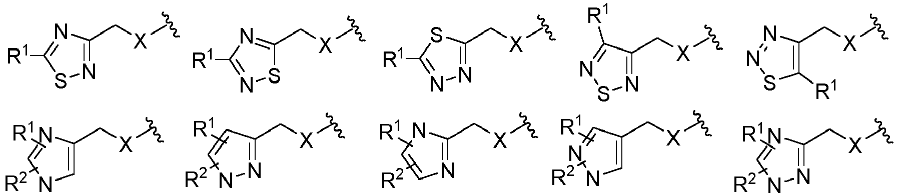

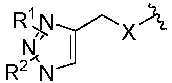

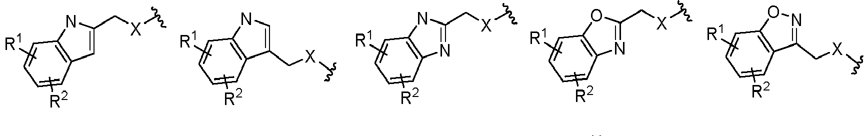

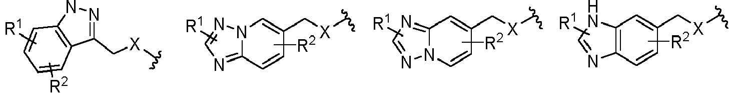

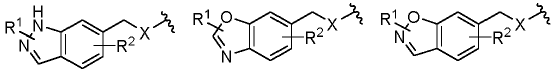

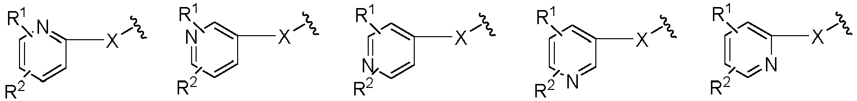

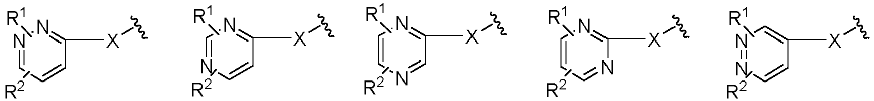

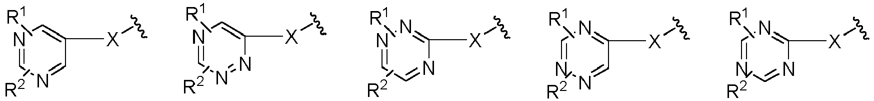

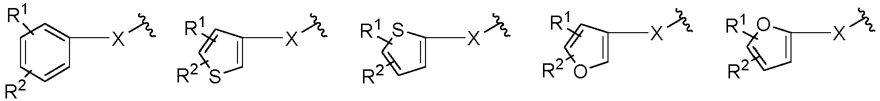

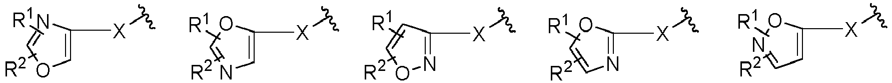

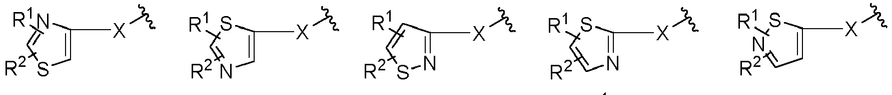

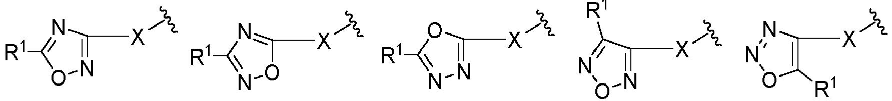

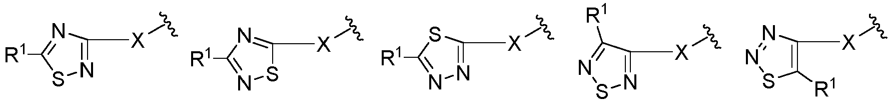

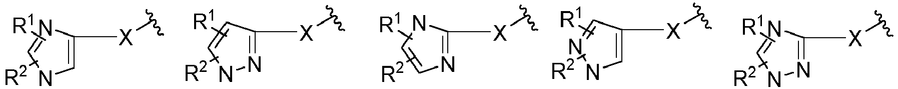

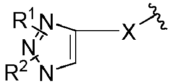

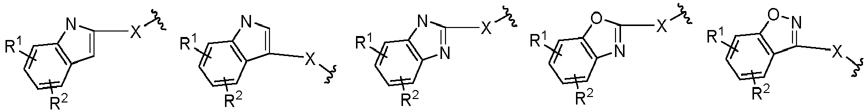

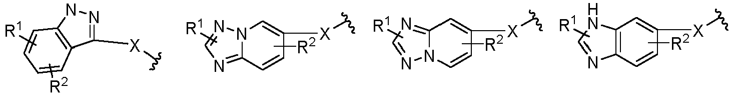

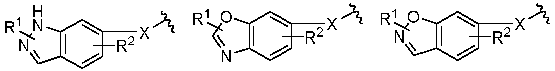

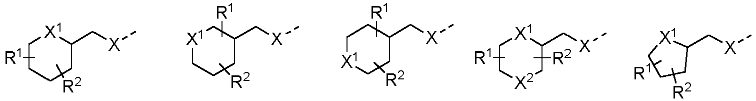

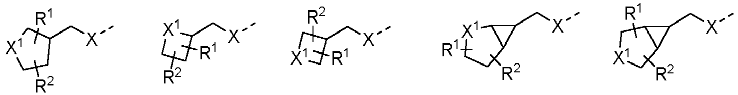

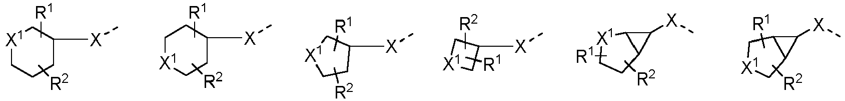

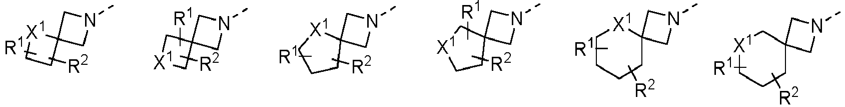

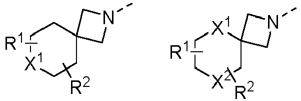

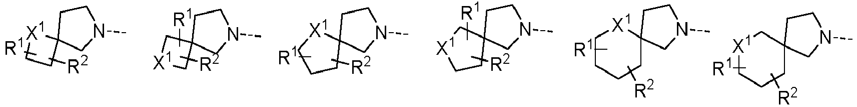

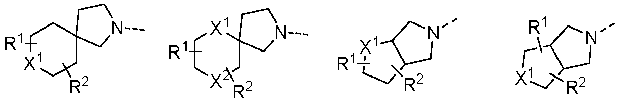

11. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** 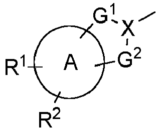 in the TB moiety of the compound has a structure as represented in the following: 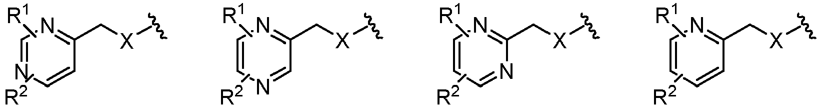 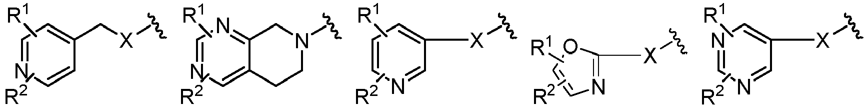 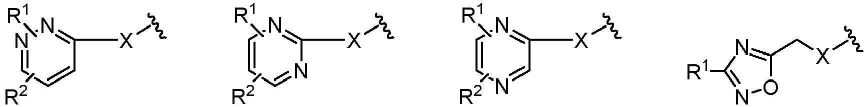 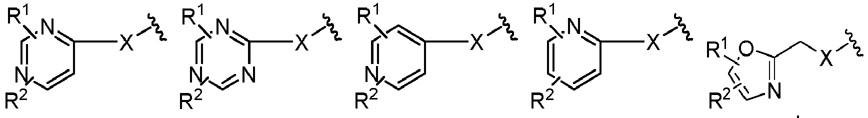 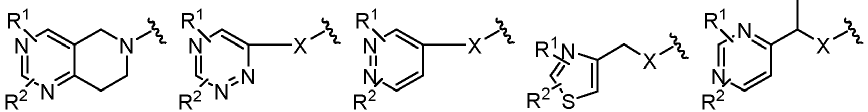 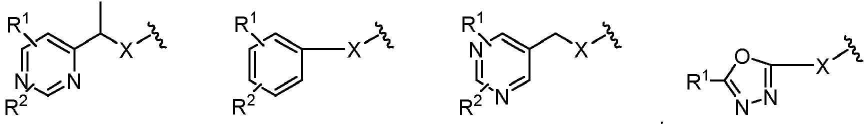 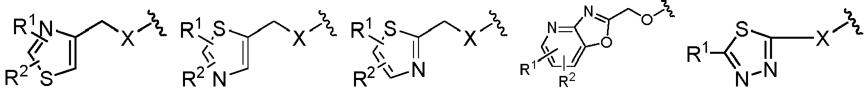 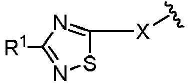 or hydroxyl.

12. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** R¹ and R² in the TB moiety of the compound are each independently selected from the group consisting of H, CF₃, or any one of the following structures: 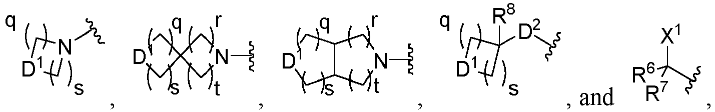
wherein, q, r, s, and t are each independently selected from an integer of 0 to 5;
wherein, D¹ and D² are each independently selected from the group consisting of - O-, -S-, -SO-, -SO₂-, -NR⁶-, -NCOR⁶-, -NSO₂R⁷-, -CR⁶X¹-, and -CR⁶R⁷-; wherein R⁶ and R⁷ are each independently selected from the group consisting of H, halogen, hydroxyl, amino, C1-C6 alkyl, C1-C6 alkoxy, and C1-C6 alkylamino; or R⁶ and R⁷ are linked to form a ring;
wherein, X¹ is selected from the group consisting of -OR⁸-, -NR⁸R⁹-, -NCOR⁸-, - NSO₂R⁹-, and -CR⁸R⁹-; wherein R⁸ and R⁹ are each independently selected from the group consisting of H, substituted or unsubstituted C1-C6 alkyl, and C3-C6 cycloalkyl; or R⁸ and R⁹ can be linked to each other to form a ring; the substituted substituent is hydroxyl or amino.

13. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** R¹ and R² in the TB moiety of the compound are each independently selected from the group consisting of H, CF₃, or any one of the following structures: H, methyl, ethyl, isopropyl, cyclopropyl, cyclobutyl, cyclopentyl, cyclohexyl, methoxy, ethoxy, propoxy, isopropoxy, cyclopropoxy, cyclobutoxy, cyclopentoxy, amino, methylamino, ethylamino, propylamino, isopropylamino, cyclopropylamino, cyclobutylamino, hydroxyethylamino, 1-hydroxymethylcyclopropylamino, N,N-dimethylamino, N,N-diethylamino, formyl, acetyl, propanoyl, isopropanoyl, cyclopropylcarbonyl, 1-hydroxymethyl, 1-hydroxyethyl, 1-hydroxypropyl, 1-hydroxyisopropyl, 1-hydroxycyclopropyl, aminocarbonyl, N-methylaminocarbonyl, N-ethylaminocarbonyl, N,N-dimethylcarbonyl, acridinylcarbonyl, pyrrolidinylcarbonyl, methoxycarbonyl, ethoxycarbonyl, acridinyl, tetrahydropyrrole, piperidine, morpholine, piperazine, N-methylpiperazine, N-ethylpiperazine, N-cyclopropylpiperazine, 2-methylpiperazine, 3-methylpiperazine, 2,2-dimethylpiperazine, 3,3-dimethylpiperazine, 2,3-dimethylpiperazine,

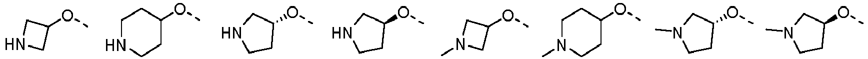

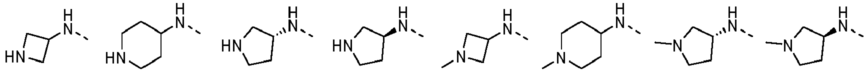

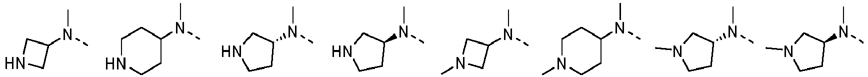

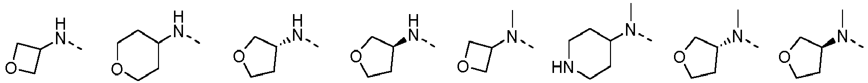

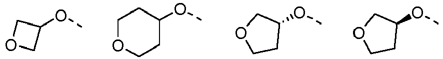

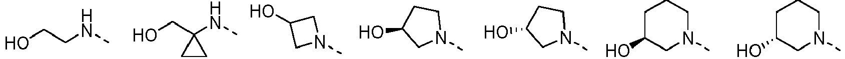

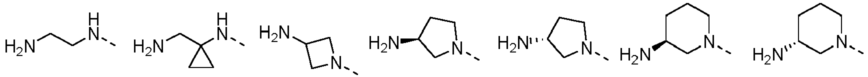

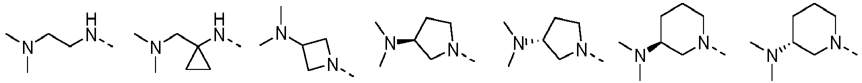

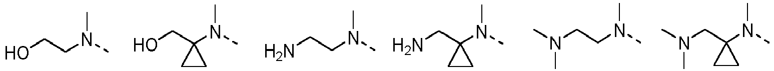

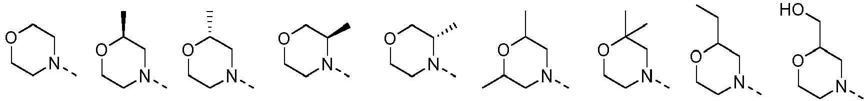

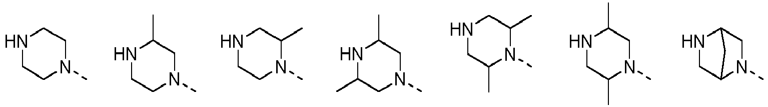

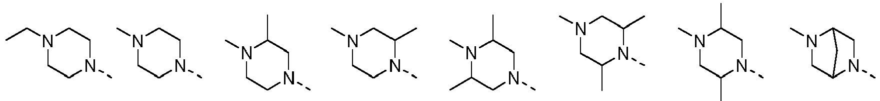

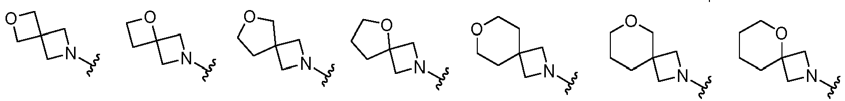

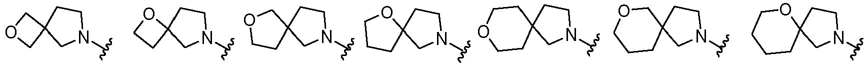

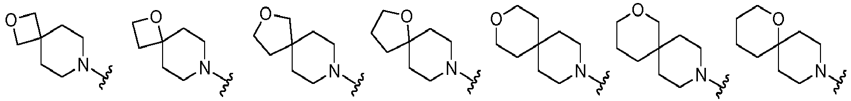

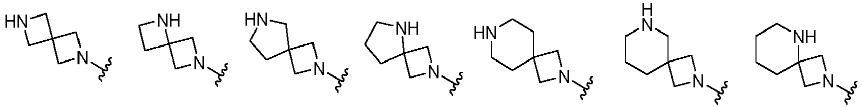

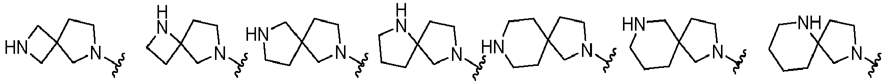

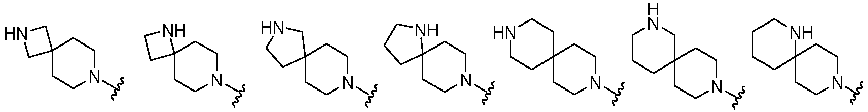

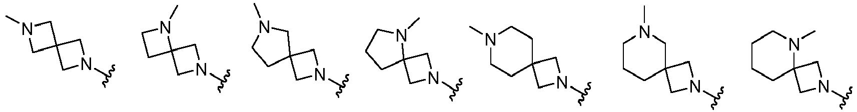

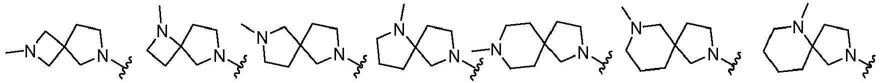

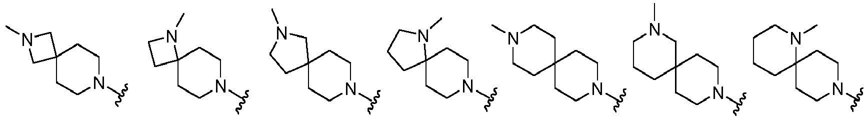

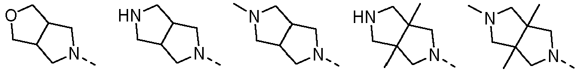

14. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** R¹ and R² in the TB moiety are each independently selected from the group consisting of H, F, Cl, Br, I, CH₃, amido, substituted or unsubstituted 1,2-diazolyl, 1,3-diazolyl, N-methyl-1,2-diazolyl, *N¹*-1,2,3-triazolyl, *N²*-1,2,3-triazolyl, 1,3,4-triazolyl, 1,2,4-triazolyl, 1,2,5-triazolyl, 1,2-oxazolyl, 1,3-oxazolyl, 1,2,3- oxadiazolyl, 1,2,4-oxadiazolyl, 1,3,5-oxadiazolyl, 1,2-thioxazolyl, 1,3-thioxazolyl, 1,2,3-thiadiazolyl, 1,2,4-thiadiazolyl, 1,3,5-thiadiazolyl, *N²*-4-fluoro-1,2,3-triazolyl, *N²*-4-methyl-1,2,3-triazolyl, 2-methyl-1,3,4-oxadiazolyl, 5-methyl-1,2,4-oxadiazolyl, 3-methyl-1,2,4-oxadiazolyl, pyridyl, pyridazinyl, pyrimidinyl, and pyrazinyl; the substituted substituent is selected from the group consisting of halogen, hydroxyl, amino, C1-C6 alkoxy, C1-C6 alkylamino, cyano, amido, C1-C6 alkyl, and C3-C6 cycloalkyl.

15. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** R¹ and R² in the TB moiety are each independently selected from the group consisting of H, amino, methylamino, dimethylamino, methylthio, methanesulfonyl, 1,2,3-triazolyl, 1,2,4-triazolyl, pyridyl, F, I, 2-methyl-1,3,4-oxadiazolyl, cyano, 5-methyl-1,2,4-oxadiazolyl, 1-hydroxyethyl, 1-hydroxypropyl, acetyl, propionyl, cyclopropylcarbonyl, cyclobutylcarbonyl, isopropionyl, 1-hydroxyisopropyl, 1-hydroxyisobutyl, methoxycarbonyl, ethoxycarbonyl, 1-hydroxymethyl, methoxy, ethoxy, cyclopropyloxy, aminocarbonyl, N-methylaminocarbonyl, 1-methyl-1,2-diazolyl, 1,2-diazolyl, 1,3-diazolyl, 3-methyl-1,2,4-oxadiazolyl, 1,2,4-oxadiazolyl, 1,3,4-oxadiazolyl, 3,5-dimethyl-1,2-oxazolyl, pyrrolidinyl, 1,3,4-thiadiazolyl, 3-methyl-1,2,4-thiadiazolyl, piperidinyl, carboxyl, 1,3-thiazolyl, -NHCN, 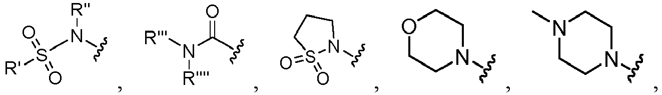 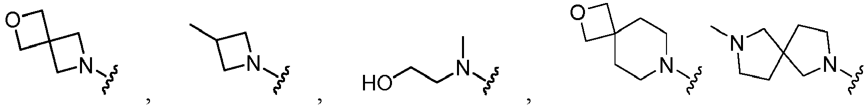 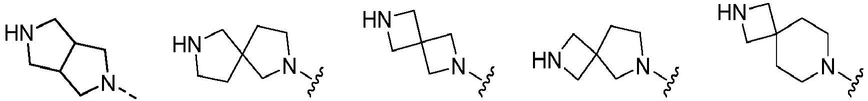 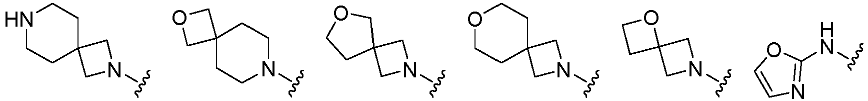 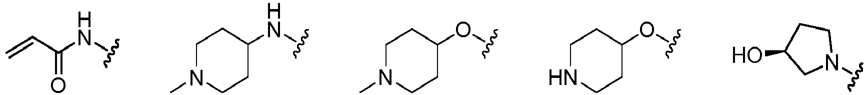 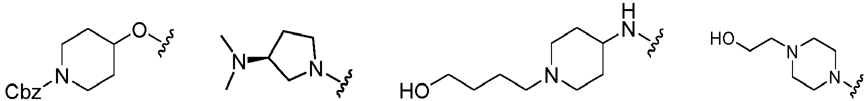 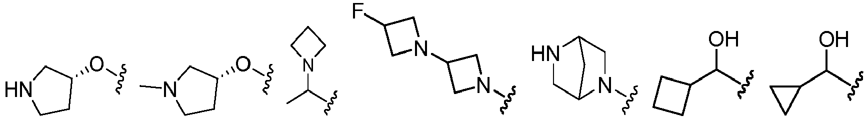 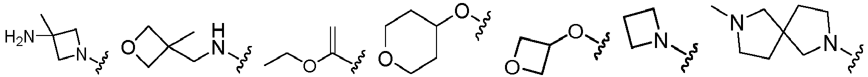 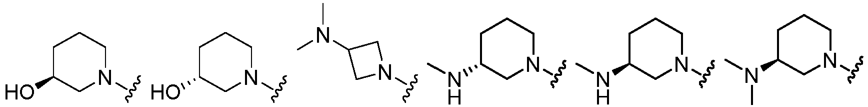 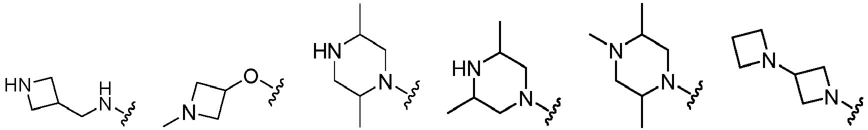 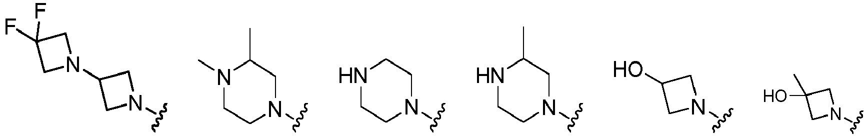 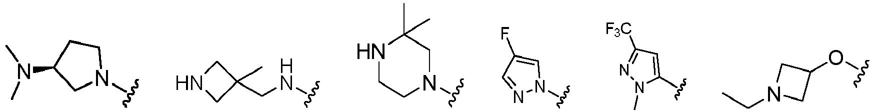 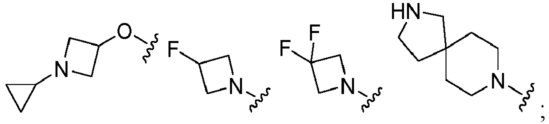
wherein, R' is selected from the group consisting of methyl, ethyl, and cyclopropyl; and R" is selected from the group consisting of H and methyl; R‴ and Rʺʺ are each independently selected from the group consisting of H, methyl, and ethyl; or, R‴ and Rʺʺ are linked to form methyl-substituted or unsubstituted 4-6-membered heterocycle;
preferably, R¹ and R² are not

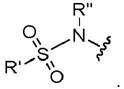

16. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** 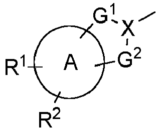 in the TB moiety of the compound has the structure selected from the group consisting of:

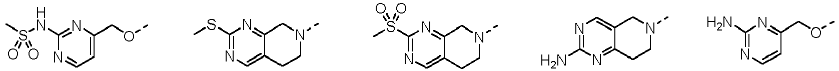

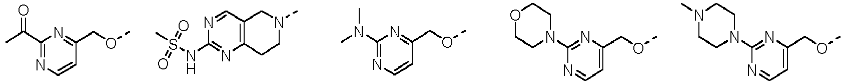

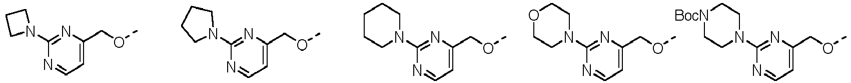

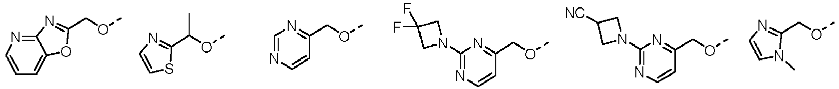

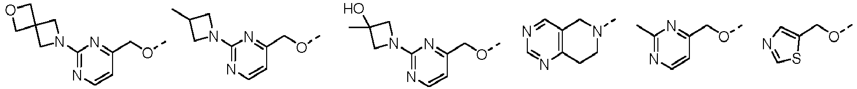

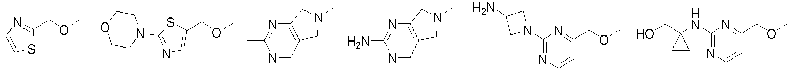

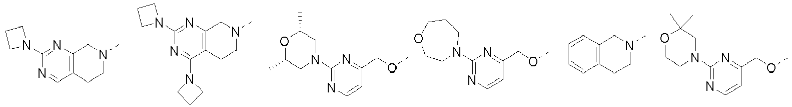

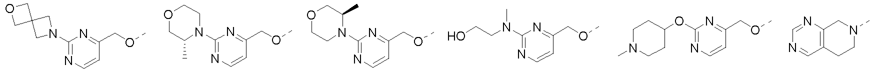

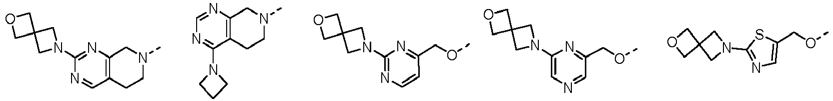

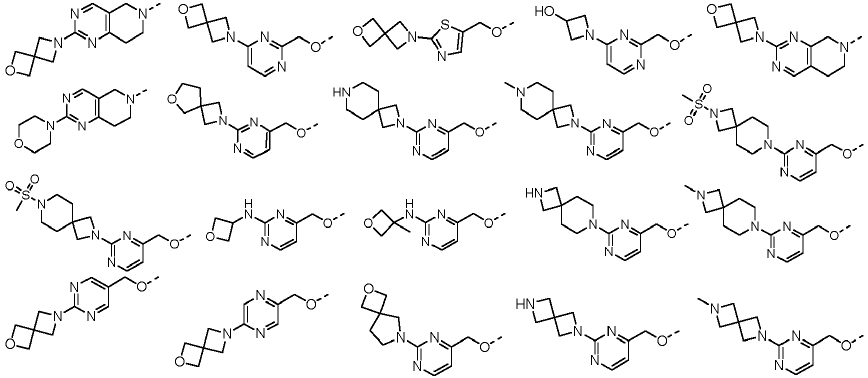

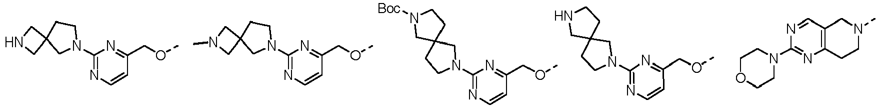

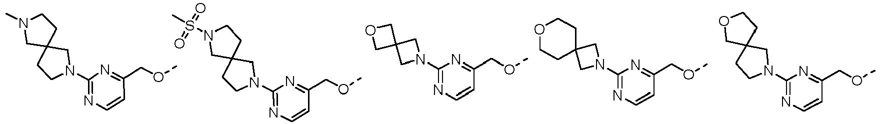

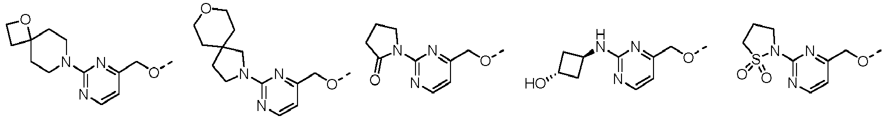

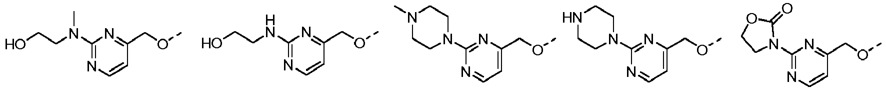

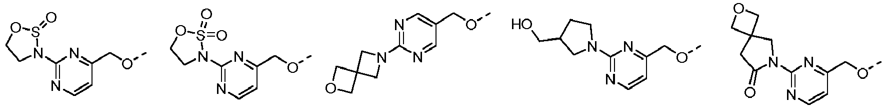

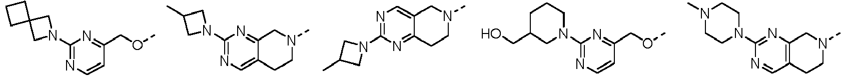

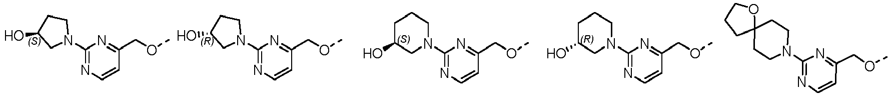

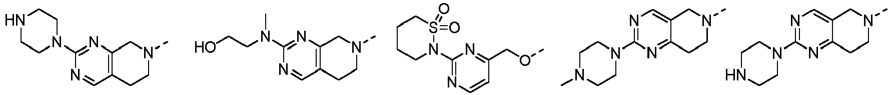

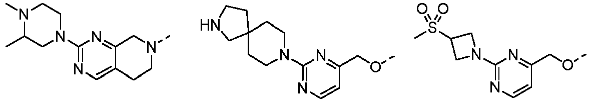

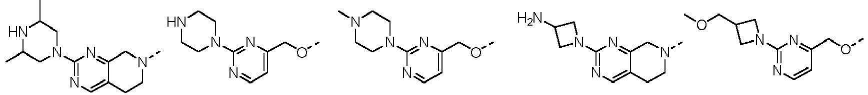

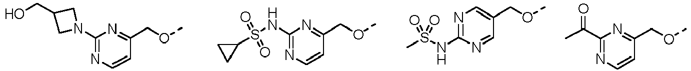

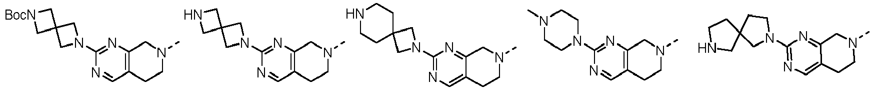

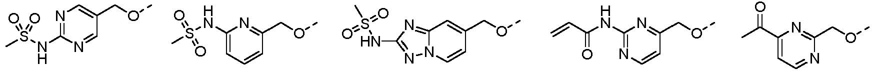

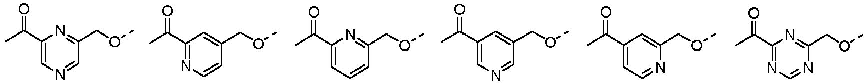

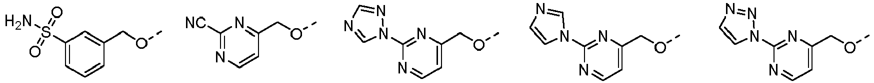

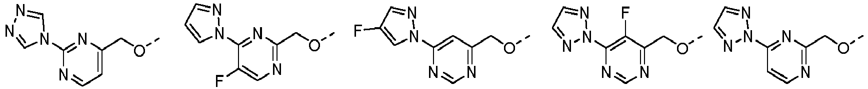

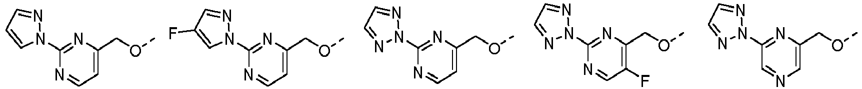

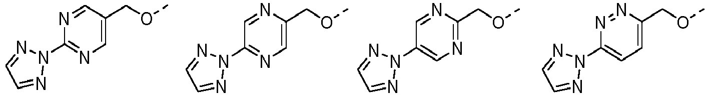

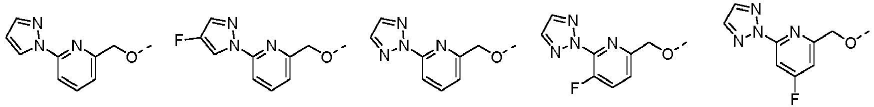

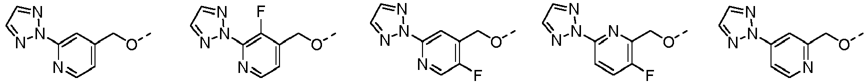

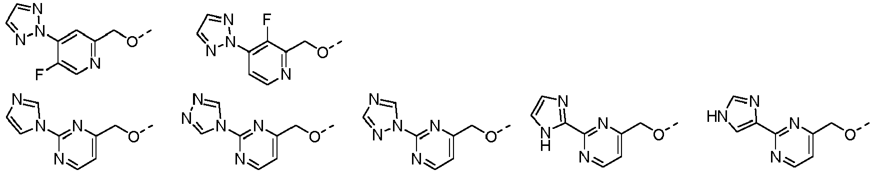

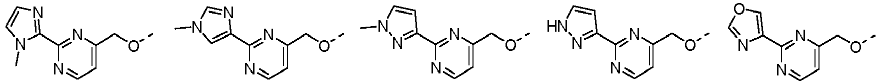

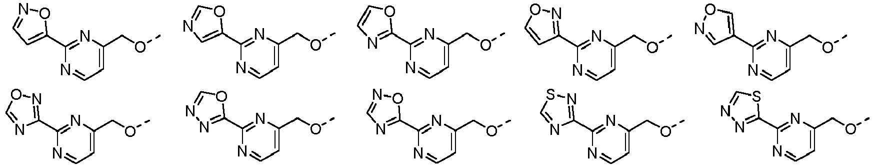

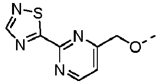

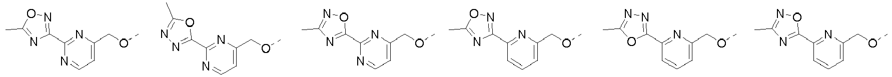

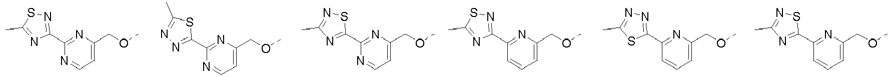

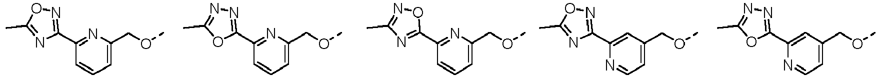

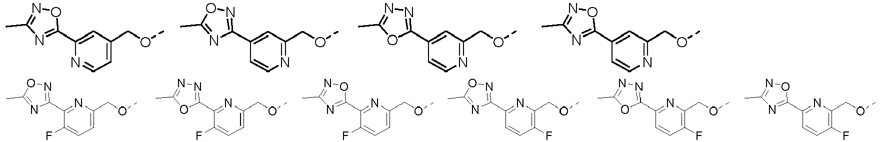

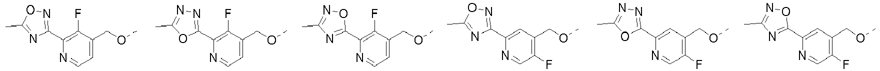

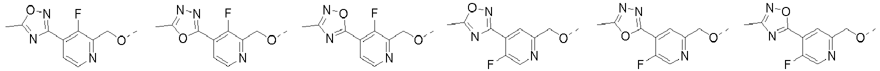

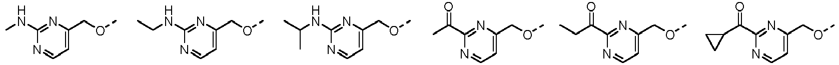

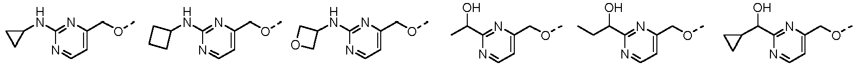

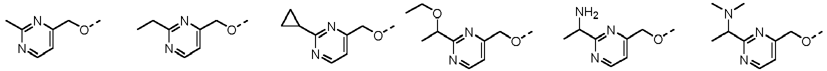

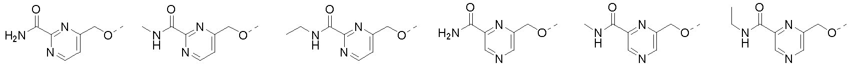

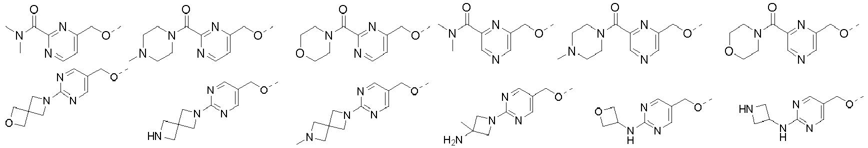

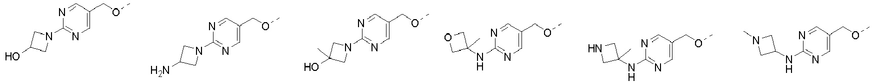

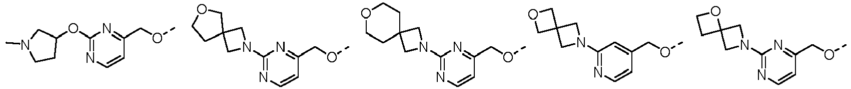

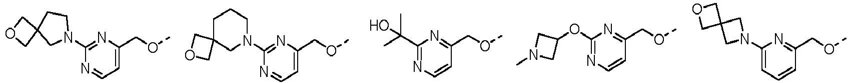

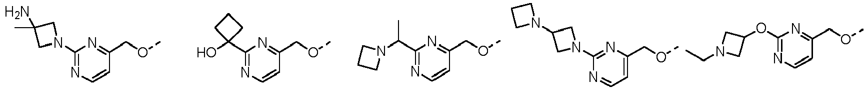

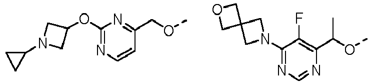

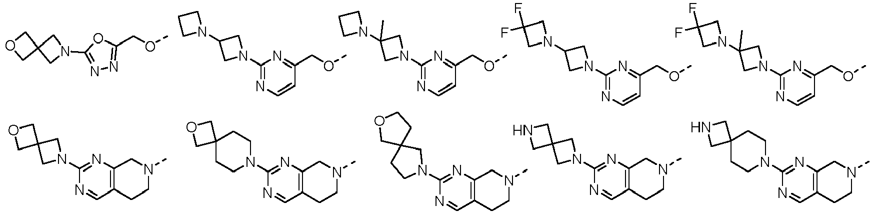

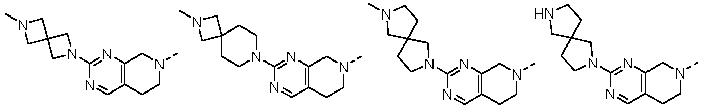

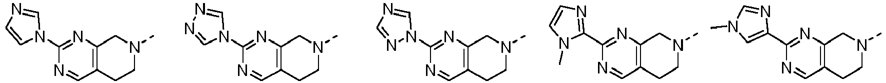

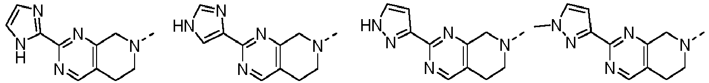

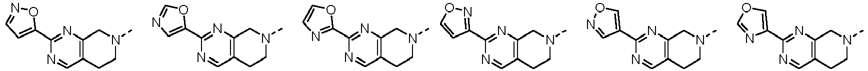

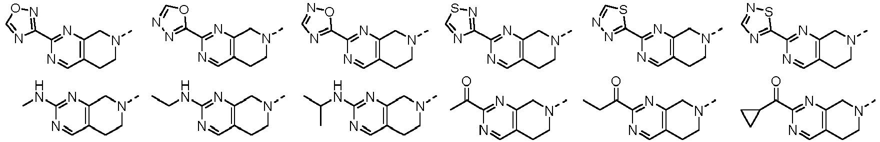

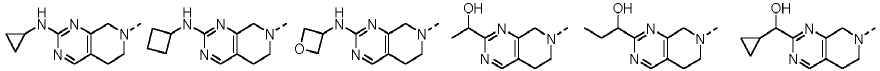

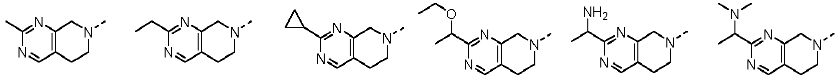

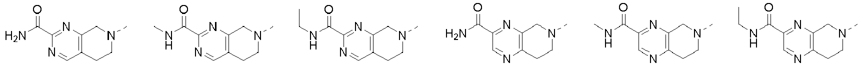

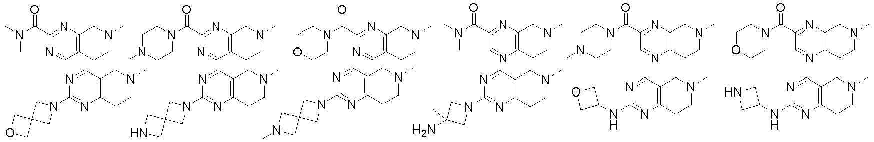

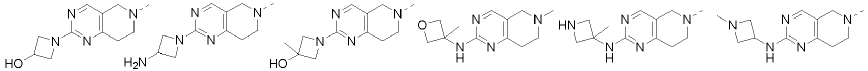

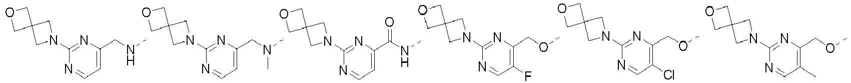

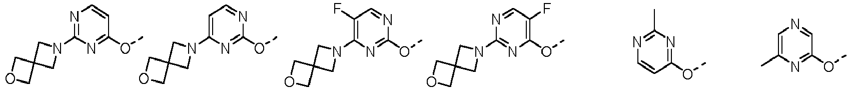

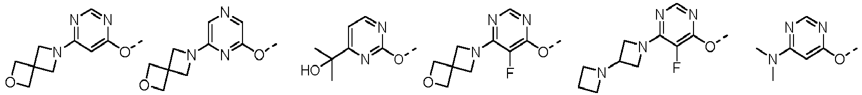

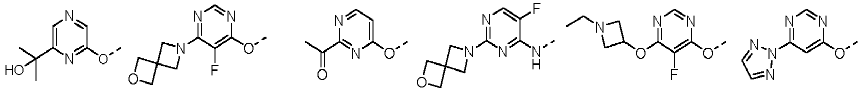

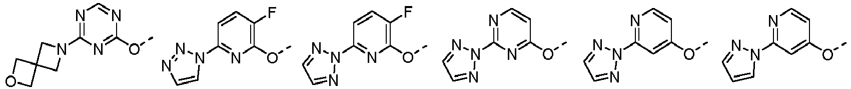

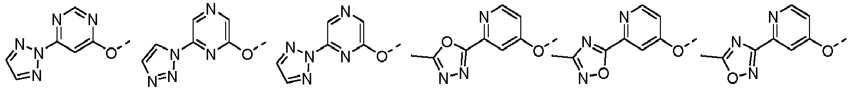

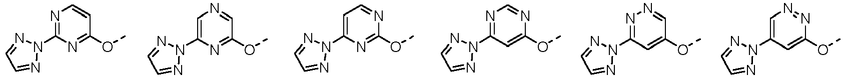

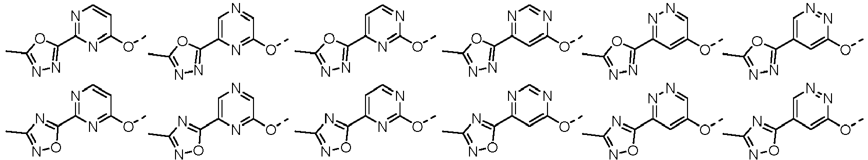

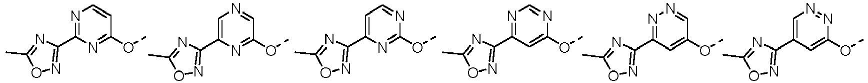

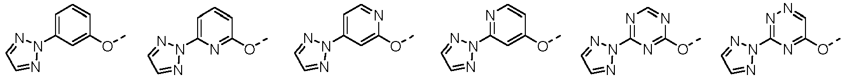

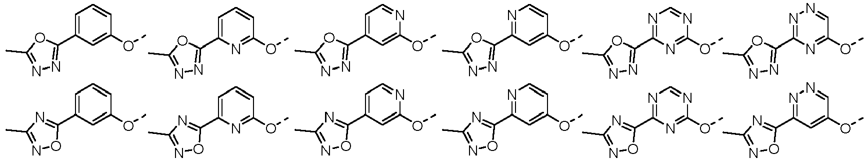

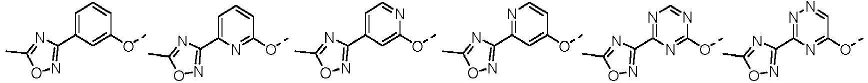

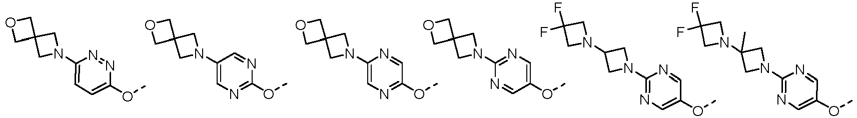

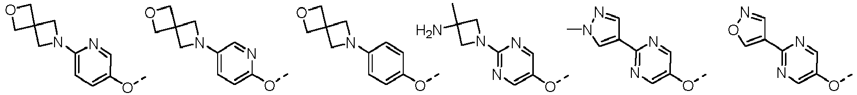

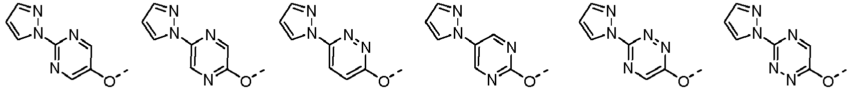

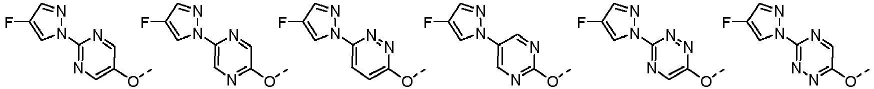

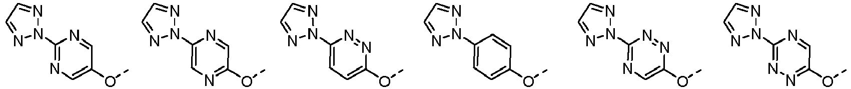

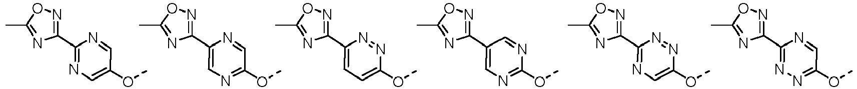

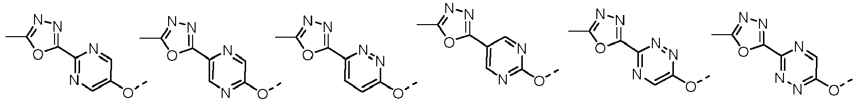

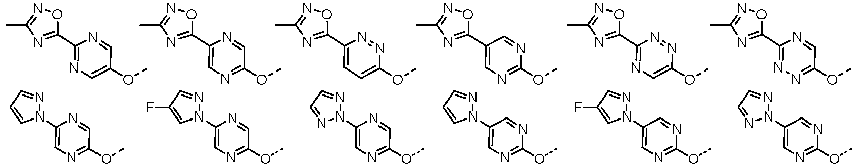

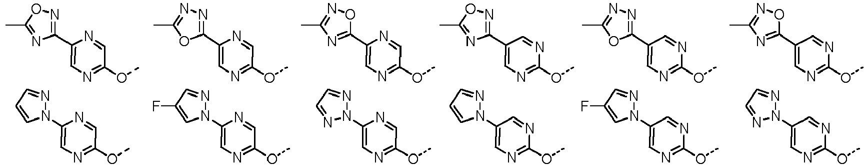

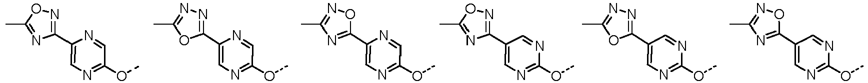

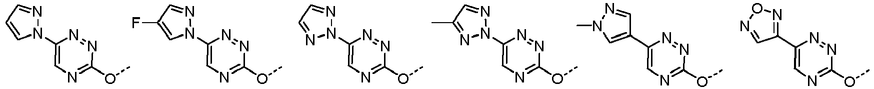

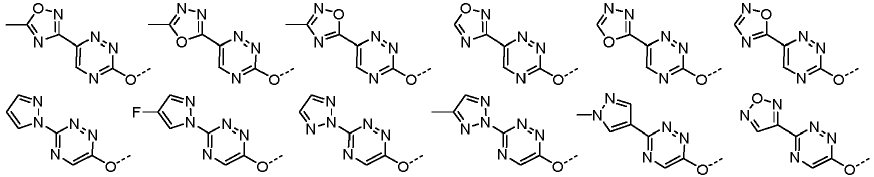

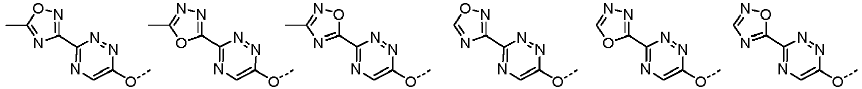

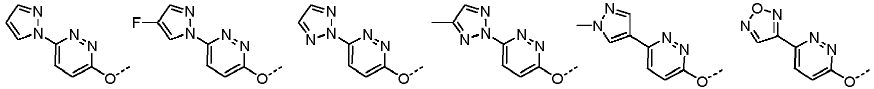

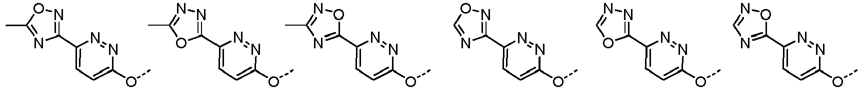

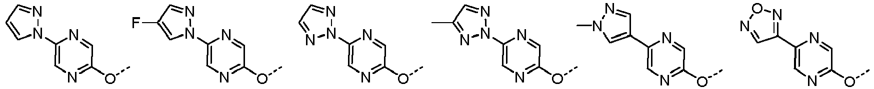

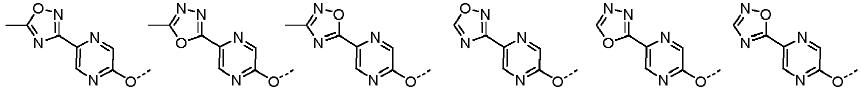

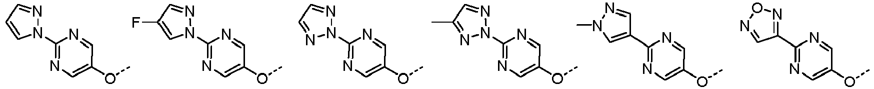

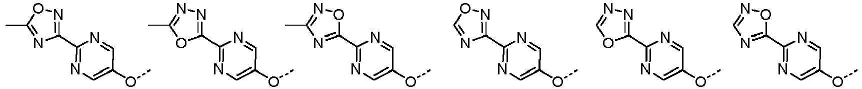

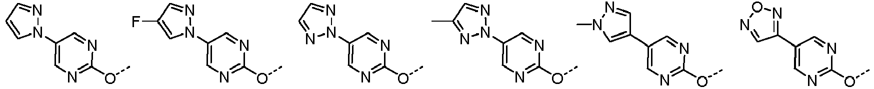

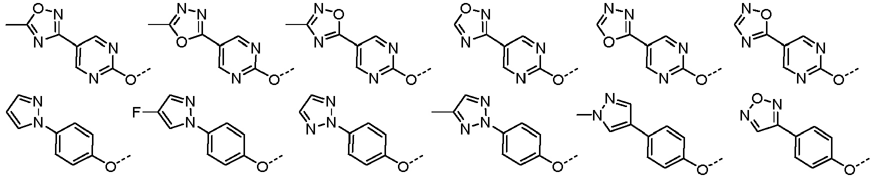

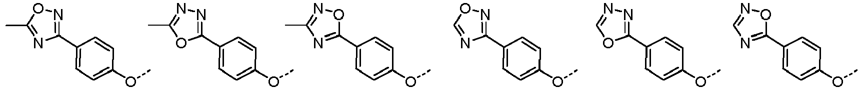

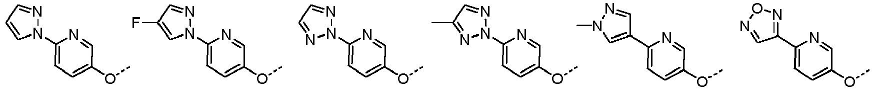

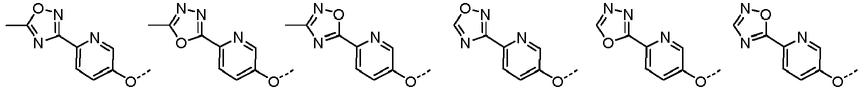

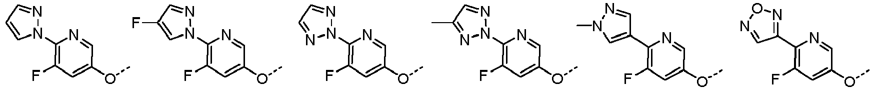

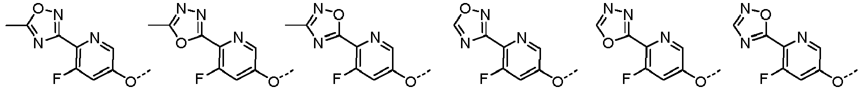

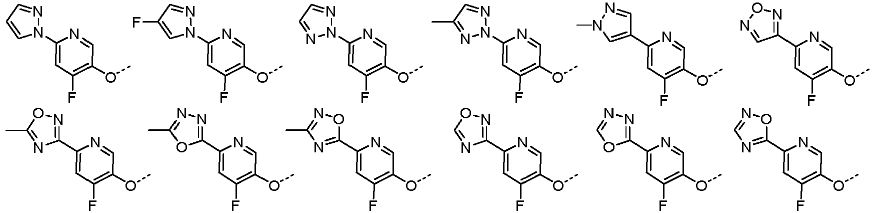

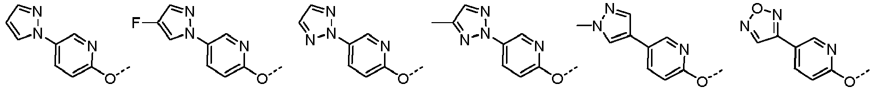

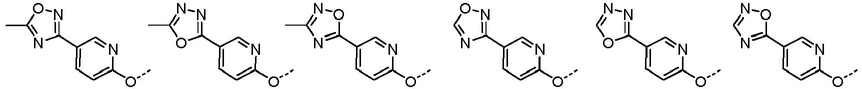

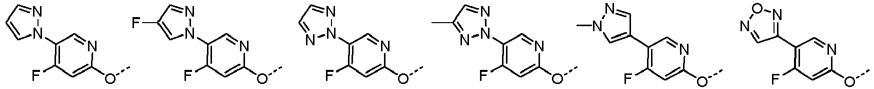

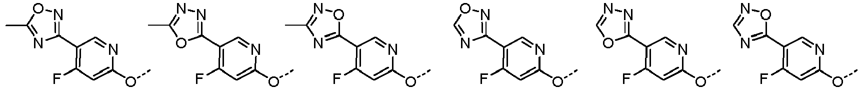

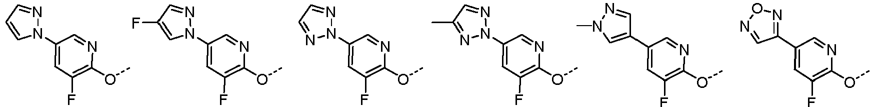

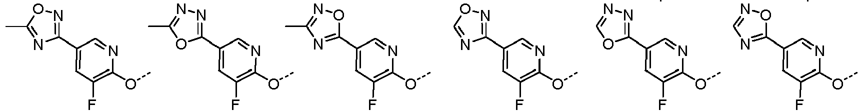

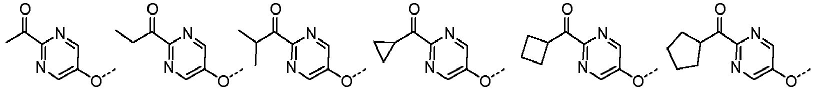

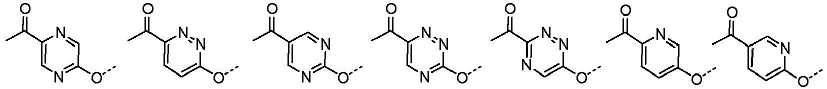

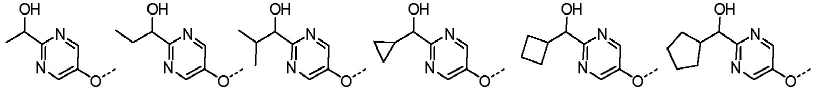

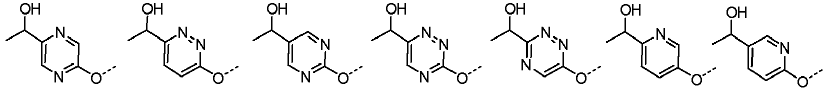

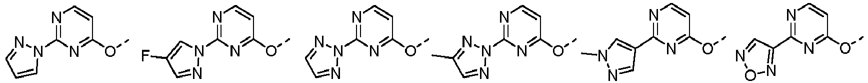

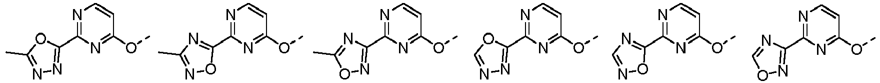

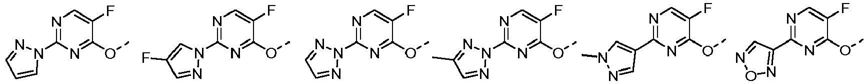

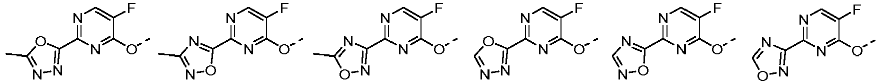

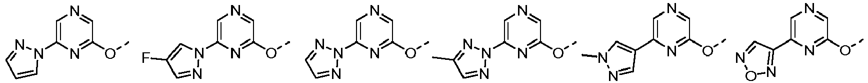

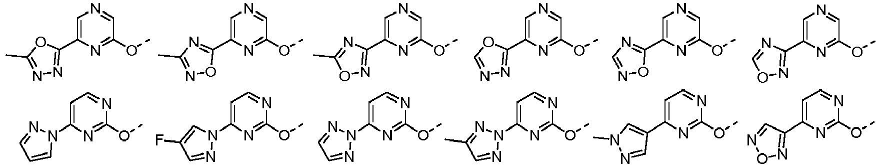

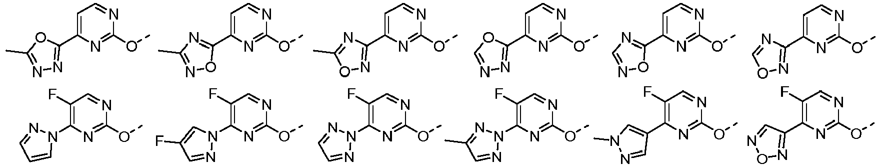

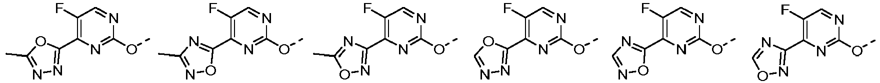

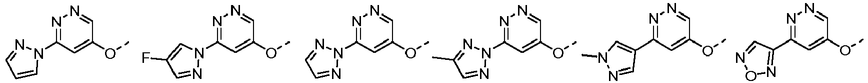

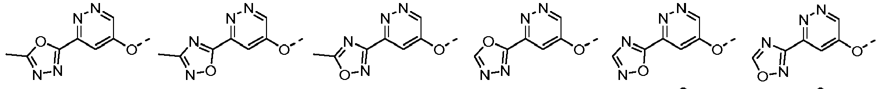

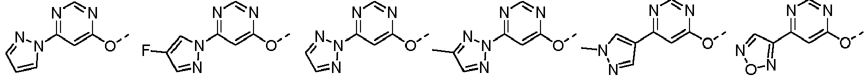

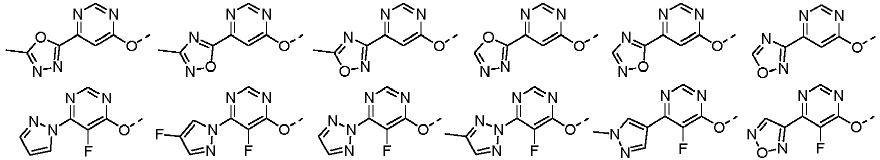

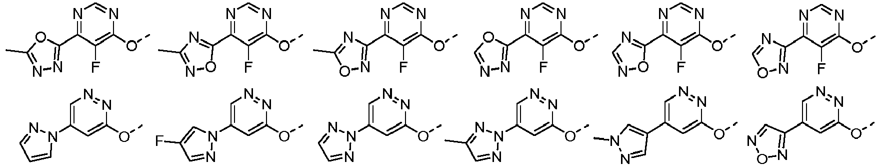

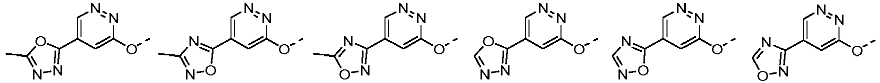

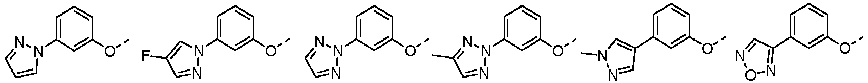

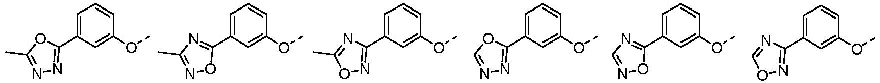

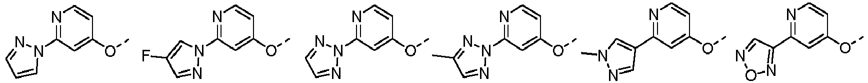

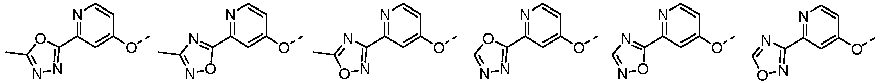

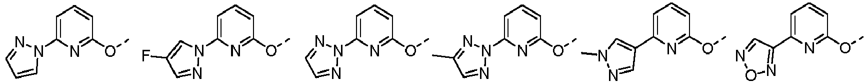

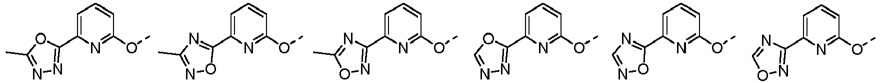

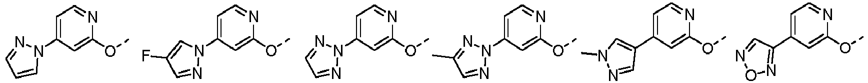

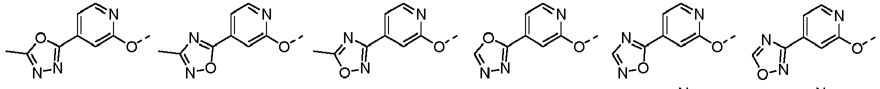

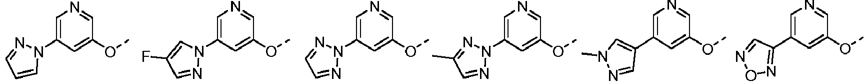

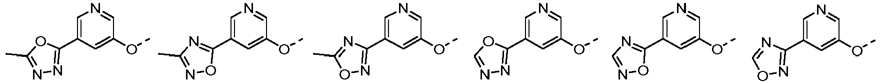

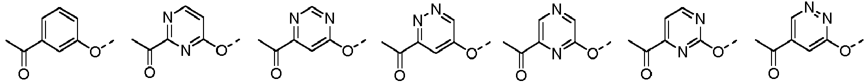

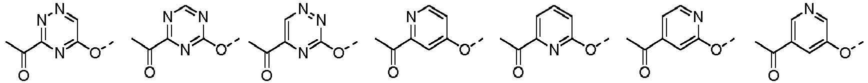

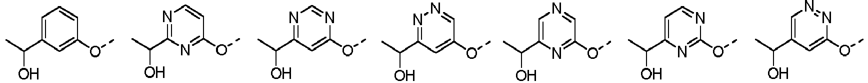

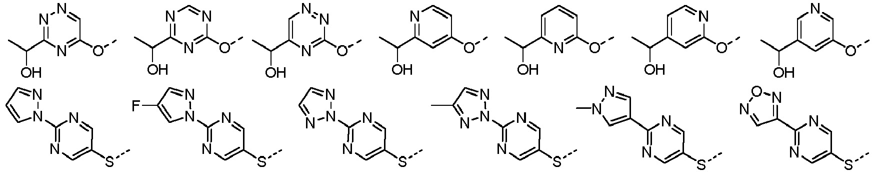

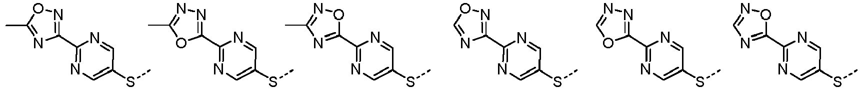

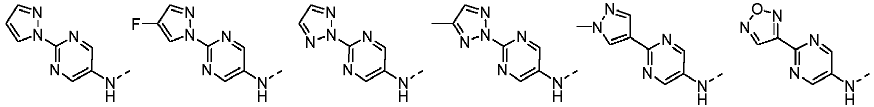

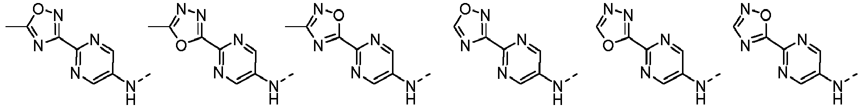

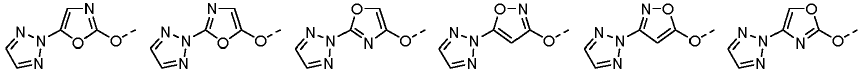

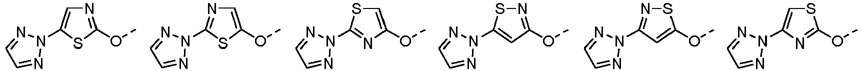

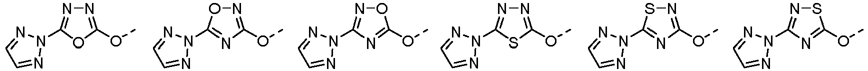

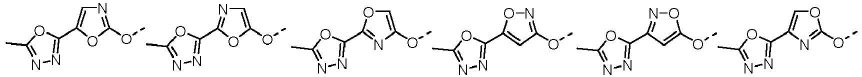

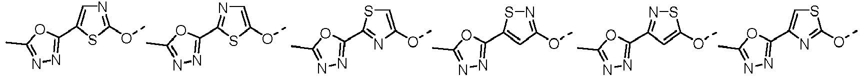

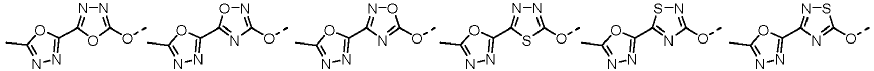

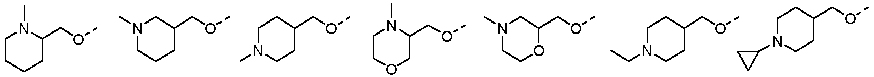

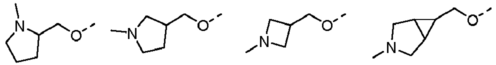

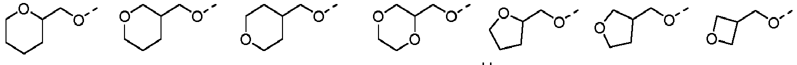

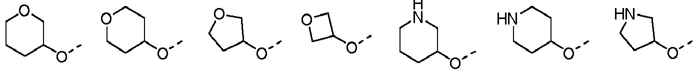

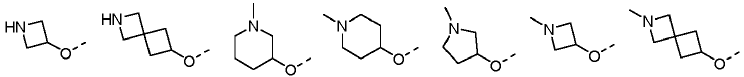

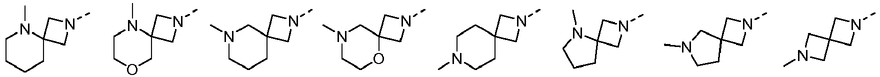

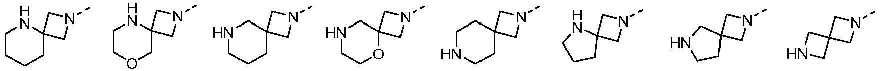

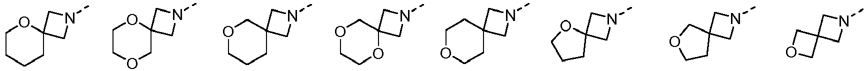

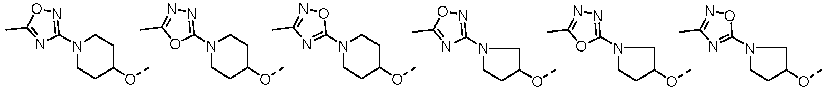

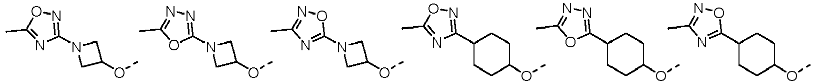

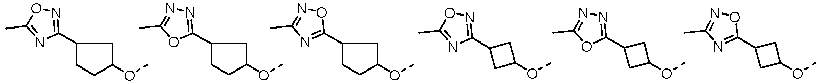

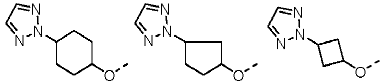

17. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of 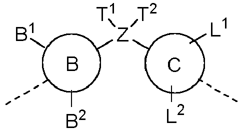 in the TB moiety is selected from the group consisting of:

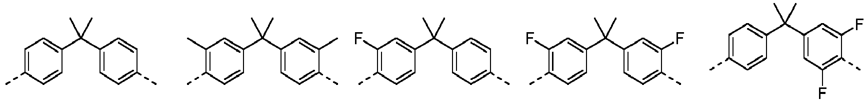

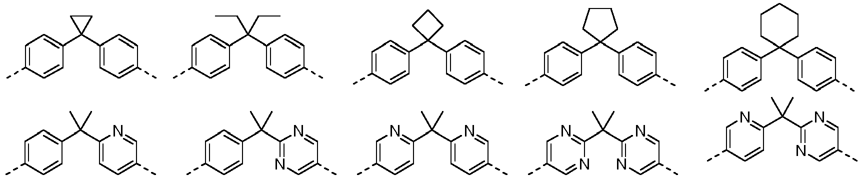

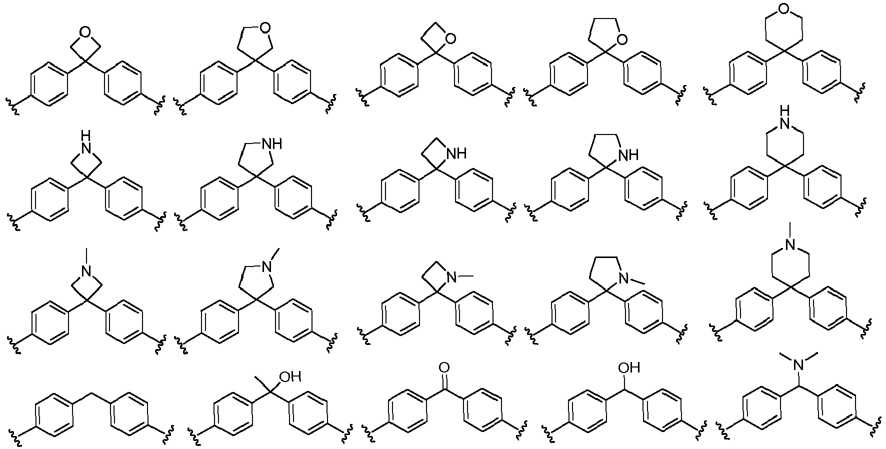

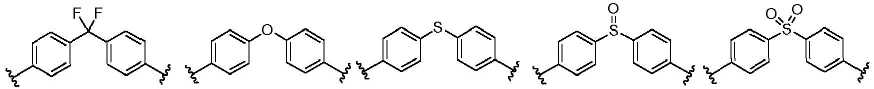

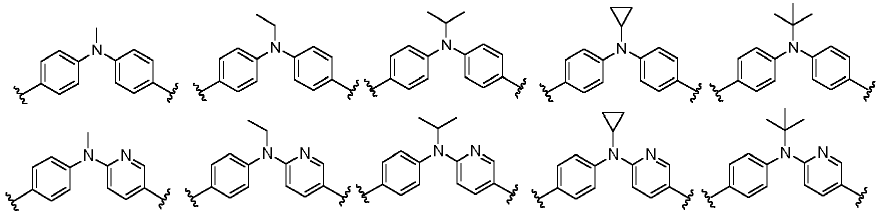

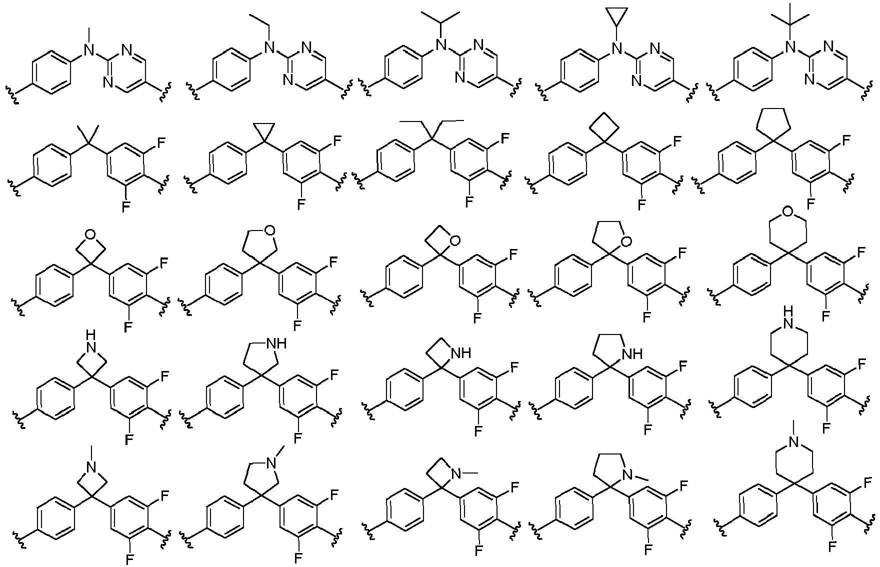

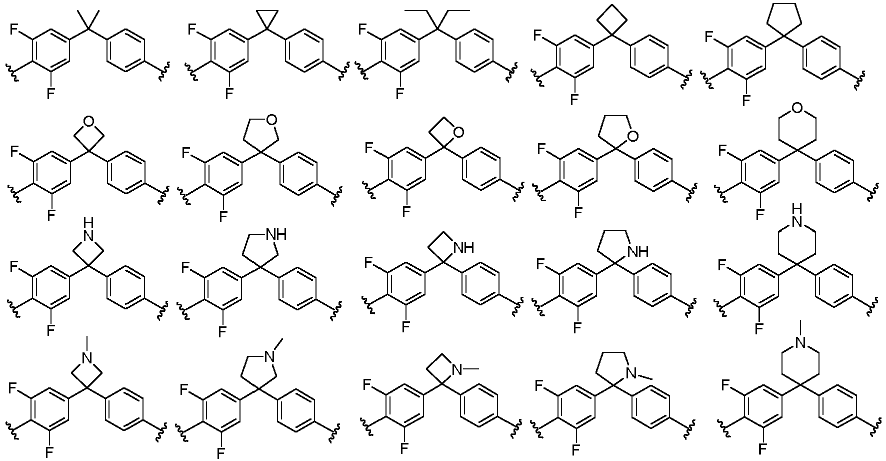

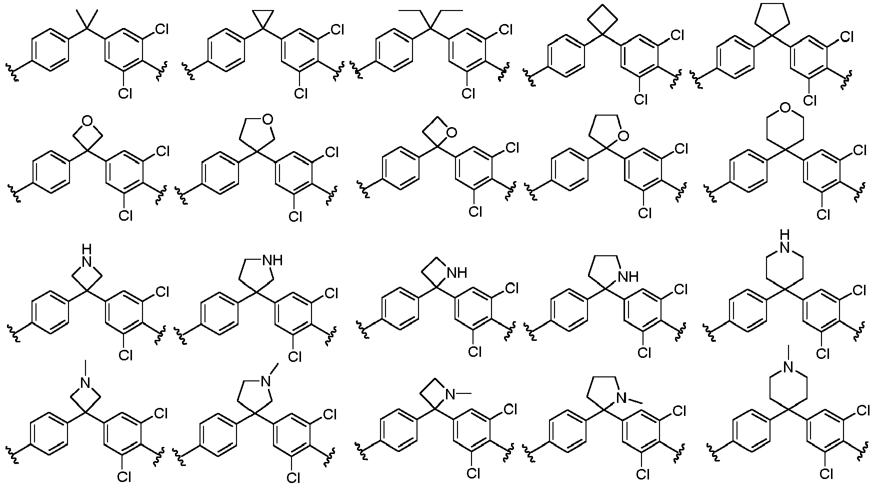

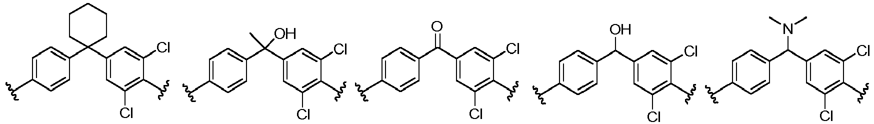

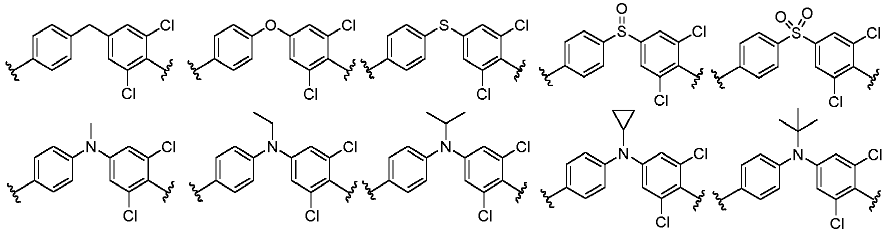

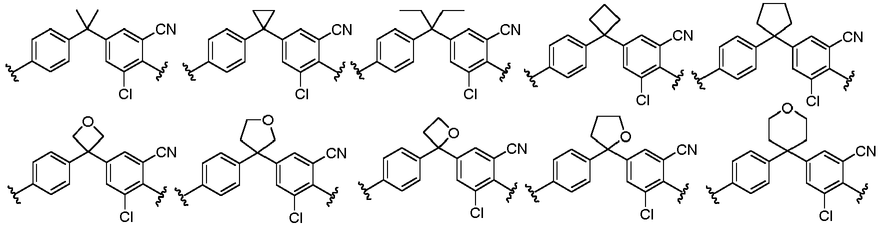

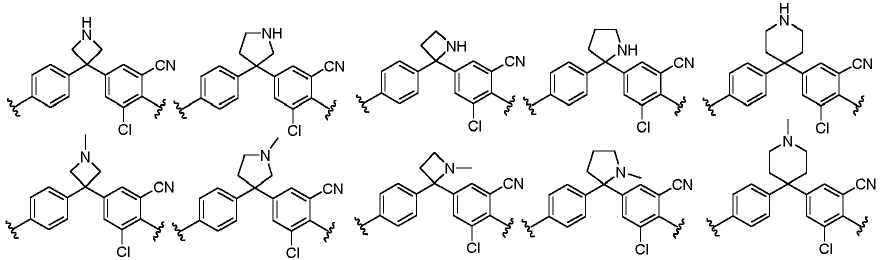

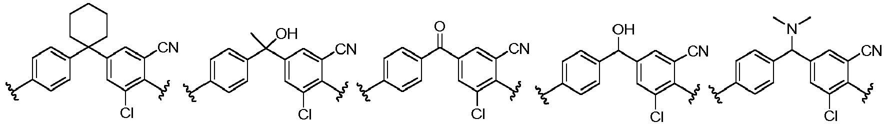

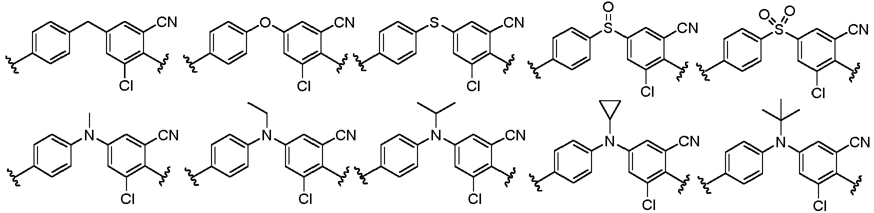

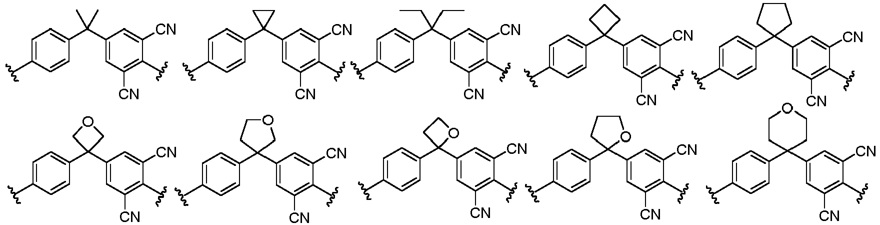

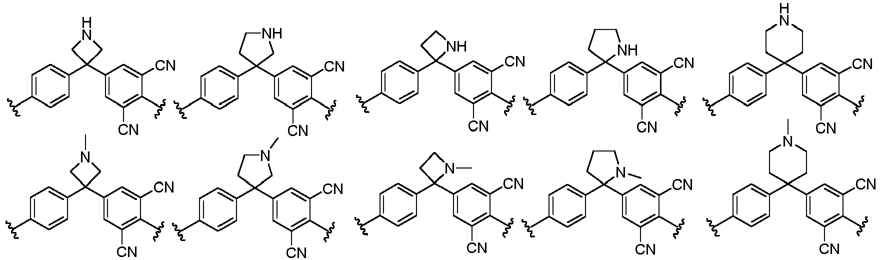

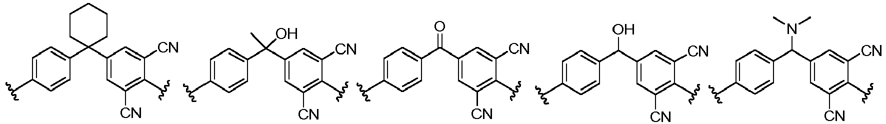

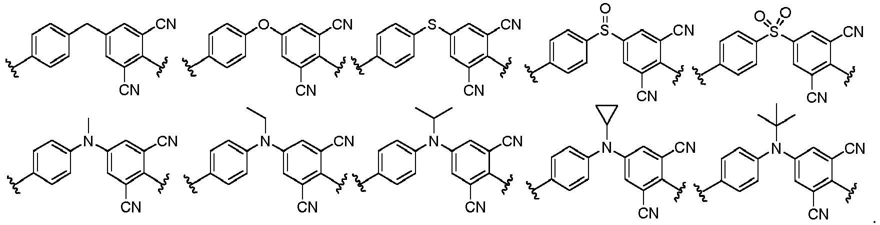

18. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of 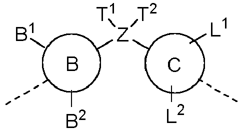 in the TB moiety is as represented by formula I-C: 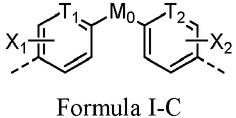 wherein, M₀ is -CRₐR_{b}- or -SO₂-; T₁ and T₂ are each independently selected from the group consisting of -CH- and -N-; X₁ and X₂ are each independently selected from the group consisting of H and halogen.

19. The compound according to claim 18, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of 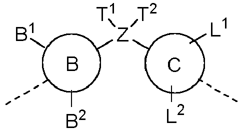 in the TB moiety is selected from the group consisting of:

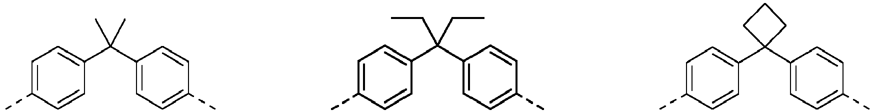

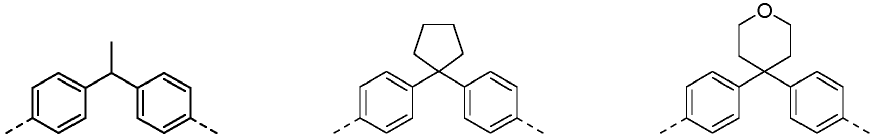

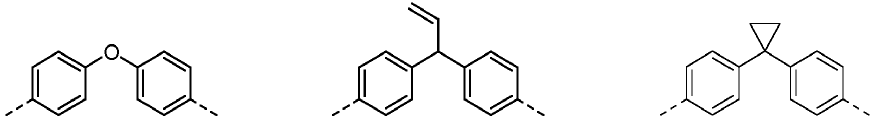

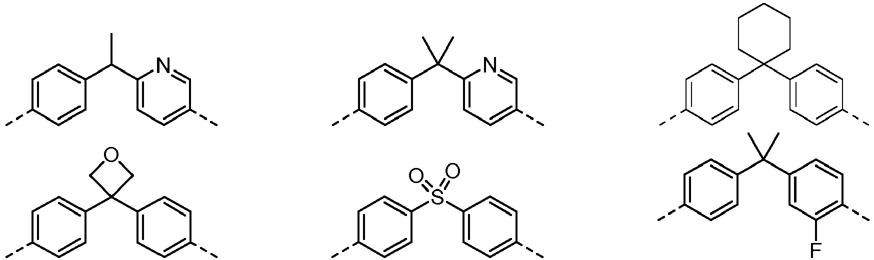

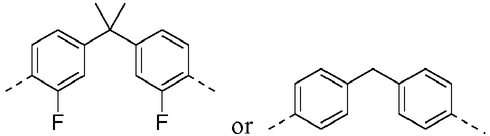

20. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of TB moiety is selected from the group consisting of:

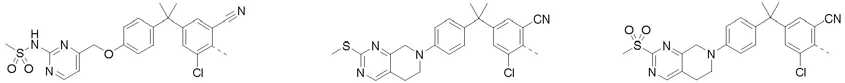

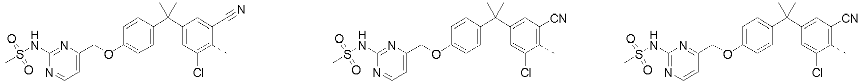

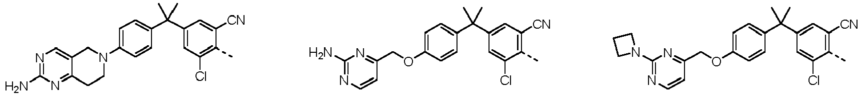

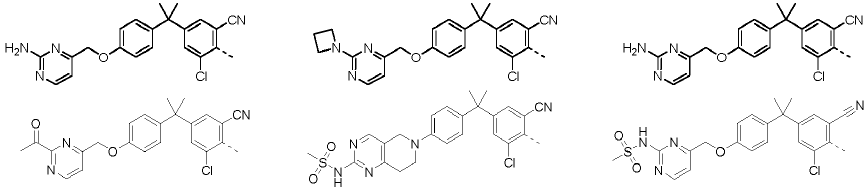

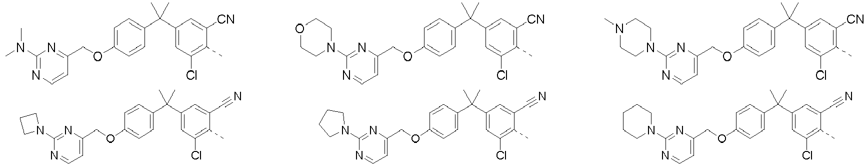

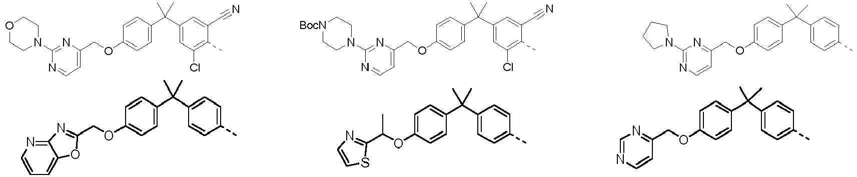

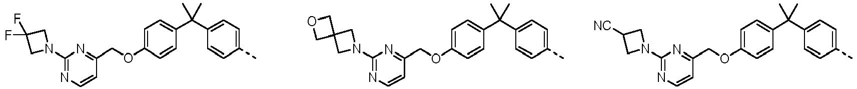

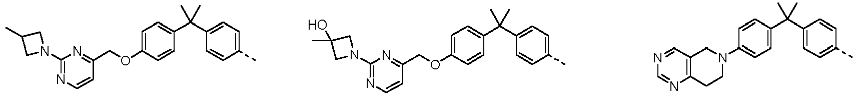

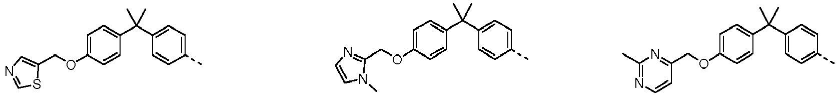

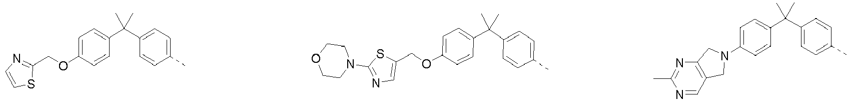

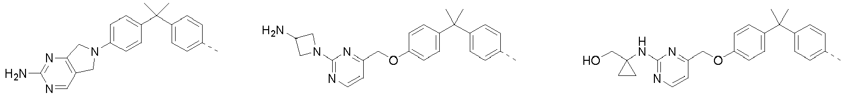

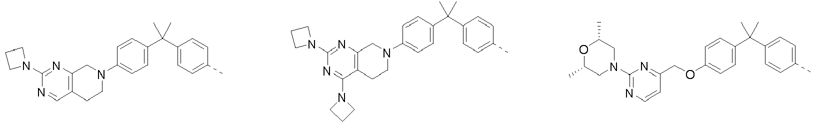

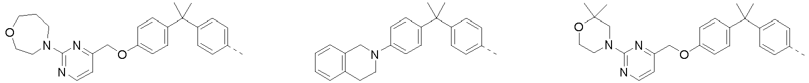

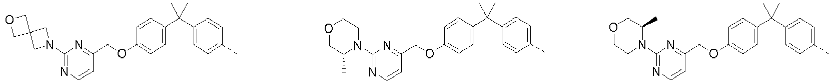

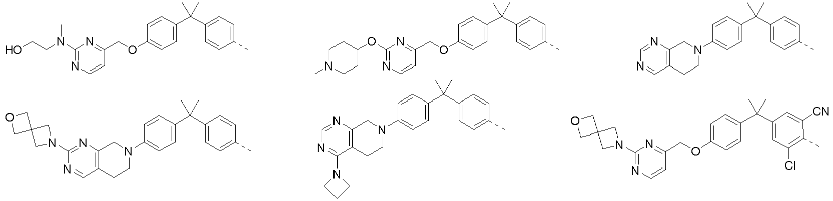

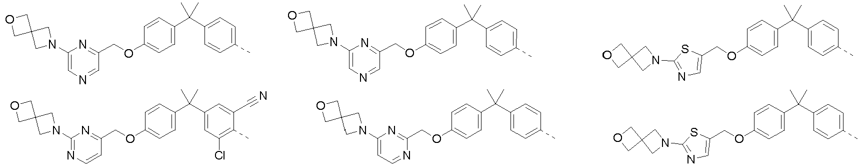

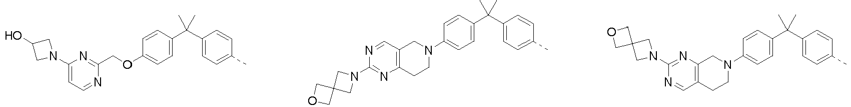

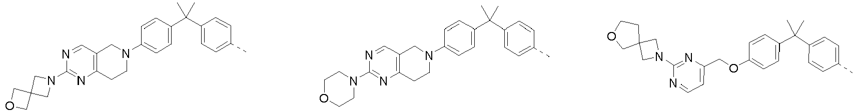

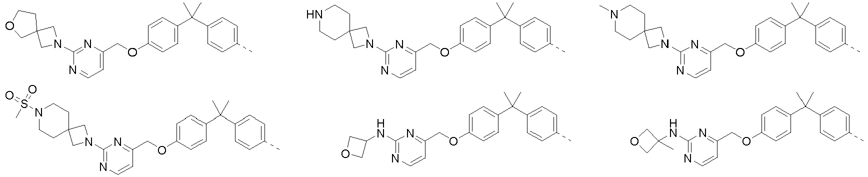

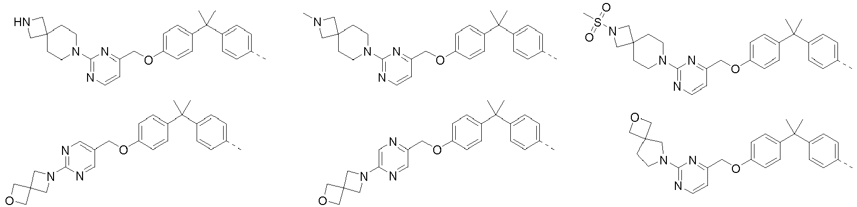

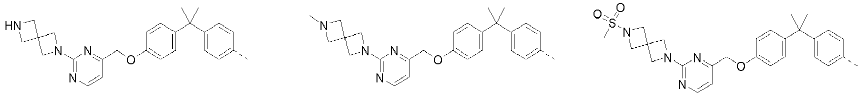

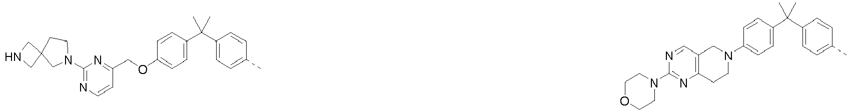

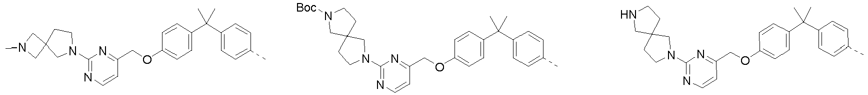

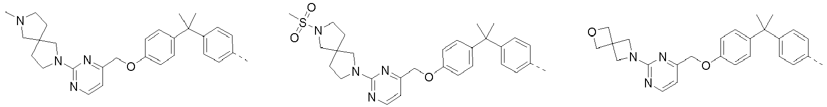

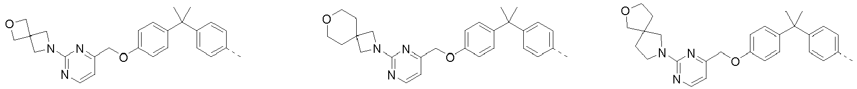

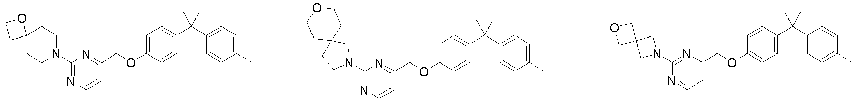

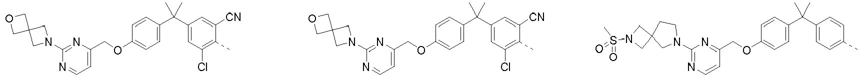

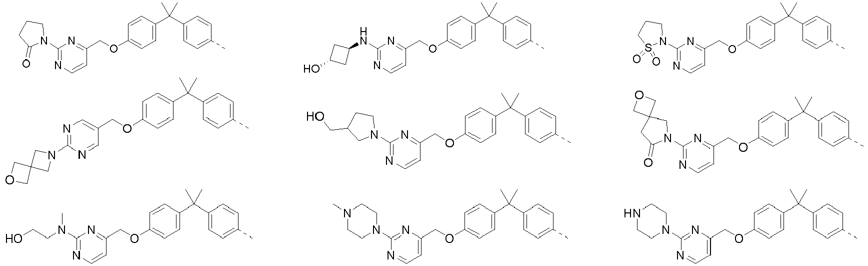

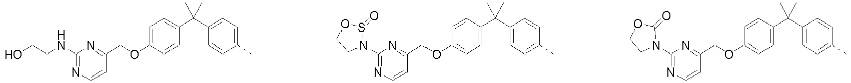

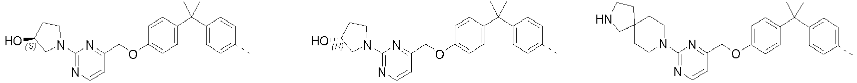

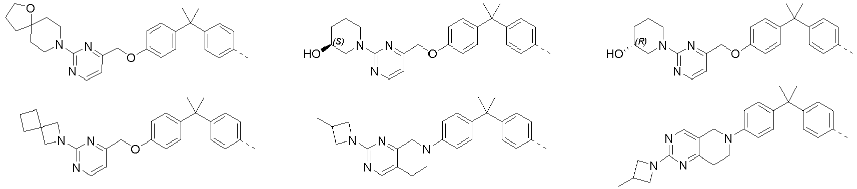

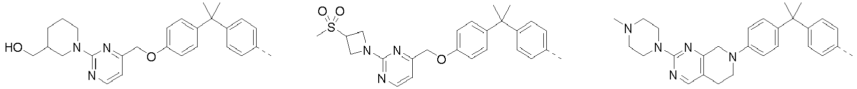

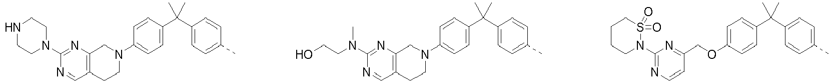

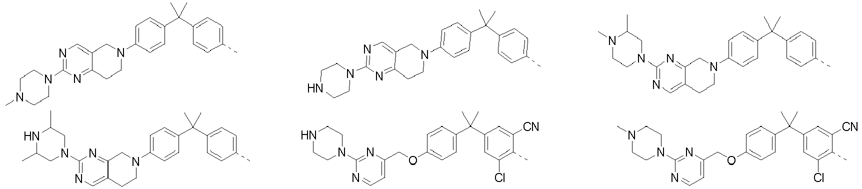

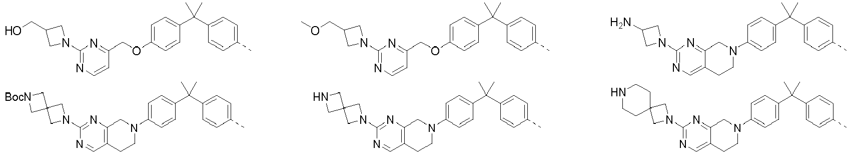

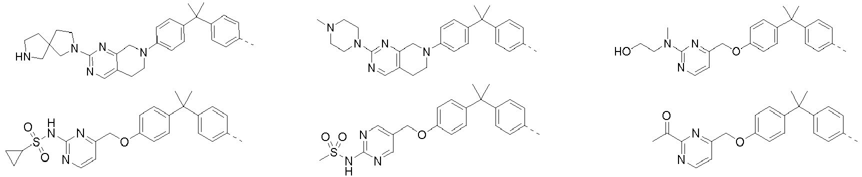

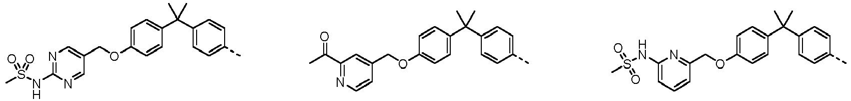

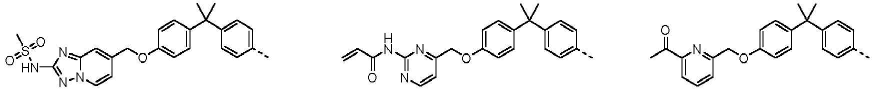

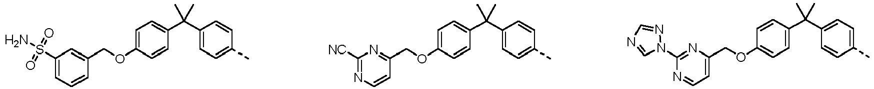

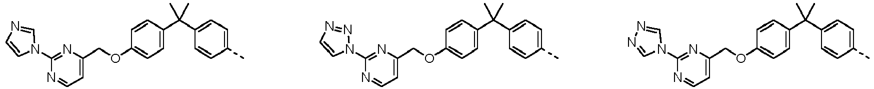

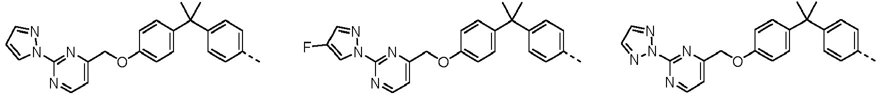

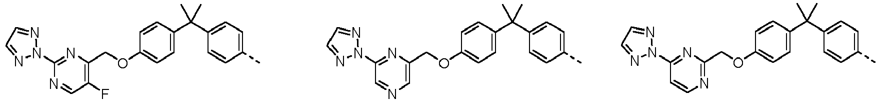

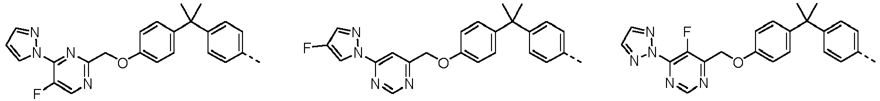

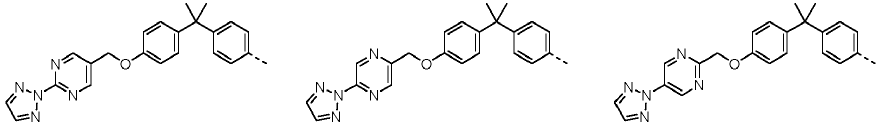

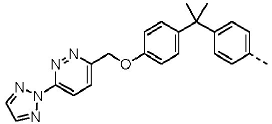

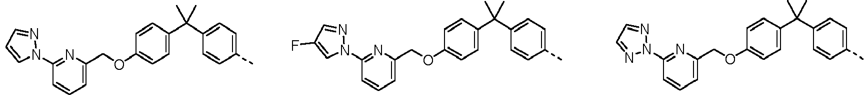

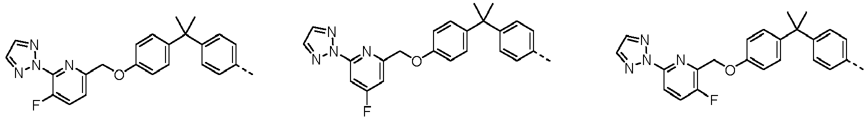

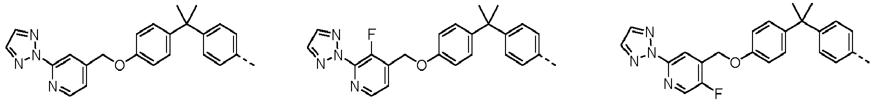

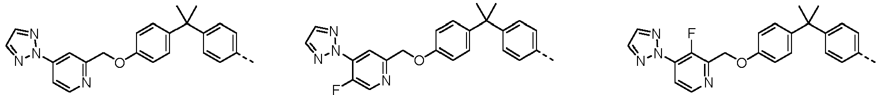

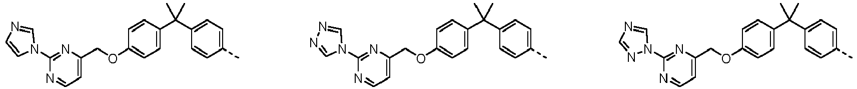

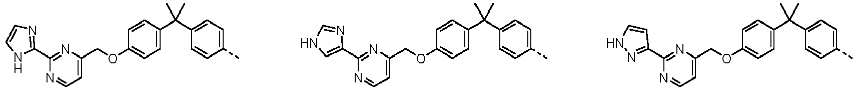

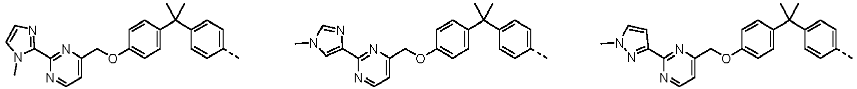

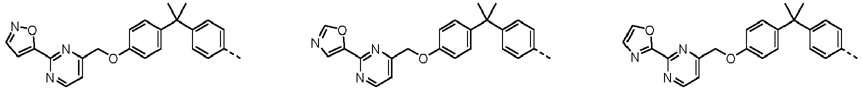

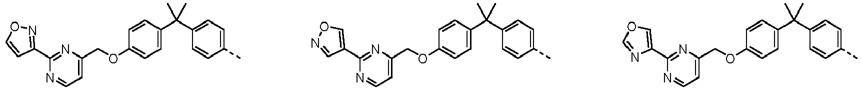

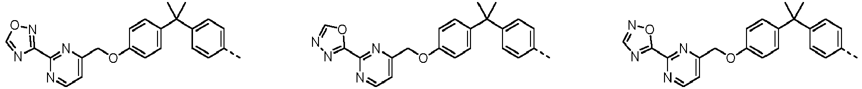

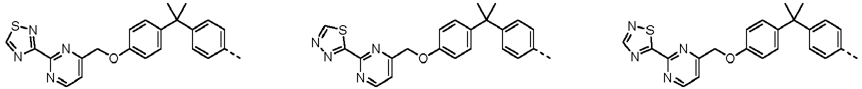

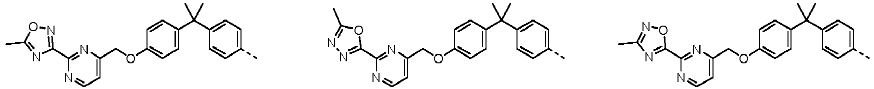

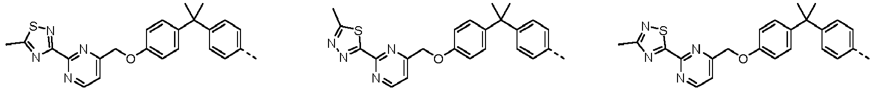

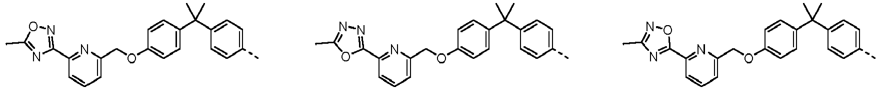

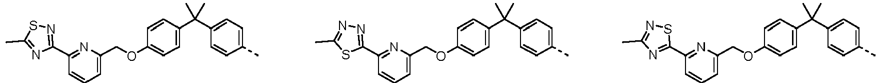

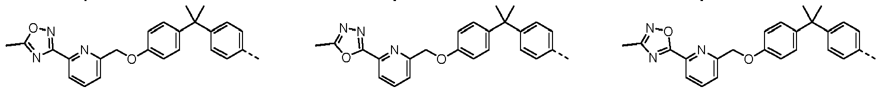

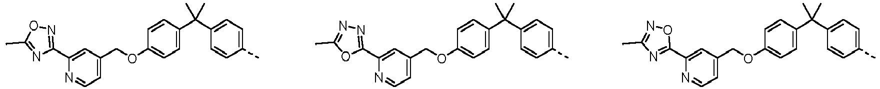

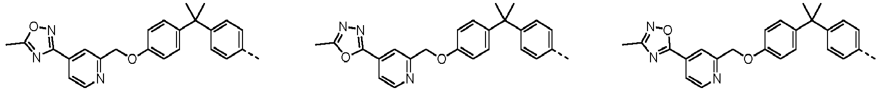

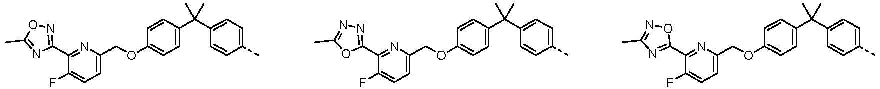

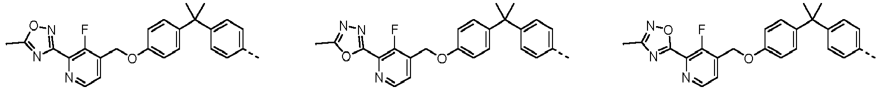

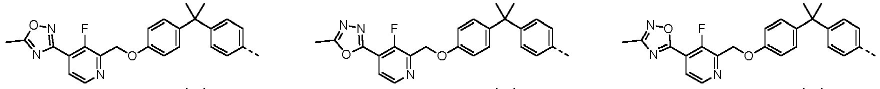

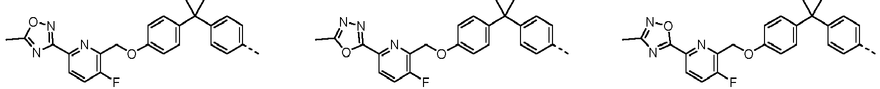

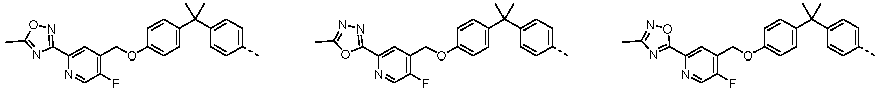

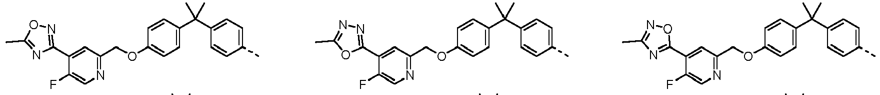

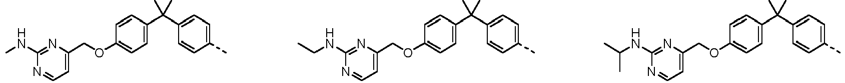

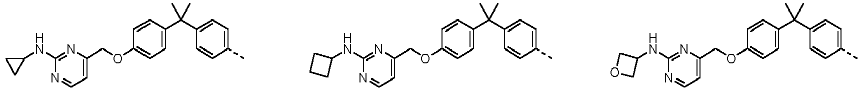

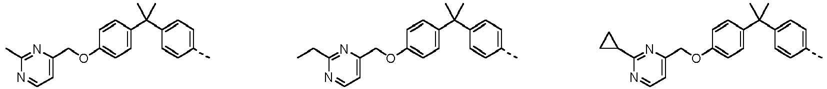

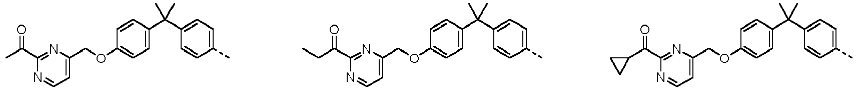

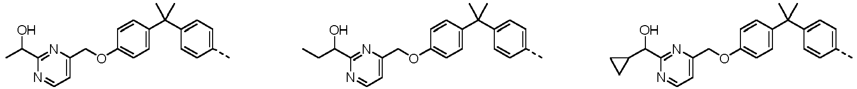

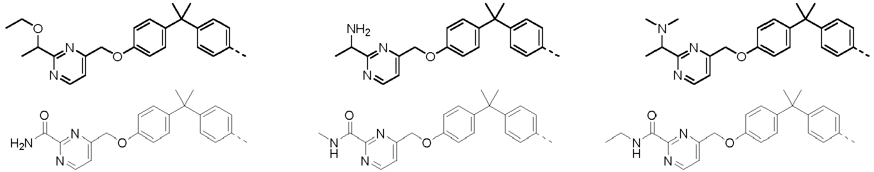

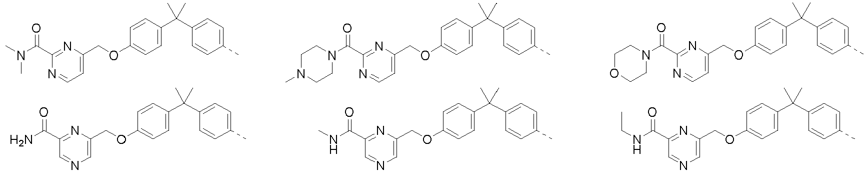

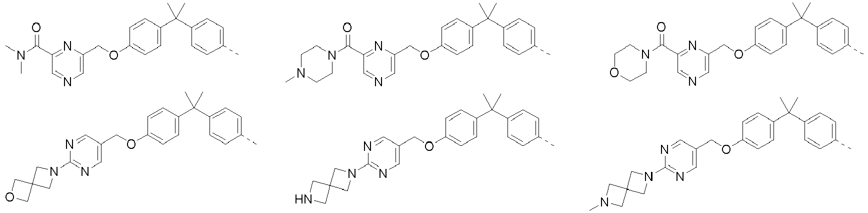

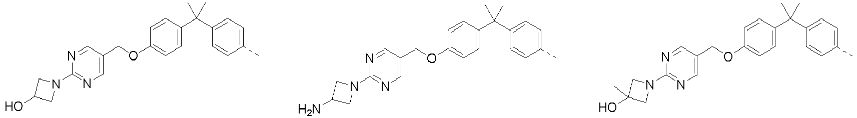

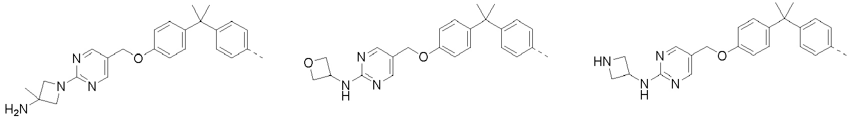

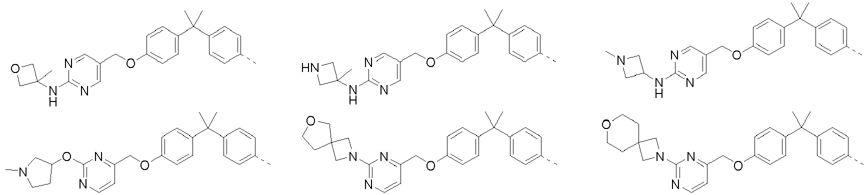

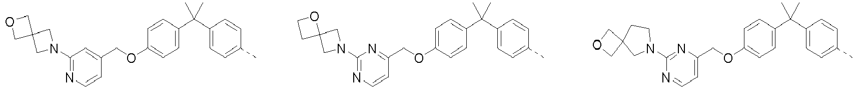

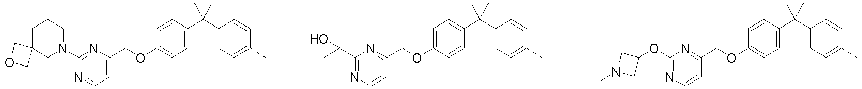

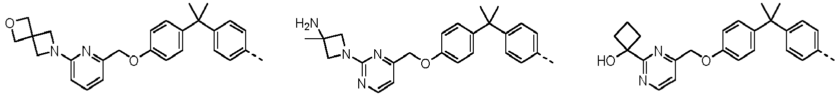

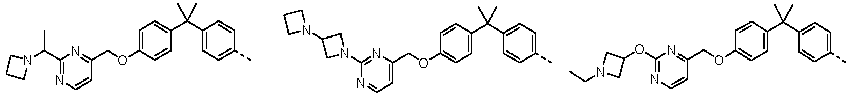

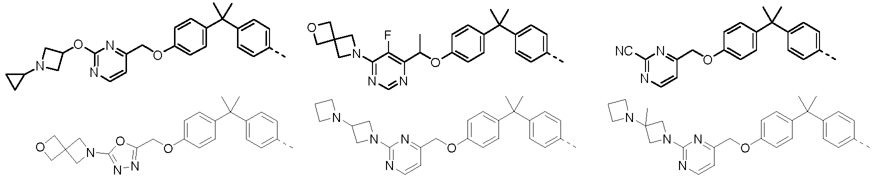

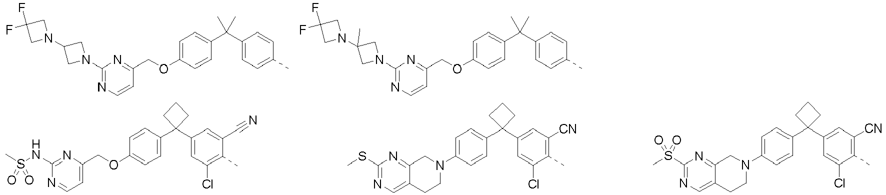

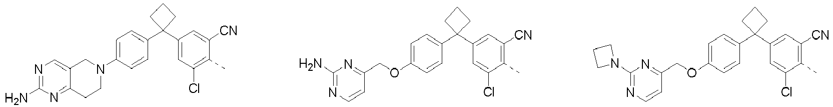

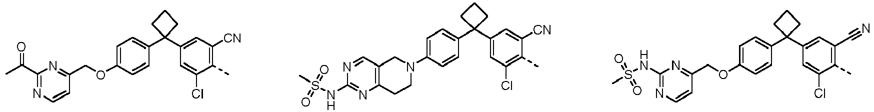

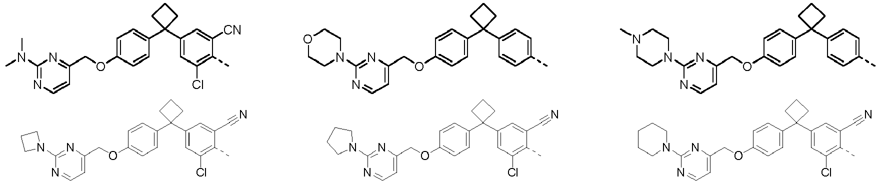

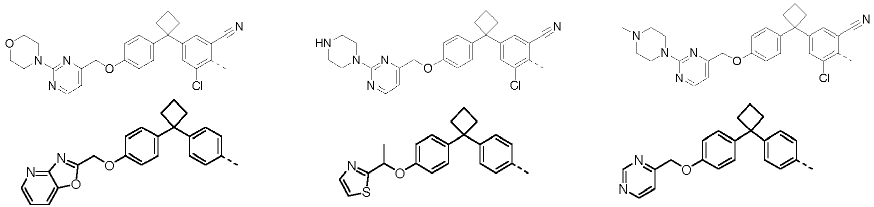

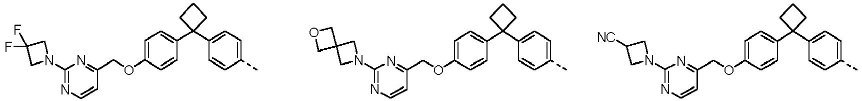

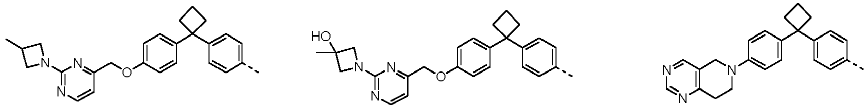

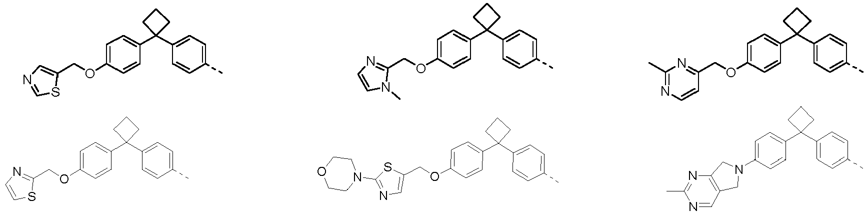

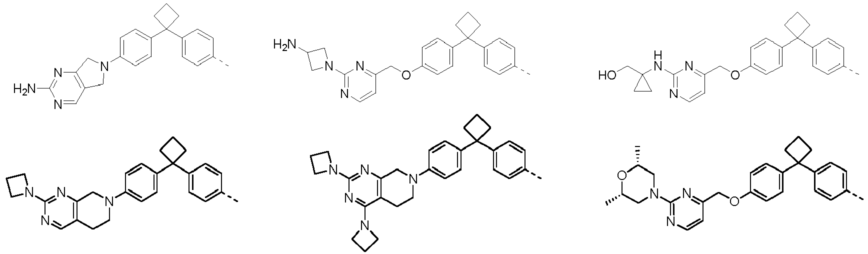

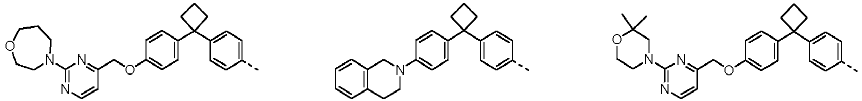

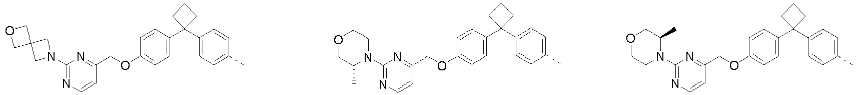

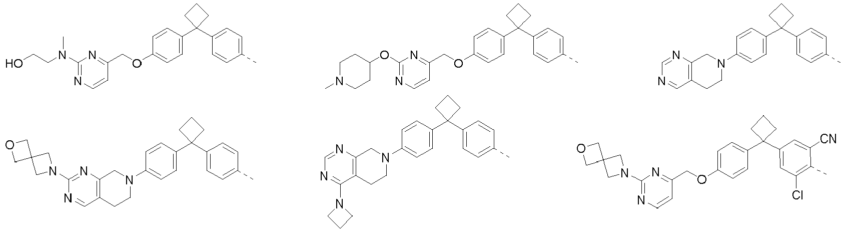

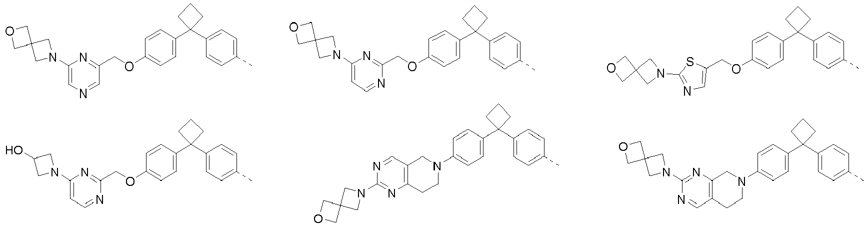

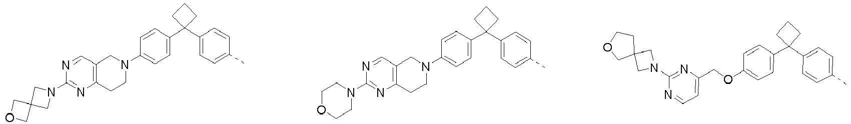

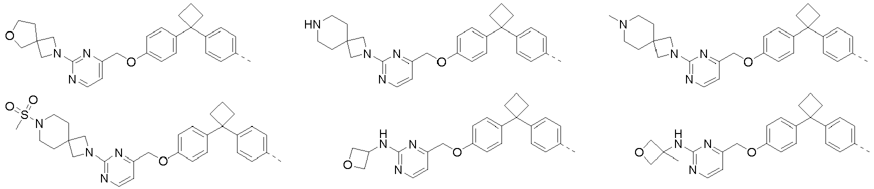

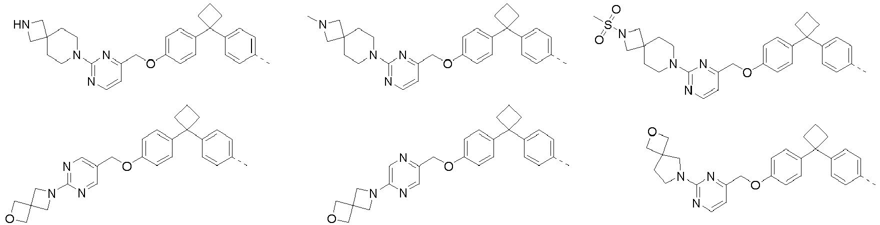

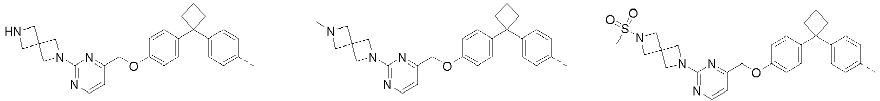

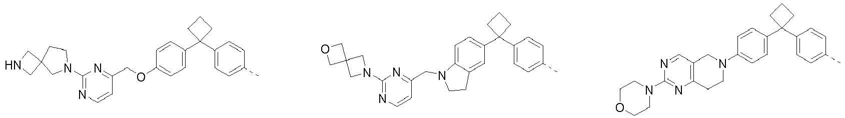

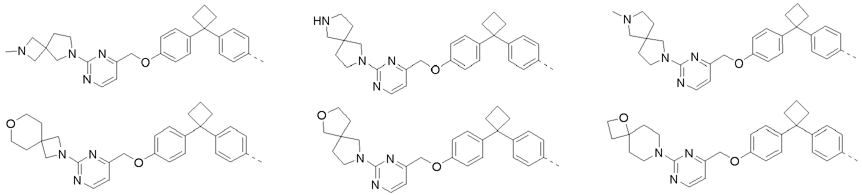

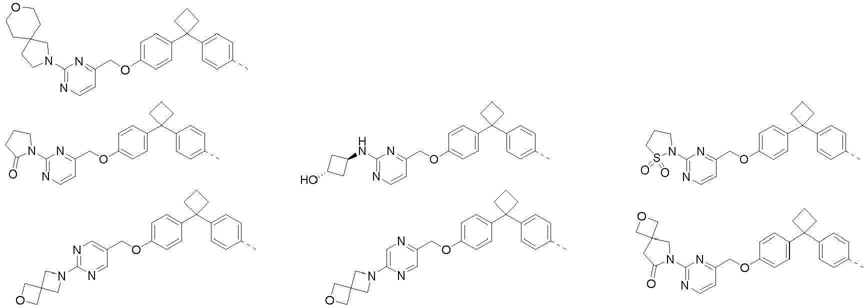

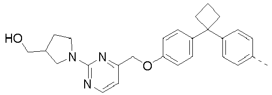

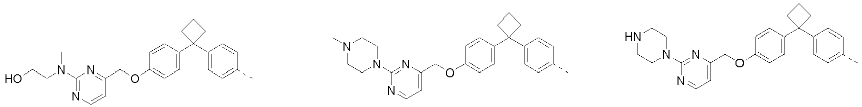

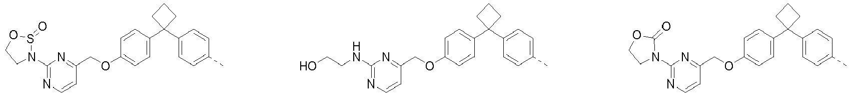

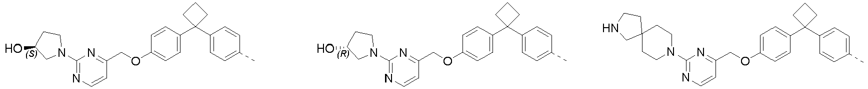

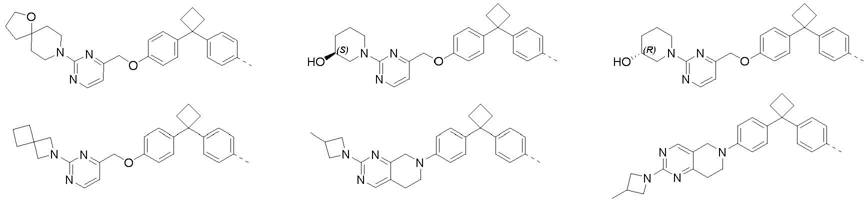

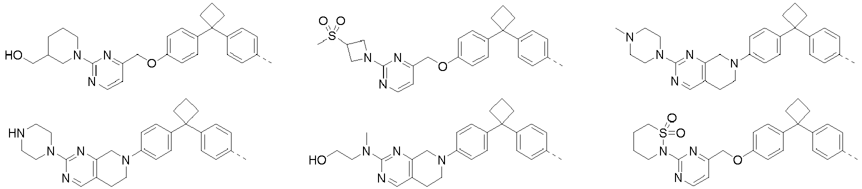

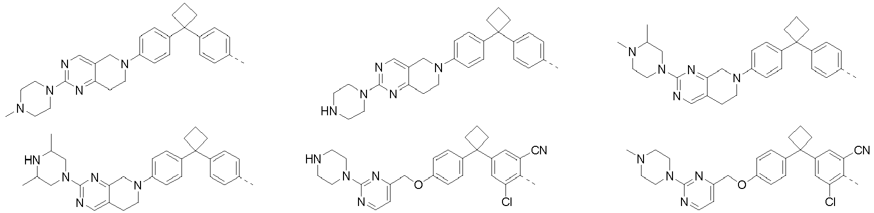

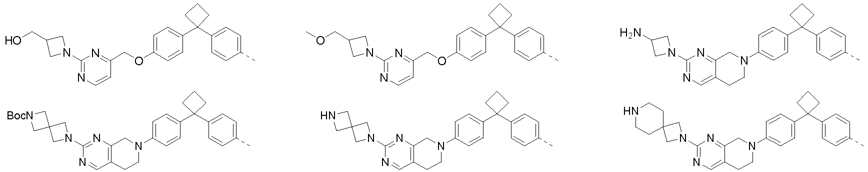

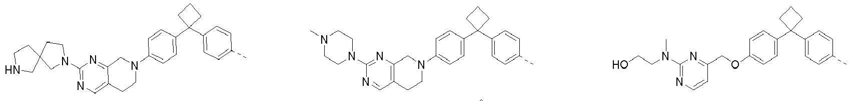

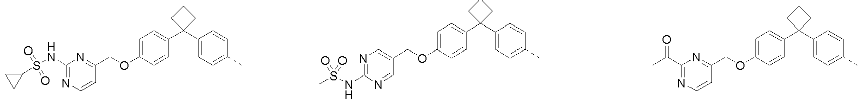

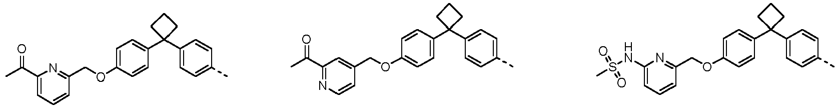

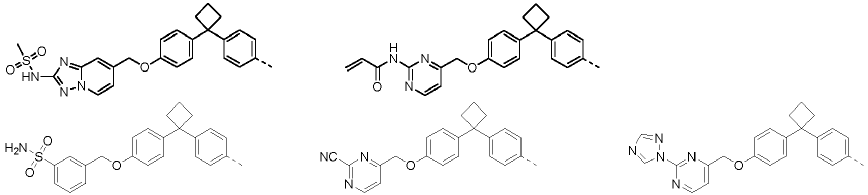

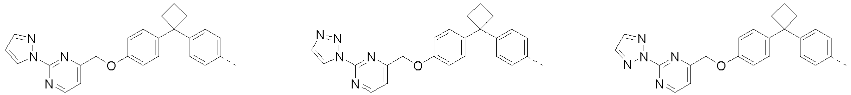

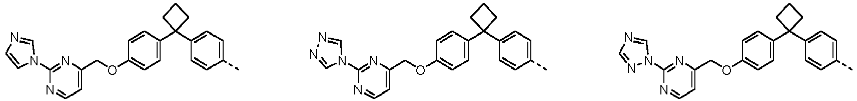

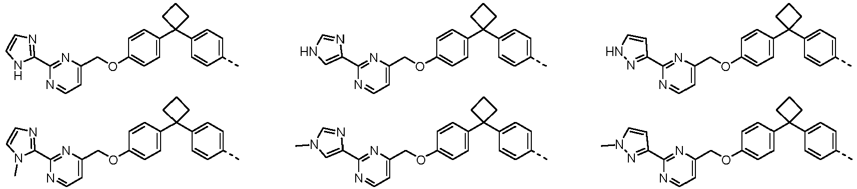

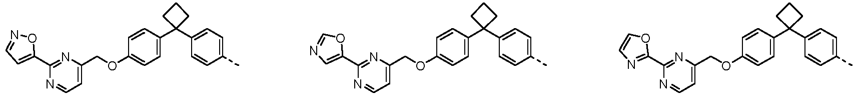

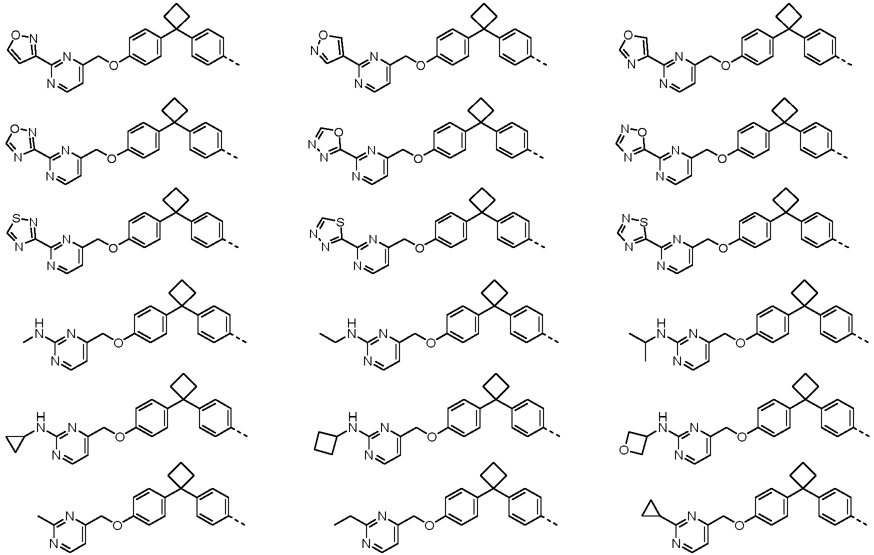

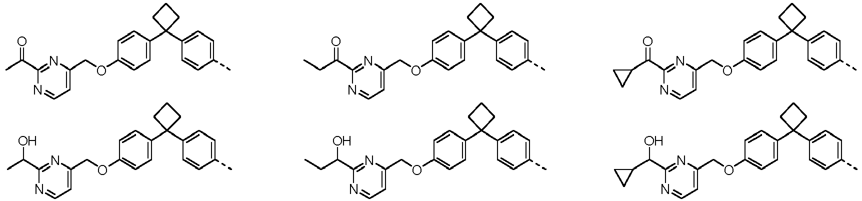

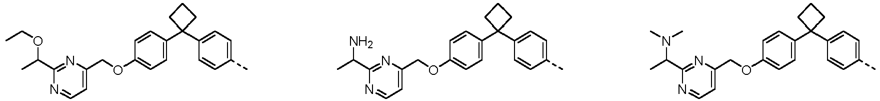

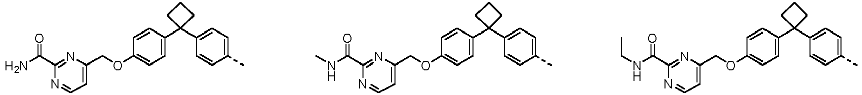

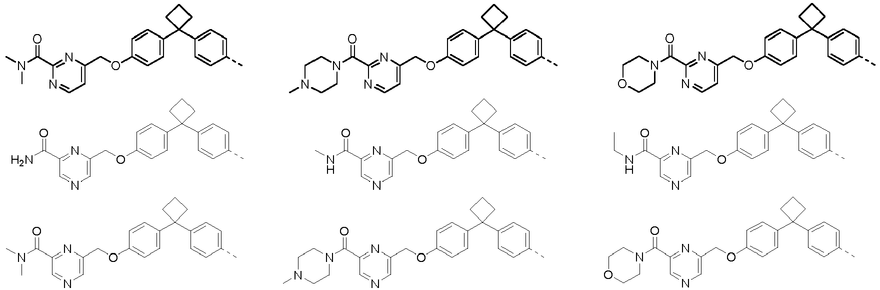

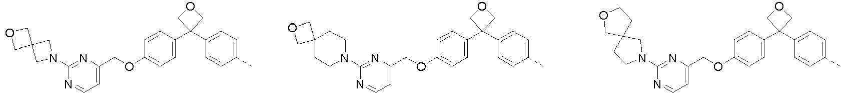

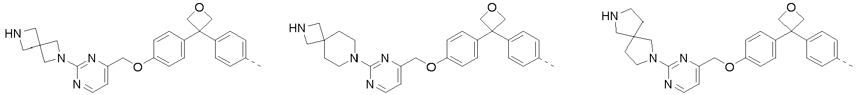

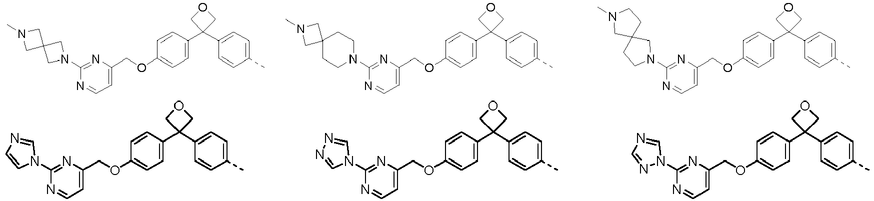

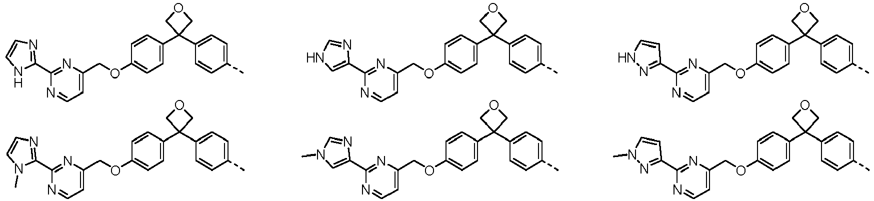

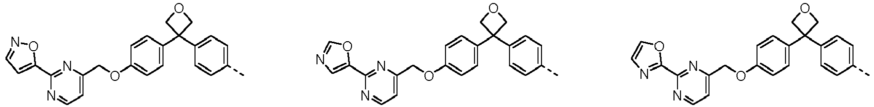

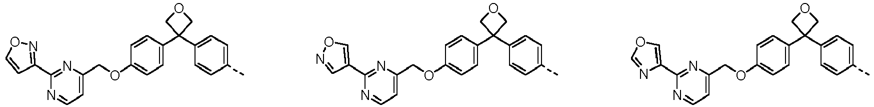

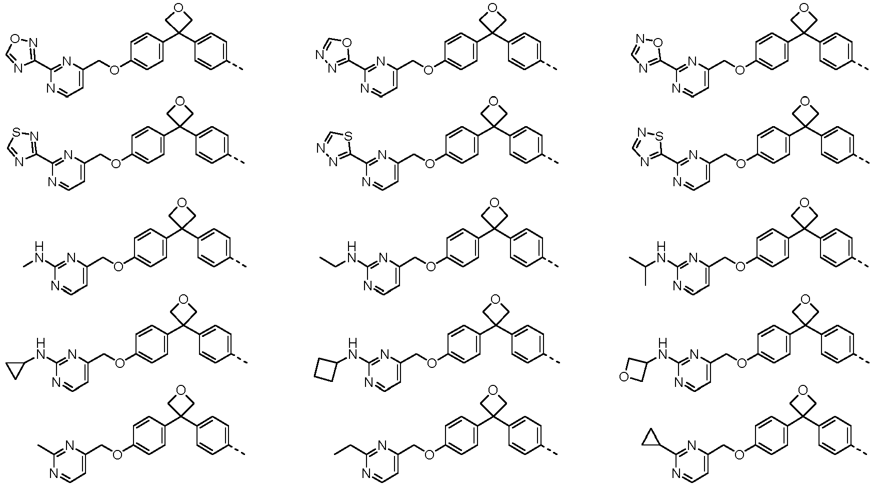

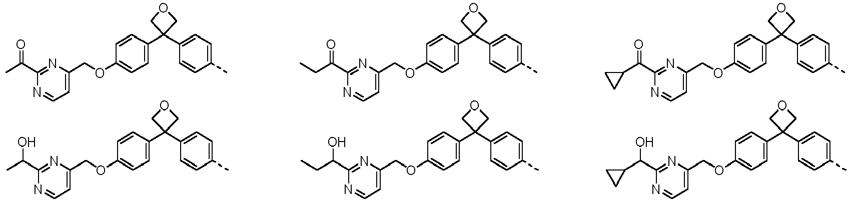

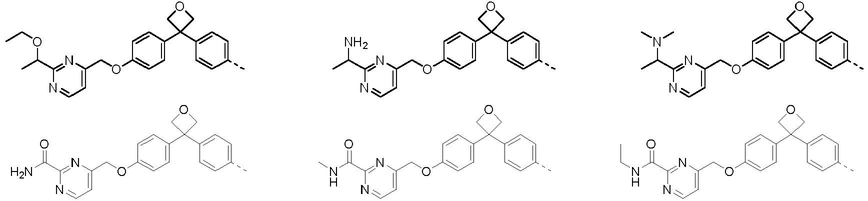

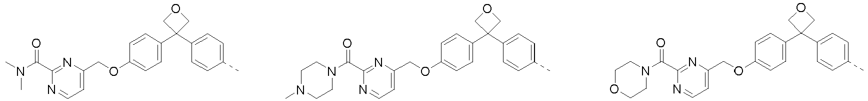

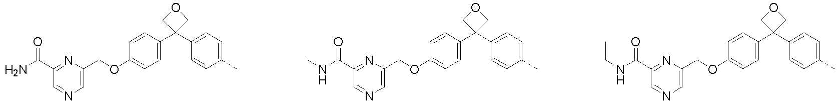

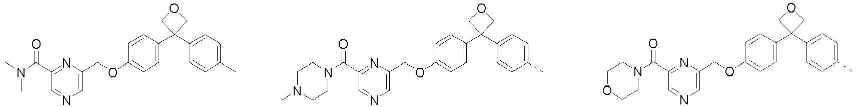

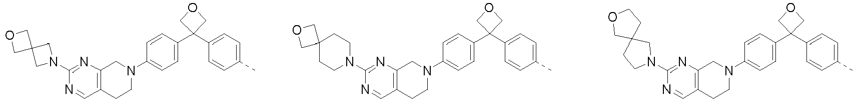

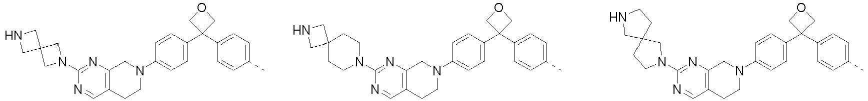

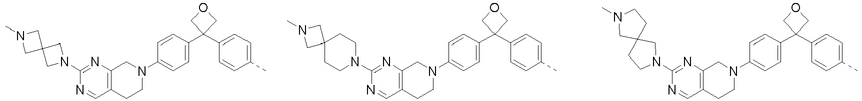

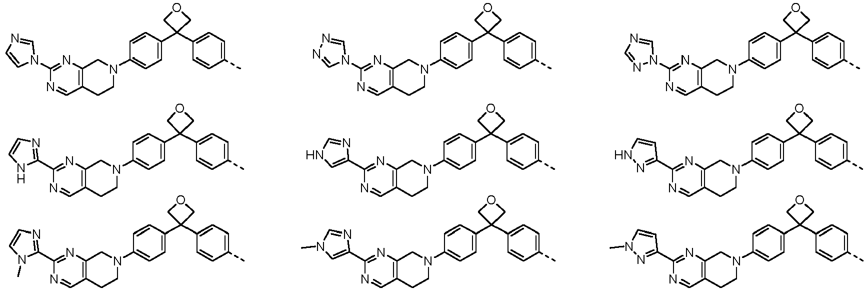

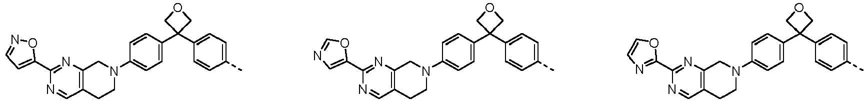

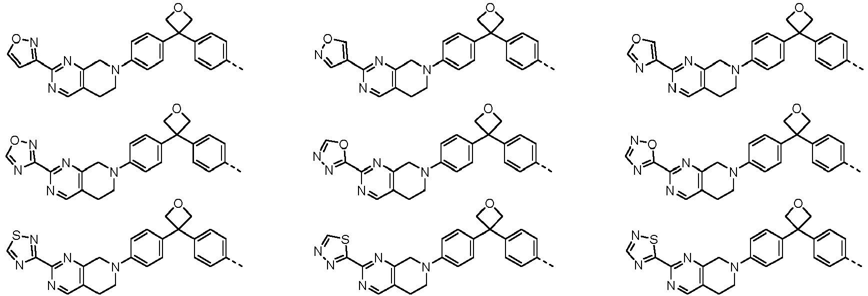

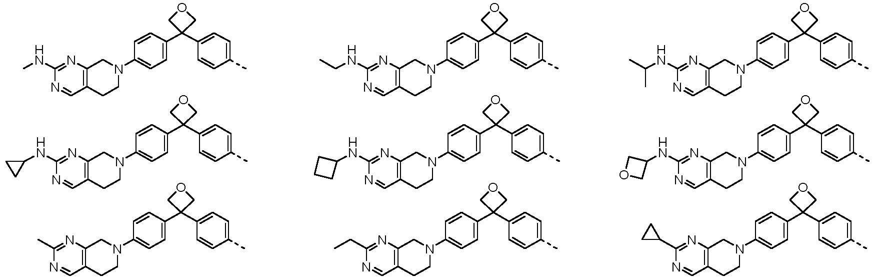

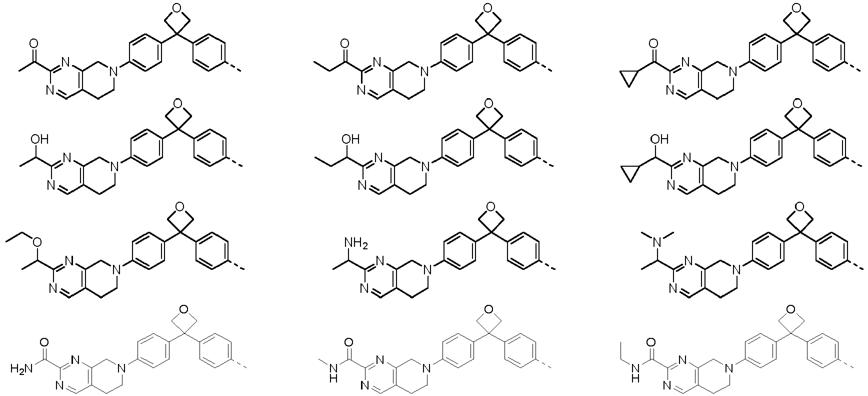

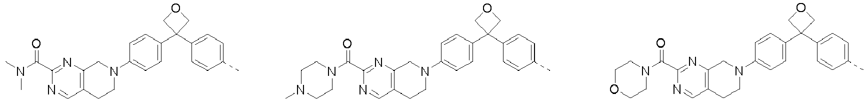

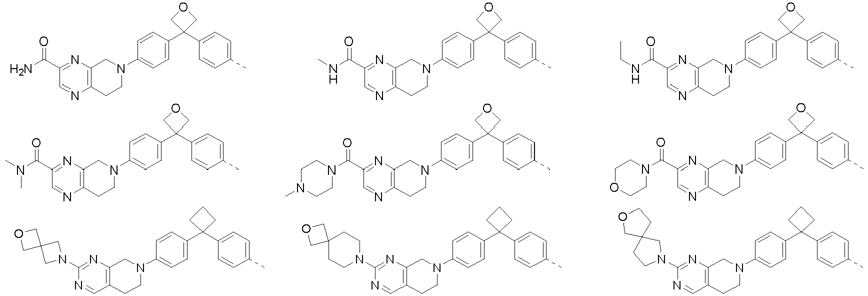

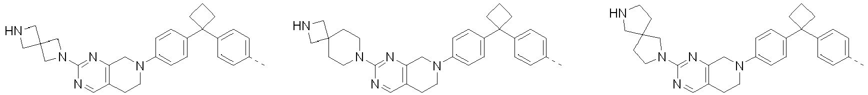

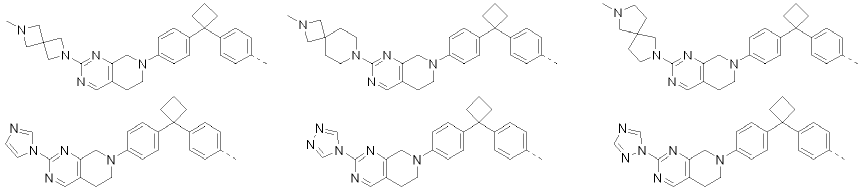

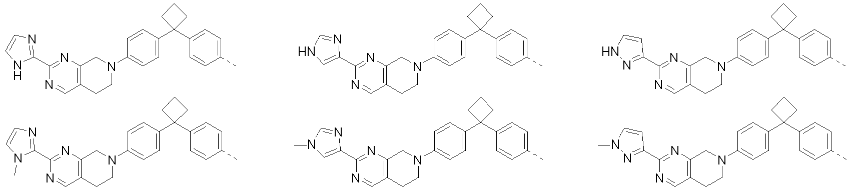

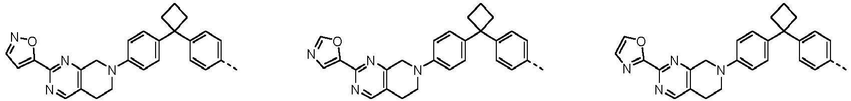

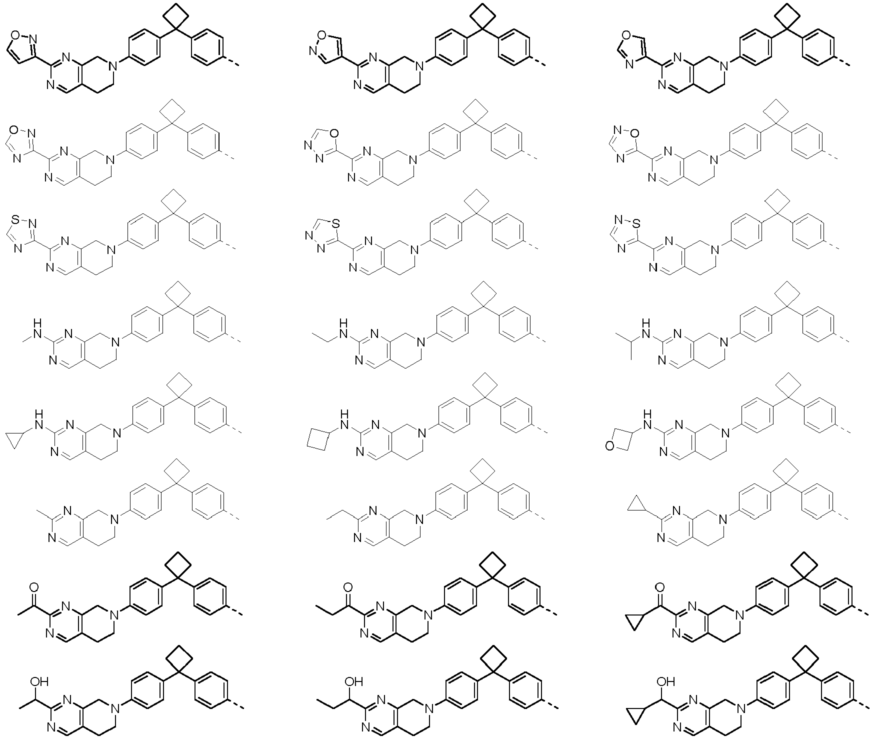

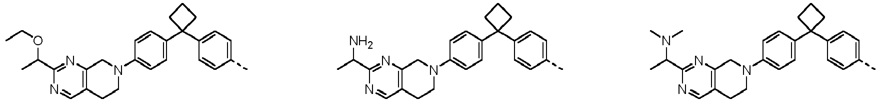

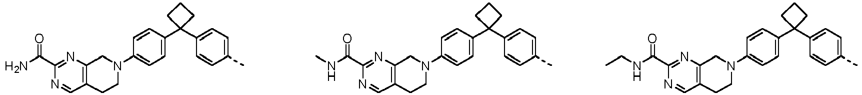

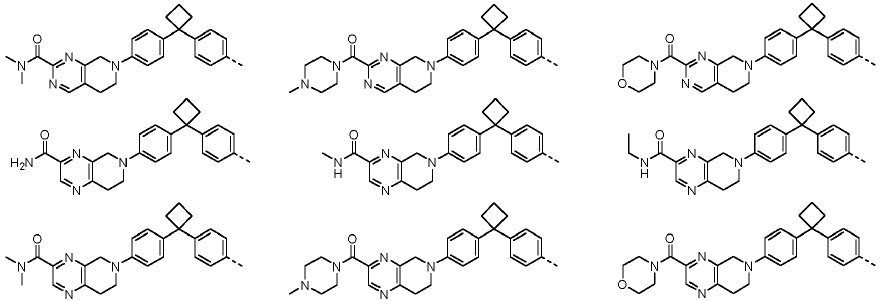

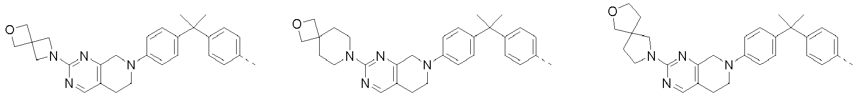

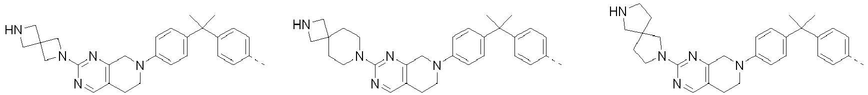

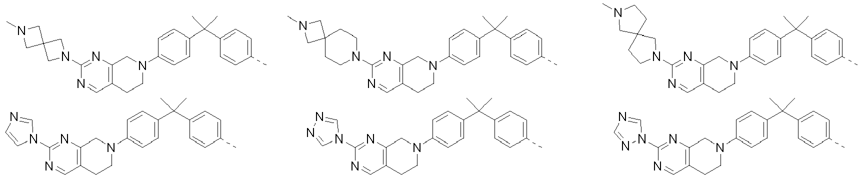

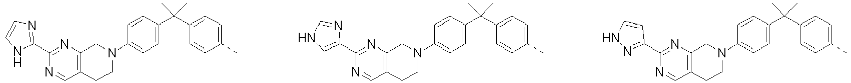

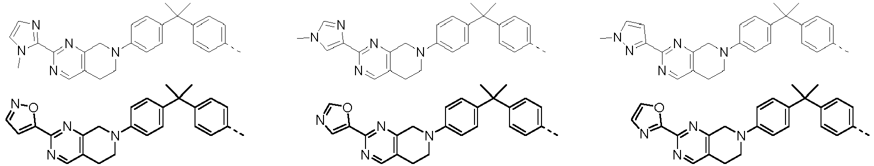

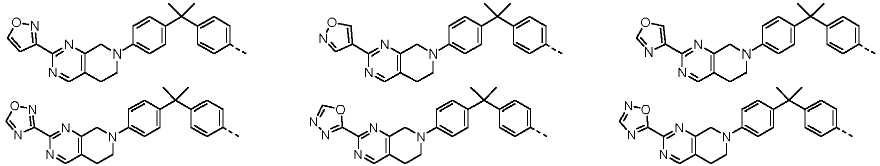

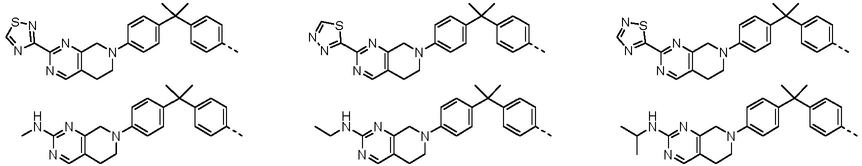

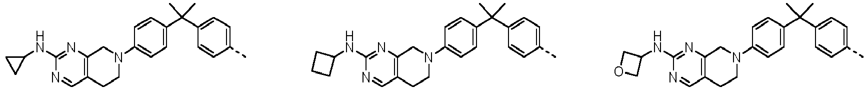

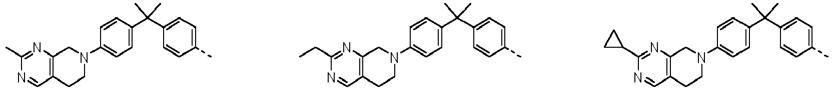

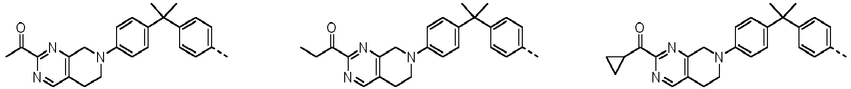

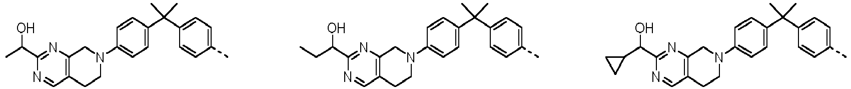

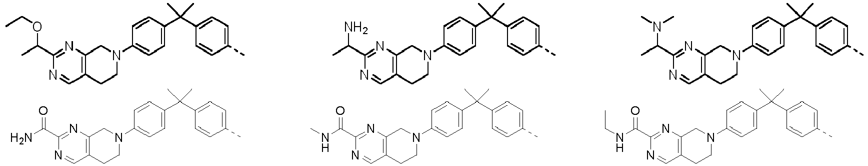

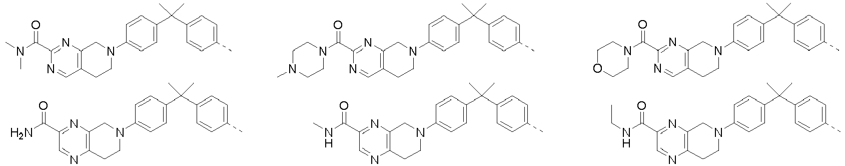

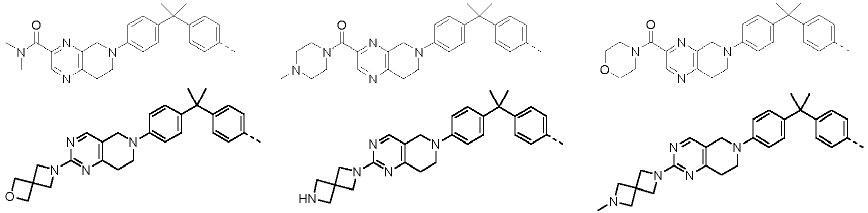

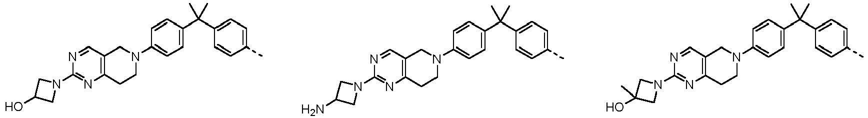

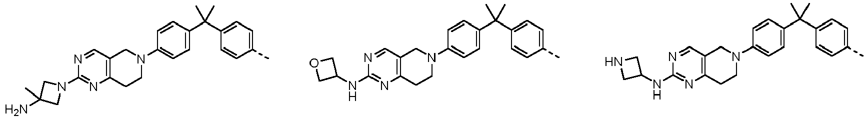

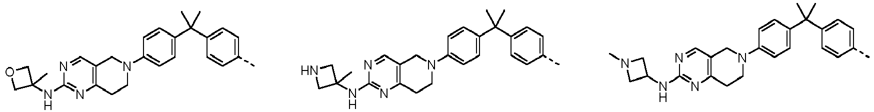

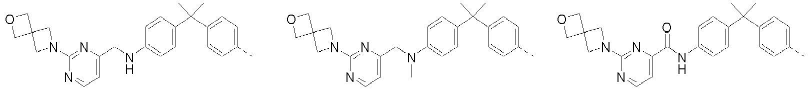

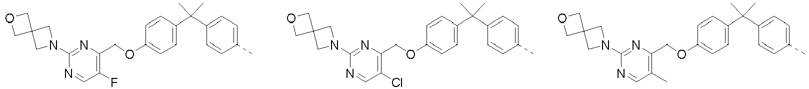

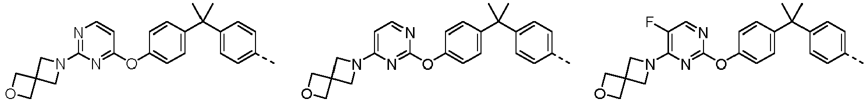

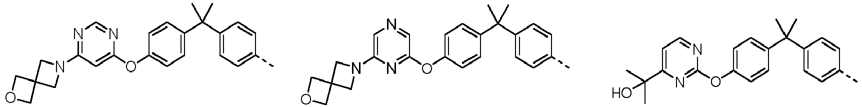

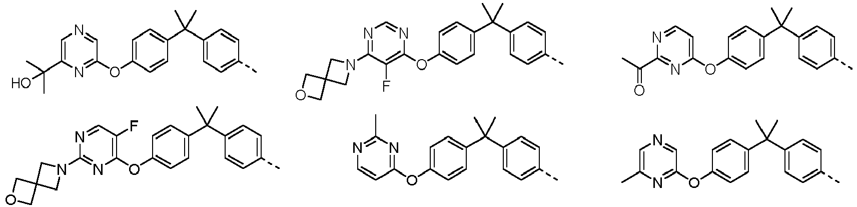

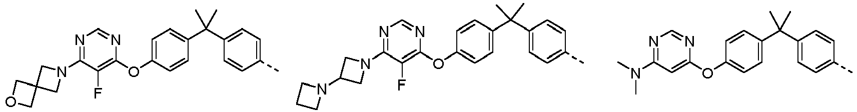

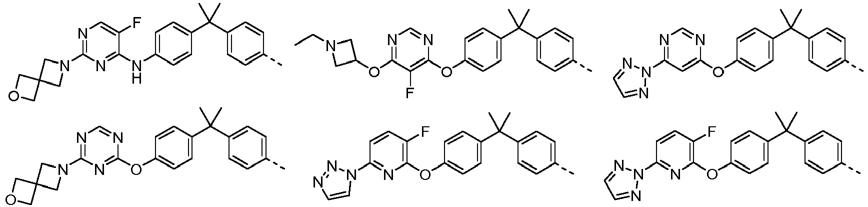

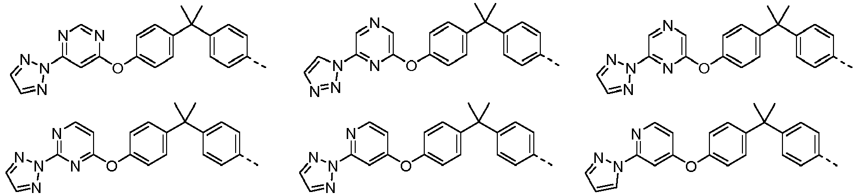

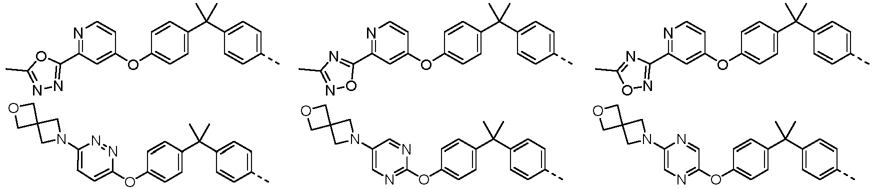

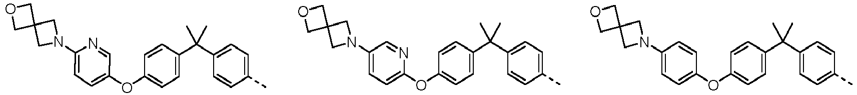

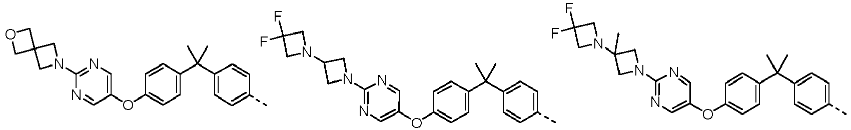

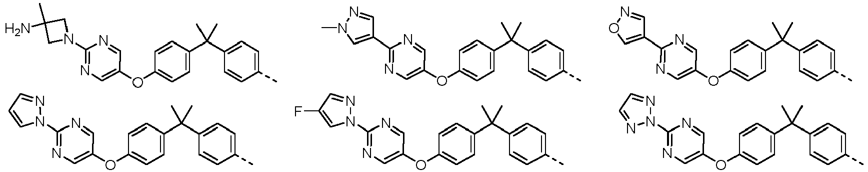

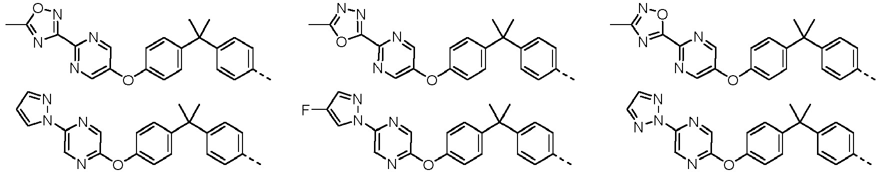

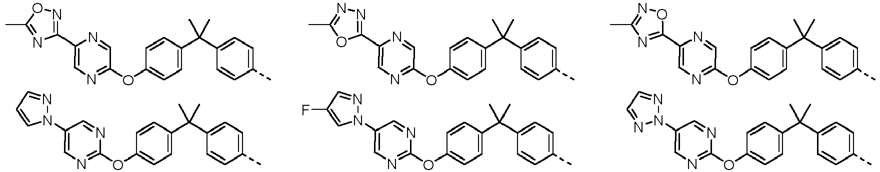

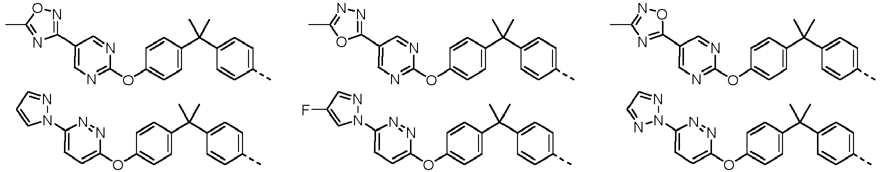

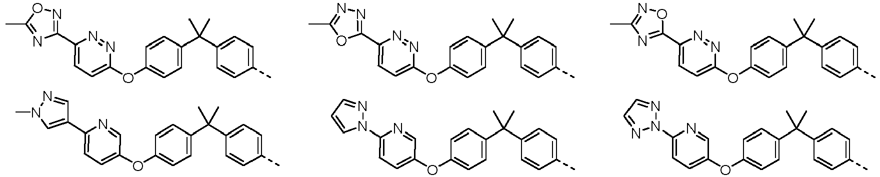

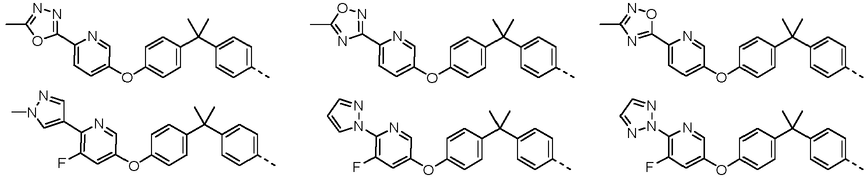

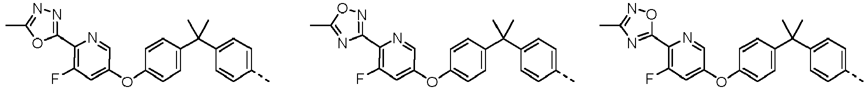

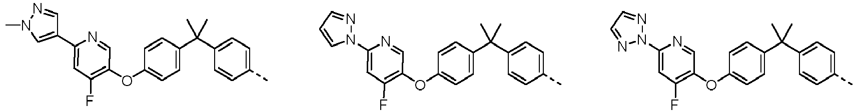

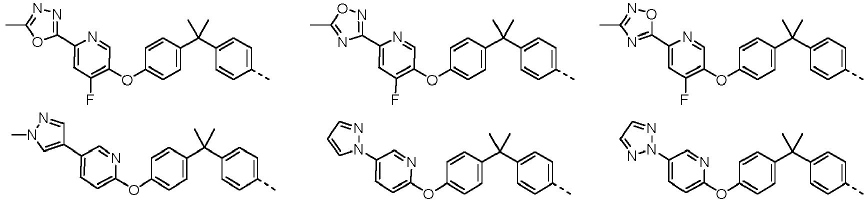

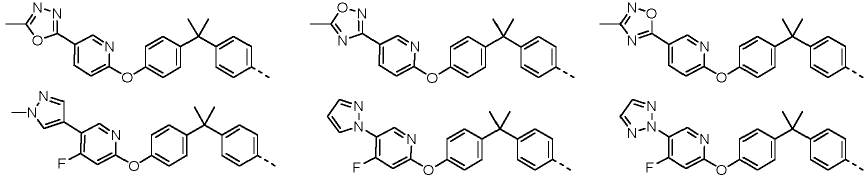

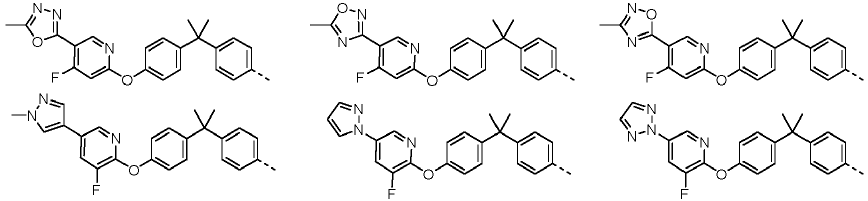

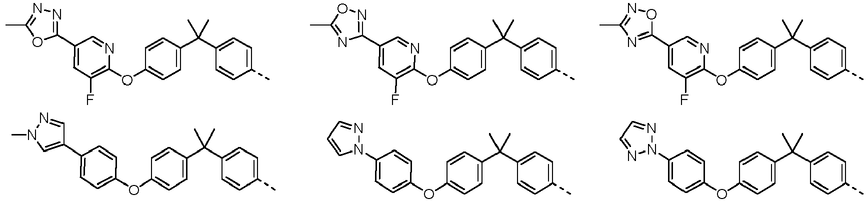

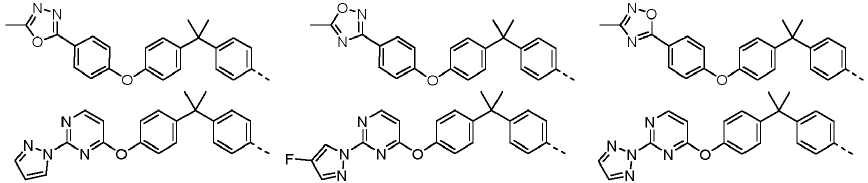

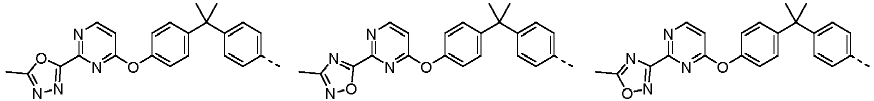

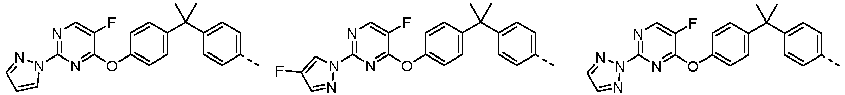

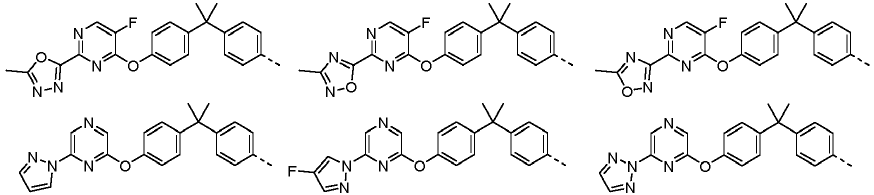

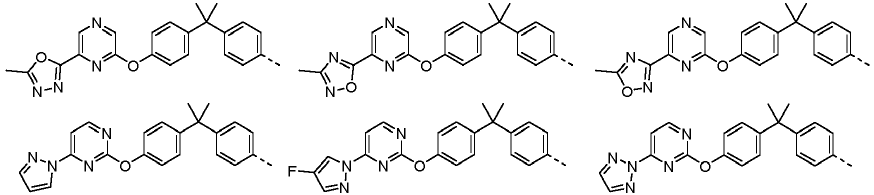

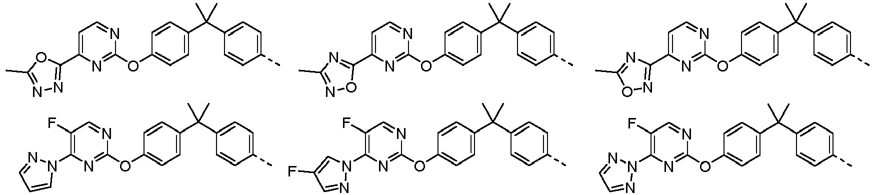

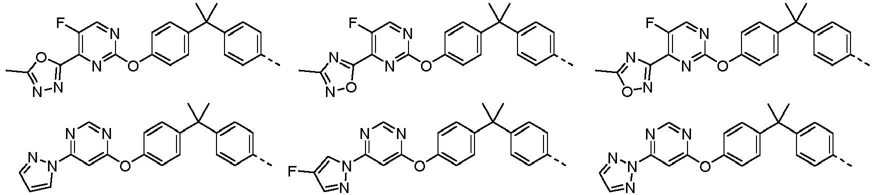

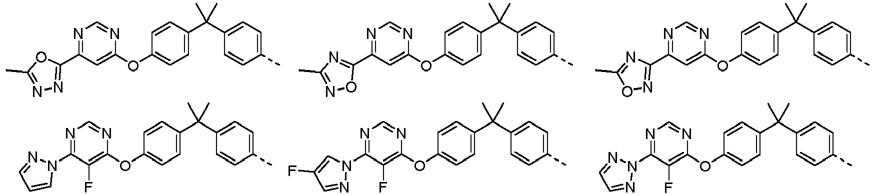

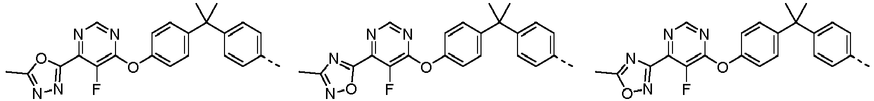

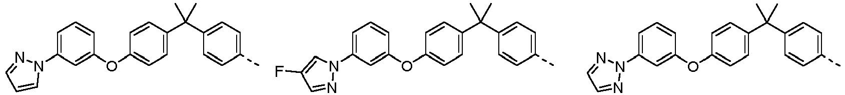

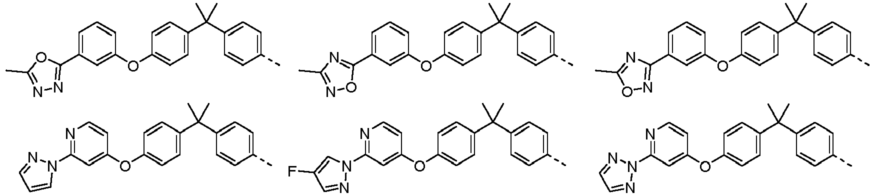

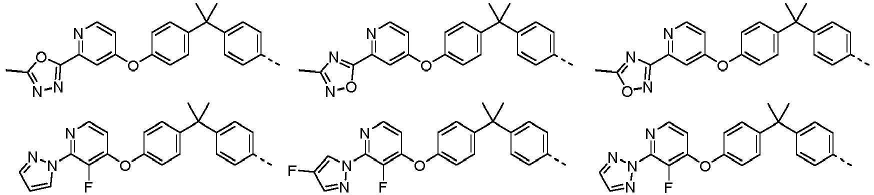

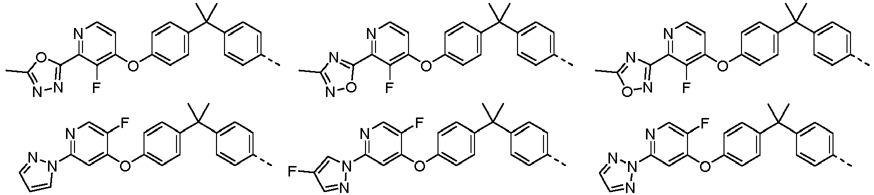

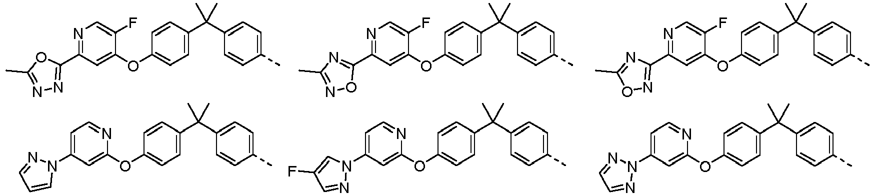

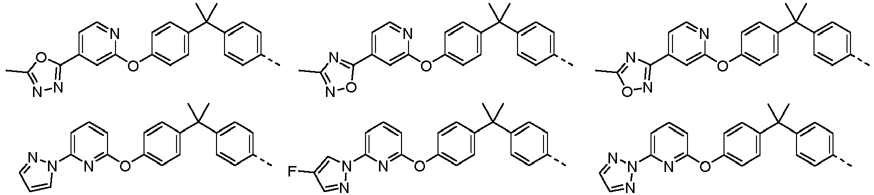

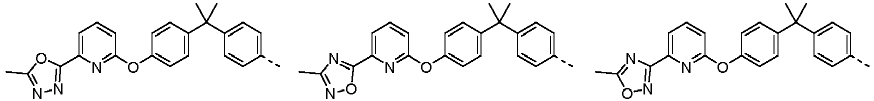

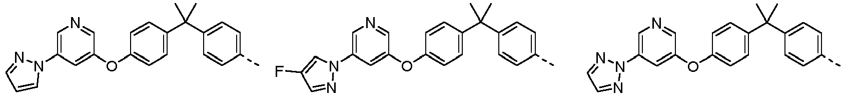

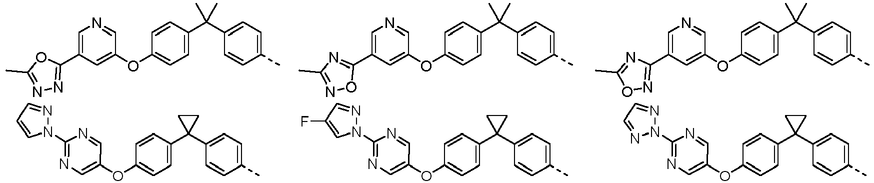

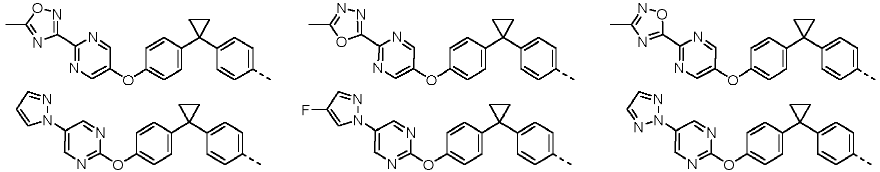

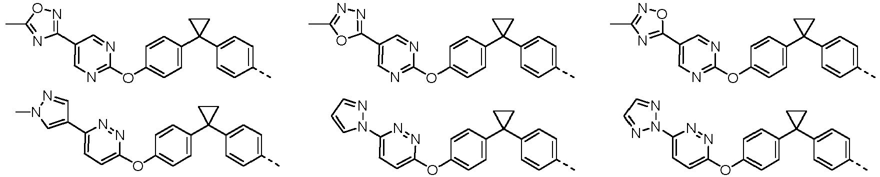

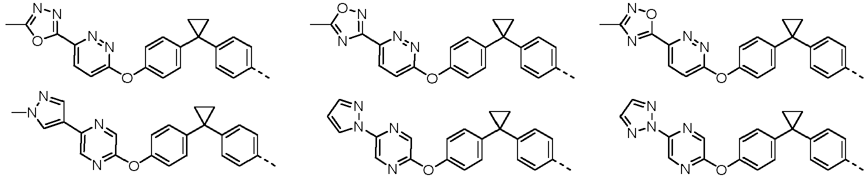

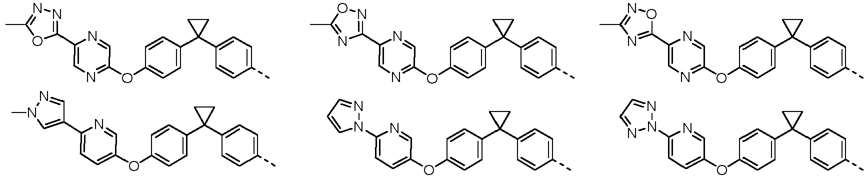

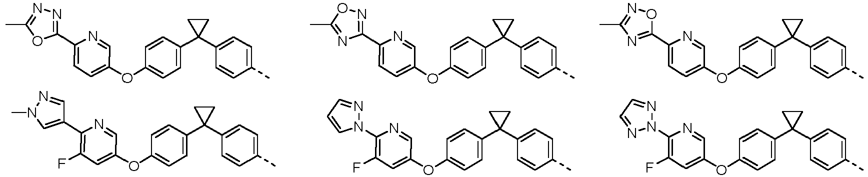

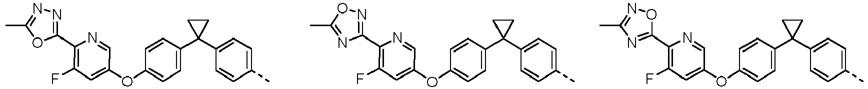

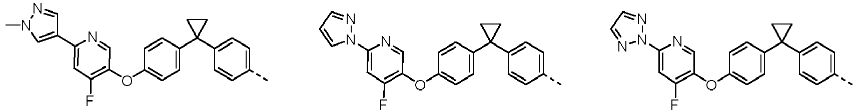

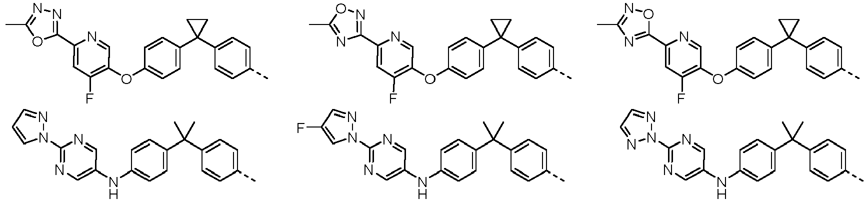

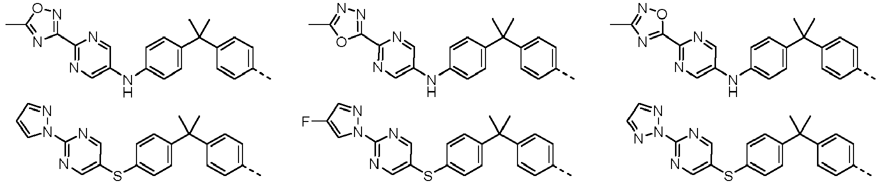

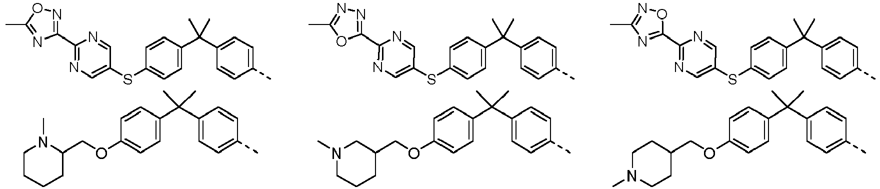

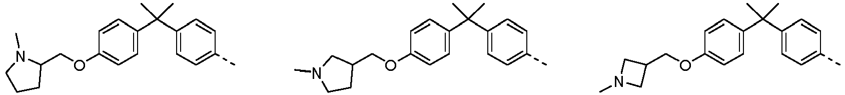

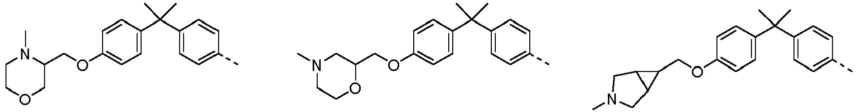

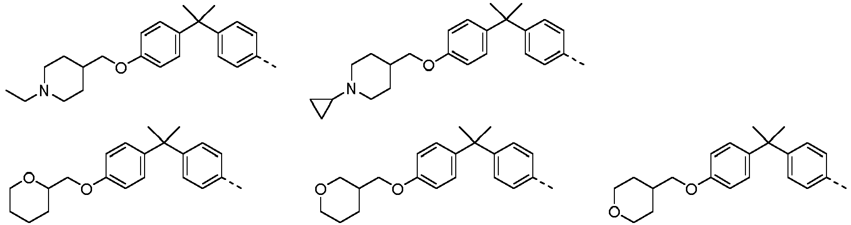

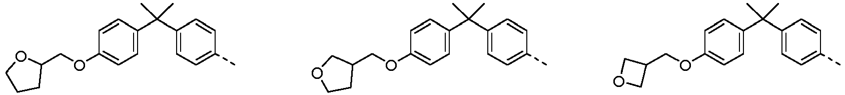

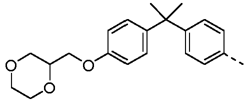

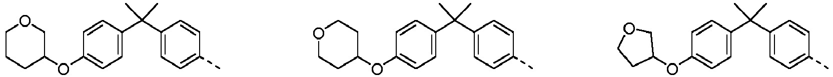

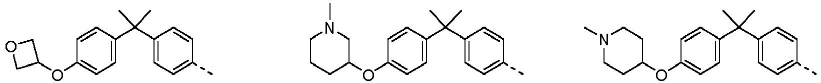

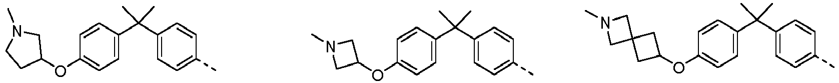

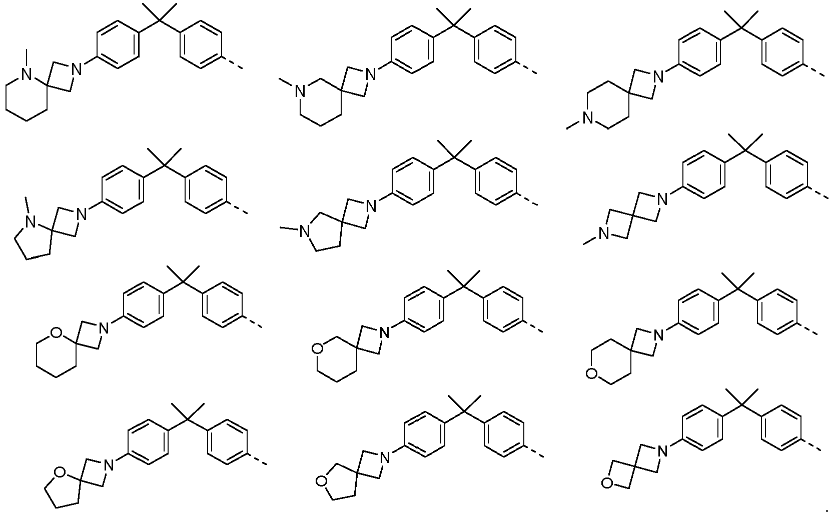

21. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of L moiety is selected from the group consisting of:

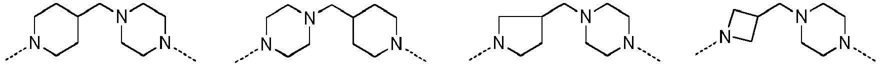

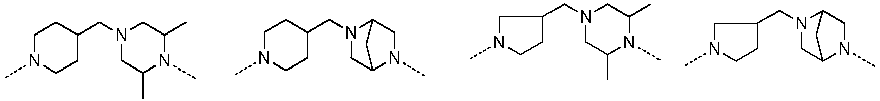

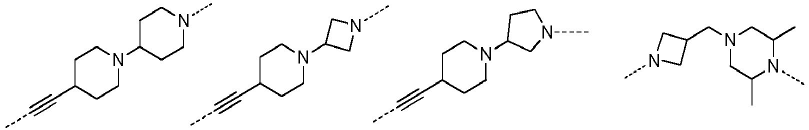

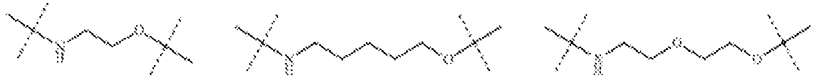

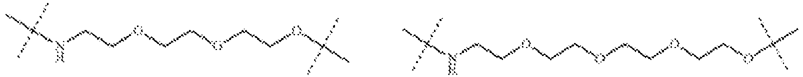

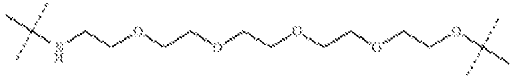

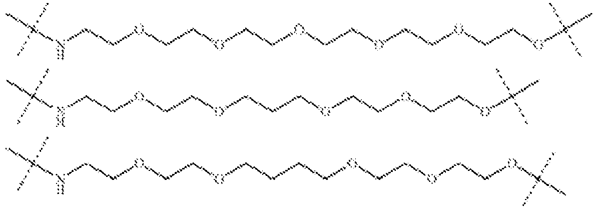

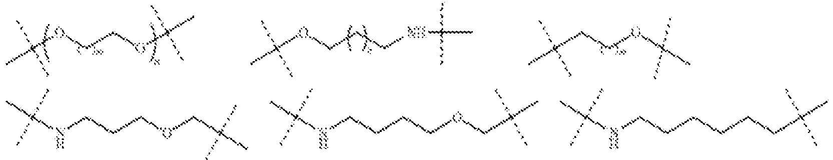

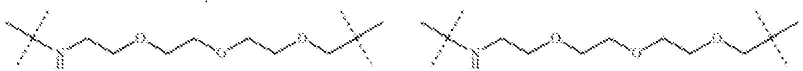

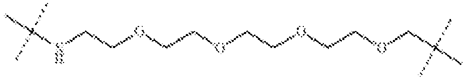

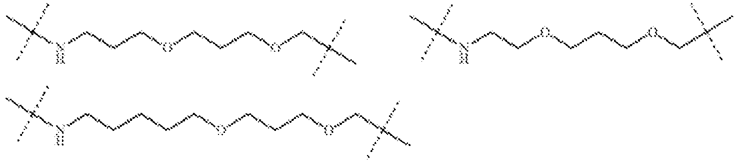

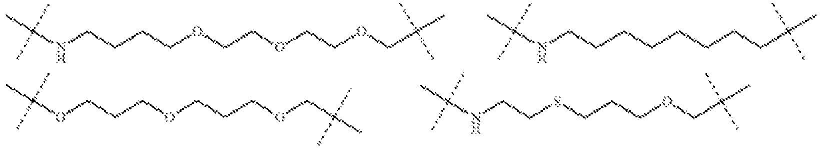

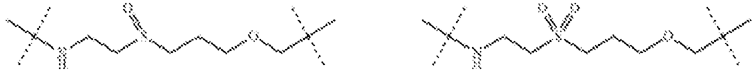

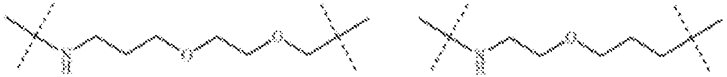

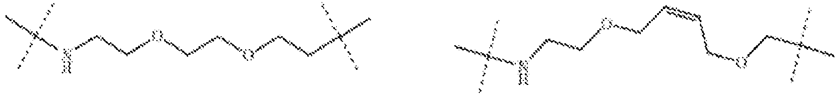

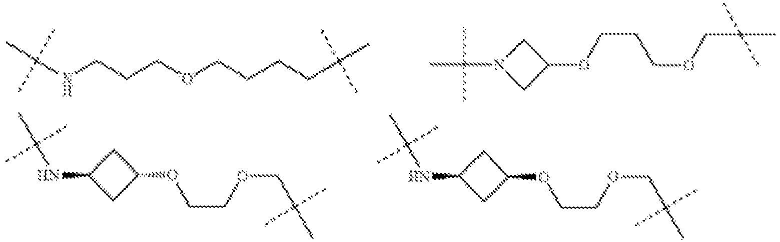

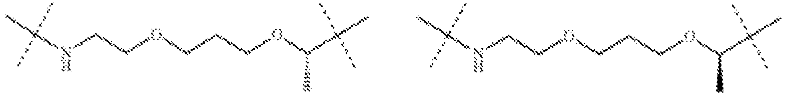

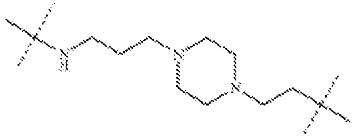

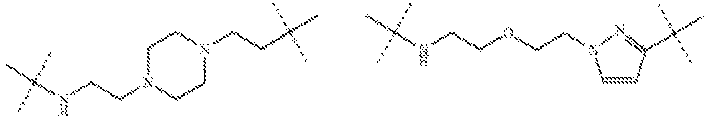

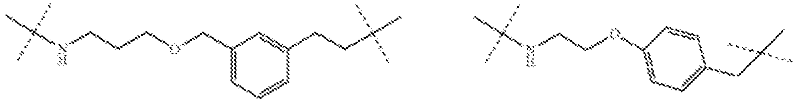

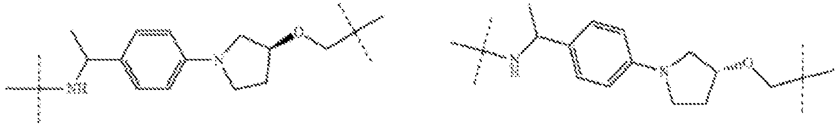

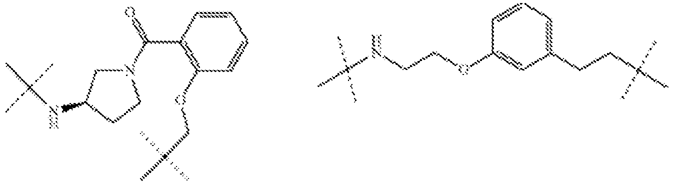

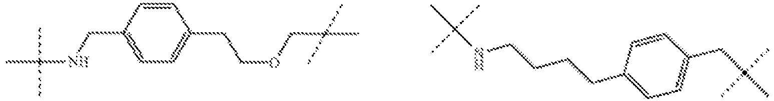

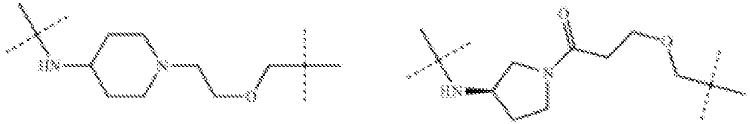

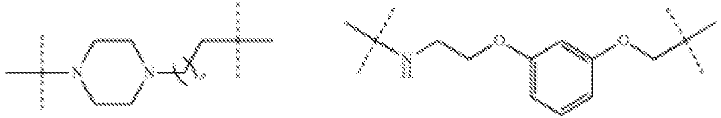

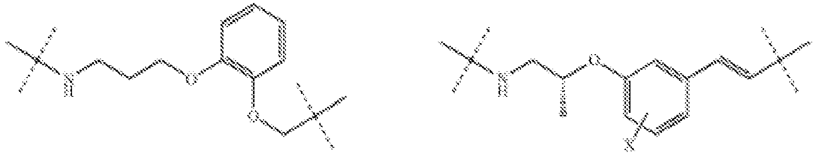

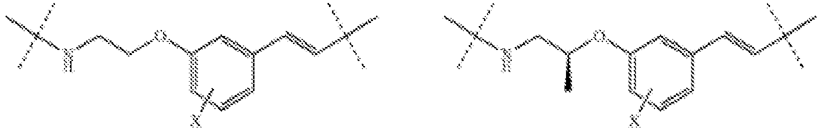

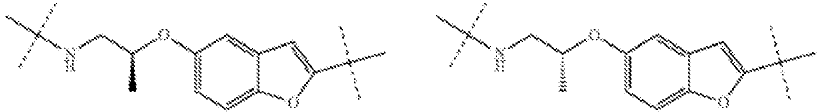

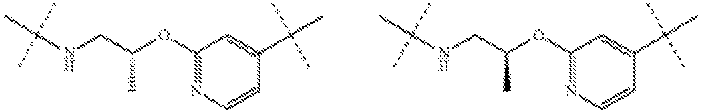

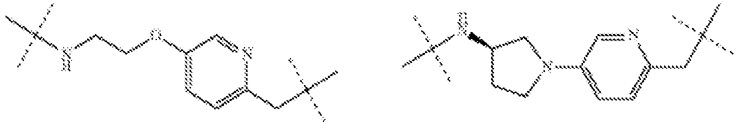

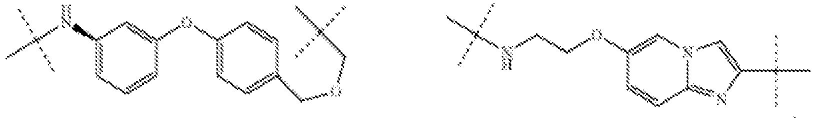

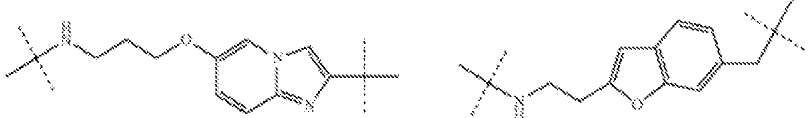

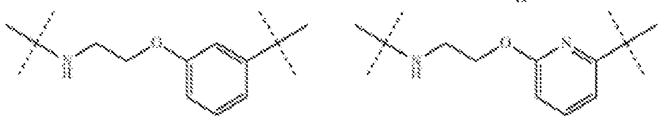

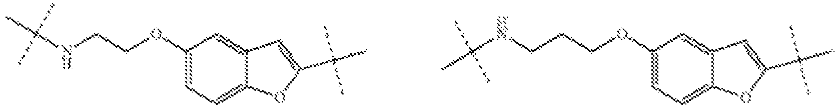

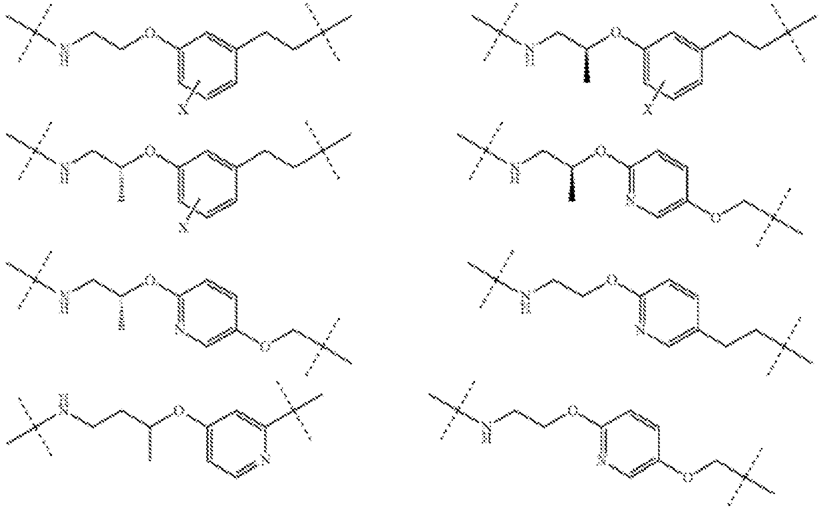

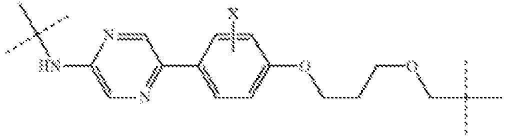

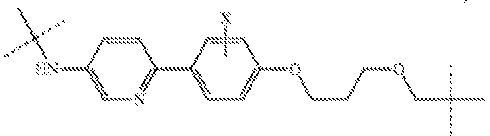

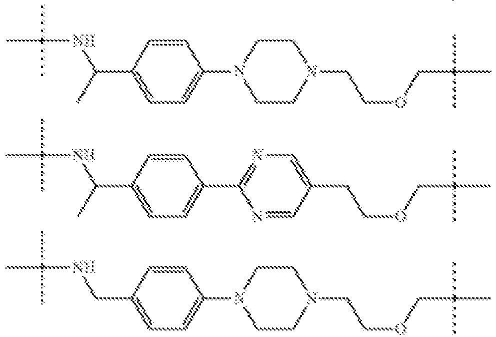

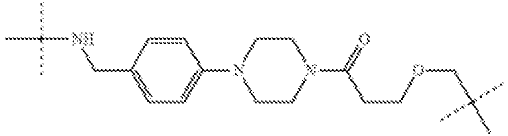

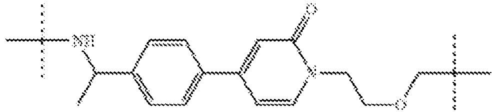

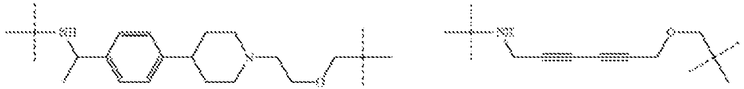

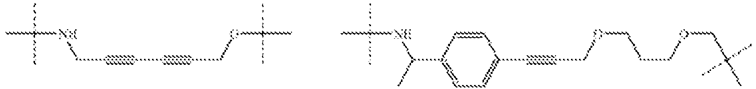

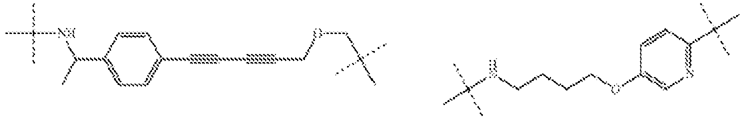

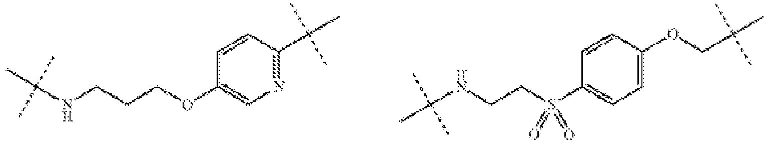

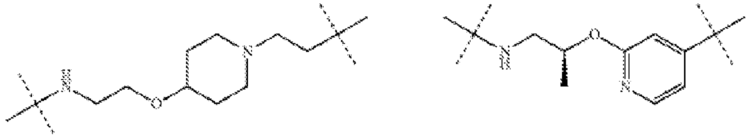

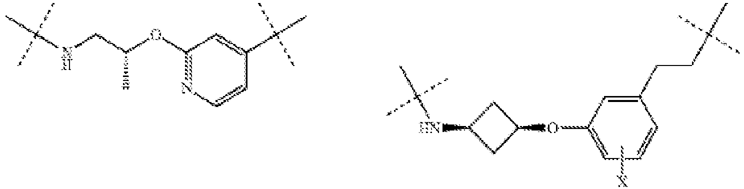

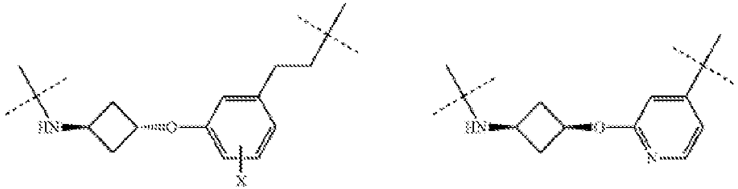

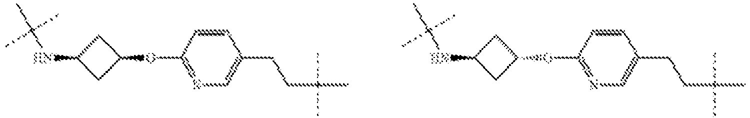

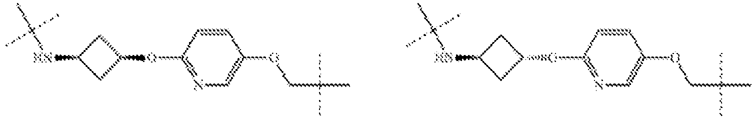

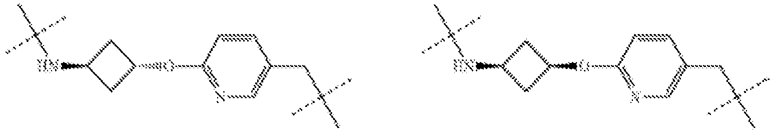

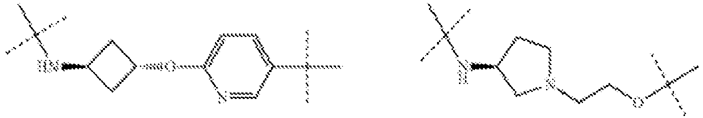

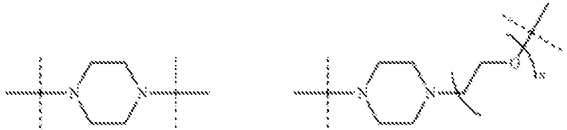

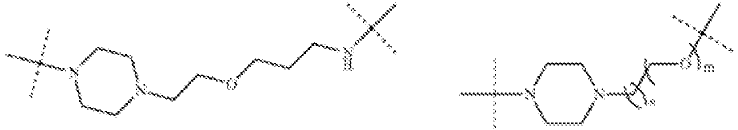

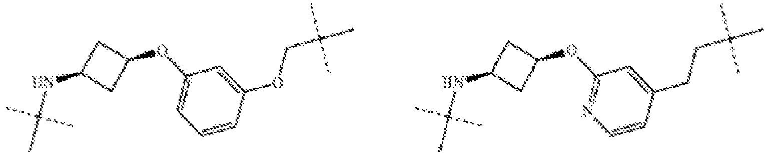

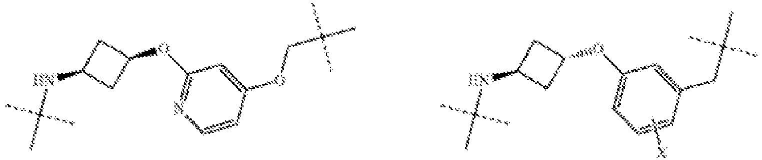

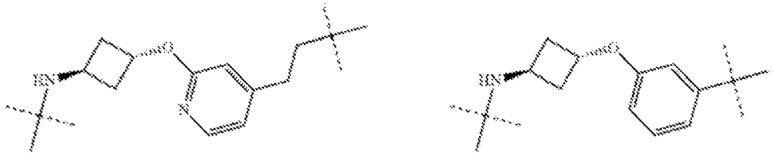

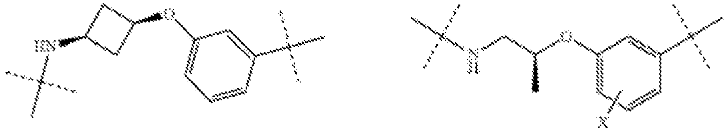

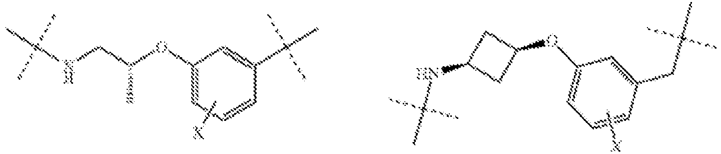

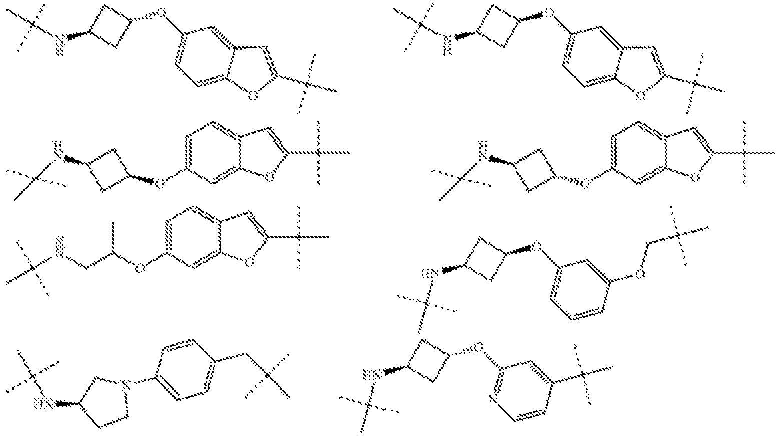

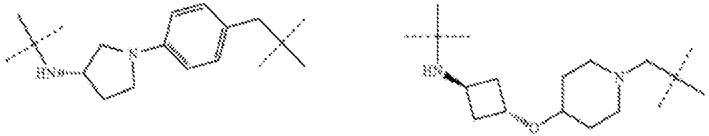

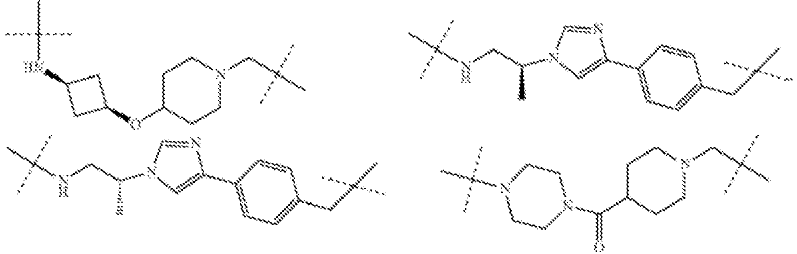

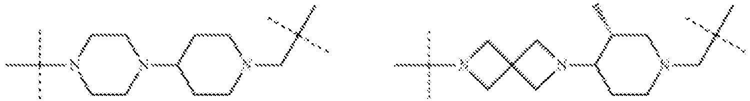

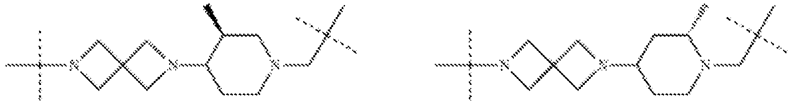

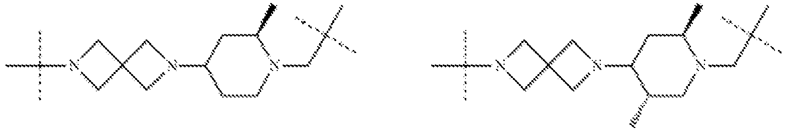

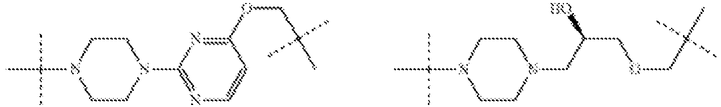

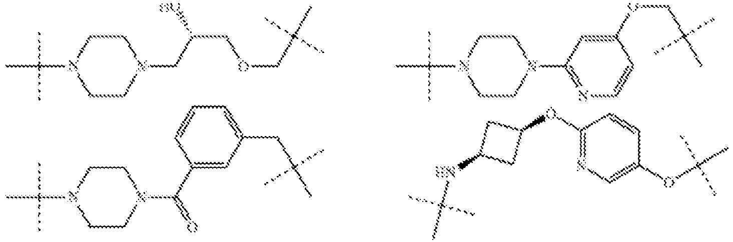

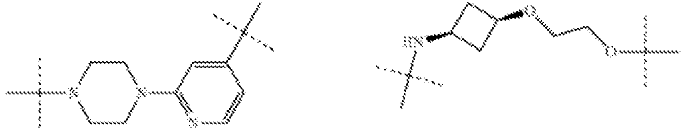

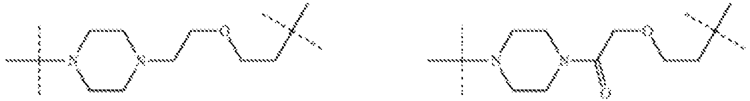

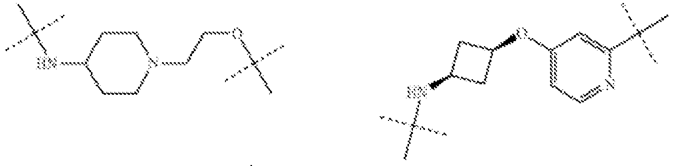

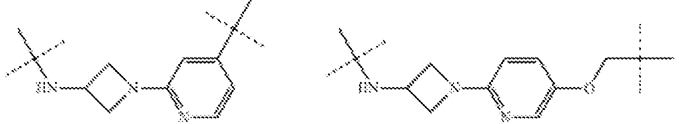

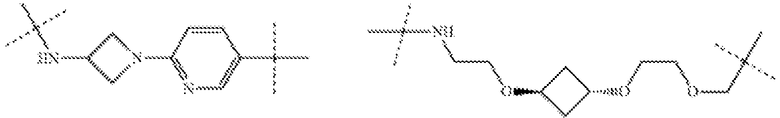

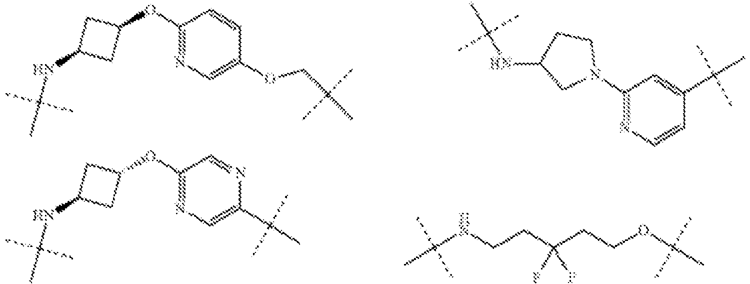

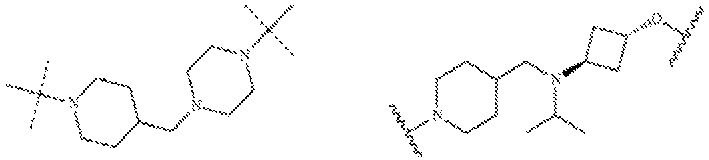

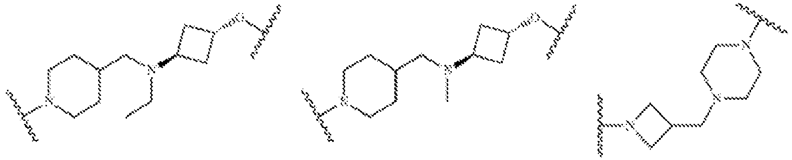

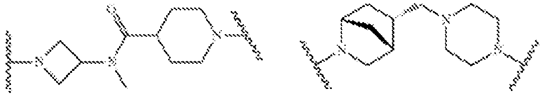

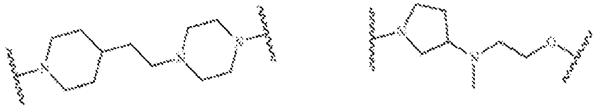

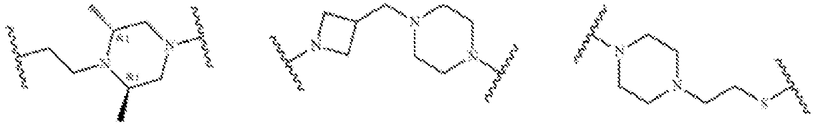

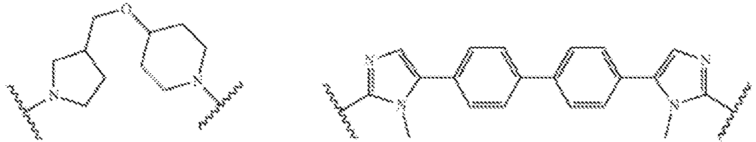

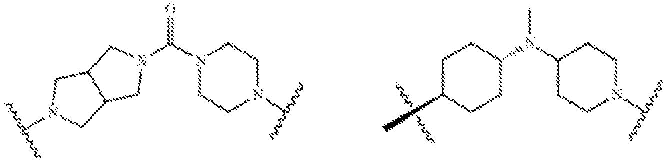

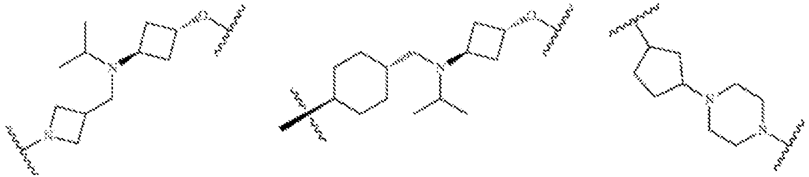

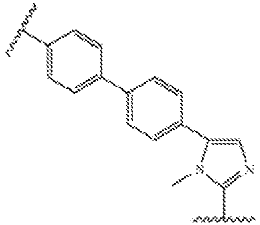

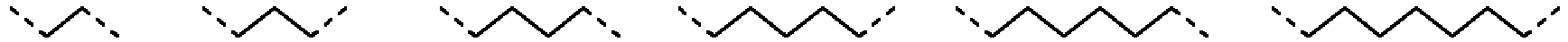

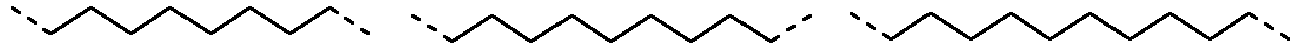

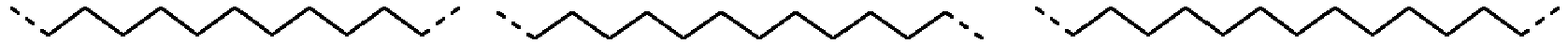

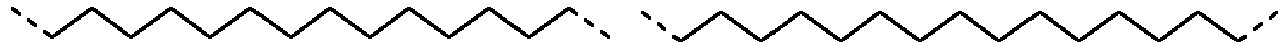

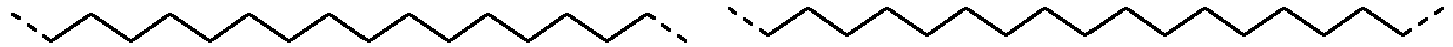

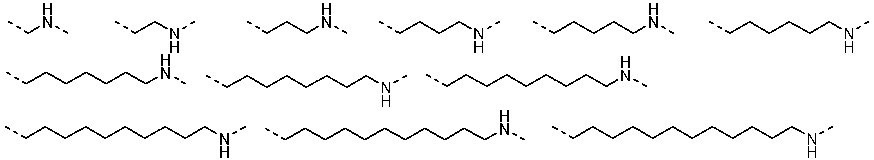

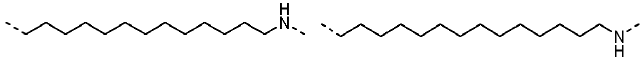

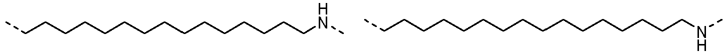

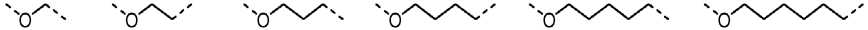

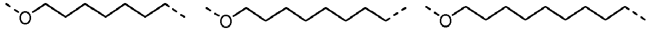

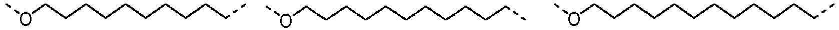

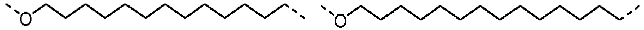

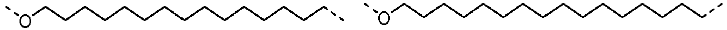

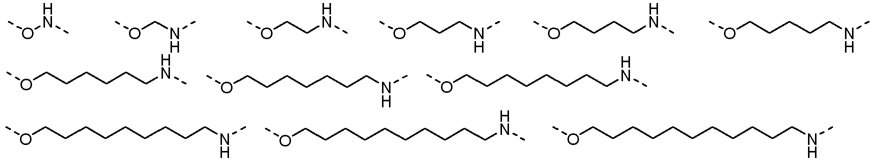

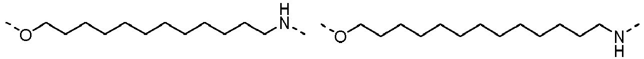

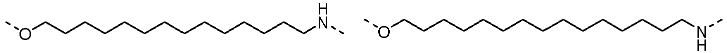

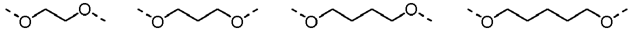

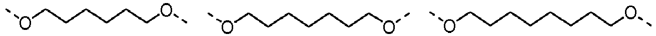

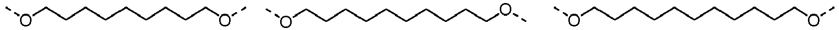

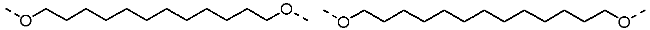

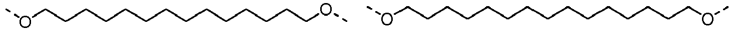

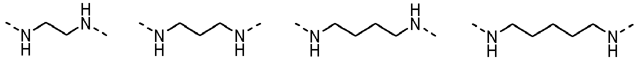

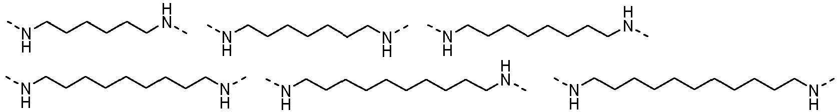

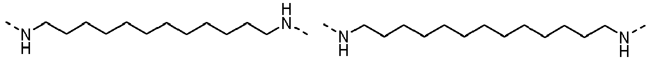

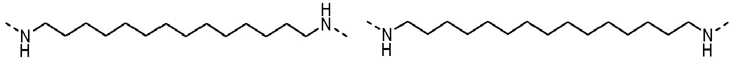

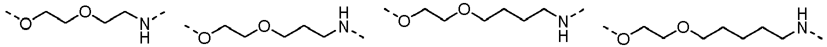

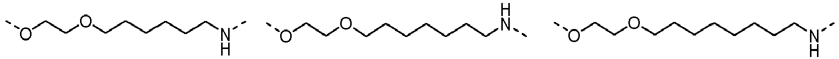

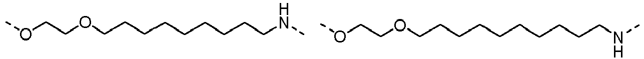

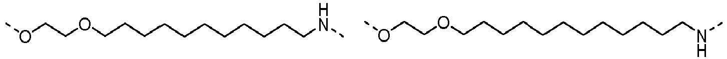

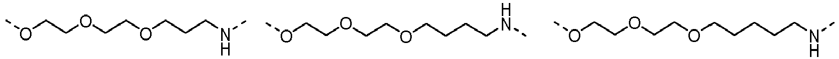

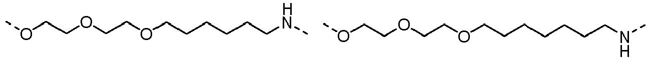

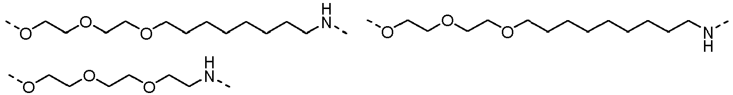

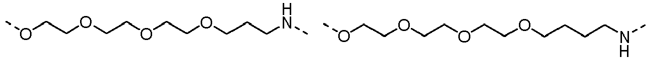

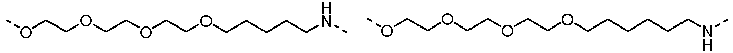

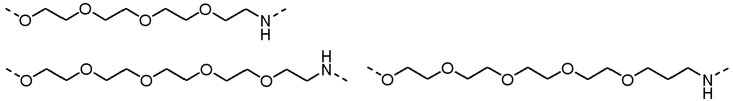

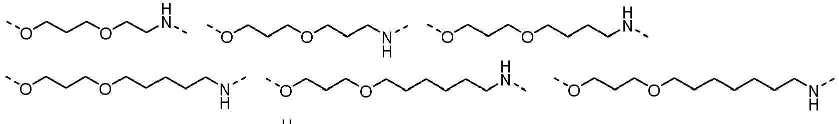

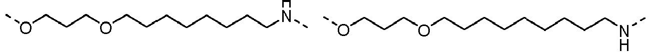

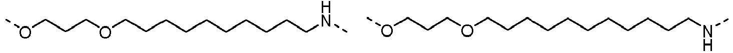

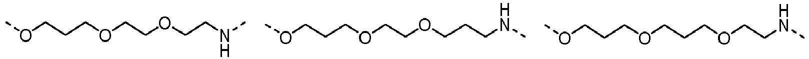

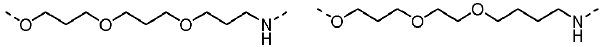

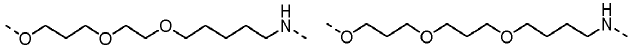

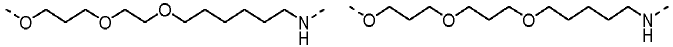

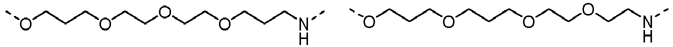

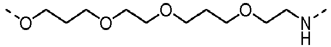

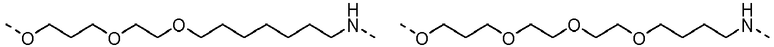

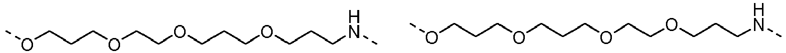

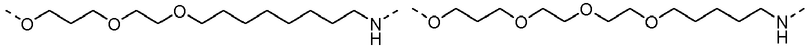

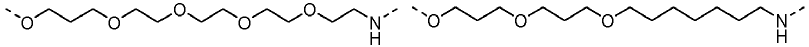

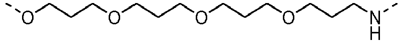

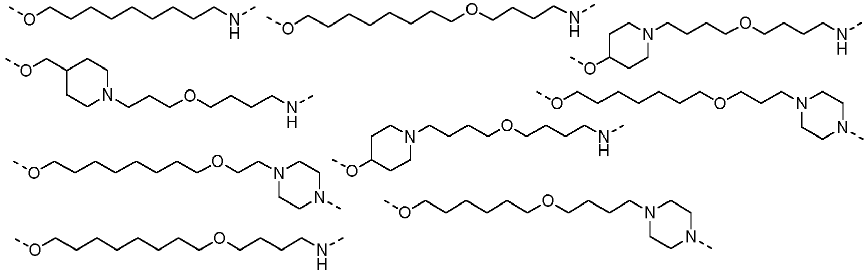

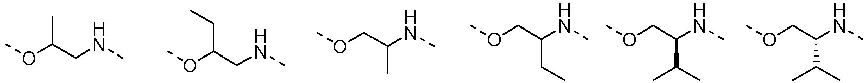

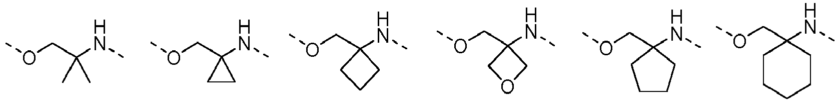

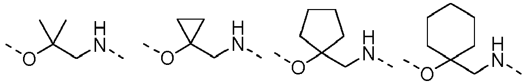

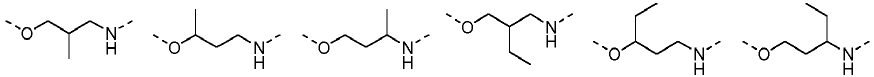

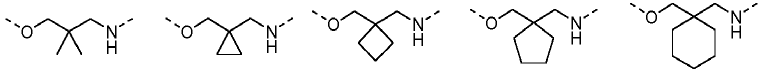

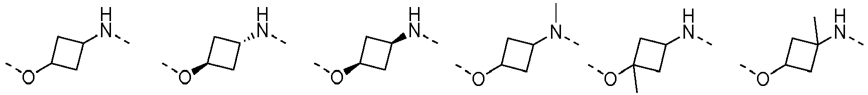

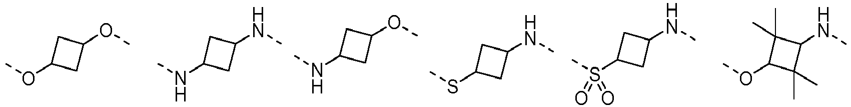

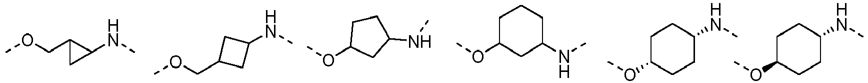

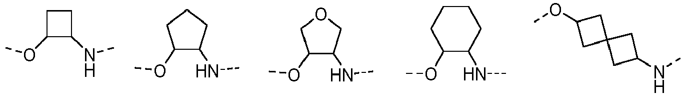

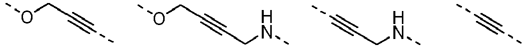

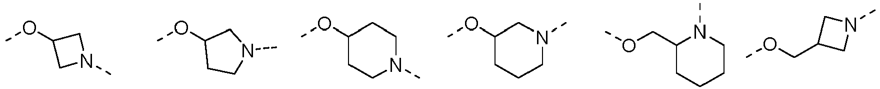

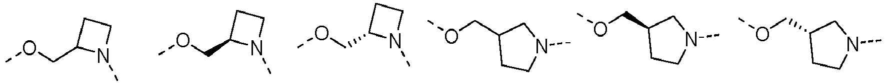

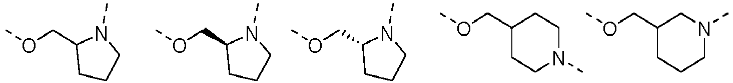

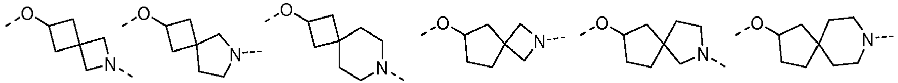

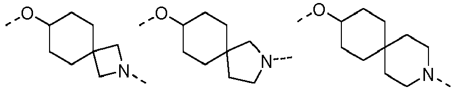

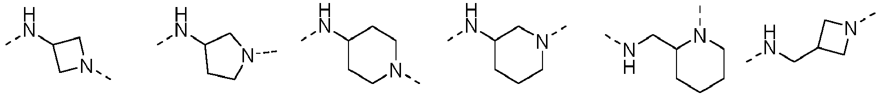

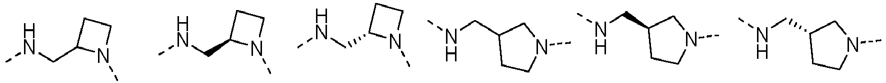

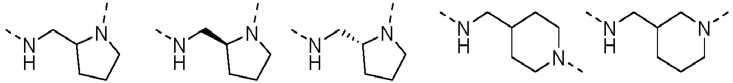

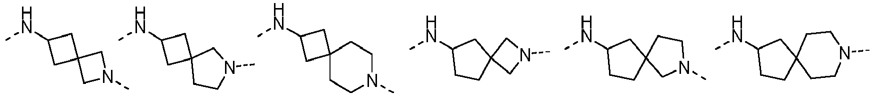

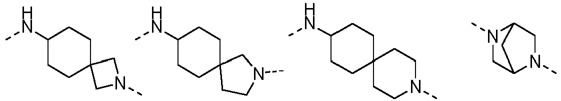

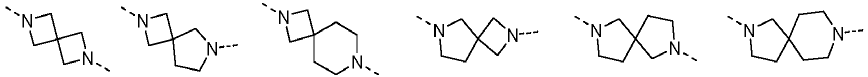

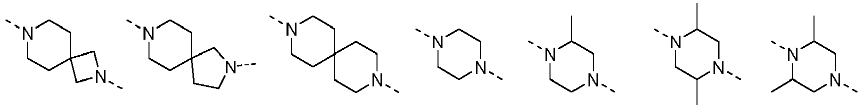

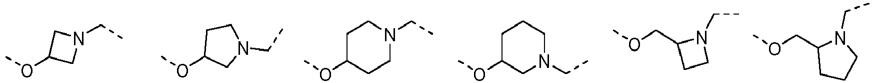

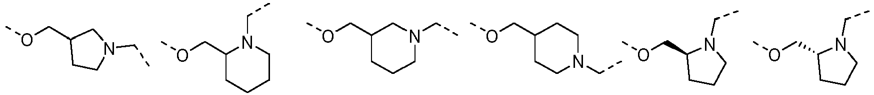

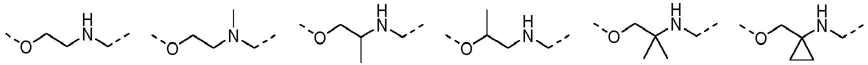

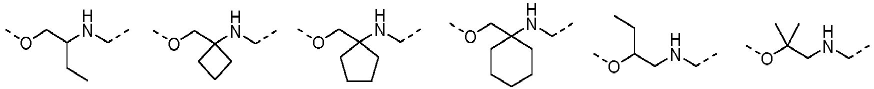

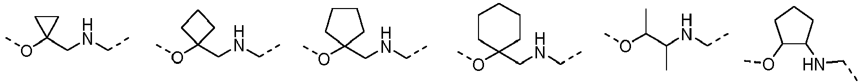

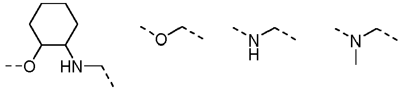

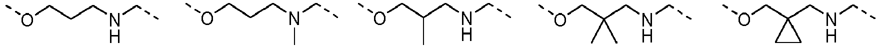

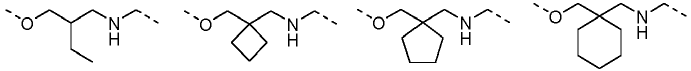

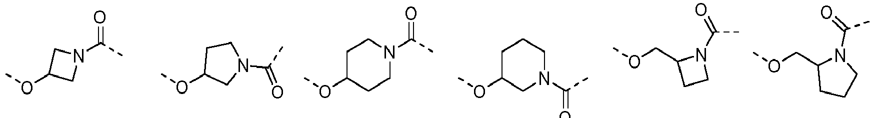

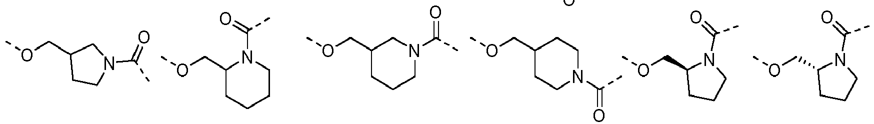

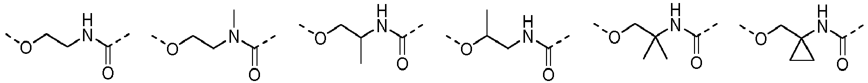

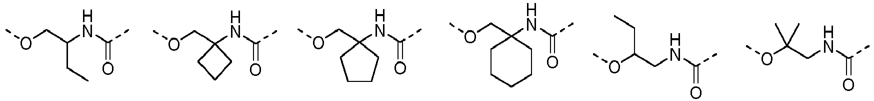

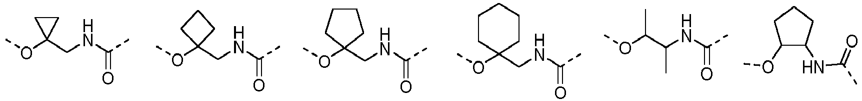

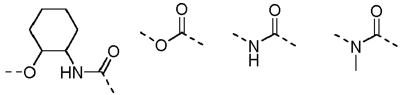

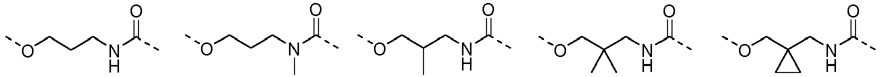

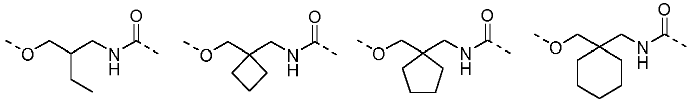

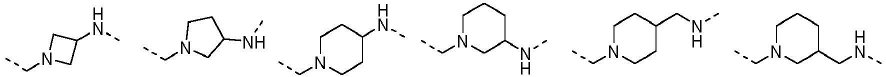

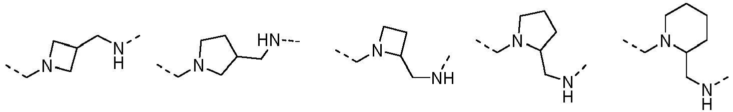

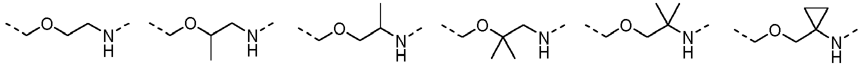

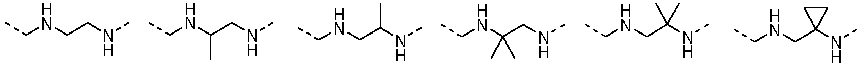

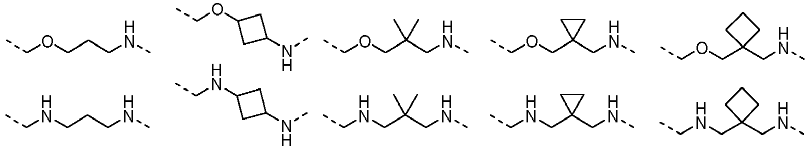

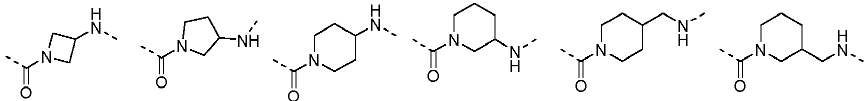

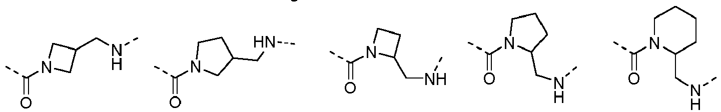

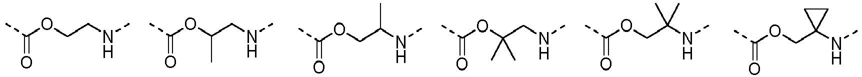

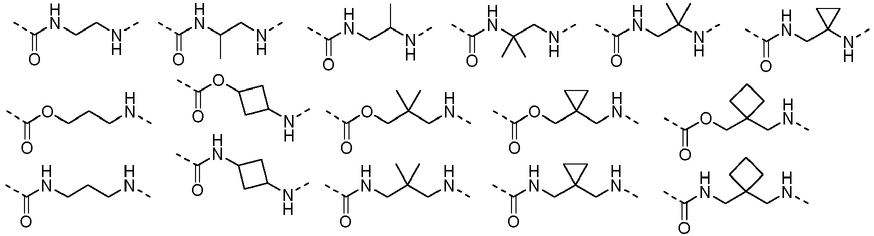

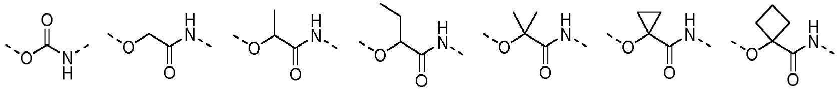

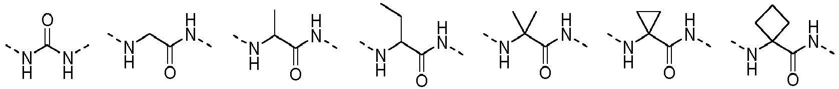

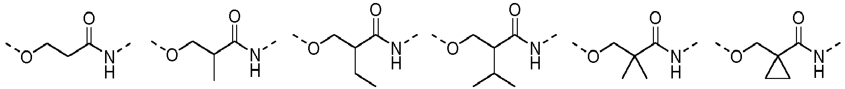

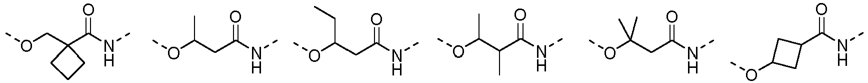

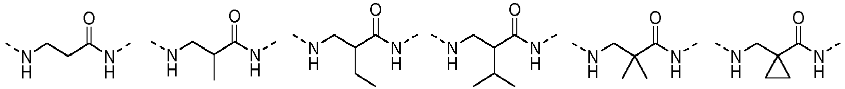

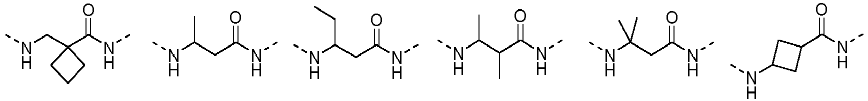

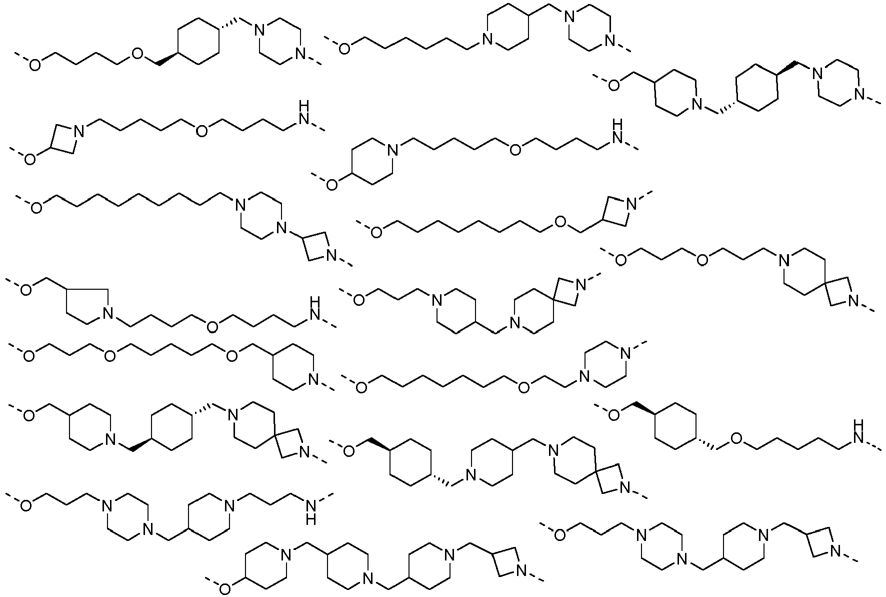

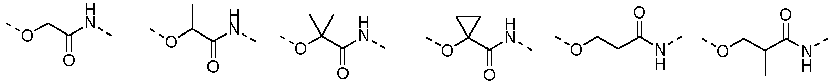

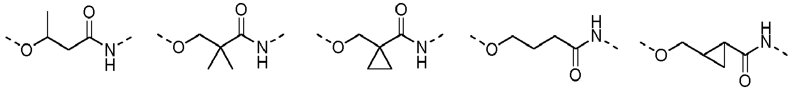

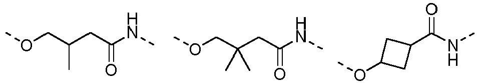

22. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of L moiety is selected from the group consisting of:

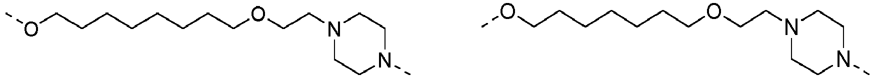

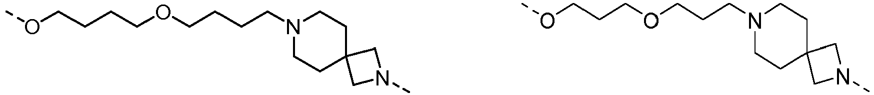

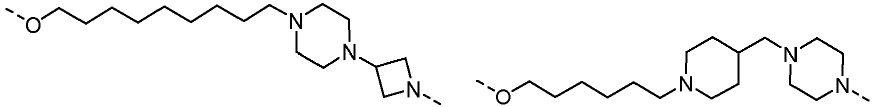

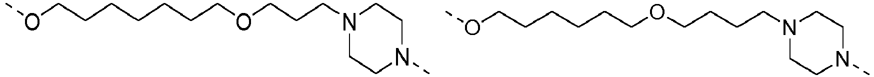

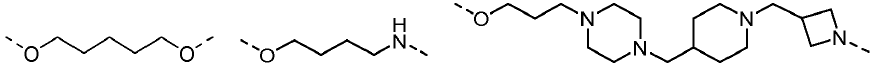

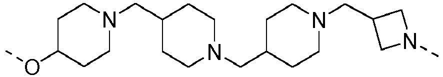

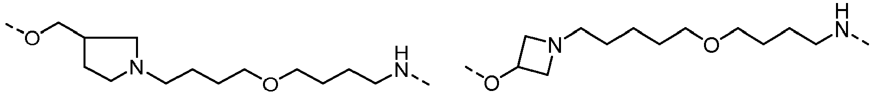

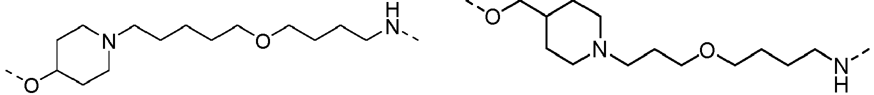

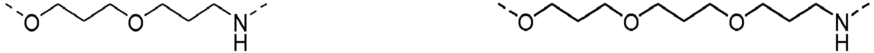

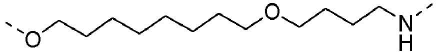

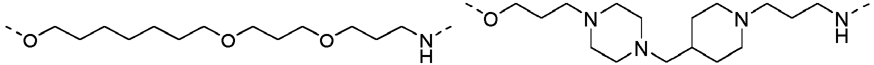

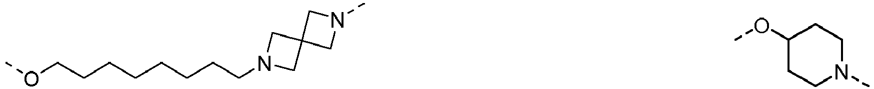

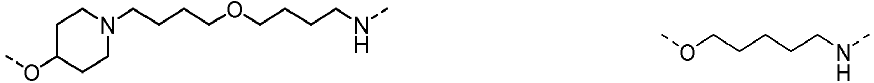

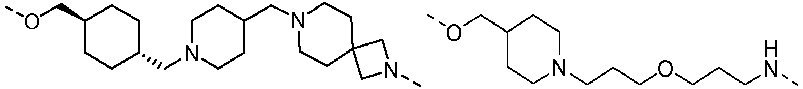

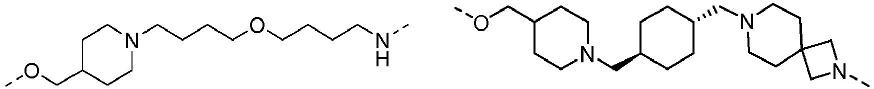

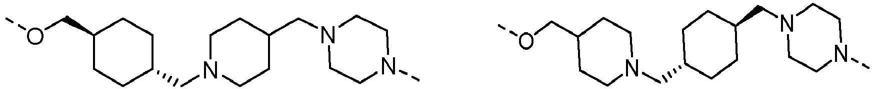

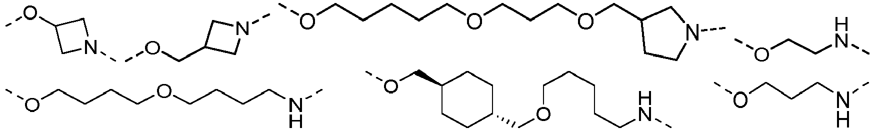

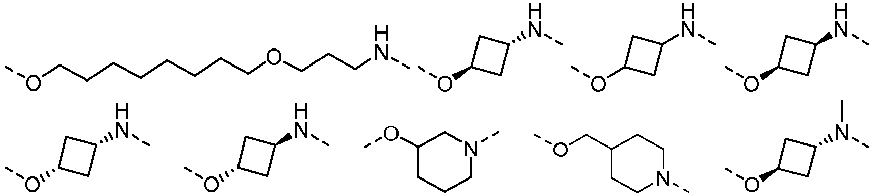

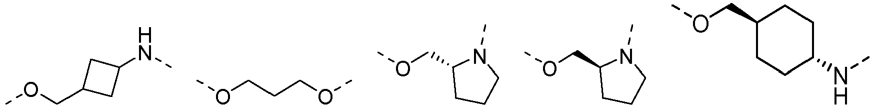

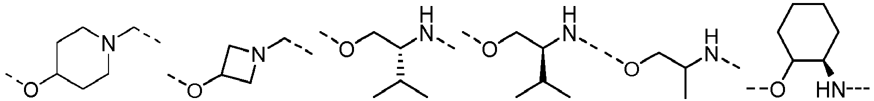

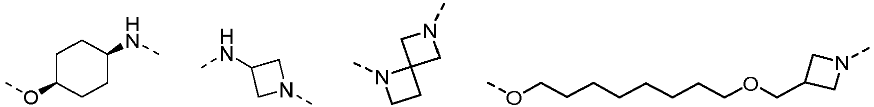

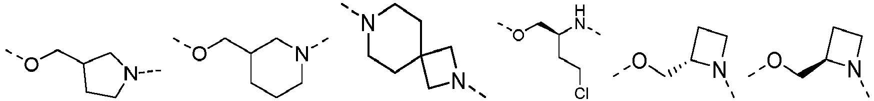

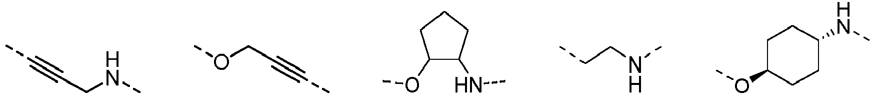

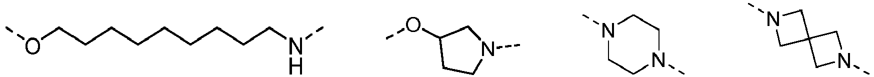

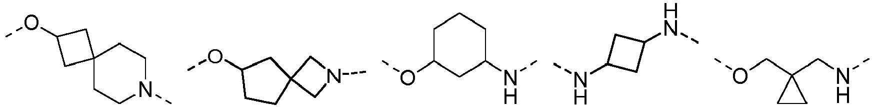

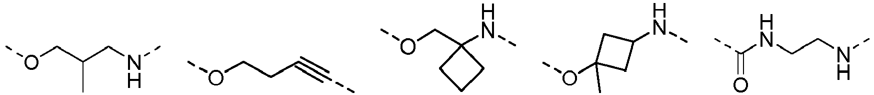

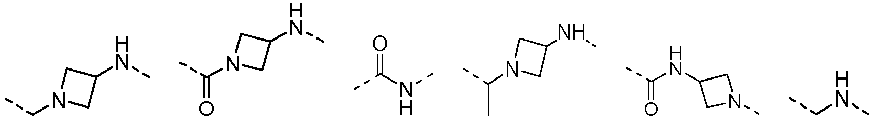

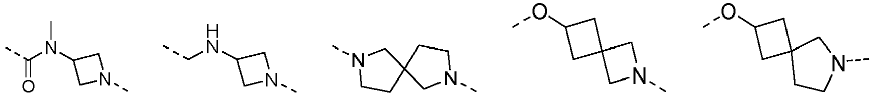

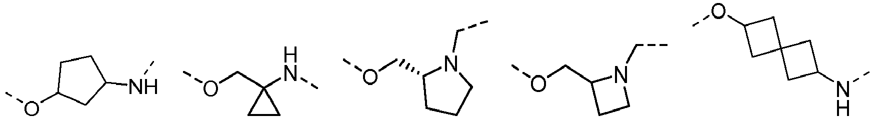

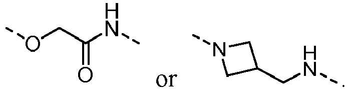

23. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of L moiety is as represented by formula X-A: 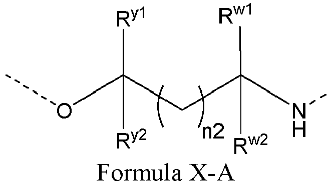
wherein, n2 is 0 or 1;
R^{y1} and R^{y2} are each independently selected from the group consisting of H and C1-C3 alkyl; R^{w1} and R^{w2} are each independently selected from the group consisting of H and halo-substituted or unsubstituted C1-C3 alkyl;
or, R^{y1} and R^{y2} are linked to form 3-4 membered saturated carbon ring; or R^{w1} and R^{w2} are linked to form 3-4 membered saturated carbon ring; or R^{y2} and R^{w2} are linked to form 3-4 membered saturated carbon ring.

24. The compound according to claim 23, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that**:
R^{y1} and R^{y2} are each independently selected from the group consisting of H and methyl; R^{w1} and R^{w2} are each independently selected from the group consisting of H, methyl, and Cl-substituted or unsubstituted propyl;
or, R^{y1} and R^{y2} are linked to form 3-membered saturated carbon ring; or R^{w1} and R^{w2} are linked to form 3-4 membered saturated carbon ring; or R^{y2} and R^{w2} are linked to form 4-membered saturated carbon ring.

25. The compound according to claim 24, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of L moiety is selected from the group consisting of:

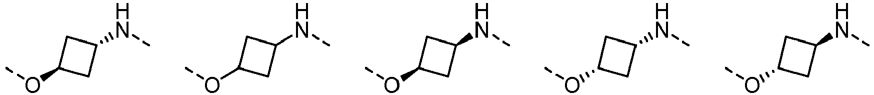

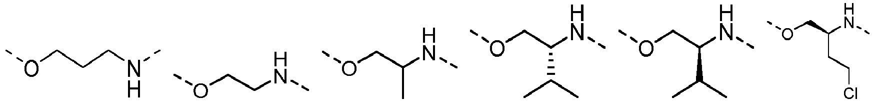

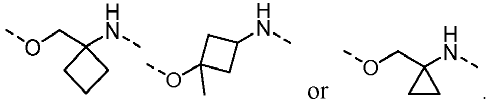

26. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of U moiety is selected from the group consisting of:

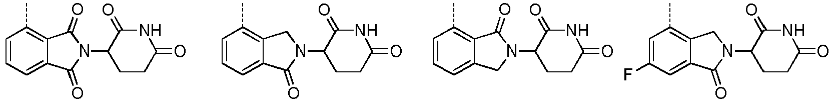

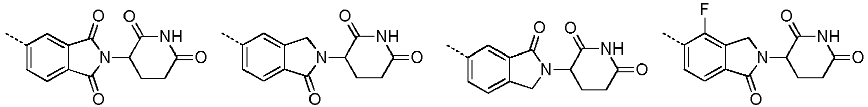

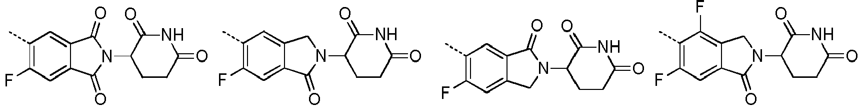

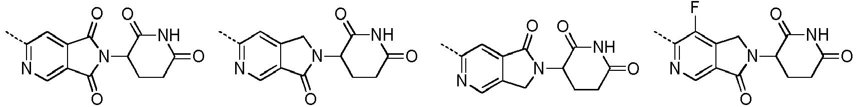

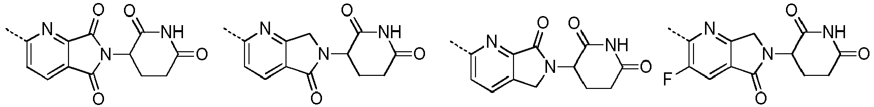

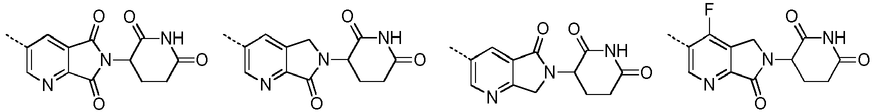

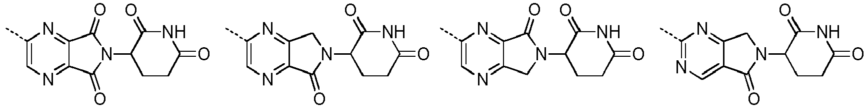

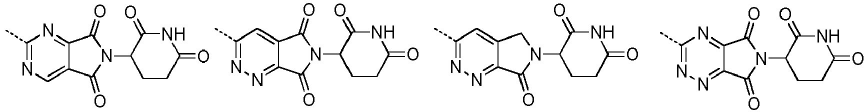

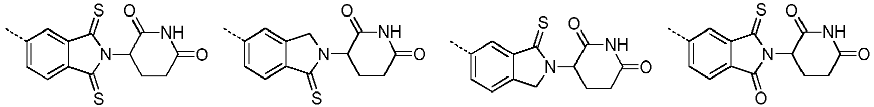

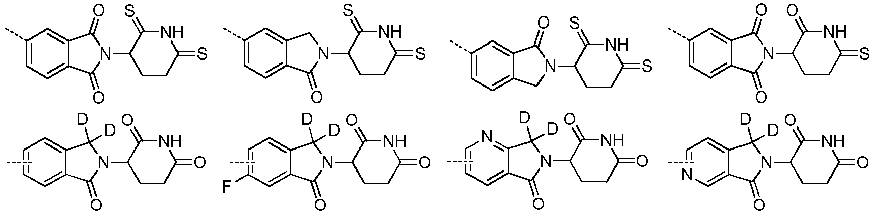

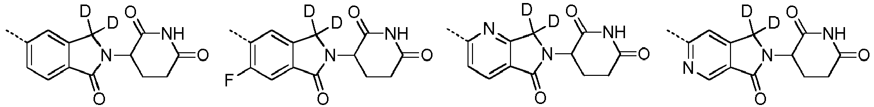

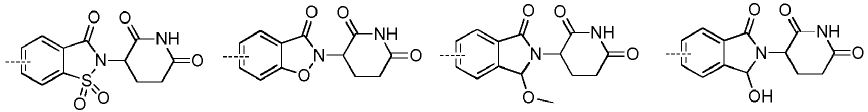

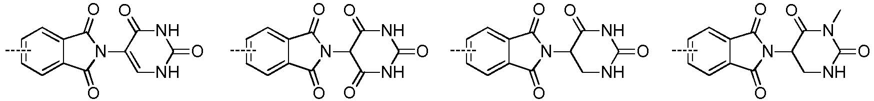

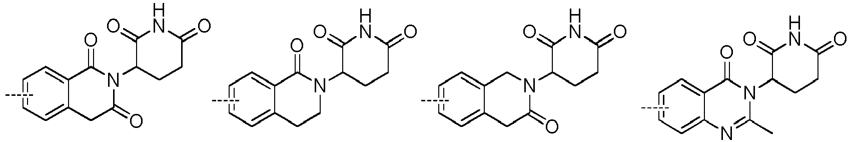

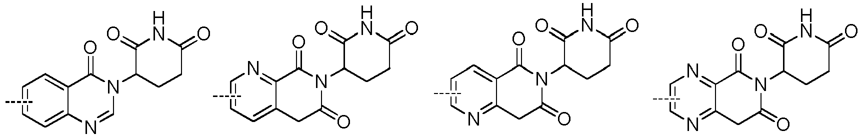

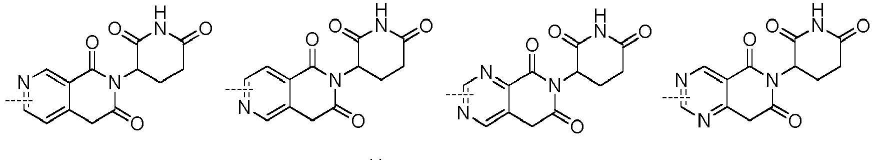

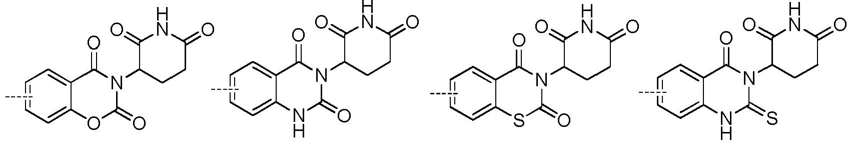

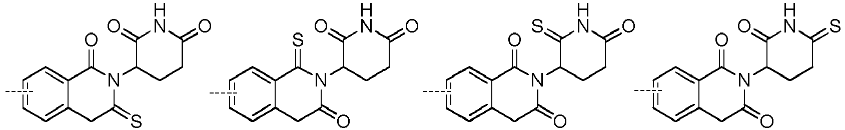

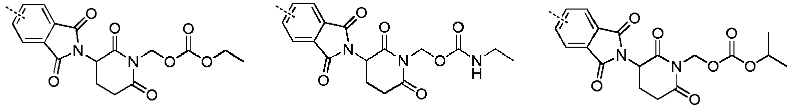

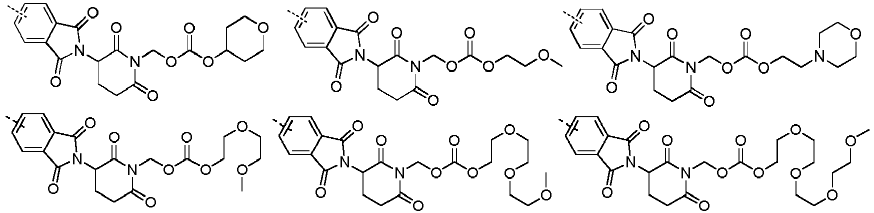

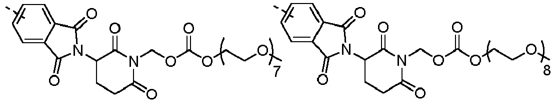

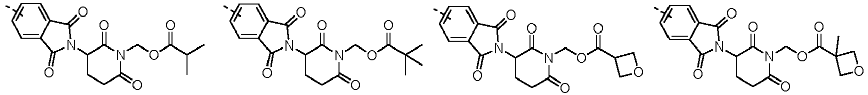

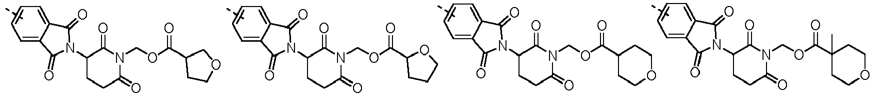

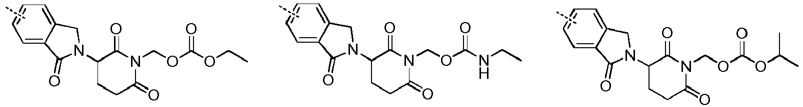

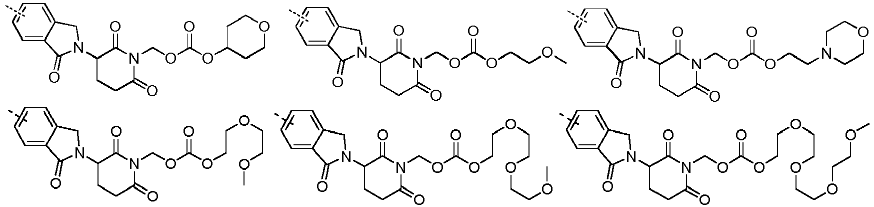

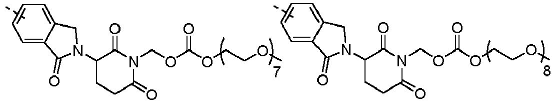

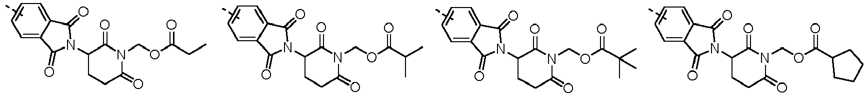

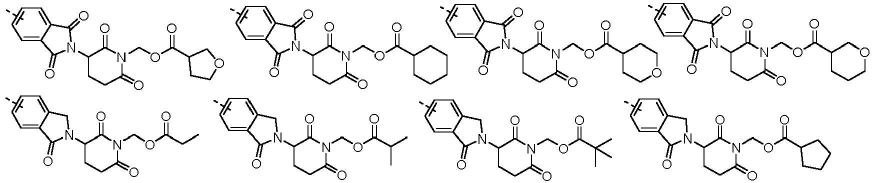

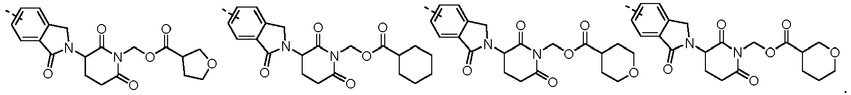

27. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of 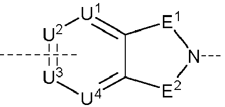 in formula I-U is selected from the group consisting of:

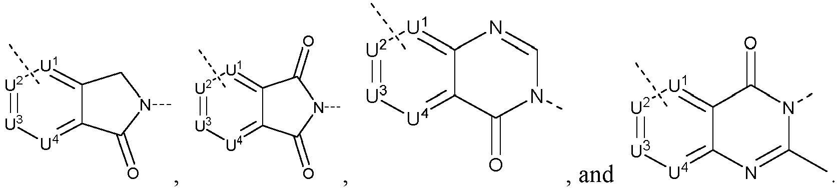

28. The compound according to claim 27, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** U¹, U², U³, and U⁴ are CH; or, one of U¹, U², U³, and U⁴ is N or CX₀, and the others are CH, wherein X₀ is halogen.

29. The compound according to any one of claims 1 to 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of 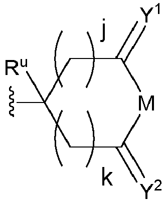 moiety in formula I-U is the following structure:

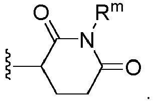

30. The compound according to claim 29, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** R^{m} is selected from the group consisting of H and methyl.

31. The compound according to claims 28 or 30, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the structure of U moiety is selected from the group consisting of:

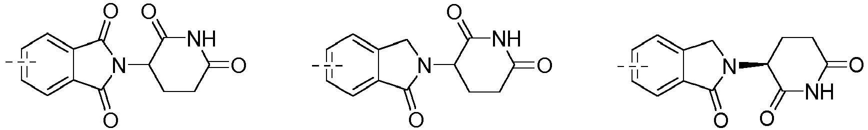

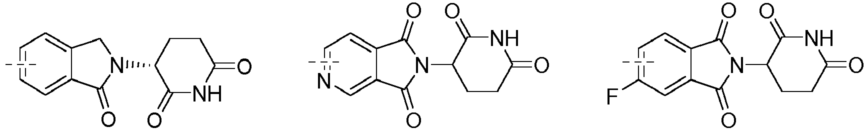

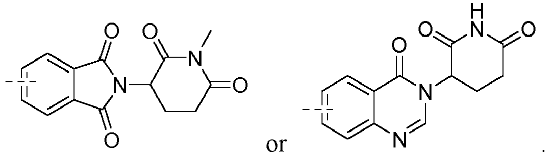

32. The compound according to any one of claims 1 to 31, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, **characterized in that** the compound is selected from the group consisting of:
| | | | |
|---|---|---|---|
| 1 | 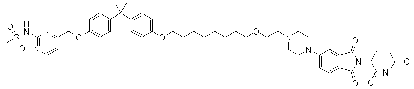 | 2 | 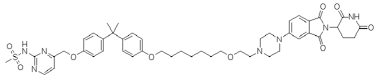 |
| 3 | 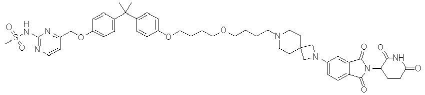 | 4 | 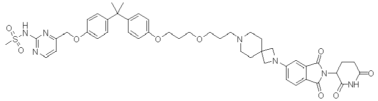 |
| 5 | 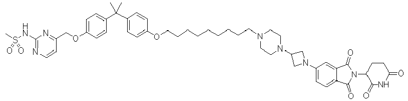 | 6 | 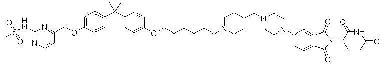 |
| 7 | 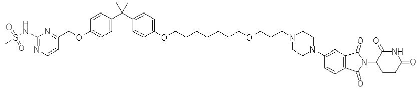 | 8 | 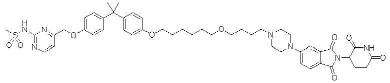 |
| 9 | 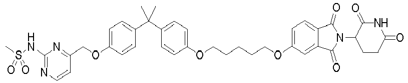 | 10 | 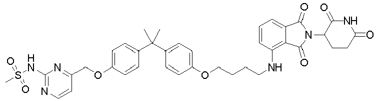 |
| 11 | 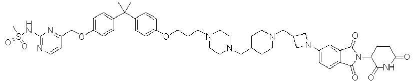 | 12 | 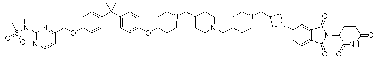 |
| 13 | 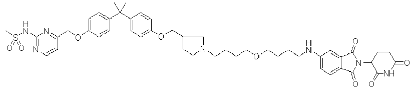 | 14 | 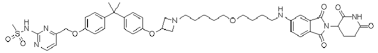 |
| 15 | 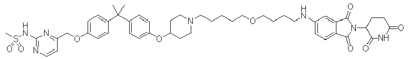 | 16 | 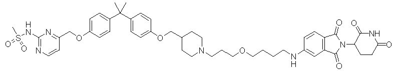 |
| 17 | 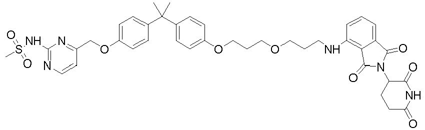 | 18 | 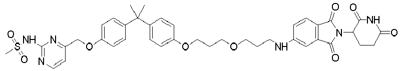 |
| 19 | 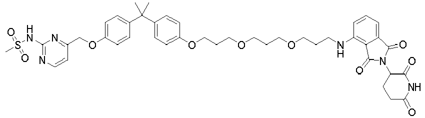 | 20 | 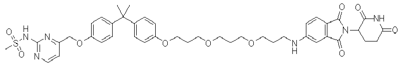 |
| 21 | 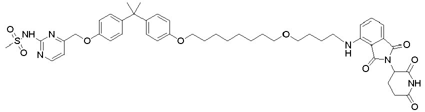 | 22 | 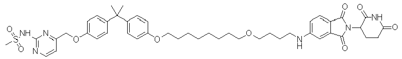 |
| 23 | 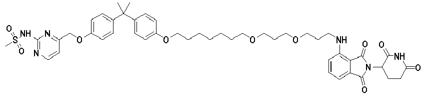 | 24 | 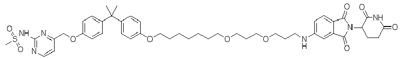 |
| 25 | 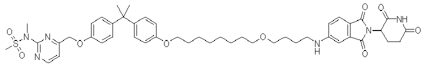 | 26 | 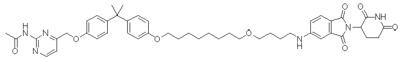 |
| 27 | 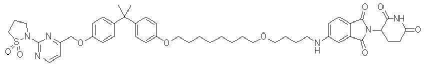 | 28 | 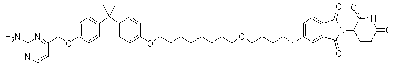 |
| 29 | 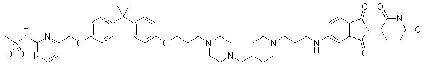 | 30 | 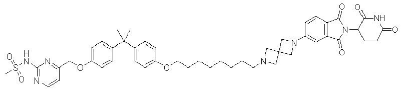 |
| 31 | 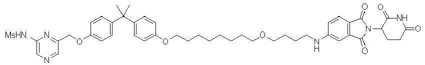 | 32 | 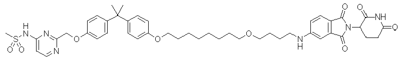 |
| 33 | 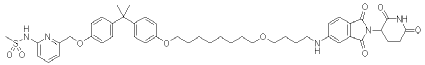 | 34 | 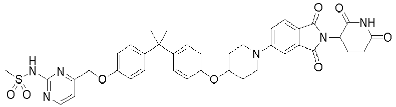 |
| 35 | 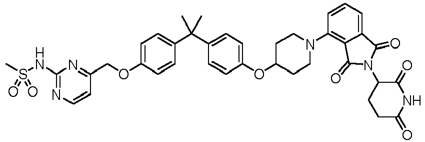 | 36 | 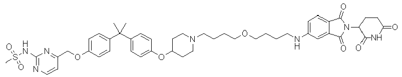 |
| 37 | 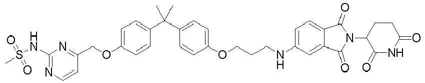 | 38 | 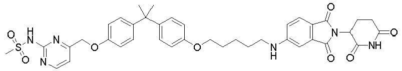 |
| 39 | 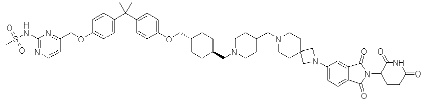 | 40 | 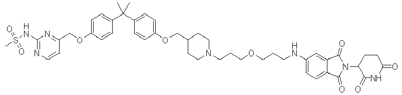 |
| 41 | 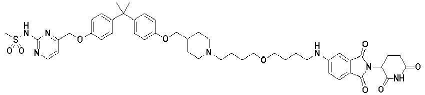 | 42 | 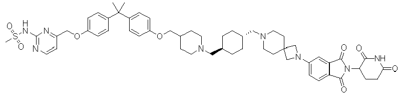 |
| 43 | 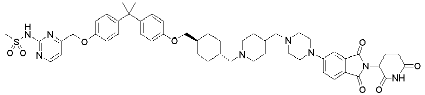 | 44 | 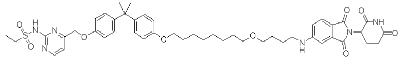 |
| 45 | 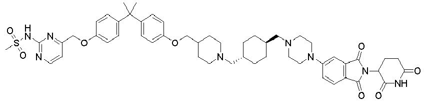 | 46 | 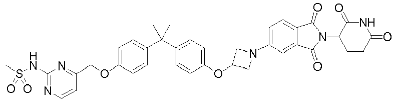 |
| 47 | 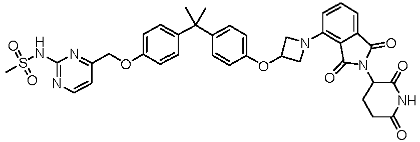 | 48 | 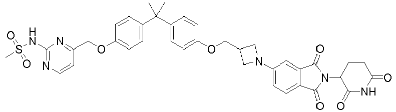 |
| 49 | 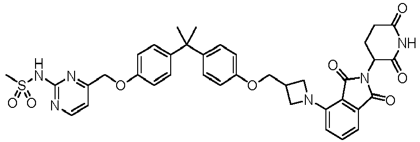 | 50 | 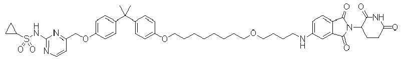 |
| 51 | 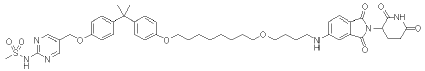 | 52 | 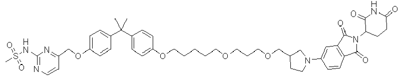 |
| 53 | 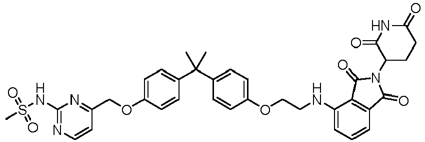 | 54 | 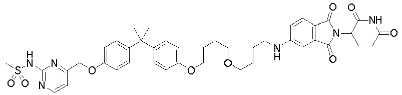 |
| 55 | 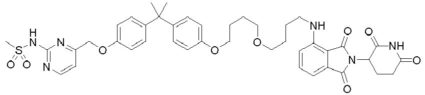 | 56 | 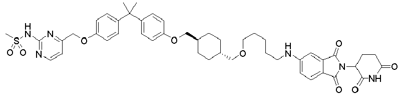 |
| 57 | 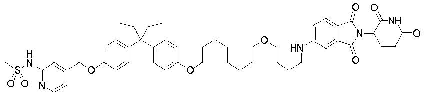 | 58 | 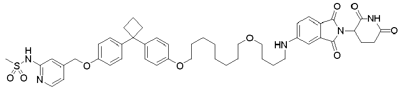 |
| 59 | 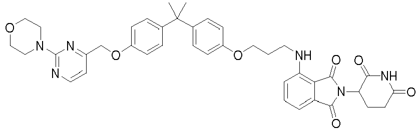 | 60 | 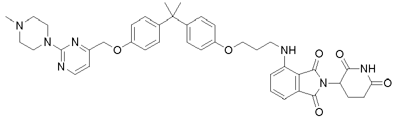 |
| 61 | 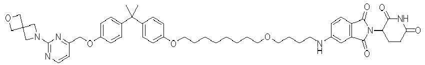 | 62 | 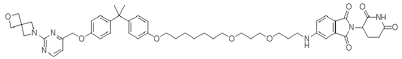 |
| 63 | 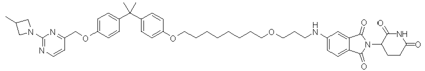 | 64 | 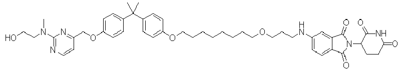 |
| 65 | 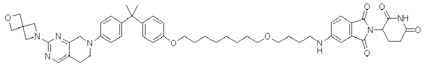 | 66 | 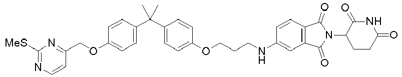 |
| 67 | 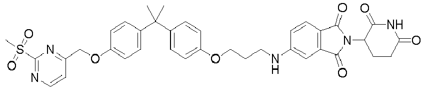 | 68 | 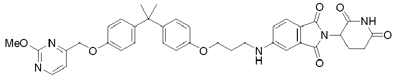 |
| 69 | 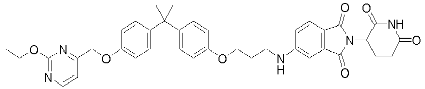 | 70 | 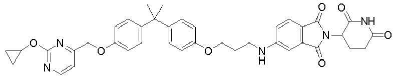 |
| 71 | 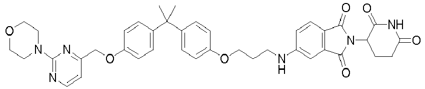 | 72 | 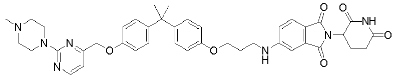 |
| 73 | 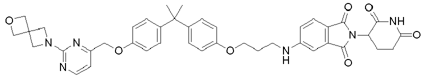 | 74 | 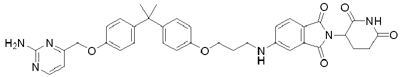 |
| 75 | 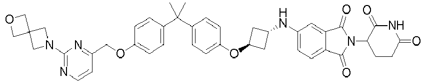 | 76 | 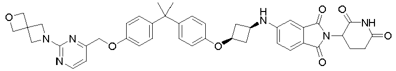 |
| 77 | 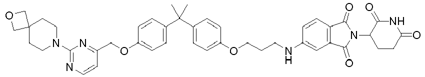 | 78 | 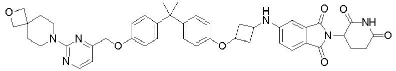 |
| 79 | 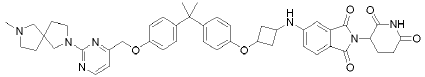 | 80 | 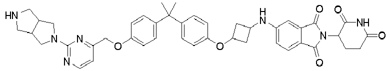 |
| 81 | 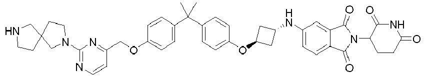 | 82 | 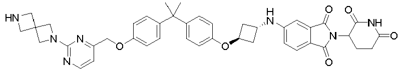 |
| 83 | 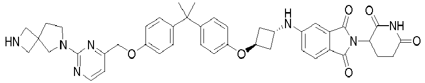 | 84 | 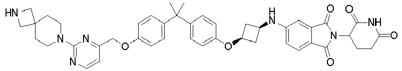 |
| 85 | 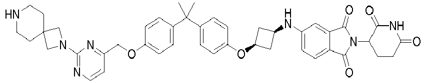 | 86 | 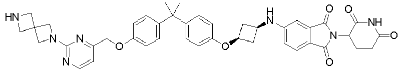 |
| 87 | 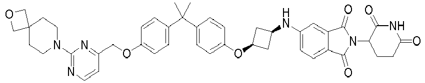 | 88 | 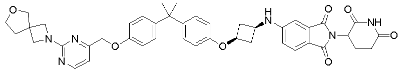 |
| 89 | 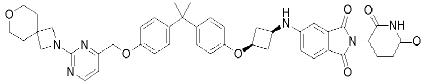 | 90 | 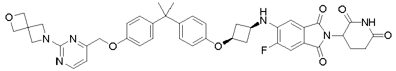 |
| 91 | 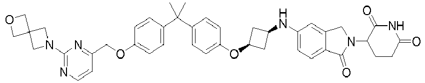 | 92 | 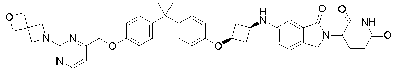 |
| 93 | 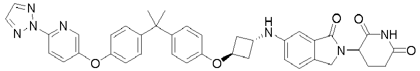 | 94 | 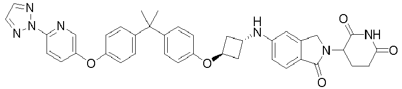 |
| 95 | 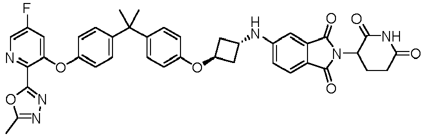 | 96 | 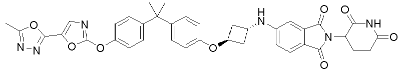 |
| 97 | 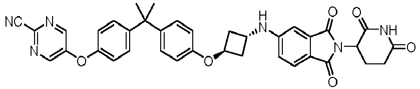 | 98 | 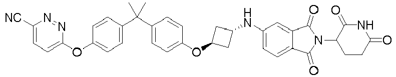 |
| 99 | 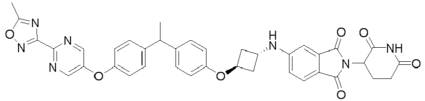 | 100 | 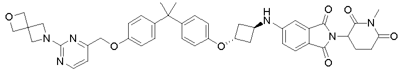 |
| 101 | 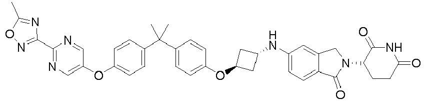 | 102 | 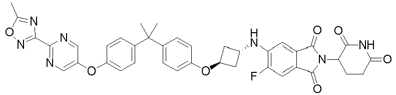 |
| 103 | 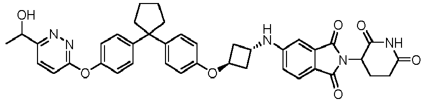 | 104 | 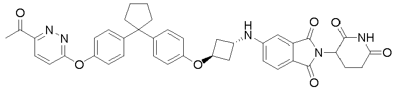 |
| 105 | 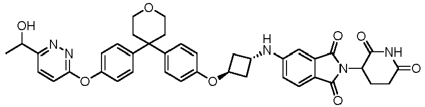 | 106 | 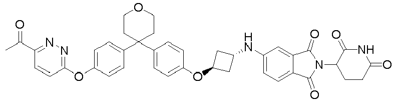 |
| 107 | 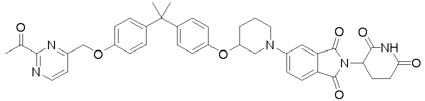 | 108 | 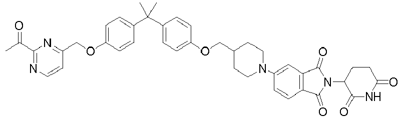 |
| 109 | 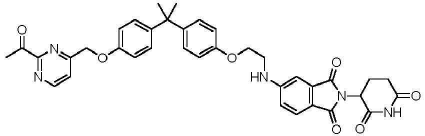 | 110 | 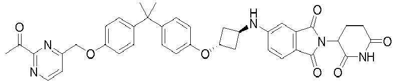 |
| 111 | 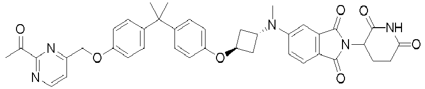 | 112 | 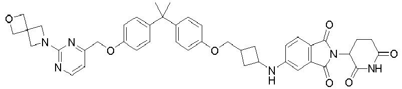 |
| 113 | 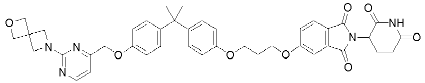 | 114 | 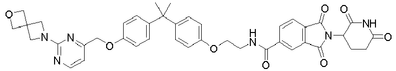 |
| 115 | 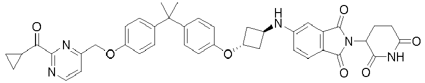 | 116 | 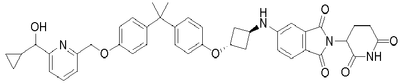 |
| 117 | 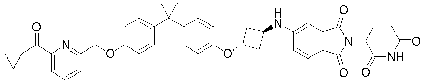 | 118 | 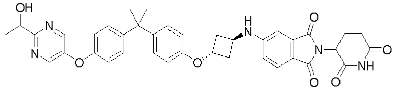 |
| 119 | 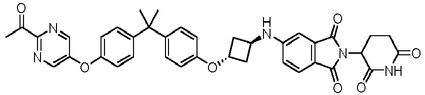 | 120 | 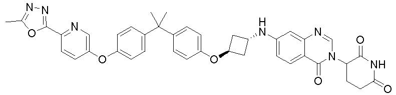 |
| | | 122 | 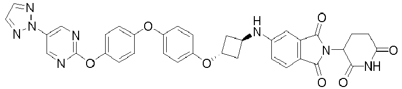 |
| 123 | 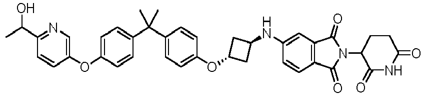 | 124 | 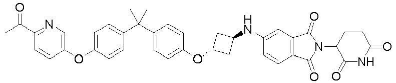 |
| 125 | 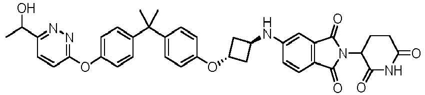 | 126 | 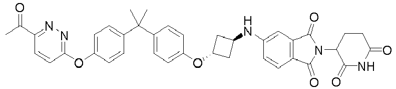 |
| 127 | 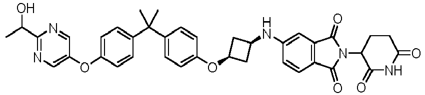 | 128 | 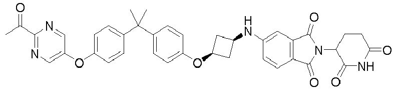 |
| 129 | 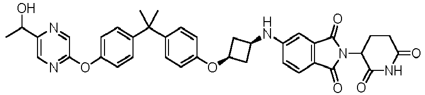 | 130 | 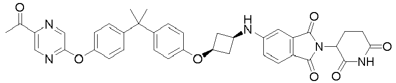 |
| 131 | 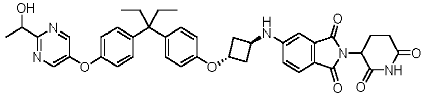 | 132 | 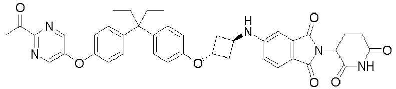 |
| 133 | 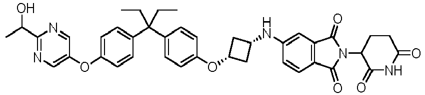 | 134 | 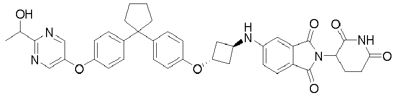 |
| 135 | 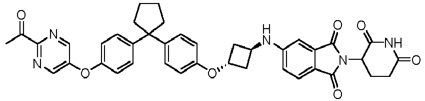 | 136 | 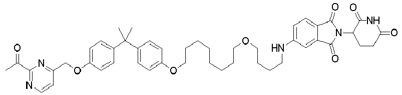 |
| 137 | 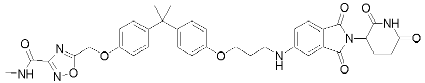 | 138 | 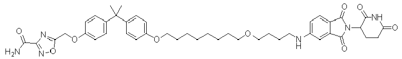 |
| 139 | 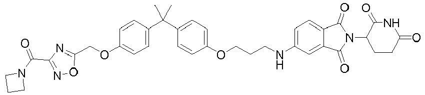 | 140 | 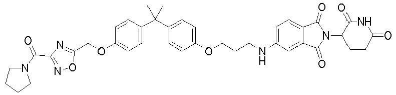 |
| 141 | 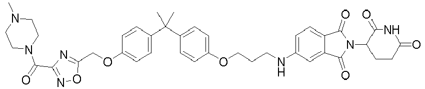 | 142 | 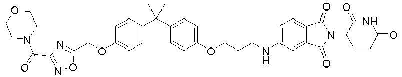 |
| 143 | 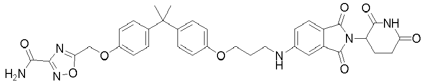 | 144 | 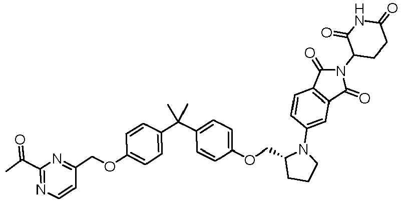 |
| 145 | 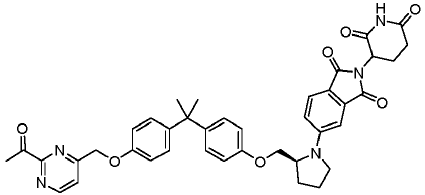 | 146 | 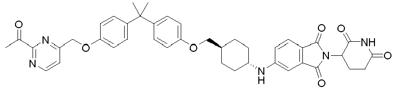 |
| 147 | 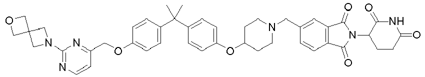 | 148 | 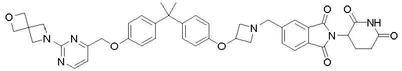 |
| 149 | 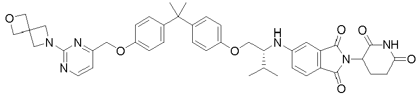 | 150 | 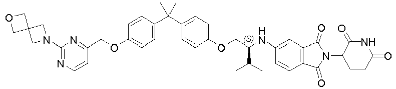 |
| 151 | 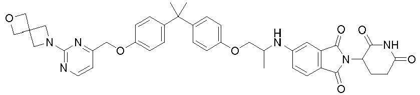 | 152 | 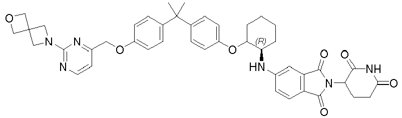 |
| 153 | 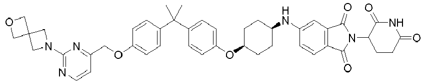 | 154 | 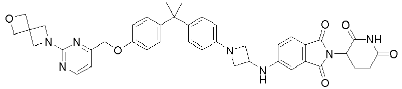 |
| 155 | 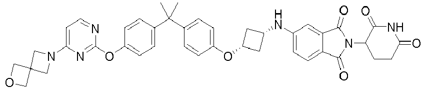 | 156 | 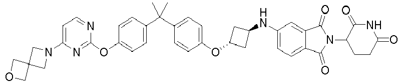 |
| 157 | 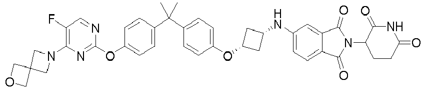 | 158 | 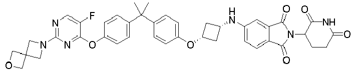 |
| 159 | 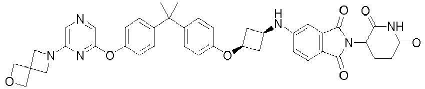 | 160 | 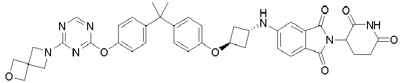 |
| 161 | 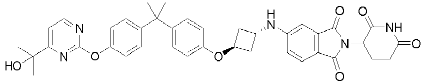 | 162 | 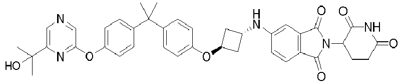 |
| 163 | 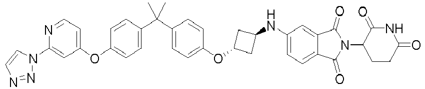 | 164 | 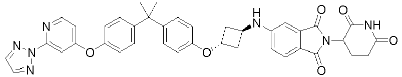 |
| 165 | 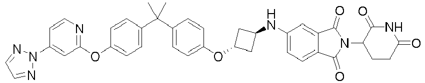 | 166 | 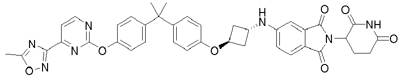 |
| 167 | 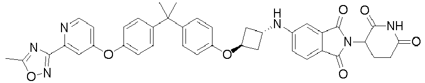 | 168 | 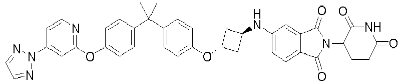 |
| 169 | 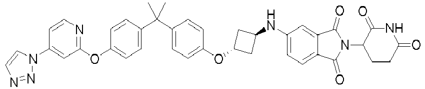 | 170 | 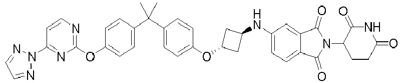 |
| 171 | 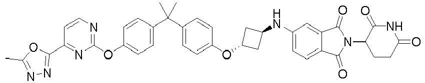 | 172 | 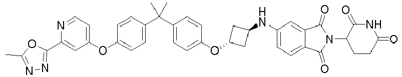 |
| 173 | 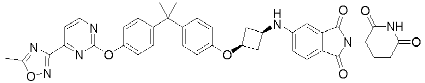 | 174 | 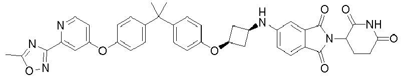 |
| 175 | 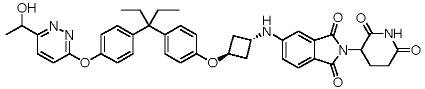 | 176 | 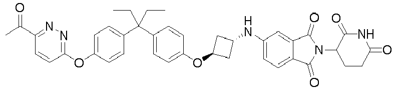 |
| 177 | 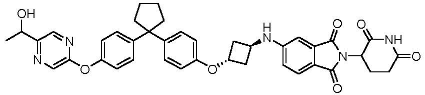 | 178 | 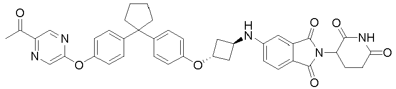 |
| 179 | 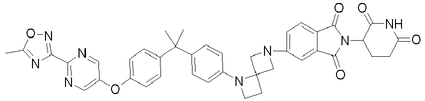 | 180 | 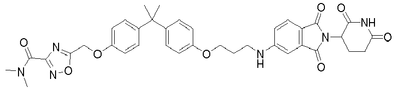 |
| 181 | 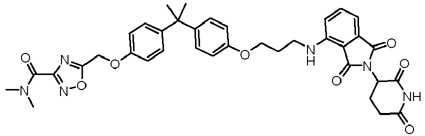 | 182 | 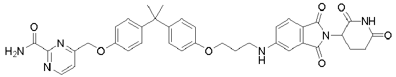 |
| 183 | 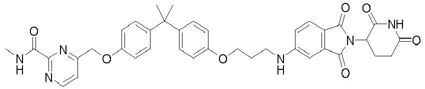 | 184 | 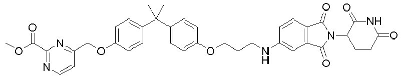 |
| 185 | 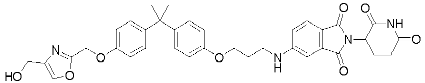 | 186 | 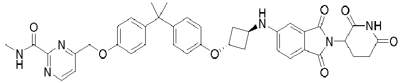 |
| 187 | 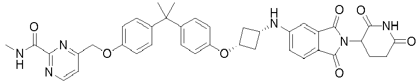 | 188 | 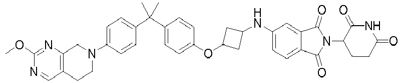 |
| 189 | 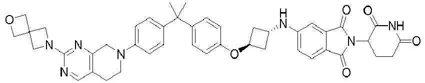 | 190 | 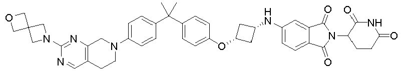 |
| 191 | 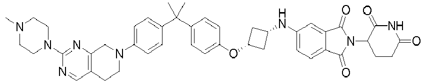 | 192 | 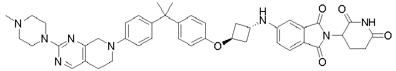 |
| 193 | 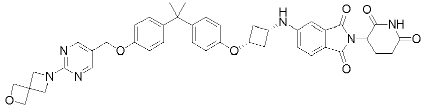 | 194 | 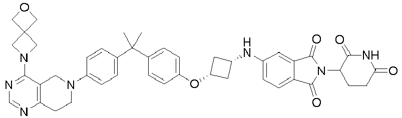 |
| 195 | 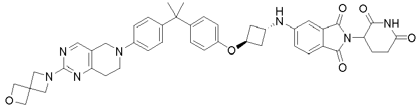 | 196 | 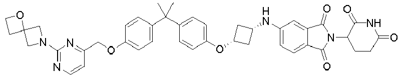 |
| 197 | 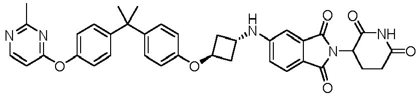 | 198 | 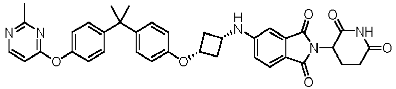 |
| 199 | 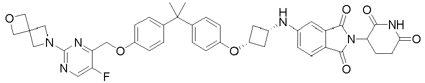 | 200 | 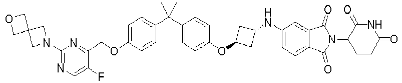 |
| 201 | 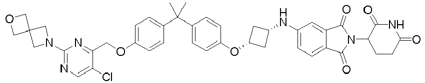 | 202 | 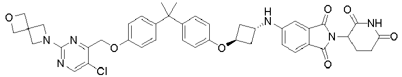 |
| 203 | 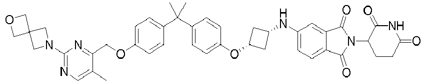 | 204 | 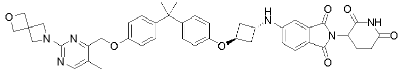 |
| 205 | 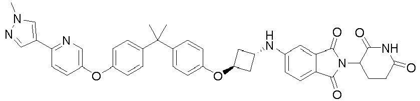 | 206 | 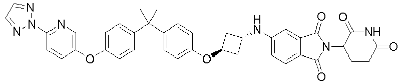 |
| 207 | 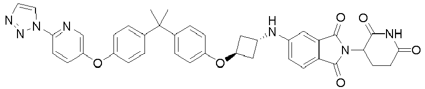 | 208 | 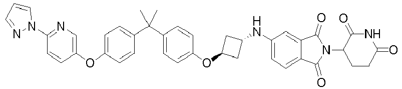 |
| 209 | 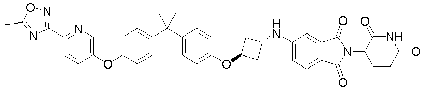 | 210 | 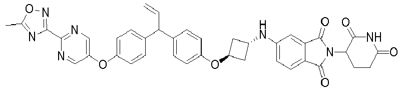 |
| 211 | 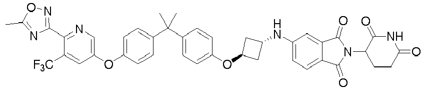 | 212 | 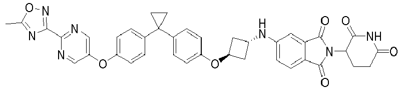 |
| 213 | 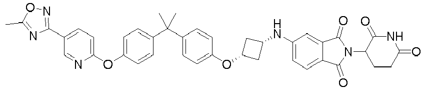 | 214 | 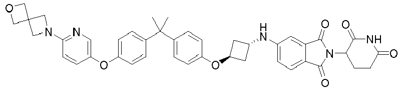 |
| 215 | 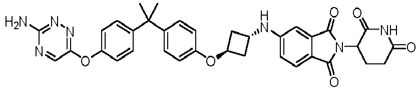 | 216 | 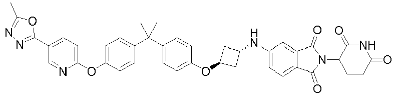 |
| 217 | 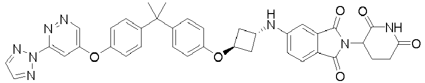 | 218 | 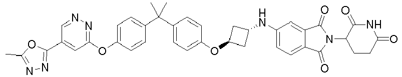 |
| 219 | 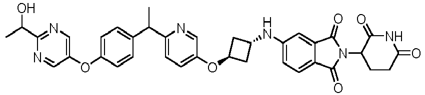 | 220 | 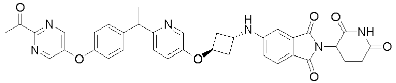 |
| 221 | 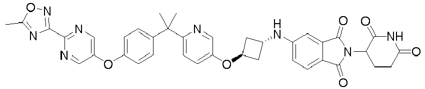 | 222 | 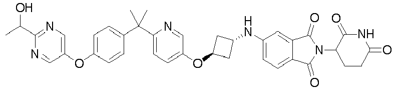 |
| 223 | 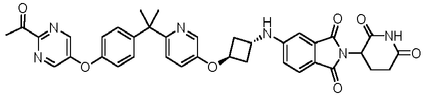 | 224 | 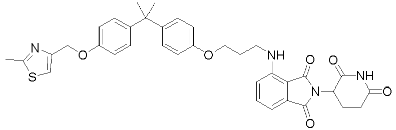 |
| 225 | 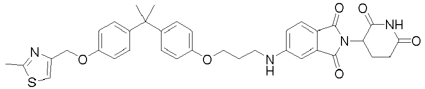 | 226 | 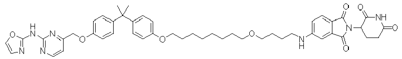 |
| 227 | 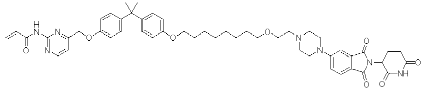 | 228 | 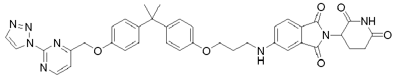 |
| 229 | 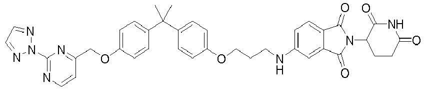 | 230 | 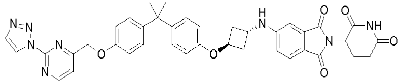 |
| 231 | 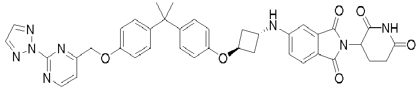 | 232 | 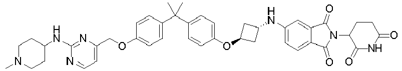 |
| 233 | 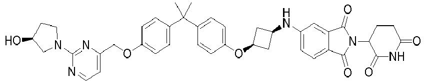 | 234 | 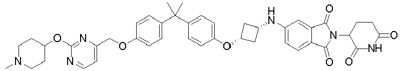 |
| 235 | 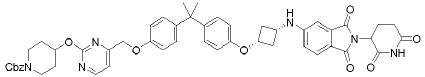 | 236 | 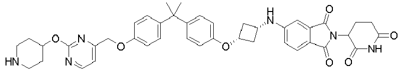 |
| 237 | 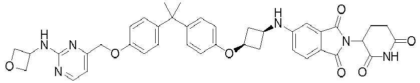 | 238 | 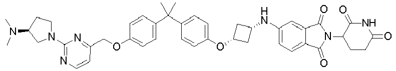 |
| 239 | 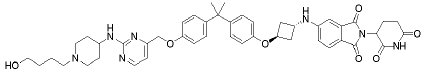 | 240 | 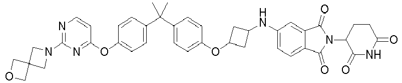 |
| 241 | 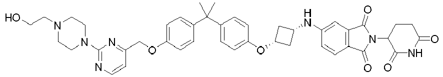 | 242 | 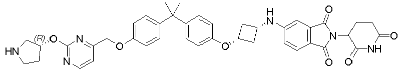 |
| 243 | 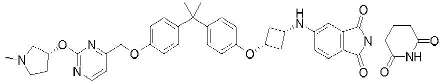 | 244 | 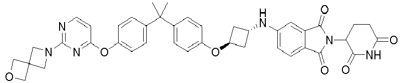 |
| 245 | 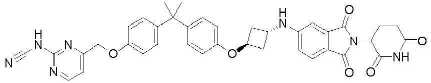 | 246 | 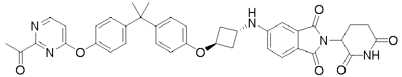 |
| 247 | 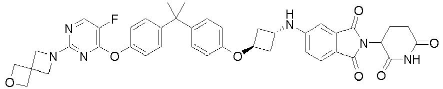 | 248 | 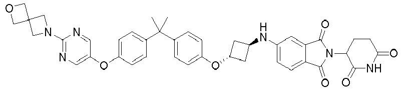 |
| 249 | 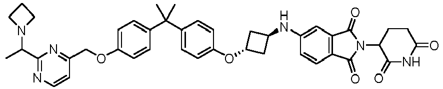 | 250 | 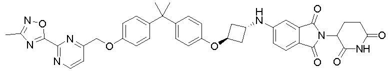 |
| 251 | 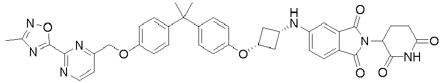 | 252 | 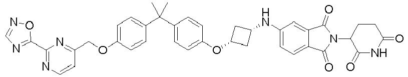 |
| 253 | 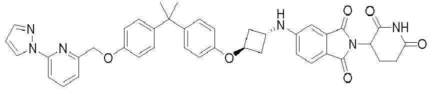 | 254 | 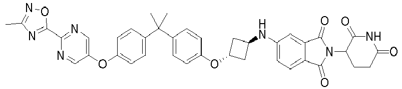 |
| 255 | 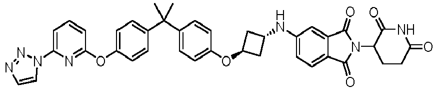 | 256 | 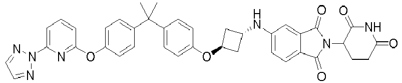 |
| 257 | 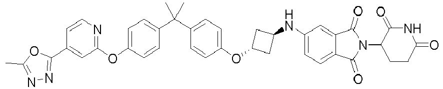 | 258 | 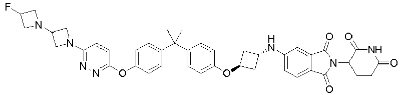 |
| 259 | 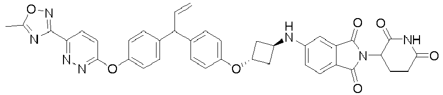 | 260 | 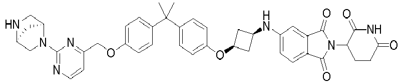 |
| 261 | 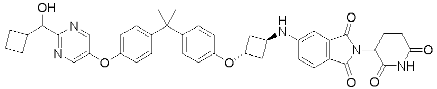 | 262 | 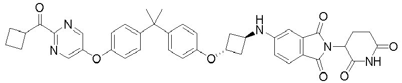 |
| 263 | 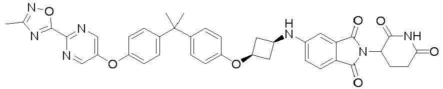 | 264 | 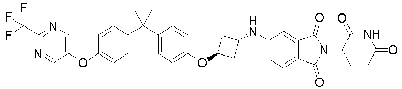 |
| 265 | 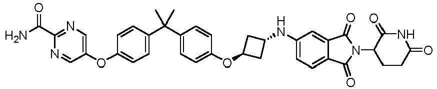 | 266 | 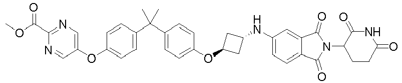 |
| 267 | 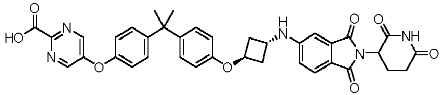 | 268 | 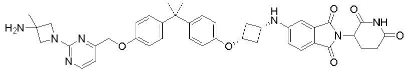 |
| 269 | 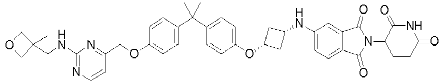 | 270 | 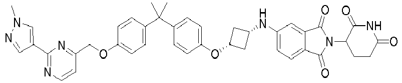 |
| 271 | 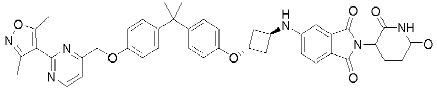 | 272 | 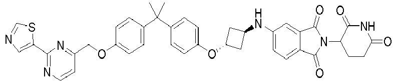 |
| 273 | 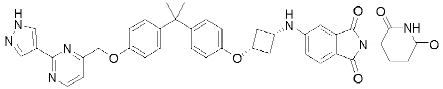 | 274 | 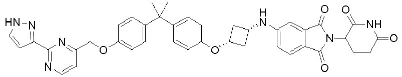 |
| 275 | 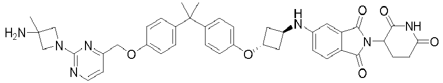 | 276 | 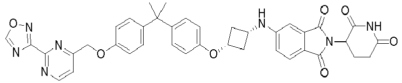 |
| 277 | 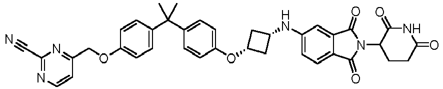 | 278 | 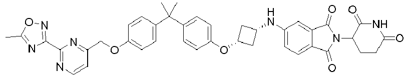 |
| 279 | 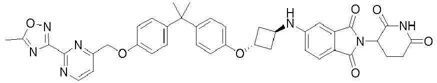 | 280 | 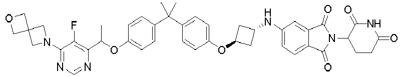 |
| 281 | 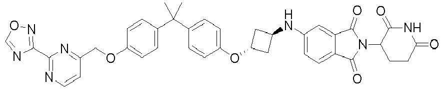 | 282 | 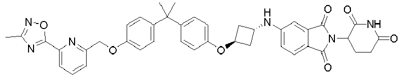 |
| 283 | 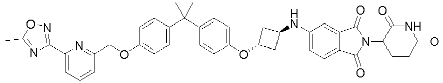 | 284 | 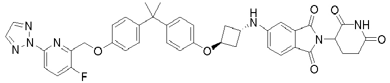 |
| 285 | 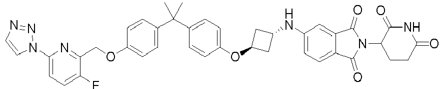 | 286 | 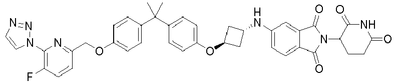 |
| 287 | 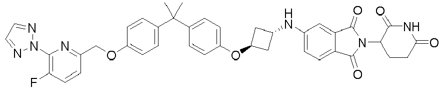 | 288 | 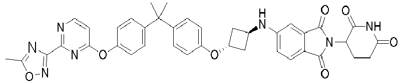 |
| 289 | 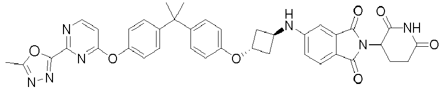 | 290 | 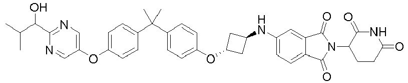 |
| 291 | 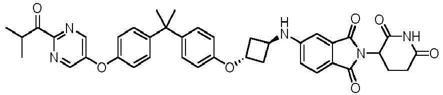 | 292 | 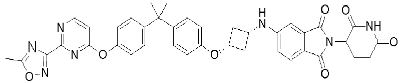 |
| 293 | 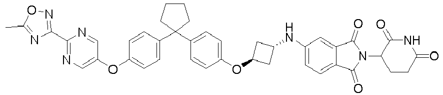 | 294 | 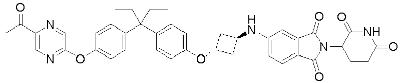 |
| 295 | 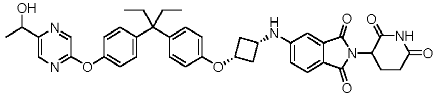 | 296 | 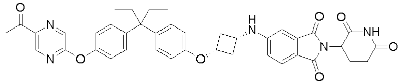 |
| 297 | 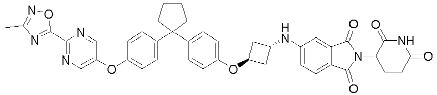 | 298 | 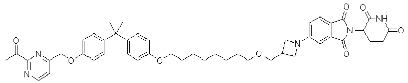 |
| 299 | 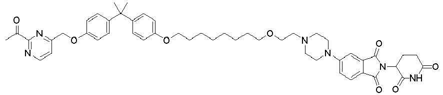 | 300 | 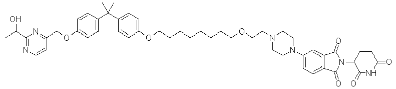 |
| 301 | 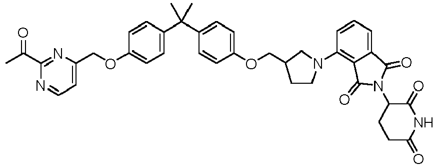 | 302 | 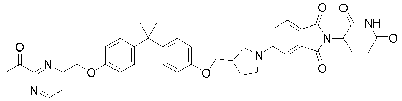 |
| 303 | 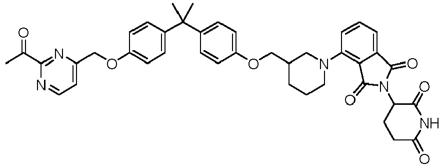 | 304 | 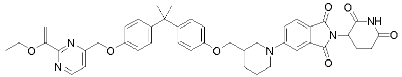 |
| 305 | 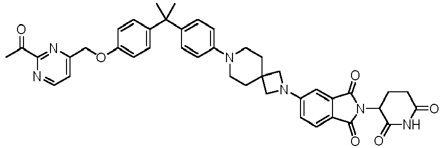 | 306 | 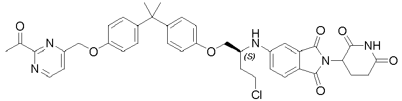 |
| 307 | 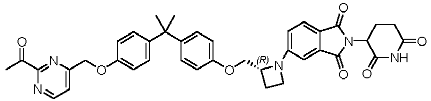 | 308 | 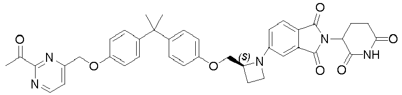 |
| 309 | 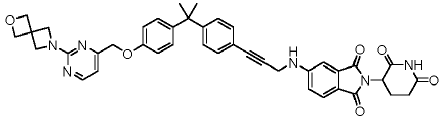 | 310 | 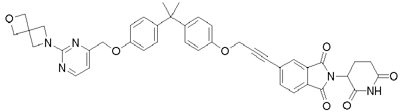 |
| 311 | 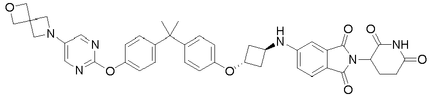 | 312 | 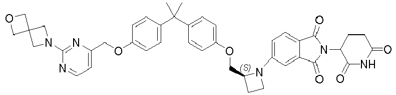 |
| 313 | 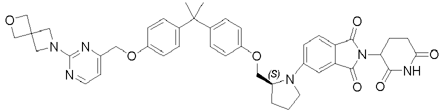 | 314 | 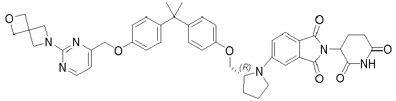 |
| 315 | 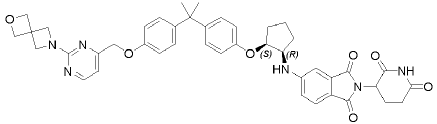 | 316 | 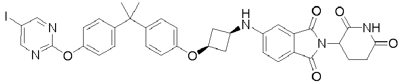 |
| 317 | 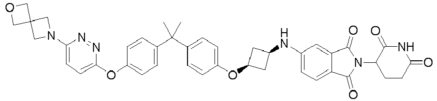 | 318 | 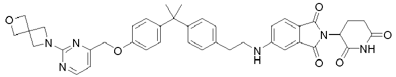 |
| 319 | 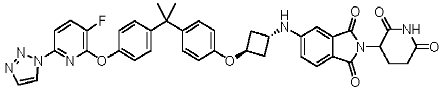 | 320 | 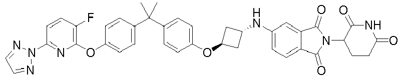 |
| 321 | 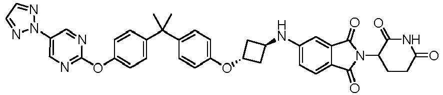 | 322 | 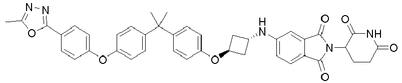 |
| 323 | 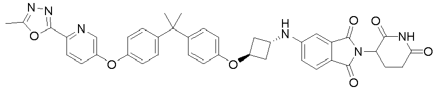 | 324 | 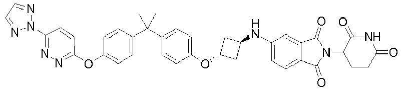 |
| 325 | 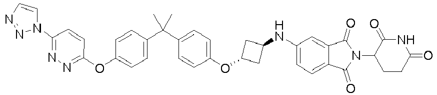 | 326 | 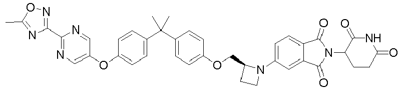 |
| 327 | 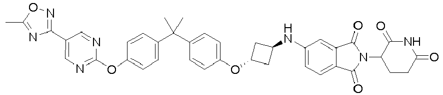 | 328 | 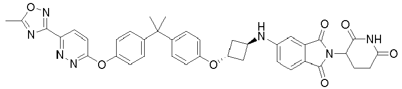 |
| 329 | 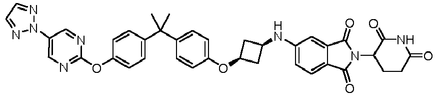 | 330 | 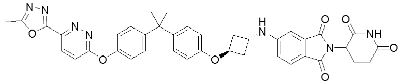 |
| 331 | 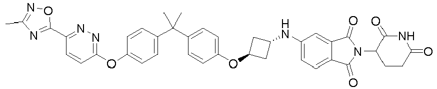 | 332 | 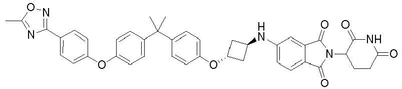 |
| 333 | 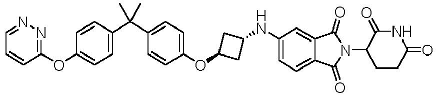 | 334 | 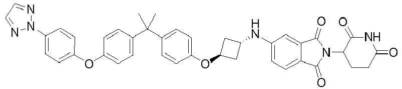 |
| 335 | 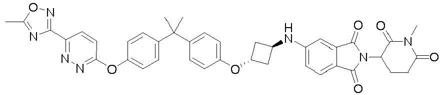 | 336 | 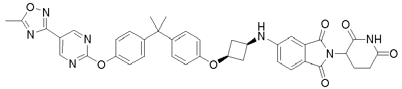 |
| 337 | 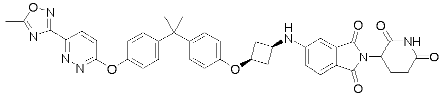 | 338 | 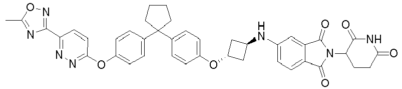 |
| 339 | 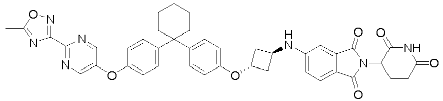 | 340 | 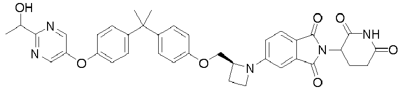 |
| 341 | 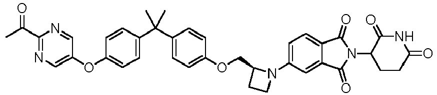 | 342 | 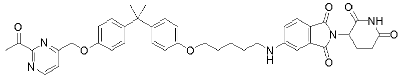 |
| 343 | 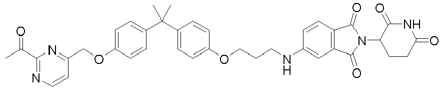 | 344 | 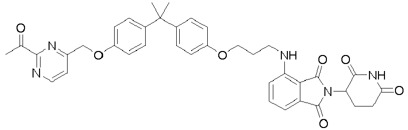 |
| 345 | 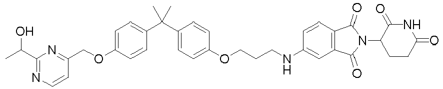 | 346 | 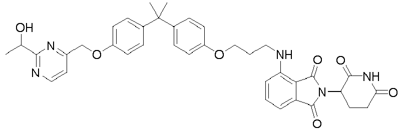 |
| 347 | 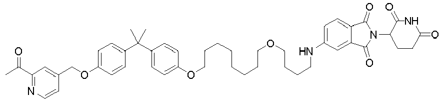 | 348 | 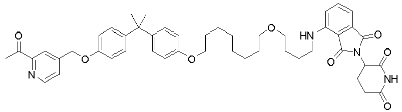 |
| 349 | 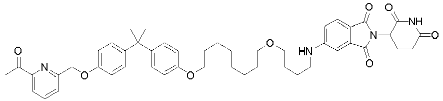 | 350 | 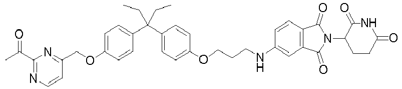 |
| 351 | 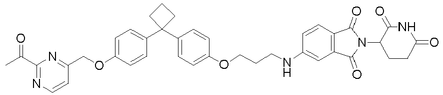 | 352 | 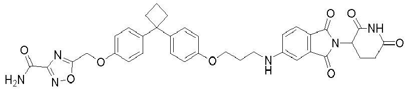 |
| 353 | 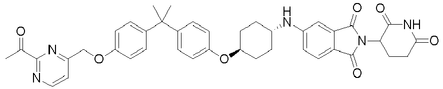 | 354 | 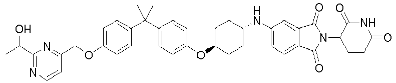 |
| 355 | 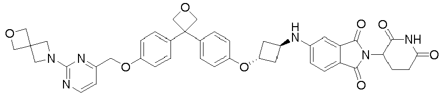 | 356 | 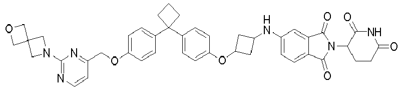 |
| 357 | 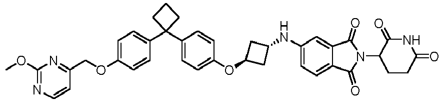 | 358 | 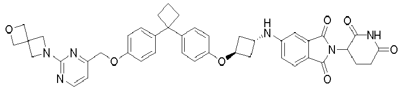 |
| 359 | 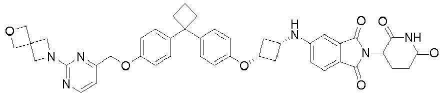 | 360 | 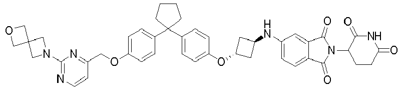 |
| 361 | 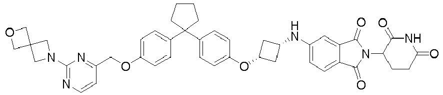 | 362 | 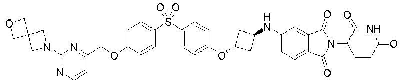 |
| 363 | 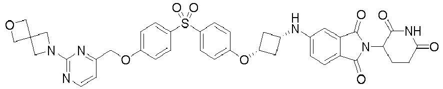 | 364 | 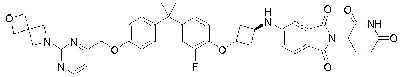 |
| 365 | 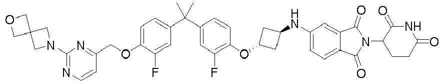 | 366 | 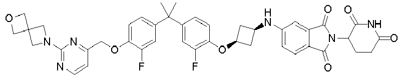 |
| 367 | 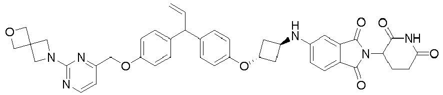 | 368 | 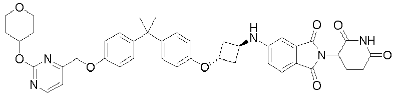 |
| 369 | 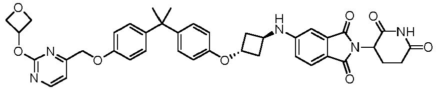 | 370 | 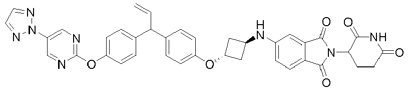 |
| 371 | 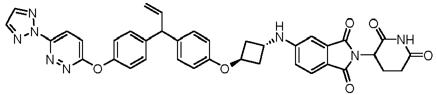 | 372 | 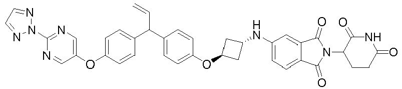 |
| 373 | 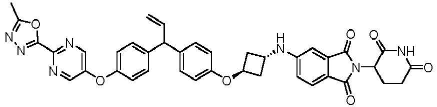 | 374 | 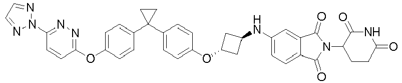 |
| 375 | 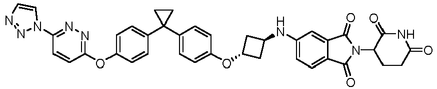 | 376 | 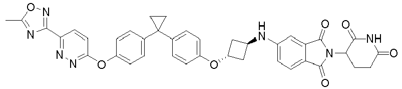 |
| 377 | 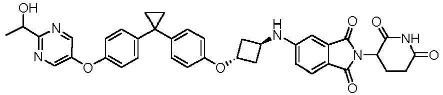 | 378 | 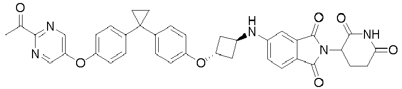 |
| 379 | 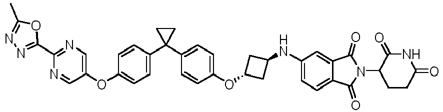 | 380 | 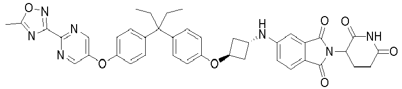 |
| 381 | 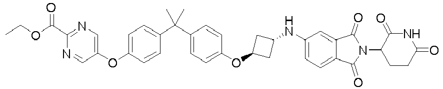 | 382 | 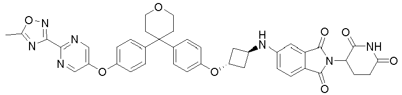 |
| 383 | 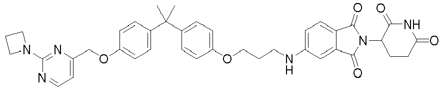 | 384 | 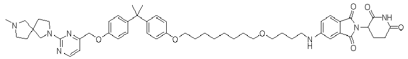 |
| 385 | 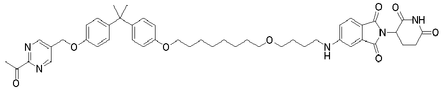 | 386 | 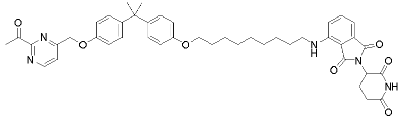 |
| 387 | 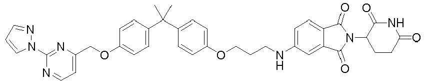 | 388 | 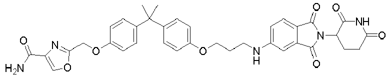 |
| 389 | 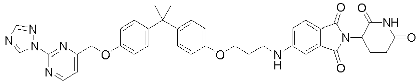 | 390 | 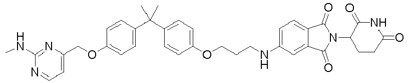 |
| 391 | 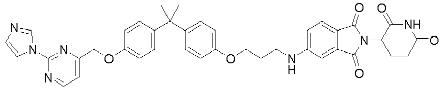 | 392 | 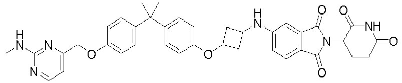 |
| 393 | 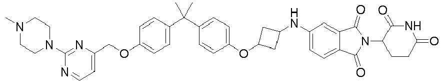 | 394 | 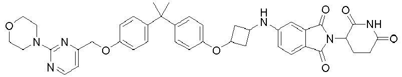 |
| 395 | 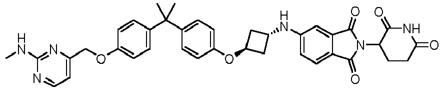 | 396 | 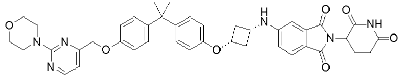 |
| 397 | 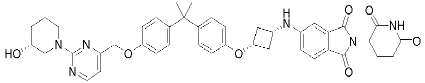 | 398 | 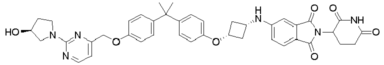 |
| 399 | 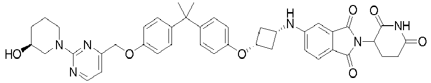 | 400 | 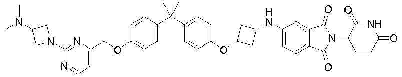 |
| 401 | 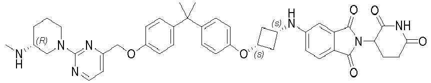 | 402 | 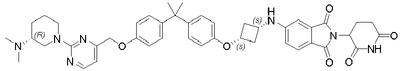 |
| 403 | 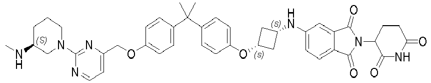 | 404 | 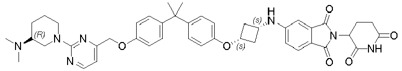 |
| 405 | 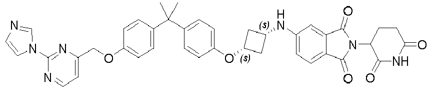 | 406 | 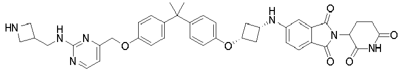 |
| 407 | 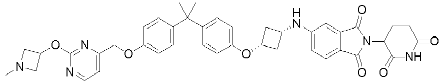 | 408 | 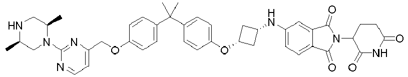 |
| 409 | 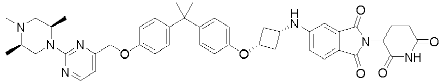 | 410 | 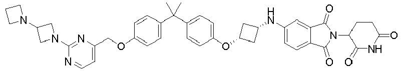 |
| 411 | 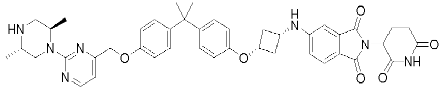 | 412 | 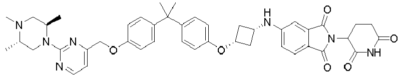 |
| 413 | 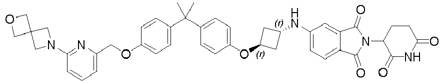 | 414 | 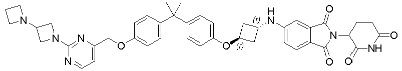 |
| 415 | 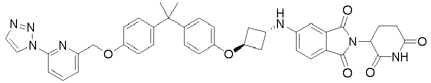 | 416 | 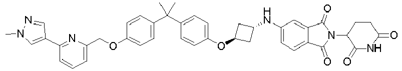 |
| 417 | 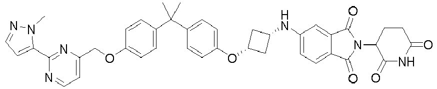 | 418 | 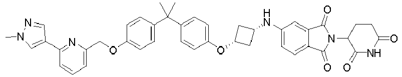 |
| 419 | 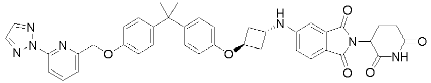 | 420 | 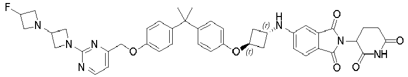 |
| 421 | 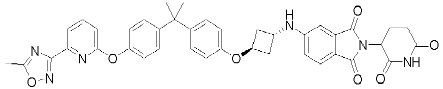 | 422 | 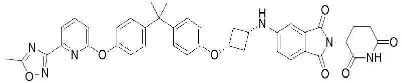 |
| 423 | 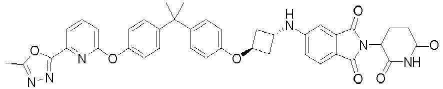 | 424 | 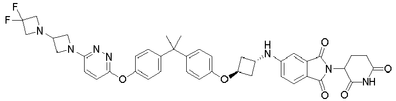 |
| 425 | 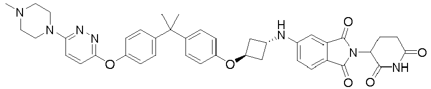 | 426 | 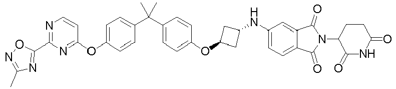 |
| 427 | 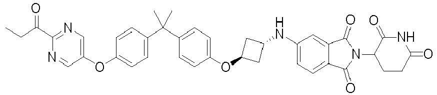 | 428 | 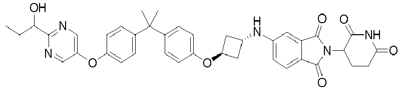 |
| 429 | 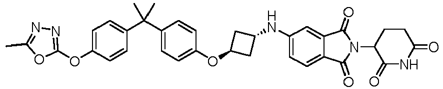 | 430 | 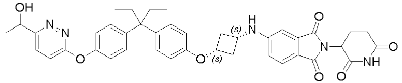 |
| 431 | 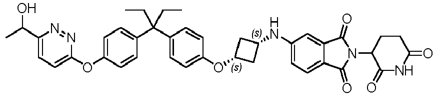 | 432 | 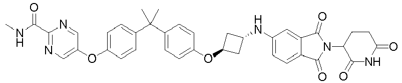 |
| 433 | 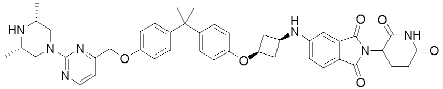 | 434 | 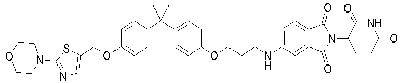 |
| 435 | 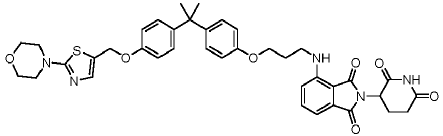 | 436 | 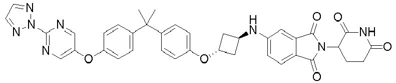 |
| 437 | 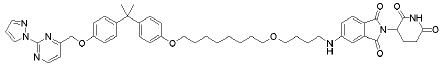 | 438 | 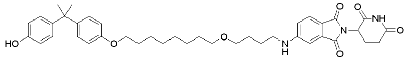 |
| 439 | 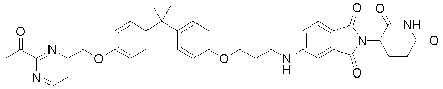 | 440 | 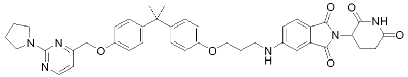 |
| 441 | 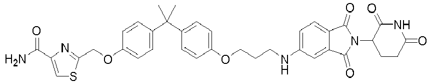 | 442 | 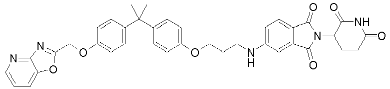 |
| 443 | 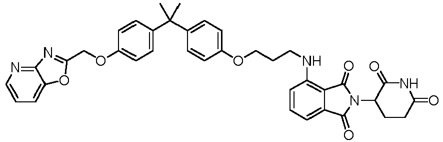 | 444 | 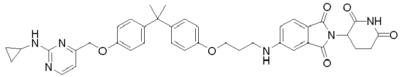 |
| 445 | 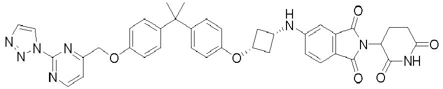 | 446 | 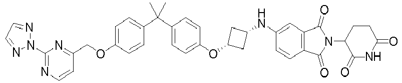 |
| 447 | 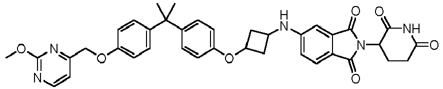 | 448 | 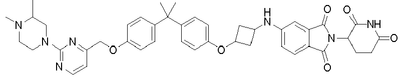 |
| 449 | 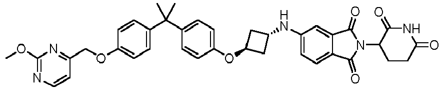 | 450 | 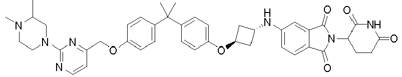 |
| 451 | 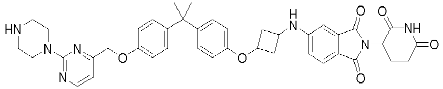 | 452 | 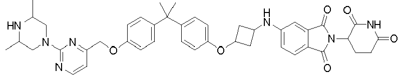 |
| 453 | 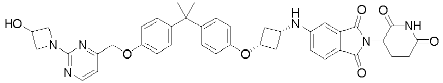 | 454 | 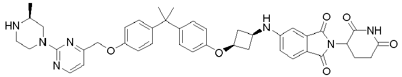 |
| 455 | 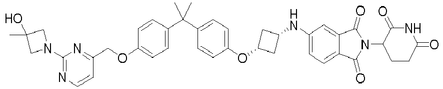 | 456 | 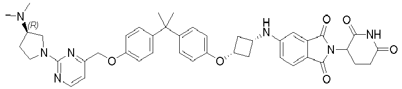 |
| 457 | 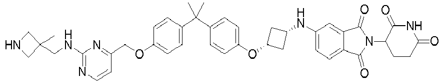 | 458 | 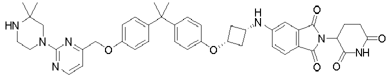 |
| 459 | 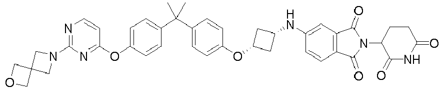 | 460 | 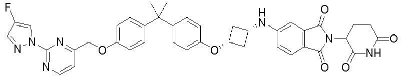 |
| 461 | 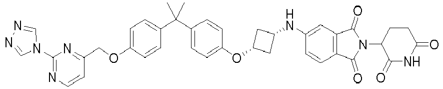 | 462 | 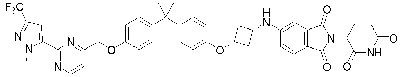 |
| 463 | 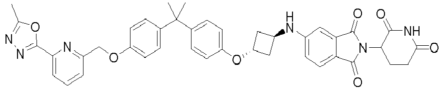 | 464 | 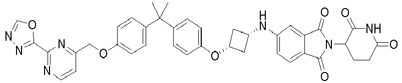 |
| 465 | 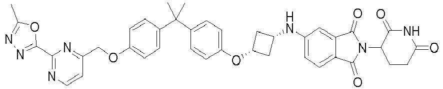 | 466 | 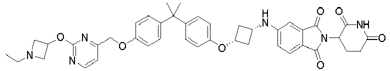 |
| 467 | 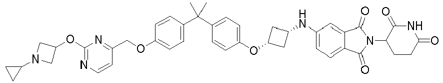 | 468 | 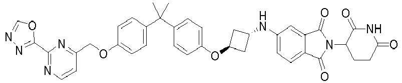 |
| 469 | 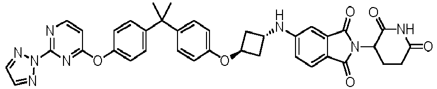 | 470 | 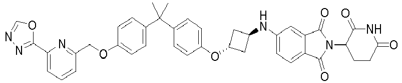 |
| 471 | 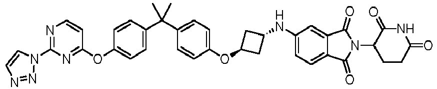 | 472 | 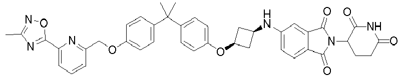 |
| 473 | 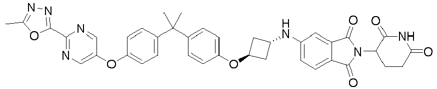 | 474 | 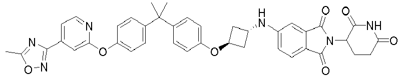 |
| 475 | 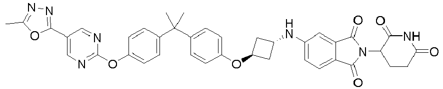 | 476 | 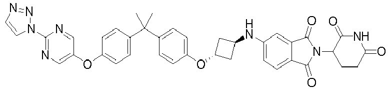 |
| 477 | 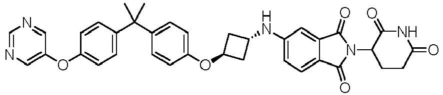 | 478 | 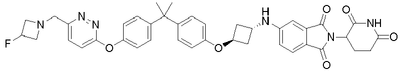 |
| 479 | 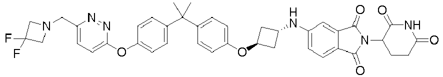 | 480 | 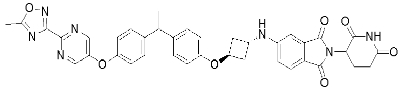 |
| 481 | 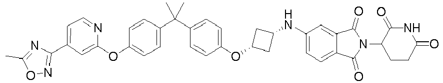 | 482 | 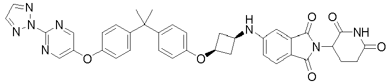 |
| 483 | 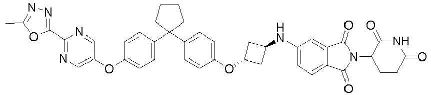 | 484 | 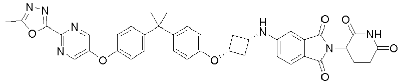 |
| 485 | 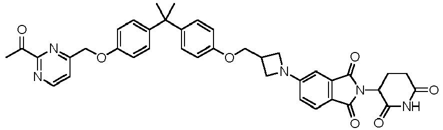 | 486 | 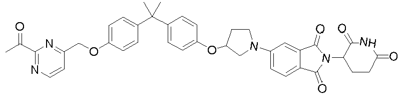 |
| 487 | 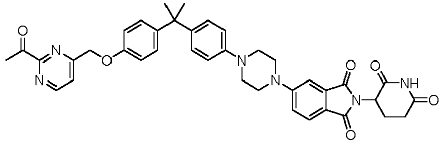 | 488 | 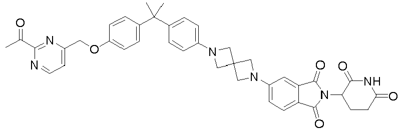 |
| 489 | 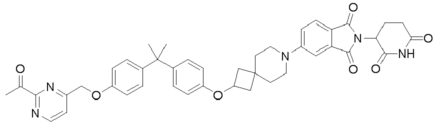 | 490 | 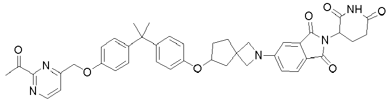 |
| 491 | 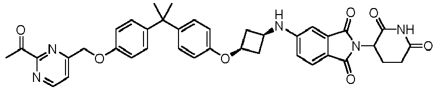 | 492 | 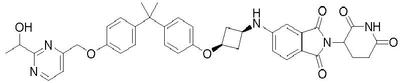 |
| 493 | 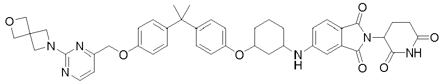 | 494 | 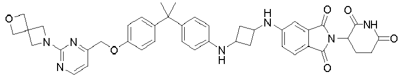 |
| 495 | 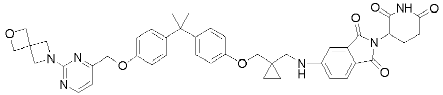 | 496 | 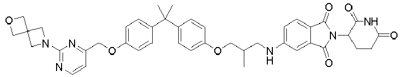 |
| 497 | 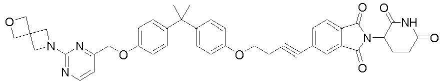 | 498 | 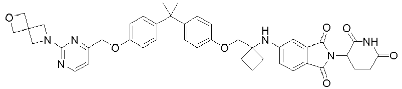 |
| 499 | 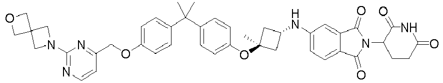 | 500 | 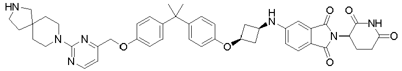 |
| 501 | 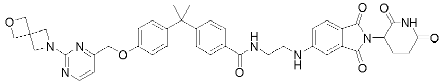 | 502 | 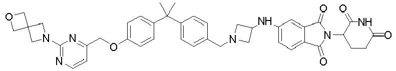 |
| 503 | 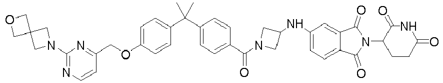 | 504 | 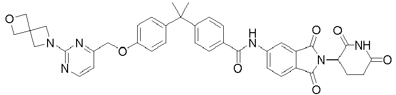 |
| 505 | 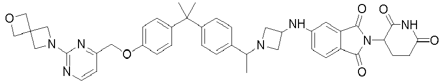 | 506 | 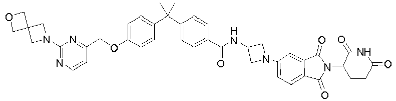 |
| 507 | 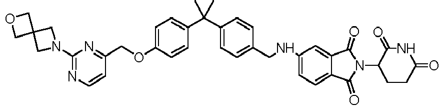 | 508 | 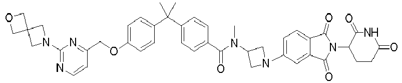 |
| 509 | 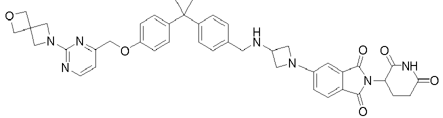 | | |
| 511 | 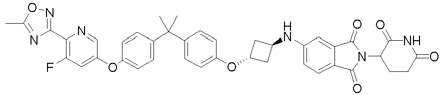 | 512 | 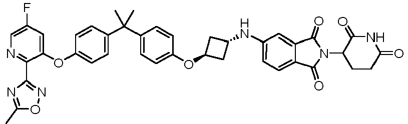 |
| 513 | 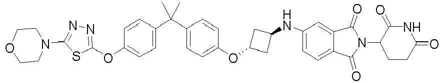 | 514 | 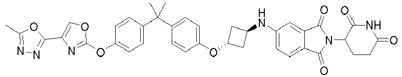 |
| 515 | 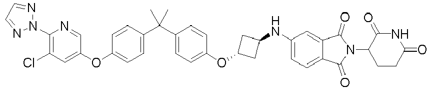 | 516 | 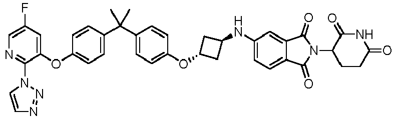 |
| 517 | 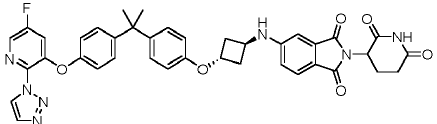 | 518 | 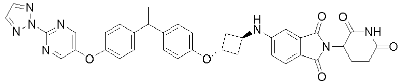 |
| 519 | 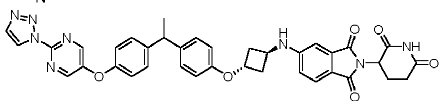 | 520 | 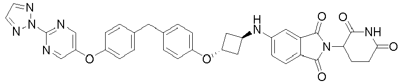 |
| 521 | 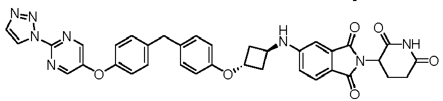 | 522 | 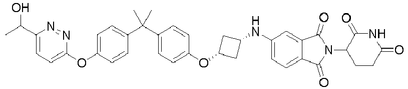 |
| 523 | 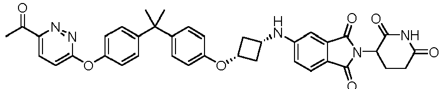 | 524 | 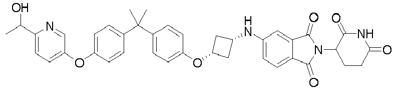 |
| 525 | 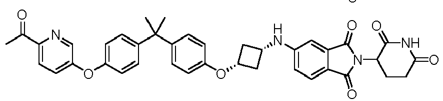 | 526 | 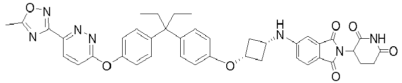 |
| 527 | 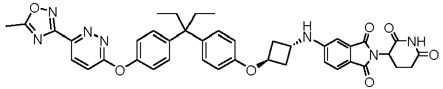 | 528 | 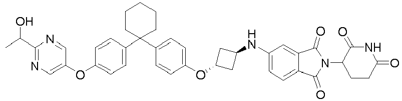 |
| 529 | 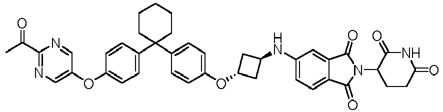 | 530 | 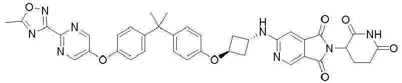 |
| 531 | 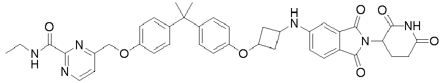 | 532 | 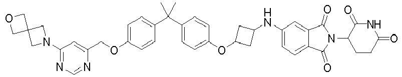 |
| 533 | 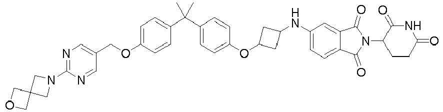 | 534 | 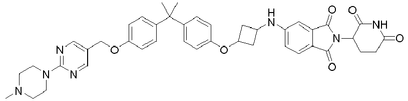 |
| 535 | 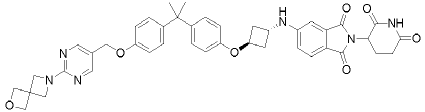 | 536 | 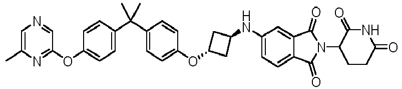 |
| 537 | 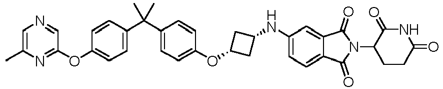 | 538 | 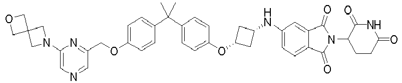 |
| 539 | 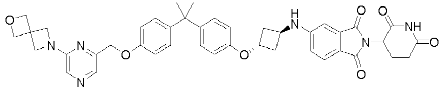 | 540 | 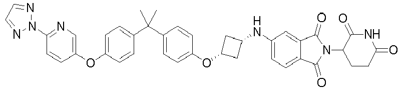 |
| 541 | 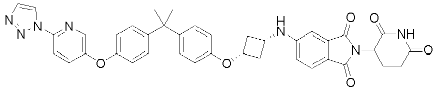 | 542 | 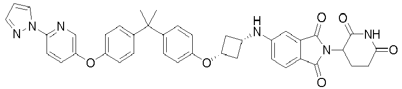 |
| 543 | 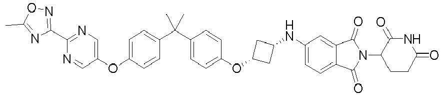 | 544 | 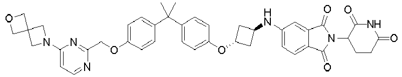 |
| 545 | 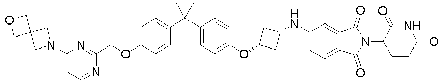 | 546 | 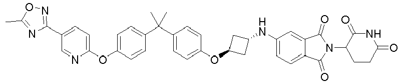 |
| 547 | 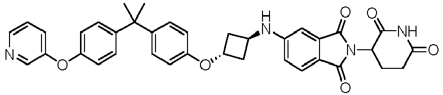 | 548 | 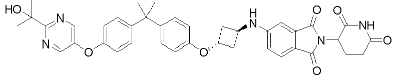 |
| 549 | 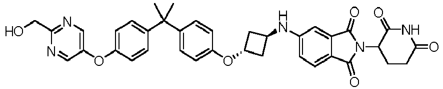 | 550 | 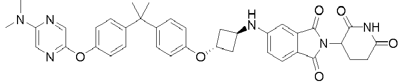 |
| 551 | 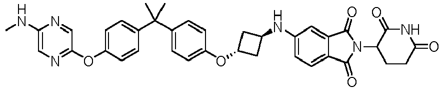 | 552 | 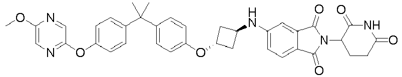 |
| 553 | 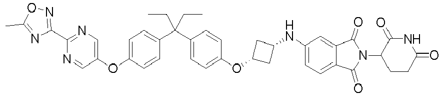 | 554 | 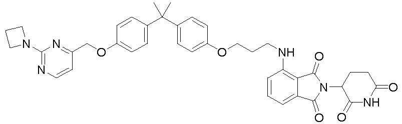 |
| 555 | 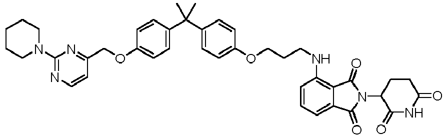 | | |
| 557 | 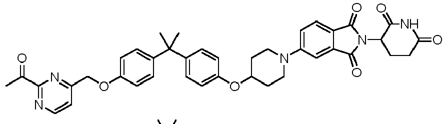 | 558 | 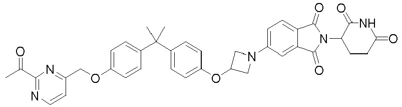 |
| 559 | 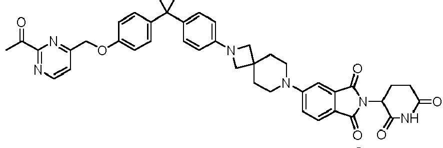 | 560 | 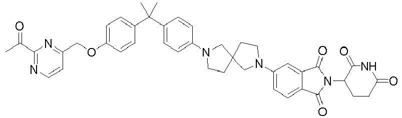 |
| 561 | 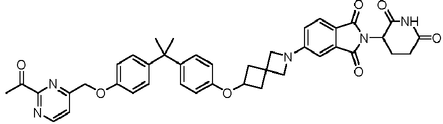 | 562 | 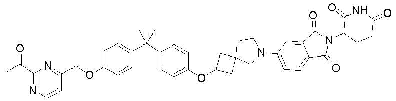 |
| 563 | 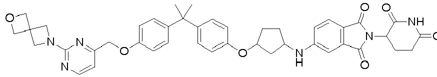 | 564 | 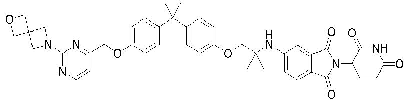 |
| 565 | 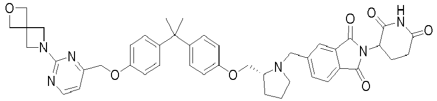 | 566 | 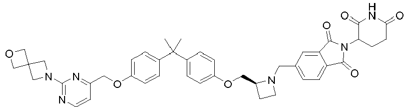 |
| 567 | 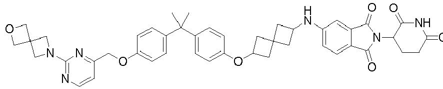 | 568 | 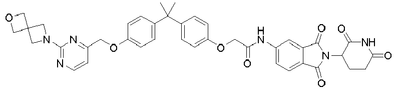 |
| 569 | 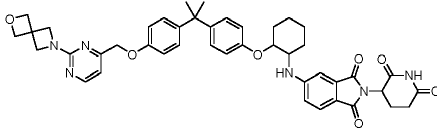 | 570 | 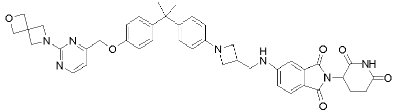 |
| 571 | 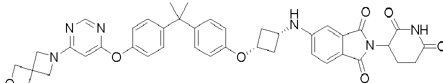 | 572 | 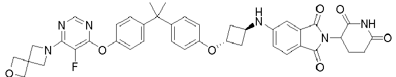 |
| 573 | 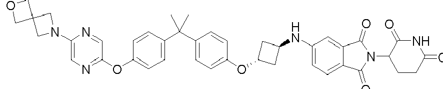 | 574 | 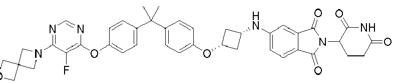 |
| 575 | 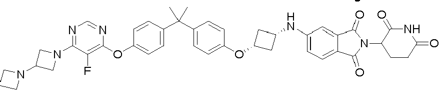 | 576 | 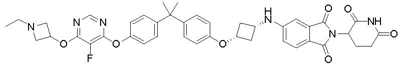 |
| 577 | 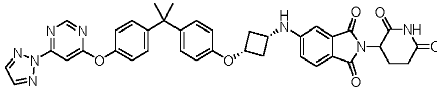 | 578 | 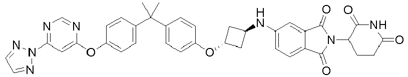 |
| 579 | 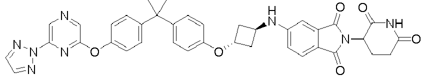 | 580 | 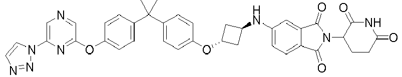 |
| 581 | 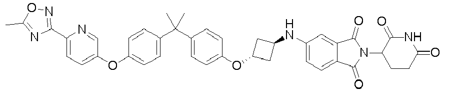 | 582 | 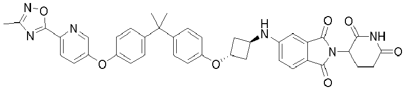 |
| 583 | 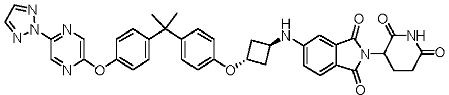 | 584 | 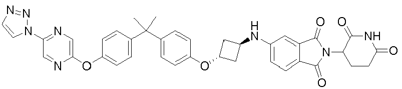 |
| 585 | 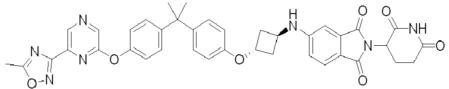 | 586 | 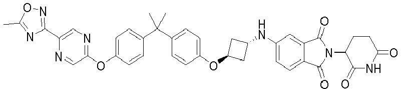 |
| 587 | 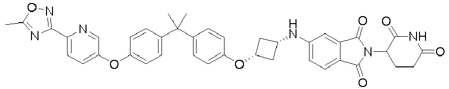 | 588 | 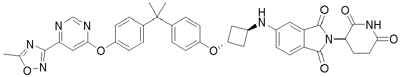 |
| 589 | 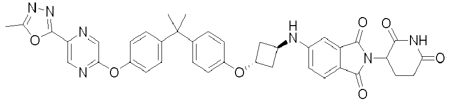 | 590 | 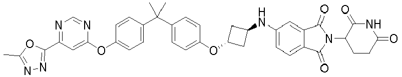 |
| 591 | 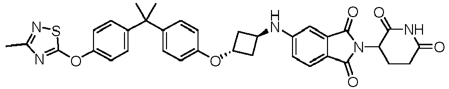 | 592 | 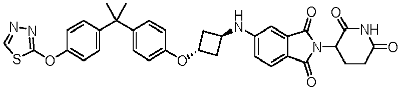 |
| 593 | 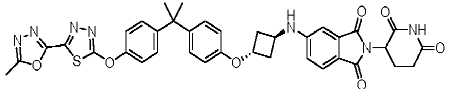 | 594 | 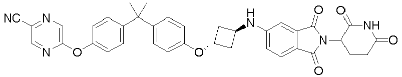 |
| 595 | 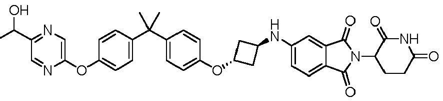 | 596 | 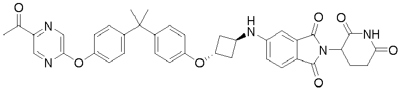 |
| 597 | 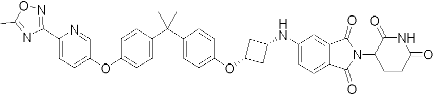 | 598 | 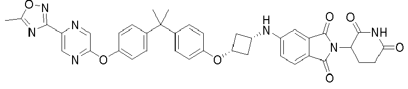 |
| 599 | 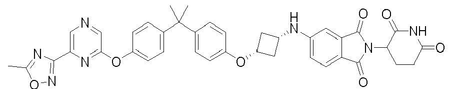 | 600 | 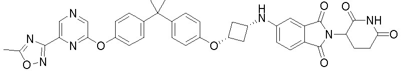 |
| 601 | 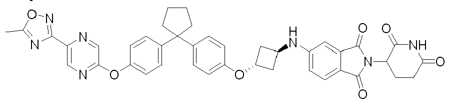 | | |

33. A medicament, **characterized in that** it is a preparation formed by the compound according to any one of claims 1 to 32, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof, as the acetive ingredient, in combination with pharmaceutically acceptable excipients.

34. The compound according to any one of claims 1 to 32, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a mixture thereof, or a metabolite thereof, or a metabolic precursor thereof, or an isotope-substituted form thereof for use in the manufacturer of proteolysis targeting chimeras (PROTAC) for androgen receptors (AR).

35. The use according to claim 34, **characterized in that** the proteolysis targeting chimeras can target the recognition/binding of androgen receptors.

36. The use according to claim 34, **characterized in that** the proteolysis targeting chimeras can degrade androgen receptors.

37. The use according to claim 34, **characterized in that** the androgen receptors include wild-type and mutant androgen receptors.

38. The use according to claim 37, **characterized in that** the mutant androgen receptor comprises those obtained by a splice site mutation and a point mutation in androgen receptors; the preferred is an androgen receptor AR-v7 obtained by a splice site mutation.

39. The use according to claim 34, **characterized in that** the proteolysis targeting chimeras are medicaments for treating diseases regulated by androgen receptors.

40. The use according to claim 39, **characterized in that** the disease is cancer, alopecia, acne or corona virus disease 2019 (COVID-19).

41. The use according to claim 40, **characterized in that** the cancer is that with positive expression of androgen receptors.

42. The use according to claim 40, **characterized in that** the cancer is drug-resistant.

43. The use according to claim 41 or 42, **characterized in that** the cancer is prostate cancer, breast cancer, ovarian cancer, bladder cancer, pancreatic cancer, hepatocellular carcinoma, endometrial cancer or salivary gland cancer.
